(19) Europäisches Patentamt
European Patent Office
Office européen des brevets

(11) EP 4 663 205 A1

(12) **EUROPEAN PATENT APPLICATION**
published in accordance with Art. 153(4) EPC

(43) Date of publication:
**17.12.2025 Bulletin 2025/51**

(21) Application number: **24796329.1**

(22) Date of filing: **28.04.2024**

(51) International Patent Classification (IPC):
***A61K 47/65*** *(2017.01)* ***A61K 47/54*** *(2017.01)*
***A61P 35/00*** *(2006.01)*

(52) Cooperative Patent Classification (CPC):
**A61K 47/60; A61K 47/54; A61K 47/645; A61K 47/65; A61P 35/00**

(86) International application number:
**PCT/CN2024/090418**

(87) International publication number:
**WO 2024/222960 (31.10.2024 Gazette 2024/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.04.2023 CN 202310474355**

(71) Applicant: **Shanghai Best-Link Bioscience, LLC**
**Shanghai 201203 (CN)**

(72) Inventors:
- **ZHANG, Fuyao**
  **Shanghai 201203 (CN)**
- **CHEN, Xianjie**
  **Shanghai 201203 (CN)**
- **WAN, Jiaxun**
  **Shanghai 201203 (CN)**

(74) Representative: **Murgitroyd & Company**
**165-169 Scotland Street**
**Glasgow G5 8PL (GB)**

(54) **POLYMER-DRUG CONJUGATE, INTERMEDIATE THEREOF, AND USE THEREOF**

(57) A nano-size controllable and stable polymer-drug conjugate, an intermediate thereof, and a use thereof. Provided is a polymer-drug conjugate as represented by formula (I) and having different polymer bodies, a controllable coupled group quantity, controllable group coupling sites and a controllable nano-size. The drug conjugate has one or more of the following advantages: a low renal clearance rate, a low liver-spleen clearance rate, long plasma half-life, strong drug accumulation capability at lesion tissues, strong drug permeability at lesion tissues, low toxic and side effects, and an excellent treatment effect.

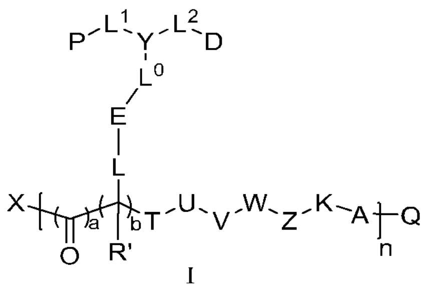

I

EP 4 663 205 A1

## Description

The present application claims priority to the Chinese Patent Application No. 2023104743553 filed on April 28, 2023, which is incorporated herein by reference in its entirety.

TECHNICAL FIELD

The present disclosure relates to the field of drug delivery, and particularly, to a polymer-drug conjugate, an intermediate thereof, and use thereof.

BACKGROUND

Cancer is one of the biggest health problems worldwide, with millions of unfortunates developing malignancies every year. With the accelerated aging of the global population and the rising morbidity of cancer, the clinical need for cancer therapy is on the rise. The global market for anti-tumor agents is steadily increasing, with an estimated increase to about $230 billion by 2026 according to a study. Chemotherapeutics are the most important therapies against tumors, which usually, however, possess the remarkable disadvantages of high toxicity, severe adverse effects, drug resistance, low *in vivo* delivery efficiency, and the like.

Anti-tumor drug delivery is a very challenging field due to the great pathological and physiological differences between tumor tissues and normal tissues, such as the high compactness and exclusive nature of tumor tissues and the diversity and complexity of intercellular substance in the tumor microenvironment, which limit the diffusion of drugs in tumor tissues and pose difficulties in infiltrating the tumor tissue matrix and the interior. The nano-drug delivery system is a very effective delivery means possessing improved bioavailability, enhanced stability and solubility, among other advantages. It may further enhance the targeted enrichment effect of the drug in tumor tissues, reduce the adverse effects of the drug on normal tissues, and improve the anti-tumor efficacy.

Nano-drugs relate to a plurality of aspects such as *in vivo* circulation stability, permeability of tumor tissues, and endocytosis of the drug by tumor cells in the system delivery process, and such processes are related to the size of the nano-drug. For example, antibody-drug conjugates (ADCs) may have a molecular weight of about 160 thousand and a nano-scale size (about 10 nm). However, ADCs are physically and chemically unstable due to environmental factors, enzymatic degradation, and storage conditions, and may be easily aggregated or disaggregated. The nano-scale size may vary due to the instability during the *in vivo* delivery. For example, the ADC may bind to a protein during blood circulation to form a super-nano-scale protein corona, or the ADC molecules may be aggregated during the preservation to form super-nano-scale particles, resulting in poor infiltration and affected efficacy.

Peptide-drug conjugates (PDCs) usually possess a molecular weight of less than 5000 and a small molecular size (less than 1 nm). PDCs have high infiltration capacities in tumor tissues in the *in vivo* delivery, but may be easily filtered and excreted in the kidneys, leading to reduced *in vivo* half-lives. In order to achieve a good therapeutic effect, the dose and frequency of PDCs may be elevated, thereby resulting in poor safety.

Polymer-conjugated drugs are drug delivery systems formed by coupling therapeutic drugs to polymers through specific linkers. For example, dendrimer-drug conjugates with certain nano-scale sizes can be obtained by coupling therapeutic drugs to dendrimers. However, such conjugated drugs are prone to aggregation and thus formation of nanoparticles with a size greater than 50 nm during the formulation manufacture and preservation (Zhang, Chengyuan, et al. Acta biomaterialia, 2017, 55:153-162; Zhang, Chengyuan, et al. Polymer Chemistry, 2014 (5)18:5227-5235.), which makes the tissue distribution, tissue infiltration, and *in vivo* active drug release rate uncertain. Also, due to the compact structure of the dendritic macromolecules, the *in vivo* active drug release rate is reduced, thus affecting the efficacy and leading to safety issues due to elevated doses to provide sufficient therapeutic effects. In addition, the dendrimer-drug conjugate poses difficulties in synthesis and scale-up, cost-inefficiency, and the like.

SUMMARY

The present disclosure is intended to solve the problems of the above conjugate drugs. The compound disclosed herein is a novel polymer-drug conjugate, particularly, a polymer-drug conjugate with natural amino acid polypeptide as the backbone, which possesses the advantages of good biocompatibility, mild adverse effects, and high safety. Through reasonable design of polypeptide molecules and linkers, the control of the number of conjugated groups and the nano-scale size can be achieved. Such novel polymer-drug conjugates are neutral, small nano-scale, stable nanoparticles. The uncharged surface of the nanoparticles can prevent the aggregation of the nanoparticles, thus ensuring the stability of the small nanoparticles in the preparation process and the storage process. Also, the neutral nature of the nanoparticles greatly reduces the possibility of forming a large protein corona by combining with proteins in the blood, and thus greatly improves the infiltration of the novel polymer-drug conjugates in tumor tissues. In addition, linkers releasing the conjugated

moieties in response to different mechanisms are designed, so as to achieve high selectivity of drugs to target tissues and the adjustable drug release speed, thus improving the efficacy while reducing adverse effects. The conjugation points of the novel polymer-drug conjugate are stable, and the size of the formed nanoparticle is greater than the filtration threshold in glomeruli, thereby providing stability in the blood circulation and prolonged half-life, reduced doses and frequency, and the balance between efficacy and safety. Therefore, the compound of the present disclosure has very broad application prospects and market potential.

The present disclosure solves the above-mentioned technical problem through the following technical solutions.

The present disclosure is intended to provide a stable polymer-drug conjugate of formula (I) with a controllable number of conjugated groups and a controllable nano-scale size,

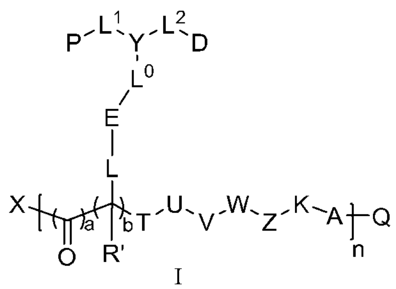

I

comprising:

(1) a polymer residue of formula (II), with a polymer backbone repeating unit number n,

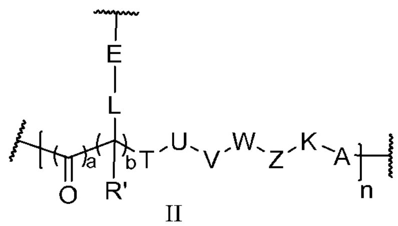

II

,

wherein n is selected from integers of 4-100;

(2) a branched center Y having at least trifunctionality;

(3) a pharmacokinetic regulator residue P;

(4) a pharmaceutically active agent residue D, wherein D may be one or more drug residues;

(5) a terminal group X;

(6) $L^0$, $L^1$, and $L^2$, each independently being a covalent bond or a $C_1$-$C_{40}$ linker either containing a heteroatom or not, wherein the heteroatom is O, S, Se, N, P, Si, or B, the number of the heteroatom(s) is one or more, and when more than one heteroatom is present, the heteroatoms are identical or different; the linker may either contain an unsaturated group or not; $L^0$ links E and Y, $L^1$ links P and Y, and $L^2$ links D and Y; and

(7) a residue Q of formula (III), which is H, $R^a$, a hydroxy protecting group, a sulfhydryl protecting group, an amino protecting group,

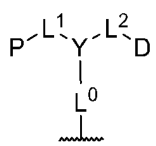

III

wherein, (8) any hydrogen in formula (I) may be substituted by deuterium;

(9) any chiral center in formula (I) may be R configuration, S configuration, or a mixture of R configuration and S configuration.

In some examples, the polymer-drug conjugate has an average nano-scale particle size range of 1-100 nm.

In some examples, the polymer-drug conjugate has an average nano-scale particle size range of 1-50 nm.

In some examples, the polymer-drug conjugate has an average nano-scale particle size range of 5-30 nm.

In some examples, the polymer residue in the polymer-drug conjugate meets one or more of the following conditions:

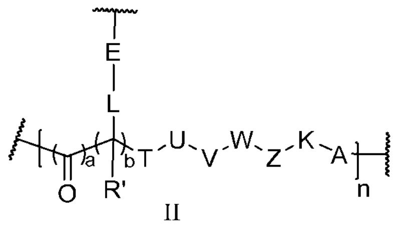

II

(1) L is independently a covalent bond,

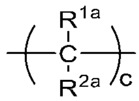

,

or a $C_1$-$C_{10}$ linker either containing a heteroatom or not, wherein the heteroatom is O, S, Se, N, P, Si, or B, the number of the heteroatom(s) is one or more, and when more than one heteroatom is present, the heteroatoms are identical or different; the linker may either contain an unsaturated group or not; L links the polymer backbone and E;

(2) E is independently a covalent bond, O, S, $NR^a$, C(=O), S(=O), $S(=O)_2$, $C(=O)NR^a$, or one of the following residues:

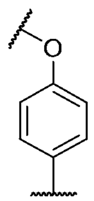 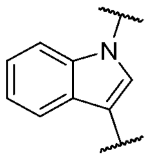 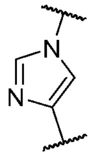 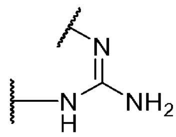 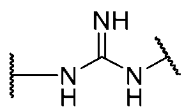 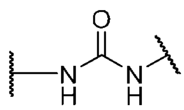 ;

(3) A is independently a covalent bond, O, S, or $NR^d$, wherein $R^d$ is selected from H, substituted or unsubstituted $C_1$-$C_{10}$ alkyl, or a residue of formula (III):

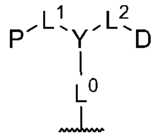

III ;

(4) T, U, V, W, Z, and K are independently a covalent bond, O, S, $NR^d$, C(=O), S(=O), $S(=O)_2$,

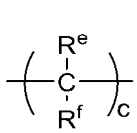 , 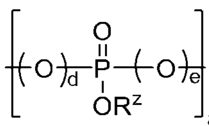 , or 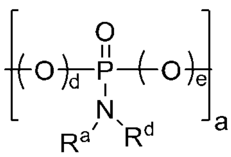 ,

with the proviso that: the -T-U-V-W-Z-K-A- chain does not contain the following linking forms: -O-O-, -O-S-, -S-O-,

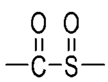 , 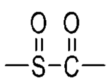 , 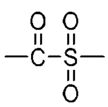 , 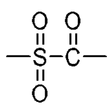 , 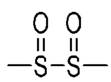 , 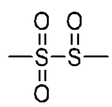 , or 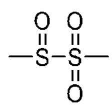 ;

(5) the configuration of the chiral carbon atom in the polymer residue of formula (II) may be *R* configuration, *S*

configuration, or a mixture of *R* configuration and *S* configuration,
wherein,

n is selected from integers of 4-100;
a is selected from 0 and 1;
b is selected from 0 and 1;
c is selected from 0 and integers of 1-10;
d is selected from 0 and 1;
e is selected from 0 and 1;
R', $R^{1a}$, and $R^{2a}$ are selected from hydrogen, deuterium, $C_1$-$C_{10}$ alkyl, $C_1$-$C_{10}$ alkoxy, $C_3$-$C_{10}$ alkenyl, $C_3$-$C_{10}$ alkynyl, $C_3$-$C_8$ cycloalkyl, $C_2$-$C_8$ heterocycloalkyl, $C_6$-$C_{10}$ aryl, or $C_5$-$C_{10}$ heteroaryl;
$R^z$ is selected from hydrogen, $C_1$-$C_{10}$ alkyl, $C_1$-$C_{10}$ alkoxy, $C_3$-$C_{10}$ alkenyl, $C_3$-$C_{10}$ alkynyl, $C_3$-$C_8$ cycloalkyl, $C_2$-$C_8$ heterocycloalkyl, $C_6$-$C_{10}$ aryl, $C_5$-$C_{10}$ heteroaryl, a hydroxy protecting group, or a residue of formula (III):

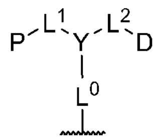

III ;

$R^e$ and $R^f$ are independently selected from hydrogen, deuterium, $C_1$-$C_{10}$ alkyl, $C_1$-$C_{10}$ alkoxy, $C_3$-$C_{10}$ alkenyl, $C_3$-$C_{10}$ alkynyl, $C_3$-$C_8$ cycloalkyl, $C_2$-$C_8$ heterocycloalkyl, $C_6$-$C_{10}$ aryl, $C_3$-$C_{10}$ heteroaryl, or a residue of formula (IV):

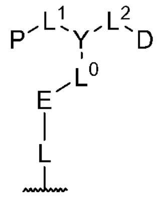

IV ;

$R^a$ is selected from H, $C_1$-$C_{10}$ alkyl, $C_1$-$C_{10}$ alkoxy, $C_3$-$C_{10}$ alkenyl, $C_3$-$C_{10}$ alkynyl, $C_3$-$C_8$ cycloalkyl, $C_2$-$C_8$ heterocycloalkyl, $C_6$-$C_{10}$ aryl, $C_5$-$C_{10}$ heteroaryl, or an amino protecting group;
$R^d$ is selected from H, $C_1$-$C_{10}$ alkyl, $C_1$-$C_{10}$ alkoxy, $C_3$-$C_{10}$ alkenyl, $C_3$-$C_{10}$ alkynyl, $C_3$-$C_8$ cycloalkyl, $C_2$-$C_8$ heterocycloalkyl, $C_6$-$C_{10}$ aryl, $C_5$-$C_{10}$ heteroaryl, an amino protecting group, or a residue of formula (III):

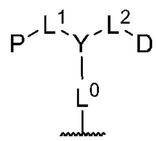

III ;

the heteroatom in the $C_2$-$C_8$ heterocycloalkyl is O, S, or N, the number of the heteroatom(s) is one or more, and when more than one heteroatom is present, the heteroatoms are identical or different; the heteroatom in the $C_5$-$C_{10}$ heteroaryl is O, S, or N, the number of the heteroatom(s) is one or more, and when more than one heteroatom is present, the heteroatoms are identical or different.

In some examples, the polymer-drug conjugate meets one or more of the following conditions:

(1) the polymer backbone repeating unit number n is an integer of 5-70, preferably an integer of 20-40;
(2) the branched center Y is a branched center containing the following structure, or a multifunctional branched center consisting of two or more branching structures:

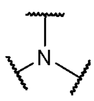 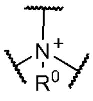 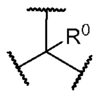 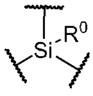 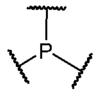 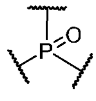 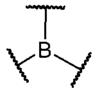

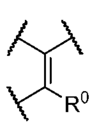 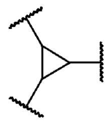 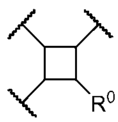 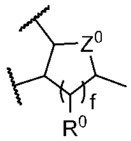 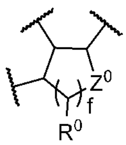 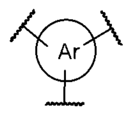

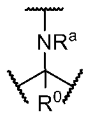 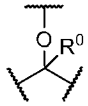 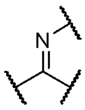 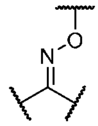 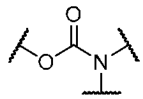 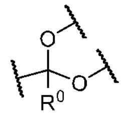 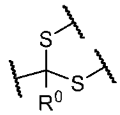

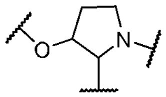 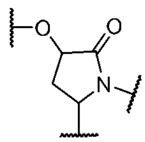 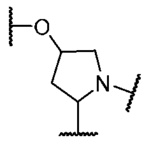

wherein, $Z^0$ is O, S, S(O), $S(O)_2$, $NR^a$, or $CHR^0$;

$R^0$ is selected from H, D, halogen, nitro, cyano, $C_1$-$C_{10}$ alkyl, $C_1$-$C_{10}$ alkoxy, $C_3$-$C_{10}$ alkenyl, $C_3$-$C_{10}$ alkynyl, $C_3$-$C_8$ cycloalkyl, $C_2$-$C_8$ heterocycloalkyl, $C_6$-$C_{10}$ aryl, $C_5$-$C_{10}$ heteroaryl, or a group containing a primary amine, secondary amine, tertiary amine, hydroxy, sulfhydryl, carboxyl, ester group, amide, boric acid, borate, phosphoric acid, sulfonic acid, sulfoxide, aldehyde group, or ketone functional group; the heteroatom in the $C_2$-$C_8$ heterocycloalkyl is O, S, or N, the number of the heteroatom(s) is one or more, and when more than one heteroatom is present, the heteroatoms are identical or different; the heteroatom in the $C_5$-$C_{10}$ heteroaryl is O, S, or N, the number of the heteroatom(s) is one or more, and when more than one heteroatom is present, the heteroatoms are identical or different;

Ar is $C_6$-$C_{20}$ aryl or $C_5$-$C_{20}$ heteroaryl; the heteroatom in the $C_5$-$C_{20}$ heteroaryl is O, S, or N, the number of the heteroatom(s) is one or more, and when more than one heteroatom is present, the heteroatoms are identical or different;

f is 0 or an integer of 1-3,

wherein $R^a$ is as defined in formula (I).

In some examples, the branched center Y of the polymer-drug conjugate is a substituted or unsubstituted amino acid or a derivative thereof having at least trifunctionality, the amino acid is a natural amino acid or an unnatural amino acid, the configuration of the amino acid is *D* or *L*, or a mixture of *D*/*L* configurations, and when the amino acid is a mixture of *D*/*L* configurations, the proportion of *L* configuration is greater than 0% but less than 100%; the branched center Y is preferably an amino acid having at least trifunctionality, and the amino acid is selected from one or more of aspartic acid, glutamic acid, lysine, ornithine, arginine, citrulline, histidine, serine, threonine, tryptophan, tyrosine, hydroxyproline, cystine, cysteine, and selenocysteine, the configuration of the amino acid is *D* or *L*, or a mixture of *D*/*L* configurations, and when the amino acid is a mixture of *D*/*L* configurations, the proportion of *L* configuration is greater than 0% but less than 100%.

In some examples, the linkers $L^0$ and $L^1$ of the polymer-drug conjugate are each independently selected from a covalent bond, an environmentally responsive linker, or a non-environmentally responsive linker; $L^2$ is an environment-responsive linker with a structure of $L^{2a}$-$L^{2b}$, wherein $L^{2a}$ or $L^{2b}$ may be present alone or together; $L^{2a}$ and $L^{2b}$ are each independently selected from a covalent bond, an environmentally responsive linker, or a non-environmentally responsive linker; the structure of the polymer-drug conjugate has a structure of formula (V):

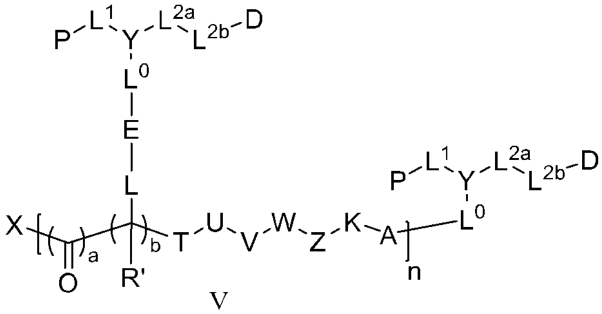

V

wherein, X, R', T, U, V, W, Z, K, A, P, $L^1$, Y, $L^{2a}$, $L^{2b}$, D, $L^0$, E, L, a, and b are each as defined in formula (I).

In some examples, the linkers $L^0$ and $L^1$ of the polymer-drug conjugate are covalent bonds, and $L^2$ is an environment-responsive linker with a structure of $L^{2a}$-$L^{2b}$, wherein $L^{2a}$ is linked to Y, $L^{2b}$ is linked to D, and $L^{2a}$ or $L^{2b}$ may be present alone or together; $L^{2a}$ and $L^{2b}$ are each independently selected from a covalent bond, an environmentally responsive linker, or a non-environmentally responsive linker; the structure of the polymer-drug conjugate has a structure of formula (VI):

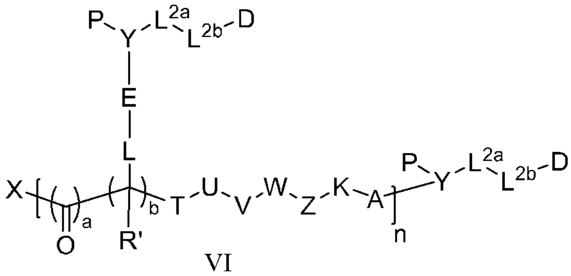

VI

wherein, X, R', T, U, V, W, Z, K, A, P, Y, $L^{2a}$, $L^{2b}$, D, E, L, a, and b are each as defined in formula (I).

In some examples, the polymer residue in the polymer-drug conjugate is selected from the following structures:

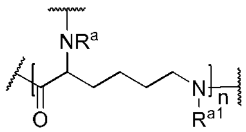
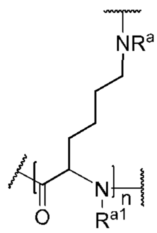
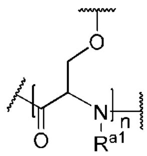
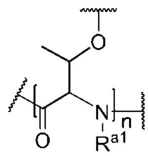
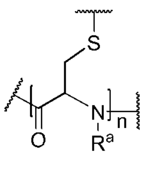
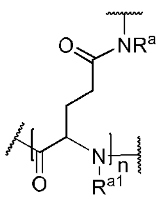
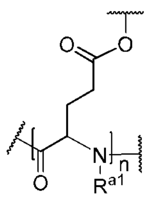
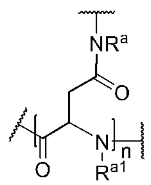
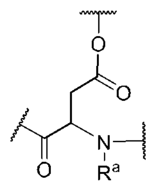
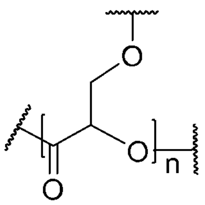
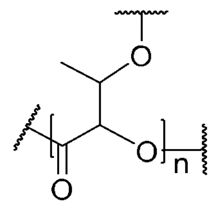
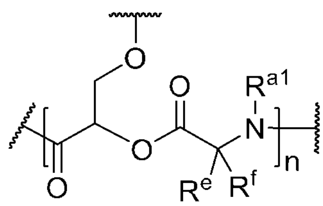

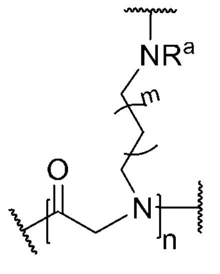
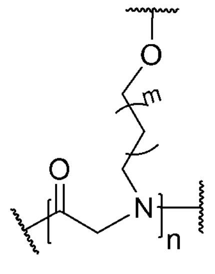
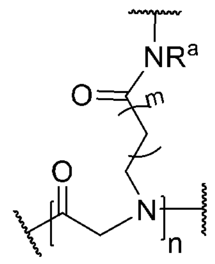
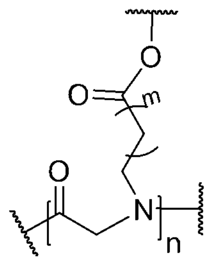

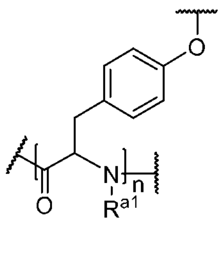
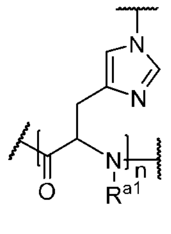
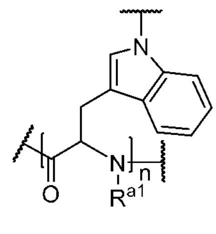
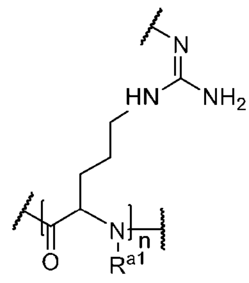

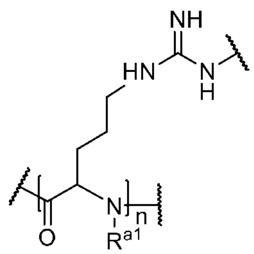
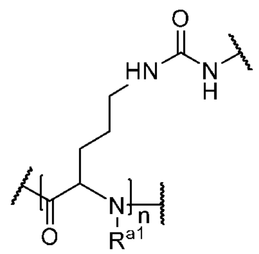
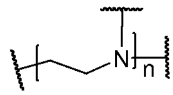

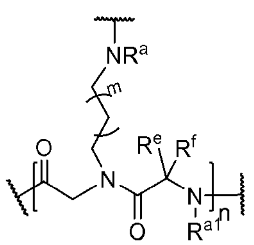
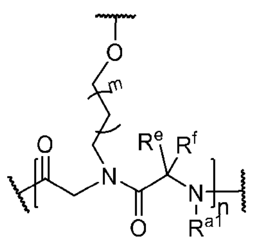
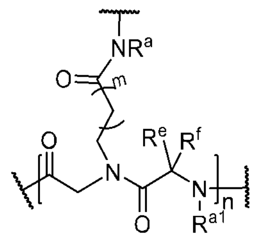
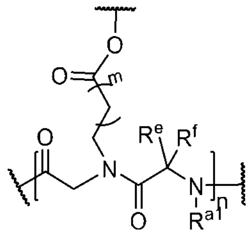

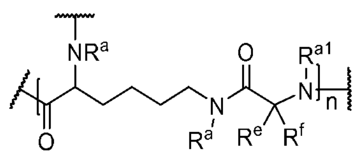
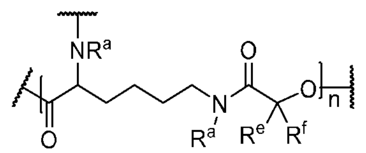

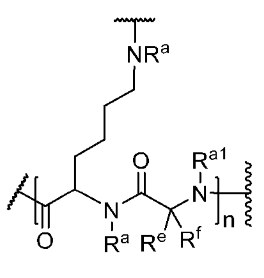
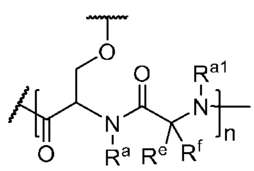
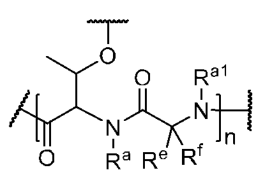
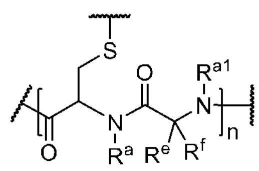

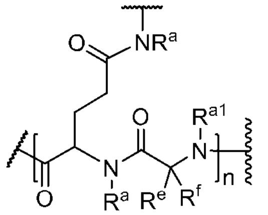
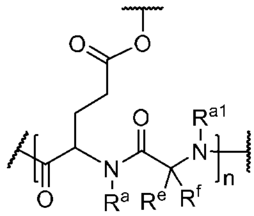
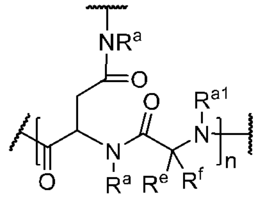
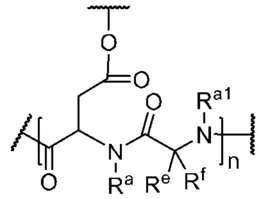

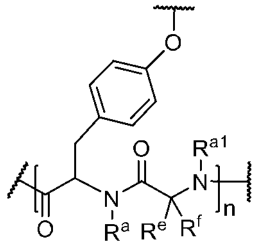
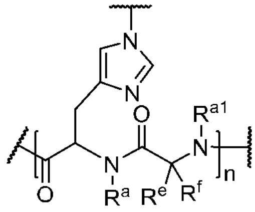
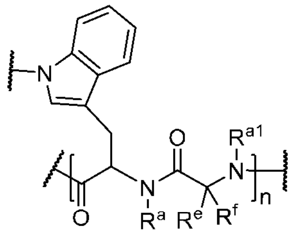

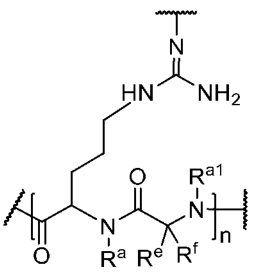
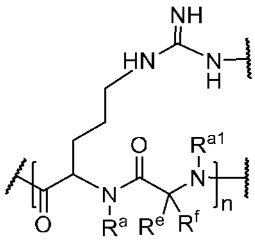
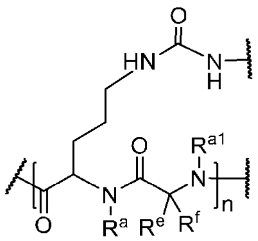

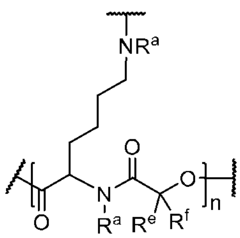
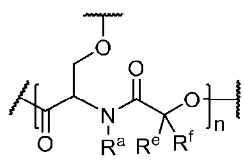
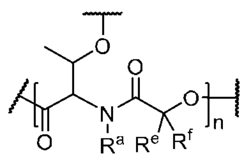
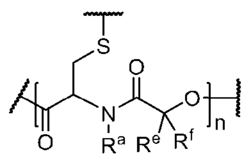

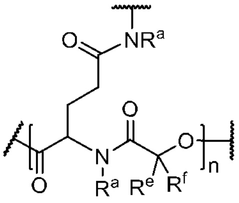
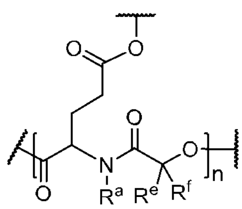
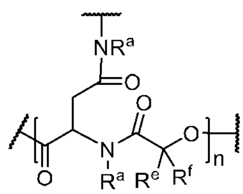
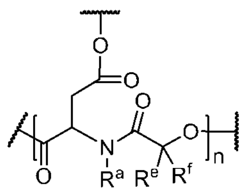

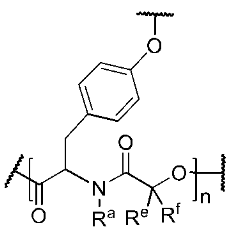
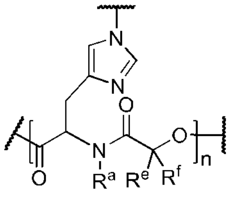
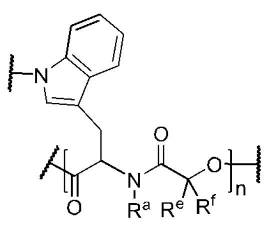

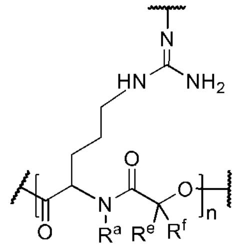
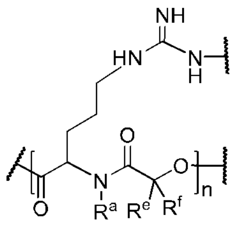
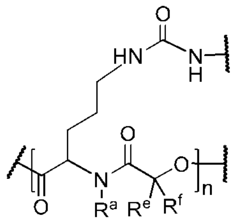

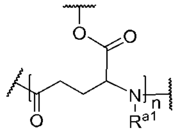
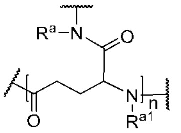
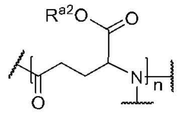
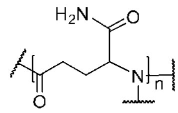
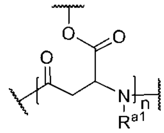

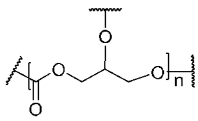
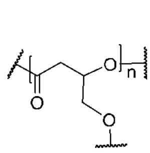
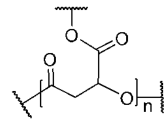
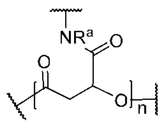
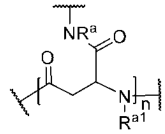

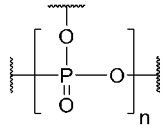
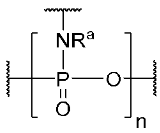
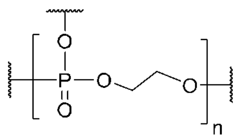
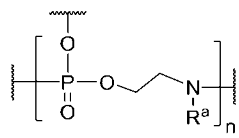

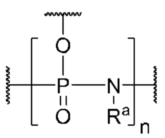
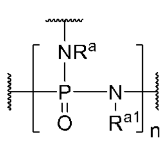
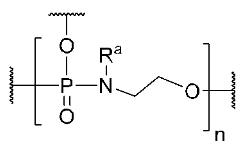
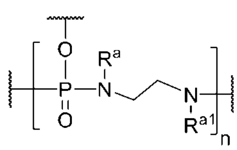

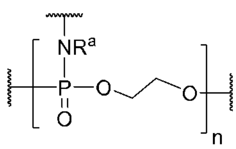
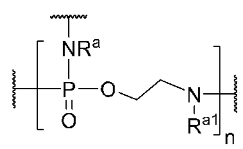
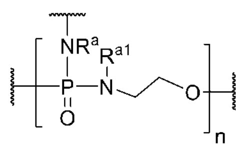
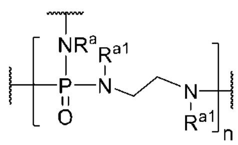

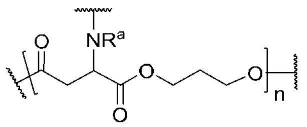
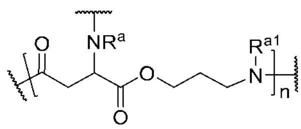
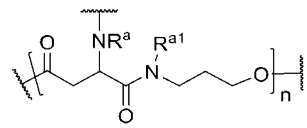

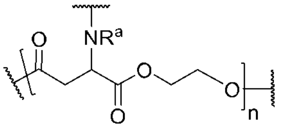
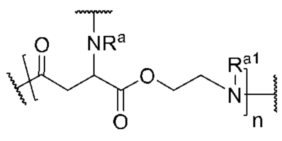
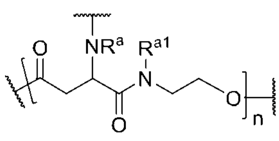

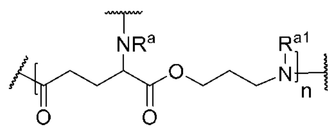
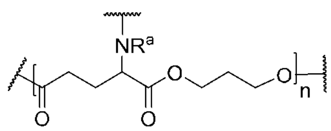
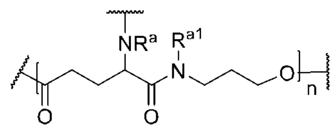

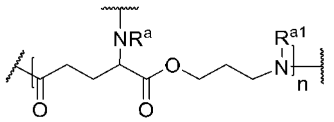 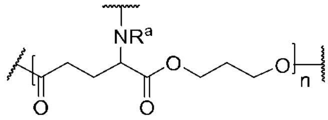 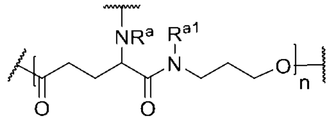

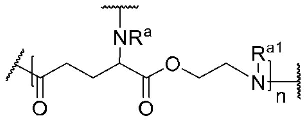 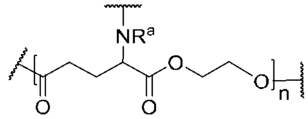 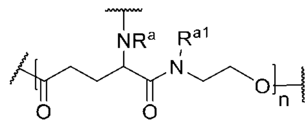

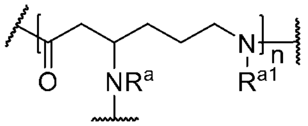 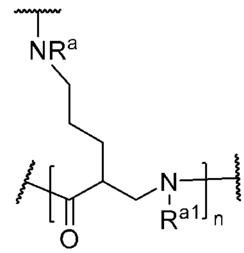 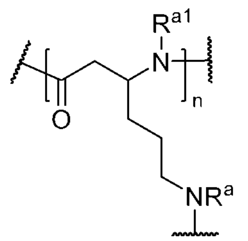

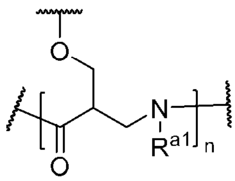 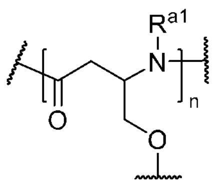 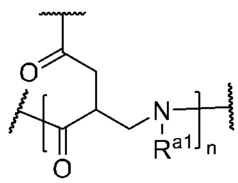 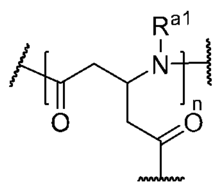

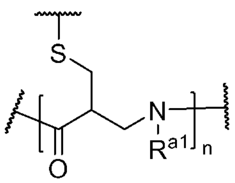 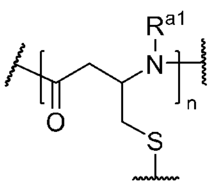 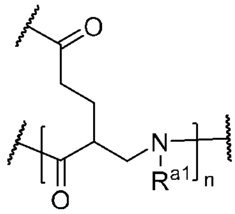 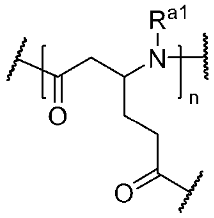

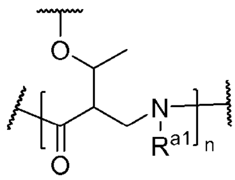 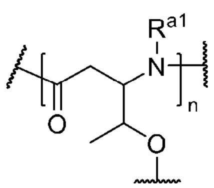

wherein, $R^{a1}$ is selected from H, $C_1$-$C_{10}$ alkyl, $C_1$-$C_{10}$ alkoxy, $C_3$-$C_{10}$ alkenyl, $C_3$-$C_{10}$ alkynyl, $C_3$-$C_8$ cycloalkyl, $C_2$-$C_8$ heterocycloalkyl, $C_6$-$C_{10}$ aryl, $C_5$-$C_{10}$ heteroaryl, an amino protecting group, or a residue of formula (III);

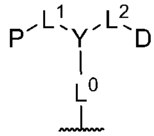

III ;

$R^{a2}$ is selected from H, $C_1$-$C_{10}$ alkyl, $C_1$-$C_{10}$ alkoxy, $C_3$-$C_{10}$ alkenyl, $C_3$-$C_{10}$ alkynyl, $C_3$-$C_8$ cycloalkyl, $C_2$-$C_8$ heterocycloalkyl, $C_6$-$C_{10}$ aryl, $C_5$-$C_{10}$ heteroaryl, or a hydroxy protecting group;
n is selected from integers of 4-100;
m is selected from integers of 0-5;
$R^a$, $R^e$, and $R^f$ are each as defined in formula (I).

In some examples, when an electrophilic group in Y is linked to $L^2$, $L^{2a}$ is absent, i.e., $L^2 = L^{2b}$, and the linkage of the trifunctional branched center Y to the linkers $L^0$, $L^1$, and $L^{2b}$ is selected from any of the following structures:

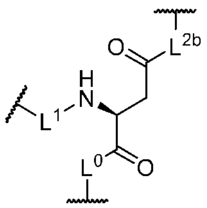 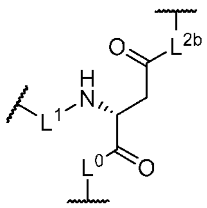 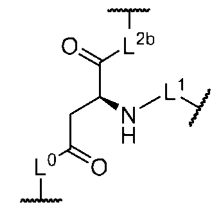 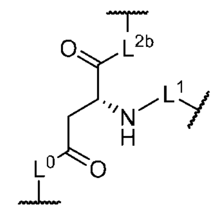

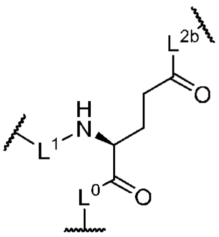 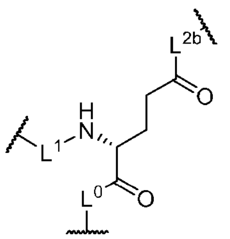 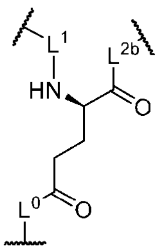 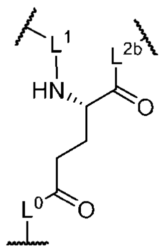 ,

preferably

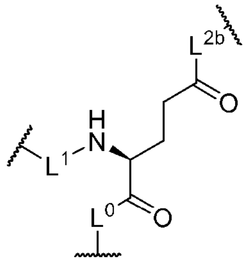 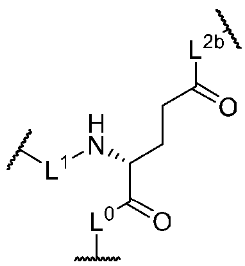 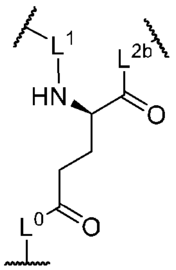 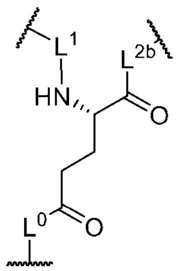 ,

and more preferably

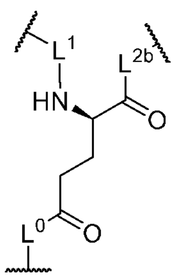 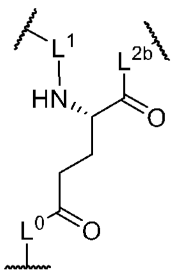 ;

or, when a nucleophilic group in Y is linked to $L^2$, $L^{2a}$ is present alone or $L^{2a}$ and $L^{2b}$ are present together, i.e., $L^2 = L^{2a}$ or $L^2 = L^{2a}$-$L^{2b}$, and the linkage of the trifunctional branched center Y to the linkers $L^0$, $L^1$, $L^{2a}$, and $L^{2b}$ is selected from any of the following structures:

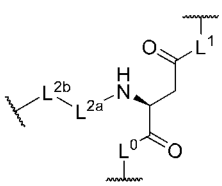 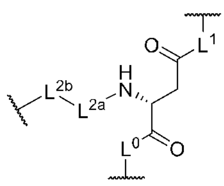 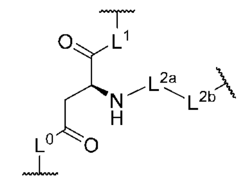 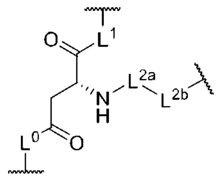

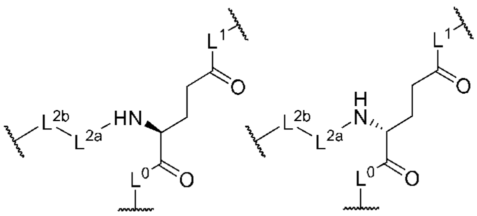 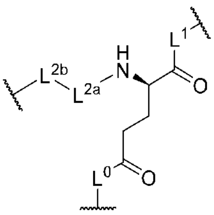 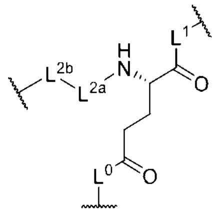

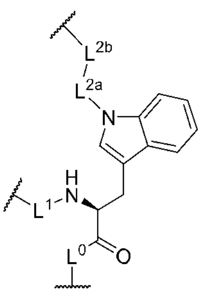 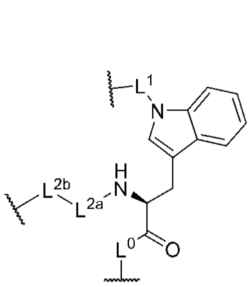 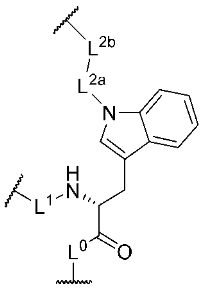 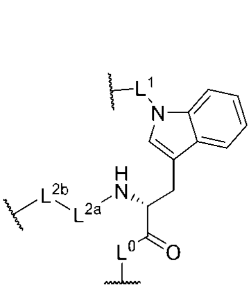

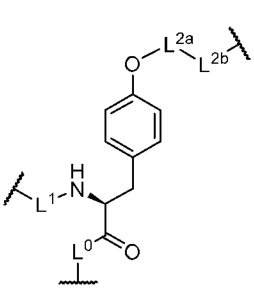 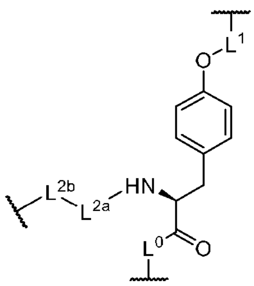 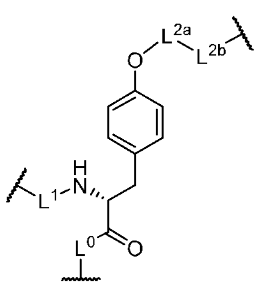 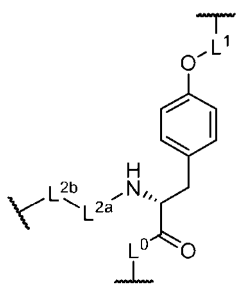

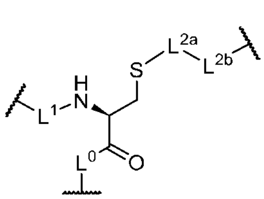 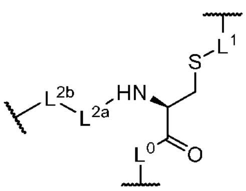 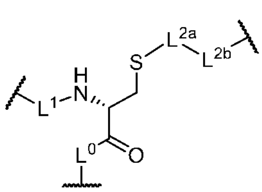 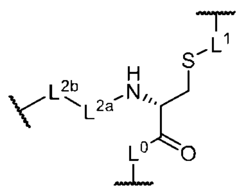

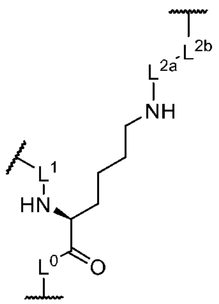 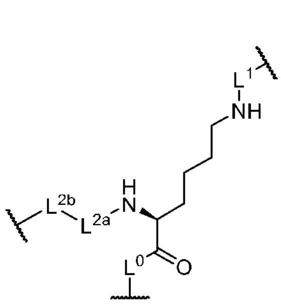 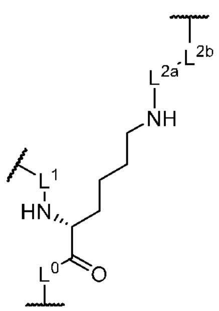 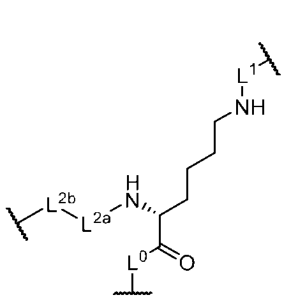

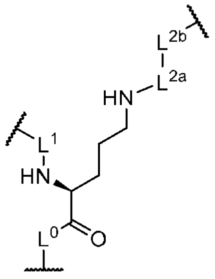
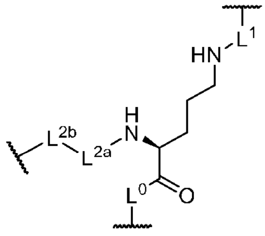
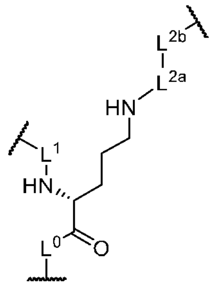
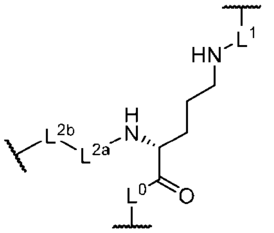

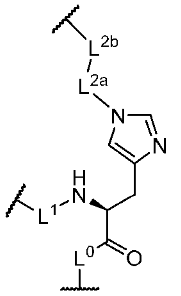
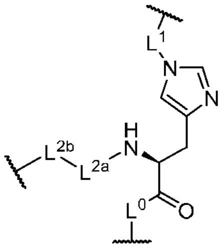
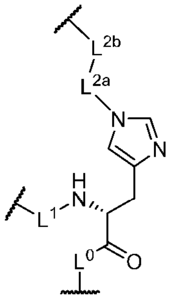
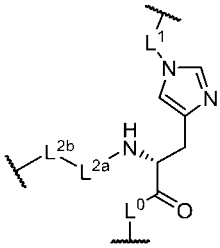

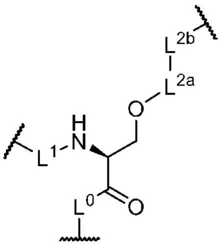
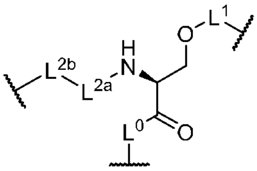
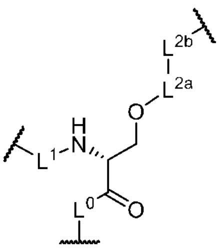
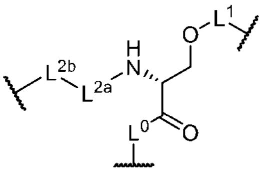

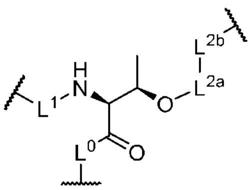
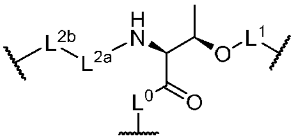
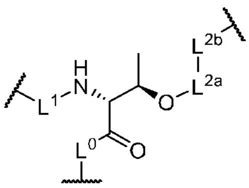
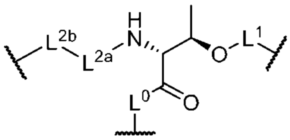

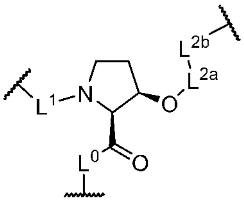
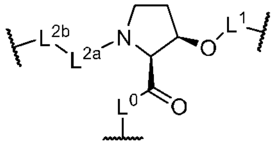
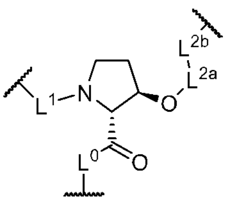
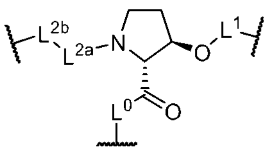

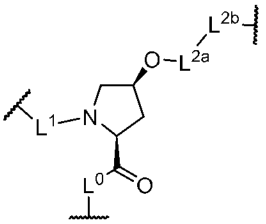
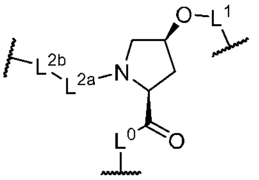
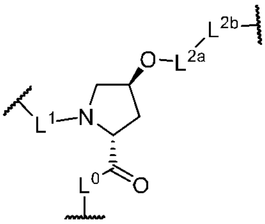
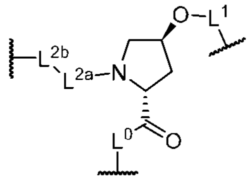

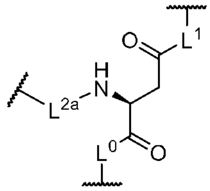
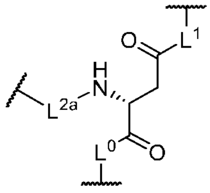
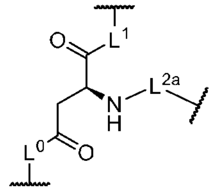
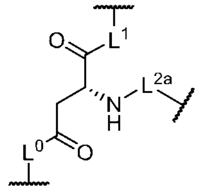

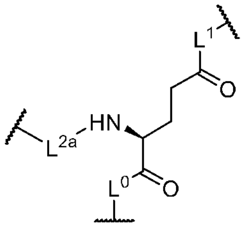
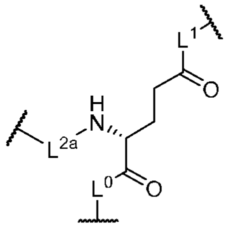
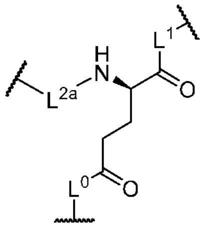
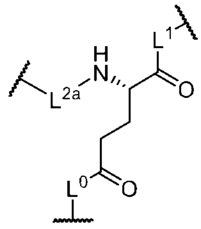

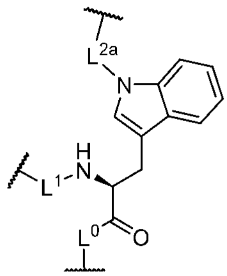
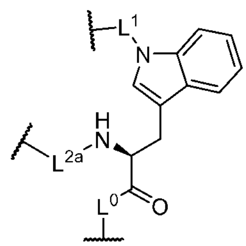
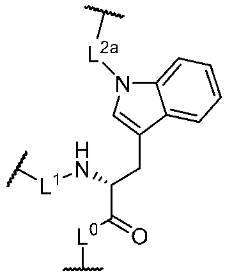
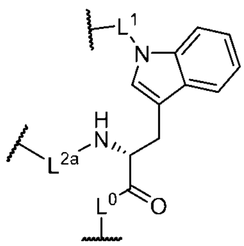

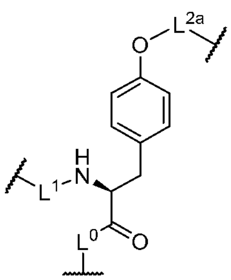
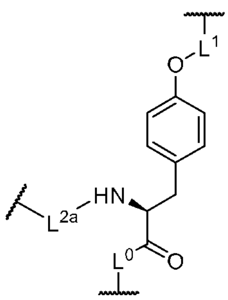
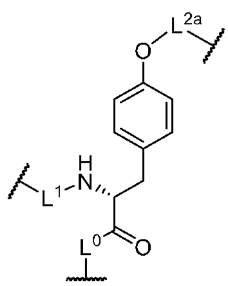
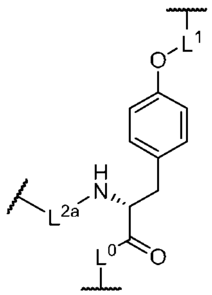

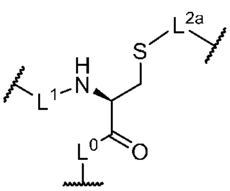
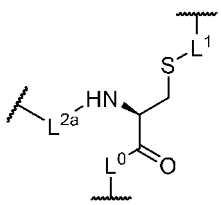
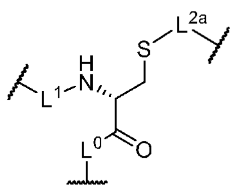
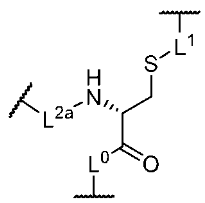

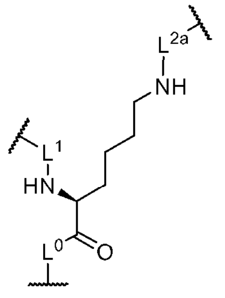
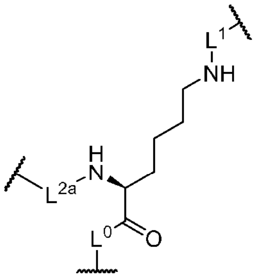
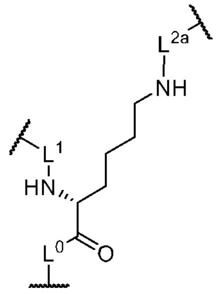
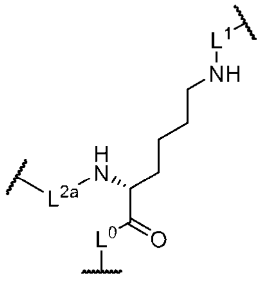

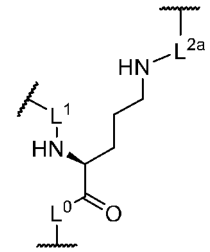 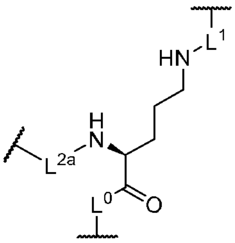 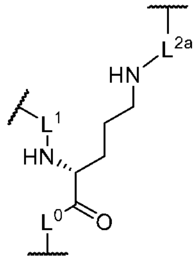 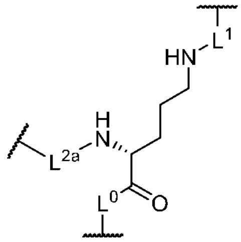

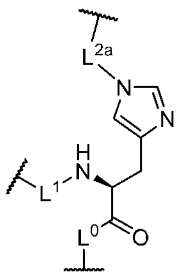 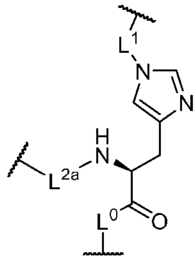 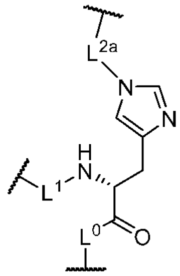 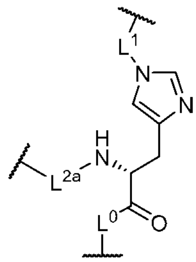

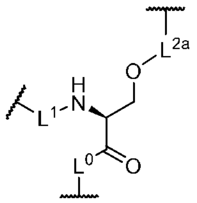 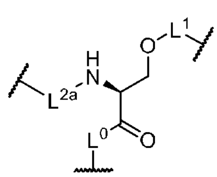 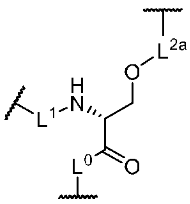 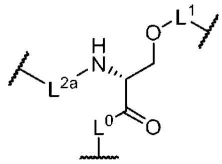

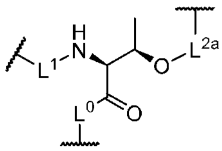 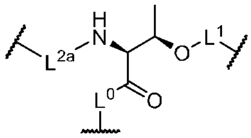 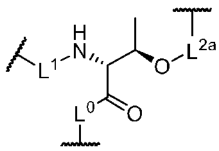 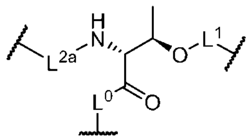

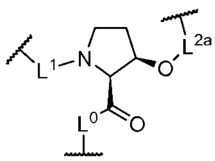 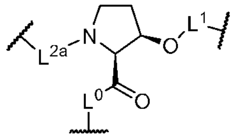 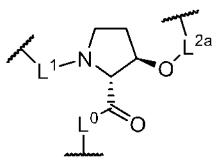 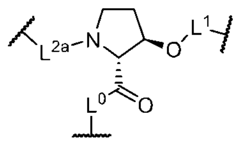

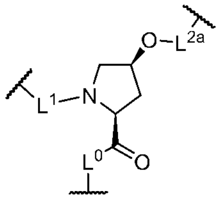 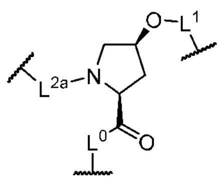 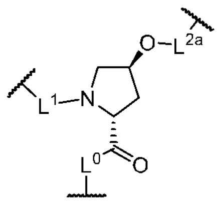 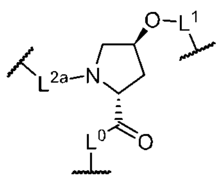 ;

preferably

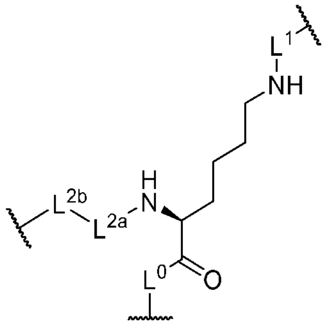

,

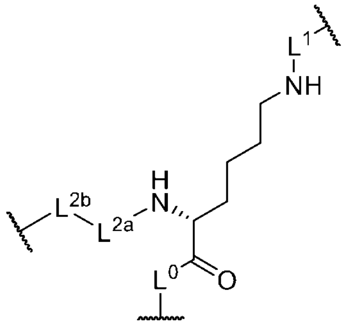

,

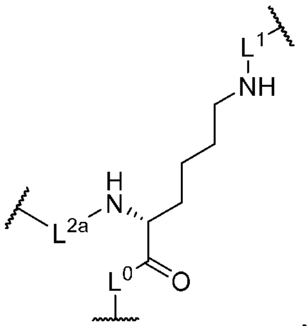

, and

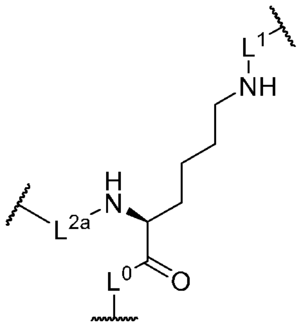

;

wherein, $L^0$, $L^1$, $L^{2a}$, and $L^{2b}$ are each as defined in formula (I).

In some examples, the terminal group X is selected from OR, SR, $NR^1R^2$, a carboxyl protecting group, or $L^{2b}$-D, and the R, $R^1$, and $R^2$ are independently selected from H, $C_1$-$C_{30}$ alkyl, $C_1$-$C_{30}$alkoxy, $C_3$-$C_{30}$ alkenyl, $C_3$-$C_{30}$ alkynyl, $C_3$-$C_8$ cycloalkyl, $C_2$-$C_8$ heterocycloalkyl, $C_6$-$C_{20}$ aryl, or $C_5$-$C_{20}$ heteroaryl; $R^1$ and $R^2$, together with the N atom to which they are linked, may form a $C_2$-$C_8$ heterocycloalkyl; the heteroatom in the $C_2$-$C_8$ heterocycloalkyl is O, S, or N, the number of the heteroatom(s) is one or more, and when more than one heteroatom is present, the heteroatoms are identical or different; the heteroatom in the $C_5$-$C_{20}$ heteroaryl is O, S, or N, the number of the heteroatom(s) is one or more, and when more than one heteroatom is present, the heteroatoms are identical or different; the terminal group X is preferably OR, SR, $NR^1R^2$, a carboxyl protecting group, or $L^{2b}$-D, and R, $R^1$, and $R^2$ are independently selected from H or $C_1$-$C_{10}$ alkyl.

In some examples, the environmentally responsive linker in the polymer-drug conjugate is one or more of an enzyme-responsive linker, a pH-responsive linker, a photo-responsive linker, or a redox-responsive linker.

In some examples, the polymer-drug conjugate meets one or more of the following conditions:

(1) the enzyme-responsive linker is cleavable via one or more of the following enzymes: secretory phospholipase A2, acid phosphatase, serum alkaline phosphatase, cytochrome P450, sulfatase, prostate specific antigen, phospholipase A1, phospholipase A2, phospholipase B, phospholipase C, phospholipase D, neutrophil elastase, cysteine protease-3, cathepsin, matrix metalloproteinase, β-glucuronidase, β-galactosidase, DTP, nitroreductase, reduced coenzyme II, aminopeptidase N, carboxylesterase, diaphorase, histone deacetylase, asparaginyl endopeptidase, urokinase-type plasminogen activator, urokinase-type plasminogen activator receptor, and collagenase, preferably, cleavable via one or more of the following enzymes: cysteine protease-3, cathepsin, matrix metalloproteinase, elastase, or β-glucuronidase;

(2) the pH-responsive linker comprises the following structures: one or more of a hydrazone, imine, oxime, carboxylate, thioester, sulfate, sulfonate, orthoester, carbonate, carbamate, substituted carbamate, ketal, acetal, silyl ether, phosphate, borate, phosphoramide, or cis-aconitic acid group;

(3) the photo-responsive linker comprises the following structures: one or more of an o-nitrophenyl, coumarin, benzoin, BODIPY, or cyanine group;

(4) the redox-responsive linker comprises the following structures: one or more of thioketal, phenylboronate, phenylboronic acid, oxalate, vinyl ether, thioether group, aminoacrylate, disulfide group, diselenide group, 2,4-dinitrobenzene sulfonate, 2-azidomethylbenzoate, 4-azidobenzyl, unsaturated ester, or azobenzene group.

In some examples, the enzyme-responsive linker among the linkers of the polymer-drug conjugate comprises the following amino acid sequences: one or more of Cit-Phe, Lys-Lys, Phe-Lys, Arg-Arg, Val-Cit, Val-Ala, Val-Lys, Ala-Lys, Phe-Cit, Leu-Cit, Ile-Cit, Trp-Cit, Phe-Arg, Asn-Pro-Val, Gly-Pro-Nle, Glu-Val-Cit, Glu-Val-Ala, Gly-Phe-Gly, Gly-Phe-Phe, Gly-Leu-Gly, Gly-Val-Ala, Gly-Phe-Leu-Gly, Gly-Phe-Phe-Leu, Gly-Leu-Leu-Gly, Gly-Phe-Tyr-Ala, Gly-Phe-Gly-Phe,

Ala-Gly-Val-Phe, Gly-Phe-Phe-Gly, Gly-Gly-Phe-Gly, Asp-Glu-Val-Asp, Gly-Phe-Leu-Gly-Phe, Gly-Phe-Ala-Gly-Leu-Phe, Gly-Leu-Ala-Ala-Val-Ala, Gly-Gly-Phe-Leu-Gly-Phe, or Gln-Ser-Phe-Arg-Phe-Lys.

In some examples, the polymer-drug conjugate meets one or more of the following conditions:

(1) the enzyme-responsive linker comprises the following structures:

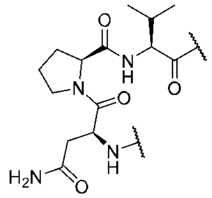 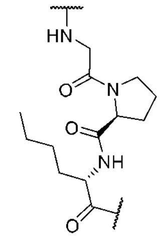 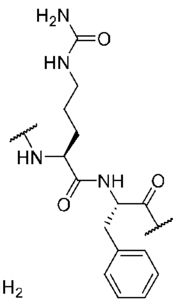 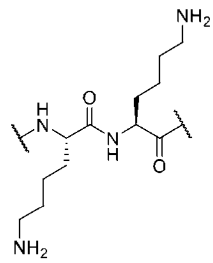

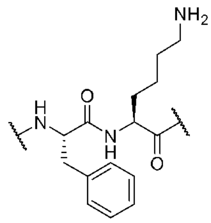 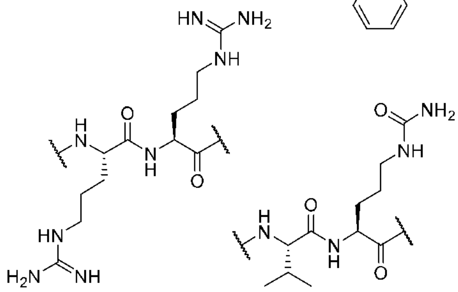 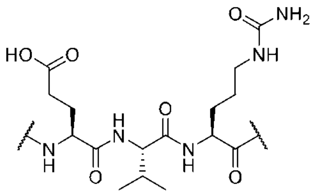

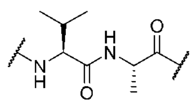 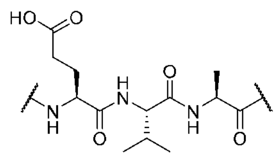 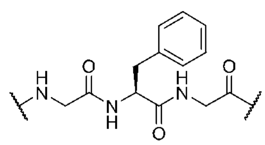 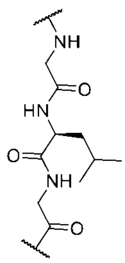

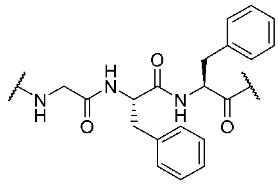 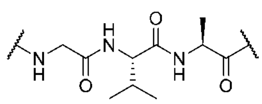 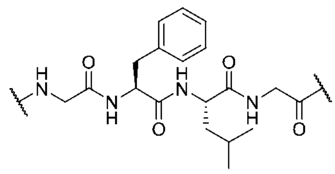

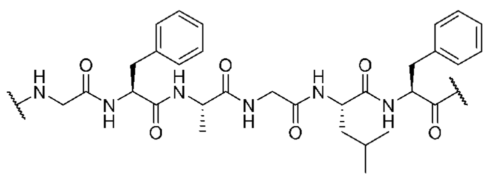 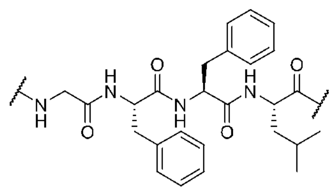

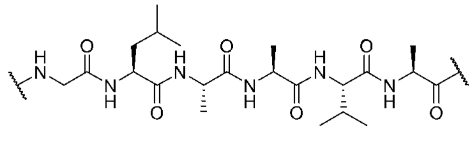 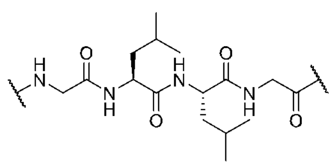

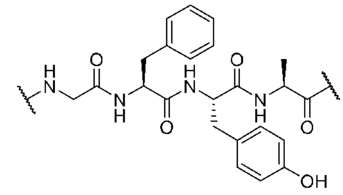

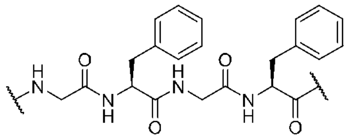

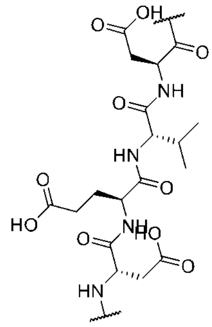

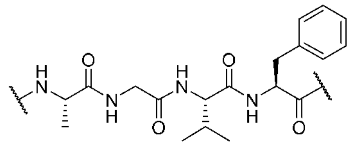

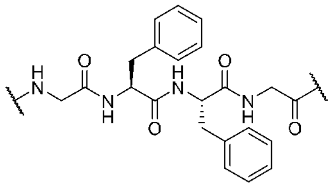

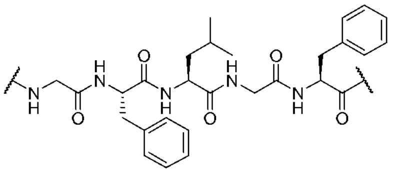

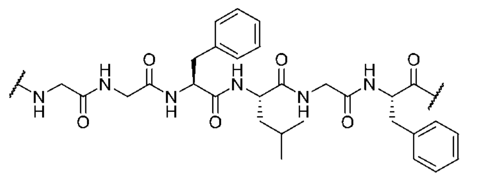

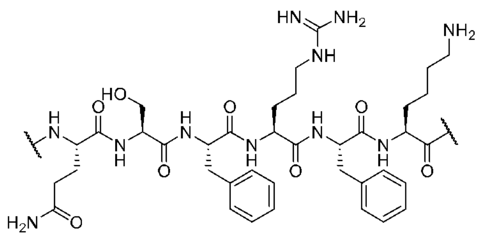

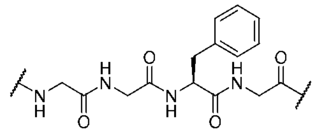

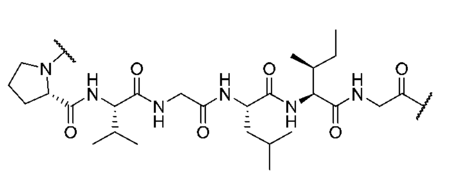

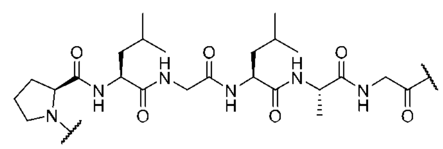

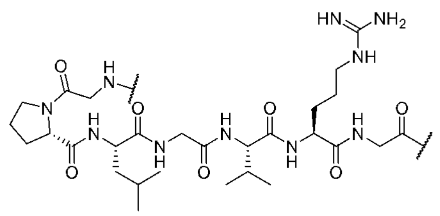

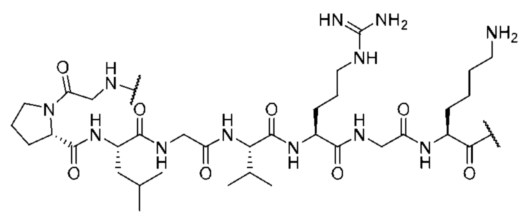

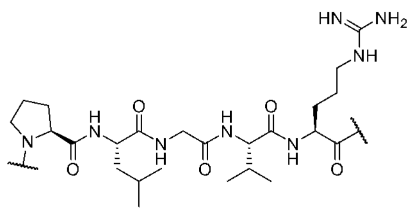

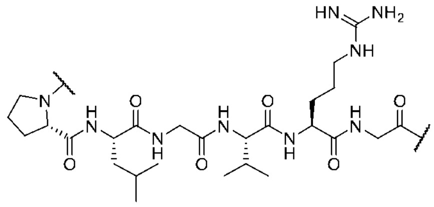

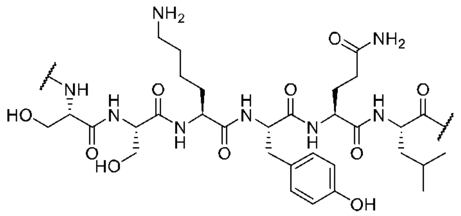

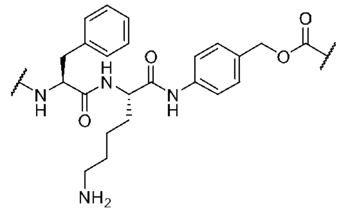

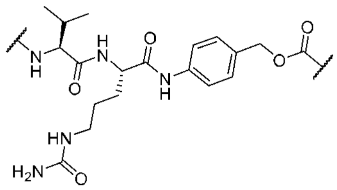

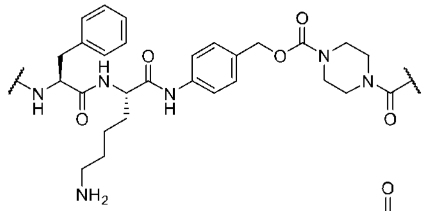

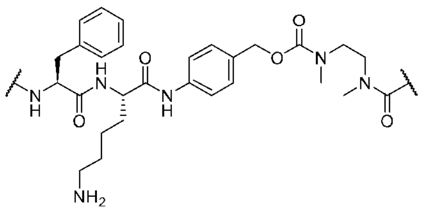

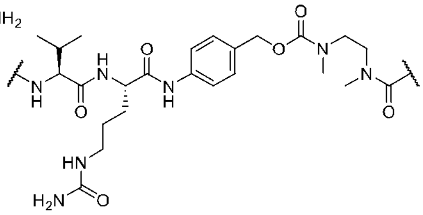

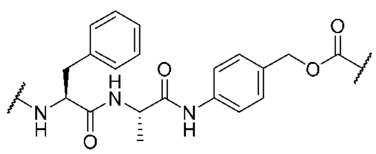

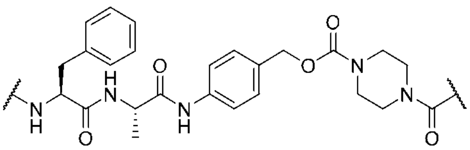

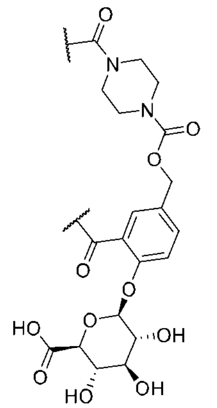

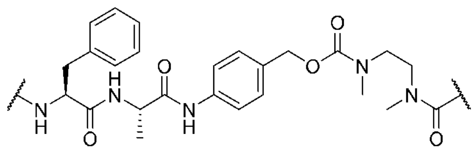

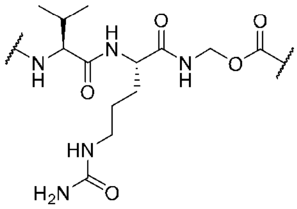

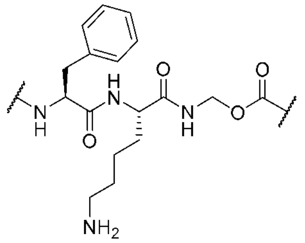

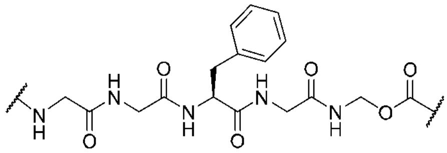

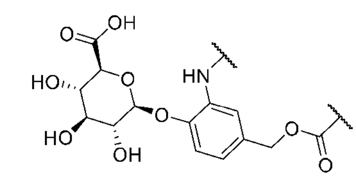

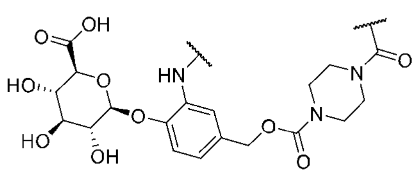

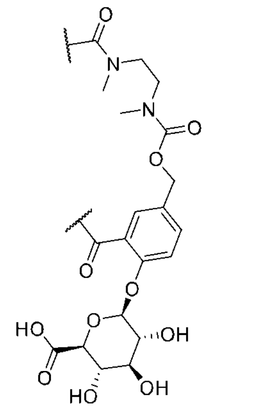

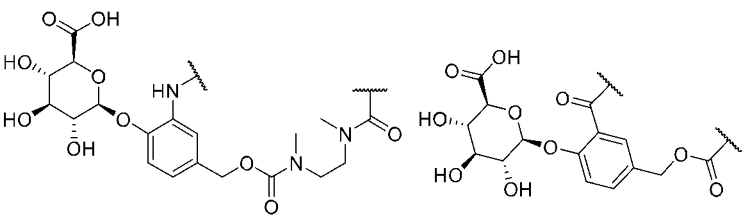

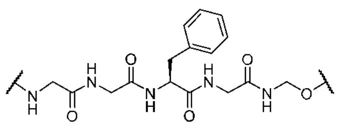 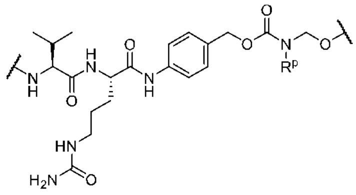 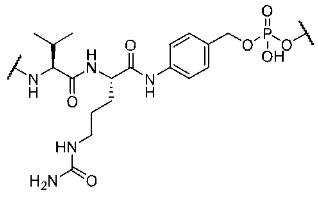

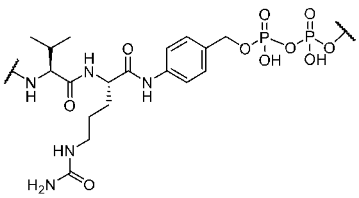 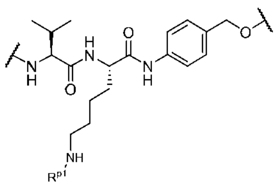 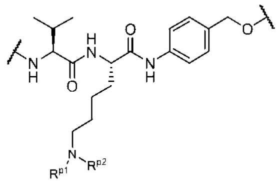 ;

wherein, $R^p$ is selected from H and $C_1$-$C_{10}$ alkyl; $R^{p1}$ and $R^{p2}$ are each independently selected from $C_1$-$C_{10}$ alkyl;
(2) the pH-responsive linker comprises the following structures:

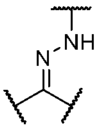 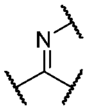 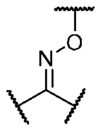 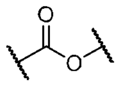 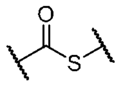 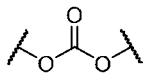 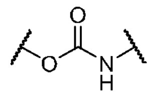

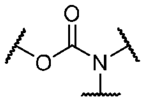 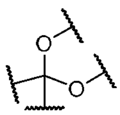 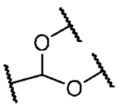 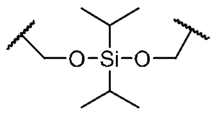 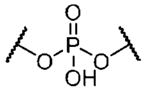 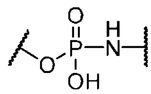

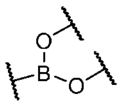 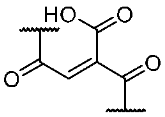 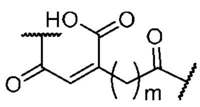 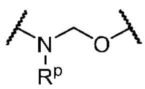 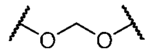

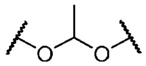 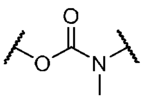 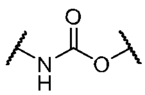 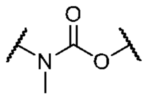 ,

wherein m = 0-4; $R^p$ is selected from H and $C_1$-$C_{10}$ alkyl;
(3) the photo-responsive linker comprises the following structures:

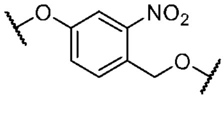 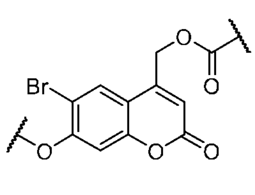 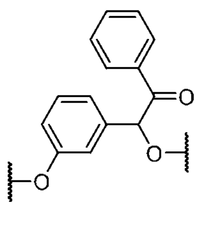

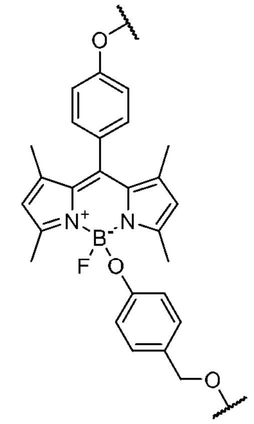 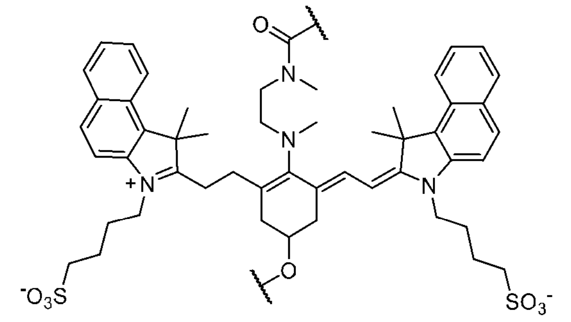

;

(4) the redox-responsive linker comprises the following structures:

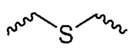 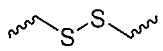 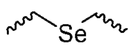 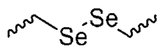 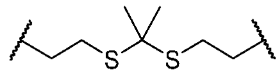

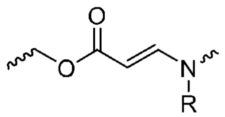 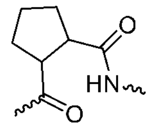 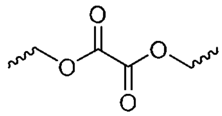 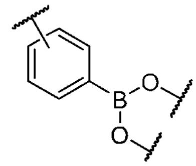

,

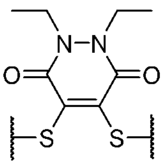 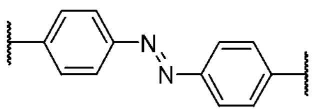

.

In some examples, the linker $L^{2a}$ in the polymer-drug conjugate is a covalent bond or a linker of formula (VII):

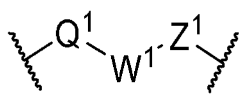

VII

$Q^1$ is selected from one of

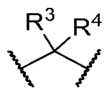 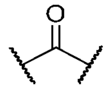 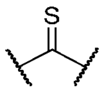,

wherein $R^3$ and $R^4$ are independently selected from H, D, $C_1$-$C_6$ alkyl, $C_1$-$C_6$ alkoxy, $C_3$-$C_6$ alkenyl, $C_3$-$C_6$ alkynyl, $C_3$-$C_8$ cycloalkyl, $C_2$-$C_8$ heterocycloalkyl, $C_6$-$C_{10}$ aryl, or $C_5$-$C_{10}$ heteroaryl; or $R^3$ and $R^4$, together with the C atom to which they are linked, may form a $C_3$-$C_8$ alkyl or heterocycloalkyl; the heteroatom in the $C_2$-$C_8$ heterocycloalkyl is O, S, or N, the number of the heteroatom(s) is one or more, and when more than one heteroatom is present, the heteroatoms are identical or different; the heteroatom in the $C_5$-$C_{10}$ heteroaryl is O, S, or N, the number of the heteroatom(s) is one or more, and when more than one heteroatom is present, the heteroatoms are identical or different;

$W^1$ is a covalent bond or a $C_0$-$C_{20}$ fragment either containing a heteroatom or not, wherein the heteroatom is O, S, Se, N, P, Si, or B, the number of the heteroatom(s) is one or more, and when more than one heteroatom is present, the heteroatoms are identical or different; the W fragment may or may not contain an unsaturated bond;

$Z^1$ is selected from one of

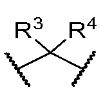 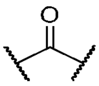 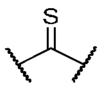 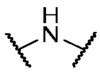 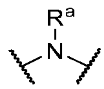 ,

wherein $R^a$ is as defined in formula (I).

In some examples, the linker $L^{2a}$ in the polymer-drug conjugate is selected from the following structures and a covalent bond:

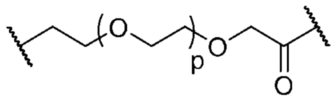 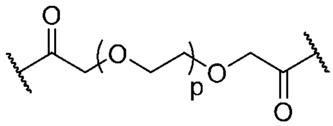 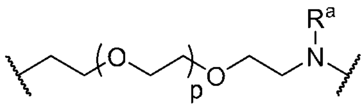

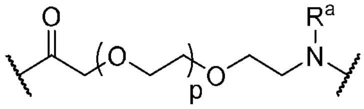 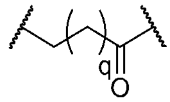 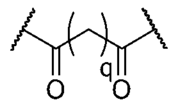

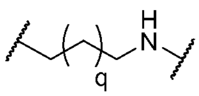 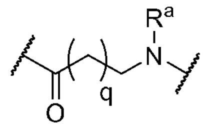 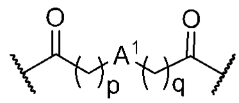

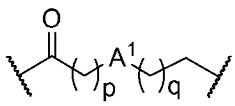 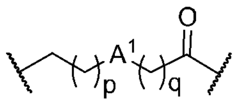 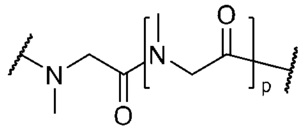

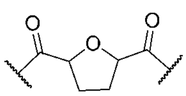 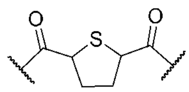 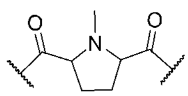 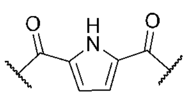 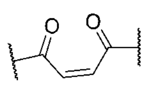

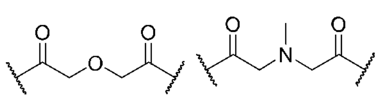 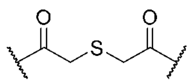 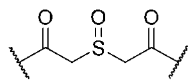 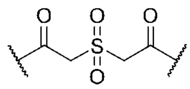

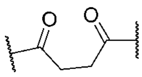 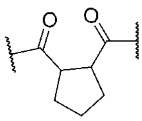 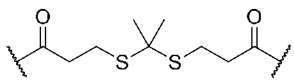 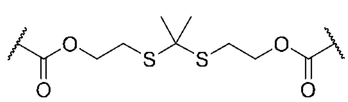 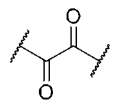

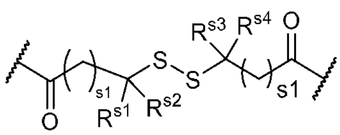 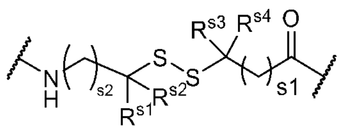 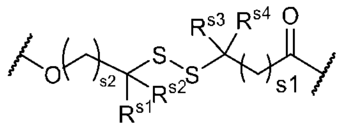

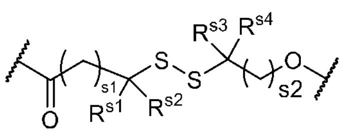 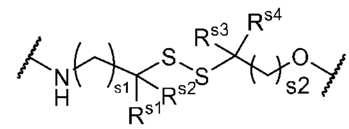 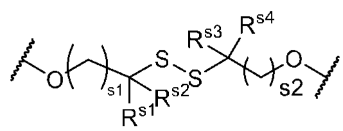

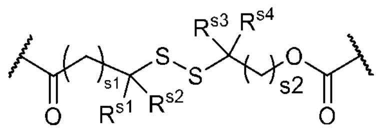 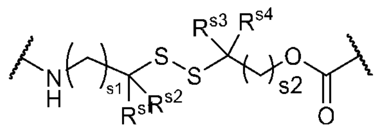 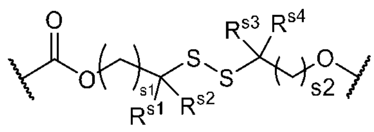

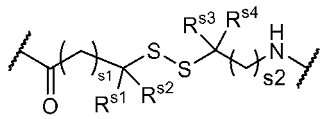 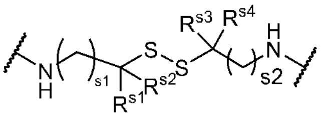 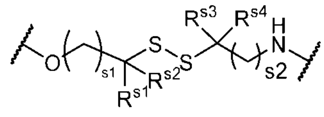

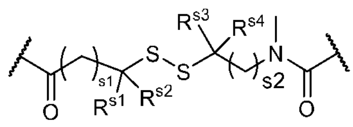 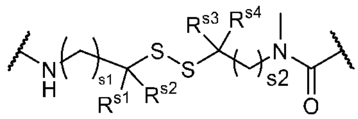 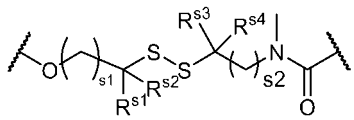

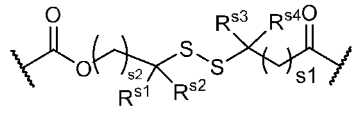 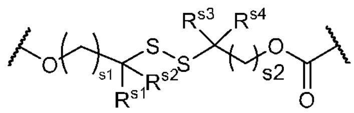 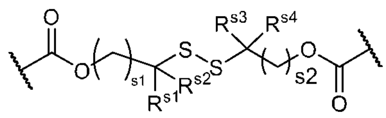

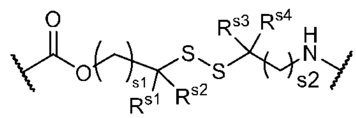 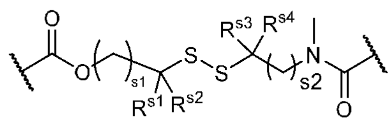

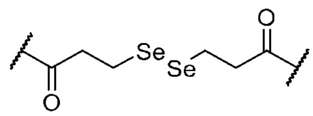 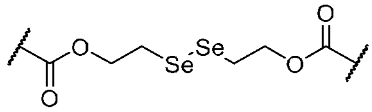 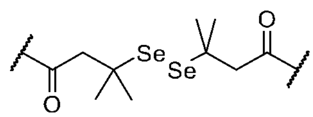

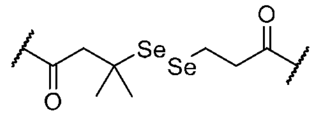 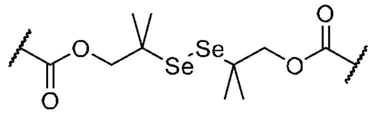 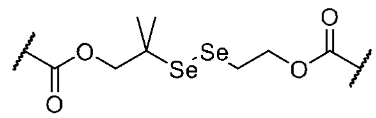

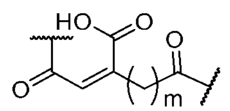

wherein,

$A^1$ is O, S, S(O), $S(O)_2$, $NR^a$, or $C(R^3R^4)$;
p is selected from integers of 0-16;
q is selected from integers of 0-16;
m is selected from integers of 0-4;
s1 is selected from integers of 0-16;
s2 is selected from integers of 1-15;
$R^{s1}$, $R^{s2}$, $R^{s3}$, and $R^{s4}$ are each independently selected from hydrogen and methyl;
$R^a$ is as defined in formula (I).

In some examples, the pharmacokinetic regulator residue P in the polymer-drug conjugate is selected from a polyethylene glycol derivative residue with a repeating unit number $a^1$ and a terminal group $R^b$, a hyaluronic acid derivative residue with a repeating unit number $b^1$, a polyphosphate residue with a repeating unit number $c^1$, a polysarcosine residue with a repeating unit number $d^1$ and a terminal group $R^{d1}$, and a polyoxazoline residue with a repeating unit number $f^1$;

wherein, $a^1$ is selected from integers of 5-250, $b^1$ is selected from integers of 5-250, $c^1$ is selected from integers of 5-250, $d^1$ is selected from integers of 5-250, and $f^1$ is selected from integers of 5-250; $R^b$ is H, $C_1$-$C_{10}$ alkyl, $C_1$-$C_{10}$ heteroalkyl, $C_3$-$C_{10}$ cycloalkyl, $C_3$-$C_{10}$ alkenyl, $C_3$-$C_{10}$ alkynyl, or a hydroxy protecting group; $R^{d1}$ is H, $C_1$-$C_{10}$ alkyl, $C_1$-$C_{10}$ alkoxy, $C_1$-$C_{10}$ heteroalkyl, $C_3$-$C_{10}$ cycloalkyl, $C_3$-$C_{10}$ alkenyl, $C_3$-$C_{10}$ alkynyl, a hydroxy protecting group, or an amino protecting group;
preferably, the pharmacokinetic regulator residue P meets one or more of the following conditions:

(1) when the pharmacokinetic regulator residue P is a polyethylene glycol derivative residue with a repeating unit number $a^1$ and a terminal group $R^b$, $a^1$ is selected from integers of 5-150, preferably integers of 10-60, and more preferably integers of 15-50, e.g., 21, 43, or 44;
(2) when the pharmacokinetic regulator residue P is a polyethylene glycol derivative residue with a repeating unit number $a^1$ and a terminal group $R^b$, $R^b$ is $C_1$-$C_{10}$ alkyl, preferably $C_1$-$C_6$ alkyl, and more preferably $C_1$-$C_3$ alkyl, e.g., methyl, ethyl, *n*-propyl, or isopropyl;
(3) when the pharmacokinetic regulator residue P is a polysarcosine residue with a repeating unit number $d^1$ and a terminal group $R^{d1}$, $R^{d1}$ is preferably hydrogen, $C_1$-$C_6$ alkyl, $C_1$-$C_6$ alkoxy, an amino substituted with $C_1$-$C_6$ alkyl, or $C_1$-$C_6$ acyl, e.g., methylamino, carboxylic acid, methyl carboxylate, or acetylamino.

In some examples, the pharmacokinetic regulator residue P in the polymer-drug conjugate is a polyethylene glycol derivative residue with a repeating unit number $a^1$ and a terminal group $R^b$, a hyaluronic acid derivative residue with a repeating unit number $b^1$, or a polysarcosine derivative residue with a repeating unit number $d^1$; the polyethylene glycol derivative residue is selected from the following structures:

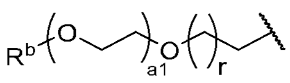 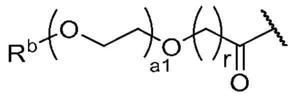

wherein $a^1$ is selected from integers of 5-150, $b^1$ is selected from integers of 5-150, and r is selected from integers of 0-8; the polyethylene glycol derivative residue is preferably

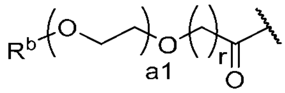,

$a^1$ is selected from integers of 20-45, $b^1$ is selected from integers of 5-10, and r is selected from integers of 0-3; the polysarcosine derivative residue is selected from the following structures:

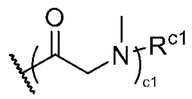 or 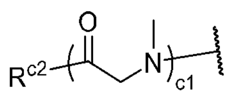

wherein $c^1$ is selected from integers of 5-150, and $R^{c1}$ is selected from H, $C_1$-$C_{10}$ alkyl, $C_1$-$C_{10}$ alkoxy, $C_3$-$C_{10}$ alkenyl, $C_3$-$C_{10}$ alkynyl, $C_3$-$C_8$ cycloalkyl, $C_2$-$C_8$ heterocycloalkyl, $C_6$-$C_{10}$ aryl, $C_5$-$C_{10}$ heteroaryl, and an amino protecting group; $R^{c2}$ is selected from $OR^{c3}$, $SR^{c3}$, and $NR^{c4}R^{c5}$, wherein $R^{c3}$ is selected from H, $C_1$-$C_{10}$ alkyl, $C_3$-$C_{10}$ alkenyl, $C_3$-$C_{10}$ alkynyl, $C_3$-$C_8$ cycloalkyl, $C_2$-$C_8$ heterocycloalkyl, $C_6$-$C_{10}$ aryl, and $C_5$-$C_{10}$ heteroaryl, and $R^{c4}$ and $R^{c5}$ are each independently selected from H, $C_1$-$C_{10}$ alkyl, $C_1$-$C_{10}$ alkoxy, $C_3$-$C_{10}$ alkenyl, $C_3$-$C_{10}$ alkynyl, $C_3$-$C_8$ cycloalkyl, $C_2$-$C_8$ heterocycloalkyl, $C_6$-$C_{10}$ aryl, $C_5$-$C_{10}$ heteroaryl, and an amino protecting group.

In some examples, the pharmacokinetic regulator residue P in the polymer-drug conjugate is a methyl-terminated polyethylene glycol derivative residue with a repeating unit number $a^1$; the methyl-terminated polyethylene glycol derivative residue is selected from the following structures:

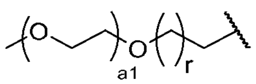 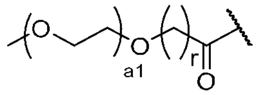

wherein $a^1$ is selected from integers of 5-150, and r is selected from integers of 0-8;

the methyl-terminated polyethylene glycol derivative residue is preferably

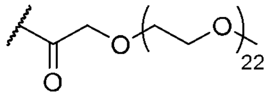 or 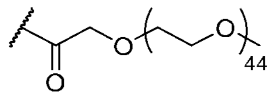 .

In some examples, the polymer residue in the polymer-drug conjugate is selected from the following structures:

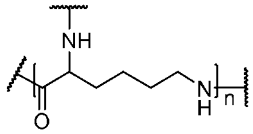
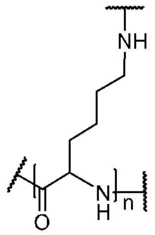
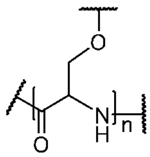
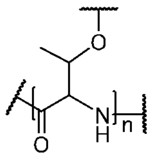

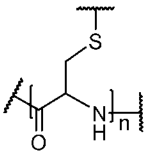
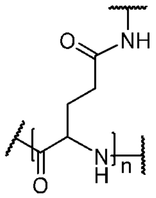
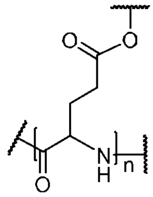
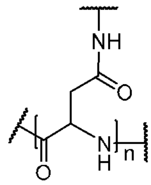
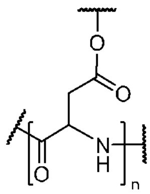

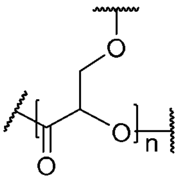
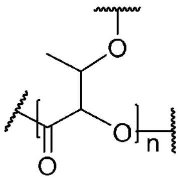
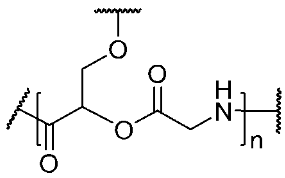

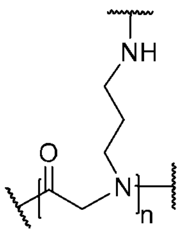
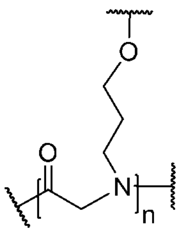
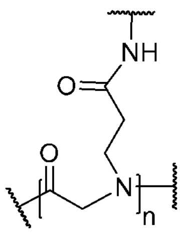
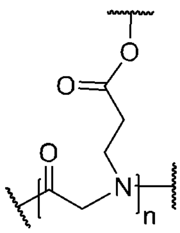

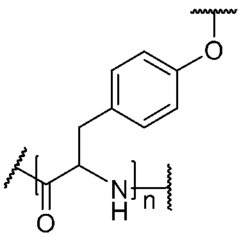
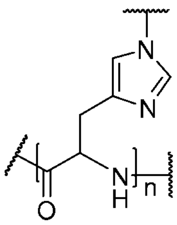
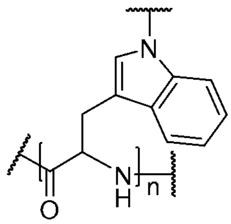

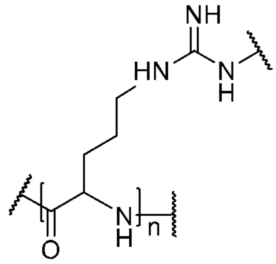
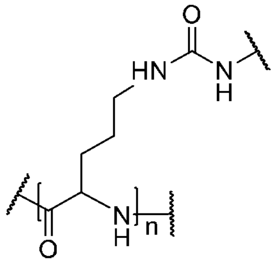
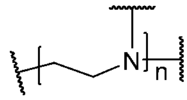

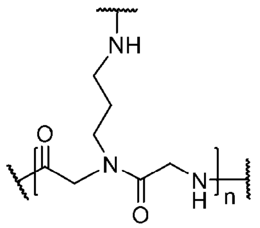
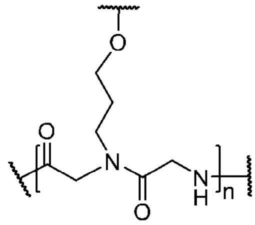
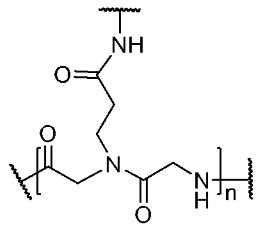
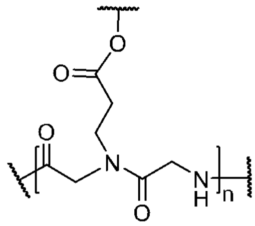

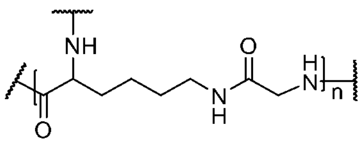
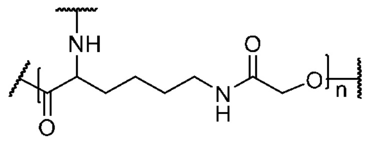

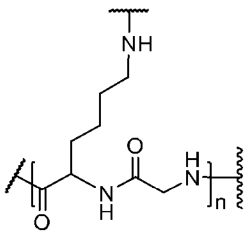
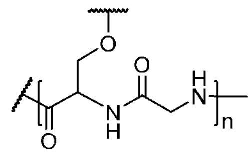
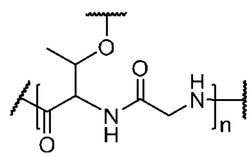
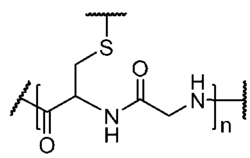

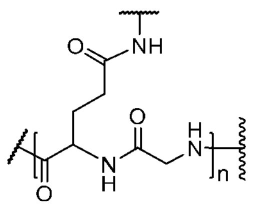
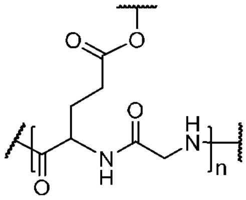
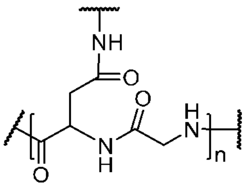
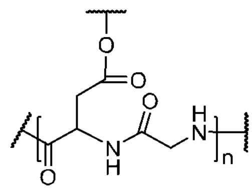

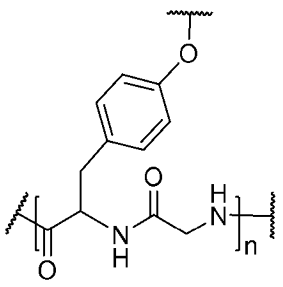
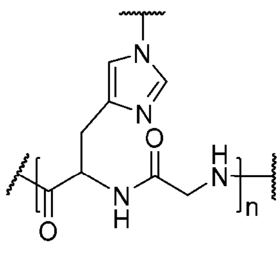
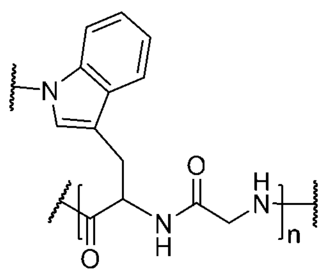

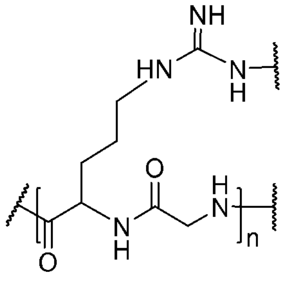
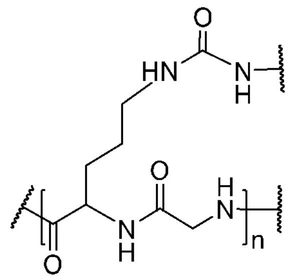

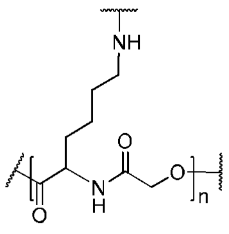
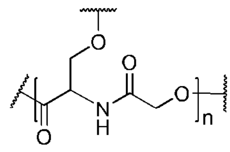
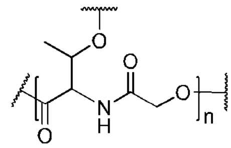
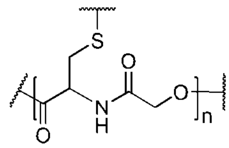

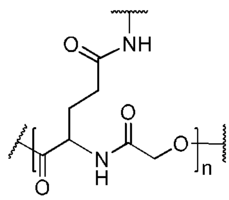
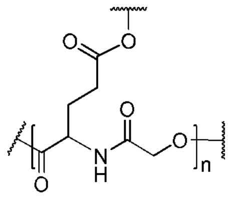
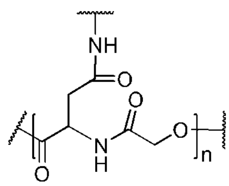
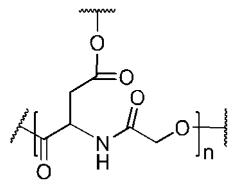

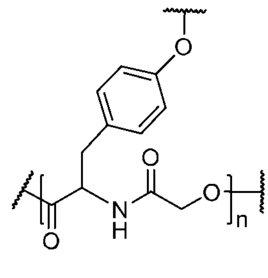
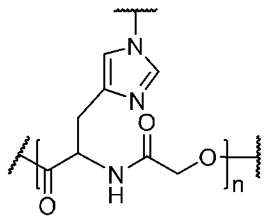
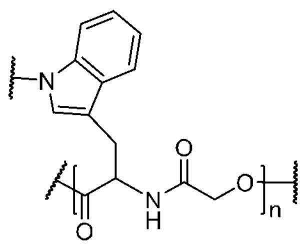

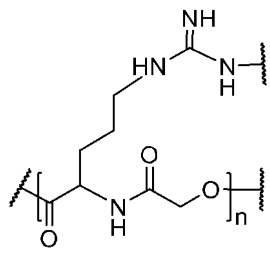
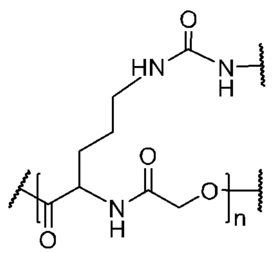

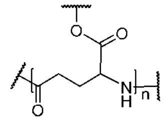
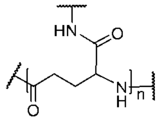
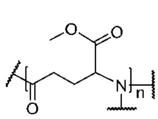
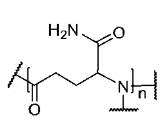
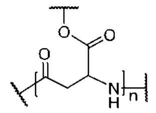

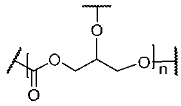 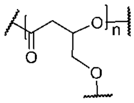 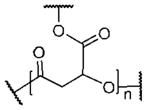 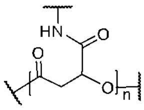 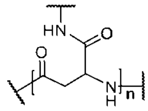

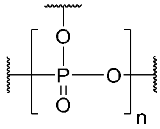 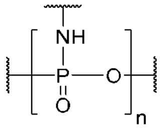 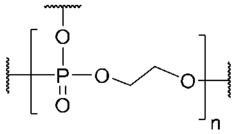 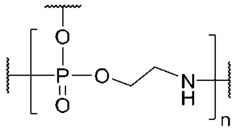

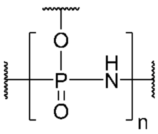 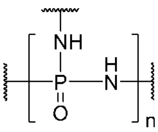 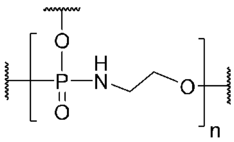 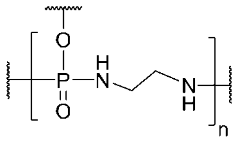

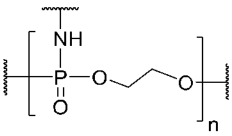 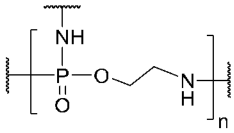 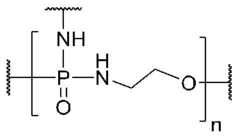 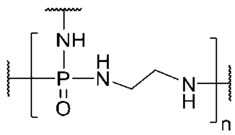

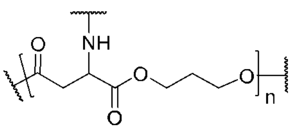 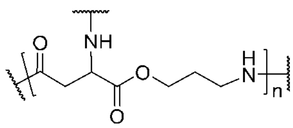 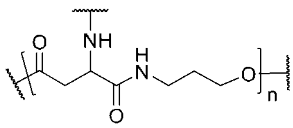

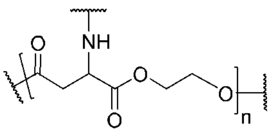 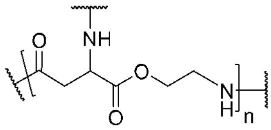 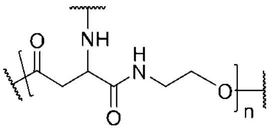

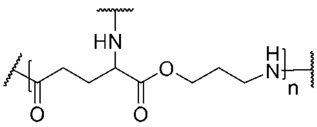 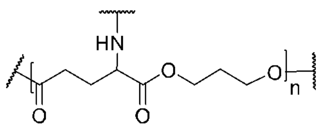 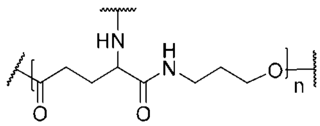

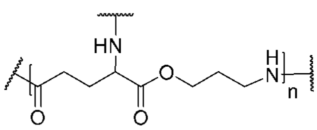 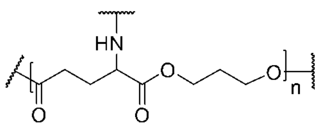 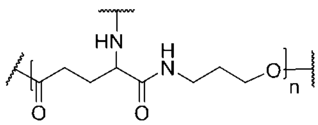

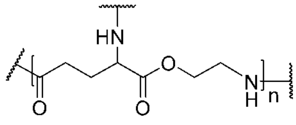 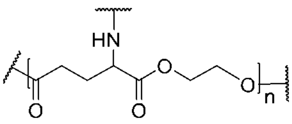 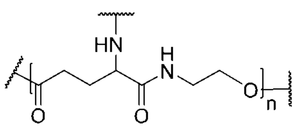

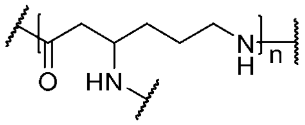
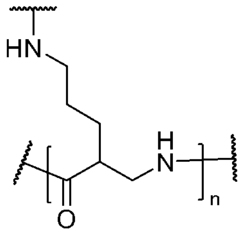
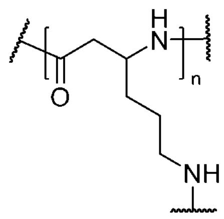

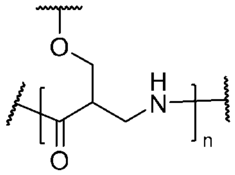
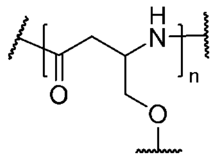
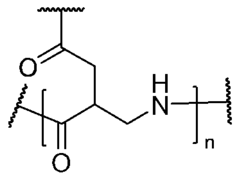
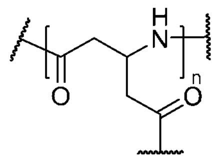

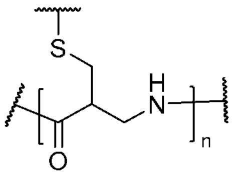
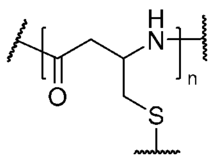
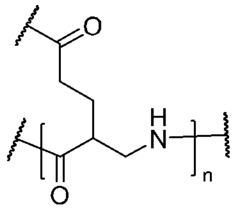
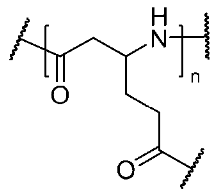

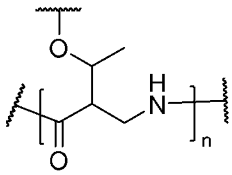
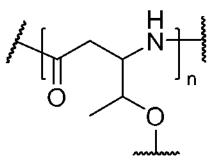

n is selected from integers of 4-100.

In some examples, the pharmaceutically active agent D in the polymer-drug conjugate has an active functional group selected from one or more of primary amine, secondary amine, tertiary amine, hydroxy, sulfhydryl, carboxyl, ester group, amide, boric acid, borate, phosphoric acid, sulfonic acid, sulfoxide, aldehyde group, and ketone group.

In some examples, the pharmaceutically active agent in the polymer-drug conjugate is selected from: one or more of an anesthetic, an antacid, an anti-infective, a cardiovascular agent, a diuretic, a hematinic, an immunosuppressant, a GLP-1 receptor agonist, a hormone and an analog, an ophthalmic drug, an analgesic, a respiratory drug, an antiarthritic, an anticonvulsant, an antihistamine, an anti-inflammatory agent, an anti-ulcer agent, a behavior modification drug, an antineoplastic, an anti-cancer antigen, a central nervous system agent, an antipsychotic, a contraceptive agent, a diabetes drug, a growth promoter, a hemostat, an immunostimulant, an immunomodulator, a muscle relaxant, an obesity drug, an osteoporosis drug, a sedative, a tranquilizer, a urinary acidifying agent, a vitamin, a polypeptide drug, an oligonucleotide drugs, an mRNA drug, an antibody drug, a biologic, a targeted protein degrader, a PROTAC drug, an oligosaccharide drug, and a targeted drug.

In some examples, the pharmaceutically active agent residue D in the polymer-drug conjugate is an antineoplastic residue; the antineoplastic is selected from one or more of an anti-tumor targeted drug, a PROTAC (proteolysis targeting chimera) drug, a molecular glue degrader, an anti-tumor immunomodulator, and a chemotherapeutic.

In some examples, the pharmaceutically active agent residue D in the polymer-drug conjugate is an antineoplastic selected from one or more of abemaciclib, abiraterone, abrocitinib, acalabrutinib, afatinib, aldesleukin, alectinib, alflutinib, almonertinib, altretamine, amcenestrant, aminoglutethimide, amsacrine, anastrozole, anlotinib, apalutamide, apatinib, arzoxifene, asciminib, asparaginase, avapritinib, avitinib, axitinib, azacitidine, baricitinib, belinostat, bendamustine, bexarotene, bicalutamide, bicyclol, binimetinib, bleomycin, boanmycin, bortezomib, bosutinib, brigatinib, buserelin,

busulfan, cabazitaxel, cabozantinib, calaspargase, calicheamycin, capecitabine, capmatinib, carboplatin, carfilzomib, carmustine, carmofur, cedazuidine, ceritinib, cetrorelix, chidamide, chlorambucil, cisplatin, cladribine, clofarabine, cobimetinib, colchicine, copanlisib, crizotinib, cyclophosphamide, cytarabine, dabrafenib, dacarbazine, dacomitinib, dactinomycin, dalpiciclib, darolutamide, dasatinib, daunorubicin, decitabine, degarelix, delgociclib, denileukin, deruxtecan, deucravacitinib, docetaxel, donafenib, doxorubicin, duvelisib, enasidenib, encorafenib, ensartinib, entrectinib, enzalutamide, enzastaurin, elacestrant, epirubicin, erdafitinib, eribulin, erlotinib, estradiol, estramustine, etoposide, everolimus, exemestane, fasudil, fedatinib, filgotinib, floxuridine, fludarabine, flumatinib, fluorouracil, flutamide, fluzoparib, formestane, fostamatinib, fruquintinib, fulvestrant, futibatinib, gefitinib, gemcitabine, gilteritinib, giredestrant, glasdegib, goserelin, histrelin, hydroxyurea, ibrutinib, ibudilast, icaritin, icotinib, idarubicin, idelalisib, ifosfamide, imatinib, imiquimod, infigratinib, ingenol mebutate, interferon alfa-2b, irinotecan, ivosidenib, ixabepilone, ixazomib, lanreotide, lapatinib, larotrectinib, lenalidomide, lenvatinib, letrozole, leucovorin, euprolide, lomustine, lonafarnib, lorlatinib, lurbinectedin, maytansine, mechlorethamine, medroxyprogesterone, megestrol, melphalan, melphlan flufenamide, mercaptopurine, methotrexate, methoxsalen, methylprednisolone, midostaurin, mitomycin, mitotane, mitoxantrone, mitozolomide, mobocertinib, monomethylauristatin E, monomethylauristatin F, nelarabine, nandrolone, neratinib, nearsudil, nilotinib, nilutamide, nintedanib, niraparib, octreotide, olaparib, olmutinib, olverembatinib, omacetaxine, orelabrutinib, osimertinib, oxaliplatin, paclitaxel, pacritinib, palbociclib, pamidronate, pamiparib, panobinostat, pazopanib, peficitinib, pegaptanib, pegaspargase, peginteferon alfa-2b, pemigatinib, pemetrexed, pentetreotide, pentostatin, pexidartinib, phenoxybenzamine, pidotimod, plinabulin, plitidepsin, pomalidomide, ponatinib, porfimer, pralatrexate, pralsetinib, prednisolone, procarbazine, pyrotinib, quizartinib, radotinib, raloxifene, raltitrexed, regorafenib, ribociclib, rintatolimod, ripretinib, romidepsin, rucaparib, ruxolitinib, savolitinib, selinexor, selpercatinib, selumetinib, sonidegib, sorafenib, sotorasib, streptozocin, sunitinib, surufatinib, talazoparib, tamoxifen, tazemetostat, tegafur, temozolomide, temsirolimus, teniposide, tepotinib, teprenone, thalidomide, thioguanine, thiotepa, thyrotropin alfa, tipiracil, tipifarnib, tirabrutinib, tirbanibulin, tivozanib, trametinib, tofacitinib, topotecan, toremifene, trabectedin, tretinoin, trifluride, trilaciclib, triptorelin, tucatinib, upadacitinib, umbralisib, utidelone, uroacitide, valrubicin, vandetanib, vemurafenib, venetoclax, vinblastine, vincristine, vindesine, vinflunine, vinorelbine, vismodegib, vorinostat, zanubrutinib, zoledronic acid, amatoxins, anthacyclines, anthracenes, anthramycins, auristatins, bryostatins, camptothecins, carmaphycins, combretastatins, cyclosporines, cryptomycins, ecteinascidins, ellipticenes, esperamicins, mustines, neothramycins, ozogamicins, phenoxazines, podophyllotoxins, pyrrolobenzodiazepines, sibiromycins, thailanstatins, tomamycns, tubulysins, taxanes, vinca alkaloids, 7-epitaxol, 2'-acetyltaxol, 10-deacetyltaxol, 10-deacetyl-7-epitaxol, 7-xylosyltaxol, 10-deacetyl-7-glutaryl taxol, 7-N,N-dimethylglycyltaxol, 7-L-propyltaxol, larotaxel, camptothecin, 9-aminocamptothecin, 9-nitrocamptothecin, lurtotecan, gimatecan, belotecan, 10-hydroxycamptothecin, 10-hydroxy-7-ethylcamptothecin (SN-38), exatecan, pirarubicin, aclacinomycin, sirolimus, tacrolimus, progesterone, estrogen, rapamycin, plicamycin, cephalotaxin, and curcumin.

In some examples, the polymer-drug conjugate meets one or more of the following conditions:

(1) the antineoplastic is an anti-tumor targeted drug selected from one or more of aldesleukin, abemaciclib, abiraterone, abrocitinib, acalabrutinib, afatinib, alectinib, alflutinib, almonertinib, amcenestrant, anastrozole, anlotinib, apalutamide, apatinib, arzoxifene, asciminib, avapritinib, avitinib, axitinib, baricitinib, belinostat, bexarotene, bicalutamide, binimetinib, bleomycin, boanmycin, bortezomib, bosutinib, brigatinib, buserelin, cabozantinib, capmatinib, carfilzomib, carmustine, ceritinib, cetrorelix, chidamide, cobimetinib, copanlisib, crizotinib, dabrafenib, dacomitinib, dalpiciclib, darolutamide, dasatinib, degarelix, delgociclib, deucravacitinib, donafenib, duvelisib, enasidenib, encorafenib, ensartinib, entrectinib, enzalutamide, enzastaurin, elacestrant, erdafitinib, erlotinib, everolimus, fedatinib, filgotinib, flumatinib, fluzoparib, formestane, fostamatinib, fruquintinib, fulvestrant, futibatinib, gefitinib, gilteritinib, giredestrant, glasdegib, goserelin, histrelin, ibrutinib, ibudilast, icotinib, idarubicin, idelalisib, imatinib, imiquimod, infigratinib, ivosidenib, ixazomib, lanreotide, lapatinib, larotrectinib, lenalidomide, lenvatinib, letrozole, leucovorin, leuprolide, lonafamib, lorlatinib, medroxyprogesterone, megestrol, methylprednisolone, midostaurin, mobocertinib, nandrolone, neratinib, nilotinib, nilutamide, nintedanib, niraparib, olaparib, olmutinib, olverembatinib, orelabrutinib, osimertinib, pacritinib, palbociclib, pamidronate, pamiparib, panobinostat, pazopanib, peficitinib, pegaptanib, pemigatinib, pexidartinib, pidotimod, pomalidomide, ponatinib, pralsetinib, pyrotinib, quizartinib, radotinib, raloxifene, regorafenib, ribociclib, rintatolimod, ripretinib, rucaparib, ruxolitinib, savolitinib, selinexor, selpercatinib, selumetinib, sonidegib, sorafenib, sotorasib, sunitinib, surufatinib, talazoparib, tamoxifen, tazemetostat, temsirolimus, tepotinib, thalidomide, tipifarnib, tirabrutinib, tivozanib, trametinib, tofacitinib, toremifene, tretinoin, trilaciclib, triptorelin, tucatinib, upadacitinib, umbralisib, vandetanib, vemurafenib, venetoclax, vismodegib, vorinostat, zanubrutinib, and zoledronic acid; and

(2) the antineoplastic is a chemotherapeutic selected from one or more of altretamine, aminoglutethimide, amsacrine, asparaginase, azacitidine, bendamustine, bexarotene, bicyclol, bleomycin, boanmycin, buserelin, busulfan, cabazitaxel, calaspargase, calicheamycin, capecitabine, carboplatin, carmustine, carmofur, cedazuidine, chlorambucil, cisplatin, cladribine, clofarabine, colchicine, cyclophosphamide, cytarabine, dacarbazine, dactinomycin, daunorubicin, decitabine, denileukin, deruxtecan, docetaxel, doxorubicin, epirubicin, eribulin, estradiol, estramustine, etoposide, exemestane, fasudil, floxuridine, fludarabine, fluorouracil, flutamide, formestane, gemcitabine, hydroxyurea, icaritin, idarubicin, ifosfamide, ingenol mebutate, irinotecan, ixabepilone, leucovorin, lomustine, lurbinctedin, maytansine, mechlorethamine, medroxyprogesterone, megestrol, melphalan, melphlan flufenamide, mercaptopurine, methotrexate, methoxsalen, methylprednisolone, mitomycin, mitotane, mitoxantrone, mitozolomide, monomethylauristatin E, monomethylauristatin F, nelarabine, nandrolone, nearsudil, octreotide, omacetaxine, oxaliplatin, paclitaxel, pamidronate, pemetrexed, pentetreotide, pentostatin, phenoxybenzamine, plinabulin, plitidepsin, porfimer, pralatrexate, prednisolone, procarbazine, procarbazine, raltitrexed, romidepsin, streptozocin, tegafur, temozolomide, teniposide, teprenone, thioguanine, thiotepa, thyrotropin alfa, tipiracil, tirbanibulin, topotecan, trabectedin, trifluride, utidelone, uroacitide, valrubicin, vinblastine, vincristine, vindesine, vinflunine, vinorelbine, amatoxins, anthacyclines, anthracenes, anthramycins, auristatins, bryostatins, camptothecins, carmaphycins, combretastatins, cyclosporines, cryptomycins, ecteinascidins, ellipticenes, esperamicins, mustines, neothramycins, ozogamicins, phenoxazines, podophyllotoxins, pyrrolobenzodiazepines, sibiromycins, thailanstatins, tomamycns, tubulysins, taxanes, vinca alkaloids, 7-epitaxol, 2'-acetyltaxol, 10-deacetyltaxol, 10-deacetyl-7-epitaxol, 7-xylosyltaxol, 10-deacetyl-7-glutaryl taxol, 7-N,N-dimethylglycyltaxol, 7-L-propyltaxol, larotaxel, camptothecin, 9-aminocamptothecin, 9-nitrocamptothecin, lurtotecan, gimatecan, belotecan, 10-hydroxycamptothecin, 10-hydroxy-7-ethylcamptothecin (SN-38), exatecan, pirarubicin, aclacinomycin, sirolimus, tacrolimus, progesterone, estrogen, rapamycin, plicamycin, cephalotaxin, and curcumin.

In some examples, the nano-scale size of the polymer-drug conjugate can be controlled by the following methods:

(1) changing the type of the hydrophilic or hydrophobic group, and adjusting the length and the hydrophobicity or hydrophilicity of the hydrophilic or hydrophobic groups;
(2) adjusting the type and length of the linker between the polymer and the drug;
(3) changing the type of conjugated drug, such as the molecular size and the hydrophilicity and hydrophobicity of the drug;

Through the above-mentioned methods, the nano-scale particle size range of the polymer-drug conjugate can be controlled at 20-100 nM; further, the nano-scale particle size range of the polymer-drug conjugate can be controlled at 10-20 nM; furthermore, the nano-scale particle size range of the polymer-drug conjugate can be controlled at 10 nM or less.

In some examples, the nano-scale particle size allows the polymer-drug conjugate of the present disclosure to remain stable and homogeneous during the manufacture and preservation without common problems such as aggregation.

In some specific examples, the pharmaceutically active agent residue D in the polymer-drug conjugate is selected from the following structures:

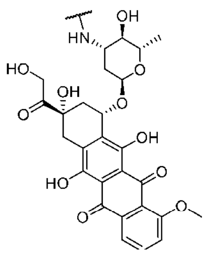 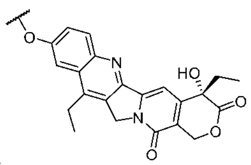 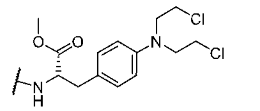 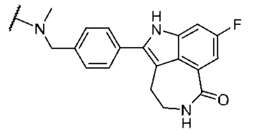

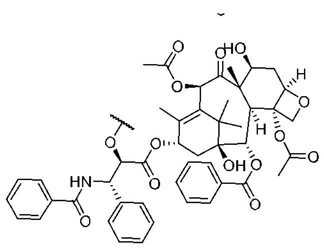 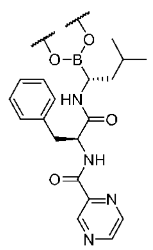 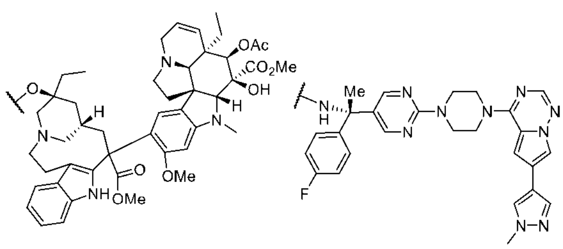

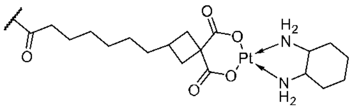

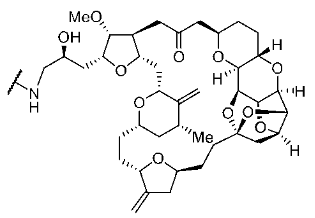

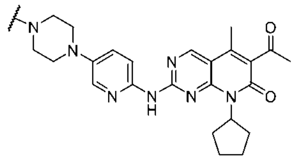

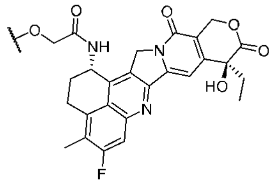

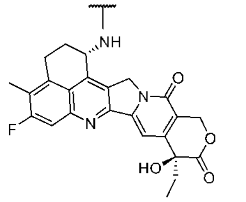

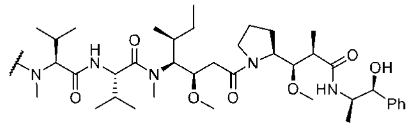

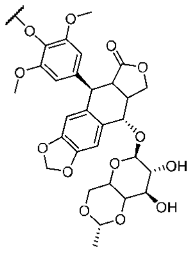
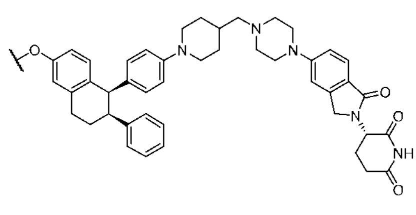
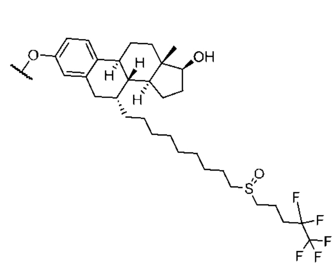
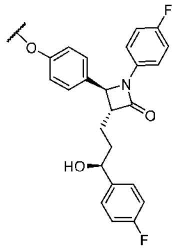
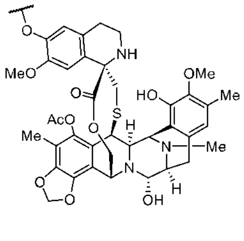
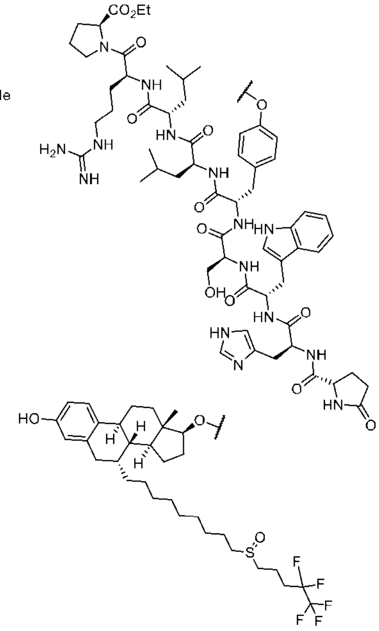
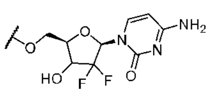
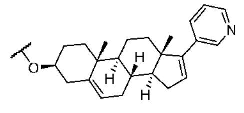
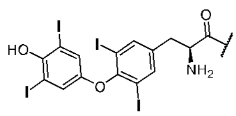
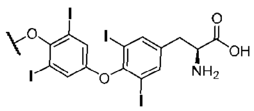
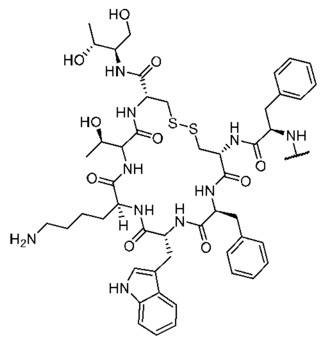
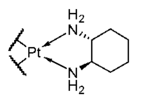
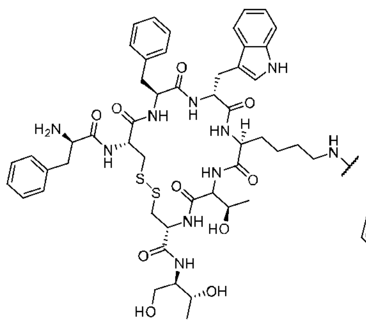
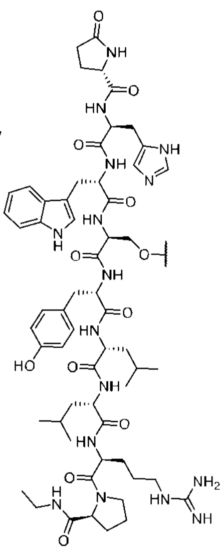
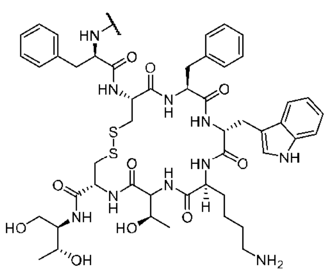
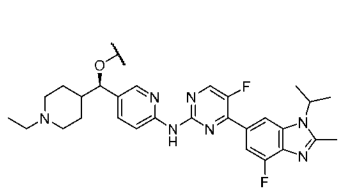
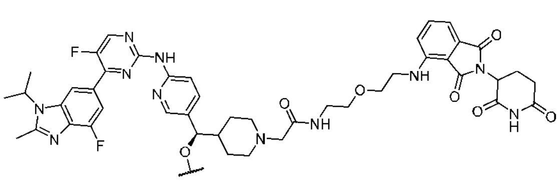
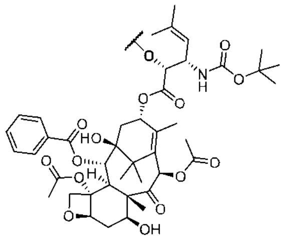

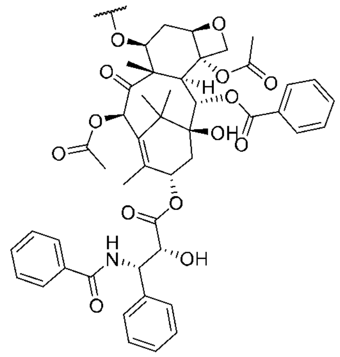
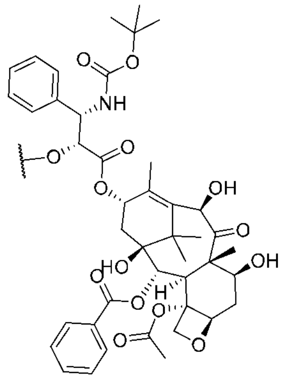
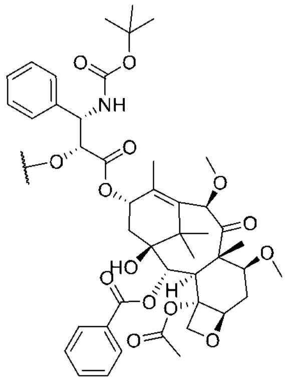

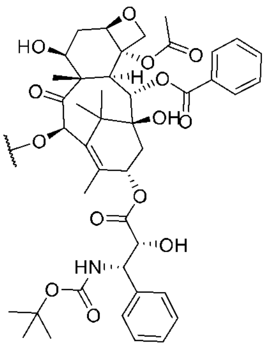
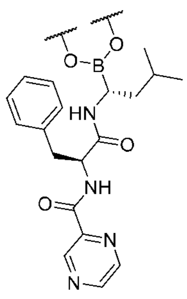
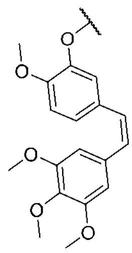
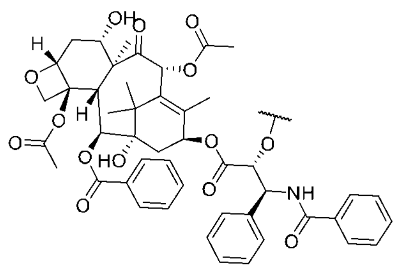

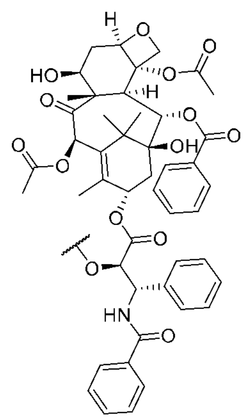
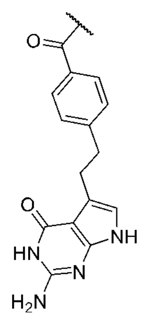
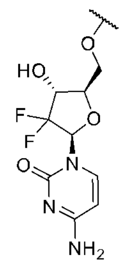
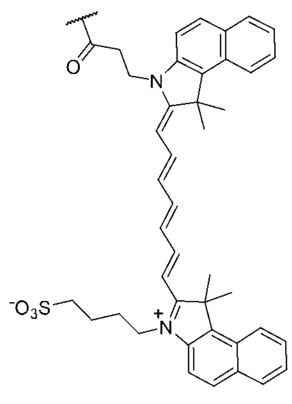

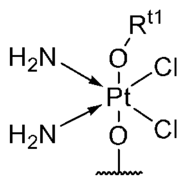
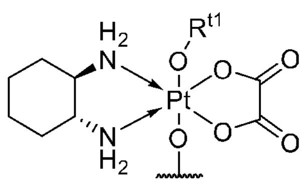
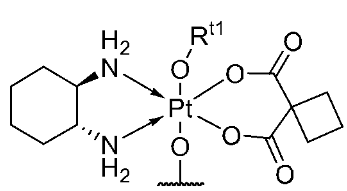

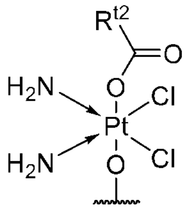
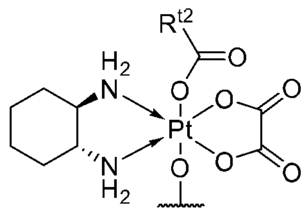
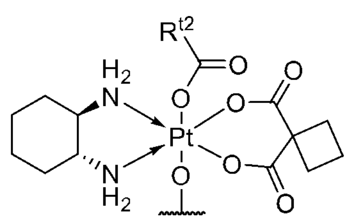
,

wherein $R^{t1}$ is selected from H and $C_1$-$C_{20}$ alkyl; $R^{t1}$ is selected from $NHR^{t1}$ and $C_1$-$C_{20}$ alkyl.

In some specific examples, the linker $L^{2a}$ in the polymer-drug conjugate is selected from the following structures:

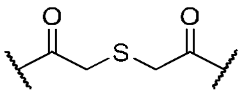, 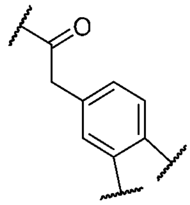, 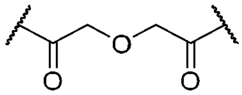,

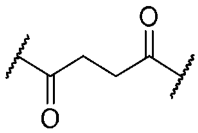, 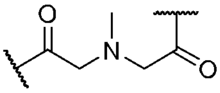 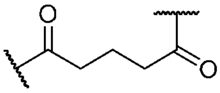, 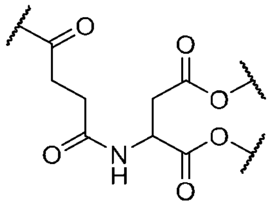,

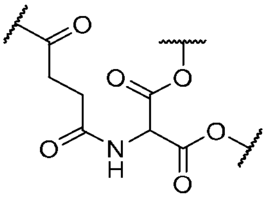, 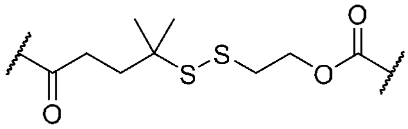, 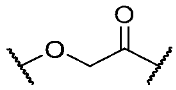, 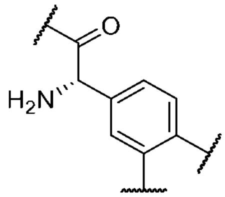,

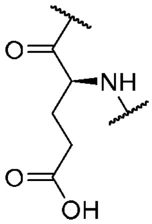, 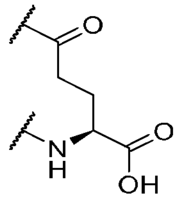, 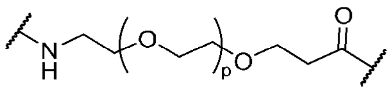, 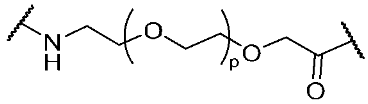,

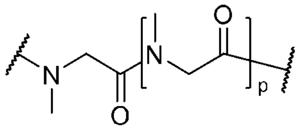, 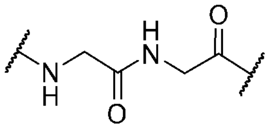, 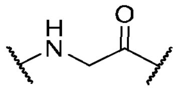, 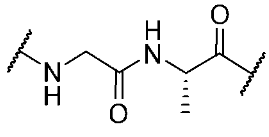,

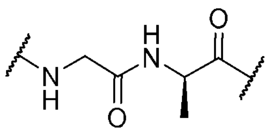, 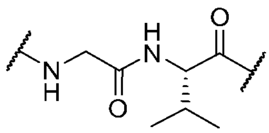, 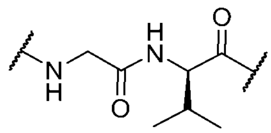,

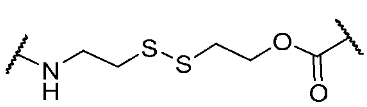, 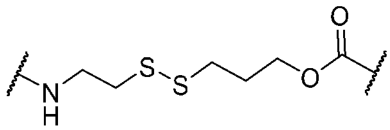, 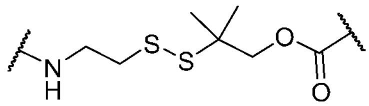,

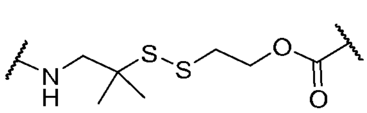, 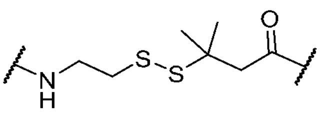, 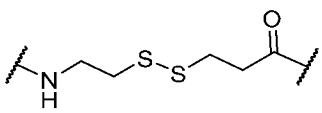,

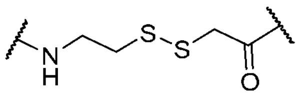, 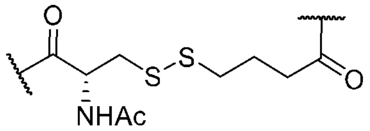, 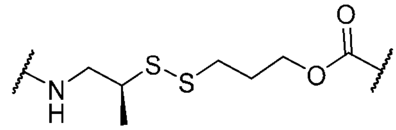,

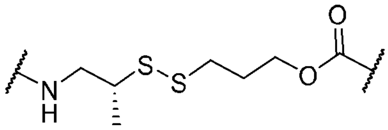, or 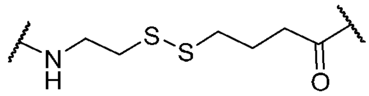.

In some specific examples, the linker $L^{2b}$ in the polymer-drug conjugate is preferably selected from the following structures:

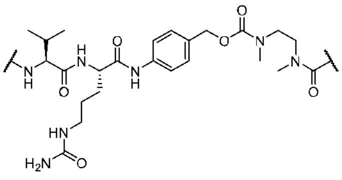
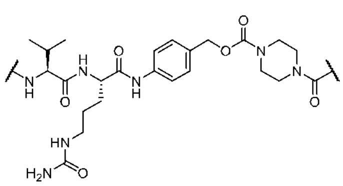
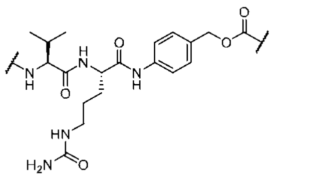

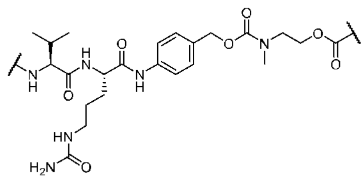
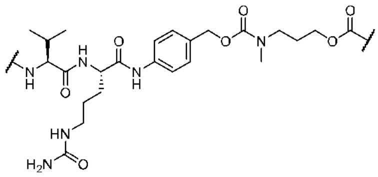
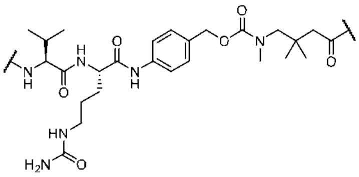

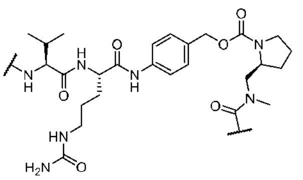
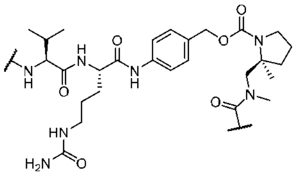
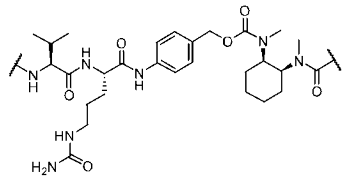

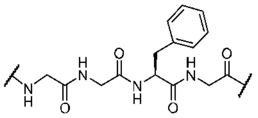
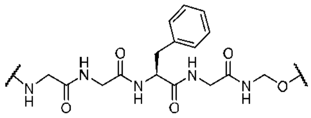
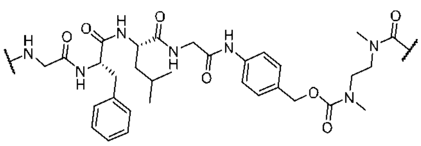

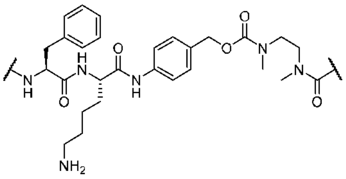
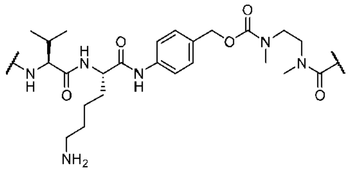
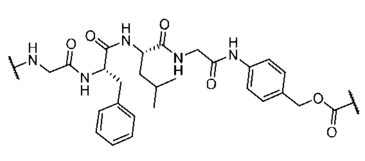

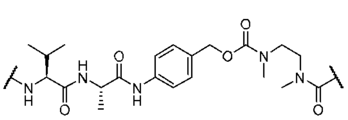
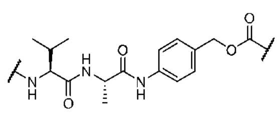
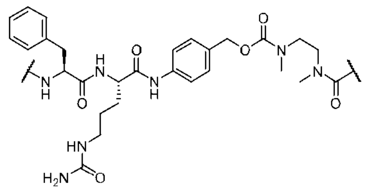

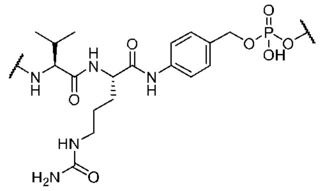
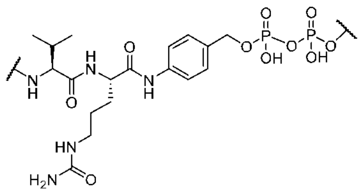
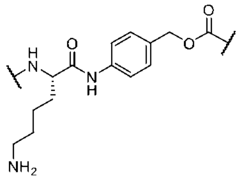
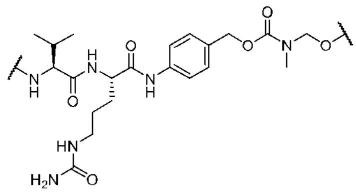
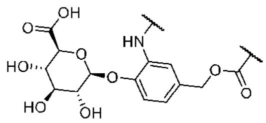
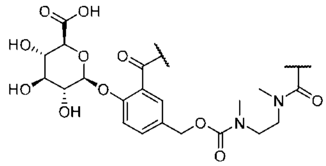
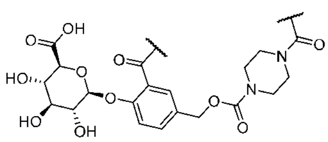
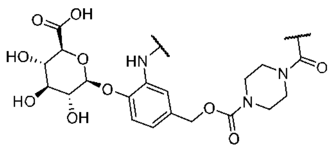
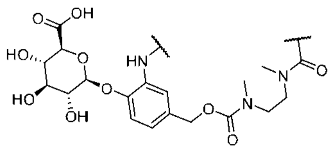
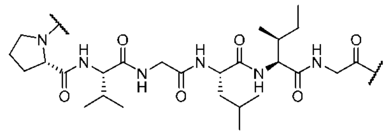

In some specific examples, the polymer-drug conjugate is selected from the following structures:

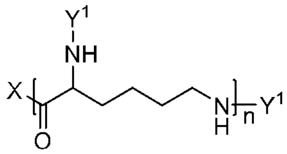
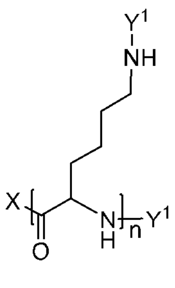
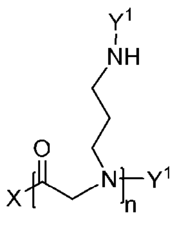
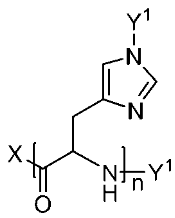
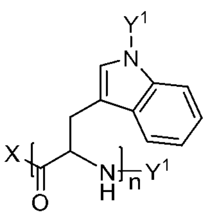
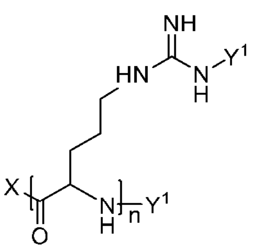
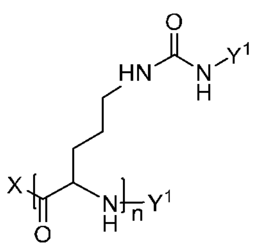
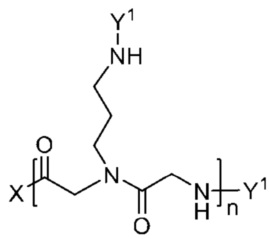

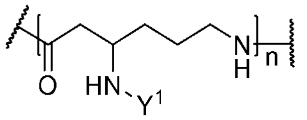
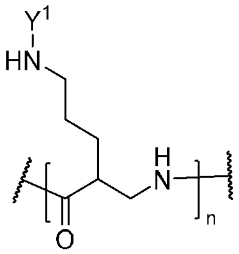
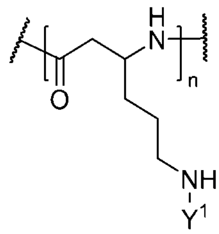

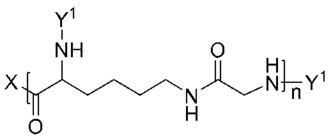
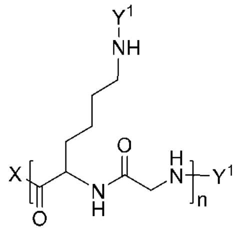
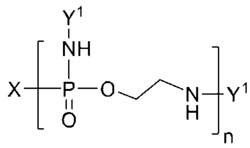

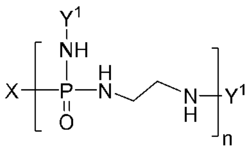
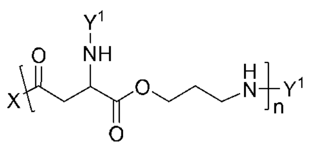
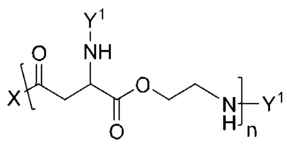

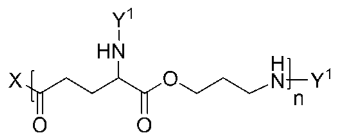
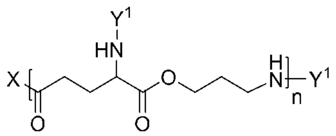
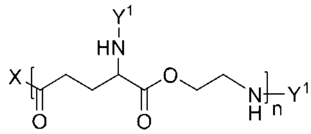

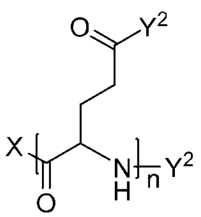
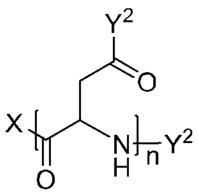
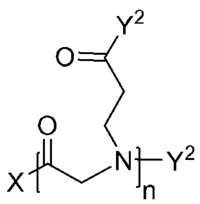

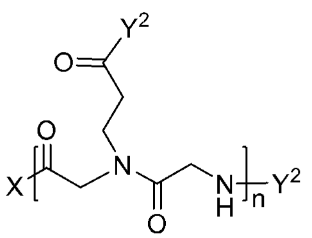
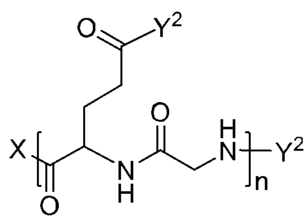
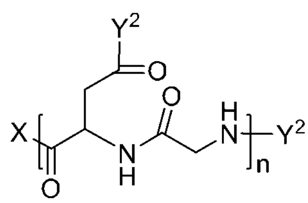

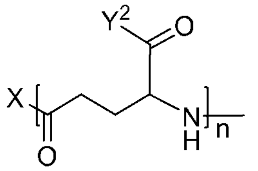
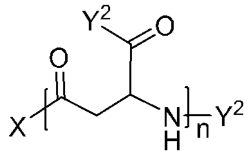

wherein, $Y^1$ =

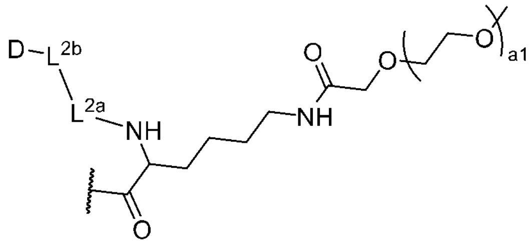

;

$Y^2$ =

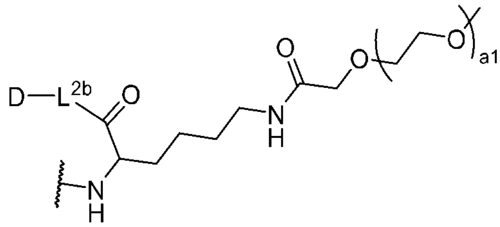

,

wherein,

$L^{2a}$, $L^{2b}$, D, and X are each as defined in formula (I);

n is an integer of 10-70;

$a^1$ is an integer of 5-150.

In some specific examples, the terminal group $R^b$ in the pharmacokinetic regulator residue P in the polymer-drug conjugate is preferably methyl, and the PEG residue has a number-average molecular weight of 550, 1000, 2000, 3000, 4000, or 5000.

In some specific examples, the polymer-drug conjugate meets one or more of the following conditions:

(1) the branched center Y is linked to the polymer residue via the linker $L^0$, and the molar ratio of the branched center Y to the polymer structural unit is 0.5:1 to 1.5:1;

(2) the pharmacokinetic regulator residue P is linked to the branched center Y via the linker $L^1$, and the molar ratio of the pharmacokinetic regulator residue to the polymer structural unit is 0.5:1 to 1.5:1; and

(3) the pharmaceutically active agent residue D is linked to the trifunctional branched center Y via the linker $L^2$, and the molar ratio of the pharmaceutically active agent residue D to the polymer structural unit is 0.5:1 to 1.5:1.

In some specific examples, -$L^2$-D in the polymer-drug conjugate is any one of the following structures:

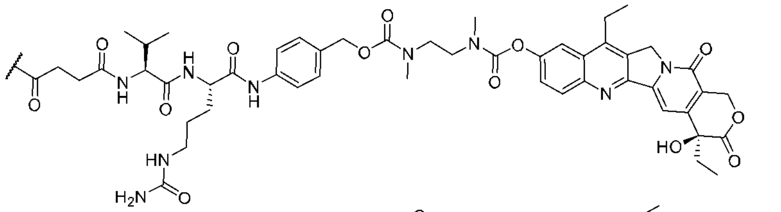

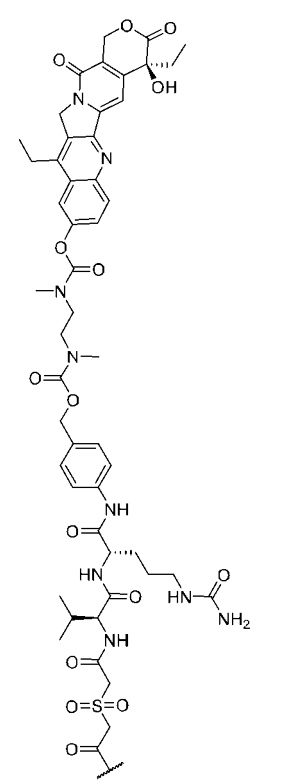

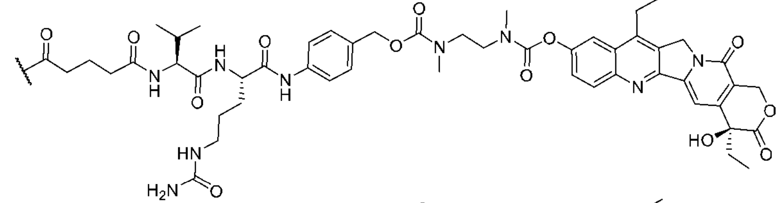

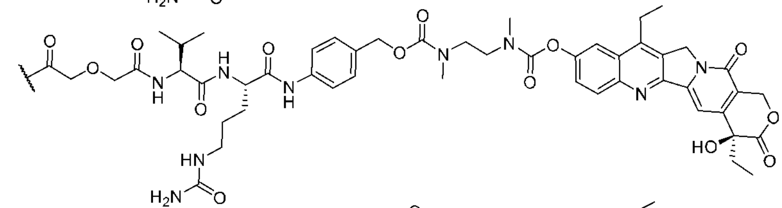

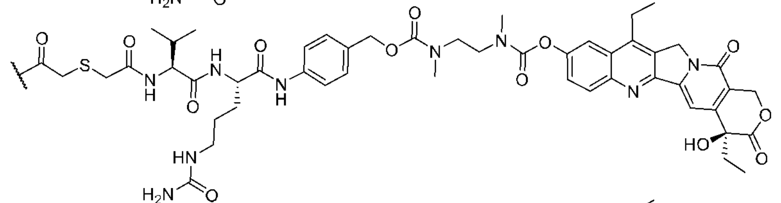

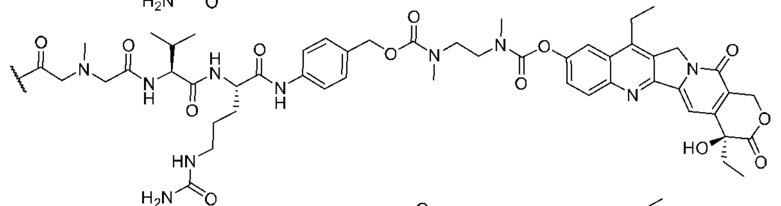

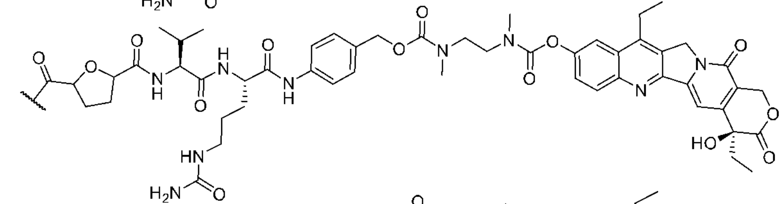

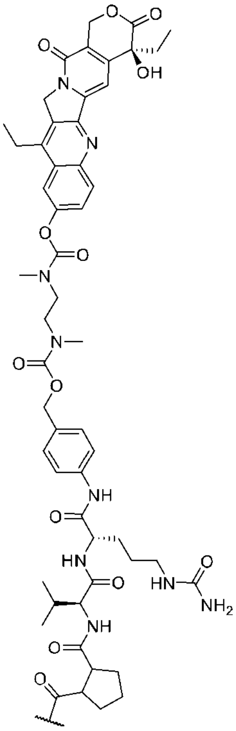

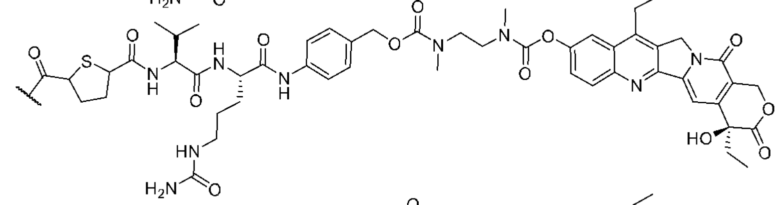

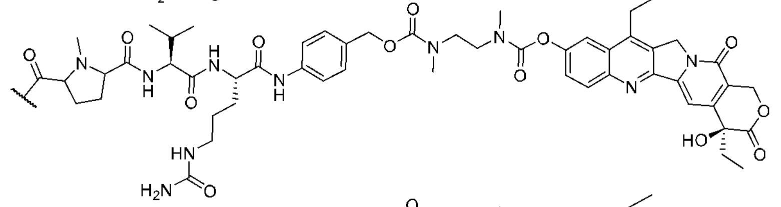

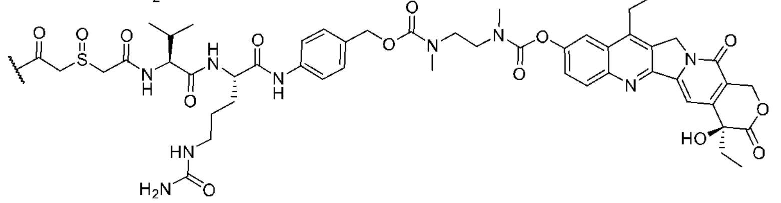

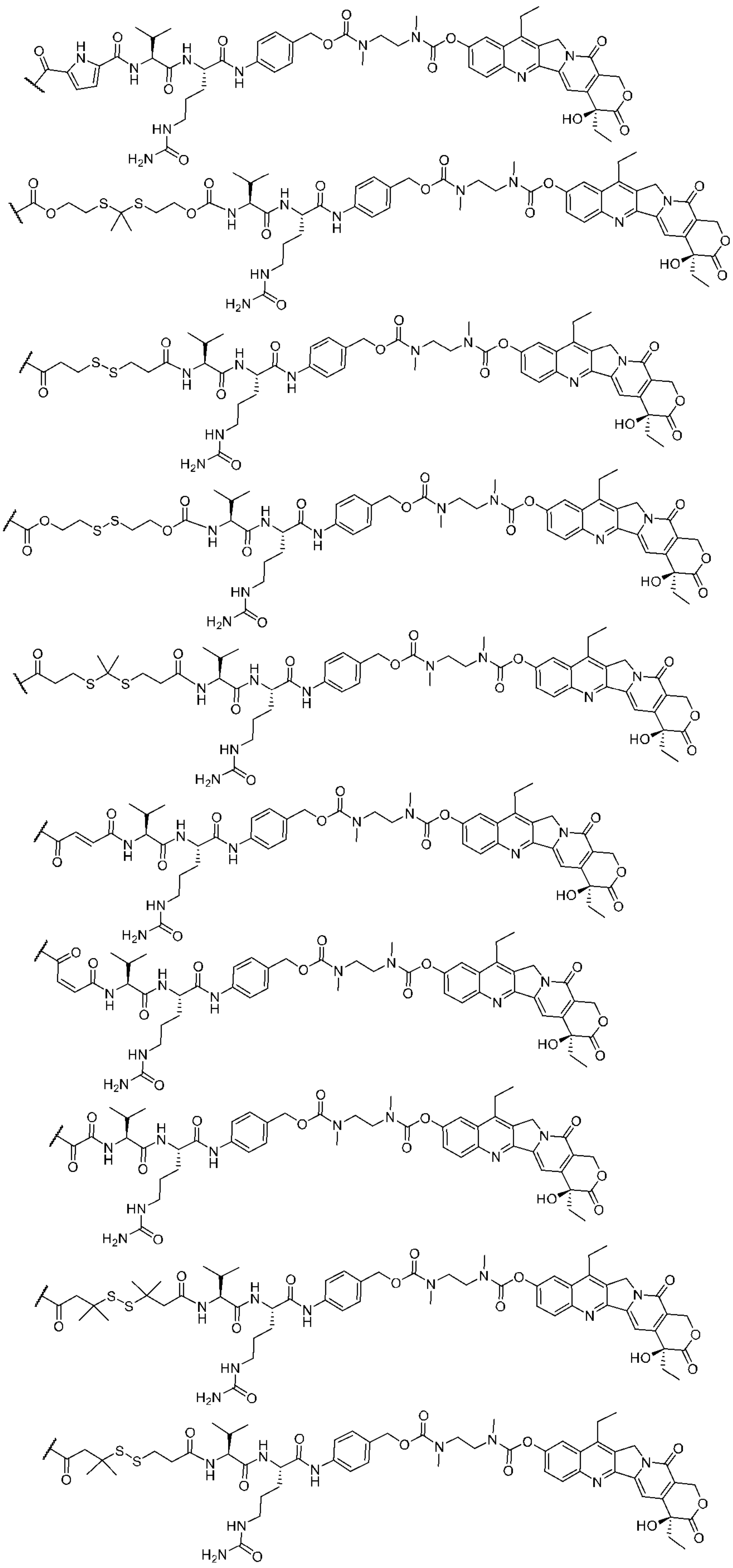

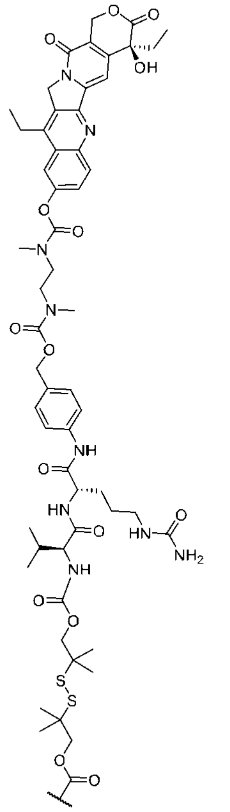

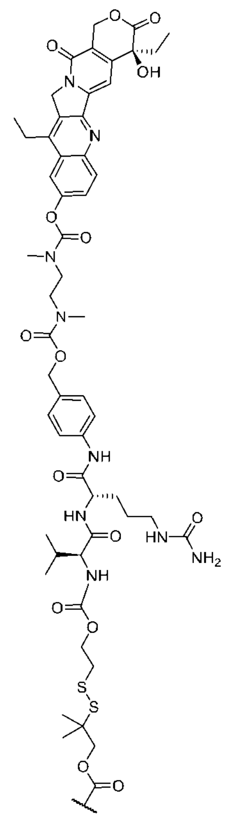

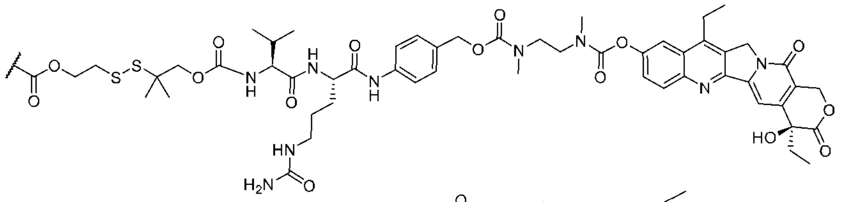

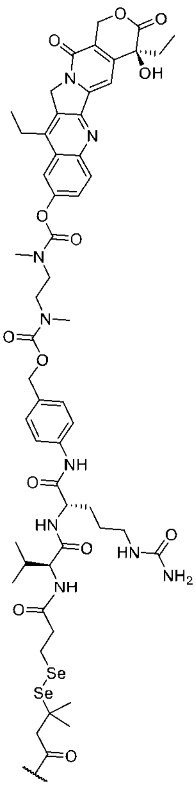

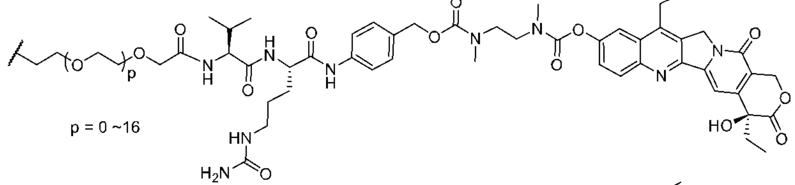

p = 0 ~16

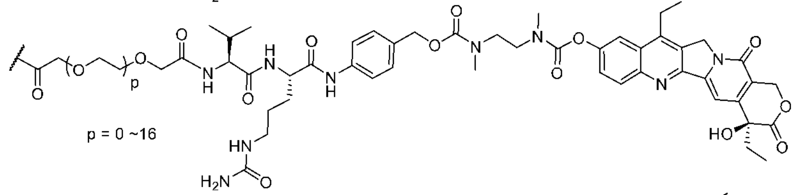

p = 0 ~16

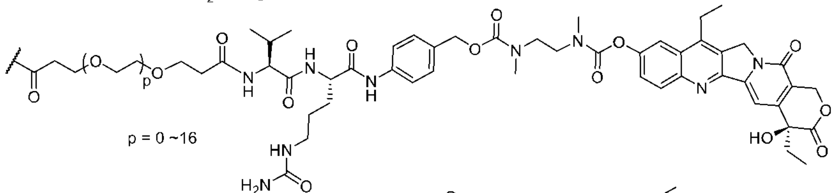

p = 0 ~16

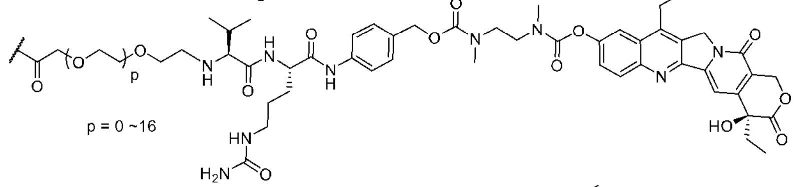

p = 0 ~16

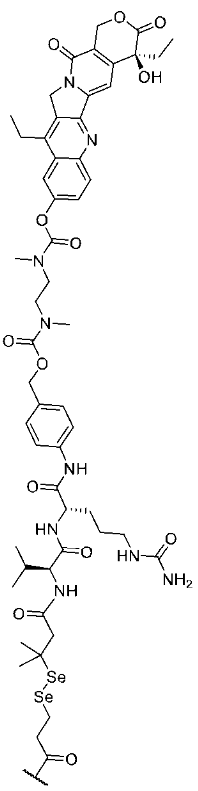

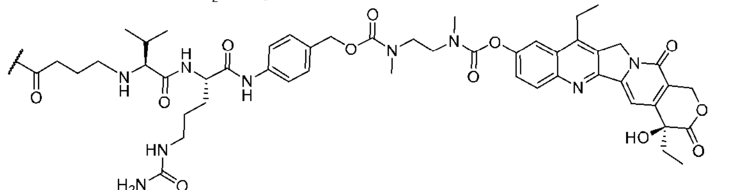

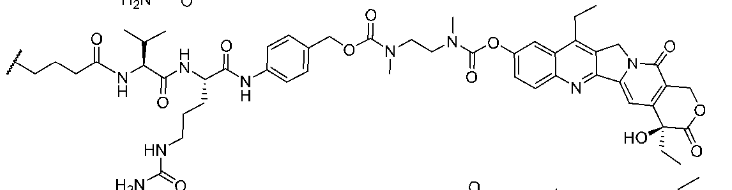

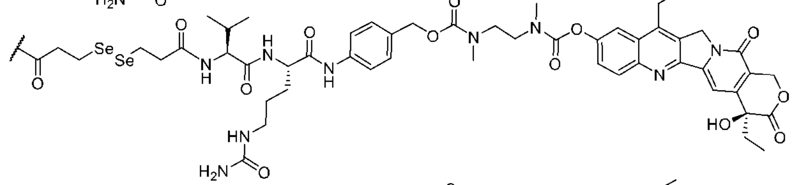

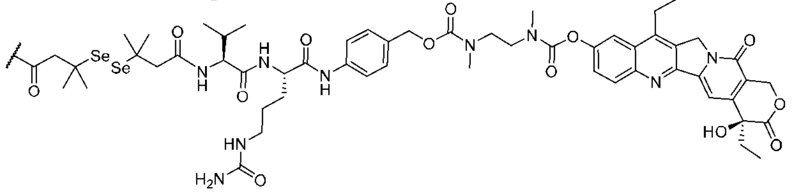

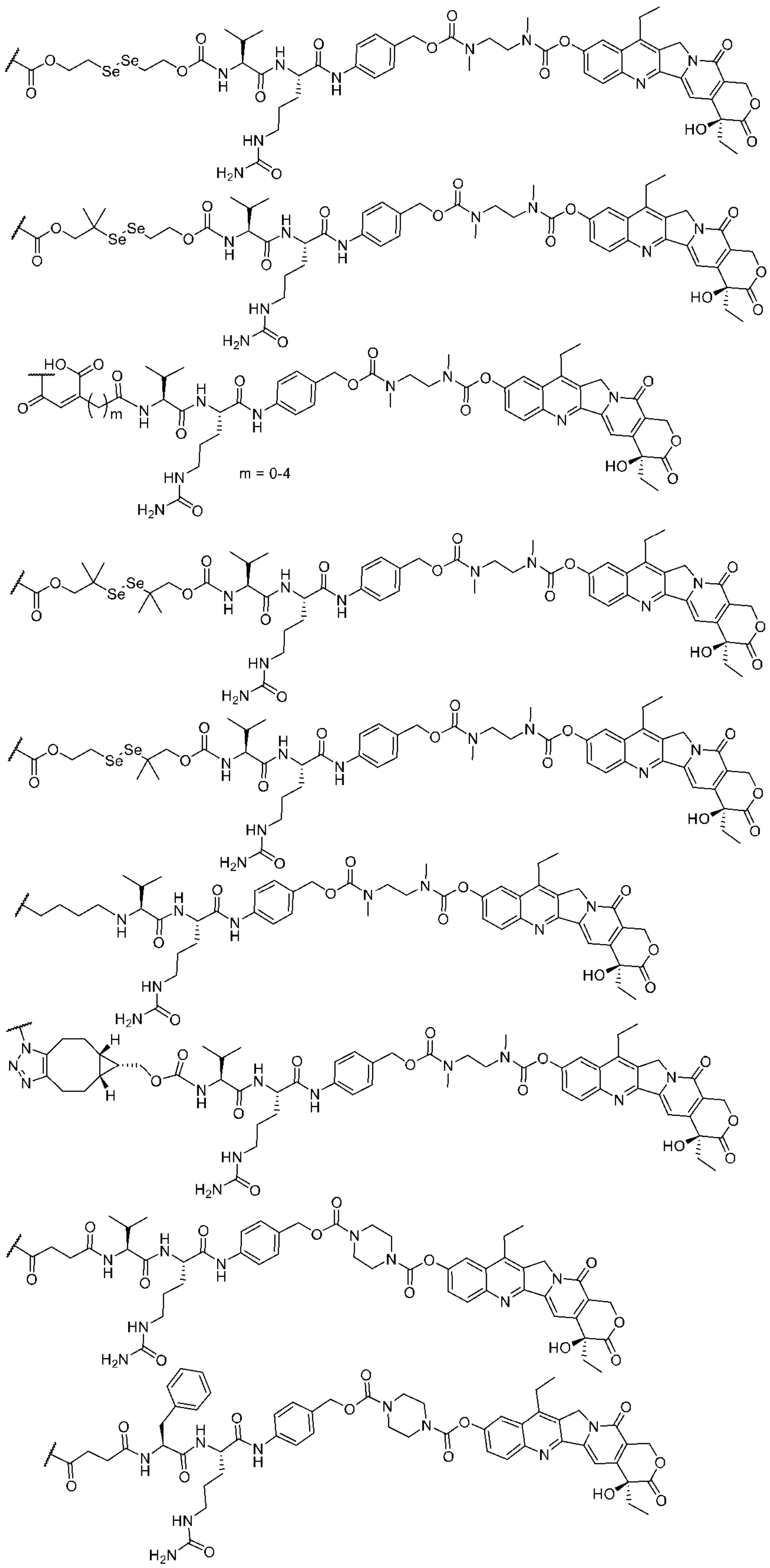

m = 0-4

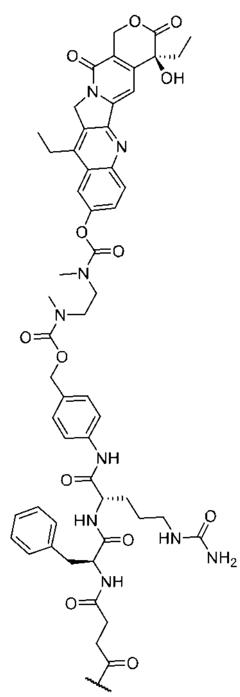

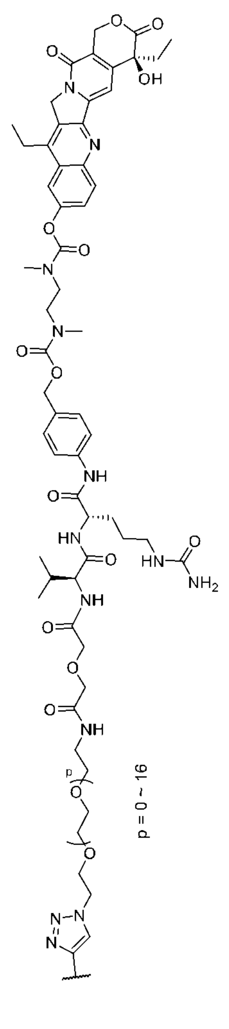

p = 0 ~ 16

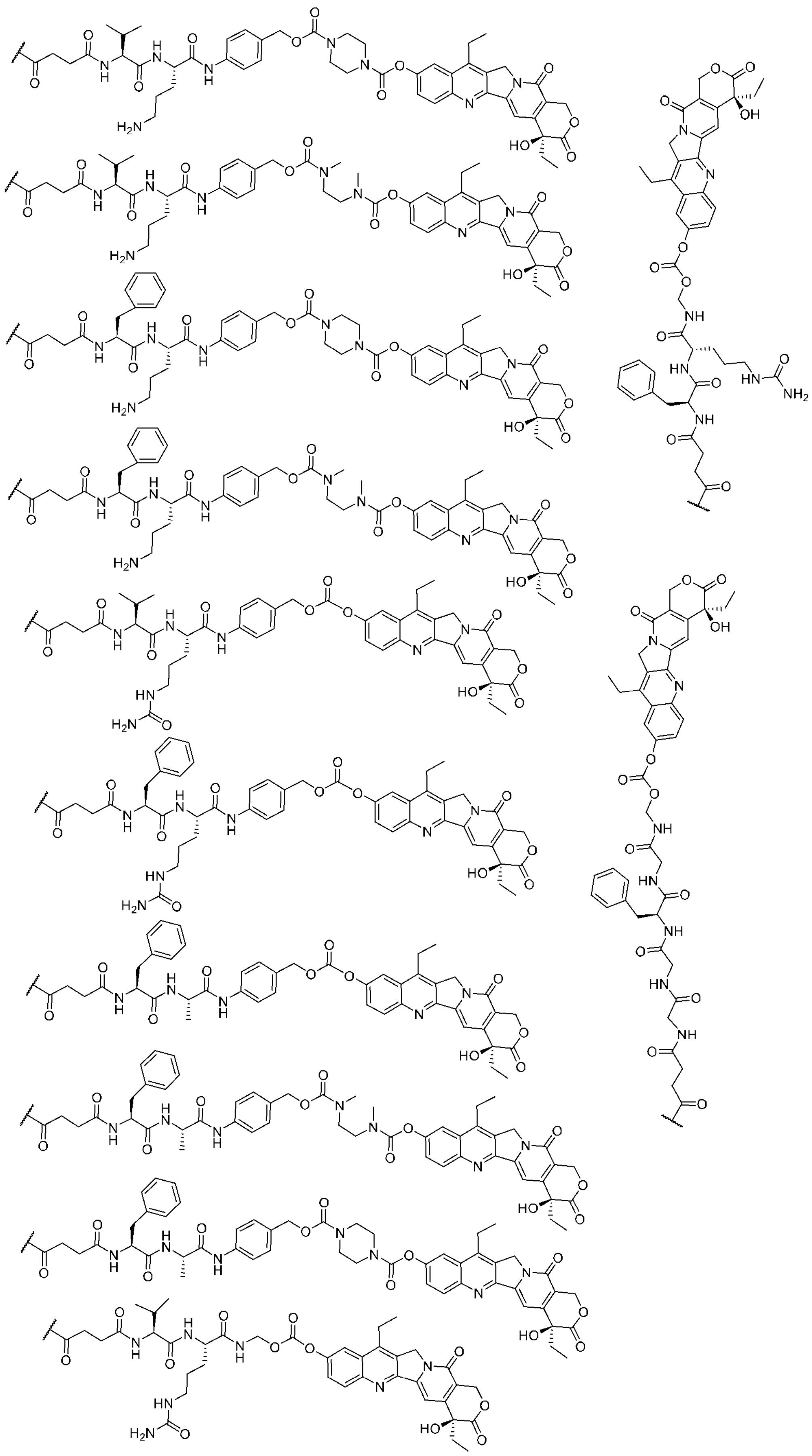

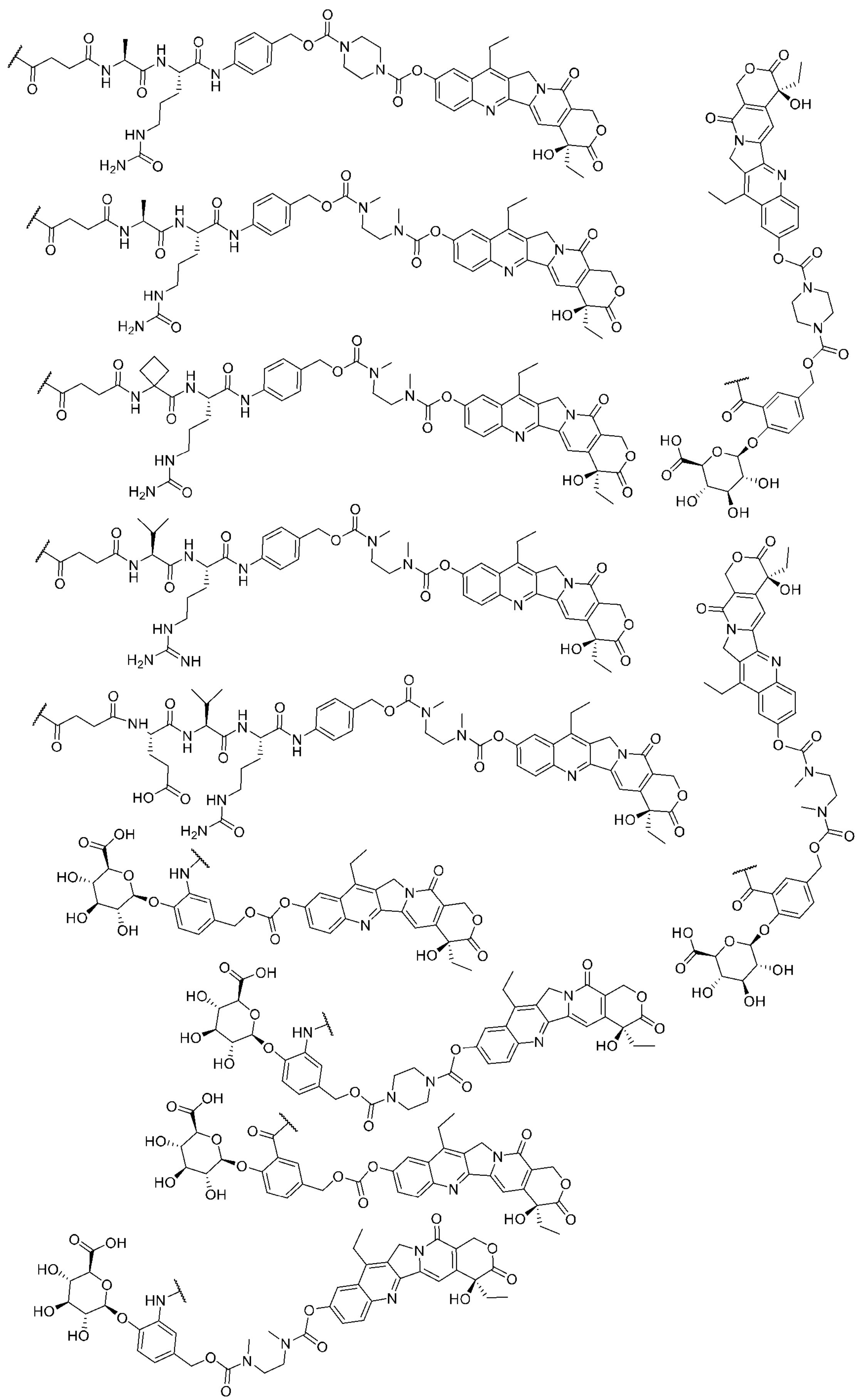

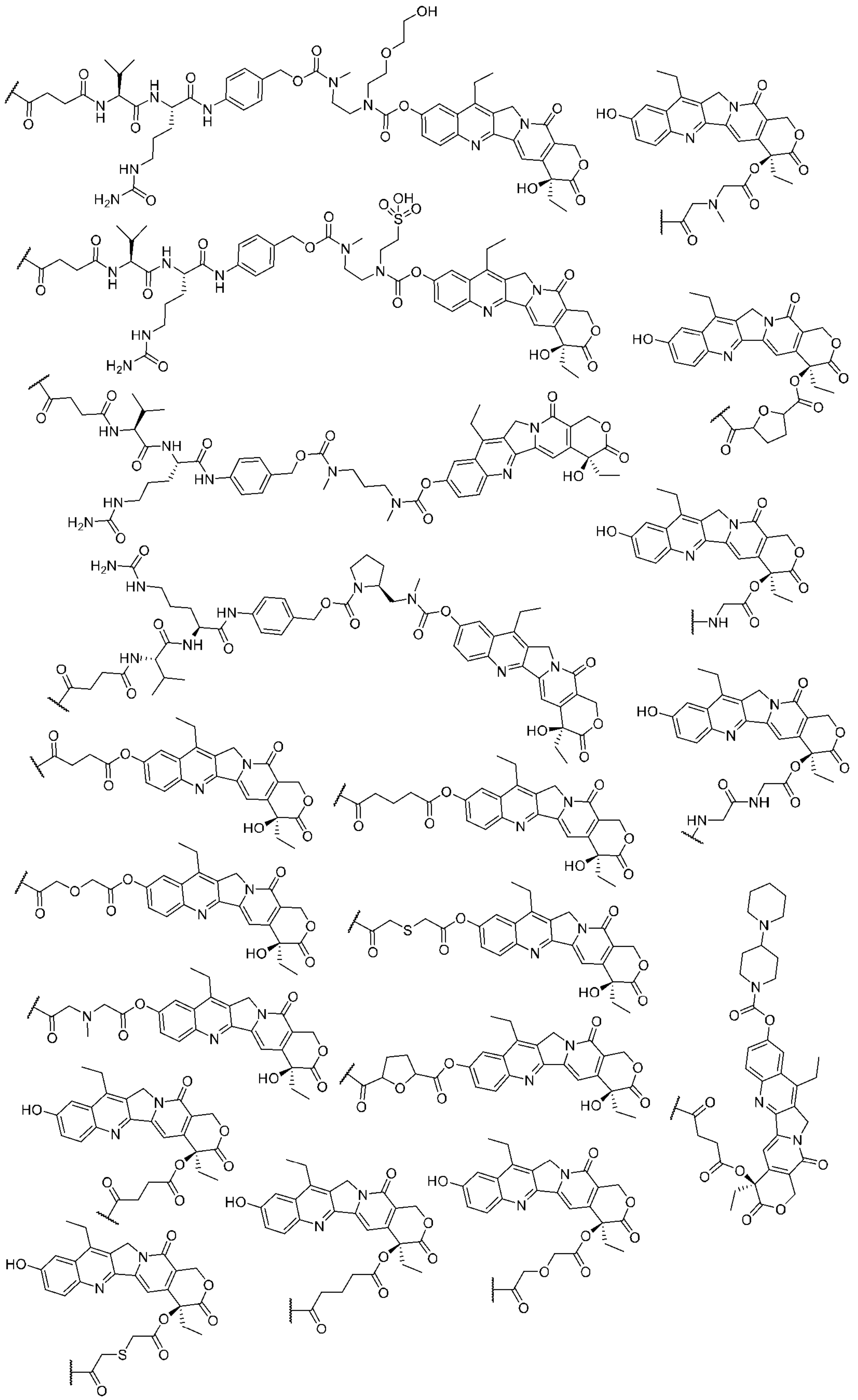

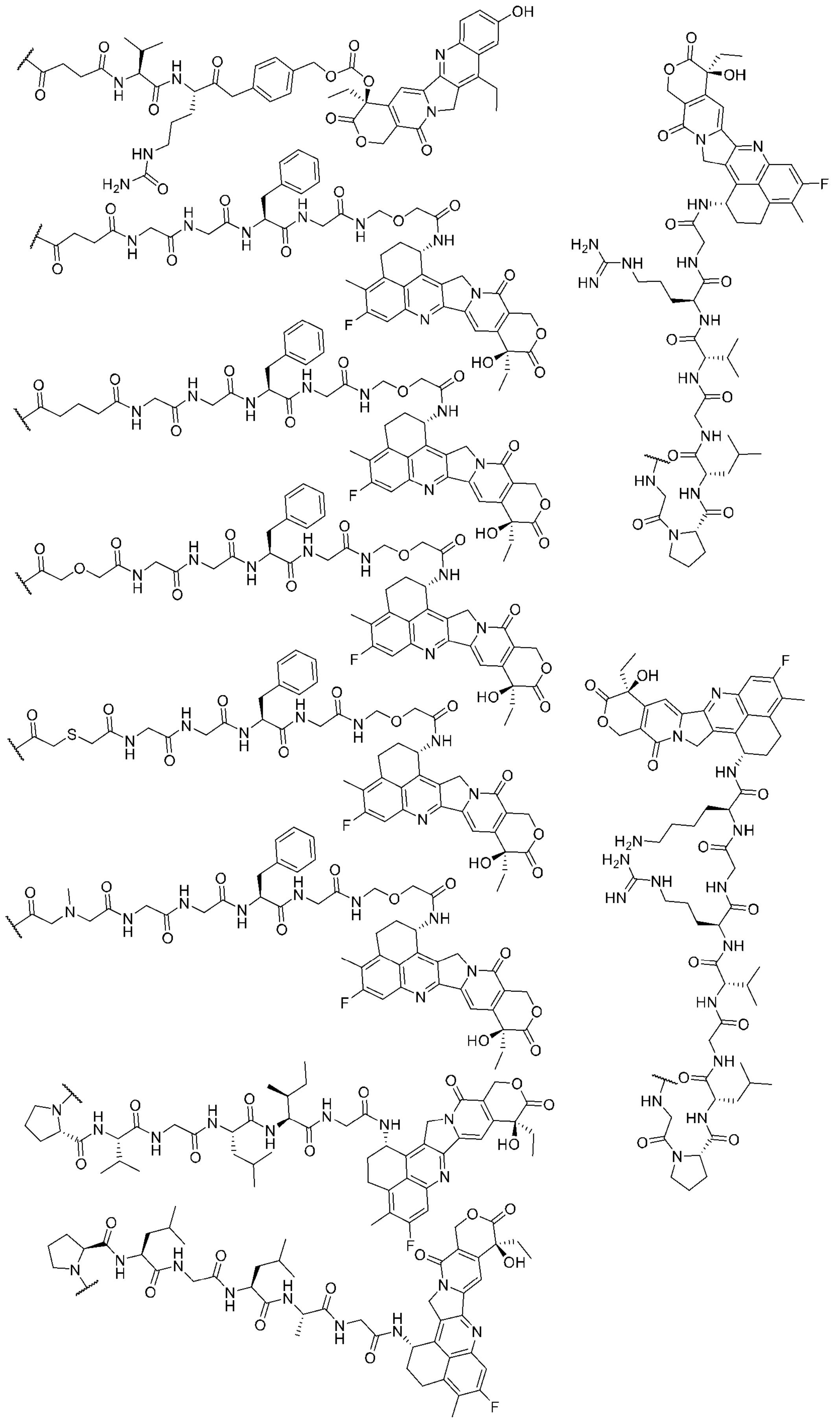

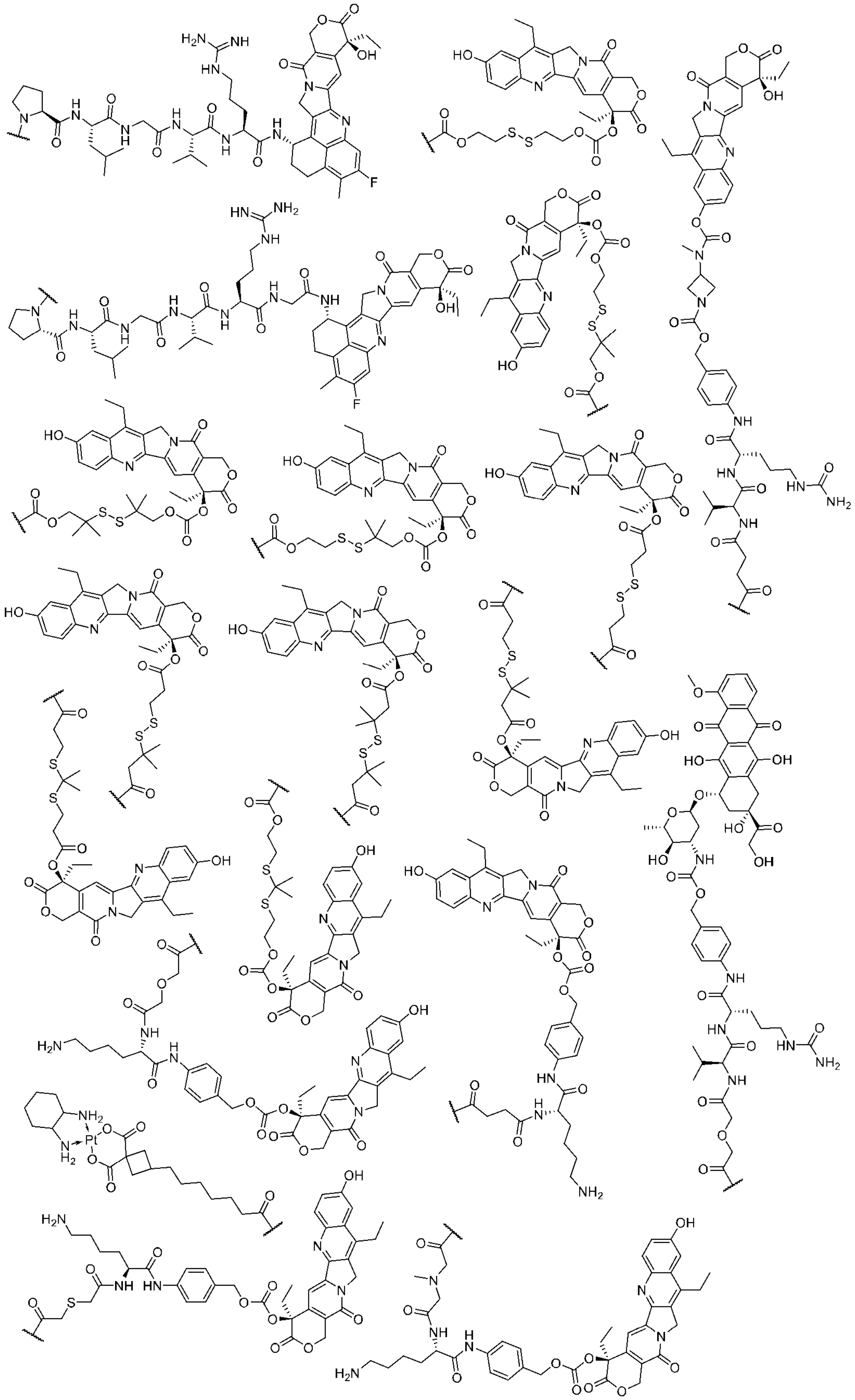

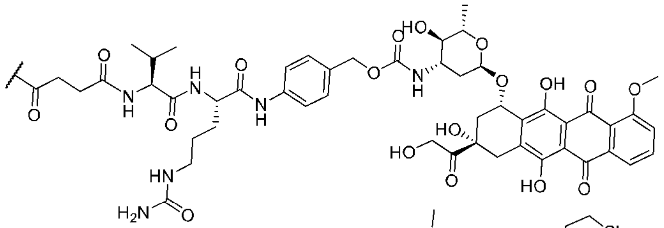
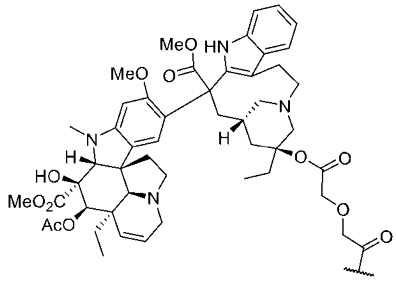
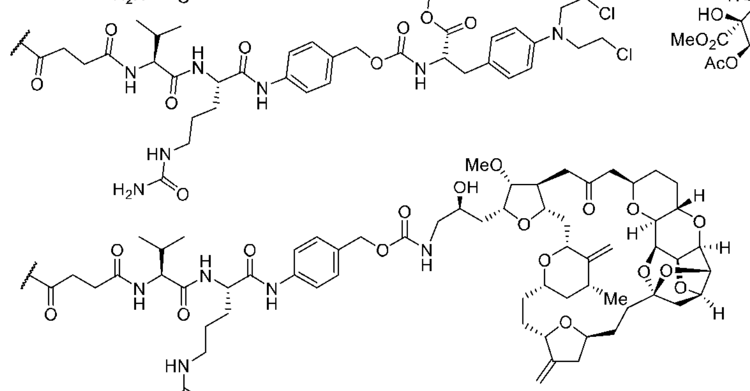
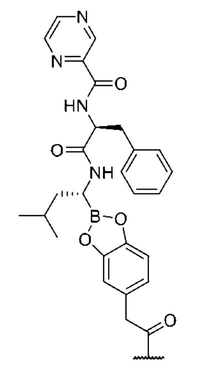
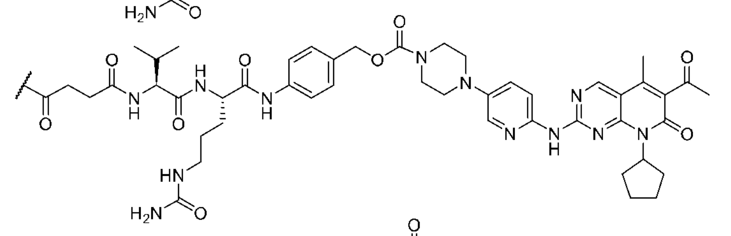
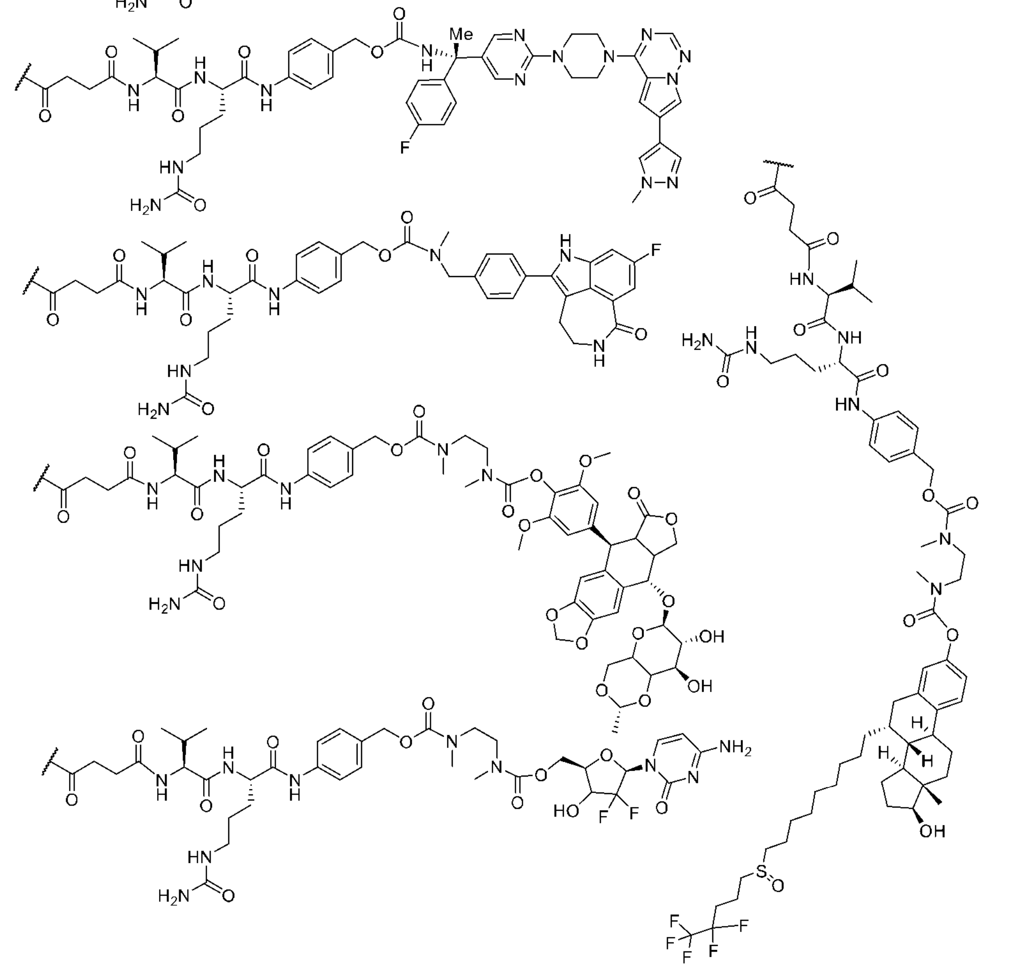

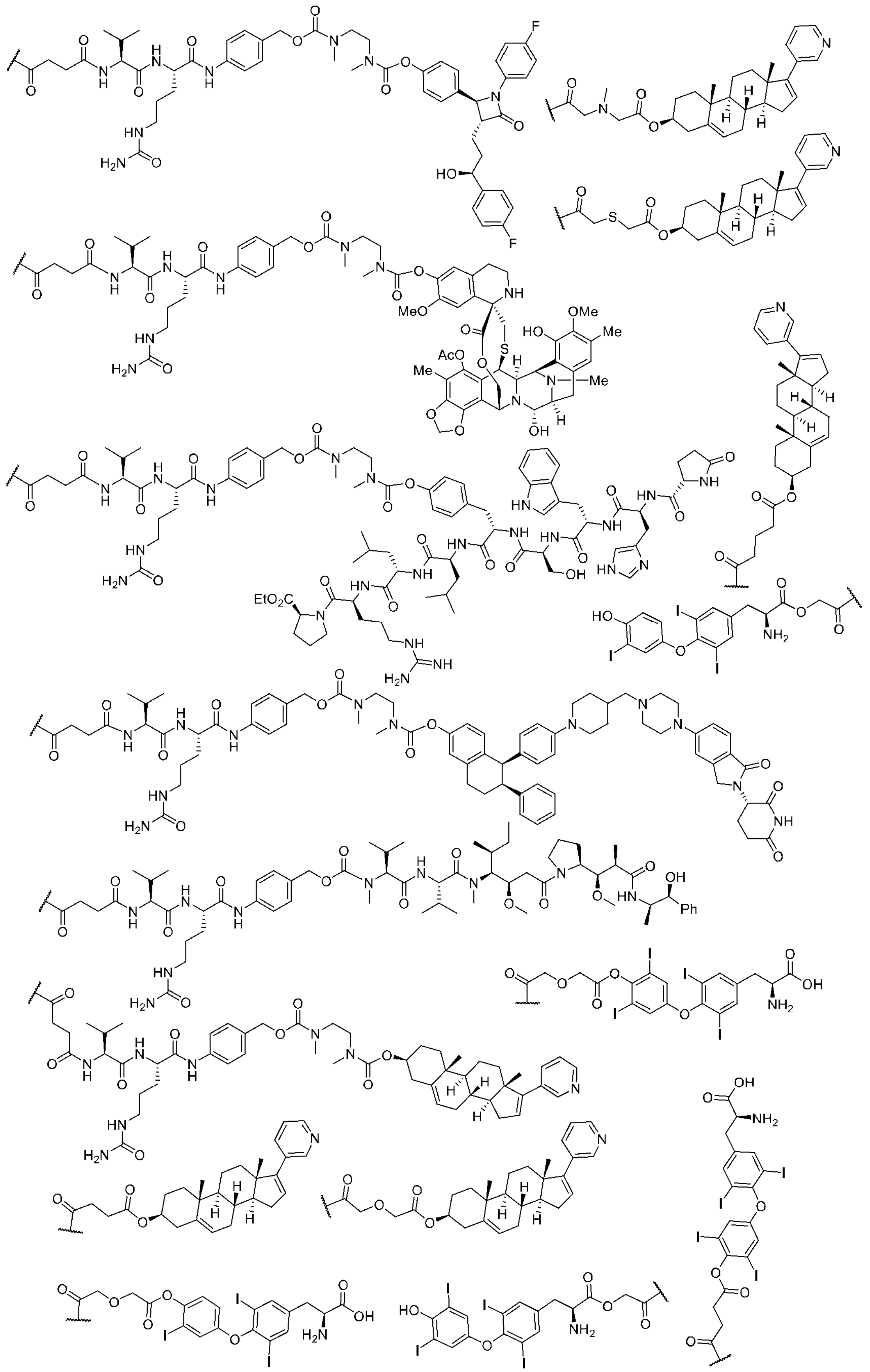

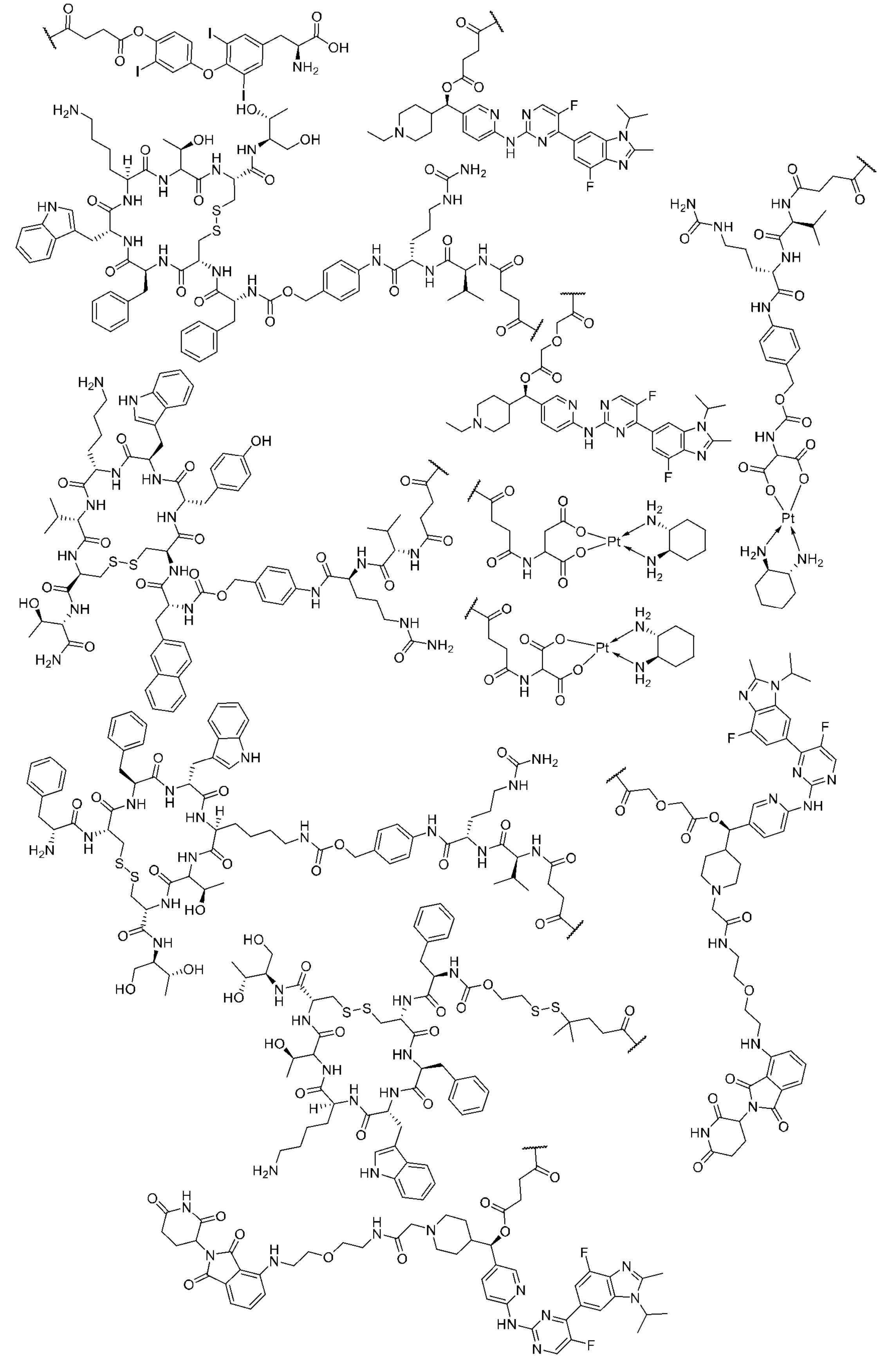

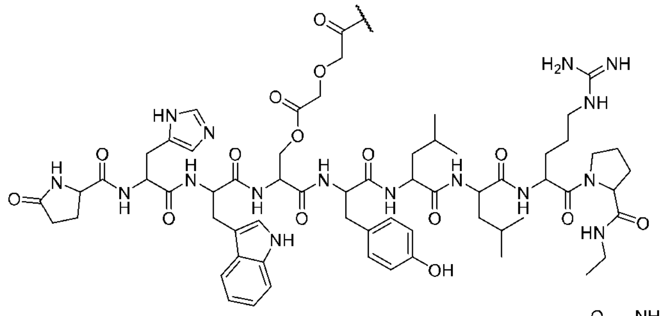

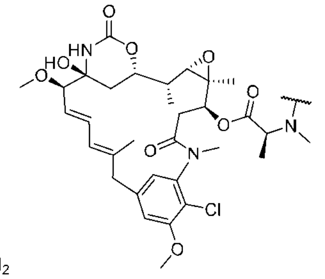

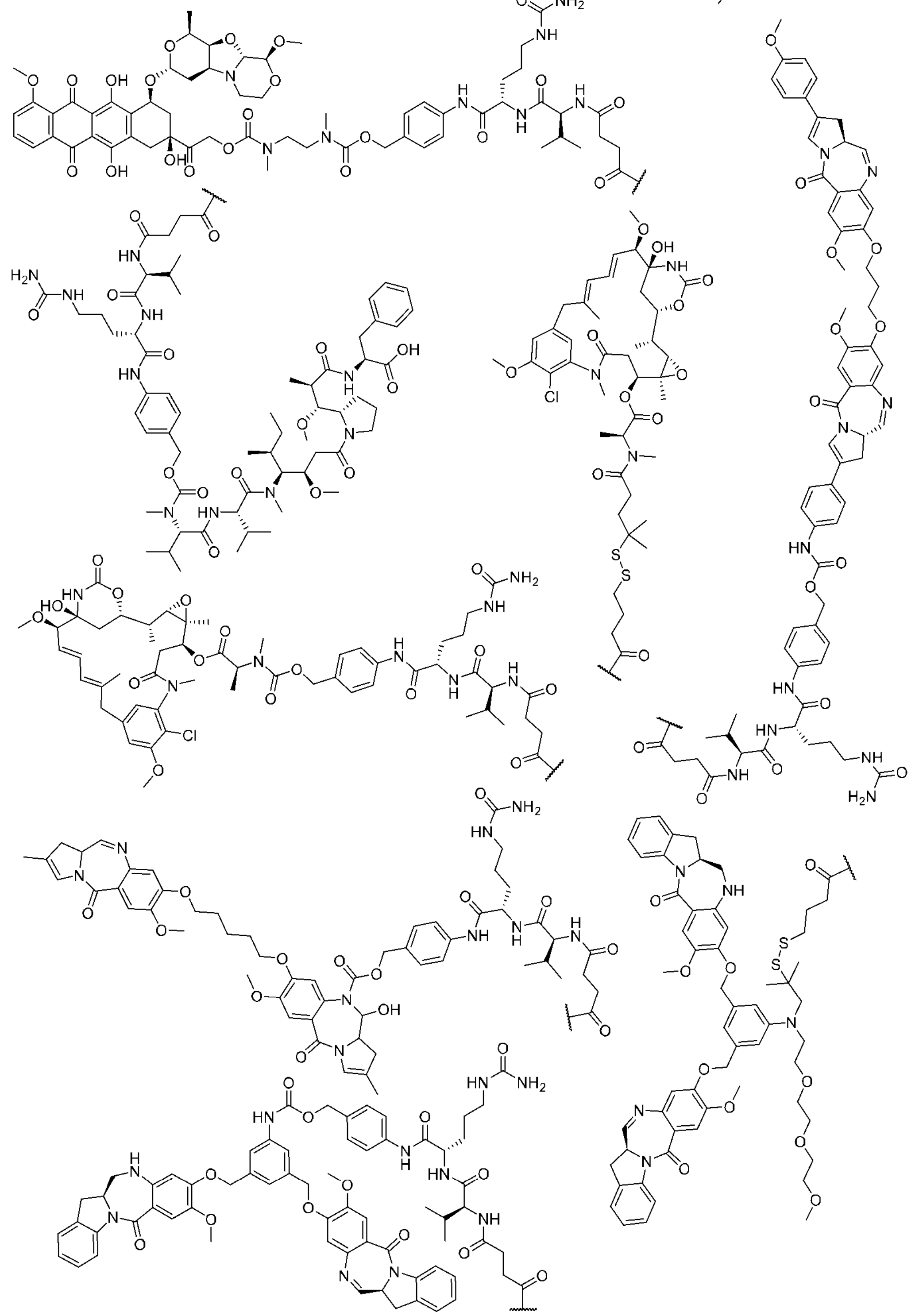

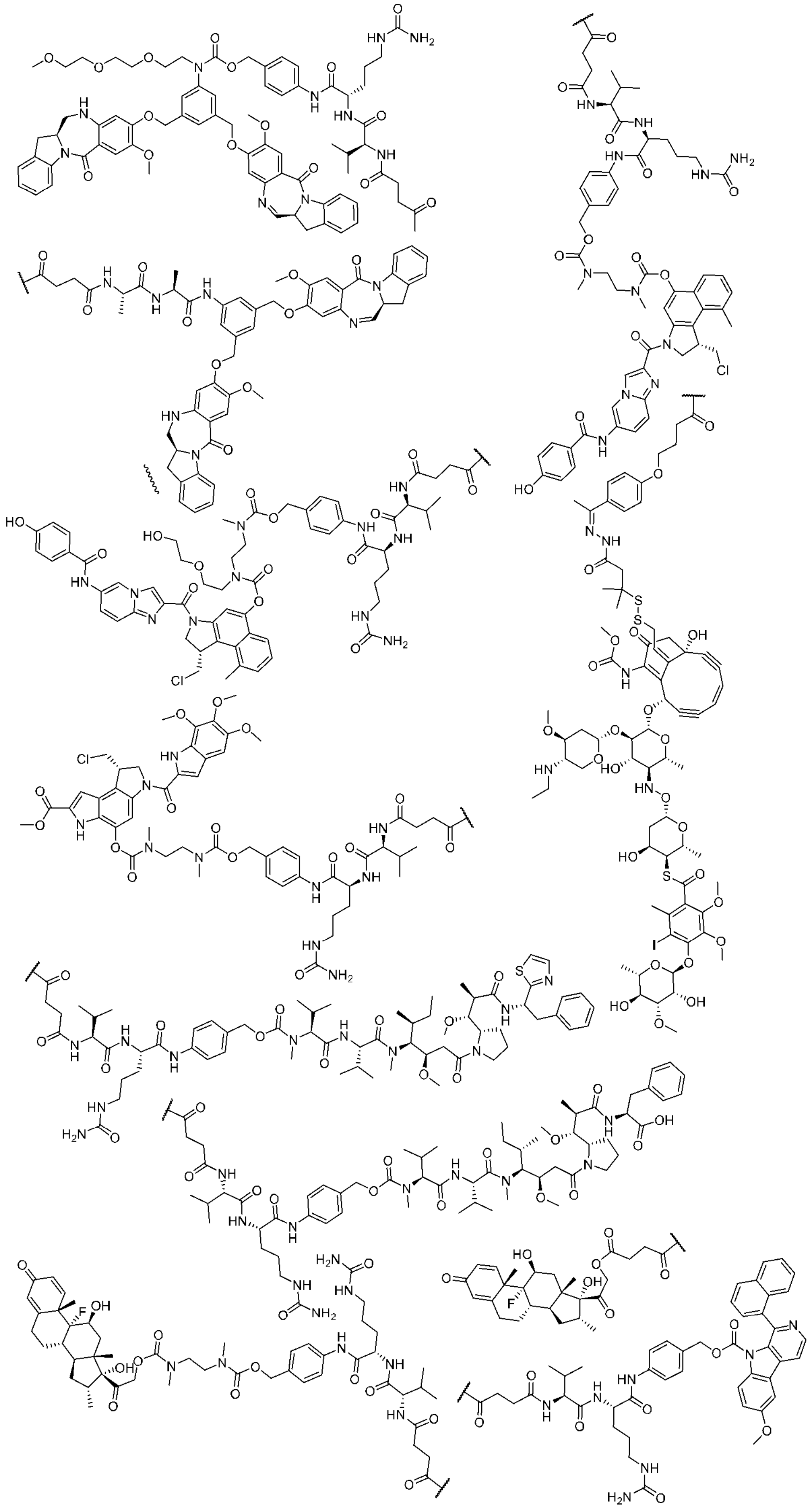

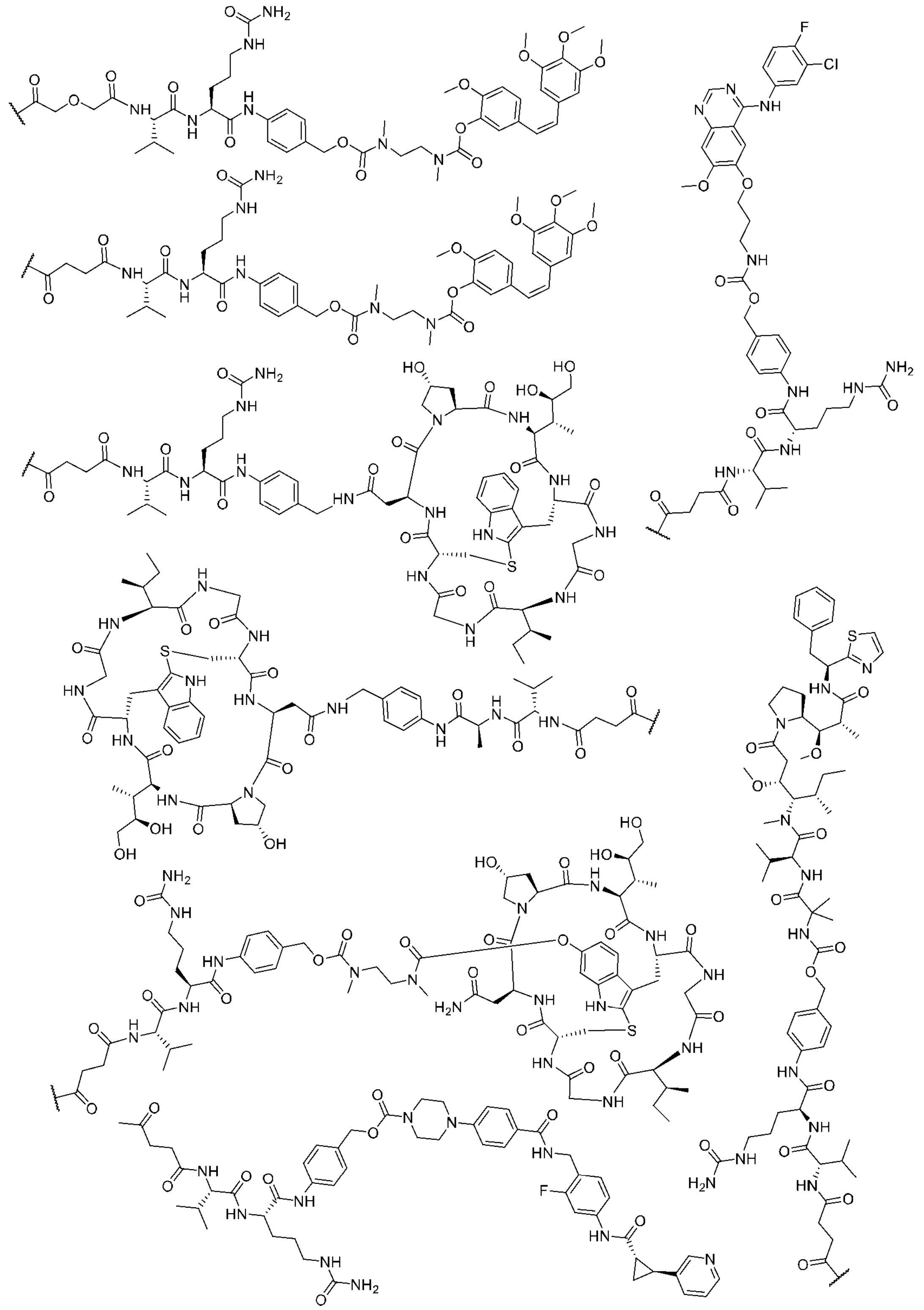

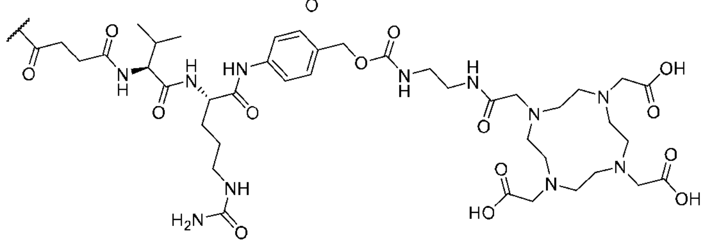

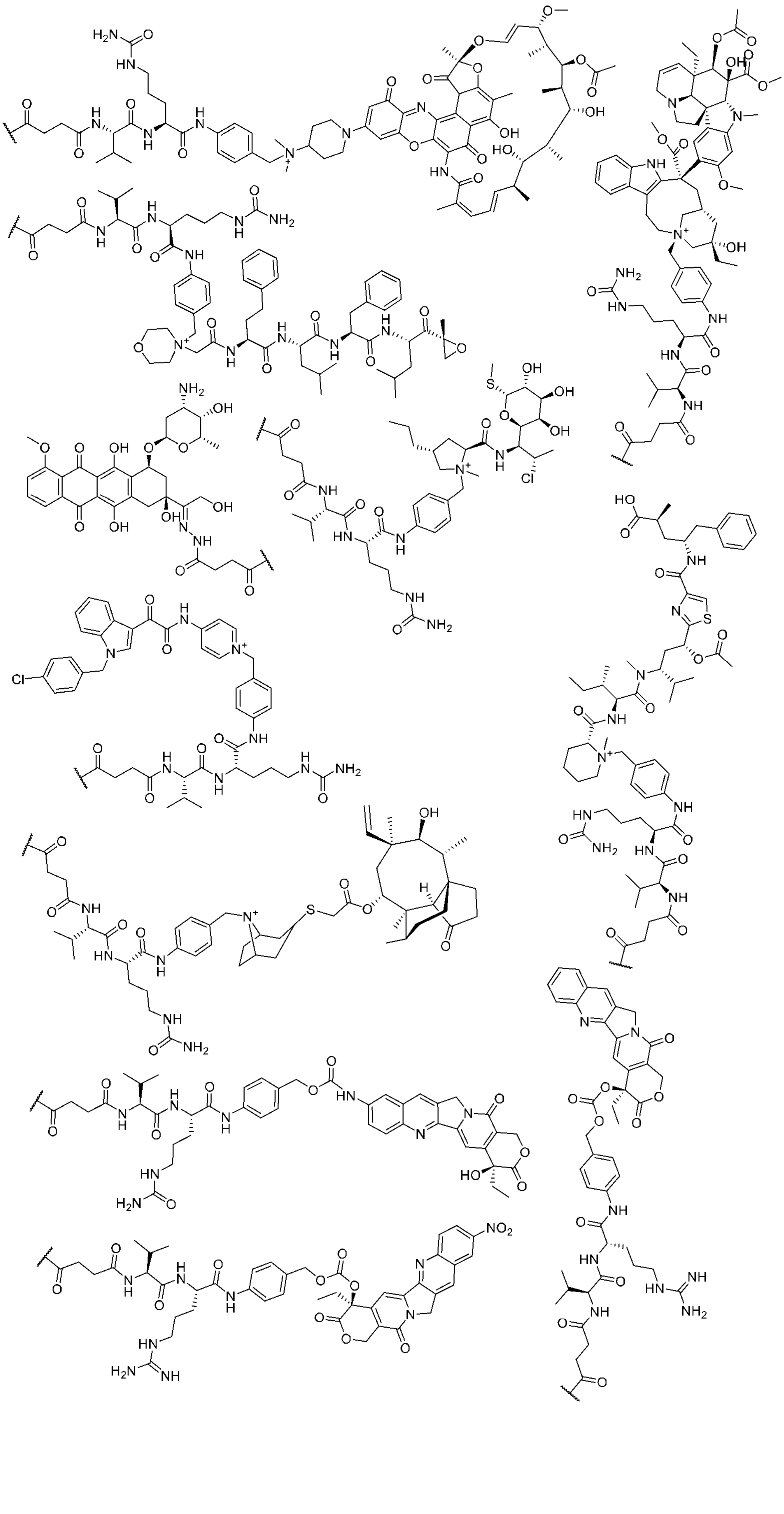

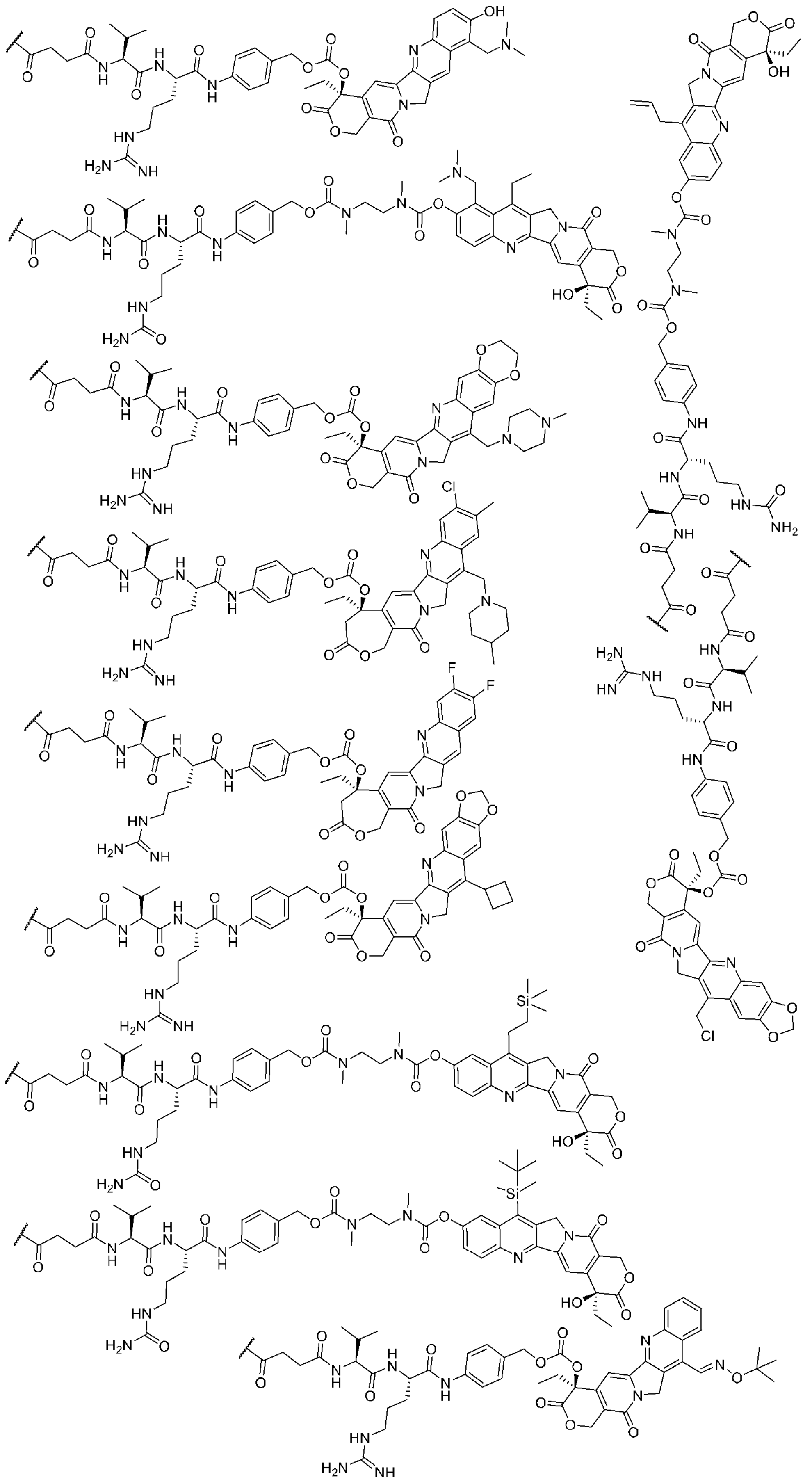

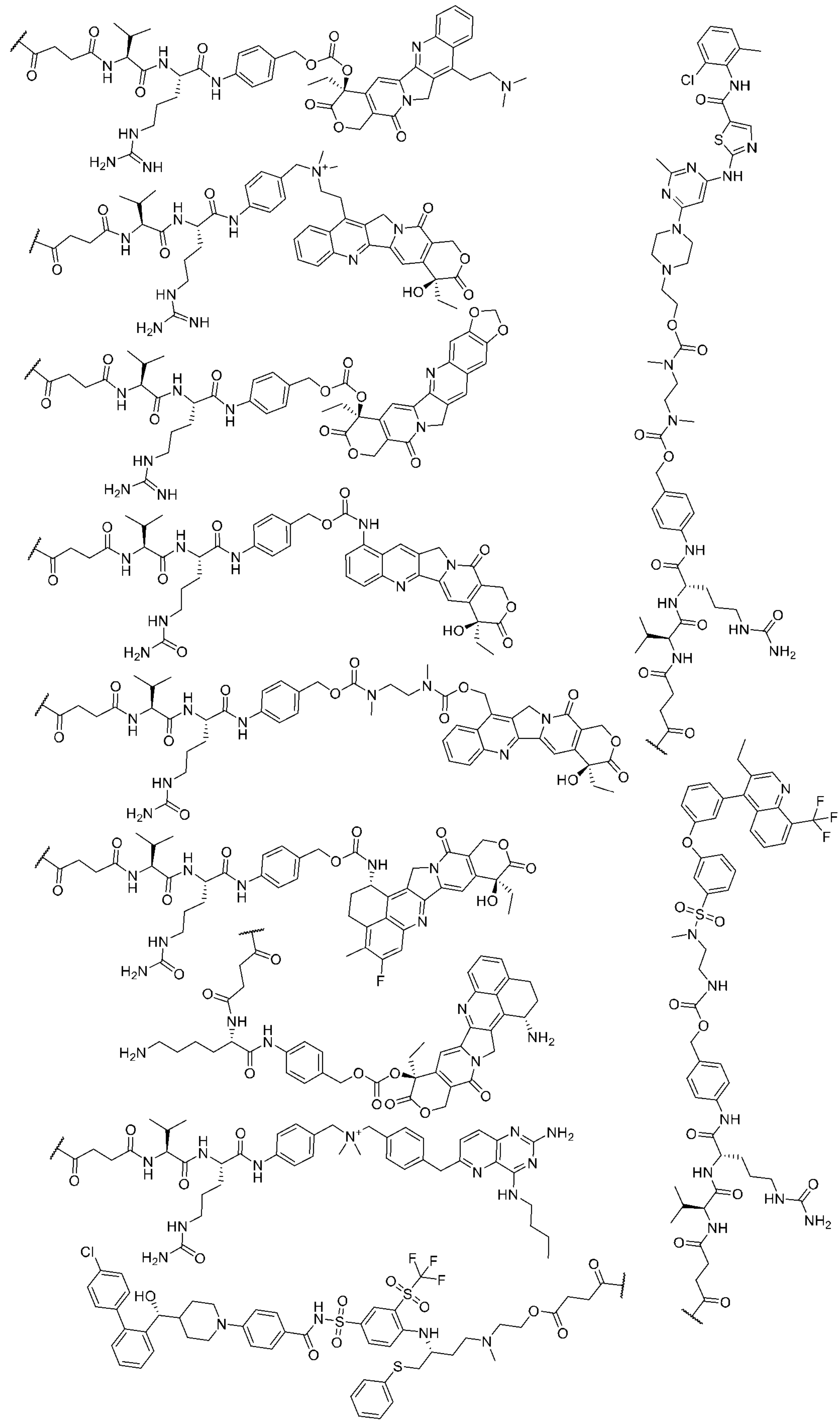

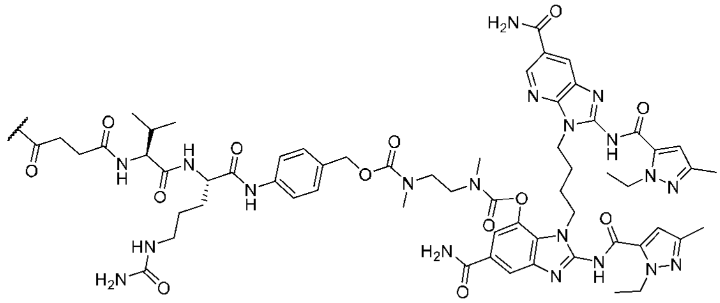

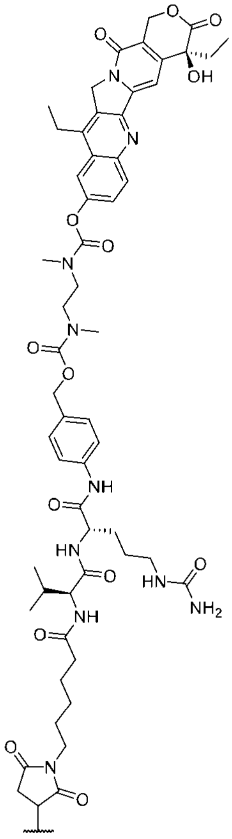

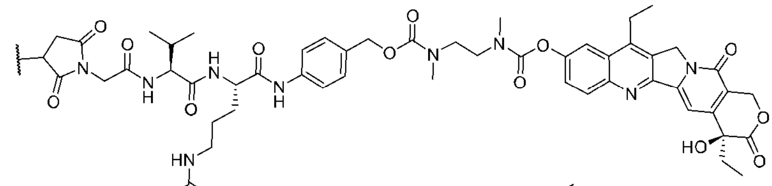

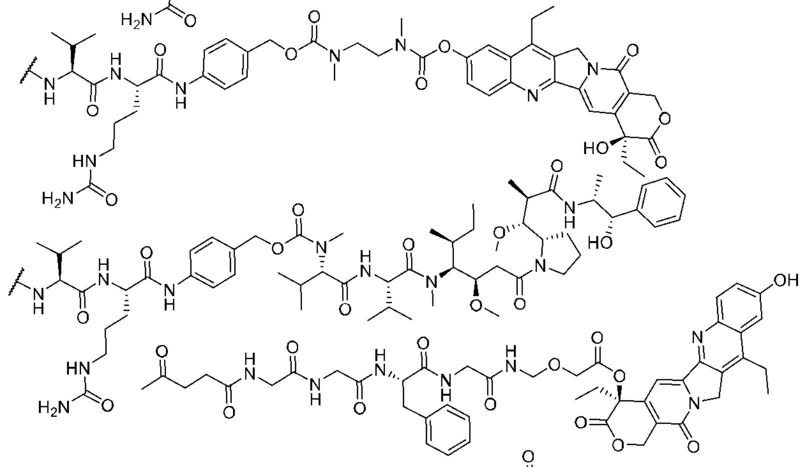

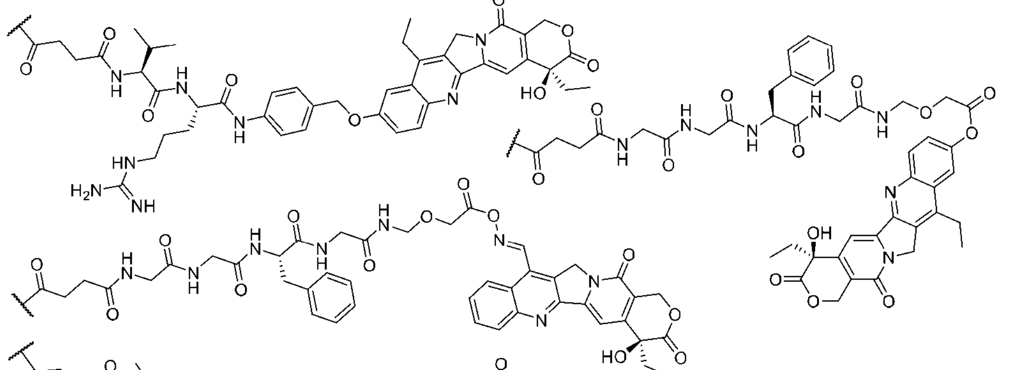

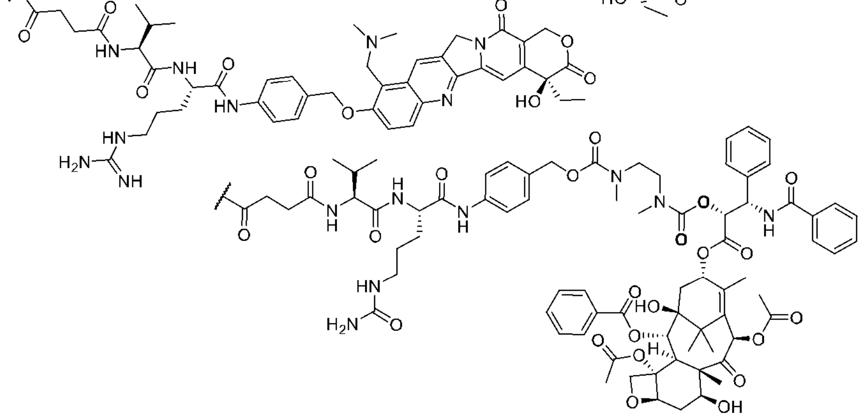

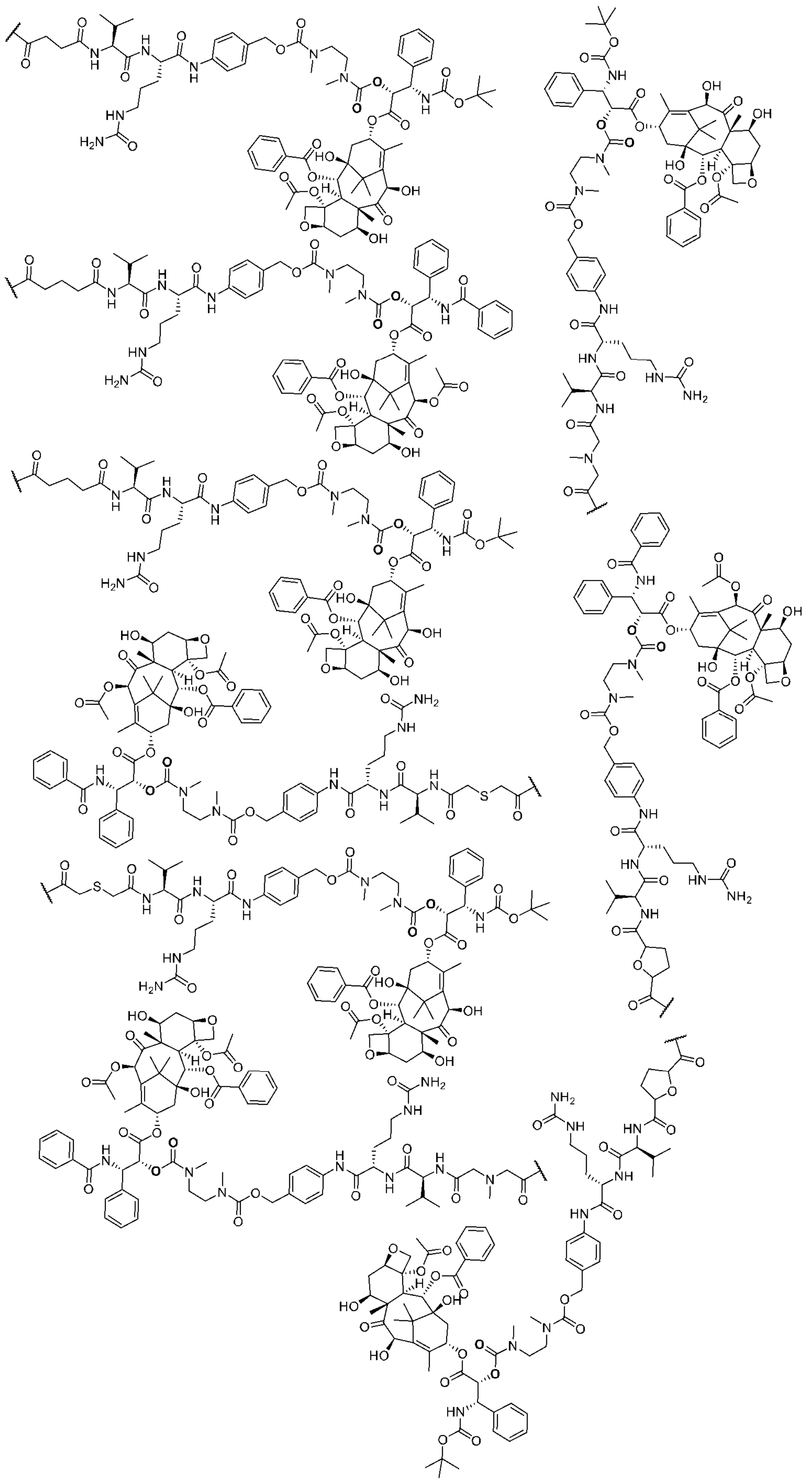

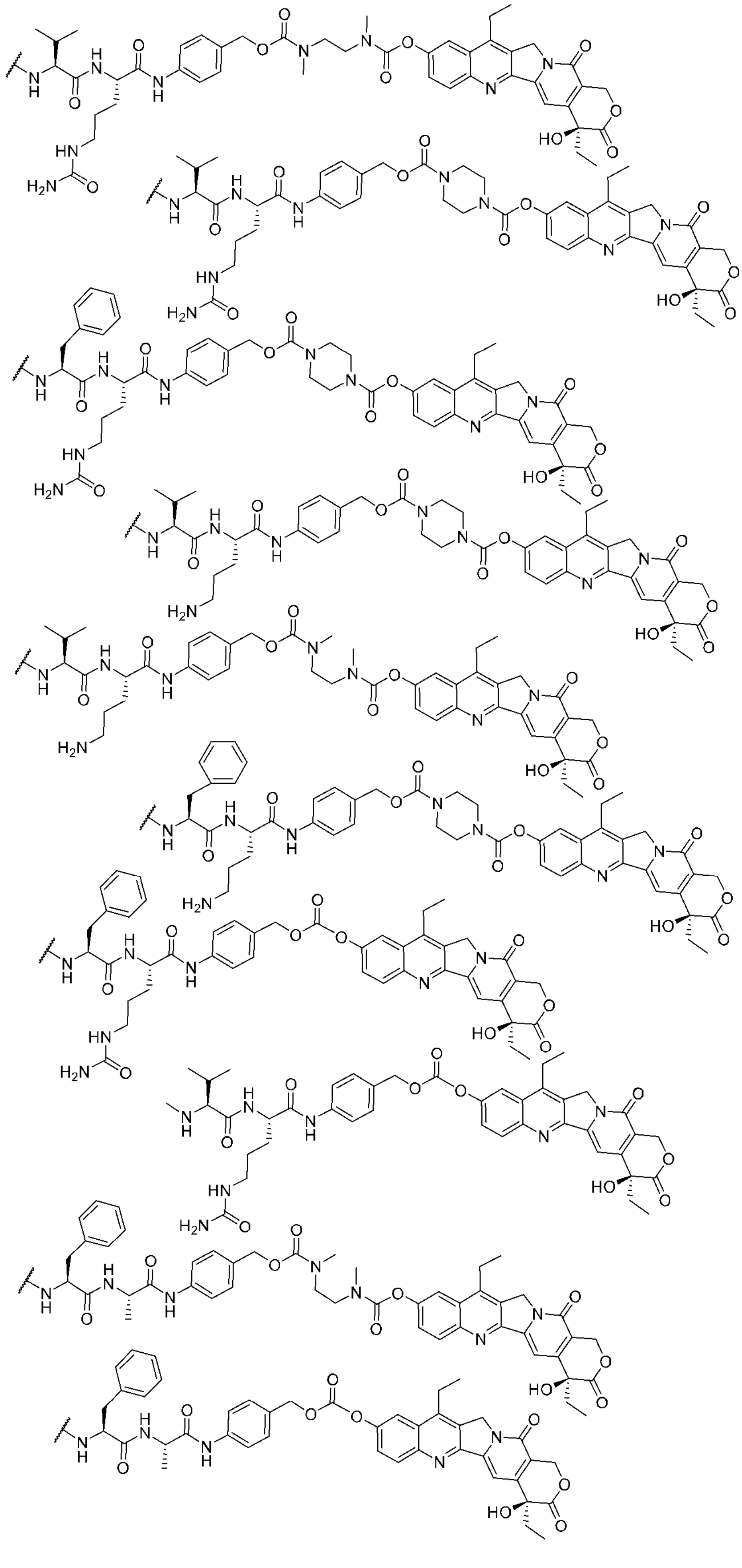

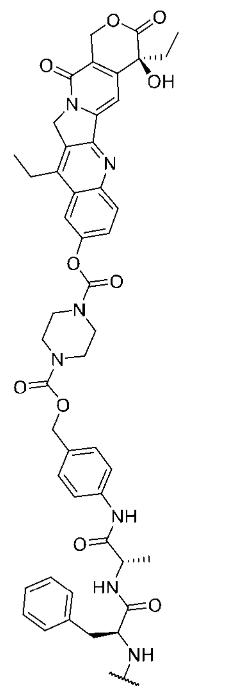

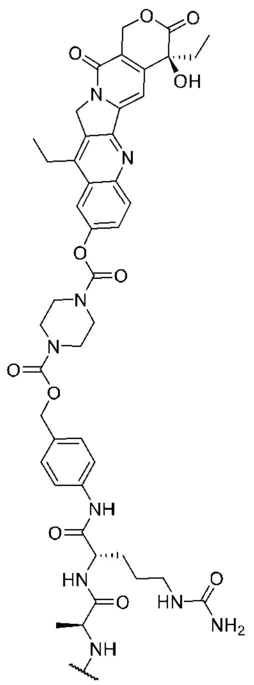

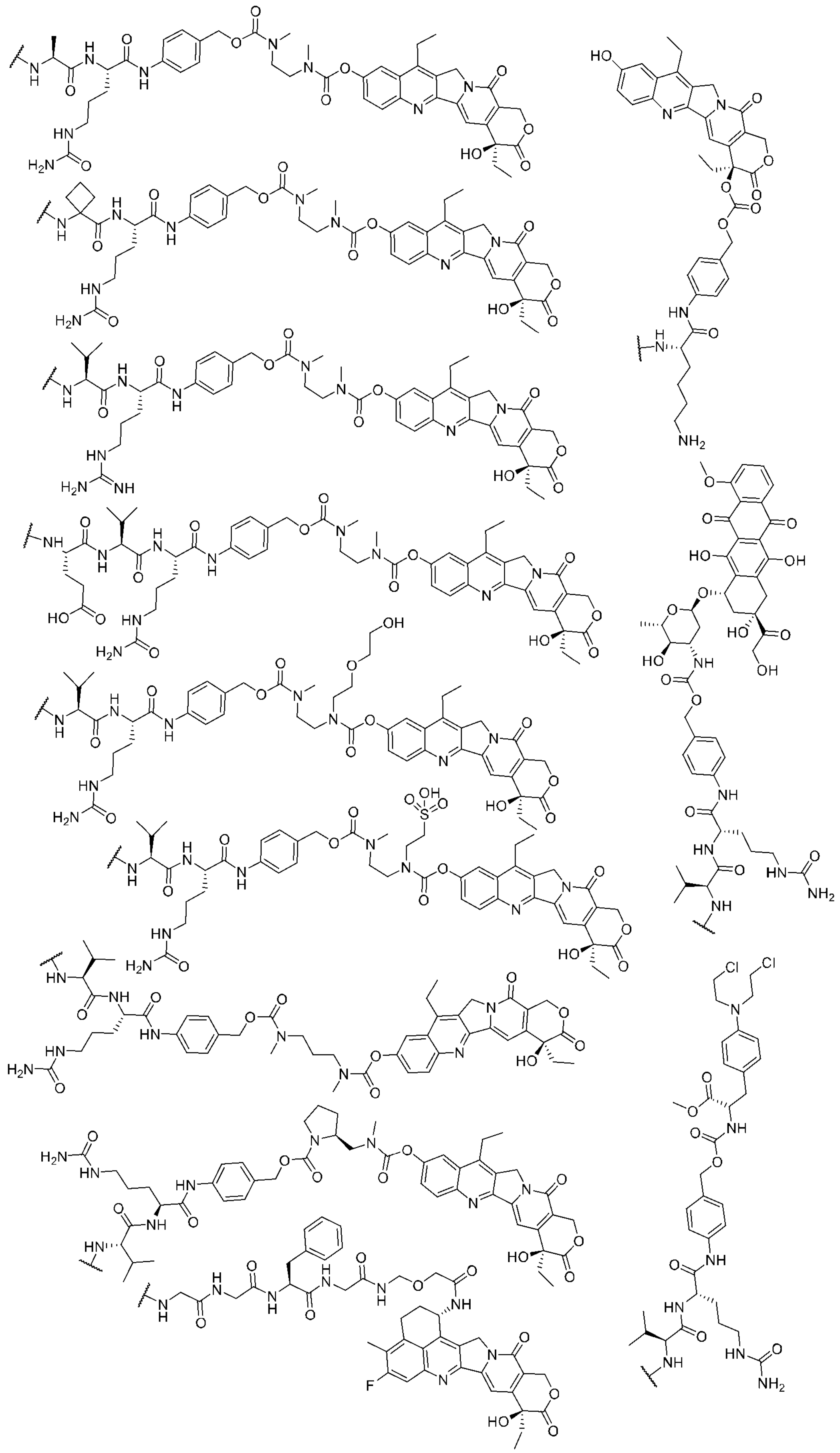

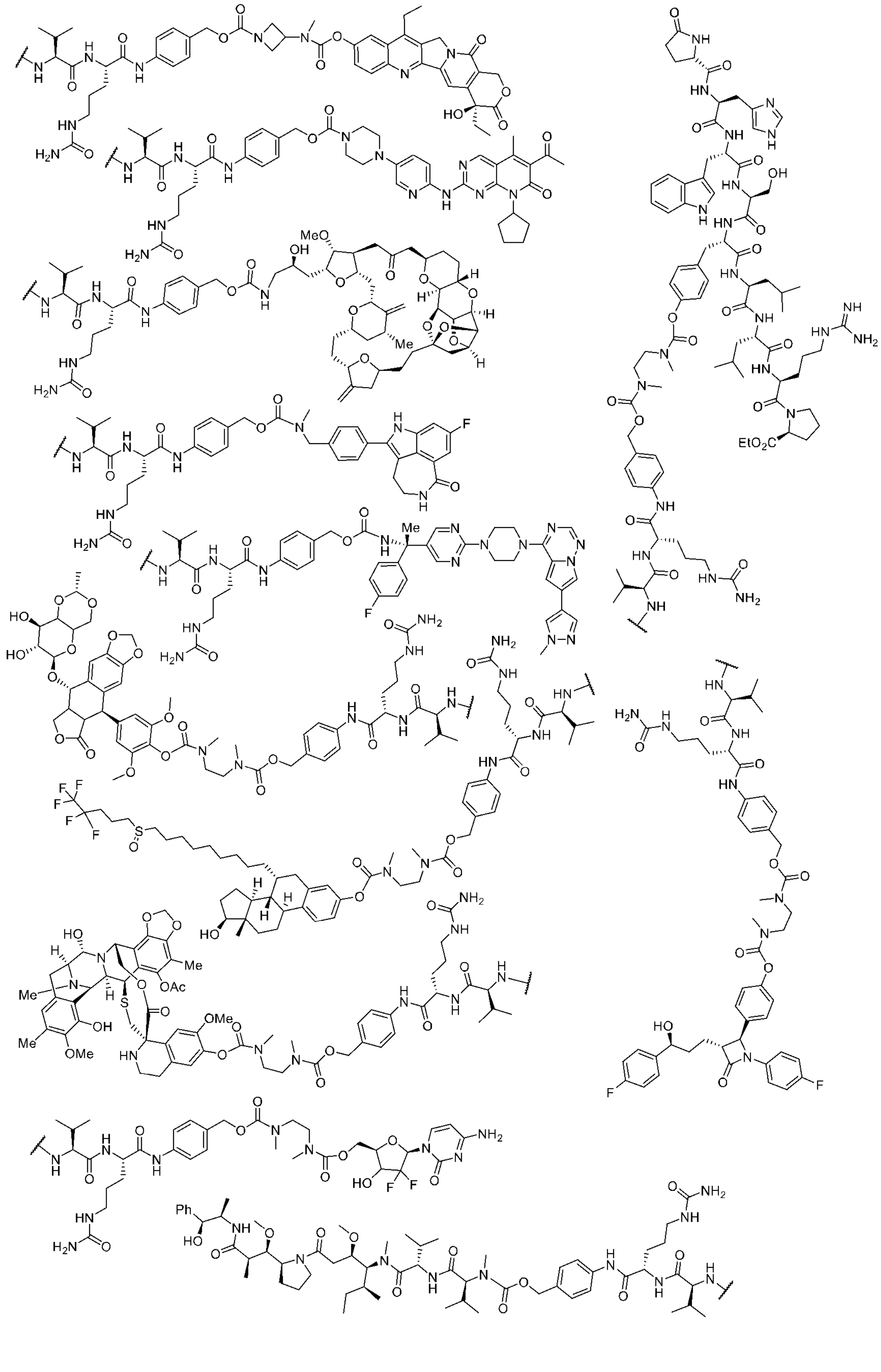

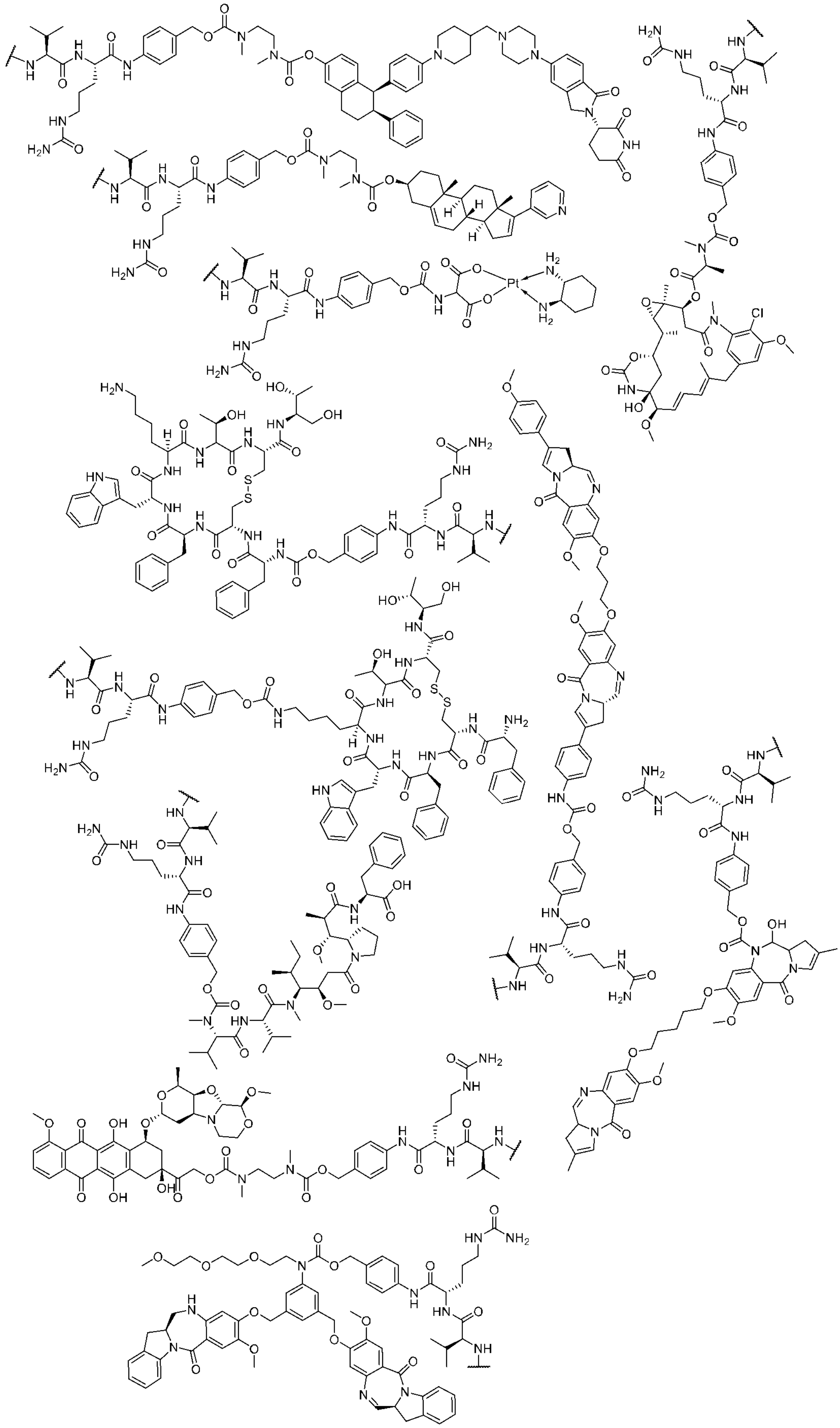

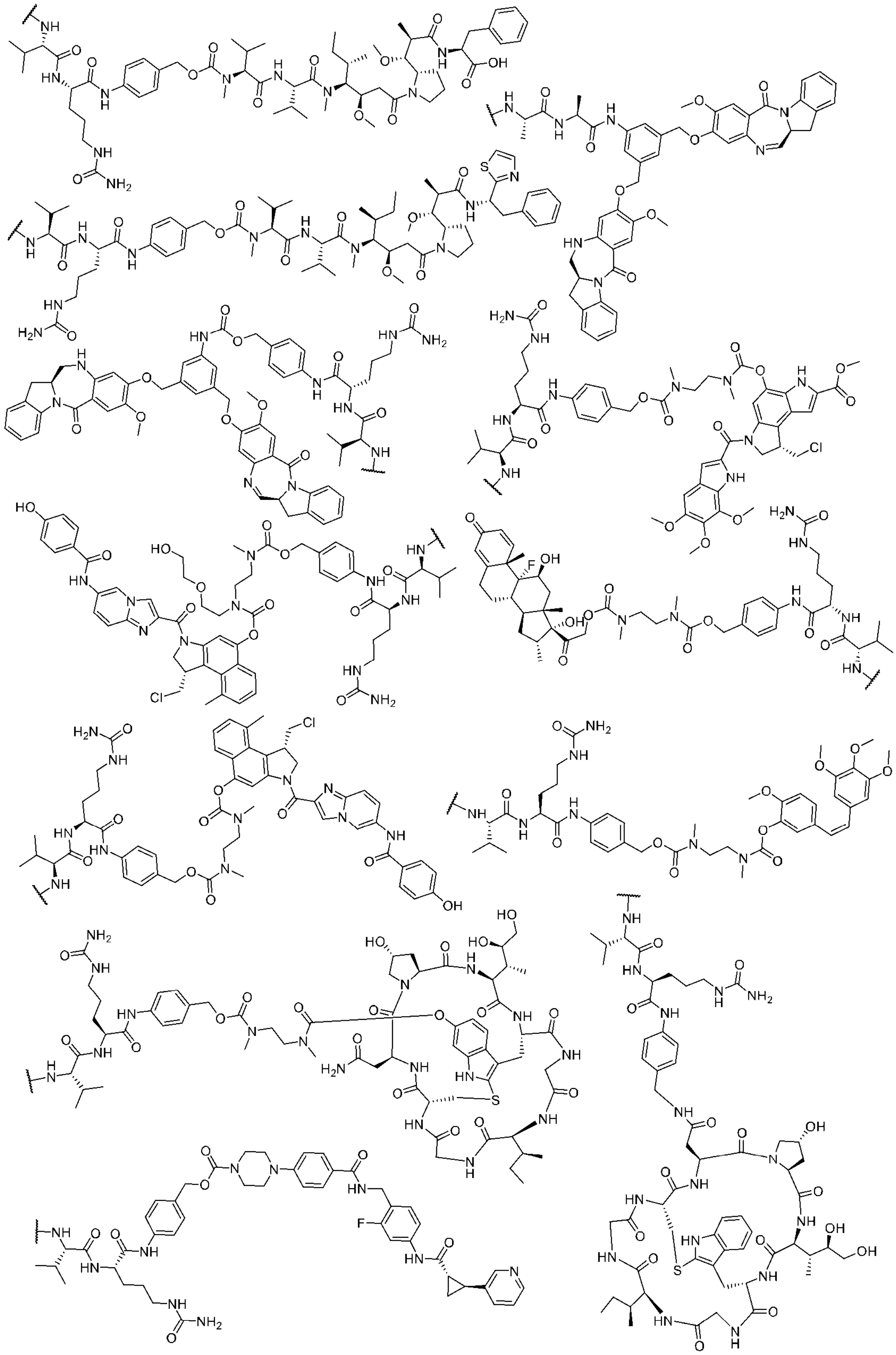

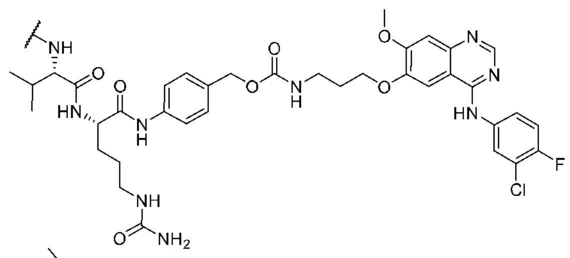

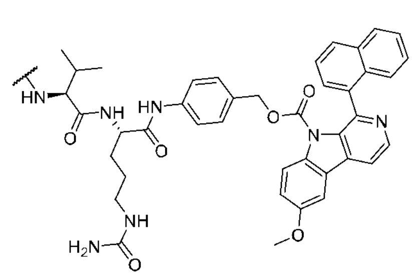

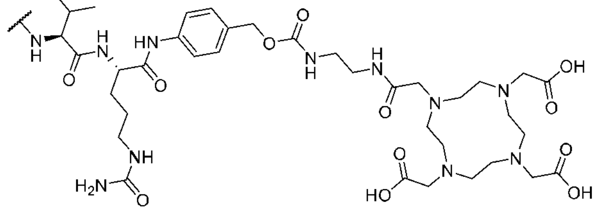

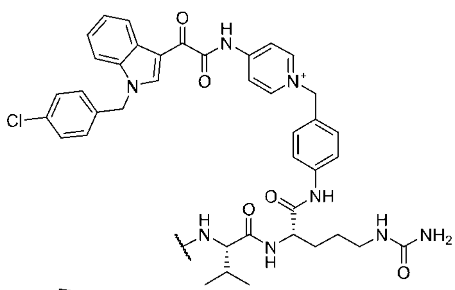

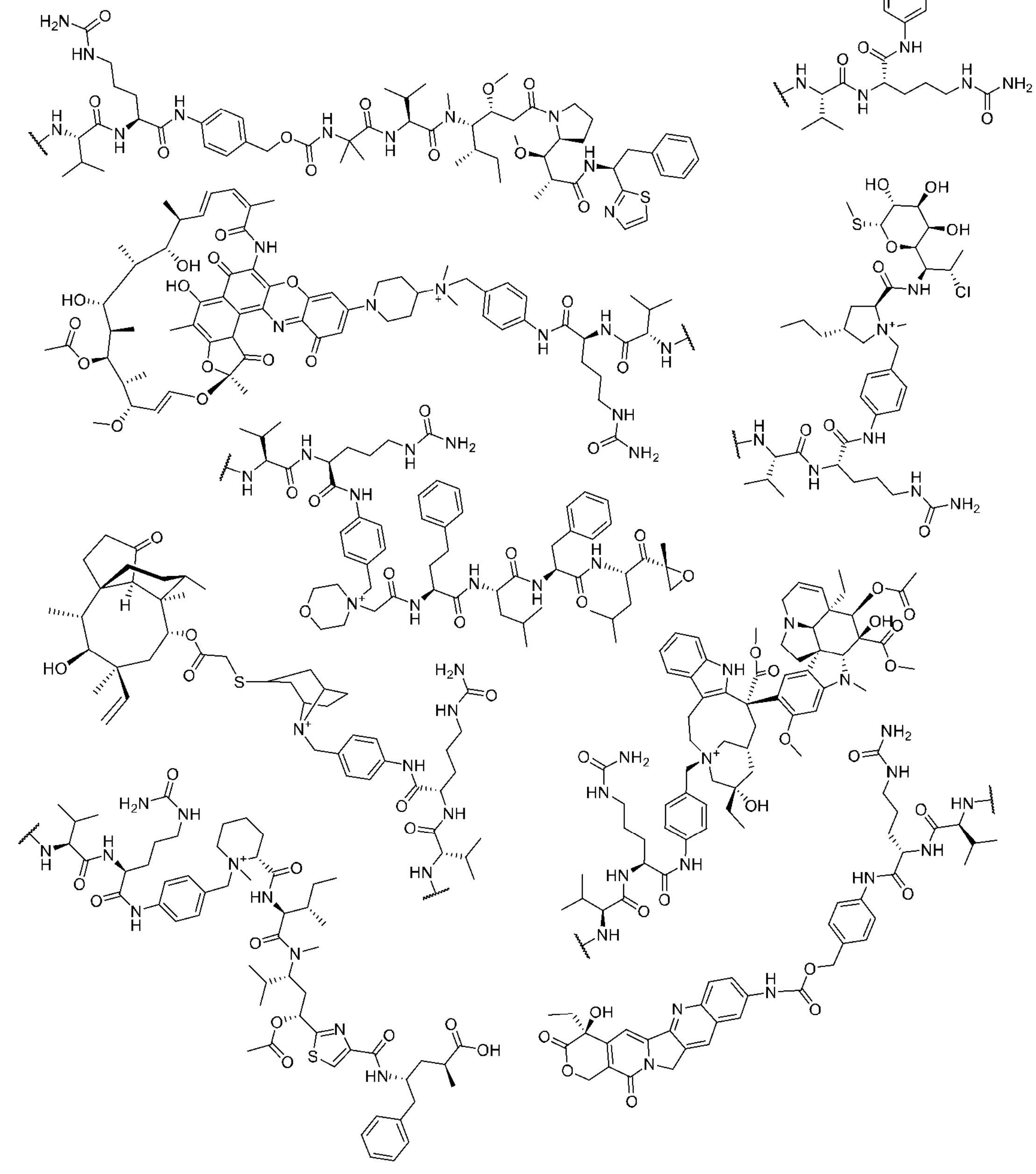

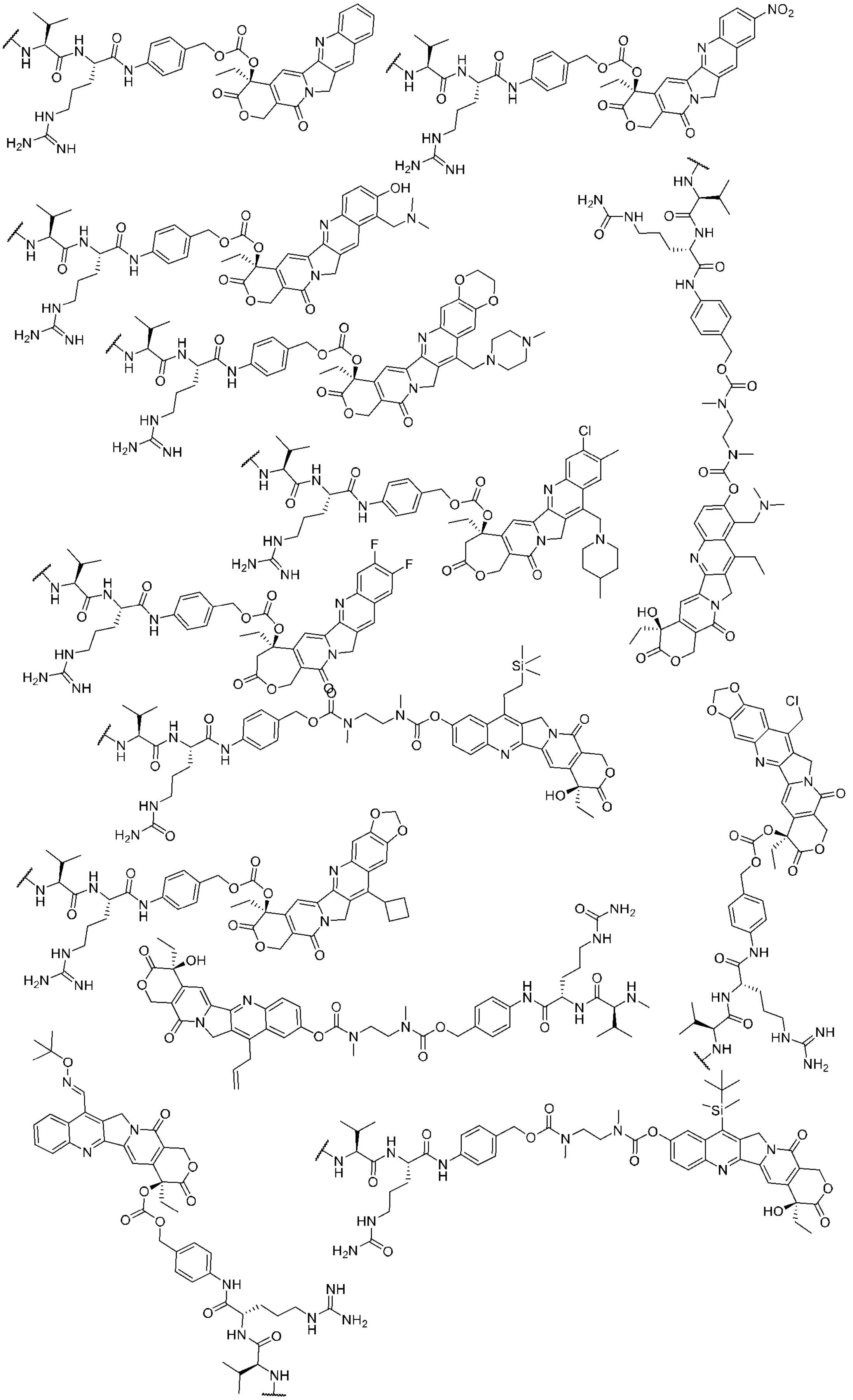

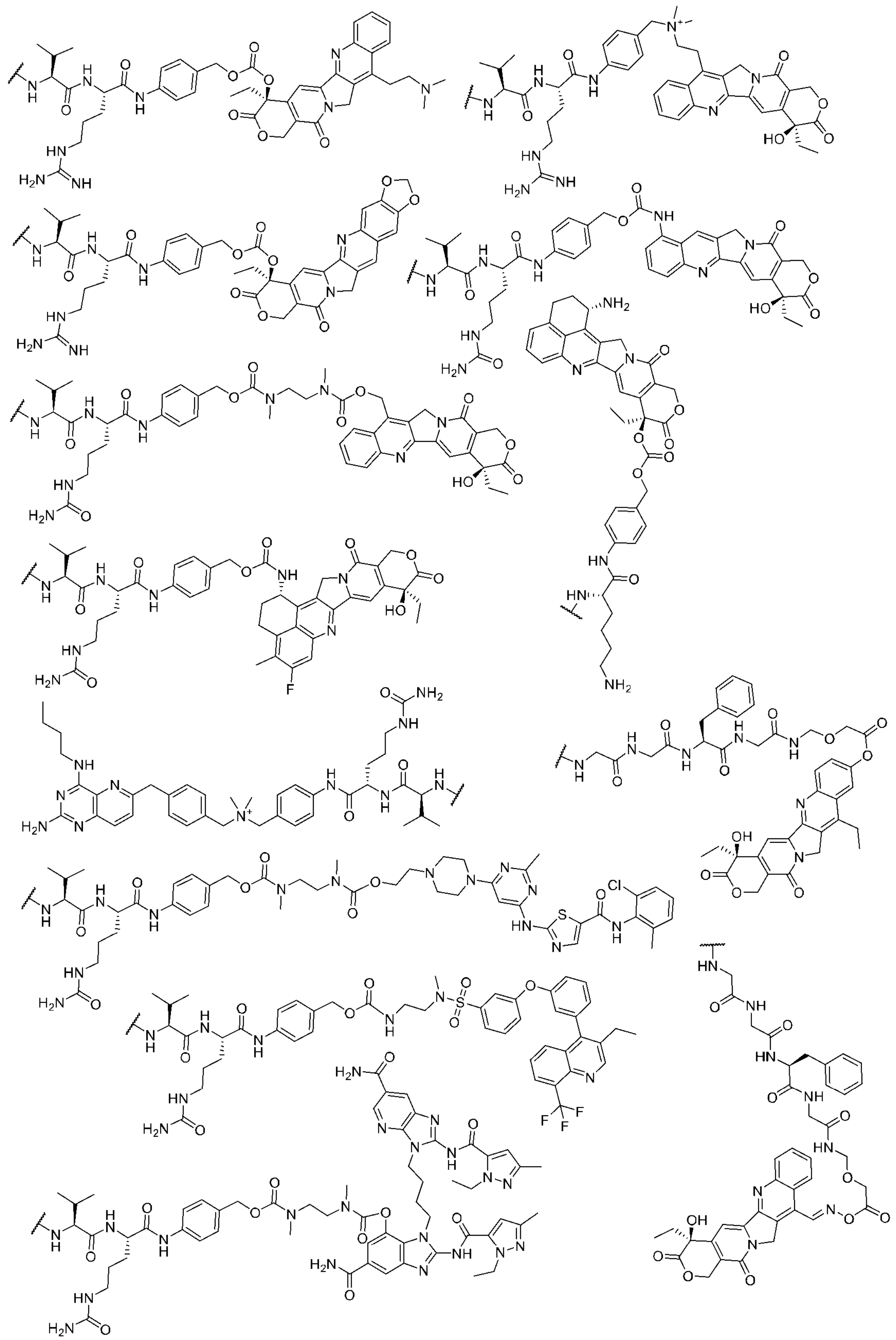

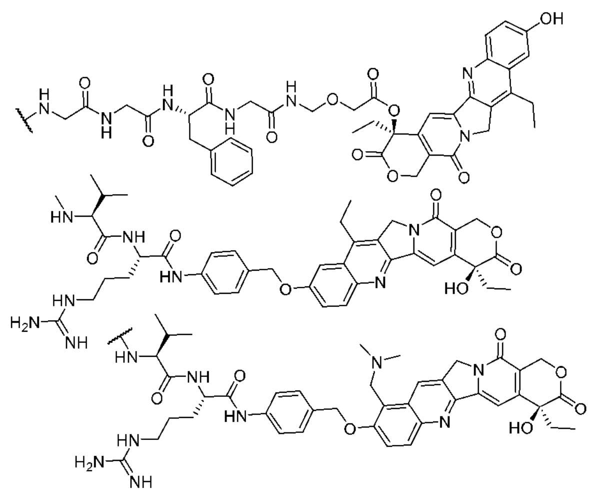

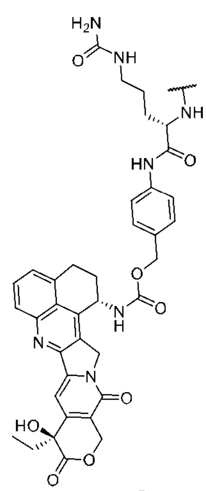

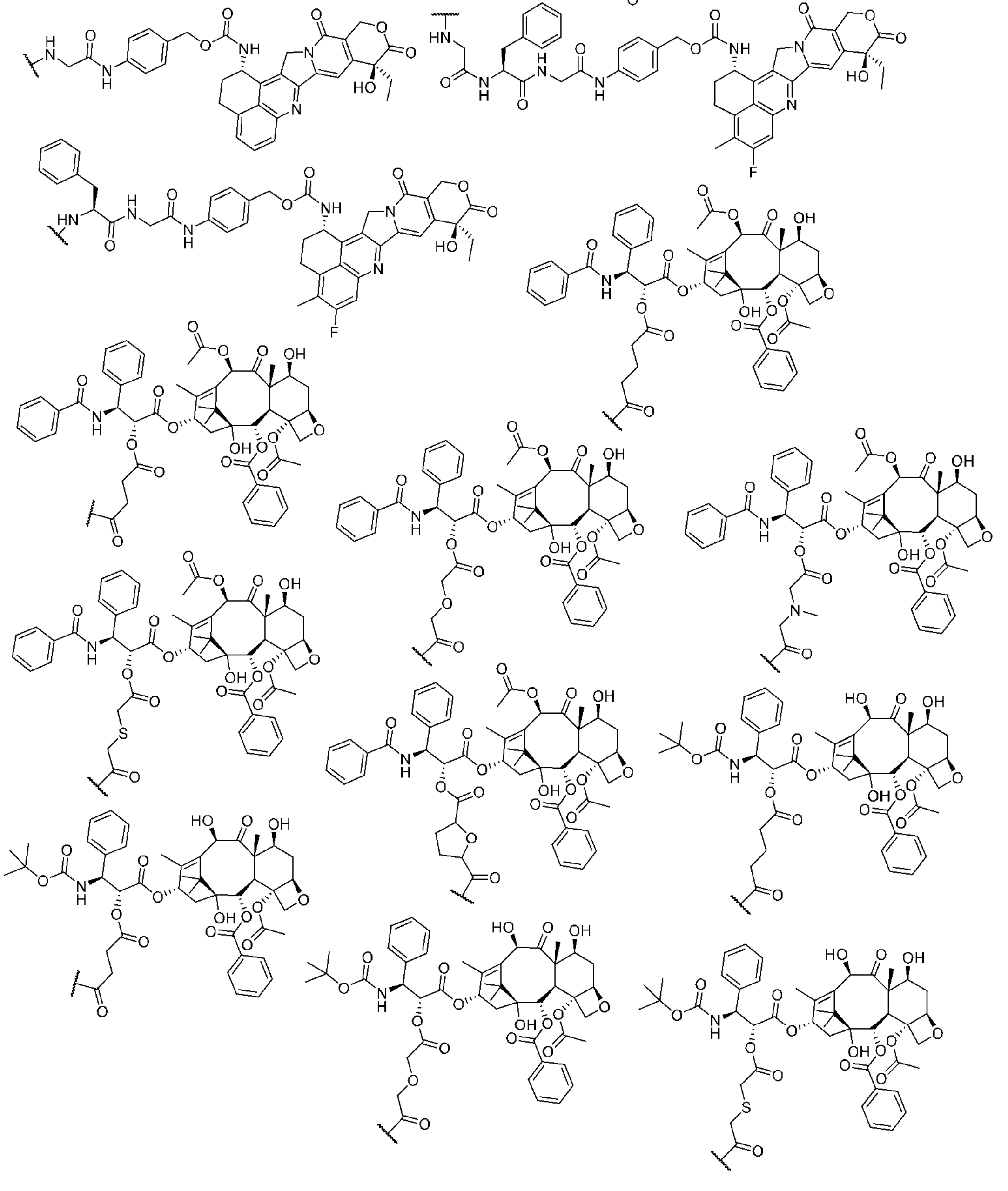

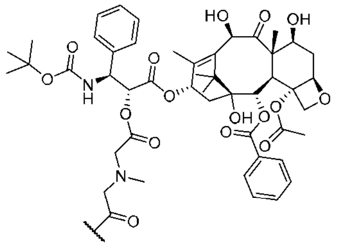
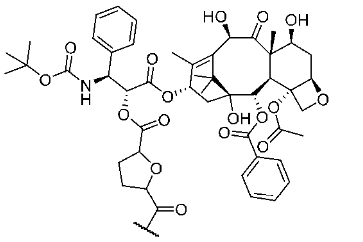
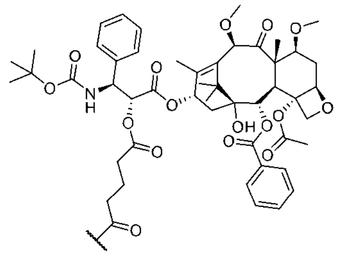
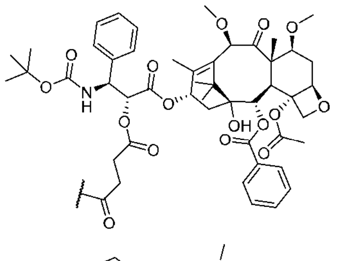
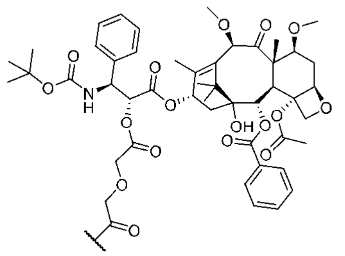
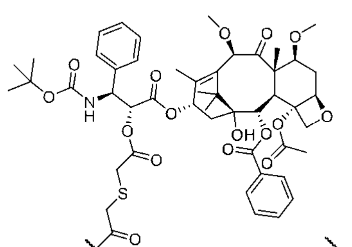
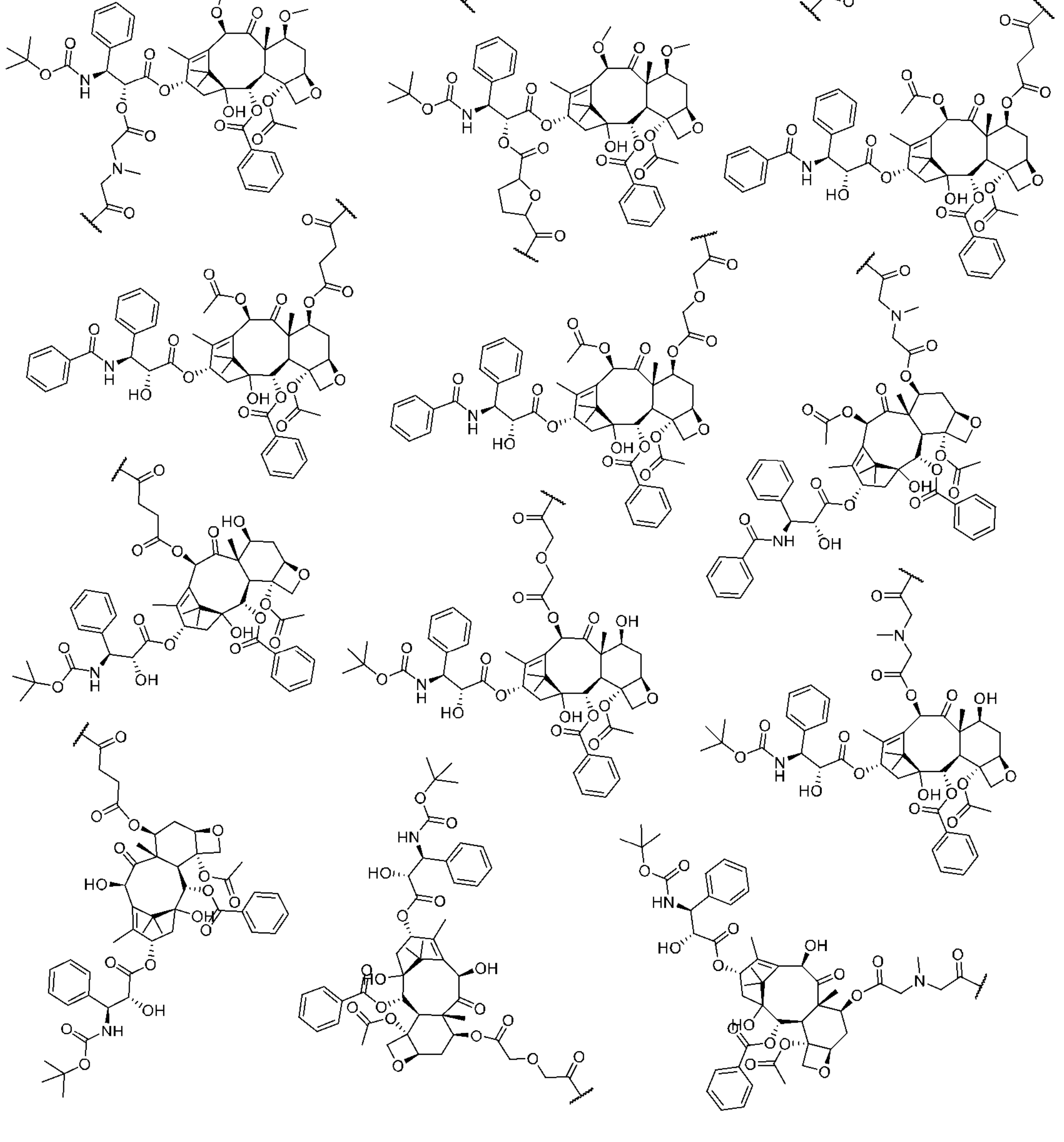

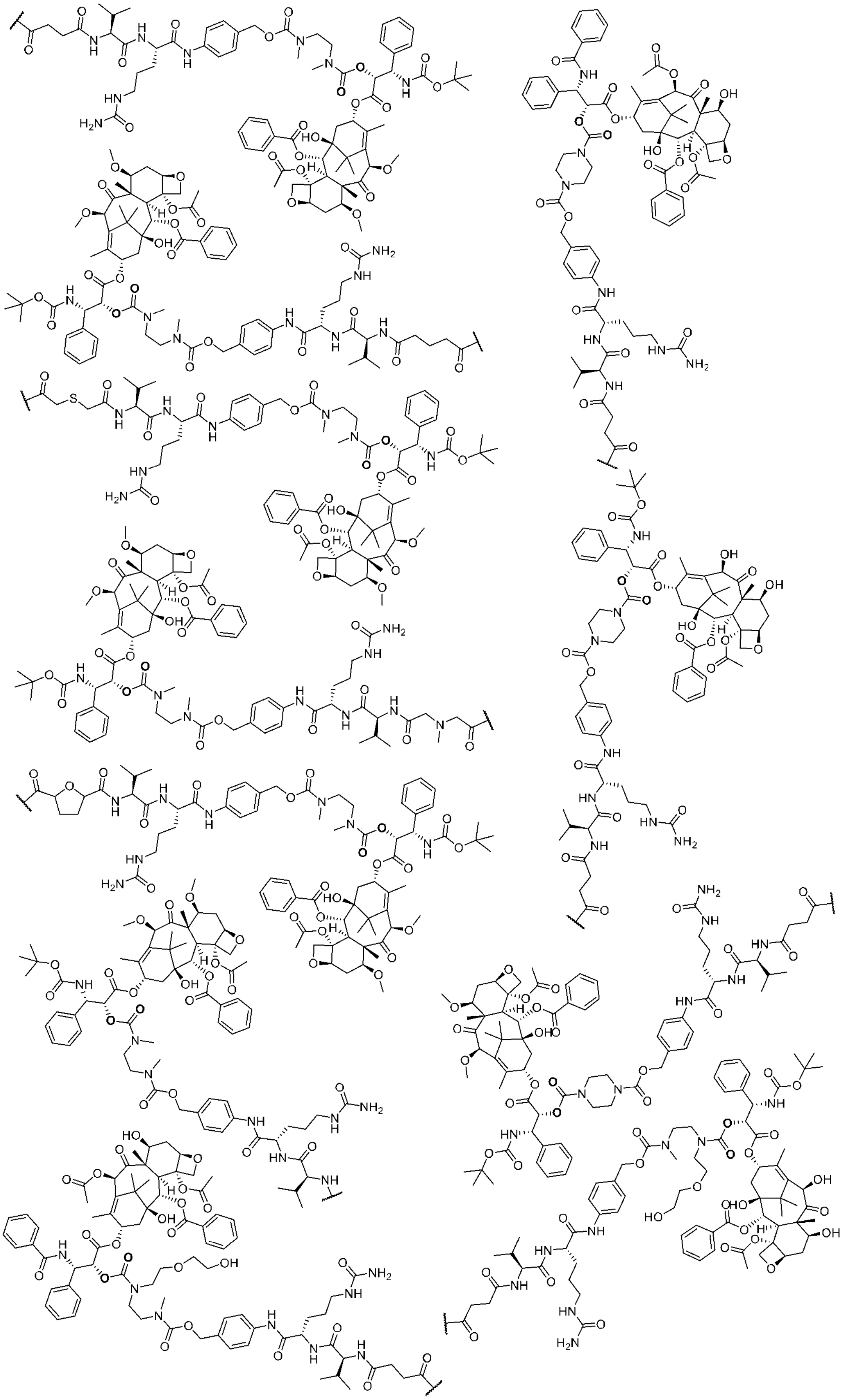

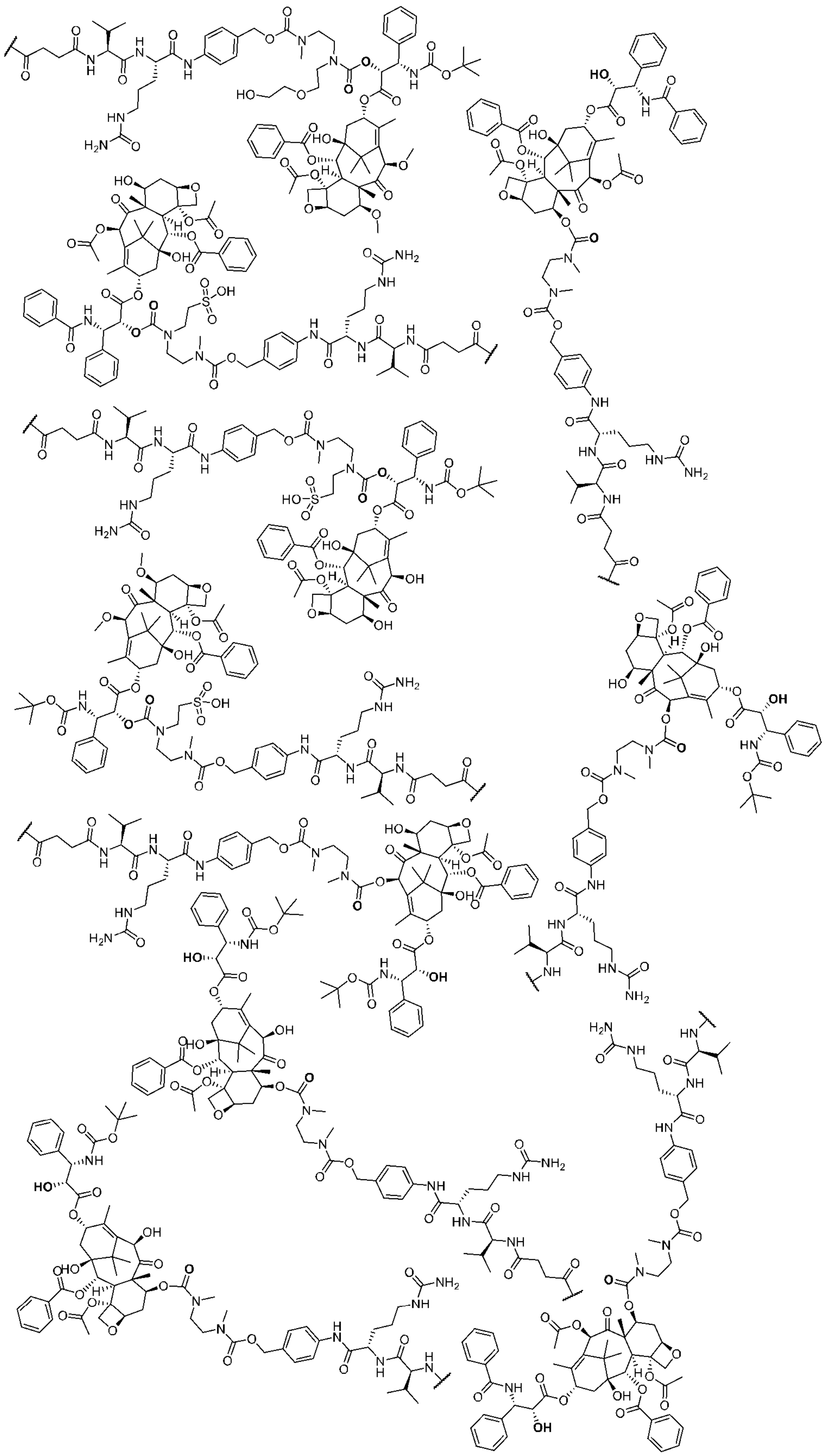

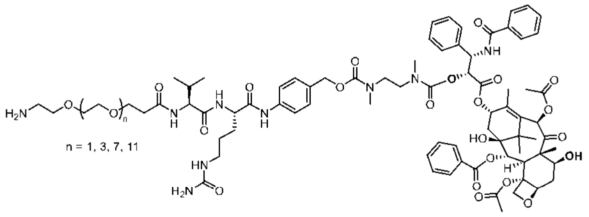

n = 1, 3, 7, 11

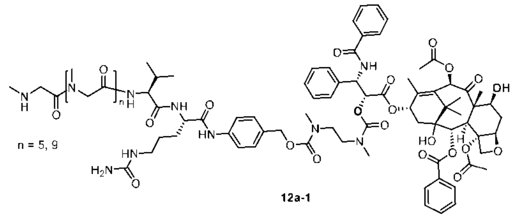

n = 5, 9

12a-1

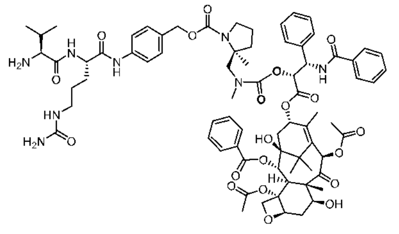

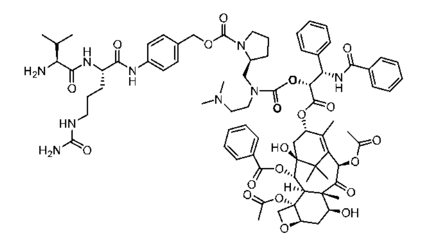

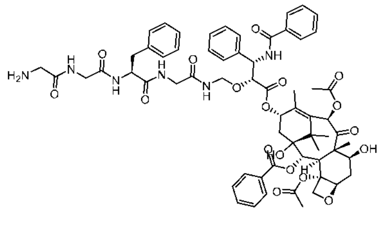

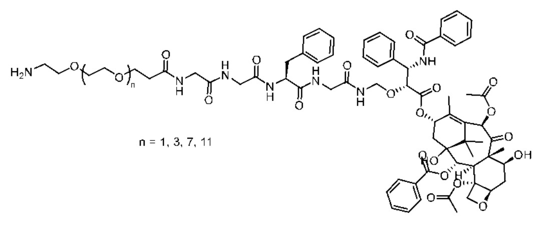

n = 1, 3, 7, 11

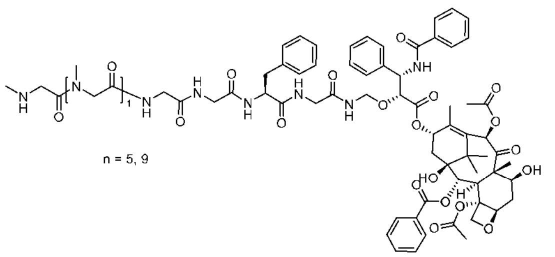

n = 5, 9

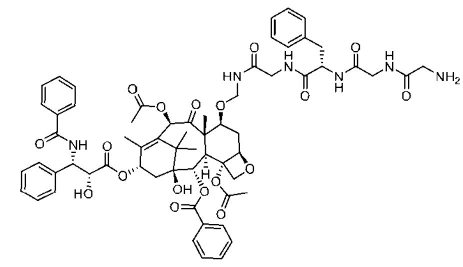

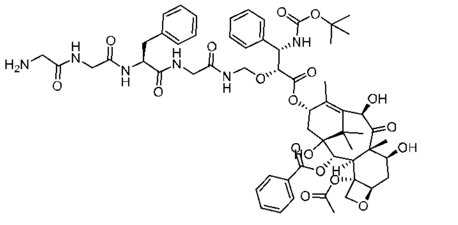

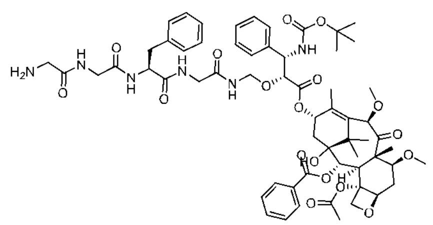

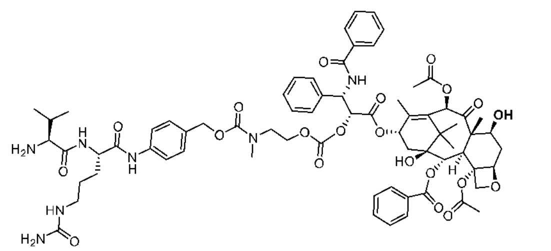

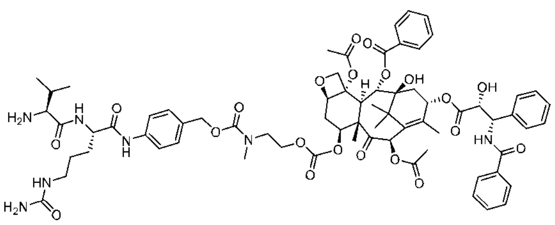

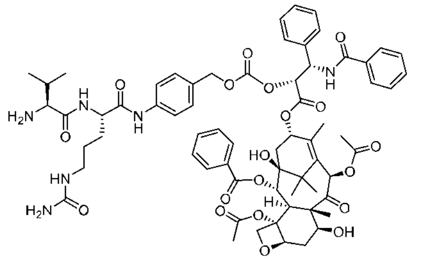

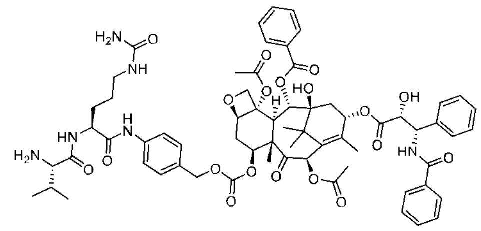

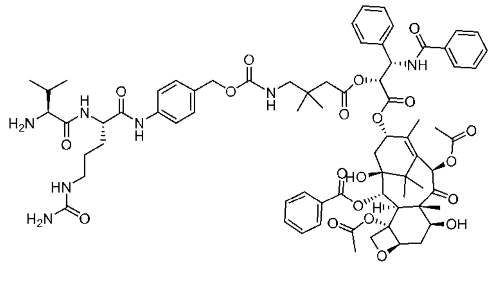

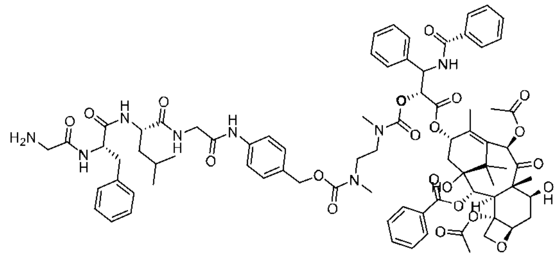

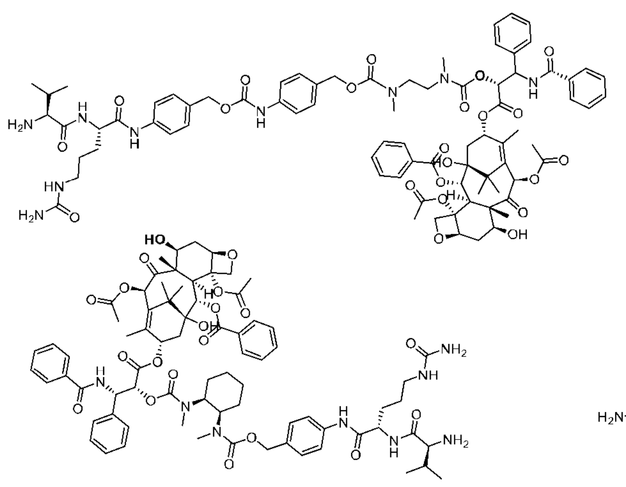

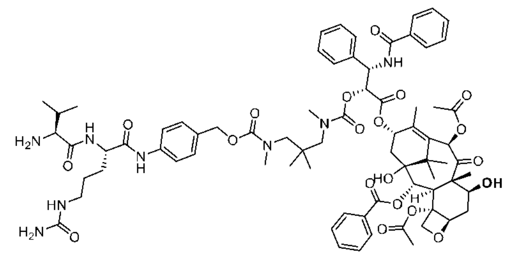

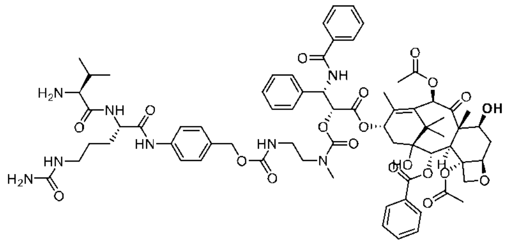

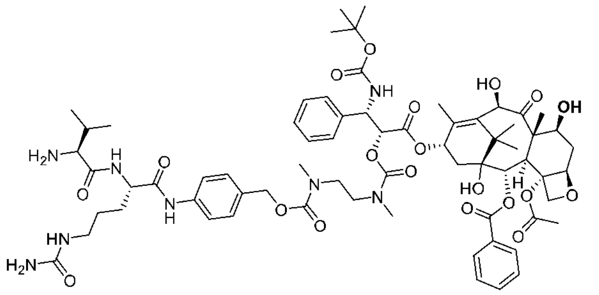

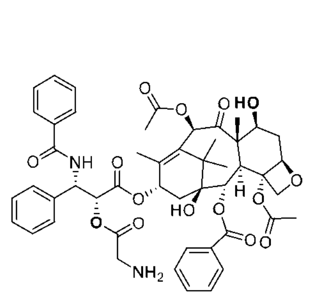

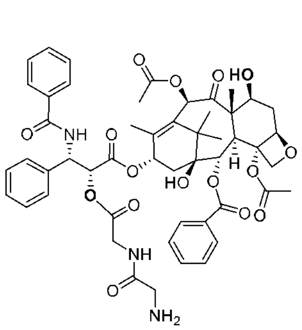

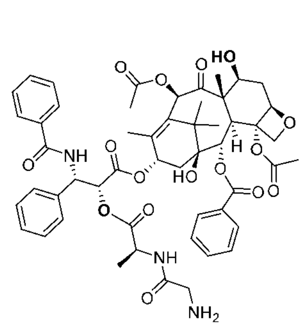

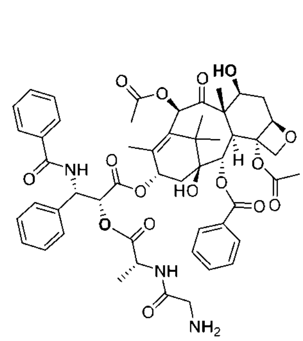

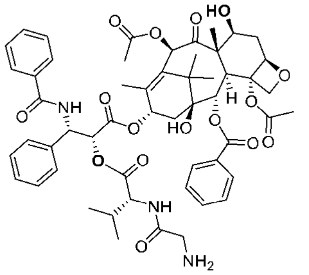
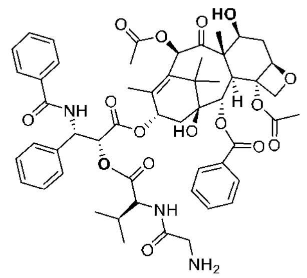
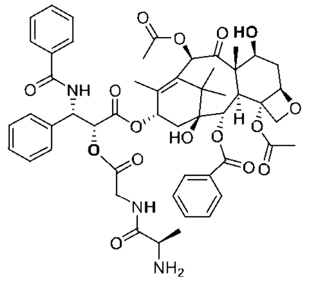

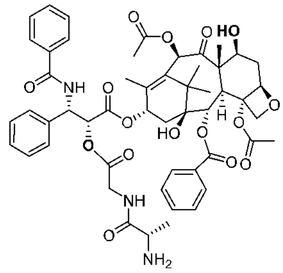
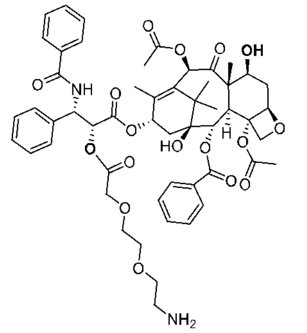
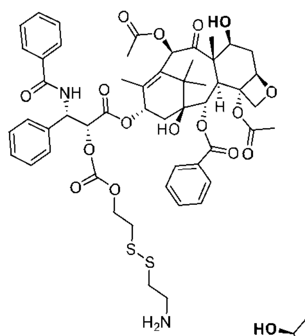

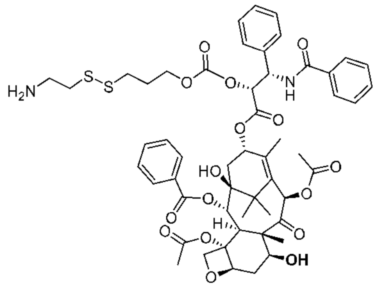
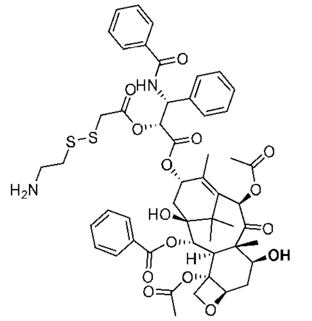
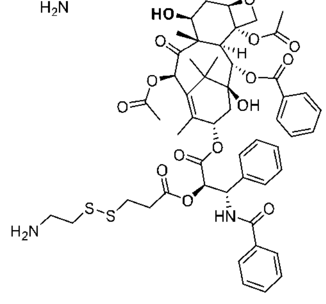

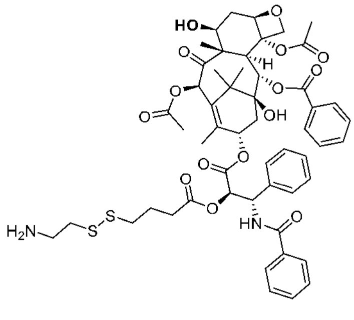
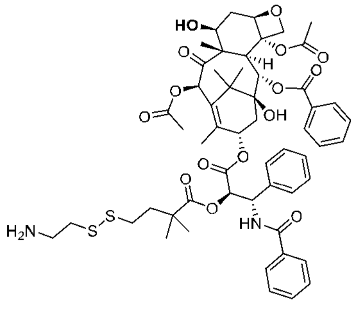
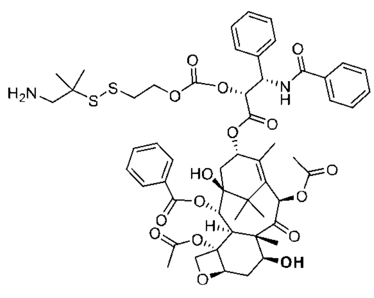

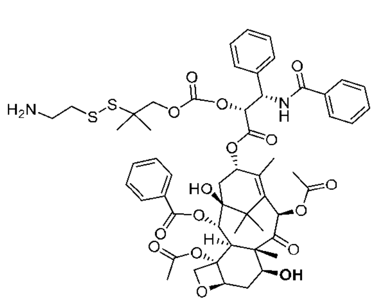
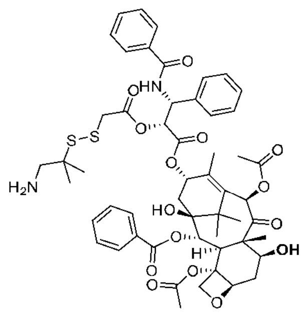
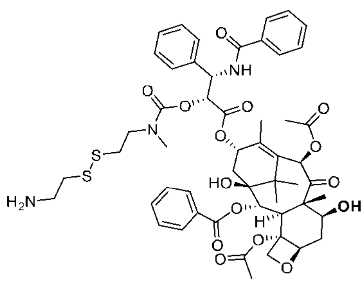

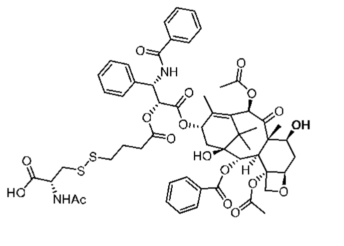

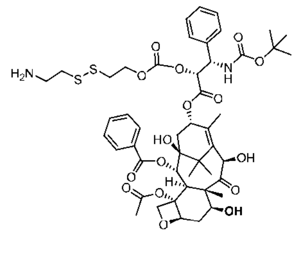

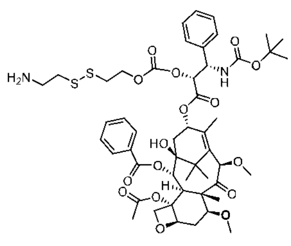

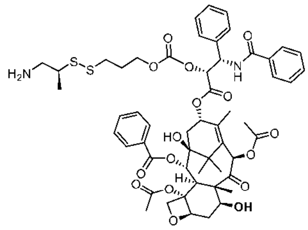

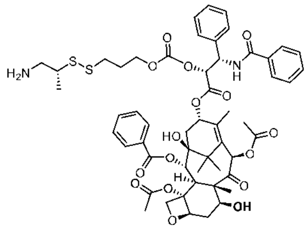

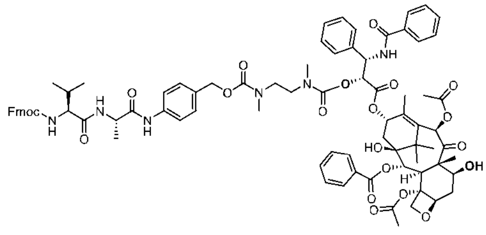

.

In some examples, $-L^2-D$ in the polymer-drug conjugate is any one of the following structures:

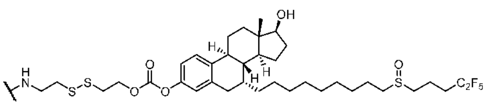

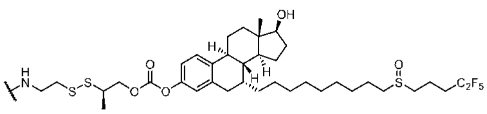

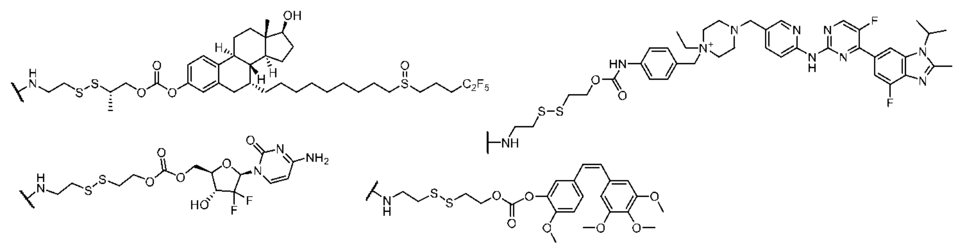

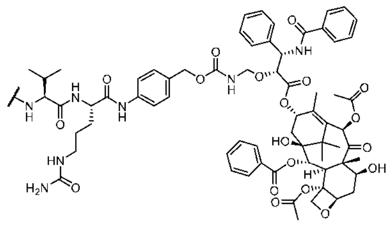

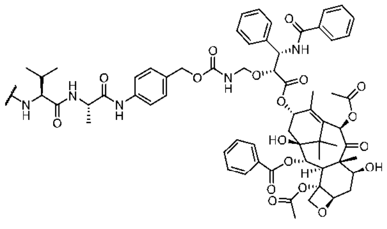

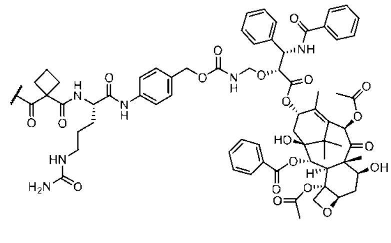

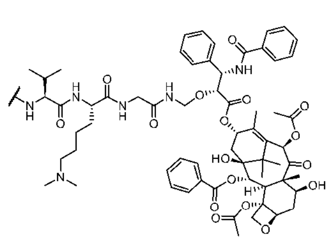

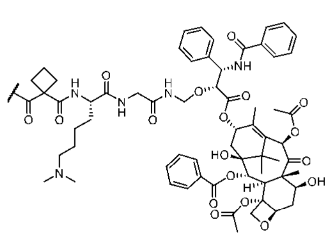

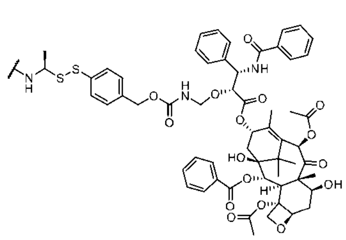

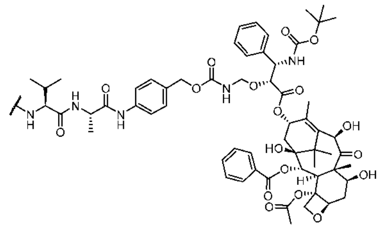
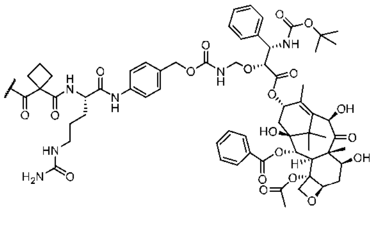
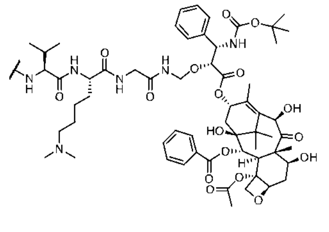

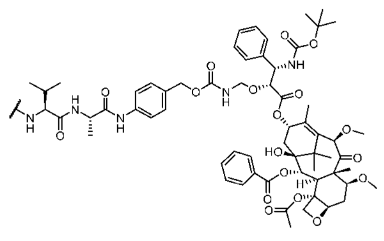
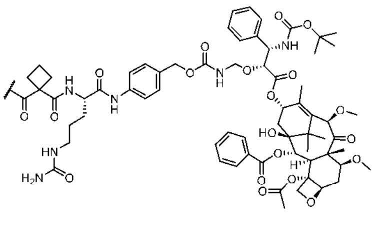
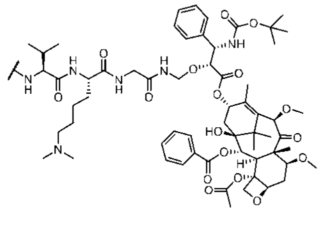

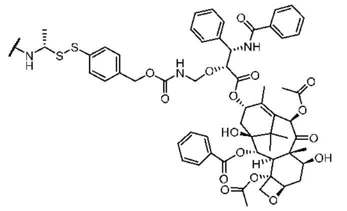
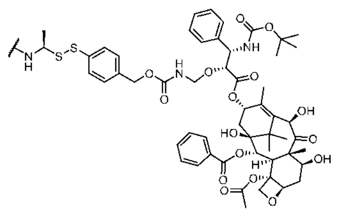
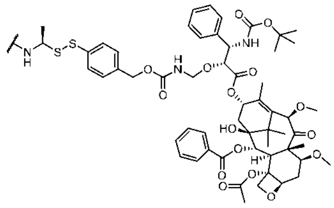

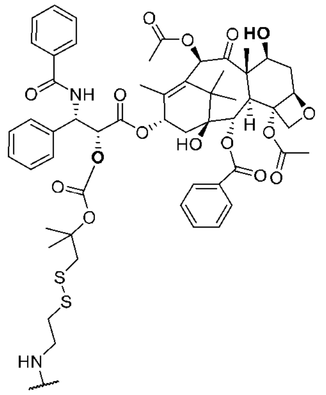
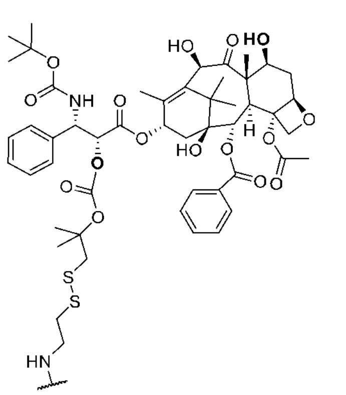
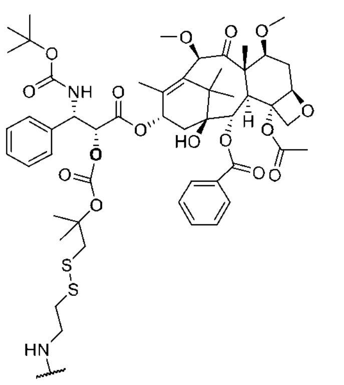

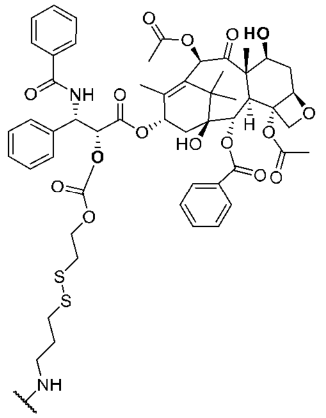
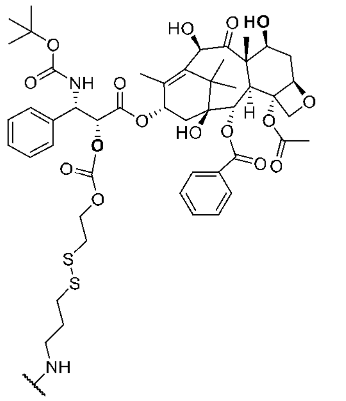
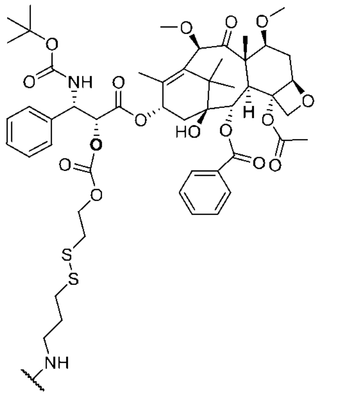

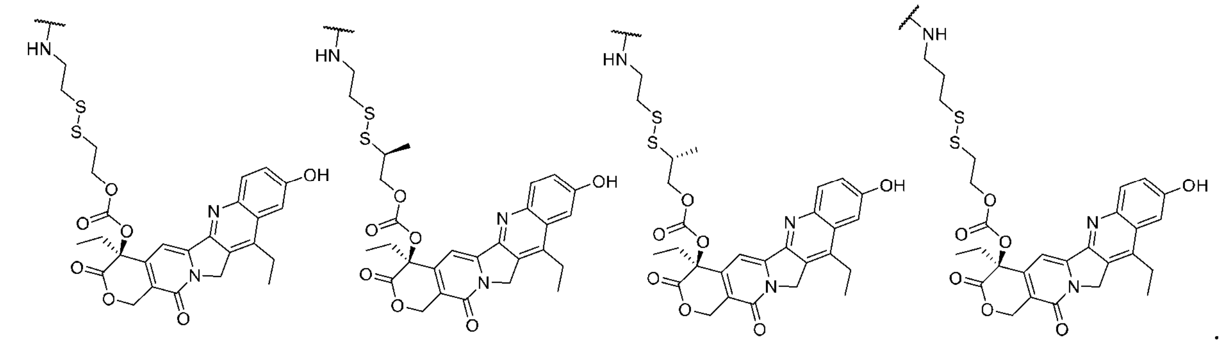

.

The present disclosure provides a compound of formula (I-1) or a pharmaceutically acceptable salt thereof:

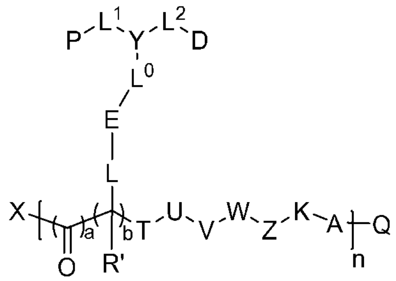

(I-1)

wherein,

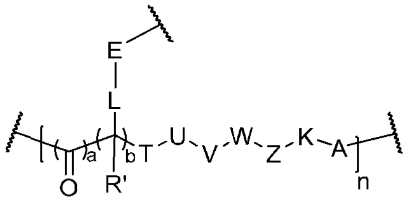

is:

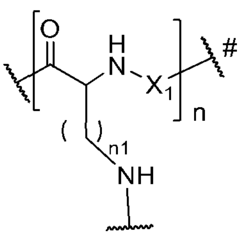

,

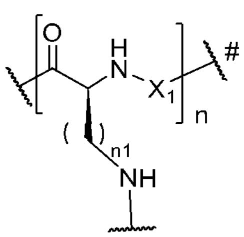

,

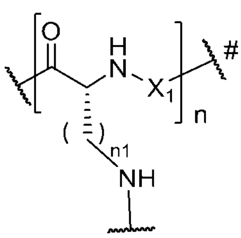

,

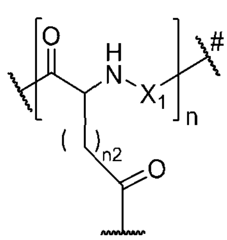

,

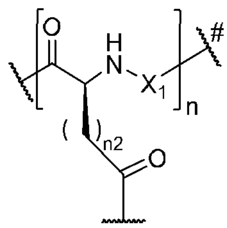

,

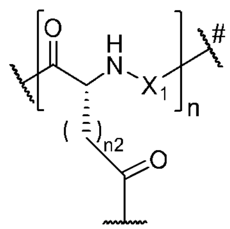

,

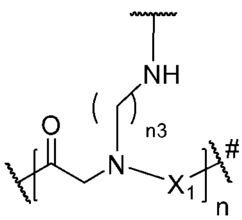

,

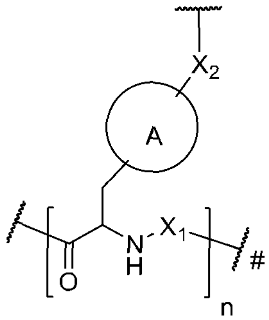

,

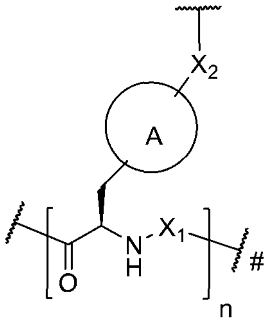, 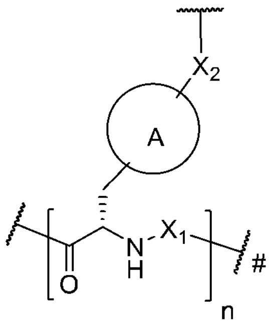, 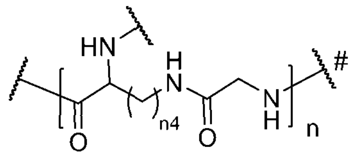,

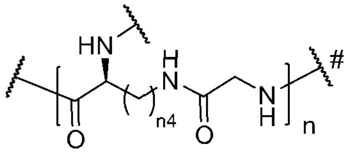, 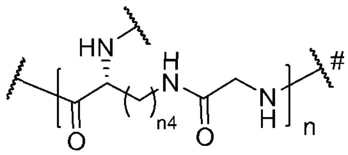, or 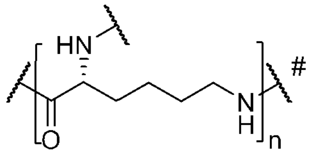,

with the "#" terminal linked to Q;

n is independently any integer of 4-100;
n1, n2, n3, and n4 are each independently 0, 1, 2, 3, 4, or 5;
$X_1$ is a covalent bond or

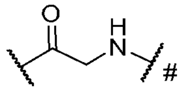;

ring A is $C_6$-$C_{10}$ aryl or 5- to 10-membered heteroaryl;
$X_2$ is a covalent bond, O, S, or NH;
$L^0$ is a covalent bond;
Y is

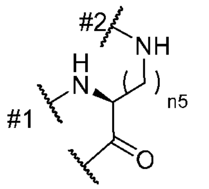 or 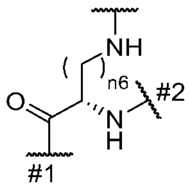,

the "#1" terminal is linked to $L^2$, and the "#2" terminal is linked to $L^1$;
n5 and n6 are each independently 0, 1, 2, 3, 4, or 5;
$L^1$ is a covalent bond;
P is

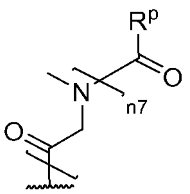 or 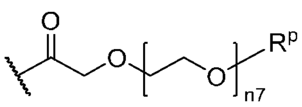;

$R^p$ is independently $C_1$-$C_6$ alkyl or $C_1$-$C_6$ alkyl substituted with one or more $R^{p-1}$;
$R^{p-1}$ is independently halogen, hydroxy, $C_1$-$C_6$ alkoxy, or $NR^{p1}R^{p2}$;
$R^{p1}$ and $R^{p2}$ are each independently -H or $C_1$-$C_6$ alkyl;
n7 is independently any integer of 4-100;

$L^2$ is $^{\#3}$-$L^{2a}$-$L^{2b}$-$^{\#4}$, the "#3" terminal is linked to Y, and the "#4" terminal is linked to D;
$L^{2a}$ is a covalent bond,

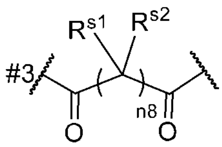

,

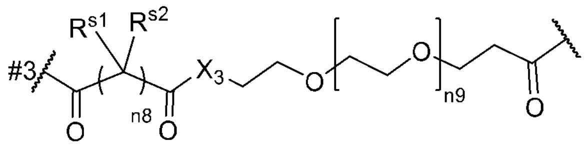

,

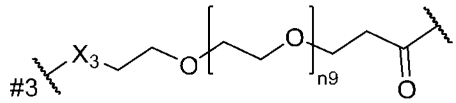

,

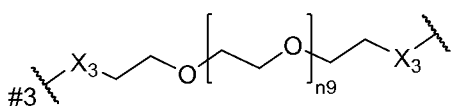

,

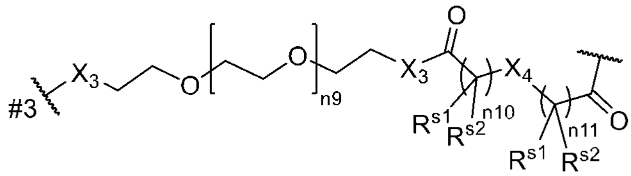

,

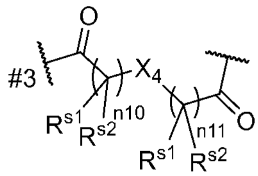

,

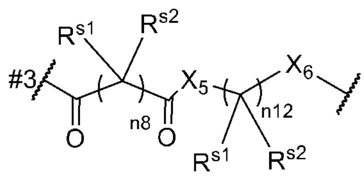

,

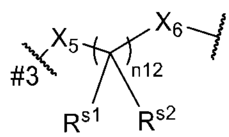

,

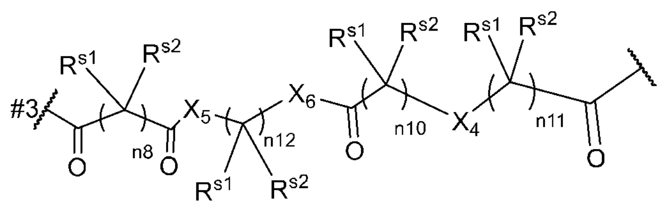

,

or

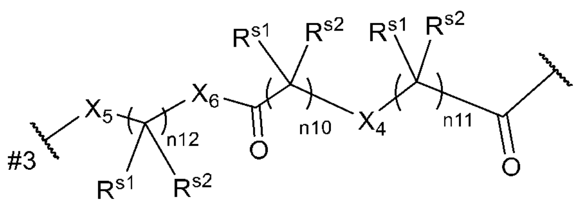

;

n8, n9, n10, n11, n12, n13, and n14 are each independently any integer of 1-20;
$R^{s1}$ and $R^{s2}$ are each independently -H or $C_1$-$C_6$ alkyl;
or, $R^{s1}$ and $R^{s2}$, together with the carbon atom to which they are linked, form a $C_3$-$C_6$ cycloalkyl or a 3- to 6-membered heterocycloalkyl, wherein in the 3- to 6-membered heterocycloalkyl, the heteroatom is selected from one or more of N, O, and S, and the number of heteroatom(s) is 1, 2, or 3;
$X_3$, $X_4$, $X_5$, $X_6$, $X_7$, and $X_8$ are each independently $NR^{s3}$ or O, and $R^{s3}$is H or $C_1$-$C_6$ alkyl;
$L^{2b}$ is a covalent bond,

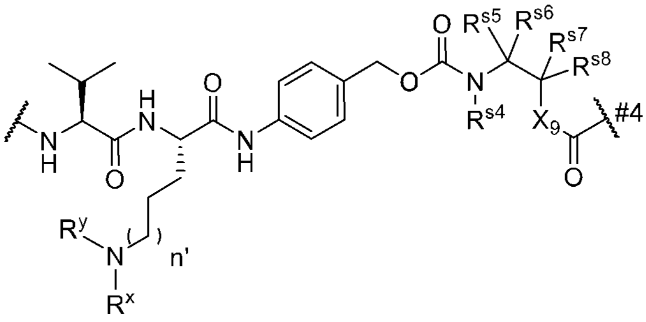

,

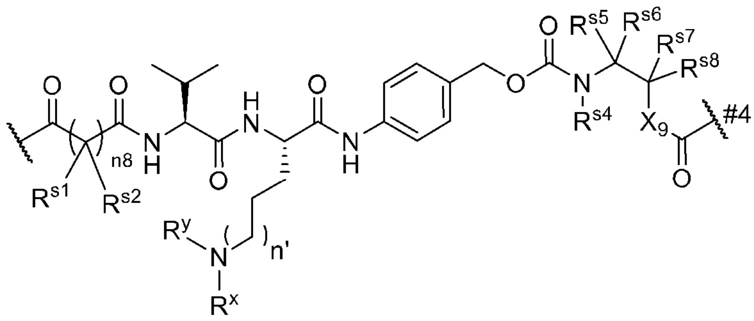

,

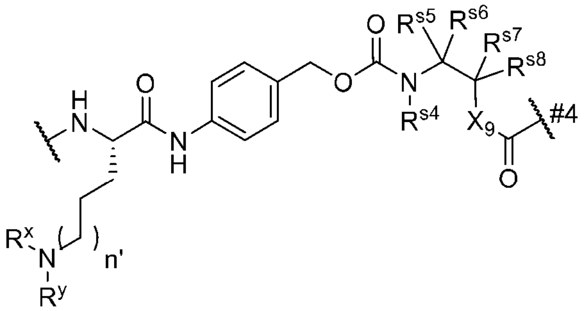

,

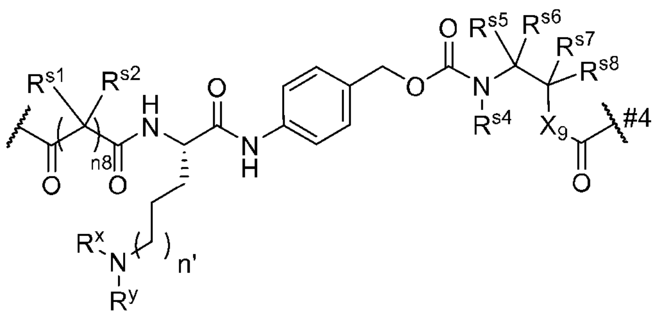

,

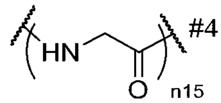

,

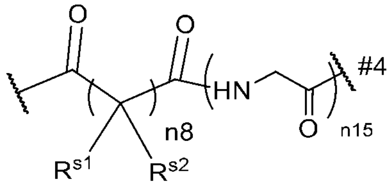

,

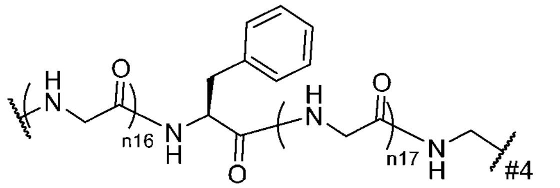

,

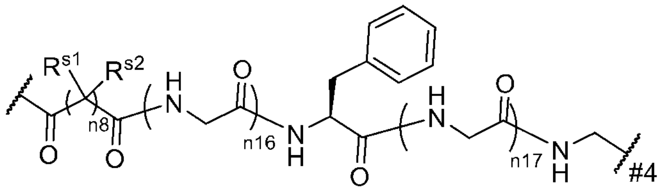, 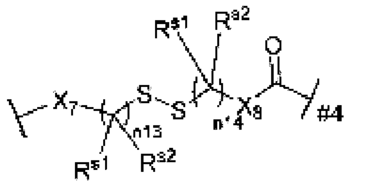,

or

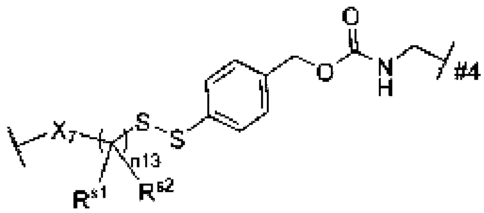

;

$R^x$ is independently -H or $C_1$-$C_3$ alkyl;
$R^y$ is independently H, $C_1$-$C_3$ alkyl, or

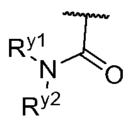

;

$R^{y1}$ and $R^{y2}$ are each independently -H or $C_1$-$C_3$ alkyl;
n' is independently 1 or 2;
$X_9$ is O or $NR^{s9}$;
$R^{s4}$, $R^{s5}$, $R^{s6}$, $R^{s7}$, $R^{s8}$, and $R^{s9}$ are each independently -H or $C_1$-$C_6$ alkyl;
or, "$R^{s4}$ and $R^{s5}$", "$R^{s6}$ and $R^{s7}$", "$R^{s8}$ and $R^{s9}$", or "$R^{s4}$ and $R^{s9}$", together with the atom to which they are linked, form a 5- to 6-membered heterocycloalkyl, wherein, in the 5- to 6-membered heterocycloalkyl, the heteroatom is N, O, or N and O, and the number of heteroatom(s) is 1 or 2;
n15, n16, and n17 are each independently 1, 2, 3, or 4;
D is a pharmaceutically active agent residue;
X is OH, $NH_2$, -$L_2$-D, -D, or

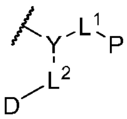

;

Q is

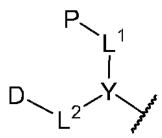

or -P.

In some examples,

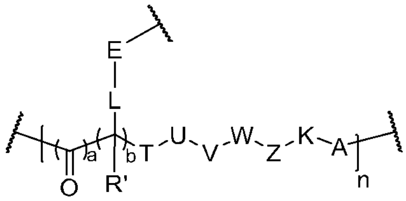

is

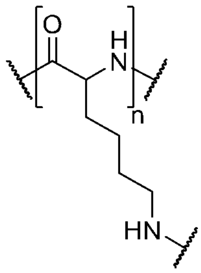 ,

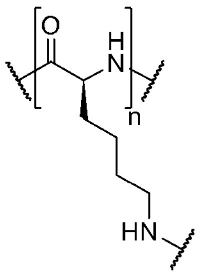 , 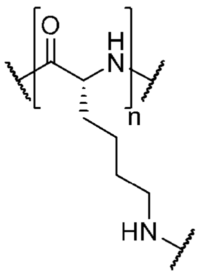 , 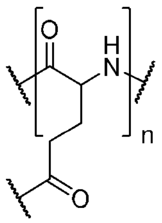 , 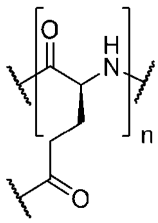 , 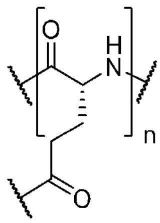 ,

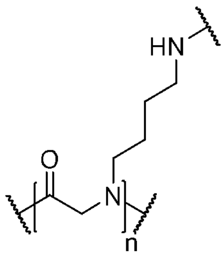 , 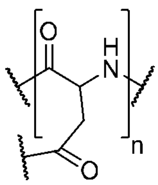 , 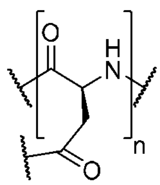 , 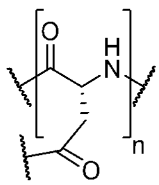 , 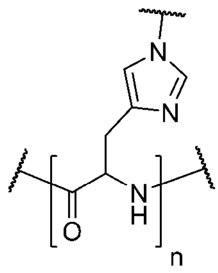 ,

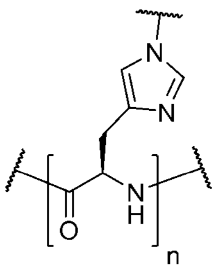 , 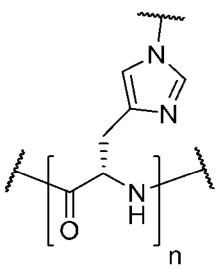 , 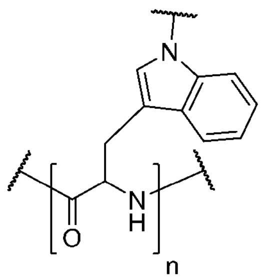 , 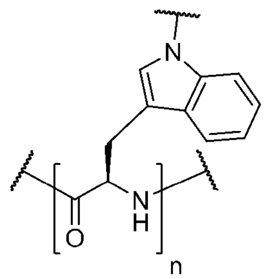 ,

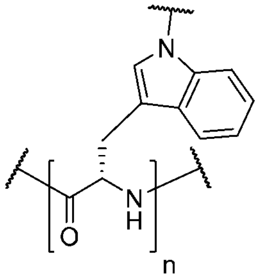 , 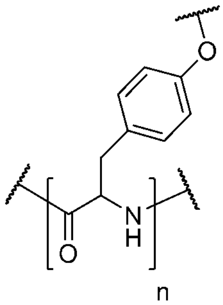 , 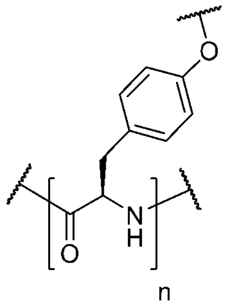 , 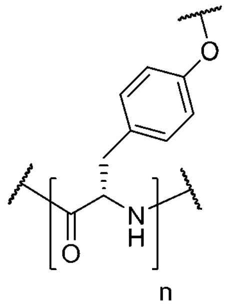 ,

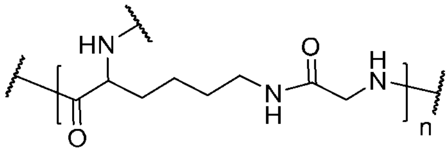 , 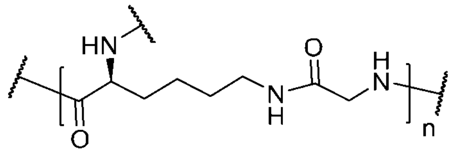 ,

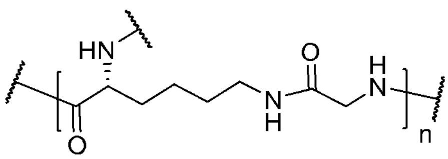 , 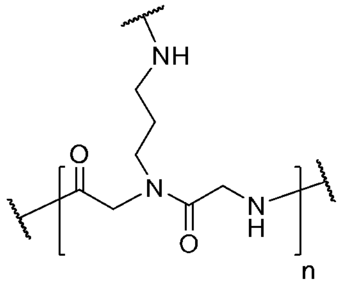 , 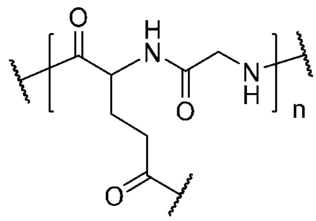 ,

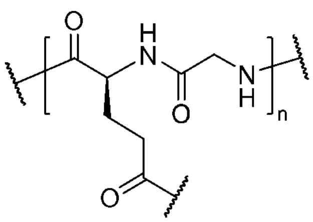 , 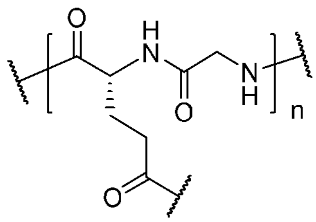 , or 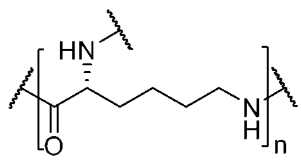 .

In some examples, n is independently any integer of 20-60, for example, 20-30 or 50-60, such as 28, 29, 30, 31, 59, or 60.

In some examples,

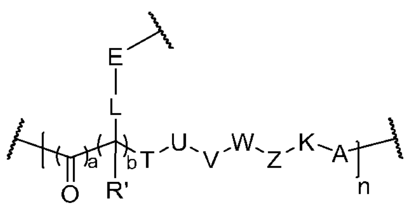

is

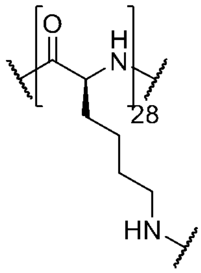,

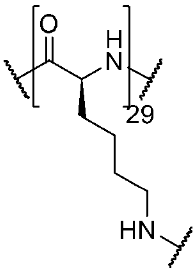, 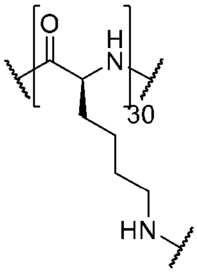, 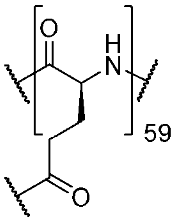, 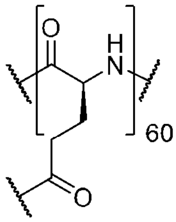, 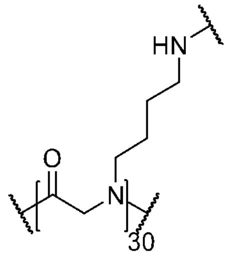,

or

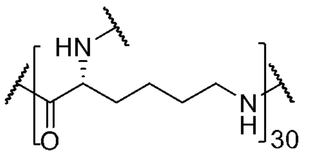.

In some examples, Y is

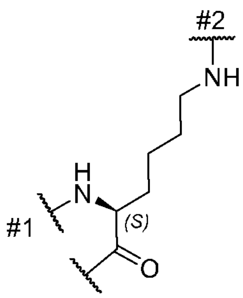 or 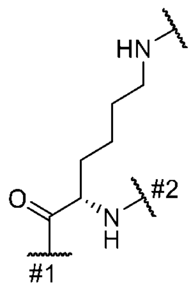.

In some examples, n7 is each independently any integer of 20-50, e.g., 22, 28, 44, or 49.

In some examples, $R^p$ is independently methyl, methoxy, $-CH_2NH_2$, or $-CH_2NHCH_3$.

In some examples, P is

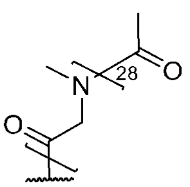, 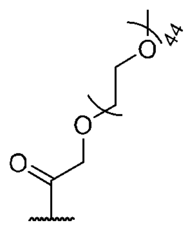, 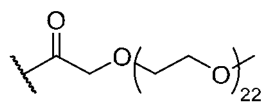,

or

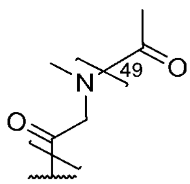

.

In some examples, n8, n9, n10, n11, n12, n13, and n14 are each independently any integer of 1-15, e.g., 1, 2, 3, 7, or 11.

In some examples, $R^{s1}$ and $R^{s2}$ are each independently -H, or $R^{s1}$ and $R^{s2}$, together with the carbon atom to which they are linked, form a $C_3$-$C_6$ cycloalkyl, e.g., cyclobutyl.

In some examples, Y is

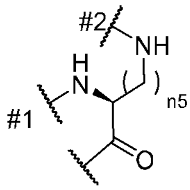

,

$L^{2a}$ is a covalent bond,

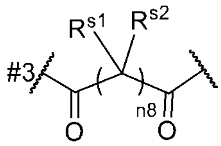

,

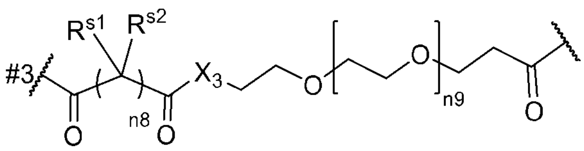

,

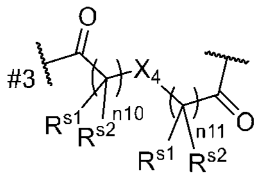

,

or

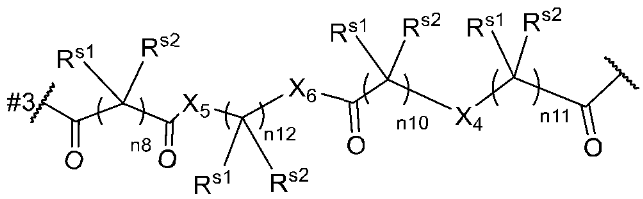

,

and $L^{2b}$ is

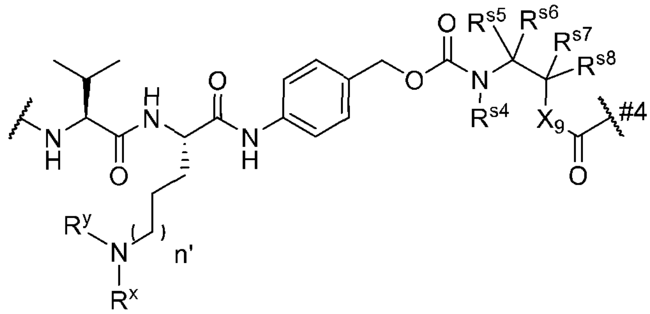

,

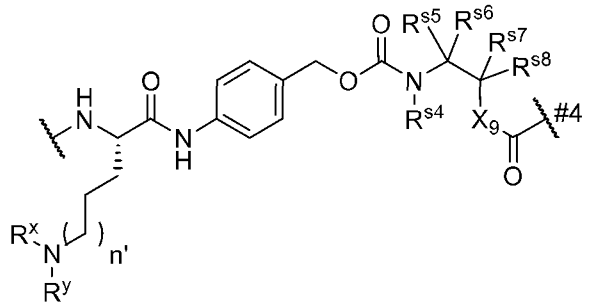

,

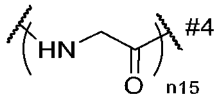

,

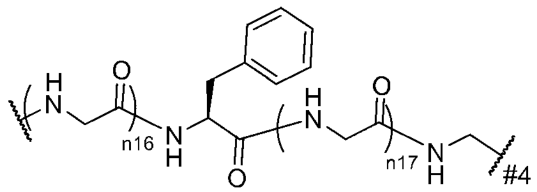

,

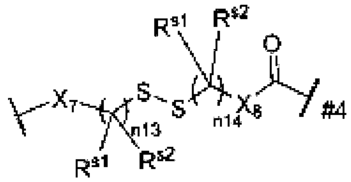

,

or

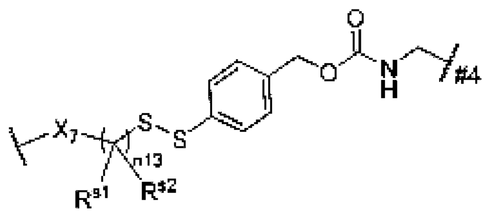

In some examples, Y is

.

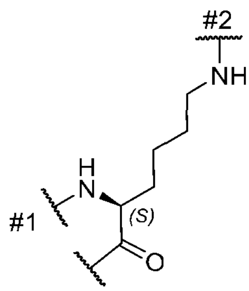

,

$L^{2a}$ is a covalent bond,

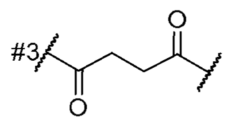

,

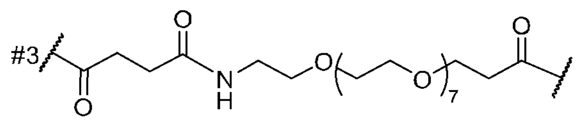

,

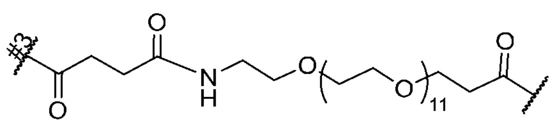

,

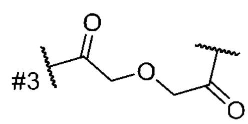

,

or

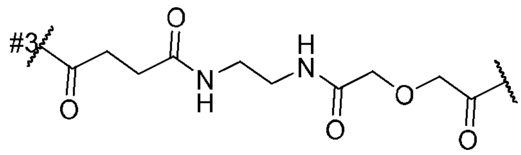

,

and $L^{2b}$ is a covalent bond,

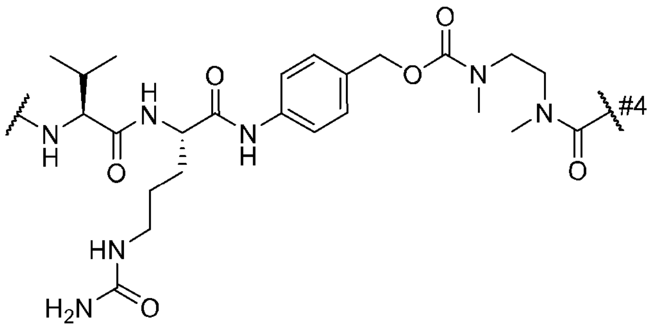

,

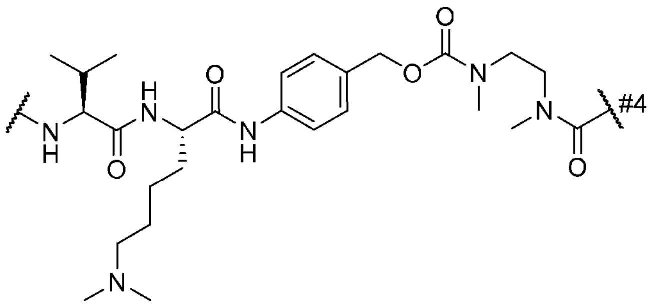

,

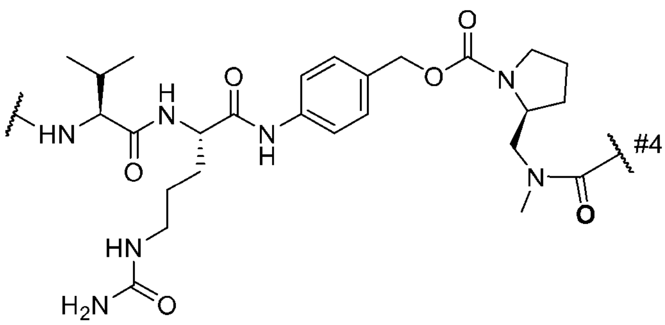

,

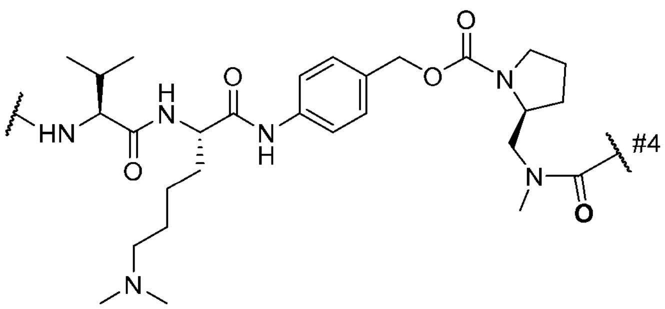

,

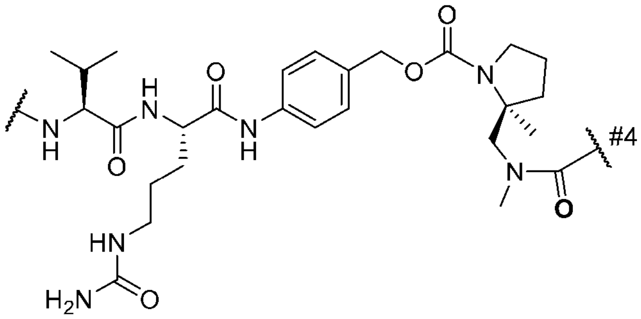

,

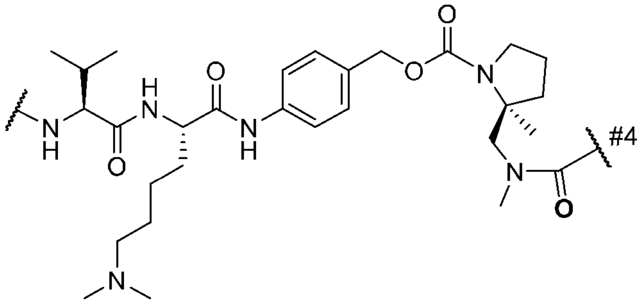

,

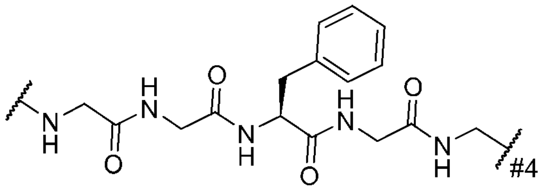

,

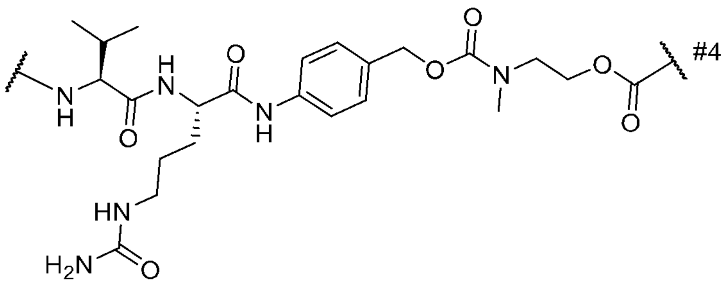

,

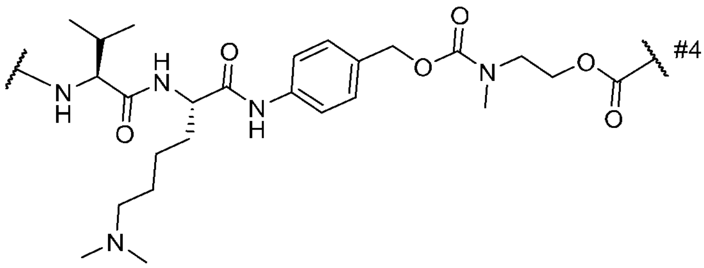

,

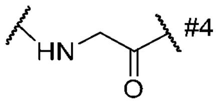

,

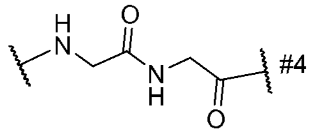,
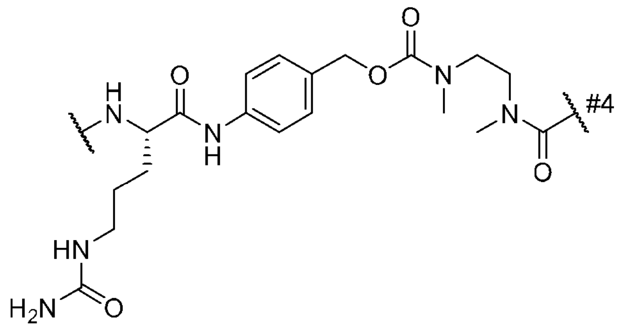,
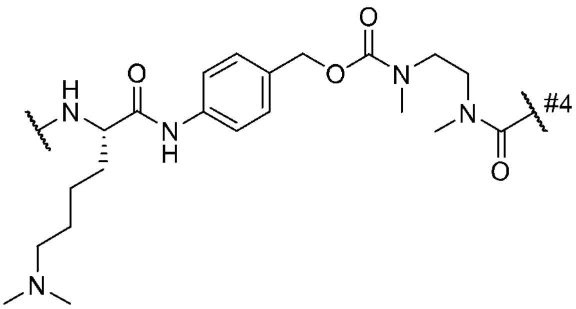,
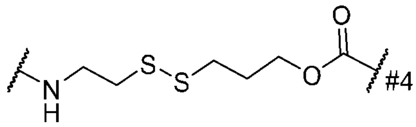,

or

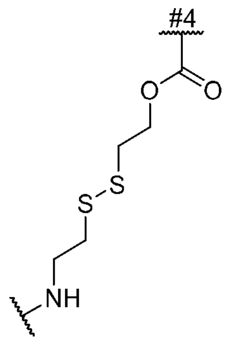.

In some examples, Y is

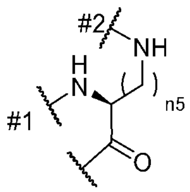,

$L^{2a}$ is a covalent bond or

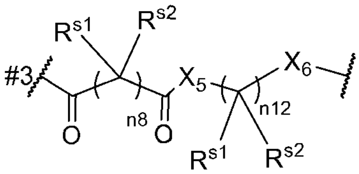,

and $L^{2b}$ is a covalent bond,

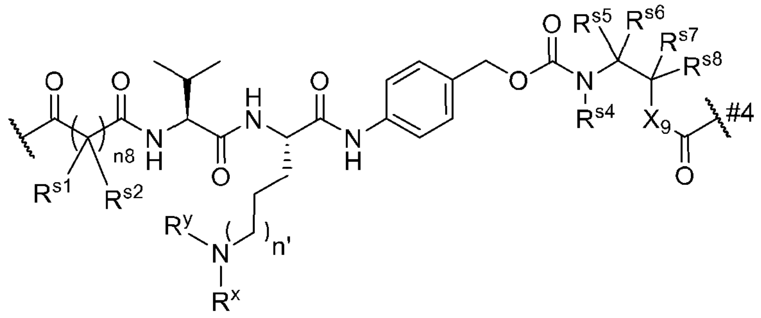

,

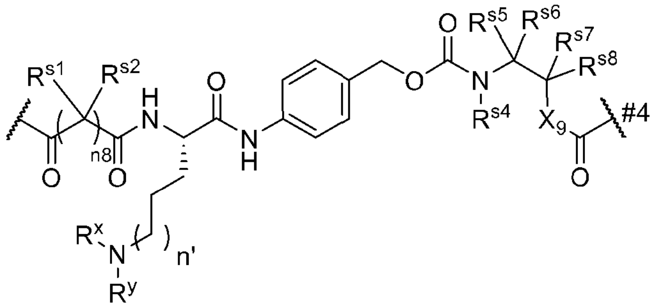

,

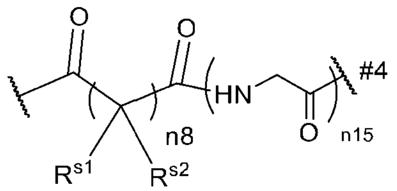

,

or

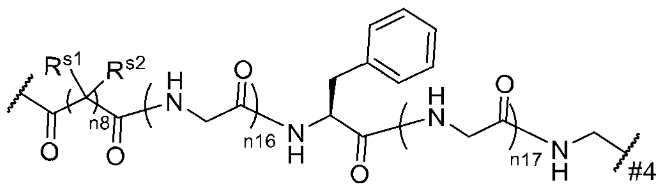

.

In some examples, Y is

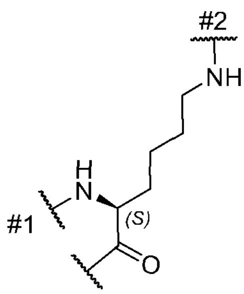

,

$L^{2a}$ is a covalent bond or

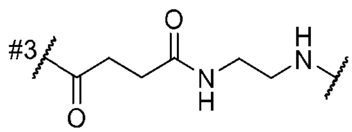

,

and $L^{2b}$ is a covalent bond,

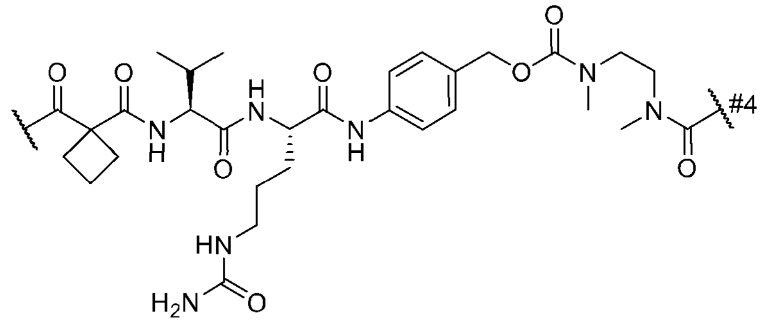

,

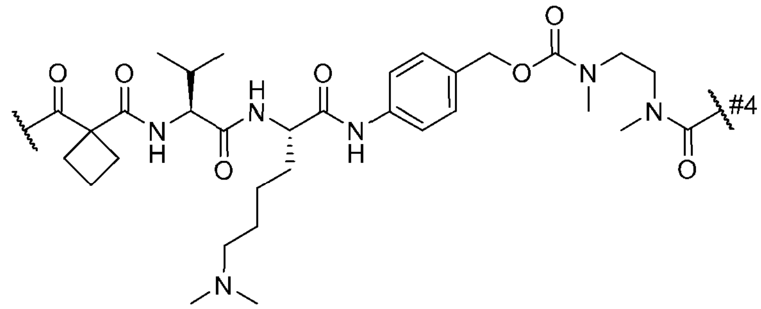

,

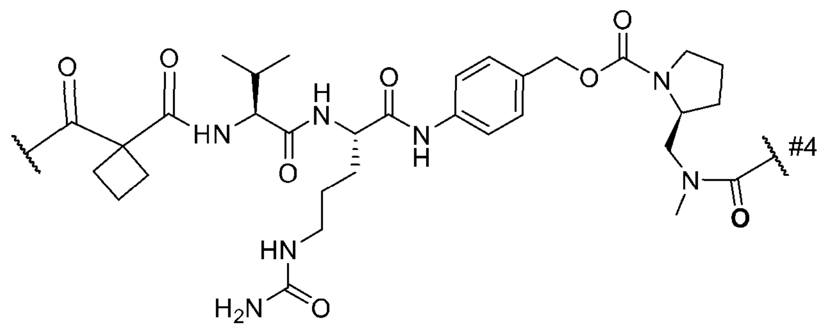

,

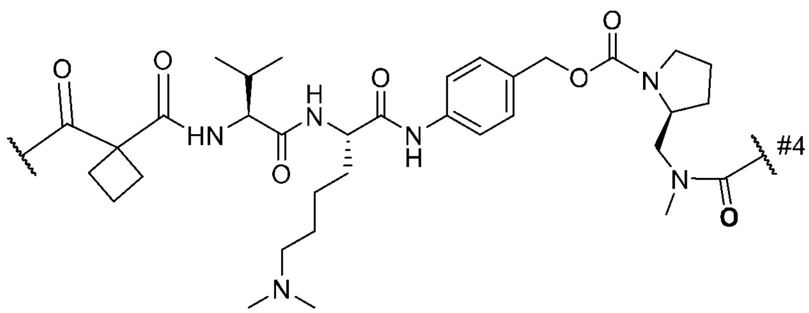

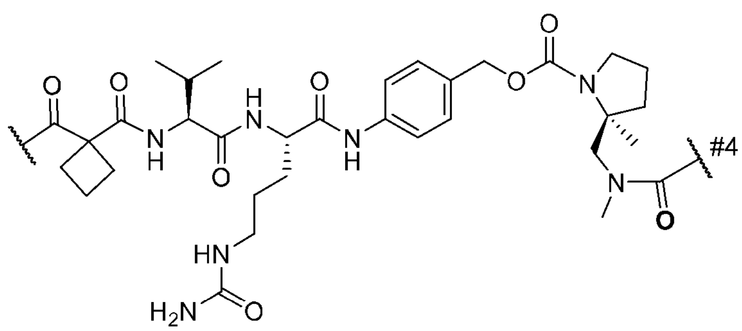

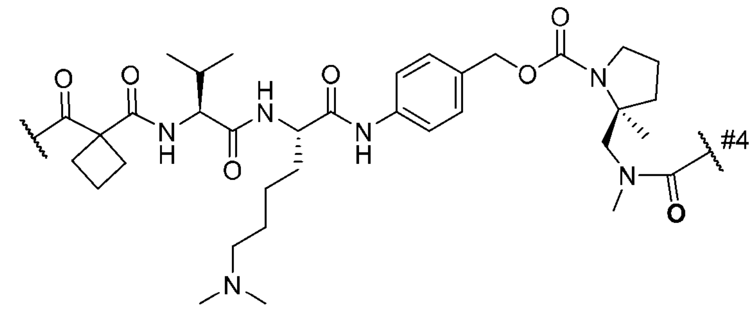

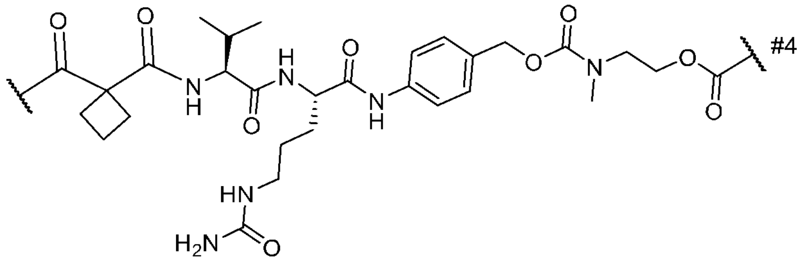

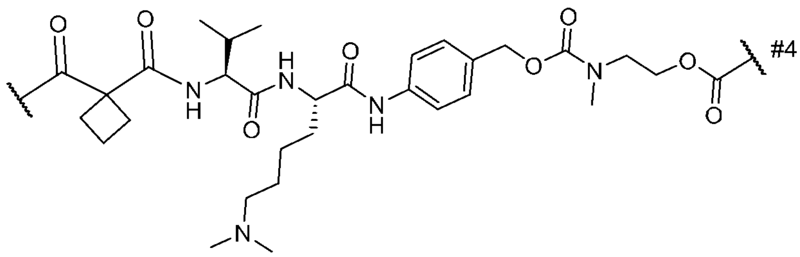

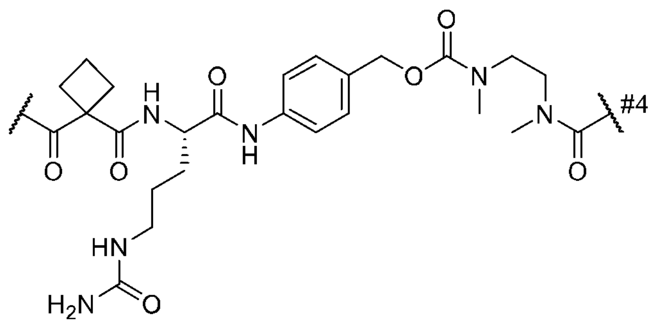

,

or

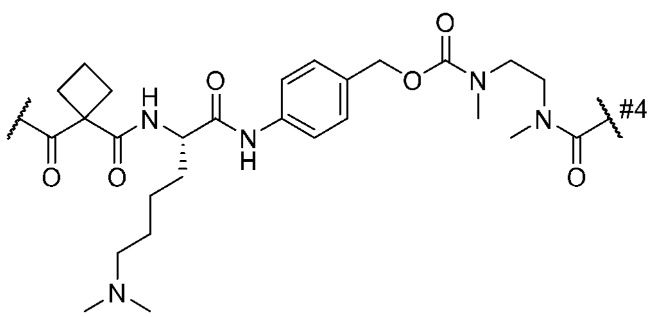

.

In some examples, Y is

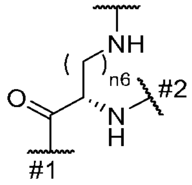

,

$L^{2a}$ is a covalent bond,

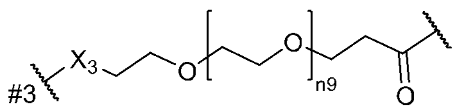

,

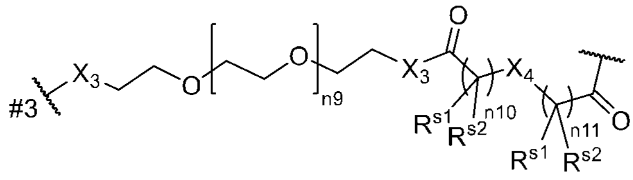

,

or

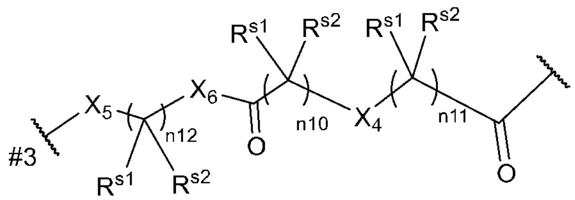

,

and $L^{2b}$ is a covalent bond,

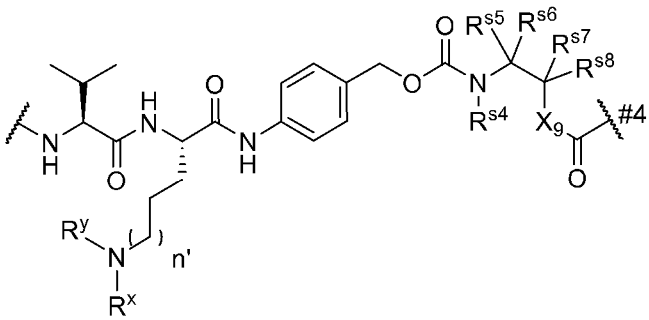

,

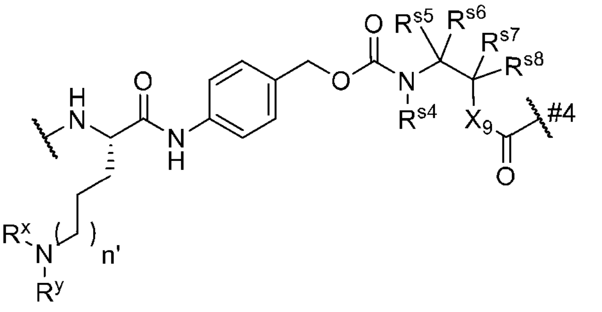

,

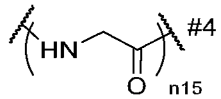

,

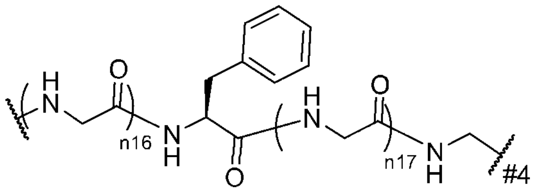,

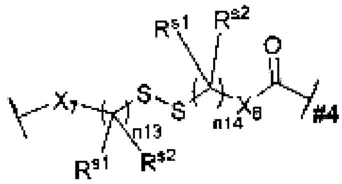,

or

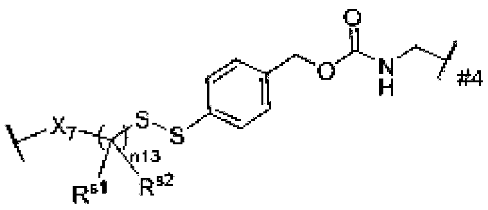.

In some examples, Y is

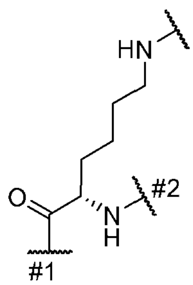,

$L^{2a}$ is a covalent bond,

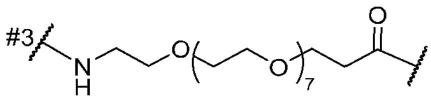,

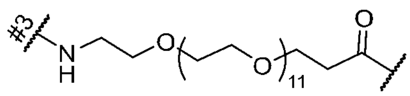,

or

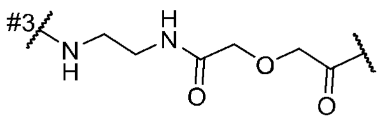,

and $L^{2b}$ is a covalent bond,

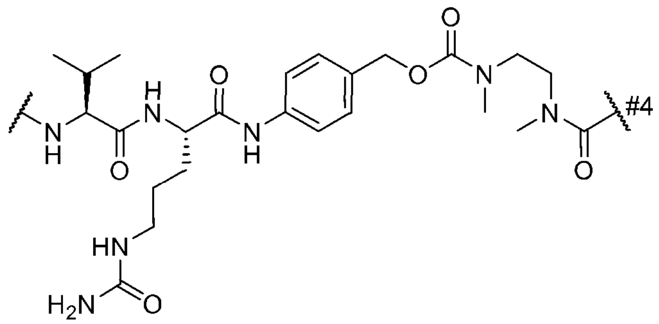

,

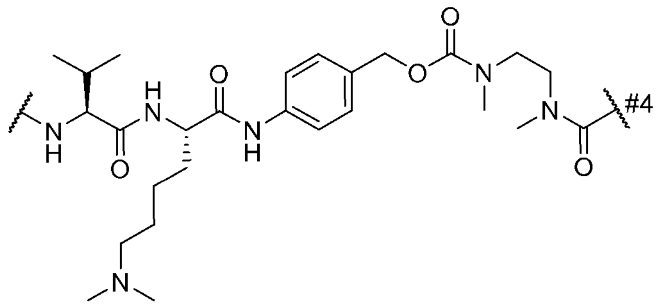

,

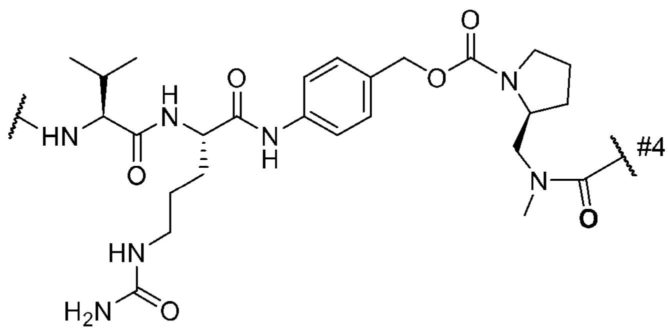

,

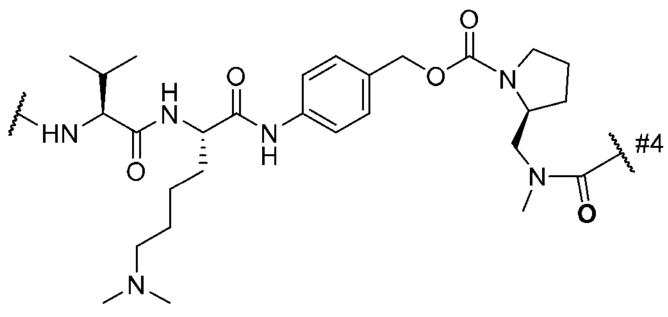

,

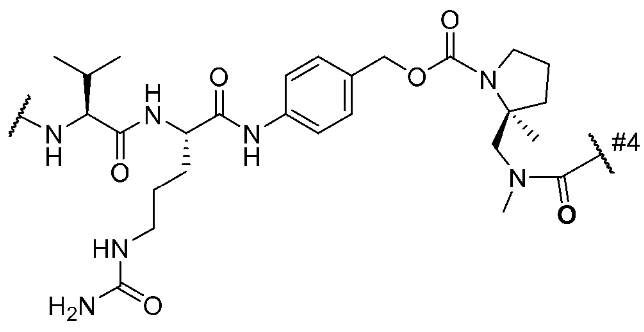

,

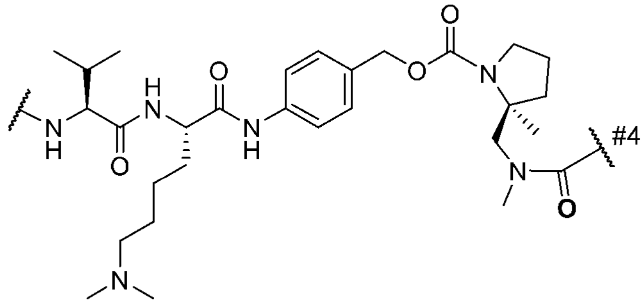

,

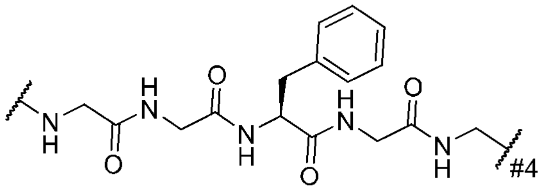

,

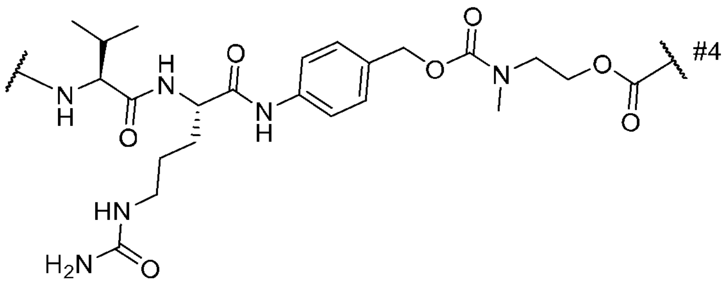

,

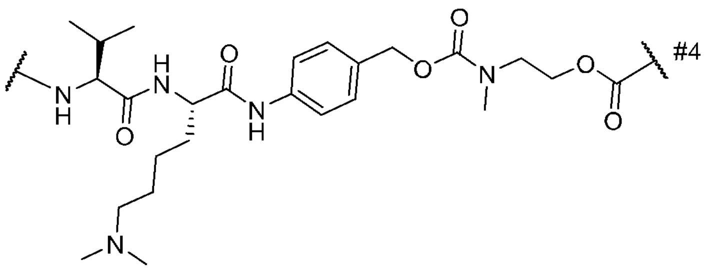

,

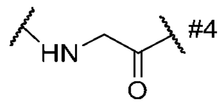

,

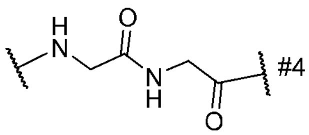

,

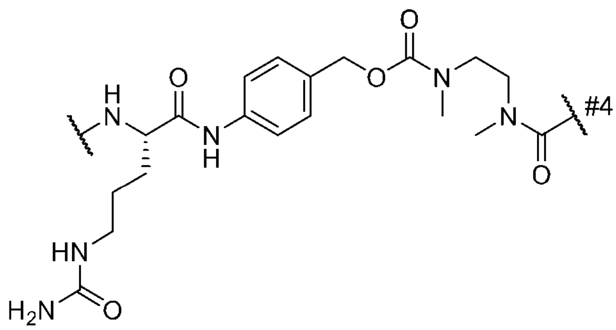

,

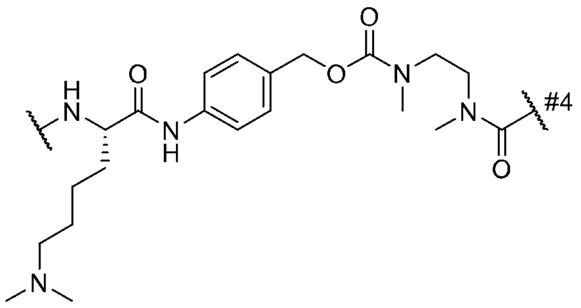

,

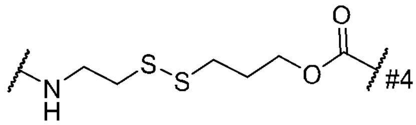

or

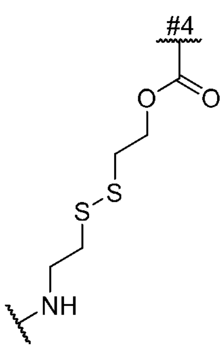

,

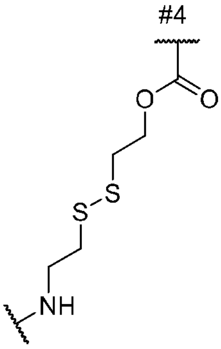

, or

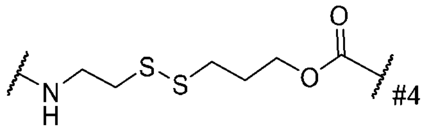

.

In some examples, Y is

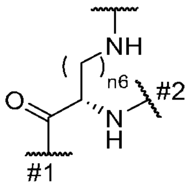

,

$L^{2a}$ is a covalent bond,

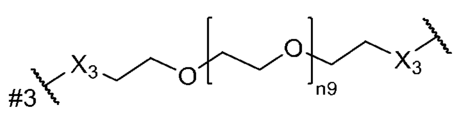

, or

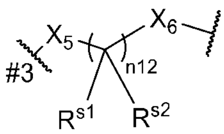

,

and $L^{2b}$ is a covalent bond,

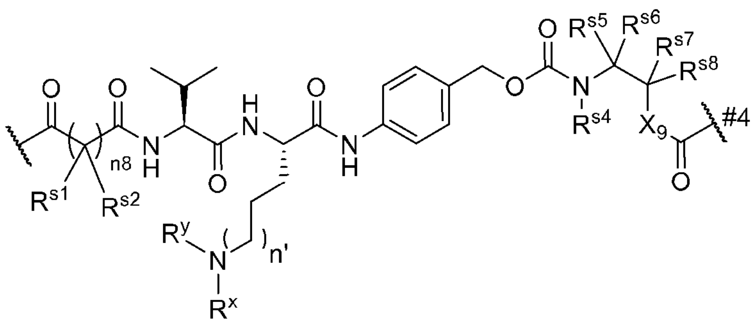

,

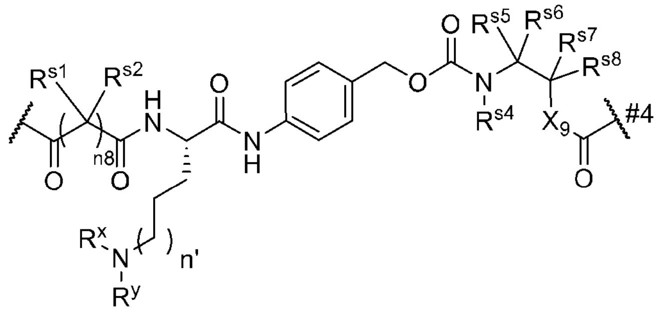

,

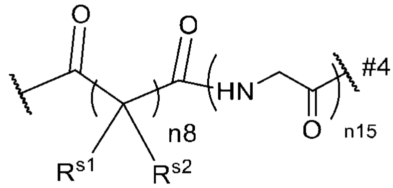

,

or

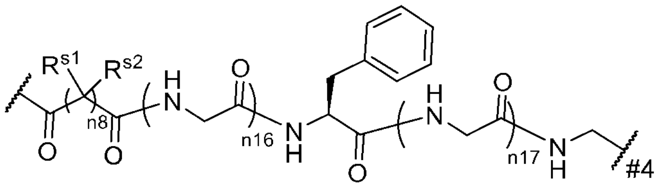

.

In some examples, Y is

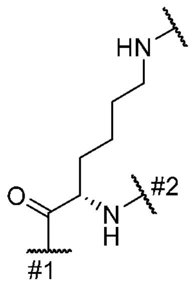

,

$L^{2a}$ is a covalent bond or

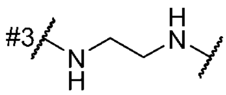

,

and $L^{2b}$ is a covalent bond,

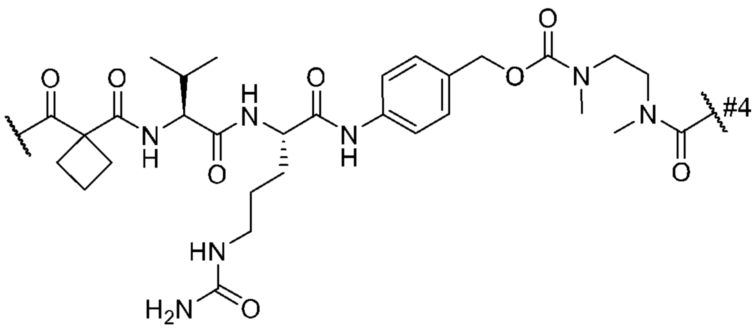

,

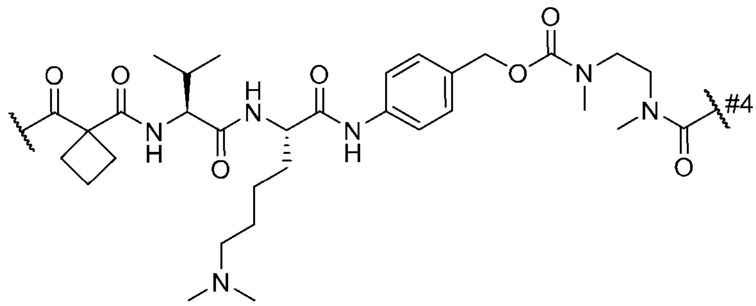

,

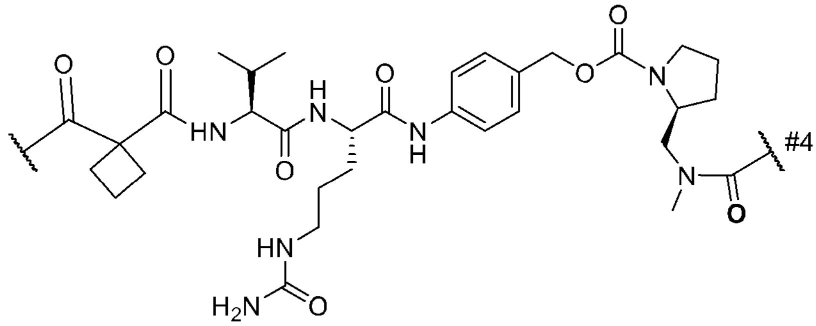

,

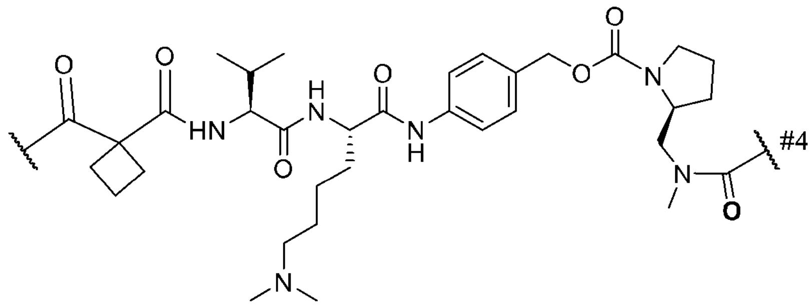

,

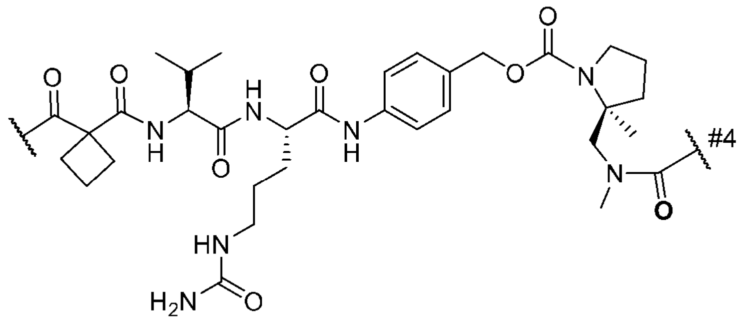

,

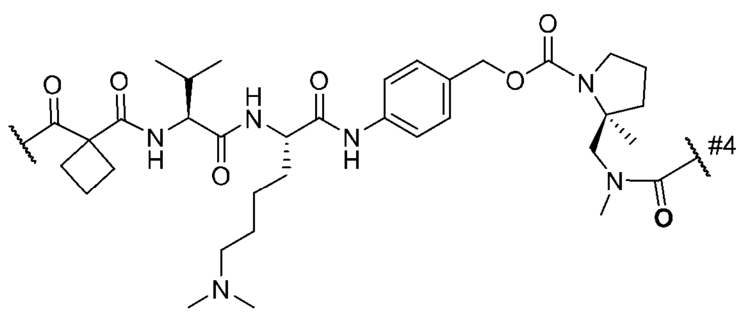

,

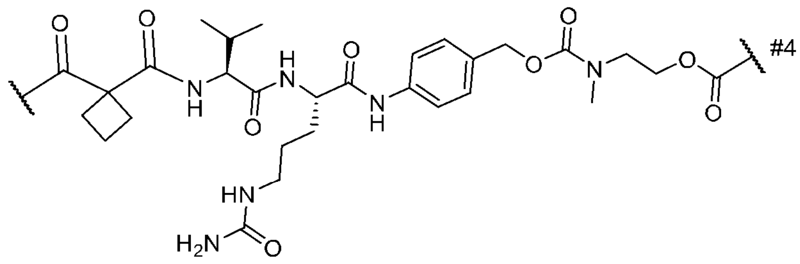

,

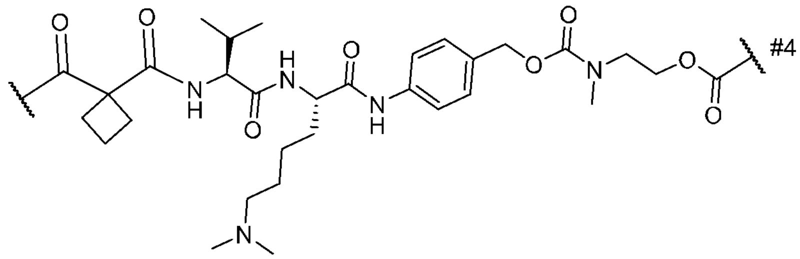

,

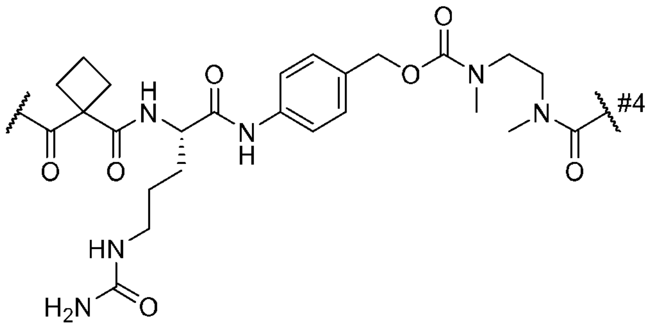

,

or

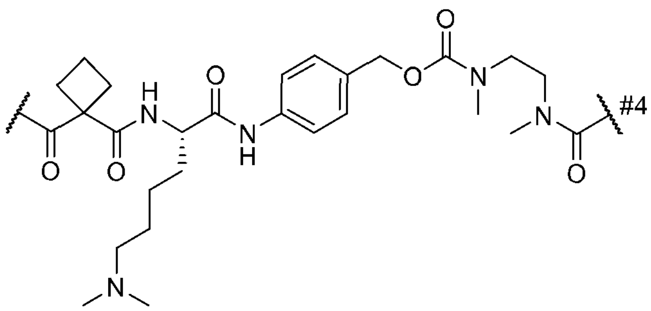

.

In some examples, Y is

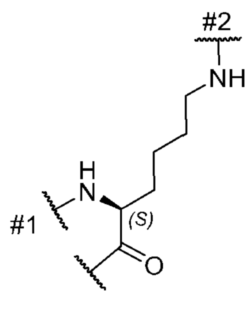

,

and $L^2$ is a covalent bond,

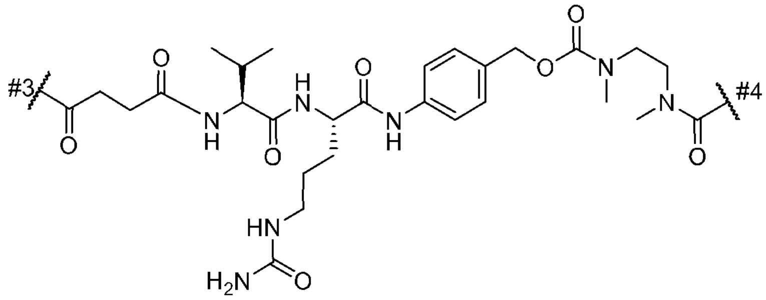

,

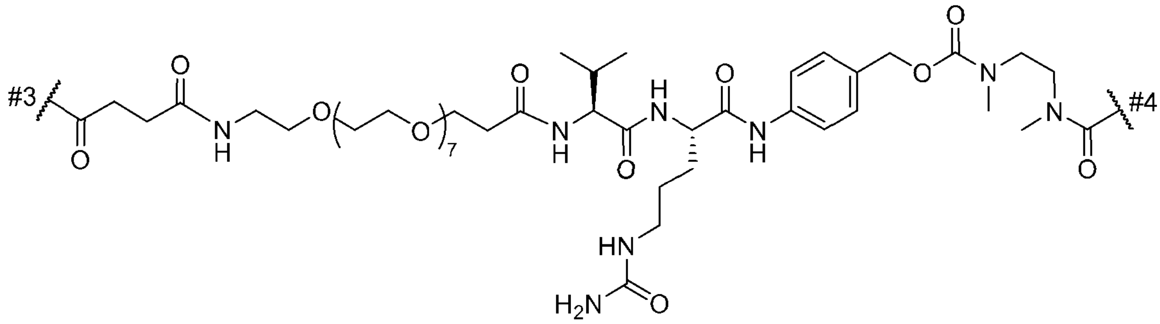

,

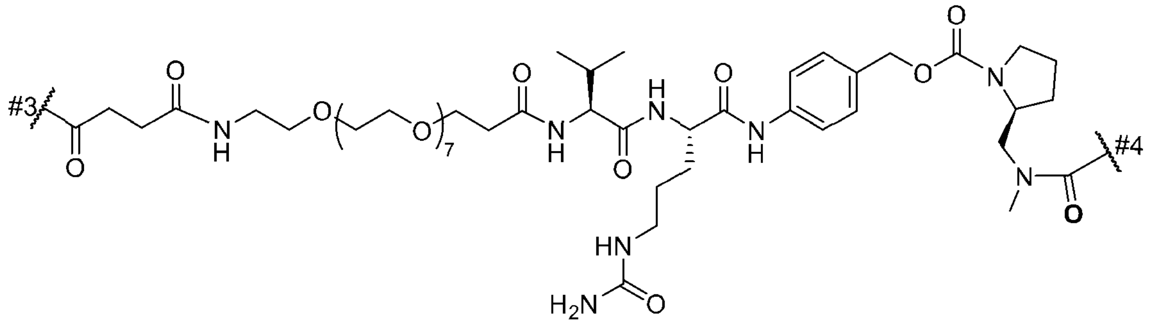

,

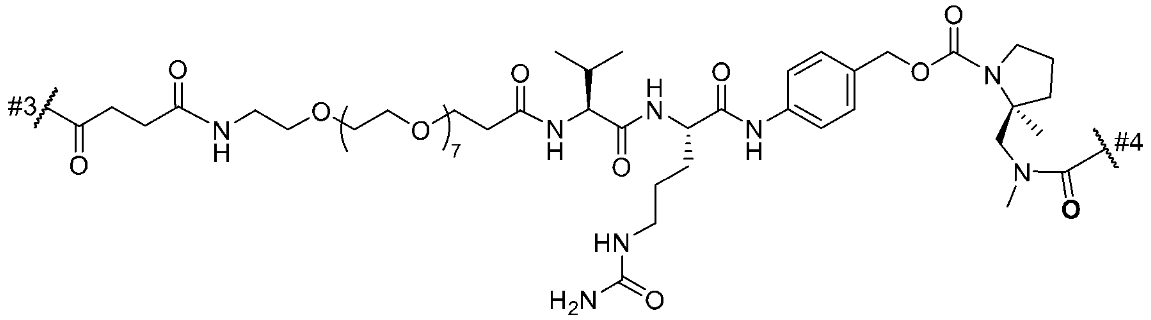

,

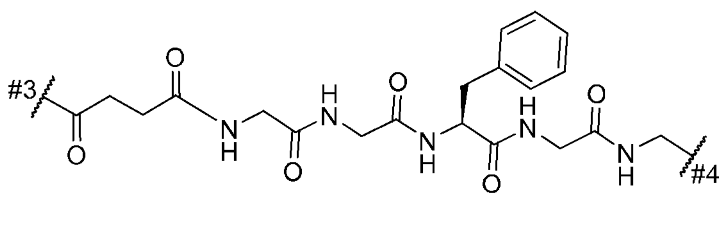

,

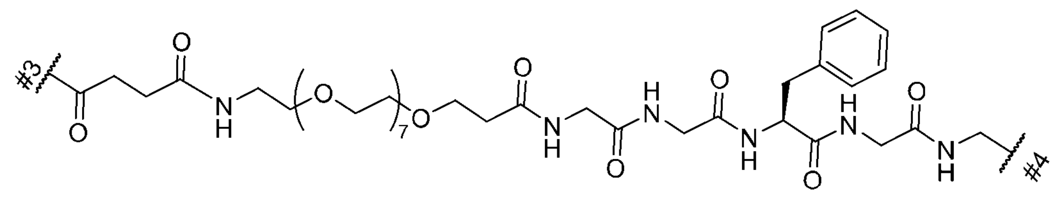

,

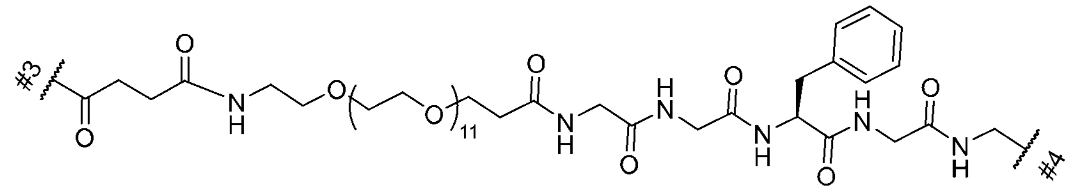

,

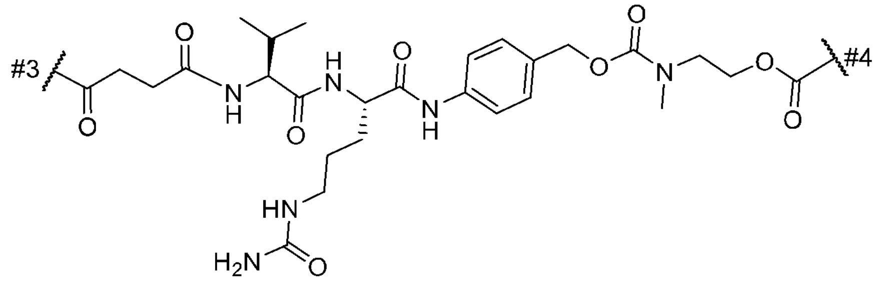

,

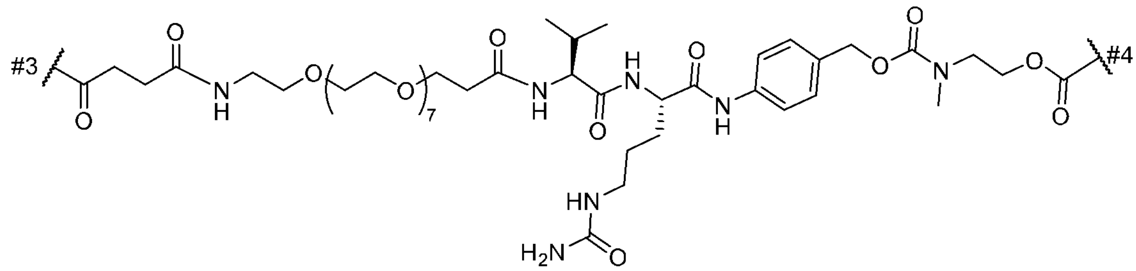

,

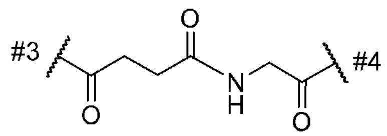

,

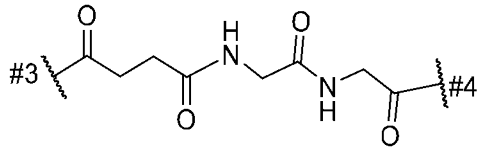

,

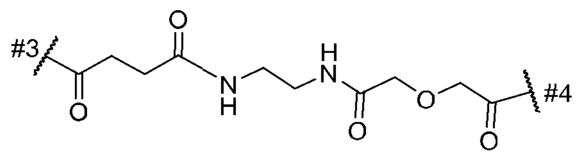

,

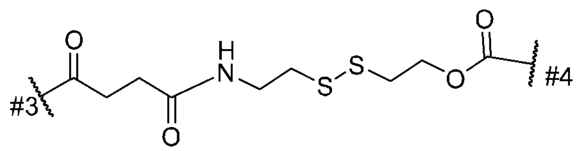

,

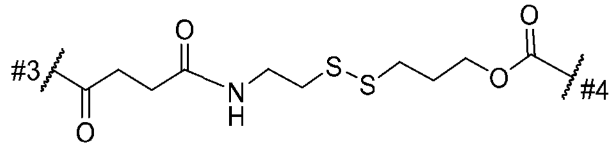

,

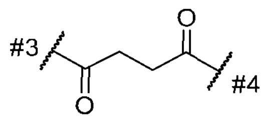

,

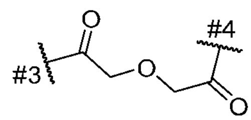

,

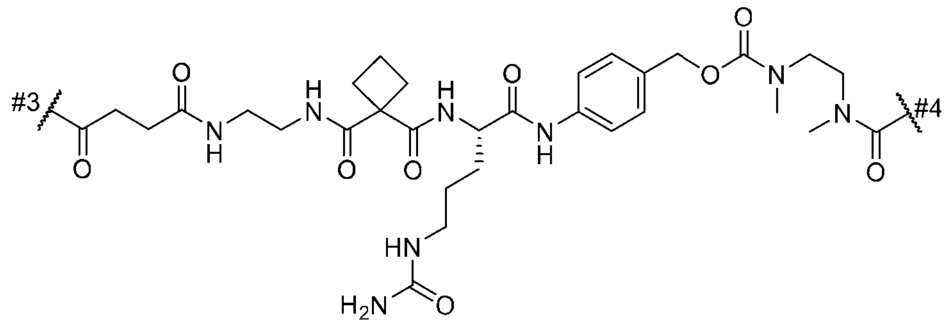

,

or

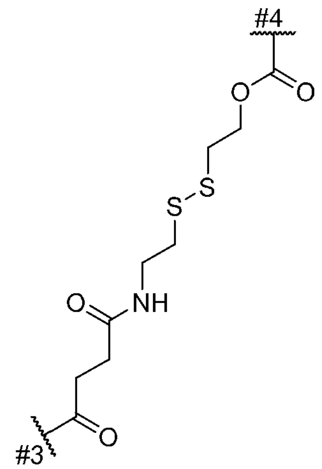

In some examples, Y is

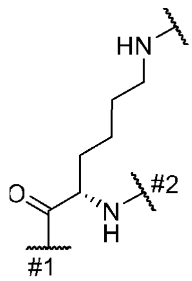

,

and $L^2$ is a covalent bond,

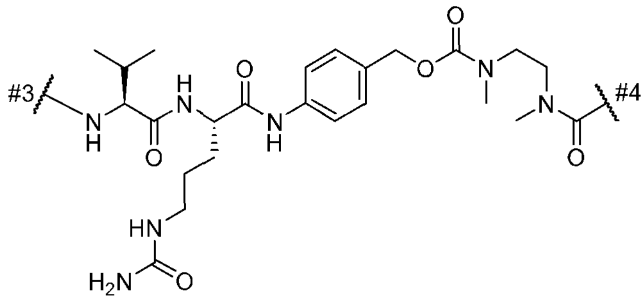

,

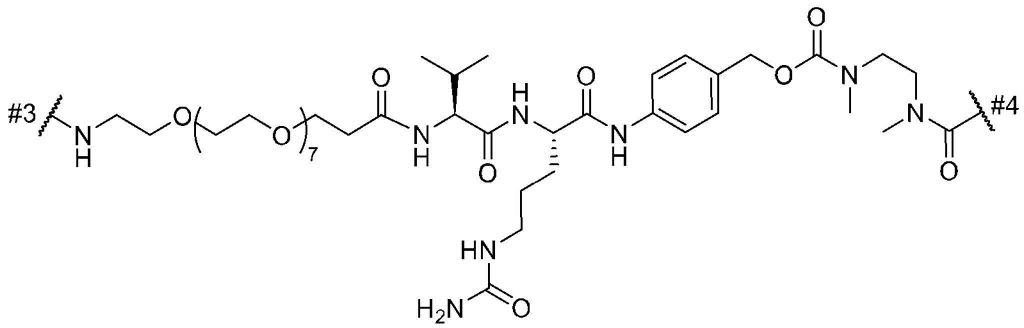

,

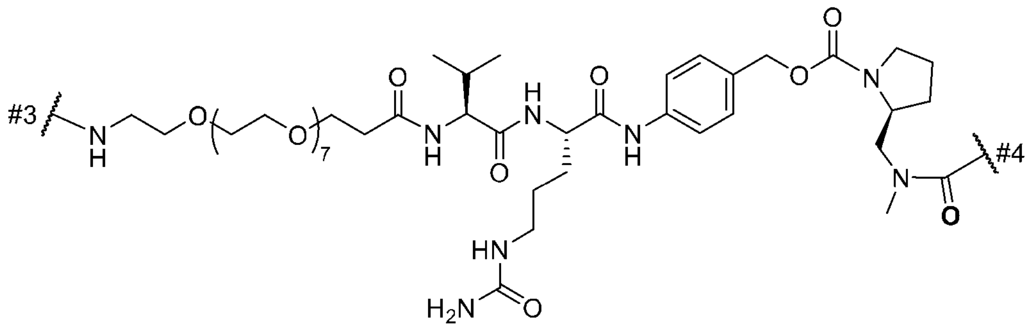

,

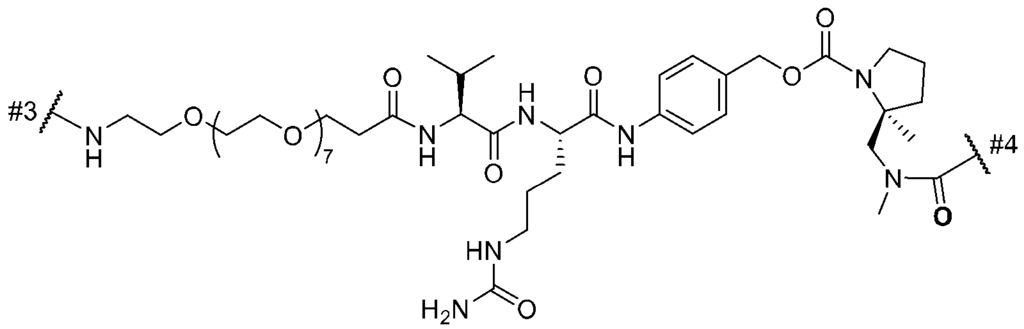

,

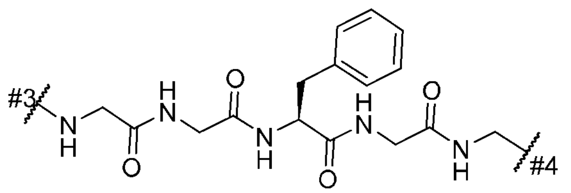

,

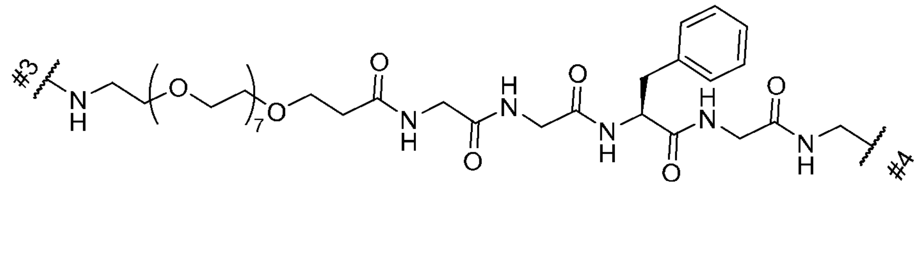

,

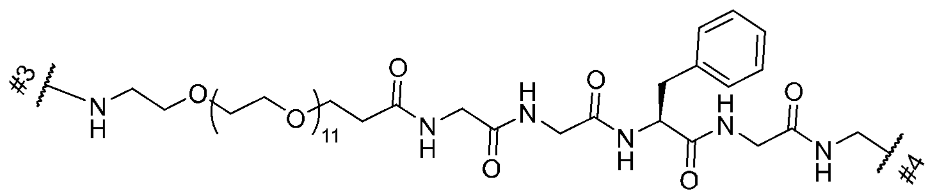

,

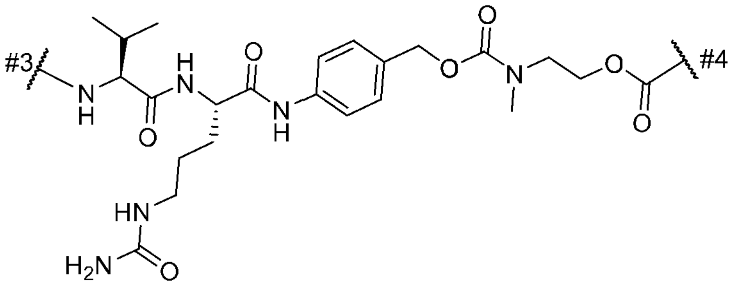

,

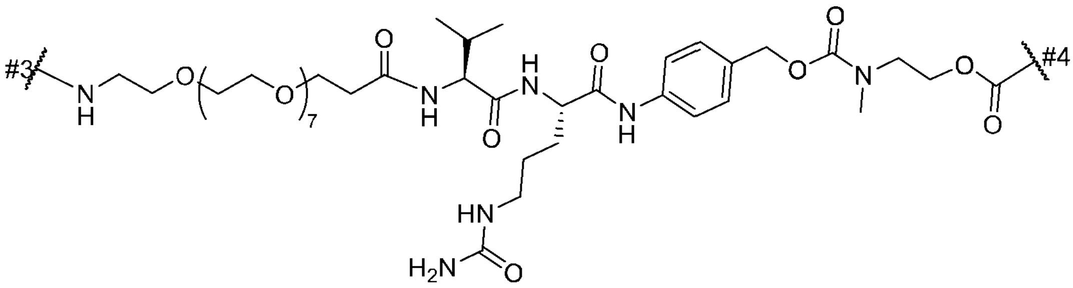

,

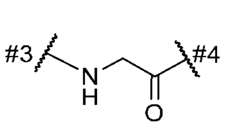 , 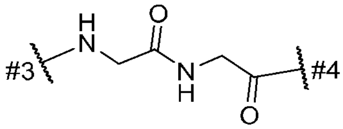 , 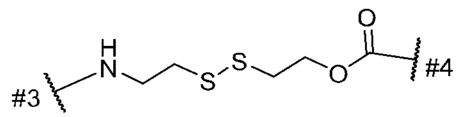 ,

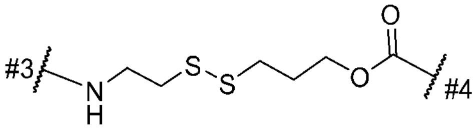 , 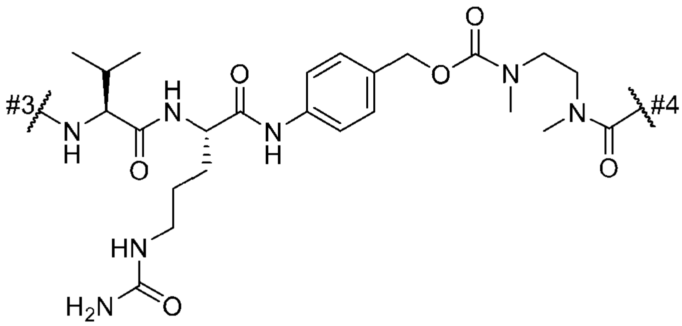 ,

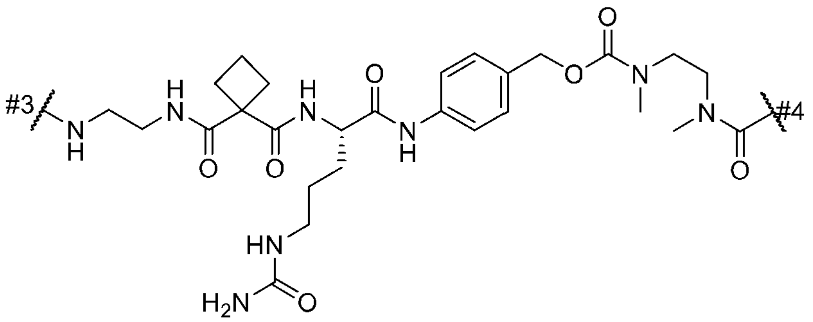, 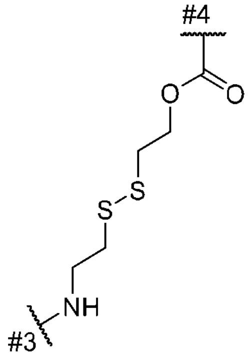,

or

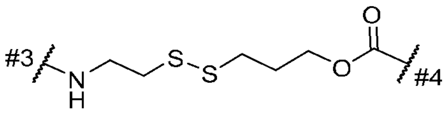.

In some examples, X is OH, $NH_2$, -D, $-L^{2b}$-D,

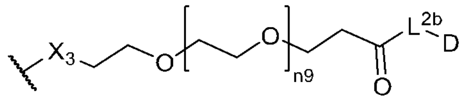,

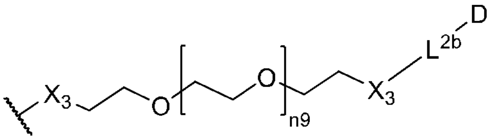, 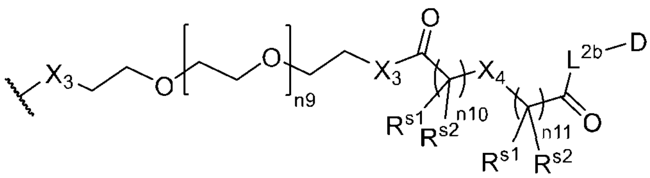,

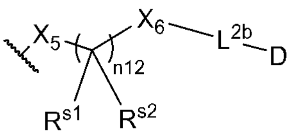, 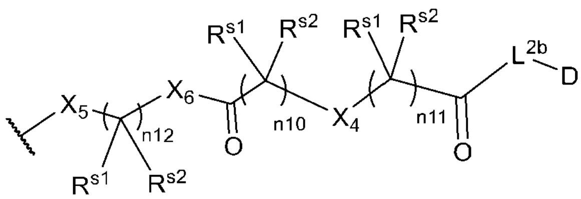, or 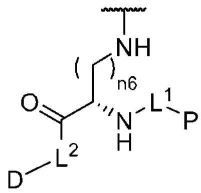,

wherein in

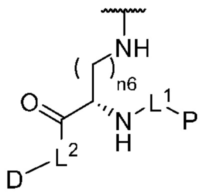,

$-L^2$-D is $-L^{2b}$ -D,

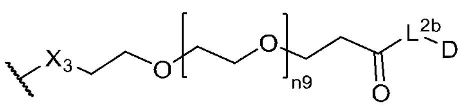,

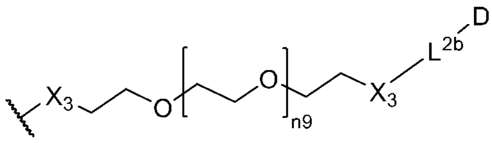 , 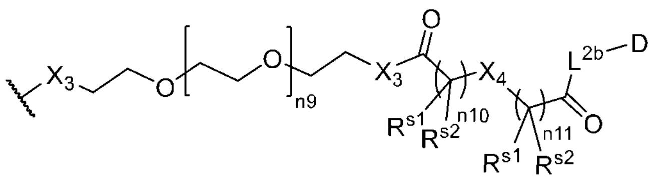 ,

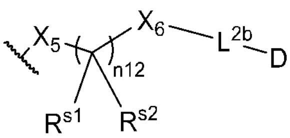 , or 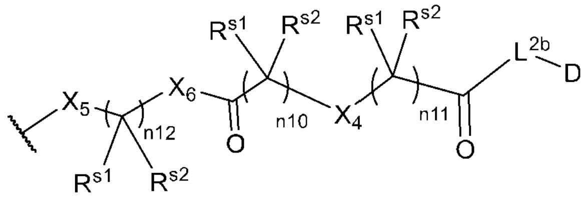 .

In some examples, X is OH, $NH_2$, -D,

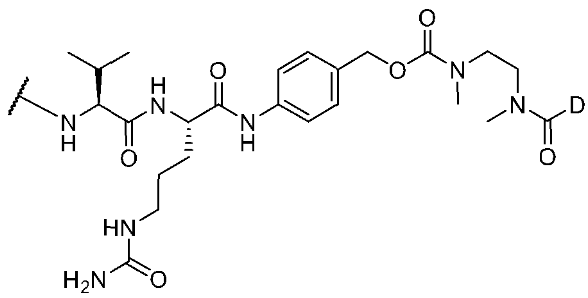 ,

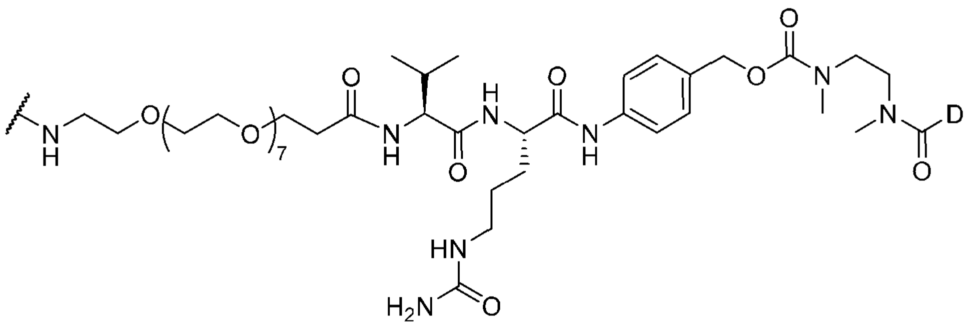 ,

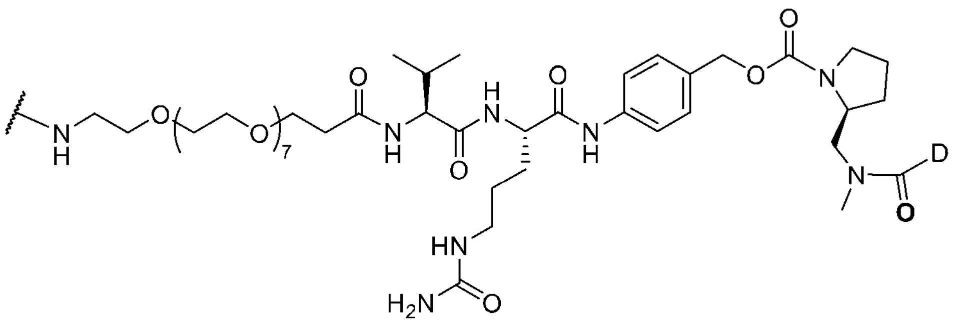 ,

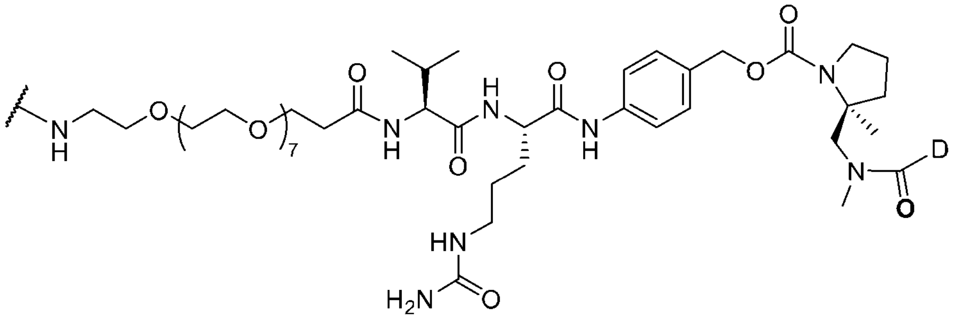

,

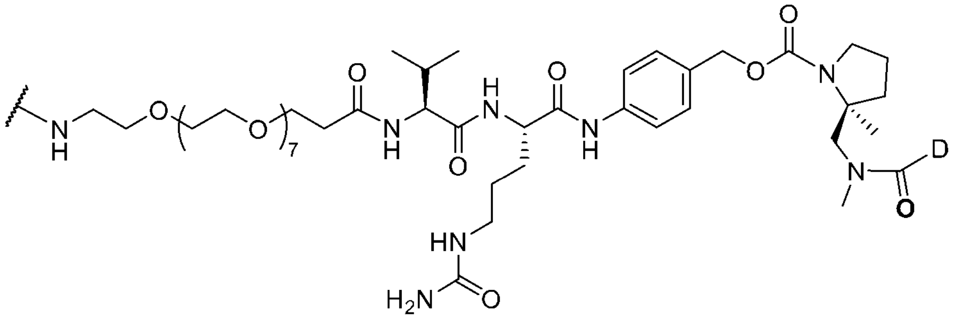

,

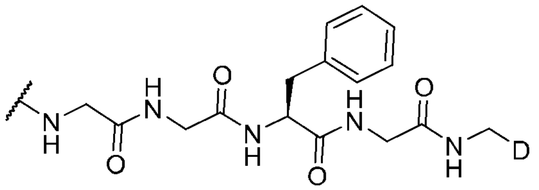

,

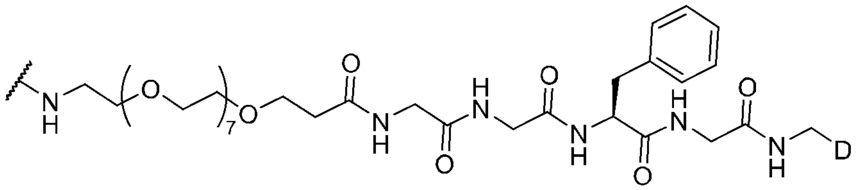

,

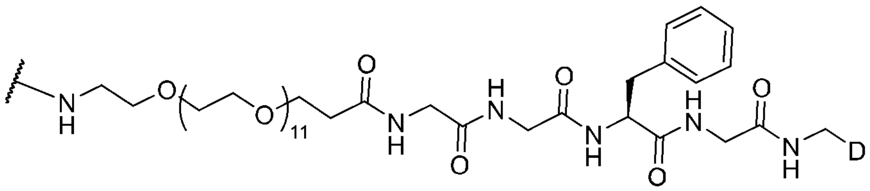

,

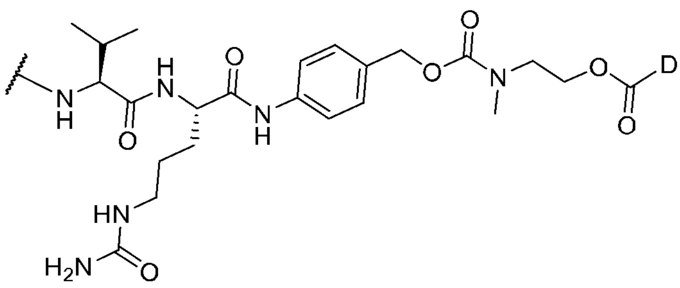

,

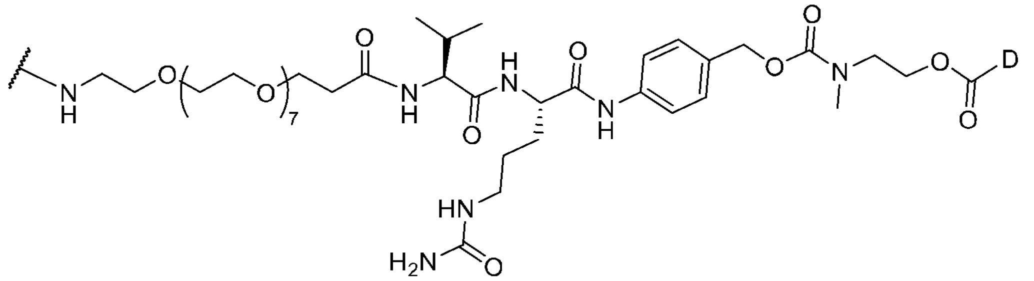

,

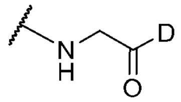

,

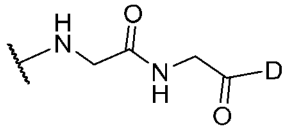

,

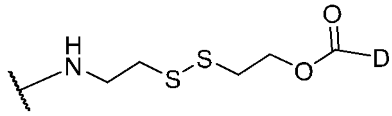

,

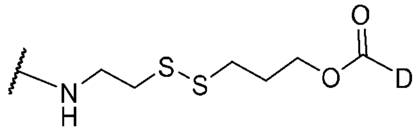

,

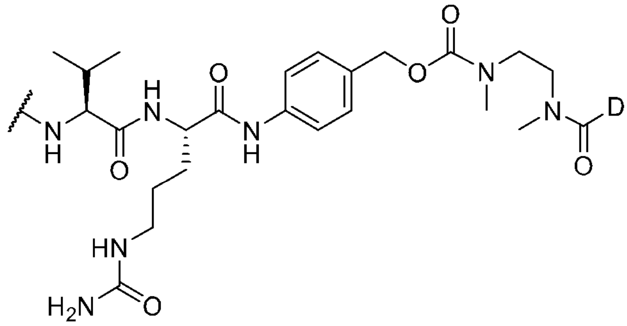

,

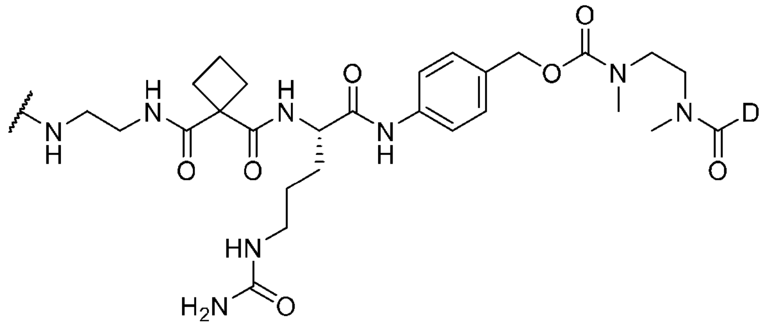

,

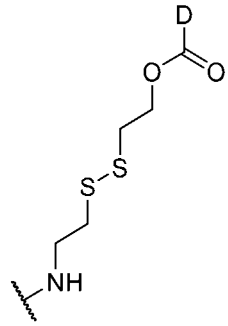

,

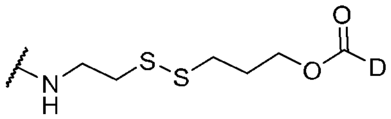

, or

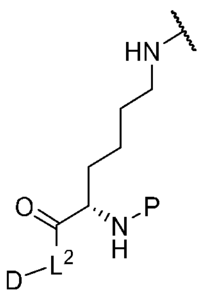

,

wherein, in

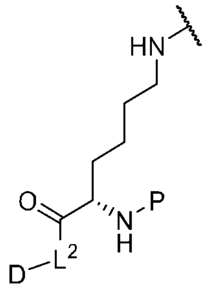

,

$-L^2-D$ is

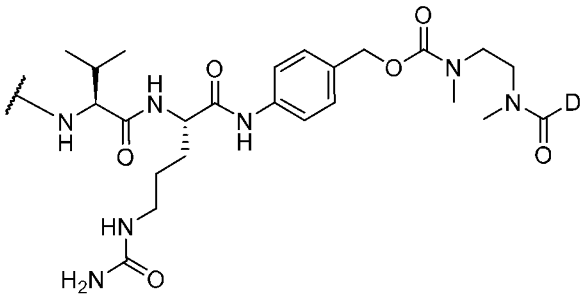

,

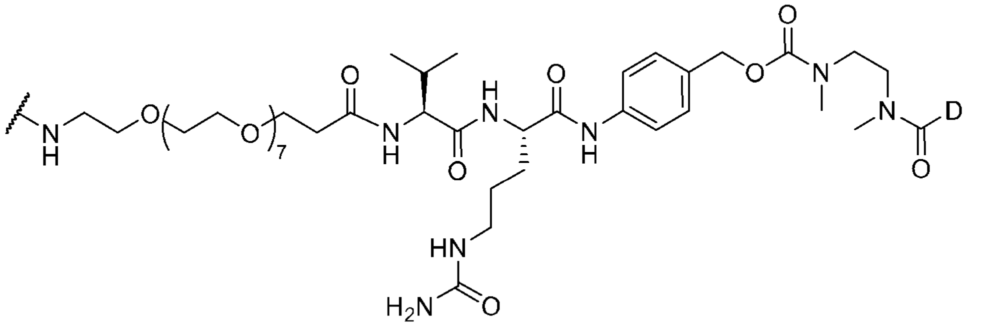

,

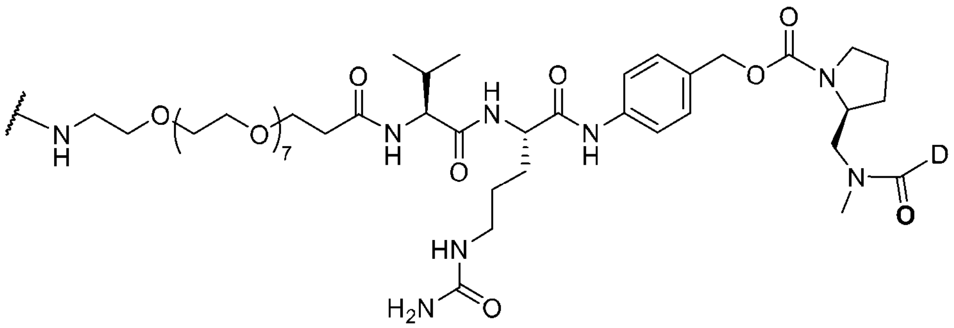

,

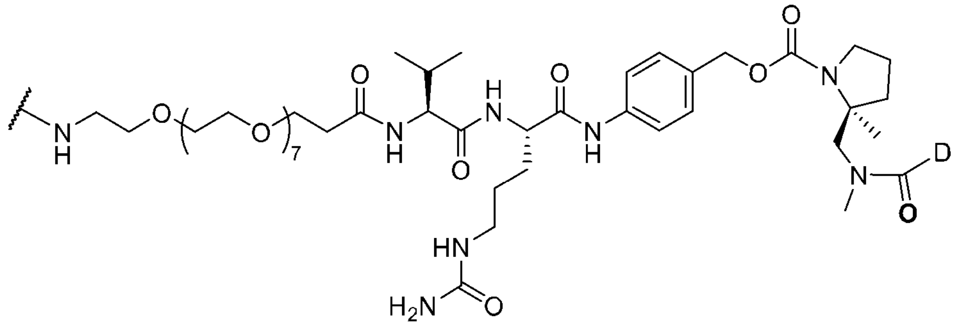

,

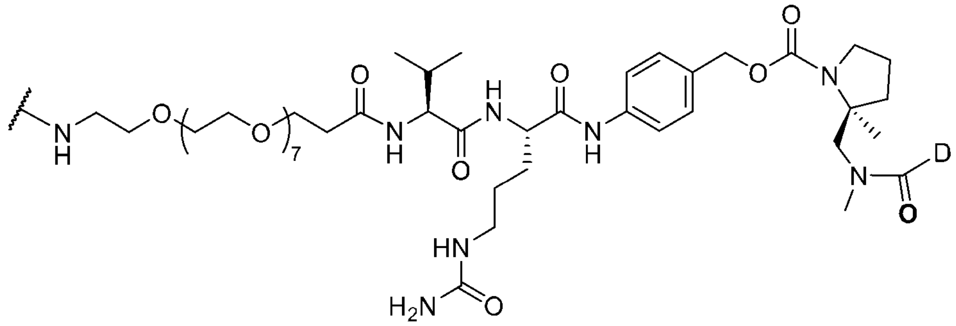

,

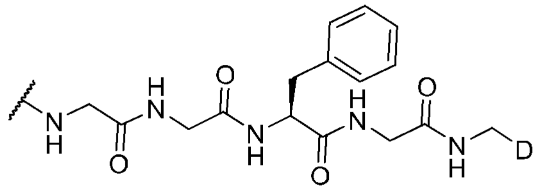

,

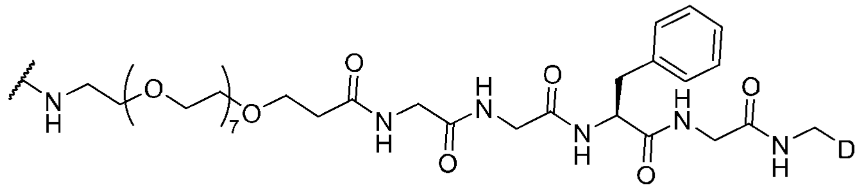

,

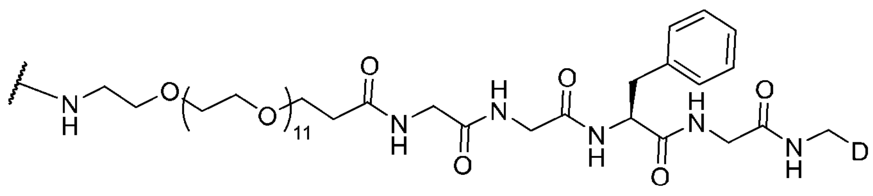

,

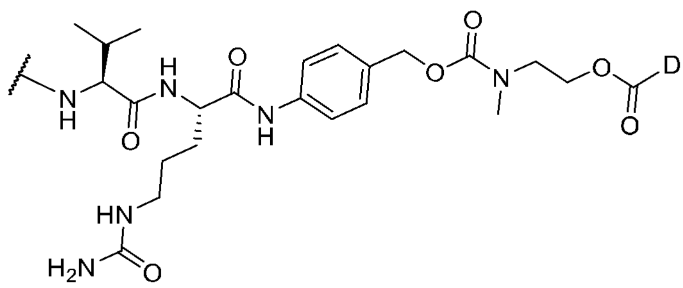

,

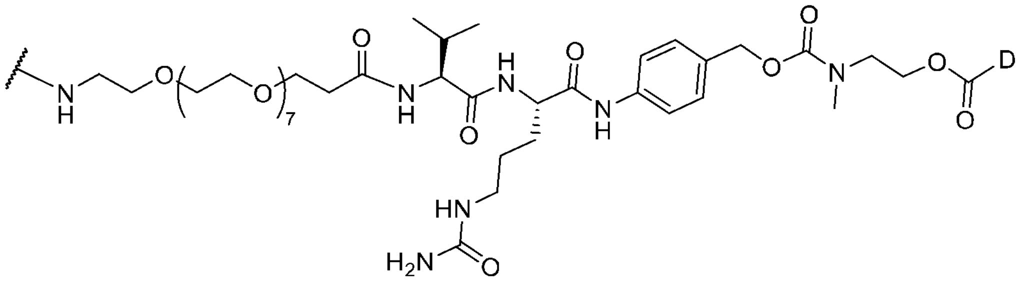

,

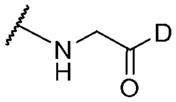

,

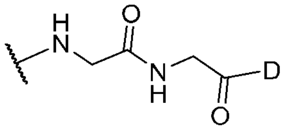

,

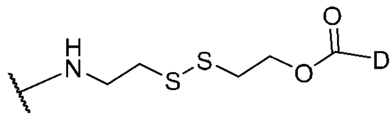

,

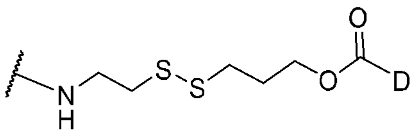

,

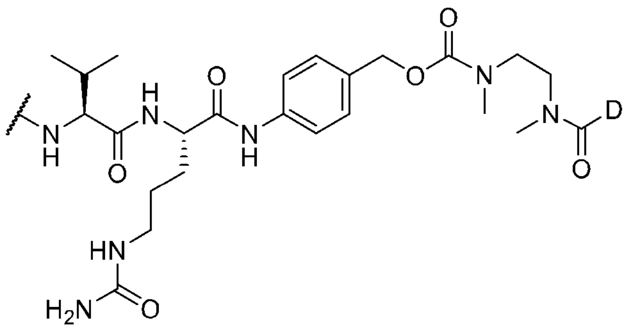

,

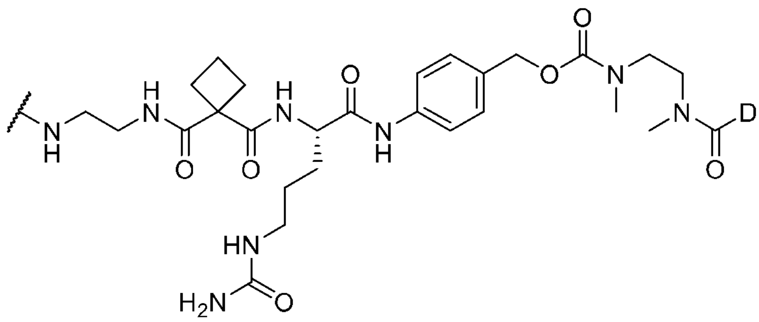

,

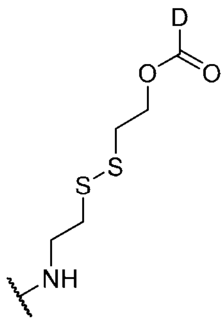

,

or

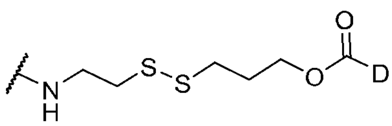

.

In some examples, Q is

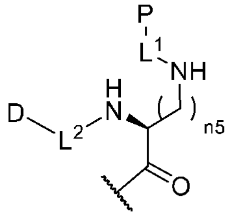

or-P, and in

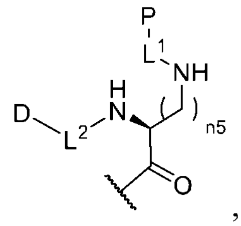,

$-L^2$-D is

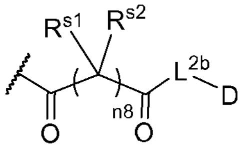, 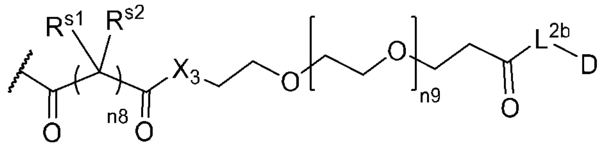, 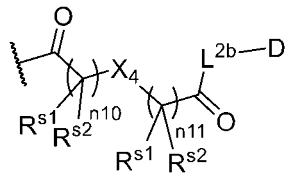,

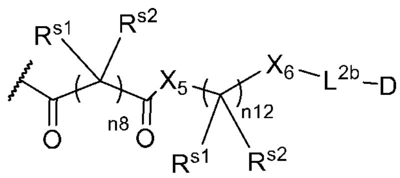, 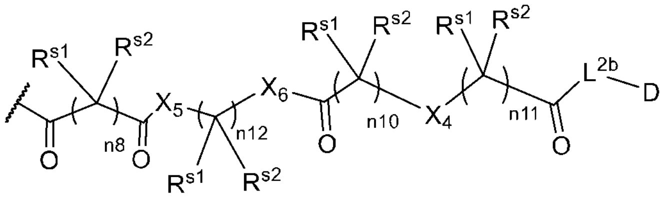,

or

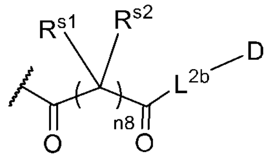.

In some examples, Q is

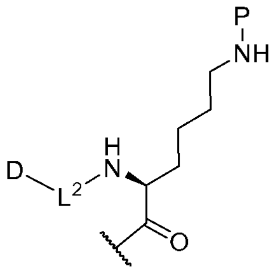

or -P, and in

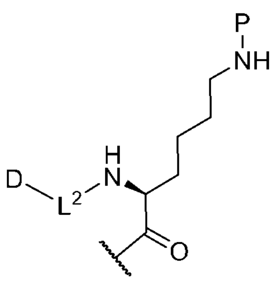, -

$L^2$-D is

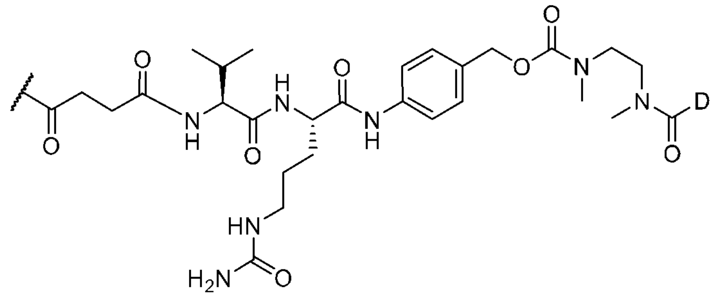

,

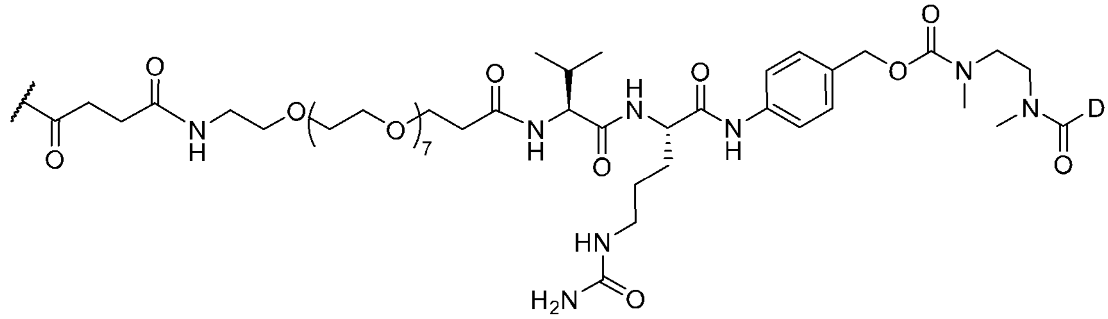

,

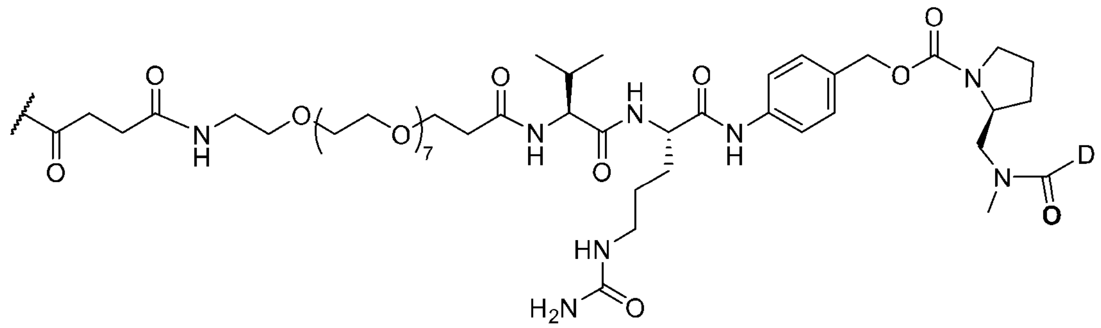

,

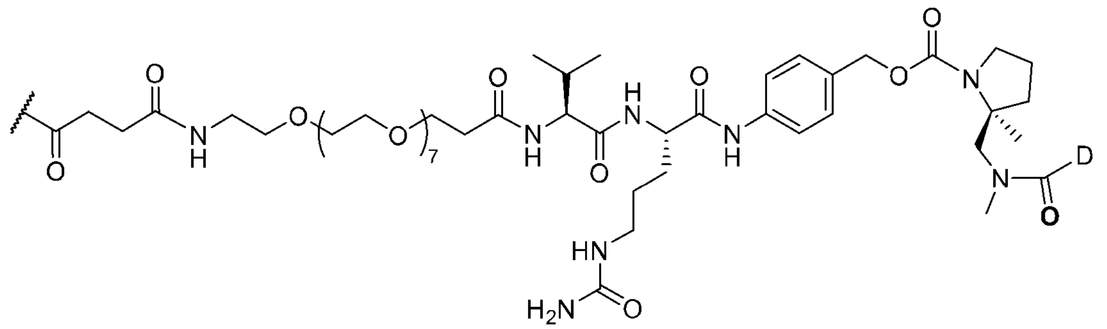

,

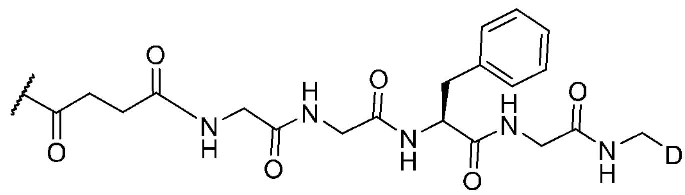

,

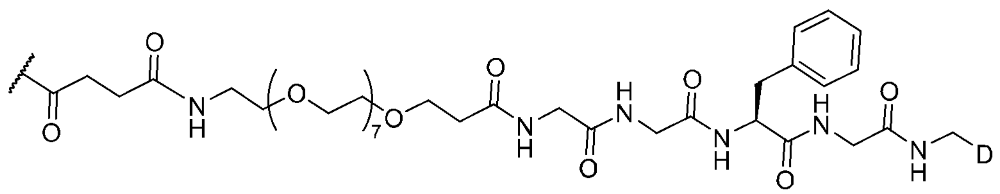

,

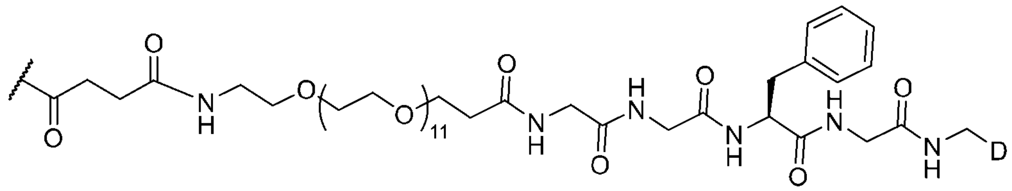

,

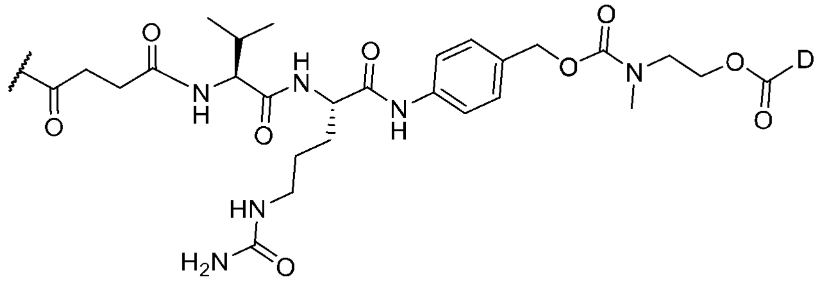

,

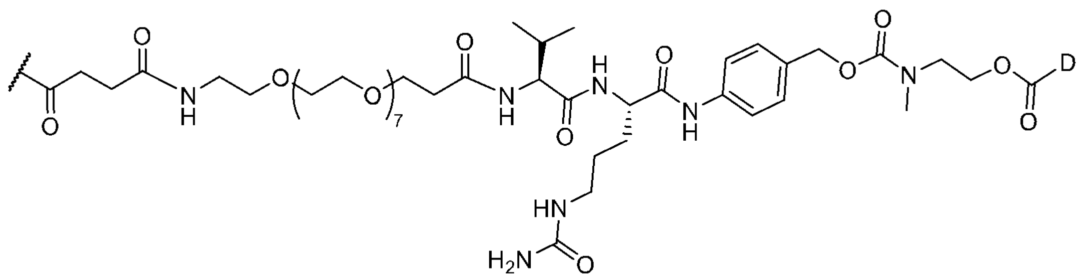

,

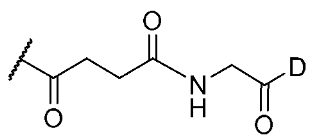

,

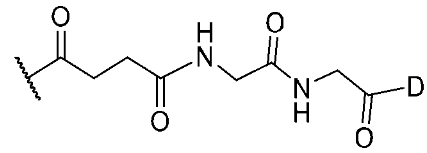

,

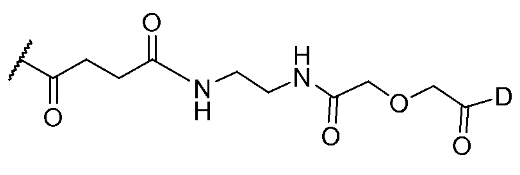

,

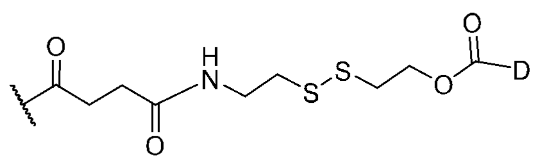

,

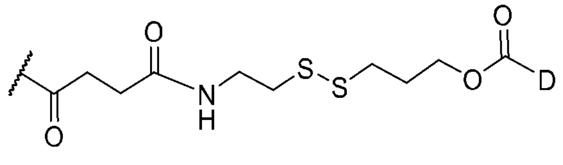

,

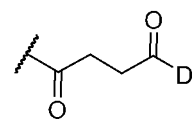

,

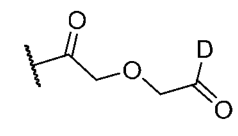

,

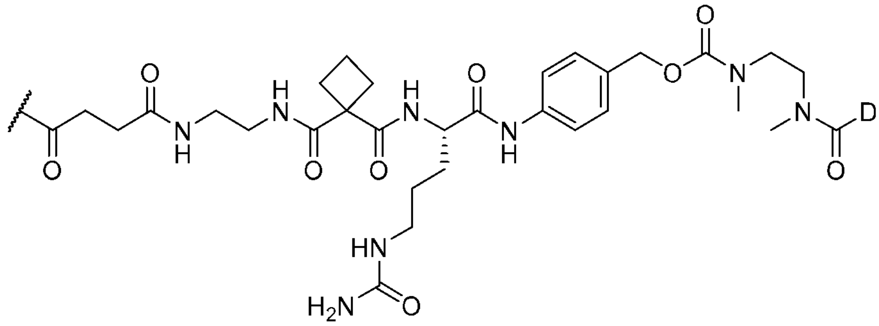

,

or

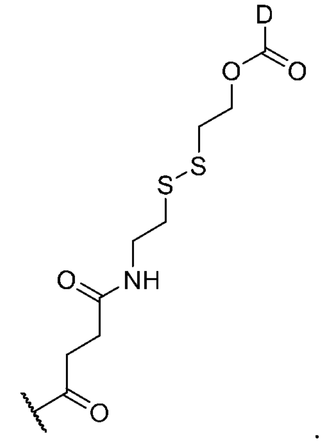

.

In some examples, D is the pharmaceutically active agent residue according to any of the above embodiments.

In some examples, D is a residue of a drug having an active functional group, and the active functional group is selected from one or more of primary amine group, secondary amine group, hydroxy, and sulfhydryl; preferably, the drug is an antineoplastic, e.g., one or more of camptothecin, a camptothecin derivative, paclitaxel, a paclitaxel derivative, cisplatin, and a cisplatin derivative.

In some examples, D is any one of the following structures:

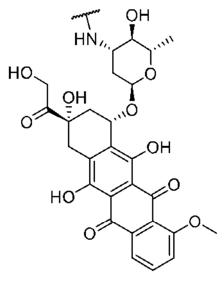

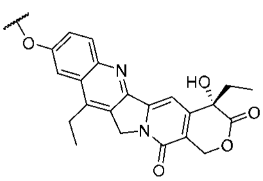

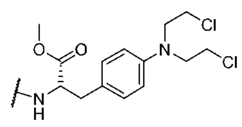

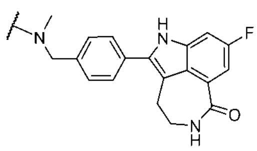

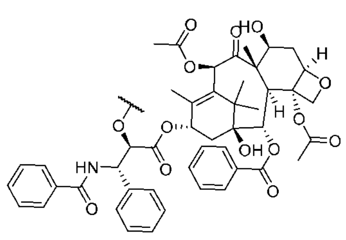

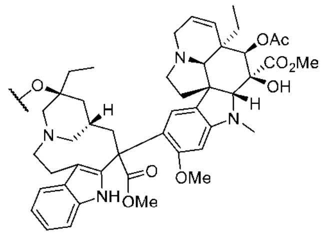

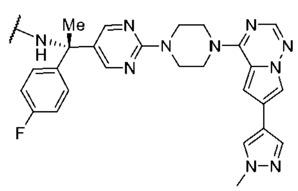

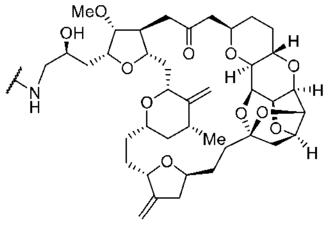

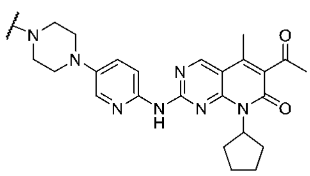

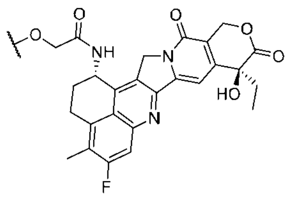

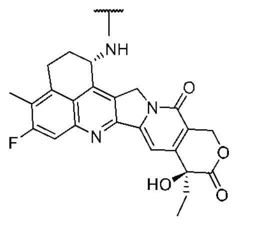

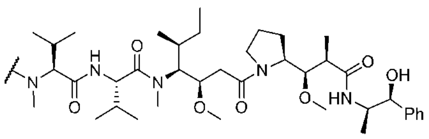

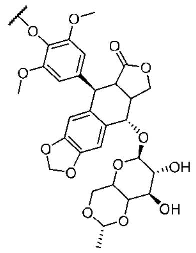

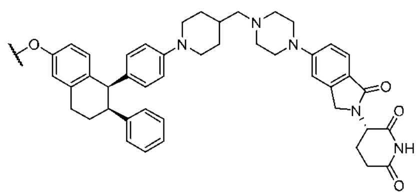

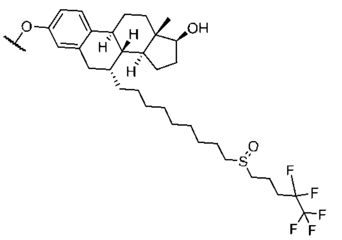

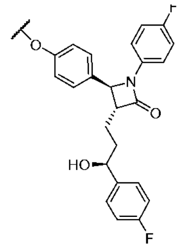

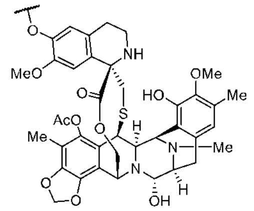

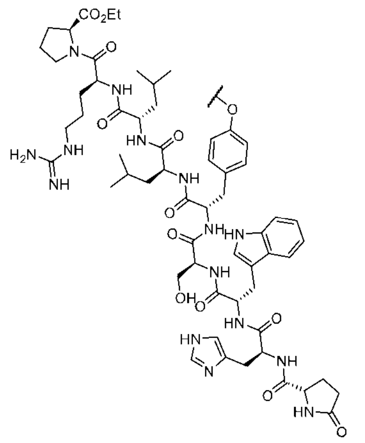

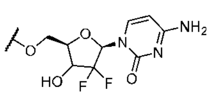

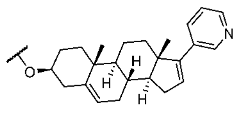

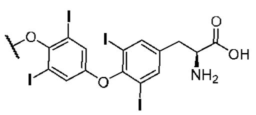

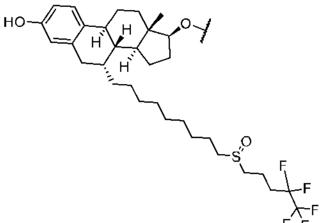

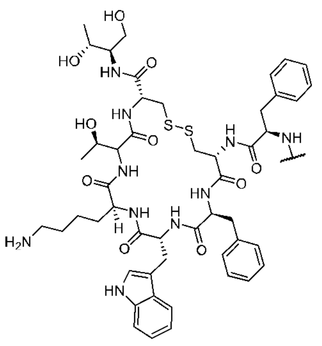

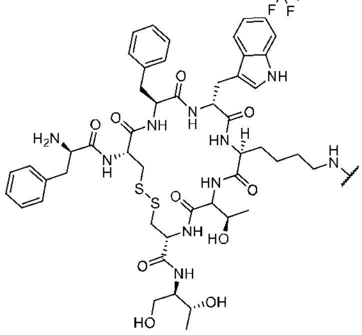

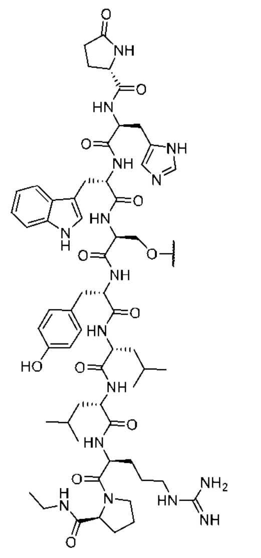

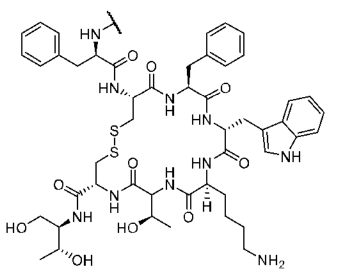

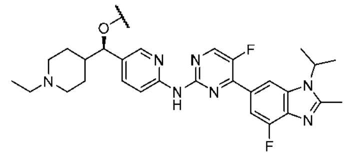

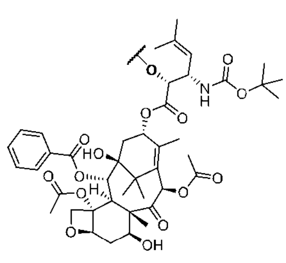

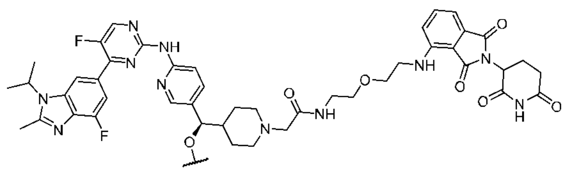

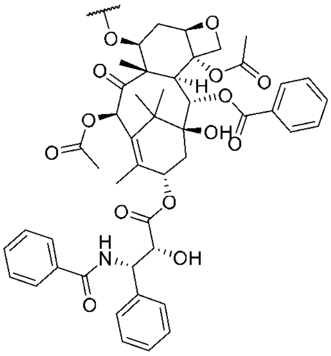
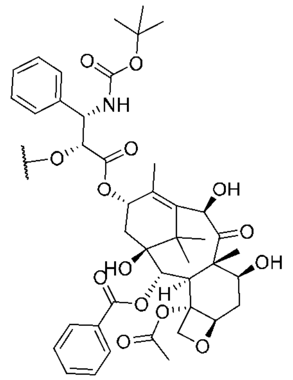
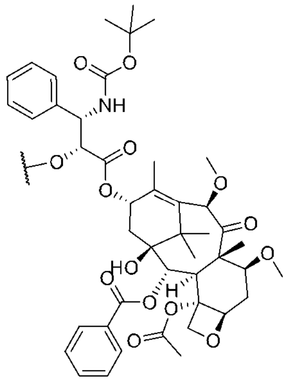
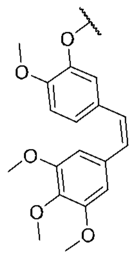

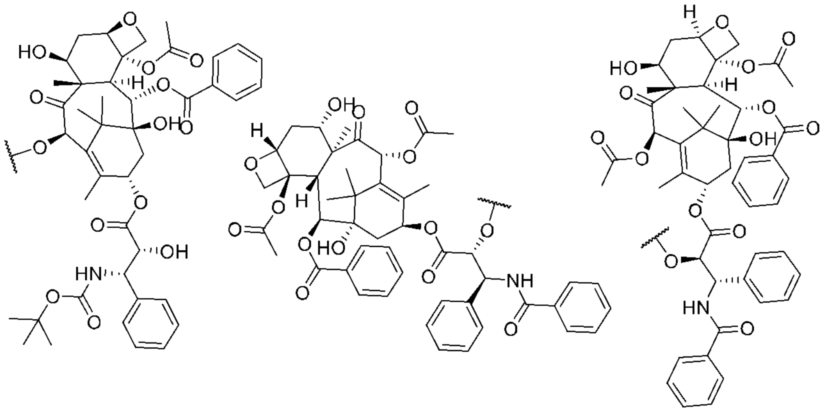
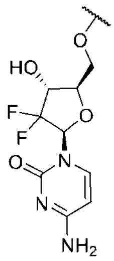

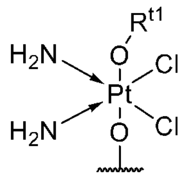
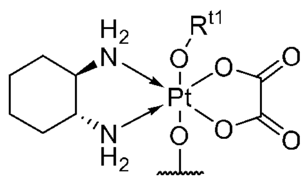
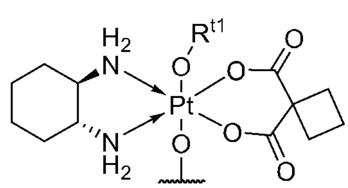

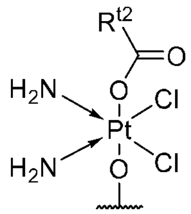
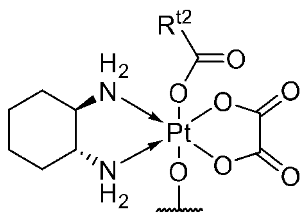
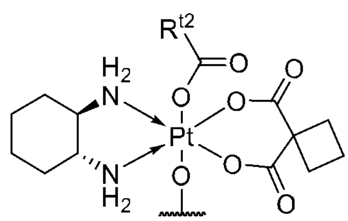

wherein $R^{t1}$ is selected from H and $C_1$-$C_{20}$ alkyl; $R^{t2}$ is selected from $NHR^{t1}$ and $C_1$-$C_{20}$ alkyl.

In some examples, D is any one of the following structures:

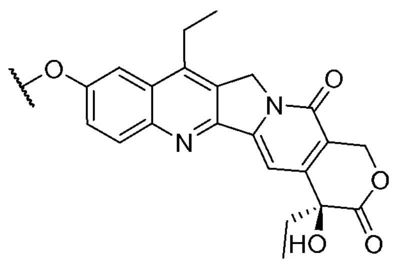, 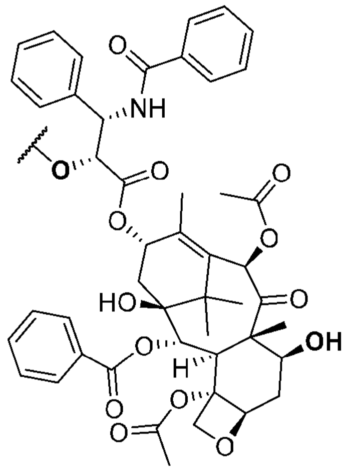, 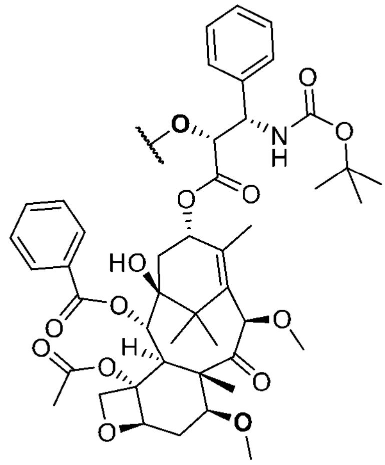,

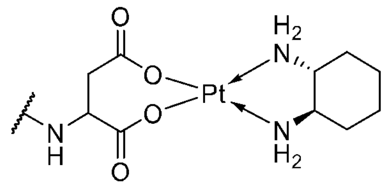, or 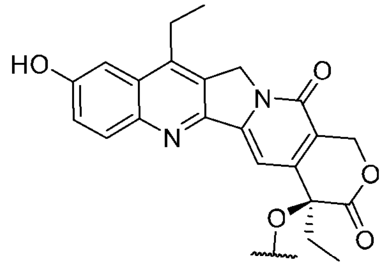.

In some examples, the compound of formula (I-1) is a compound of formula (A) or formula (B):

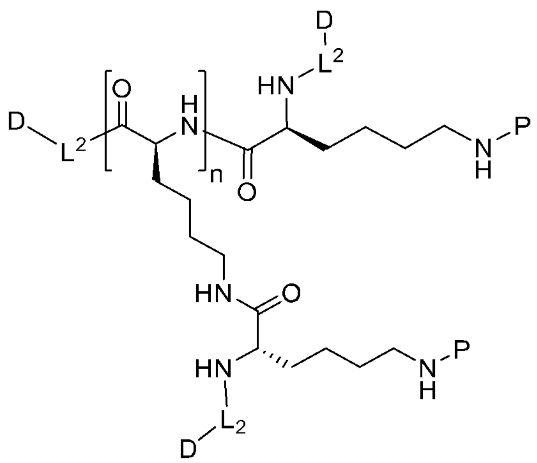
(A)

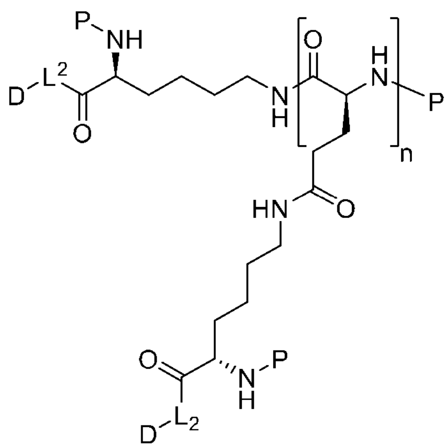
(B)

;

wherein the variables are as defined in any one of the above embodiments.

The present disclosure further provides a compound of any one of the following structures:

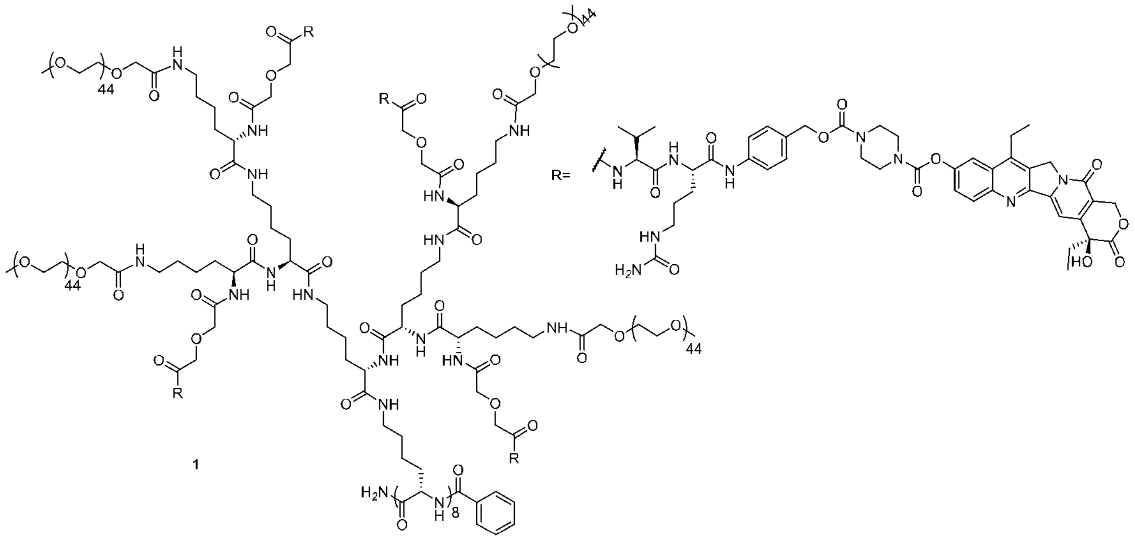

1

,

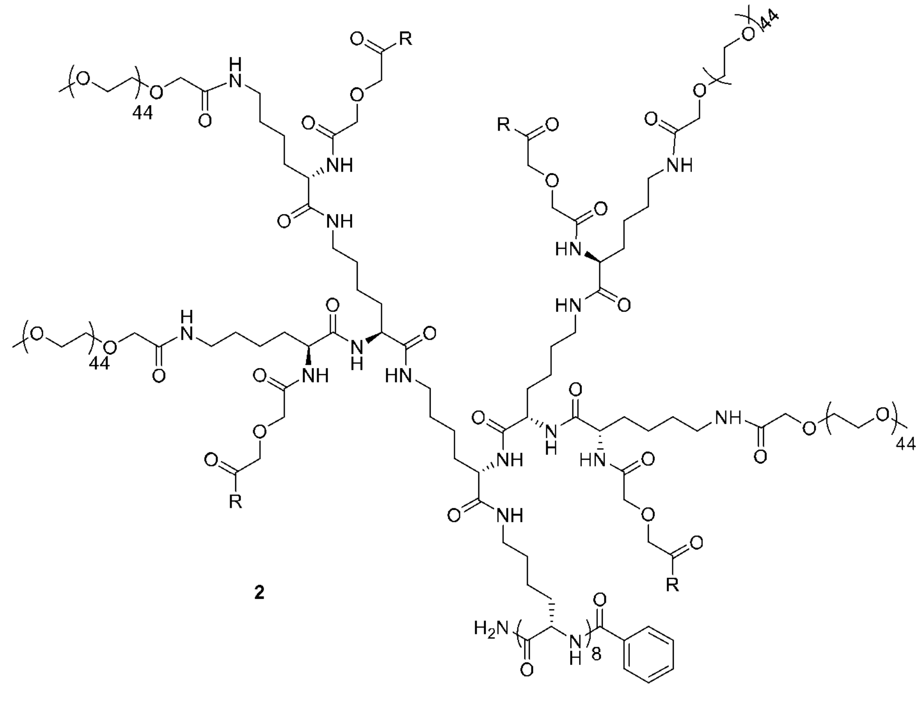

2

R= 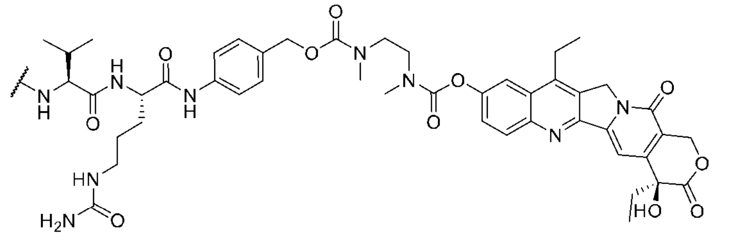

,

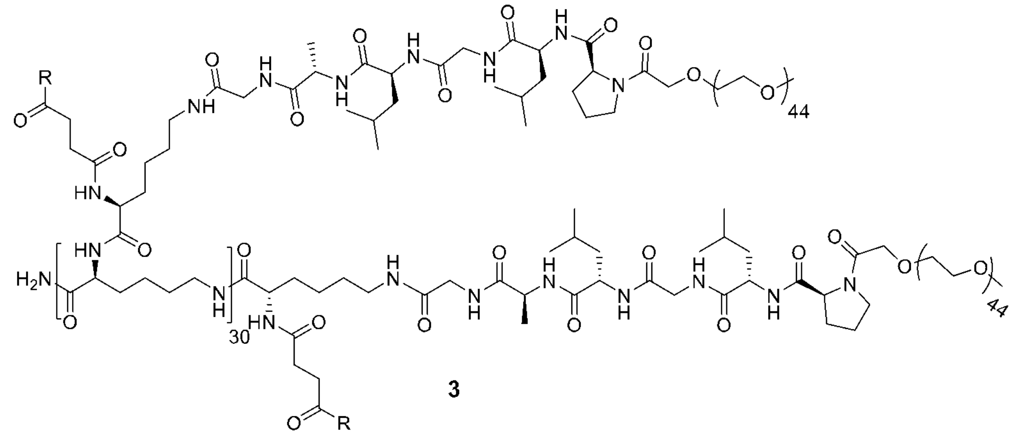

3

R = 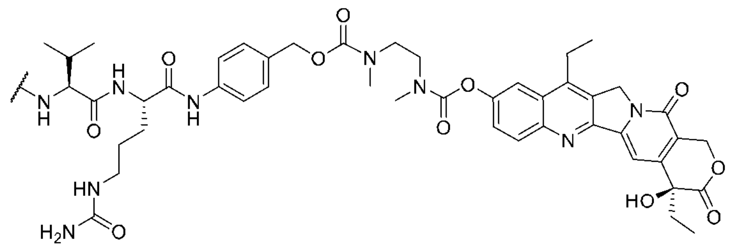

,

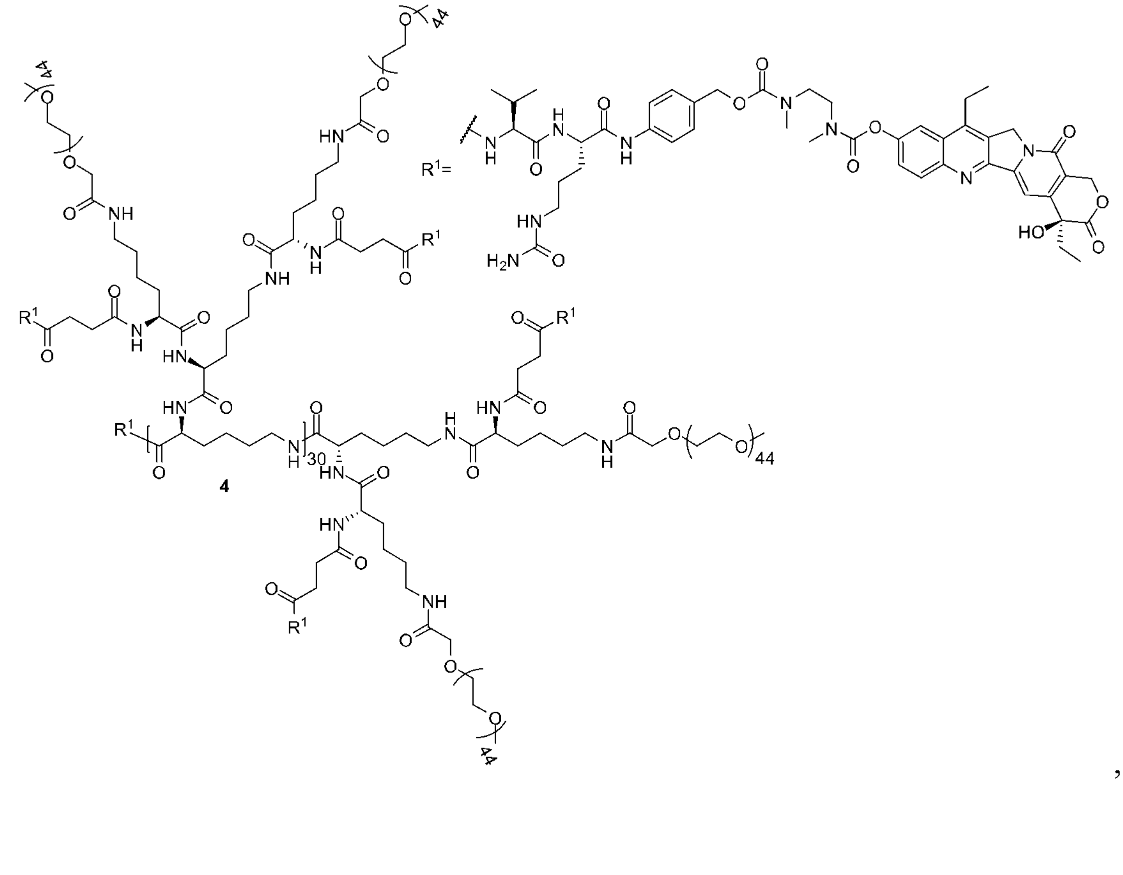

$R^1$=

4

,

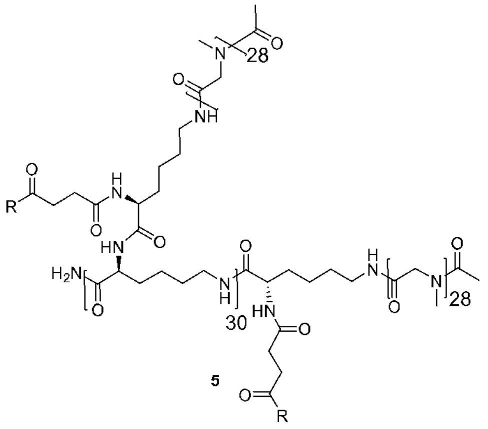

5

R =

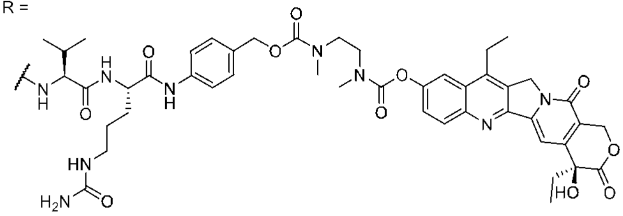

,

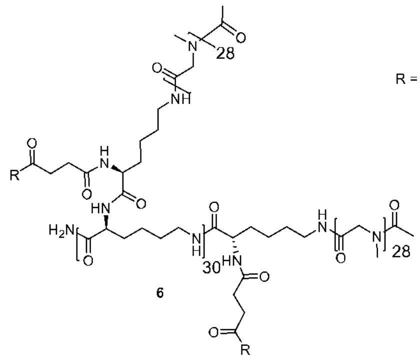

6

R =

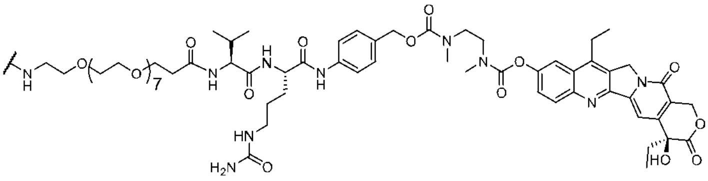

,

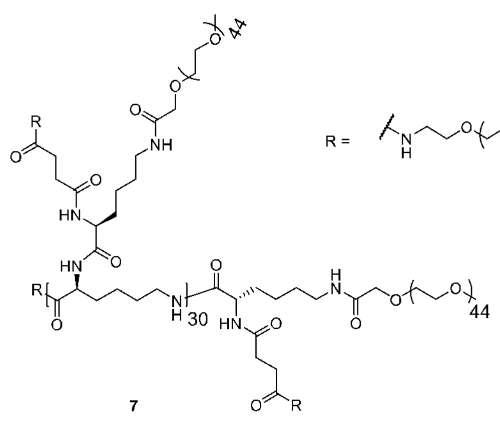

7

R =

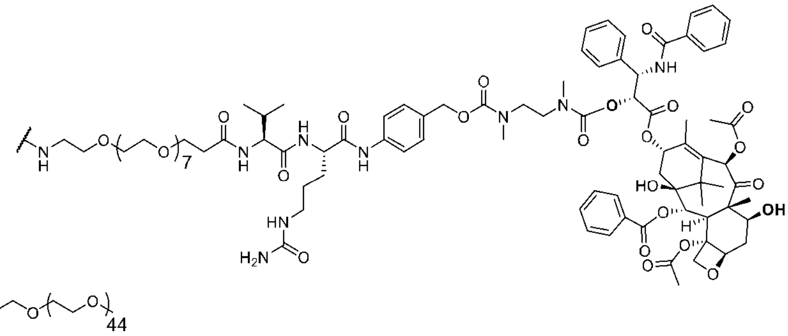

,

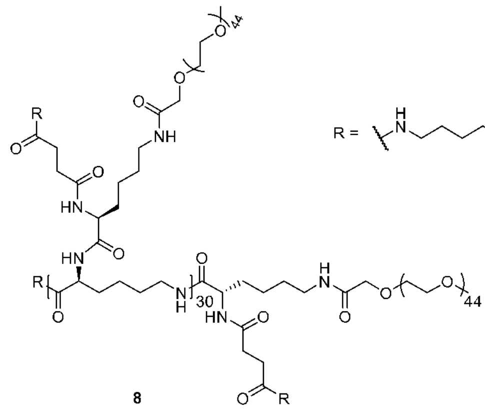

8

R =

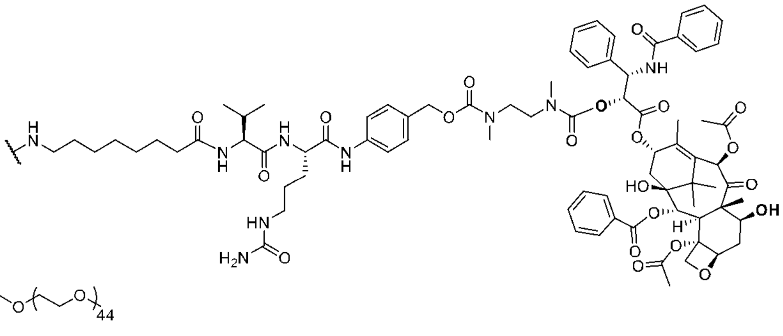

,

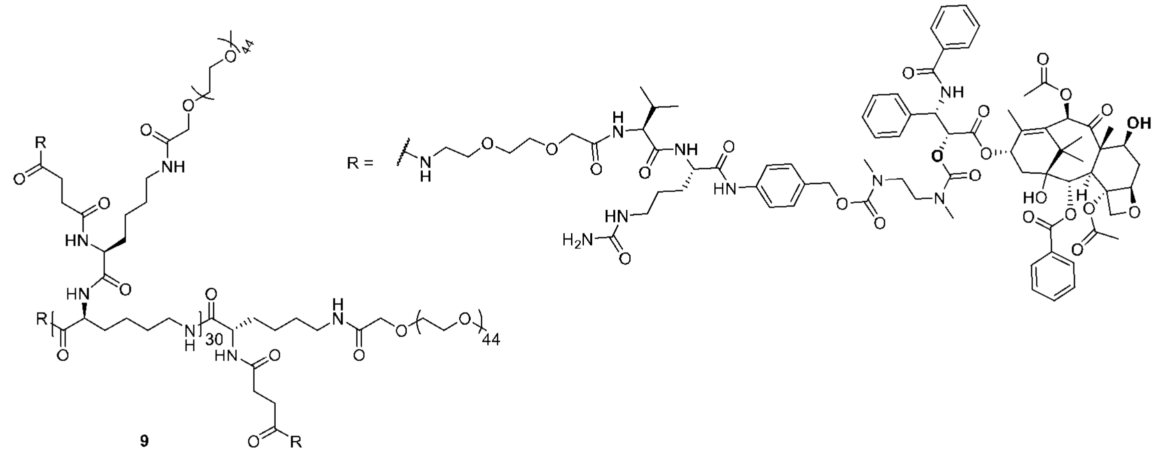
R =

9

,

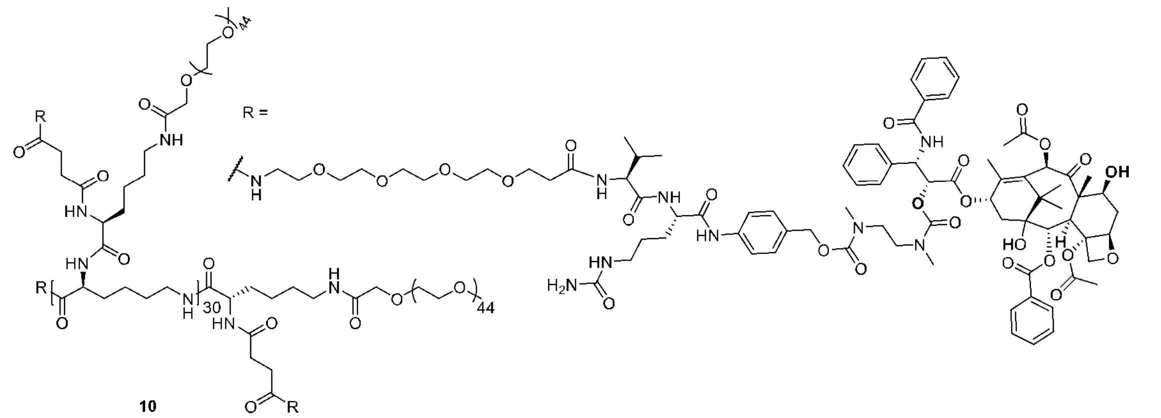
R =

10

,

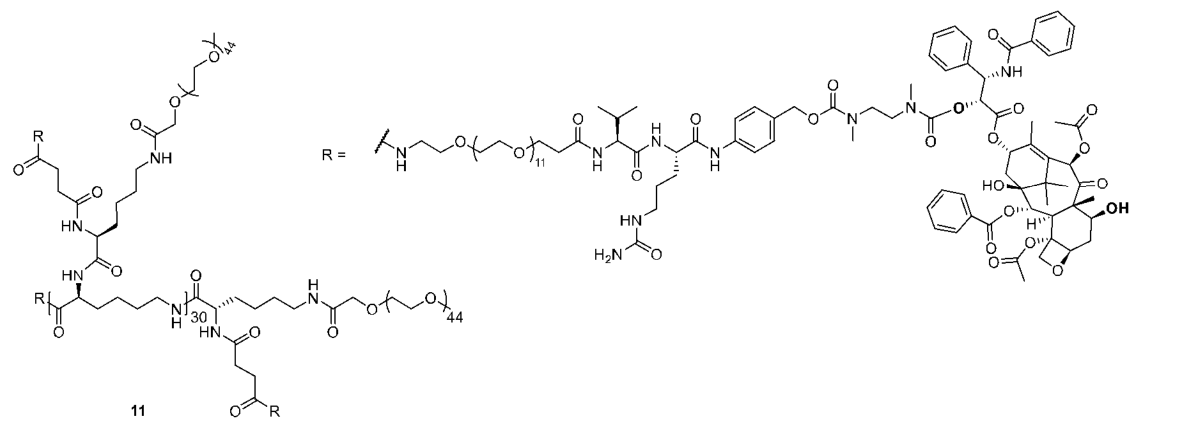
R =

11

,

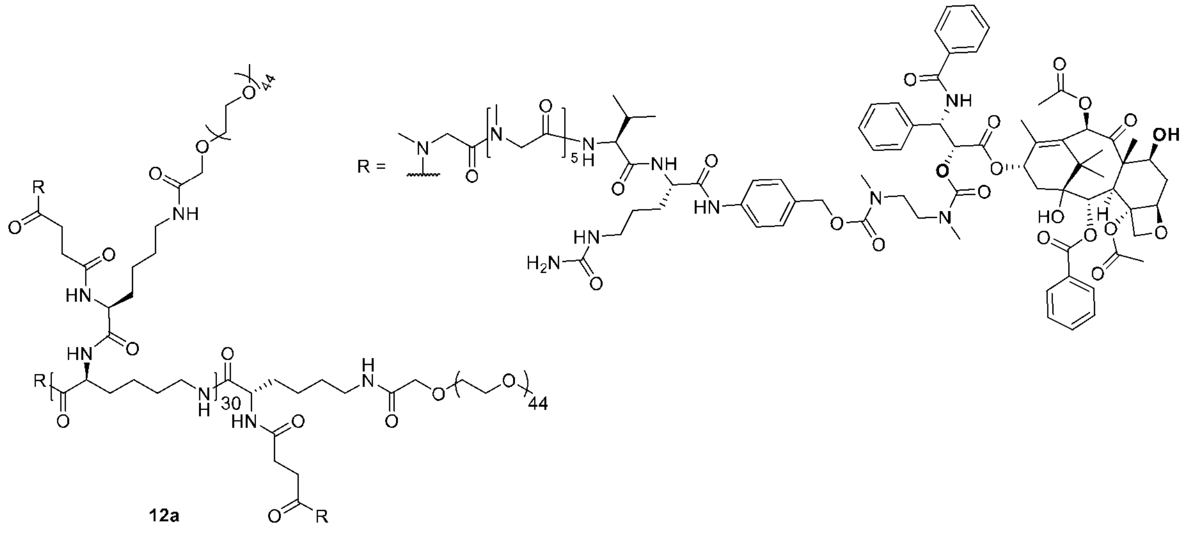

12a

,

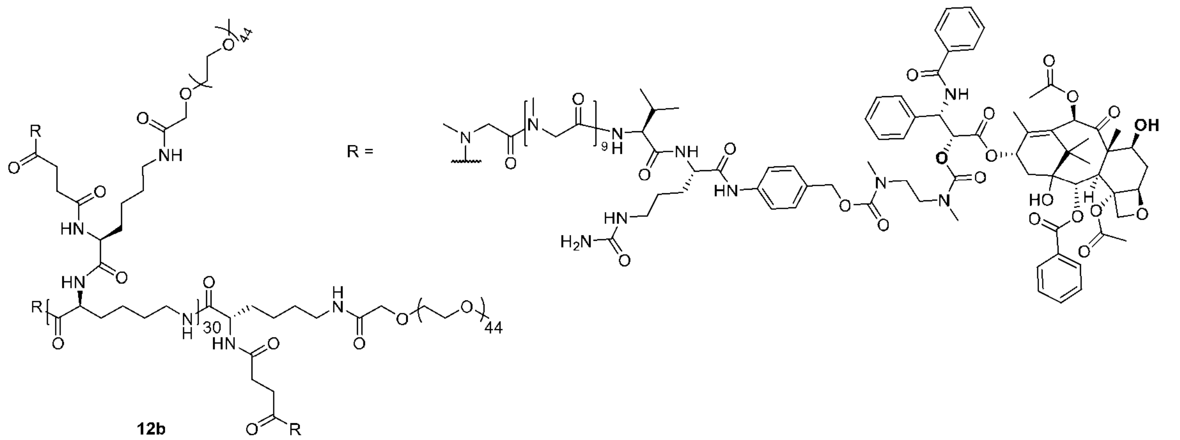

12b

,

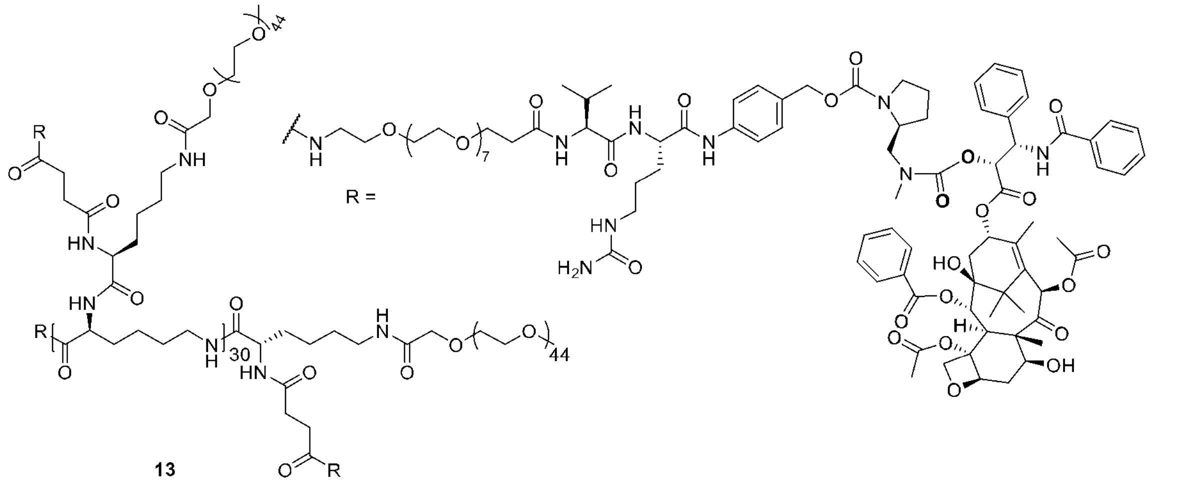

13

,

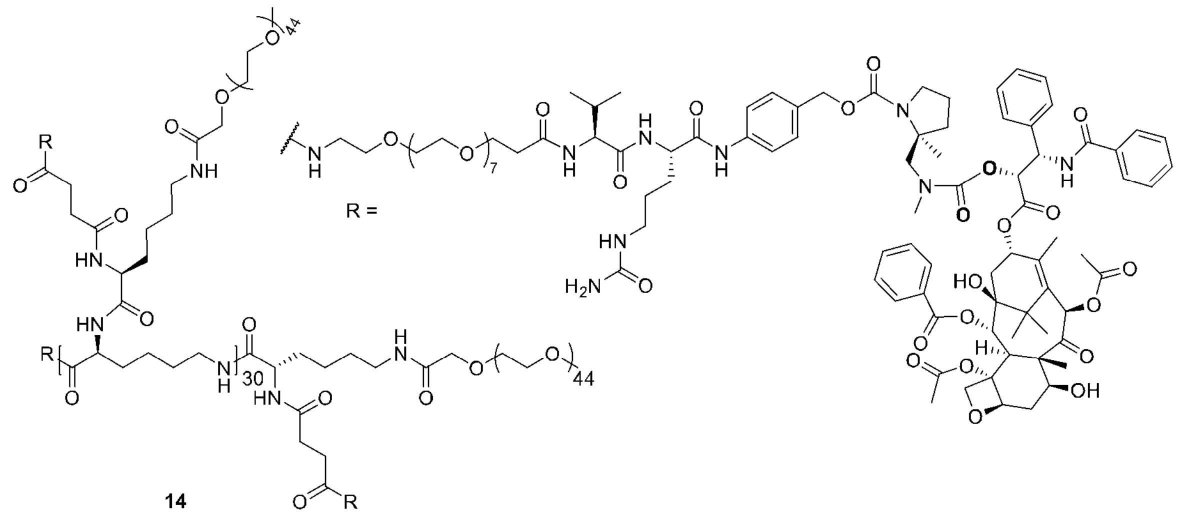

R =

14

,

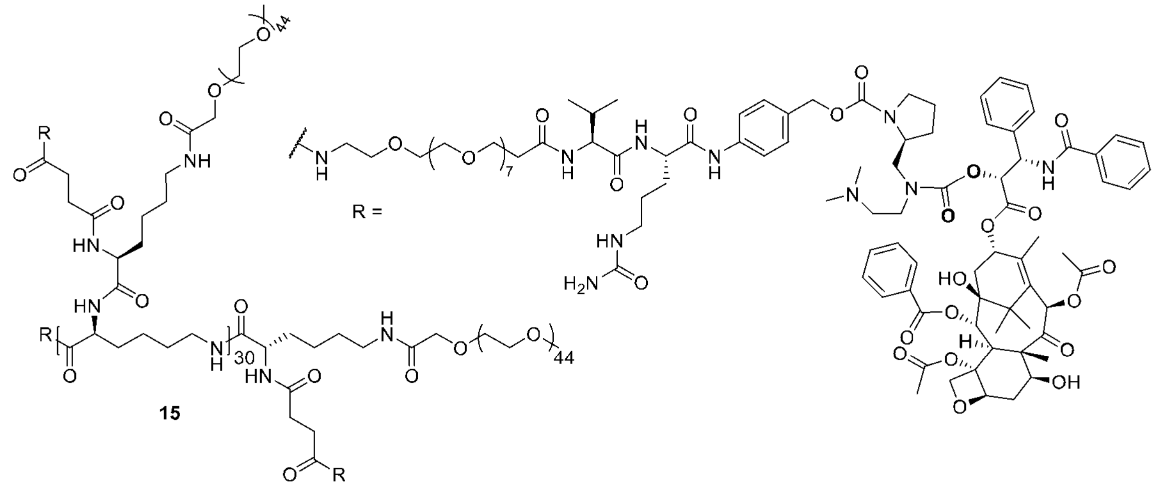

R =

15

,

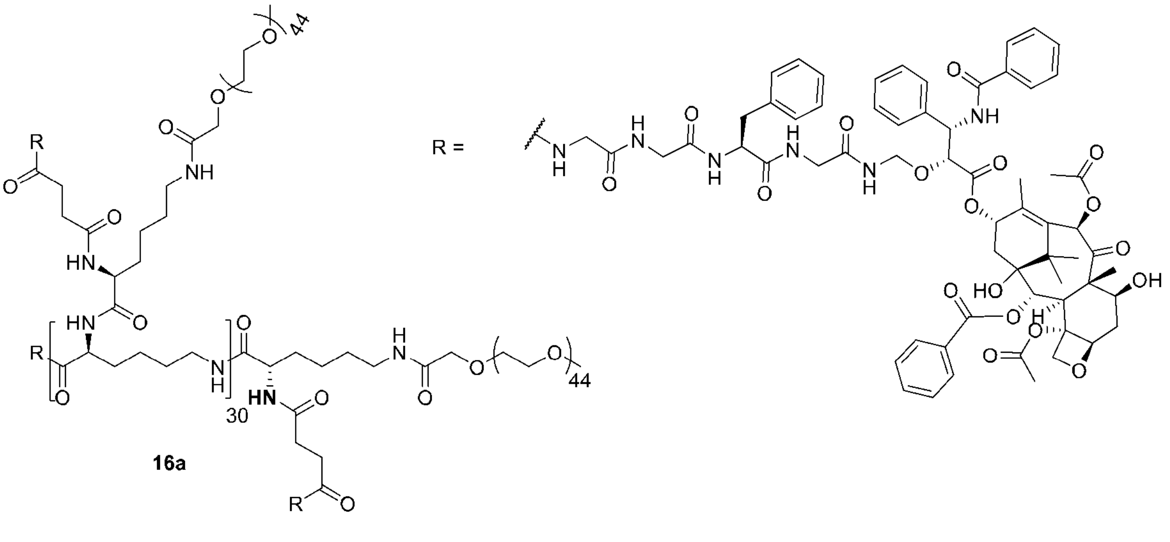

R =

16a

,

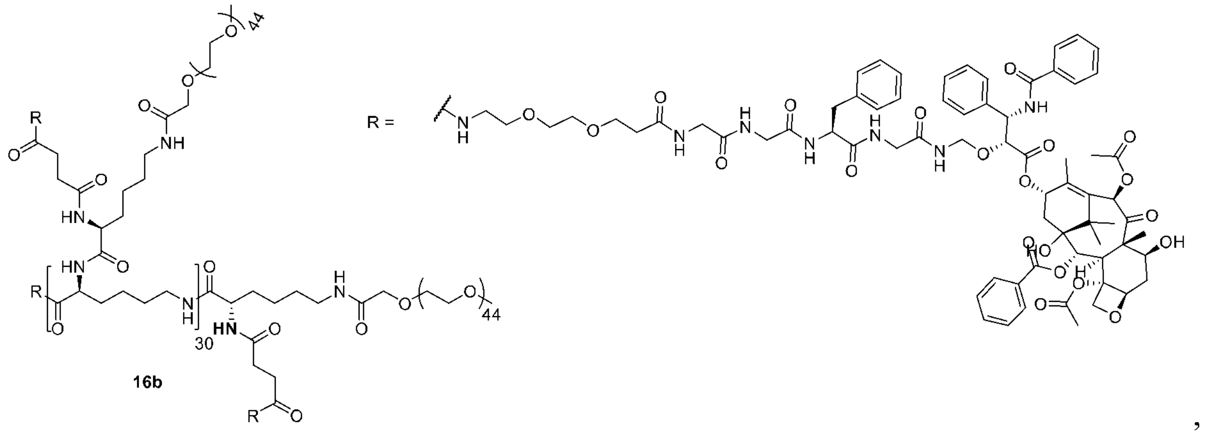

16b

R =

,

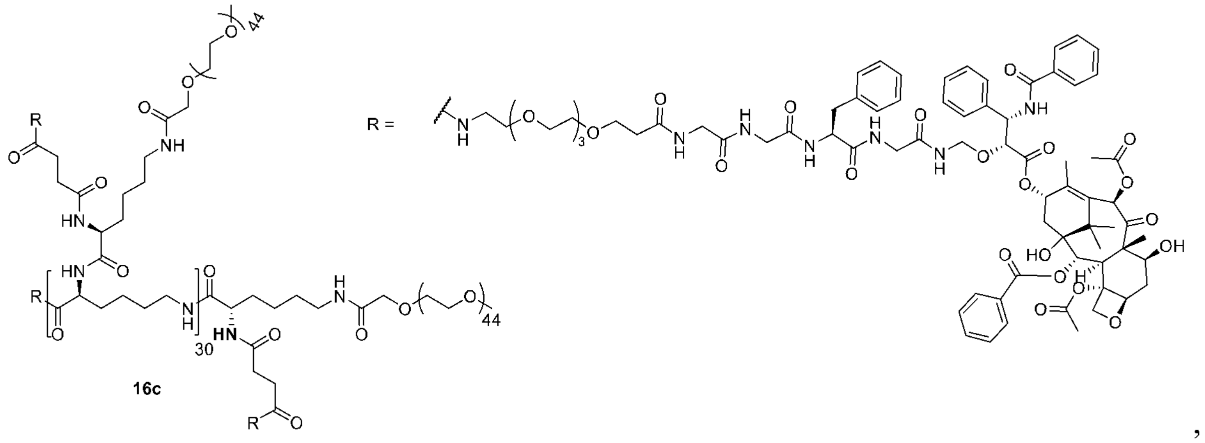

16c

R =

,

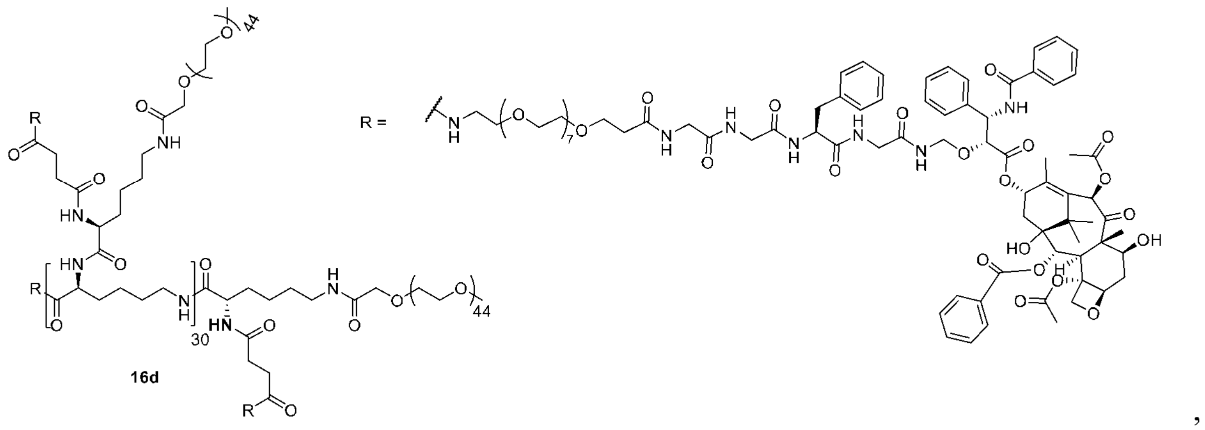

16d

R =

,

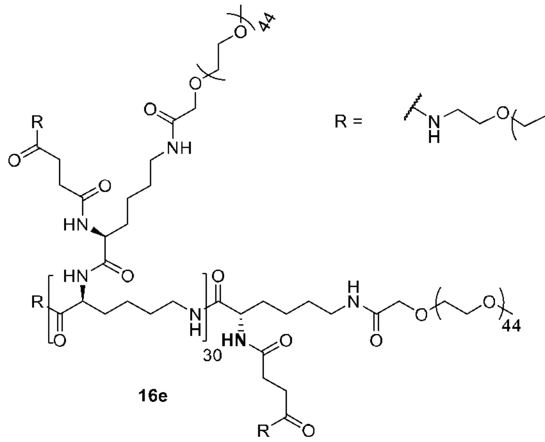

R =

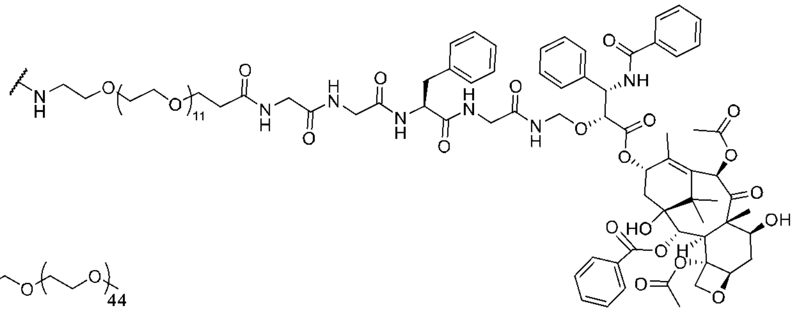

16e

,

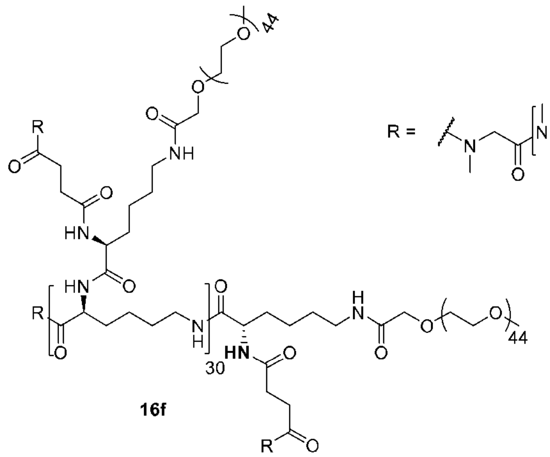

R =

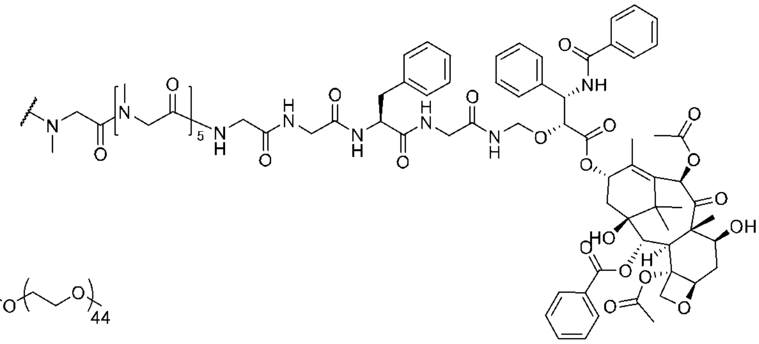

16f

,

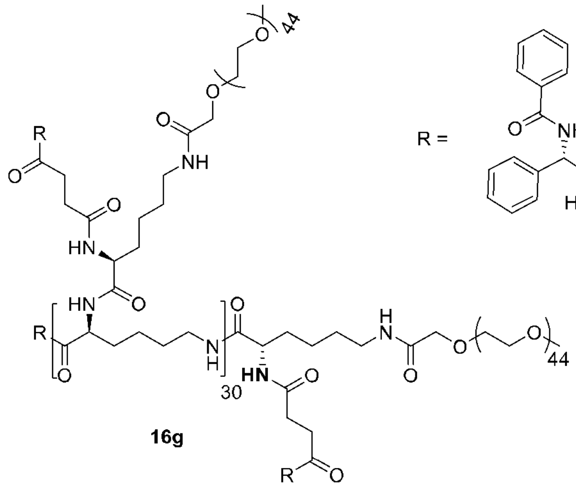

R =

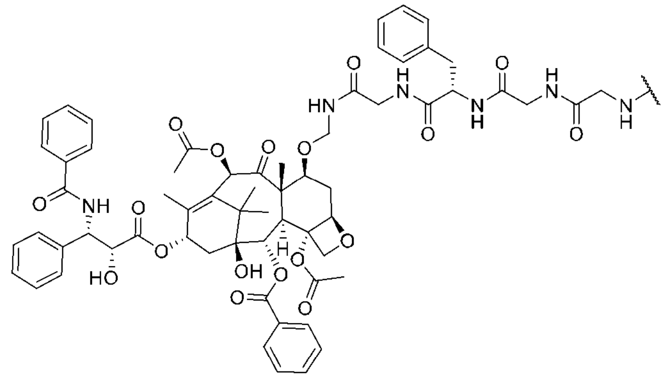

16g

,

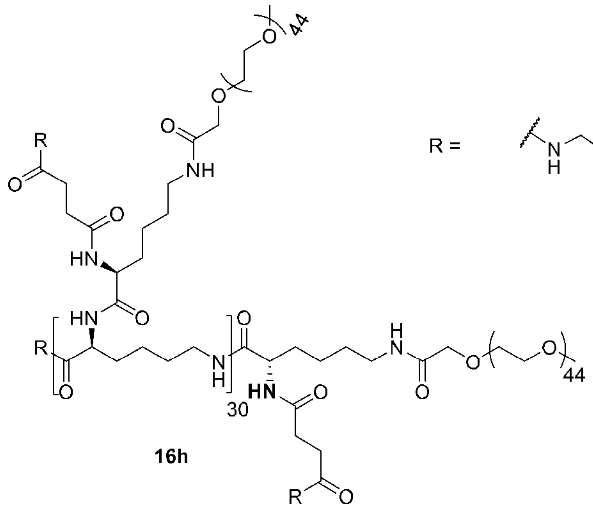

16h

R =

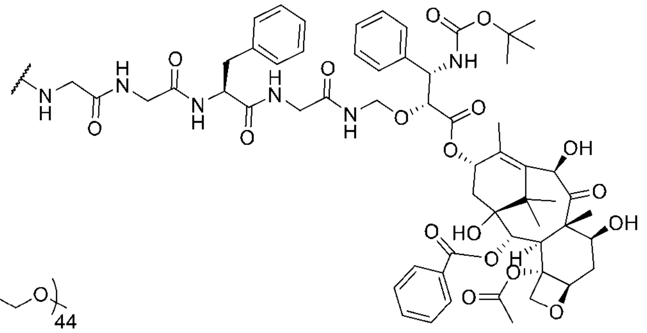

,

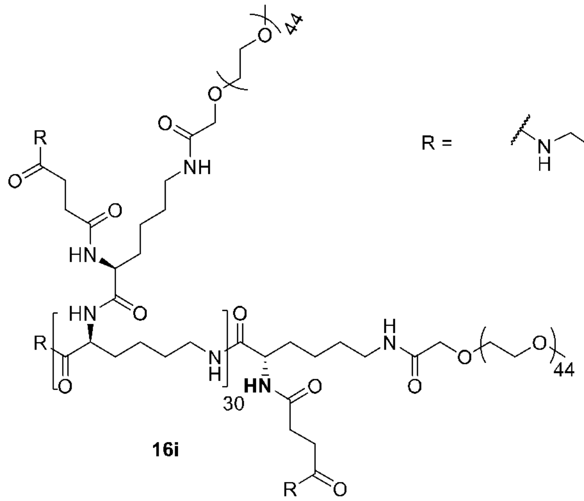

16i

R =

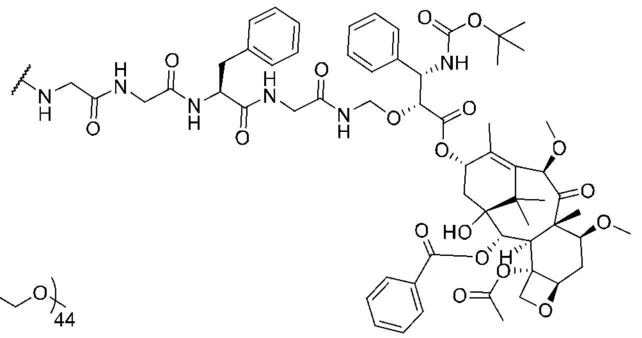

,

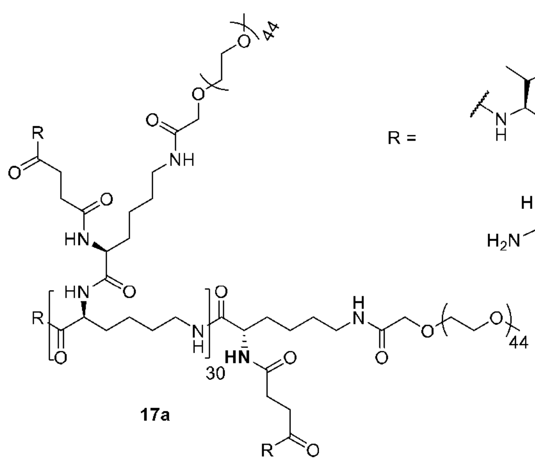

17a

R =

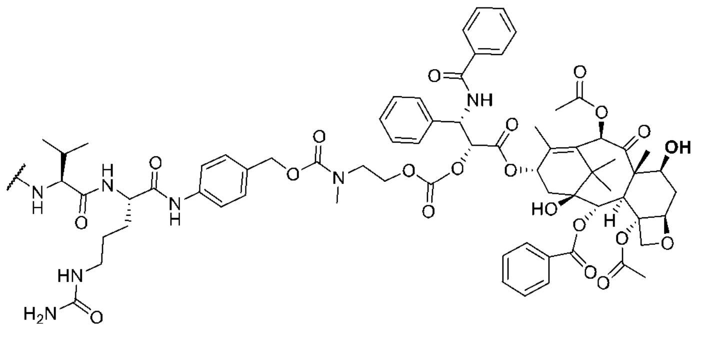

,

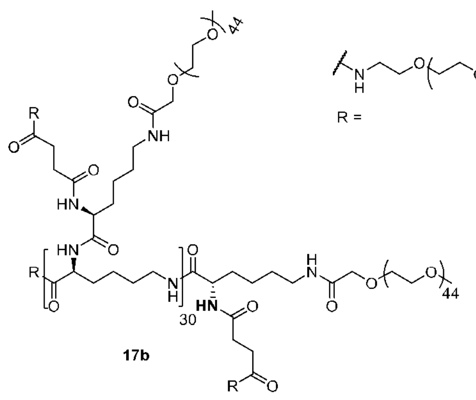

17b

R = 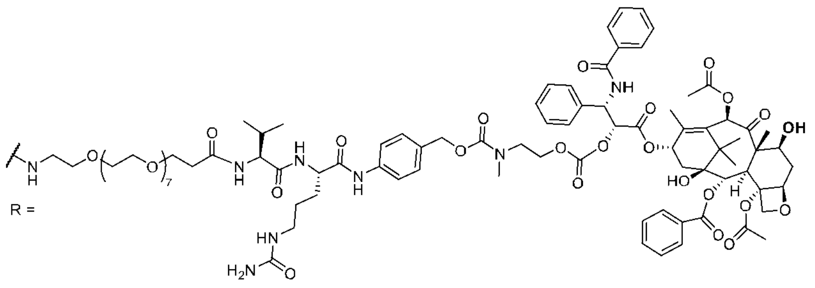

,

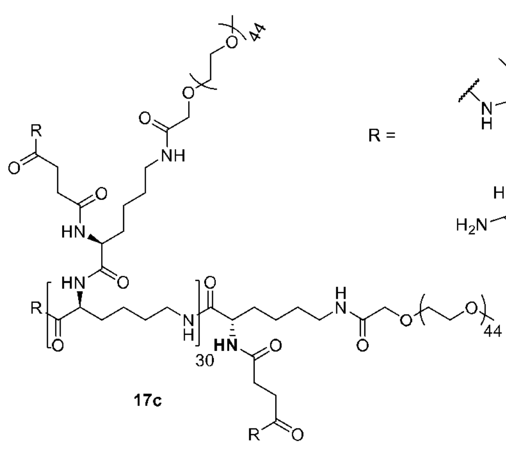

17c

R = 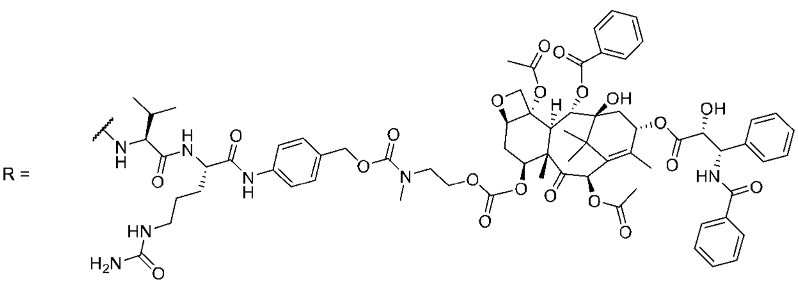

,

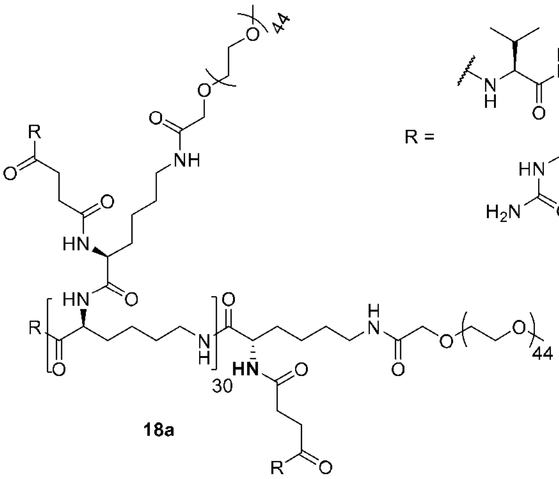

18a

R = 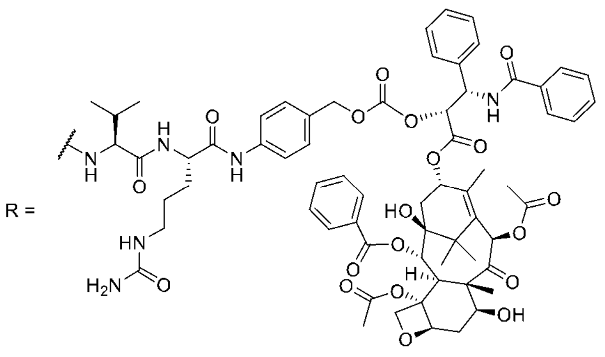

,

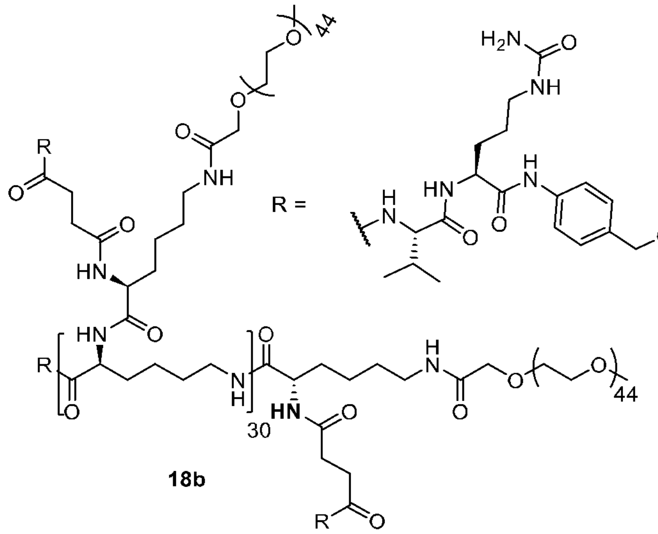

R =

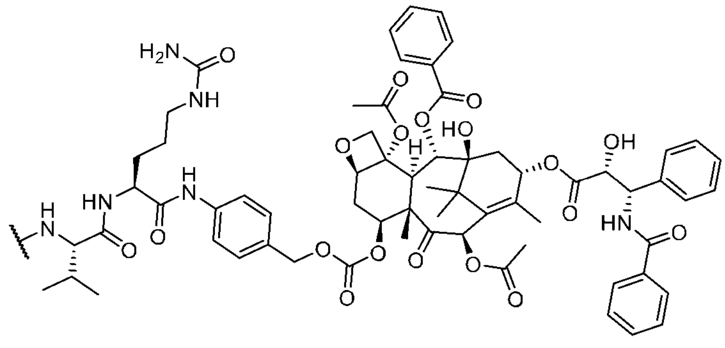

18b

,

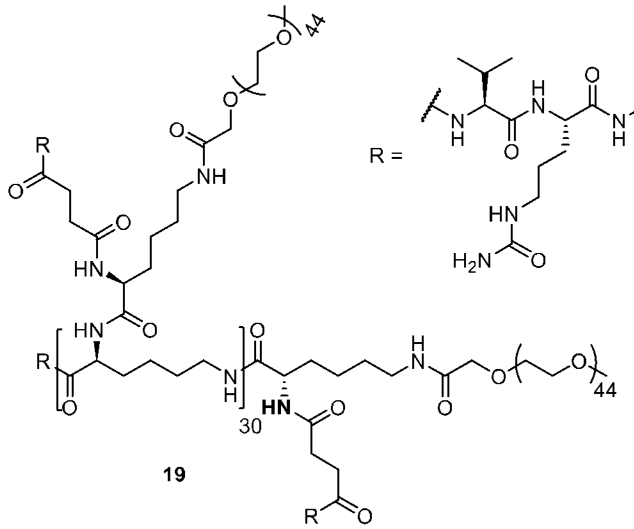

R =

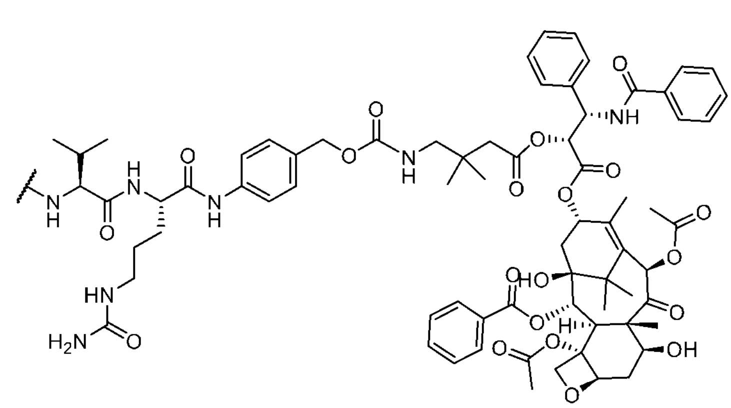

19

,

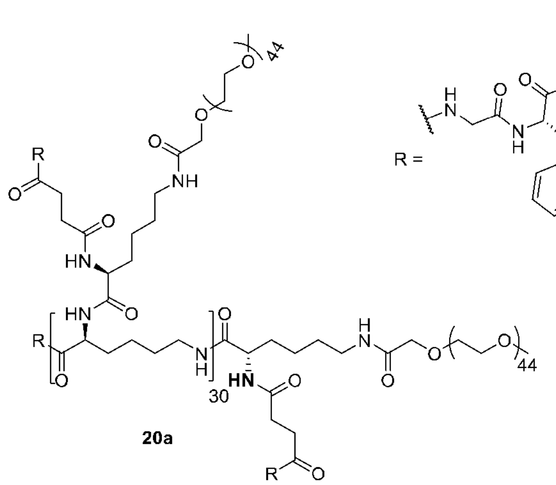

R =

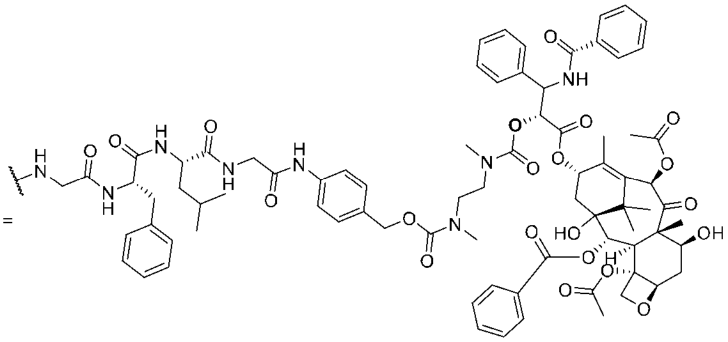

20a

,

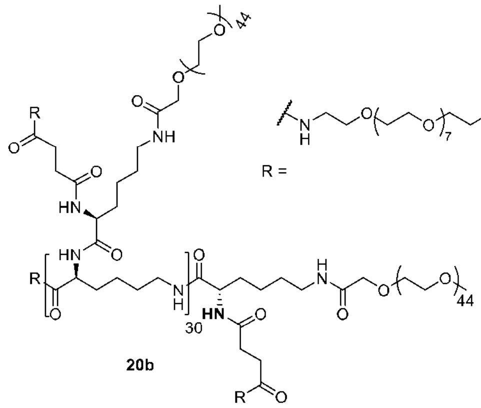

R =

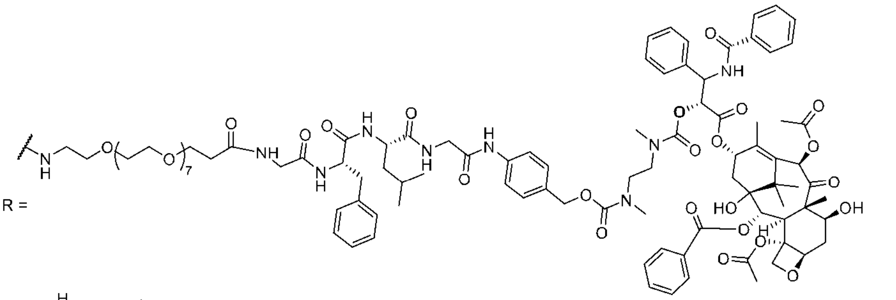

20b

,

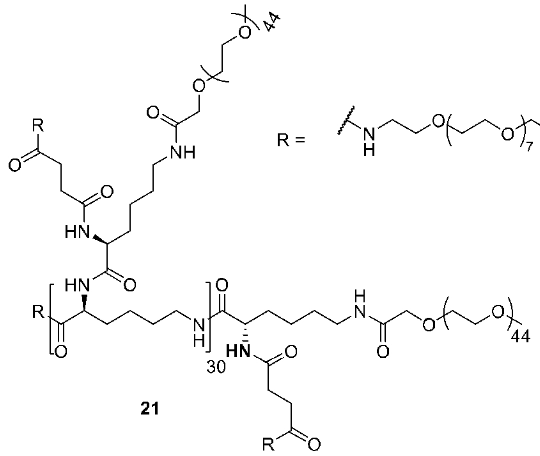

R =

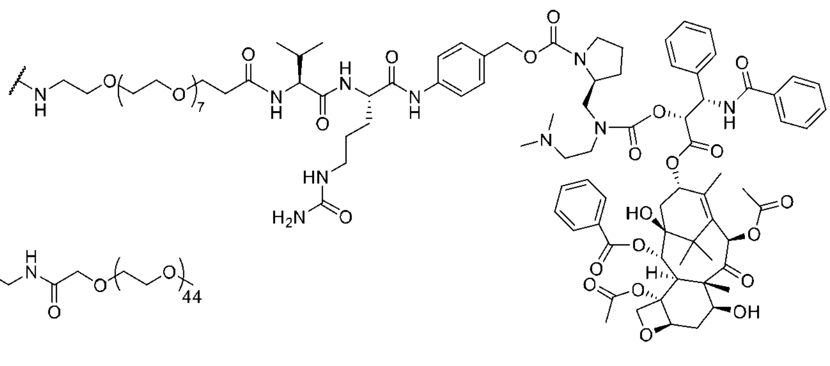

21

,

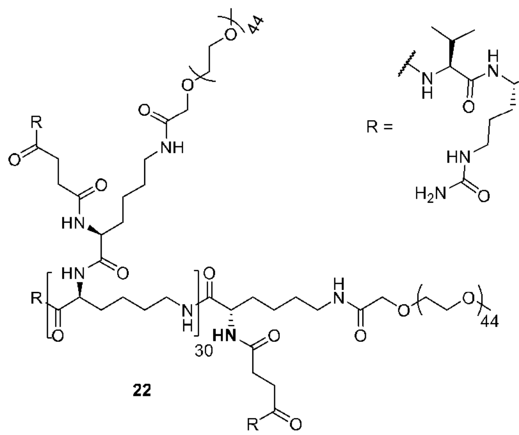

R =

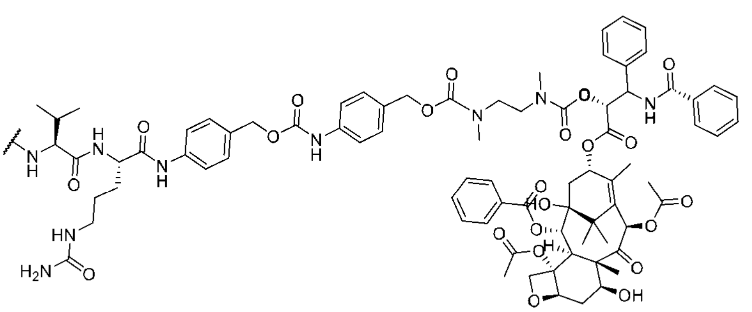

22

,

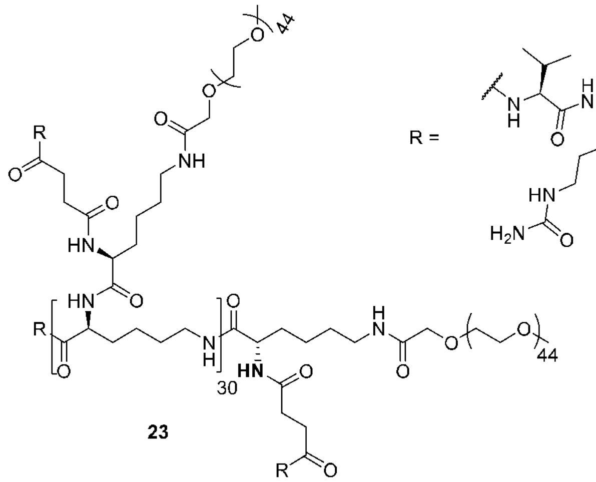

R =

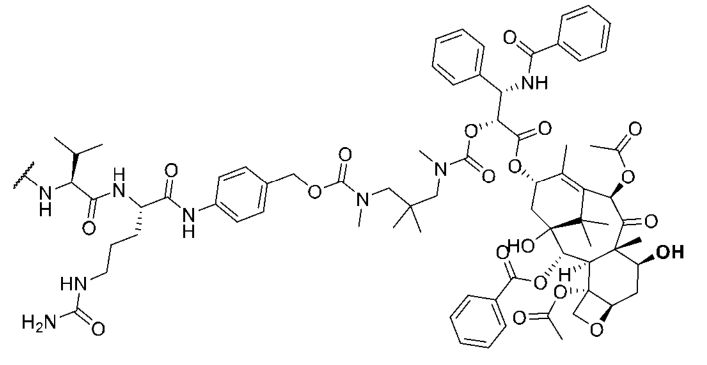

**23**

,

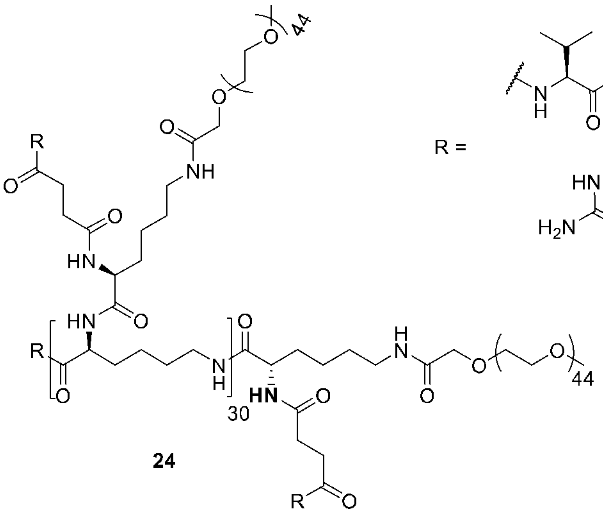

R =

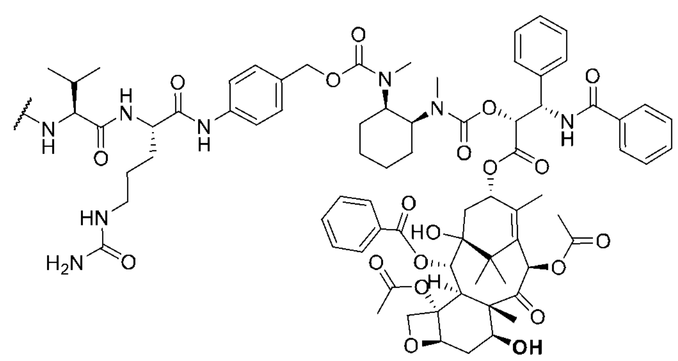

**24**

,

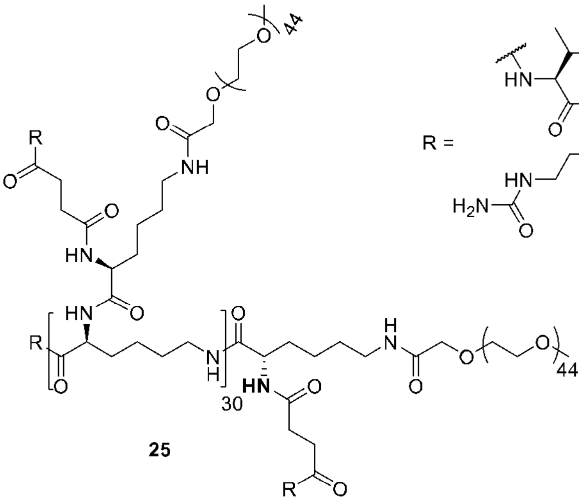

R =

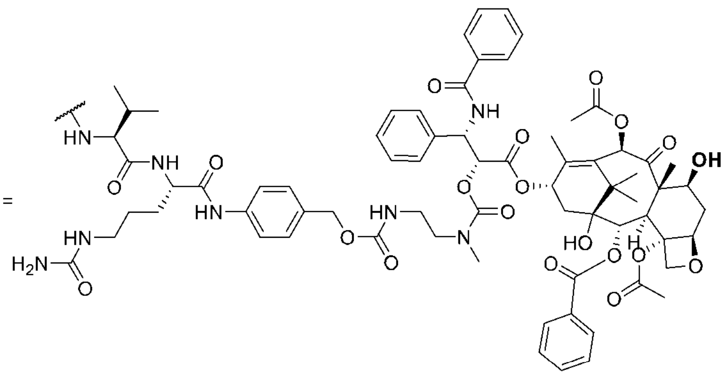

**25**

,

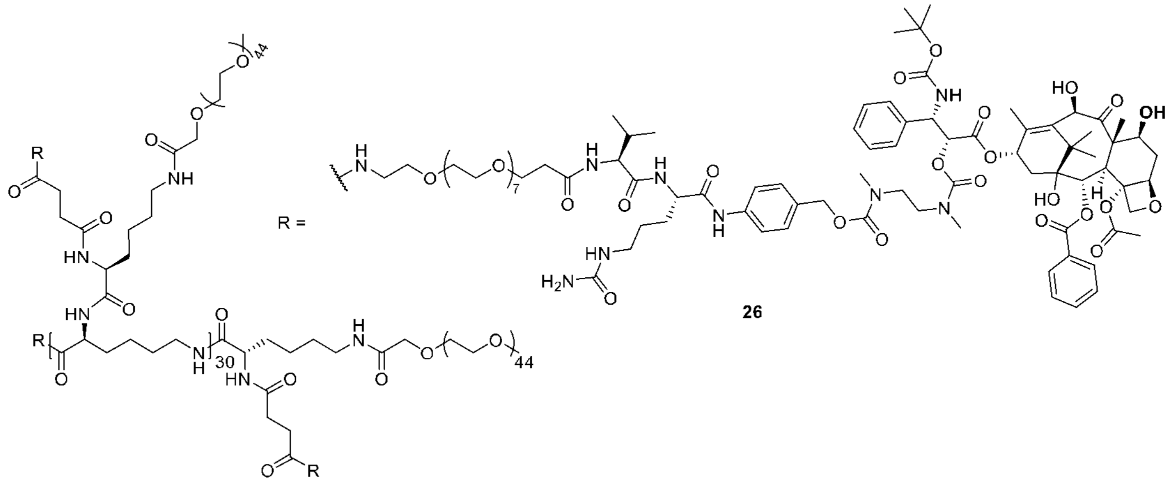

26

,

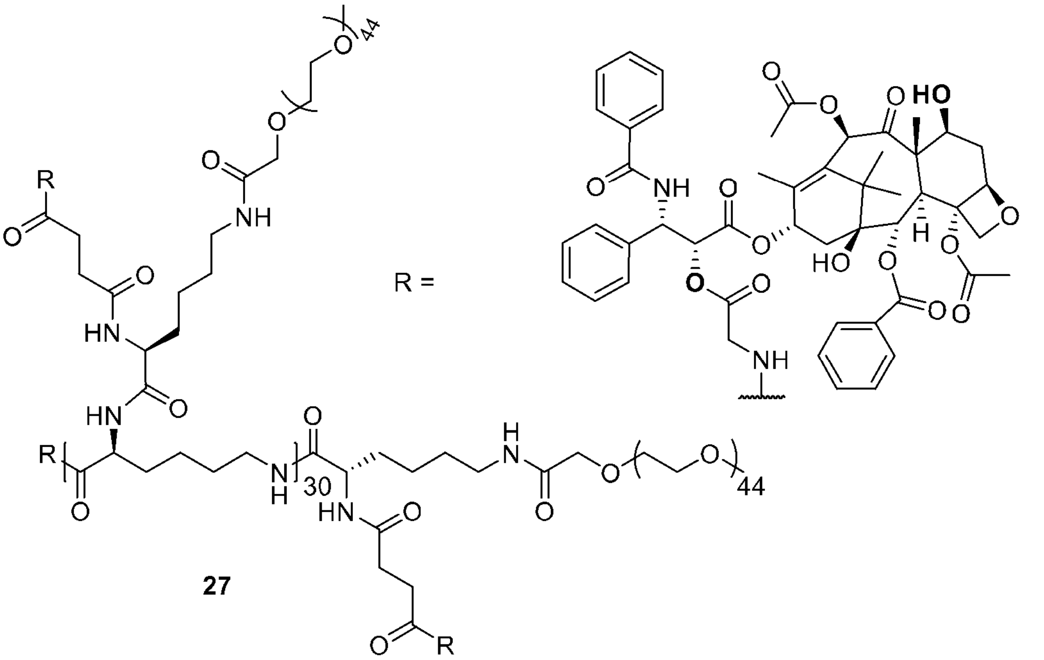

27

,

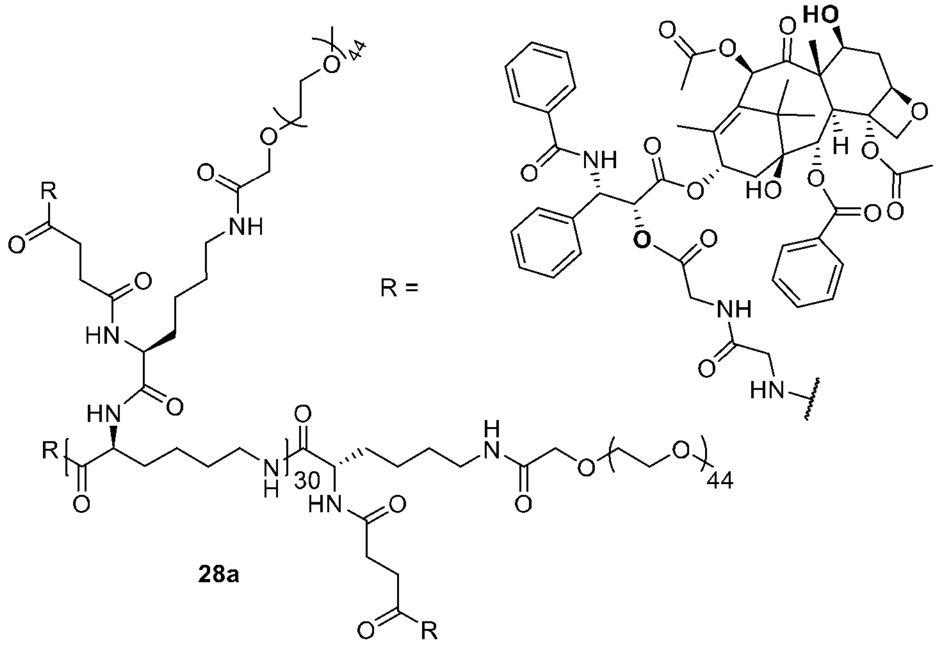

R =

28a

,

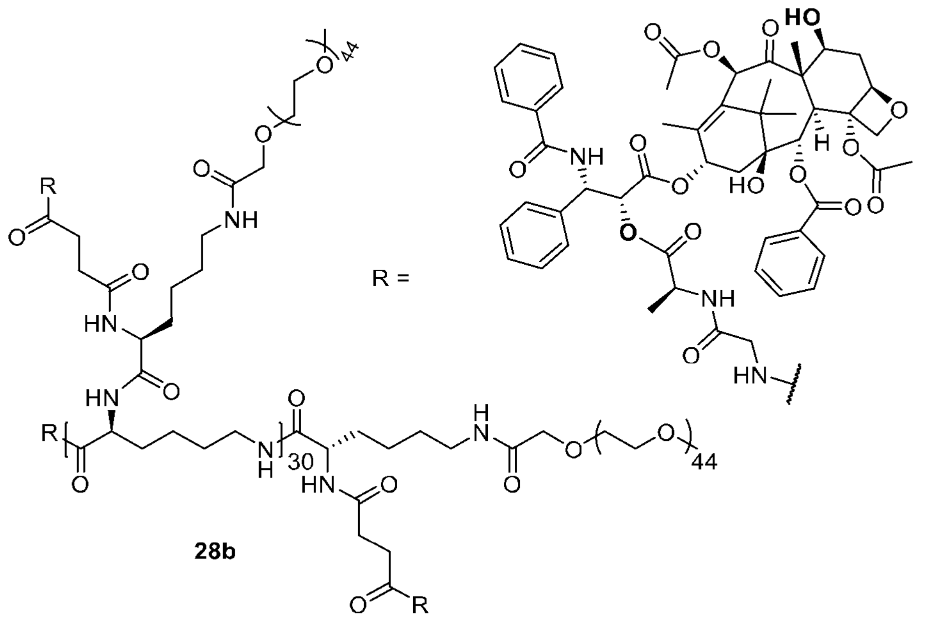

R =

28b

,

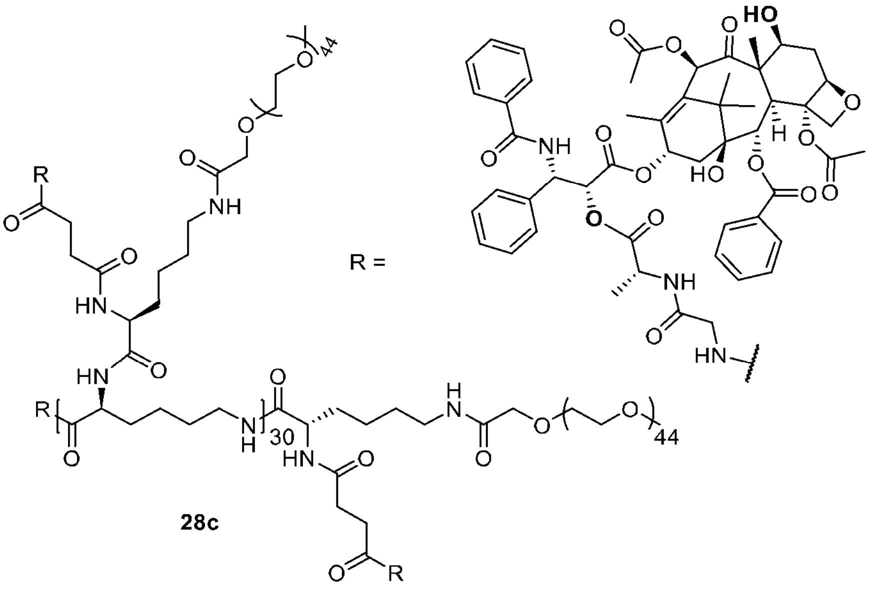

28c

,

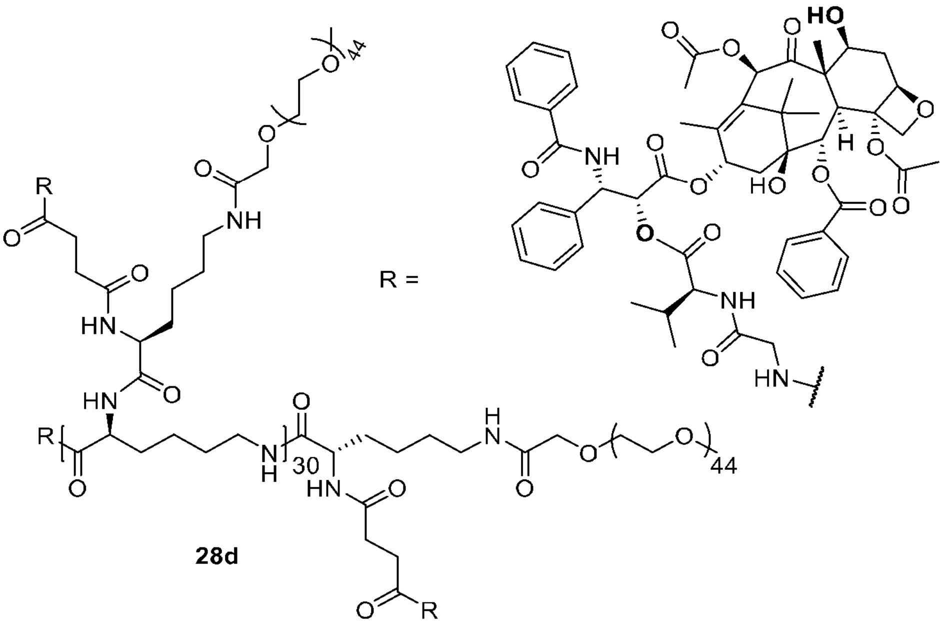

28d

,

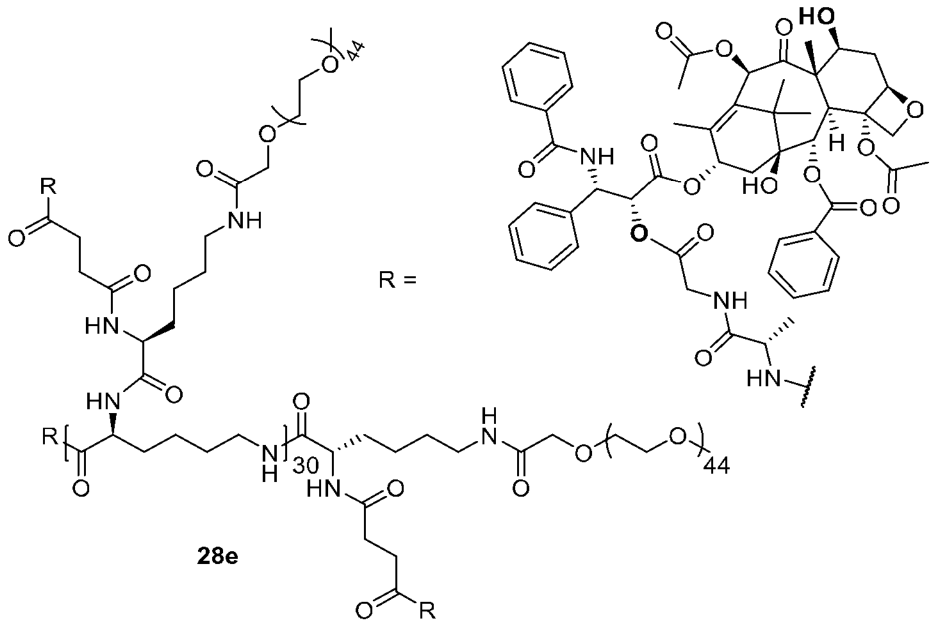

R =

28e

,

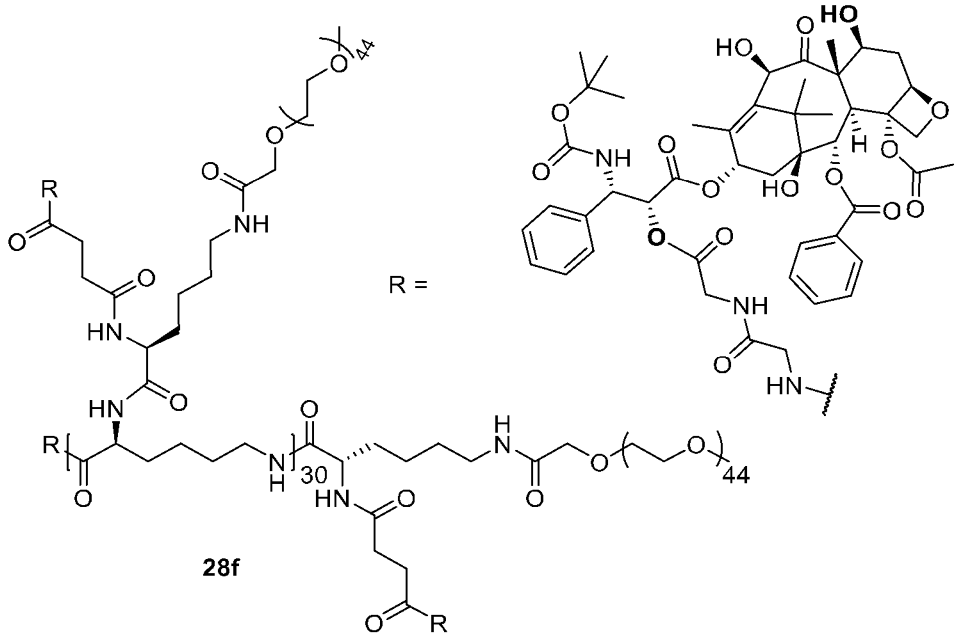

R =

28f

,

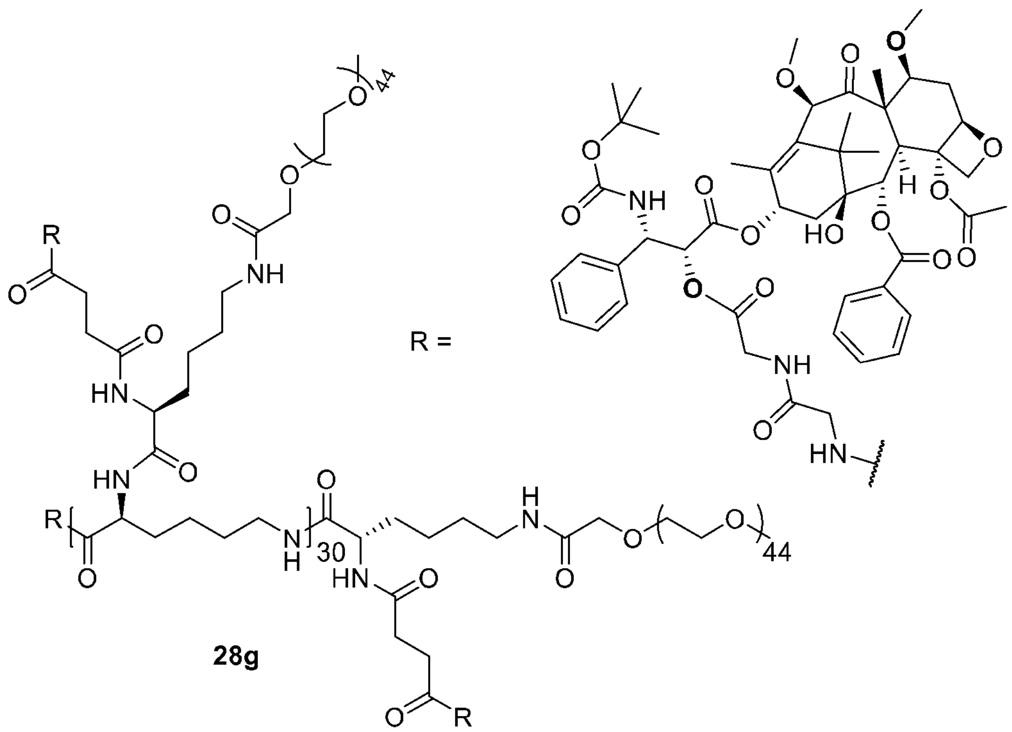

R =

28g

,

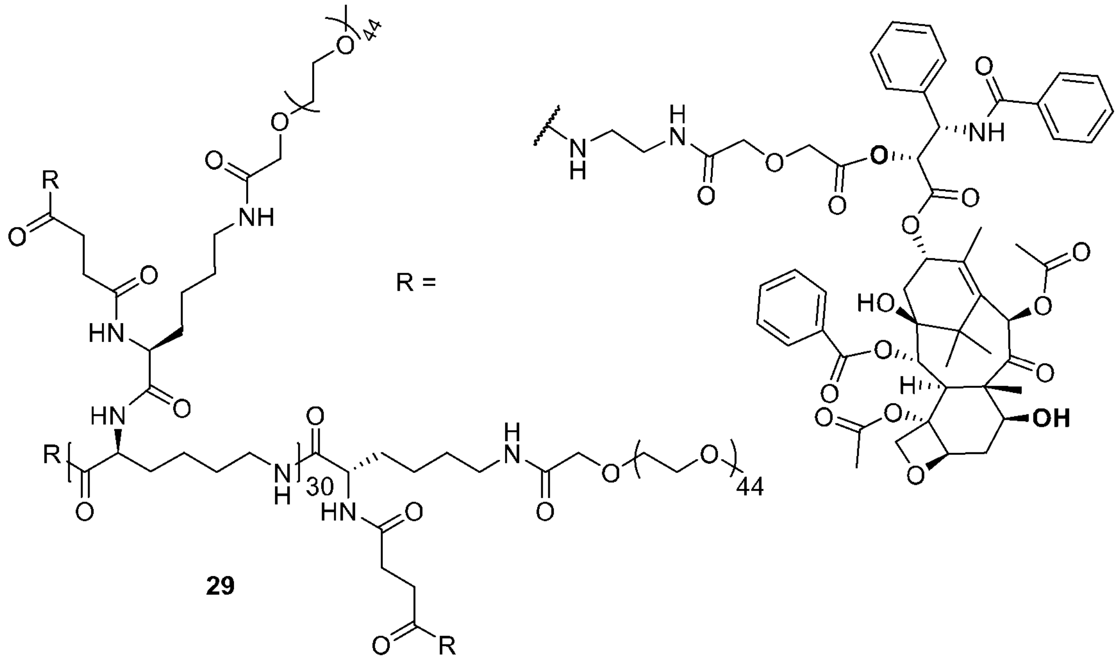

R =

29

,

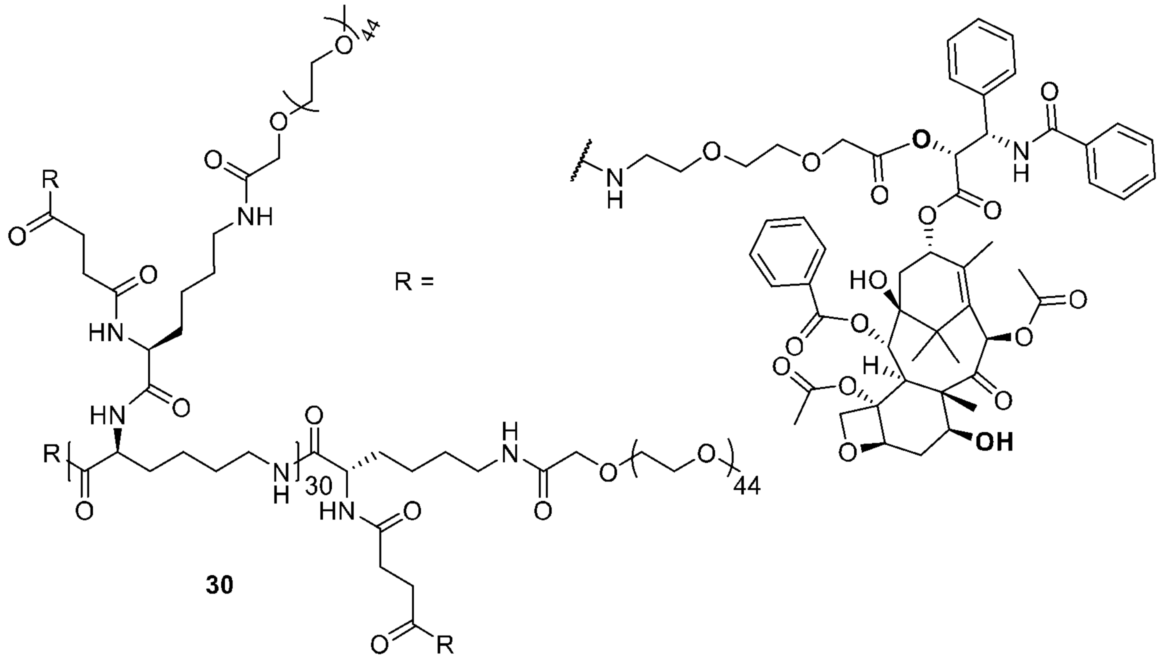

R =

**30**

,

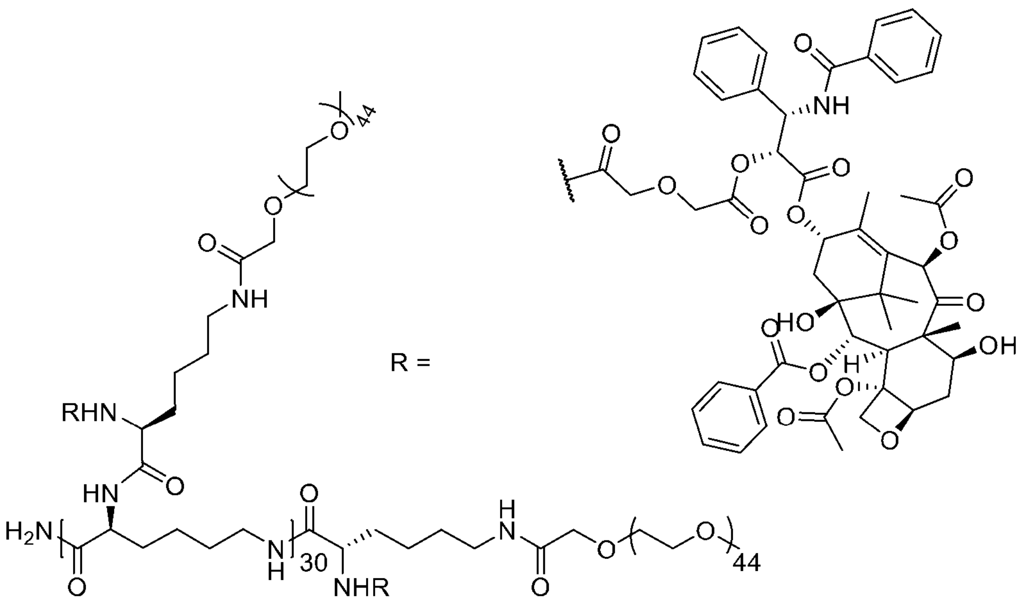

R =

**31**

,

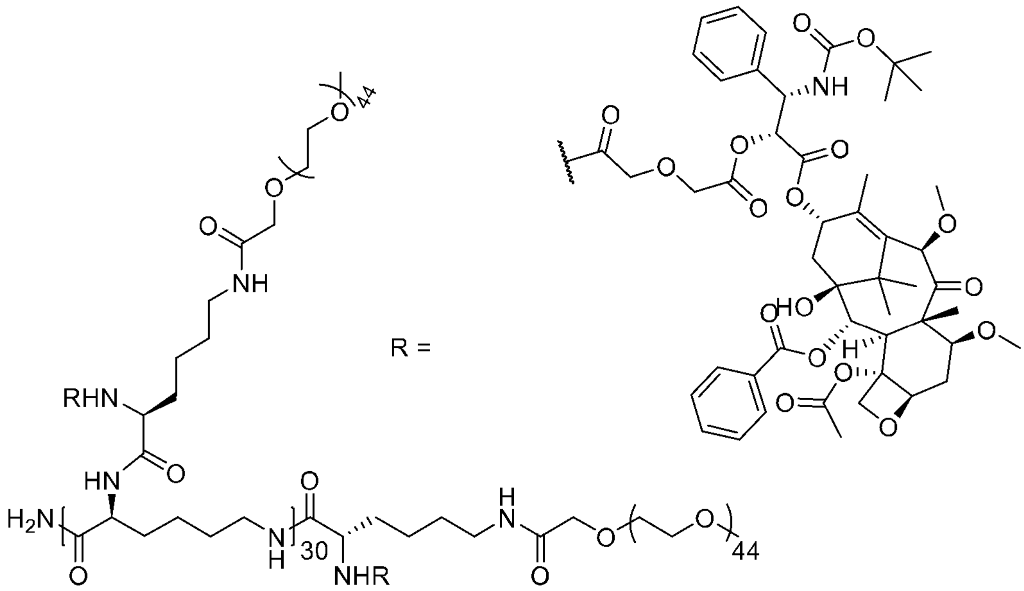

**32**

,

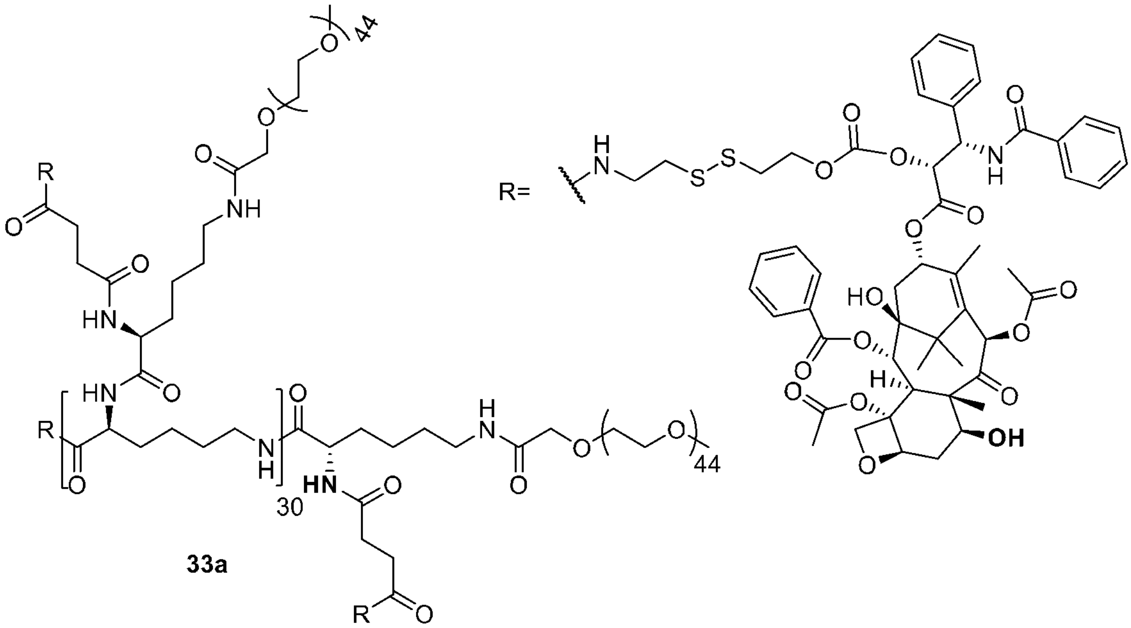

**33a**

,

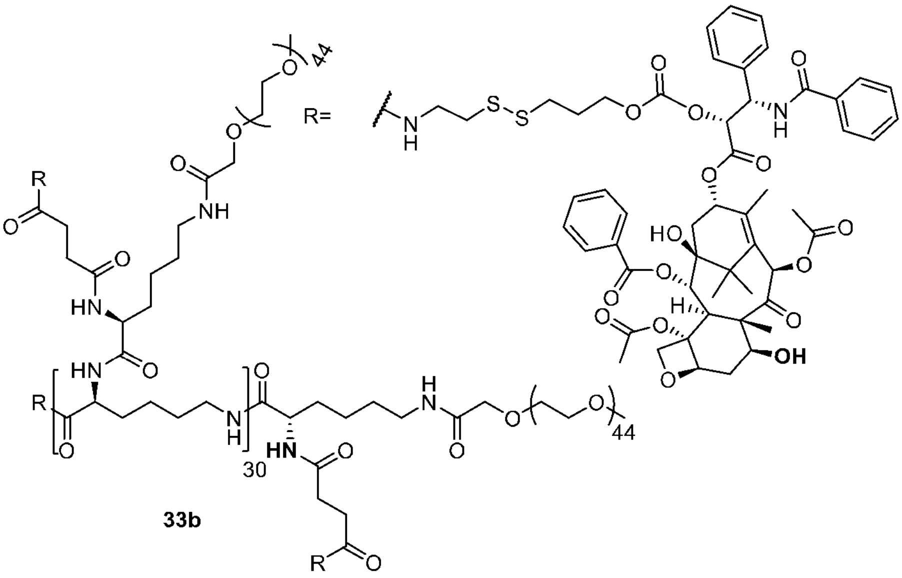

33b

,

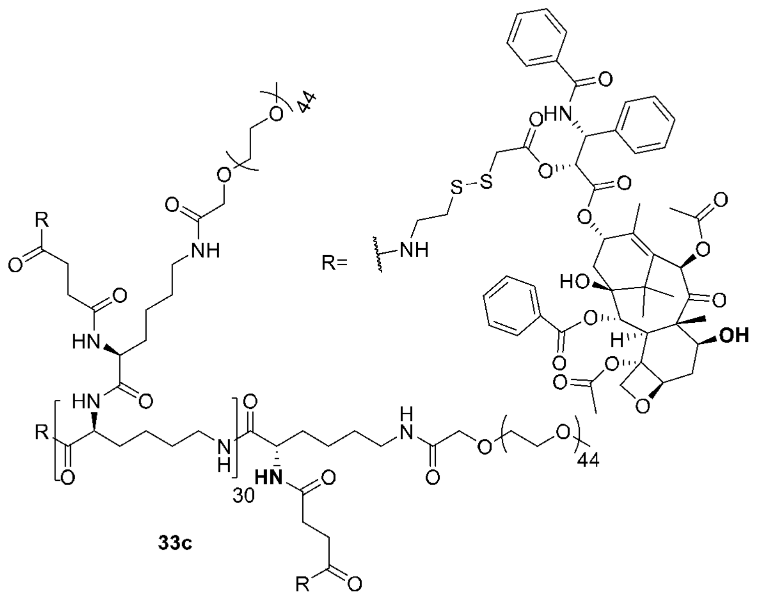

33c

,

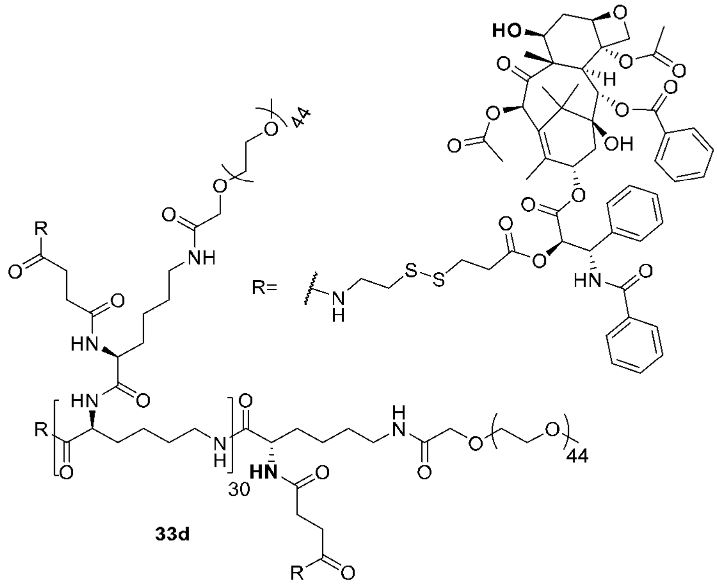

33d

,

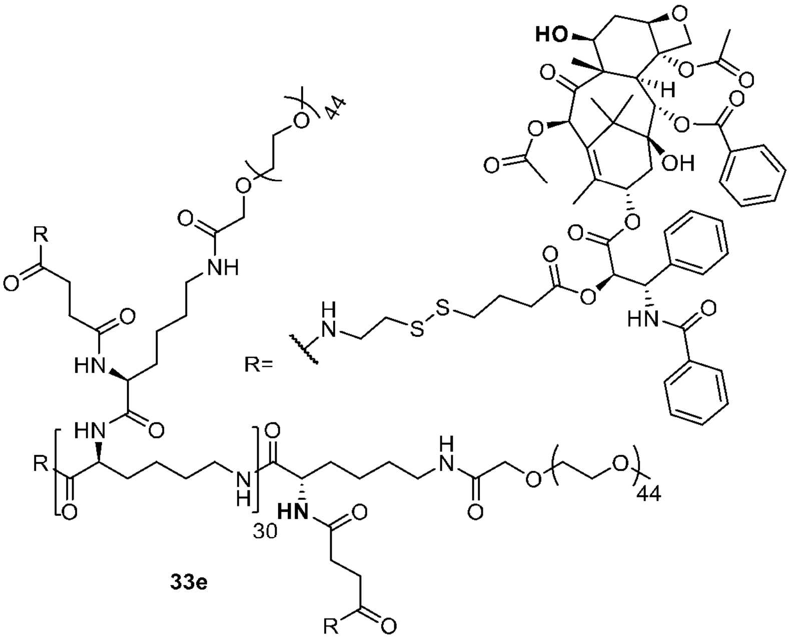

33e

,

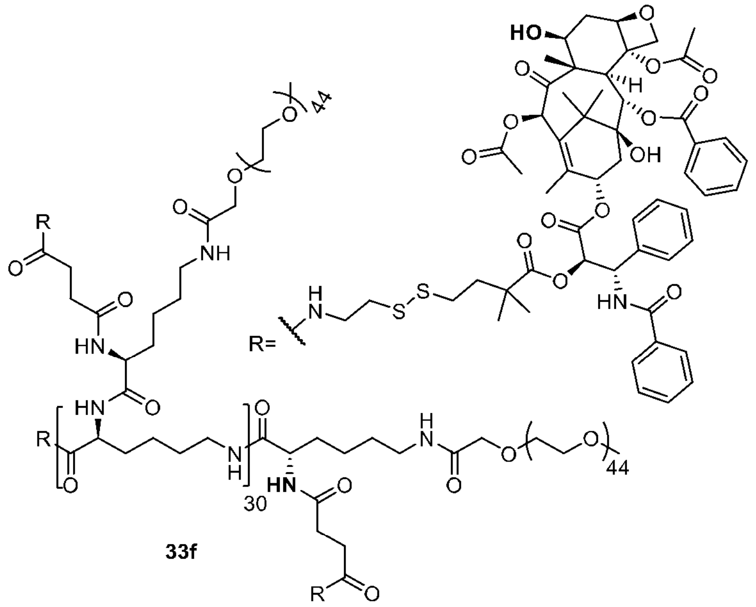

33f

,

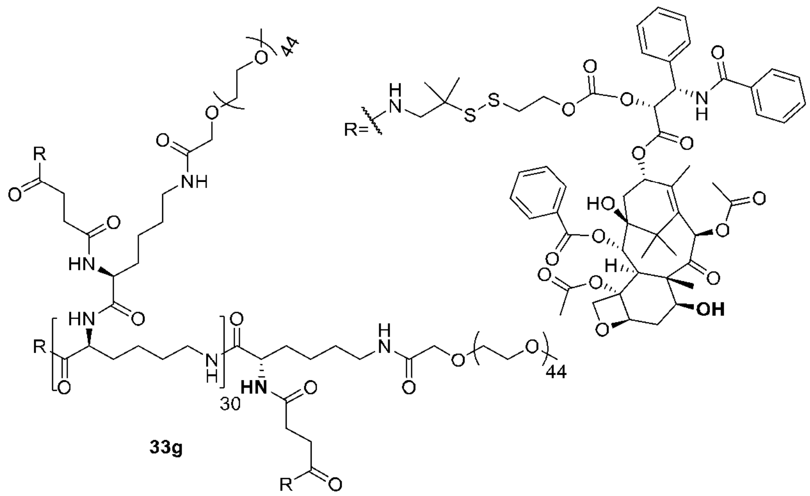

33g

,

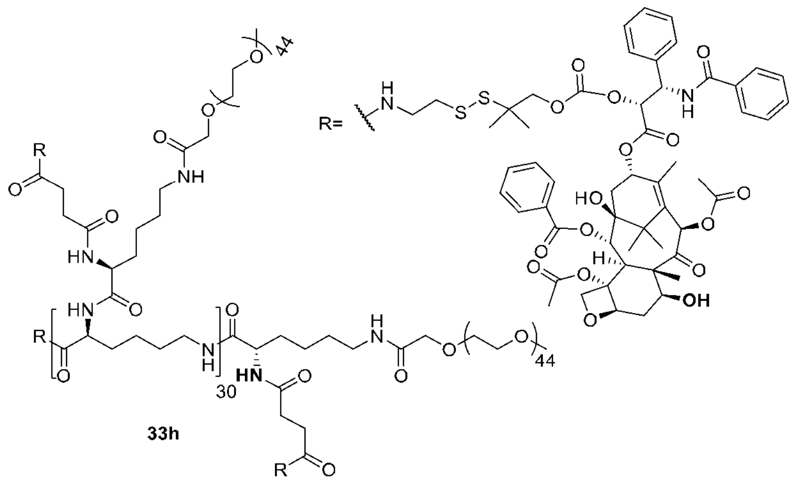

33h

,

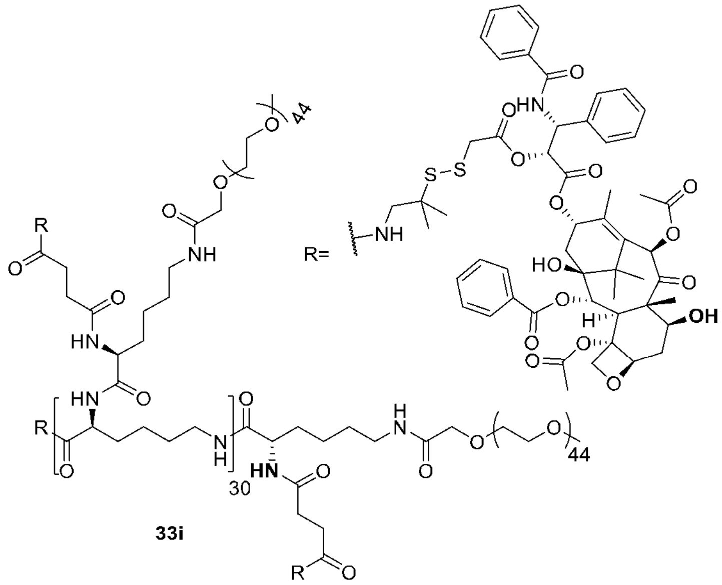

33i

,

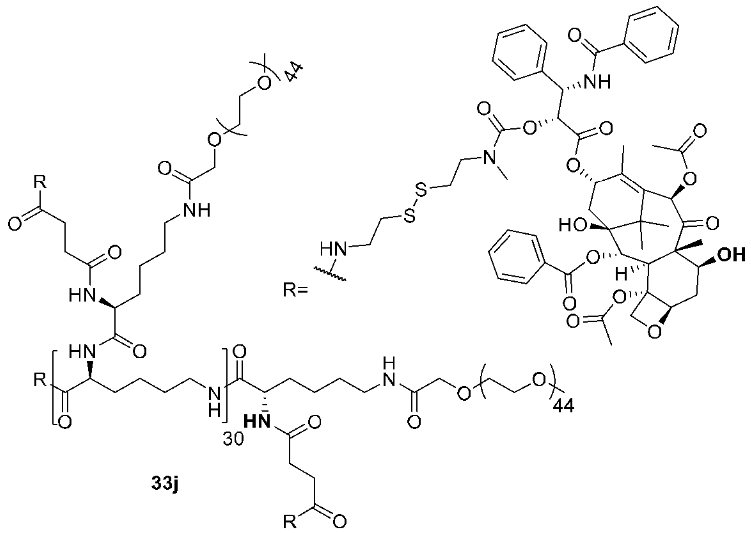

33j

,

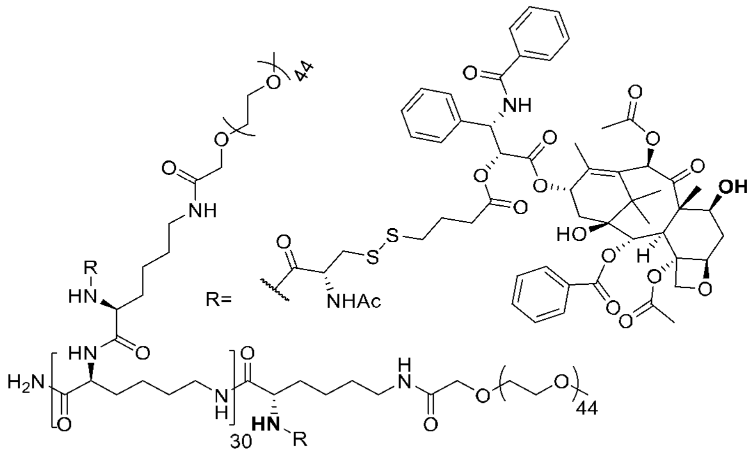

33k

,

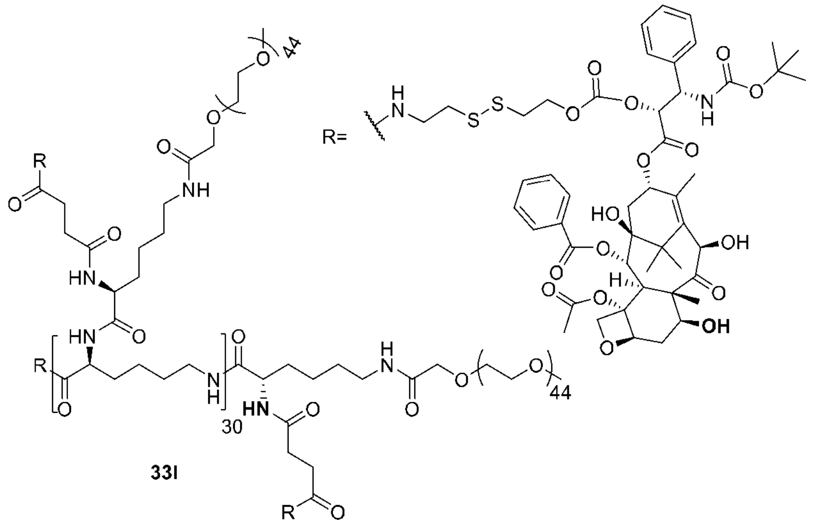

33l

,

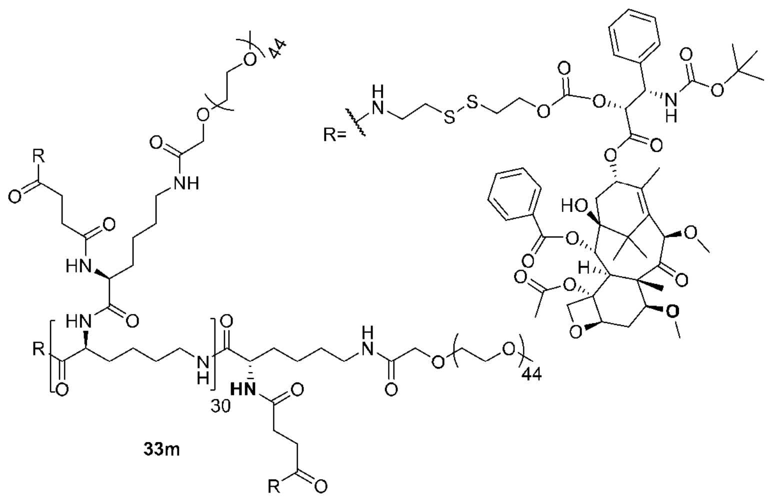

33m

,

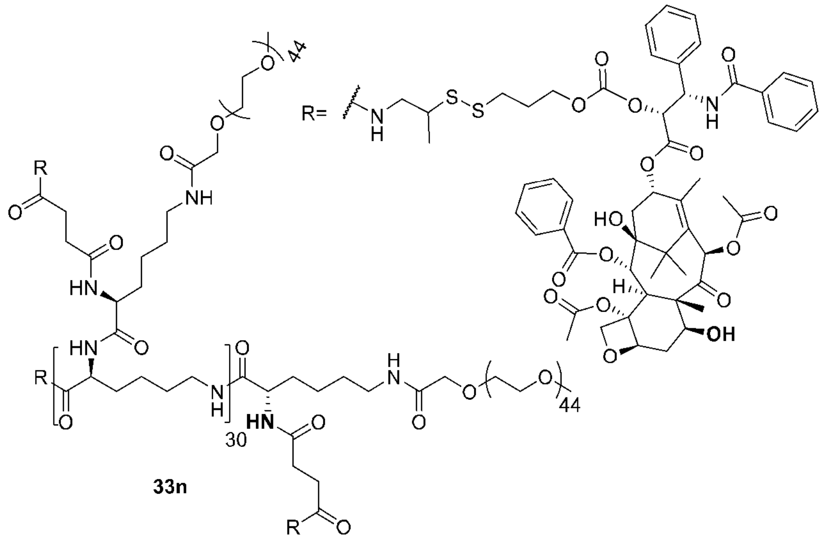

33n

,

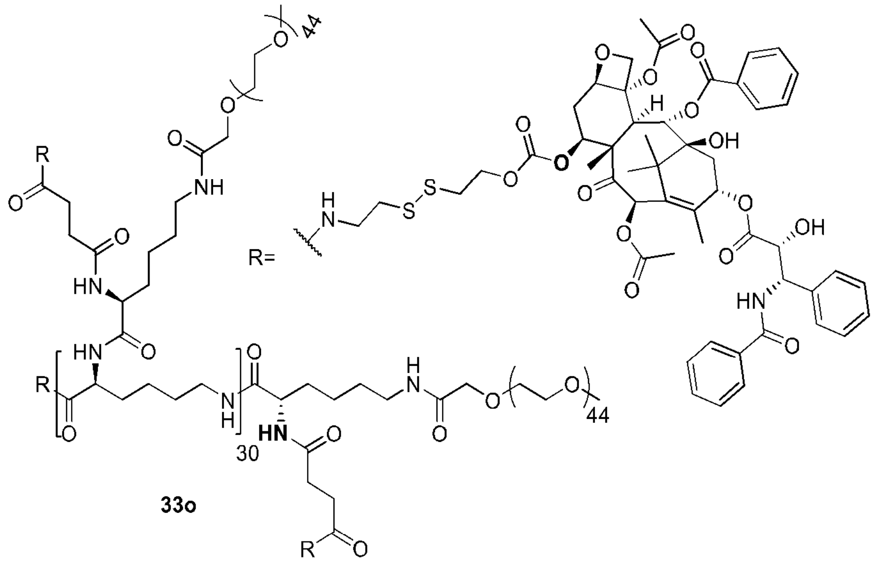

33o

,

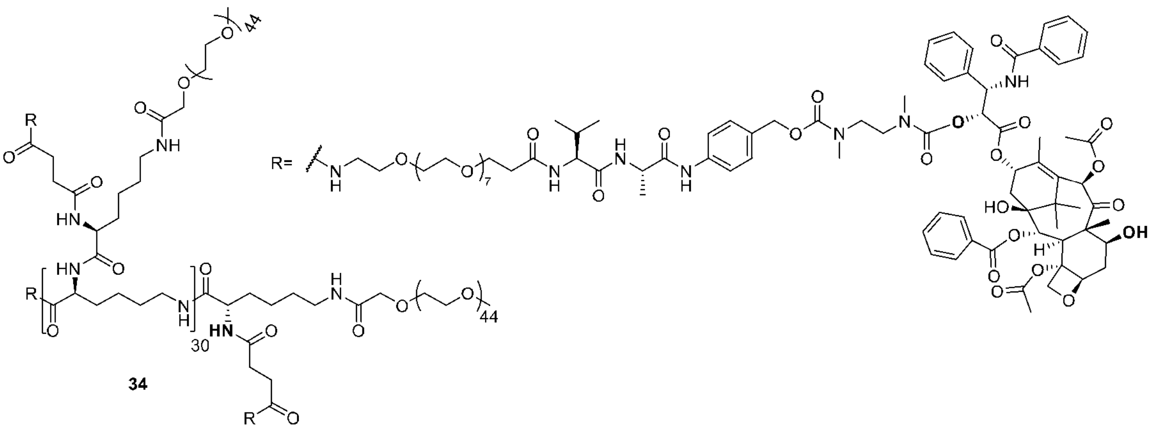

34

,

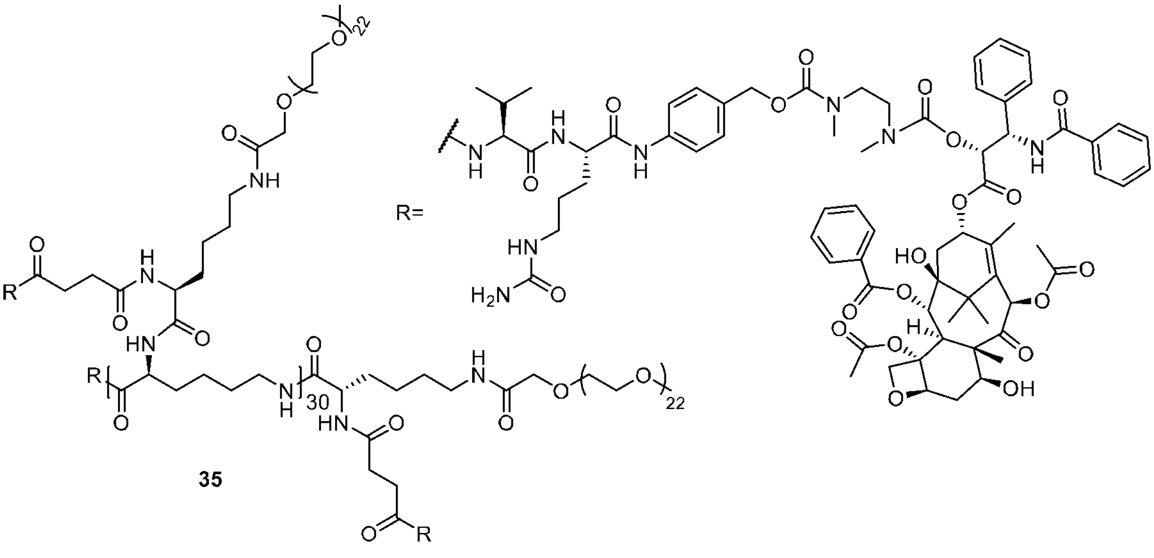

35

,

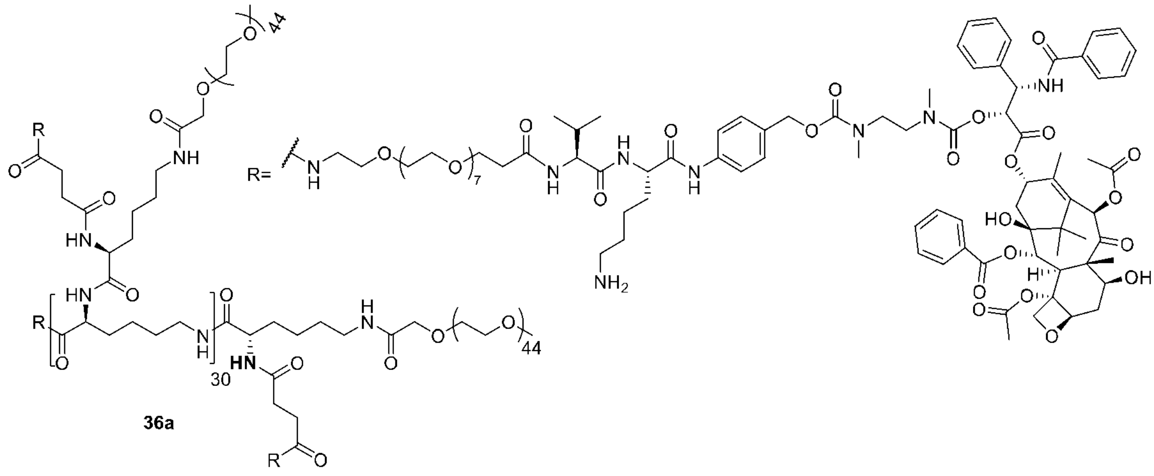

36a

,

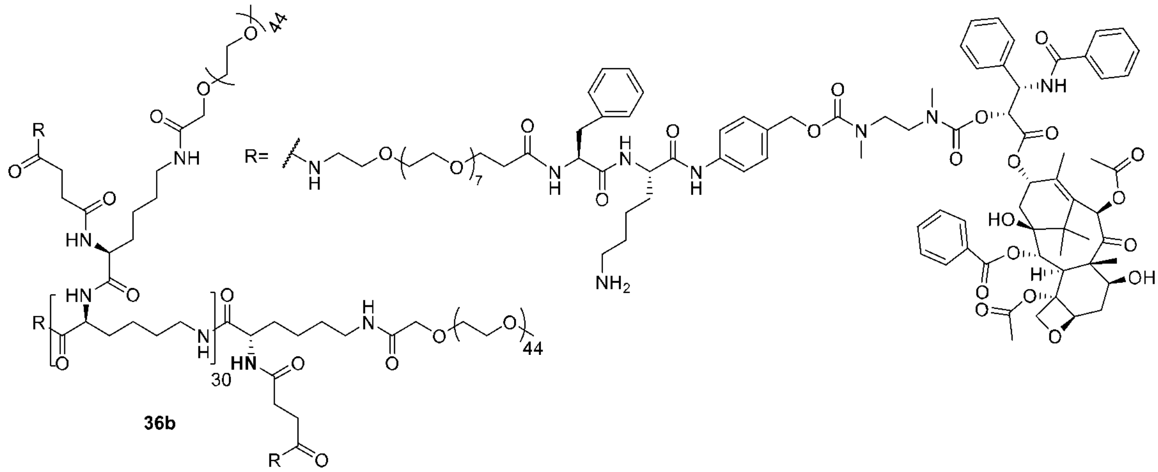

36b

,

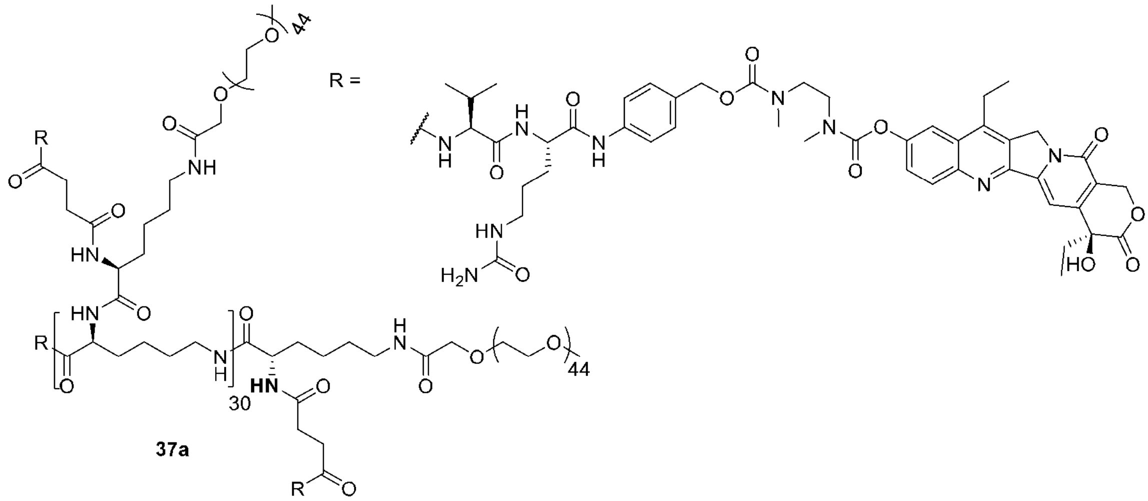

37a

,

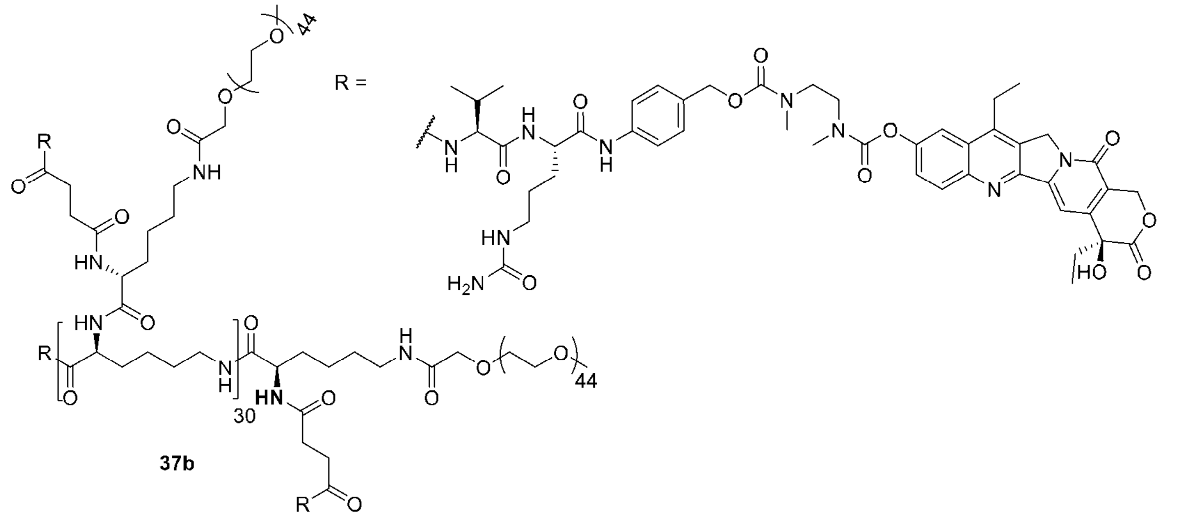

37b

,

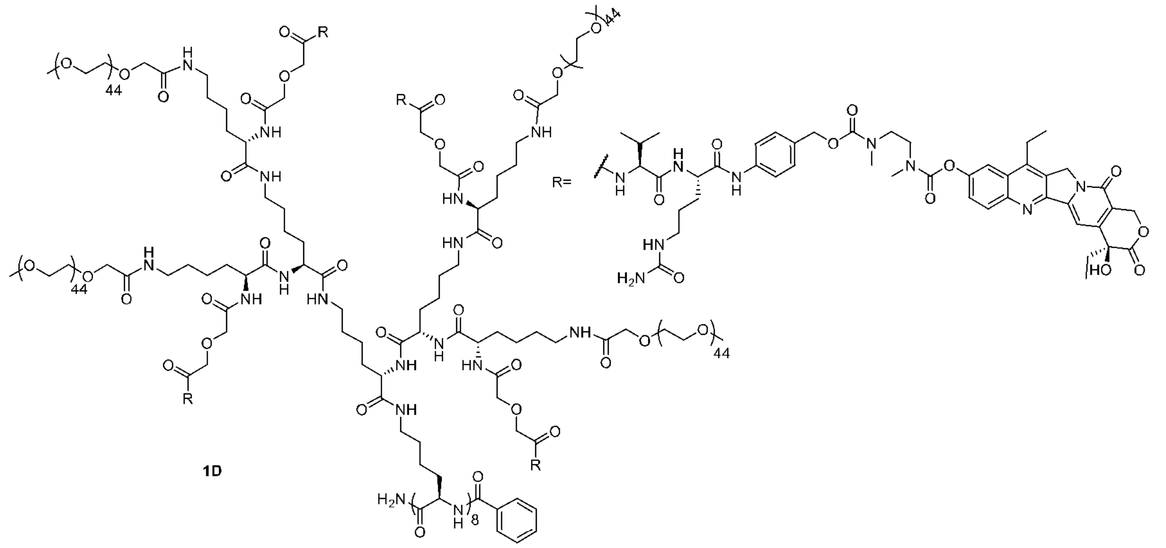

1D

,

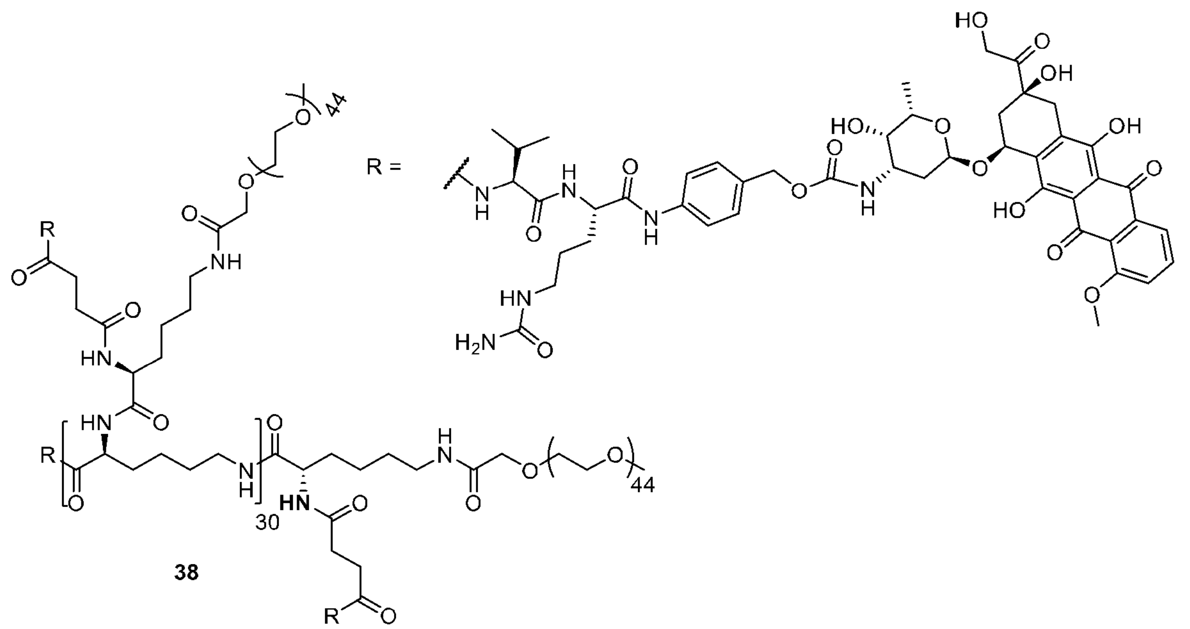

38

,

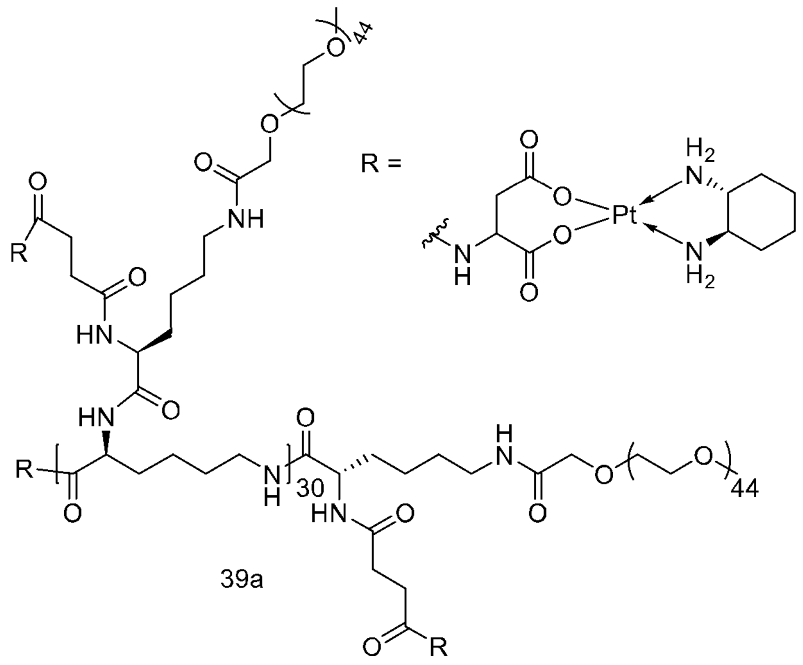

39a

,

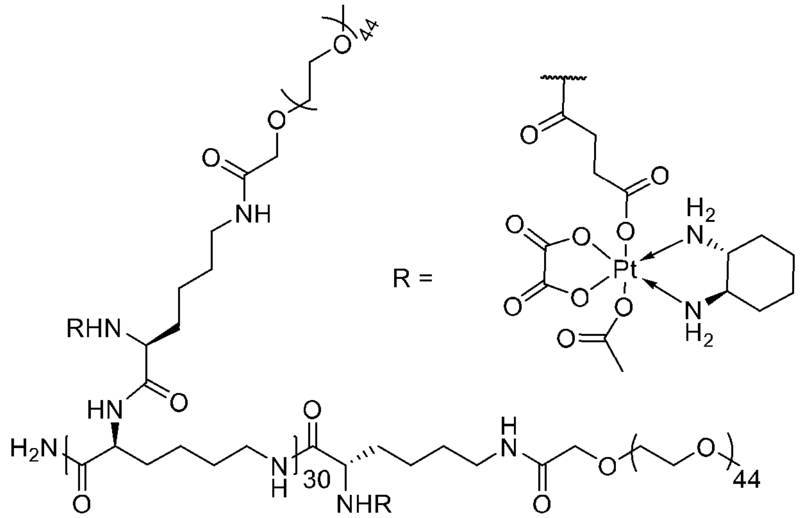

**39b**

,

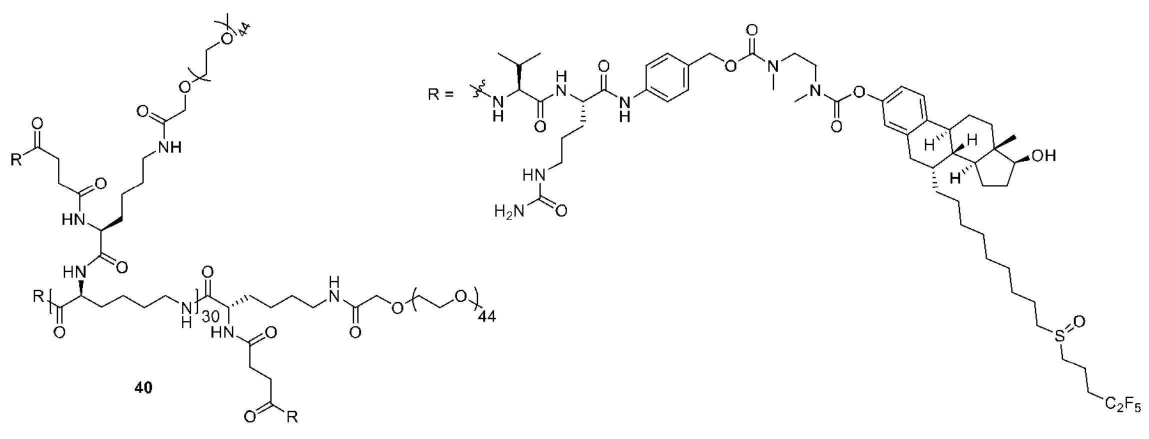

**40**

,

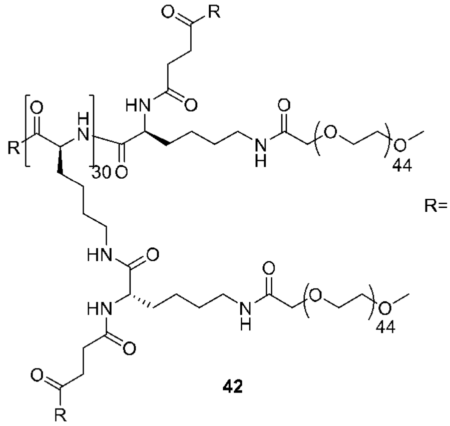

42

R=

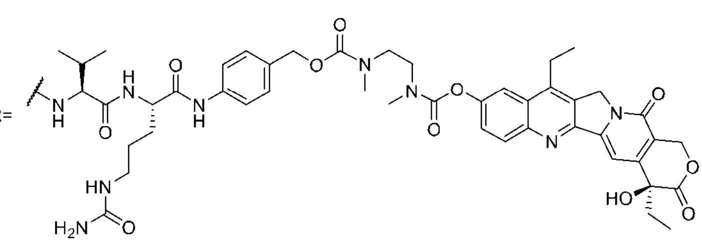

,

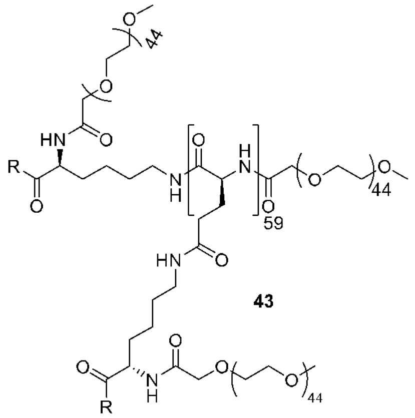

43

R=

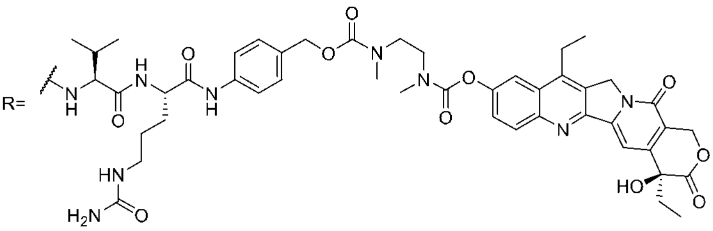

,

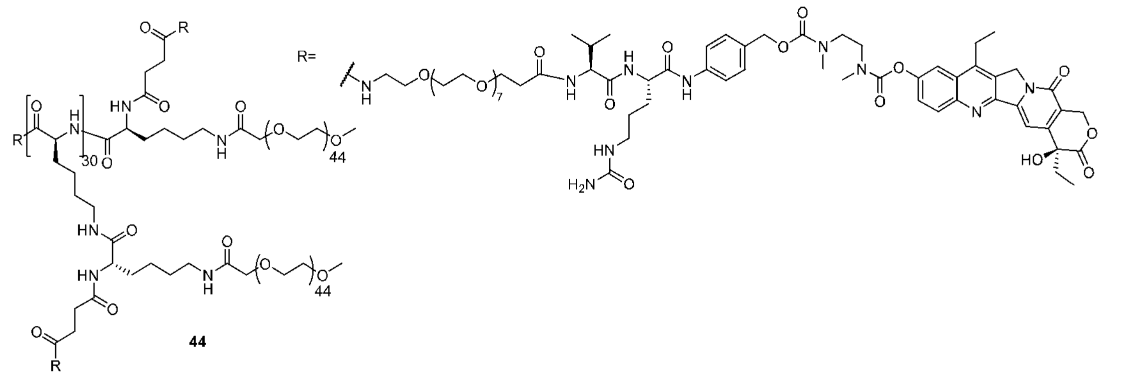

R=

44

.
,

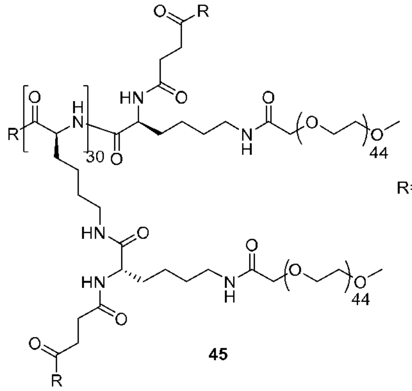

45

R=

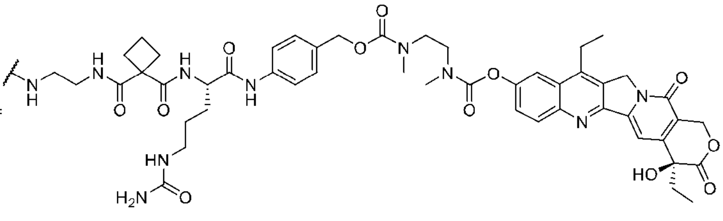

,

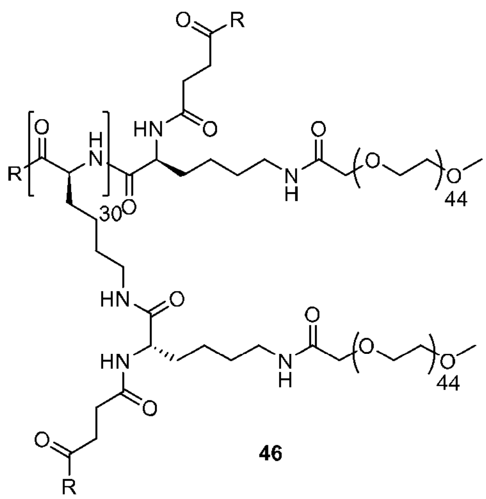

46

R=

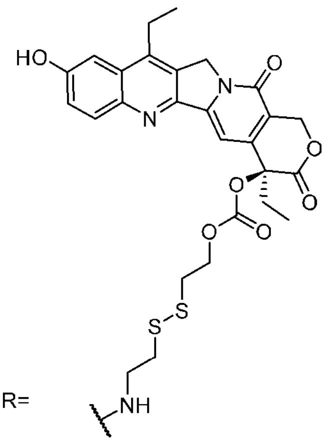

,

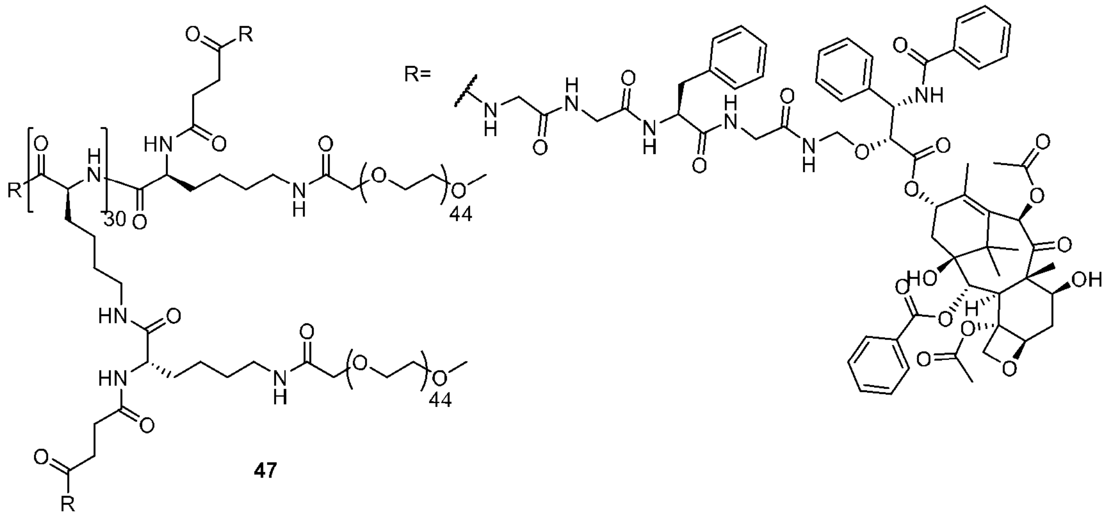

R=

47

,

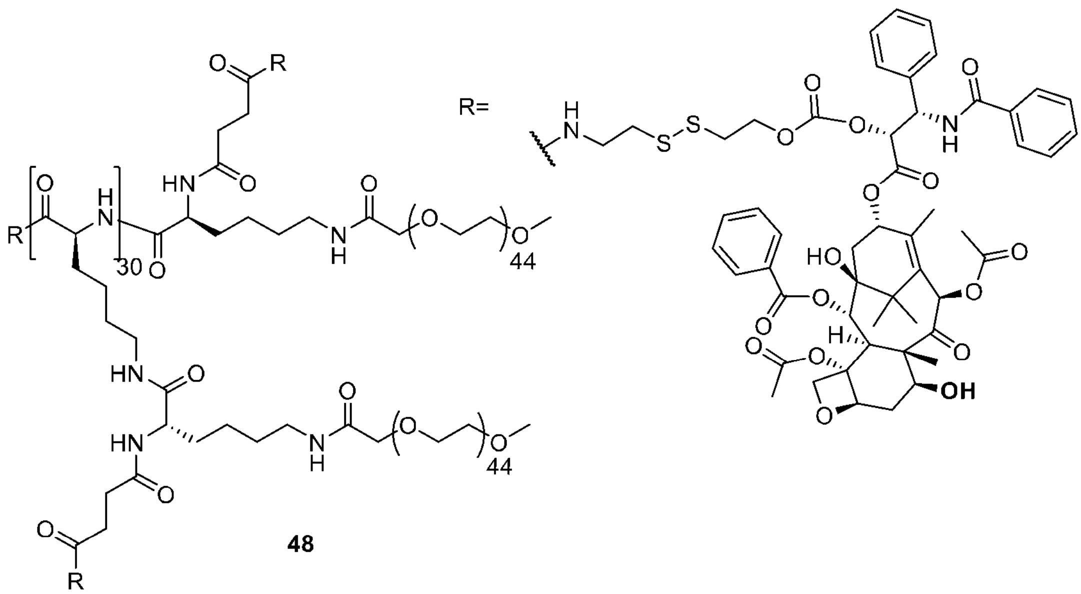

R=

48

,

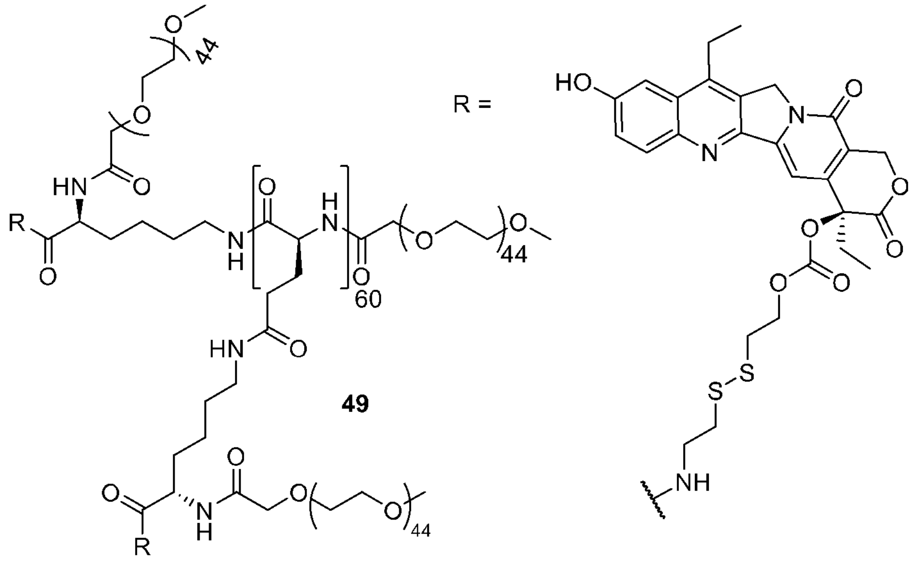

49

,

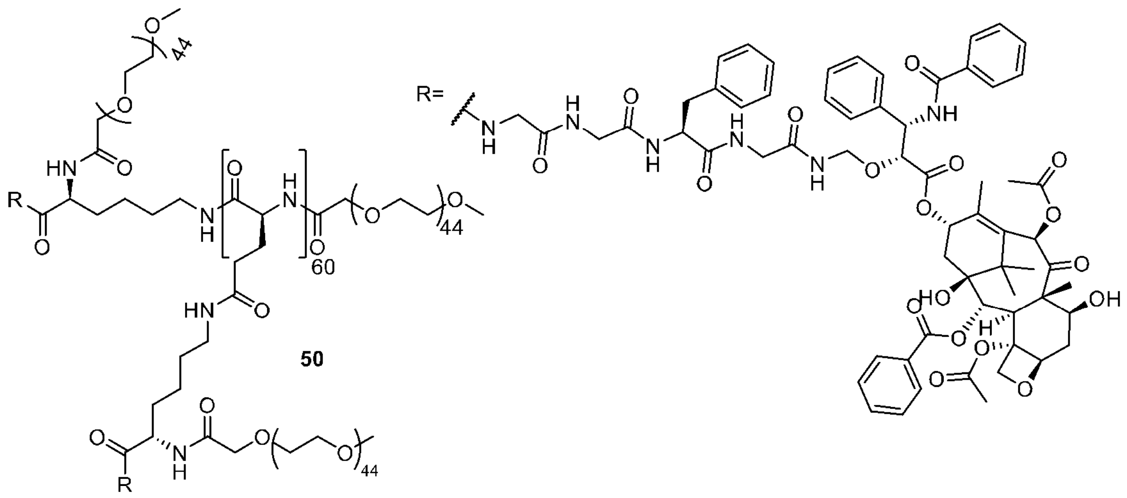

50

,

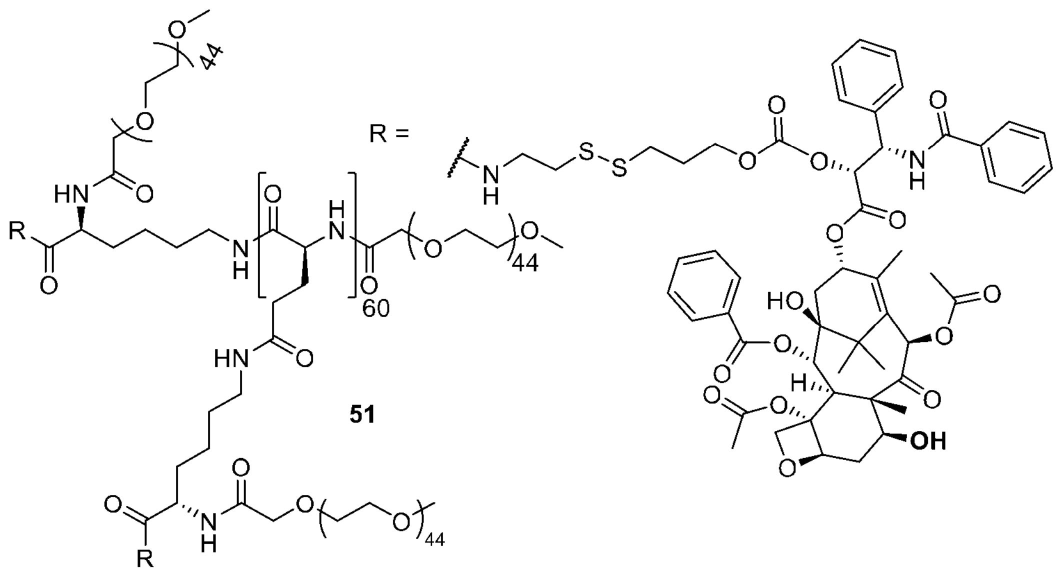

51

,

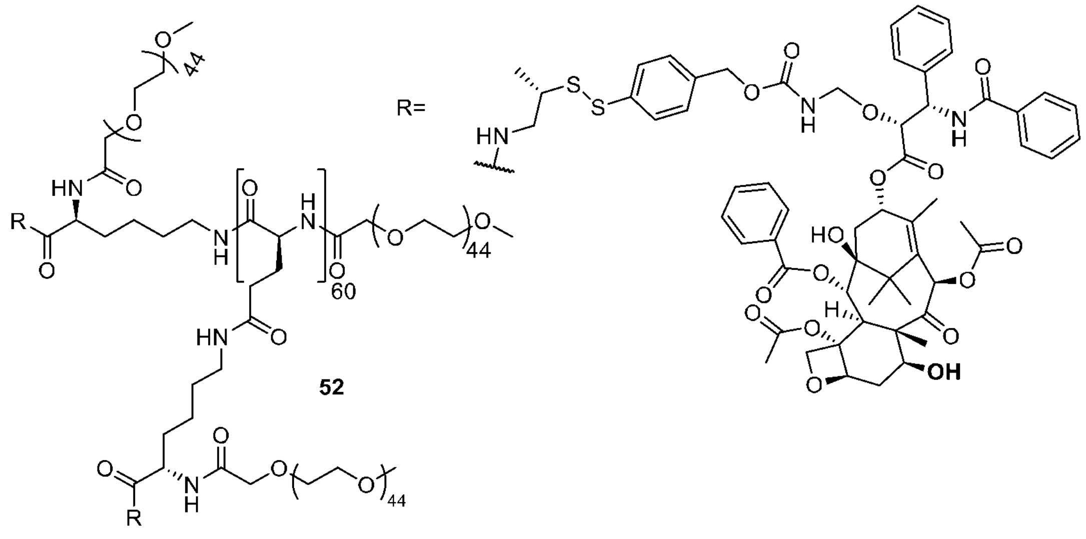

R=

52

,

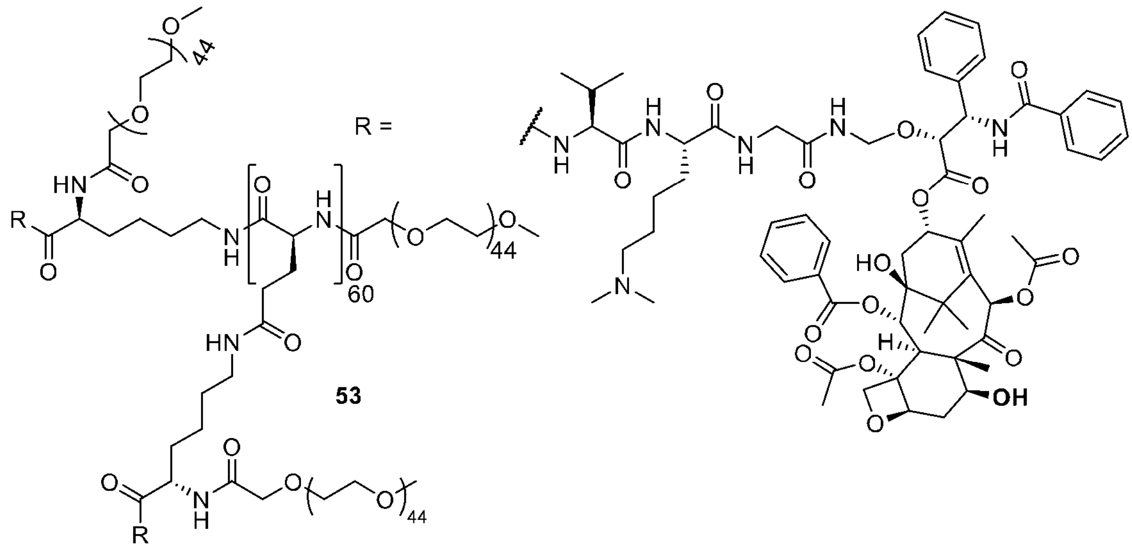

R =

53

,

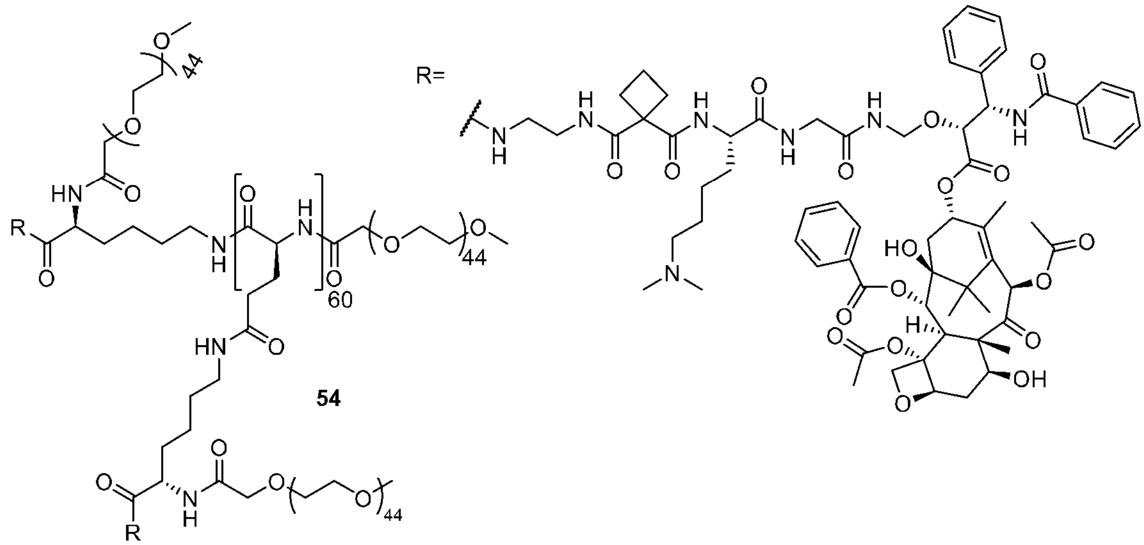

R=

54

,

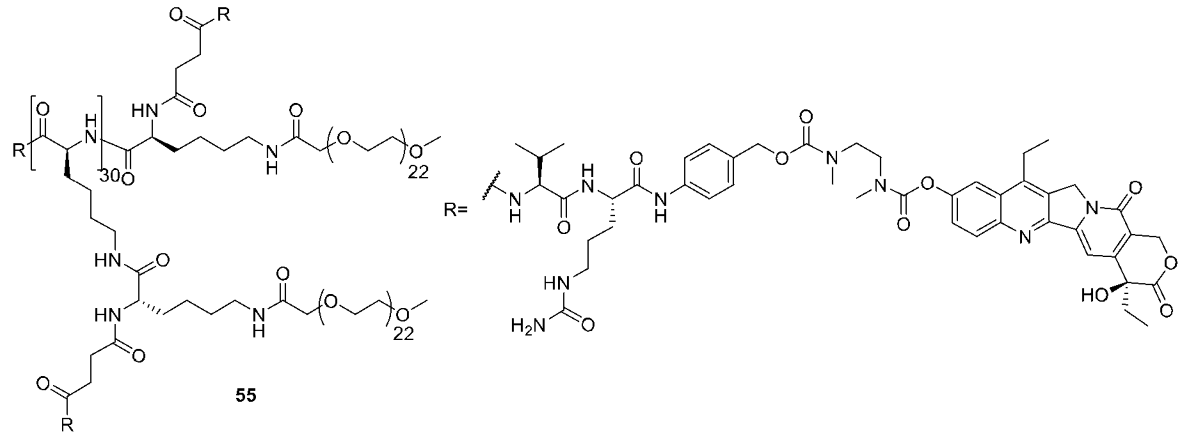

55

,

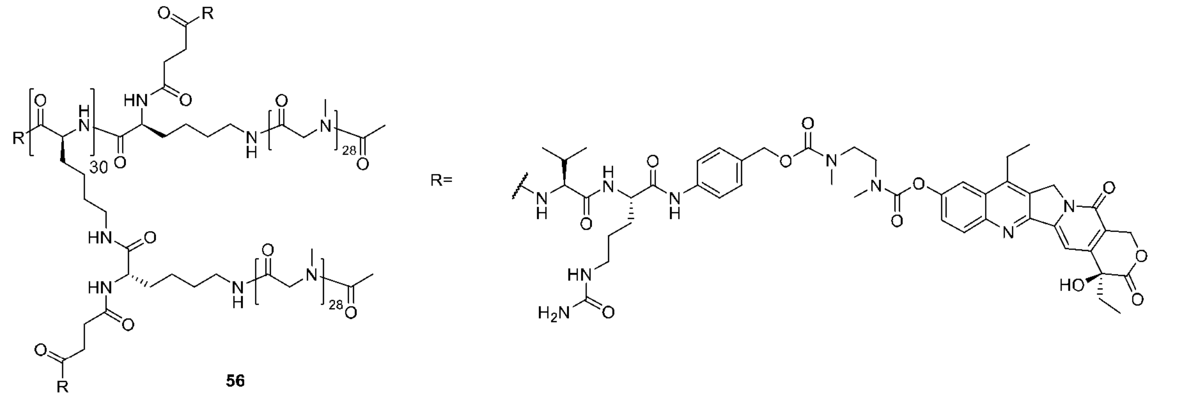

56

,

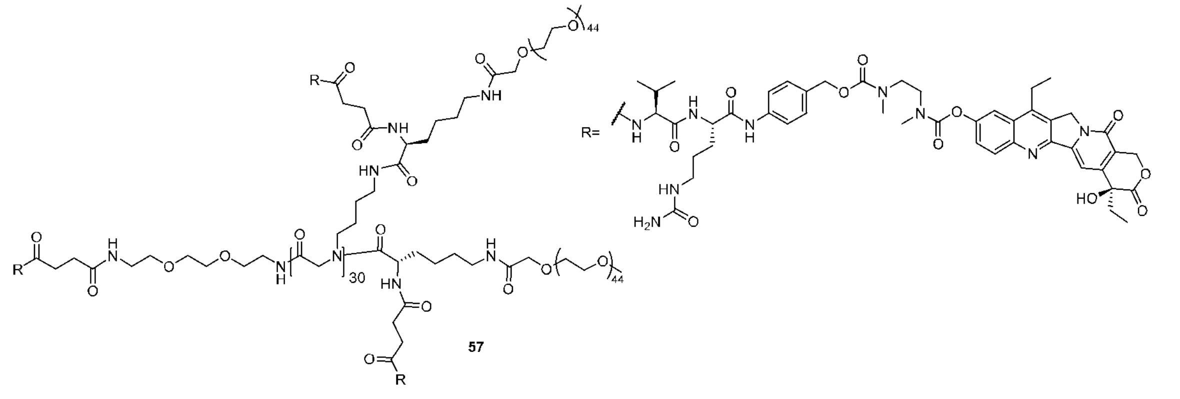

57

,

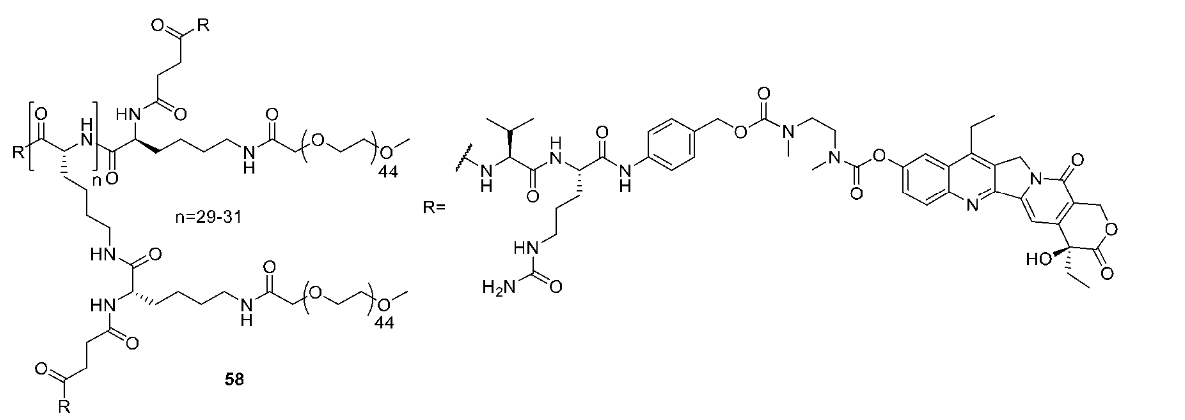

58

,

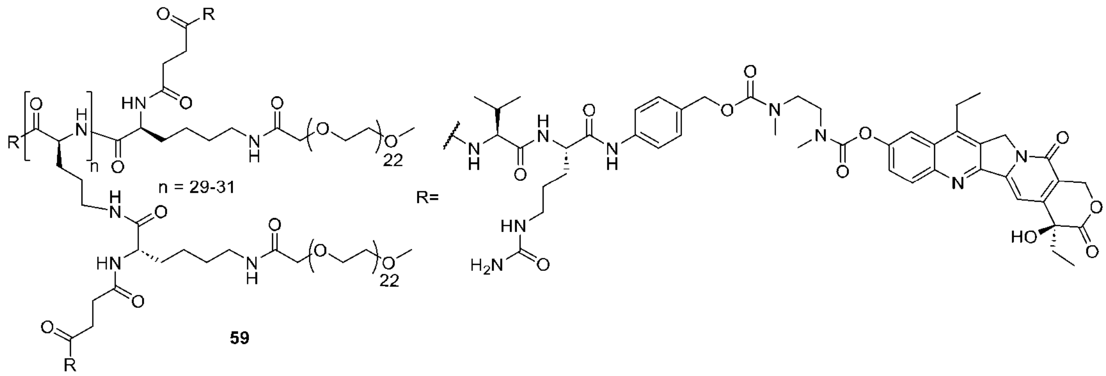

59

,

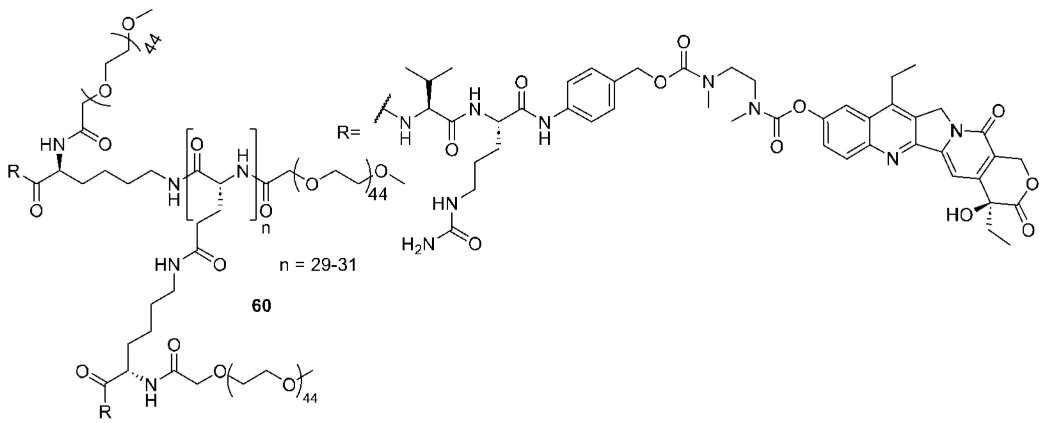

60

,

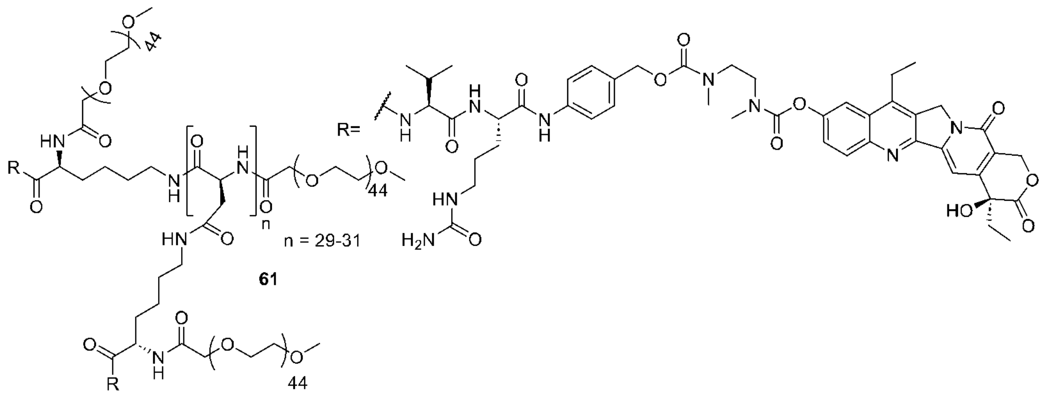

61

,

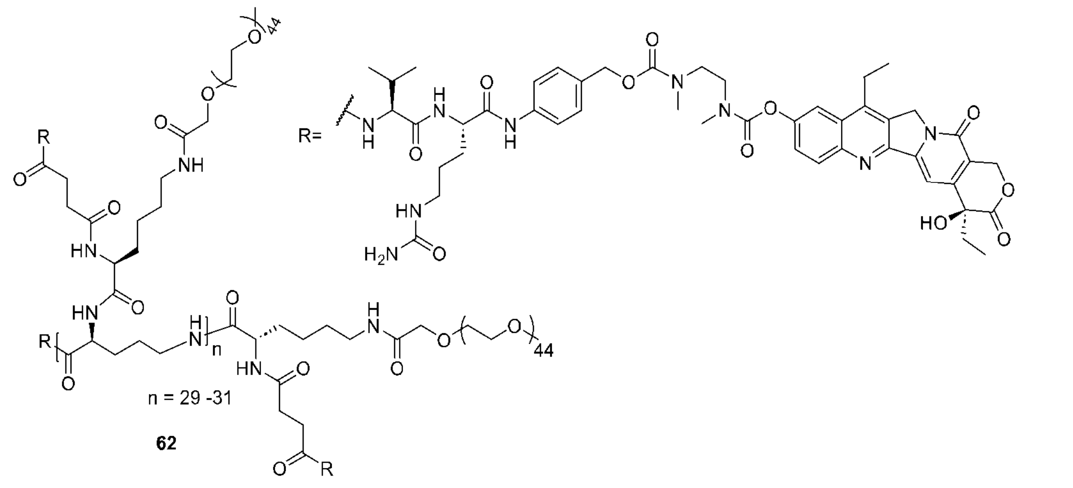

62

,

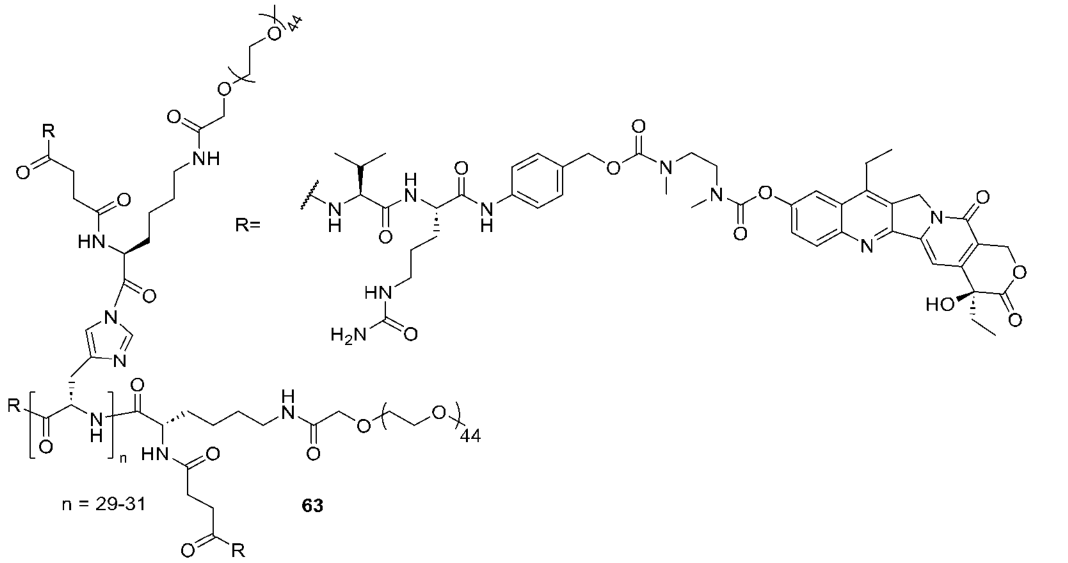

63

,

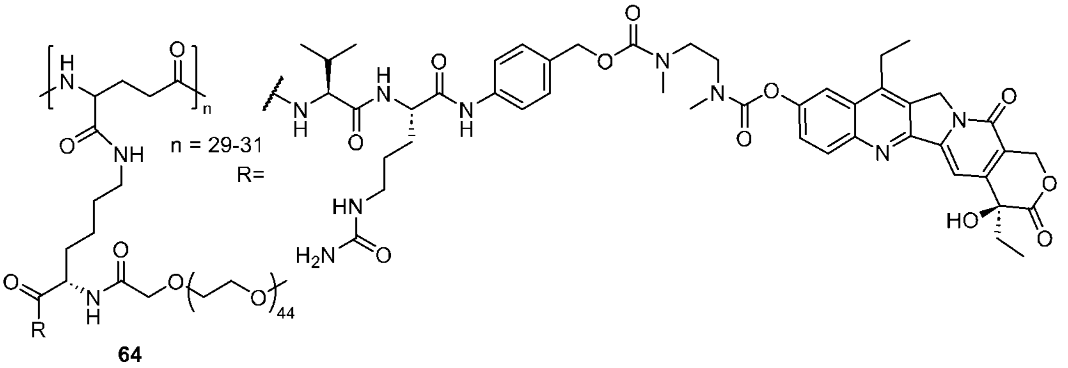

64

,

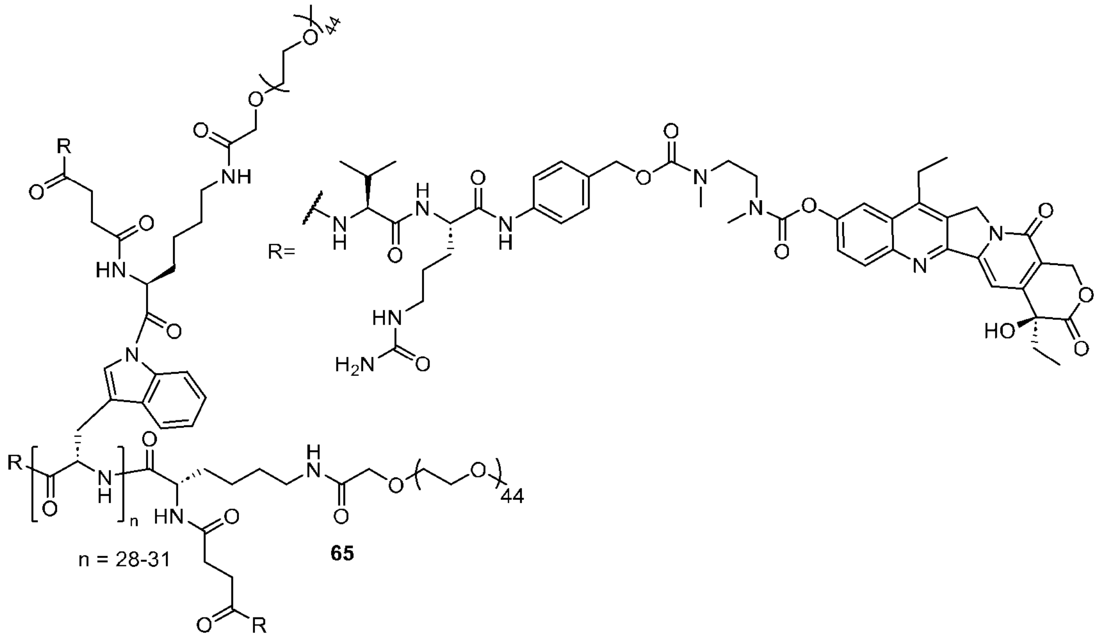

65

,

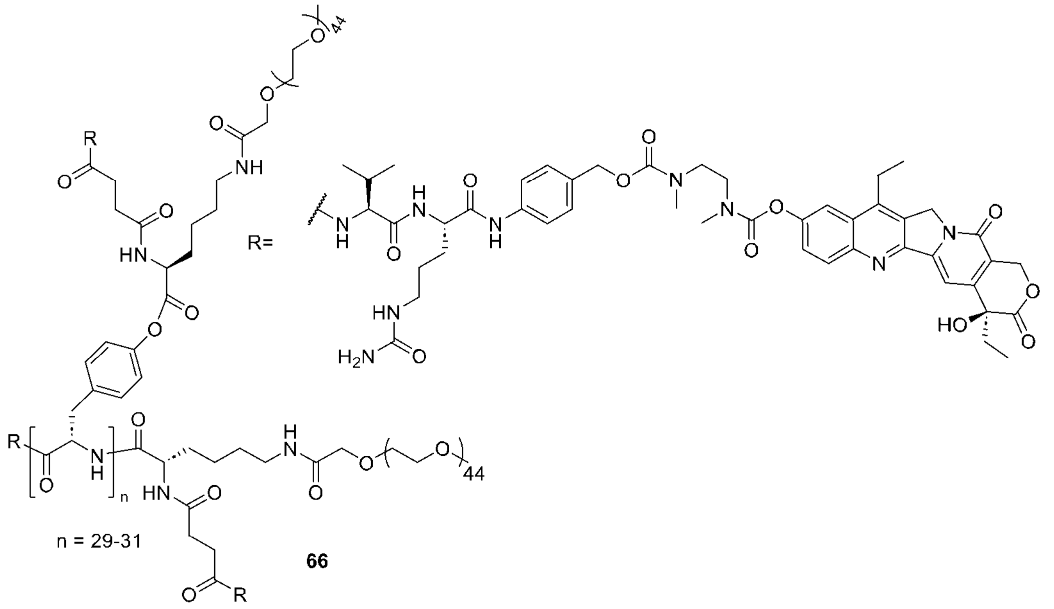

66

,

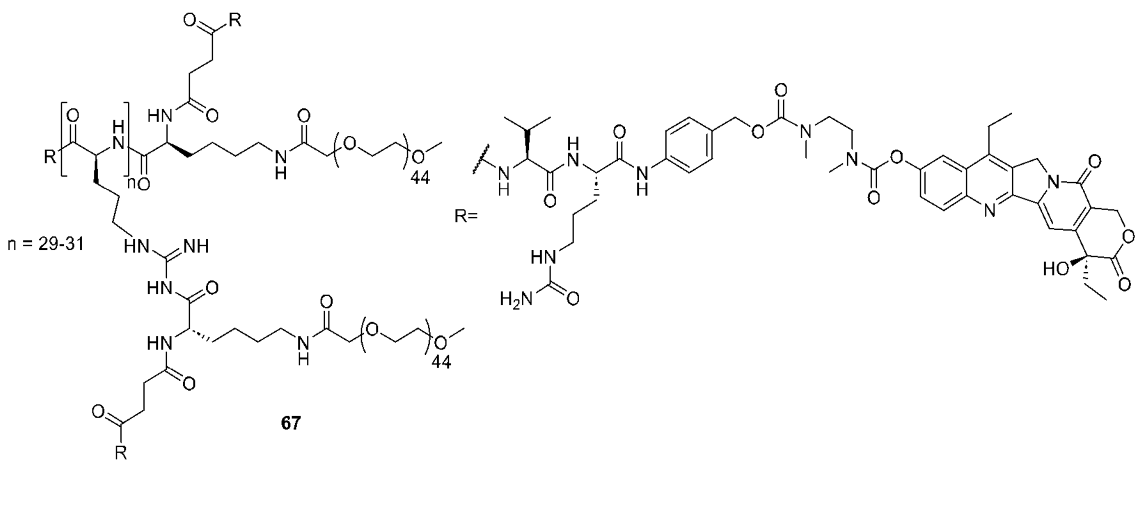

67

,

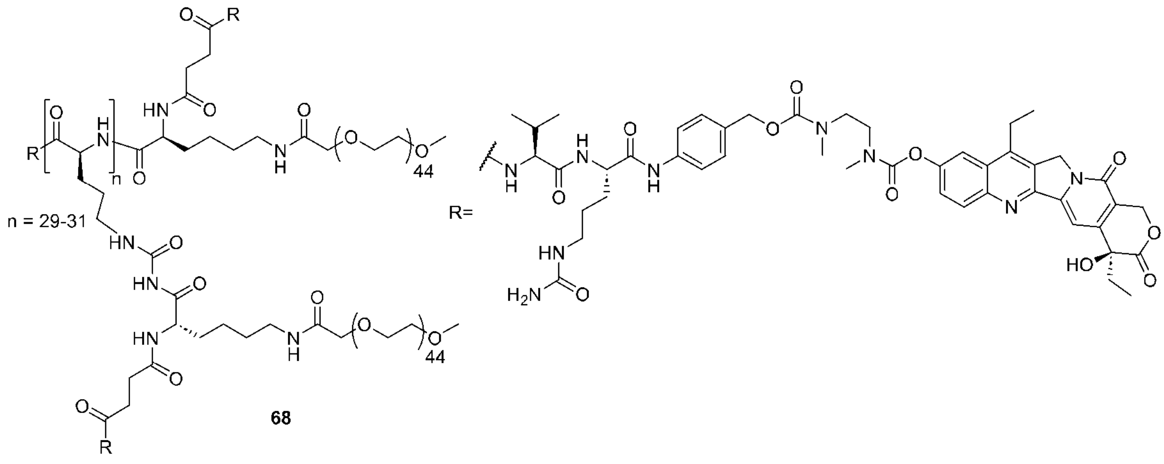

n = 29-31

68

,

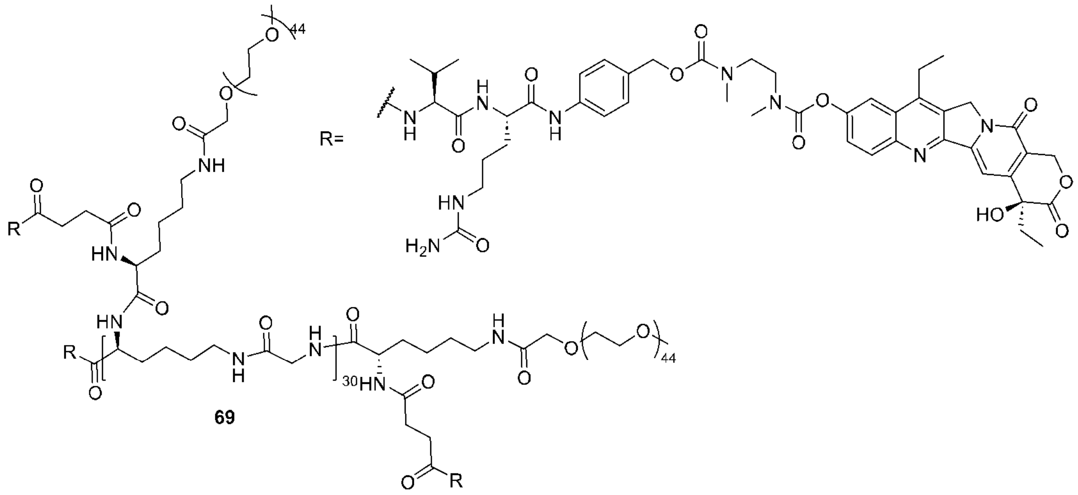

69

,

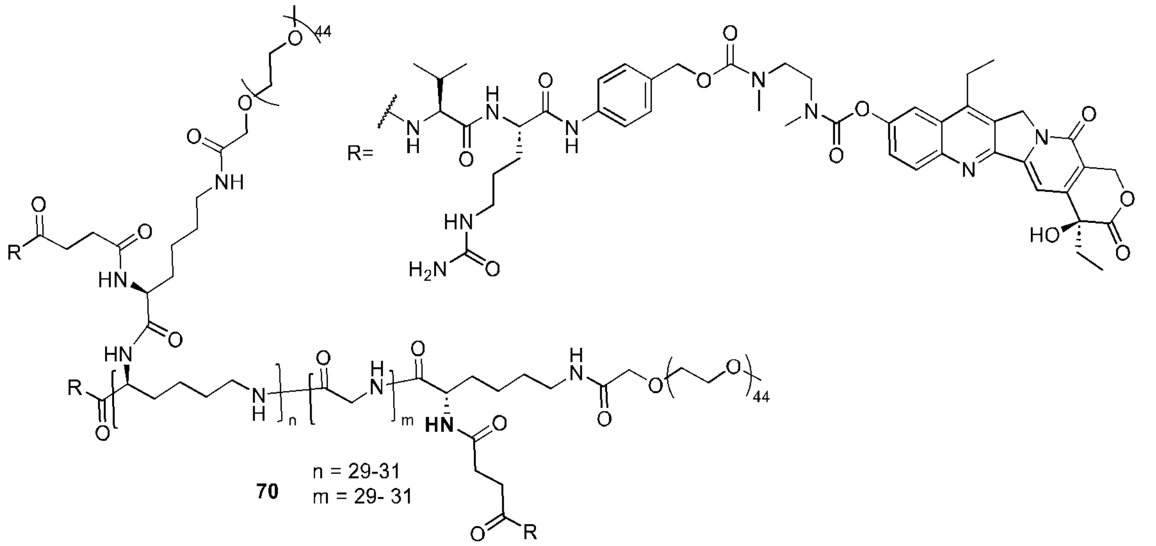

70

n = 29-31
m = 29- 31

,

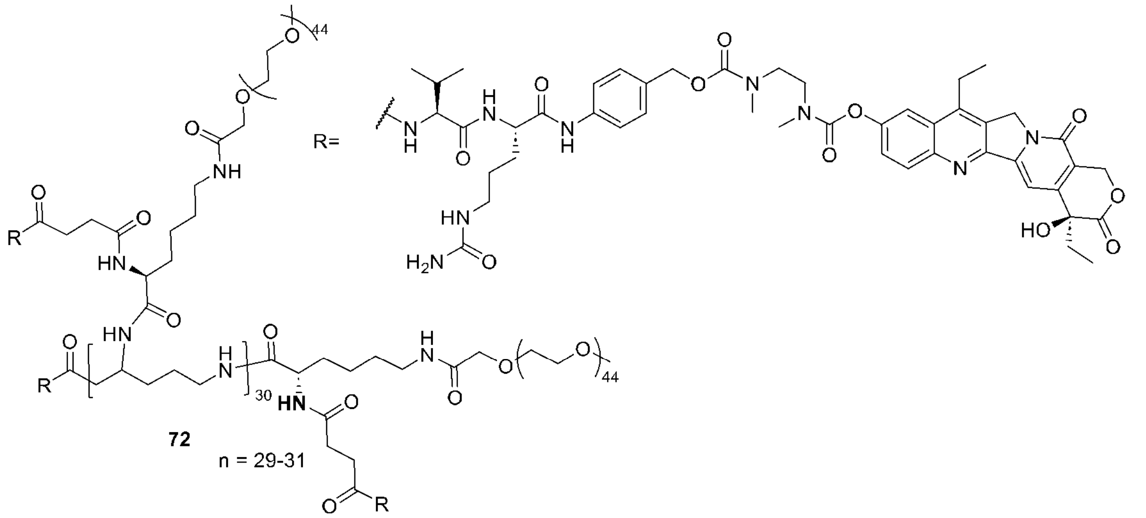

72

,

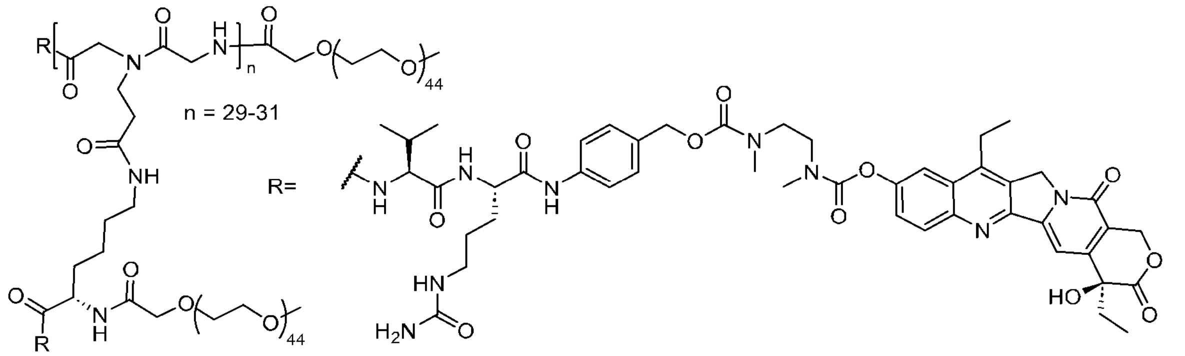

73

,

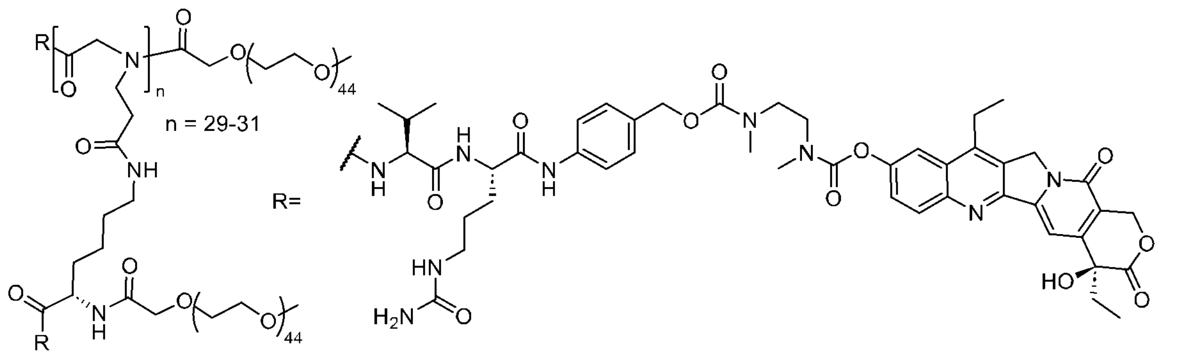

74

,

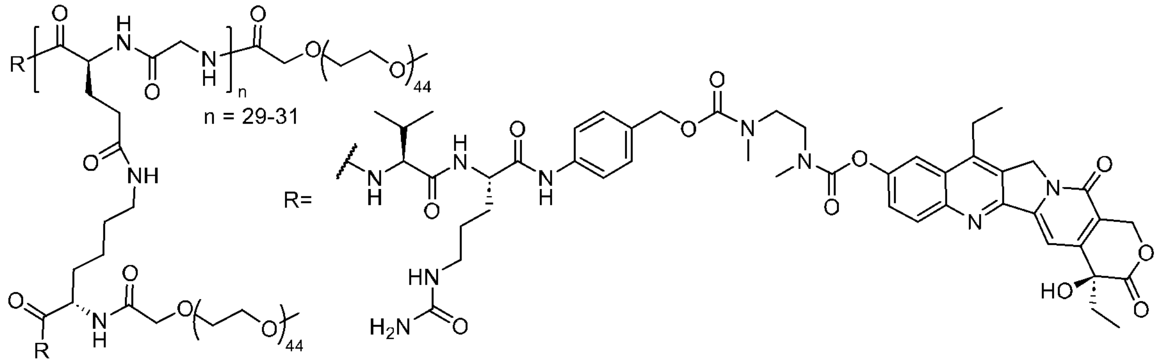

n = 29-31

R=

75

,

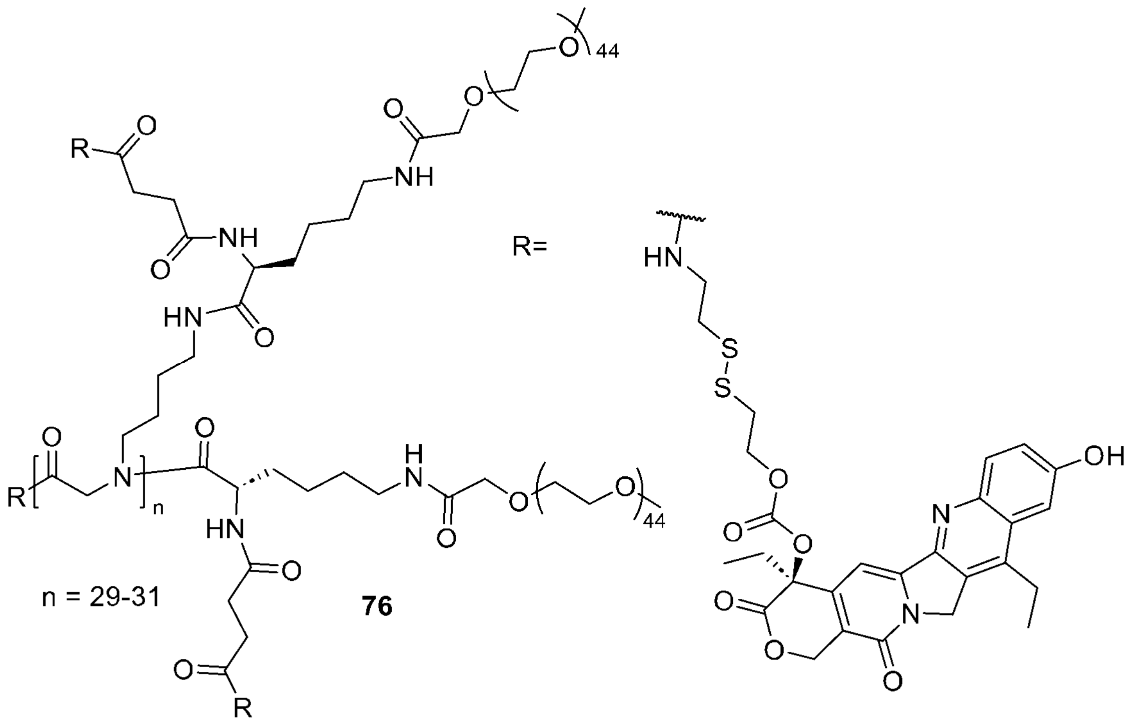

R=

n = 29-31

76

,

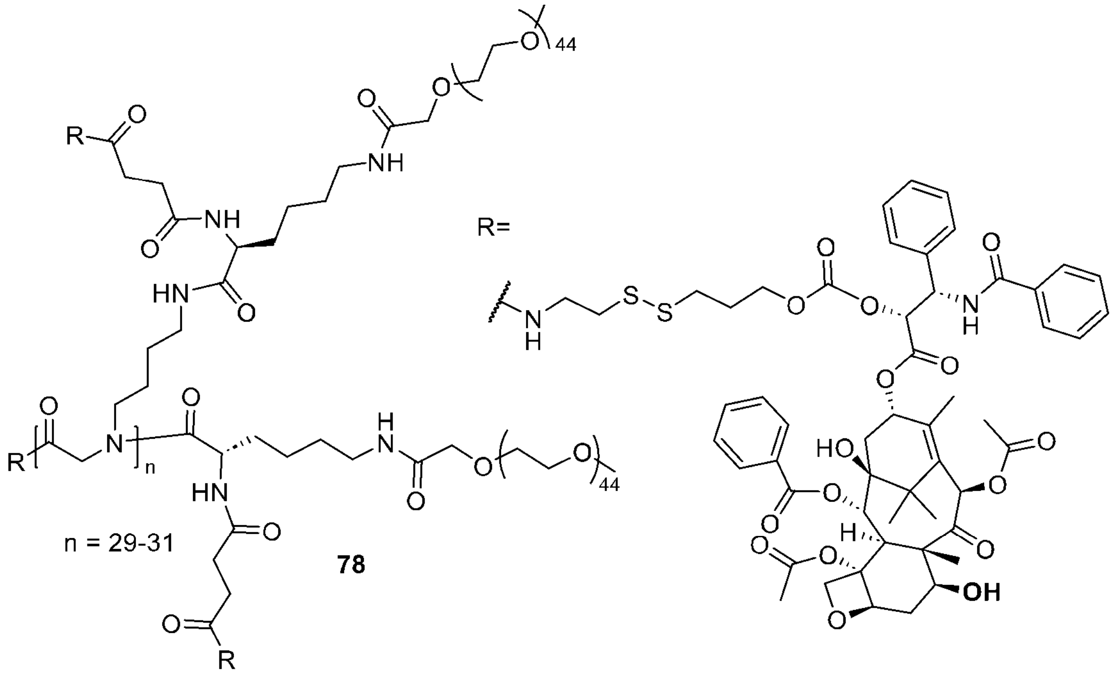

78

,

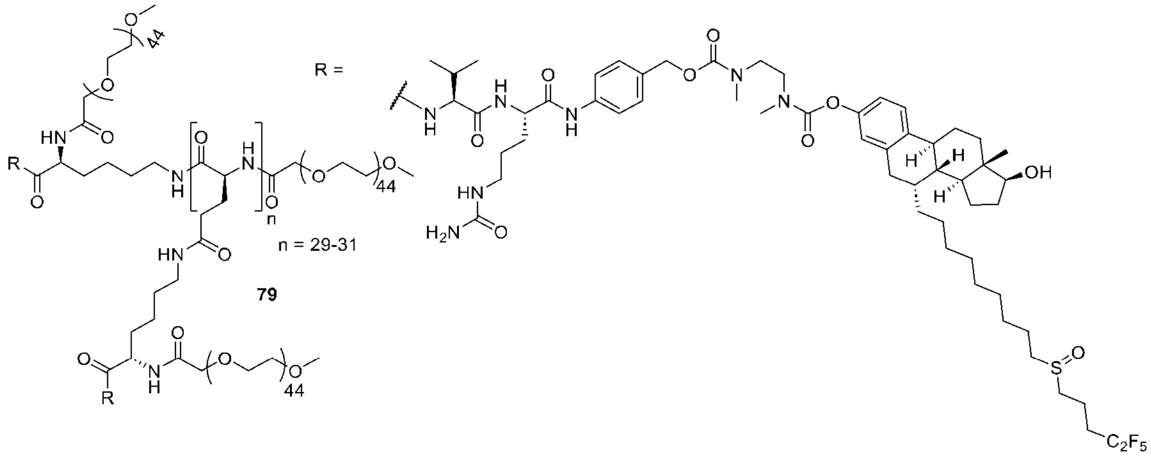

79

,

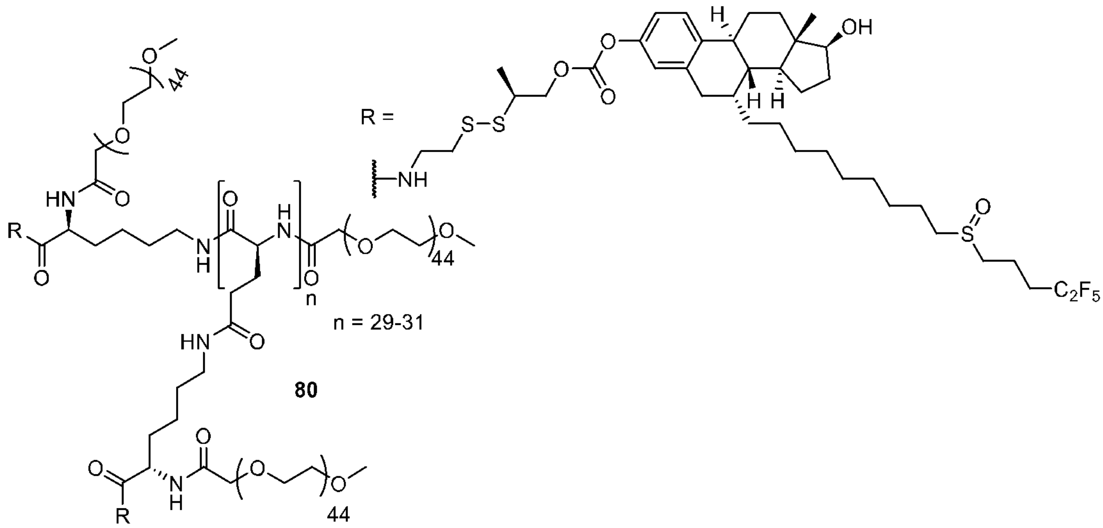

80

,

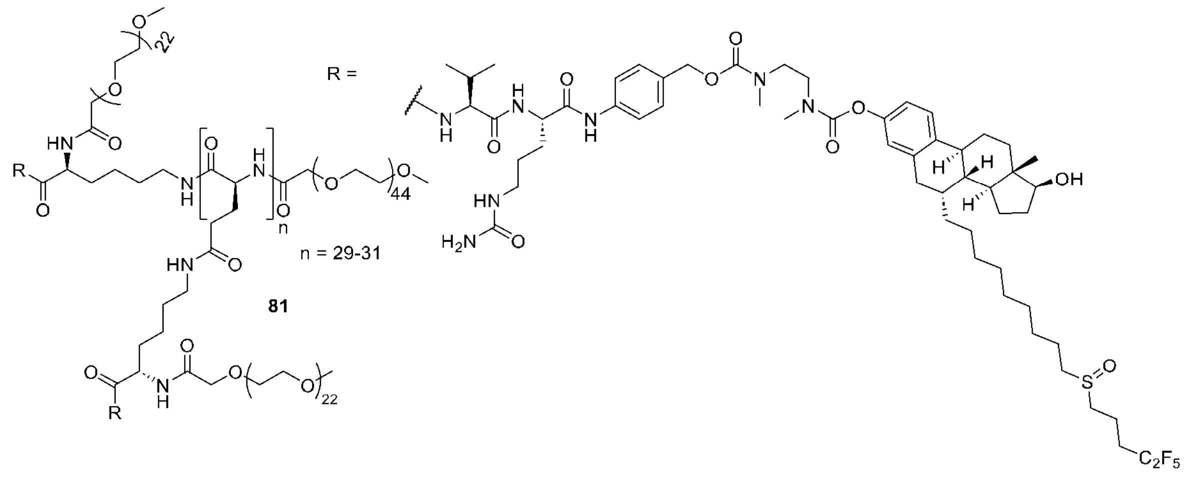

R =

n = 29-31

**81**

,

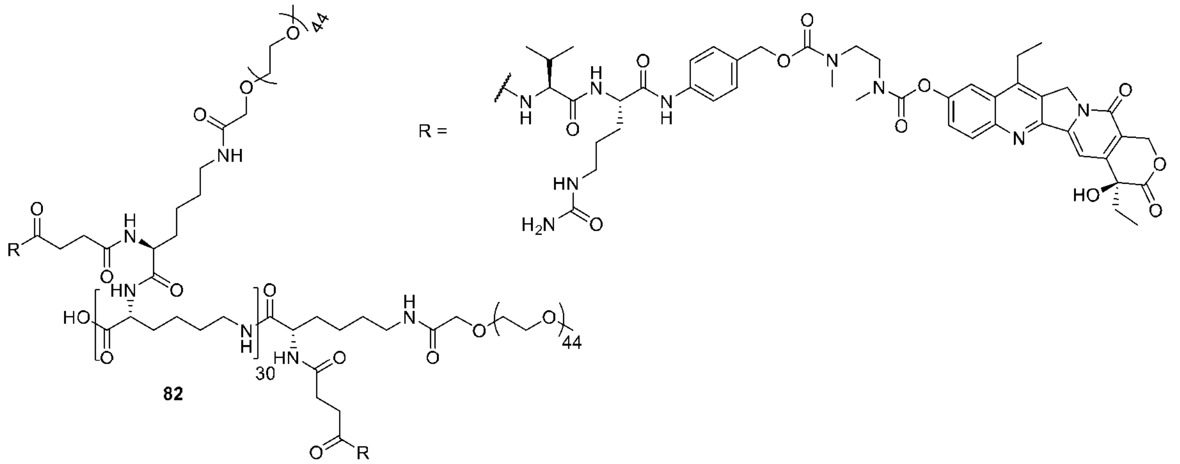

R =

**82**

,

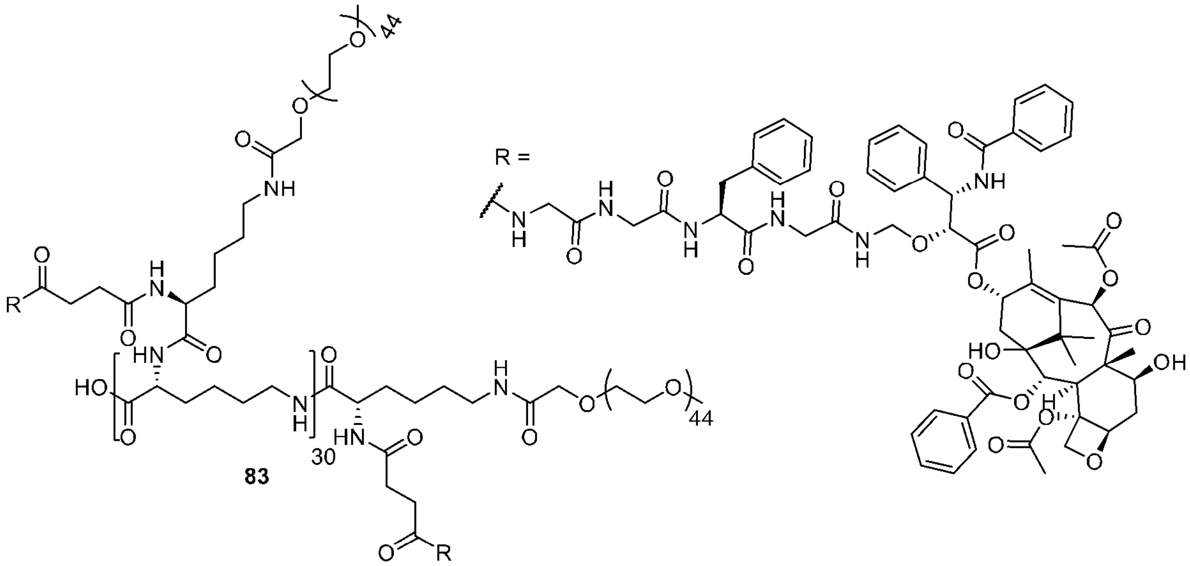

R =

**83**

,

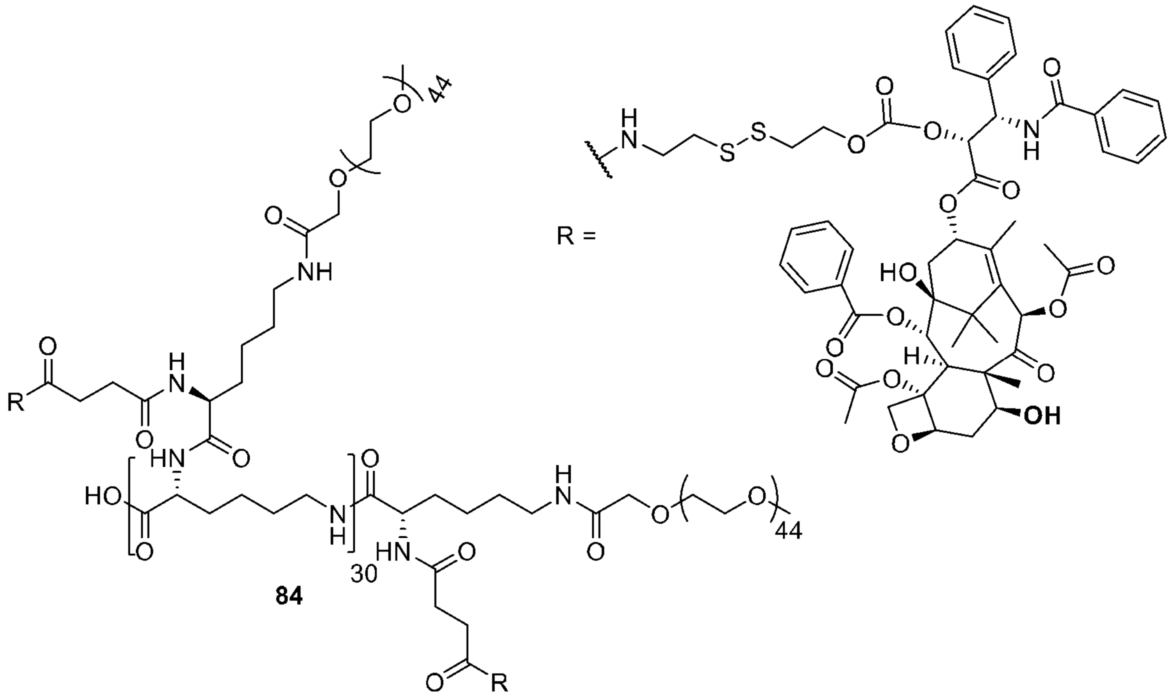

R =

84

,

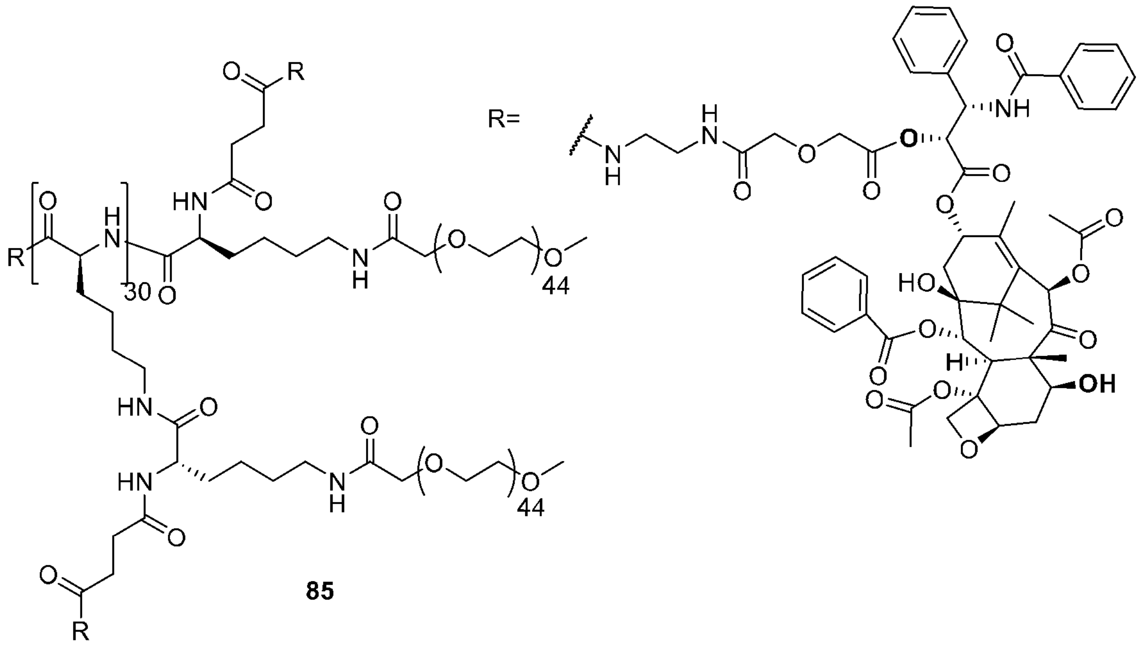

R=

85

,

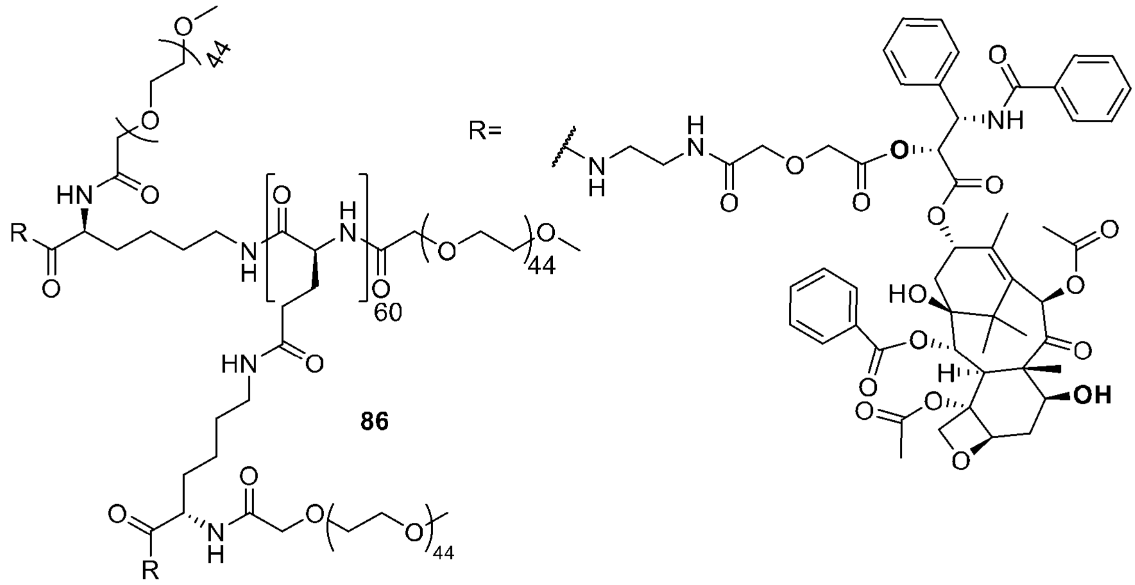

86

,

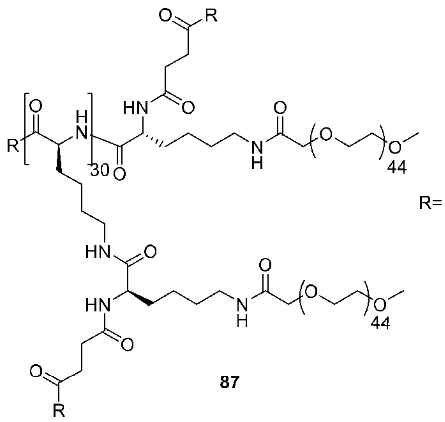

R=

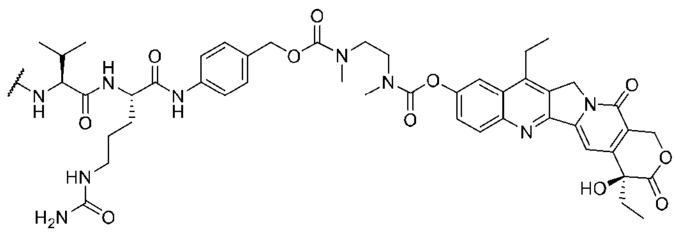

87

,

or

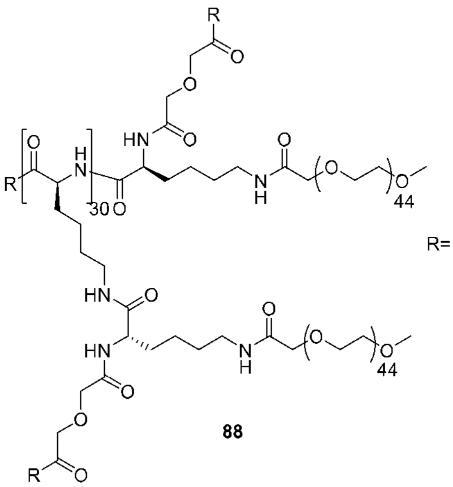

R=

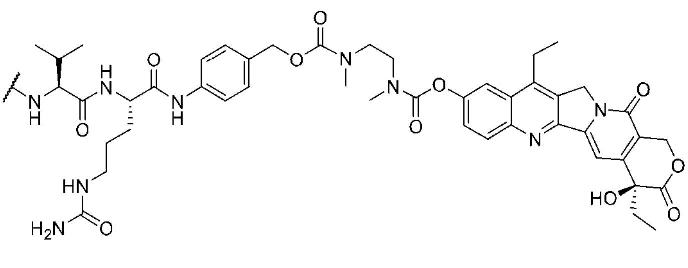

88

.

The present disclosure further provides a pharmaceutical composition, comprising the compound or the pharmaceutically acceptable salt thereof according to any one of the above embodiments, and a pharmaceutically acceptable excipient.

The present disclosure further provides use of the compound according to any one of the above embodiments or

the pharmaceutical composition described above in preparing a medicament for preventing and/or treating a disease, wherein the disease is a cancer selected from, for example: one or more of breast cancer, ovarian cancer, prostate cancer, melanoma, brain cancer, nasopharyngeal cancer, esophageal cancer, gastric cancer, liver cancer, pancreatic cancer, colorectal cancer, lung cancer, renal cancer, skin cancer, glioblastoma, neuroblastoma, sarcoma, liposarcoma, osteochondroma, bone cancer, osteosarcoma, seminoma, testicular tumor, uterine tumor, head and neck tumor, multiple myeloma, malignant lymphoma, polycythemia vera, leukemia, thyroid tumor, ureteral tumor, bladder tumor, gallbladder cancer, bile duct cancer, chorioepithelial cancer, and pediatric tumor, preferably one or more of pancreatic cancer, liver cancer, colon cancer, myeloma, lung cancer (e.g., small cell lung cancer), fibrosarcoma, and breast cancer.

The present disclosure further provides a method for preventing and/or treating a disease, comprising: administering to a subject in need a therapeutically effective amount of the compound or the pharmaceutically acceptable salt thereof according to any one of the above embodiments, wherein the disease is a cancer selected from, for example: one or more of breast cancer, ovarian cancer, prostate cancer, melanoma, brain cancer, nasopharyngeal cancer, esophageal cancer, gastric cancer, liver cancer, pancreatic cancer, colorectal cancer, lung cancer, renal cancer, skin cancer, glioblastoma, neuroblastoma, sarcoma, liposarcoma, osteochondroma, bone cancer, osteosarcoma, seminoma, testicular tumor, uterine tumor, head and neck tumor, multiple myeloma, malignant lymphoma, polycythemia vera, leukemia, thyroid tumor, ureteral tumor, bladder tumor, gallbladder cancer, bile duct cancer, chorioepithelial cancer, and pediatric tumor, preferably one or more of pancreatic cancer, liver cancer, colon cancer, myeloma, lung cancer (e.g., small cell lung cancer), fibrosarcoma, and breast cancer.

The present disclosure further provides a compound of formula (I-1-A), (I-1-B), or (I-1-C):

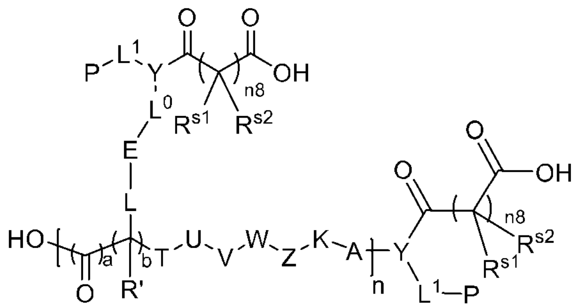

(I-1-A)

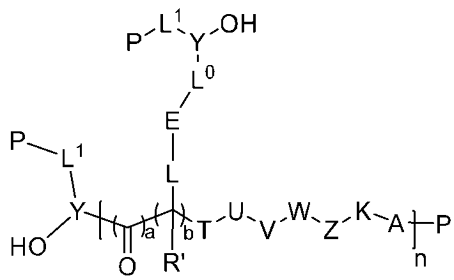

(I-1-B)

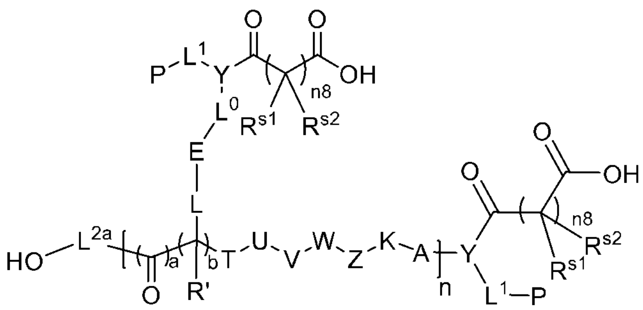

(I-1-C)

wherein, in the compound of formula (I-1-A), Y is

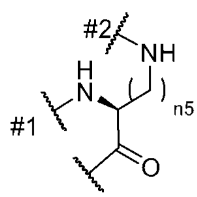

,

the "#1" terminal is linked to $L^2$, and the "#2" terminal is linked to $L^1$;
in the compound of formula (I-1-B), Y is

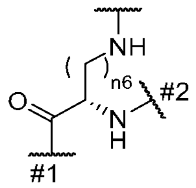

,

the "#1" terminal is linked to $L^2$, and the "#2" terminal is linked to $L^1$;
in the compound of formula (I-1-C), Y is

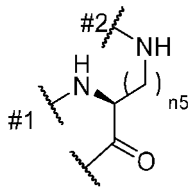

,

the "#1" terminal is linked to $L^2$, and the "#2" terminal is linked to $L^1$;
in the compound of formula (I-1-A), (I-1-B), or (I-1-C), the variables are as defined in any one of the above embodiments.

The present disclosure further provides a compound of any one of the following structures:

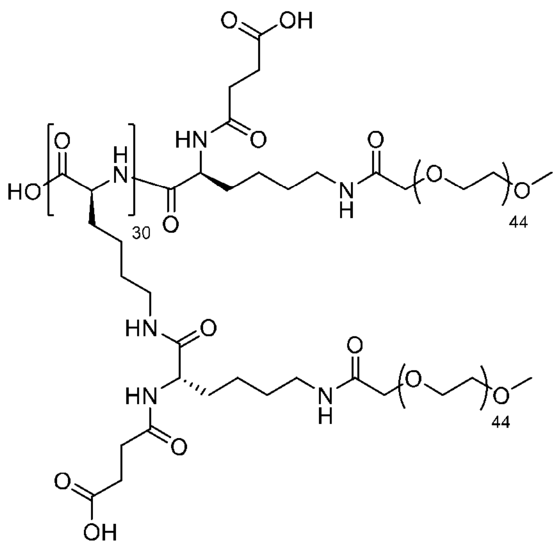

,

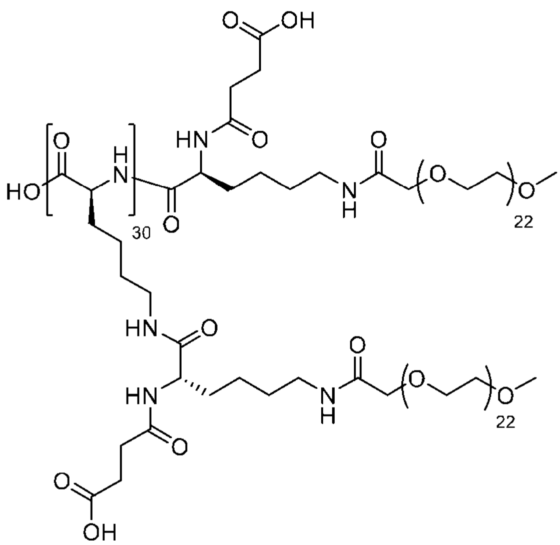

,

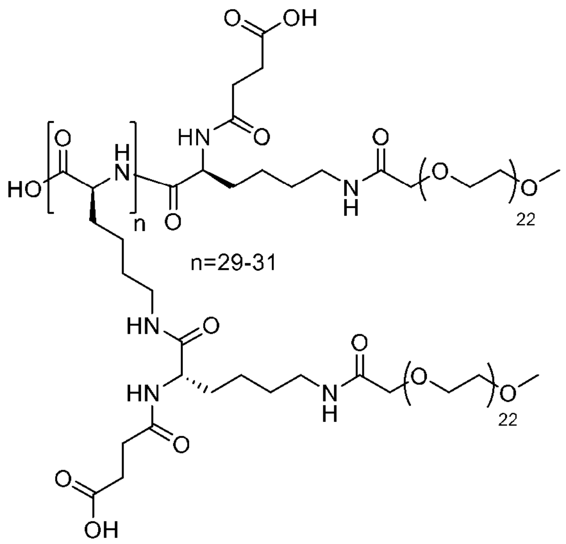

,

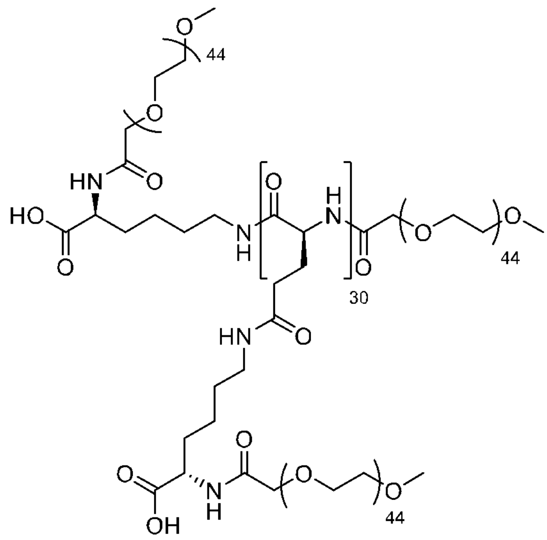

,

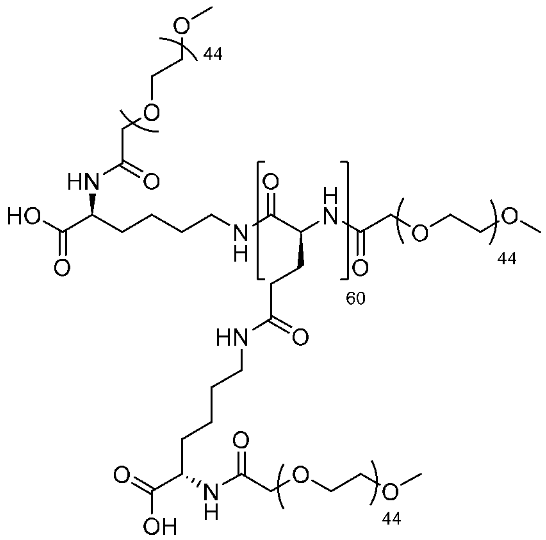

,

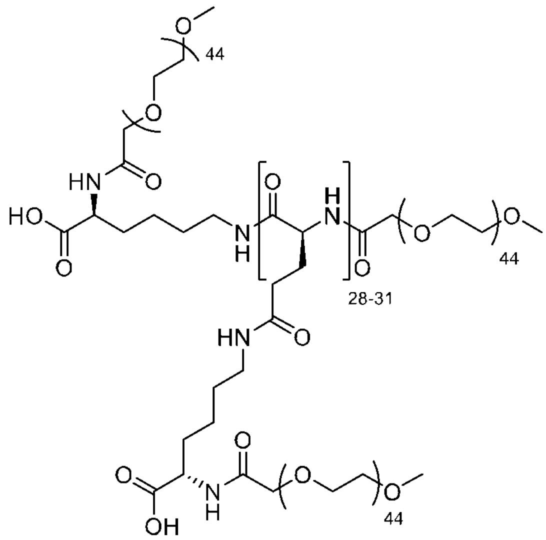

,

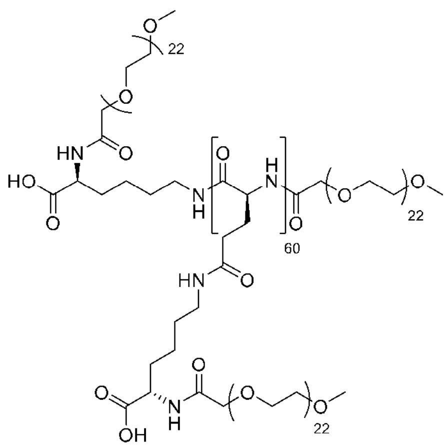

,

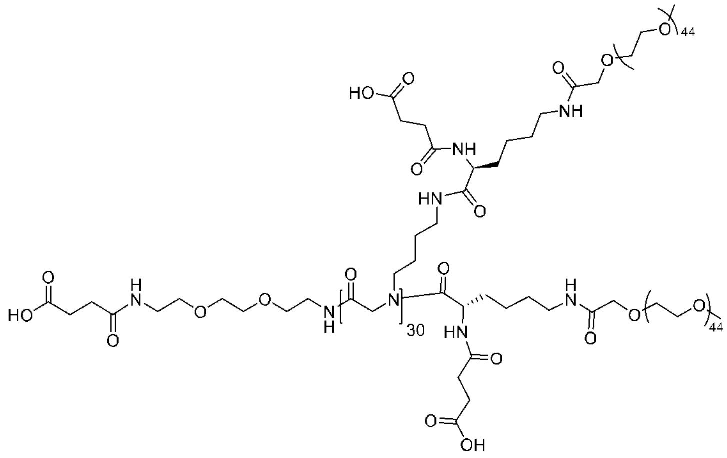

,

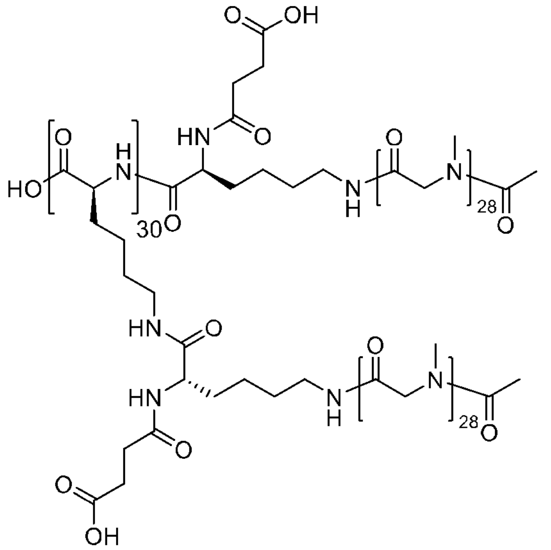

,

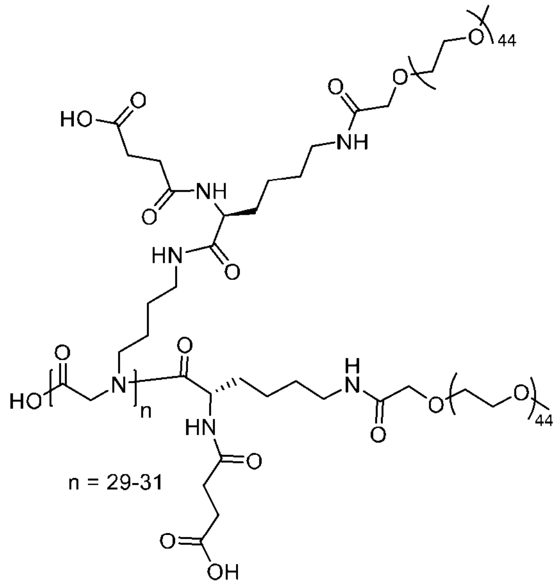

n = 29-31

,

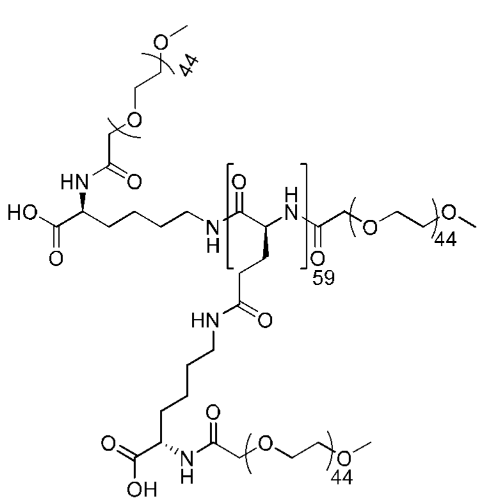

,

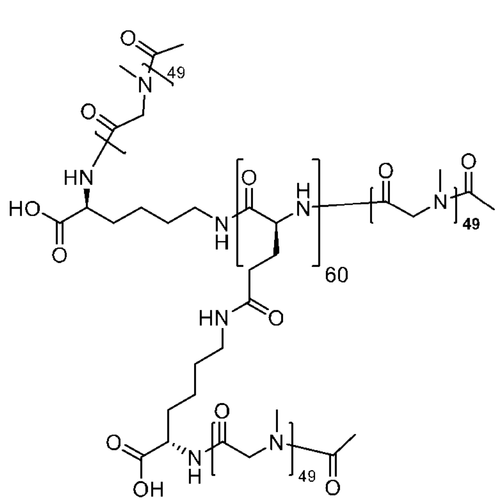

,

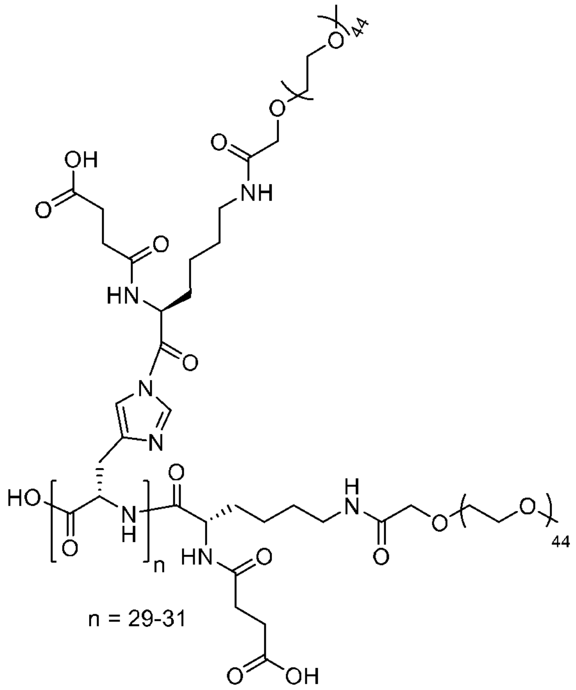

,

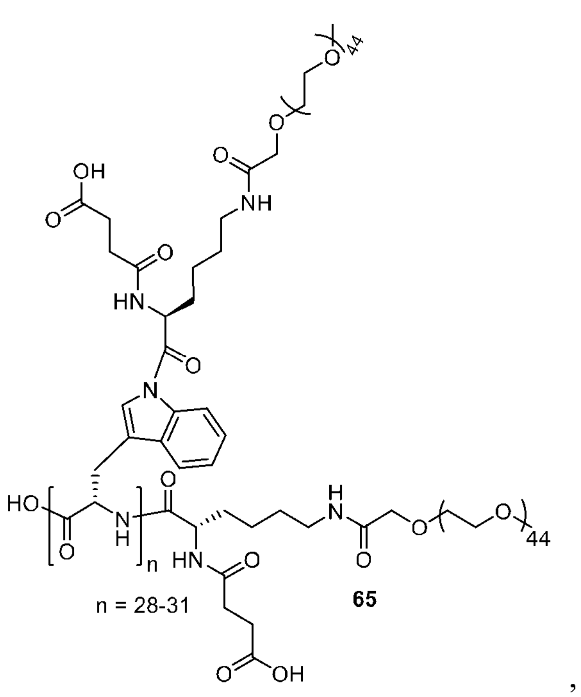

65

,

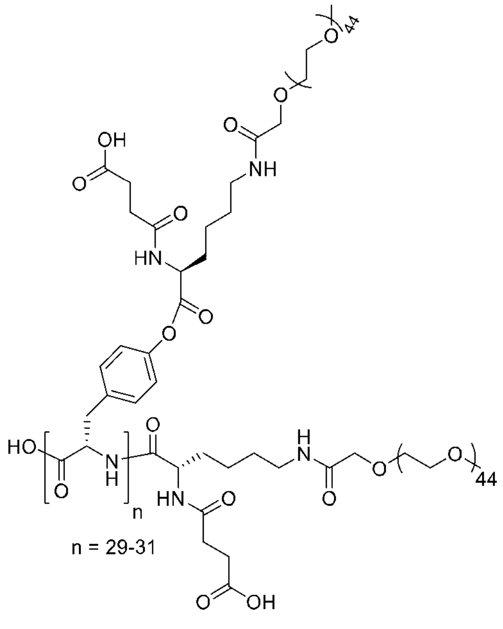

,

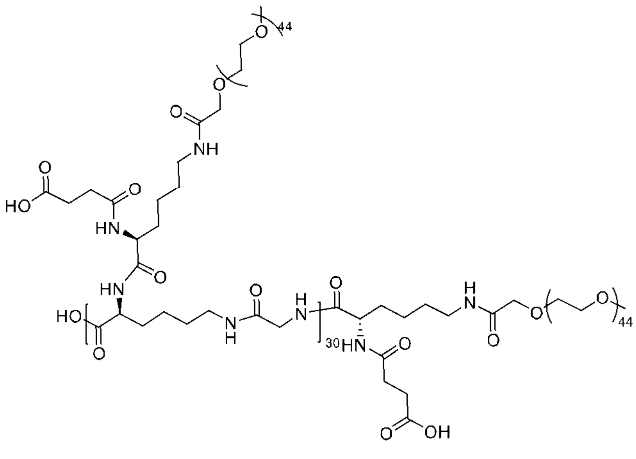

,

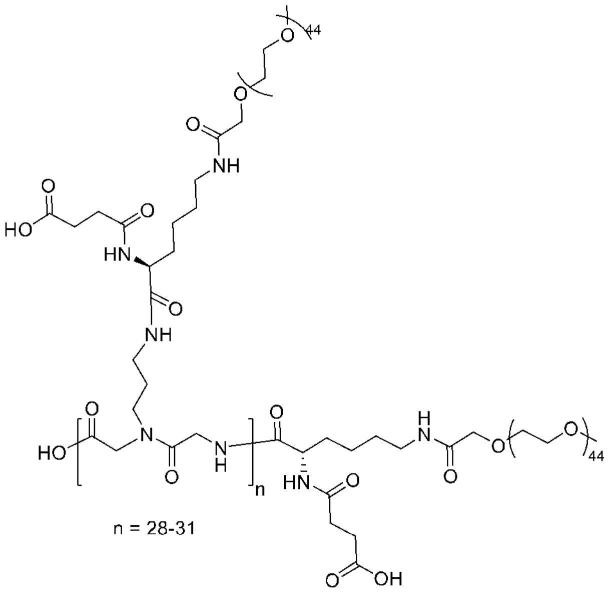

,

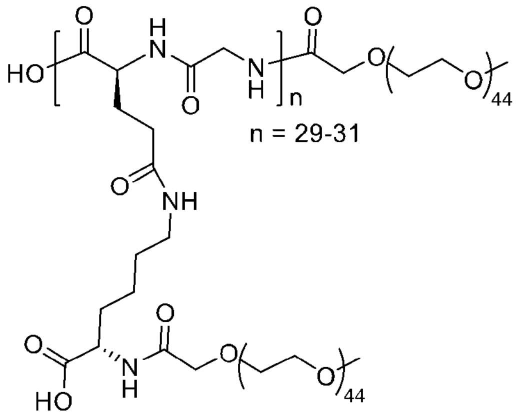

,

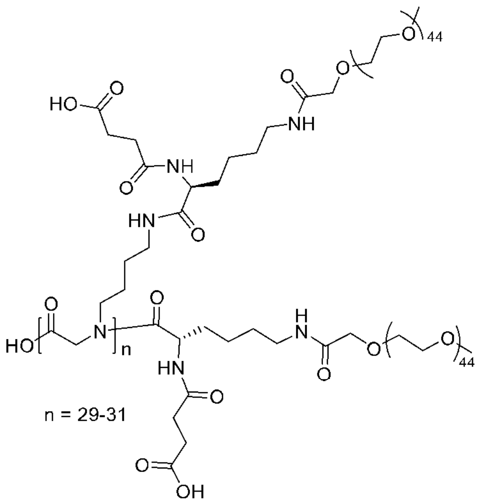

, or

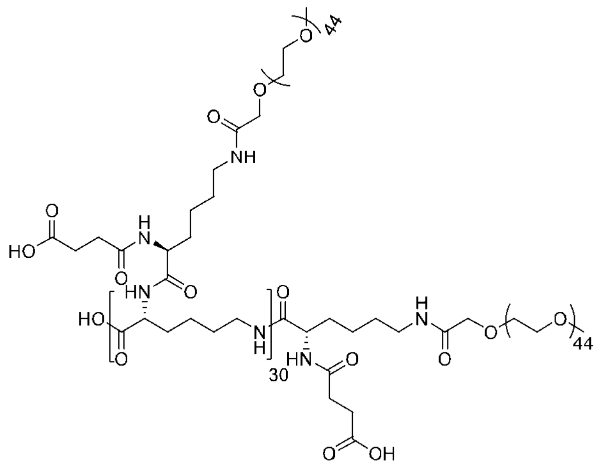

.

The terms used in the present disclosure have the following meanings, unless otherwise stated:

The term "repeating unit number n" may also be interpreted as the degree of polymerization (DP). In the present disclosure, unless otherwise specified, the repeating unit number n denotes the number-average degree of polymerization, i.e., the average of the repeating unit number contained in the polymer macromolecule chain. When ε-poly-L-lysine is synthesized by solid phase synthesis, the repeating unit number n is a single value, and when ε-poly-L-lysine is synthesized by biological fermentation or other chemical polymerization manners, the repeating unit number n may have a certain distribution, and the value is a number-average value.

The amino protecting group, hydroxy protecting group, and carboxyl protecting group of the present disclosure are known groups in the art and suitable for amino, hydroxy, and carboxyl protection. See the Protective Groups in Organic Synthesis, 5th Ed. T. W. Greene & P. G. M. Wuts, for various protecting groups.

The term **"alkyl"** refers to a saturated aliphatic hydrocarbon group, including linear and branched groups of 1-30 carbon atoms, preferably alkyl containing 1-10 carbon atoms, more preferably 1-8 carbon atoms. Non-limiting examples include, but are not limited to: methyl, ethyl, *n*-propyl, *n*-butyl, isobutyl, *tert*-butyl, *sec*-butyl, *n*-pentyl, 2-methylbutyl, 3-methylbutyl, 1,1-dimethylpropyl, 1,2-dimethylpropyl, 2,2-dimethylpropyl, 1-ethylpropyl, *n*-hexyl, 2-methylpentyl, 3-methylpentyl, 4-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1,1,2-trimethylpropyl, 1-ethyl-2-methylpropyl, *n*-heptyl, 2-methylhexyl, 3-methylhexyl, 4-methylhexyl, 5-methylhexyl, 2,2-dimethylpentyl, 2,3-dimethylpentyl, 2,4-dimethylpentyl, 3,3-dimethylpentyl, 3,4-dimethylpentyl, 2-ethylpentyl, 3-ethylpentyl, octyl, nonyl, decyl, undecyl, dodecyl, and their various isomers, etc. The "alkyl" may be substituted or unsubstituted.

The term **"cycloalkyl"** refers to a saturated or partially unsaturated monocyclic or polycyclic substituent

containing 3-20 carbon atoms, preferably 3-12 carbon atoms, more preferably 3-10 carbon atoms, and most preferably 3-6 carbon atoms. Non-limiting examples of monocyclic cycloalkyl include, but are not limited to: cyclopropyl, cyclobutyl, cyclopentyl, cyclopentenyl, cyclohexyl, cyclohexenyl, cyclohexadienyl, cycloheptyl, cyclooctyl, etc. Non-limiting examples of polycyclic cycloalkyl include, but are not limited to: spiro, fused, and bridged cycloalkyl. The "cycloalkyl" may be substituted or unsubstituted.

The term **"alkenyl"** denotes an alkyl group as defined herein consisting of at least two carbon atoms and at least one carbon-carbon double bond, preferably $C_2$-$C_{10}$ alkenyl, and more preferably $C_2$-$C_6$ alkenyl, e.g., ethenyl, propenyl, 1-propenyl, etc. The "alkenyl" may be substituted or unsubstituted.

The term **"alkynyl"** denotes an alkyl group as defined herein consisting of at least two carbon atoms and at least one carbon-carbon triple bond, preferably $C_2$-$C_{10}$ alkynyl, and more preferably $C_2$-$C_6$ alkynyl, e.g., ethynyl, 1-propynyl, 2-propynyl, etc. The "alkynyl" may be substituted or unsubstituted.

The term **"heterocycloalkyl"** refers to a saturated or partially unsaturated monocyclic or polycyclic hydrocarbon substituent containing 3-20 ring atoms, wherein one or more ring atoms are selected from heteroatom(s) of N, O, Si, B, $S(O)_m$, and $P(O)_m$ (wherein m is an integer of 0-2), excluding ring portions of -O-O, -O-S-, or -S-S-, while the remaining ring atoms are carbon. Preferably, the heterocycloalkyl contains 3-12 ring atoms, including 1-4 heteroatoms. Non-limiting examples of monocyclic heterocycloalkyl include pyrrolyl, piperidinyl, piperazinyl, morpholinyl, tetrahydrofuryl, pyranyl, etc. Polycyclic heterocycloalkyl includes spiro, fused, and bridged heterocycloalkyl. The "heterocycloalkyl" may be substituted or unsubstituted.

The term **"alkoxy"** refers to -O-(alkyl) and -O-(cycloalkyl), wherein alkyl and cycloalkyl are as defined herein. Non-limiting examples include, but are not limited to, methoxy, ethoxy, propoxy, butoxy, cyclopropyloxy, cyclobutoxy, cyclopentyloxy, cyclohexyloxy, etc. The "alkoxy" may be substituted or unsubstituted.

The term **"alkylsulfhydryl"** refers to -S-(alkyl) and -S-(cycloalkyl), wherein alkyl and cycloalkyl are as defined herein. Non-limiting examples include, but are not limited to, methylsulfhydryl, ethylsulfhydryl, propylsulfhydryl, butylsulfhydryl, cyclopropylsulfhydryl, cyclobutylsulfhydryl, cyclopentylsulfhydryl, cyclohexylsulfhydryl, etc. The "alkylsulfhydryl" may be substituted or unsubstituted.

The term **"substituted or unsubstituted amino"** refers to $NH_2$, mono-substituted $NH_2$, and di-substituted $NH_2$. In the substituted case, the substituents in the mono- or di-substituted amino are preferably independently selected from deuterium, alkyl, hydroxy, sulfhydryl, alkenyl, alkynyl, alkoxy, alkylsulfhydryl, alkylamino, cycloalkyl, heterocycloalkyl, aryl, heteroaryl, cycloalkoxy, heterocycloalkoxy, cycloalkylsulfhydryl, heterocycloalkylsulfhydryl, oxo, amino, haloalkyl, hydroxyalkyl, carboxyl, and carboxylate ester group, etc. The substituents in di-substituted amino may, together with the nitrogen atom to which they are linked, form a non-aromatic heterocyclic structure.

The term **"aryl"** refers to any group of a stable conjugated hydrocarbon ring system containing 6-18 carbon atoms, preferably 6-10 carbon atoms, which may be monocyclic, bicyclic, tricyclic, or higher aromatic groups such as phenyl, naphthyl, anthracene, and the like. The aryl may include aryl having heterocycloalkyl or cycloalkyl on the aromatic ring. The "aryl" may be substituted or unsubstituted.

The term **"heteroaryl"** refers to an aromatic ring system formed by the replacement of at least 1 ring carbon atom with a heteroatom selected from N, O, and S, preferably a 5- to 7-membered monocyclic structure or a 7- to 12-membered bicyclic structure, and more preferably a 5- to 6-membered heteroaryl, such as pyrrolyl, imidazolyl, pyridyl, pyrimidinyl, thiazolyl, thienyl, pyrazinyl, triazolyl, tetrazolyl, oxazolyl, indazolyl, etc. The heteroaryl may include a fused heteroaryl of a heteroaryl to a heteroaryl, heterocycloalkyl, or cycloalkyl ring. The "heteroaryl" may be substituted or unsubstituted.

The term **"sulfonyl"** refers to

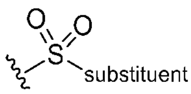,

wherein the substituent is preferably alkyl, alkenyl, alkynyl, amino, alkoxy, alkylsulfhydryl, alkylamino, cycloalkyl, haloalkyl, heterocycloalkyl, aryl, heteroaryl, cycloalkoxy, heterocycloalkoxy, cycloalkylsulfhydryl, or heterocycloalkylsulfhydryl.

The term **"sulfinyl"** refers to

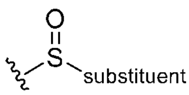,

wherein the substituent is preferably alkyl, alkenyl, alkynyl, amino, alkoxy, alkylsulfhydryl, alkylamino, cycloalkyl, haloalkyl, heterocycloalkyl, aryl, heteroaryl, cycloalkoxy, heterocycloalkoxy, cycloalkylsulfhydryl, or heterocycloalkylsulfhydryl.

The term **"alkylsulfinyl"** refers to

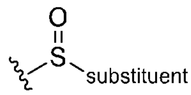

,

wherein the substituent is preferably alkyl, as defined above.

The term **"hydroxy"** refers to -OH.

The term **"halogen"** refers to fluorine, chlorine, bromine, or iodine.

The term **"nitro"** refers to $-NO_2$.

The term **"amino"** refers to $-NH_2$.

The term **"cyano"** refers to -CN.

The term **"carboxylic acid"** refers to -C(O)OH.

The term **"sulfhydryl"** refers to -SH.

The term **"carboxylate ester group"** refers to -C(O)O-alkyl, aryl, or cycloalkyl, wherein the alkyl, aryl, and cycloalkyl are as defined above.

The term **"thioester"** refers to -C(O)S-alkyl, aryl, or cycloalkyl, wherein the alkyl, aryl, and cycloalkyl are as defined above.

The term **"sulfate"** refers to $O-S(O)_2O$-alkyl, aryl, or cycloalkyl, wherein the alkyl, aryl, and cycloalkyl are as defined above.

The term **"sulfonate"** refers to $S(O)_2O$-alkyl, aryl, or cycloalkyl, wherein the alkyl, aryl, and cycloalkyl are as defined above.

The term **"boronic acid"** refers to $B(OH)_2$.

The term **"borate"** refers to $B(OR)_2$, wherein R = alkyl, aryl, or cycloalkyl, and the alkyl, aryl, and cycloalkyl are as defined above.

The term **"substituted"** refers to one or more hydrogen atoms in a group being independently substituted with a corresponding number of deuterium atoms or substituents.

The **"pharmaceutically acceptable salt"** refers to a salt that retains the biological efficacy of the free base with no additional adverse effects, and may be an acidic group, a basic group, or an amphoteric group. Non-limiting examples include, but are not limited to: a salt with an acid, including hydrochloride, hydrobromide, sulfate, pyrosulfate, bisulfate, sulfite, bisulfite, phosphate, hydrogen phosphate, dihydrogen phosphate, metaphosphate, pyrophosphate, nitrate, acetate, propionate, decanoate, octanoate, formate, acrylate, isobutyrate, hexanoate, heptanoate, oxalate, malonate, succinate, suberate, benzoate, methylbenzoate, phthalate, maleate, methanesulfonate, *p*-toluenesulfonate, benzenesulfonate, (*D*, *L*)-tartarate, citrate, maleate, (*D*, *L*)-malate, fumarate, stearate, oleate, cinnamate, laurate, glutamate, aspartate, trifluoromethanesulfonate, mandelate, ascorbate, salicylate, etc. When the compound of the present disclosure contains an acidic group, the pharmaceutically acceptable salt thereof may further include: an alkali metal salt (e.g., sodium salt or potassium salt), an alkaline earth metal salt (e.g., calcium salt or magnesium salt), and an organic base salt (e.g., alkyl, aryl, amino, amino acid, etc.).

The **"pharmaceutical composition"** refers to a mixture containing one or more of the compounds described herein or a physiologically/pharmaceutically acceptable salt or pro-drug thereof, and other chemical components, for example physiologically/pharmaceutically acceptable carriers and excipients. The pharmaceutical composition is intended to promote the administration to an organism and facilitate the absorption of the active ingredient to exert the bioactivity.

The abbreviations for any protecting groups, amino acids, and other compounds used herein, unless otherwise specified, are provided on the basis of their commonly used and accepted abbreviations, or by referring to IUPAC-IUBC Commission on Biochemical Nomenclature (see Biochem. 1972, 11, 942-944).

The positive and progressive effects of the present disclosure are as follows: The compound of the present disclosure has one or more of the following advantages:

(1) Through reasonable design of polypeptide molecules and linkers, the control of the number of conjugated groups and the nano-scale size can be achieved;
(2) The nanoparticle formed by the compound of the present disclosure has good stability;
(3) The compound of the present disclosure has good tumor tissue infiltration capacity;
(4) The compound of the present disclosure has good tumor inhibitory effects and controllable adverse effects.

BRIEF DESCRIPTION OF THE DRAWINGS

FIG. 1 illustrates the nano-scale particle size distribution of the target compound of Example 7
FIG. 2 illustrates the nano-scale particle size distribution of the target compound of Example 20
FIG. 3 illustrates the nano-scale particle size distribution of the target compound of Example 27
FIG. 4 illustrates the nano-scale particle size distribution of the target compound of Example 40
FIG. 5 illustrates the nano-scale particle size distribution of the target compound of Example 41
FIG. 6 illustrates the nano-scale particle size distribution of the target compound of Example 48
FIG. 7 illustrates the nano-scale particle size distribution of the target compound of Example 50
FIG. 8 illustrates the nano-scale particle size distribution of the target compound of Example 75
FIG. 9 illustrates the nano-scale particle size distribution of the target compound of Comparative Example 78
FIG. 10 illustrates the particle size distribution of the target compound of Example 71 on day 0 of preservation at 25 °C
FIG. 11 illustrates the particle size distribution of the target compound of Example 71 on day 30 of preservation at 25 °C
FIG. 12 illustrates the particle size distribution of the target compound of Example 71 on day 60 of preservation at 25 °C
FIG. 13 illustrates the particle size distribution of the target compound of Example 71 on day 90 of preservation at 25 °C
FIG. 14 illustrates the inhibitory effects of the target compound of Example 20, the target compound of Example 52, and Abraxane in a BxPC-3 tumor model
FIG. 15 illustrates the inhibitory effects of the target compound of Example 71 and the target compound of Example 78 in a HepG2 tumor model
FIG. 16 illustrates the inhibitory effects of the target compound of Example 71 and irinotecan hydrochloride in a human colon cancer HT-29 tumor model
FIG. 17 illustrates the inhibitory effects of the target compound of Example 71 and an irinotecan liposome in a human myeloma NCI-H929 tumor model
FIG. 18 illustrates the inhibitory effects of the target compound of Example 71 and an irinotecan liposome in a human small cell lung cancer NCI-H69 tumor model
FIG. 19 illustrates the inhibitory effect of the target compound of Example 79 in a human fibrosarcoma HT-1080 tumor model
FIG. 20 illustrates the inhibitory effect of the target compound of Example 80 in a human small cell lung cancer NCI-H69 tumor model
FIG. 21 illustrates the inhibitory effects of the target compounds of Example 82 and others in a human small cell lung cancer NCI-H69 tumor model
FIG. 22 illustrates the inhibitory effects of the target compounds of Example 84 and others in a human breast cancer cell BCaP-37 tumor model
FIG. 23 illustrates the inhibitory effects of the target compounds of Examples 80 and 93 in a human breast cancer cell MCF-7 tumor model
FIG. 24 illustrates the inhibitory effects of the target compounds of Example 100 and others in a human liver cancer cell HepG2 tumor model
FIG. 25 illustrates a nano-scale particle size study on the target compound of Example 79
FIG. 26 illustrates a nano-scale particle size study on the target compound of Example 80
FIG. 27 illustrates a nano-scale particle size study on the target compound of Example 84
FIG. 28 illustrates a nano-scale particle size study on the target compound of Example 85
FIG. 29 illustrates a nano-scale particle size study on the target compound of Example 97
FIG. 30 illustrates a nano-scale particle size study on the target compound of Example 112
FIG. 31 illustrates the particle size stability data of the target compound of Example 79 on day 0 at 25 °C
FIG. 32 illustrates the particle size stability data of the target compound of Example 79 on day 60 at 25 °C
FIG. 33 illustrates the particle size stability data of the target compound of Example 80 on day 0 at 25 °C
FIG. 34 illustrates the particle size stability data of the target compound of Example 80 on day 60 at 25 °C
FIG. 35 illustrates the inhibitory effects of the target compounds of Example 71 and others in a human breast cancer cell MCF-7 tumor model
FIG. 36 illustrates the inhibitory effects of the target compounds of Example 79 and others in a human small cell lung cancer NCI-H69 tumor model
FIG. 37 illustrates the inhibitory effect of the target compound of Example 119 in a human liver cancer cell HepG2 tumor model

DETAILED DESCRIPTION

The following examples are intended to further illustrate the present disclosure, rather than limit the present disclosure. Experimental procedures without specified conditions in the following examples were performed in accordance with conventional procedures and conditions, or in accordance with the product instructions.

The abbreviations used in the present disclosure are shown in the following table:

| Abbreviation | Full version |
|---|---|
| Cit, C | Citrulline |
| Phe | Phenylalanine |
| Lys | Lysine |
| Arg | Arginine |
| Val, V | Valine |
| Ala | Alanine |
| Ile | Isoleucine |
| Trp | Tryptophan |
| Asn | Asparagine |
| Pro | Proline |
| Nle | *n*-Leucine |
| Glu | Glutamic acid |
| Gly | Glycine |
| Leu | Leucine |
| Asp | Aspartic acid |
| BODIPY | Dipyrrometheneboron difluoride dyes |
| cyanine | Cyanine dyes |
| PBD | pyrrolobenzodiazepine |
| PEG | Polyethylene glycol |
| mPEG | Polyethylene glycol monomethyl ether |
| mPEG-1K | Polyethylene glycol monomethyl ether with a number-average molecular weight of 1000 |
| mPEG-2K | Polyethylene glycol monomethyl ether with a number-average molecular weight of 2000 |
| DOX | Doxorubicin |
| PAB | 4-Aminobenzyl alcohol |
| DMEDA | *N*,*N*-Dimethyl ethylenediamine |
| SN-38 | 7-Ethyl-10-hydroxycamptothecin |
| PTX | Paclitaxel |
| CDX | Cell line-derived xenograft model |
| MTBE | Methyl *tert*-butyl ether |
| DMF | *N,N'*-Dimethylformamide |
| DCM | Dichloromethane |
| DIPEA | *N*,*N*-Diisopropylethylamine |
| DMSO | Dimethyl sulfoxide |
| THF | Tetrahydrofuran |
| EA | Ethyl acetate |

(continued)

| Abbreviation | Full version |
|---|---|
| TFA | Trifluoroacetic acid |
| DEA | Diethylamine |
| DMAP | 4-Dimethylaminopyridine |
| Boc | *tert*-Butoxycarbonyl |
| Cbz | Benzyloxycarbonyl |
| DCC | Dicyclohexylcarbodiimide |
| NHS | *N*-Hydroxysuccinimide |
| EDCI | 1-(3-Dimethylaminopropyl)-3-ethylcarbodiimide hydrochloride |
| PyBOP | Benzotriazol-1-yl-oxytripyrrolidino-phosphonium hexafluorophosphate |
| TEMPO | Tetramethylpiperidine oxide |
| EEDQ | 2-Ethoxy-1-ethoxycarbonyl-1,2-dihydroquinoline |
| Melphalan | Melphalan |
| Teoc | 2-(trimethylsilyl)ethoxycarbonyl |
| Cisplatin | Cisplatin |
| Docetaxel | Docetaxel |
| EtOAc | Ethyl acetate |
| pNB | *p*-Nitrophenol |
| DME | Ethylene glycol dimethyl ether |
| Fmoc | Fluorenylmethoxycarbonyl |
| DIPEA | *N*,*N*-Diisopropylethylamine |
| TBSCl | *tert*-Butyldimethylsilyl chloride |
| BHA | Benzhydrylamine |
| ESI | Electrospray ionization |
| MS | Mass spectrometer |
| DGA | Diglycolic acid |
| SA | Succinic acid |
| VC | Valine-citrulline |
| PBS | Phosphate-buffered saline |
| PB | Phosphate buffer |
| Cys | Cysteine |
| EDTA | Ethylenediaminetetraacetic acid disodium salt |
| DTT | Dithiothreitol |
| HOBt | 1-Hydroxybenzotriazole |
| DIC | *N*,*N*'-Diisopropylcarbodiimide |
| HBTU | Benzotriazol-*N,N,N',N'*-tetramethyluronium hexafluorophosphate |
| FmocOSU | 9-Fluorenylmethyl-*N*-succinimidyl carbonate |
| TSTU | 2-Succinimidyl-1,1,3,3-tetramethyluronium tetrafluoroborate |

Since the present disclosure has been described by means of specific embodiments, certain modifications and equivalent variations will be apparent to those skilled in the art and shall fall within the scope of the present disclosure.

**Methodology for drug molecule assay by HPLC:**

Instrument: Agilent 1260 HPLC;
Column: two-way valve, 1 m pulse dampener, column temperature: 40 °C;
Phase A: methanol, flow rate: 0.3 mL/min, detection wavelength: 254 nm, isocratic: 0.0-5.0 min, 100%, phase A.

**General methodology for compound purity assay by HPLC:**

Chromatography conditions:

| | |
|---|---|
| Instrument | Agilent high performance liquid chromatograph 1260 |
| Chromatography column | Welch XB-C8, 3*100 mm 300A 3 um |
| DAD detector (wavelength 200-400 nm) | Wavelength: 210 nm and 254 nm |
| Flow rate | 0.7 mL/min |
| Column temperature | 40 °C |
| Sample tray temperature | Room temperature |
| Injection volume | 5 μL |
| Run time | 19 min |

Solvent gradient conditions:

| Time (min) | Mobile phase A (%) | Mobile phase B (%) |
|---|---|---|
| 0 | 90 | 10 |
| 2 | 90 | 10 |
| 10 | 10 | 90 |
| 14 | 10 | 90 |
| 14.1 | 90 | 10 |
| 19 | 90 | 10 |

The structures of all compounds of the present disclosure were determined by nuclear magnetic resonance (NMR) or mass spectrometry (MS). The NMR shifts (δ) are given in $10^{-6}$ (ppm). The instrument for NMR was the Bruker AVANCE-400 spectrometer. The deuterated solvents tested were deuterated chloroform ($CDCl_3$), deuterated methanol (MeOD), deuterated dimethyl sulfoxide (DMSO-$D_6$), and the internal standard was tetramethylsilane (TMS).

The low-resolution mass spectrum (MS) was determined on the Agilent 6120 quadruple LCMS mass spectrometer.

The degree of polymerization for the polymer of the present disclosure was calculated by NMR integration. When the NMR integration is A for the terminal group (such as benzyl, benzoyl, Boc, acetyl, etc.) and B for the α hydrogen atom in the amino acid, and the hydrogen atom number of the functional group is p, the degree of polymerization is n = (B × p/A).

Example 1: Synthesis of compound 1

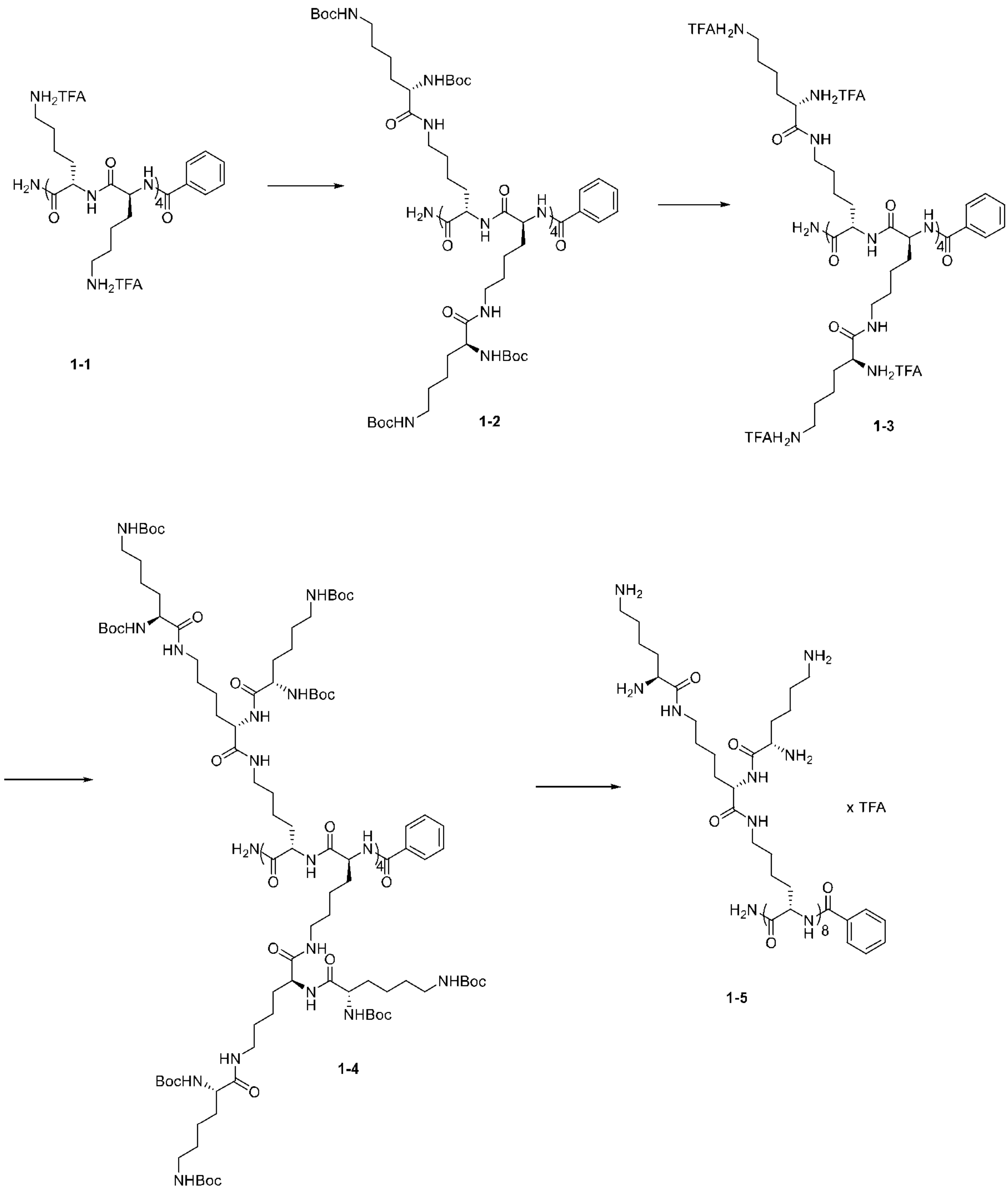

1-1

1-2

1-3

1-4

1-5

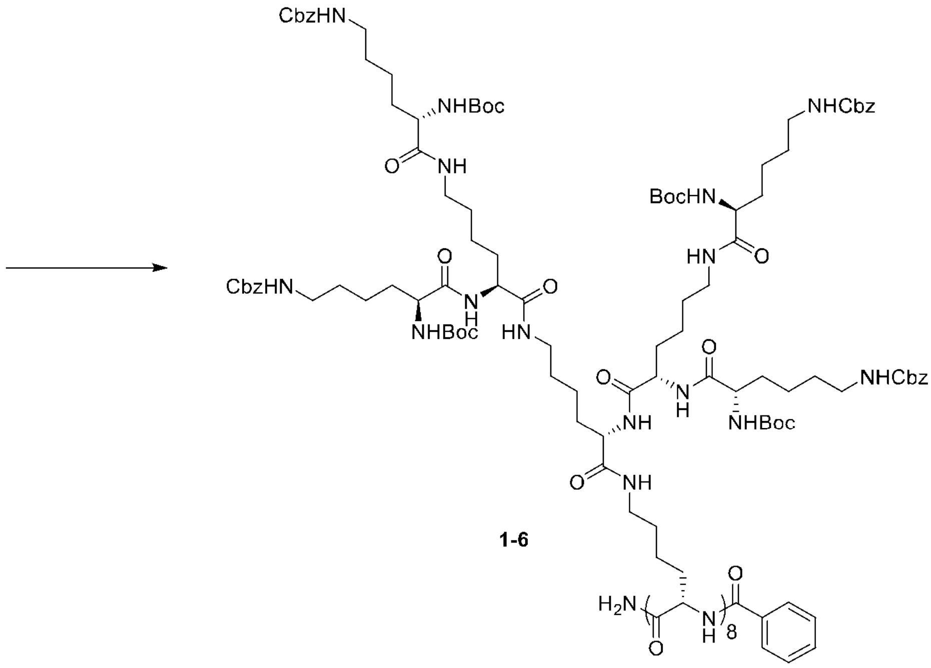

1-6

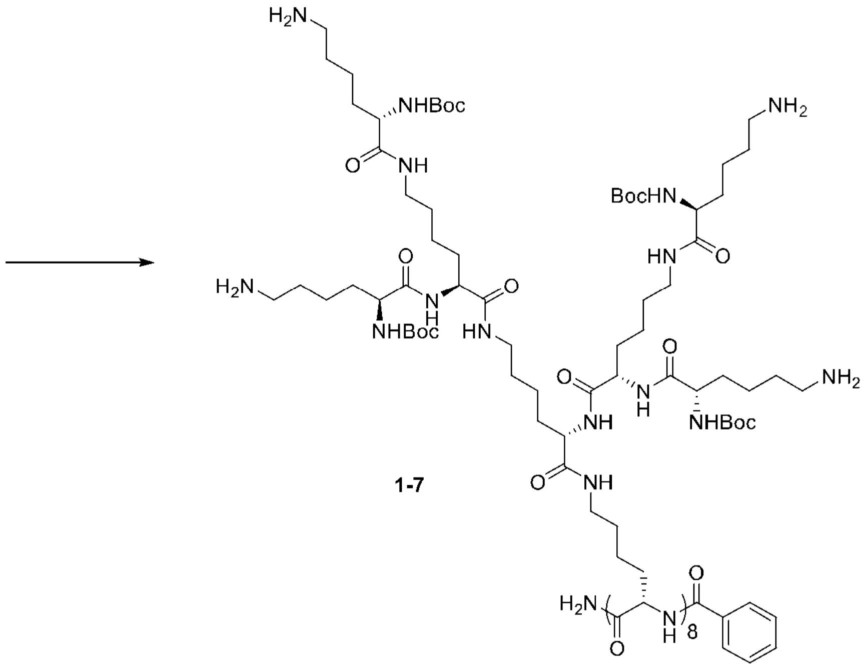

1-7

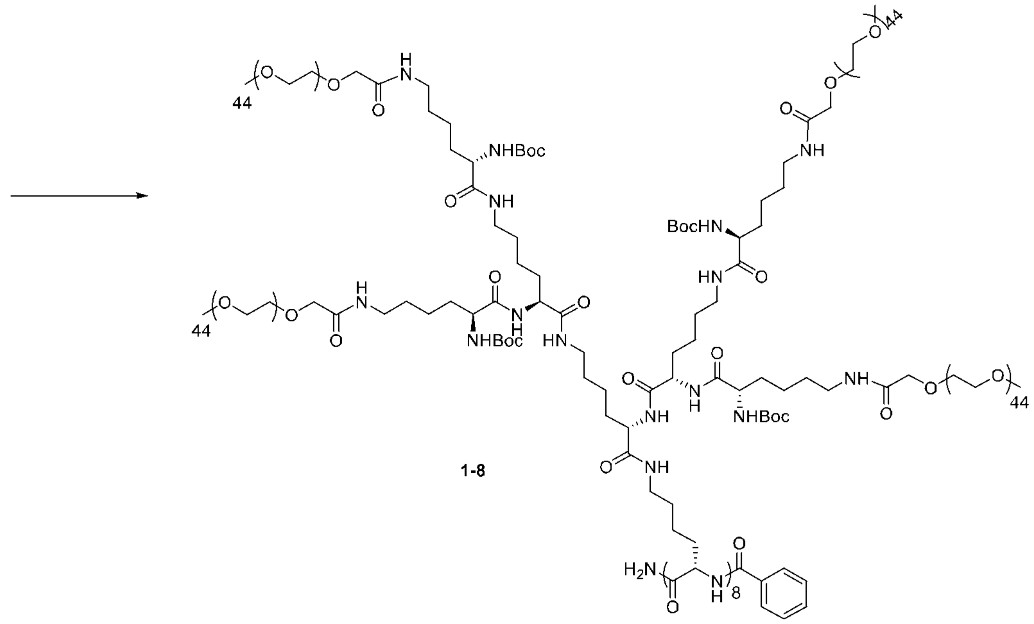

1-8

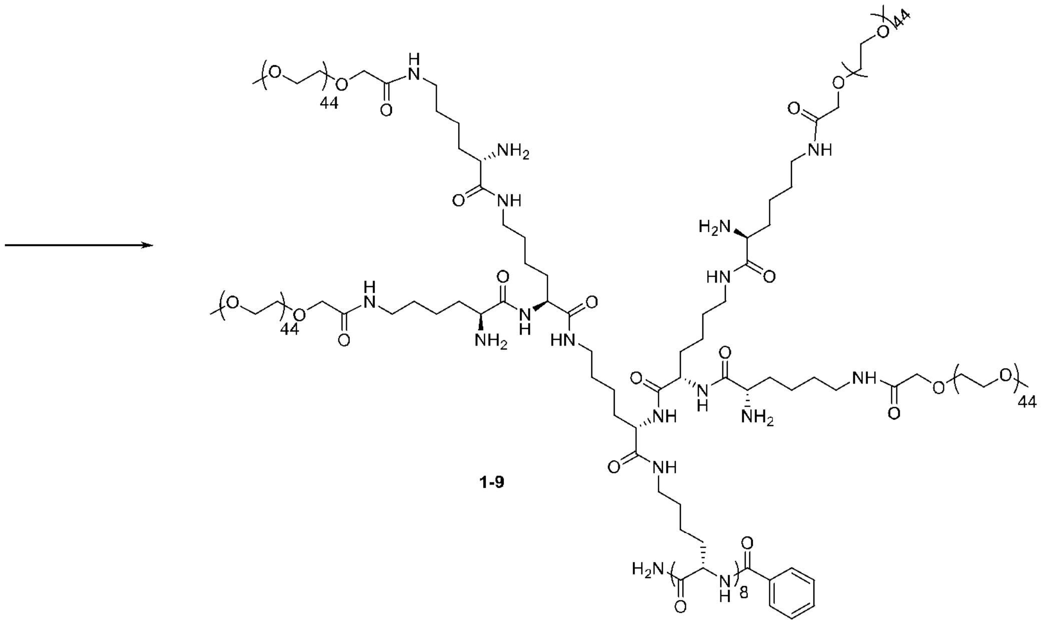

1-9

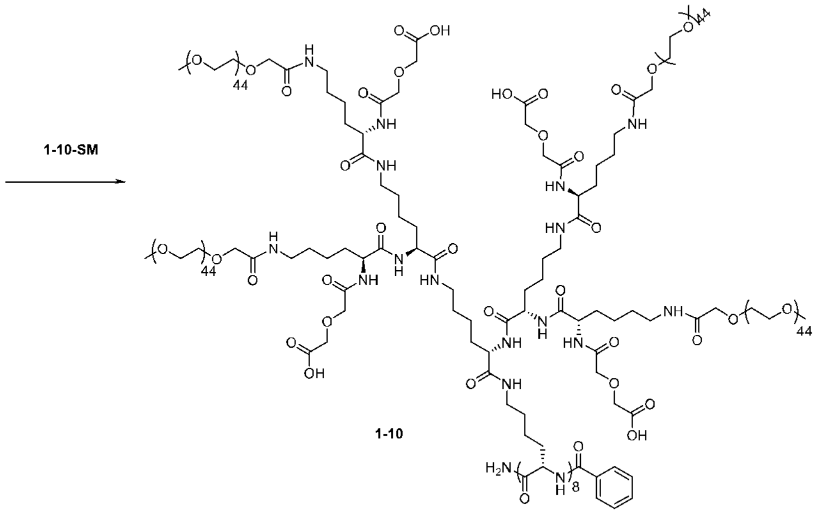


1-10

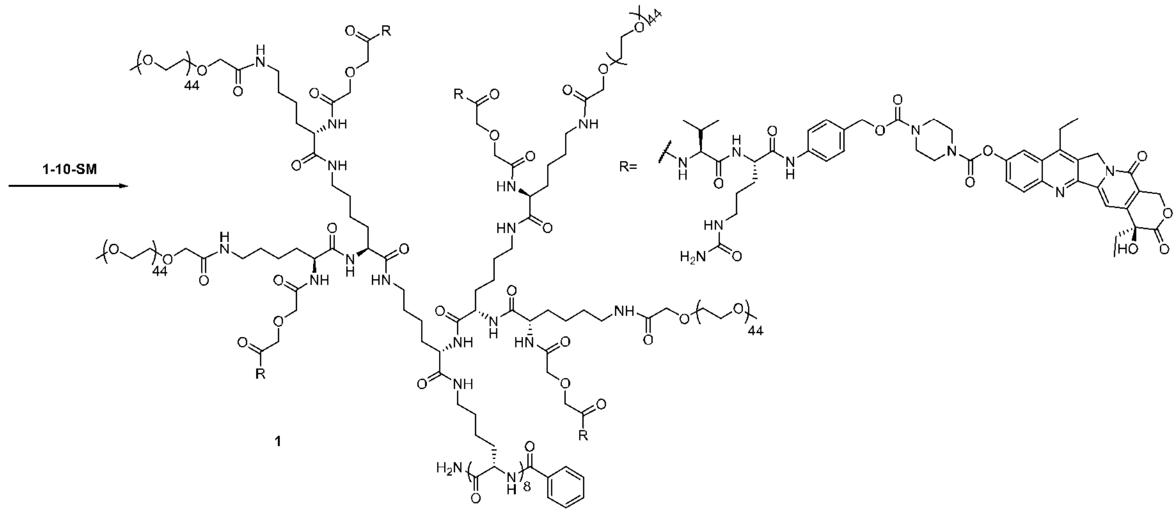


1

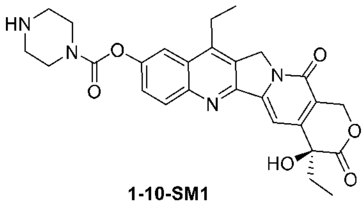

1-10-SM1

+

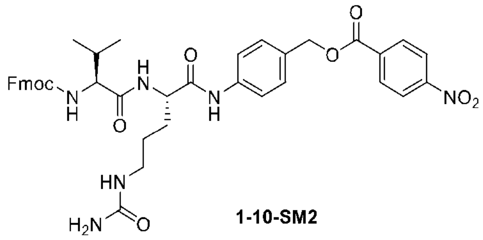

1-10-SM2

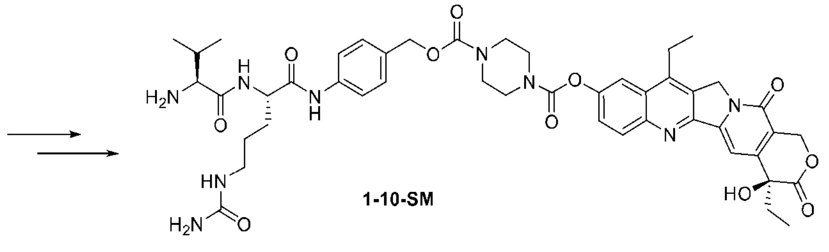

1-10-SM

Synthesis of compound **1-1**

Linear α-octalysine was synthesized according to standard Fmoc solid-phase synthesis, as exemplified by the

following:

The resin used for solid-phase synthesis is MBHA resin (25 g, Loading = 0.65 mmol/g). After the Fmoc protecting group removal, the starting material Fmoc-Lys (Boc)-OH (1.5 eq), the coupling reagents HOBT (1.5 eq) and DIC (1.5 eq), and the solvent DMF were mixed. The mixture was stirred for 2 h with nitrogen bubbling. A small amount of resin was taken and subjected to Kaiser test (chromogenic agent A: 1 g of ninhydrin/100 mL of ethanol; chromogenic agent B: 20 g of phenol/100 mL of ethanol; chromogenic agent C: pyridine; a small amount of resin was added to a chromogenic agent mixture in a volume ratio of A:B:C = 3:2:1, for 3 min of chromogenic reaction at 100 °C). If the resin turned blue, halved amounts of materials were added; no color change indicated that the reaction was completed. The solvent was removed by filtration *in vacuo,* and the residue was subjected to 2 series of sequential washings with DMF, MeOH, DCM, and DMF. Fmoc was removed by adding a 20% piperidine/DMF solution and stirring for 20 min. The washing, coupling, and Fmoc removal procedures were repeated until the Fmoc of the eighth coupled lysine was removed, and the synthesis proceeded to the next reaction.

To a 1 L glass beaker were added benzoic acid (3.25 g, 26.6 mmol, 1.6 eq), HOBT (4.73 g, 35 mmol, 2 eq), and DIC (4.5 g, 35 mmol, 2 eq), and 0.4 L of DMF was then added. The mixture was stirred to allow the complete dissolution of benzoic acid and added to a reactor. A proper amount of DMF was added to ensure that the reaction could persist for 4 h with nitrogen bubbling (the resin on the inner wall of the reactor was flushed into the reaction mixture with a small amount of DMF every 30 min during the reaction). After the reaction was completed, the reaction mixture was drained, and procedure II was conducted: A small amount of resin was taken and subjected to Kaiser test until the result was colorless. The resin was dried *in vacuo* and transferred into a flask. A removing reagent TFA/water/triisopropylsilane = 95/2.5/2.5 was pre-cooled to 0 °C and added to the resin at 1.5 L/kg resin. The mixture was mechanically stirred for 2 h for removal and filtered *in vacuo.* The cake was washed with a proper solvent, and the removal solution was concentrated. Pre-cooled MTBE (more than 10 folds in volume) was added for precipitation, and the mixture was centrifuged to give a crude product. The crude product was dissolved in acetonitrile/water/TFA (50/50/0.1), and the mixture was lyophilized to give compound **1-1** (20.0 g, 60% yield).

Synthesis of compound **1-2**

To a solution of compound **1-1** (2.06 g) and *N*,*N*-di-*tert*-butoxycarbonyl-L-lysine *p*-nitrophenol ester (5.61 g) in DMF (24 mL) was added TEA (2.08 mL). When turning from colorless to deep yellow, the reaction mixture was stirred at 20 °C for 18 h. DCM and water were added to wash the product. The organic phase was dried over anhydrous sodium sulfate, and the solvent was removed by rotavap to give compound **1-2** (3.60 g).

Synthesis of compound **1-3**

To a solution of compound **1-2** (3.6 g) in DCM (150 mL) was added TFA (50 mL). When a gas was produced and the solution turned turbid, the reaction mixture was stirred at room temperature for 18 h and concentrated by rotavap to remove most of the solvent. Methyl *tert*-butyl ether was added for precipitation. The mixture was centrifuged, and the supernatant was discarded. To a solution of the precipitate in methanol was added methyl *tert*-butyl ether for another precipitation. The mixture was dried *in vacuo* to give compound **1-3** (3.98 g).

Synthesis of compound **1-4**

To a solution of compound **1-3** (1.32 g) and *N,N*-di-*tert*-butoxycarbonyl-L-lysine *p*-nitrophenol ester (3.83 g) in DMF (20 mL) was added TEA (2.121 mL). When the yellow color turned deeper, the reaction mixture was stirred for 18 h at room temperature. DCM and water were added to wash the product. The organic phase was dried over anhydrous sodium sulfate, and the solvent was removed by rotavap to give a crude product of compound **1-4,** which was directly used in the next step.

Synthesis of compound **1-5**

To a solution of compound **1-4** in DCM (30 mL) was added TFA (20 mL). The reaction mixture was stirred at room temperature overnight and concentrated by rotavap to remove the solvent. The residue was repeatedly washed with methyl *tert*-butyl ether and dried *in vacuo* to give compound **1-5** (white solid, 2.14 g).

Synthesis of compound **1-6**

To a solution of compound **1-5** (2.14 g) and BOC-LYS(Z)-ONP (6.96 g) in DMF (40 mL) was added TEA (3.84 mL). The reaction mixture was stirred at 25 °C for 18 h under nitrogen atmosphere. DCM and water were added for washing. The

mixture was concentrated by rotavap, washed with acetonitrile, and dried *in vacuo* to give compound **1-6** (white powder, 3.66 g).

Synthesis of compound **1-7**

To acetic acid (21 mL) was added compound **1-6** (1.50 g), and 10% Pd/C (750 mg) was then added. The mixture was purged with hydrogen and stirred at 25 °C for 24 h. Celite was added, and the mixture was filtered through a 0.45 μm filter membrane *in vacuo.* The cake was washed with methanol. The filtrates were combined, concentrated by rotavap, and dried *in vacuo* to give compound **1-7** (1.56 g, pale yellow solid).

Synthesis of compound **1-8**

To a solution of compound **1-7** (0.8 g, 69.3 μmol) in DMSO/DMF (25/25 mL) were added $mPEG_{2k}$-NHS (14.08 g, 3.33 mmol) and DIPEA (4.73 mL, 28.6 mmol). The mixture was stirred at room temperature for 24 h. Methyl *tert*-butyl ether (4V) was added to precipitate the product. The mixture was frozen at -20 °C for 1 h and centrifuged. The solid was suspended in methanol, and methyl *tert*-butyl ether was added to precipitate the product. The mixture was centrifuged, and the precipitate was dissolved in water and lyophilized to give compound **1-8** (7.87 g).

Synthesis of compound **1-9**

To a solution of compound **1-8** (4 g, 51.59 μmol) in DCM (32 mL) was added TFA (32 mL). The reaction mixture was stirred at room temperature for 12 h. The mixture was concentrated by rotavap to remove the solvent, and water was added. The mixture was lyophilized to give compound **1-9,** which was directly used in the next step.

Synthesis of compound **1-10**

To a solution of compound **1-9** (51.59 μmol) and diglycolic anhydride (406 mg, 3.5 mmol) in DMF (9 mL) was added TEA (1.8 mL, 7.74 mmol). The mixture was stirred at 25 °C under nitrogen atmosphere. The mixture was stirred for 24 h. Methyl *tert*-butyl ether was added to precipitate the product. The precipitation was dissolved in methanol, and methyl *tert*-butyl ether was added for precipitation. The mixture was subjected to dialysis for 1 day and lyophilized to give compound **1-10** (2.93 g, pale yellow solid).

Synthesis of compound **1-10-SM**

To a solution of compounds **1-10-SM1** (2.71 g; see WO2014141094A1 for the synthesis of compound of formula 7) and **1-10-SM2** (FMoc-Val-Cit-PAB-PNP; 3.36 g) in DMF (34 mL) was added DIPEA (2.29 mL). The mixture was stirred at room temperature for 3 h, and 40 mL of MTBE was added. The mixture was filtered and dried *in vacuo* to give an intermediate (4.71 g). The intermediate was dissolved in 10% piperidine/DMF (32 mL), the mixture was stirred at room temperature for 10 min, and 210 mL of MTBE was added. The mixture was filtered *in vacuo* to give a pale yellow cake, and the cake was dried *in vacuo* to give compound **1-10-SM** (3.58 g, 94.6% yield).

Synthesis of compound **1**

To a solution of compounds **1-10** (375 mg, 4.81 μmol) and **1-10-SM** (280 mg, 308 μmol) in DMF (9 mL) were added PyBOP (240 mg, 460 μmol) and DIPEA (229 μL, 1.38 mol). The mixture was stirred at 25 °C for 18 h. Methyl *tert*-butyl ether was added for precipitation. The mixture was let stand at -20 °C until the completion of the precipitation. The precipitate was dissolved in methanol and purified on an LH-20 gel column. The solvent was removed by rotavap, and the residue was redissolved in water. The mixture was filtered and lyophilized to give compound **1** (450 mg).

Example 2: Synthesis of compound 2

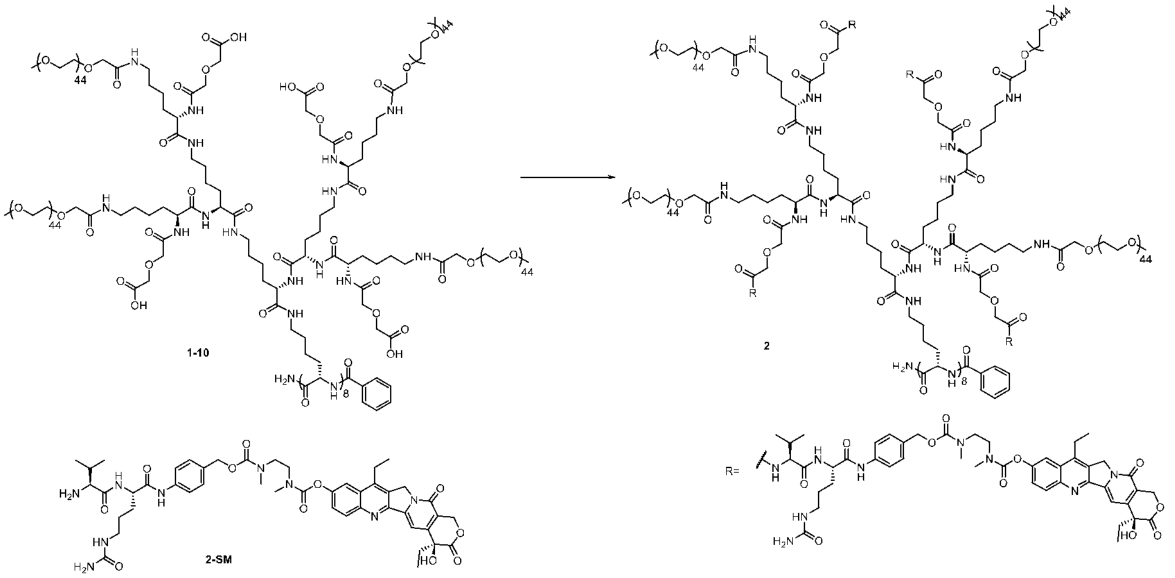

1-10

2

2-SM

R=

Synthesis of compound **2**

To a solution of compounds **1-10** (375 mg, 4.81 μmol) and **2-SM** (280 mg, 308 μmol, prepared according to the synthetic method in Example 78 of WO2016046574A1) in DMF (9 mL) were added PyBOP (240 mg, 460 μmol) and DIPEA (229 μL, 1.38 mol). The mixture was stirred at 25 °C for 18 h. Methyl *tert*-butyl ether was added for precipitation. The mixture was let stand at -20 °C until the completion of the precipitation. The precipitate was dissolved in methanol and purified on an LH-20 gel column. The solvent was removed by rotavap, and the residue was redissolved in water. The mixture was filtered and lyophilized to give compound **2** (460 mg).

Example 3: Synthesis of compound 3

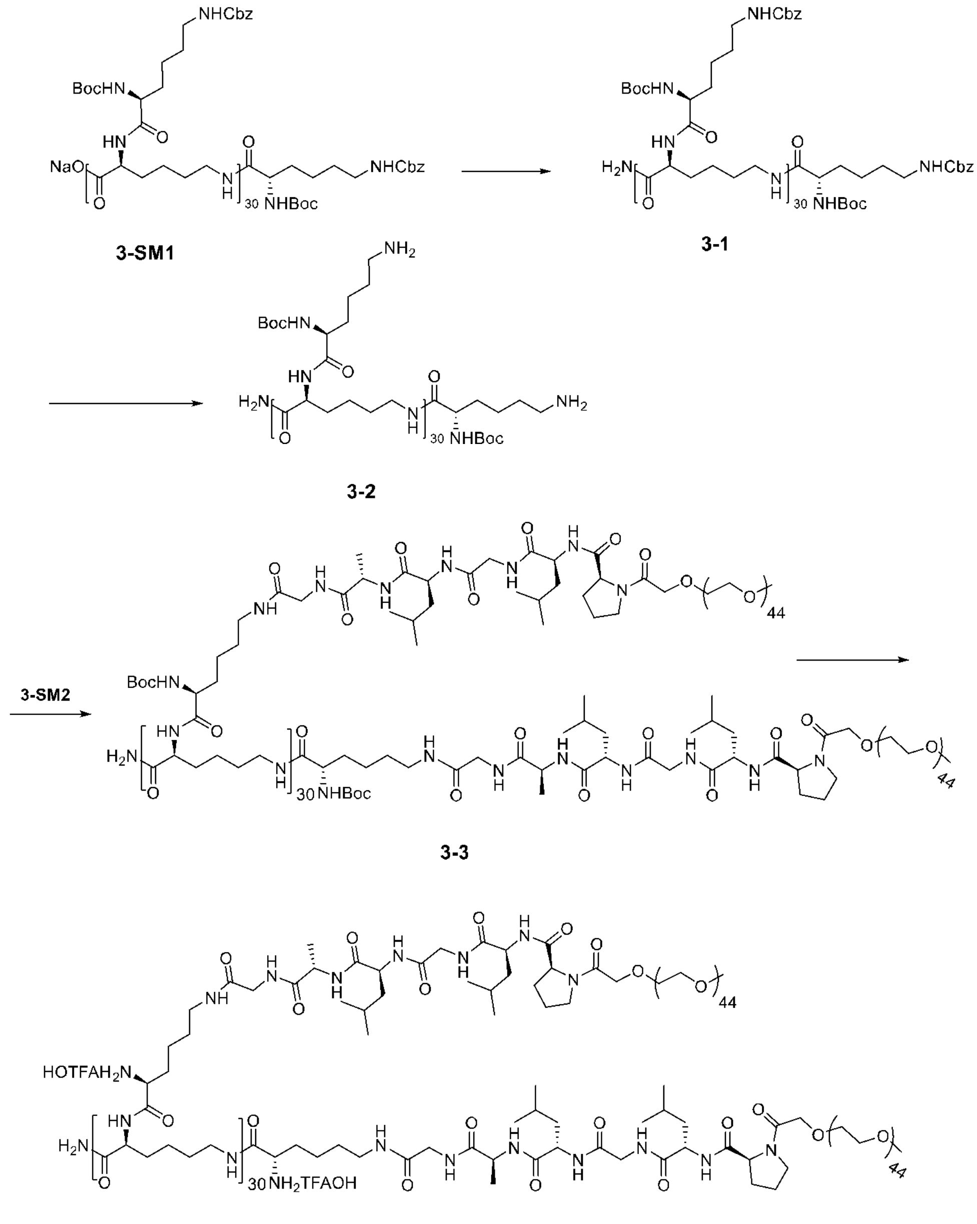


**3-SM1**

**3-1**

**3-2**

**3-3**

**3-4**

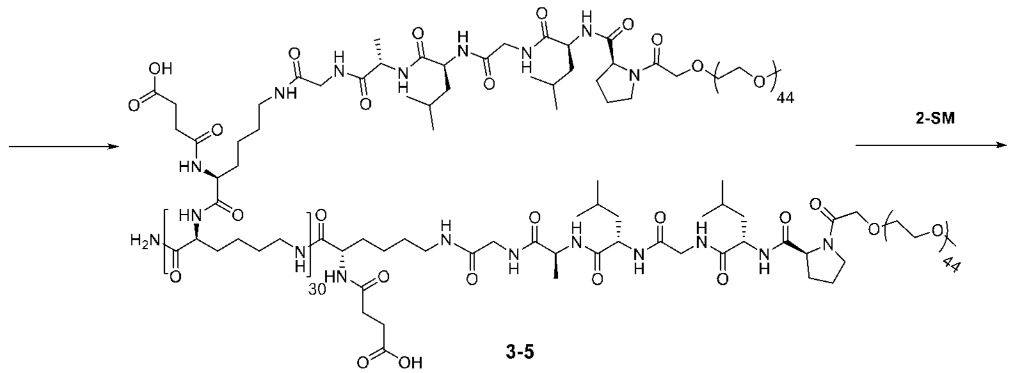


3-5

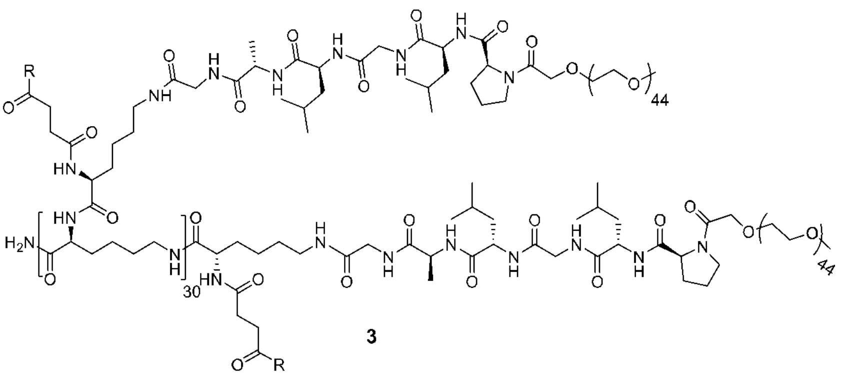

3

R =

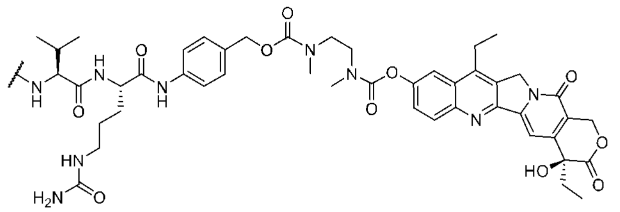

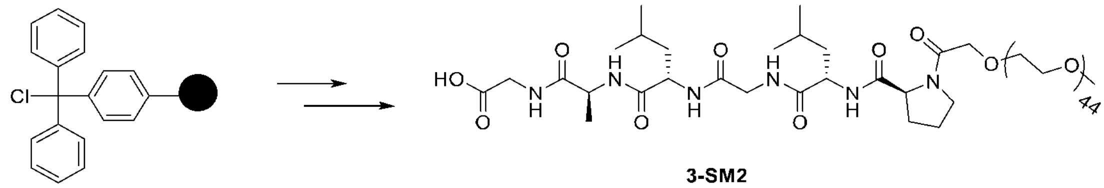

3-SM2

Synthesis of compound **3-SM1**

To a suspension of ε-poly-L-lysine hydrochloride (4.792 g, 29.09 mmol, molar quantity of structural unit) in DMSO (100 g) was added triethylamine (8.89 g, 87.27 mmol), and BOC-LYS(Z)-ONP (20.83 g, 46.63 mmol). The mixture was stirred at 30 °C for 13 h under nitrogen atmosphere until the reaction was completed. The reaction mixture was transferred into a beaker, and acetonitrile (800 mL) was added. The mixture was filtered *in vacuo,* and the cake was washed with acetonitrile, water, and acetonitrile sequentially and dried *in vacuo* to give compound **3-SM1** (12.13 g, 85% yield, white solid).

$^1$H NMR (400 MHz, DMSO-$d_6$) 7.74 (m, 62H), 7.27 (m, 179H), 6.90 (m, 28H), 5.16 - 4.80 (s, 60H), 4.31 - 4.01 (br, 30H), 4.00 - 3.68 (br, 30H), 3.12 - 2.82 (m, 120H), 1.91 - 0.58 (m, 642H)

Synthesis of compound **3-SM2**

Solid-phase synthesis was conducted using dichloro resin (14.5 g, 0.67 mmol/g) as per the standard Fmoc method. The starting material was Fmoc-Gly-OH, the coupling reagents were HOBT and DIC, the solvent was DMF, and the following amino acids and the reagents were sequentially added: Fmoc-Ala-OH, Fmoc-Leu-OH, Fmoc-Gly-OH, Fmoc-Leu-OH, Fmoc-Pro-OH, and PEG-2K acid (prepared according to the synthetic method of compound 2 in Patent No.

US2021093729A1). A resin-removing reagent TFA/water/triisopropylsilane = 95/2.5/2.5 was pre-cooled to 0 °C and added to the resin at 1.5 L/kg resin. The mixture was mechanically stirred for 2 h for removal and filtered *in vacuo.* The cake was washed with a proper solvent, and the removal solution was concentrated. Pre-cooled MTBE (more than 10 folds in volume) was added for precipitation, and the mixture was centrifuged to give a crude product. The crude product was dissolved in acetonitrile/water/TFA (50/50/0.1), and the mixture was lyophilized to give compound **3-SM2** (18.2 g).

Synthesis of compound **3-1**

Compound **3-SM1** (10.01 g) and 0.19 g of TsOH·$H_2O$were heated to 40 °C and dissolved in 50 mL of DMSO, and 30% aqueous ammonia (0.93 g), PyBOP (3.45 g), and DIPEA (1.15 mL) were added. The mixture was stirred at 25 °C for 18 h. The reaction mixture was added to 400/20 mL of water/AcOH, and a white solid was precipitated. The mixture was filtered *in vacuo,* and the cake was washed with 500 mL of water, triturated in 400 mL of acetonitrile for 1 h, filtered, and dried *in vacuo* at 35 °C to give compound **3-1** (9.71 g, 97.2% yield).

Synthesis of compound **3-2**

To a solution of compound **3-1** (9.71 g) in 97 mL of acetic acid were added 31 mL of MeOH and 1.94 g of 10% Pd/C. The mixture was purged with hydrogen thrice and stirred in a water bath at 30 °C for 24 h. The mixture was filtered through celite and rinsed with MeOH. The filtrate was then filtered through a 220 nm filter membrane and concentrated by rotavap to remove the solvent. 100 mL of MTBE was added for trituration, and a viscous precipitate was produced. The supernatant was discarded, and 50 mL of MTBE was added for further trituration. The supernatant was discarded, and the residue was dried *in vacuo* to give a product (8.96 g, 94.5% yield, containing 26.98% of AcOH).

1.56 g of the product was dissolved in 15 mL of water and filtered through IRA-900 resin (100 g). Fractions containing the sample were combined and lyophilized to give 1.11 g of product, containing 0.11% (mass fraction) of acetic acid.

Synthesis of compound **3-3**

To compound **3-2** (100 mg; GPC Rt = 14.649 min) was added 0.5 mL of DMF to give a paste, and 0.5 mL of DMSO was added. The mixture was stirred until the solid was completely dissolved. Compound 3-SM2 (1.0 g) was dissolved in 4 mL of DMF, and to the above system was added the mixture. 0.29 g of PyBOP and 98 μL of DIPEA were added, and the mixture was stirred at room temperature for 2 h. 146.2 mg of PyBOP and 98 μL of DIPEA were then added, and the mixture was further stirred at room temperature for 18 h. A sample of 50 μL was taken and dried with nitrogen flow, and 1 mL of water was added. GPC characterization indicated that the molecular weight complied with the requirement. MTBE (40 mL) was added to form an oily precipitate. The mixture was centrifuged, and the supernatant was discarded. The residue was dissolved in 4 mL of MeOH, and 40 mL MTBE was added to precipitate a solid pellet. The supernatant was discarded. The above procedures were repeated once. The residue was dried *in vacuo* to give a crude product (0.98 g, >99% yield), which was directly used in the next step.

Synthesis of compound **3-4**

To a solution of compound 3-3 (0.8 g) in DCM (2.8 mL) was added 1.2 mL of TFA. The mixture was stirred overnight at 25 °C and concentrated *in vacuo,* and MTBE (20 mL) was added for precipitation. The mixture was frozen in dry ice/ethanol to precipitate a pellet and thawed to room temperature. The supernatant was discarded. The precipitate was dried *in vacuo* to give a crude product (0.81 g, >99% yield), which was directly used in the next step.

Synthesis of compound **3-5**

To a solution of compound **3-4** (772.1 mg) in ethyl acetate (4 mL, 30 °C) was added 42 mg of succinic anhydride. The mixture was stirred for dissolution. DIPEA (0.3 mL) was added, and the mixture was stirred at 30 °C for 2 h. The reaction mixture was in the form of gel. 3 mL of DMF was added to dissolve the mixture. The mixture was stirred overnight at 30 °C. The mixture was concentrated *in vacuo,* and 40 mL of MTBE was added to precipitate a viscous substance. The mixture was centrifuged. The precipitate was dissolved in MeOH/water, purified by ultrafiltration (30K MW), and lyophilized to give compound **3-4** (513.0 mg, 64.1% yield).

Synthesis of compound **3**

To a solution of compounds **3-5** (501 mg) and **2-SM** (270 mg) and HOBt (47 mg) in 2 mL of DMF was added

PyBOP (181.3 mg), and the mixture was stirred for dissolution. 86 μL of N-methylmorpholine was added, and the mixture was stirred in a water bath at 30 °C for 4 h. 20 mL of MTBE was added to precipitate a pale yellow solid. The mixture was centrifuged, and to 10 mL of MTBE was added the precipitate for trituration for 10 min. The triturated mixture was centrifuged and dried *in vacuo* to give a crude product. The crude product was dissolved in 15 mL of MeOH and diluted with 15 mL of filtered water. The mixture was purified by ultrafiltration (30K MW) and lyophilized to give a product (654.9 mg, 99.7% yield, calculated SN38 conjugation content 10.41%, measured 10.5%).

Example 4: Synthesis of Compound 4

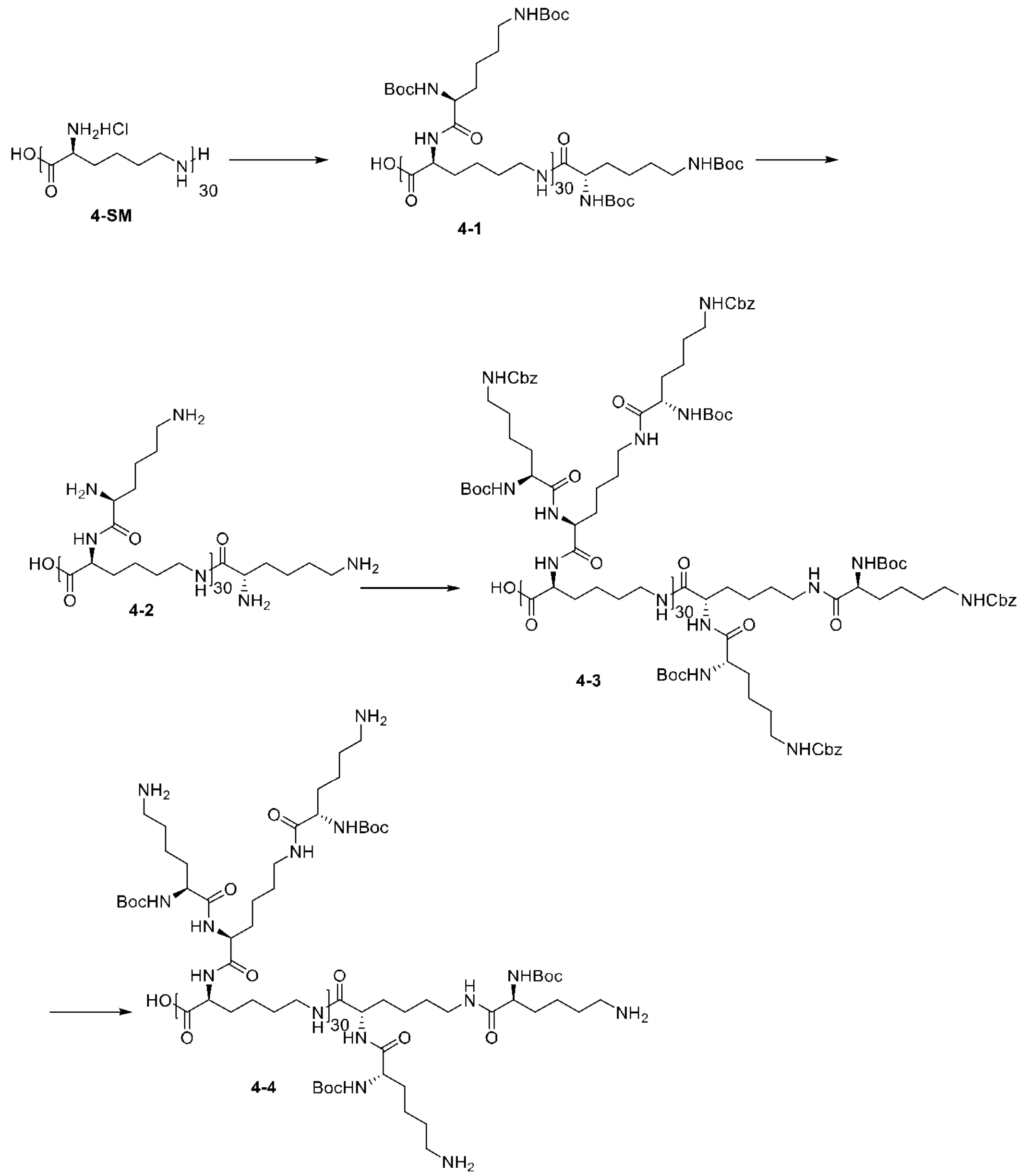

4-SM

4-1

4-2

4-3

4-4

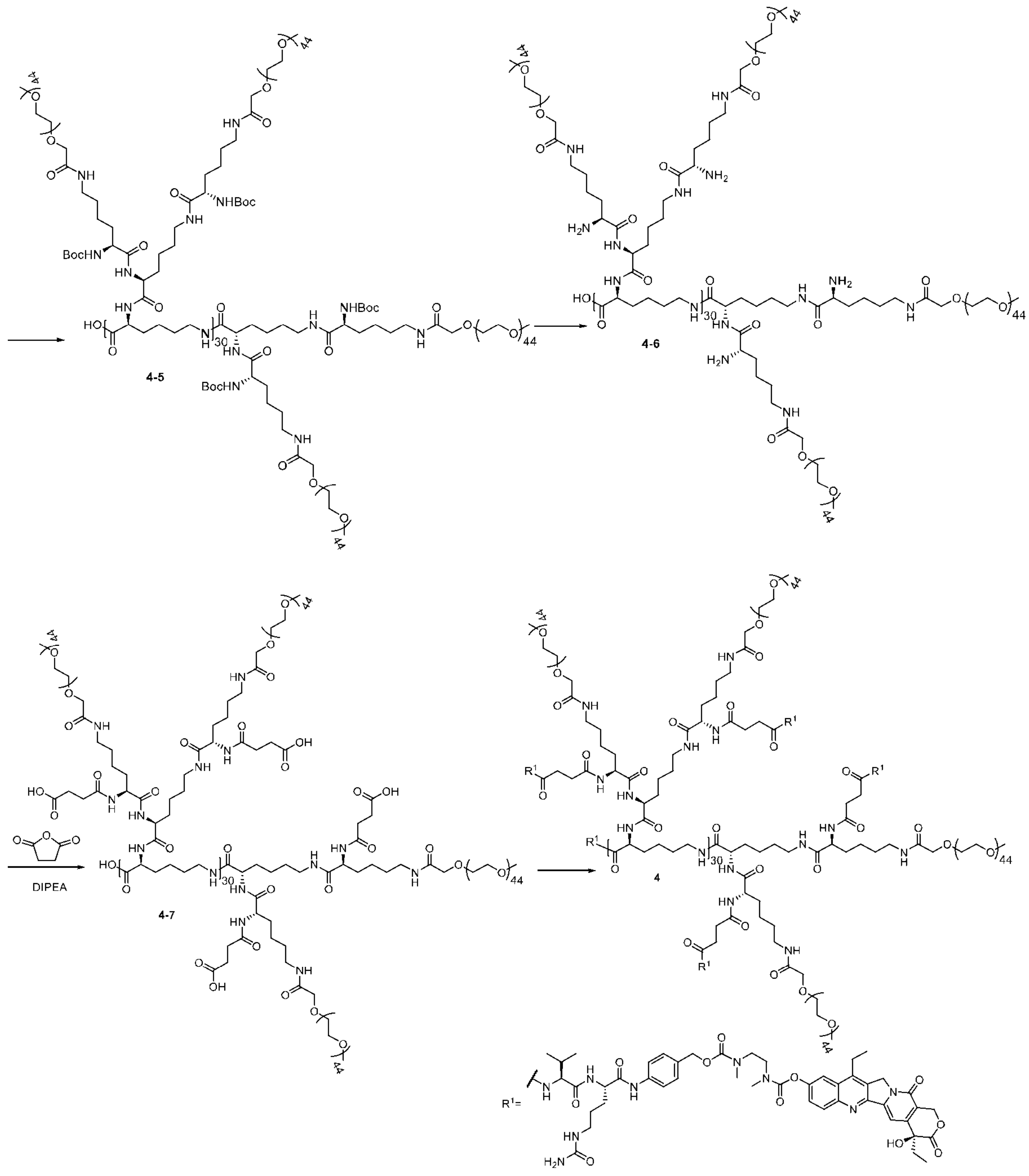


4-5

4-6

4-7

4

Synthesis of compound **4-1**

To a solution of ε-poly-L-lysine hydrochloride (0.76 g) in 1.12 mL of water were added 16 mL of DMSO, 2.76 mL of DIPEA, *N,N*-di-*tert*-butoxycarbonyl-L-lysine *p*-nitrophenol ester (3.6 g) sequentially. The mixture was ultrasonicated for dissolution and stirred at room temperature for 14 h. 15 mL of 0.5 M NaOH was added. The mixture was stirred at room temperature for hydrolysis and filtered *in vacuo.* The cake was washed with water, triturated in acetonitrile, filtered *in vacuo,* and dried *in vacuo* to give compound **4-1** (white powder solid, 2.0 g, 84.5% yield).

Synthesis of compound **4-2**

To a solution of compound **4-1** (1.50 g) in 15 mL of DCM was added TFA (5 mL). The mixture was stirred at room temperature for 18 h, and concentrated by rotavap to remove the solvent. The residue was washed with MTBE, and dried *in vacuo* to give a crude product of compound **4-2,** which was directly used in the next step.

Synthesis of compound **4-3**

Compound **4-2** (1.5 g, 3.09 mmol) and DMSO (12.5 mL) were added into a 100 mL single-neck flask and ultrasonicated for dissolution, and BOC-LYS(Z)-ONP (4.66 g, 9.29 mmol) and DIPEA (2.4 g, 18.58 mmol) were added. The mixture was purged with nitrogen thrice and stirred at room temperature for 2 h under nitrogen atmosphere. The Kaiser reagent chromogenic test of the samples exhibited a pale blue color, which indicated an incomplete reaction. The mixture was clarified and stirred at room temperature for 16 h. When the Kaiser reagent chromogenic test of the samples exhibited a colorless result, 0.5 N aqueous sodium hydroxide solution (20 mL) was added to the mixture. The mixture was stirred for 30 min, and acetonitrile (40 mL) was added. The mixture was then stirred for another 15 min and filtered. The cake was washed until it turned white, and the solid was dried *in vacuo* at 40 °C to give compound **4-3** (white solid, 2.62 g, 87.6% yield).

Synthesis of compound **4-4**

Compound **4-3** (2.4 g, 0.079 mmol) and acetic acid (24 mL) were added into a 10 mL single-neck flask. The mixture was heated to 40 °C for complete dissolution, and palladium on carbon (960 mg, 40%) was added. The mixture was purged with hydrogen thrice, stirred at 30 °C in an oil bath for 20 h under hydrogen atmosphere, cooled to room temperature, and filtered through celite. The cake was washed with methanol twice. The filtrate was filtered once through a 0.22 syringe filter and concentrated *in vacuo* to give a viscous solid. MTBE was added for trituration to give a gray solid. The supernatant was discarded. The procedures were repeated once. The residue was dried with an oil pump to give compound **4-4** (dark solid, 1.86 g).

Synthesis of compound **4-5**

Compound **4-4** (1.0 g) was transferred into a 50 mL centrifuge tube, and methanol (16.6 mL) was added. The mixture was ultrasonicated for dissolution. A proper amount of PEG-2K-NHS (6.43 g, prepared from PEG-2K acid and NHS by EDCI condensing agent) was added into a 100 mL single-neck flask, and acetonitrile (27 mL) was added. The mixture was stirred for dissolution, and methanol (27 mL), the above solution of compound **4-4** in methanol, and DIPEA (2.17 mL) were sequentially added. The mixture was stirred at room temperature for 15 min. A sample was taken for GPC detection, which indicated an incomplete reaction. The mixture was stirred for 45 min. A sample was taken for GPC detection, which indicated the completion of the reaction. The reaction mixture was directly filtered through a 0.22 syringe filter, purified by ultrafiltration (30K MW), concentrated, and lyophilized to give compound **4-5** (2.84 g, brown solid).

Synthesis of compound **4-6**

To a 50 mL single-neck flask were added compound **4-5** (2.84 g, 0.097 mmol), TFA (4.26 mL), and DCM (9.94 mL). The mixture was stirred at room temperature for 16 h under nitrogen atmosphere. A sample was taken, dried with nitrogen flow, and monitored by NMR. The reaction mixture was directly concentrated to give a crude product, and to the crude product was added 26 mL of MTBE (room temperature) with stirring to give an oily precipitate. The oily precipitate was cooled to below -10 °C with vigorous stirring, and pellet and powder solids were precipitated. The mixture was stirred, heated to above 15 °C, and filtered. The flask and the cake were washed with 40 mL of MTBE. The cake was dried *in vacuo* with an oil pump for 2 h to give compound **4-6** (2.75 g, white solid).

Synthesis of compound **4-7**

To a 50 mL single-neck flask were added compound **4-6** (2.75 g, 0.61 mmol) and ethyl acetate (14 mL), and the mixture was purged with nitrogen thrice and stirred at 30 °C for dissolution under nitrogen atmosphere. Succinic anhydride (158 mg, 1.57 mmol) was added after complete dissolution, and the mixture was stirred for dissolution again. DIPEA (941 mg, 7.28 mmol) was added slowly dropwise after complete dissolution. After the addition, the mixture was stirred for 1 h at 30 °C, warmed to room temperature, and stirred for 16 h. To MTBE was added a sample of 50 $\mu$L for precipitation, the mixture was centrifuged, and the supernatant was discarded. The residue was subjected to a chromogenic reaction with Kaiser reagent for three minutes at 100 °C, and the mixture was colorless, indicating the depletion of amino. The reaction mixture was transferred into a 100 mL flask. MTBE (50 mL) was added with vigorous stirring, and a large amount of solid was precipitated. After the addition, the mixture was stirred for 10 min and filtered. The cake was dissolved in ethyl acetate (18 mL) at 30 °C with stirring. MTBE (60 mL) was added with vigorous stirring, and a large amount of solid was precipitated. After the addition, the mixture was stirred for 10 min and filtered. The cake was washed twice with MTBE, and the solid was dried *in vacuo* to give compound **4-7** (2.54 g, 71.8% yield, white solid).

Synthesis of compound **4**

To a solution of compound **4-7** (1.2 g, 0.25 mmol, based on the polymer structural unit) in DMF (6 mL) in a 25 mL single-neck flask was added **2-SM** (0.65 g, 0.7 mmol) for dissolution. Then HOBT (0.14 g, 1.01 mmol), PyBOP (0.49 g, 0.94 mmol), and NMM (0.23 g, 2.28 mmol) were sequentially added, and the mixture was purged with nitrogen thrice and stirred at room temperature for 16 h under nitrogen atmosphere. A sample was taken for HPLC detection, which indicated the depletion of **2-SM. 2-SM** (116 mg, 0.13 mmol) and PyBOP (344 mg, 0.17 mmol) were added, and the mixture was stirred for 2 h. A sample was taken for GPC detection, which indicated an 18% residue of **2-SM** and the depletion of starting material **4-7.** To MTBE (56 mL) was added the reaction mixture for precipitation. The mixture was centrifuged, and the supernatant was discarded. The residual crude product was dissolved in methanol (30 mL), and the mixture was filtered through a syringe filter and diluted with water (30 mL) to 60 mL. The dilution was purified to 99.95% by ultrafiltration (30K MW) and lyophilized to give a white solid (1.44 g).

Example 5: Synthesis of Compound 5

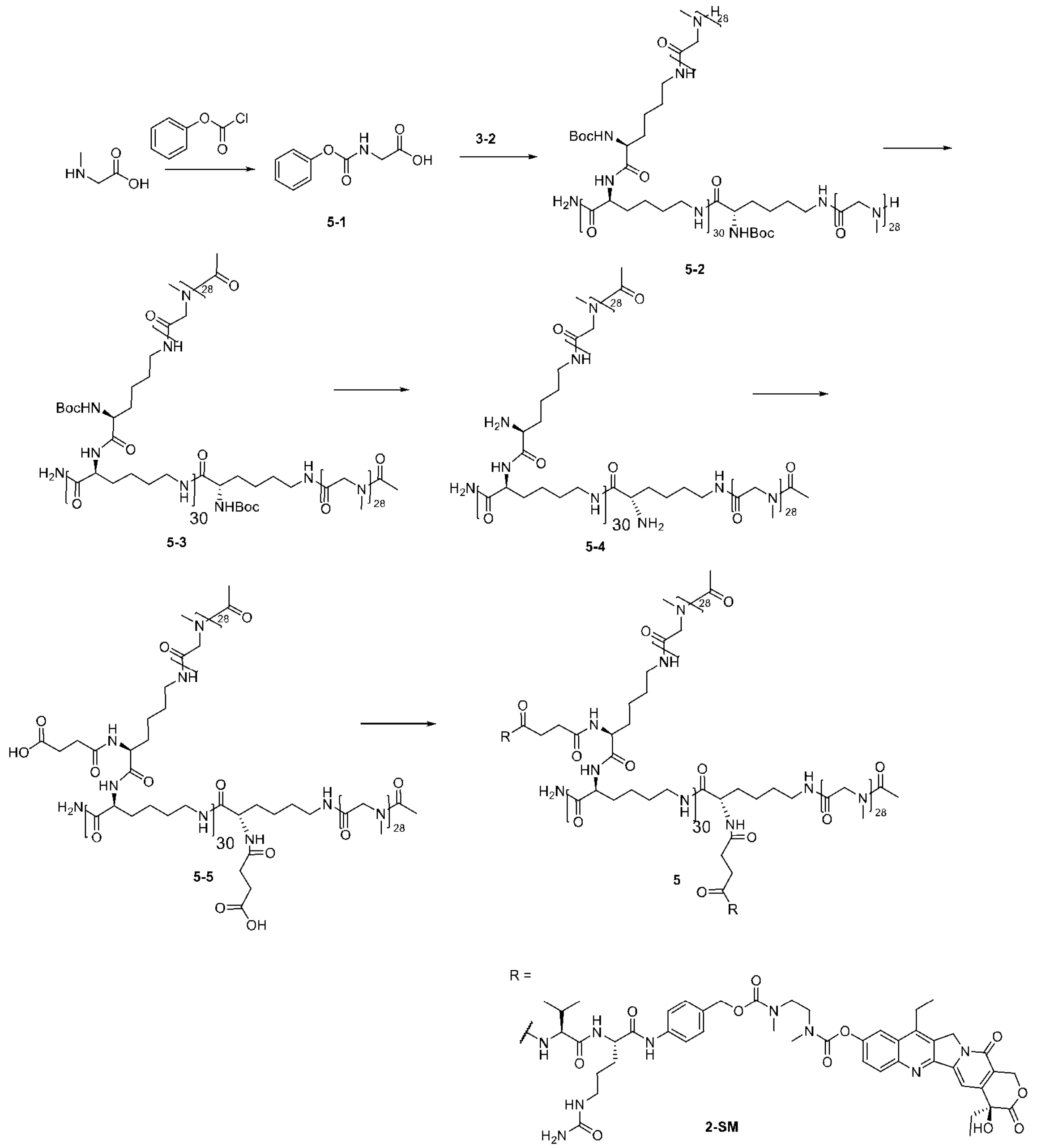


5-1

5-2

5-3

5-4

5-5

5

R =

2-SM

Synthesis of compound **5-1**

Sarcosine (70 g, 0.78 mol) and water (800 mL) were added into a 1 L three-necked flask. The mixture was purged with nitrogen thrice and cooled in ice water to 0 °C under nitrogen atmosphere, and potassium carbonate (130.3 g, 0.94 mol) was added in portions with the temperature controlled below 5 °C. After the addition, benzoyl chloride (135.3 g, 0.86 mmol) was added dropwise over 70 min, and the mixture was warmed to room temperature and stirred for 16 h. A sample was taken for LCMS detection, which indicated the depletion of the starting materials. The reaction mixture was washed thrice with MTBE, adjusted to pH 2-3 with concentrated hydrochloric acid, and extracted thrice with MTBE. The organic phases were combined, washed once with saturated brine, dried over anhydrous sodium sulfate, and concentrated *in vacuo* to give compound **5-1** (colorless transparent oily substance, 166.7 g).

Synthesis of compound **5-2**

Compounds **3-2** (200 mg, 0.56 mmol) and **5-1** (5.63 g, 26.93 mmol) were dissolved in DMSO (30 mL) with ultrasonication in a 10 mL single-neck flask. When the dissolution was completed, DIPEA (580.3 g, 4.49 mmol) was added, and the mixture was purged with nitrogen thrice and stirred at 60 °C for 16 h in an oil bath under nitrogen atmosphere. A sample was taken for GPC detection, and the results were rt = 12.191 min, $M_n = 3.87 \times 10^4$, $M_w = 4.32 \times 10^4$, and PDI = 1.11, substantially consistent with those the reference: rt = 12.31, $M_n = 3.70 \times 10^4$, $M_w = 3.98 \times 10^4$, and PDI = 1.07. To ethyl acetate (240 mL) was added the reaction mixture for precipitation, and a large amount of solid was precipitated. The mixture was filtered, and the cake was washed twice with ethyl acetate. The crude solid product was dissolved in methanol (20 mL), purified by ultrafiltration (30K MW) to remove 1 L of solvent, and lyophilized to give compound **5-2** (pale orange solid, 715 mg).

Synthesis of compound **5-3**

To a 50 mL single-neck flask were added compound **5-2** (1.43 g, 0.598 mmol), acetic anhydride (91.7 mg, 0.898 mmol), pyridine (142 mg, 1.78 mmol), and DCM (40 mL), and the mixture was stirred at room temperature for 16 h. The reaction mixture was concentrated *in vacuo,* and ethyl acetate (7 mL) was added. A solid was precipitated at the bottom, and the supernatant was discarded. Ethyl acetate (10 mL) was added, and the solid was ground with a spoon. The mixture was triturated, stirred for 1 h, and filtered. The cake was washed twice with ethyl acetate and dried *in vacuo* at 35 °C to give a white solid (1.54 g).

Synthesis of compound **5-4**

To a 25 mL single-neck flask were added compound **5-3** (1.54 g, 0.64 mmol), trifluoroacetic acid (2.31 mL), and dichloromethane (5.39 mL). The mixture was stirred at room temperature for 16 h and directly concentrated to give a crude product (orange semi-oily semi-solid substance, 3.04 g).

Synthesis of compound **5-5**

To a 25 mL single-neck flask were added compound **5-4** (3.04 g, 0.64 mmol, crude) and DMF (8 mL). The mixture was ultrasonicated for dissolution, and succinic anhydride (84 mg, 0.84 mmol) was added. The mixture was stirred for dissolution under nitrogen atmosphere, and DIPEA (501 mg, 3.87 mmol) was added. After the addition, the mixture was adjusted to pH 2, DIPEA (1.5 g, 11.62 mmol) was added. The mixture was adjusted to pH 8, purged with nitrogen thrice, and stirred at room temperature for 16 h under nitrogen atmosphere. A sample was taken and added to MTBE for precipitation. The mixture was centrifuged, and the supernatant was discarded. To the residue was added ninhydrin/phenol/pyridine = 3/2/1. The mixture was subjected to chromogenic reaction at 100 °C for 3 min, and the result was colorless, indicating the depletion of the starting materials. The reaction mixture was added slowly dropwise to ethyl acetate (50 mL), and a large amount of solid was precipitated. The mixture was filtered, and the cake was washed with ethyl acetate and dissolved in DMF (8 mL). The mixture was added slowly dropwise to ethyl acetate (50 mL) again, and a large amount of solid was precipitated. The mixture was filtered, and the cake was washed with ethyl acetate. The solid was dried *in vacuo* to give a white solid (1.3 g).

Synthesis of compound **5**

Compound **5-5** (600 mg, 0.25 mmol, based on the polymer structural unit) was dissolved with ultrasonication in DMF (10 mL) in a 25 mL single-neck flask, and **2-SM** (401 mg, 0.44 mmol) was added. Then HOBt (68 mg, 0.502 mmol), PyBOP (262 mg, 0.502 mmol), and N-methylmorpholine (115 mg, 1.13 mmol) were sequentially added, and the mixture

was purged with nitrogen thrice and stirred at room temperature for 3.5 h under nitrogen atmosphere. A sample was taken for HPLC detection, which indicated a 14% residue of **2-SM.** The mixture was further stirred for 2 h. A sample was taken for HPLC detection, which indicated the 14% residue **of 2-SM** was depleted. To ethyl acetate (88 mL) was added the reaction mixture slowly dropwise, and a large amount of solid was precipitated. The mixture was filtered, and the cake was washed with ethyl acetate. The solid was dried *in vacuo* to give a crude product (1.4 g). The crude product was insoluble in methanol (15 mL) but soluble in water (15 mL). The solution was filtered, ultrafiltered (30K MW), and lyophilized to give a white solid (816 mg).

Example 6: Synthesis of compound 6

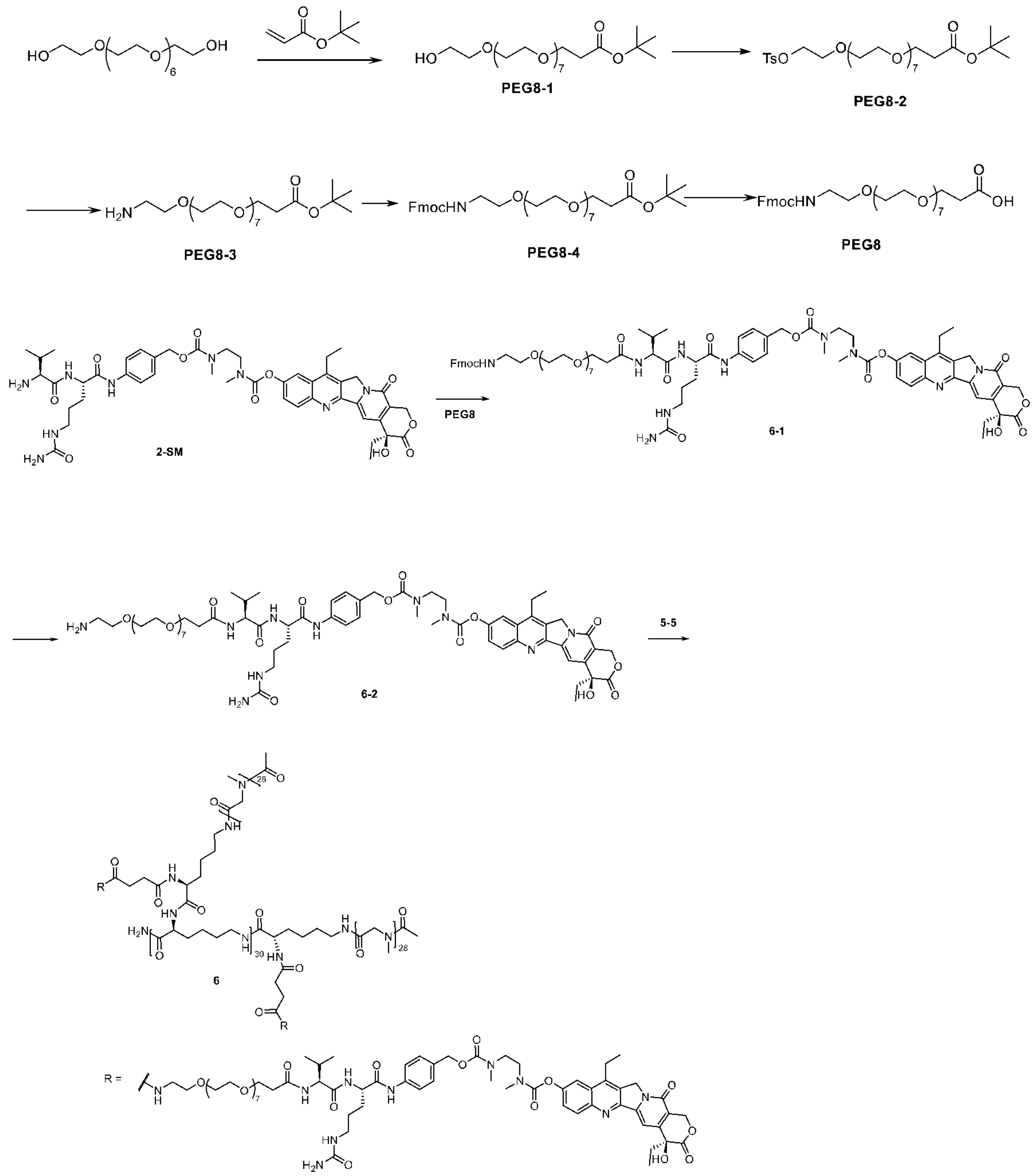


Synthesis of compound **PEG8-1**

To a solution of octaethylene glycol (25 g, 67.5 mmol, 1.0 eq) in dichloromethane (100 mL) was added sodium

metal (100 mg) at room temperature. The mixture was stirred at 30 °C for 3 h under nitrogen atmosphere. *tert*-Butyl 1-buten-4-oate (6.5 g, 50.6 mmol, 0.75 eq) was added. The mixture was stirred at room temperature for 16 h. When LCMS indicated the completion of the reaction, water (200 mL) was added and the mixture was extracted thrice with a total of 1.5 L of dichloromethane. The organic phases were combined, washed with saturated sodium chloride, dried over sodium sulfate, and concentrated *in vacuo* by rotavap to give a crude product (23 g), which was directly used in the next reaction.

MS (ESI), m/z, 499.2 $[M+H]^+$.

Synthesis of compound **PEG8-2**

Compound **PEG8-1** (23 g, 46.1 mmol, 1.0 eq) and triethylamine (9.32 g, 92.3 mmol, 2.0 eq) were dissolved in dichloromethane (100 mL) at room temperature. *p*-Toluenesulfonyl chloride (8.79 g, 46.1 mmol, 1.0 eq) was added at 30 °C under nitrogen atmosphere. The mixture was stirred at room temperature for 16 h. When LCMS indicated the completion of the reaction, the mixture was concentrated *in vacuo* to give compound **PEG8-2** (white solid), which was directly used in the next reaction.

MS (ESI), m/z, 597.2 $[M-56]^+$.

Synthesis of compound **PEG8-3**

Compound **PEG8-2** (crude) was dissolved in acetonitrile/aqueous ammonia (150 mL/150 mL) at room temperature. The reaction mixture was stirred at 40 °C for 5 h under nitrogen atmosphere. When LCMS indicated the completion of the reaction, the mixture was concentrated *in vacuo* to give a crude product (20.7 g), which was directly used in the next reaction.

MS (ESI), m/z, 498.2 $[M+H]^+$.

Synthesis of compound **PEG8-4**

To a solution of compound **PEG8-3** (20.7 g, 41.6 mmol, 1.0 eq) in dichloromethane (100 mL) were added FmocOSU (14 g, 41.6 mmol, 1.0 eq) and DIPEA (21.5 g, 166.4 mmol, 4.0 eq) at room temperature, and the mixture was stirred at 30 °C for 2 h under nitrogen atmosphere. When LCMS indicated the completion of the reaction, the reaction was quenched with water, and the mixture was extracted with dichloromethane. The organic phases were combined and concentrated *in vacuo* to give compound **PEG8-4** (colorless oil, 18 g, impure), which was directly used in the next reaction.

MS (ESI), m/z, 720.2 $[M+H]^+$.

Synthesis of compound **PEG8**

To a solution of compound **PEG8-4** (crude, 18 g) in dichloromethane (15 mL) was added trifluoroacetic acid (15 mL) at room temperature under nitrogen atmosphere. The mixture was stirred for 3 h. When LCMS indicated the completion of the reaction, the mixture was concentrated *in vacuo* to give compound **PEG8** (colorless oil, 7.3 g, 22% yield over the four steps).

MS (ESI), m/z, 644.2 $[M+H]^+$.

Synthesis of compound 6-1

Compounds **2-SM** (900 mg, 0.986 mmol) and **PEG8** (786 mg, 1.18 mmol) were dissolved in DMF (20 mL) with ultrasonication in a 50 mL single-neck flask, and PyBOP (617 mg, 1.18 mmol) and DIPEA (255 mg, 1.97 mmol) were sequentially added. The mixture was purged with nitrogen thrice and stirred at room temperature for 3 h under nitrogen atmosphere. A sample was taken for TLC detection, which indicated the depletion of the starting materials. To MTBE (160 mL) was added the reaction mixture slowly dropwise at -68 °C, and a large amount of solid was precipitated. The mixture was warmed to room temperature, and the solid particles turned into oily sediment. The supernatant was discarded, and the oily substance was transferred into a 50 mL single-neck flask and concentrated *in vacuo* to give a crude product (2.1 g). By wet loading, the crude product was separated by silica gel column chromatography (50:1-30:1-20:1-10:1-5:1) to give a white solid (1.23 g).

Synthesis of compound **6-2**

To a 50 mL single-neck flask were added compound **6-1** (1.2 g, 0.71 mmol), *N,N*-dimethylethylamine (827 mg, 11.3 mmol), and DMF (10 mL). The mixture was purged with nitrogen thrice and stirred at room temperature for 16 h under nitrogen atmosphere. A sample was taken for TLC detection, which indicated that the starting materials were depleted. To

MTBE (90 mL) was added the reaction mixture slowly dropwise, and an oily substance was precipitated. The supernatant was discarded, and the oily precipitate was dissolved in DCM (9 mL). Again to MTBE (90 mL) was added the mixture slowly dropwise, and a large amount of solid was precipitated. The mixture was filtered, and the cake was washed twice with MTBE. The solid was dried *in vacuo* to give a white solid (982 mg).

Synthesis of compound **6**

Compounds **6-2** (653 mg, 0.488 mmol) and **5-5** (648 mg, 0.27 mmol, based on the polymer structural unit) and DMF (10 mL) were added into a 50 mL single-neck flask, and HOBt (74 mg, 0.54 mmol), PyBOP (283 mg, 0.54 mmol), and N-methylmorpholine (124 mg, 1.22 mmol) were sequentially added. The mixture was ultrasonicated for dissolution, and the mixture was purged with nitrogen thrice. The mixture was stirred at room temperature for 16 h under nitrogen atmosphere. To ethyl acetate (90 mL) was added the reaction mixture slowly dropwise, and a large amount of solid was precipitated. The mixture was filtered, and the cake was washed with ethyl acetate. The solid crude product was insoluble in methanol (10 mL) but soluble in water (10 mL). The solution was filtered, ultrafiltered (30K MW), and lyophilized to give a white solid (786 mg).

Example 7: Synthesis of Compound 7

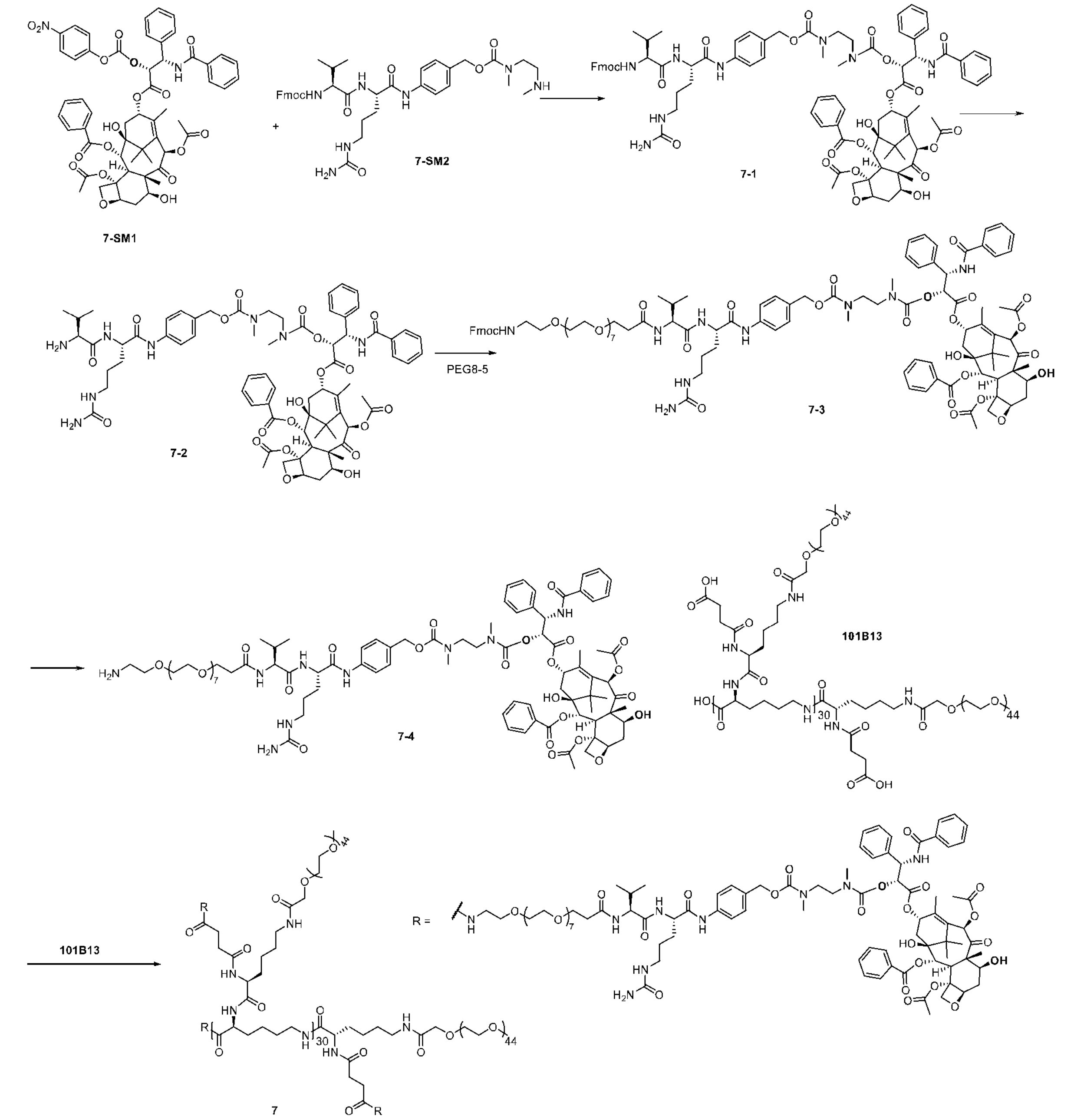


7-SM1, 7-SM2, 7-1, 7-2, 7-3, 7-4, 101B13, 7

Synthesis of compound **7-1**

**7-SM2** (513 mg, 0.62 mmol, 1.1 eq, see the synthetic method in Example 96 of Patent No. US2016271270A1) and anhydrous DMF (5 mL) were added sequentially under nitrogen atmosphere. When the solid was partially dissolved, the mixture was cooled to 0 °C in an ice bath. **7-SM1** (573 mg, 0.56 mmol, 1.0 eq, according to the synthetic method of compound 7 in J. Med. Chem. 2000, 43, 16, 3093-3102) and DIPEA (0.2 mL, 1.12 mmol, 2.0 eq) were added. The reaction mixture was stirred at 0 °C for 2 h until LCMS monitoring indicated the depletion of the starting materials. The reaction mixture was directly used in the next step without further processing.

Synthesis of compound **7-2**

To the reaction mixture was added diethylamine (0.5 mL, 4.96 mmol, 8.0 eq) at room temperature (25 °C). The reaction mixture was stirred at room temperature for 1 h until LCMS monitoring indicated the depletion of the starting materials. After the preparation, the mixture was lyophilized to give a product **7-2** (618 mg, white solid, 80% yield over the two steps).

$^1$H NMR (400 MHz, DMSO-$d_6$) δ 10.19 (s, 1H), 9.25 - 9.10 (m, 1H), 8.68 (d, $J$ = 7.6 Hz, 1H), 8.15 - 7.95 (m, 5H),

7.88 - 7.80 (m, 2H), 7.79 - 7.39 (m, 13H), 7.33 - 7.13 (m, 4H), 6.30 (s, 1H), 6.12 - 5.97 (m, 1H), 5.90 - 5.78 (m, 1H), 5.65 - 5.10 (m, 5H), 4.98 - 4.82 (m, 3H), 4.73 - 4.41 (m, 3H), 4.19 - 3.94 (m, 3H), 2.92 - 2.67 (m, 7H), 2.40 - 1.94 (m, 11H), 1.88 - 1.56 (m, 8H), 1.55 - 1.37 (m, 6H), 1.08 - 0.90 (m, 13H).

Synthesis of compound **7-3**

To a solution of compound **7-2** (860 mg, 0.626 mmol, 1.0 eq) in DMF (5.0 mL) were added **PEG-8** (416 mg, 0.626 mmol, 1.0 eq), PyBOP (489 mg, 0.939 mmol, 1.5 eq), and DIPEA (242 mg, 1.878 mmol, 3.0 eq) sequentially at 25 °C. After the addition, the mixture was stirred for 2 h at the temperature. After the reaction was completed, the reaction mixture was directly used in the next step without post-treatment.

Synthesis of compound **7-4**

To the reaction mixture (compound **7-3)** from the previous step was directly added diethylamine (2.0 mL), and the mixture was stirred for 2 h at 25 °C. After the reaction was completed, the reaction mixture was purified on a C18 column reversed-phase column. The impurities were firstly eluted with an eluent system $CH_3CN$:$H_2O$(0.05% $NH_3$.$H_2O$) = 5-95% to 95%-95%, and the target product was then eluted with $CH_3CN$ (0.05% TFA):$H_2O$ (0.05% TFA) = 95%-95%. The product solution was collected and concentrated by rotavap to give a purified white solid product (1.1 g).

MS (ESI), m/z, 1796.80 [M+H]+.

Synthesis of compound **7-5**

To a solution of compound **7-4** (800 mg, 0.419 mmol, 1.5 eq) in DMF (8.0 mL) were added **101B13** (660 mg, 0.279 mmol, 1.0 eq, based on the polymer structural unit), PyBOP (218 mg, 0.419 mmol, 1.5 eq), and DIPEA (163 mg, 1.26 mmol, 4.5 eq) sequentially at 25 °C. After the addition, the reaction mixture was returned to room temperature and stirred for 16 h. After the reaction was completed, the mixture was concentrated *in vacuo* to half of the original volume and ultrafiltered with a 30K MW ultrafiltration membrane. The pure product solution was lyophilized to give a white, gummy solid (850 mg) with a measured PTX content of 17.8% (calculated: 21.0%).

The content assay: the contents of fragment **7-2** or **7-4** in compound 7 were determined by HPLC UV = 254 nM, and the paclitaxel content was calculated on this basis.

Example 8: Synthesis of Compound 8

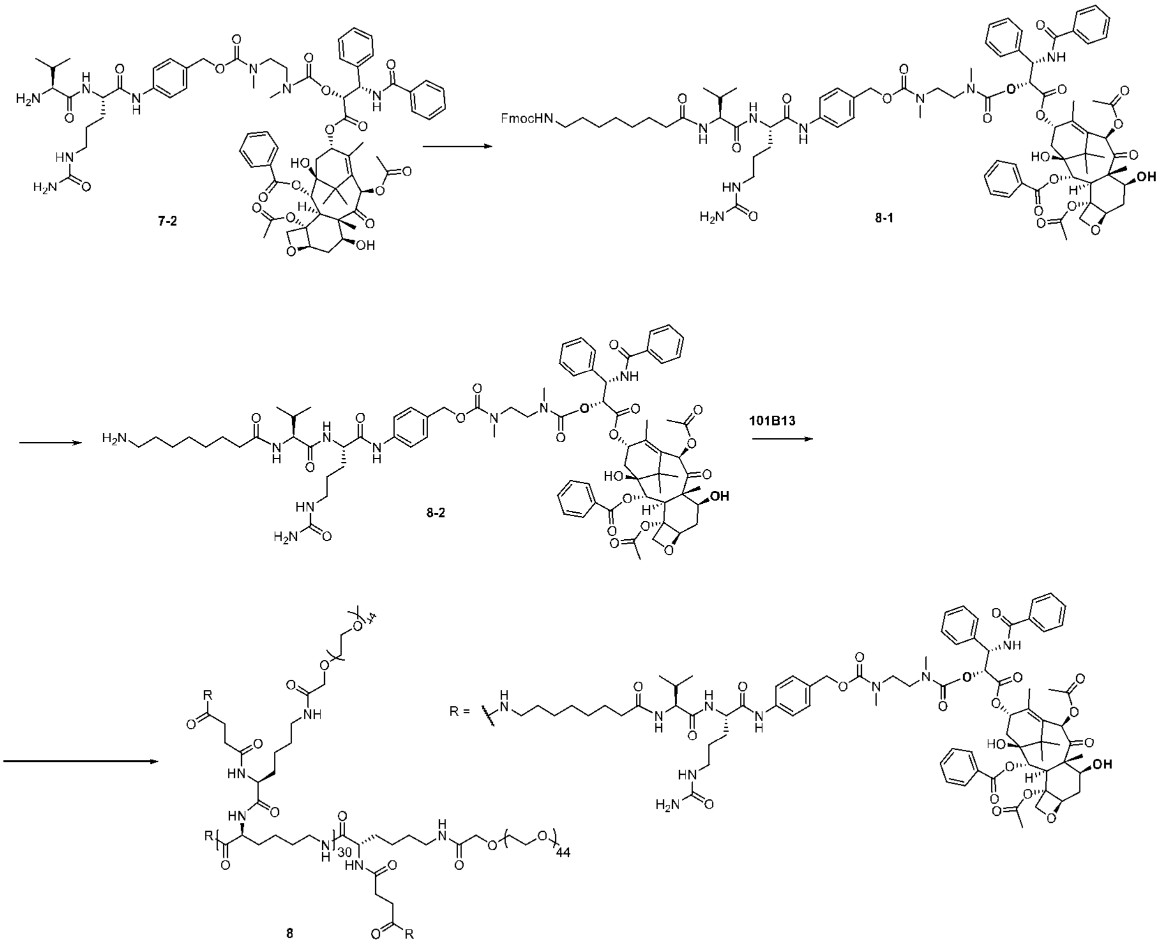


7-2 8-1 8-2 8

Synthesis of compound **8-1**

To a solution of **7-2** (85 mg, 0.062 mmol, 1.0 eq) in DMF (5 mL) were added N-Fmoc-8-aminooctanoic acid (23 mg, 0.062 mmol, 1.0 eq), DIPEA, and PyBOP (50 mg, 0.093 mmol, 1.5 eq) at room temperature under nitrogen atmosphere. The reaction mixture was stirred overnight. After the reaction was completed, the mixture was directly concentrated and purified by Pre-HPLC to give a white solid (68 mg, 63% yield).

MS (ESI), m/z, 1736.2 $[M+H]^+$.

Synthesis of compound **8-2**

To a solution of compound **8-1** (68 mg, 0.039 mmol, 1.0 eq) in DMF (50 mL) was added diethylamine (0.2 mL) at room temperature under nitrogen atmosphere. The reaction mixture was stirred overnight. After the reaction was completed, the mixture was directly concentrated and purified by Pre-HPLC to give a white solid (42 mg, 70% yield).

MS (ESI), m/z, 1514.2 $[M+H]^+$.

Synthesis of compound **8**

To a solution of compound **8-2** (42 mg, 0.027 mmol, 1.5 eq) in DMF (5 mL) were added **101B13** (44 mg, 0.018 mmol, 1.0 eq, based on the polymer structural unit), DIPEA (0.1 mL), and PyBOP (28 mg, 0.054 mmol) at room temperature (25 °C) under nitrogen atmosphere. The reaction mixture was stirred overnight. After the reaction was completed, the reaction mixture was concentrated, ultrafiltered with a 30K MW ultrafiltration membrane, concentrated, and lyophilized to give a product (45 mg, 99% purity).

Example 9: Synthesis of compound 9

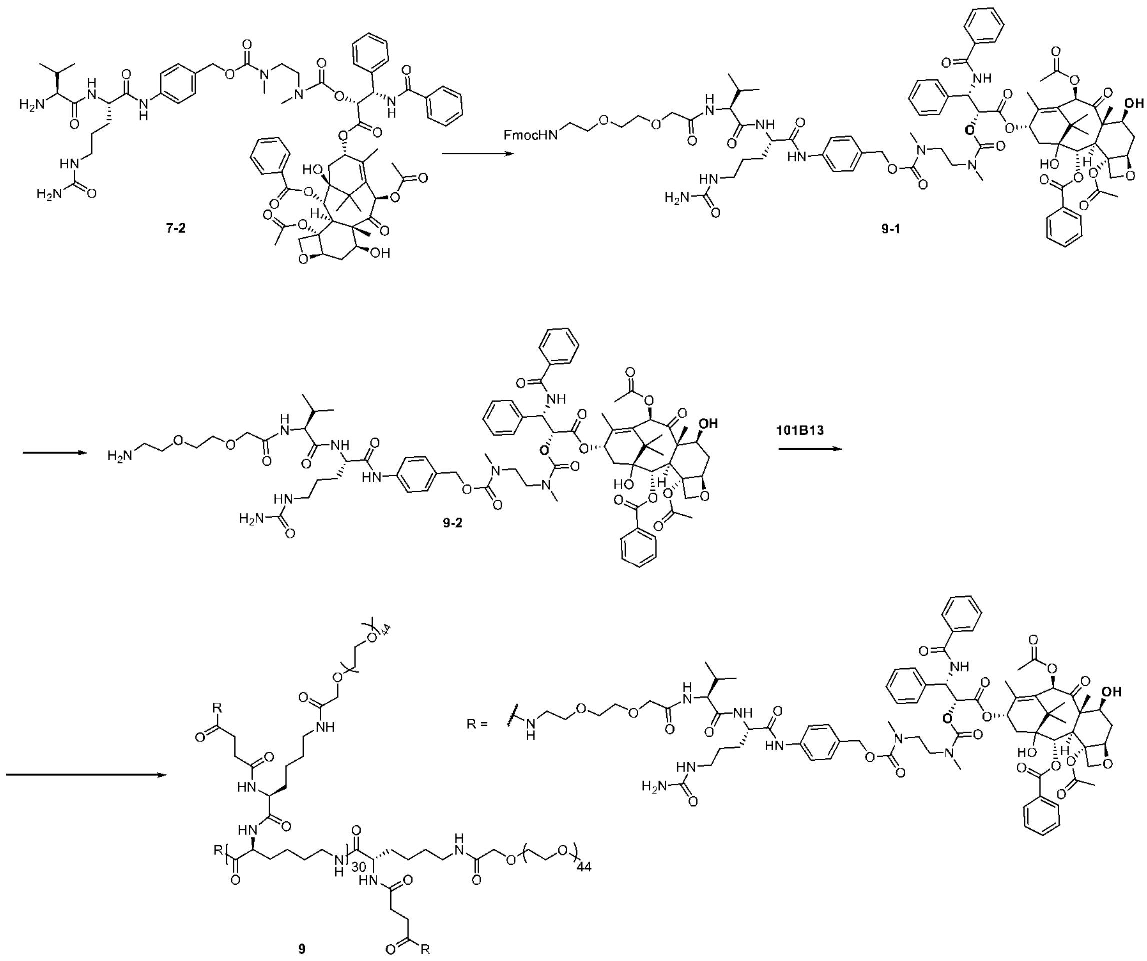


7-2 9-1 9-2 9

Synthesis of compound **9-1**

To a solution of [2-[2-(Fmoc-amino)ethoxy]ethoxy]acetic acid (168 mg, 0.436 mmol, 1.0 eq) in DMF (8 mL) were added DIPEA (225 mg, 1.744 mmol, 4.0 eq) and TSTU (131 mg, 0.436 mmol, 1.0 eq) at 0 °C under nitrogen atmosphere. The mixture was stirred at room temperature for 1 h. To the reaction mixture was added a solution of **7-2** (600 mg, 0.436 mmol, 1.0 eq) in DMF (4 mL), and the mixture was stirred at room temperature for 1 h. When LCMS indicated the completion of the reaction, the reaction mixture was directly used in the next reaction.

Synthesis of compound **9-2**

To a solution of compound **9-1** (500 mg, 0.287 mmol, 1.0 eq) in DMF (15 mL) was added diethylamine (420 mg, 5.74 mmol, 20.0 eq) at room temperature under nitrogen atmosphere, and the reaction mixture was stirred at room temperature for 1 h. When LCMS indicated the completion of the reaction, the reaction mixture was purified on a C18 column (ACN/TFA 0.05% $H_2O$) to give compound **9-2** (white solid, 400 mg, 91% yield).

Synthesis of compound **9**

To a solution of **101B13** (686 mg, 0.29 mmol, 1.0 eq, based on the polymer structural unit), compound **9-2** (800 mg, 0.435 mmol, 1.5 eq), and PyBOP (227 mg, 0.435 mmol, 1.5 eq) in DMF (8 mL) was added DIPEA (150 mg, 1.16 mmol, 4.0 eq) at room temperature under nitrogen atmosphere. The mixture was stirred at room temperature for 16 h. The reaction mixture was concentrated by rotavap using an oil pump, and methyl *tert*-butyl ether was added. The mixture was stirred for 20 min, and the supernatant was discarded. The oily substance at the bottom of the flask was dissolved in methanol, and an equal volume of water was added. The mixture was filtered and ultrafiltered to remove about 1.5 L of solvent. A sample was taken for HPLC detection, which indicated purities of 99% at 254 nm and 98% at 210 nm. The mixture was ultrafiltered with a 30K MW ultrafiltration membrane, concentrated to remove methanol, and lyophilized to give compound **9** (white solid, 361 mg, 85% yield).

Example 10: Synthesis of compound 10

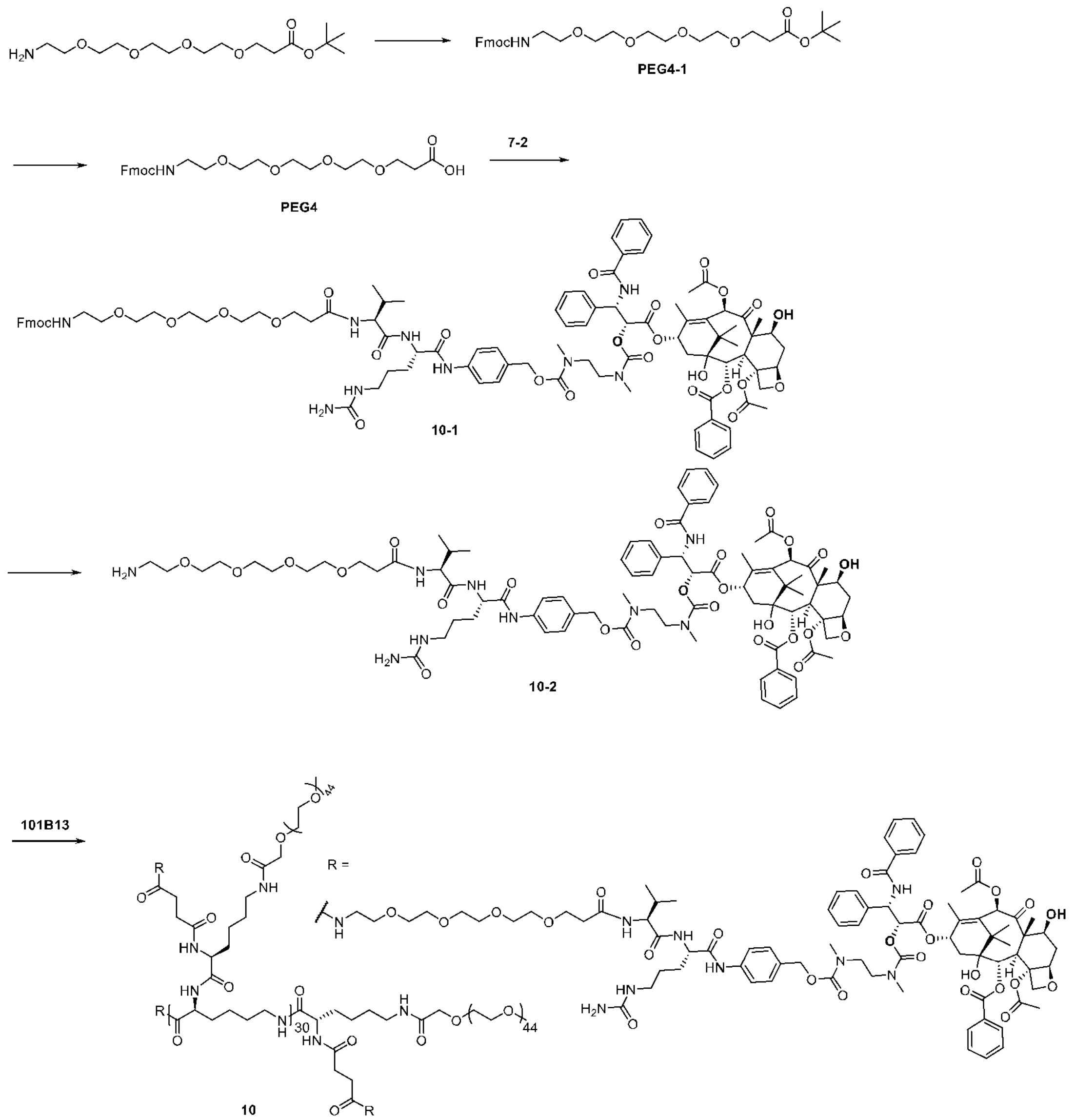


PEG4-1
PEG4
10-1
10-2
R =
10

Synthesis of compound **PEG4-1**

To a solution of compound *tert*-butyl 15-amino-4,7,10,13-tetraoxapentadecanoate (1.9 g, 5.91 mmol, 1.0 eq) and DIPEA (1.52 g, 11.8 mmol, 2.0 eq) in dichloromethane (40 mL) was added FmocOSU (1.99 g, 5.91 mmol, 1.0 eq) at room temperature under nitrogen atmosphere. The mixture was stirred at room temperature for 16 h. When LCMS indicated the completion of the reaction, water was added to terminate the reaction, and the phases were separated. The aqueous phase was extracted with dichloromethane, and the organic phase was concentrated, and purified by column chromatography to give compound **PEG4-1** (colorless oil, 3.4 g, 100% yield).
MS (ESI), m/z, 544.2 $[M+H]^+$.

Synthesis of compound **PEG4**

To a solution of compound **PEG4-1** (3.3 g, 6.07 mmol, 1.0 eq) in dichloromethane (20 mL) was added TFA (10 mL) at room temperature. The mixture was stirred at 30 °C for 3 h under nitrogen atmosphere. When LCMS indicated the completion of the reaction, the mixture was concentrated *in vacuo* to give a yellow oily substance (4 g, crude), which was directly used in the next reaction.

MS (ESI), m/z,488.2 $[M+H]^+$.

Synthesis of compound **10-1**

Compound **PEG4** (3.6 g, 7.38 mmol, 1.0 eq) and DIPEA (9 mL) were dissolved in dichloromethane (90 mL) at room temperature under nitrogen atmosphere. TSTU (3.3 g, 11.1 mmol, 1.5 eq) was added at 30 °C under nitrogen atmosphere, and the mixture was stirred at room temperature for 2 h. When LCMS indicated the completion of the reaction, the mixture was concentrated *in vacuo* and separated by reversed-phase column chromatography (A = TFA (0.1% + $H_2O$,) B = acetonitrile). The prepared mixture was extracted with ethyl acetate, dried over sodium sulfate, filtered, and dried *in vacuo* to give a yellow oil (2.65 g, 61.6% yield).

MS (ESI), m/z, 585.2 [M+H]+.

To a solution of the above intermediate (127.7 mg, 0.2184 mmol, 1.0 eq) and DIPEA (112.7 mg, 0.8736 mmol, 4.0 eq) in DMSO (5 mL) was added compound 7-**2** (300 mg, 0.2184 mmol, 1.0 eq) at 30 °C under nitrogen atmosphere, and the mixture was stirred at room temperature for 1 h. When LCMS indicated the completion of the reaction, the reaction mixture was directly used in the next reaction.

MS (ESI), m/z, 1843.0 [M+H]+.

Synthesis of compound **10-2**

To the reaction mixture of compound **10-1** was added diethylamine (2.5 mL) at room temperature under nitrogen atmosphere, and the mixture was stirred at room temperature for 1 h. When LCMS indicated the completion of the reaction, the mixture was separated by reversed-phase column chromatography (A = TFA (0.1% + $H_2O$,) B = acetonitrile) and lyophilized to give a white solid (226 mg, 69.7% yield).

MS (ESI), m/z, 1621.0 $[M+H]^+$.

Synthesis of compound **10**

**101B13** (80 mg, 0.034 mmol, 1.0 eq, based on the polymer structural unit), compound **10-2** (82.7 mg, 0.051 mmol, 1.5 eq), PyBOP (26.5 mg, 0.051 mmol, 1.5 eq), DIPEA (0.025 mL, 0.136 mmol, 4.0 eq), and DMF (1.5 mL) were added sequentially at room temperature (25 °C) under nitrogen atmosphere. The reaction mixture was stirred at room temperature for 15 h until HPLC monitoring indicated the depletion of the starting materials. To the above DMF mixture was added MTBE (30 mL). The solution turned turbid and a white solid was precipitated. The supernatant was discarded, and the white solid was washed with MTBE and dried. The remaining white solid was dissolved in MeOH and ultrafiltered (30K MW) with MeOH/water to remove small molecule impurities. When HPLC monitoring indicated the complete removal of small molecule impurities, the mixture was collected and concentrated by rotavap to remove MeOH. The aqueous solution was lyophilized to give compound **10** (white solid, 156 mg, 99.33% purity).

Example 11: Synthesis of compound 11

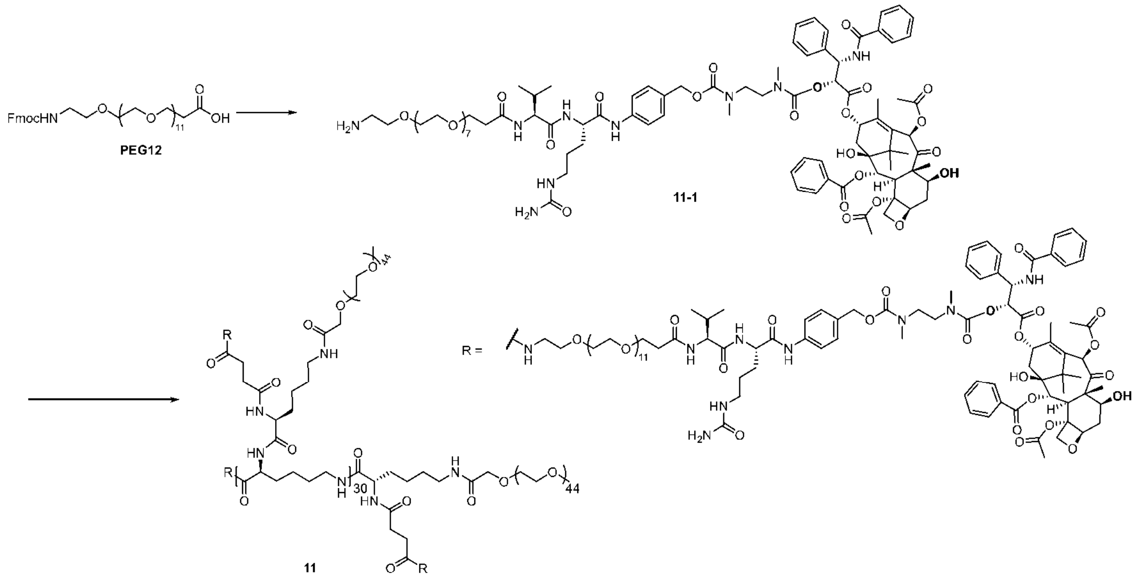

PEG12

11-1

11

Synthesis of compound **11-1**

To a solution of **PEG12** (367 mg, 0.437 mmol, 1.0 eq) in DMSO (10 mL) were added DIPEA (225.4 mg, 1.747 mmol, 4.0 eq) and TSTU (131.5 mg, 0.437 mmol, 1.0 eq) sequentially at room temperature (25 °C) under nitrogen atmosphere. The mixture was stirred at room temperature for 1 h, and compound 7-2 (600 mg, 0.437 mmol, 1.0 eq) was added. The mixture was stirred at room temperature for 1 h. When LCMS indicated the completion of the reaction, diethylamine (1 mL) was added. The mixture was stirred at room temperature for 1 h, concentrated, and directly purified by reversed-phase column chromatography to give a white solid (0.5 g, 58% yield).

MS (ESI), m/z, 1973.2 $[M+H]^+$.

Synthesis of compound **11**

To a solution of compound **11-1** (500 mg, 0.253 mmol, 1.5 eq, based on the polymer unit) in DMF were added **101B13** (399.5 mg, 0.169 mmol, 1.0 eq, based on the polymer structural unit), PyBOP (131.8 mg, 0.253 mmol, 1.5 eq), and DIPEA (87.2 mg, 0.676 mmol, 4.0 eq) at room temperature under nitrogen atmosphere, and the mixture was stirred for 16 h. The reaction mixture was ultrafiltered (30K MW) and lyophilized to give compound **11** (white solid, 0.336 g, 45.9% yield, about 19.1% PTX content).

Example 12: Synthesis of compound 12a

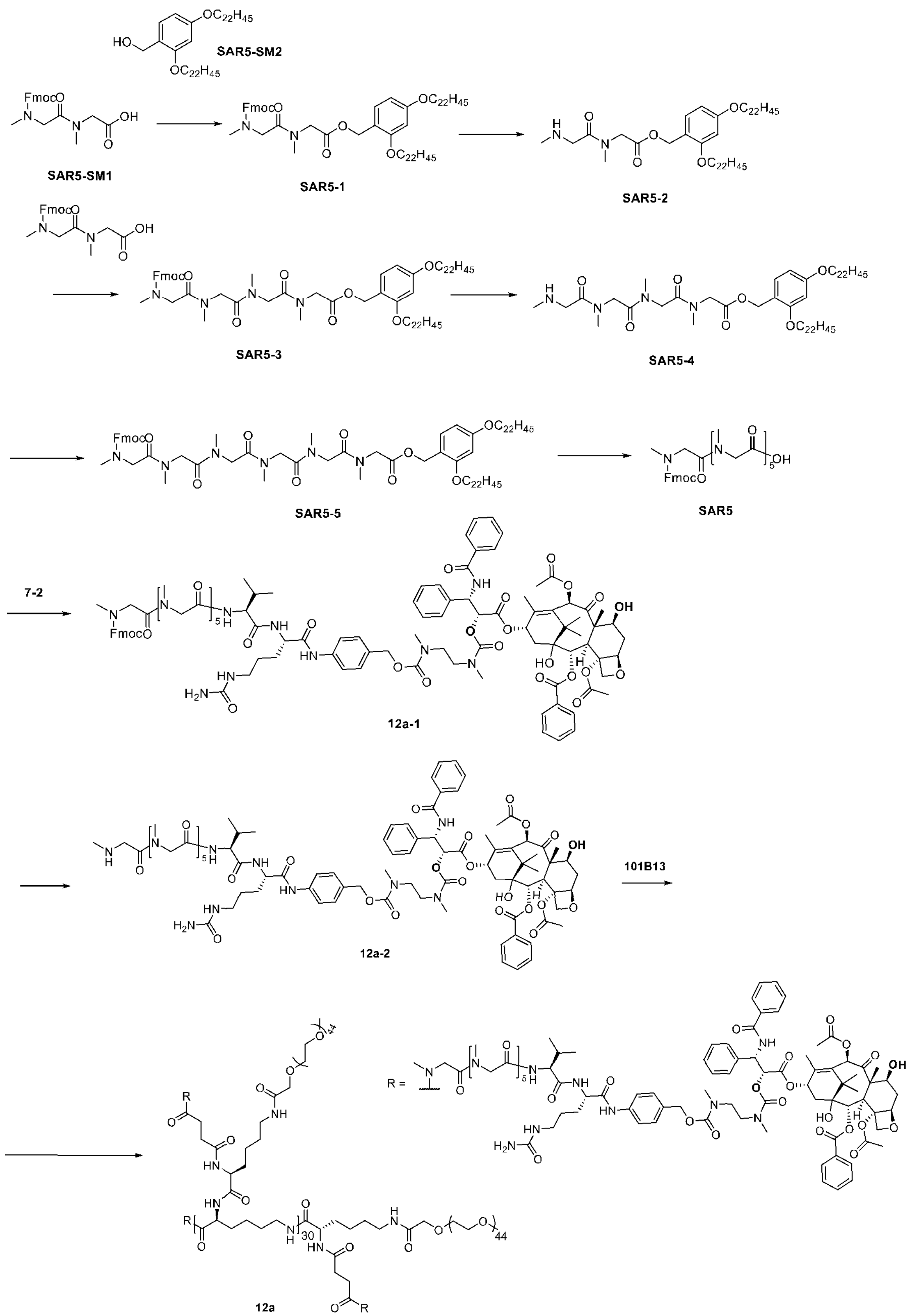

SAR5-SM2
SAR5-SM1
SAR5-1
SAR5-2
SAR5-3
SAR5-4
SAR5-5
SAR5
12a-1
12a-2
12a

Synthesis of compound **SAR5-1**

To a solution of **SAR5-SM2** (5.0 g, 6.61 mmol, 1.0 eq, according to the synthesis of Fmoc-Sar-Sar-OH in Patent No. WO2019081455A1) and **SAR5-SM1** (5.0 g, 13.2 mmol, 2.0 eq, according to the synthesis in Example 2 in Patent No.

US2012296074A1) in dry $CH_2Cl_2$ (100 mL) were added DIC (1.7 g, 13.2 mmol, 2.0 eq) and DMAP (0.16 g, 1.32 mmol, 0.2 eq) at room temperature (25 °C) under nitrogen atmosphere, and the mixture was stirred for 12 h. When TLC indicated the completion of the reaction, the mixture was concentrated to 20 mL, and acetonitrile (300 mL) was added. The mixture was filtered and dried *in vacuo* to give compound **SAR5-1** (white solid, 7.0 g, 95% yield).

Synthesis of compound **SAR5-2**

To a solution of **SAR5-1** (7.0 g, 6.25 mmol) in THF (100 mL) were added piperidine (1 mL) and DBU (1 mL) at room temperature (25 °C) under nitrogen atmosphere, and the mixture was stirred at room temperature for 1 h. When TLC monitoring indicated the completion of the reaction, the reaction mixture was cooled to 5 °C, and 6 N HCl was added to adjust to pH 7. Acetonitrile (300 mL) was added, and the mixture was filtered. The solid was collected and dried *in vacuo* to give compound **SAR5-2** (white solid, 5.0 g, 89% yield).

Synthesis of compound **SAR5-3**

To a solution of **SAR5-2** (4.0 g, 4.45 mmol, 1.0 eq) and **SAR5-SM1** (2.5 g, 6.67 mmol, 1.5 eq) in dry THF (80 mL) were added HBTU (2.5 g, 6.67 mmol, 1.5 eq), HOBt (0.9 g, 6.67 mmol, 1.5 eq), and DIPEA (1.7 g, 13.4 mmol, 3.0 eq) at room temperature (25 °C) under nitrogen atmosphere, and the mixture was stirred for 2 h. When TLC indicated the completion of the reaction, n-propylamine (0.8 g) was added to quench the reaction, and the mixture was stirred for 1 h. Acetonitrile (500 mL) and celite (40 g) were added to assist filtration, and the mixture was filtered. The solid was dissolved in 100 mL of THF, and the mixture was again filtered. The filtrate was concentrated and dried *in vacuo* to give compound **SAR5-3** (white solid, 5.0 g, 88% yield).

Synthesis of compound **SAR5-4**

To a solution of **SAR5-3** (4.0 g, 3.2 mmol) in THF (100 mL) were added piperidine (1 mL) and DBU (1 mL) at room temperature (25 °C) under nitrogen atmosphere, and the mixture was stirred at room temperature for 1 h. When TLC monitoring indicated the completion of the reaction, the reaction mixture was cooled to 5 °C, and 6 N HCl was added to adjust to pH 7. Acetonitrile (400 mL) and celite (40 g) were added to assist filtration, and the mixture was filtered. The solid was dissolved in 100 mL of THF, and the mixture was again filtered. The filtrate was concentrated and dried *in vacuo* to give compound **SAR5-4** (white solid, 3.0 g, 91% yield).

Synthesis of compound **SAR5-5**

To a solution of **SAR5-4** (3.0 g, 2.88 mmol, 1.0 eq) and **SAR5-SM1** (1.65 g, 2.88 mmol, 1.5 eq) in dry THF (70 mL) were added HBTU (1.64 g, 4.33 mmol, 1.5 eq), HOBt (0.58 g, 4.33 mmol, 1.5 eq), and DIPEA (1.12 g, 8.65 mmol, 3.0 eq) at room temperature (25 °C) under nitrogen atmosphere, and the mixture was stirred for 2 h. When TLC indicated the completion of the reaction, *n*-propylamine (0.5 g) was added to quench the reaction, and the mixture was stirred for 1 h. Acetonitrile (500 mL) and celite (40 g) were added to assist filtration, and the mixture was filtered. The solid was dissolved in 100 mL of THF, and the mixture was again filtered. The filtrate was concentrated and dried *in vacuo* to give compound **SAR5-5** (white solid, 3.5 g, 86% yield).

Synthesis of compound **SAR5**

To **SAR5-5** (3.0 g, 2.88 mmol, 1.0 eq) was added 50 mL of a pre-formulated solution (5 mL of 2,2,2-trifluoroethanol and 0.5 mL of trifluoroacetic acid in 50 mL of dichloromethane) at room temperature (25 °C) under nitrogen atmosphere, and the mixture was stirred for 0.5 h. When TLC indicated the completion of the reaction, celite (40 g) was added to assist filtration, and the mixture was filtered. The filtrate was concentrated and purified by Pre-HPLC to give compound **SAR5** (1.0 g, 71% yield).

MS (ESI), m/z, 667.3 $[M+H]^+$.

Synthesis of compound **12a-1**

To a solution of **SAR5** (291 mg, 0.44 mmol, 1.2 eq) in dry $CH_2Cl_2$ (5 mL) were added TSTU (165 mg, 0.55 mmol, 1.5 eq) and DIPEA (94 mg, 0.73 mmol, 3.0 eq) in an ice-water bath (5 °C) under nitrogen atmosphere, and the mixture was stirred for 5 min (LCMS monitoring indicated the production of an active ester). A solution of **7-2** (500 mg, 0.36 mmol, 1.0 eq) in dichloromethane (2 mL) was added, and the mixture was stirred for 5 min. Methanol was added to quench the reaction, and the mixture was concentrated to give a crude product **12a-1,** which was directly used in the next reaction.

Synthesis of compound **12a-2**

To a solution of crude compound **12a-1** in dichloromethane was added diethylamine (5 mL) under nitrogen atmosphere. The mixture was stirred for 1 h at room temperature and concentrated directly, and the crude product was purified by Pre-HPLC ($CH_3CN/H_2O$, 0.05% TFA) to give a product **12a-2** (500 mg, colorless oil, 73% yield).
MS (ESI), m/z, 1799.1 $[M+H]^+$.

Synthesis of compound **12a**

To a solution of **101B13** (250 mg, 0.003 mmol, 1.0 eq) in dry DMF (5 mL) were added **12a-2** (276 mg, 0.15 mmol, 45.0 eq), PyBOP (142 mg, 0.27 mmol, 80.0 eq), and DIPEA (56 mg, 0.044 mmol, 128.0 eq) at room temperature (25 °C) under nitrogen atmosphere. The mixture was then heated to 28 °C, stirred for 12 h, concentrated, filtered from methanol to methanol/water (1/1, 100 mL), purified by ultrafiltration (30K MW), concentrated, and lyophilized to give a product **12a** (350 mg, 85% yield, 99% purity, about 20% PTX content).

Example 13: Synthesis of compound 12b

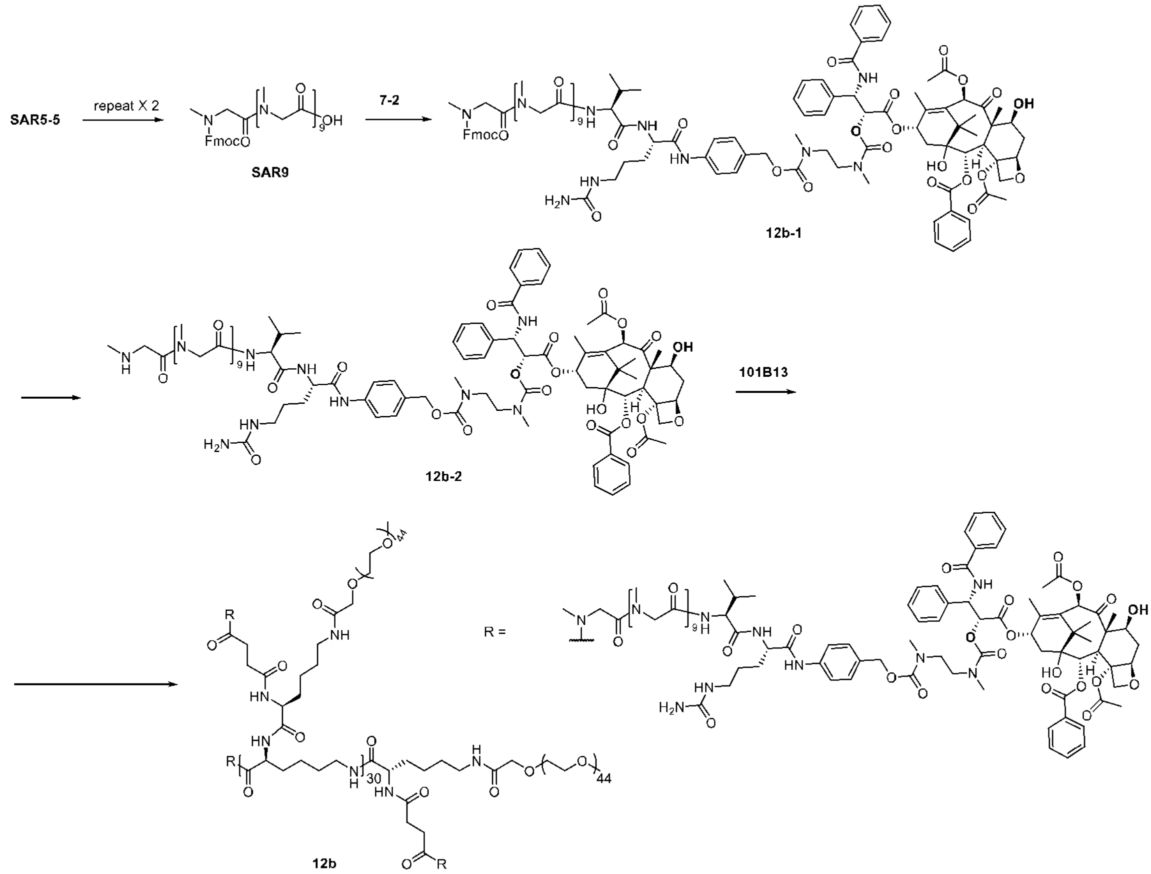

SAR5-5
SAR9
12b-1
12b-2
12b

Synthesis of compound **SAR9**

The starting material **SAR5-5** was subjected to two condensations, two Fmoc removals, and TAG protecting group removal to give compound SAR9 (1.2 g, 50% yield over the five steps).
MS (ESI), m/z, 951.3 $[M+H]^+$.

Synthesis of compound **12b-1**

To a solution of **SAR9** (416 mg, 0.44 mmol, 1.2 eq) in dry $CH_2Cl_2$ (5 mL) were added TSTU (165 mg, 0.55 mmol, 1.5 eq) and DIPEA (94 mg, 0.73 mmol, 3.0 eq) in an ice-water bath (5 °C) under nitrogen atmosphere, and the mixture was

stirred for 5 min (LCMS monitoring indicated the production of an active ester). A solution of 7-**2** (500 mg, 0.36 mmol, 1.0 eq) in dichloromethane (2 mL) was added, and the mixture was stirred for 5 min. Methanol was added to quench the reaction, and the mixture was concentrated to give a crude product **12b-1,** which was directly used in the next reaction.

Synthesis of compound **12b-2**

To a solution of crude compound **12b-1** in dichloromethane was added diethylamine (5 mL) under nitrogen atmosphere. The mixture was stirred for 1 h at room temperature and concentrated directly, and the crude product was purified by Pre-HPLC ($CH_3CN/H_2O$, 0.05% TFA) to give a product **12b-2** (650 mg, colorless oil, 85% yield).

MS (ESI), m/z, 1041.1 $[M/2]^+$.

Synthesis of compound **12b**

To a solution of **101B13** (250 mg, 0.003 mmol, 1.0 eq) in dry DMF (5 mL) were added **12b-2** (319 mg, 0.15 mmol, 45.0 eq), PyBOP (142 mg, 0.27 mmol, 80.0 eq), and DIPEA (56 mg, 0.044 mmol, 128.0 eq) at room temperature (25 °C) under nitrogen atmosphere. The mixture was then heated to 28 °C, stirred for 12 h, concentrated, filtered from methanol to methanol/water (1/1, 100 mL), purified by ultrafiltration (30K MW), and lyophilized to give a product **12b** (400 mg, 86% yield, 98% purity, about 19% PTX content).

Example 14: Synthesis of compound 13

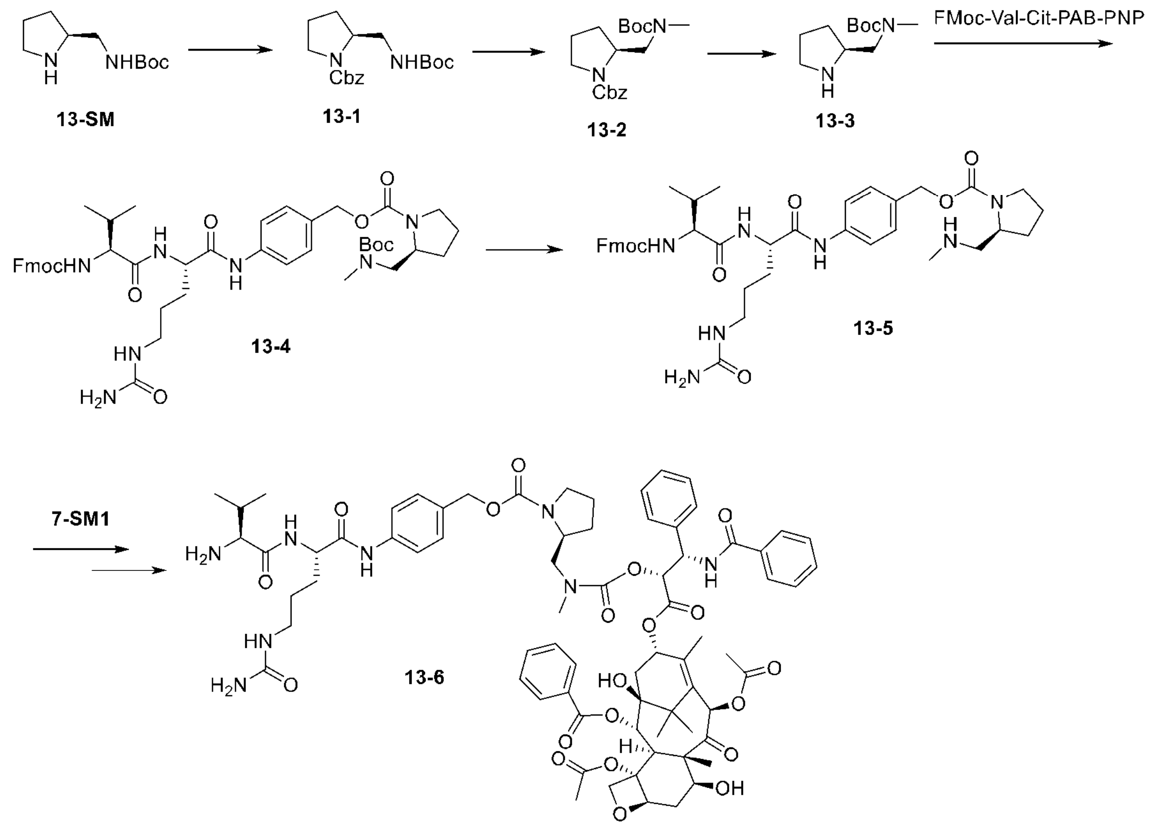


13-SM 13-1 13-2 13-3 13-4 13-5 13-6

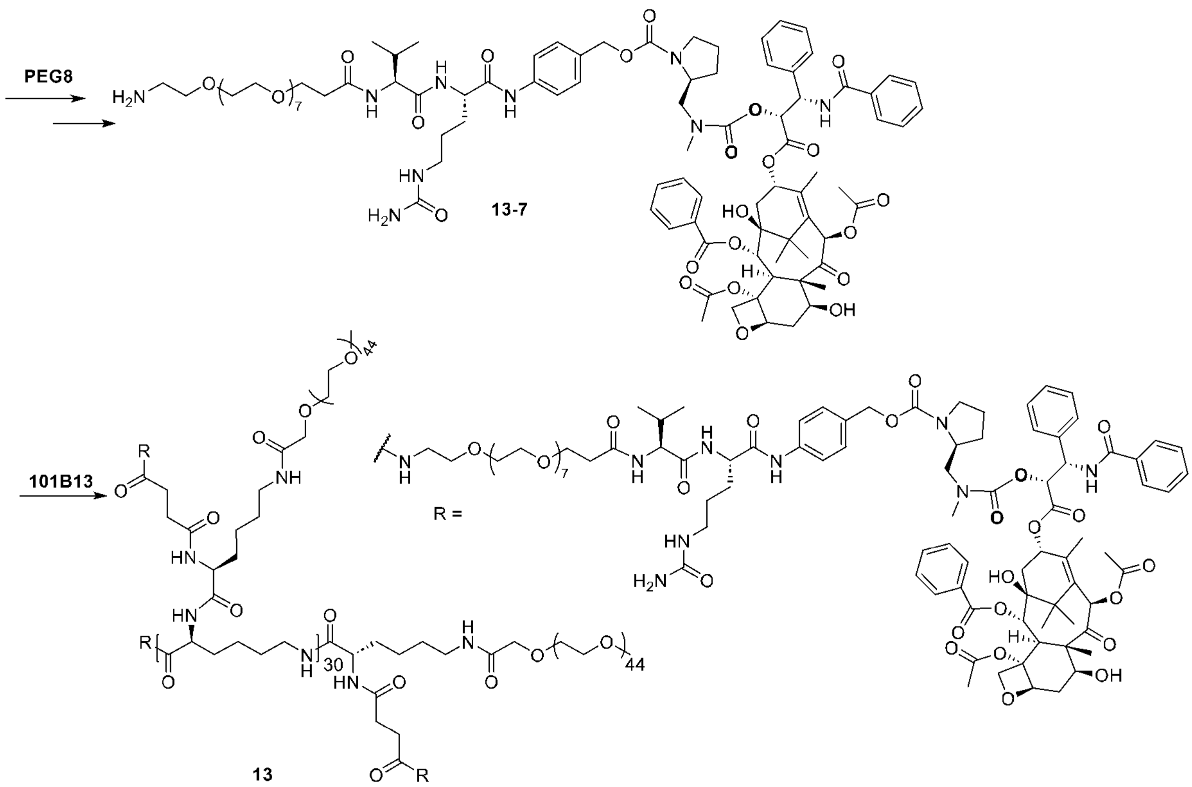


13-7

13

Synthesis of compound **13-1**

**13-SM** (5.0 g, 25 mmol, 1.0 eq), anhydrous DCM (50 mL), and triethylamine (7.0 mL, 50 mmol, 2.0 eq) were added sequentially at room temperature under nitrogen atmosphere, and benzyl chloroformate (4.6 mL, 32.5 mmol, 1.3 eq) was added dropwise. The reaction mixture was stirred at room temperature for 3 h until LCMS monitoring indicated the depletion of the starting materials. The mixture was concentrated, and a small amount of DCM was added for dissolution. The mixture was filtered and separated by column chromatography (PE/EA = 1:1-1:3) to give a product **13-1** (colorless oily substance, 7.6 g, 91% yield).

$^1$H NMR (400 MHz, $CDCl_3$) δ 7.49 - 7.27 (m, 5H), 5.14 (s, 2H), 4.03 - 3.81 (m, 1H), 3.60 - 3.13 (m, 4H), 2.01 - 1.72 (m, 4H), 1.43 (s, 9H).

Synthesis of compound **13-2**

**13-1** (669 mg, 2.0 mmol, 1.0 eq), anhydrous THF (6 mL), and methyl iodide (0.3 mL, 5.0 mmol, 2.5 eq) were added sequentially at room temperature under nitrogen atmosphere. The mixture was cooled to 5 °C in an ice-water bath, and NaH (475 mg, 11.4 mmol, 5.7 eq) was added. The reaction mixture was warmed to room temperature slowly and stirred for 3 h until LCMS monitoring indicated the depletion of the starting materials. The reaction was quenched by adding an $NH_4Cl$ solution. The mixture was extracted with EA, and the organic phases were combined, dried, concentrated by rotavap, and separated on a column (PE/EA = 2:1) to give a product **13-2** (colorless oily substance, 378 mg, 54% yield).

$^1$H NMR (400 MHz, $CDCl_3$) δ 7.41 - 7.28 (m, 5H), 5.21 - 5.03 (m, 2H), 4.19 - 3.85 (m, 1H), 3.58 - 3.02 (m, 4H), 2.96 - 2.65 (m, 3H), 2.05 - 1.76 (m, 4H), 1.45 (s, 9H).

Synthesis of compound **13-3**

**13-2** (378 mg, 1.1 mmol, 1.0 eq), $H_2O$ (5 mL), THF (5 mL), and acetic acid (0.8 mL) were added sequentially at room temperature under nitrogen atmosphere. The atmosphere was replaced with hydrogen atmosphere via a balloon, and Pd/C (113 mg, 0.11 mmol, 0.1 eq) was added. The reaction mixture was stirred at room temperature for 3 h until LCMS monitoring indicated the depletion of the starting materials. The mixture was filtered and concentrated by rotavap to give a product **13-3** (oily substance, 398 mg).

Synthesis of compound **13-4**

**13-3** (398 mg, 1.0 mmol, 1.0 eq), DMF (5 mL), DIPEA (0.52 mL, 3.0 mmol, 3.0 eq), and FMOC-Val-Cit-PAB-PNP (1.15 g, 1.5 mmol, 1.5 eq) were added sequentially at room temperature under nitrogen atmosphere. The reaction mixture was stirred at room temperature for 1.5 h until LCMS monitoring indicated the depletion of the starting materials. The reaction mixture was purified by reversed-phase column chromatography to give a product **13-4** (white solid, 700 mg, 83% yield over the two steps).

$^1$H NMR (400 MHz, DMSO) δ 10.06 (s, 1H), 8.12 (d, *J* = 7.7 Hz, 1H), 7.89 (d, *J* = 7.5 Hz, 2H), 7.75 (t, *J* = 7.8 Hz, 2H), 7.62 - 7.53 (m, 2H), 7.47 - 7.38 (m, 3H), 7.35 - 7.27 (m, 4H), 6.12 - 5.84 (m, 1H), 4.99 (s, 2H), 4.51 - 4.21 (m, 5H), 3.41 - 2.89 (m, 6H), 2.86 - 2.64 (m, 3H), 2.09 - 1.55 (m, 7H), 1.38 (d, *J* = 4.9 Hz, 9H), 0.87 (dd, *J* = 10.7, 6.7 Hz, 6H).

Synthesis of compound **13-5**

At room temperature, **13-4** (625 mg), DCM (10 mL), and TFA (10 mL) were added sequentially. The reaction mixture was stirred at room temperature for 0.5 h until LCMS monitoring indicated the depletion of the starting materials. To the mixture was added MTBE, and a white solid was precipitated. The mixture was filtered to give a product **13-5** (white solid, 750 mg).

$^1$H NMR (400 MHz, DMSO) δ 10.09 (s, 1H), 8.43 - 8.24 (m, 2H), 8.14 (d, *J* = 7.5 Hz, 1H), 7.90 (d, *J* = 7.5 Hz, 2H), 7.75 (t, *J* = 7.8 Hz, 2H), 7.61 (d, *J* = 8.2 Hz, 2H), 7.48 - 7.39 (m, 4H), 7.37 - 7.29 (m, 4H), 6.26 - 5.93 (m, 1H), 5.42 - 4.90 (m, 2H), 4.47 - 4.19 (m, 4H), 4.06 (d, *J* = 7.1 Hz, 1H), 3.93 (dd, *J* = 8.9, 7.0 Hz, 1H), 3.43 - 3.29 (m, 1H), 3.06 - 2.91 (m, 3H), 2.58 (t, *J* = 5.3 Hz, 2H), 2.07 - 1.31 (m, 8H), 0.87 (dd, *J* = 10.6, 6.8 Hz, 6H).

Synthesis of compound **13-6**

**13-5** (750 mg, 1.0 mmol, 1.0 eq), **7-SM1** (1.5 g, 1.5 mmol, 1.5 eq), and DMF (5 mL) were added sequentially at room temperature under nitrogen atmosphere. The mixture was cooled to 5 °C in an ice bath, and DIPEA (0.36 mL, 2.0 mmol, 2.0 eq) was added. The reaction mixture was stirred for 1 h at 5 °C. When LCMS monitoring indicated the depletion of the starting materials, diethylamine (0.8 mL, 8.0 mmol, 8.0 eq) was added. The reaction mixture was stirred at room temperature for 0.5 h until LCMS monitoring indicated the depletion of the starting materials. The mixture was purified and lyophilized to give a product **13-6** (404 mg, white solid, 36% yield over the three steps).

$^1$H NMR (400 MHz, DMSO) δ 10.21 (s, 1H), 9.32 - 9.02 (m, 1H), 8.70 (d, *J* = 7.5 Hz, 1H), 8.18 - 8.04 (m, 3H), 8.03 - 7.93 (m, 2H), 7.88 - 7.79 (m, 2H), 7.77 - 7.39 (m, 12H), 7.37 - 7.15 (m, 3H), 6.37 - 6.25 (m, 1H), 6.09 (s, 1H), 5.95 - 5.51 (m, 2H), 5.46 - 5.15 (m, 2H), 5.06 - 4.86 (m, 3H), 4.58 - 4.48 (m, 1H), 4.20 - 3.90 (m, 4H), 3.31 - 2.71 (m, 9H), 2.38 - 2.01 (m, 8H), 1.94 - 1.57 (m, 12H), 1.50 (s, 6H), 1.07 - 0.92 (m, 12H).

Synthesis of compound **13-7**

**PEG8** (86.3 mg, 0.13 mmol, 1.1 eq), DMF (1.5 mL), and DIPEA (0.18 mL, 0.36 mmol, 3.0 eq) were added sequentially at room temperature under nitrogen atmosphere. The reaction mixture was stirred at room temperature for 0.5 h until LCMS monitoring indicated the depletion of the starting materials. The mixture was then cooled to 5 °C in an ice bath, and **13-6** (180 mg, 0.12 mmol, 1.0 eq) was added. The mixture was stirred at 5 °C for 1 h until LCMS monitoring indicated the depletion of the starting materials. The reaction mixture was used in the next step without further processing. To the above reaction mixture was added diethylamine (0.1 mL, 0.95 mmol, 8.0 eq) at room temperature under nitrogen atmosphere. The reaction mixture was stirred at room temperature for 1 h until LCMS monitoring indicated the depletion of the starting materials. The mixture was directly purified by reversed-phase column chromatography (MeCN/$H_2O$-0.05% TFA) and lyophilized to give a product **13-7** (135 mg, white solid, 59% yield over the two steps).

$^1$H NMR (400 MHz, DMSO) δ 9.99 (s, 1H), 9.28 - 9.02 (m, 1H), 8.12 (d, *J* = 7.4 Hz, 1H), 8.03 - 7.94 (m, 2H), 7.92 - 7.38 (m, 21H), 7.36 - 7.05 (m, 3H), 6.30 (s, 1H), 6.08 - 5.15 (m, 6H), 5.05 - 3.92 (m, 10H), 3.04 - 2.66 (m, 10H), 2.43 - 1.54 (m, 28H), 1.29 - 1.20 (m, 7H), 1.04 - 0.99 (m, 6H), 0.87 - 0.82 (m, 6H).

Synthesis of compound **13**

**101B13** (93.6 mg, 0.04 mmol, 1.0 eq, based on the polymer structural unit), **13-7** (115 mg, 0.059 mmol, 1.5 eq), PyBOP (30.7 mg, 0.059 mmol, 1.5 eq), DIPEA (0.03 mL, 0.16 mmol, 4.0 eq), and DMF (1 mL) were added sequentially at room temperature (25 °C) under nitrogen atmosphere. The reaction mixture was stirred at room temperature for 15 h until HPLC monitoring indicated the depletion of the starting materials. To the above DMF mixture was added MTBE (30 mL). The solution turned turbid and a white solid was precipitated. The supernatant was discarded, and the white solid was washed with MTBE and dried. The remaining white solid was dissolved in MeOH, and the mixture was purified by

ultrafiltration (30K MW) and lyophilized to give a product **13** (white solid, 155 mg, 100% purity, about 20.8% PTX content).

Example 15: Synthesis of compound 14

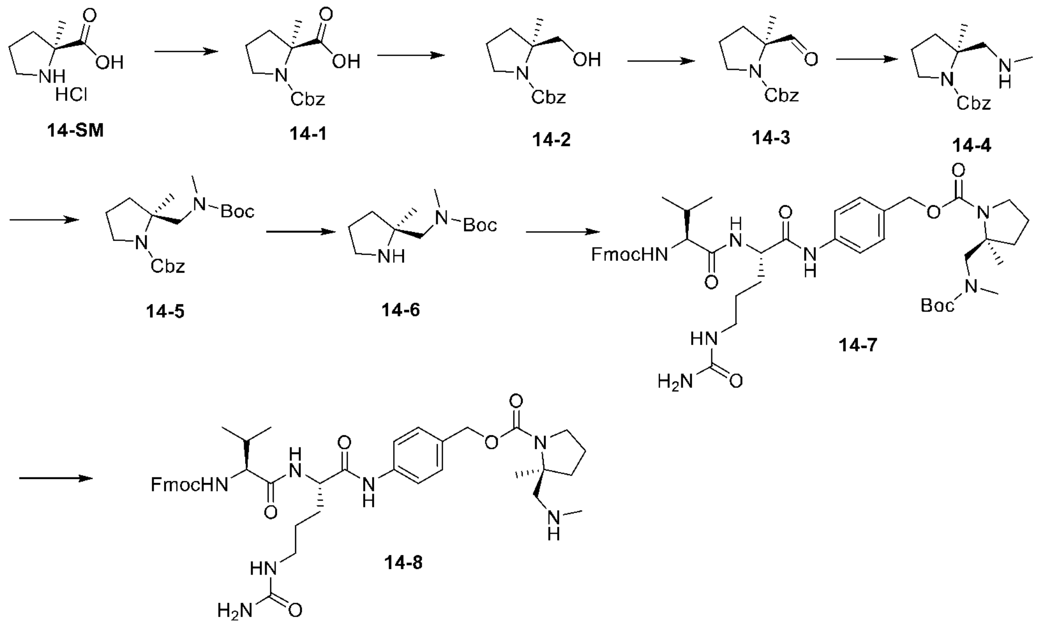

14-SM 14-1 14-2 14-3 14-4

14-5 14-6 14-7

14-8

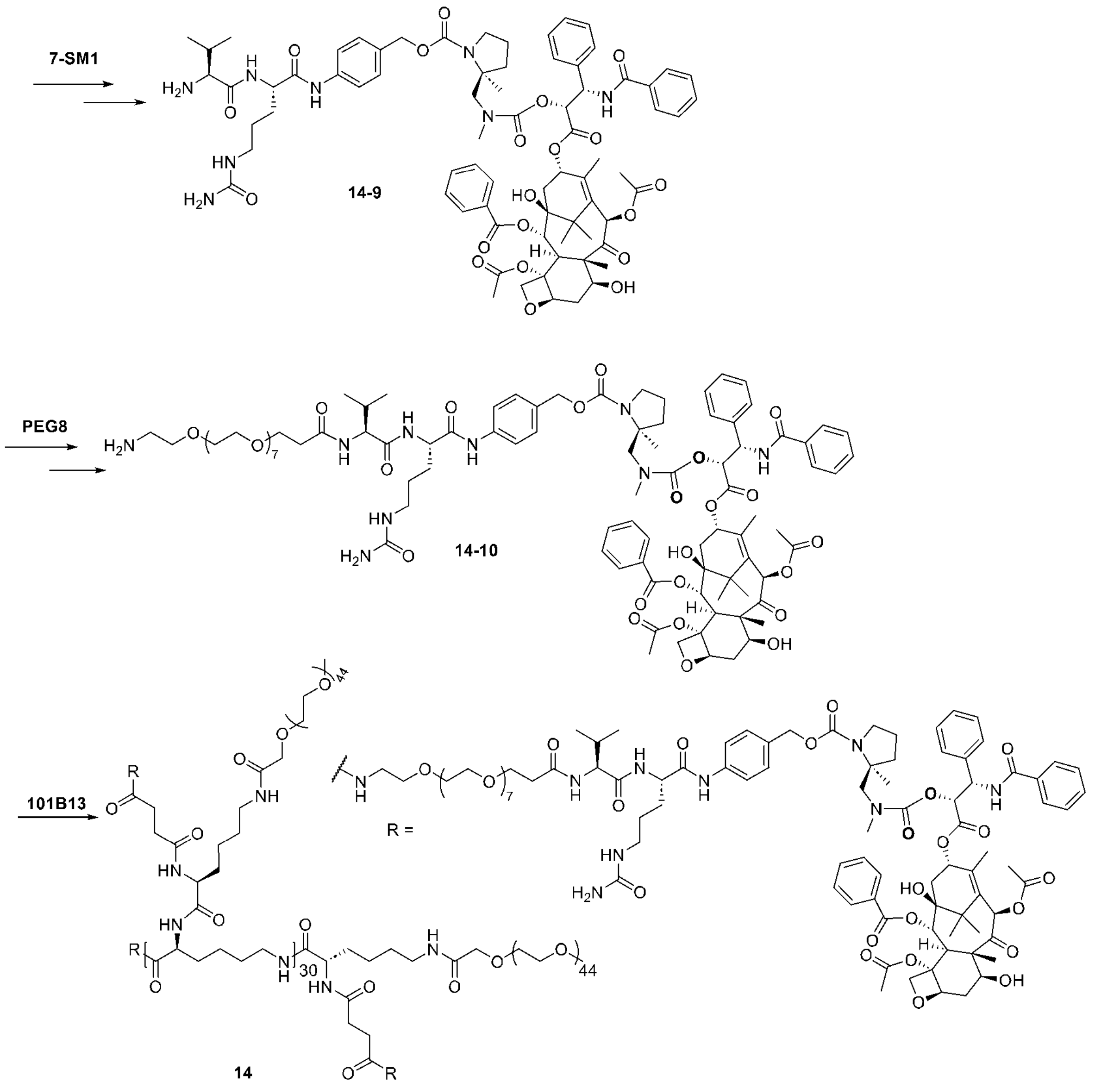


14-9

14-10

14

Synthesis of compound **14-1**

To a solution of **14-SM** (4.0 g, 24.15 mmol, 1.0 eq) in DCM (50 mL) was added triethylamine (4.9 g, 48.3 mmol, 2.0 eq) at room temperature (25 °C) under nitrogen atmosphere. CbzCl (4.9 g, 28.98 mmol, 1.2 eq) in DCM (10 mL) was added dropwise, and after the addition, the reaction mixture was stirred at room temperature for 4 h. When LCMS indicated the completion of the reaction, the reaction mixture was concentrated, dissolved in DMF, and purified by reversed-phase column chromatography (ACN/TFA 0.05% $H_2O$) to give a product **14-1** (colorless oil, 4.0 g, 63% yield).

Synthesis of compound **14-2**

To a solution of **14-1** (3.0 g, 11.39 mmol, 1.0 eq) in THF (30 mL) was added borane dimethyl sulfide (2 M, 17 mL, 3.0 eq) in portions at room temperature (25 °C) under nitrogen atmosphere, and the reaction mixture was stirred at reflux for 6 h. When LCMS indicated the completion of the reaction, to the reaction mixture was added methanol dropwise. The mixture was stirred for 30 min until no bubbles were produced, and incubated at reflux for another 1 h until no bubbles were produced. The reaction mixture was concentrated to give **14-2** (white solid, 2.8 g, 99% yield).

Synthesis of compound **14-3**

To a solution of **14-2** (2.3 g, 9.22 mmol, 1.0 eq) in DCM (30 mL) was added Dess-Martin oxidant (4.3 g, 10.14

mmol, 1.1 eq) at room temperature (25 °C) under nitrogen atmosphere. The mixture was stirred at room temperature for 4 h. When LCMS indicated the completion of the reaction, the reaction mixture was filtered, and the filtrate was concentrated by rotavap and purified on a silica gel column to give a product **14-3** (colorless oil, 2.1 g, 92% yield).

Synthesis of compound **14-4**

To a solution of **14-3** (1.8 g, 7.28 mmol, 1.0 eq) in DCM (20 mL) were added a catalytic amount of acetic acid (0.2 mL), methylamine hydrochloride (984 mg, 14.56 mmol, 2.0 eq), and sodium cyanoborohydride (686 mg, 10.92 mmol, 1.5 eq) at room temperature (25 °C) under nitrogen atmosphere. The mixture was stirred at room temperature for 16 h. When LCMS indicated the completion of the reaction, the reaction mixture was directly purified on a silica gel column to give compound **14-4** (white solid, 1.5 g, 79% yield).

Synthesis of compound **14-5**

To a solution of **14-4** (1.5 g, 5.72 mmol, 1.0 eq) in DCM (20 mL) were added triethylamine (1.73 g, 17.16 mmol, 3.0 eq) and $Boc_2O$ (2.5 g, 11.44 mmol, 2.0 eq) at room temperature under nitrogen atmosphere, and the reaction mixture was stirred at room temperature for 4 h. When LCMS indicated the completion of the reaction, the reaction mixture was concentrated by rotavap and purified on a silica gel column to give a product **14-4** (colorless oil, 1.8 g, 87% yield).

Synthesis of compound **14-6**

To a solution of **14-5** (1.8 g, 4.96 mmol, 1.0 eq) in methanol (30 mL) was added wet palladium on carbon (10%, 500 mg) at room temperature, and the mixture was stirred at 30 °C for 2 h under hydrogen atmosphere. When LCMS indicated the completion of the reaction, the reaction mixture was concentrated by rotavap to give a product **14-6** (colorless oil, 1.1 g, 97% yield).

Synthesis of compound **14-7**

To a solution of FMoc-Val-Cit-PAB-PNP (1.0 g, 1.3 mmol, 1.0 eq) in DMF (40 mL) were added **14-6** (300 mg, 1.3 mmol, 1.0 eq) and DIPEA (168 mg, 1.3 mmol, 1.0 eq) at room temperature under nitrogen atmosphere. The mixture was stirred at 15 °C for 16 h. When LCMS indicated the completion of the reaction, the reaction mixture was purified on a C18 column (ACN/TFA 0.05% $H_2O$) to give a product **14-7** (white solid, 500 mg, 45% yield).

Synthesis of compound **14-8**

To a solution of **14-7** (120 mg, 0.14 mmol, 1.0 eq) in DCM (15 mL) was added TFA (5 mL) at room temperature under nitrogen atmosphere. The mixture was stirred at room temperature for 2 h. When LCMS indicated the completion of the reaction, the reaction mixture was concentrated to give a product **14-8** (brown oil, 100 mg, 100% yield).

Synthesis of compound **14-9**

To a solution of **14-8** (100 mg, 0.132 mmol, 1.0 eq) in DMF (10 mL) were added DIPEA (34 mg, 0.264 mmol, 2.0 eq) and **7-SM1** (135 mg, 0.132 mmol, 1.0 eq) at room temperature under nitrogen atmosphere. The mixture was stirred at 15 °C for 16 h. When LCMS indicated the completion of the reaction, the reaction mixture was purified on a C18 column (ACN/TFA 0.05% $H_2O$) to give a product **14-9** (white solid, 150 mg). To a solution of the product in DMF (6 mL) was added diethylamine (133 mg, 1.82 mmol, 20.0 eq). The mixture was stirred at room temperature for 1 h. When LCMS indicated the completion of the reaction, the reaction mixture was directly purified by reversed-phase column chromatography to give a product **14-9** (white solid, 85 mg, 50% yield).

Synthesis of compound **14-10**

To a solution of **PEG8** (282 mg, 0.424 mmol, 1.0 eq) in DMSO (10 mL) were added DIPEA (219 mg, 1.69 mmol, 4.0 eq) and TSTU (128 mg, 0.424 mmol, 1.0 eq) sequentially at 0 °C under nitrogen atmosphere. The mixture was stirred for 30 min at 0 °C and for 1 h at room temperature. To the reaction mixture was added a solution of **14-9** (128 mg, 0.424 mmol, 1.0 eq) in DMSO (10 mL), and the mixture was stirred at room temperature for 2 h. When LCMS indicated the completion of the reaction, diethylamine (604 mg, 8.255 mmol, 20.0 eq) was added, and the mixture was stirred at room temperature for 1 h. When LCMS indicated the completion of the reaction, the reaction mixture was purified on a C18 column (ACN/TFA 0.05% $H_2O$) to give a product **14-10** (white solid, 800 mg, ~100% yield).

Synthesis of compound **14**

To a solution of **101B13** (686 mg, 0.29 mmol, 1.0 eq, based on the polymer structural unit), **14-10** (800 mg, 0.435 mmol, 1.5 eq), and PyBOP (227 mg, 0.435 mmol, 1.5 eq) in DMF (8 mL) was added DIPEA (150 mg, 1.16 mmol, 4.0 eq) at room temperature under nitrogen atmosphere. The mixture was stirred at room temperature for 16 h. The reaction mixture was concentrated by rotavap using an oil pump, and methyl *tert*-butyl ether was added. The mixture was stirred for 20 min, and the supernatant was discarded. The oily substance at the bottom of the flask was dissolved in methanol, and an equal volume of water was then added. The mixture was filtered, purified by ultrafiltration (30K MW), and lyophilized to give **14** (white solid, 1.0 g, 83% yield, about 24.3% PTX content).

Example 16: Synthesis of compound 15

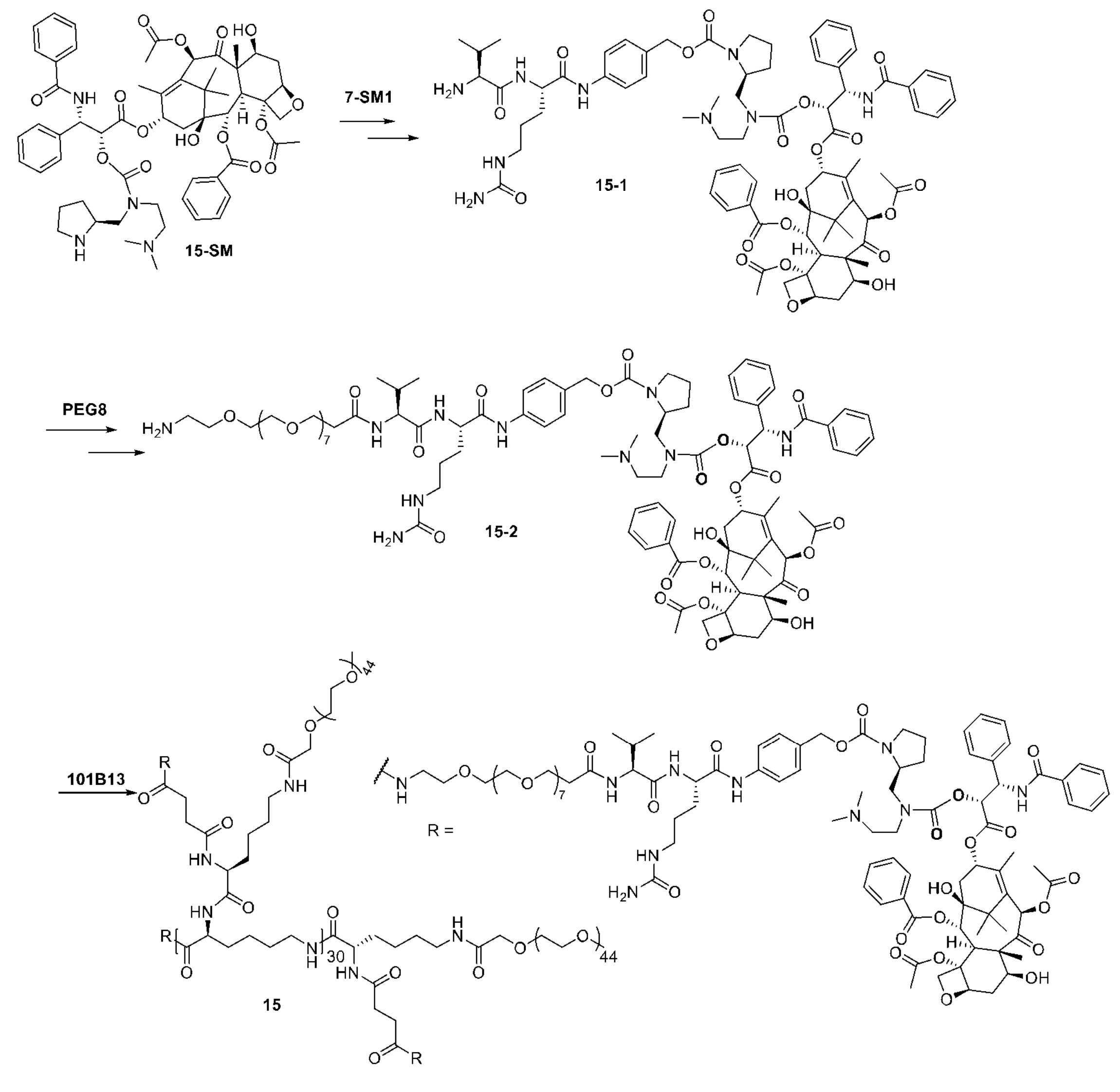


Synthesis of compound **15-1**

To a solution of **15-SM** (prepared according to the synthetic method in ChemMedChem 2022, e202200279.; 190 mg, 0.18 mmol, 1.0 eq) in DMF (10 mL) were added **7-SM1** (260 mg, 1.17 mmol, 1.2 eq) and DIPEA (0.26 g, 2 mmol, 2.0 eq) at room temperature under nitrogen atmosphere. The reaction mixture was stirred overnight. The reaction mixture was concentrated and purified by Pre-HPLC to give a white solid (120 mg). To a solution of the white solid in DMF (5 mL) was added diethylamine (1 mL). The reaction mixture was stirred overnight. The reaction mixture was concentrated, and purified by Pre-HPLC to give a white solid (100 mg, 96% yield).

MS (ESI), m/z, 1456.0 [M+H]+.

Synthesis of compound **15-2**

To a solution of **PEG8** (40 mg, 0.061 mmol, 1.3 eq) in DCM (20 mL) was added TSTU (40 mg, 0.13 mmol) at room temperature under nitrogen atmosphere. The mixture was stirred for 15 min and a solution of **15-1** (90 mg, 0.061 mmol) in dichloromethane was added. The mixture was stirred for 2 h. When LCMS monitoring indicated the completion of the reaction, the reaction mixture was concentrated and purified by Pre-HPLC to give a white solid (90 mg, 70% yield).

MS (ESI), m/z, 1051.2 [M/2+H]$^+$.

Synthesis of compound **15**

To a solution of **101B13** (70 mg, 1.0 eq), **15-2** (86 mg, 0.05 mmol, 48.0 eq), and PyBOP (40 mg, 0.08 mmol, 80.0 eq) in DMF (2 mL) was added DIPEA (16 mg, 0.12 mmol, 128.0 eq) at room temperature under nitrogen atmosphere. The mixture was stirred at room temperature for 16 h. The reaction mixture was purified by ultrafiltration (30K MW) and lyophilized to give **15** (white solid, 100 mg, 78% yield, about 19.3% PTX content).

Example 17: Synthesis of compound 16a

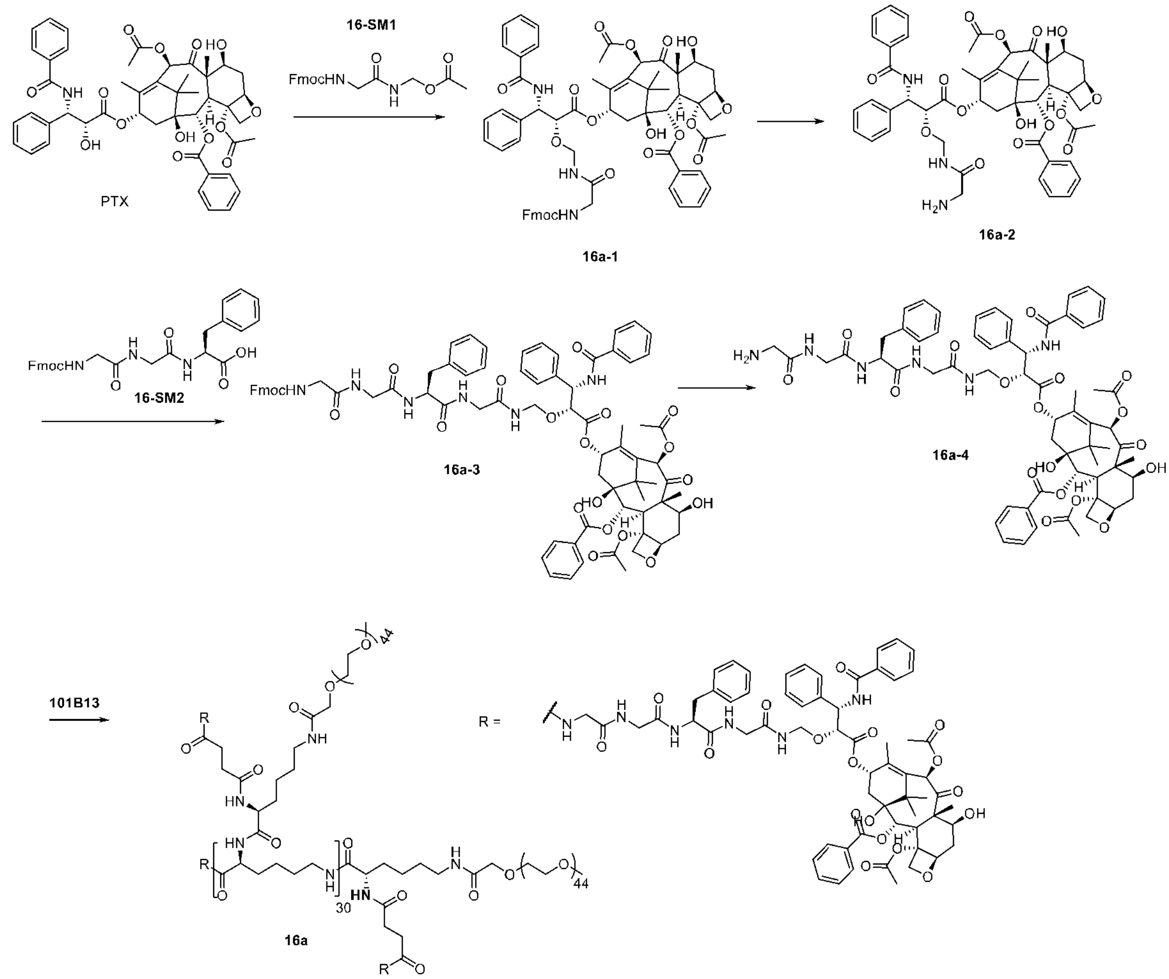


Synthesis of compound **16a-1**

To a solution of paclitaxel (2.0 g, 2.34 mmol, 1.0 eq) and **16-SM1** (2.6 g, 7.03 mmol, 3.0 eq; prepared according to the synthetic method in Tetrahedron, ***2018***, 1951 - 1956) in dry THF (50 mL) was added 1.0 M lithium *tert*-butoxide (3.6 mL,

11.7 mmol, 5.0 eq) dropwise in a dry ice/ethanol bath (-60 °C) under nitrogen atmosphere (2.0 eq was added first, where LCMS indicated the half consumption of the materials; the remaining was then added until 5.0 eq, where LCMS indicated the substantial depletion of the materials), and the mixture was stirred for 1 h. The reaction was quenched with ammonium chloride and water, and the mixture was extracted with ethyl acetate (100 mL × 3). The organic phases were combined, washed with brine, dried over anhydrous sodium sulfate, filtered, concentrated, and purified by column chromatography ($SiO_2$, 0-100% EtOAc in PE/$SiO_2$ to elute most of the product, followed by 0-20% MeOH in $CH_2Cl_2$) to give a product **16a-1** (2.3 g, white solid, 96% yield).

MS (ESI), m/z, 1162 $[M+1]^+$.

$^1H$ NMR (400 MHz, Chloroform-*d*) δ 8.25 - 8.04 (m, 2H), 7.78 (dd, *J* = 7.5, 4.3 Hz, 4H), 7.65 - 7.45 (m, 6H), 7.45 - 7.28 (m, 8H), 7.11 (d, *J* = 9.0 Hz, 1H), 6.71 (s, 1H), 6.26 (s, 1H), 6.20 (t, *J* = 9.0 Hz, 1H), 5.75 (dd, *J* = 9.0, 3.7 Hz, 1H), 5.66 (d, *J* = 7.1 Hz, 1H), 5.28 (d, *J* = 11.3 Hz, 1H), 4.92 (d, *J* = 9.6 Hz, 2H), 4.77 (d, J= 3.8 Hz, 1H), 4.63 (dd, *J* = 11.2, 5.9 Hz, 1H), 4.47 (d, *J* = 6.5 Hz, 2H), 4.40 (dd, *J* = 10.9, 6.6 Hz, 1H), 4.24 - 4.16 (m, 2H), 4.12 (q, *J* = 7.1 Hz, 2H), 3.75 (dd, *J* = 13.1, 6.5 Hz, 2H), 3.64 (d, *J* = 15.3 Hz, 1H), 2.50 (dt, *J* = 15.7, 8.5 Hz, 1H), 2.39 (s, 3H), 2.21 (s, 4H), 2.04 (s, 3H), 1.92 - 1.84 (m, 4H), 1.67 (s, 3H), 1.32 - 1.21 (m, 6H), 1.12 (s, 3H).

Synthesis of compound **16a-2**

To a solution of **16a-1** (2.3 g, 1.98 mmol, 1.0 eq) in dry $CH_2Cl_2$ (10 mL) was added diethylamine (4 mL) at room temperature (10 °C) under nitrogen atmosphere. The mixture was then heated to 25 °C and stirred for 12 h. When LCMS indicated the completion of the reaction, the mixture was concentrated, and the residue was dissolved in dichloromethane. To MTBE was added the solution dropwise, and the mixture was filtered to give **16a-2** (1.7 g, white solid, 92% yield).

MS (ESI), m/z, 940 $[M+1]^+$.

Synthesis of compound **16a-3**

To a solution of **16a-2** (1.5 g, 1.6 mmol, 1.0 eq) and Fmoc-Gly-Gly-Phe-OH (960 mg, 1.91 mmol, 1.2 eq) in dry DMF (10 mL) were added EDCI (460 mg, 1.39 mmol, 1.5 eq), HOBt (323 mg, 2.39 mmol, 1.5 eq), and DIPEA (411 mg, 3.19 mmol, 2.0 eq) in an ice-water bath (5 °C) under nitrogen atmosphere, and the mixture was stirred for 1 h. LCMS indicated that the starting materials were not depleted. The mixture was warmed to room temperature (10 °C) and stirred for 1 h. LCMS indicated that the starting materials were still not depleted (the reaction did not proceed), but the reactions were more complicated than before. The starting materials and the condensing agent were added, but no effect was observed. The reaction mixture was poured into water. The mixture was filtered, and the solid was dissolved in dichloromethane. The phases were separated, dried, concentrated, and purified by column chromatography ($SiO_2$, 0-10% MeOH in $CH_2Cl_2$) to give a product **16a-3** (1.2 g, foamy solid, 70% yield). The product was directly used in the next step.

MS (ESI), m/z, 1423 $[M+1]^+$.

Synthesis of compound **16a-4**

To a solution of **16a-3** (1.2 g, 0.84 mmol, 1.0 eq) in dry $CH_2Cl_2$ (10 mL) was added diethylamine (3-4 mL) at room temperature (10 °C) under nitrogen atmosphere. The mixture was then heated to 25 °C and stirred for 3 h. When LCMS indicated the completion of the reaction, the mixture was concentrated, and purified by Pre-HPLC ($CH_3CN/H_2O$, 0.05% TFA) to give a product **16a-4** (900 mg, white solid, 82% yield, 93% purity).

MS (ESI), m/z, 1201 $[M+1]^+$.

Synthesis of compound **16a**

To a solution of **101B13** (250 mg, 0.003 mmol, 1.0 eq) in dry DMF (5 mL) were added a solution of **16a-4** (196 mg, 0.16 mmol, 48.0 eq) in dry DMF (2 mL, ultrasonicated for dissolution), PyBOP (142 mg, 0.27 mmol, 80.0 eq), and DIPEA (56 mg, 0.44 mmol, 128.0 eq) at room temperature (25 °C) under nitrogen atmosphere. The mixture was then heated to 28 °C, stirred for 12 h, concentrated, filtered from methanol to methanol/water (1/1, 100 mL), purified by ultrafiltration (30K MW), and lyophilized to give a product **16a** (390 mg, oily solid, 96% yield, 100% purity, about 24.0% PTX content).

Example 18: Synthesis of compound 16b

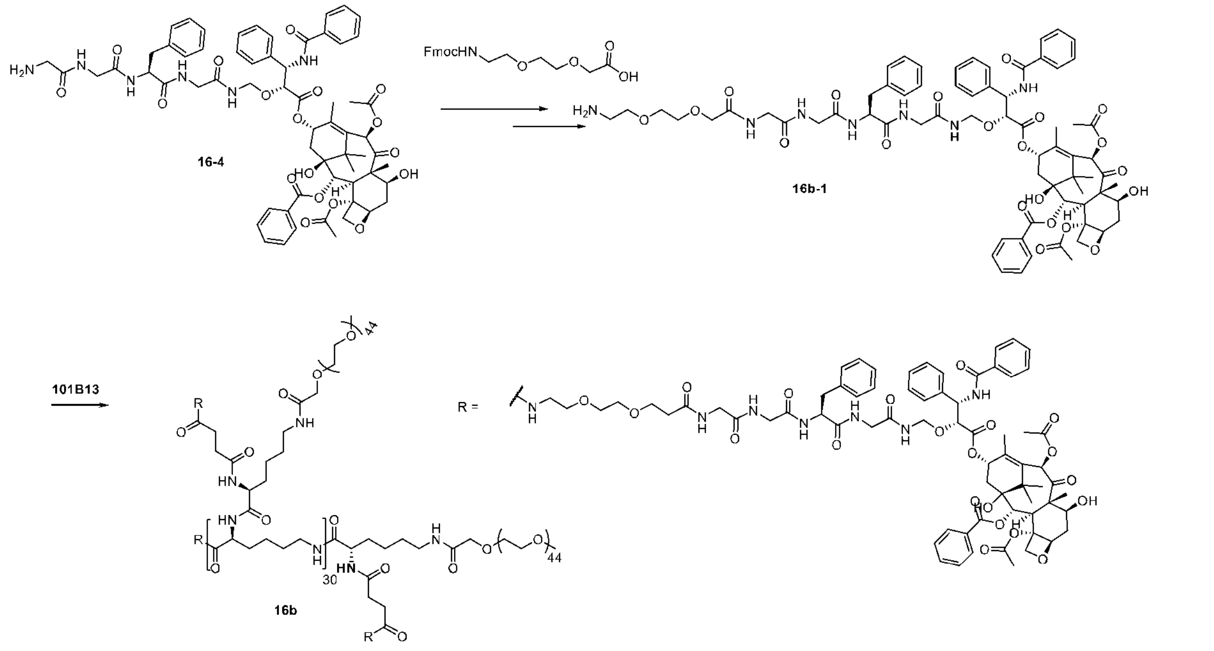


16-4 16b-1 16b

Synthesis of compound **16b-1**

To a solution of [2-[2-(Fmoc-amino)ethoxy]ethoxy]acetic acid (Fmoc-AEEA-OH; 115 mg, 0.30 mmol, 1.2 eq) in dry $CH_2Cl_2$ (5 mL) were added TSTU (113 mg, 0.37 mmol, 1.5 eq) and DIPEA (64 mg, 0.50 mmol, 2.0 eq) in an ice-water bath (5 °C) under nitrogen atmosphere, and the mixture was stirred for 5 min (LCMS monitoring indicated the production of an active ester). A solution of **16a-4** (300 mg, 0.25 mmol, 1.0 eq) in dichloromethane was added, and the mixture was stirred for 5 min. Methanol was added to quench the reaction, and the mixture was concentrated. To a solution of the residue in dry dichloromethane (5 mL) was added diethylamine (5 mL). The mixture was stirred for 1-2 h at room temperature and directly concentrated. The crude product was purified by Pre-HPLC ($CH_3CN/H_2O$, 0.05% TFA) to give a product **16b-1** (300 mg, colorless oily substance), which was directly used in the next reaction.

MS (ESI), m/z, 1346.5 $[M+1]^+$.

Synthesis of compound **16b**

To a solution of **101B13** (250 mg, 0.003 mmol, 1.0 eq) in dry DMF (5 mL) were added a solution of **16b-1** (206 mg, 0.15 mmol, 45.0 eq) in dry DMF (2 mL, ultrasonicated for dissolution), PyBOP (142 mg, 0.27 mmol, 80.0 eq), and DIPEA (56 mg, 0.44 mmol, 128.0 eq) at room temperature (25 °C) under nitrogen atmosphere. The mixture was then heated to 28 °C, stirred for 12 h, concentrated, filtered from methanol to methanol/water (1/1, 100 mL), purified by ultrafiltration (30K MW), and lyophilized to give a product **16b** (310 mg, oily solid, 80% yield, 98.8% purity, about 22.3% PTX content) (**16b-1** was the standard reference for HPLC content assay).

Example 19: Synthesis of compound 16c

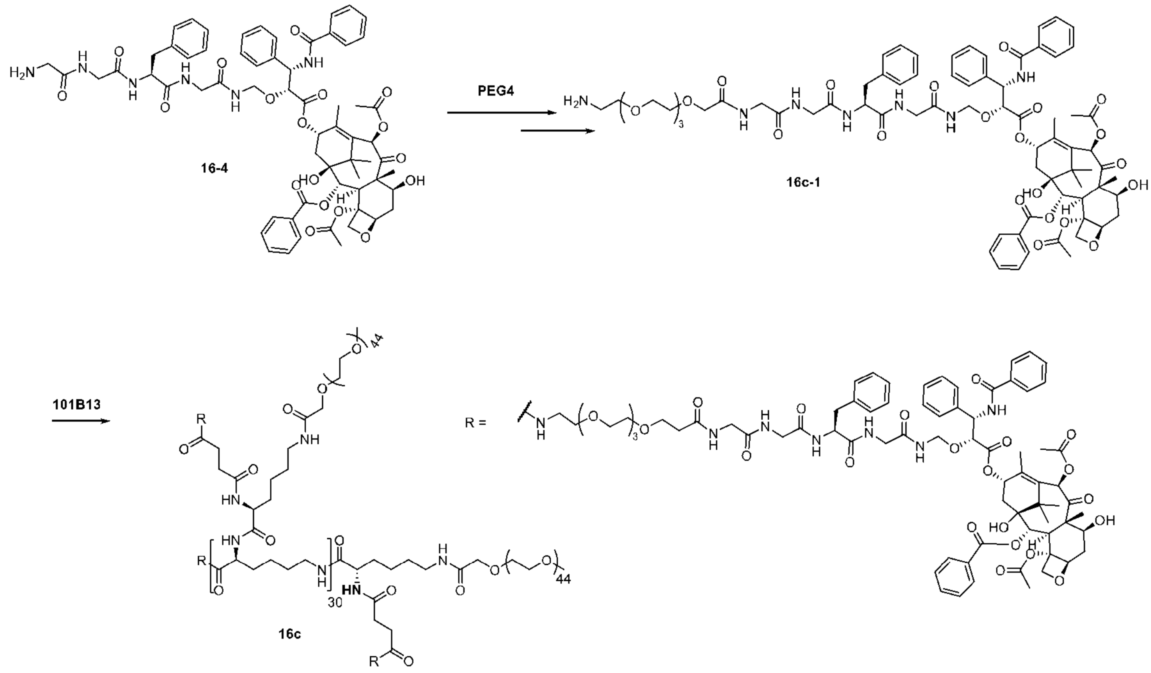


16-4 16c-1 16c

Synthesis of compound **16c-1**

To a solution of **PEG4** (146 mg, 0.30 mmol, 1.2 eq) in dry $CH_2Cl_2$ (5 mL) were added TSTU (113 mg, 0.37 mmol, 1.5 eq) and DIPEA (64 mg, 0.50 mmol, 2.0 eq) in an ice-water bath (5 °C) under nitrogen atmosphere, and the mixture was stirred for 5 min (LCMS monitoring indicated the production of an active ester). A solution of **16a-4** (300 mg, 0.25 mmol, 1.0 eq) in dichloromethane was added, and the mixture was stirred for 5 min. Methanol was added to quench the reaction, and the mixture was concentrated. To a solution of the residue in dry dichloromethane (5 mL) was added diethylamine (5 mL). The mixture was stirred for 1-2 h at room temperature and directly concentrated. The crude product was purified by Pre-HPLC ($CH_3CN/H_2O$, 0.05% TFA) to give a product **16c-1** (350 mg, colorless oily substance), which was directly used in the next reaction.

MS (ESI), m/z, 1434.3 $[M+1]^+$.

Synthesis of compound **16c**

To a solution of **101B13** (250 mg, 0.003 mmol, 1.0 eq) in dry DMF (5 mL) were added a solution of **16c-1** (220 mg, 0.15 mmol, 45.0 eq) in dry DMF (2 mL, ultrasonicated for dissolution), PyBOP (142 mg, 0.27 mmol, 80.0 eq), and DIPEA (56 mg, 0.44 mmol, 128.0 eq) at room temperature (25 °C) under nitrogen atmosphere. The mixture was then heated to 28 °C, stirred for 12 h, concentrated, filtered from methanol to methanol/water (1/1, 100 mL), purified by ultrafiltration (30K MW), and lyophilized to give a product **16c** (320 mg, oily solid, 81% yield, 99% purity, about 21.0% PTX content).

Example 20: Synthesis of compound 16d

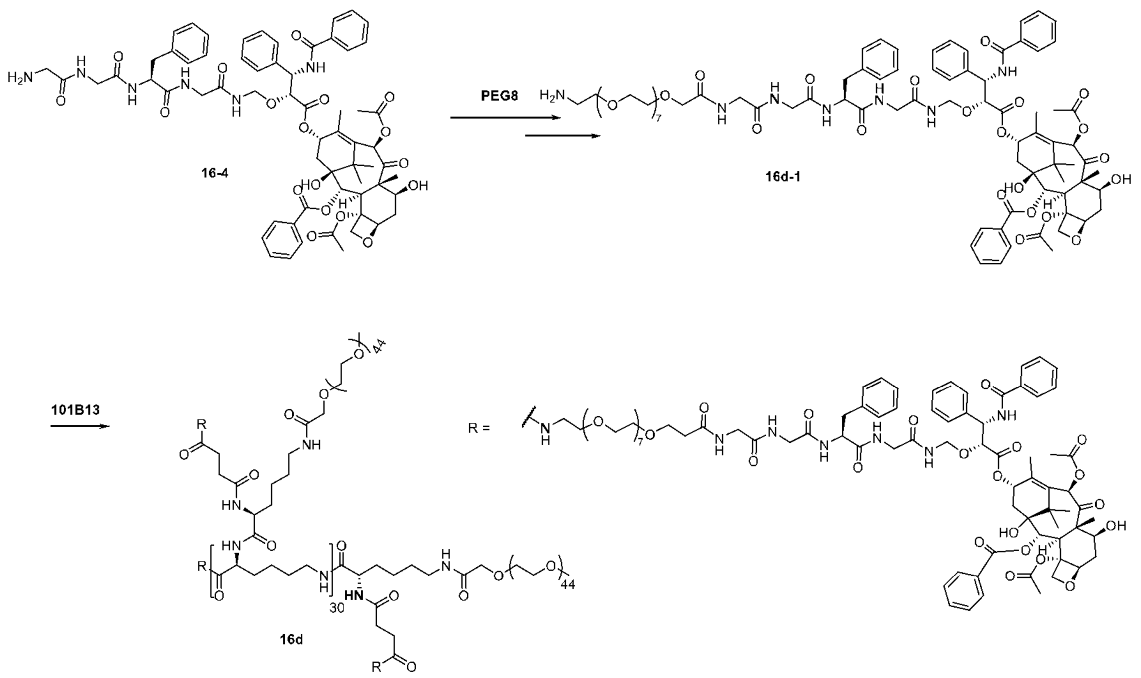


Synthesis of compound **16d-1**

To a solution of **PEG8** (260 mg, 0.39 mmol, 1.2 eq) in dry $CH_2Cl_2$ (5 mL) were added TSTU (177 mg, 0.59 mmol, 1.0 eq) and DIPEA (126 mg, 0.98 mmol, 3.0 eq) in an ice-water bath (5 °C) under nitrogen atmosphere, and the mixture was stirred for 5 min (LCMS monitoring indicated the production of an active ester). A solution of **16a-4** (430 mg, 0.33 mmol, 1.0 eq) in dichloromethane (2 mL) was added, and the mixture was stirred for 5 min. Methanol was added to quench the reaction, and the mixture was concentrated. To a solution of the residue in dry dichloromethane (5 mL) was added diethylamine (5 mL). The mixture was stirred for 3 h at room temperature and directly concentrated. The crude product was purified by Pre-HPLC ($CH_3CN/H_2O$, 0.05% TFA) to give a product **16d-1** (260 mg, colorless oily substance, 46% yield). The product was directly used in the next step.

MS (ESI), m/z, 1625 $[M+1]^+$.

Synthesis of compound **16d**

To a solution of **16d-1** (255 mg, 0.15 mmol, 45.0 eq) in dry DMF (5 mL) were added a solution of **101B13** (240 mg, 0.003 mmol, 1.0 eq) in dry DMF (2 mL, ultrasonicated for dissolution), PyBOP (136 mg, 0.26 mmol, 80.0 eq), and DIPEA (54 mg, 0.42 mmol, 128.0 eq) at room temperature (25 °C) under nitrogen atmosphere. The mixture was then heated to 28 °C, stirred for 12 h, concentrated, filtered from methanol to methanol/water (1/1, 100 mL), purified by ultrafiltration (30K MW), and lyophilized to give a product **16d** (390 mg, oily solid, 96% yield, 100% purity, about 17.4% PTX content).

Example 21: Synthesis of compound 16e

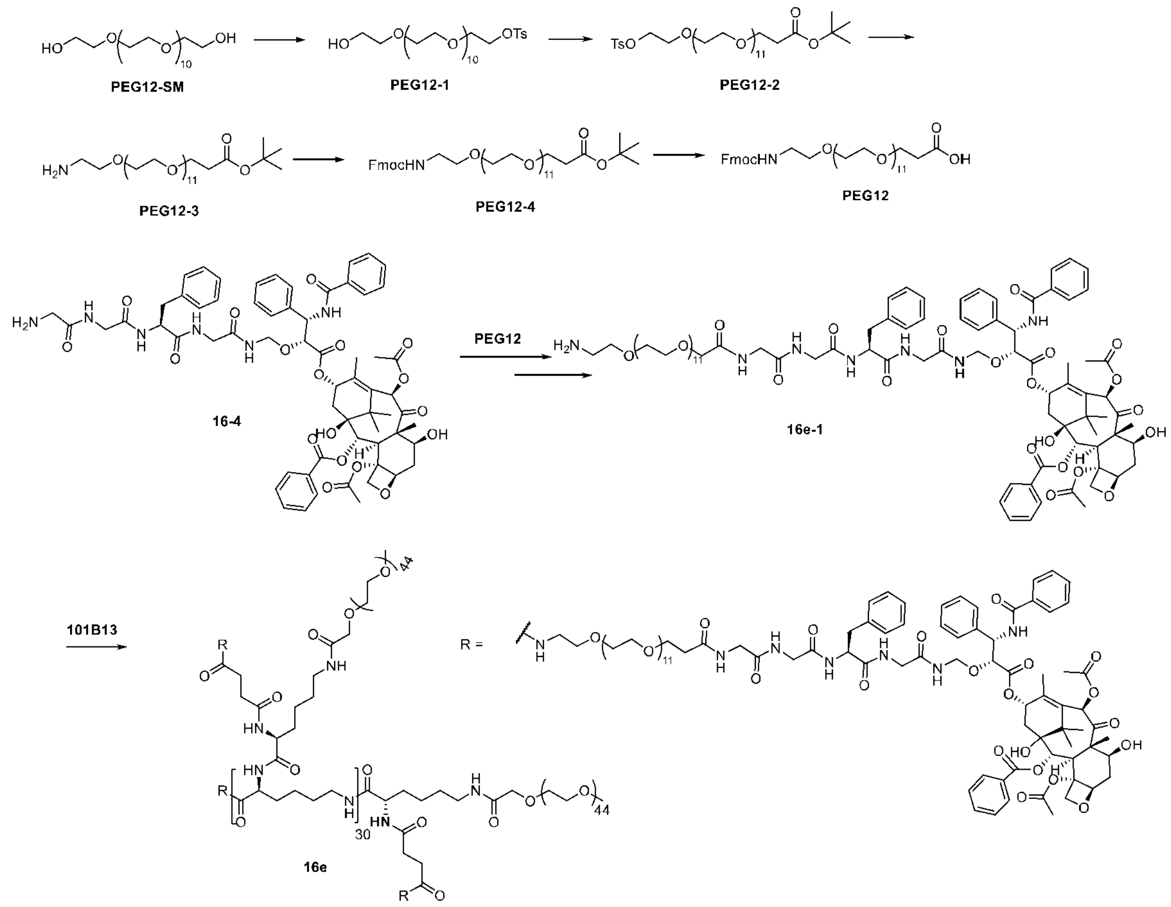

PEG12-SM PEG12-1 PEG12-2 PEG12-3 PEG12-4 PEG12 16-4 16e-1 16e

Synthesis of compound **PEG12-1**

Dodecaethylene glycol (4.5 g, 8.24 mmol, 1.0 eq), TEBA (0.18 g, 0.824 mmol, 0.1 eq), and 20% sodium hydroxide solution (45 mL) was dissolved in toluene (45 mL) at room temperature. TsCl in toluene (1.57 g, 8.24 mmol, 1.0 eq) was added dropwise at 0 °C under argon atmosphere. The mixture was stirred at room temperature for 16 h. When LCMS indicated the completion of the reaction, water was added to terminate the reaction. The phases were separated and the aqueous phase was extracted with ethyl acetate, dried over sodium sulfate, concentrated by rotavap *in vacuo,* and separated by column chromatography (dichloromethane:methanol = 10:1) to give compound **PEG12-1** (colorless oil, 5 g, 86.7% yield).

MS (ESI), m/z, 701.2 $[M+H]^+$.

Synthesis of compound **PEG12-2**

To a solution of compound **PEG12-1** (5 g, 7.13 mmol, 1.0 eq) in dichloromethane (100 mL) was added sodium metal (100 mg) at room temperature. The mixture was stirred at 30 °C for 3 h under nitrogen atmosphere. *tert*-Butyl 1-buten-4-oate (2.03 g, 14.3 mmol, 2.0 eq) was added. The mixture was stirred at room temperature for 16 h. When LCMS indicated the completion of the reaction, the mixture was concentrated *in vacuo,* and the product was directly used in the next reaction.

MS (ESI), m/z, 773.2 $[M-56]^+$.

Synthesis of compound **PEG12-3**

Compound **PEG12-2** (crude) was dissolved in acetonitrile/aqueous ammonia (50 mL/50 mL) at room temperature. The reaction mixture was stirred at 40 °C for 5 h under nitrogen atmosphere. When LCMS indicated the completion of the reaction, the mixture was concentrated *in vacuo,* and the product was directly used in the next reaction.

MS (ESI), m/z, 674.2 $[M+H]^+$.

Synthesis of compound **PEG12-4**

To a solution of compound **PEG12-3** (crude) in dichloromethane (100 mL) were added FmocOSU (2.8 g, 8.24 mmol, 1.0 eq) and DIPEA (2.12 g, 16.5 mmol, 2.0 eq) at room temperature, and the mixture was stirred at 30 °C for 16 h under nitrogen atmosphere. When LCMS indicated the completion of the reaction, the mixture was concentrated *in vacuo,* and purified by normal-phase and reversed-phase column chromatography to give compound **PEG12-4** (colorless oil, 3 g, impure, directly used in the next reaction).

MS (ESI), m/z, 896.2 $[M+H]^+$.

Synthesis of compound **PEG12**

To a solution of compound **PEG12** (crude) in dichloromethane (10 mL) was added trifluoroacetic acid (5 mL) at room temperature under nitrogen atmosphere. The mixture was stirred for 16 h. When LCMS indicated the completion of the reaction, the mixture was directly concentrated *in vacuo* to give compound **PEG12** (colorless oil, 1.53 g, 53.5% yield).

MS (ESI), m/z, 840.2 $[M+H]^+$.

Synthesis of compound **16e-1**

To a solution of **PEG12** (126 mg, 0.15 mmol, 1.2 eq) in dry $CH_2Cl_2$ (5 mL) were added TSTU (56 mg, 0.19 mmol, 1.5 eq) and DIPEA (32 mg, 0.25 mmol, 2.0 eq) in an ice-water bath (5 °C) under nitrogen atmosphere, and the mixture was stirred for 5 min (LCMS monitoring indicated the production of an active ester). A solution of **16a-4** (150 mg, 0.12 mmol, 1.0 eq) in dichloromethane (2 mL) was added, and the mixture was stirred for 5 min. Methanol was added to quench the reaction, and the mixture was concentrated. To a solution of the residue in dry dichloromethane (3 mL) was added diethylamine (5 mL). The mixture was stirred for 3 h at room temperature and directly concentrated. The crude product was purified by Pre-HPLC ($CH_3CN/H_2O$, 0.05% TFA) to give a product **16e-1** (200 mg, colorless oily substance, 78% yield). The product was directly used in the next step.

MS (ESI), m/z, 1786.3 $[M+1]^+$.

Synthesis of compound **16e**

To a solution of **16e-1** (100 mg, 0.06 mmol, 48.0 eq) in dry DMF (2 mL) were added a solution of **101B13** (85 mg, 0.001 mmol, 1.0 eq) in dry DMF (0.5 mL, ultrasonicated for dissolution), PyBOP (48 mg, 0.09 mmol, 80.0 eq), and DIPEA (19 mg, 0.15 mmol, 128.0 eq) at room temperature (25 °C) under nitrogen atmosphere. The mixture was then heated to 28 °C, stirred for 12 h, concentrated, filtered from methanol to methanol/water (1/1, 50 mL), purified by ultrafiltration (30K MW), and lyophilized to give a product **16e** (120 mg, oily solid, 80% yield, 99% purity, about 19.0% PTX content).

Example 22: Synthesis of compound 16f

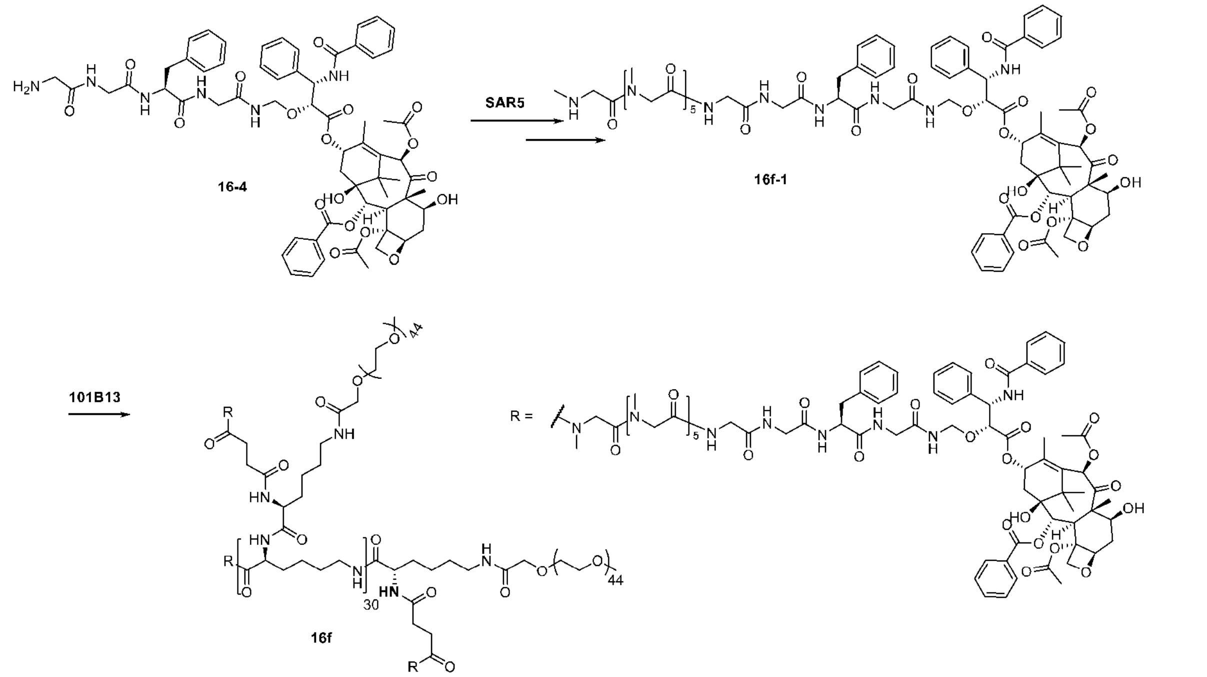


16-4, 16f-1, 16f

Synthesis of compound **16f-1**

To a solution of **SAR5** (199 mg, 0.30 mmol, 1.2 eq) in dry $CH_2Cl_2$ (5 mL) were added TSTU (113 mg, 0.37 mmol, 1.5 eq) and DIPEA (65 mg, 0.50 mmol, 2.0 eq) in an ice-water bath (5 °C) under nitrogen atmosphere, and the mixture was stirred for 5 min (LCMS monitoring indicated the production of an active ester). A solution of **16a-4** (300 mg, 0.25 mmol, 1.0 eq) in dichloromethane (2 mL) was added, and the mixture was stirred for 5 min. Methanol was added to quench the reaction, and the mixture was concentrated. To a solution of the residue in dry dichloromethane (3 mL) was added diethylamine (5 mL). The mixture was stirred for 3 h at room temperature and directly concentrated. The crude product was purified by Pre-HPLC ($CH_3CN/H_2O$, 0.05% TFA) to give a product **16f-1** (380 mg, colorless oily substance, 81% yield). The product was directly used in the next step.

MS (ESI), m/z, 1627.1 $[M+1]^+$.

Synthesis of compound **16f**

To a solution of **101B13** (250 mg, 0.003 mmol, 1.0 eq) in dry DMF (5 mL) were added a solution of **16f-1** (250 mg, 0.15 mmol, 45.0 eq) in dry DMF (2 mL, ultrasonicated for dissolution), PyBOP (142 mg, 0.27 mmol, 80.0 eq), and DIPEA (56 mg, 0.44 mmol, 128.0 eq) at room temperature (25 °C) under nitrogen atmosphere. The mixture was then heated to 28 °C, stirred for 12 h, concentrated, filtered from methanol to methanol/water (1/1, 100 mL), purified by ultrafiltration (30K MW), and lyophilized to give a product **16f** (315 mg, oily solid, 75% yield, 98.9% purity, about 20.8% PTX content).

Example 23: Synthesis of compound 16g

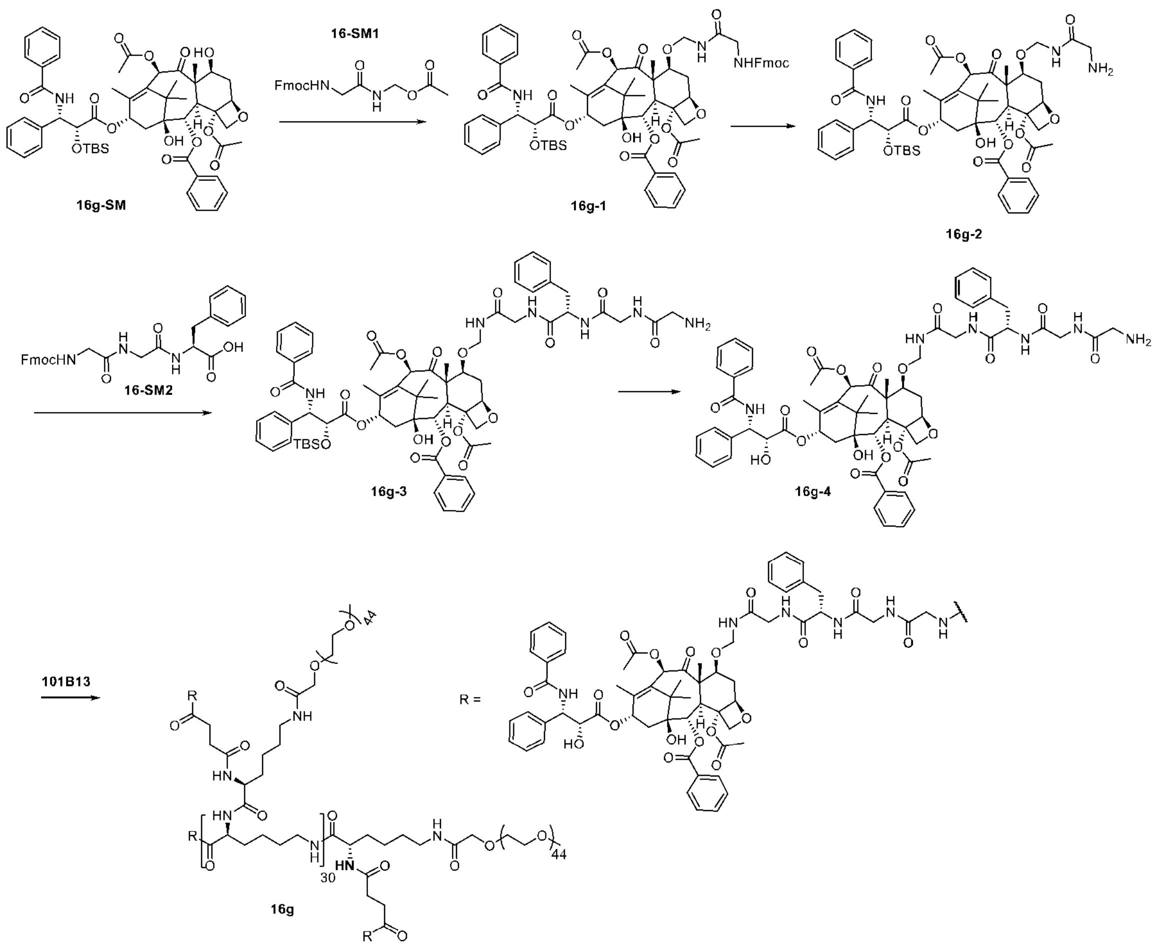


Synthesis of compound **16g-1**

To a solution of **16g-SM** (2.0 g, 2.07 mmol, 1.0 eq, prepared according to the synthetic method in J. Am. Chem. Soc. 2007, 129, 37, 11653-11661) and **16-SM1** (2.6 g, 7.03 mmol, 3.0 eq) in dry THF (50 mL) was added dropwise 1.0 M lithium *tert*-butoxide (3.6 mL, 11.7 mmol, 5.0 eq) in a dry ice/ethanol bath (-60 °C) under nitrogen atmosphere, and the mixture was warmed to -60 °C and stirred for 1 h. The reaction was quenched with ammonium chloride and water, and the mixture was extracted with ethyl acetate (100 mL × 3). The organic phases were combined, washed with brine, dried over anhydrous sodium sulfate, filtered, concentrated, and purified by column chromatography ($SiO_2$, 0-100% EtOAc in PE/$SiO_2$ to elute most of the product, followed by 0-20% MeOH in $CH_2Cl_2$) to give a product **16g-1** (1.5 g, white solid, 52% yield).

MS (ESI), m/z, 1276.5 $[M+1]^+$.

Synthesis of compound **16g-2**

To a solution of **16g-1** (1.0 g, 0.78 mmol, 1.0 eq) in dry $CH_2Cl_2$ (10 mL) was added diethylamine (4 mL) at room temperature (10 °C) under nitrogen atmosphere. The mixture was then heated to 25 °C and stirred for 12 h. When LCMS indicated the completion of the reaction, the mixture was concentrated, and the residue was dissolved in dichloromethane. The solution was added dropwise to MTBE, and the mixture was filtered to give **16g-2** (0.7 g, white solid, 85% yield).

MS (ESI), m/z, 1054.2 $[M+1]^+$.

Synthesis of compound **16g-3**

To a solution of **16g-2** (500 mg, 0.47 mmol, 1.0 eq) and Fmoc-Gly-Gly-Phe-OH (285 mg, 0.57 mmol, 1.2 eq) in dry DMF (5 mL) were added EDCI (136 mg, 0.71 mmol, 1.5 eq), HOBt (96 mg, 0.71 mmol, 1.5 eq), and DIPEA (122 mg, 0.95 mmol, 2.0 eq) in an ice-water bath (5 °C) under nitrogen atmosphere, and the mixture was warmed to return to room temperature (20 °C) and stirred for 1 h. When LCMS indicated the completion of the reaction, the mixture was concentrated. To a solution of the residue in dichloromethane (5 mL) was added diethylamine (5 mL). The reaction

mixture was stirred for 3 h, concentrated directly, and purified by column chromatography ($SiO_2$, 0-10% MeOH in $CH_2Cl_2$) to give a product **16g-3** (0.37 g, foamy solid, 60% yield).

MS (ESI), m/z, 1315.6 $[M+1]^+$.

Synthesis of compound **16g-4**

To a solution of **16g-3** (300 mg, 0.22 mmol, 1.0 eq) in dry THF (5 mL) was added a pyridine hydrogen fluoride solution (1 mL) in an ice-water bath (5 °C) under nitrogen atmosphere, and the mixture was warmed to return to room temperature (20 °C) and stirred for 1 h. When LCMS indicated the completion of the reaction, water was added to quench the reaction, and the mixture was extracted with dichloromethane. The organic phase was concentrated, and purified by reversed-phase column chromatography to give a product **16g-4** (0.15 g, 55% yield).

MS (ESI), m/z, 1201.1 $[M+1]^+$.

Synthesis of compound **16g**

To a solution of **101B13** (150 mg, 0.002 mmol, 1.0 eq) in dry DMF (3 mL) were added a solution of **16g-4** (110 mg, 0.09 mmol, 45.0 eq) in dry DMF (0.5 mL, ultrasonicated for dissolution), PyBOP (85 mg, 0.16 mmol, 80.0 eq), and DIPEA (34 mg, 0.26 mmol, 128.0 eq) at room temperature (25 °C) under nitrogen atmosphere. The mixture was then heated to 28 °C, stirred for 12 h, concentrated, filtered from methanol to methanol/water (1/1, 100 mL), purified by ultrafiltration (30K MW), and lyophilized to give a product **16g** (150 mg, white solid, 67% yield, 99% purity, about 22.5% PTX content).

Example 24: Synthesis of compound 16h

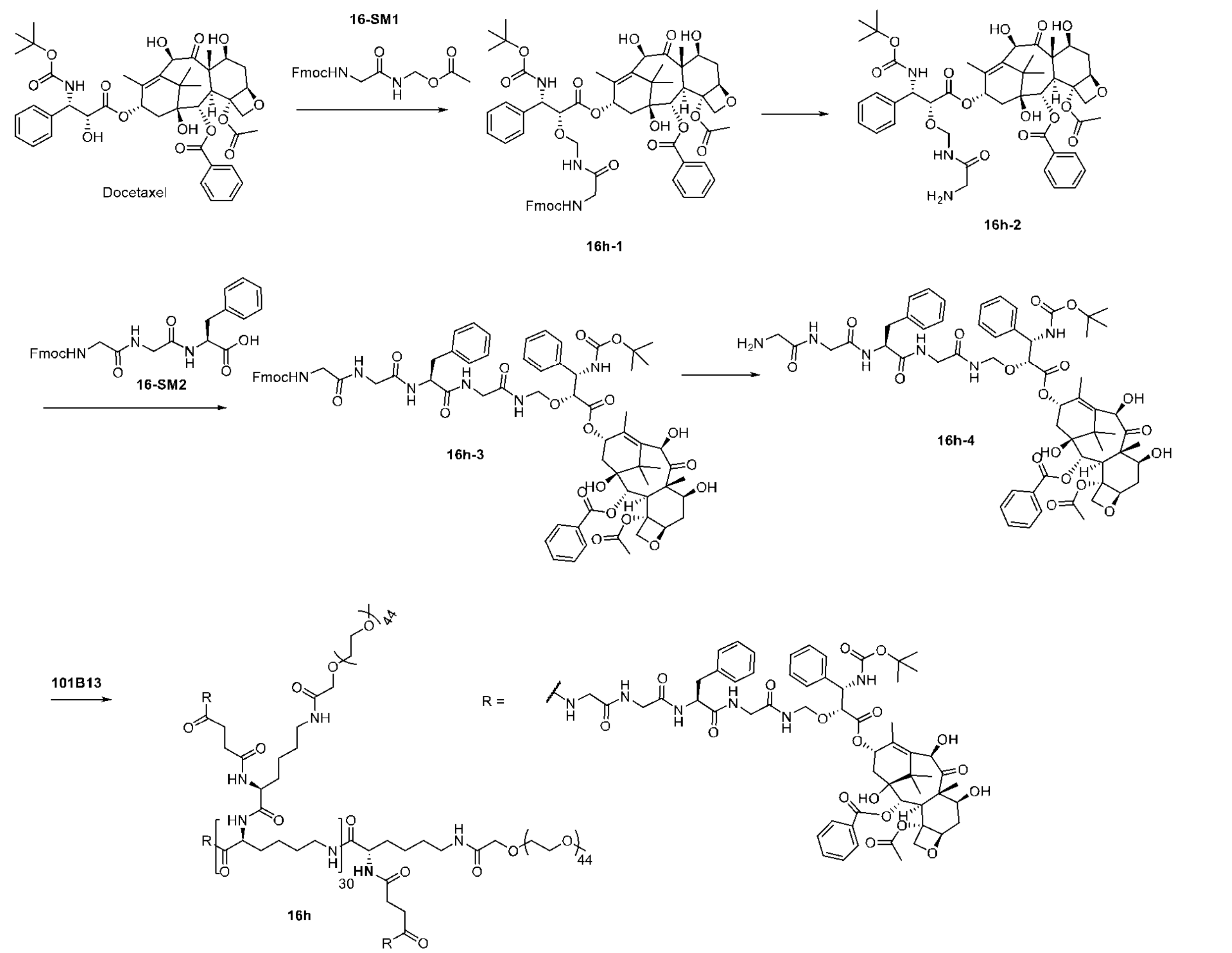


Docetaxel
16h-1
16h-2
16h-3
16h-4
16h

Synthesis of compounds **16h-1, 16h-2, 16h-3, and 16h-4**

The synthesis was conducted according to the synthetic method in Example **17.**

Synthesis of compound **16h**

To a solution of **101B13** (250 mg, 0.003 mmol, 1.0 eq) in dry DMF (5 mL) were added a solution of **16h-4** (177 mg, 0.15 mmol, 45.0 eq) in dry DMF (2 mL, ultrasonicated for dissolution), PyBOP (142 mg, 0.27 mmol, 80.0 eq), and DIPEA (56 mg, 0.44 mmol, 128.0 eq) at room temperature (25 °C) under nitrogen atmosphere. The mixture was then heated to 28 °C, stirred for 12 h, concentrated, filtered from methanol to methanol/water (1/1, 100 mL), purified by ultrafiltration (30K MW), and lyophilized to give a product **16h** (350 mg, white solid, 95% yield, 100% purity, about 22.5% docetaxel content).

Example 25: Synthesis of compound 16i

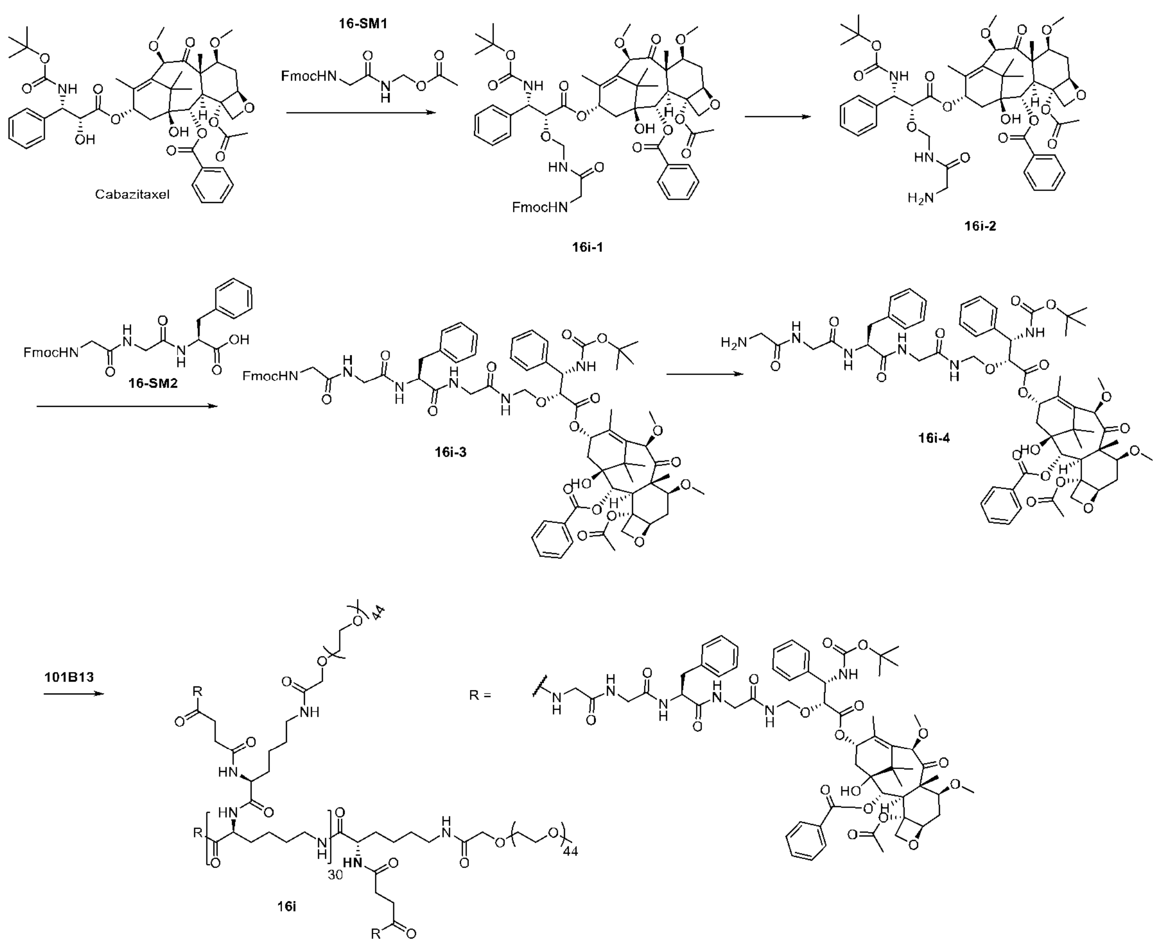

Cabazitaxel
16i-1
16i-2
16i-3
16i-4
16i

Synthesis of compounds **16i-1, 16i-2, 16i-3, and 16i-4**

The synthesis was conducted according to the synthetic method in Example **16a.**

Synthesis of compound **16i**

To a solution of **101B13** (250 mg, 0.003 mmol, 1.0 eq) in dry DMF (5 mL) were added a solution of **16i-4** (182 mg, 0.15 mmol, 45.0 eq) in dry DMF (2 mL, ultrasonicated for dissolution), PyBOP (142 mg, 0.27 mmol, 80.0 eq), and DIPEA (56 mg, 0.44 mmol, 128.0 eq) at room temperature (25 °C) under nitrogen atmosphere. The mixture was then heated to 28 °C, stirred for 12 h, concentrated, filtered from methanol to methanol/water (1/1, 100 mL), purified by ultrafiltration (30K MW), and lyophilized to give a product **16i** (330 mg, white solid, 89% yield, 99% purity, about 20.4% cabazitaxel content).

Example 26: Synthesis of compound 17a

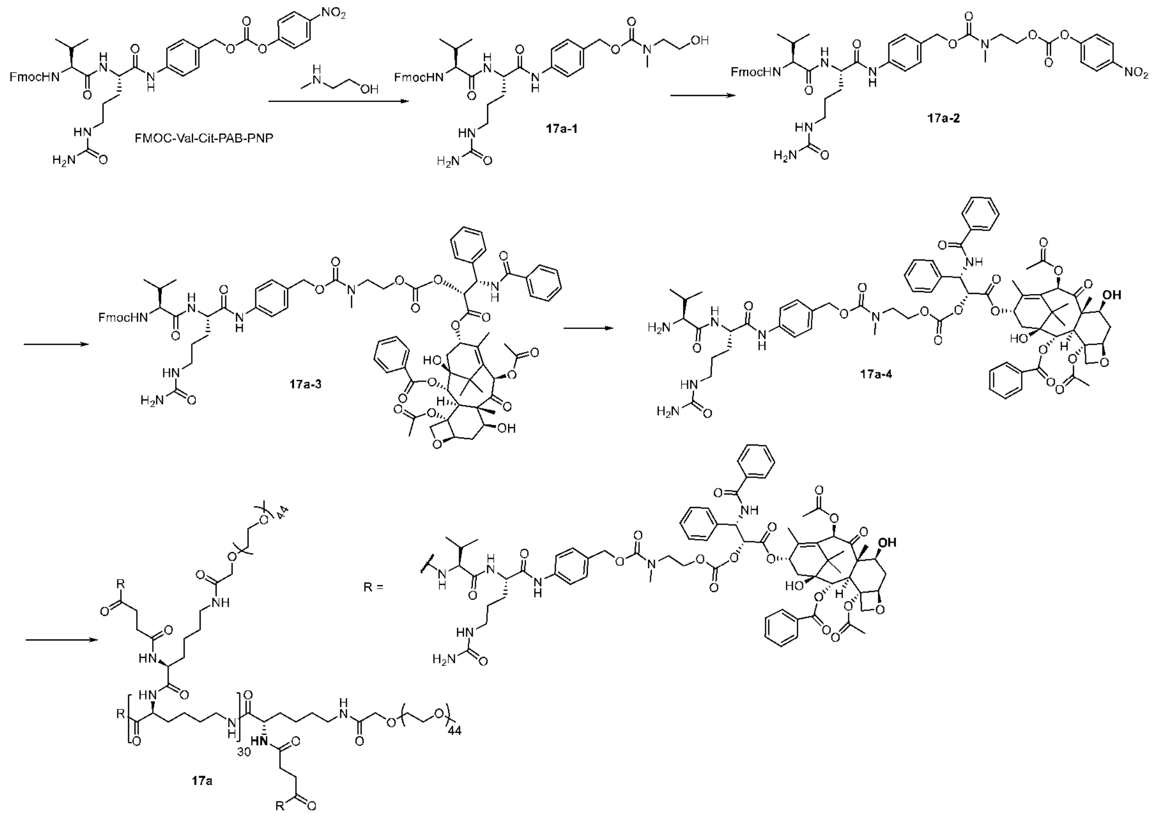


Synthesis of compound **17a-1**

Fmoc-Val-Cit-PAB-PNP (3.83 g, 5.0 mmol, 1.0 eq) and anhydrous DMF (20 mL) were added sequentially at 0 °C under nitrogen atmosphere. After partial dissolution, N-methyl-2-hydroxyethylamine (0.42 mL, 5.25 mmol, 1.05 eq) was added slowly dropwise at 0 °C. The reaction mixture was stirred at 0 °C for 2 h until LCMS monitoring indicated the depletion of the starting materials. MTBE (100 mL) was added for precipitation, and the mixture was filtered. The precipitate was washed with MTBE and then dried *in vacuo* to give a product **17a-1** (yellow solid, 3.2 g, 91% yield).

Synthesis of compound **17a-2**

**17a-1** (2.11 g, 3.0 mmol, 1.0 eq), anhydrous DMF (20 mL), bis(4-nitrophenyl) carbonate (2.01 g, 6.6 mmol, 2.2 eq), and DIPEA (0.8 mL, 4.5 mmol, 1.5 eq) were added sequentially at 0 °C under nitrogen atmosphere. The reaction mixture was stirred at 0 °C for 6 h until LCMS monitoring indicated the depletion of the starting materials. MTBE (100 mL) was added for precipitation, and the mixture was filtered. The precipitate was washed with MTBE and then dried *in vacuo* to give a product **17a-2** (white solid, 2.2 g, 86% yield).

Synthesis of compound **17a-3**

**17a-2** (434 mg, 0.5 mmol, 1.0 eq), paclitaxel (853.9 mg, 1.0 mmol, 2.0 eq), and anhydrous DMF (10 mL) were added sequentially at room temperature under nitrogen atmosphere. After partial dissolution, DMAP (45.8 mg, 0.375 mmol, 0.75 eq) was added. The reaction mixture was stirred at room temperature for 15 h until LCMS monitoring indicated the depletion of the starting materials. The reaction mixture was directly used in the next step without further processing.

Synthesis of compound **17a-4**

To the above **17a-3** reaction mixture was added diethylamine (0.4 mL, 4.0 mmol, 8.0 eq) at room temperature under nitrogen atmosphere. The reaction mixture was stirred at room temperature for 1 h until LCMS monitoring indicated

the depletion of the starting materials. The mixture was purified and lyophilized to give a product **17a-4** (560 mg, white solid, 76% yield over the two steps).

Synthesis of compound **17a**

**101B13** (80 mg, 0.034 mmol, 1.0 eq, based on the polymer structural unit), **17a-4** (75.2 mg, 0.051 mmol, 1.5 eq), PyBOP (26.5 mg, 0.051 mmol, 1.5 eq), DIPEA (0.025 mL, 0.136 mmol, 4.0 eq), and DMF (1.5 mL) were added sequentially at room temperature (25 °C) under nitrogen atmosphere. The reaction mixture was stirred at room temperature for 15 h until HPLC monitoring indicated the depletion of the starting materials. To the above DMF mixture was added MTBE (30 mL). The solution turned turbid and a white solid was precipitated. The supernatant was discarded, and the white solid was washed with MTBE and dried. The remaining white solid was dissolved in MeOH, and the mixture was purified by ultrafiltration (30K MW) and lyophilized to give a product **17a** (98 mg, white solid, 100.00% purity).

Example 27: Synthesis of compound 17b

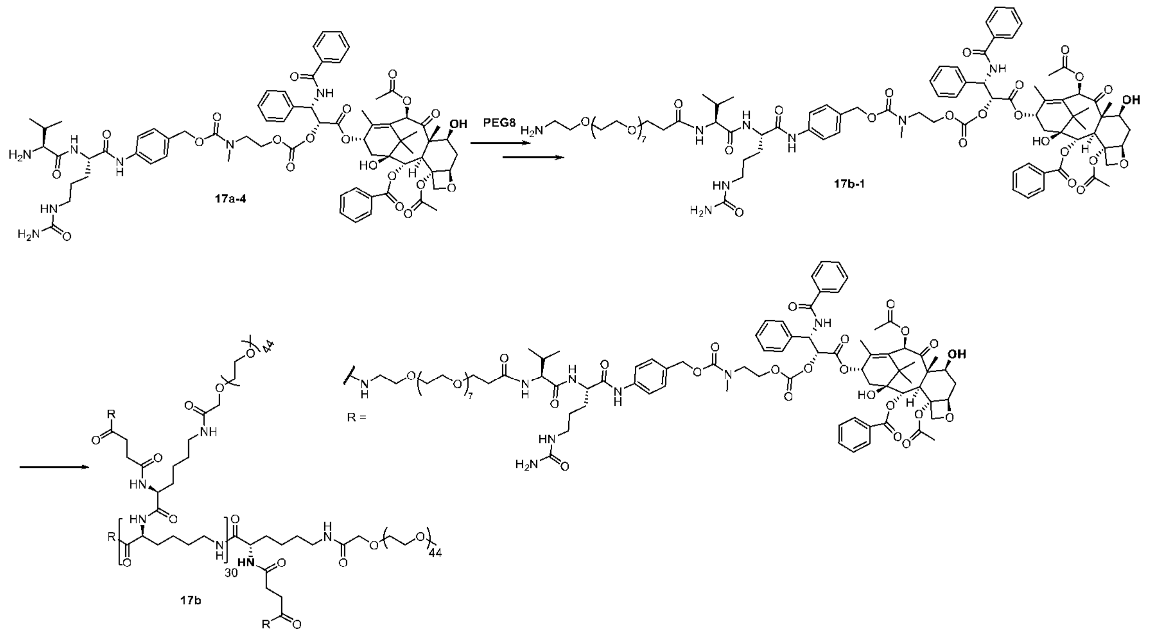


Synthesis of compound **17b-1**

To a solution of **PEG8** (176 mg, 0.265 mmol, 1.0 eq) in DMF (8 mL) were added DIPEA (137 mg, 1.06 mmol, 4.0 eq) and TSTU (80 mg, 0.265 mmol, 1.0 eq) sequentially at 0 °C under nitrogen atmosphere. The mixture was stirred at room temperature for 1 h. To the reaction mixture was added a solution of **17a-4** (360 mg, 0.265 mmol, 1.0 eq) in DMF (5 mL), and the mixture was stirred at room temperature for 3 h. When LCMS indicated the completion of the reaction, diethylamine (364 mg, 4.98 mmol, 20.0 eq) was added, and the mixture was stirred at room temperature for 1 h. When LCMS indicated the completion of the reaction, the reaction mixture was purified on a C18 column (ACN/TFA 0.05% $H_2O$) to give a product **17b-1** (white solid, 300 mg, 68% yield).

Synthesis of compound **17b**

To a solution of **101B13** (185 mg, 0.078 mmol, 1.0 eq, based on the polymer structural unit), **17b-1** (210 mg, 0.117 mmol, 1.5 eq), and PyBOP (61 mg, 0.117 mmol, 1.5 eq) in DMF (6 mL) was added DIPEA (61 mg, 0.468 mmol, 4.0 eq) at room temperature under nitrogen atmosphere. The mixture was stirred at room temperature for 16 h. The reaction mixture was concentrated by rotavap using an oil pump, and methyl tert-butyl ether was added. The mixture was stirred for 20 min, and the supernatant was removed. The oil at the bottom of the flask was first dissolved in methanol, and then purified by ultrafiltration (30K MW), and lyophilized to give compound **17b** (310 mg, 95% yield, about 22.5% paclitaxel content).

Example 28: Synthesis of compound 17c

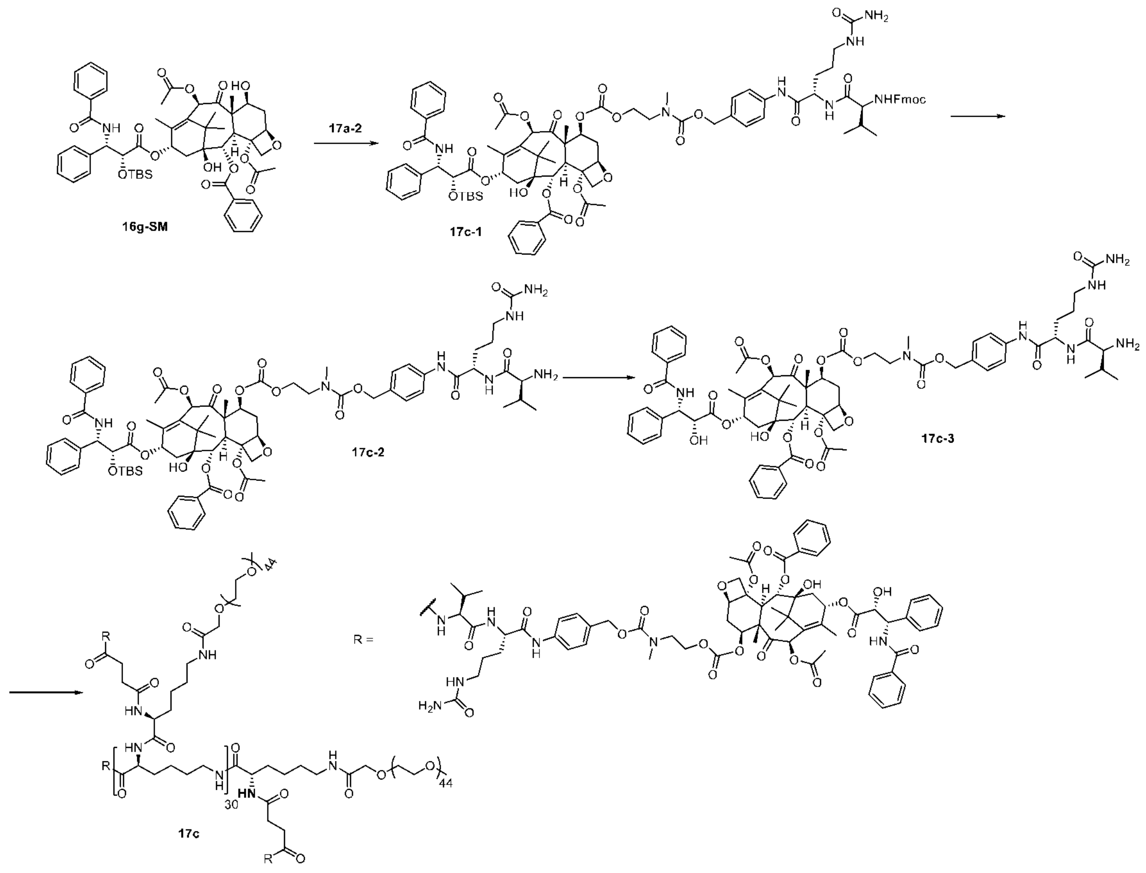


Synthesis of compound **17c-1**

**16g-SM** (500 mg, 0.52 mmol, 1.0 eq), **17a-2** (895 mg, 1.03 mmol, 2.0 eq), and anhydrous DMF (10 mL) were added sequentially at room temperature under nitrogen atmosphere. After partial dissolution, DMAP (64 mg, 0.52 mmol, 1.0 eq) was added. The reaction mixture was stirred at room temperature for 12 h until LCMS monitoring indicated the substantial depletion of the starting materials. The reaction mixture was directly used in the next step without further processing.

Synthesis of compound **17c-2**

Diethylamine (2 mL) was added to the above reaction solution of **17c-1** at room temperature under nitrogen atmosphere. The reaction mixture was stirred at room temperature for 1 h until LCMS monitoring indicated the depletion of the starting materials. The mixture was purified and lyophilized to give a product **17c-2** (450 mg, white solid, 59% yield over the two steps).

Synthesis of compound **17c-3**

To a solution of **17c-2** (300 mg, 0.20 mmol, 1.0 eq) in dry THF (5 mL) was added a pyridine hydrogen fluoride solution (1 mL) in an ice-water bath (5 °C) under nitrogen atmosphere, and the mixture was warmed to room temperature (20 °C) and stirred for 1 h. When LCMS indicated the completion of the reaction, water was added to quench the reaction, and the mixture was extracted with dichloromethane. The organic phase was concentrated, and purified by reversed-phase column chromatography to give a product **17c-3** (0.19 g, 69% yield).

Synthesis of compound **17c**

To a solution of **101B13** (200 mg, 0.003 mmol, 1.0 eq), **17c-3** (167 mg, 0.12 mmol, 45.0 eq), and PyBOP (113 mg, 0.22 mmol, 80 eq) in DMF (6 mL) was added DIPEA (45 mg, 0.35 mmol, 128.0 eq) at room temperature under nitrogen atmosphere. The mixture was stirred at room temperature for 16 h. The reaction mixture was concentrated by rotavap using an oil pump, and methyl *tert*-butyl ether was added. The mixture was stirred for 20 min, and the supernatant was removed. The oil at the bottom of the flask was first dissolved in methanol, and then purified by ultrafiltration (30K MW), and lyophilized to give compound **17c** (220 mg, 72% yield, about 23.1% paclitaxel content).

Example 29: Synthesis of compound 18a

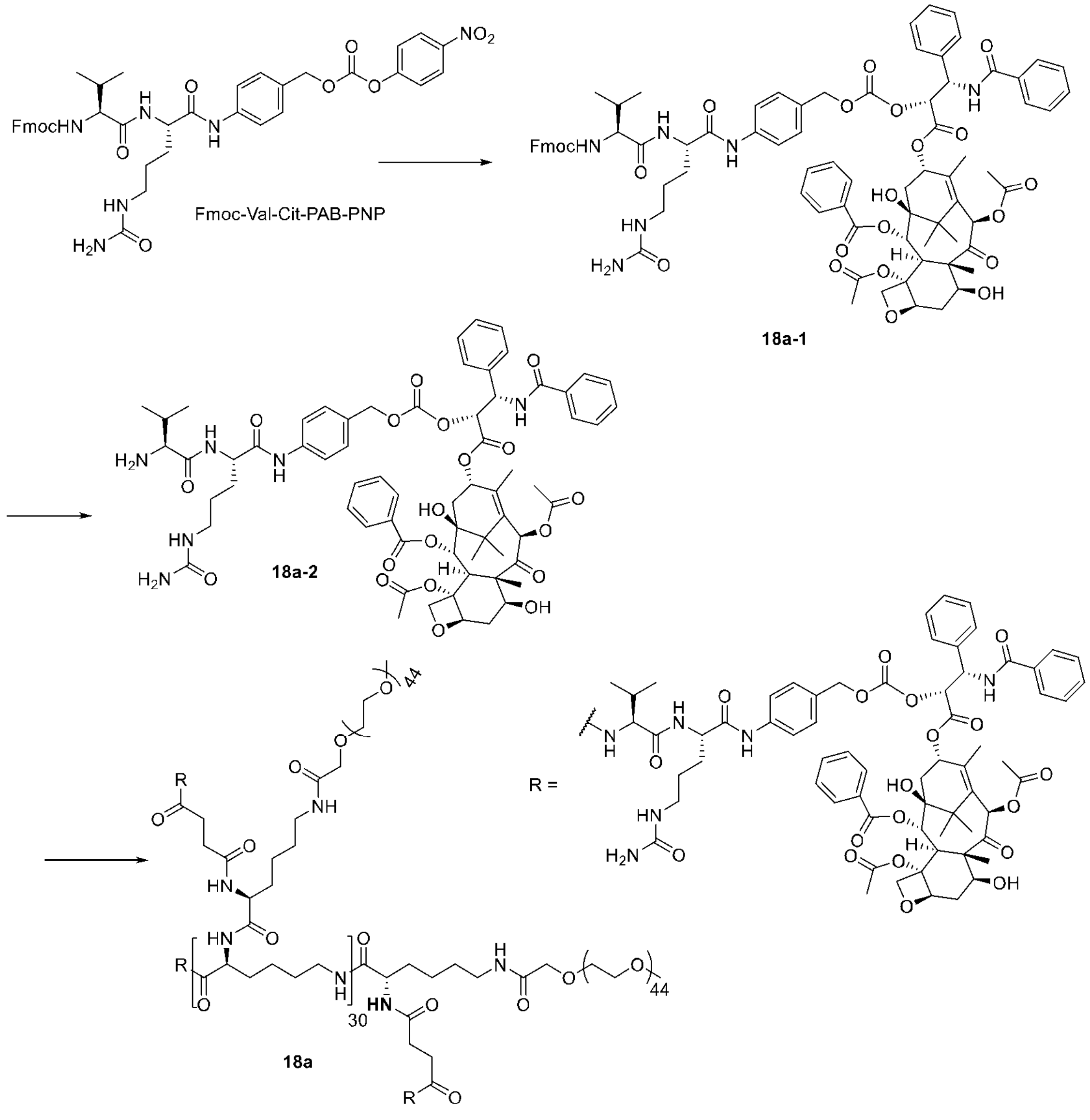

Fmoc-Val-Cit-PAB-PNP

**18a-1**

**18a-2**

**18a**

Synthesis of compound **18a-1**

Fmoc-Val-Cit-PAB-PNP (383.4 mg, 0.5 mmol, 1.0 eq), paclitaxel (853.9 mg, 1.0 mmol, 2.0 eq), and anhydrous DMF (10 mL) were added sequentially at room temperature under nitrogen atmosphere. After partial dissolution, DMAP (45.8 mg, 0.375 mmol, 0.75 eq) was added. The reaction mixture was stirred at room temperature for 15 h until LCMS monitoring indicated the depletion of the starting materials. The reaction mixture was directly used in the next step without further processing.

Synthesis of compound **18a-2**

To the above reaction mixture was added diethylamine (0.4 mL, 4.0 mmol, 8.0 eq) at room temperature under nitrogen atmosphere. The reaction mixture was stirred at room temperature for 1 h until LCMS monitoring indicated the depletion of the starting materials. The mixture was purified and lyophilized to give a product **18a-2** (415 mg, white solid, 60% yield over the two steps).

$^1H$ NMR (400 MHz, DMSO) δ 10.26 (s, 1H), 9.28 (d, *J* = 8.4 Hz, 1H), 8.70 (d, *J* = 7.5 Hz, 1H), 8.09 (d, *J* = 5.5 Hz, 3H), 8.03 - 7.95 (m, 2H), 7.85 - 7.81 (m, 2H), 7.76 - 7.70 (m, 1H), 7.68 - 7.42 (m, 12H), 7.32 (d, *J* = 8.3 Hz, 2H), 7.23 - 7.16 (m, 1H), 6.31 (s, 1H), 6.12 - 6.01 (m, 1H), 5.83 (t, *J* = 9.1 Hz, 1H), 5.53 (t, *J* = 8.6 Hz, 1H), 5.43 (d, *J* = 7.2 Hz, 1H), 5.36 (d, *J* = 8.9 Hz, 1H), 5.21 - 5.09 (m, 2H), 4.96 - 4.89 (m, 1H), 4.65 (s, 1H), 4.58 - 4.47 (m, 1H), 4.12 (dd, *J* = 10.8, 6.7 Hz, 1H), 4.02 (q, *J* = 8.3 Hz, 2H), 3.70 - 3.64 (m, 1H), 3.11 - 2.93 (m, 2H), 2.39 - 2.23 (m, 4H), 2.16 - 2.03 (m, 4H), 1.87 - 1.39 (m, 14H), 1.04 (s, 3H), 1.01 (s, 3H), 0.98 - 0.93 (m, 7H).

Synthesis of compound **18a**

**101B13** (80 mg, 0.034 mmol, 1.0 eq, based on the polymer structural unit), **18a-2** (70 mg, 0.051 mmol, 1.5 eq), PyBOP (26.5 mg, 0.051 mmol, 1.5 eq), DIPEA (0.025 mL, 0.136 mmol, 4.0 eq), and DMF (1.5 mL) were added sequentially at room temperature (25 °C) under nitrogen atmosphere. The reaction mixture was stirred at room temperature for 15 h until HPLC monitoring indicated the depletion of the starting materials. To the above DMF mixture was added MTBE (30 mL). The solution turned turbid and a white solid was precipitated. The supernatant was discarded, and the white solid was washed with MTBE and dried. The remaining white solid was dissolved in MeOH, and the mixture was purified by ultrafiltration (30K MW) and lyophilized to give a product **18a** (94 mg, white solid, 99.92% purity).

Example 30: Synthesis of compound 18b

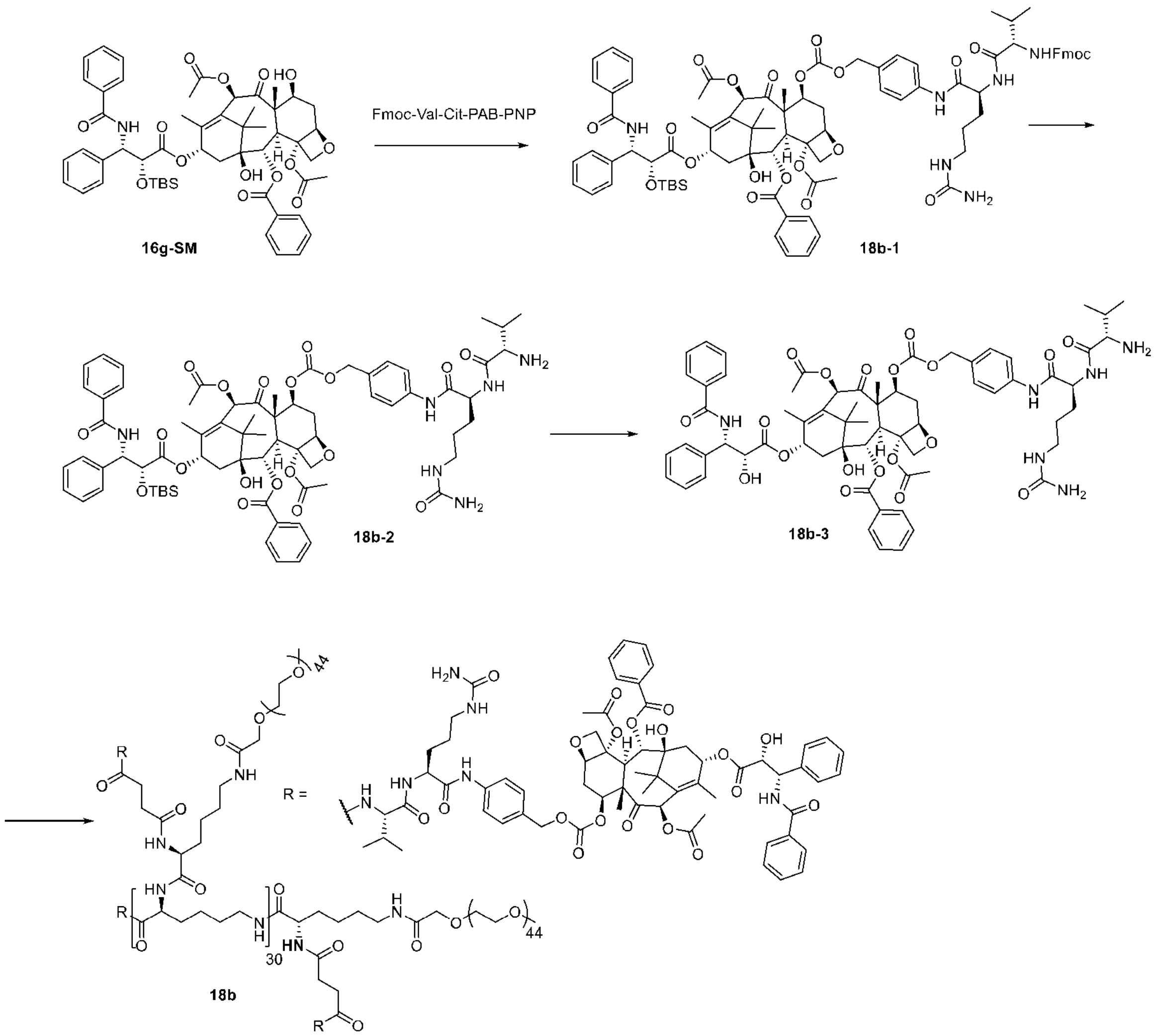


16g-SM 18b-1 18b-2 18b-3 18b

Synthesis of compounds **18b-1, 18b-2, and 18b-3**

The synthesis was conducted according to the synthetic method in Example **28.**

Synthesis of compound **18b**

To a solution of **101B13** (250 mg, 0.003 mmol, 1.0 eq) in dry DMF (5 mL) were added a solution of **18b-3** (208 mg, 0.15 mmol, 45.0 eq) in dry DMF (2 mL, ultrasonicated for dissolution), PyBOP (142 mg, 0.27 mmol, 80.0 eq), and DIPEA (56 mg, 0.44 mmol, 128.0 eq) at room temperature (25 °C) under nitrogen atmosphere. The reaction mixture was then heated to 28 °C, stirred for 12 h, concentrated, purified by ultrafiltration (30K MW), and lyophilized to give a product **18b** (340 mg, white solid, 87% yield, 99.1% purity, paclitaxel content of about 19.4%).

Example 31: Synthesis of compound 19

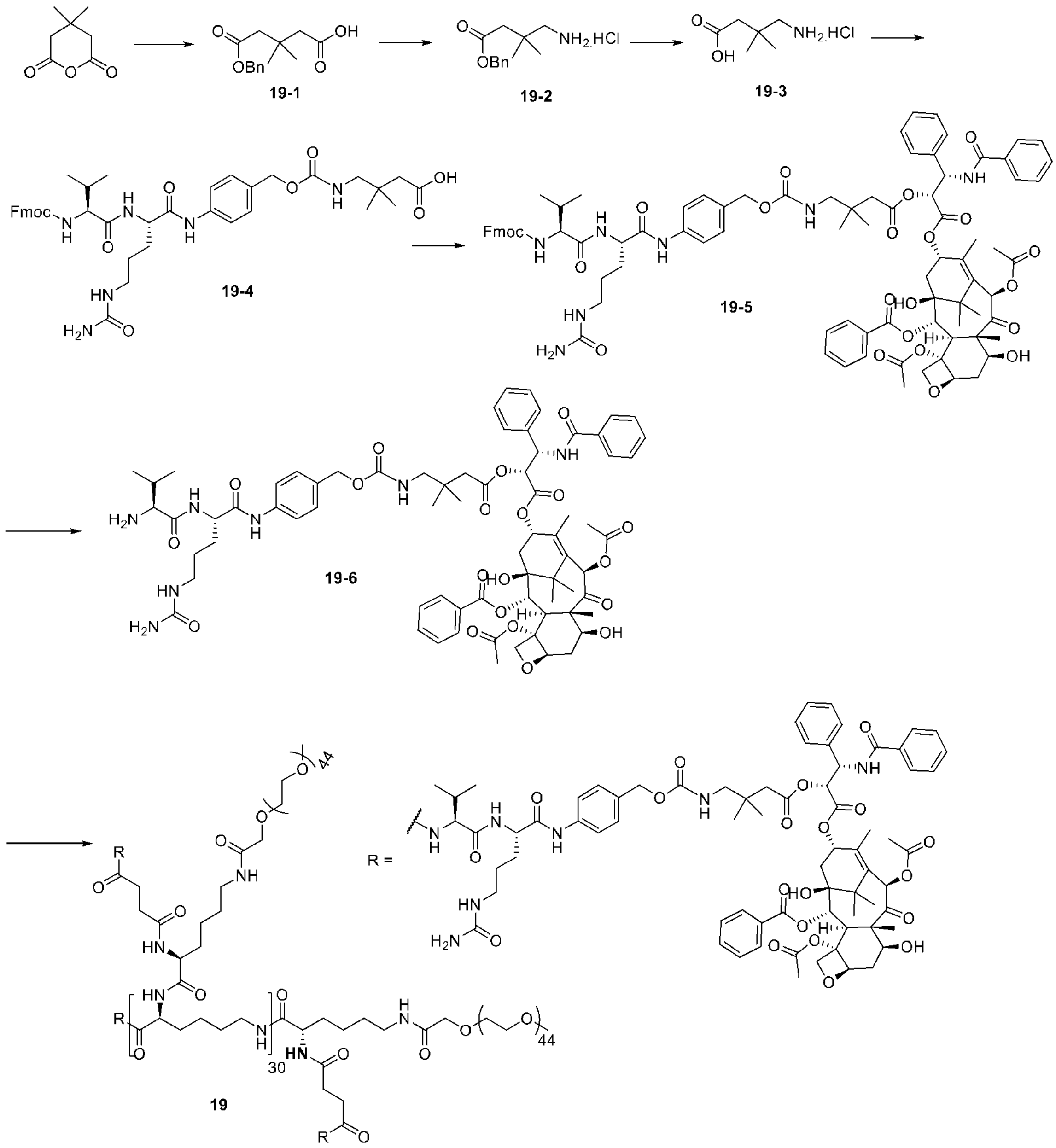

Synthesis of compound **19-1**

To a solution of 3,3-dimethylglutaric anhydride (6.43 g, 45.2 mmol, 1.0 eq) in DCM (50 mL) were added benzyl alcohol (5.87 g, 54.3 mmol, 1.2 eq), triethylamine (18.2 g, 180 mmol, 4.0 eq), and DMAP (1.66 g, 13.6 mmol, 0.3 eq) sequentially at 25 °C, and the reaction mixture was stirred for 2 h at the temperature. When LCMS monitoring indicated the completion of the reaction, the mixture was concentrated by rotavap to remove the volatile solvent and bases. The residue was redissolved in DCM (200 mL), and the mixture was transferred to a separatory funnel, and washed sequentially with 1 M HCl (aq; 200 mL) and saturated NaCl (aq; 200 mL). The organic phases were combined, dried over anhydrous $Na_2SO_4$, and concentrated by rotavap. The resulting crude product was purified on a reversed-phase column ($CH_3CN$:$H_2O$ = 5-95%). The collected mixture was concentrated by rotavap to give a product (7.8 g, colorless oil).

MS (ESI), m/z, 251.20 $[M+H]^+$.

Synthesis of compound **19-2**

To a solution of **19-1** (5.20 g, 20.8 mmol, 1.0 eq) in THF (100 mL) were added triethylamine (2.31 g, 22.9 mmol, 1.1 eq) and DPPA (6.30 g, 22.9 mmol, 1.1 eq) sequentially at 25 °C, and the reaction mixture was stirred for 8 h at the temperature. When LCMS monitoring indicated the completion of the reaction, 6 M HCl (aq; 50 mL) was added, and the

reaction mixture was heated to reflux for 1 h. The heat source was removed, and the reaction mixture was cooled to room temperature and concentrated by rotavap. The resulting crude product was purified on a reversed-phase column ($CH_3CN$:$H_2O$ = 5-70%). The collected mixture was concentrated by rotavap to give a white solid (2.50 g).

MS (ESI), m/z, 222.20 [M -Cl]$^+$.

Synthesis of compound **19-3**

To a solution of **19-2** (2.5 g, 9.67 mmol, 1.0 eq) in methanol (150 mL) was added Pd/C (10 wt%, 500 mg) at 25 °C. The mixture was purged with hydrogen and stirred at the temperature for 2 h. After the reaction was completed, the reaction mixture was filtered through celite *in vacuo.* The filtrate was concentrated by rotavap to give a white solid (2.2 g).

MS (ESI), m/z, 132.20 [M-Cl]$^+$.

Synthesis of compound **19-4**

To a solution of **19-3** (3.35 g, 4.45 mmol, 1.0 eq) in DMF (10 mL) were added Fmoc-Val-Cit-PAB-PNP (2.07 g, 8.01 mmol, 1.8 eq) and DIPEA (2.88 g, 22.3 mmol, 5.0 eq) sequentially at 25 °C. The mixture was stirred at the temperature for 2 h. When LCMS monitoring indicated the completion of the reaction, the reaction mixture was concentrated, and the resulting crude product was purified by reversed-phase column chromatography ($CH_3CN$ (0.05% TFA):$H_2O$ (0.05% TFA) = 5%-80%). The collected eluate was concentrated by rotavap to give a white solid (2.1 g).

MS (ESI), m/z, 759.20 [M+H]$^+$.

Synthesis of compound **19-5**

To a solution of **19-4** (1.06 g, 1.40 mmol, 1.0 eq) in DMF (8.0 mL) was added DCM (20 mL) at 25 °C. The mixture was stirred homogeneously, and paclitaxel (1.44 g, 1.68 mmol, 1.0 eq), DMAP (512 mg, 4.20 mmol, 3.0 eq), and EDCI (802 mg, 4.20 mmol, 3.0 eq) were then added sequentially. After the addition, the mixture was stirred for 1 h with the temperature maintained. The reaction mixture was directly used in the next step without post-treatment.

MS (ESI), m/z, 1595.20 [M+H]$^+$.

Synthesis of compound **19-6**

To the **19-5** reaction mixture from the previous step was added diethylamine (6.0 mL) directly, and the mixture was stirred at 25 °C for 2 h. After the reaction was completed, the reaction mixture was concentrated, poured into water (100 mL), and extracted with ethyl acetate (50 mL × 3). The organic phases were combined and concentrated by rotavap. The crude product was purified on a silica gel column (DCM/MeOH = 50:1-10:1) to give a crude product (1.1 g) (with paclitaxel and part of DMAP removed). The crude product was further purified on a C18 column ($CH_3CN$ (0.05% TFA):$H_2O$ (0.05% TFA) = 5%-95%). The product solution was collected and lyophilized to give a purified white solid product (810 mg).

MS (ESI), m/z, 1373.20 [M+H-TFA]$^+$.

Synthesis of compound **19**

To a solution of **101B13** (250 mg, 0.003 mmol, 1.0 eq) in dry DMF (5 mL) were added a solution of **19-6** (210 mg, 0.15 mmol, 45.0 eq) in dry DMF (2 mL, ultrasonicated for dissolution), PyBOP (142 mg, 0.27 mmol, 80.0 eq), and DIPEA (56 mg, 0.44 mmol, 128.0 eq) at room temperature (25 °C) under nitrogen atmosphere. The mixture was then heated to 28 °C, stirred for 12 h, concentrated, purified by ultrafiltration (30K MW), and lyophilized to give a product **19** (350 mg, white solid, 90% yield, 98.7% purity, about 21.3% paclitaxel content).

Example 32: Synthesis of Compound 20a

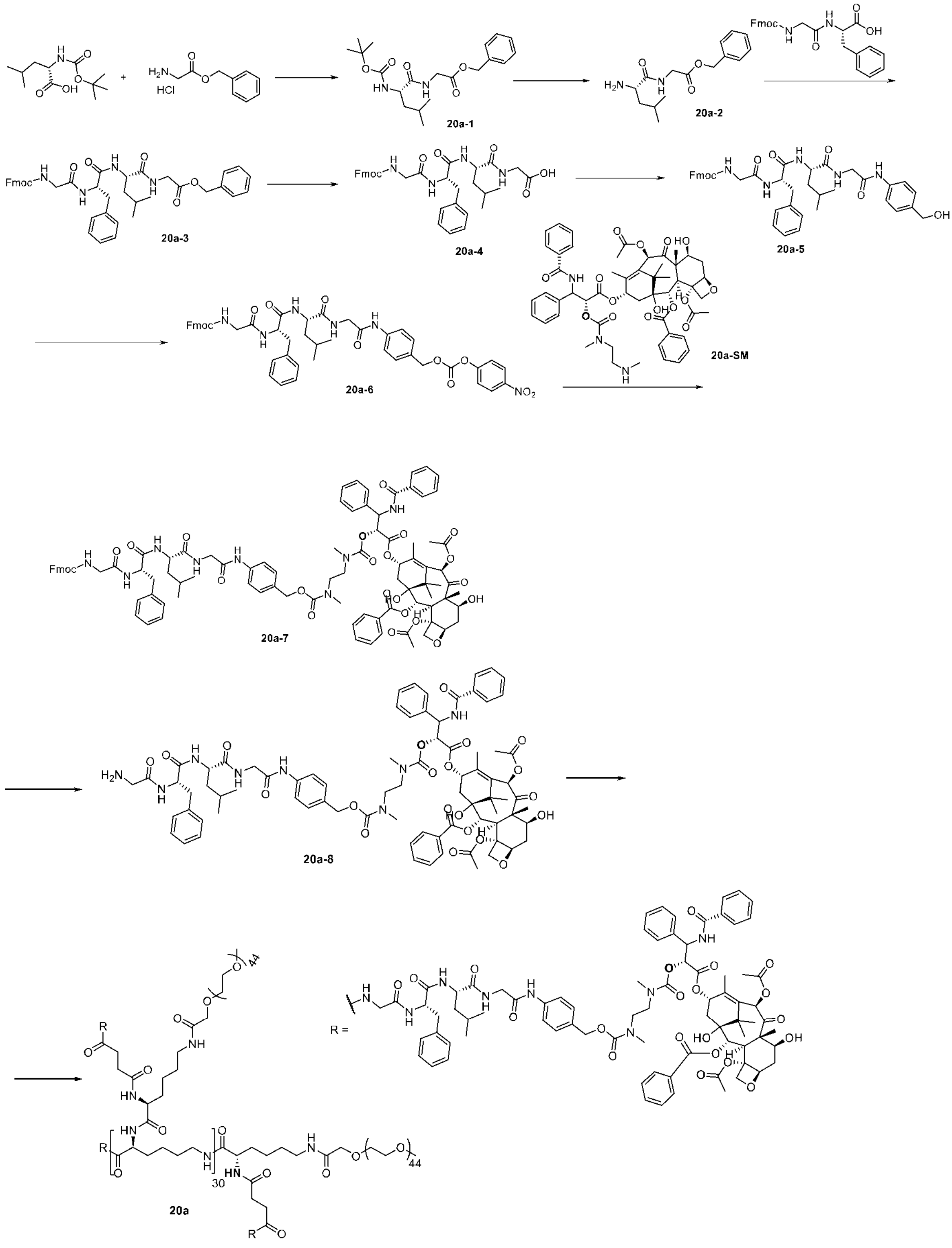

20a-1 20a-2 20a-3 20a-4 20a-5 20a-SM 20a-6 20a-7 20a-8 20a

Synthesis of compound **20a-1**

To a solution of Boc-Leu-OH (2.0 g, 8.65 mmol, 1.0 eq) in DMF (20 mL) were added glycine benzyl ester hydrochloride (1.7 g, 8.65 mmol, 1.0 eq), HATU (3.2 g, 8.65 mmol), and DIEA (2.2 g, 17.3 mmol, 2.0 eq) at room temperature under nitrogen atmosphere. The reaction mixture was stirred overnight. After the reaction was completed, methyl *tert*-butyl ether was added directly, and the mixture was filtered to give a white solid (3.2 g, 99% yield).

MS (ESI), m/z, 379.2 $[M+H]^+$.

Synthesis of compound **20a-2**

To a solution of **20a-2** (3.2 g, 8.46 mmol, 1.0 eq) in DCM (20 mL) was added TFA (5 mL) at room temperature under nitrogen atmosphere. The reaction mixture was stirred overnight. After the reaction was completed, the mixture was directly concentrated *in vacuo* and purified by column chromatography to give a white solid (2.6 g, 99% yield).
MS (ESI), m/z, 279.2 $[M+H]^+$.

Synthesis of compound **20a-3**

To a solution of **20a-2** (1.5 g, 5.39 mmol, 1.0 eq) in DMF (20 mL) were added Fmoc-Gly-Phe-OH (2.4 g, 5.39 mmol, 1.0 eq), HATU (3.2 g, 8.65 mmol), and DIEA (2.2 g, 17.3 mmol, 2.0 eq) at room temperature under nitrogen atmosphere. The reaction mixture was stirred overnight. After the reaction was completed, the mixture was purified by column chromatography to give a white solid (2.5 g, 65.7% yield).
MS (ESI), m/z, 705.2 $[M+H]^+$.

Synthesis of compound **20a-4**

To a solution of **20a-3** (2.5 g, 3.55 mmol, 1.0 eq) in MeOH (20 mL) was added Pd/C (10 mg) at room temperature under nitrogen atmosphere, and the mixture was purged with hydrogen. The reaction mixture was stirred overnight. After the reaction was completed, the mixture was filtered and purified by column chromatography to give a white solid (1.2 g, 57% yield).
MS (ESI), m/z, 615.2 $[M+H]^+$.

Synthesis of compound **20a-5**

To a solution of **20a-4** (1.2 g, 1.95 mmol, 1.0 eq) in DMF (20 mL) were added 4-aminobenzyl alcohol (0.3 g, 1.95 mmol, 1.0 eq), HATU (2.2 g, 1.95 mmol), and DIEA (1.2 g, 7.3 mmol, 2.0 eq) at room temperature under nitrogen atmosphere. The reaction mixture was stirred overnight. After the reaction was completed, the mixture was purified by column chromatography to give a white solid (1.25 g, 89% yield).
MS (ESI), m/z, 720.2 $[M+H]^+$.

Synthesis of compound **20a-6**

To a solution of **20a-5** (1.2 g, 1.66 mmol, 1.0 eq) in DMF (20 mL) were added bis(4-nitrophenyl) carbonate (0.63 g, 2.0 mmol, 1.2 eq) and DIPEA (0.44 g, 3.4 mmol, 2.0 eq) at room temperature under nitrogen atmosphere. The reaction mixture was stirred overnight. After the reaction was completed, the mixture was purified by column chromatography to give a white solid (0.95 g, 64.5% yield).
MS (ESI), m/z, 885.2 $[M+H]^+$.

Synthesis of compound **20a-7**

To a solution of **20a-6** (100 mg, 0.11 mmol, 1.0 eq) in DMF (20 mL) were added **20a-SM** (prepared according to the synthesis method in J. Org. Chem. 2001, 66, 26, 8815-8830, 109 mg, 0.11 mmol, 1.0 eq) and DIPEA (28 mg, 0.22 mmol, 2.0 eq) at room temperature under nitrogen atmosphere, and the mixture was stirred overnight. After the reaction was completed, the mixture was purified by column chromatography to give a white solid (0.1 g, 52% yield).
MS (ESI), m/z, 1714.2 $[M+H]^+$.

Synthesis of compound **20a-8**

To a solution of **20a-7** (100 mg, 0.058 mmol, 1.0 eq) in DMF (50 mL) was added diethylamine (0.2 mL) at room temperature under nitrogen atmosphere, and the mixture was stirred overnight. After the reaction was completed, the mixture was purified by preparative chromatography to give a white solid (25 mg, 28% yield).
MS (ESI), m/z, 1491.2 $[M+H]^+$.

Synthesis of compound **20a**

To a solution of **101B13** (250 mg, 0.003 mmol, 1.0 eq) in dry DMF (5 mL) were added a solution of **20a-8** (229 mg, 0.15 mmol, 45.0 eq) in dry DMF (2 mL, ultrasonicated for dissolution), PyBOP (142 mg, 0.27 mmol, 80.0 eq), and DIPEA

(56 mg, 0.44 mmol, 128.0 eq) at room temperature (25 °C) under nitrogen atmosphere. The mixture was then heated to 28 °C, stirred for 12 h, concentrated, purified by ultrafiltration (30K MW), and lyophilized to give a product **20a** (350 mg, white solid, 90% yield, 98.7% purity, about 21.3% paclitaxel content).

Example 33: Synthesis of Compound 20b

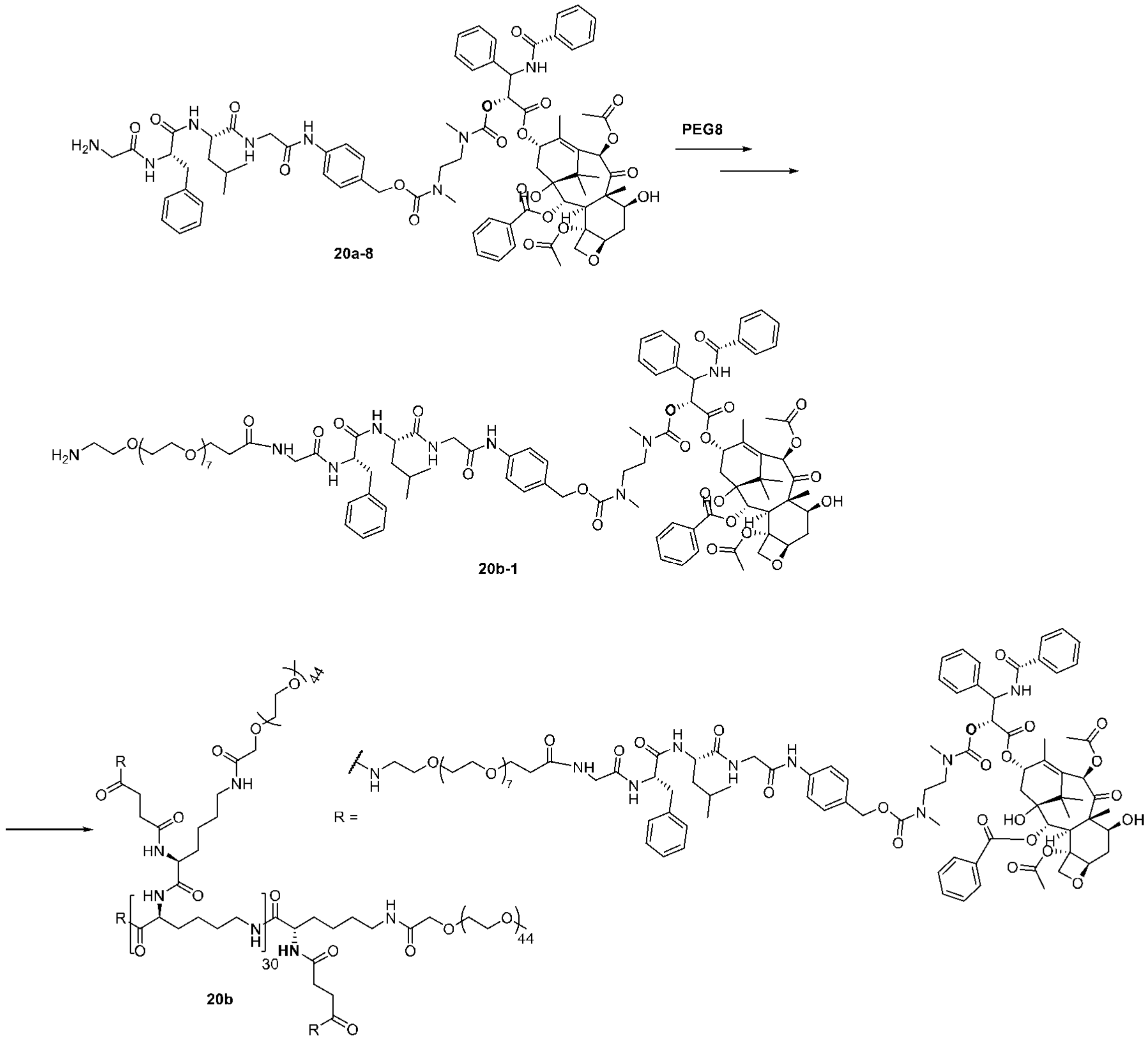


20a-8

20b-1

20b

Synthesis of compound **20b-1**

To a solution of **PEG8** (80 mg, 0.13 mmol, 1.3 eq) in DCM (20 mL) was added TSTU (40 mg, 0.13 mmol) at room temperature under nitrogen atmosphere. The mixture was stirred for 15 min, and to the mixture was added a solution of **20a-8** (150 mg, 0.1 mmol) in dichloromethane. The mixture was stirred for 2 h. After the reaction was completed, the mixture was purified by preparative chromatography to give a white solid (120 mg, 56% yield).

MS (ESI), m/z, 1069.2 $[M/2+H]^+$.

To a solution of the above intermediate (120 mg, 0.056 mmol, 1.0 eq) in DMF (50 mL) was added diethylamine (0.2 mL) at room temperature under nitrogen atmosphere, and the mixture was stirred overnight. After the reaction was completed, the mixture was purified by preparative chromatography to give a white solid (51 mg, 47% yield).

MS (ESI), m/z, 1914.2 $[M+H]^+$.

Synthesis of compound **20b**

To a solution of **101B13** (100 mg, 0.001 mmol, 1.0 eq) in dry DMF (2 mL) were added a solution of **20b-1** (117 mg,

0.06 mmol, 45.0 eq) in dry DMF (0.5 mL, ultrasonicated for dissolution), PyBOP (57 mg, 0.11 mmol, 80.0 eq), and DIPEA (23 mg, 0.17 mmol, 128.0 eq) at room temperature (25 °C) under nitrogen atmosphere. The mixture was then heated to 28 °C, stirred for 12 h, concentrated, filtered from methanol to methanol/water (1/1, 100 mL), purified by ultrafiltration (30K MW), and lyophilized to give a product **20b** (150 mg, white solid, 85% yield, 99.7% purity, about 19.3% paclitaxel content).

Example 34: Synthesis of Compound 21

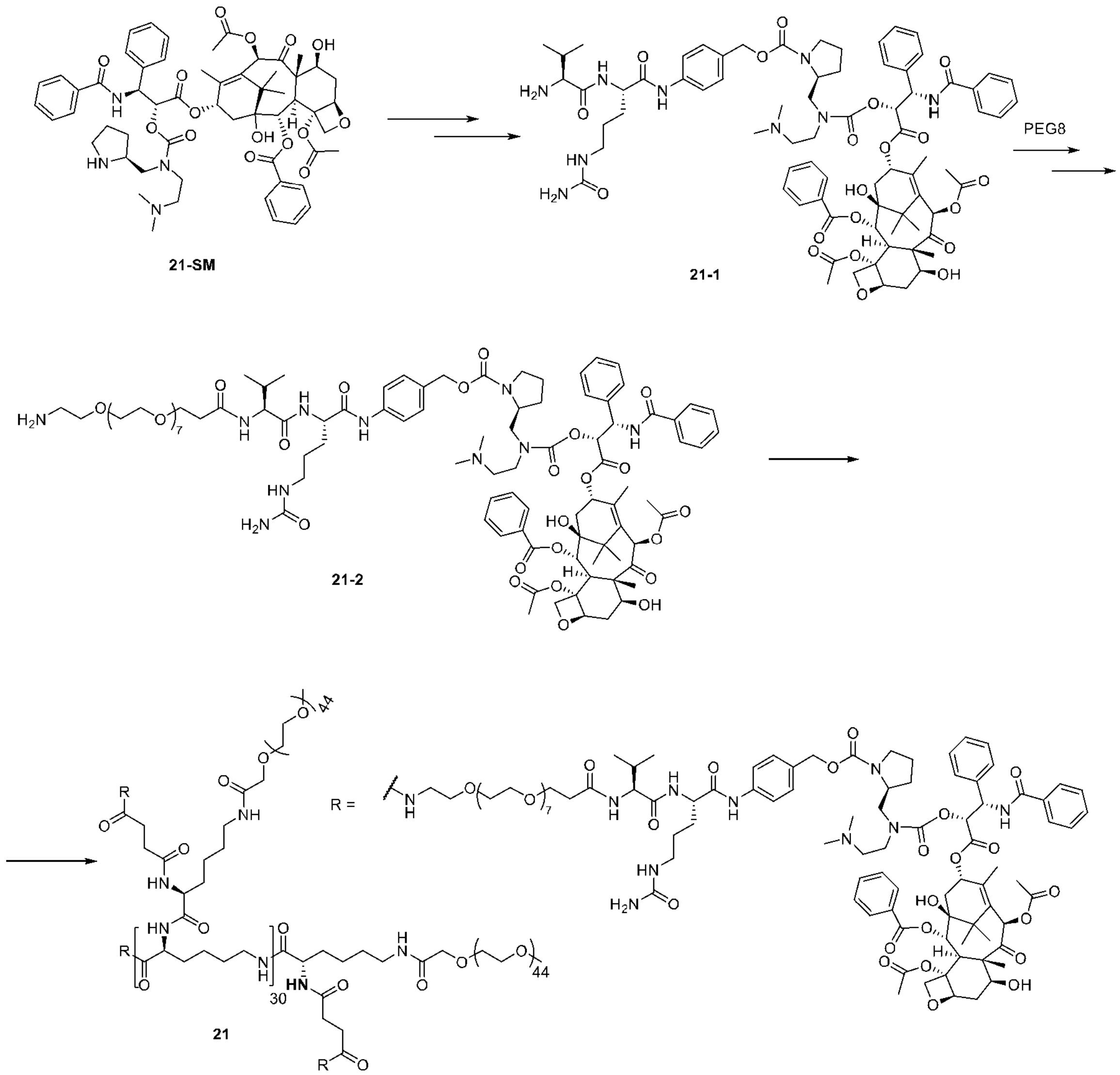


21-SM

21-1

21-2

21

Synthesis of compound **21-1**

To a solution of **21-SM** (190 mg, 0.18 mmol, 1.0 eq, synthesized according to the method in ChemMedChem ***2022*,** e202200279) in DMF (10 mL) were added Fmoc-Val-Cit-PAB-PNP (260 mg, 1.17 mmol, 1.2 eq) and DIPEA (0.26 g, 2 mmol, 2.0 eq) at room temperature under nitrogen atmosphere, and the mixture was stirred overnight. After the reaction was completed, the mixture was directly concentrated, and purified by Pre-HPLC to give a white solid (120 mg, 40% yield).

To a solution of the above compound in DMF (5 mL) was added diethylamine (0.2 mL). The reaction mixture was stirred overnight. After the reaction was completed, the mixture was directly concentrated, and purified by Pre-HPLC to give a white solid (100 mg, 96% yield).

MS (ESI), m/z, 1456.0 $[M+H]^+$.

Synthesis of compound **21-2**

To a solution of **PEG8** (40 mg, 0.061 mmol, 1.3 eq) in DCM (20 mL) was added TSTU (40 mg, 0.13 mmol) at room temperature under nitrogen atmosphere, and the mixture was stirred for 15 min. To the above mixture was added a solution of **21-1** (90 mg, 0.061 mmol) in dichloromethane. The mixture was stirred for 2 h. After the reaction was completed, the mixture was purified by preparative chromatography to give a white solid (90 mg, 70% yield).

To a solution of the above compound (90 mg, 0.98 mmol, 1.0 eq) in DMF (20 mL) was added diethylamine (0.26 g, 2 mmol, 2.0 eq), and the mixture was stirred overnight. After the reaction was completed, the mixture was directly concentrated, and purified by Pre-HPLC to give a white solid (70 mg).

MS (ESI), m/z, 1880.2 $[M+H]^+$.

Synthesis of compound **21**

To a solution of **101B13** (60 mg, 0.001 mmol, 1.0 eq) in dry DMF (2 mL) were added a solution of **21-2** (69 mg, 0.04 mmol, 45.0 eq) in dry DMF (0.5 mL, ultrasonicated for dissolution), PyBOP (34 mg, 0.07 mmol, 80.0 eq), and DIPEA (14 mg, 0.10 mmol, 128.0 eq) at room temperature (25 °C) under nitrogen atmosphere. The mixture was then heated to 28 °C, stirred for 12 h, concentrated, purified by ultrafiltration (30K MW), and lyophilized to give a product **21** (90 mg, white solid, 85% yield, 100% purity, about 21.2% paclitaxel content) (**21-2** was the standard reference for HPLC content assay).

Example 35: Synthesis of Compound 22

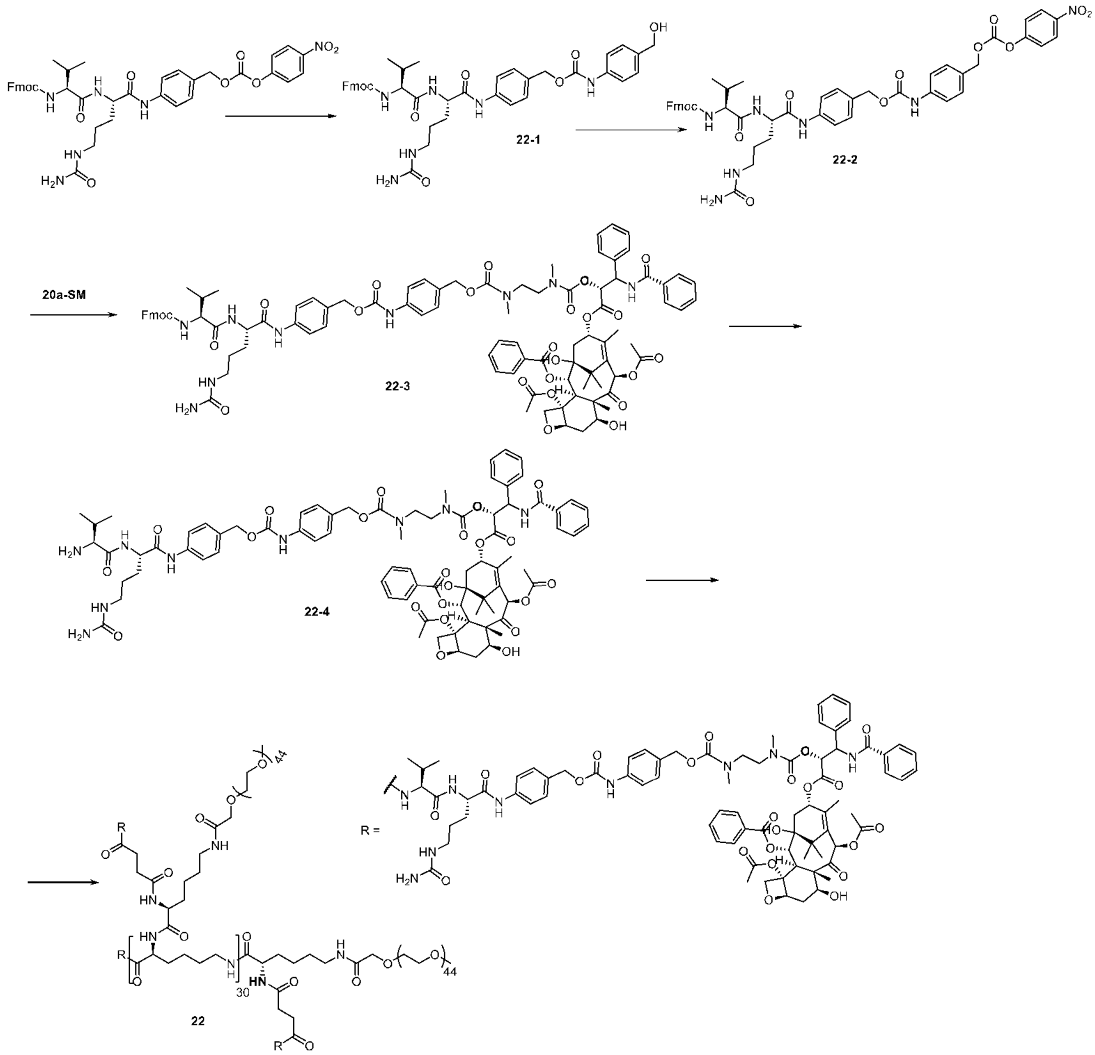

Synthesis of compound **22-1**

To a solution of Fmoc-Val-Cit-PAB-PNP (2.0 g, 2.6 mmol, 1.0 eq) in DMF (20 mL) were added 4-aminobenzyl alcohol (321 mg, 2.6 mmol, 1.0 eq) and DIPEA (0.67 g, 5.2 mmol, 2.0 eq) at room temperature under nitrogen atmosphere, and the mixture was stirred overnight. After the reaction was completed, methyl *tert*-butyl ether was added directly, and the mixture was filtered to give a white solid (2.2 g, 95.9% yield, 90% purity).

MS (ESI), m/z, 751.2 $[M+H]^+$.

Synthesis of compound **22-2**

To a solution of **22-1** (2.2 g, 2.9 mmol, 1.0 eq) in DMF (20 mL) were added bis(4-nitrophenyl) carbonate (790 mg, 2.6 mmol, 1.0 eq) and DIPEA (0.67 g, 5.2 mmol, 2.0 eq) at room temperature under nitrogen atmosphere, and the mixture was stirred overnight. After the reaction was completed, the mixture was directly concentrated, and purified by Pre-HPLC to give a white solid (1.9 g, 75.9% yield).

MS (ESI), m/z, 916.2 $[M+H]^+$.

Synthesis of compound **22-3**

To a solution of **20a-SM** (100 mg, 0.1 mmol, 1.0 eq) in DMF (10 mL) were added **22-2** (94 mg, 0.1 mmol, 1.0 eq) and DIPEA (30 mg, 10.2 mmol, 2.0 eq) at room temperature under nitrogen atmosphere, and the mixture was stirred overnight. After the reaction was completed, the mixture was directly concentrated, and purified by Pre-HPLC to give a white solid (100 mg, 57% yield). To a solution of the above intermediate (100 mg, 0.057 mmol, 1.0 eq) in DMF (510 mL) was added diethylamine (0.2 mL), and the mixture was stirred overnight. After the reaction was completed, the mixture was directly concentrated, and purified by Pre-HPLC to give a white solid (75 mg, 86% yield).

MS (ESI), m/z, 1522.2 $[M+H]^+$.

Synthesis of compound **22**

**101B13** (50 mg, 0.02 mmol, 1.0 eq, based on the polymer structural unit), **22-3** (51.9 mg, 0.03 mmol, 1.5 eq), PyBOP (15.6 mg, 0.03 mmol, 1.5 eq), DIPEA (0.015 mL, 0.08 mmol, 4.0 eq), and DMF (0.8 mL) were added sequentially at room temperature (25 °C) under nitrogen atmosphere. The reaction mixture was stirred at room temperature for 15 h until HPLC monitoring indicated the depletion of the starting materials. To the above DMF mixture was added MTBE (30 mL). The solution turned turbid and a white solid was precipitated. The supernatant was discarded, and the white solid was washed with MTBE and dried. The remaining white solid was dissolved in MeOH, and the mixture was purified by ultrafiltration (30K MW) and lyophilized to give a product **22** (white solid, 81 mg, 99.85% purity).

Example 36: Synthesis of Compound 23

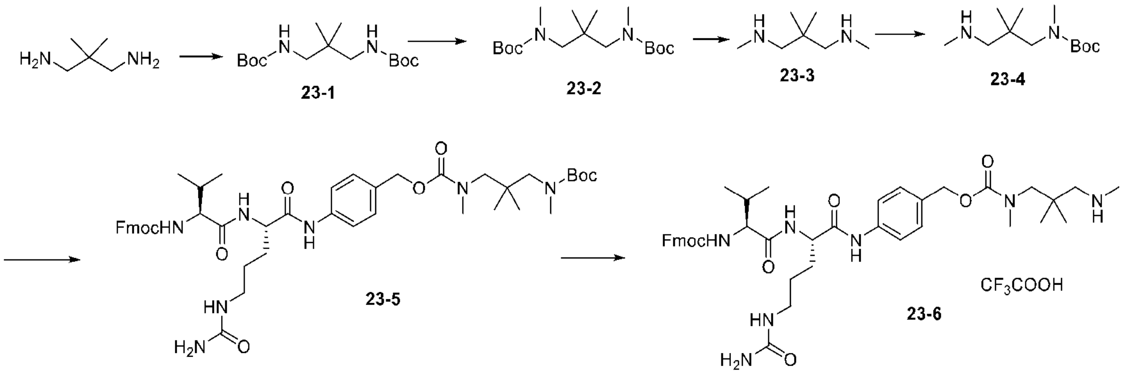

**23-1** **23-2** **23-3** **23-4**

**23-5** **23-6**

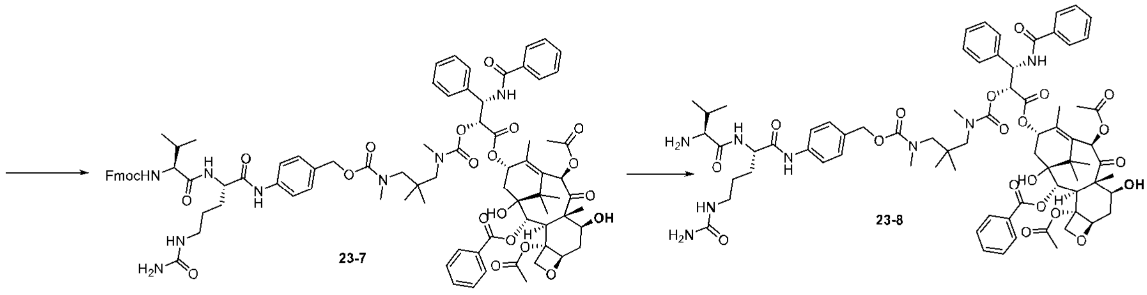

23-7

23-8

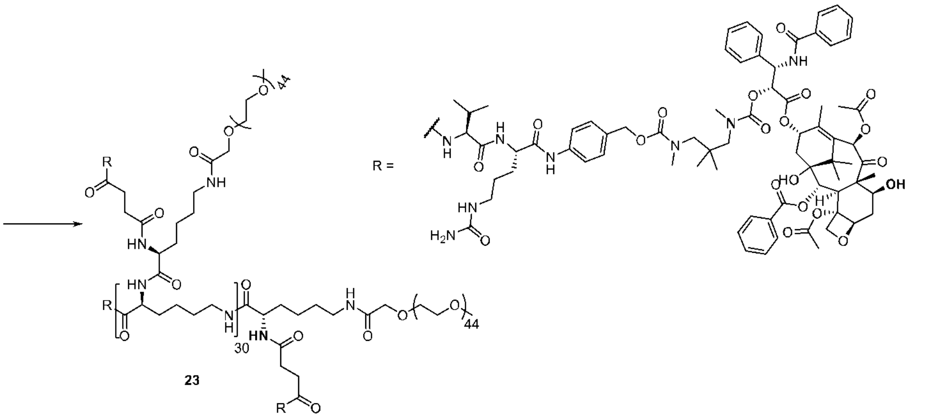

23

Synthesis of compound **23-1**

To a solution of dimethylpropanediamine (3.0 g, 29.35 mmol, 1.0 eq) in DCM (40 mL) were added triethylamine (8.9 g, 88.05 mmol, 3.0 eq) and $Boc_2O$ (16 g, 73.38 mmol, 2.5 eq) separately at room temperature (25 °C) under nitrogen atmosphere. The mixture was stirred at room temperature for 2 h. When LCMS indicated the completion of the reaction, the reaction mixture was concentrated by rotavap and purified on a silica gel column to give **23-1** (white solid, 8.3 g, 94% yield).

Synthesis of compound **23-2**

To a solution of **23-1** (4.0 g, 13.22 mmol, 1.0 eq) in THF (40 mL) was added NaH (1.3 g, 60%, 33.05 mmol, 2.5 eq) in portions at room temperature (0 °C) under nitrogen atmosphere. The reaction mixture was stirred for 2 h at 0 °C. Methyl iodide (9.4 g, 66.10 mmol, 5.0 eq) was added, and the mixture was stirred at room temperature for 16 h. When LCMS indicated the completion of the reaction, the reaction mixture was added in portions to ice water and extracted with ethyl acetate. The resulting solution was purified on a silica gel column to give **23-2** (colorless oil, 4.0 g, 91% yield).

Synthesis of compound **23-3**

To a solution of **23-2** (4.0 g, 12 mmol, 1.0 eq) in DCM (25 mL) was added TFA (5 mL) at room temperature (25 °C) under nitrogen atmosphere. The mixture was stirred at room temperature for 2 h. When TLC indicated the completion of the reaction, the reaction mixture was concentrated by rotavap to give a product **23-3** (colorless oil, 1.6 g, 100% yield).

Synthesis of compound **23-4**

To a solution of **23-3** (1.6 g, 12.28 mmol, 1.0 eq) in DCM (30 mL) was added triethylamine (2.5 g, 24.56 mmol, 2.0 eq) at room temperature (25 °C) under nitrogen atmosphere. The reaction mixture was cooled to 0 °C, and a solution of $Boc_2O$ (2.7 g, 12.28 mmol, 1.0 eq) in DCM (10 mL) was added dropwise. After the addition, the mixture was stirred for 1 h, warmed to room temperature, and stirred for another 3 h. When LCMS indicated the completion of the reaction, the reaction mixture was concentrated by rotavap and purified on a silica gel column to give a product **23-4** (colorless oil, 600 mg, 21% yield).

Synthesis of compound **23-5**

To a solution of Fmoc-Val-Cit-PAB-PNP (1.0 g, 1.3 mmol, 1.0 eq) in DMF (10 mL) were added **23-4** (299 mg, 1.3 mmol, 1.0 eq) and DIPEA (336 mg, 2.6 mmol, 2.0 eq) at room temperature under nitrogen atmosphere. The mixture was

stirred at 15 °C for 16 h. When LCMS indicated the completion of the reaction, the reaction mixture was purified on a C18 column (ACN/TFA 0.05% $H_2O$) to give a product **23-5** (white solid, 500 mg, 45% yield).

Synthesis of compound **23-6**

To a solution of **23-5** (500 mg, 0.583 mmol, 1.0 eq) in DCM (15 mL) was added TFA (5 mL) at room temperature under nitrogen atmosphere. The mixture was stirred at room temperature for 2 h. When LCMS indicated the completion of the reaction, the reaction mixture was concentrated by rotavap to give a product **23-6** (brown oil, 440 mg, 100% yield).

Synthesis of compound **23-7**

To a solution of **23-6** (100 mg, 0.132 mmol, 1.0 eq) in DMF (10 mL) were added DIPEA (70 mg, 0.528 mmol, 4.0 eq) and **7-SM1** (135 mg, 0.132 mmol, 1.0 eq) at room temperature under nitrogen atmosphere. The mixture was stirred at 15 °C for 16 h. When LCMS indicated the completion of the reaction, the reaction mixture was purified on a C18 column (ACN/TFA 0.05% $H_2O$) to give a product **23-7** (white solid, 100 mg, 46% yield).

Synthesis of compound **23-8**

To a solution of **23-7** (100 mg, 0.06 mmol, 1.0 eq) in DMF (5 mL) was added diethylamine (90 mg, 1.2 mmol, 20.0 eq) at room temperature under nitrogen atmosphere. The mixture was stirred at room temperature for 1 h. When LCMS indicated the completion of the reaction, the reaction mixture was subjected to preparative chromatography to give a product **23-8** (white solid, 13.5 mg, 16% yield).

Synthesis of compound **23**

To a solution of **101B13** (250 mg, 0.003 mmol, 1.0 eq) in dry DMF (5 mL) were added a solution of **23-8** (217 mg, 0.15 mmol, 45.0 eq) in dry DMF (2 mL, ultrasonicated for dissolution), PyBOP (142 mg, 0.27 mmol, 80.0 eq), and DIPEA (56 mg, 0.44 mmol, 128.0 eq) at room temperature (25 °C) under nitrogen atmosphere. The mixture was then heated to 28 °C, stirred for 12 h, concentrated, purified by ultrafiltration (30K MW), and lyophilized to give a product **23** (360 mg, white solid, 91% yield, 99.7% purity, about 22.3% paclitaxel content).

Example 37: Synthesis of Compound 24

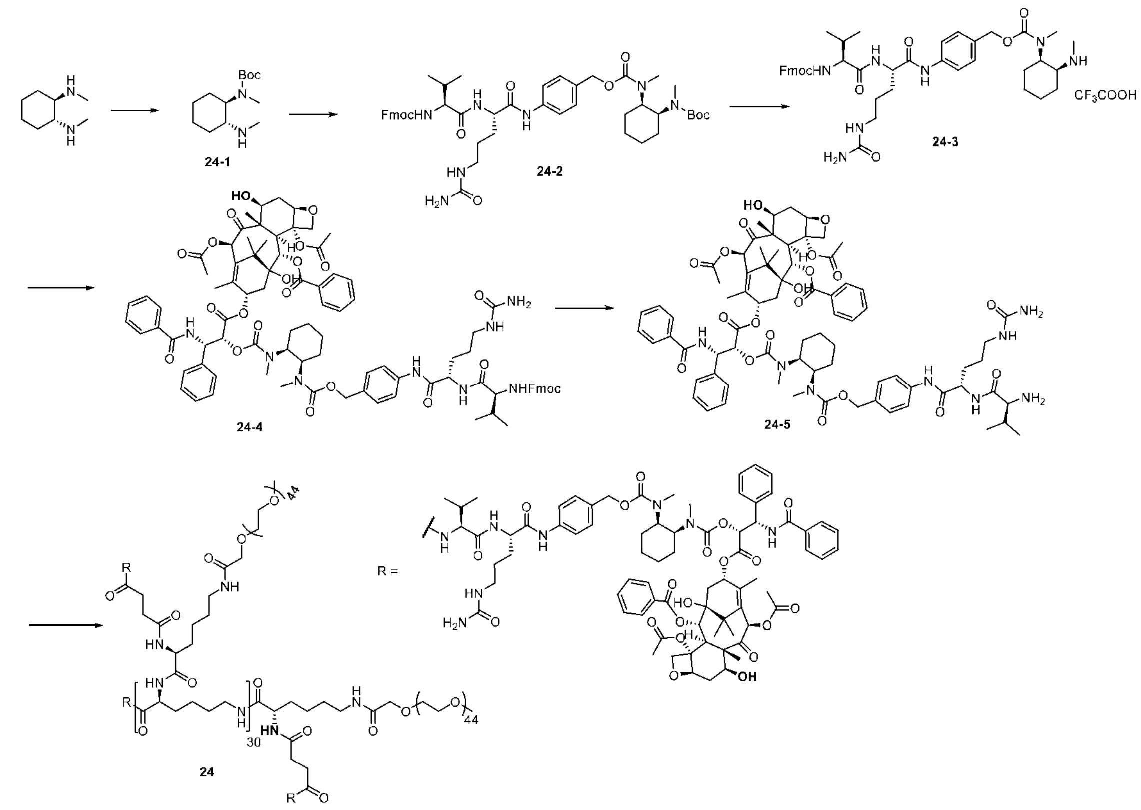

24-1, 24-2, 24-3, 24-4, 24-5, 24

Synthesis of compound **24-1**

To a solution of *trans*-(1*R*,2*R*)-N,N-dimethyl-1,2-cyclohexanediamine (2.0 g, 14 mmol, 1.0 eq) in DCM (40 mL) was added triethylamine (2.8 g, 28 mmol, 2.0 eq) at room temperature under nitrogen atmosphere. The reaction mixture was cooled to 0 °C, and a solution of $Boc_2O$ (3.06 g, 14 mmol, 1.0 eq) in DCM (10 mL) was added dropwise. After the addition, the mixture was stirred for 1 h, warmed to room temperature, and stirred for another 1 h. When LCMS indicated the completion of the reaction, the mixture was concentrated and purified on a silica gel column to give a product **24-1** (colorless oil, 1.5 g, 44% yield).

Synthesis of compound **24-2**

To a solution of Fmoc-Val-Cit-PAB-PNP (500 mg, 0.65 mmol, 1.0 eq) in DMF (10 mL) were added **24-1** (157 mg, 0.65 mmol, 1.0 eq) and DIPEA (336 mg, 2.6 mmol, 4.0 eq) at room temperature under nitrogen atmosphere. The mixture was stirred at 15 °C for 16 h. When LCMS indicated the completion of the reaction, the reaction mixture was purified on a C18 column (ACN/TFA 0.05% $H_2O$) to give a product **24-2** (white solid, 300 mg, 53% yield).

Synthesis of compound **24-3**

To a solution of **24-2** (300 mg, 0.34 mmol, 1.0 eq) in DCM (15 mL) was added TFA (5 mL) at room temperature under nitrogen atmosphere. The mixture was stirred at room temperature for 1 h. When LCMS indicated the completion of the reaction, the reaction mixture was concentrated by rotavap to give a product **24-3** (brown oil, 270 mg, 100% yield).

Synthesis of compound **24-4**

To a solution of **24-3** (60 mg, 0.077 mmol, 1.0 eq) in DMF (5 mL) were added DIPEA (40 mg, 0.308 mmol, 2.0 eq) and **7-SM1** (80 mg, 0.077 mmol, 1.0 eq) at room temperature under nitrogen atmosphere. The mixture was stirred at 15 °C for 16 h. When LCMS indicated the completion of the reaction, the reaction mixture was purified on a C18 column (ACN/TFA 0.05% $H_2O$) to give a product **24-4** (white solid, 100 mg, 79% yield).

Synthesis of compound **24-5**

To a solution of **24-4** (100 mg, 0.06 mmol, 1.0 eq) in DMF (5 mL) was added diethylamine (90 mg, 1.2 mmol, 20.0 eq) at room temperature under nitrogen atmosphere. The mixture was stirred at room temperature for 1 h. When LCMS indicated the completion of the reaction, the reaction mixture was subjected to preparative chromatography to give a product **24-5** (white solid, 13.5 mg, 16% yield).

Synthesis of compound **24**

To a solution of **101B13** (250 mg, 0.003 mmol, 1.0 eq) in dry DMF (5 mL) were added a solution of **24-5** (219 mg, 0.15 mmol, 45.0 eq) in dry DMF (2 mL, ultrasonicated for dissolution), PyBOP (142 mg, 0.27 mmol, 80.0 eq), and DIPEA (56 mg, 0.44 mmol, 128.0 eq) at room temperature (25 °C) under nitrogen atmosphere. The mixture was then heated to 28 °C, stirred for 12 h, concentrated, purified by ultrafiltration (30K MW), and lyophilized to give a product **24** (350 mg, white solid, 89% yield, 100% purity, about 18.9% paclitaxel content).

Example 38: Synthesis of Compound 25

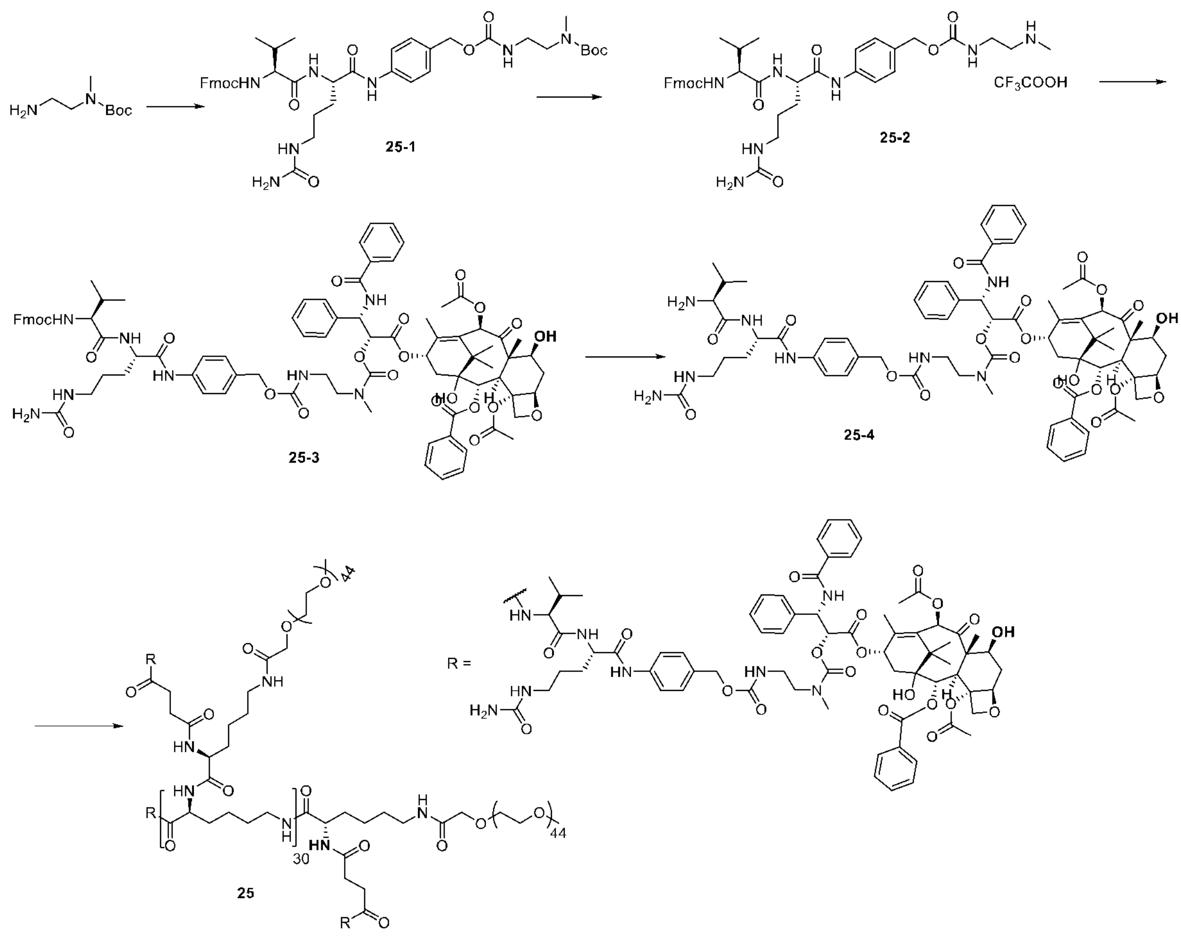

25-1

25-2

25-3

25-4

R =

25

Synthesis of compound **25-1**

To a solution of Fmoc-Val-Cit-PAB-PNP (1.0 g, 1.3 mmol, 1.0 eq) in DMF (15 mL) was added N-Boc-N-methyl ethylenediamine (227 mg, 1.3 mmol, 1.0 eq) at room temperature under nitrogen atmosphere. The mixture was stirred at room temperature for 4 h. When LCMS indicated the completion of the reaction, the reaction mixture was purified on a C18 column (ACN/TFA 0.05% $H_2O$) to give a product **25-1** (white solid, 800 mg, 77% yield).

Synthesis of compound **25-2**

To a solution of **25-1** (800 mg, 0.99 mmol, 1.0 eq) in DCM (15 mL) was added TFA (5 mL) at room temperature under nitrogen atmosphere. The mixture was stirred at room temperature for 1 h. When LCMS indicated the completion of the reaction, the reaction mixture was concentrated by rotavap to give a product **25-2** (brown oil, 700 mg, 100% yield).

Synthesis of compound **25-3**

To a solution of **25-2** (100 mg, 0.142 mmol, 1.0 eq) in DMF (5 mL) were added DIPEA (74 mg, 0.568 mmol, 4.0 eq) and **7-SM1** (145 mg, 0.142 mmol, 1.0 eq) at room temperature under nitrogen atmosphere. The mixture was stirred at 15 °C for 16 h. When LCMS indicated the completion of the reaction, the reaction mixture was purified on a C18 column (ACN/TFA 0.05% $H_2O$) to give a product **25-3** (white solid, 100 mg, 44% yield).

Synthesis of compound **25-4**

To a solution of **25-3** (120 mg, 0.075 mmol, 1.0 eq) in DMF (6 mL) was added diethylamine (110 mg, 1.5 mmol, 20.0 eq) at room temperature under nitrogen atmosphere. The mixture was stirred at room temperature for 1 h. When LCMS indicated the completion of the reaction, the reaction mixture was purified on a C18 column (ACN/TFA 0.05% $H_2O$) to give a product **25-4** (white solid, 90 mg, 88% yield).

Synthesis of compound **25**

To a solution of **101B13** (250 mg, 0.003 mmol, 1.0 eq) in dry DMF (5 mL) were added a solution of **25-4** (208 mg, 0.15 mmol, 45.0 eq) in dry DMF (2 mL, ultrasonicated for dissolution), PyBOP (142 mg, 0.27 mmol, 80.0 eq), and DIPEA (56 mg, 0.44 mmol, 128.0 eq) at room temperature (25 °C) under nitrogen atmosphere. The mixture was then heated to 28 °C, stirred for 12 h, concentrated, purified by ultrafiltration (30K MW), and lyophilized to give a product **25** (370 mg, white solid, 95% yield, 98.5% purity, about 22.3% paclitaxel content).

Example 39: Synthesis of Compound 26

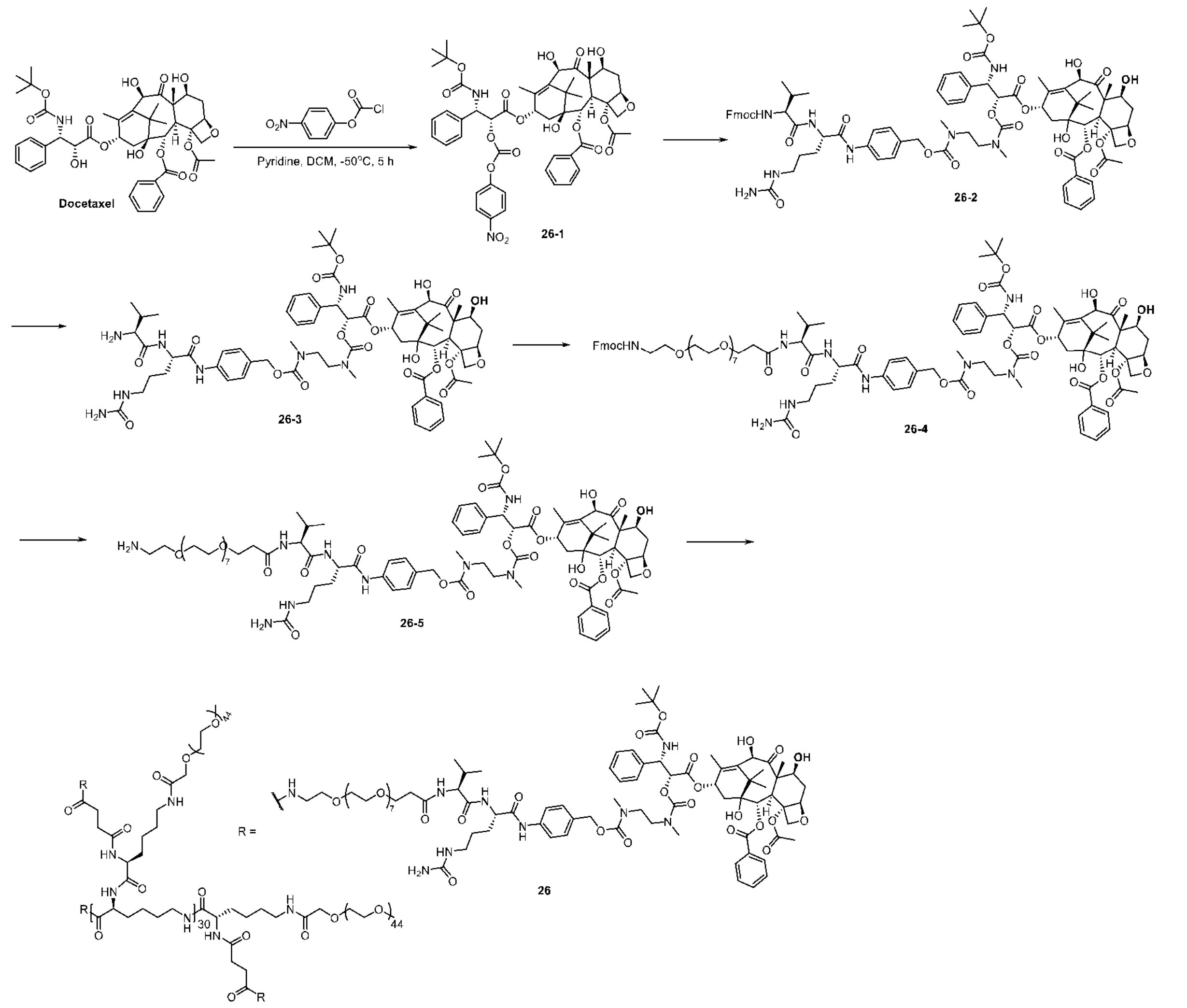


Synthesis of compound **26-1**

To a solution of docetaxel (2.0 g, 2.47 mmol, 1.0 eq) in DCM (30 mL) was added pyridine (300 mg, 3.71 mmol, 1.5 eq) at -50 °C under nitrogen atmosphere, and then a solution of *p*-nitrobenzoyl chloride (550 mg, 2.72 mmol, 1.1 eq) in DCM (10 mL) was added dropwise. After the addition, the reaction mixture was stirred for 5 h at -50 °C. A sample was taken for LCMS detection, which indicated that the reaction was completed. The mixture was washed with a sodium bisulfate solution and then washed twice with a sodium chloride solution, dried over anhydrous sodium sulfate, and concentrated by rotavap to remove the solvent. The residue was purified on a silica gel column (PE/EA = 1:1) to give a product **26-1** (white solid, 1.0 g, 42% yield).

Synthesis of compound **26-2**

To a solution of **7-SM2** (692 mg, 0.966 mmol, 1.0 eq) in DMF (10 mL) were added DIPEA (250 mg, 1.932 mmol, 2.0 eq) and **26-1** (940 mg, 0.966 mmol, 1.0 eq) at room temperature under nitrogen atmosphere. The mixture was stirred at room temperature for 4 h. When LCMS indicated the completion of the reaction, the reaction mixture was directly used in the next step.

Synthesis of compound **26-3**

To a solution of **26-2** (1.0 g, 0.645 mmol, 1.0 eq) in DMF (15 mL) was added diethylamine (943 mg, 12.90 mmol, 20.0 eq) at room temperature under nitrogen atmosphere. The mixture was stirred at room temperature for 1 h. When LCMS indicated the completion of the reaction, the reaction mixture was purified on a C18 column (ACN/TFA 0.05% $H_2O$) to give a product **26-3** (white solid, 600 mg, 70% yield).

Synthesis of compound **26-4**

To a solution of **PEG8** (300 mg, 0.452 mmol, 1.0 eq) in DMF (8 mL) were added DIPEA (233 mg, 1.81 mmol, 4.0 eq) and TSTU (136 mg, 0.452 mmol, 1.0 eq) sequentially at 0 °C under nitrogen atmosphere. The mixture was stirred at room temperature for 1 h. To the reaction mixture was added a solution of **26-3** (600 mg, 0.452 mmol, 1.0 eq) in DMF (5 mL), and the mixture was stirred at room temperature for 1 h. When LCMS indicated the completion of the reaction, the reaction mixture was directly used in the next step without posttreatment.

Synthesis of compound **26-5**

To a solution of **26-4** (500 mg, 0.253 mmol, 1.0 eq) in DMF (15 mL) was added DEA (370 mg, 5.06 mmol, 20.0 eq) at room temperature under nitrogen atmosphere, and the reaction mixture was stirred at room temperature for 1 h. When LCMS indicated the completion of the reaction, the reaction mixture was purified on a C18 column (ACN/TFA 0.05% $H_2O$) to give a product **26-5** (white solid, 350 mg, 78% yield).

Synthesis of compound **26**

To a solution of **101B13** (686 mg, 0.29 mmol, 1.0 eq, based on the polymer structural unit), **26-5** (800 mg, 0.435 mmol, 1.5 eq), and PyBOP (227 mg, 0.435 mmol, 1.5 eq) in DMF (8 mL) was added DIPEA (150 mg, 1.16 mmol, 4.0 eq) at room temperature under nitrogen atmosphere. The mixture was stirred at room temperature for 16 h. The reaction mixture was concentrated by rotavap using an oil pump, and methyl *tert*-butyl ether was added. The mixture was stirred for 20 min, and the supernatant was discarded. The oily substance at the bottom of the flask was dissolved in methanol, purified by ultrafiltration (30K MW), and lyophilized to give **26** (white solid, 384 mg, 98% yield).

Example 40: Synthesis of Compound 27

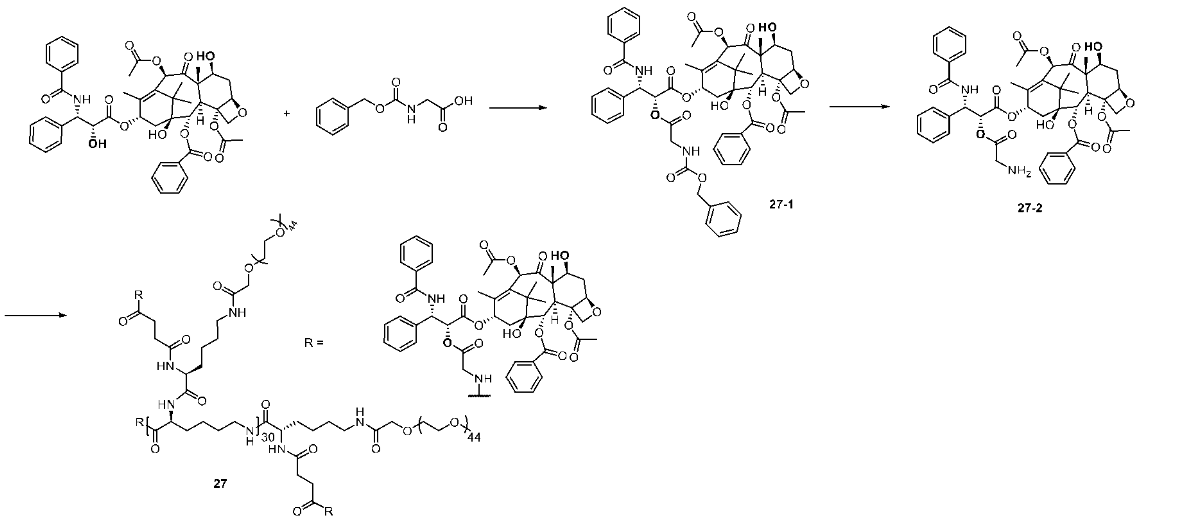
27-1
27-2
27

Synthesis of compound **27-1**

To a solution of paclitaxel (2 g, 2.34 mmol) in DCM (40 mL) was added EDCI (449 mg, 2.34 mmol). The mixture was stirred for 25 min. Subsequently, DMAP (343 mg, 2.81 mmol) was added, and the mixture was stirred for 5 min, and *N*-benzyloxycarbonyl-glycine (735 mg, 3.51 mmol) was added. The mixture was stirred at room temperature for 16 h. The reaction mixture was concentrated, purified by C18 reversed-phase chromatography, and lyophilized to give **27-1** (2.1 g, 86% yield, white solid).

MS (ESI), m/z, 1045.0 $[M+H]^+$.

Synthesis of compound **27-2**

Compound **27-1** (1.1 g, 1.05 mmol) was added to THF (28 mL) and MeOH (1.1 mL), and MsOH (93 mg, 0.97

mmol) and 10% Pd/C (396 mg) were added sequentially. The mixture was purged with hydrogen thrice and stirred for 16 h under hydrogen atmosphere. After the reaction was completed, the mixture was filtered, concentrated to 3 mL, triturated with n-heptane (100 mL), and filtered, and the resulting cake was purified by reversed-phase chromatography to give **27-2** (1 g, 100% yield, white solid).

MS (ESI), m/z,911.0 [M+H]+.

Synthesis of compound **27**

To DMF (10 mL) was added compound **101B13** (1000 mg, 0.42 mmol, based on the polymer structural unit), and **27-2** (710 mg, 0.63 mmol), PyBOP (330 mg, 0.63 mmol), and DIPEA (295 μL, 1.69 mmol) were then added sequentially. The mixture was stirred at room temperature for 2 h. MTBE (100 mL) was added, and the supernatant was removed. The oil was dissolved in methanol, purified by ultrafiltration (30K MW), and lyophilized to give **27** (1.3 g, 93% yield, 100% purity, and about 25.0% paclitaxel content).

Example 41: Synthesis of Compound 28a

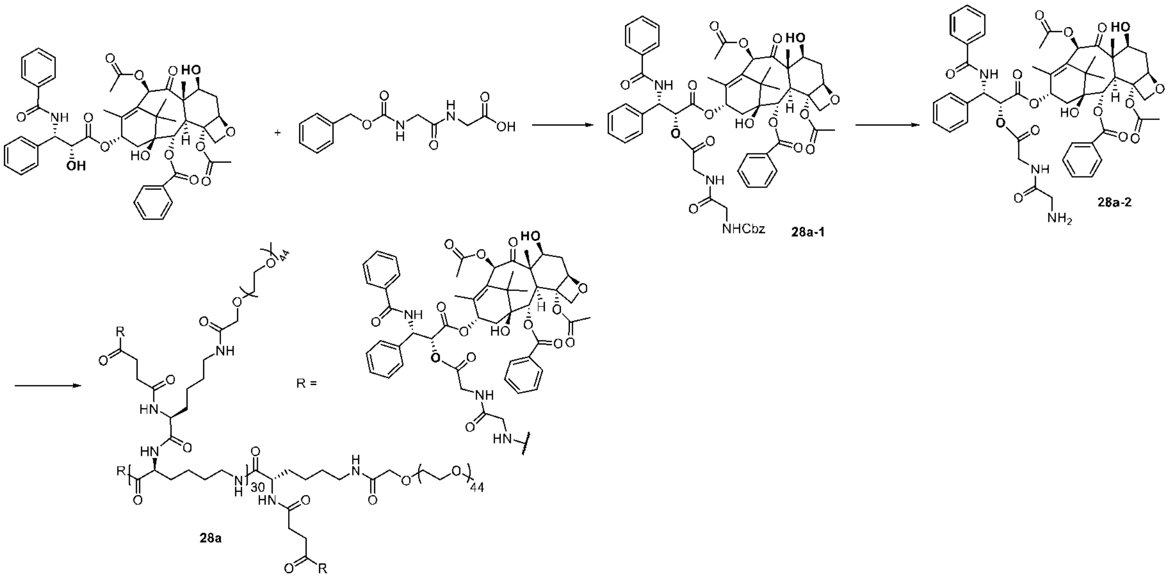

28a-1

28a-2

R =

28a

Synthesis of compound **28a-1**

To a solution of paclitaxel (1.5 g, 1.76 mmol) in DCM (30 mL) was added EDCI (337 mg, 1.76 mmol). The mixture was stirred for 25 min. Subsequently, DMAP (257 mg, 2.11 mmol) was added, and the mixture was stirred for 5 min, and *N*-benzyloxycarbonyl-glycyl-glycine (702 mg, 2.63 mmol) was added. The mixture was stirred at room temperature for 16 h. The reaction mixture was concentrated, purified by C18 reversed-phase chromatography, and lyophilized to give **28a-1** (1.3 g, 67% yield, white solid).

Synthesis of compound **28a-2**

Compound **28a-1** (1.3 g, 1.18 mmol) was added to THF (33 mL), and MsOH (104 mg, 1.09 mmol) and 10% Pd/C (468 mg) were added sequentially, and then MeOH (1.3 mL) was added. The mixture was purged with hydrogen thrice and stirred for 16 h under hydrogen atmosphere. After the reaction was completed, the mixture was filtered, concentrated to 3 mL, triturated with n-heptane (80 mL), and filtered, and the resulting cake was purified by reversed-phase chromatography to give **28a-2** (1.1 g, 96% yield, white solid).

MS (ESI), m/z,968.0 [M+H]+.

Synthesis of compound **28a**

To DMF (10 mL) was added compound **101B13** (1000 mg, 0.42 mmol, based on the polymer structural unit), and

**28a-2** (610 mg, 0.63 mmol), PyBOP (330 mg, 0.63 mmol), and DIPEA (295 µL, 1.69 mmol) were then added sequentially. The mixture was stirred at room temperature for 2 h. MTBE (100 mL) was added, and the supernatant was removed. The oil was dissolved in methanol, purified by ultrafiltration, and lyophilized to give **28a** (1.3 g, 93% yield, about 24.6% paclitaxel content).

Examples 42 to 47: Synthesis of Compounds 28b-g

The synthesis was conducted according to the synthetic method in Example 41 based on different starting materials

| No. | Starting material | Compound | Purity and drug content |
|---|---|---|---|
| Example **42**<br>Compound **28b** | 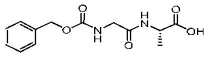<br>Paclitaxel | 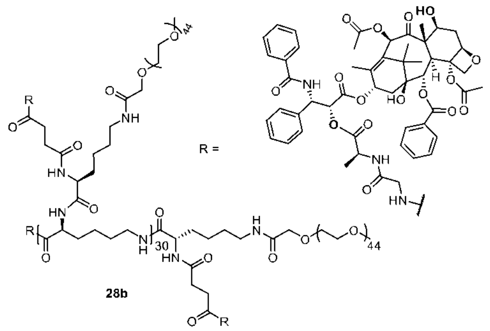<br>28b | 99%<br>25.1% |
| Example **43**<br>Compound **28c** | 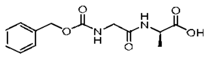<br>Paclitaxel | 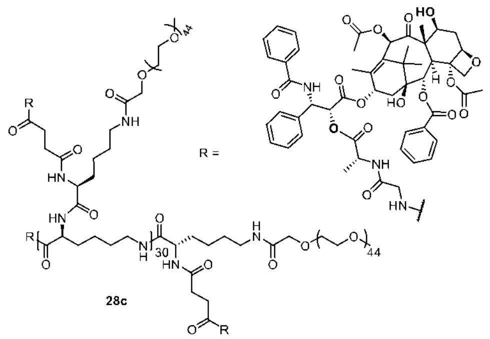<br>28c | 100%<br>24.3% |
| Example **44**<br>Compound **28d** | 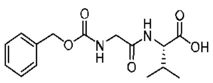<br>Paclitaxel | 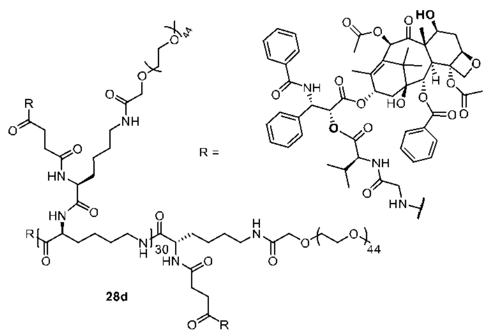<br>28d | 99%<br>24.9% |
| Example **45**<br>Compound **28e** | 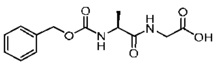<br>Paclitaxel | 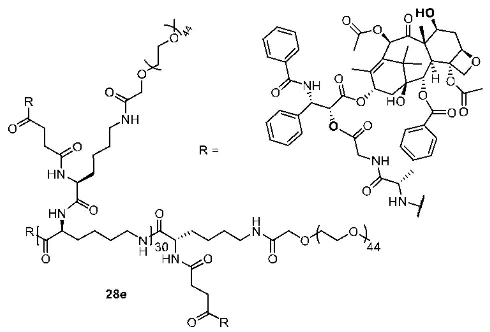<br>28e | 98.7%<br>23.3% |

(continued)

| No. | Starting material | Compound | Purity and drug content |
|---|---|---|---|
| Example **46**<br>Compound **28f** | 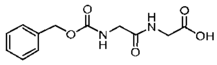<br>Docetaxel | 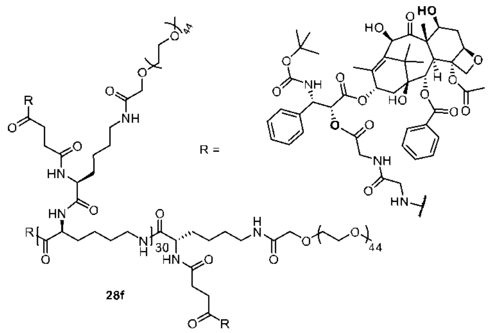<br>28f | 100%<br>24.0% |
| Example **47**<br>Compound **28g** | 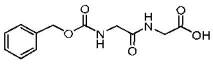<br>Cabazitaxel | 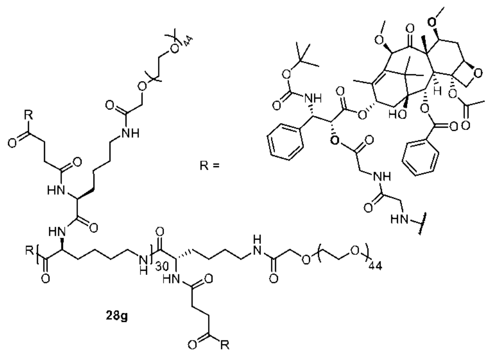<br>28g | 99.2%<br>25.1% |

Example 48: Synthesis of Compound 29

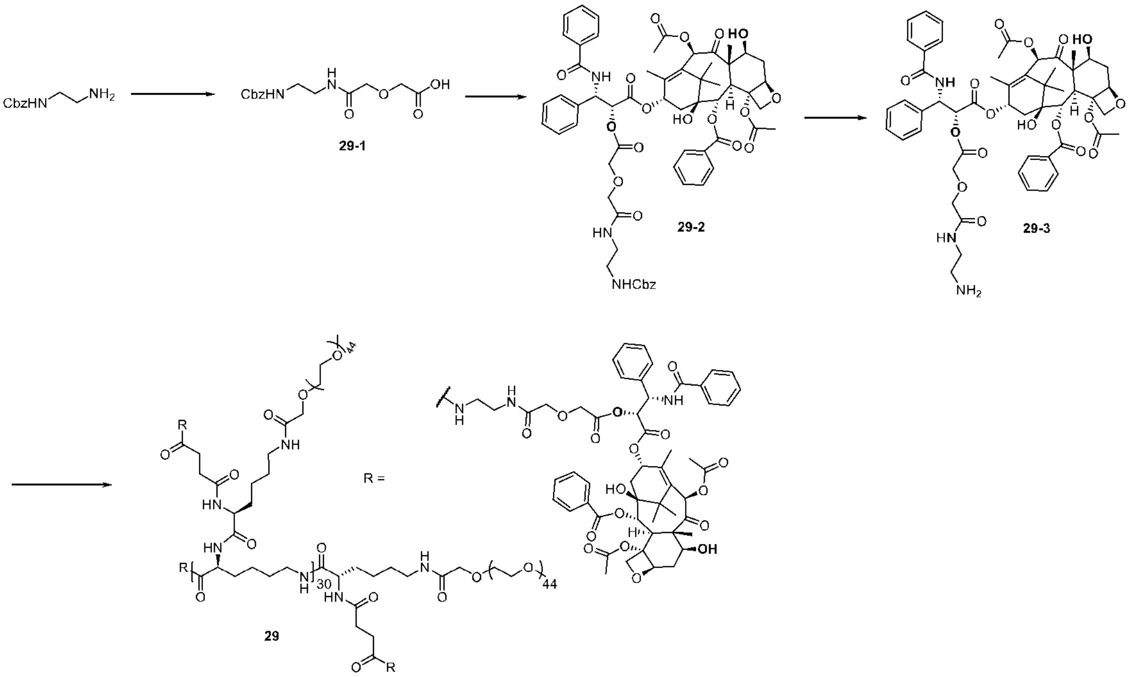

29-1, 29-2, 29-3, 29

Synthesis of compound **29-1**

To a solution of *N*-Cbz-1,2-diaminoethane (1.0 g, 5.1 mmol, 1.0 eq) in dichloromethane (20 mL) were added diglycolic anhydride (900 mg, 7.7 mmol, 1.5 eq) and DMAP (1.2 g, 10.2 mmol, 2.0 eq) at room temperature under nitrogen

atmosphere, and the mixture was stirred overnight. After the reaction was completed, the mixture was directly concentrated, and purified by Pre-HPLC to give a white solid (1.2 g, 75.9% yield).

MS (ESI), m/z, 311.2 $[M+H]^+$.

Synthesis of compound **29-2**

To a solution of **29-1** (1.0 g, 3.22 mmol, 1.0 eq) and DMAP (780 mg, 6.44 mmol) in DCM (50 mL) was added paclitaxel (2.7 g, 3.22 mmol) at room temperature (25 °C) under nitrogen atmosphere, and the mixture was stirred overnight. After the reaction was completed, the mixture was purified by column chromatography to give a product (520 mg, 15% yield).

MS (ESI), m/z, 1146.2 $[M+H]^+$.

Synthesis of compound **29-3**

To a solution of **29-2** (0.9 g, 0.45 mmol, 1.0 eq) and 10% Pd/C (10 mg) in MeOH (20 mL) was added TFA (1 mL) at room temperature (25 °C) under hydrogen atmosphere, and the mixture was stirred overnight. After the reaction was completed, the mixture was purified by normal-phase column chromatography to give a product (700 mg, 88% yield).

MS (ESI), m/z, 1012.2 $[M+H]^+$.

Synthesis of compound **29**

To a solution of **29-3** (0.15 g, 0.14 mmol, 1.5 eq) in DMF (10 mL) were added **101B13** (0.233 g, 0.098 mmol, 1.0 eq, based on the polymer structural unit), DIPEA (0.2 mL), and PyBOP (0.073 g, 0.14 mmol) at room temperature (25 °C) under nitrogen atmosphere, and the reaction mixture was stirred overnight, concentrated, ultrafiltered (30K MW), and lyophilized to give a product (320 mg, 98% purity, and 21% paclitaxel content as detected by HPLC).

Example 49: Synthesis of Compound 30

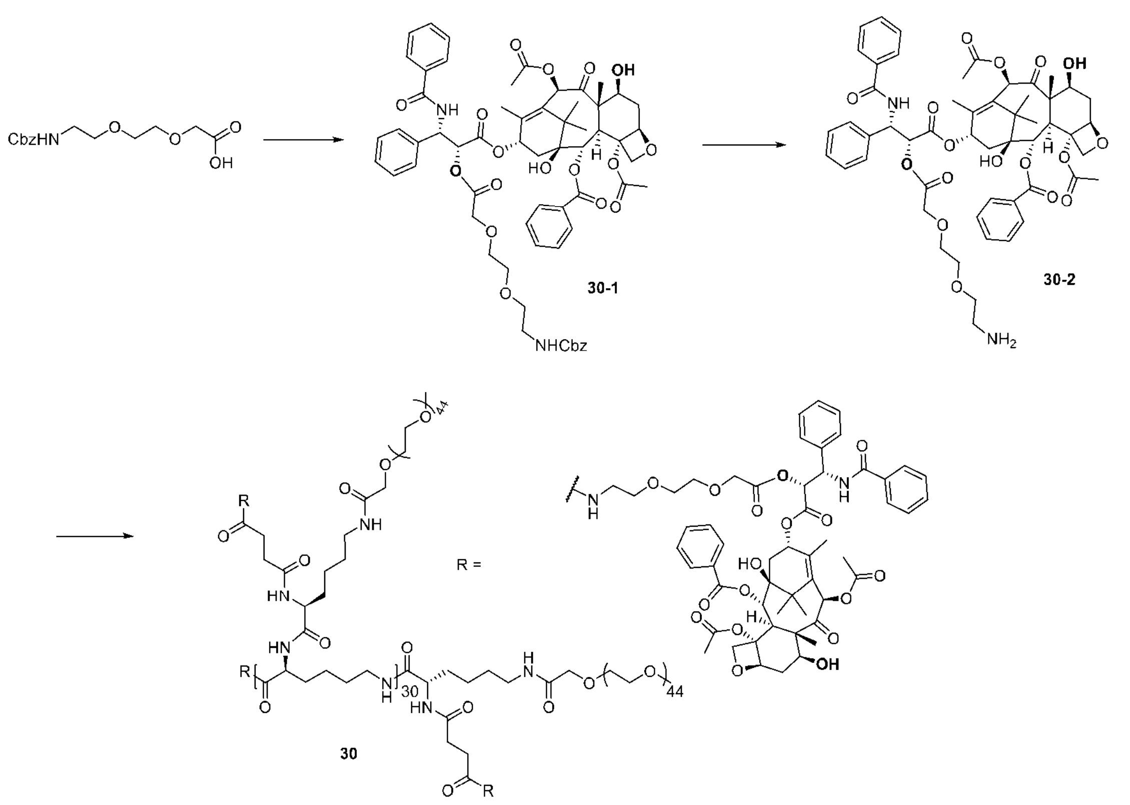

**30-1**

**30-2**

R =

**30**

Synthesis of compound **30-1**

To a solution of paclitaxel (1.5 g, 1.76 mmol) in DCM (30 mL) was added EDCI (337 mg, 1.76 mmol). The mixture was stirred for 25 min. Subsequently, DMAP (257 mg, 2.11 mmol) was added, and the mixture was stirred for 5 min, and 8-benzyloxycarbonylamino-3,6-dioxaoctanoic acid (899 mg, 2.63 mmol) was added. The mixture was stirred at room temperature for 16 h. The reaction mixture was concentrated, purified by C18 reversed-phase chromatography, and lyophilized to give **30-1** (1.3 g, 68% yield, white solid).
MS (ESI), m/z, 1101.80 $[M+H]^+$.

Synthesis of compound **30-2**

Compound **30-1** (1.36 g, 1.20 mmol) was added to THF (34 mL) and MeOH (1.3 mL), and MsOH (72 μL, 1.1 mmol) and 10% Pd/C (489 mg) were added sequentially. The mixture was purged with hydrogen thrice and stirred for 16 h under hydrogen atmosphere. After the reaction was completed, the mixture was filtered, concentrated to 5 mL, triturated with n-heptane (80 mL), and filtered, and the resulting cake was purified by reversed-phase chromatography to give **30-2** (1.1 g, 92% yield, white solid).
MS (ESI), m/z 999.0 $[M+H]^+$.

Synthesis of compound **30**

To DMF (10 mL) was added compound **101B13** (1000 mg, 0.42 mmol, based on the polymer structural unit), and **30-2** (630 mg, 0.63 mmol), PyBOP (330 mg, 0.63 mmol), and DIPEA (295 μL, 1.69 mmol) were then added sequentially. The mixture was stirred at room temperature for 2 h. MTBE (100 mL) was added, and the supernatant was removed. The oil was dissolved in methanol, purified by ultrafiltration, and lyophilized to give compound **30** (1.2 g, 86% yield, about 25.1% paclitaxel content).

Example 50: Synthesis of Compound 31

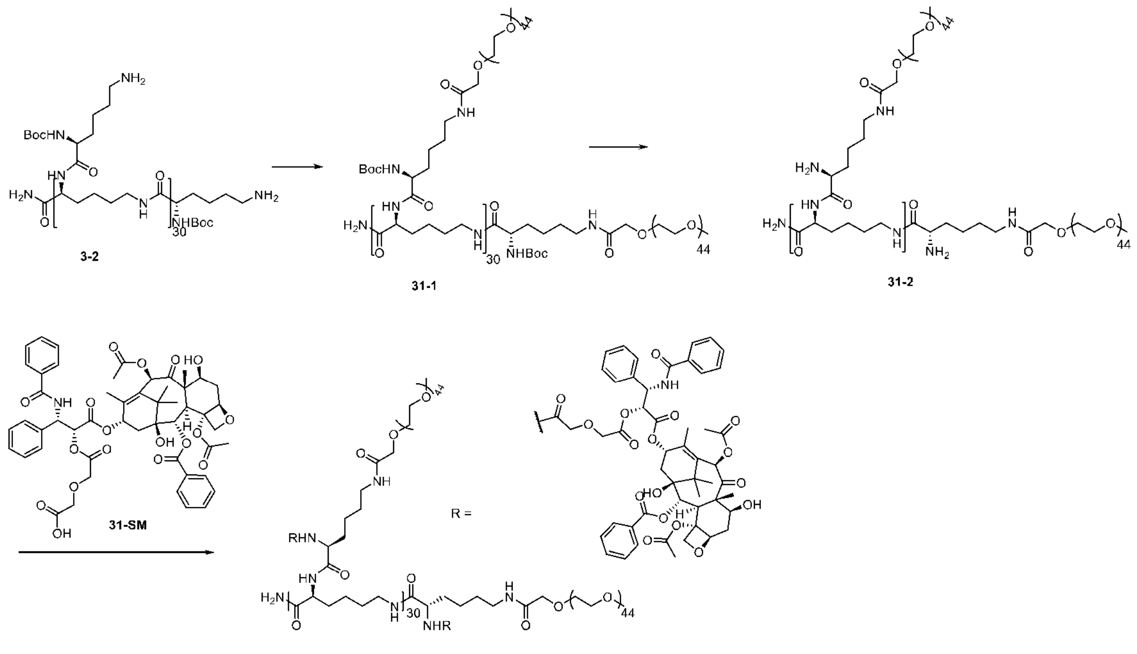

3-2

31-1

31-2

31-SM

31

R =

Synthesis of compound **31-1**

To a solution of **3-2** (1.5 g, 3.62 mmol, 1.0 eq) in methanol/acetonitrile (40 mL/40 mL) was added a solution of $mPEG_{2k}$-NHS (9.9 g, 4.70 mmol, 1.3 eq) in methanol (20 mL) dropwise at room temperature. DIPEA (2.34 g, 18.1 mmol, 5.0 eq) was then added, and the mixture was stirred at room temperature overnight. After the reaction was completed, the

reaction mixture was concentrated by rotavap and triturated with MTBE (100 mL) at room temperature to give a crude product (11 g), which was directly used in the next step without purification.

Synthesis of compound **31-2**

To a solution of **31-1** (11.0 g, crude) in DCM (20 mL) was added TFA (20 mL), and the mixture was stirred at room temperature overnight. The reaction mixture was concentrated by rotavap, and TFA was completely removed using an oil pump. The mixture was then ultrafiltered through an ultrafiltration membrane (30K MW), with MeOH/$H_2O$ = 1:1 as the solvent system. The pure product solution was lyophilized to give a dry product (7.0 g).

Synthesis of compound **31**

To a solution of **31-2** (1.0 g, 0.423 mmol, 1.0 eq, based on the polymer structural unit) in DMF (10 mL) were added **31-SM** (synthesized according to the method in Bioconjugate Chem. 2012, 23, 8, 1610-1622, 493 mg, 0.508 mmol, 1.2 eq), PyBOP (0.634 mmol, 1.5 eq), and DIPEA (164 mg, 1.269 mmol, 3.0 eq) sequentially, and the mixture was stirred at room temperature overnight. When HPLC indicated the completion of the reaction, the reaction mixture was concentrated in high vacuum to half the original volume and purified by ultrafiltration (30K MW), and the pure product solution was lyophilized to give a product (900 mg, 98% purity, 21.1% paclitaxel content as detected by HPLC).

Example 51: Synthesis of Compound 32

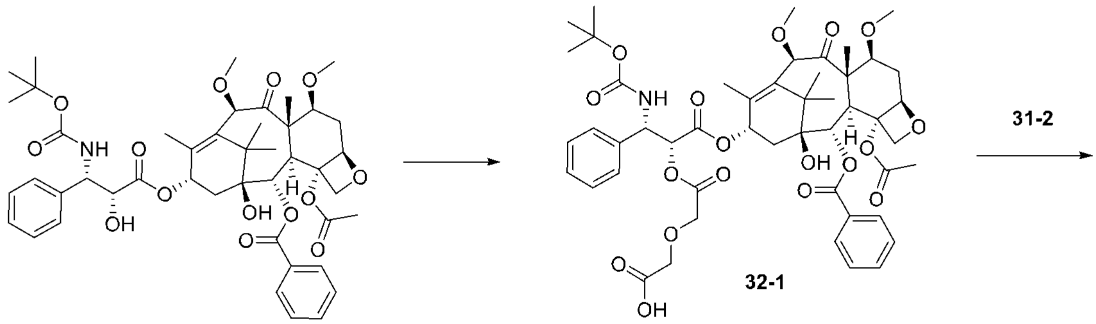


Cabazitaxel

32-1

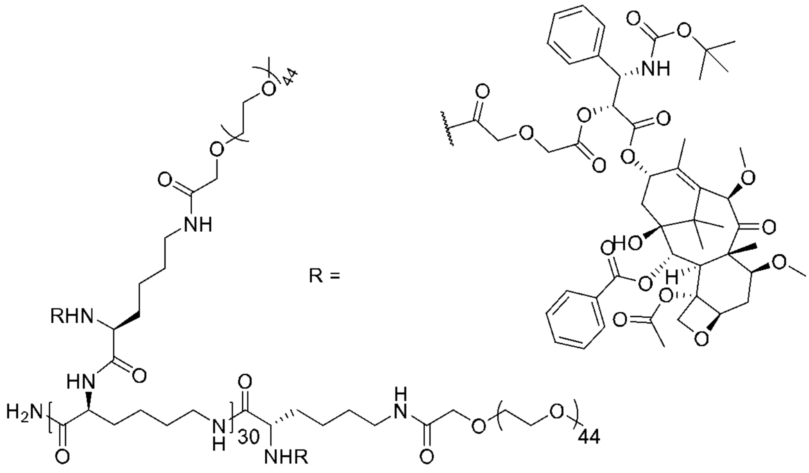

R =

32

Synthesis of compound **32-1**

To a solution of cabazitaxel (700 mg, 0.837 mmol, 1.0 eq) in DCM (8.0 mL) were added diglycolic anhydride (292 mg, 2.512 mmol, 3.0 eq), DMAP (409 mg, 3.35 mmol, 4.0 eq), and DIPEA (432 mg, 3.35 mmol, 4.0 eq) sequentially at 25

°C, and the mixture was stirred for 4 h. After the reaction was completed, the reaction mixture was transferred to a separatory funnel and subjected to acid washing using 0.2 M aqueous citric acid solution (50 mL × 3). The phases were separated. The organic phase was washed with water and concentrated by rotavap, and the resulting crude product was purified on a C18 column with $CH_3CN$:$H_2O$ (0.05% TFA) = 5-95% as the eluent to give a purified product (720 mg).

MS (ESI), m/z, 952.00 $[M+H]^+$.

Synthesis of compound **32**

To a solution of **32-1** (500 mg, 0.525 mmol, 1.5 eq) in DMF (5.0 mL) were added **31-2** (827 mg, 0.350 mmol, 1.0 eq, based on the polymer unit), PyBOP (273 mg, 0.525 mmol, 1.5 eq), and DIPEA (135 mg, 1.05 mmol, 3.0 eq) sequentially, and the mixture was stirred at room temperature overnight. When HPLC indicated the completion of the reaction, the reaction mixture was concentrated in high vacuum to half the original volume and purified by ultrafiltration (30K MW), and the pure product solution was lyophilized to give a product (750 mg, 98% purity, 26.0% cabazitaxel content as detected by HPLC).

Example 52: Synthesis of Compound 33a

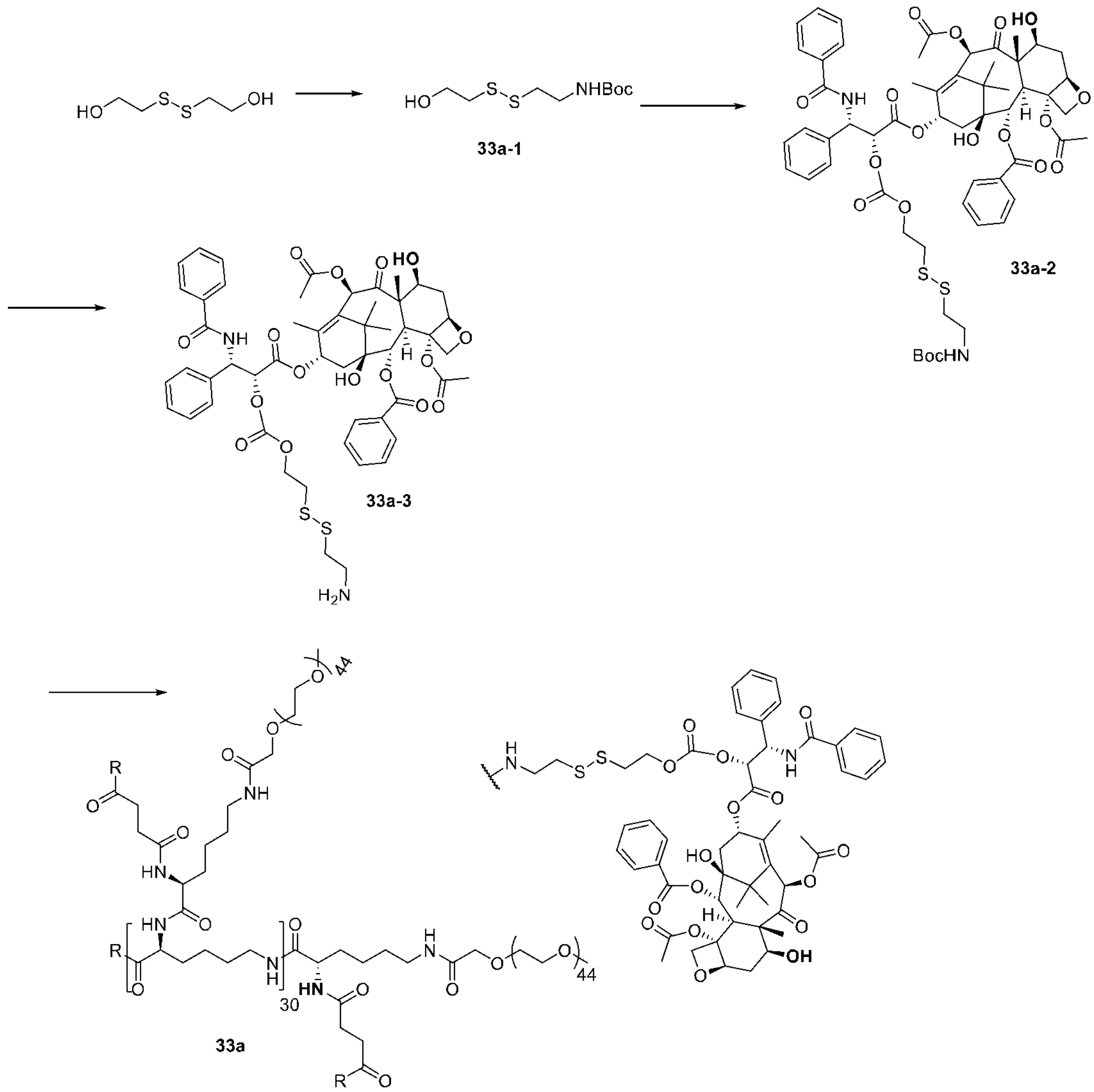

33a-1

33a-2

33a-3

33a

Synthesis of compound **33a-1**

Cysteamine hydrochloride (1.50 g, 13.2 mmol, 1.0 eq) was dissolved in methanol (18 mL) at room temperature.

The mixture was cooled to 0 °C under nitrogen atmosphere, and triethylamine (1.85 mL, 26.4 mmol, 2.0 eq) and a solution of 2-hydroxyethyl disulfide (1.50 g, 13.2 mmol, 1.0 eq) in dichloromethane were added separately. The mixture was stirred at room temperature for 16 h. When LCMS indicated the completion of the reaction, The reaction mixture was cooled to 0 °C, and di-tert-butyl dicarbonate (4.32 g, 19.8 mmol, 1.5 eq) was added. The mixture was stirred at room temperature for 4 h. The reaction mixture was mixed with silicon dioxide (100-200 mesh) and separated by normal phase chromatography (petroleum ether:ethyl acetate = 3:1) to give a compound **33a-1** (colorless oil, 0.9 g, 26.9% yield).

MS (ESI), m/z, 154.0 $[M-100]^+$.

Synthesis of compound **33a-2**

To a solution of **33a-1** (300 mg, 1.18 mmol, 1.0 eq) in dichloromethane (20 mL) were added triethylamine (120 mg, 1.18 mmol, 1.0 eq) and bis(4-nitrophenyl) carbonate (360 mg, 1.18 mmol, 1.0 eq) separately at room temperature under nitrogen atmosphere. The mixture was stirred at room temperature for 16 h. 4-Dimethylaminopyridine (144.6 mg, 1.18 mmol, 1.0 eq) and paclitaxel (1010 mg, 1.18 mmol, 1.0 eq) were then added. The mixture was stirred at room temperature for 2 h. When LCMS detection indicated the termination of the reaction, the mixture was dried *in vacuo,* separated by reversed-phase chromatography (A = TFA (0.1% + $H_2O$), B = acetonitrile), and lyophilized to give a compound **33a-2** (white solid, 0.8 g, 59.7% yield).

MS (ESI), m/z, 1133.0 $[M+H]^+$.

Synthesis of compound **33a-3**

**33a-2** (800 mg, 0.706 mmol, 1.0 eq) was dissolved in formic acid (10 mL) at room temperature. The mixture was stirred at room temperature for 2 h under nitrogen atmosphere. When LCMS monitoring indicated the completion of the reaction, the mixture was concentrated, separated by reversed-phase chromatography (A = TFA (0.1% + $H_2O$), B = acetonitrile), and lyophilized to give a compound **33a-3** (white solid, 0.4 g, 54.8% yield).

MS (ESI), m/z, 1033.0 $[M+H]^+$.

Synthesis of compound **33a**

To a solution of **101B13** (1220 mg, 0.516 mmol, 1.0 eq, based on the polymer unit) in DMF (20 mL) were added **33a-3** (800 mg, 0.774 mmol, 1.5 eq), PyBOP (403 mg, 0.774 mmol, 1.5 eq), and DIPEA (266 mg, 2.06 mmol, 4 eq) at room temperature under nitrogen atmosphere. The mixture was stirred at 20 °C for 16 h. The reaction mixture was concentrated, ultrafiltered (30K MW), and lyophilized to give compound **33a** (white solid, 1.5 g, 85.7% yield, 100% purity, 24.7% paclitaxel content as detected by HPLC).

Examples 53 to 66: Synthesis of Compounds 33b-o

The synthesis was conducted according to the synthetic method in Example **52** based on different starting materials

| No. | Starting material | Compound | Purity and drug content |
|---|---|---|---|
| Example **53** | 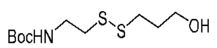 | 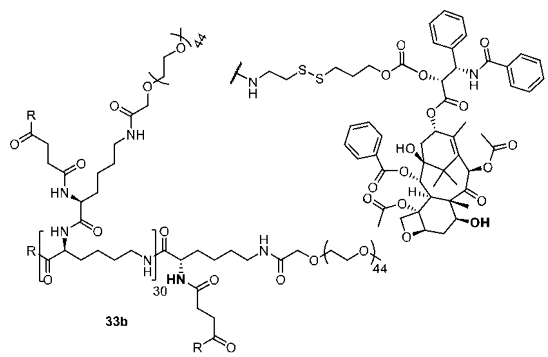 33b | 100% |
| Compound **33b** | Paclitaxel | | 24.5% |

(continued)

| No. | Starting material | Compound | Purity and drug content |
|---|---|---|---|
| Example **54**<br><br>Compound **33c** | 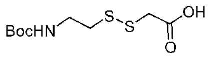<br><br>Paclitaxel | 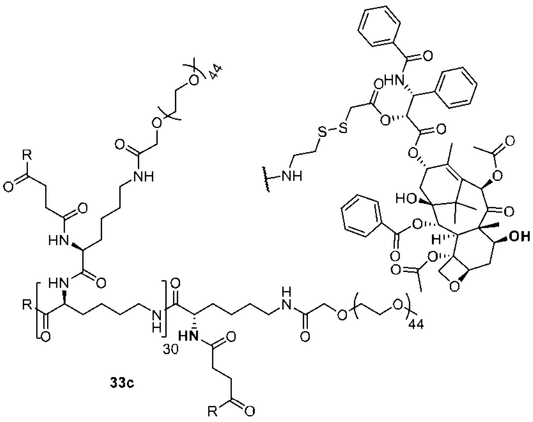<br>**33c** | 97.8%<br><br>26.0% |
| Example **55**<br><br>Compound **33d** | 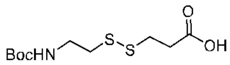<br><br>Paclitaxel | 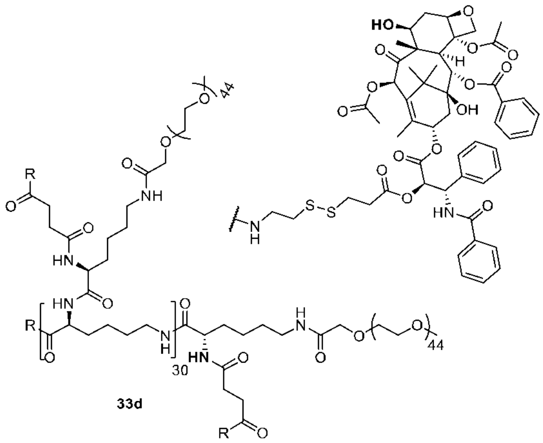<br>**33d** | 98.8%<br><br>23.2% |
| Example **56**<br><br>Compound **33e** | 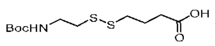<br><br>Paclitaxel | 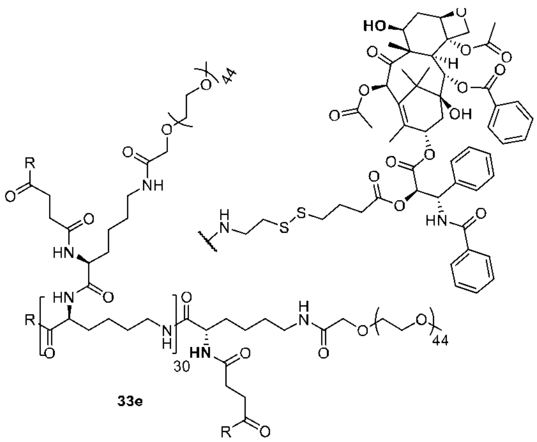<br>**33e** | 99.2%<br><br>24.6% |

(continued)

| No. | Starting material | Compound | Purity and drug content |
|---|---|---|---|
| Example **57**<br>Compound **33f** | 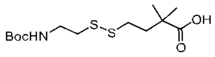<br>Paclitaxel | 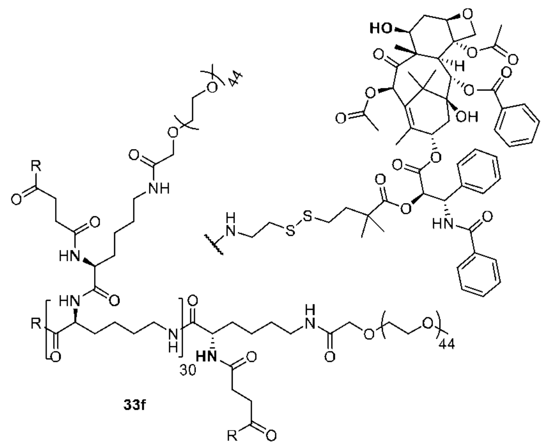<br>33f | 100%<br>25.6% |
| Example **58**<br>Compound **33g** | 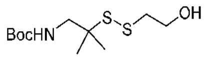<br>Paclitaxel | 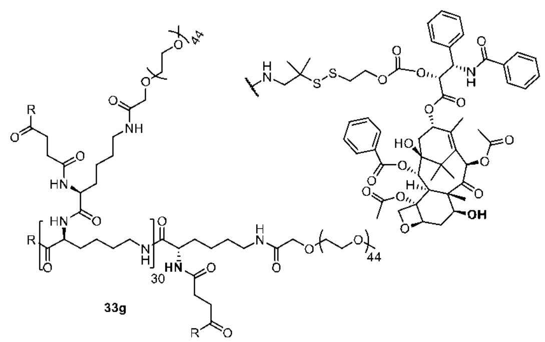<br>33g | 99.3%<br>24.7% |
| Example **59**<br>Compound **33g** | 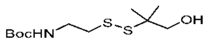<br>Paclitaxel | 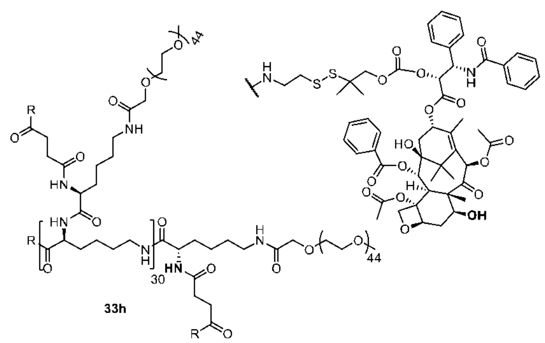<br>33h | 98.5%<br>25.2% |
| Example **60**<br>Compound **33i** | 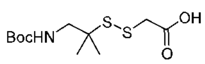<br>Paclitaxel | 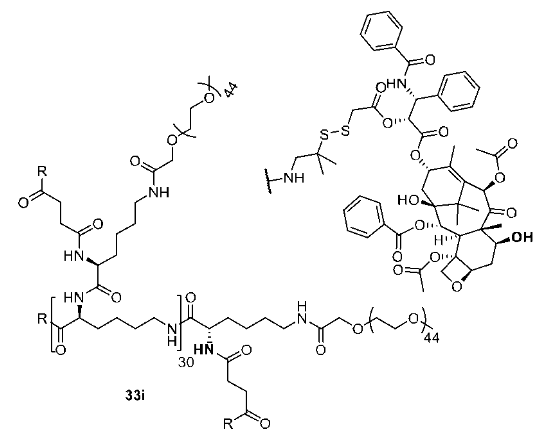<br>33i | 99.6%<br>23.2% |

(continued)

| No. | Starting material | Compound | Purity and drug content |
|---|---|---|---|
| Example **61**<br><br>Compound **33j** | 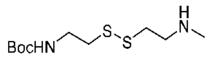<br><br>Paclitaxel | 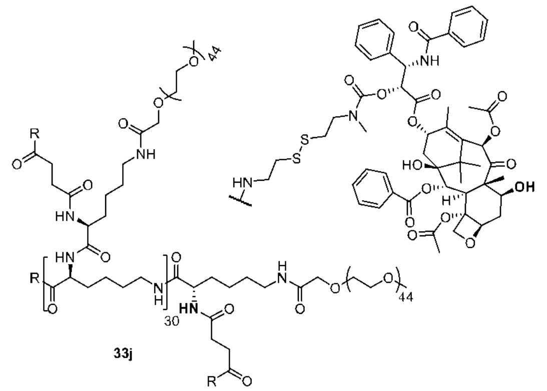<br>33j | 99.0%<br><br>22.3% |
| Example **62**<br><br>Compound **33k** | 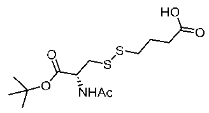<br><br>Paclitaxel | 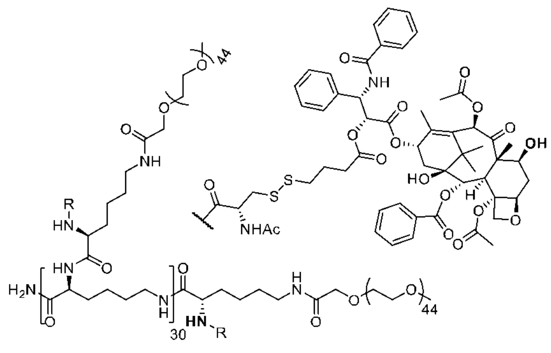<br>33k | 98.7%<br><br>24.1% |
| Example **63**<br><br>Compound **33l** | 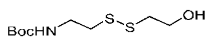<br><br>Docetaxel | 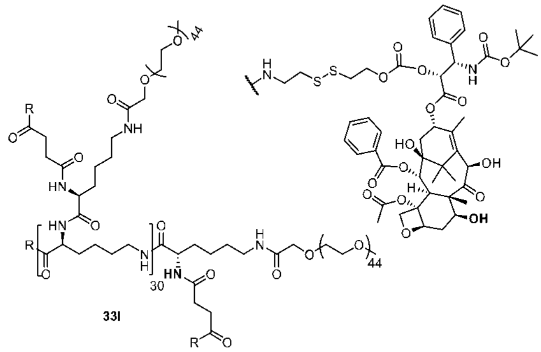<br>33l | 99.7%<br><br>24.7% |
| Example **64**<br><br>Compound **33m** | 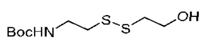<br><br>Cabazitaxel | 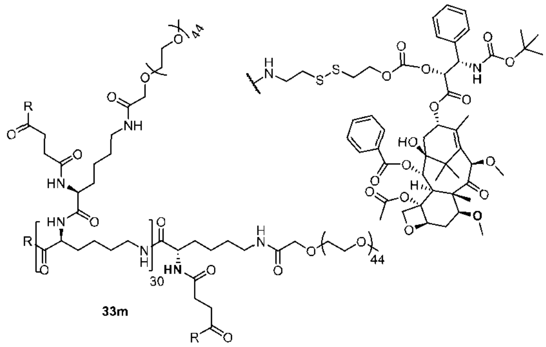<br>33m | 99.3%<br><br>25.2% |
| Example **65** | 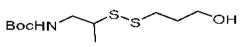 | | 100% |

(continued)

| No. | Starting material | Compound | Purity and drug content |
|---|---|---|---|
| Compound **33n** | Paclitaxel | 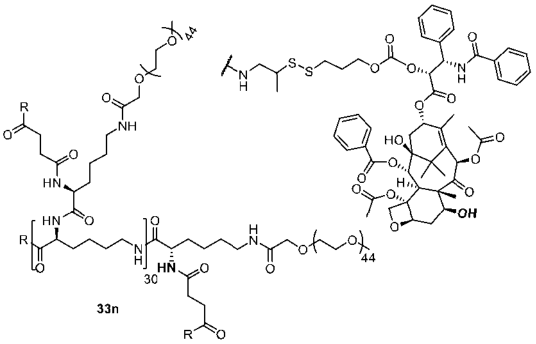 33n | 23.6% |
| Example **66**<br>Compound **33o** | 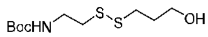<br>Paclitaxel | 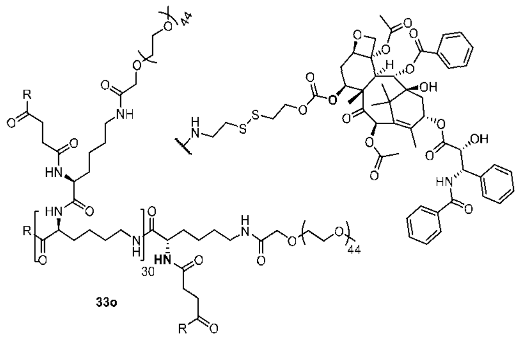 33o | 99%<br>24.8% |

Example 67: Synthesis of Compound 34

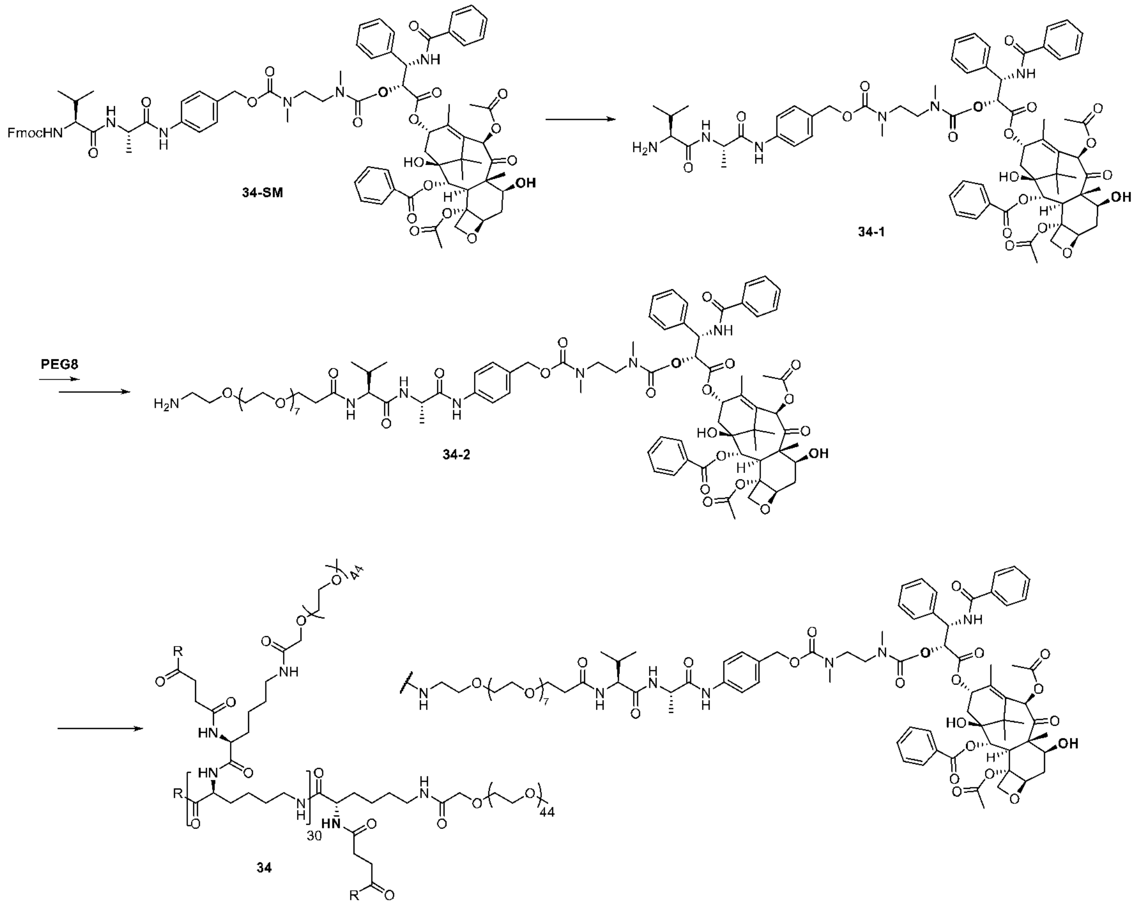


34-SM

34-1

34-2

34

Synthesis of compound **34-1**

To a solution of **33-SM** (prepared according to the compound synthesis method in Eur. J. Org. Chem. 2021, 2383-2387, 1.02 g, 0.676 mmol, 1.0 eq) in DMF (6 mL) was added diethylamine (1.5 mL) at room temperature under nitrogen atmosphere, and the mixture was stirred for 2 h with the temperature maintained. After the reaction was completed, the reaction mixture was concentrated, and directly purified by reversed-phase column chromatography ($CH_3CN$:$H_2O$ = 5-95%). The collected eluate was concentrated by rotavap to give a purified product (680 mg, white solid).

MS (ESI), m/z, 1287.80 $[M+H]^+$.

Synthesis of compound **34-2**

To a solution of **34-1** (680 mg, 0.529 mmol, 1.0 eq) in DMF (5.0 mL) were added **PEG8** (351 mg, 0.529 mmol, 1.0 eq), PyBOP (413 mg, 0.794 mmol, 1.5 eq), and DIPEA (205 mg, 1.588 mmol, 3.0 eq) sequentially at room temperature under nitrogen atmosphere. After the addition, the mixture was stirred at room temperature for 2 h. After the reaction was completed, to the reaction mixture was added diethylamine (1.5 mL) directly, and the mixture was stirred for 2 h. After the reaction was completed, the reaction mixture was purified on a C18 reversed-phase column. The impurities were firstly eluted with an eluent system $CH_3CN$:$H_2O$ (0.05% $NH_3.H_2O$) = 5-95% to 95%-95%, and the target product was then eluted with $CH_3CN$ (0.05% TFA):$H_2O$ (0.05% TFA) = 95%-95%. The product solution was collected and concentrated by rotavap to give a purified white solid product (1.14 g).

MS (ESI), m/z, 1711.10 $[M+H]^+$.

Synthesis of compound **34**

To a solution of **34-2** (1.14 g, 0.63 mmol, 1.5 eq) in DMF (8.0 mL) were added **101B13** (994 mg, 0.42 mmol, 1.0 eq, based on the polymer unit), PyBOP (328 mg, 0.63 mmol, 1.5 eq), and DIPEA (217 mg, 1.26 mmol, 4.0 eq) sequentially at room temperature under nitrogen atmosphere. After the addition, the reaction mixture was returned to room temperature and stirred for 16 h. After the reaction was completed, the reaction mixture was concentrated in high vacuum and purified by ultrafiltration (30K MW), and the pure product solution was lyophilized to give a dry product (810 mg, 98.9% purity,

19.2% paclitaxel content as detected by HPLC).

Example 68: Synthesis of Compound 35

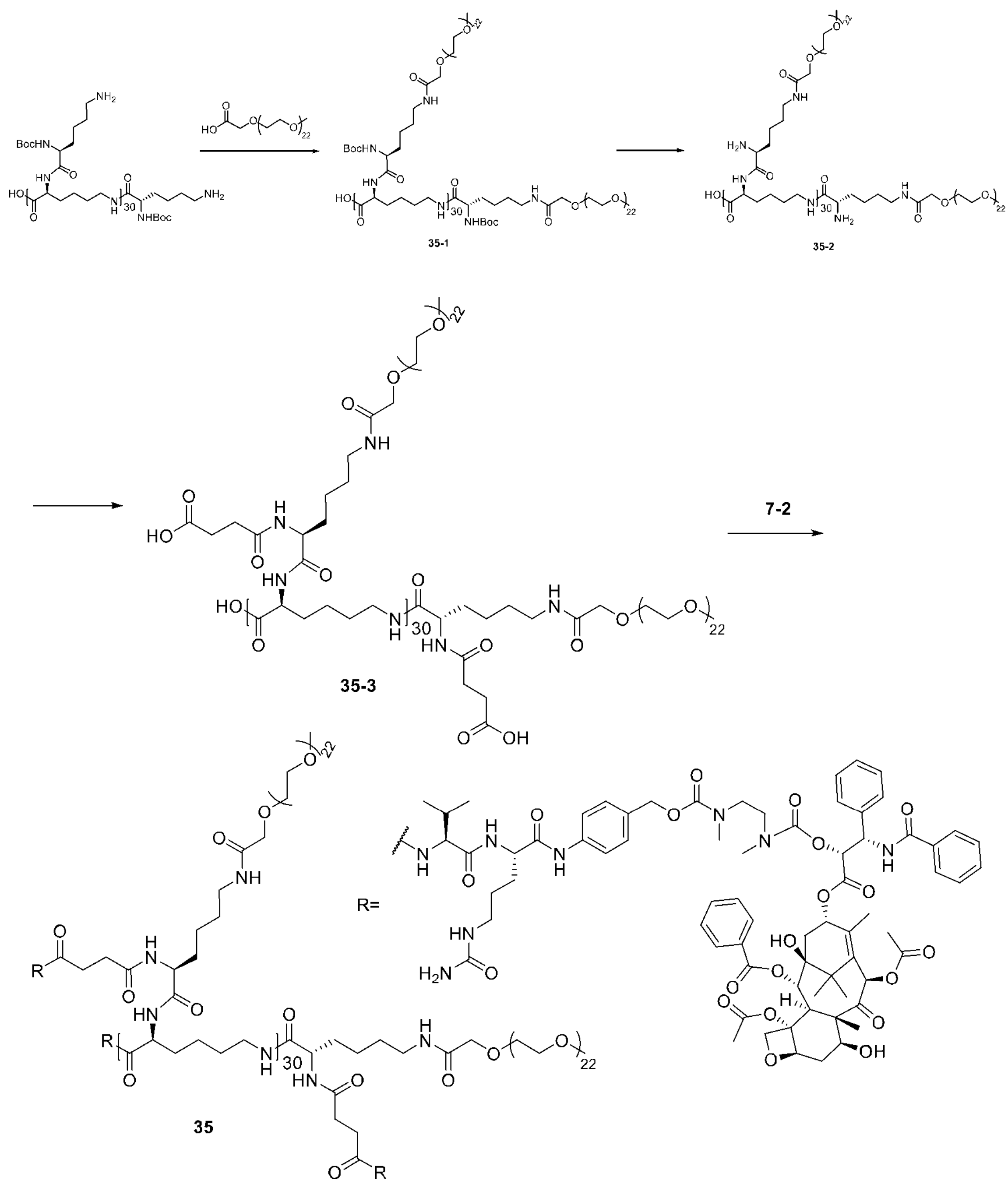


35-1

35-2

35-3

35

Synthesis of compound **35-1**

To a solution of PEG$_{1k}$-CO$_2$H (3.7 g, 3.7 mmol, 1.0 eq) in dichloromethane were added TSTU (1.4 g, 4.4 mmol, 1.2 eq) and DIPEA (1.4 g, 5.5 mmol, 1.5 eq) at room temperature under nitrogen atmosphere. The mixture was stirred for 0.5 h and then concentrated. To a solution of the residue in acetonitrile (30 mL) was added methanol (25 mL). To a solution of the polymer (1.15 g, 2.75 mmol, 1.0 eq) in methanol (30 mL) were added sequentially the above solution and DIPEA (2.2 mL, 12.4 mmol, 4.5 eq). The reaction mixture was stirred at room temperature for 2 h. After the solvent was concentrated by rotavap, the mixture was triturated with MTBE twice and purified to give a product **35-1** (3.6 g, white solid).

Synthesis of compound **35-2**

To a solution of **35-1** (2.6 g) in dry DCM (30 mL) was added TFA (10 mL) at room temperature under nitrogen atmosphere. The reaction mixture was stirred at room temperature for 15 h until LCMS monitoring indicated the depletion of the starting materials. After the reaction mixture was concentrated by rotavap, the mixture was washed with MTBE several times and dried using an oil pump to give an oily product **35-2** (2.9 g, TFA salt).

Synthesis of compound **35-3**

To a solution of **35-2** (2.0 g, 0.05 mmol) in DMF (10 mL) was added DIPEA (750 mg, 5.85 mmol, 128.0 eq) at room temperature (25 °C) under nitrogen atmosphere, and succinic anhydride (200 mg, 2.06 mmol, 45.0 eq) was added. The mixture was stirred for 12 h. When the reaction was substantially completed as detected by ninhydrin assay, MTBE (100 mL) was added, and the mixture was stirred for 10 min. The supernatant was removed, and the procedures were repeated thrice. The residue was dried *in vacuo* to give a product **35-3** (1.5 g).

Synthesis of compound **35**

To a solution of **35-3** (500 mg, 0.01 mmol, 1.0 eq) in dry DMF (10 mL) were added **7-2** (712 mg, 0.52 mmol, 45.0 eq), PyBOP (480 mg, 0.92 mmol, 80.0 eq), and DIPEA (190 mg, 1.48 mmol, 128.0 eq) at room temperature (25 °C) under nitrogen atmosphere. The mixture was then heated to 28 °C, stirred for 12 h, concentrated, purified by ultrafiltration (30K MW), and lyophilized to give a product **35** (870 mg, white solid, 89% yield, 98.9% purity, about 41.3% paclitaxel content).

Example 69: Synthesis of Compound 36a

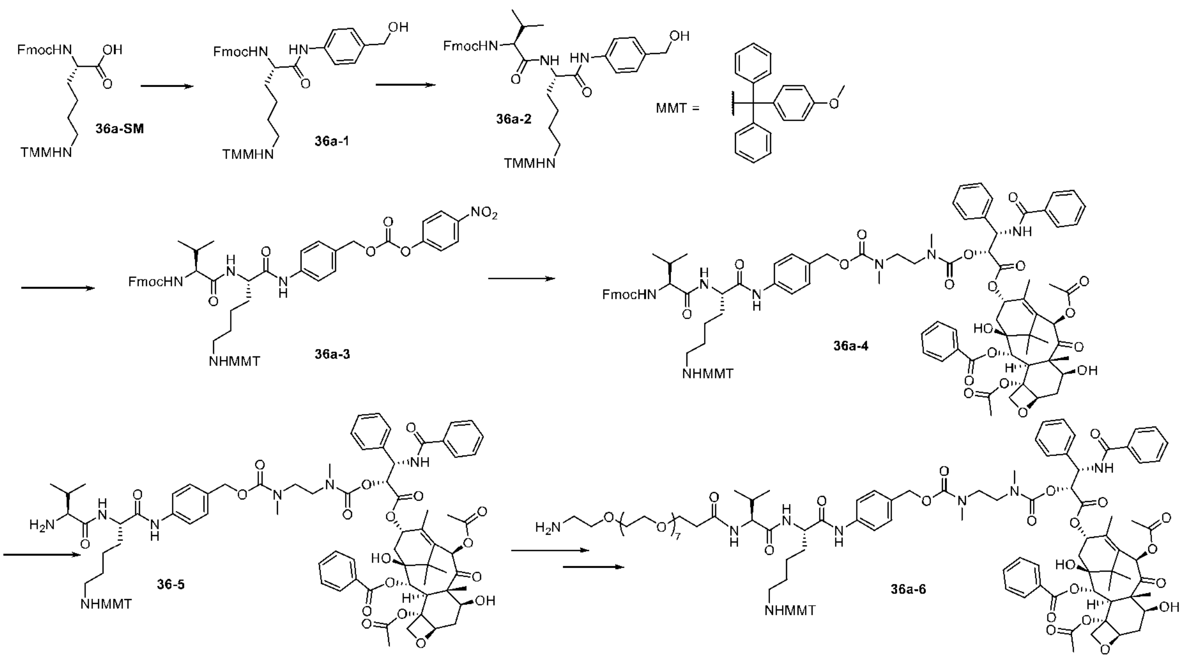

36a-SM

36a-1

36a-2

MMT =

36a-3

36a-4

36-5

36a-6

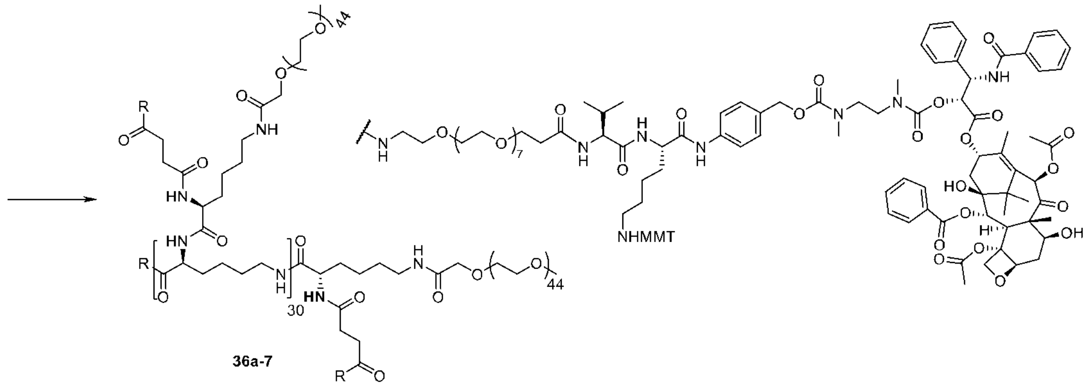

36a-7

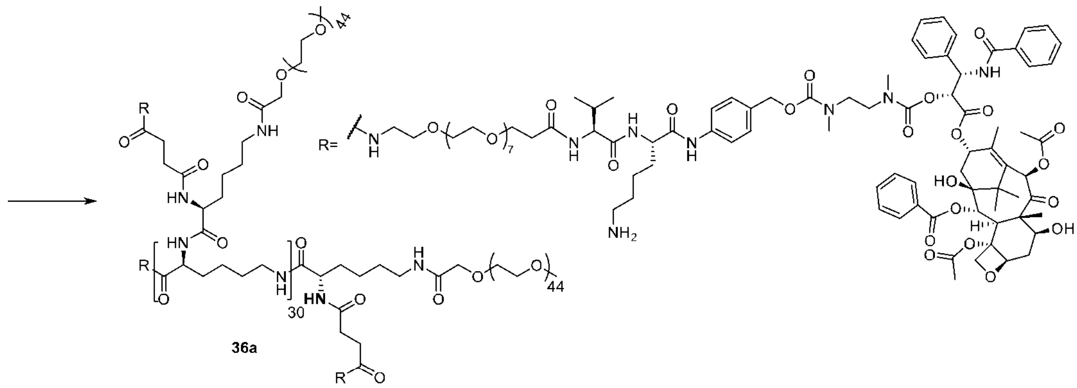

36a

Synthesis of compound **36a-1**

To a solution of compound **36a-SM** (CAS: 159857-60-0, 5.0 g, 6.703 mmol, 1.0 eq) in dichloromethane (50 mL) was added diethylamine (10 mL) at room temperature under nitrogen atmosphere, and the mixture was stirred for 16 h. When LCMS indicated the completion of the reaction, the reaction mixture was concentrated, and purified by column chromatography to give a white solid (3 g, 85.5% yield).

Synthesis of compound **36a-2**

To a solution of compound **36a-1** (0.7 g, 1.34 mmol, 1.0 eq) in dichloromethane (50 mL) were added DIPEA (0.35 g, 2.67 mmol, 2.0 eq) and Fmoc-Val-NHC (0.7 g, 1.604 mmol, 1.2 eq) at room temperature under nitrogen atmosphere. The mixture was stirred at room temperature for 16 h. When LCMS indicated the completion of the reaction, a solid was precipitated. The mixture was filtered *in vacuo* to give a white solid (1 g, 88.5% yield).

Synthesis of compound **36a-3**

To a solution of compound **36a-2** (0.9 g, 1.065 mmol, 1.0 eq) in THF/DMF (10 mL/5 mL) were added pyridine (0.25 g, 3.195 mmol, 3.0 eq) and *p*-nitrophenyl chloroformate (0.65 g, 3.195 mmol) at room temperature under nitrogen atmosphere. The mixture was stirred at room temperature for 16 h. When the reaction was substantially completed as detected by LCMS, to methyl *tert*-butyl ether was added the reaction mixture, and the mixture was filtered to remove the solid and dried *in vacuo* to give a white solid (0.76 g, 72% yield).

Synthesis of compound **36a-4**

To a solution of compound **36a-3** (0.39 g, 0.386 mmol, 1.0 eq) in DMF (5 mL) were added **20a-SM** (0.374 g, 0.386 mmol, 1.0 eq) and DIPEA (0.099 g, 0.772 mmol, 2.0 eq) at room temperature under nitrogen atmosphere. The mixture was stirred at room temperature for 16 h. When LCMS indicated the completion of the reaction, the mixture was directly used in the next step.

Synthesis of compound **36a-5**

To the above reaction mixture was added diethylamine (1 mL) at room temperature under nitrogen atmosphere, and the mixture was stirred at room temperature for 1 h. When LCMS indicated the completion of the reaction, to methyl *tert*-butyl ether was added the reaction mixture, and the mixture was filtered to remove the solid and dried *in vacuo* to give a white solid (0.61 g, 86.6% yield).

Synthesis of compound **36a-6**

To a solution of **PEG8** (250 mg, 0.377 mmol, 1.0 eq) in DMSO (5 mL) were added DIPEA (97.4 mg, 0.755 mmol, 2.0 eq) and TSTU (113.6 mg, 0.377 mmol, 1.0 eq) sequentially at room temperature (25 °C) under nitrogen atmosphere. To the above reaction mixture was added **36a-5** (610 mg, 0.377 mmol, 1.0 eq). The mixture was stirred at room temperature for 1 h. When LCMS indicated the completion of the reaction, to methyl *tert*-butyl ether was added the reaction mixture, and the mixture was filtered to remove the solid and dried *in vacuo* to give a white solid (0.33 g, 42.5% yield).

Synthesis of compound **36a-7**

To a solution of **101B13** (250 mg, 0.003 mmol, 1.0 eq) in dry DMF (5 mL) were added a solution of **36a-6** (312 mg, 0.15 mmol, 45.0 eq) in dry DMF (2 mL, ultrasonicated for dissolution), PyBOP (142 mg, 0.27 mmol, 80.0 eq), and DIPEA (56 mg, 0.44 mmol, 128.0 eq) at room temperature (25 °C) under nitrogen atmosphere. The mixture was then heated to 28 °C and stirred for 12 h. The mixture was concentrated and directly used in the next step.

Synthesis of compound **36a**

To a solution of **36a-7** (crude) in dry DCM (10 mL) were added dichloroacetic acid (1 mL) and anisole (1 mL) at room temperature (25 °C) under nitrogen atmosphere, and the mixture was stirred for 1 h. MTBE (50 mL) was added, the mixture was filtered, and the solid was dissolved in methanol and water. The mixture was purified by ultrafiltration (30K MW) and lyophilized to give a product **36a** (300 mg, white solid, 98.5% purity, about 19.3% paclitaxel content).

Example 70: Synthesis of Compound 36b

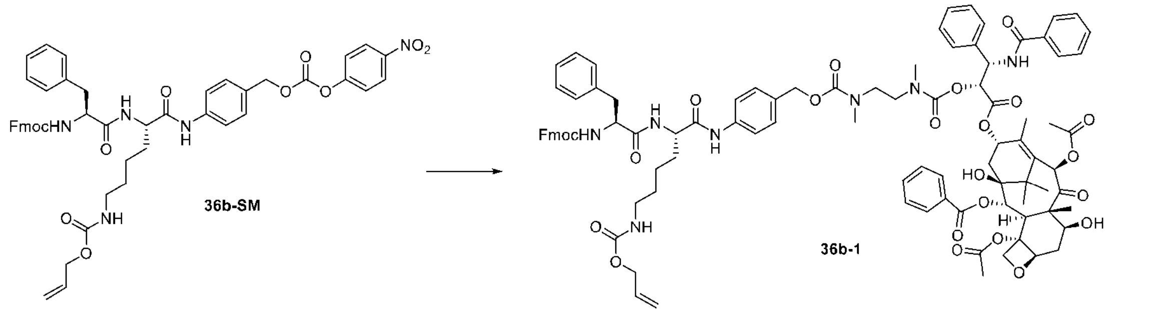

36b-SM

36b-1

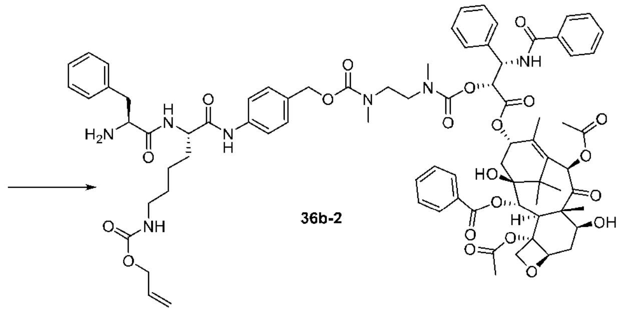

36b-2

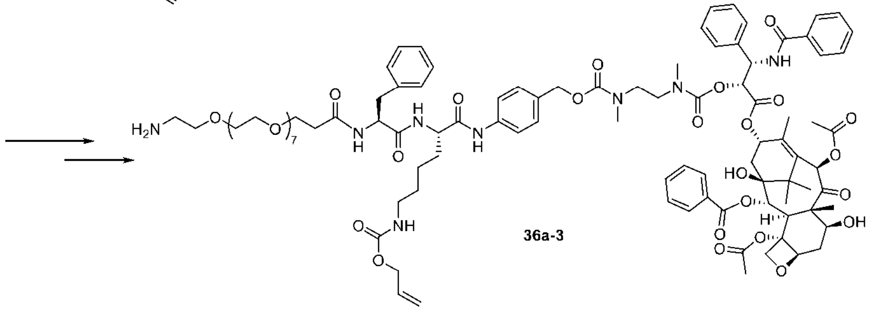

36a-3

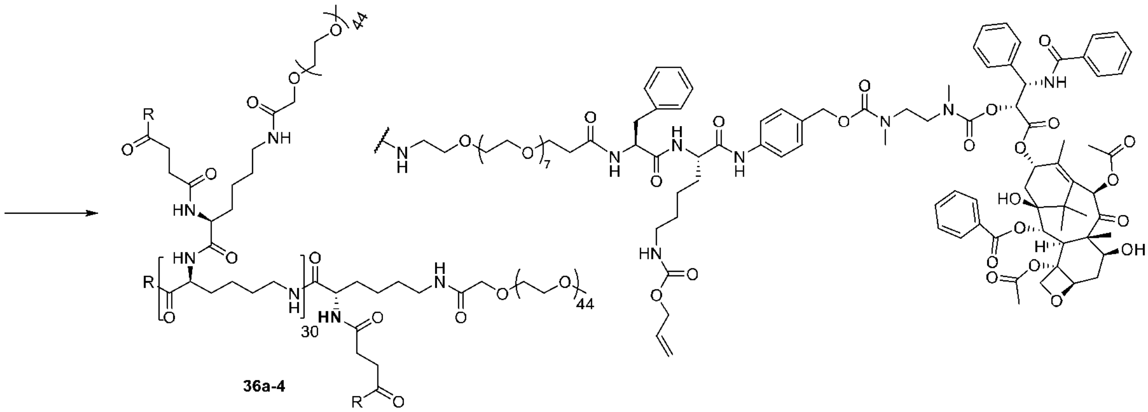

36a-4

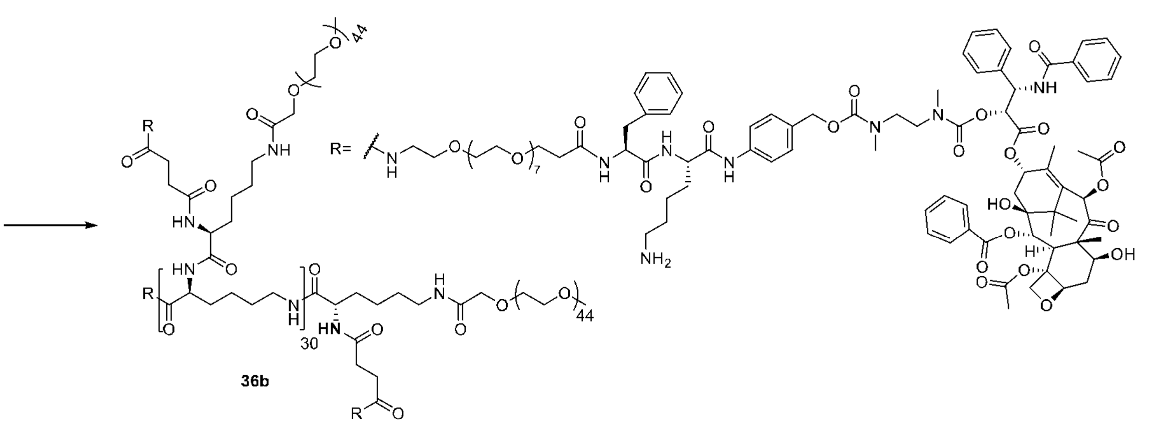

36b

Synthesis of compound **36b-1**

To a solution of compound **36b-SM** (synthesized according to the method in Chem. Eur. J. 2015, 21, 1-10, 100 mg, 0.115 mmol, 1.0 eq) in DMF (5 mL) were added **20a-SM** (11.3 mg, 0.115 mmol, 1.0 eq) and DIPEA (14.8 mg, 0.115 mmol, 1.0 eq) at room temperature under nitrogen atmosphere. The mixture was stirred for 16 h. When LCMS indicated the completion of the reaction, the mixture was directly used in the next step.

MS (ESI), m/z, 1699.2 $[M+H]^+$.

Synthesis of compounds **36b-2, 36b-3,** and **36b-4**

The synthesis was conducted according to the synthetic method for **36a-5, 36a-6,** and **36a-7** in Example **69**

Synthesis of compound **36b**

To a solution of **36b-4** (crude product, 300 mg) in dry DMF (5 mL) were added $Pd(PPh_3)4$ (15 mg), $Bu_3SnH$ (3 drops), and AcOH (4 drops) at room temperature (25 °C) under nitrogen atmosphere. The reaction was monitored by HPLC until the depletion of the starting materials. MTBE (50 mL) was added, the mixture was filtered, and the solid was dissolved in methanol and water. The mixture was purified by ultrafiltration (30K MW) and lyophilized to give a product **36b** (230 mg, white solid, 99.5% purity, about 22.5% paclitaxel content).

Example 71: Synthesis of Compound 37a

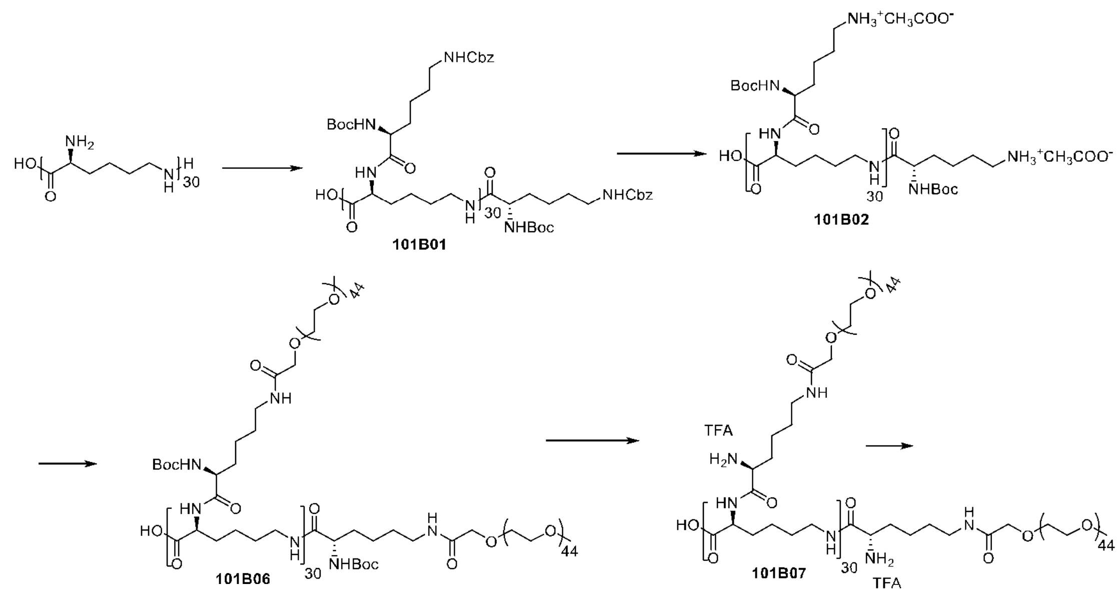

**101B01** **101B02** **101B06** **101B07**

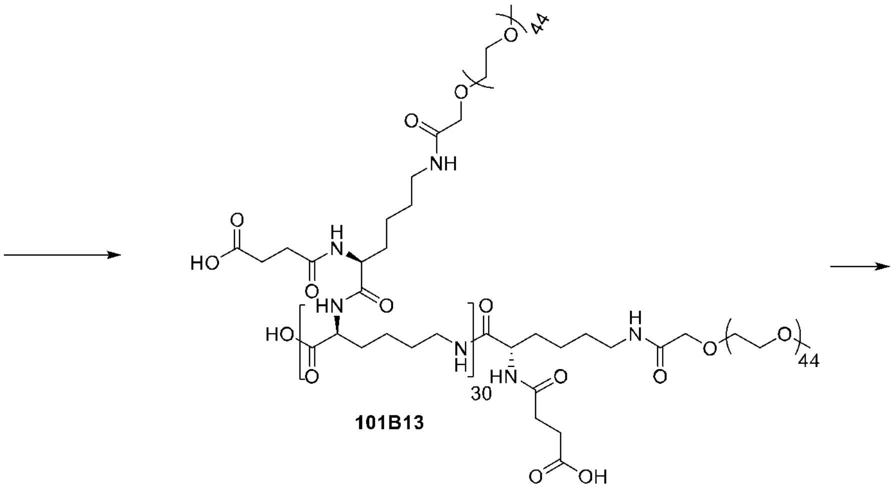

101B13

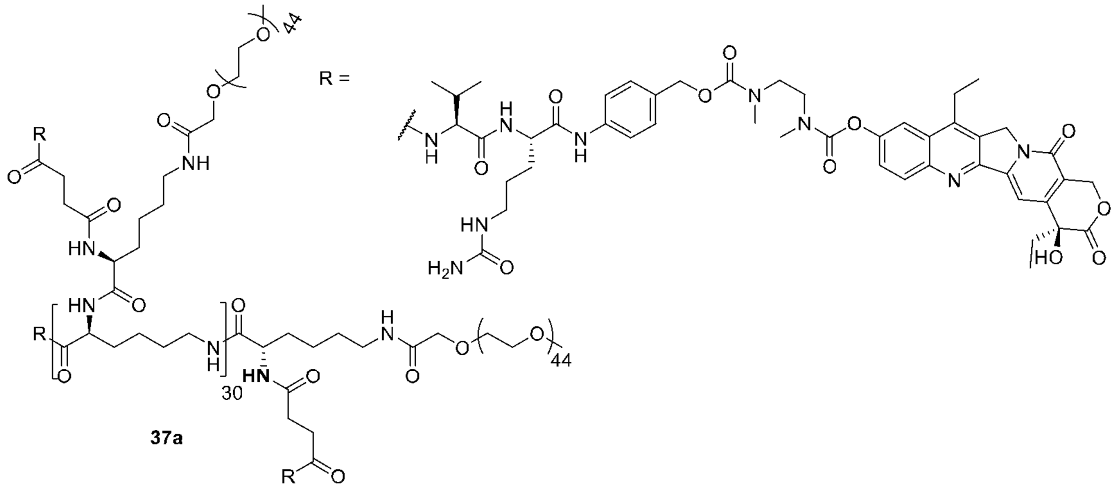

37a

Synthesis of compound **101B01**

To a suspension of ε-poly-L-lysine hydrochloride (4.792 g, 29.09 mmol, molar quantity of structural unit) in DMSO (100 g) was added triethylamine (8.89 g, 87.27 mmol), and α-Boc-ε-Cbz-L-lysine-NHS active ester **(10102,** prepared in Example 2; 20.83 g, 46.63 mmol) was then added. The mixture was stirred at 30 °C for 13 h under nitrogen atmosphere until the reaction was completed. The reaction mixture was transferred into a beaker, and ACN (800 mL) was added. The mixture was filtered *in vacuo,* and the cake was washed with acetonitrile, water, and acetonitrile sequentially and dried *in vacuo* to give a product ε-PolyLys$_{30}$-[Lys(α-Boc, ε-Cbz)]$_{30}$ **(101B01;** white solid, 12.13 g, 85%).

$^1$H NMR (400 MHz, DMSO-$d_6$) 7.74 (m, 62H), 7.27 (m, 179H), 6.90 (m, 28H), 5.16 - 4.80 (s, 60H), 4.31 - 4.01 (br, 30H), 4.00 - 3.68 (br, 30H), 3.12 - 2.82 (m, 120H), 1.91 - 0.58 (m, 642H).

Synthesis of compound **101B02**

α-[Boc-Lys(Cbz)]$_{30}$-ε-PolyLys$_{30}$ **(101B01;** 2.92 g) was dissolved in acetic acid (30 mL) and methanol (30 mL) while heating, and palladium on carbon (591 mg, 10%) was added. The mixture was purged with hydrogen and stirred at 25 °C for 18 h. Celite was added, and the mixture was filtered *in vacuo.* The filtrate was concentrated by rotavap, and methyl *tert*-butyl ether was added for precipitation. The mixture was dried *in vacuo* to give α-[Boc-Lys(NH3$^+$COO$^-$)]$_{30}$-ε-PolyLys$_{30}$ **(101B02;** white powder).

$^1$H NMR (400 MHz, $D_2O$) δ 4.20 - 3.98 (m, 30H), 3.88 (t, *J* = 7.0 Hz, 31H), 3.04 (s, 61H), 2.87 (t, *J* = 7.6 Hz, 59H), 1.84 (s, 122H), 1.78 - 1.10 (m, 594H).

Synthesis of compound **101B06**

α-[Boc-Lys(NH3$^+$COO$^-$)]$_{30}$-ε-PolyLys$_{30}$ **(101B02)** (prepared in Example 46) (4.76 g) was dissolved in water (20

mL), and 1014-2K (10.00 g) was dissolved in acetonitrile (20 mL). The two solutions were mixed, and triethylamine (1.39 mL) was added. The mixture was stirred for 10 min, and a solution of 1014-2K (20.00 g) in acetonitrile (40 mL) and triethylamine (2.79 mL) were then added sequentially. The mixture was stirred at 25 °C for 4 h, and water was added. The mixture was ultrafiltered. The filtrate was concentrated and lyophilized to give a product α-[Boc-Lys(mPEG$_{2k}$)]$_{30}$-ε-PolyLys$_{30}$ **(101B06)** (white powder, 18.4 g).

$^1$H NMR (400 MHz, $D_2O$) δ 4.25 (s, 32H), 4.09 (s, 95H), 3.93-3.45 (m, 5537H), 3.41 (s, 95H), 3.36 - 2.90 (m, 126H), 2.20-0.86 (m, 645H).

Synthesis of compound **101B07**

To a solution of **101B06** (9.0 g) in dichloromethane (90 mL) was added TFA (30 mL), and the mixture was stirred at room temperature for 6 h, concentrated by rotavap to remove the solvent dichloromethane, and dried *in vacuo* to give **101B07** (white solid, 8.84 g, 97.6%).

$^1$H NMR (400 MHz, $D_2O$) δ 4.23 (br, 35H), 4.04 (br, 94H), 3.70 (br, 5255H), 3.38 (br, 92H), 3.25 (m, 128H), 2.10 - 1.11 (m, 376H).

Synthesis of compound **101B13**

To a solution of **101B07** (27.37 g) in DMF (104 mL) were added succinic anhydride (2.373 g) and DIPEA (8.174 mL), and the mixture was stirred at room temperature for 4 h. A small amount of methyl tert-butyl ether was added for precipitation. The mixture was subjected to chromogenic reaction using ninhydrin at 100 °C for 3 min, and the result was colorless. MTBE (300 mL) was added for precipitation to give a gum, and the gum was centrifuged. The supernatant was diluted and analyzed by GPC, which showed no product. To a solution of the gum in ACN (100 mL) was slowly added MTBE (350 mL) to precipitate a product. The solution was removed, and the gum precipitate was dissolved in ACN (100 mL). The product was precipitated after MTBE (400 mL) was slowly added, filtered and dried *in vacuo* to give a compound **101B13** (21.8 g, 8.7 mmol, 76%).

$^1$H NMR (400 MHz, $D_2O$) δ 4.16 (br, 61H), 4.00 (br, 63H), 3.64 (br, 5113H), 3.32 (br, 91H), 3.27 - 2.84 (m, 124H), 2.80 - 2.30 (br, 127H), 2.14 - 0.71 (br, 366H).

Synthesis of compound **37a**

To a solution of **101B13** (0.705 g), **2-SM** (0.384 g), and PyBOP (0.293 g) in DMF (6 mL) was added DIPEA, and the mixture was stirred at room temperature for 4 h. MTBE was added for precipitation. To methanol (60 mL) was added the precipitate, and water (70 mL) was added. The mixture was purified by ultrafiltration and lyophilized to give a final product **37a** (0.832 g, pale yellow cotton-like product).

HPLC analysis method D: RT = 13.824 min.

$^1$H NMR (400 MHz, DMSO-*d*$_6$) δ 8.12 (br, 62H), 7.89 (br, 66H), 7.61 (br, 132H), 7.28 (br, 98H), 5.35 (br, 112H), 4.98 (br, 70H), 4.26 (br, 150H), 3.84 (br, 77H), 3.50 (br, 6237H), 3.23 (br, 90H), 3.18 - 2.74 (m, 374H), 1.34 (m, 899H).

Example 72: Synthesis of Compound 37b

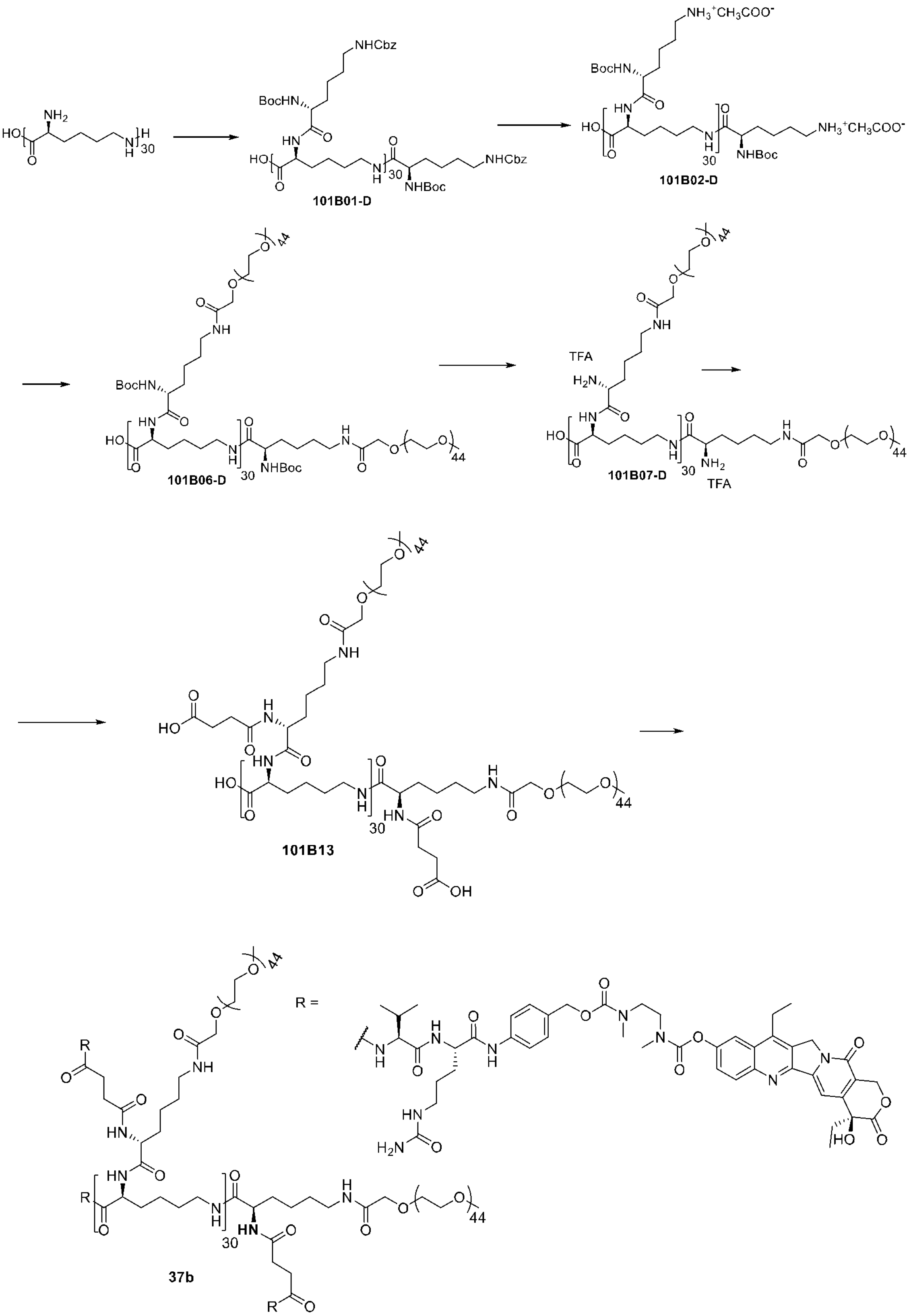

101B01-D

101B02-D

101B06-D

101B07-D

101B13

37b

Synthesis of compound **101B01-D**

To a suspension of ε-poly-L-lysine hydrochloride (4.7 g, 29.09 mmol, molar quantity of structural unit) in DMSO (100 g) was added triethylamine (8.89 g, 87.27 mmol), and α-Boc-ε-Cbz-D-lysine-NHS active ester (synthesized according to the method in Bioorganic & medicinal chemistry, 2005, 13(7): 2523-2536; 20.83 g, 46.63 mmol) was then

added. The mixture was stirred at 30 °C for 13 h under nitrogen atmosphere until the reaction was completed. The reaction mixture was transferred into a beaker, and ACN (800 mL) was added. The mixture was filtered *in vacuo,* and the cake was washed with acetonitrile, water, and acetonitrile sequentially and dried *in vacuo* to give a product ε-PolyLys$_{30}$-[Lys(α-Boc, ε-Cbz)]$_{30}$ **(101B01-D;** white solid, 12.13 g, 85%).

Synthesis of compound **101B02-D**

Acetic acid (40 mL) was placed in a 250 mL flask, and **101B01-D** (3.65 g, 7.44 mmol) was added. The mixture was stirred and heated at 40 °C until dissolved. To the above reaction flask were added methanol (40 mL) and palladium on carbon, and the system was vacuumized and stirred at 30 °C under hydrogen atmosphere. Celite was added, and the mixture was filtered *in vacuo.* The cake was washed with methanol, and the resulting pale brown filtrate was filtered through a nylon filter membrane (0.22 μm). The filtered solution was concentrated by rotavap to a viscous state. Methyl *tert*-butyl ether was added for precipitation, and the supernatant was discarded. The remaining solvent was removed using a water pump, and the residue was lyophilized to give a white solid product (3.6 g).

Synthesis of compound **101B06-D**

**101B02-D** (1.5 g, 3.4 mmol) was dissolved in MeOH (20 mL). PEG-2K-NHS (8.69 g, 4.1 mmol) was dissolved in ACN (37 mL), and after complete dissolution, MeOH (37 mL) and the pre-dissolved **101B02-D** were added sequentially. MeOH (7 mL) was added for rinsing, DIPEA was added, and the mixture was stirred for 15 min. A sample was taken and dried with nitrogen, and dissolved in water. The mixture was then filtered and analyzed by GPC. After the reaction was completed, HOAc (1.07 g) was added to adjust the pH, and the reaction mixture was filtered through a nylon filter membrane (0.22 μm). The filter membrane was rinsed with MeOH, and an equal volume of water was added. The mixture was purified by ultrafiltration, then concentrated, washed with water (50 mL) thrice, and concentrated to a viscous state. Ethyl acetate (20 mL) was added, and to MTBE (200 mL) was added the mixture for precipitation. The mixture was filtered to give a white solid product (4.4 g).

Synthesis of compound **101B07-D**

To a solution of **101B06-D** (4 g, 1.69 mmol) in DCM (14 mL) was added TFA (6 mL), with no significant heat release observed, and the mixture was stirred at room temperature overnight under nitrogen atmosphere. A sample (about 50 μL) was taken, dried with nitrogen, and detected by NMR, which indicated the depletion of the starting materials. The mixture was concentrated by rotavap at 37 °C. To the crude product was added MTBE (40 mL) with stirring to give an oily precipitate. The oily precipitate was cooled to below -10 °C with vigorous stirring, and pellet and powder solids were precipitated. The mixture was stirred, heated to above 15 °C, and filtered. The flask and the cake were washed with 40 mL of MTBE. The cake was dried *in vacuo* with an oil pump for 2 h to give a white solid (4 g).

Synthesis of compound **101B13-D**

To a 100 mL round-bottom flask was added **101B07-D** (4 g, 1.76 mmol), and EtOAc (20 mL) was added. The mixture was stirred at 30 °C for dissolution under nitrogen atmosphere. After complete dissolution, succinic anhydride (0.23 g, 2.29 mmol) was added and dissolved by stirring. DIPEA was added dropwise at 30 °C. After the addition was complete, 1 drop of the solution was taken out and added into water, the pH was 8. The mixture was stirred at room temperature for 4 h, and the reaction was monitored. A sample (50 μL) was taken and added to MTBE for precipitation. The mixture was centrifuged and subjected to chromogenic reaction using ninhydrin/phenol/pyridine (50 μL/100 μL/50 μL) at 100 °C for 3 min. The result was pale purple, indicating the depletion of the amino. The mixture was transferred to a 250 mL flask, and MTBE (60 mL) was added with vigorous stirring. After the addition, the mixture was stirred for another 10 min and filtered *in vacuo.* The cake was dissolved in EtOAc (20 mL) with heating at 30 °C, and MTBE (60 mL) was then added with vigorous stirring. After the addition, the mixture was stirred for another 10 min and filtered *in vacuo.* The residue was dried *in vacuo* at 35 °C for 1 h to give a white solid product (3.5 g).

Synthesis of compound **37b**

To a solution of **101B13-D** (1.5 g, 601.44 μmol, based on the polymer structural unit) and **2-SM** (877.6 mg, 962.31 μmol) in DMF (5 mL) was added HOBt (162.77 mg, 1202.89 μmol), and the mixture was stirred for dissolution. PyBOP (626 mg, 1202.89 μmol) was added and dissolved by stirring, and N-methylmorpholine (297.5 μL, 2706.50 μmol) was then added. The mixture was stirred at room temperature overnight under nitrogen atmosphere. A sample was taken for HPLC detection, which showed that 9.6% of 10114A remained. To MTBE (50 mL) was added the reaction mixture for

precipitation, and the mixture was centrifuged. The precipitate was dissolved in MeOH (25 mL). The mixture was purified by ultrafiltration and lyophilized to give a white solid product (1.6242 g, 99.97% purity, 11.7% SN-38 conjugation content).

Example 73: Synthesis of Compound 1D

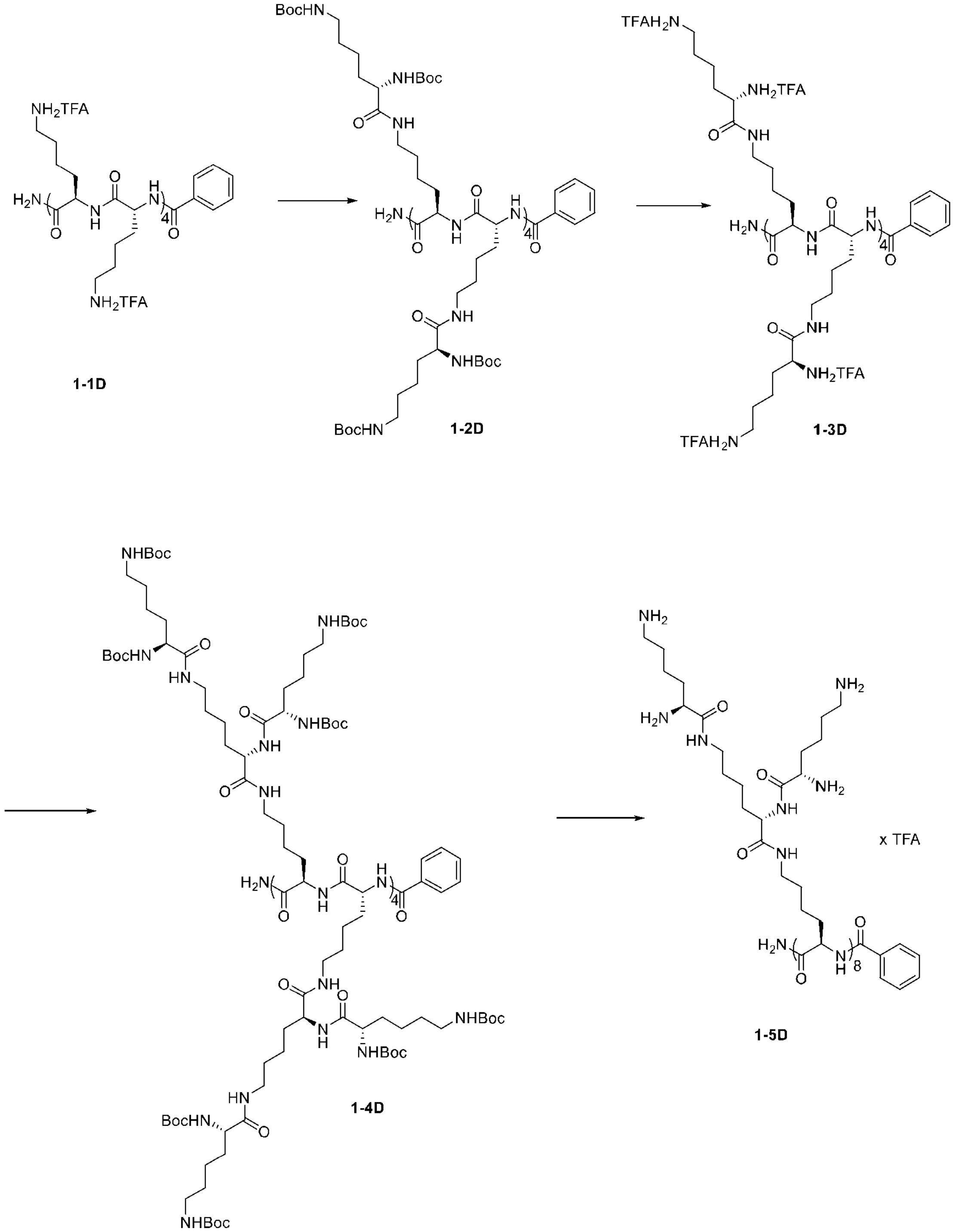

1-1D 1-2D 1-3D 1-4D 1-5D

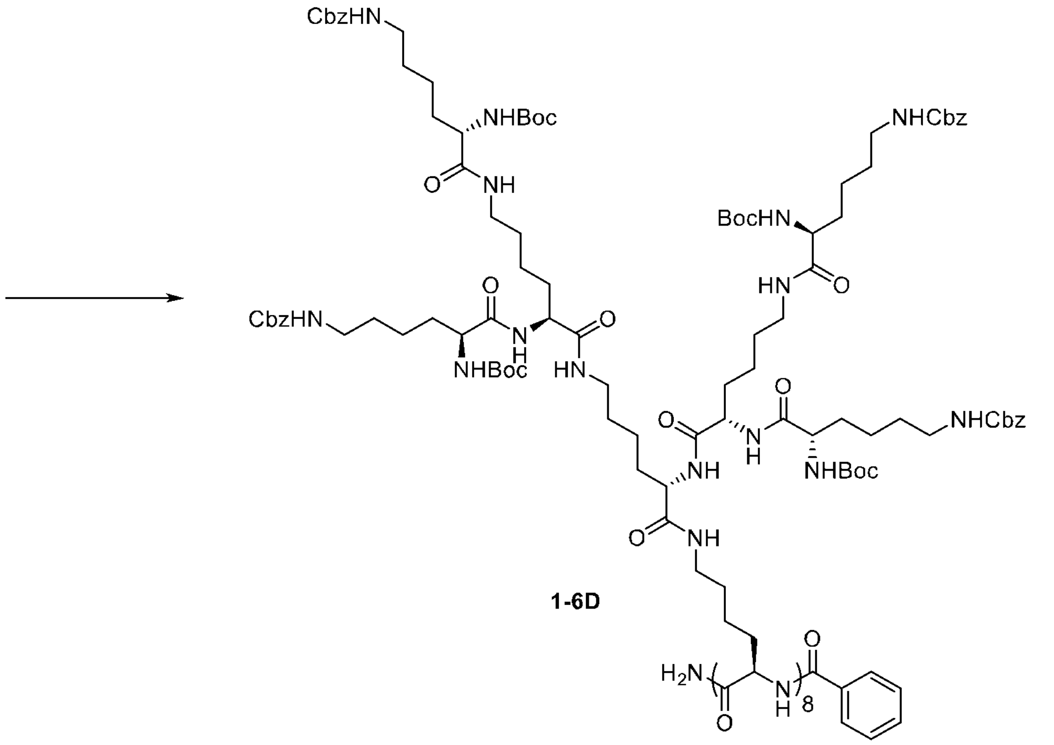

1-6D

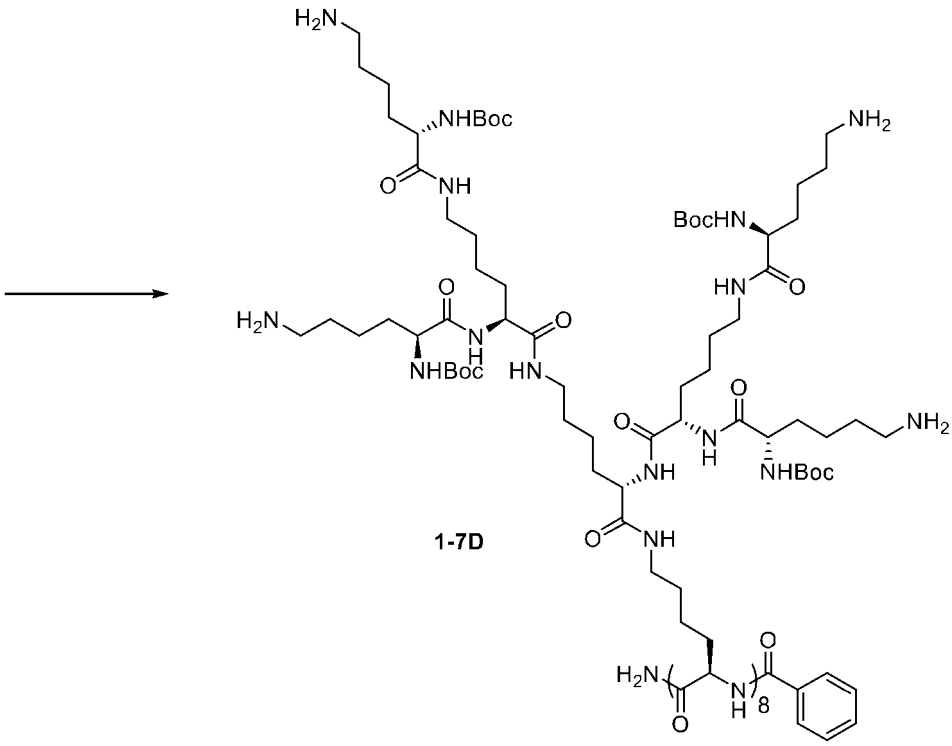

1-7D

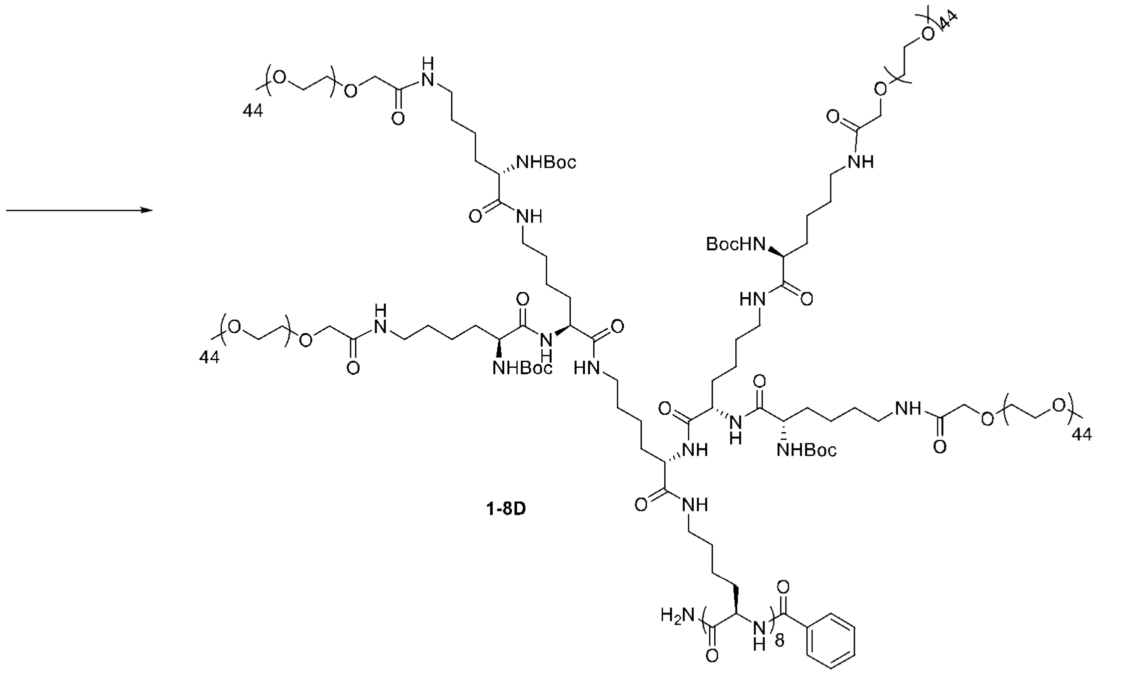

1-8D

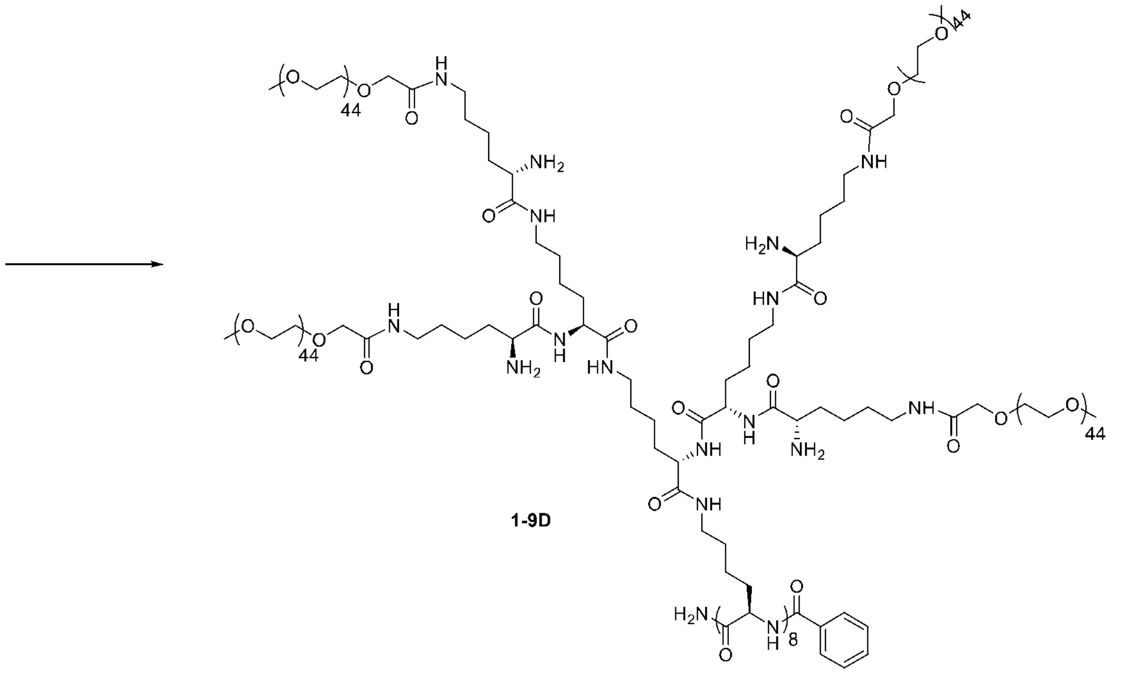

1-9D

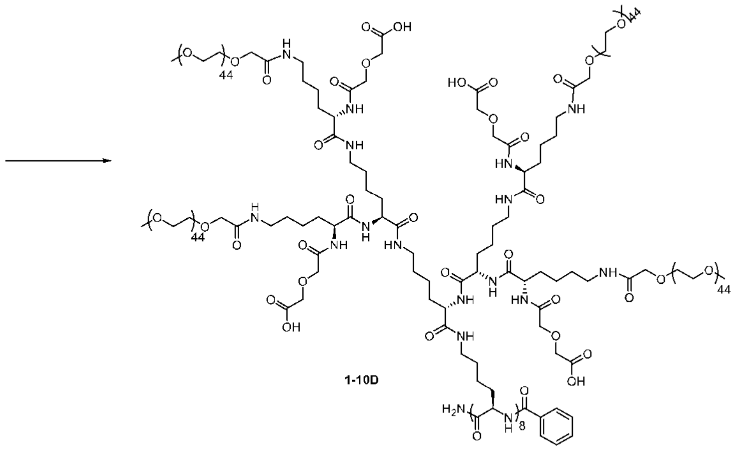

1-10D

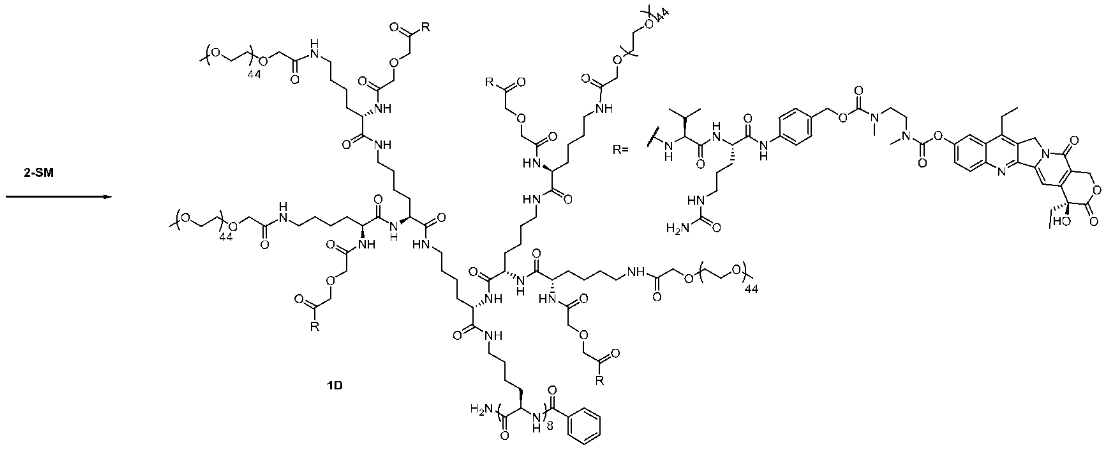


1D

Synthesis of compound **1-1D to 1-10D**

The preparation was performed according to the synthesis method for **1-1 to 1- 10** in Example **1** with D-lysine as the starting material.

Synthesis of compound **1D**

To a solution of compound **1-10D** (376 mg, 4.81 μmol) and **2-SM** (280 mg, 308 μmol) in DMF (9 mL) were added PyBOP (240 mg, 460 μmol) and DIPEA (230 μL, 1.40 mol). The mixture was stirred at room temperature for 24 h. Methyl *tert*-butyl ether was added for precipitation. The mixture was let stand at -20 °C until the completion of the precipitation. The precipitate was dissolved in methanol and purified on an LH-20 gel column. The mixture was concentrated, and the residue was redissolved in water. The mixture was filtered and lyophilized to give compound 1D (420 mg).

Example 74: Synthesis of Compound 38

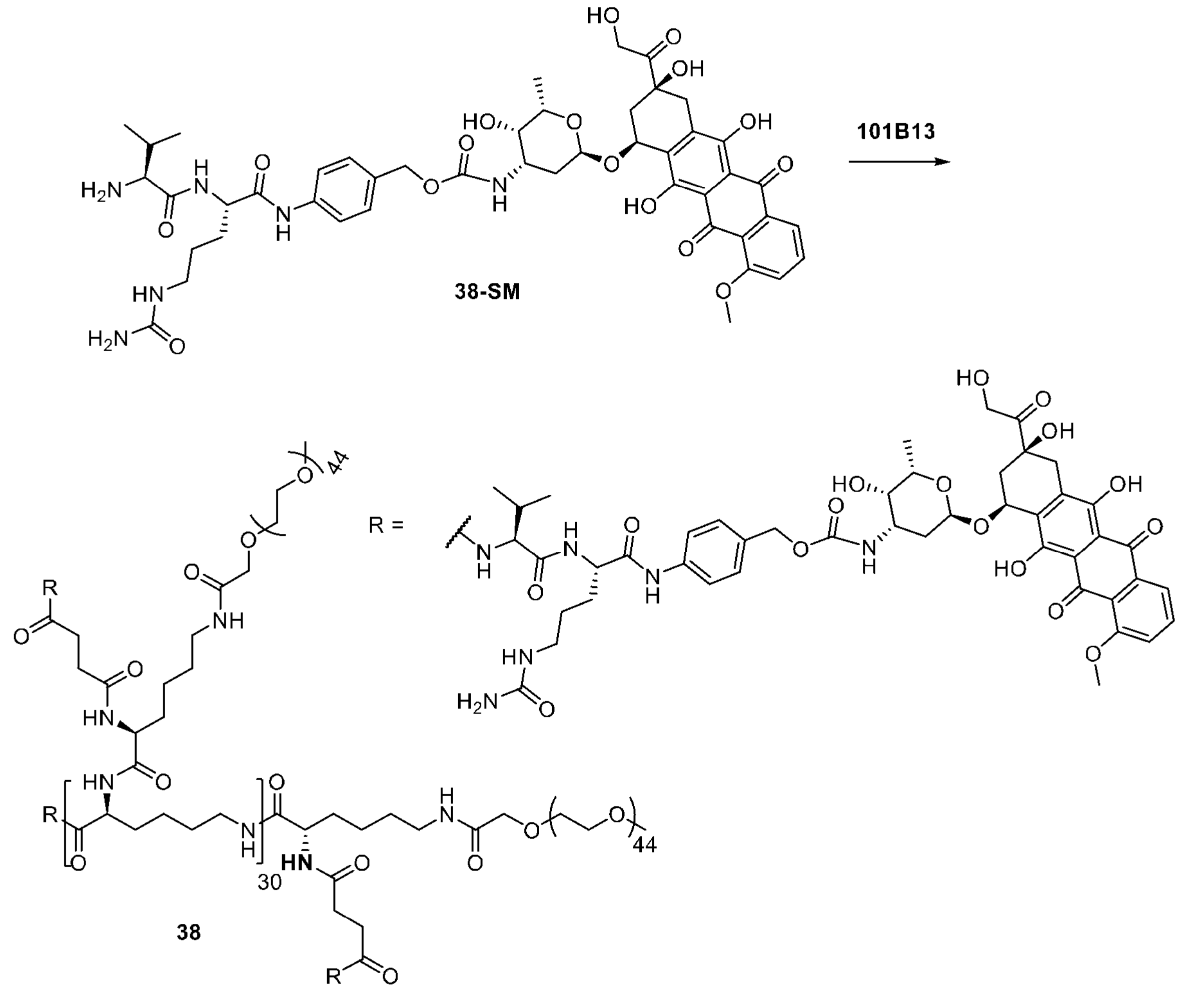


38-SM

38

Synthesis of compound **38**

To a solution of **101B13** (0.41 g) and **38-SM** (0.24 g) in DMF (5 mL) were added PyBOP (0.13 g) and DIPEA (0.13 mL) at room temperature under nitrogen atmosphere, and the mixture was stirred at room temperature overnight. To MTBE (50 mL) was added the reaction mixture for precipitation and centrifuged. The precipitate was washed with dichloromethane, dried *in vacuo,* and then dissolved in methanol (70 mL). Water (30 mL) was added, and the mixture was purified by ultrafiltration (30K MW) and lyophilized to give a final product **(38)** (450 mg, red solid, 98.8% purity by HPLC).

Example 75: Synthesis of Compound 39a

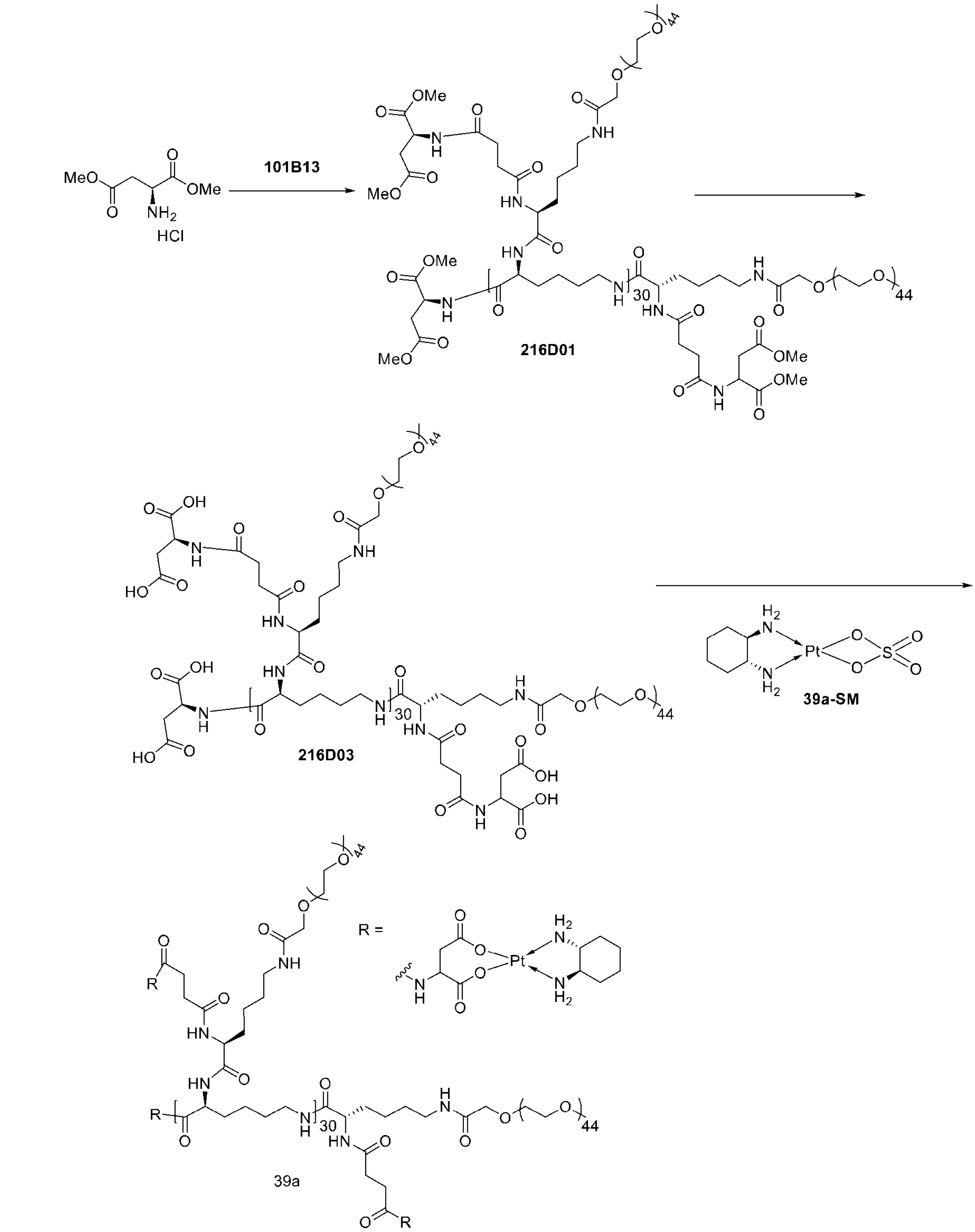


216D01

216D03

39a-SM

39a

Synthesis of compound **21601**

**101B13** (500 mg, 0.21 mmol, 1.0 eq, based on the molar quantity of the polymer unit), amine hydrochloride (62.6 mg, 0.32 mmol, 1.5 eq), PyBOP (165.0 mg, 0.32 mmol, 1.5 eq), DIPEA (0.19 mL, 1.06 mmol, 5.0 eq), and DMF (4.0 mL) were added sequentially at room temperature (25 °C) under nitrogen atmosphere. The reaction mixture was stirred at room temperature for 15 h until HPLC monitoring indicated the depletion of the starting materials. To the above DMF mixture was added MTBE (40 mL). The solution turned turbid, and a white solid was precipitated. The supernatant was discarded, and the white solid was washed with MTBE and dried. The remaining white solid was dissolved in MeOH and ultrafiltered with MeOH/water to remove small molecule impurities. When HPLC monitoring indicated the complete

removal of small molecule impurities, the mixture was collected and concentrated by rotavap to remove MeOH. The aqueous solution was lyophilized to give a product **216D01** (455 mg, white solid).

Synthesis of compound **216D03**

**216D01** (277 mg, 0.11 mmol, 1.0 eq) and an NaOH solution (0.1 M, 5.6 mL, 0.56 mmol, 5.0 eq) were added sequentially at room temperature (25 °C). The reaction mixture was stirred at room temperature for 4 h until HPLC monitoring indicated the depletion of the starting materials. The solution was ultrafiltered with water to remove small molecule impurities and lyophilized to give a product **216D03** (244 mg, white solid).

Synthesis of compound **39a**

To an aqueous solution (0.5 mL) of **216D03** (55 mg, 0.022 mmol, 1.0 eq) was added a $Ba(OH)_2$ solution (0.1 M, 0.22 mL, 0.022 mmol, 1.0 eq) at room temperature (25 °C). The reaction mixture was stirred at room temperature for 10 min. To the solution was added a **39a-SM** solution (synthesized according to the method in Angewandte Chemie, 2016, 128(7): 2596-2600. 0.2 M, 0.5 mL, 0.11 mmol, 5.0 eq). The reaction mixture was stirred at room temperature for 15 h until HPLC monitoring indicated the depletion of the starting materials. The mixture was purified by ultrafiltration (30K MW) and lyophilized to give a product 39a (51 mg, white solid, 98.5% purity).

Example 76: Synthesis of Compound 39b

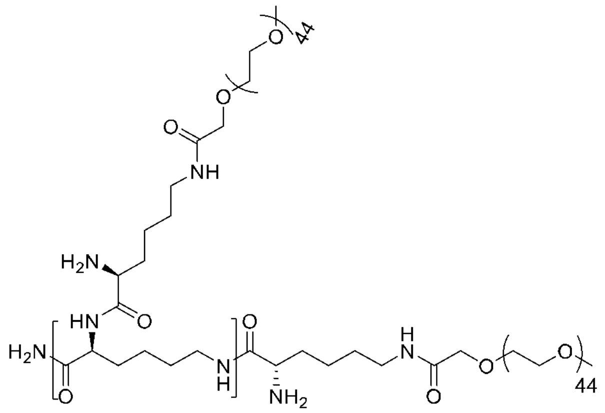

**31-2**

+

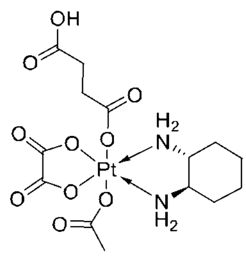

**39b-SM**

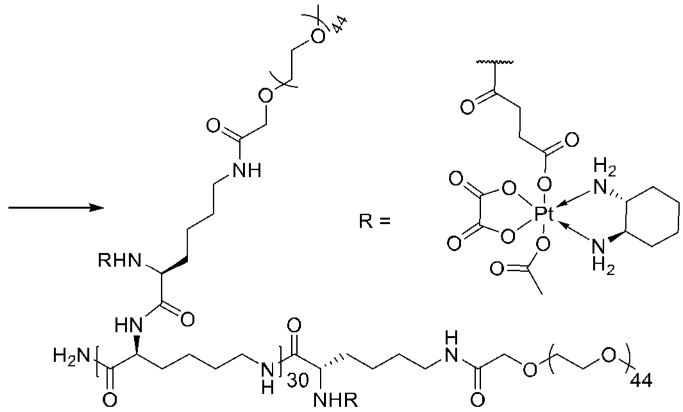

**39b**

Synthesis of compound **39b**

To a solution of **31-2** (1.0 g, 0.423 mmol, 1.0 eq, based on the polymer unit) in DMF (10 mL) were added **3b-SM** (synthesized according to the method in European Journal of Inorganic Chemistry, 2014, 2014(3): 484-492, 250 mg, 0.508 mmol, 1.3 eq), PyBOP (0.61 mmol, 1.5 eq), and DIPEA (170 mg, 1.27 mmol, 3.0 eq) sequentially, and the mixture was stirred at room temperature for 12 h. The reaction mixture was concentrated and purified by ultrafiltration (30K MW), and the pure product solution was lyophilized to give a product (860 mg, 99% purity).

Example 77: Synthesis of Compound 40

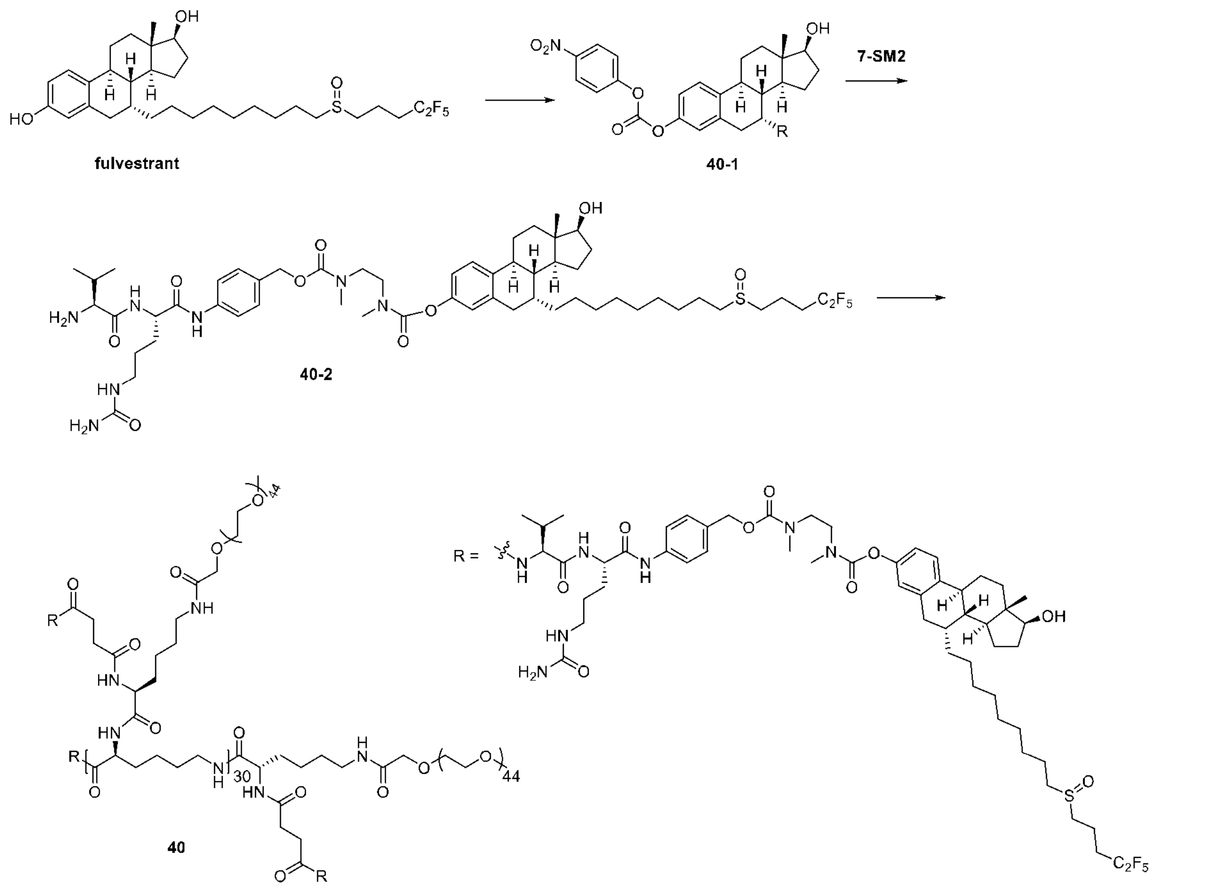


fulvestrant

40-1

40-2

40

Synthesis of compound **40-1**

Fulvestrant (1.82 g, 3.0 mmol, 1.0 eq), anhydrous DCM (10 mL), and DIPEA (1.1 mL, 6.0 mmol, 2.0 eq) were added sequentially at 0 °C under nitrogen atmosphere. After the starting materials were partially dissolved by stirring, *p*-nitrophenyl chloroformate (726 mg, 3.6 mmol, 1.2 eq) was added. The reaction mixture was then slowly warmed to room temperature and stirred for 1 h until LCMS monitoring indicated the depletion of fulvestrant. The reaction mixture was directly used in the next step without further processing.

Synthesis of compound **40-2**

To the above reaction mixture were added DMF (30 mL), **7-SM2** (3.24 g, 3.9 mmol, 1.3 eq), and DIPEA (1.1 mL, 6.0 mmol, 2.0 eq). The reaction mixture was stirred at room temperature for 4 h. When LCMS monitoring indicated the completion of the reaction, diethylamine (2.4 mL, 24 mmol, 8.0 eq) was added. The reaction mixture was stirred at room temperature for 1 h until LCMS monitoring indicated the completion of the reaction. The mixture was concentrated, and purified by reversed-phase column chromatography (MeCN/$H_2O$-0.05% TFA) to give a product **40-2** (white solid, 2.86 g, 77% yield over the three steps).

$^1H$ NMR (400 MHz, $CDCl_3$) δ 9.66 (s, 1H), 8.28 (s, 1H), 7.75 - 7.45 (m, 2H), 7.25 - 7.03 (m, 2H), 6.76 (d, *J* = 10.3 Hz, 2H), 5.88 (s, 1H), 5.29 - 4.92 (m, 4H), 4.77 (s, 1H), 3.71 (s, 1H), 3.65 - 2.56 (m, 24H), 2.58 - 1.99 (m, 22H), 1.95 - 1.09 (m, 38H), 1.04 - 0.71 (m, 13H).

Synthesis of compound **40**

**101B13** (1.73 g, 0.73 mmol, 1.0 eq, based on the polymer structural unit), **40-2** (1.36 g, 1.1 mmol, 1.5 eq), PyBOP (572.4 mg, 1.1 mmol, 1.5 eq), DIPEA (0.54 mL, 2.92 mmol, 4.0 eq), and DMF (10 mL) were added sequentially at room temperature (25 °C) under nitrogen atmosphere. The reaction mixture was stirred at room temperature for 15 h until HPLC monitoring indicated the depletion of the starting materials. To the above DMF mixture was added MTBE (60 mL). The solution turned turbid, and a white solid was precipitated. The mixture was purified by ultrafiltration (30K MW) and lyophilized to give a product **40** (2.1 g, white solid, 100.00% purity).

Example 78 (Reference Example): Synthesis of Compound 41

**Synthesis of compound 101A01**

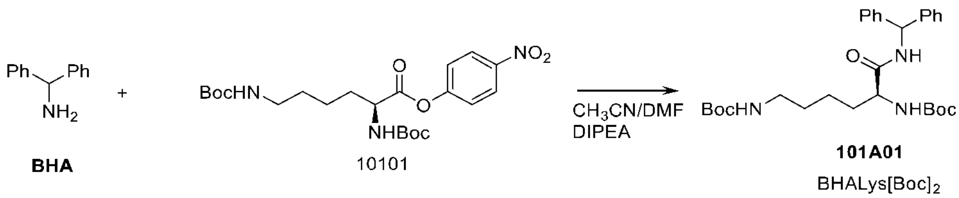


BHA 10101 101A01 BHALys[Boc]2

To a solution of benzhydrylamine (BHA, 2.50 g) in $CH_3CN$/DMF (20/10 mL) was added DIPEA (2.53 g) in an ice water bath at 5 °C under nitrogen atmosphere. **10101** (7.02 g) was added in portions, and the reaction mixture was stirred for 12 h. The above reaction mixture was added with a 0.5 N sodium hydroxide solution (100 mL). The mixture was extracted with ethyl acetate, dried over anhydrous sodium sulfate, and concentrated to give a crude product BHA-Lys(α, ε-Boc) **(101A01)** (3.8 g, 55% yield). MS (ESI), m/z, 512.2 $[M+H]^+$.

Synthesis of compound 101A02:

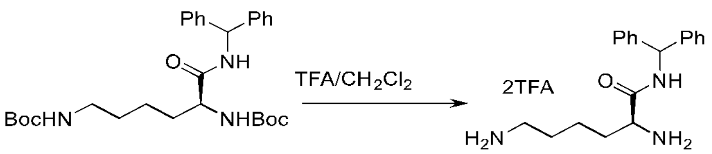


101A01 BHALys[Boc]2

G0 101A02 BHALys[TFA]2

To a solution of **101A01** (3.80 g) in dichloromethane (10 mL) was added TFA (2 mL) at room temperature (25 °C) under nitrogen atmosphere, and the mixture was stirred for 12 h. The above reaction mixture was concentrated and dissolved with dichloromethane (10 mL). MTBE (30 mL) was added, and the mixture was concentrated to give product 101A02, BHA-Lys(α, ε-NH3+CF3COO-) **(101A02)** (foamy solid, 3.0 g, 89% yield). MS (ESI), m/z, 312.1 $[M+H]^+$.

Synthesis of compound 101A03:

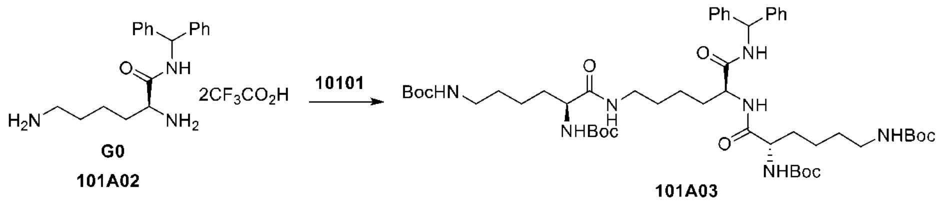


G0

101A02

BHALys[TFA]$_2$

101A03

BHALys[Lys]$_2$[Boc]$_4$

To a solution of **101A02** (TFA salt, 1.00 g) in DMF (10 mL) was added DIPEA (1.70 g) in an ice water bath at 5 °C under nitrogen atmosphere. **10101** (1.91 g) was added in portions, and the reaction mixture was stirred for 12 h. A 0.5 N sodium hydroxide solution (50 mL) was poured into the above reaction mixture, and the mixture was stirred for 1 h and filtered. The cake was washed with a 0.5 N sodium hydroxide solution and dried *in vacuo* to give a product **101A03** (white solid, 1.50 g, 84% yield). MS (ESI), m/z, 968.5 $[M+H]^+$.

Synthesis of compound 101A04:

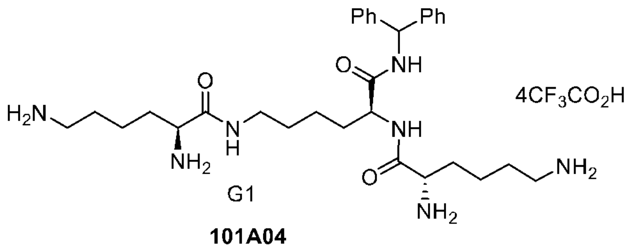


G1

**101A04**

BHALys[Lys]2[TFA]4

To a solution of **101A03** (1.5 g, 1.55 mmol) in dichloromethane (10 mL) was added TFA (2 mL) at room temperature (25 °C) under nitrogen atmosphere, and the mixture was stirred for 12 h. The above reaction mixture was concentrated to give a product **101A04** (foamy solid, 1.50 g, 95% yield).

$^1$H NMR (400 MHz, $D_2O$) δ 7.41 - 7.19 (m, 10H), 6.03 (s, 1H), 4.36 (t, J= 7.4 Hz, 1H), 3.94 (t, *J* = 6.5 Hz, 1H), 3.85 - 3.76 (m, 1H), 3.10 (t, *J* = 7.3 Hz, 2H), 2.90 (t, *J* = 7.9 Hz, 2H), 2.63 (t, *J* = 7.7 Hz, 2H), 2.03 - 0.99 (m, 18H).

MS (ESI), m/z, 568.3 $[M+H]^+$.

Synthesis of compound 101A05:

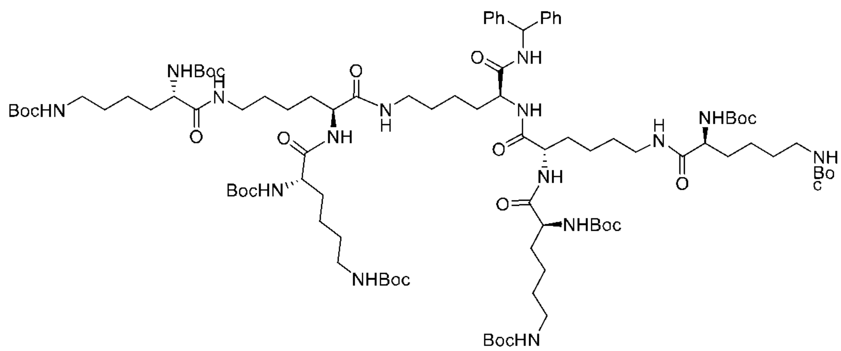

BHALys[Lys]$_4$[Boc]$_8$

**101A05**

To a solution of **101A04** (TFA salt, 1.50 g) in DMF (30 mL) was added DIPEA (2.60 g) in an ice water bath at 5 °C under nitrogen atmosphere. **10101** (prepared in Example 1; 3.01 g) was added, and the reaction mixture was stirred for 12

h. A 0.5 N sodium hydroxide solution (100 mL) was poured into the above reaction mixture, and the mixture was stirred for 1 h and filtered. The solid was collected and dried *in vacuo* to give a product **101A05** (white solid, 2.3 g, 83% yield).

MS (ESI), m/z, 941.0 [M/2+H]$^+$.

Synthesis of compound 101A06:

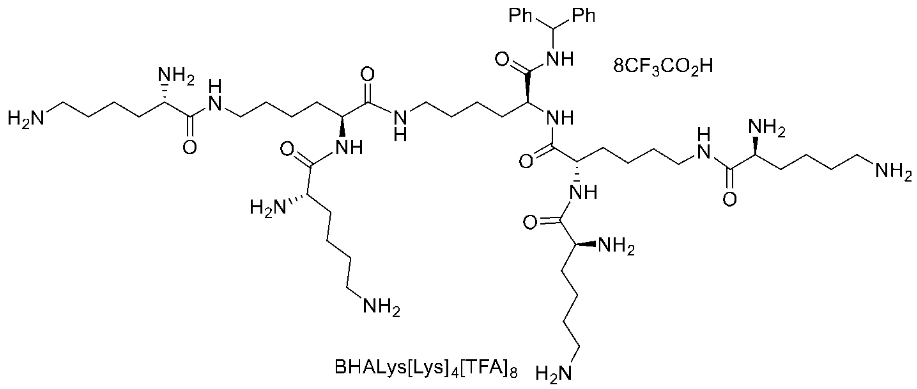

BHALys[Lys]$_4$[TFA]$_8$

**101A06**

To a solution of **101A05** (2.30 g) in dichloromethane (10 mL) was added TFA (10 mL) at room temperature (25 °C) under nitrogen atmosphere, and the mixture was stirred for 4 h. The above reaction mixture was concentrated and dissolved with dichloromethane/MeOH. MTBE was added, and the mixture was concentrated to give a product **101A06** (foamy solid, 2.41 g, 98.5% yield). MS (ESI), m/z, 540.8 [M/2+H]$^+$

$^1$H NMR (400 MHz, $D_2O$) δ 7.45 - 7.11 (m, 10H), 6.02 (s, 1H), 4.32 - 4.09 (m, 3H), 4.01 - 3.92 (m, 2H), 3.85 (t, *J* = 6.7 Hz, 1H), 3.78 (t, *J* = 6.7 Hz, 1H), 3.22 - 2.80 (m, 14H), 1.94 - 1.06 (m, 42H).

Synthesis of compound 101A07:

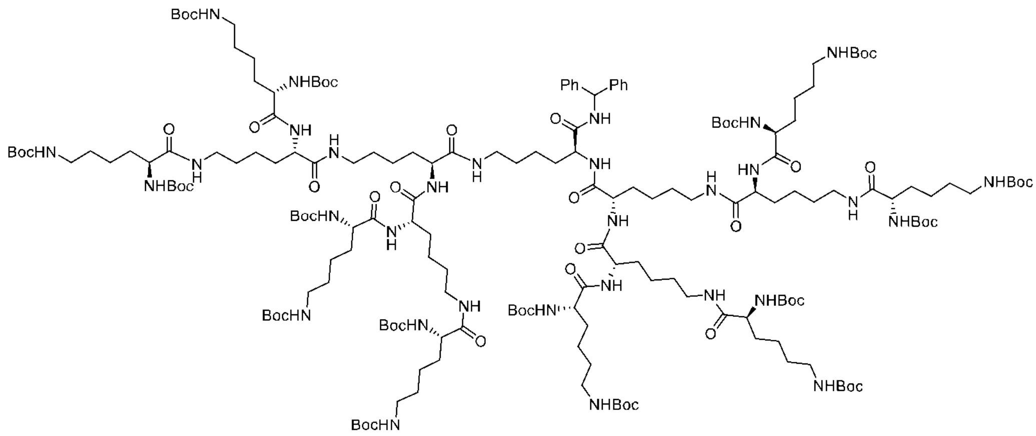

BHALys[Lys]$_8$[Boc]$_{16}$

**101A07**

To a solution of **101A06** (TFA salt, 2.40 g) in DMF (20 mL) was added DIPEA (3.73 g) in an ice water bath at 5 °C under nitrogen atmosphere. **10101** (prepared in Example 1; 5.41 g) was added, and the reaction mixture was stirred for 12 h. A 0.5 N sodium hydroxide solution (150 mL) was poured into the above reaction mixture, and the mixture was stirred for 1 h and filtered. The solid was collected and dissolved in acetonitrile, and water (1/1 volume) was added for precipitation. The mixture was stirred for 1 h and filtered. The solid was collected and dried *in vacuo* to give a product **101A07** (white solid, 3.82 g, 85% yield).

Synthesis of compound 101A08:

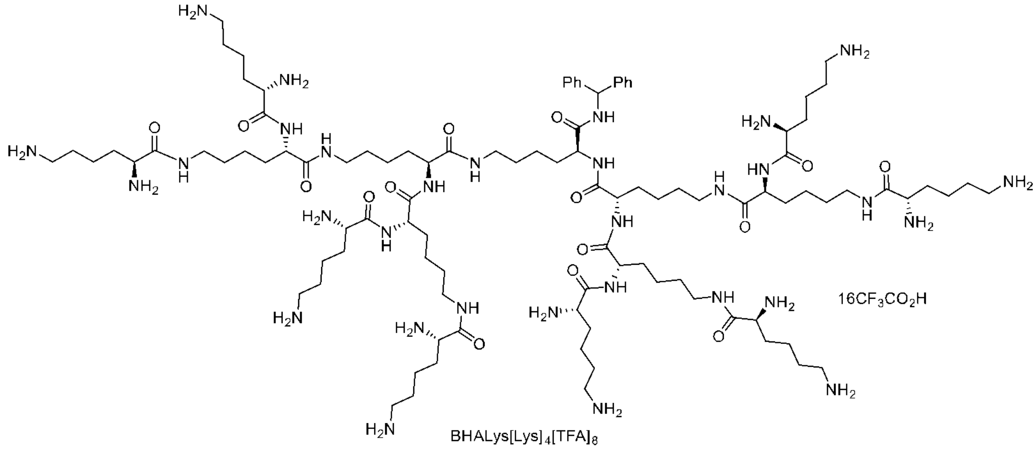

$16CF_3CO_2H$

$BHALys[Lys]_4[TFA]_8$

**101A08**

To a solution of **101A07** (3.0 g) in dichloromethane (10 mL) was added TFA (10 mL) at room temperature (25 °C) under nitrogen atmosphere, and the mixture was stirred for 4 h. The above reaction mixture was concentrated and dissolved in acetonitrile (30 mL). MTBE (150 mL) was added for precipitation, and the mixture was filtered. The solid was collected and dried *in vacuo* to give a product **101A08** (white solid, 2.9 g, 91% yield). MS (ESI), m/z, 702.7 $[M/3+H]^+$.

$^1H$ NMR (400 MHz, $D_2O$) $\delta$ 7.64 - 6.97 (m, 10H), 6.02 (s, 1H), 4.31 - 4.04 (m, 7H), 3.96 (t, *J* = 6.7 Hz, 4H), 3.90 - 3.79 (m, 5H), 3.30 - 2.77 (m, 30H), 1.93 - 0.96 (m, 90H).

Synthesis of compound 101A09:

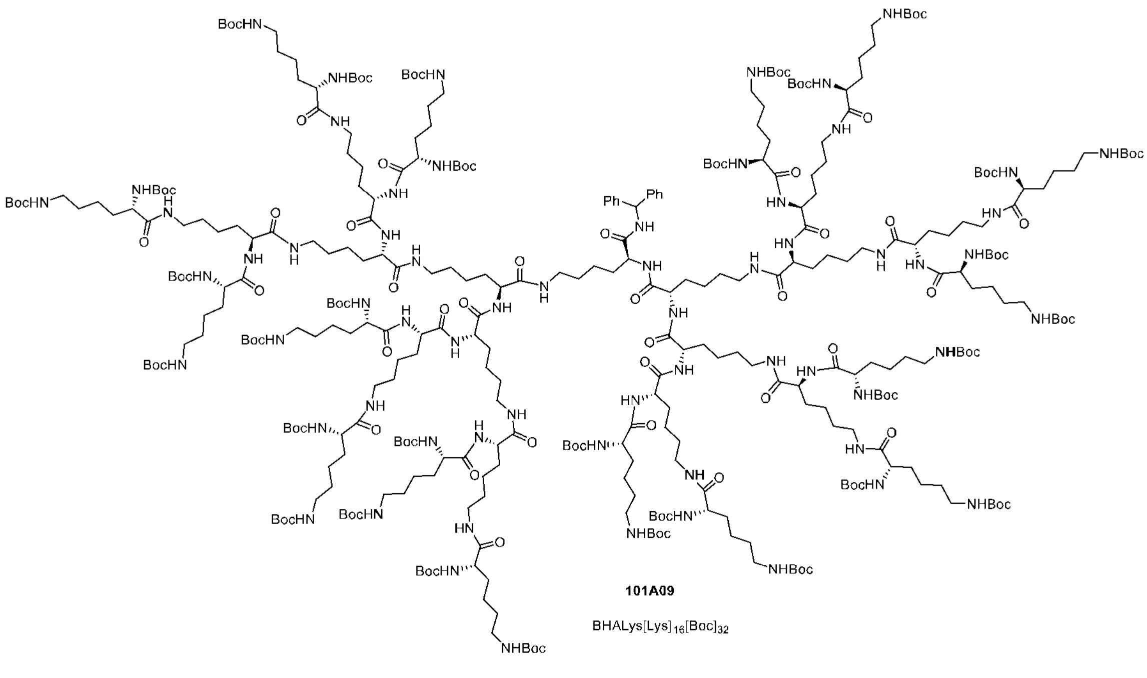

**101A09**

$BHALys[Lys]_{16}[Boc]_{32}$

To a solution of **101A08** (TFA salt, 2.50 g) in DMF (20 mL) was added DIPEA (3.94 g) in an ice water bath at 5 °C under nitrogen atmosphere. **10101** (5.94 g) was added, and the reaction mixture was stirred for 12 h. A 0.5 N sodium hydroxide solution (100 mL) was poured into the above reaction mixture, and the mixture was stirred for 1 h and filtered. The solid was collected and dried *in vacuo* to give a product **101A09** (white solid, 3.7 g, 80% yield).

Synthesis of compound 101A10:

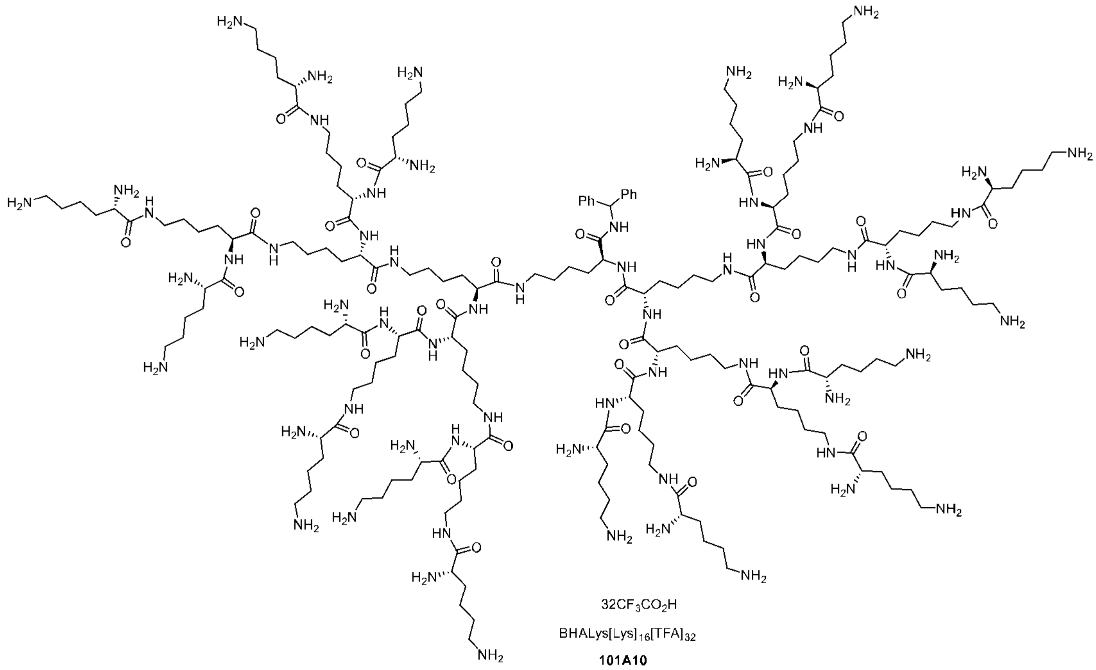

$32CF_3CO_2H$

BHALys[Lys]$_{16}$[TFA]$_{32}$

**101A10**

To a solution of **101A09** (3.0 g) in dichloromethane (10 mL) was added TFA (10 mL) at 25 °C under nitrogen atmosphere, and the mixture was stirred for 12 h. The above reaction mixture was concentrated and dissolved in acetonitrile (20 mL). MTBE (100 mL) was added for precipitation, and the mixture was filtered. The solid was collected and dried *in vacuo* to give a product **101A10** (white solid, 3.0 g, 94% yield). MS (ESI), m/z,832.1 $[M/5+H]^+$.

$^1H$ NMR (400 MHz, $D_2O$) $\delta$ 7.45 - 7.03 (m, 10H), 6.01 (s, 1H), 4.34 - 4.06 (m, 16H), 4.02 - 3.92 (m, 8H), 3.90 - 3.79 (m, 8H), 3.29 - 2.72 (m, 62H), 2.00 - 0.93 (m, 186H).

Synthesis of compound 101A11:

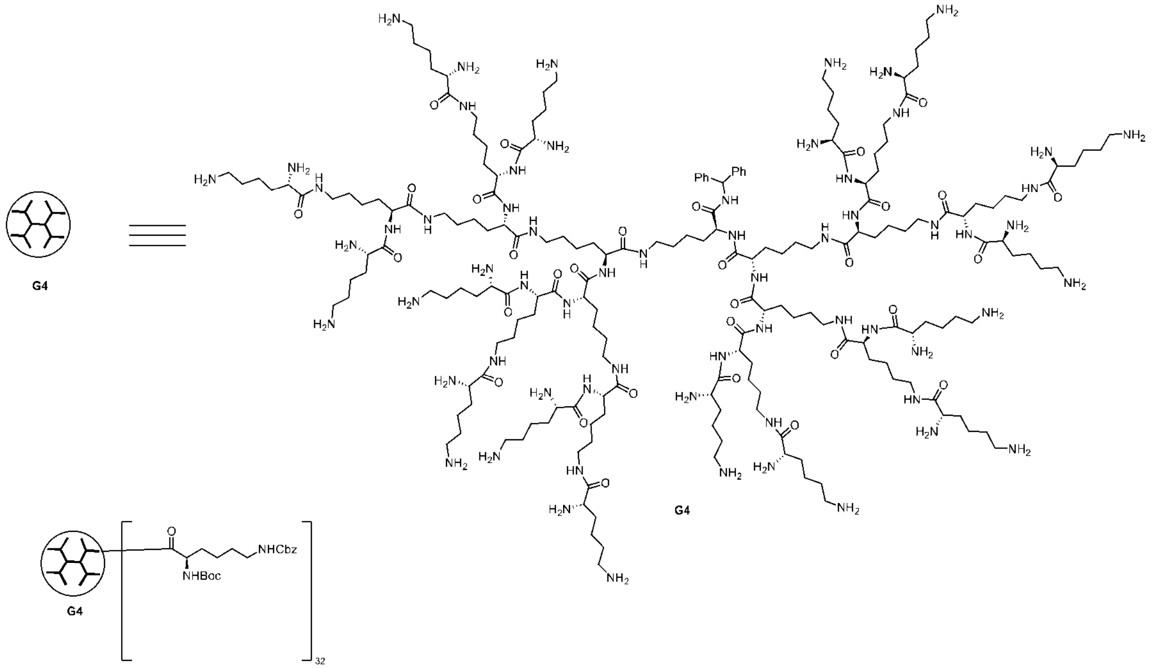

G4

G4

G4

BHALys[Lys]$_{32}$[α-Boc]$_{32}$[ε-Cbz]$_{32}$

101A11

To a solution of **101A10** (TFA salt, 2.0 g) in DMF (20 mL) was added DIPEA (3.3 g) in an ice water bath at 5 °C under nitrogen atmosphere. **10102** (4.79 g) was added in portions, and the reaction mixture was stirred for 6 h. A 0.5 N sodium hydroxide solution (100 mL) was poured into the above reaction mixture, and the mixture was stirred for 1 h and filtered. The solid was collected and suspended in $CH_3CN$ (50 mL), and an aqueous solution (200 mL) was added. The mixture was stirred for 1 h and filtered, and the procedures were repeated twice. The solid was collected and dried *in vacuo* to give a product **101A11** (white solid, 3.8 g, 94% yield).

Synthesis of compound 101A12:

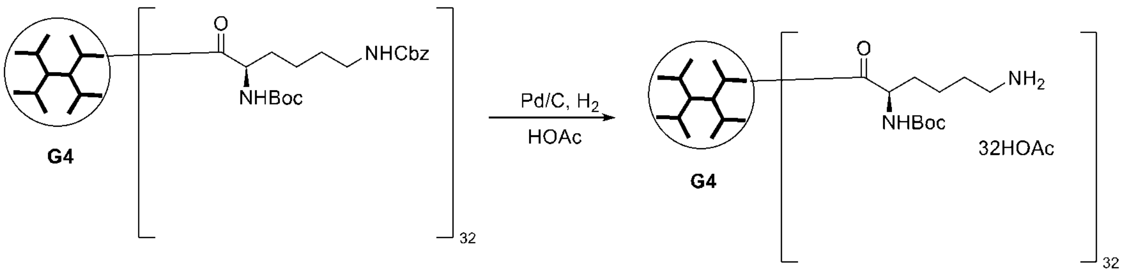


G4 G4

BHALys[Lys]$_{32}$[α-Boc]$_{32}$[ε-Cbz]$_{32}$

101A11

BHALys[Lys]$_{32}$[α-Boc]$_{32}$[HOAc]$_{32}$

101A12

To a solution of **101A11** (2.50 g, 0.16 mmol) in acetic acid (50 mL, filtered through celite if not clear) was added Pd/C or $Pd(OH)_2$ (10%, 1.0 g, 10 mL) at room temperature (25 °C) under nitrogen atmosphere. The mixture was purged with hydrogen and stirred for 24 h (30 °C) under hydrogen atmosphere (hydrogen balloon). The above reaction mixture was concentrated and dissolved in methanol (20 mL). MTBE (100 mL-200 mL) was added for precipitation, and the mixture was filtered. The solid was collected and dried *in vacuo* to give a product **101A12** (off-white solid, 1.7 g, 80% yield). MS (ESI), m/z,832.1 $[M/5+H]^+$.

$^1H$ NMR (400 MHz, $D_2O$) δ 7.53 - 6.98 (m, 10H), 6.02 (s, 1H), 4.15 (s, 34H), 3.89 (d, *J* = 26.3 Hz, 38H), 3.38 - 2.71 (m, 126H), 1.86 (s, 118H), 2.21 - 0.70 (m, 669H).

Synthesis of compound 101A13-2K

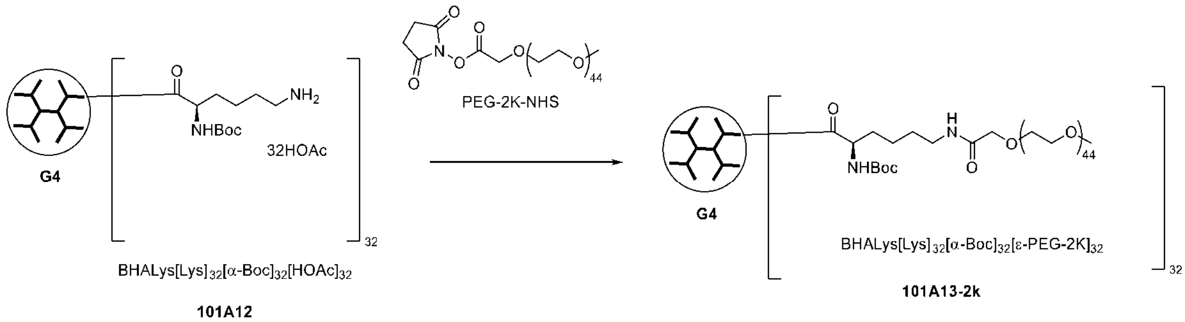


To a solution of **101A12** (1.0 g) in DMF (10 mL) was added DIPEA (0.96 g) at 25 °C under nitrogen atmosphere, and **PEG-2K-NHS** (6.48 g) was then added. The reaction mixture was stirred for 12 h. The above reaction mixture was added with MTBE (100 mL), and the mixture was filtered *in vacuo.* The cake was dried *in vacuo* to give a product **101A13-2K** (4.0 g, 70% yield).

Synthesis of compound 101A14-2K

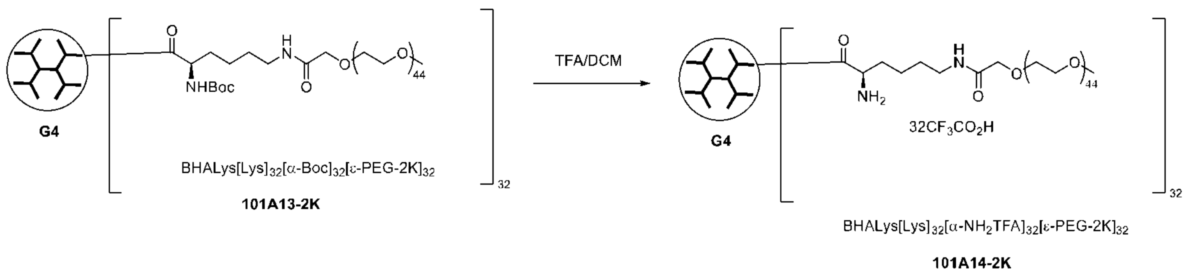


To a solution of **101A13-2K** (4.0 g) in dichloromethane (50 mL) was added TFA (30 mL) at 25 °C under nitrogen atmosphere, and the mixture was stirred for 12 h. MTBE was added, and the mixture was cooled from room temperature to -20 °C for crystallization and then filtered. The solid was collected and dried *in vacuo* to give a product **101A14-2K** (off-white solid, 3.8 g, 95% yield).

Synthesis of compound 101A15-SA-2K

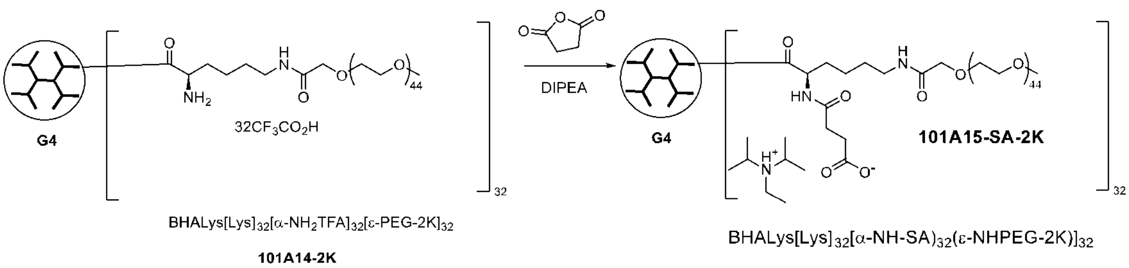


To a solution of **101A14-2K** (1.0 g, 0.013 mmol) in DMF (5 mL) was added DIPEA (166 mg, 1.29 mmol, 100.0 eq) at 25 °C under nitrogen atmosphere, and succinic anhydride (82 mg, 0.82 mmol, 64.0 eq) was then added. The mixture was stirred for 12 h. After the reaction was completed, MTBE (50 mL) was added, and the mixture was stirred for 10 min. The supernatant was removed, and the procedures were repeated thrice. The residue was dried *in vacuo* to give a product **101A15-SA-2K** (0.8 g, 76% yield).

Synthesis of compound 41

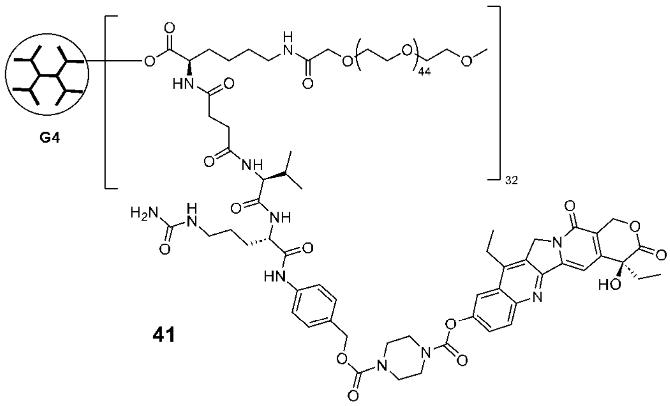

41

To a solution of **101A15-SA-2K** (838 mg, 0.01 mmol, 1.0 eq) in DMF (5 mL) were added DIPEA (170 mg, 1.32 mmol, 128.0 eq), **1-10-SM** (451 mg, 0.49 mmol, 48.0 eq), and PyBOP (429 mg, 0.82 mmol, 80.0 eq) in an ice water bath at 5 °C under nitrogen atmosphere, and the mixture was warmed to room temperature and stirred for 12 h. When HPLC indicated the completion of the reaction, MTBE (50 mL) was added, and the mixture was stirred for 10 min. The supernatant was removed, and the residue was purified by ultrafiltration (30K MW) and lyophilized to give a final product **41** (900 mg, pale yellow solid, 99.3% purity by HPLC).

Example 79: Synthesis of Compound 42

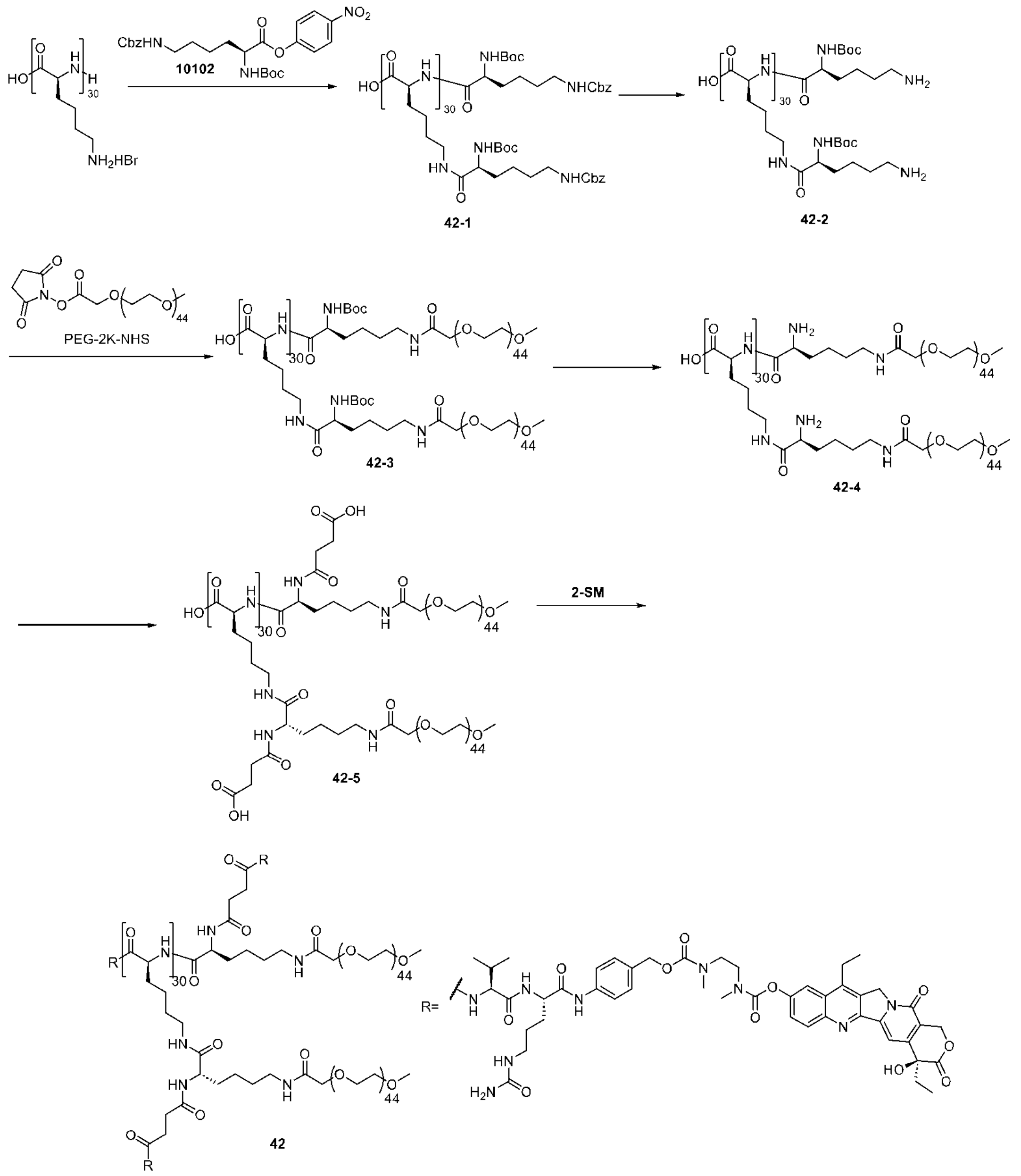


42-1

42-2

42-3

42-4

42-5

42

## Synthesis of compound 42-1

To a solution of poly-L-lysine hydrobromide (2 g, 9.56 mmol) in water (3 mL) were added DIPEA (3.7 g, 28.7 mmol) and DMSO (40 mL) under nitrogen atmosphere. **10102** (7.2 g, 14.35 mmol) was added, and the mixture was stirred for 16 h at room temperature. Acetonitrile (100 mL) was added for precipitation, and the mixture was filtered. The cake was collected to give a white solid (5.45 g).

$^1H$ NMR (400 MHz, DMSO-$d_6$) δ 7.99 - 7.57 (m, 58H), 7.48 - 7.12 (m, 191H), 7.02 - 6.83 (m, 28H), 5.16 - 4.84 (m, 66H), 4.30 - 4.06 (m, 31H), 3.97 - 3.74 (m, 34H), 3.12 - 2.82 (m, 133H), 1.77 - 1.01 (m, 708H).

## Synthesis of compound 42-2

A mixture of **42-1** (5.45 g, 11.11 mmol) and acetic acid (55 mL) was dissolved by heating, and methanol (55 mL) and 10% palladium on carbon (927 mg) were added. The mixture was purged with hydrogen thrice, stirred for 16 h, and filtered through celite, and the filtrate was triturated with MTBE and filtered. The solid was dried *in vacuo* to give a white solid (1.77 g).

$^1H$ NMR (400 MHz, $D_2O$) δ 4.27 - 4.19 (m, 30H), 4.09 - 3.94 (m, 30H), 3.31 - 3.11 (m, 69H), 3.01 (t, $J$ = 7.6 Hz, 62H), 1.86 - 1.29 (m, 646H).

**Synthesis of compound 42-3**

To a solution of **42-2** (900 mg, 0.16 mmol) in methanol (25 mL) was added a solution of PEG-2K-NHS (5.6 g, 2.64 mmol) in acetonitrile (25 mL), and DIPEA (1.4 g, 10.8 mmol) was added. The mixture was stirred at room temperature for 30 min and purified by ultrafiltration, and the ultrafiltrate was lyophilized to give a white solid (4.74 g, 99% purity by GPC).

**Synthesis of compound 42-4**

A mixture of **42-3** (1 g, 0.42 mmol), TFA (3 mL), and DCM (7 mL) was stirred at room temperature for 16 h. To MTBE (90 mL) was added the reaction mixture slowly for precipitation, and a large amount of solid was precipitated. The mixture was filtered and dried *in vacuo* to give a white solid (1 g).

**Synthesis of compound 42-5**

To a solution of **42-4** (1 g, 0.42 mmol) in DMF (8 mL) was added succinic anhydride (63 mg, 0.63 mmol), and DIPEA (326 mg, 2.52 mmol) was added. The mixture was stirred at room temperature for 16 h. To MTBE (90 mL) was added the reaction mixture slowly dropwise, and a large amount of solid was precipitated. The mixture was filtered, and the cake was washed twice with MTBE. The solid was dried *in vacuo* to give a white solid (799 mg).

**Synthesis of compound 42**

To a solution of **42-5** (778 mg, 0.33 mmol), **2-SM** (525 mg, 0.57 mmol), HOBt (89 mg, 0.66 mmol), and PyBOP (342 mg, 0.66 mmol) in DMF (8 mL) was added NMM (150 mg, 1.48 mmol). The mixture was stirred at room temperature for 16 h. To MTBE (80 mL) was added the reaction mixture slowly dropwise, and a large amount of solid was precipitated. The solid was collected and dissolved in methanol (40 mL) and water (40 mL), and the mixture was purified by ultrafiltration. The ultrafiltrate was lyophilized to give a white solid (623 mg, 99% purity by HPLC, 11.71% SN38 conjugation content).

Example 80: Synthesis of Compound 43

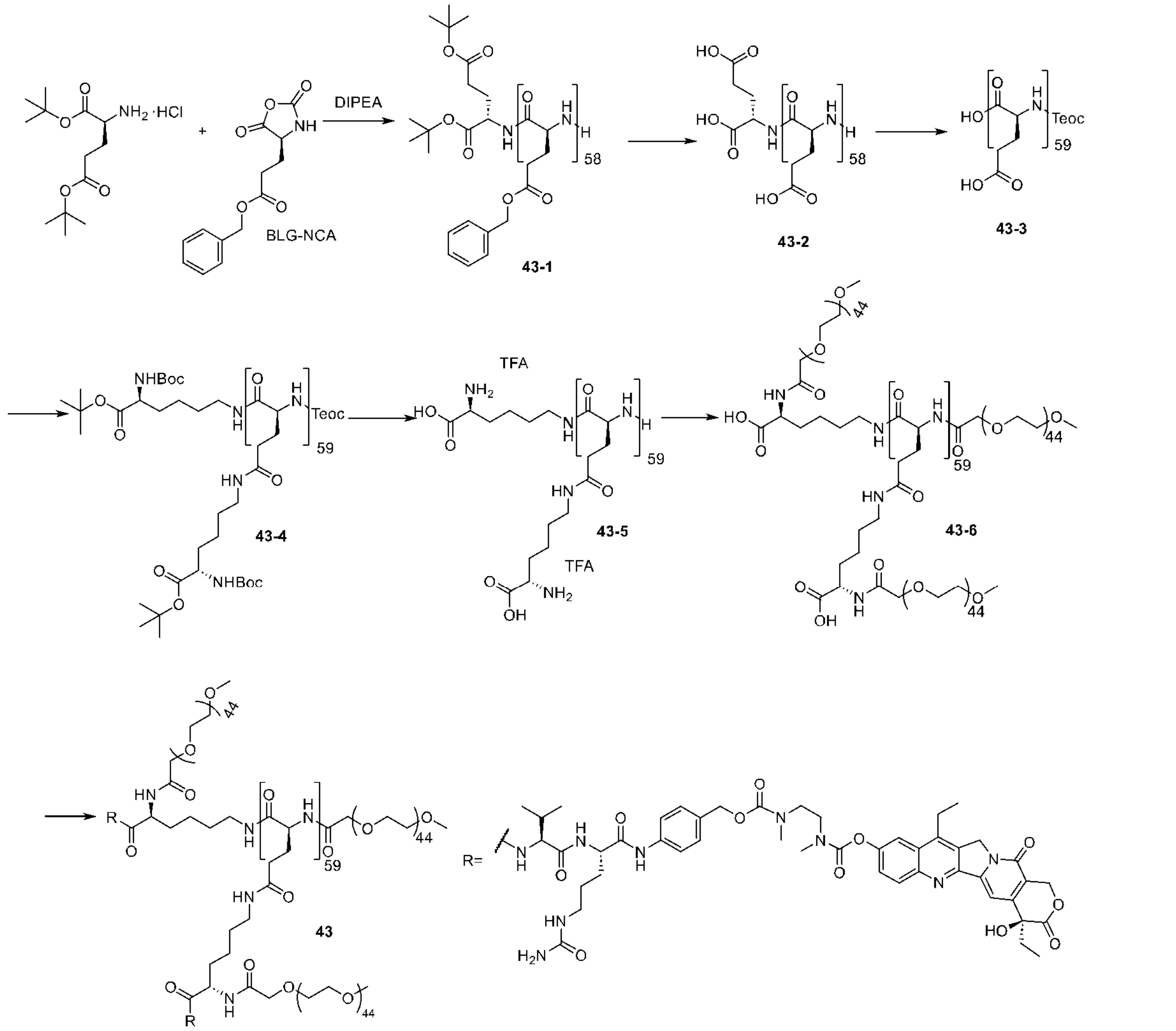


BLG-NCA, 43-1, 43-2, 43-3, 43-4, 43-5, 43-6, 43

**Synthesis of compound 43-1**

To a solution of BLG-NCA (5.694 g, 40.0 eq) and di-tert-butyl glutamate hydrochloride (160 mg, 1.0 eq) in anhydrous DMF (56 mL) were added molecular sieve (4.14 g) and DIPEA (94 μL) in an ice bath under nitrogen atmosphere. The mixture was stirred for 2 h and filtered, and to the filtrate was added methanol for precipitation. The mixture was centrifuged to give a solid, and the solid was dried *in vacuo* to give a product (3.30 g, 17.3 mmol, 70%, 58.8 polymerization degree (Benzyl $CH_2$) characterized by NMR).

$^1$H NMR (400 MHz, TFA-*d*) δ 8.09 - 7.84 (m, 327H), 5.95 - 5.71 (m, 122H), 5.57 - 5.31 (m, 64H), 3.46 - 3.07 (m, 138H), 3.07 - 2.56 (m, 133H).

**Synthesis of compound 43-2**

**43-1** was dissolved in TFA (4.6 mL), and the mixture was cooled to 0 °C. Hydrobromic acid (2.25 mL) was added, and the mixture was stirred at 0 °C overnight. MTBE (5V) was added for precipitation, and the mixture was filtered *in vacuo* and dried *in vacuo* to give a white powder (773.5 mg, more than 99% yield).

$^1$H NMR (400 MHz, $D_2O$) δ 4.41 - 4.23 (m, 59H), 3.20 (s, 19H), 2.99 (s, 15H), 2.83 (d, *J* = 0.8 Hz, 13H), 2.24 (dd, *J* = 6.3, 2.6 Hz, 131H), 2.11 - 1.80 (m, 130H), 1.22 (s, 25H), 1.19 (s, 57H).

**Synthesis of compound 43-3**

To a solution of **43-2** (400 mg) in DMF (5 mL) was added DIPEA (0.54 mL). Teoc-NHS (136 mg) was then added. The mixture was stirred at room temperature for 2 h, and MTBE was added for precipitation. The mixture was centrifuged, and the solid was collected and dried *in vacuo* to give a product (415 mg).

**Synthesis of compound 43-4**

To a solution of **43-3** (0.42 g, 3.21 mmol, 1.0 eq), HOBt (0.12 g, 0.90 mmol, 0.28 eq), and N2-tert-butoxycarbonyl-L-lysine tert-butyl ester (1.26 g, 4.18 mmol, 1.3 eq) in DMF (5 mL) were added PyBOP (3.18 g, 6.11 mmol, 1.9 eq) and NMM (1.48 mL, 14.1 mmol, 4.4 eq). The mixture was stirred at room temperature for 2 h. MTBE (100 mL) was added for precipitation, and the mixture was centrifuged and dried *in vacuo* to give a yellow solid (927.1 mg, 69.8%).

$^{1}$H NMR (400 MHz, DMSO) δ 8.50 - 7.45 (m, 98H), 7.28 - 6.86 (m, 39H), 6.71 (d, *J* = 6.6 Hz, 10H), 4.51 - 3.88 (m, 48H), 3.85 - 3.47 (m, 48H), 3.14 - 2.86 (m, 99H), 2.10 (s, 80H), 1.94 - 1.79 (m, 82H), 1.78 - 1.65 (m, 48H), 1.65 - 1.46 (m, 104H), 1.44 - 1.13 (m, 964H).

**Synthesis of compound 43-5**

To a solution of **43-4** (0.74 g, 1.79 mmol) in DCM (6 mL) was added TFA (6 mL). The mixture was stirred at room temperature for 8 h and concentrated. MTBE was added for precipitation, and the mixture was centrifuged. The solid was collected and dried *in vacuo* to give a pale yellow solid (705 mg).

$^{1}$H NMR (400 MHz, $D_2O$) δ 4.48 - 4.20 (m, 47H), 4.15 - 3.90 (m, 51H), 3.29 - 3.07 (m, 130H), 2.34 (br, 89H), 2.21 - 1.82 (m, 193H), 1.67 - 1.33 (m, 200H).

**Synthesis of compound 43-6**

To a solution of **43-5** (0.51 g, 1.38 mmol, 1.0 eq) in water (3.8 mL) was added DIPEA (0.84 mL, 4.84 mmol, 3.5 eq). To the above solution was added a solution of PEG-2K-NHS (2.93 g, 1.38 mmol, 1.0 eq) in acetonitrile (8 mL) dropwise, and the mixture was stirred at room temperature for 3 h. The reaction was monitored by GPC. The reaction mixture was purified by ultrafiltration (30K MW) with 40% ethanol, and the concentrate was collected, concentrated, and lyophilized to give a product (2.2 g, 71% yield).

$^{1}$H NMR (400 MHz, $D_2O$) δ 4.30 (br, 92H), 4.13 (s, 99H), 4.01 - 3.49 (m, 8017H), 3.40 (d, *J*= 1.3 Hz, 151H), 3.29 - 3.05 (m, 145H), 2.34 (br, 89H), 2.18 - 1.66 (m, 178H), 1.63 - 1.44 (m, 106H).

**Synthesis of compound 43**

To a solution of **43-6** (0.87 g, 0.38 mmol, 1.0 eq), 2-SM (0.63 g, 0.69 mmol, 1.8 eq), and HOBt (0.11 g, 0.76 mmol, 2.0 eq) in DMF (6 mL) were added PyBOP (0.40 g, 0.76 mmol, 2.0 eq) and NMM (0.17 g, 1.72 mmol, 4.5 eq). The mixture was stirred at room temperature for 1 h, and MTBE was added for precipitation. The solid was collected, dried, and then dissolved in 40% ethanol, and the mixture was purified by ultrafiltration (30K MW). The concentrate was collected, concentrated, and lyophilized to give a white solid (997 mg, 82.5% yield, 11.76% SN-38 conjugation content).

**Example 81: Synthesis of Compound 44**

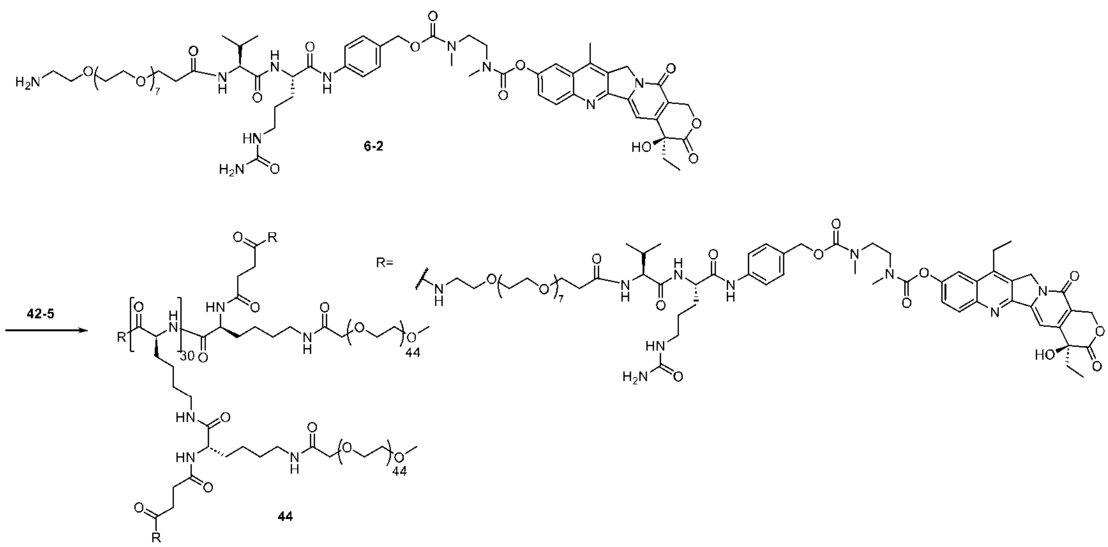


6-2

44

**Synthesis of compound 42**

To a solution of **42-5** (778 mg, 0.33 mmol), **6-2** (750 mg, 0.57 mmol), HOBt (89 mg, 0.66 mmol), PyBOP (342 mg, 0.66 mmol) in DMF (8 mL) was added NMM (150 mg, 1.48 mmol), and the mixture was stirred for 16 h at room temperature. MTBE (50 mL) was added, and the mixture was stirred for 30 min. The supernatant was discarded, and the residue was dried and dissolved in methanol (40 mL) and water (40 mL). The mixture was purified by ultrafiltration (30K MW, in a methanol/water ultrafiltrate). The ultrafiltrate was lyophilized to give a pale yellow solid (520 mg, 98.2% purity by HPLC, 11.2% SN38 conjugation content).

**Example 82: Synthesis of Compound 45**

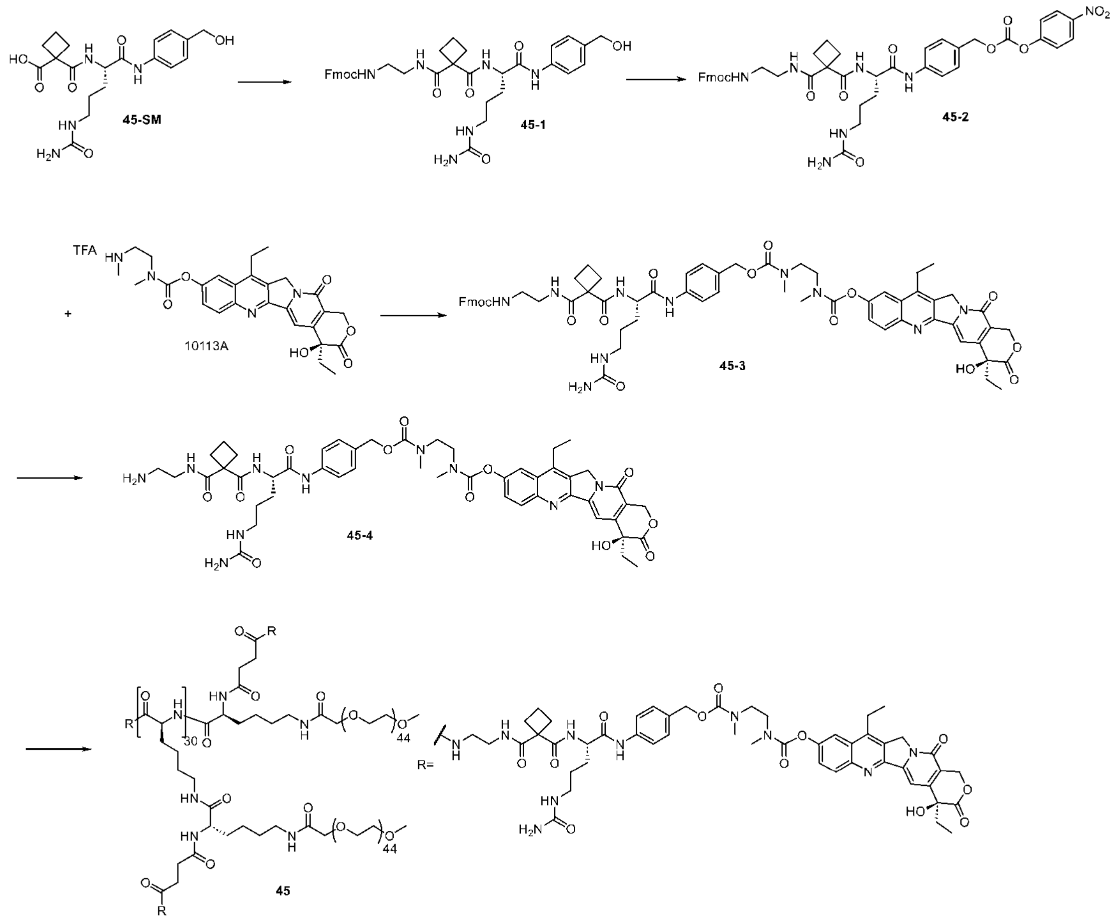

**Synthesis of compound 45-1**

To a solution of **45-SM** (synthesized according to the method for compound 13 in Patent WO2015095223A2, 1.0 g, 2.46 mmol, 1.0 eq) and Fmoc-ethylenediamine (0.83 g, 2.96 mmol, 1.2 eq) in anhydrous DMF (10 mL) were added HATU (1.40 g, 3.70 mmol, 1.5 eq) and DIPEA (0.95 g, 7.39 mmol, 3.0 eq) in an ice bath under nitrogen atmosphere. The mixture was warmed to room temperature and stirred for 12 h. The reaction was quenched with water, and the reaction mixture was extracted with dichloromethane. The organic phase was washed with brine, dried over anhydrous sodium sulfate, and filtered. The filtrate was concentrated and purified by column chromatography (dichloromethane and methanol system) to give a product (1.4 g, 85% yield).
MS (ESI), m/z, 671.3 $[M+H]^+$.

**Synthesis of compound 45-2**

To a solution of **45-1** (1.4 g, 2.09 mmol, 1.0 eq) in anhydrous DMF (10 mL) were added bis(4-nitrophenyl)

carbonate (0.95 g, 3.13 mmol, 1.5 eq) and DIPEA (0.81 g, 6.27 mmol, 3.0 eq) in an ice bath under nitrogen atmosphere. The mixture was warmed to room temperature and stirred for 2 h. The reaction was quenched with water, and the reaction mixture was extracted with dichloromethane. The organic phase was washed with brine, dried over anhydrous sodium sulfate, and filtered. The filtrate was concentrated to give a crude product (1.6 g, 92% yield).
MS (ESI), m/z, 836.2 $[M+H]^+$.

**Synthesis of compound 45-3**

To a solution of **45-2** (1.6 g, 1.92 mmol, 1.0 eq) in anhydrous DMF (10 mL) were added **10113A** (prepared according to the method for compound 10113A in Patent WO2023078464A1), bis(4-nitrophenyl) carbonate, (1.16 g, 1.92 mmol, 1.0 eq), and DIPEA (0.74 g, 5.75 mmol, 3.0 eq) in an ice bath under nitrogen atmosphere. The mixture was warmed to room temperature and stirred for 12 h. The reaction was quenched with water, and the reaction mixture was extracted with dichloromethane. The organic phase was washed with brine, dried over anhydrous sodium sulfate, and filtered. The filtrate was concentrated and purified by column chromatography (dichloromethane and methanol system) to give a product (2.0 g, 87% yield).
MS (ESI), m/z, 1203.2 $[M+H]^+$.

**Synthesis of compound 45-4**

To a solution of **45-3** (1.9 g, 1.58 mmol, 1.0 eq) in anhydrous DMF (10 mL) was added diethylamine (3 mL) in an ice bath under nitrogen atmosphere. The mixture was warmed to room temperature and stirred for 12 h. The mixture was concentrated and purified directly by reversed-phase column chromatography (0.05% TFA $H_2O$ and methanol) to give a product (1.3 g, 84% yield).
MS (ESI), m/z, 981.1 $[M+H]^+$.

**Synthesis of compound 45**

To a solution of **42-5** (700 mg, 0.32 mmol) and **45-4** (470 mg, 0.48 mmol) in DMF (10 mL) were added PyBOP (312 mg, 0.60 mmol) and DIPEA (190 mg, 1.48 mmol) at room temperature under nitrogen atmosphere, and the mixture was stirred for 20 h. MTBE (50 mL) was added, and the mixture was stirred for 30 min. The supernatant was discarded, and the residue was dried and dissolved in methanol (50 mL) and water (50 mL). The mixture was purified by ultrafiltration (30K MW, in a methanol/water ultrafiltrate). The ultrafiltrate was lyophilized to give a pale yellow solid (630 mg, 99.3% purity by HPLC, 9.8% SN38 conjugation content).

**Example 83: Synthesis of Compound 46**

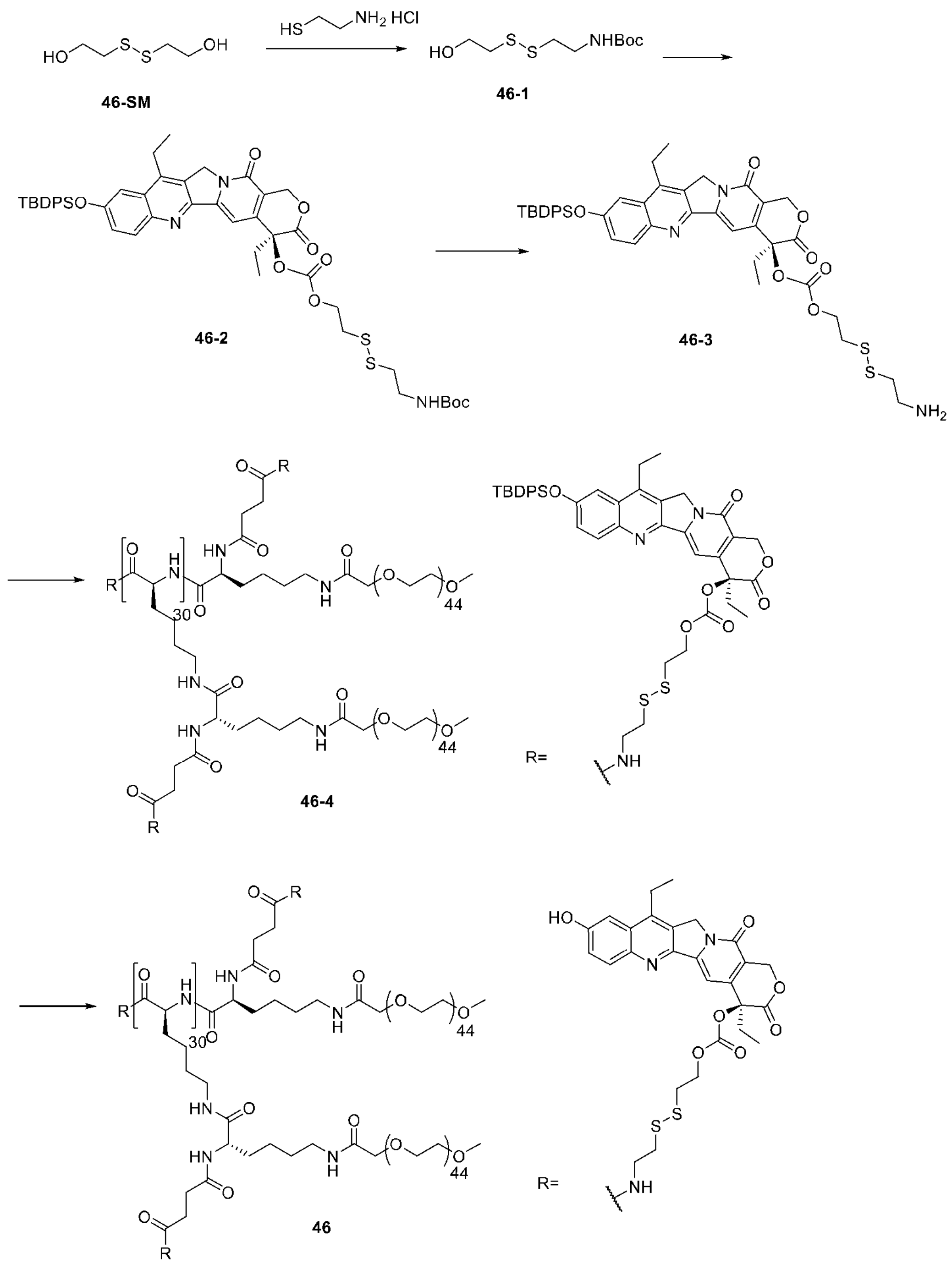

46-SM

46-1

46-2

46-3

46-4

46

## Synthesis of compound 46-1

To a solution of cysteamine hydrochloride (1.50 g, 13.2 mmol, 1.0 eq) in methanol (18 mL) was added a solution of triethylamine (1.85 mL, 26.4 mmol, 2.0 eq) and 2-hydroxyethyl disulfide (1.50 g, 13.2 mmol, 1.0 eq) in dichloromethane in an ice bath under nitrogen atmosphere. The mixture was stirred at room temperature for 16 h. The above solution was cooled to 0 °C, and di-tert-butyl dicarbonate (4.32 g, 19.8 mmol, 1.5 eq) was added. The mixture was stirred at room temperature for 4 h. When LCMS detection indicated the termination of the reaction, the reaction mixture was directly concentrated, and purified by column chromatography (petroleum ether:ethyl acetate = 3:1) to give a compound (colorless oil, 0.9 g, 26.9% yield).

MS (ESI), m/z, 154.0 $[M-100]^+$.

**Synthesis of compound 46-2**

To a solution of **46-1** (1.90 g, 7.5 mmol, 1.5 eq) in $CH_2Cl_2$ (25 mL) were added $Et_3N$ (757 mg, 7.5 mmol, 1.5 eq) and bis(4-nitrophenyl) carbonate (2.28 g, 7.5 mmol, 1.5 eq) sequentially at 0 °C. The mixture was stirred at the temperature for 2 h. To the above reaction mixture were added DMAP (610 mg, 5.00 mmol, 1.0 eq) and 10-TBDPS-protected SN38 (3.15 g, 5.00 mmol, 1.0 eq). The mixture was stirred at room temperature for 2 h. After the reaction was completed, the reaction mixture was poured into sat. aq. $NH_4Cl$ and washed thoroughly (150 mL × 3). The organic phases were combined, dried over anhydrous $Na_2SO_4$, concentrated *in vacuo,* and subjected to silica gel column chromatography with PE/EA = 1:1 as the eluent to give a product (2.1 g).

MS (ESI), m/z, 909.1 $[M+H]^+$.

**Synthesis of compound 46-3**

A solution of **46-2** (1.7 g, 1.80 mmol, 1.0 eq) in formic acid (20 mL) was stirred at 0 °C for 2 h. After the reaction was completed, the reaction mixture was concentrated *in vacuo* and purified by C18 reversed-phase column chromatography with $CH_3CN/H_2O$ = 5-75% to give a yellow solid (1.2 g).

MS (ESI), m/z, 809.0 $[M+H]^+$.

**Synthesis of compound 46-4**

To a solution of **42-5** (700 mg, 0.32 mmol) and **46-3** (390 mg, 0.48 mmol) in DMF (10 mL) were added PyBOP (312 mg, 0.60 mmol) and DIPEA (190 mg, 1.48 mmol) at room temperature under nitrogen atmosphere, and the mixture was stirred for 20 h. MTBE (50 mL) was added, and the mixture was stirred for 30 min. The supernatant was discarded, and the residue was dried to give a crude product (900 mg), which was directly used in the next step.

**Synthesis of compound 46**

To a solution of **46-4** (900 mg) in THF (5 mL) was added a TBAF solution (1.0 M in THF, 1.0 mL) at room temperature under nitrogen atmosphere, and the mixture was stirred at room temperature for 20 h. MTBE (50 mL) was added, and the mixture was stirred for 30 min. The supernatant was discarded, and the residue was dried and dissolved in methanol (50 mL) and water (50 mL). The mixture was purified by ultrafiltration (30K MW, in a methanol/water ultrafiltrate). The ultrafiltrate was lyophilized to give a pale yellow solid (520 mg, 99.3% purity by HPLC, 11.0% SN38 conjugation content).

**Example 84: Synthesis of Compound 47**

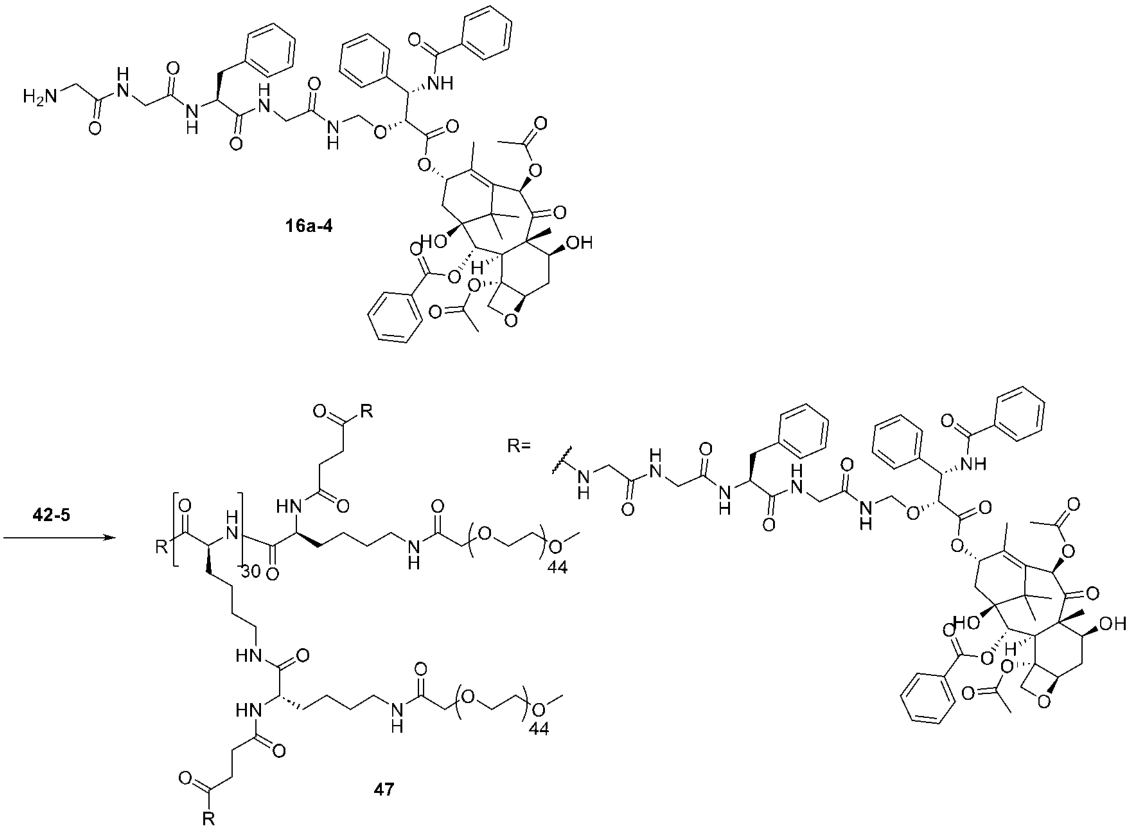


### Synthesis of compound 47

To a solution of **42-5** (700 mg, 0.32 mmol) and **16a-4** (577 mg, 0.48 mmol) in DMF (10 mL) were added PyBOP (312 mg, 0.60 mmol) and DIPEA (190 mg, 1.48 mmol) at room temperature under nitrogen atmosphere, and the mixture was stirred for 24 h. MTBE (50 mL) was added, and the mixture was stirred for 30 min. The supernatant was discarded, and the residue was dried and dissolved in methanol (60 mL) and water (40 mL). The mixture was purified by ultrafiltration (30K MW, in a methanol/water ultrafiltrate). The ultrafiltrate was lyophilized to give a white solid (580 mg, 97.8% purity by HPLC, 19.0% PTX conjugation content).

### Example 85: Synthesis of Compound 48

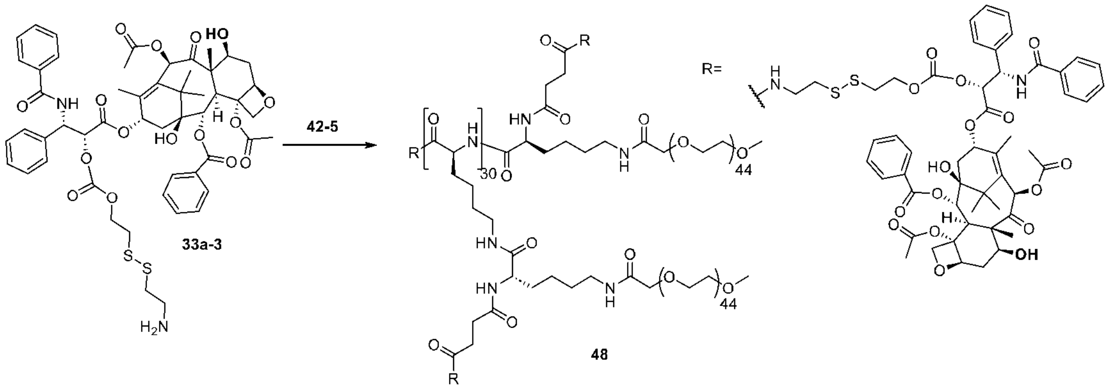


### Synthesis of compound 48

To a solution of **42-5** (700 mg, 0.32 mmol) and **33a-3** (516 mg, 0.50 mmol) in DMF (10 mL) were added PyBOP

(312 mg, 0.60 mmol) and NMM (150 mg, 1.48 mmol) at room temperature under nitrogen atmosphere, and the mixture was stirred for 12 h. MTBE (50 mL) was added, and the mixture was stirred for 30 min. The supernatant was discarded, and the residue was dried and dissolved in methanol (60 mL) and water (40 mL). The mixture was purified by ultrafiltration (30K MW, in a methanol/water ultrafiltrate). The ultrafiltrate was lyophilized to give a white solid (580 mg, 98.5% purity by HPLC, 19.5% PTX conjugation content).

**Example 86: Synthesis of Compound 49**

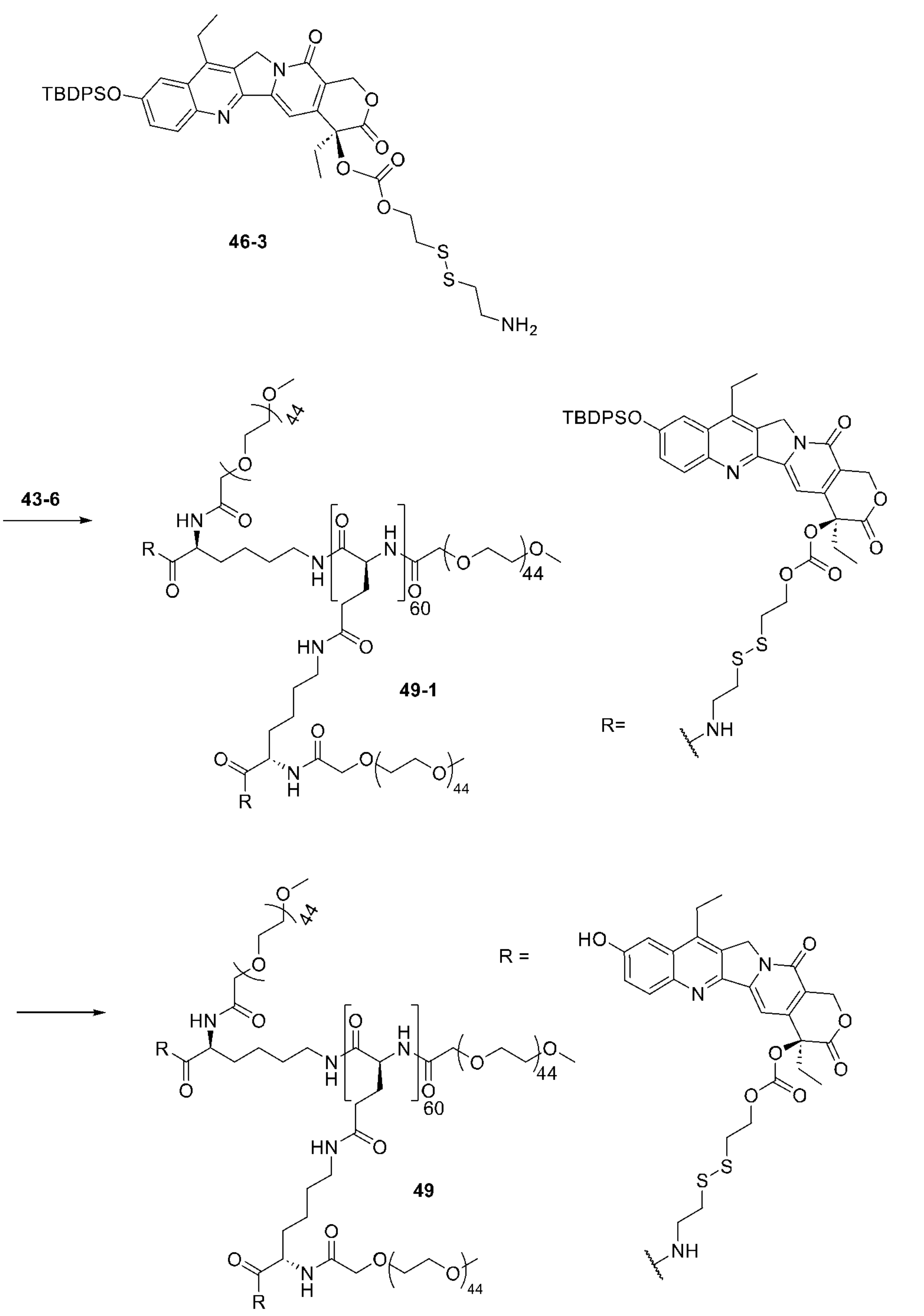


46-3

49-1

49

**Synthesis of compound 49-1**

To a solution of **43-6** (0.87 g, 0.38 mmol, 1.0 eq) and **46-3** (0.56 g, 0.69 mmol, 1.8 eq) in DMF (10 mL) were added PyBOP (0.40 g, 0.76 mmol, 2.0 eq) and NMM (0.17 g, 1.72 mmol, 4.5 eq) at room temperature under nitrogen atmosphere, and the mixture was stirred at room temperature for 12 h. MTBE (50 mL) was added, and the mixture was stirred for 30 min. The supernatant was discarded, and the residue was dried to give a crude product (1.1 g), which was directly used in the next step.

**Synthesis of compound 49**

To a solution of **49-1** (1.1 g, crude) in THF (10 mL) was added a TBAF solution (1.0 M in THF, 2.0 mL) at room temperature under nitrogen atmosphere, and the mixture was stirred at room temperature for 20 h. MTBE (50 mL) was added, and the mixture was stirred for 30 min. The supernatant was discarded, and the residue was dried and dissolved in methanol (50 mL) and water (50 mL). The mixture was purified by ultrafiltration (30K MW, in a methanol/water ultrafiltrate). The ultrafiltrate was lyophilized to give a pale yellow solid (730 mg, 99.5% purity by HPLC, 10.5% SN38 conjugation content).

**Example 87: Synthesis of Compound 50**

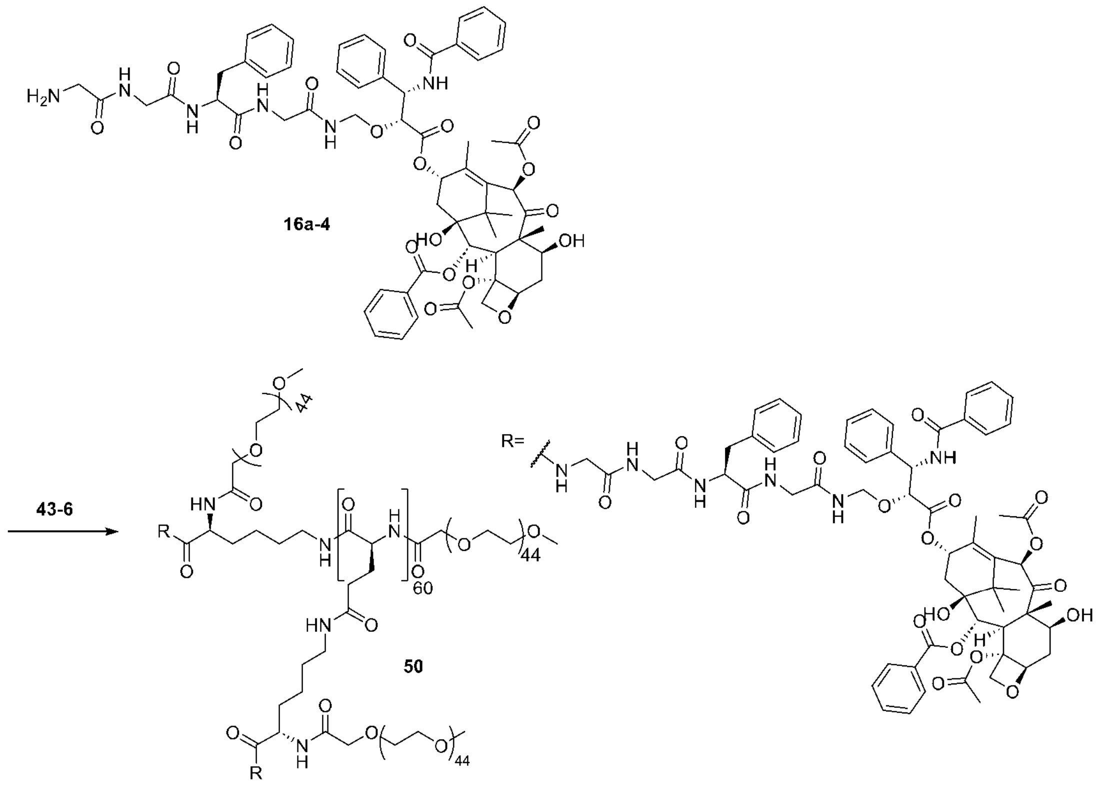


16a-4

50

**Synthesis of compound 50**

To a solution of **43-6** (730 mg, 0.32 mmol) and **16a-4** (577 mg, 0.48 mmol) in DMF (10 mL) were added PyBOP (312 mg, 0.60 mmol) and DIPEA (190 mg, 1.48 mmol) at room temperature under nitrogen atmosphere, and the mixture was stirred for 24 h. MTBE (50 mL) was added, and the mixture was stirred for 30 min. The supernatant was discarded, and the residue was dried and dissolved in methanol (50 mL) and water (50 mL). The mixture was purified by ultrafiltration (30K MW, in a methanol/water ultrafiltrate). The ultrafiltrate was lyophilized to give a white solid (580 mg, 99.9% purity by HPLC, 16.5% PTX conjugation content).

**Example 88: Synthesis of Compound 51**

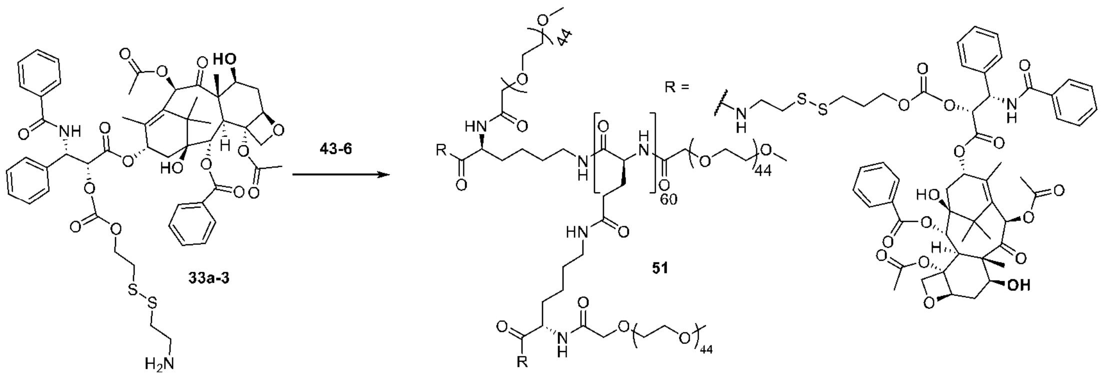


33a-3

51

**Synthesis of compound 50**

To a solution of **43-6** (730 mg, 0.32 mmol) and **33a-3** (516 mg, 0.50 mmol) in DMF (10 mL) were added PyBOP (312 mg, 0.60 mmol) and NMM (150 mg, 1.48 mmol) at room temperature under nitrogen atmosphere, and the mixture was stirred for 12 h. MTBE (50 mL) was added, and the mixture was stirred for 30 min. The supernatant was discarded, and the residue was dried and dissolved in methanol (60 mL) and water (40 mL). The mixture was purified by ultrafiltration (30K MW, in a methanol/water ultrafiltrate). The ultrafiltrate was lyophilized to give a white solid (490 mg, 97.9% purity by HPLC, 18.4% PTX conjugation content).

**Example 89: Synthesis of Compound 52**

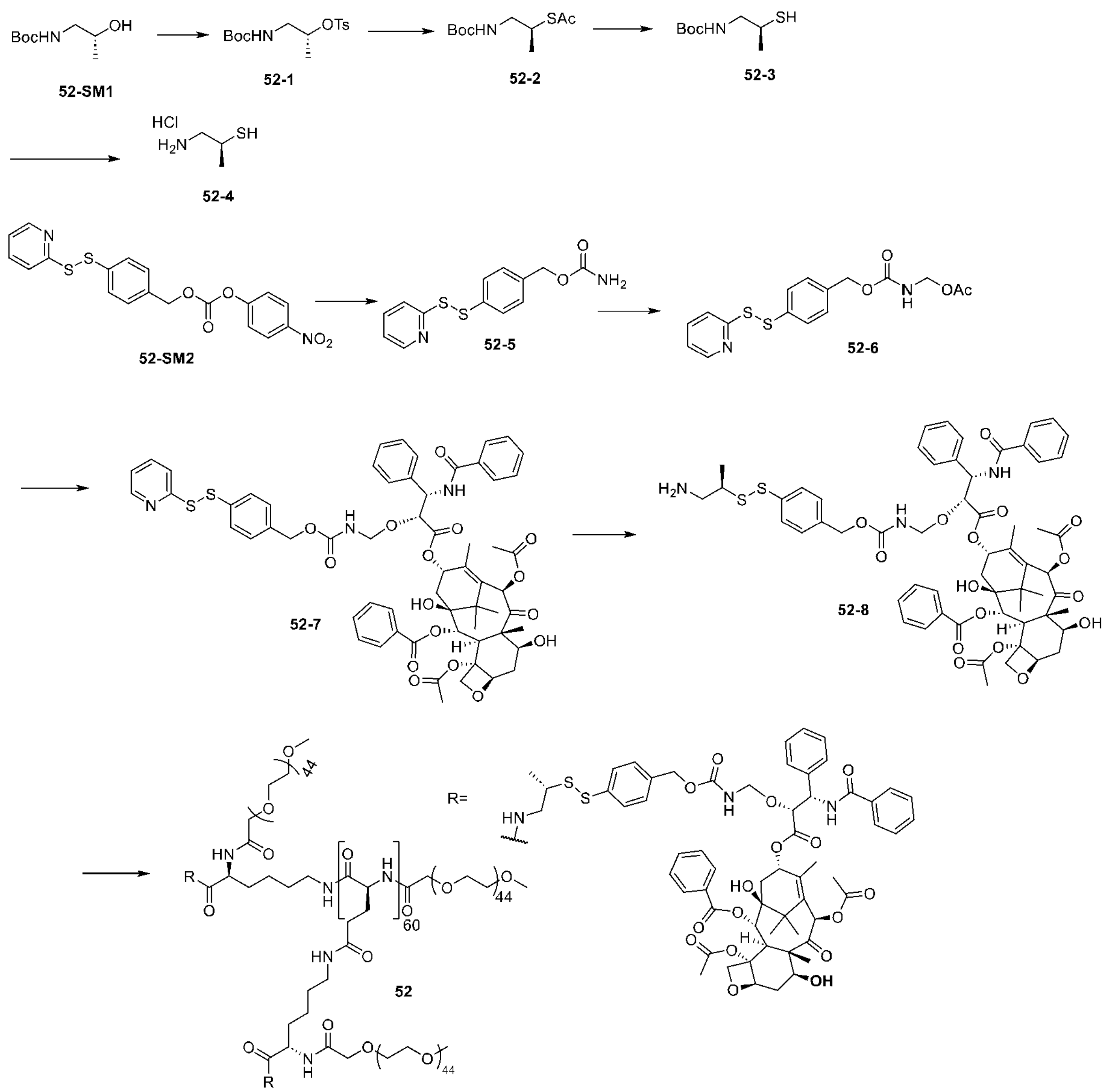

52-SM1 52-1 52-2 52-3

52-4

52-SM2 52-5 52-6

52-7 52-8

52

R=

### Synthesis of compound 52-1

To a solution of starting material (*R*)-1-(BOC-amino)-2-propanol (2.0 g, 11.41 mmol) in DCM (20 mL) were added TsCl (2.6 g, 13.70 mmol), DMAP (1.4 g, 11.41 mmol), and triethylamine (3.8 mL, 27.39 mL) separately. The mixture was stirred at room temperature for 12 h. A product was generated as detected by LCMS. The reaction mixture was washed with saturated brine and purified water, and the organic phase was dried over anhydrous sodium sulfate and concentrated *in vacuo* to give a compound **52-1** (pale yellow solid, 3.7 g, 98% yield).

MS (ESI), m/z, 330.4 $[M+H]^+$.

### Synthesis of compound 52-2

To a solution of **52-1** (2.4 g, 7.29 mmol) in DMF (10 mL) was added KSAc (998 mg, 8.74 mmol), and the mixture was heated to 60 °C and stirred for 12 h. When LCMS indicated the depletion of the starting materials, the reaction mixture was cooled to room temperature. Ethyl acetate (100 mL) was added, and the mixture was washed with water (100 mL × 2). The organic phase was dried over anhydrous sodium sulfate and concentrated *in vacuo* to give a compound **52-2** (yellow solid, 1.7 g, 100% yield).

MS (ESI), m/z, 330.4 $[M+H]^+$.

**Synthesis of compound 52-3**

To a solution of the compound **52-2** (1.7 g, 7.29 mmol) in MeOH (20 mL) was added potassium carbonate (2.0 g, 14.57 mmol). The mixture was stirred at room temperature for 12 h, filtered, and concentrated, and the crude product was purified on a normal-phase silica gel column to give **52-3** (1.0 g, 72% yield, colorless oil).

$^1$H NMR (400 MHz, Chloroform-d) δ 5.17 (s, 1H), 3.38 - 3.22 (m, 2H), 2.98 (p, J = 6.7 Hz, 1H), 1.30 (d, J = 6.9 Hz, 3H).

**Synthesis of compound 52-4**

Compound **52-3** (1.0 g) and a solution of HCl in dioxane (4.0 M, 5 mL) were stirred for 2 h at room temperature under nitrogen atmosphere and concentrated to give a product (0.6 g).

**Synthesis of compound 52-5**

To a mixed solvent of **52-SM2** (6.0 g, 14.47 mmol, 1.0 eq) in DCM (20 mL) and THF (20 mL) was added aqueous ammonia (25 wt%, 3.0 g, 21.71 mmol, 1.5 eq) at room temperature. The mixture was stirred at room temperature for 16 h. When LCMS indicated the completion of the reaction, the reaction mixture was concentrated by rotavap and purified on a silica gel column to give the product **52-5** (white solid, 3.5 g, 83% yield).

MS (ESI), m/z, 293.0 $[M+H]^+$.

**Synthesis of compound 52-6**

To a solution of **52-5** (3.5 g, 11.98 mmol, 1.0 eq) in acetic acid (15 mL) and acetic anhydride (50 mL) was added paraformaldehyde (719 mg, 23.97 mmol, 2.0 eq) at room temperature under nitrogen atmosphere. The reaction mixture was warmed to 50 °C and stirred for 16 h. When LCMS indicated the completion of the reaction, the reaction mixture was concentrated by rotavap and triturated with a mixed solvent of DCM/MTBE to give a product **52-6** (white solid, 2.5 g, 57% yield).

MS (ESI), m/z, 365.0 $[M+H]^+$.

**Synthesis of compound 52-7**

To a solution of **52-6** (2.0 g, 5.49 mmol, 2.0 eq) and paclitaxel (2.35 g, 2.75 mmol, 1.0 eq) in anhydrous tetrahydrofuran (40 mL) was added *tert*-butyllithium (1.0 M, 5.49 mL, 2.0 eq) dropwise at -78 °C under nitrogen atmosphere. After the addition, the mixture was stirred at -78 °C for 0.5 h. When LCMS indicated the completion of the reaction, the reaction was quenched with sat. aq. $NH_4Cl$, and the mixture was extracted with EA and purified on a silica gel column to give a product **52-7** (white solid, 2.0 g, 63% yield).

MS (ESI), m/z, 1158.0 $[M+H]^+$.

**Synthesis of compound 52-8**

To a solution of **52-7** (400 mg, 0.345 mmol, 1.0 eq) in methanol (15 mL) was added **52-4** (44 mg, 0.345 mmol, 1.0 eq) at room temperature under nitrogen atmosphere. After the addition, the mixture was stirred at room temperature for 2 h. When LCMS indicated the completion of the reaction, the reaction mixture was concentrated by rotavap and purified on a C18 column (TFA system) to give a product **52-8** (white solid, 200 mg, 51% yield).

MS (ESI), m/z, 1138.0 $[M+H]^+$.

**Synthesis of compound 52**

To a solution of **43-6** (730 mg, 0.32 mmol) and **52-8** (570 mg, 0.50 mmol) in DMF (10 mL) were added PyBOP (312 mg, 0.60 mmol) and NMM (150 mg, 1.48 mmol) at room temperature under nitrogen atmosphere, and the mixture was stirred for 12 h. MTBE (50 mL) was added, and the mixture was stirred for 30 min. The supernatant was discarded, and the residue was dried and dissolved in methanol (60 mL) and water (40 mL). The mixture was purified by ultrafiltration (30K MW, in a methanol/water ultrafiltrate). The ultrafiltrate was lyophilized to give a white solid (850 mg, 98.9% purity by HPLC, 17.9% PTX conjugation content).

**Example 90: Synthesis of compound 53**

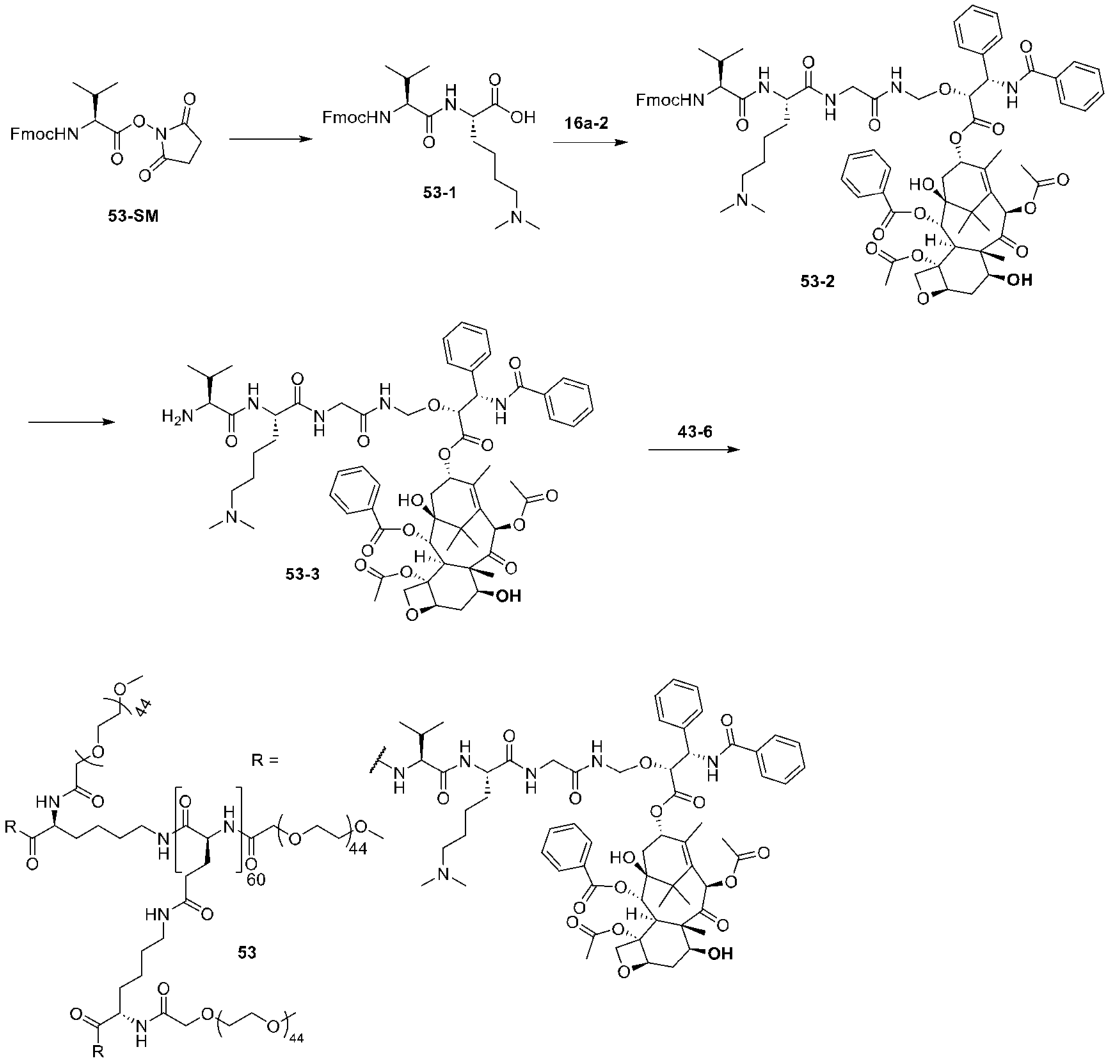


53-SM 53-1 53-2 53-3 R = 53

**Synthesis of compound 53-1**

To a solution of H-Lys(Me)2-OH·HCl (2.0 g, 9.52 mmol) in DMF (10 mL) were added **53-SM** (4.2 g, 9.52 mmol) and DIPEA (3.7 g, 28.57 mmol) at room temperature under nitrogen atmosphere, and the mixture was stirred for 12 h. The reaction was quenched with water, and the mixture was extracted with dichloromethane, concentrated, and purified by column chromatography (dichloromethane and methanol) to give a white solid (3.5 g).

MS (ESI), m/z, 496.2 $[M+H]^+$.

**Synthesis of compound 53-2**

To a solution of **16a-2** (1.5 g, 1.6 mmol, 1.0 eq) and **53-1** (950 mg, 1.91 mmol, 1.2 eq) in dry DMF (10 mL) were added EDCI (460 mg, 1.39 mmol, 1.5 eq), HOBt (323 mg, 2.39 mmol, 1.5 eq), and DIPEA (411 mg, 3.19 mmol, 2.0 eq) in an ice-water bath (5 °C) under nitrogen atmosphere, and the mixture was stirred for 2 h. The reaction was quenched with water. The mixture was extracted with dichloromethane, and the organic phase was dried, concentrated, and purified by column chromatography ($SiO_2$, 0-10% MeOH in $CH_2Cl_2$) to give a product 53-2 (1.5 g, 66% yield).

MS (ESI), m/z, 1417.0 $[M+H]^+$.

### Synthesis of compound 53-3

To a solution of **53-2** (1.2 g) in dry $CH_2Cl_2$ (10 mL) was added diethylamine (3 mL) at room temperature under nitrogen atmosphere. The mixture was stirred for 2 h, concentrated, and purified by Pre-HPLC ($CH_3CN/H_2O$, 0.05% TFA) to give a product **53-3** (900 mg, white solid, 89% yield).

MS (ESI), m/z, 1195.0 $[M+H]^+$.

### Synthesis of compound 53

To a solution of 43-6 (730 mg, 0.32 mmol) and 53-3 (600 mg, 0.50 mmol) in DMF (10 mL) were added PyBOP (312 mg, 0.60 mmol) and NMM (150 mg, 1.48 mmol) at room temperature under nitrogen atmosphere, and the mixture was stirred for 12 h. MTBE (50 mL) was added, and the mixture was stirred for 30 min. The supernatant was discarded, and the residue was dried and dissolved in methanol (60 mL) and water (40 mL). The mixture was purified by ultrafiltration (30K MW, in a methanol/water ultrafiltrate). The ultrafiltrate was lyophilized to give a white solid (890 mg, 99.2% purity by HPLC, 19.9% PTX conjugation content).

### Example 91: Synthesis of compound 54

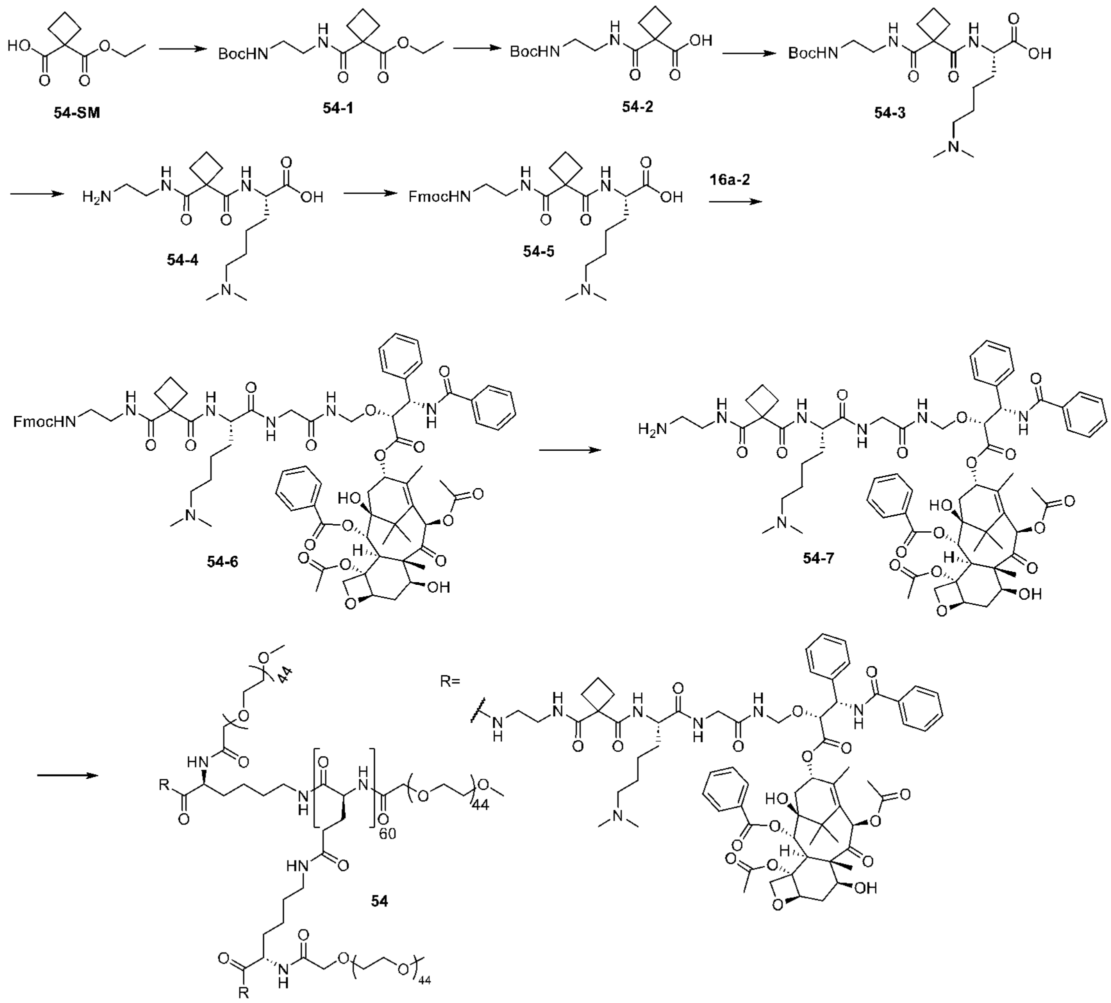


### Synthesis of compound 54-1

To a solution of **54-SM** (2.0 g, 11.6 mmol, 1.0 eq) and N-Boc-ethylenediamine (2.2 g, 14.0 mmol, 1.2 eq) in anhydrous DMF (20 mL) were added HATU (6.6 g, 17.4 mmol, 1.5 eq) and DIPEA (4.5 g, 34.9 mmol, 3.0 eq) in an ice bath under nitrogen atmosphere. The mixture was warmed to room temperature and stirred for 12 h. The reaction was

quenched with water, and the reaction mixture was extracted with dichloromethane. The organic phase was washed with brine, dried over anhydrous sodium sulfate, and filtered. The filtrate was concentrated and purified by column chromatography (dichloromethane and methanol system) to give a product (2.9 g, 79% yield).

MS (ESI), m/z, 315.2 $[M+H]^+$.

**Synthesis of compound 54-2**

To a solution of **54-1** (2.0 g) in ethanol (20 mL) was added 4.0 M NaOH (20 mL). The mixture was stirred at room temperature for 12 h, cooled in an ice-water bath, neutralized with dilute hydrochloric acid, and extracted with dichloromethane. The organic phases were combined, washed with brine, dried over anhydrous sodium sulfate, and filtered. The filtrate was concentrated to give a crude product, which was directly used in the next reaction.

MS (ESI), m/z, 287.2 $[M+H]^+$.

**Synthesis of compound 54-3**

To a solution of **54-2** (2.0 g, 7.0 mmol, 1.0 eq) and H-Lys$(Me)_2$-OH·HCl (1.8 g, 8.4 mmol, 1.2 eq) in DMF (50 mL) were added HATU (4.0 g, 10.49 mmol, 1.5 eq) and DIPEA (2.7 g, 21.0 mmol, 3.0 eq) at room temperature under nitrogen atmosphere, and the mixture was stirred for 12 h. The reaction was quenched with water, and the mixture was extracted with dichloromethane, concentrated, and purified by column chromatography (dichloromethane and methanol) to give a white solid (2.3 g).

MS (ESI), m/z, 443.2 $[M+H]^+$.

**Synthesis of compound 54-4**

To a solution of **54-3** (2.0 g) in dichloromethane (20 mL) was added TFA (7 mL), and the mixture was stirred at room temperature for 4 h, cooled in an ice-water bath, and concentrated directly to give a crude product, which was directly used in the next reaction.

MS (ESI), m/z, 343.0 $[M+H]^+$.

**Synthesis of compound 54-5**

To a solution of **54-4** (crude) in THF (40 mL) were added Fmoc-OSu (1.8 g, 1.2 eq) and DIPEA (1.7 g, 3.0 eq), and the mixture was stirred for 4 h at room temperature. The reaction was quenched with water, and the mixture was extracted with dichloromethane. The organic phase was directly concentrated and purified by column chromatography (dichloromethane and methanol) to give a white solid (1.9 g).

MS (ESI), m/z, 565.0 $[M+H]^+$.

**Synthesis of compound 54-6**

To a solution of **16a-2** (2.0 g, 2.1 mmol, 1.0 eq) and **54-5** (1.4 g, 2.6 mmol, 1.2 eq) in dry DMF (30 mL) were added EDCI (610 mg, 3.2 mmol, 1.5 eq), HOBt (431 mg, 3.2 mmol, 1.5 eq), and DIPEA (824 mg, 6.4 mmol, 3.0 eq) in an ice-water bath (5 °C) under nitrogen atmosphere, and the mixture was stirred for 2 h. The reaction was quenched with water. The mixture was extracted with dichloromethane, and the organic phase was dried, concentrated, and purified by column chromatography ($SiO_2$, 0-10% MeOH in $CH_2Cl_2$) to give a product **54-6** (2.4 g, 76% yield).

MS (ESI), m/z, 1486.2 $[M+H]^+$.

**Synthesis of compound 54-7**

To a solution of **53-6** (1.2 g) in dry $CH_2Cl_2$ (10 mL) was added diethylamine (3 mL) at room temperature under nitrogen atmosphere. The mixture was stirred for 2 h, concentrated, and purified by Pre-HPLC ($CH_3CN/H_2O$, 0.05% TFA) to give a product **54-7** (850 mg, white solid, 83 % yield).

MS (ESI), m/z, 1264.0 $[M+H]^+$.

**Synthesis of compound 54**

To a solution of **43-6** (730 mg, 0.32 mmol) and **54-7** (632 mg, 0.50 mmol) in DMF (10 mL) were added PyBOP (312 mg, 0.60 mmol) and NMM (150 mg, 1.48 mmol) at room temperature under nitrogen atmosphere, and the mixture was stirred for 12 h. MTBE (50 mL) was added, and the mixture was stirred for 30 min. The supernatant was discarded, and the

residue was dried and dissolved in methanol (60 mL) and water (40 mL). The mixture was purified by ultrafiltration (30K MW, in a methanol/water ultrafiltrate). The ultrafiltrate was lyophilized to give a white solid (1.1 g, 98.6% purity by HPLC, 18.3% PTX conjugation content).

**Example 92: Synthesis of compound 55**

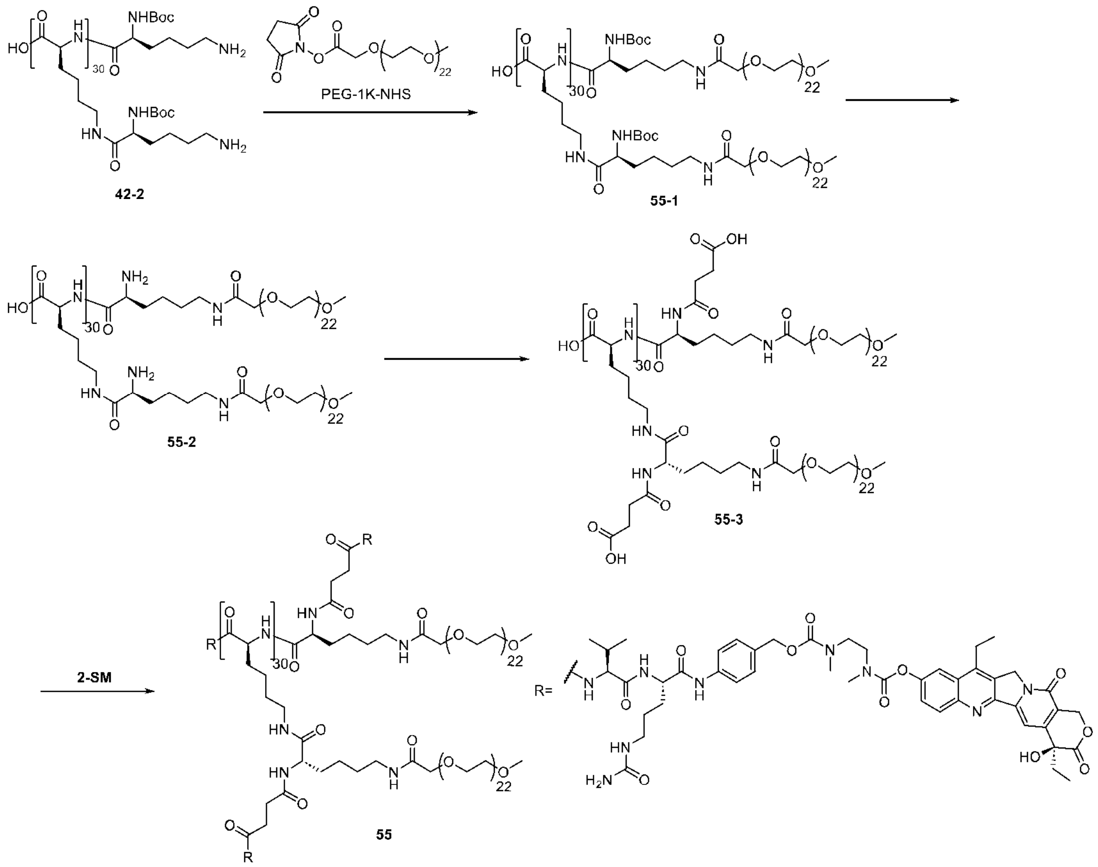


**Synthesis of compound 55-1**

To a solution of **42-2** (900 mg, 0.16 mmol) in methanol (25 mL) was added a solution of PEG-1K-NHS (prepared according to the synthetic method for compound 10104-1k in Patent No. WO2023078464A1; 2.8 g, 2.64 mmol) in acetonitrile (25 mL), and DIPEA (1.4 g, 10.8 mmol) was added. The mixture was stirred at room temperature for 30 min and purified by ultrafiltration, and the ultrafiltrate was lyophilized to give a white solid (2.5 g, 99% purity by GPC).

**Synthesis of compound 55-2**

A mixture of **55-1** (1.5 g), TFA (4 mL), and DCM (10 mL) was stirred at room temperature for 20 h and slowly added to MTBE (100 mL) for precipitation. The mixture was filtered and dried *in vacuo* to give a white solid (1.1 g).

**Synthesis of compound 55-3**

To a solution of **55-2** (0.56 g, 0.42 mmol) in ethyl acetate (8 mL) was added succinic anhydride (63 mg, 0.63 mmol), and DIPEA (326 mg, 2.52 mmol) was added. The mixture was stirred at room temperature for 12 h. To MTBE (80 mL) was added the reaction mixture slowly dropwise, and a large amount of solid was precipitated. The mixture was filtered, and the cake was washed twice with MTBE. The solid was dried *in vacuo* to give a white solid (450 mg).

### Synthesis of compound 55

To a solution of **55-3** (450 mg, 0.33 mmol), **2-SM** (525 mg, 0.57 mmol), HOBt (89 mg, 0.66 mmol), and PyBOP (342 mg, 0.66 mmol) in DMF (10 mL) was added NMM (150 mg, 1.48 mmol). The mixture was stirred at room temperature for 24 h. To MTBE (100 mL) was added the reaction mixture slowly dropwise, and a large amount of solid was precipitated. The solid was collected and dissolved in methanol (40 mL) and water (40 mL), and the mixture was purified by ultrafiltration. The ultrafiltrate was lyophilized to give a white solid (520 mg, 99.3% purity by HPLC, 16.8% SN38 conjugation content).

### Example 93: Synthesis of compound 56

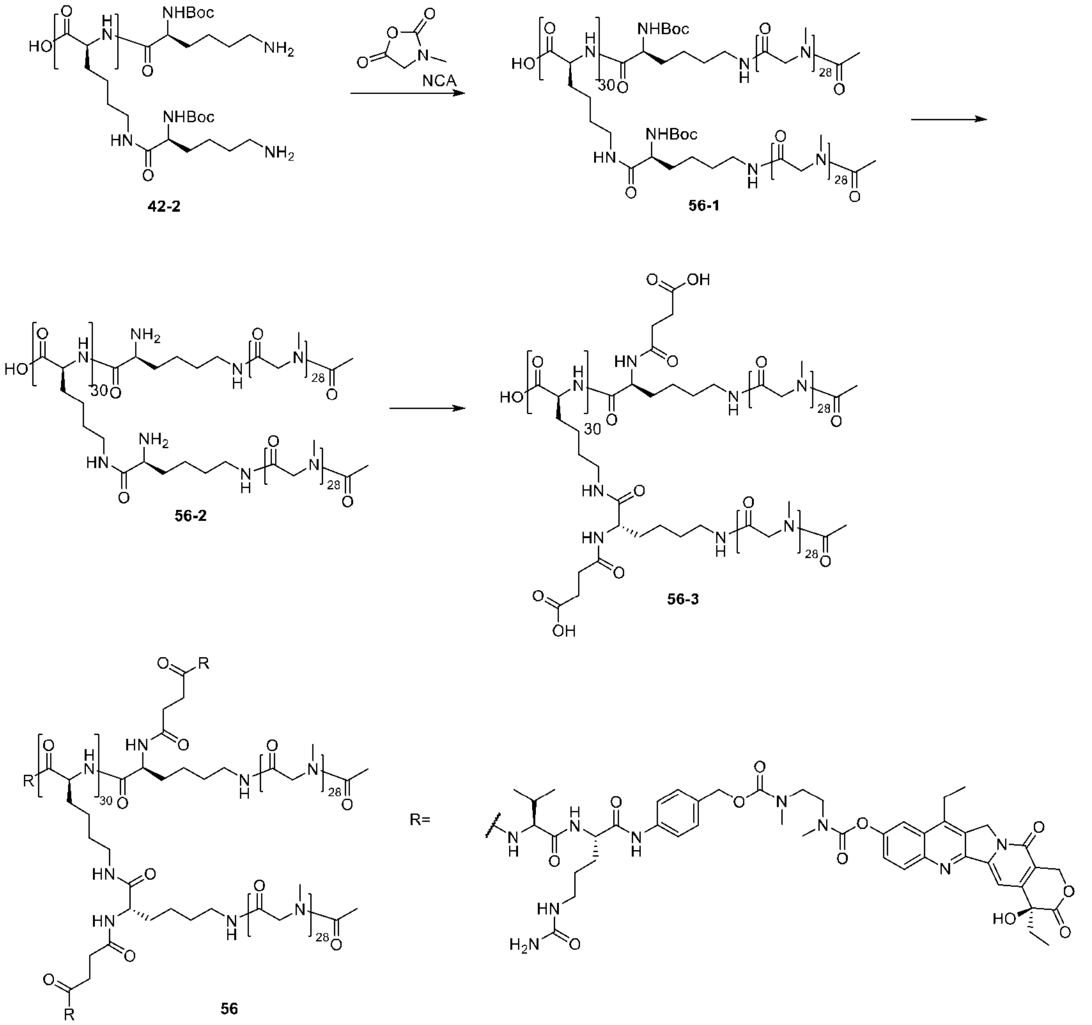


42-2 56-1

56-2 56-3

56 R=

### Synthesis of compound 56-1

To a mixture **42-2** (500 mg, 1.3 mmol), NCA (4.3 g, 37.4 mmol), and benzoic acid (815 mg, 6.7 mmol) in DCM (20 mL) was added DIPEA (430 mg, 3.4 mmol). The mixture was stirred at room temperature for 2 h, and acetic anhydride (272 mg, 2.7 mmol) and DIPEA (860 mg, 6.7 mmol) were added. The mixture was purged with nitrogen thrice, stirred at room temperature for 16 h under nitrogen atmosphere, and added slowly dropwise to MTBE (100 mL). A solid was precipitated. The mixture was filtered and dried *in vacuo* to give a white solid (1.1 g, a degree of polymerization of 28, and >99% purity by GPC).

### Synthesis of compound 56-2

A mixture of **56-1** (1.1 g), TFA (3 mL), and DCM (8 mL) was stirred at room temperature for 12 h. To MTBE (90 mL)

was added the reaction mixture slowly for precipitation, and a large amount of solid was precipitated. The mixture was filtered and dried *in vacuo* to give a white solid (0.9 g).

**Synthesis of compound 56-3**

To a solution of **56-2** (0.9 g, 0.4 mmol) in DMF (8 mL) was added succinic anhydride (80 mg, 0.8 mmol), and DIPEA (650 mg, 5.0 mmol) was added. The mixture was stirred at room temperature for 12 h. To MTBE (100 mL) was added the reaction mixture slowly dropwise, and a solid was precipitated. The mixture was filtered, and the solid cake was dried *in vacuo* to give a white solid (750 mg).

**Synthesis of compound 56**

To a solution of **56-3** (700 mg, 0.3 mmol), **2-SM** (500 mg, 0.5 mmol), HOBt (80 mg, 0.6 mmol), and PyBOP (310 mg, 6 mmol) in DMF (8 mL) was added NMM (150 mg, 1.48 mmol). The mixture was stirred at room temperature for 16 h. To MTBE (70 mL) was added the reaction mixture slowly dropwise, and a solid was precipitated. The solid was collected and dissolved in methanol (40 mL) and water (40 mL), and the mixture was purified by ultrafiltration. The ultrafiltrate was lyophilized to give a white solid (590 mg, 98.9% purity by HPLC, 10.6% SN38 conjugation content).

Example 94: Synthesis of compound 57

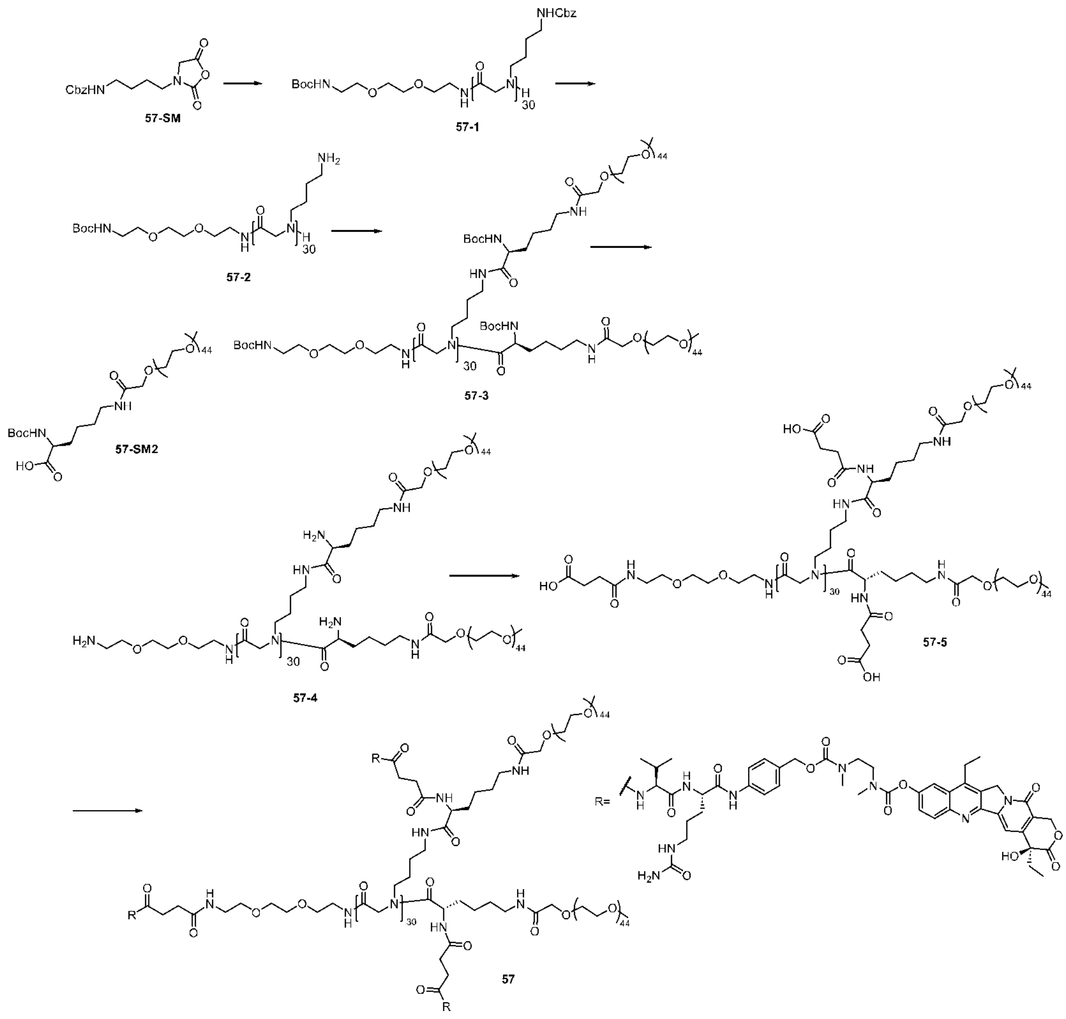

57-SM

57-1

57-2

57-3

57-SM2

57-4

57-5

57

**Synthesis of compound 57-1**

*tert*-Butyl 2-(2-(2-aminoethoxy)ethoxy)ethylcarbamate was dissolved in DMF to prepare a 50 mg/mL solution, and tert-butyric acid was dissolved in the above solution to prepare a 114.8 mg/mL solution. To a solution of 2.0 g of **57-SM** (synthesized according to the method in J. Am. Chem. Soc. 2021, 143, 10, 3697-3702) in DCM (15 mL) was added 1 mL of the above DMF solution. The mixture was stirred at room temperature, and the reaction was monitored by on-line GPC. When the degree of polymerization was about 30, MTBE was added, and the mixture was centrifuged and dried to give the compound (1.5 g).

**Synthesis of compound 57-2**

To a mixed solution of **57-1** (1.5 g) in acetic acid (10 mL) and methanol (10 mL) was added 10% Pd/C (0.2 g). The mixture was purged with hydrogen, heated to 50 °C, stirred for 12 h, concentrated, and added to MTBE for precipitation. The mixture was filtered and dried to give the compound (1.0 g). NMR indicated the absence of aromatic peaks, suggesting the completion of the reaction.

**Synthesis of compound 57-3**

To a solution of **57-2** (100 mg, 0.02 mmol, 1.0 eq) in DMF (10 mL) were added **57-SM2** (2.48 g, 1.10 mmol, 45.0 eq; synthesized according to the method in the examples of Patent No. WO2020102852A1), PyBOP (570 mg, 1.10 mmol, 45.0 eq), and DIPEA (280 mg, 2.20 mmol, 90.0 eq). The reaction mixture was stirred overnight at room temperature, concentrated, dissolved in methanol and water, and purified by ultrafiltration into a methanol and water system (30K MW). The concentrate was collected, concentrated, and lyophilized to give a product (1.2 g).

**Synthesis of compound 57-4**

To a solution of **57-3** (1.0 g) in 10 mL of dichloromethane was added 10 mL of TFA. The mixture was stirred overnight, directly concentrated, dissolved in dichloromethane, and re-concentrated. The procedures were repeated thrice to give a crude mixture (1.0 g), which was directly used in the next step.

**Synthesis of compound 57-5**

To a solution of the crude product of **57-4** (1.0 g) in 10 mL of DMF were added succinic anhydride (100 mg) and DIPEA (0.2 mL). A pH indicator paper indicated that the solution was alkaline. The solution was stirred overnight at room temperature, concentrated, dissolved in methanol and water, and purified by ultrafiltration into a methanol and water system (30K MW). The concentrate was collected and lyophilized to give a product (0.8 g).

**Synthesis of compound 57**

To a solution of **57-5** (800 mg, 0.01 mmol, 1.0 eq) in DMF (10 mL) were added **2-SM** (0.5 g, 0.49 mmol, 45.0 eq), PyBOP (256 mg, 0.49 mmol, 45.0 eq), and DIPEA (180 mg, 1.40 mmol, 128.0 eq). The reaction mixture was stirred overnight at room temperature, concentrated, dissolved in methanol and water, and purified by ultrafiltration into a methanol and water system (30K MW). The concentrate was collected, concentrated, and lyophilized to give a product (0.9 g, 99% purity, 11.2% SN38 conjugation content).

Examples 95 to 118: Synthesis of compounds 58 to 81

The syntheses were conducted according to the synthetic methods in Examples **79-80** with different polymer backbones:

| No. | Compound | Purity and drug content |
|---|---|---|
| Example **95**<br><br>Compound **58** | 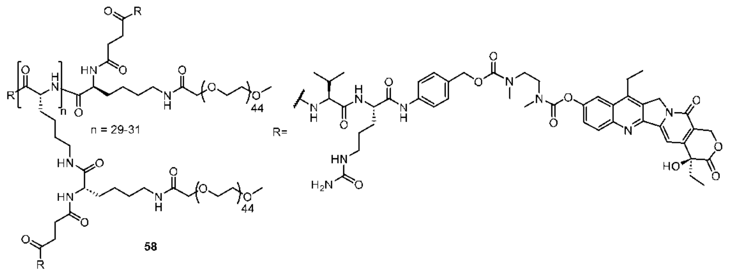<br>**58** | 99.2%<br><br>11.2% |
| Example **96**<br><br>Compound **59** | 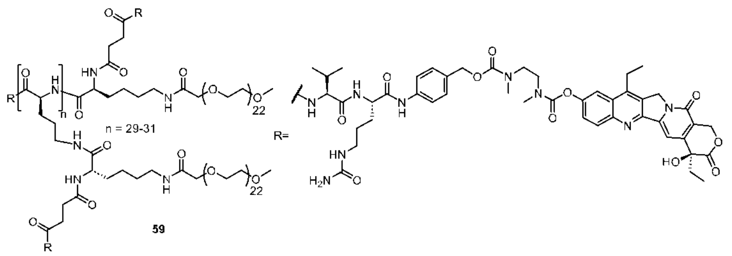<br>**59** | 97.2%<br><br>15.4% |
| Example **97**<br><br>Compound **60** | 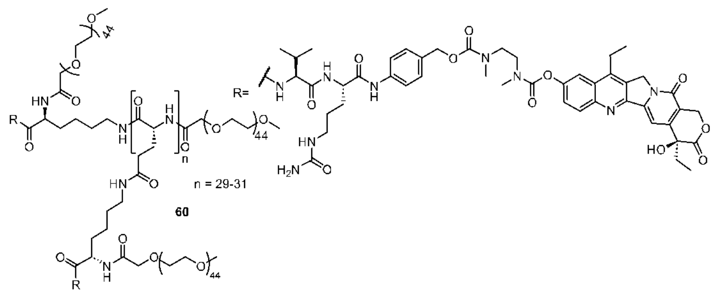<br>**60** | 98.0%<br><br>8.7% |
| Example **98**<br><br>Compound **61** | 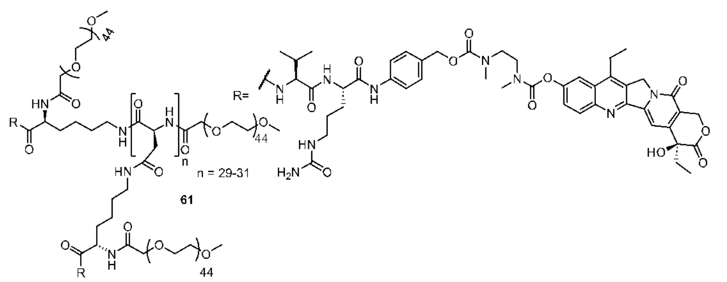<br>**61** | 99.3%<br><br>9.7% |
| Example 99<br><br>Compound 62 | 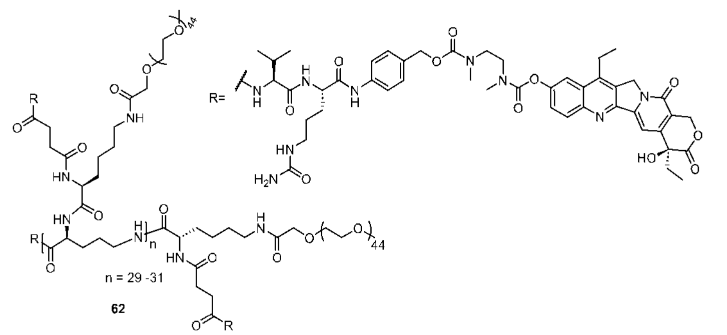<br>**62** | 98.1%<br><br>7.9% |

(continued)

| No. | Compound | Purity and drug content |
|---|---|---|
| Example **100**<br>Compound **63** | 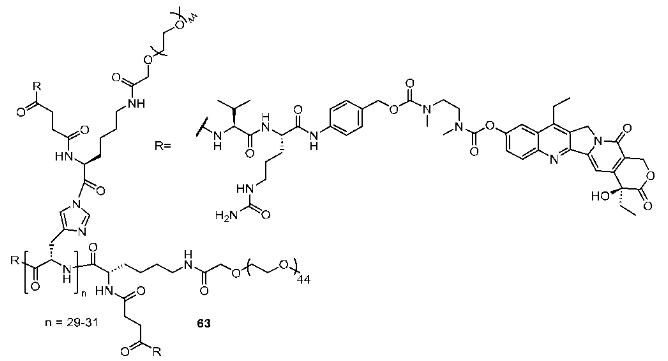 63 | 97.8%<br>8.5% |
| Example **101**<br>Compound **64** | 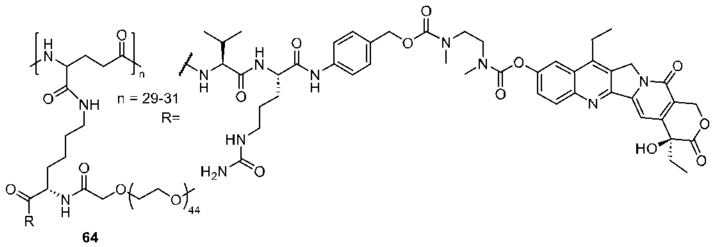 64 | 95.8%<br>10.2% |
| Example **102**<br>Compound **65** | 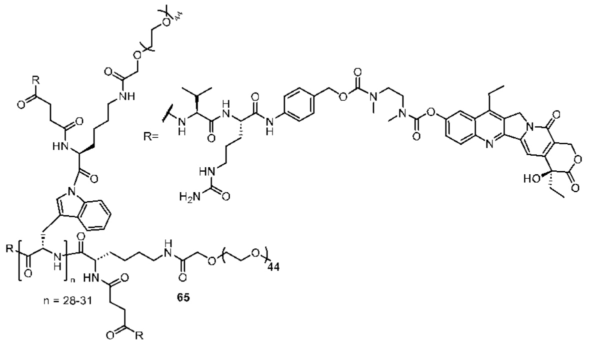 65 | 98.8%<br>11.2% |
| Example **103**<br>Compound **66** | 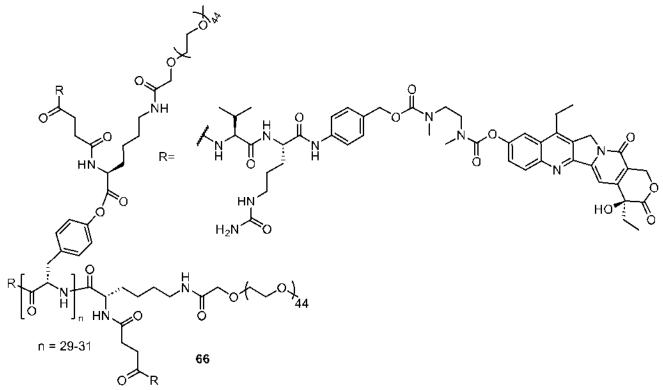 66 | 96.9%<br>7.8% |
| Example **104**<br>Compound **67** | 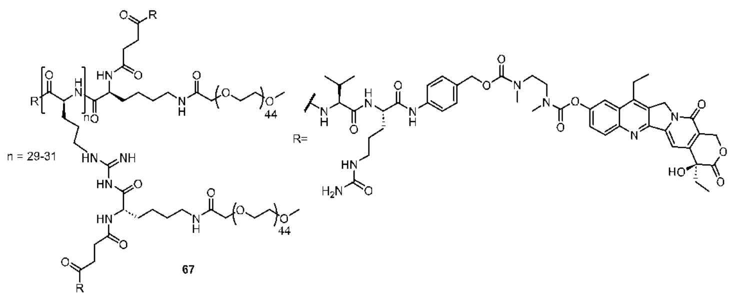 67 | 99.9%<br>9.6% |

(continued)

| No. | Compound | Purity and drug content |
|---|---|---|
| Example **105**<br><br>Compound **68** | 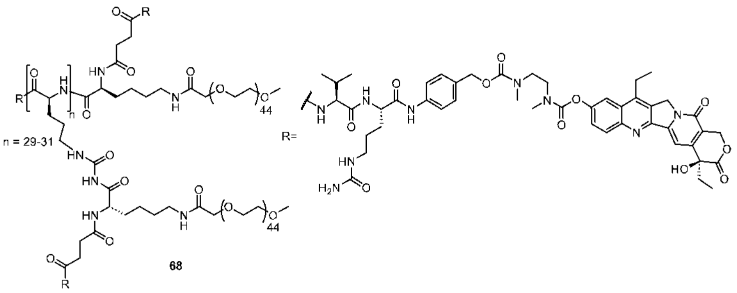 | 98.7%<br><br>10.9% |
| Example **106**<br><br>Compound **69** | 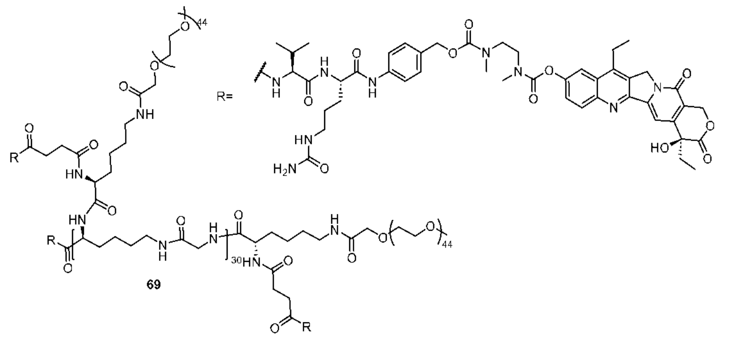 | 97.3%<br><br>6.9% |
| Example **107**<br><br>Compound **70** | 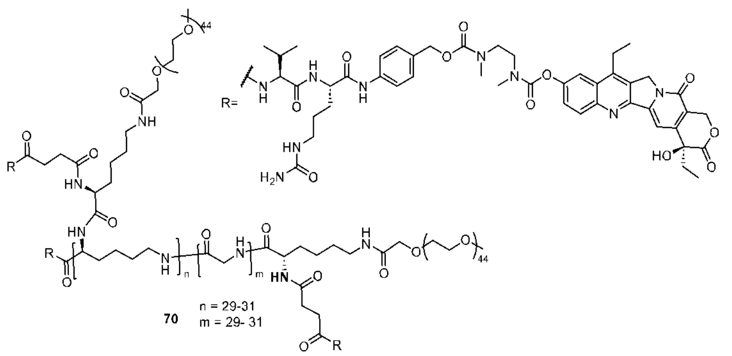 | 99.3%<br><br>8.8% |
| Example **108**<br><br>Compound **71** | 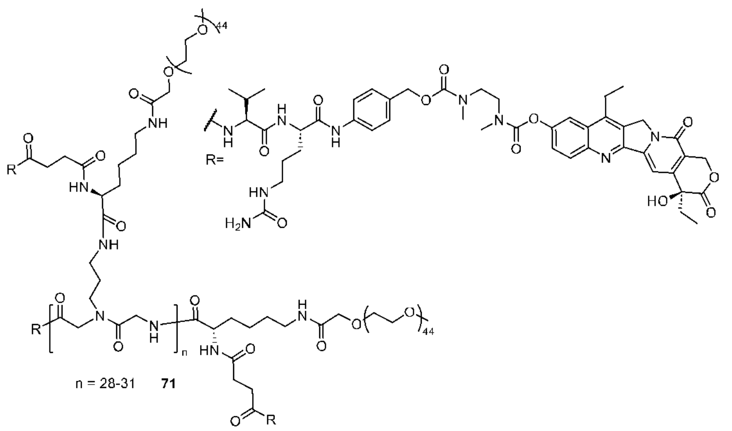 | 97.7%<br><br>9.7% |

(continued)

| No. | Compound | Purity and drug content |
|---|---|---|
| Example **109**<br><br>Compound **72** | 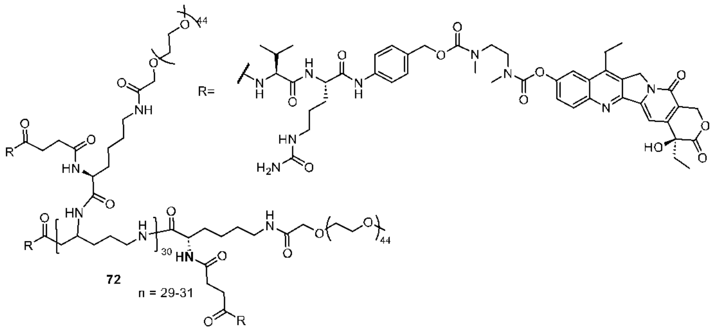<br>**72**<br>n = 29-31 | 96.7%<br><br>9.2% |
| Example 110<br><br>Compound **73** | 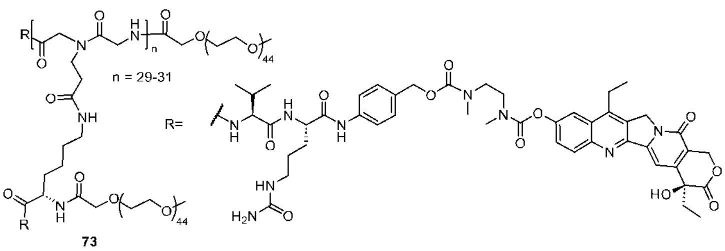<br>n = 29-31<br>**73** | 94.7%<br><br>10.9% |
| Example **111**<br><br>Compound **74** | 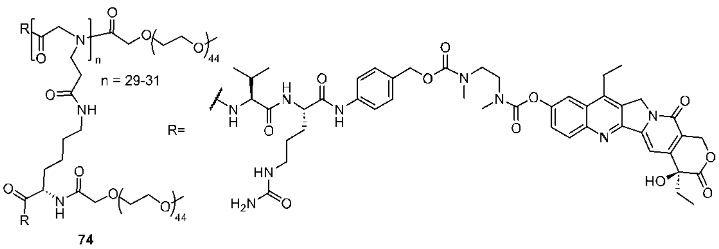<br>n = 29-31<br>**74** | 97.6%<br><br>11.9% |
| Example **112**<br><br>Compound **75** | 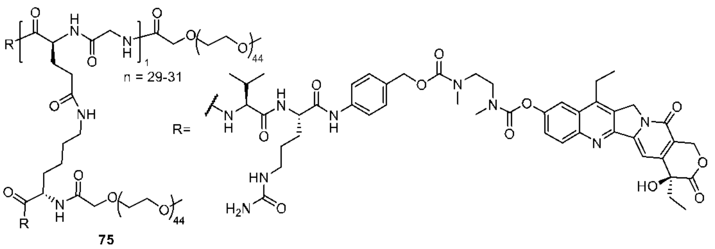<br>n = 29-31<br>**75** | 96.6%<br><br>9.1% |
| Example **113**<br><br>Compound **76** | 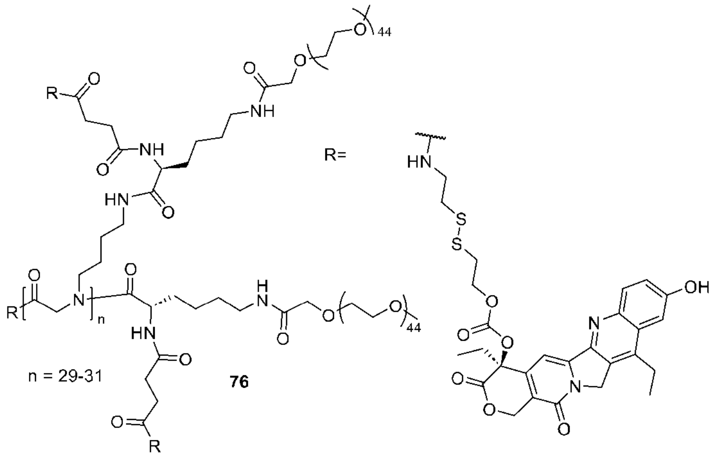<br>n = 29-31<br>**76** | 93.6%<br><br>9.1% |

(continued)

| No. | Compound | Purity and drug content |
|---|---|---|
| Example **114**<br><br>Compound **77** | 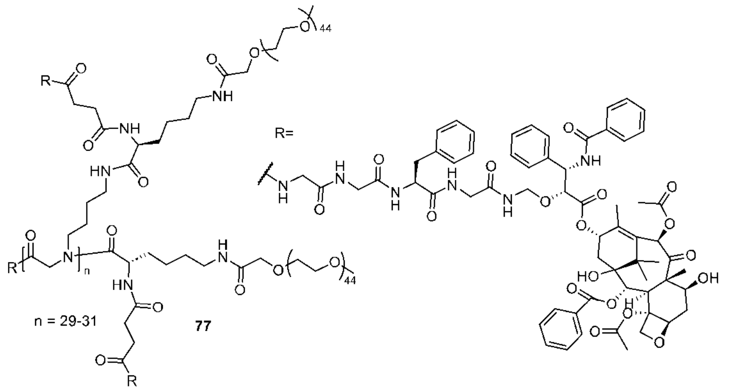<br>77 | 95.6%<br><br>19.3% |
| Example **115**<br><br>Compound **78** | 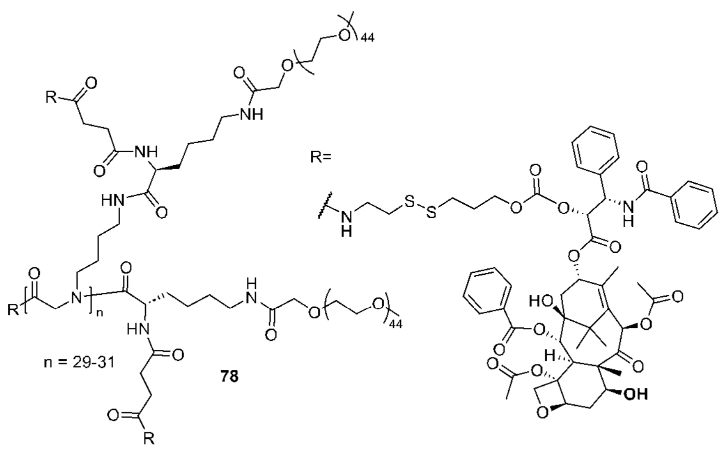<br>78 | 99.6%<br><br>21.0% |
| Example **116**<br><br>Compound **79** | 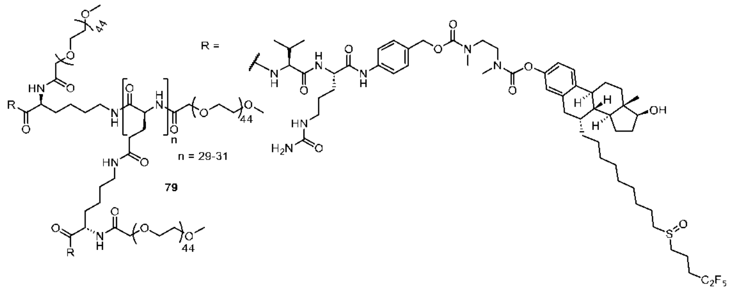<br>79 | 99.4%<br><br>12.5% |
| Example **117**<br><br>Compound **80** | 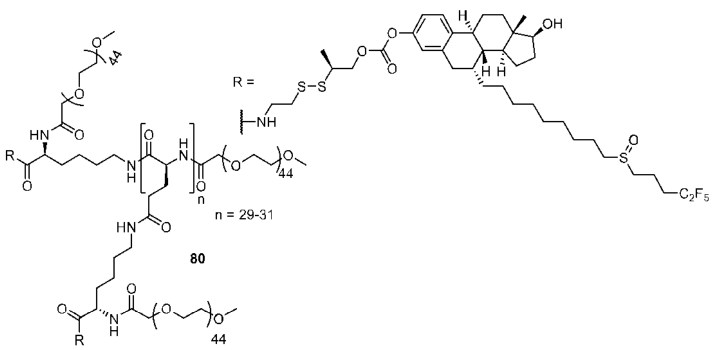<br>80 | 98.9%<br><br>15.7% |

(continued)

| No. | Compound | Purity and drug content |
|---|---|---|
| Example **118**<br><br>Compound **81** | 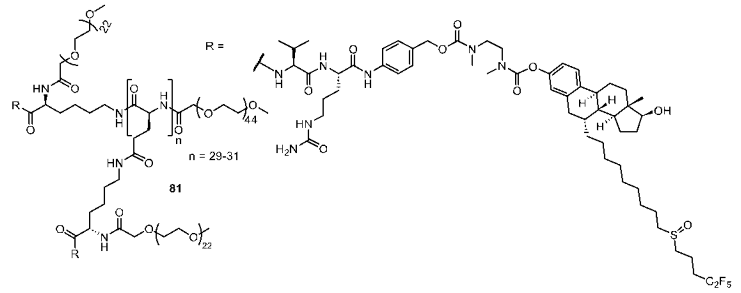 | 99.7%<br><br>21.3% |

Example 119: Synthesis of compound 82

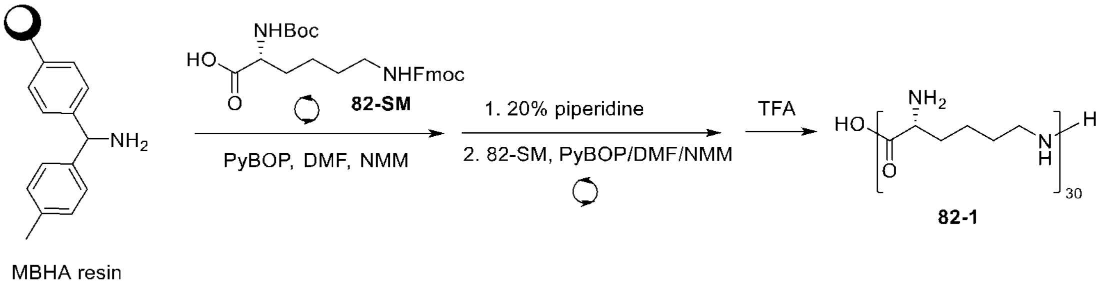

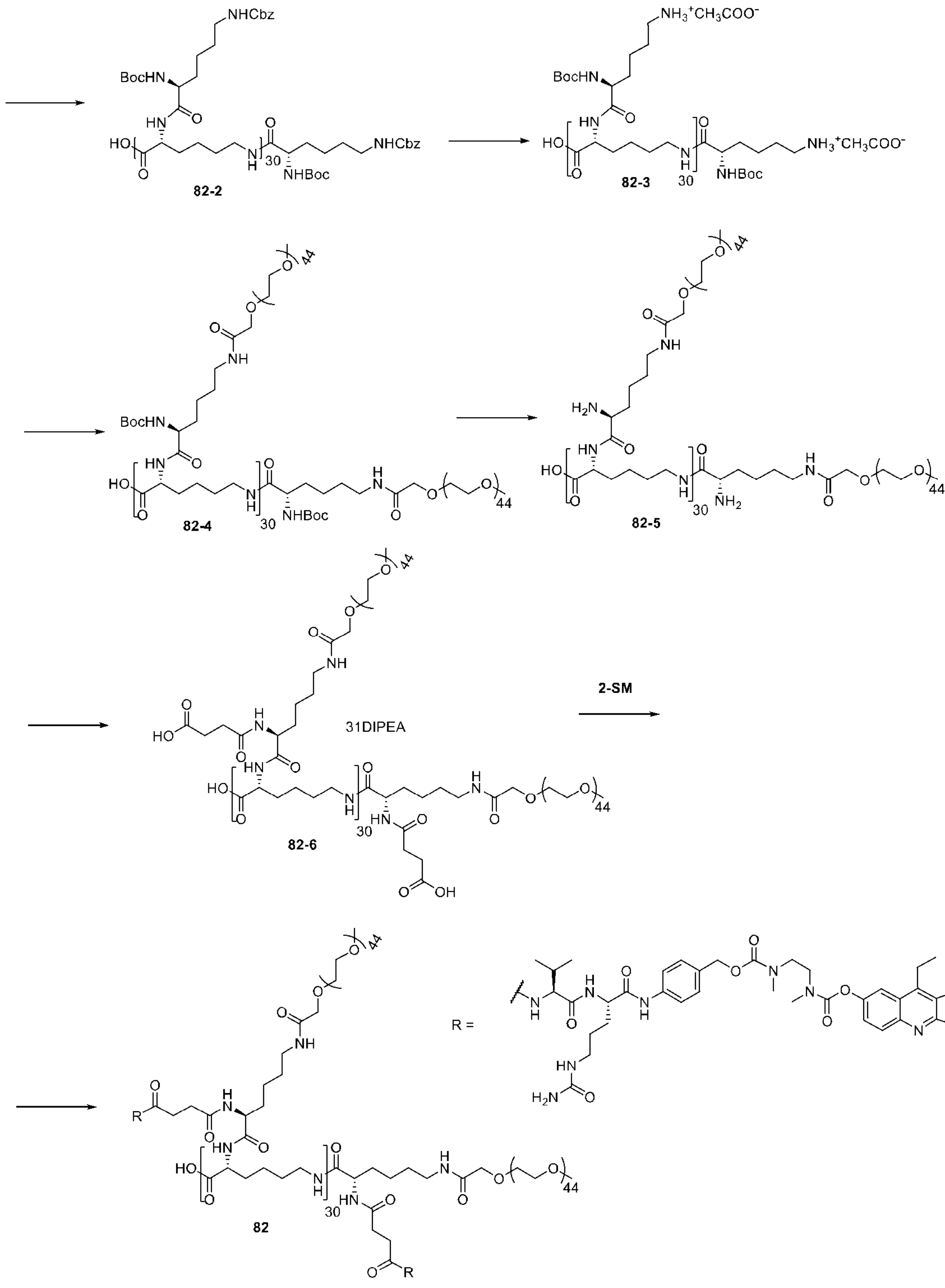


82-2 82-3 82-4 82-5 82-6 82

R =

Synthesis of compound **82-2**

According to the synthetic method in Example 1, the synthesis was conducted by Fmoc solid-phase synthesis using starting material **82-1** and MBHA resin (25 g, Loading = 0.65 mmol/g) as the resin for solid-phase synthesis. After the Fmoc protecting group removal, the solid-phase synthesis was conducted. The starting material was Boc-D-Lys(Fmoc)-OH (1.5 eq), the coupling reagents were PyBOP (1.5 eq) and NMM (1.5 eq), the solvent was DMF, the Fmoc removal reagent was 20% piperidine/DMF solution, and the resin removal reagent was TFA/water/triisopropylsilane 95/2.5/2.5.

To a suspension of **82-1** (4.8 g, 29.1 mmol, molar quantity of structural unit) in DMSO (100 g) were added triethylamine (8.9 g, 87.3 mmol) and Boc-Lys(Z)-ONP (23.0 g, 46.6 mmol) sequentially. The mixture was stirred at 30 °C for 13 h under nitrogen atmosphere until the reaction was completed. The reaction mixture was transferred into a beaker, and

ACN (800 mL) was added. The mixture was filtered *in vacuo,* and the cake was washed with acetonitrile, water, and acetonitrile sequentially and dried *in vacuo* to give product **82-2** (11.5 g, white solid).

Synthesis of compound **82-3**

**82-2** (5.0 g) was dissolved in acetic acid (50 mL) and methanol (50 mL) while heating, and palladium on carbon (1.2 g, 10%) was added. The mixture was purged with hydrogen and stirred at 40 °C for 24 h. Celite was added, and the mixture was filtered *in vacuo.* The filtrate was concentrated by rotavap, and methyl *tert*-butyl ether was added for precipitation. The mixture was dried *in vacuo* to give a product **82-3** (white powder).

Synthesis of compound **82-4**

To an aqueous solution (20 mL) of **82-3** (4.00 g) were added a solution of PEG-2K-NHS (6.8 g) in acetonitrile (25 mL) and 2.2 mL of DIPEA. The mixture was stirred overnight at room temperature, concentrated by rotavap to remove the solvent, extracted with dichloromethane, dried over anhydrous sodium sulfate, and concentrated by rotavap to give a product **82-4** (9.8 g).

Synthesis of compound **82-5**

To a solution of **82-4** (8.0 g) in 50 mL of dichloromethane was added 22 mL of TFA. The mixture was stirred overnight at room temperature under nitrogen atmosphere and concentrated *in vacuo* to give compound **82-5** (6.8 g).

Synthesis of compound **82-6**

To a solution of **82-5** (6.2 g) in DMF (21 mL) were added DIPEA (4.0 mL) and succinic anhydride (1.3 g). The mixture was stirred at room temperature for 12 h. To methyl tert-butyl ether was added the product for precipitation. The mixture was filtered *in vacuo,* and the cake was dried *in vacuo* to give a product **82-6** (6.0 g).

Synthesis of compound **82**

To a solution of **82-6** (500 mg, 0.01 mmol, 1.0 eq) and **2-SM** (280 mg, 0.31 mmol, 45.0 eq) in DMF (10 mL) were added DIPEA (113 mg, 0.87 mmol, 128.0 eq) and PyBOP (228 mg, 0.44 mmol, 64.0 eq) separately at room temperature (25 °C) under nitrogen atmosphere. The mixture was stirred at room temperature for 12 h. The reaction mixture was concentrated, and methyl tert-butyl ether was added. The mixture was stirred for 10 min, and the supernatant was discarded. The residue was purified by ultrafiltration into a methanol and water system (30K MW). The concentrate was collected, concentrated, and lyophilized to give compound **82** (white solid, 0.6 g, 86% yield, 99% purity by HPLC, 11.8% SN38 conjugation content).

Example 120: Synthesis of compound 83

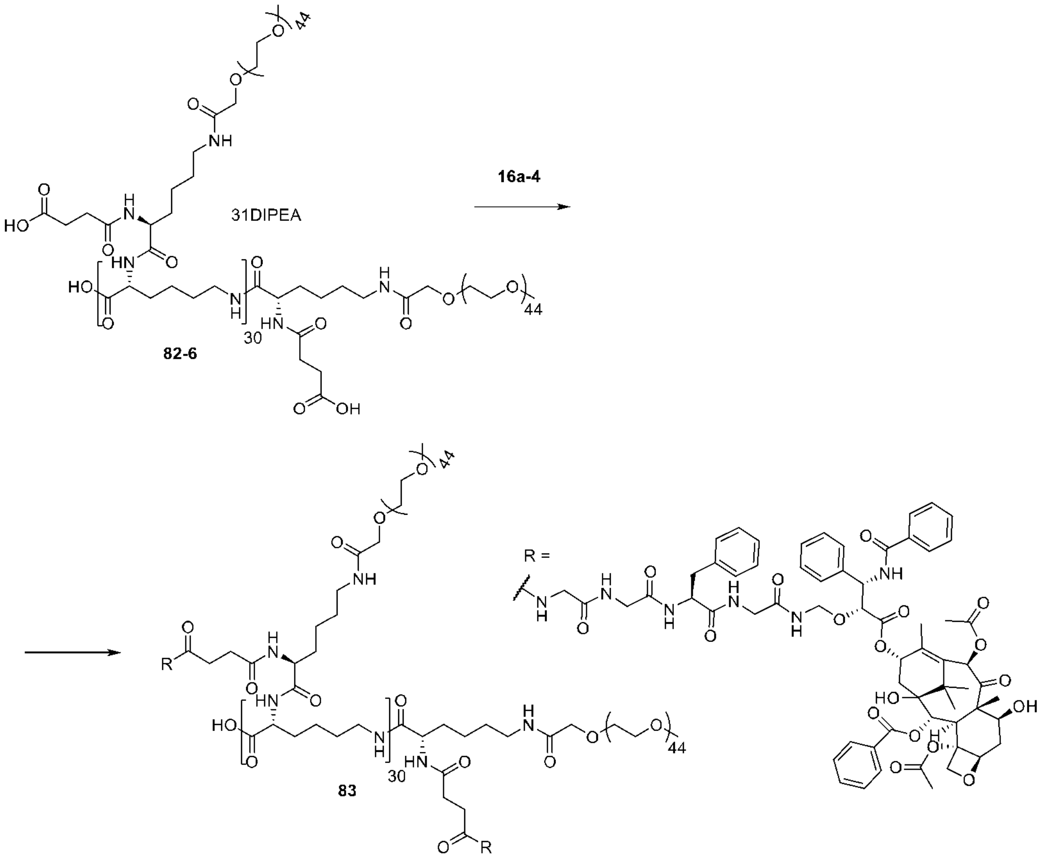


Synthesis of compound **83**

To a solution of **82-6** (250 mg, 0.003 mmol, 1.0 eq) in dry DMF (5 mL) were added a solution of **16a-4** (196 mg, 0.16 mmol, 48.0 eq) in dry DMF (2 mL, ultrasonicated for dissolution), PyBOP (142 mg, 0.27 mmol, 80.0 eq), and DIPEA (56 mg, 0.44 mmol, 128.0 eq) at room temperature (25 °C) under nitrogen atmosphere. The mixture was then heated to 28 °C, stirred for 12 h, concentrated, filtered from methanol to methanol/water (1/1, 100 mL), purified by ultrafiltration (30K MW), and lyophilized to give a product **83** (360 mg, 99% purity, about 23.6% PTX conjugation content).

Example 121: Synthesis of compound 84

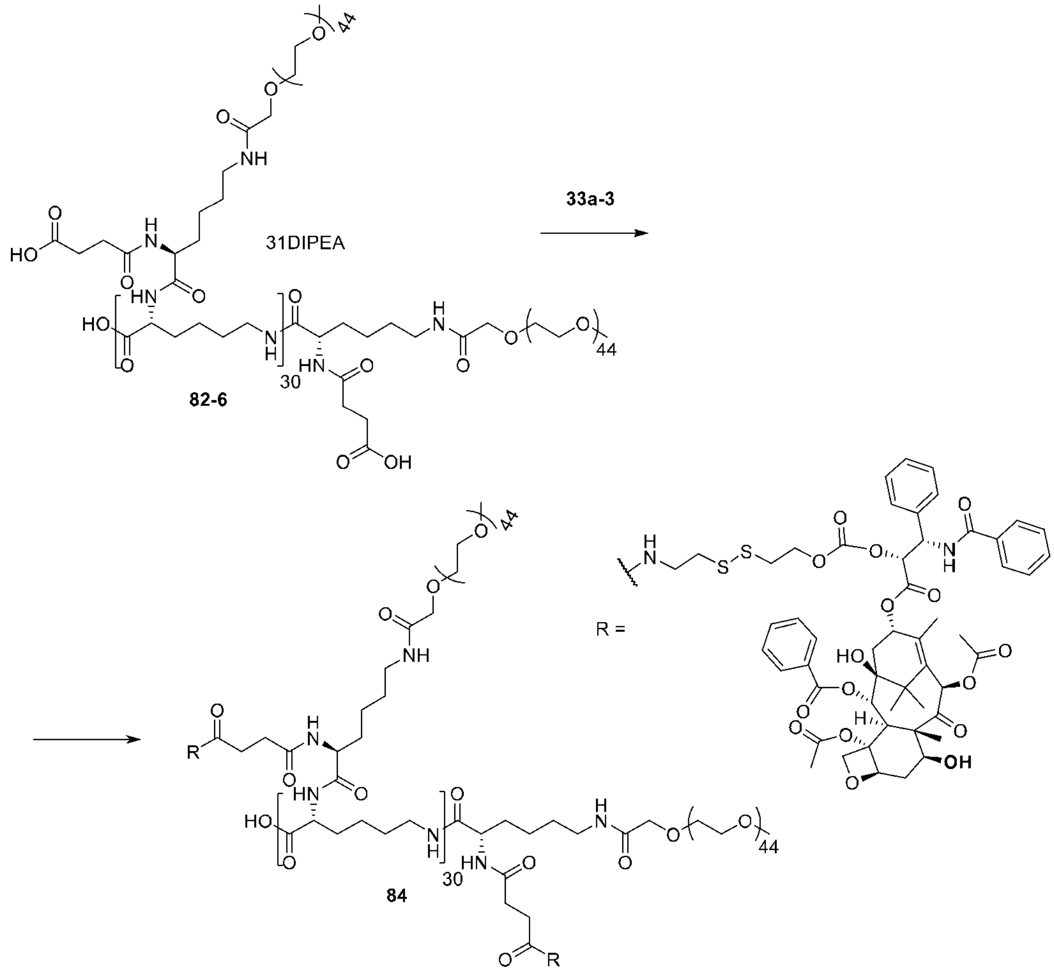


82-6

84

Synthesis of compound **84**

To a solution of **82-6** (1220 mg, 0.516 mmol, 1.0 eq, based on the polymer unit) in DMF (20 mL) were added **33a-3** (800 mg, 0.774 mmol, 1.5 eq), PyBOP (403 mg, 0.774 mmol, 1.5 eq), and DIPEA (266 mg, 2.06 mmol, 4 eq) at room temperature under nitrogen atmosphere. The mixture was stirred at 20 °C for 12 h and concentrated, and 50 mL of MTBE was added. The mixture was stirred for 10 min, and the supernatant was discarded. The residue was dissolved in methanol and water, ultrafiltered (30K MW), concentrated, and lyophilized to give compound 84 (white solid, 1.4 g, 98.6% purity, 24.7% paclitaxel content).

**Example 122: Synthesis of compound 85**

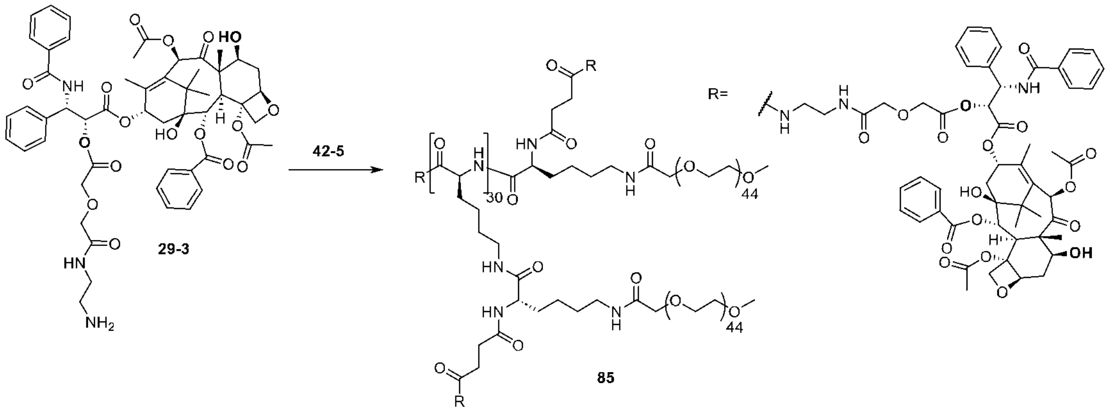

29-3
85

Synthesis of compound **85**

To a solution of **42-5** (778 mg, 0.33 mmol) and **29-3** (580 mg, 0.57 mmol) in DMF (8 mL) were added HOBt (89 mg, 0.66 mmol), PyBOP (342 mg, 0.66 mmol) and NMM (150 mg, 1.48 mmol) at room temperature under nitrogen atmosphere, and the mixture was stirred for 12 h at room temperature. MTBE (50 mL) was added, and the mixture was stirred for 30 min. The supernatant was discarded, and the residue was dried and dissolved in methanol (40 mL) and water (40 mL). The mixture was purified by ultrafiltration (30K MW, in a methanol/water ultrafiltrate). The ultrafiltrate was lyophilized to give a pale yellow solid (520 mg, 99.7% purity by HPLC, 21.3% PTX conjugation content).

**Example 123: Synthesis of compound 86**

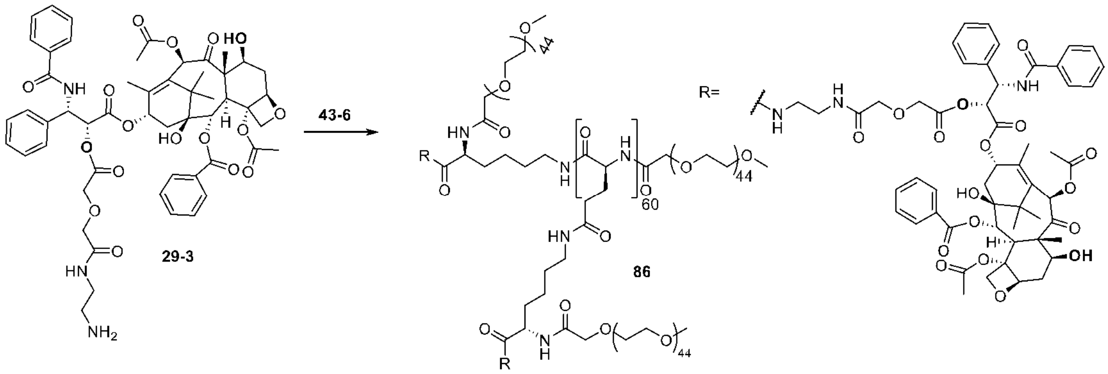

29-3
86

Synthesis of compound **86**

To a solution of **43-6** (700 mg, 0.32 mmol) and **29-3** (490 mg, 0.48 mmol) in DMF (10 mL) were added PyBOP (312 mg, 0.60 mmol) and DIPEA (190 mg, 1.48 mmol) at room temperature under nitrogen atmosphere, and the mixture was stirred for 24 h. MTBE (50 mL) was added, and the mixture was stirred for 30 min. The supernatant was discarded, and the residue was dried and dissolved in methanol (60 mL) and water (40 mL). The mixture was purified by ultrafiltration (30K MW, in a methanol/water ultrafiltrate). The ultrafiltrate was lyophilized to give a white solid (690 mg, 98.8% purity by HPLC, 22.6% PTX conjugation content).

**Example 124: Synthesis of compound 87**

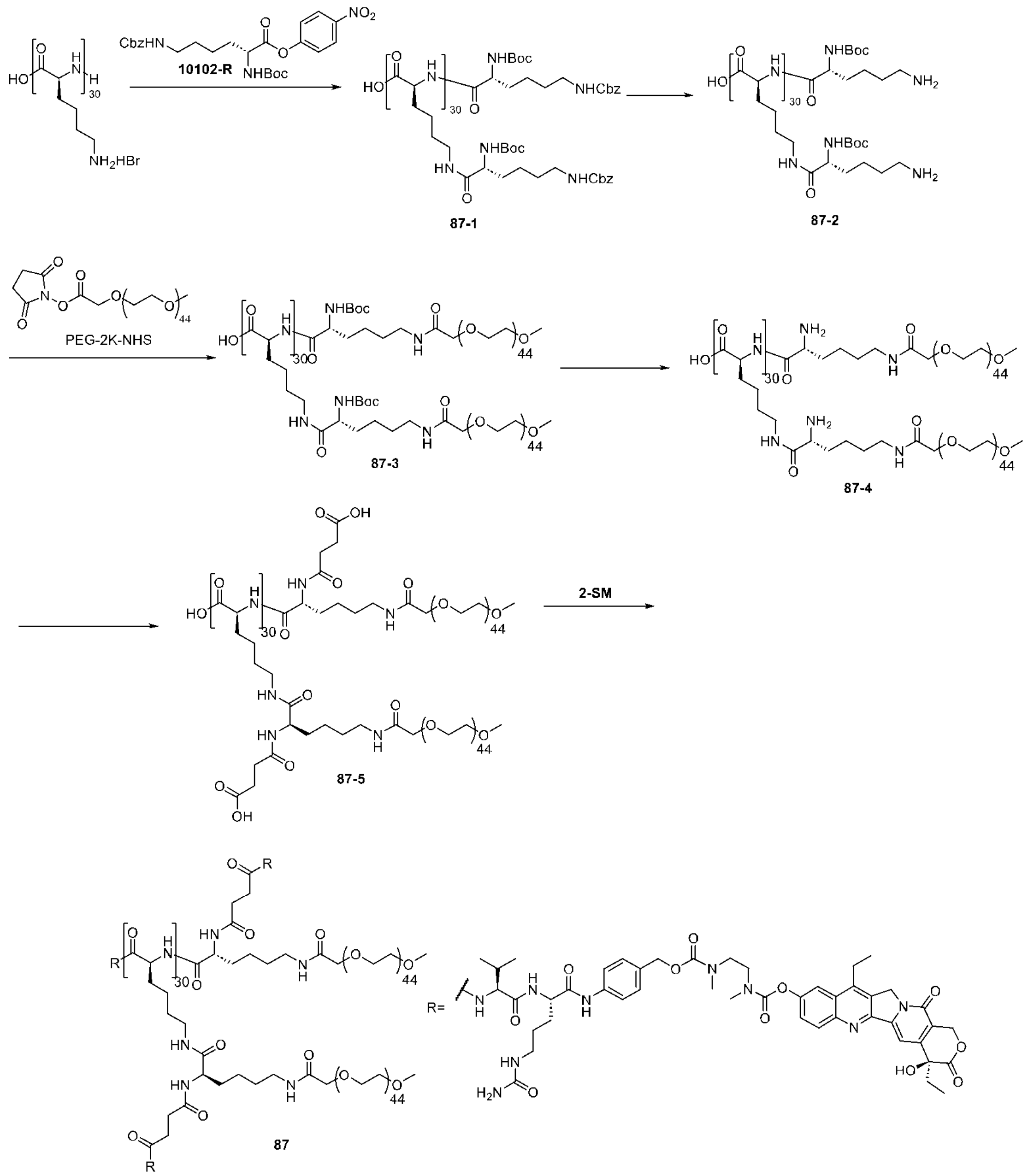


87-1 87-2 87-3 87-4 87-5 87

**Synthesis of compound 87-1**

To a solution of poly-L-lysine hydrobromide (2 g, 9.56 mmol) in water (3 mL) were added DIPEA (3.7 g, 28.7 mmol) and DMSO (40 mL) under nitrogen atmosphere. 10102-R (synthesized according to the method for 10102 from D-amino acid starting materials; 7.2 g, 14.35 mmol) was added, and the mixture was stirred for 24 h at room temperature. Acetonitrile (100 mL) was added for precipitation, and the mixture was filtered. The cake was collected to give a white solid (5.2 g).

**Synthesis of compound** 87-2

A mixture of 87-1 (5.2 g, 11.0 mmol) and acetic acid (55 mL) was dissolved by heating, and methanol (55 mL) and 10% palladium on carbon (927 mg) were added. The mixture was purged with hydrogen thrice, stirred for 16 h, and filtered through celite, and the filtrate was triturated with MTBE and filtered. The solid was dried *in vacuo* to give a white solid (1.6 g).

**Synthesis of compound 87-3**

To a solution of **87-2** (900 mg, 0.16 mmol) in methanol (25 mL) was added a solution of PEG-2K-NHS (5.6 g, 2.64 mmol) in acetonitrile (25 mL), and DIPEA (1.4 g, 10.8 mmol) was added. The mixture was stirred at room temperature for 30 min and purified by ultrafiltration, and the ultrafiltrate was lyophilized to give a white solid (4.6 g, 99% purity by GPC).

**Synthesis of compound 87-4**

A mixture of **87-3** (1.0 g, 0.42 mmol), TFA (3 mL), and DCM (7 mL) was stirred at room temperature for 16 h. To MTBE (90 mL) was added the reaction mixture slowly for precipitation, and a large amount of solid was precipitated. The mixture was filtered and dried *in vacuo* to give a white solid (1.05 g).

**Synthesis of compound 87-5**

To a solution of **87-4** (1 g, 0.42 mmol) in DMF (8 mL) was added succinic anhydride (63 mg, 0.63 mmol), and DIPEA (326 mg, 2.52 mmol) was added. The mixture was stirred at room temperature for 16 h. To MTBE (90 mL) was added the reaction mixture slowly dropwise, and a large amount of solid was precipitated. The mixture was filtered, and the cake was washed twice with MTBE. The solid was dried *in vacuo* to give a white solid (810 mg).

**Synthesis of compound 87**

To a solution of **87-5** (770 mg, 0.32 mmol), **2-SM** (525 mg, 0.57 mmol), HOBt (89 mg, 0.66 mmol), and PyBOP (342 mg, 0.66 mmol) in DMF (8 mL) was added NMM (150 mg, 1.48 mmol). The mixture was stirred at room temperature for 16 h. To MTBE (80 mL) was added the reaction mixture slowly dropwise, and a large amount of solid was precipitated. The solid was collected and dissolved in methanol (40 mL) and water (40 mL), and the mixture was purified by ultrafiltration. The ultrafiltrate was lyophilized to give a white solid (832 mg, 99.2% purity by HPLC, 12.21% SN38 conjugation content).

**Example 125: Synthesis of compound 88**

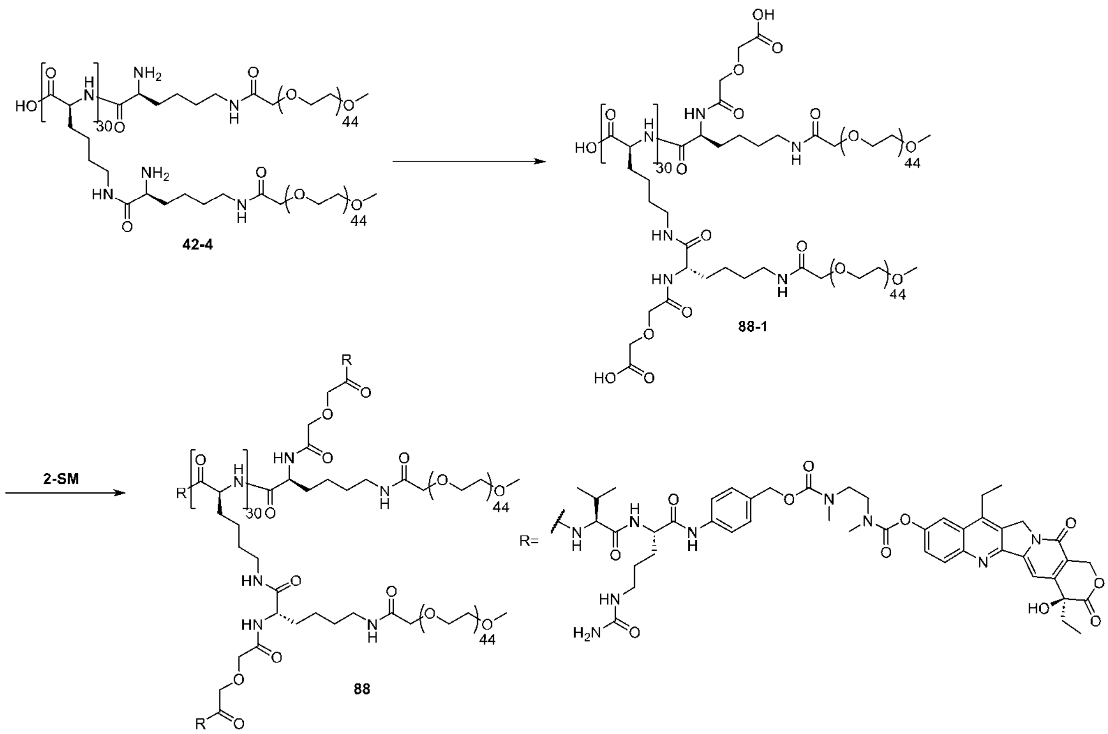


42-4

88-1

88

**Synthesis of compound 88-1**

To a solution of **42-4** (1 g, 0.42 mmol) in DMF (8 mL) was added diglycolic anhydride (73 mg, 0.63 mmol), and DIPEA (326 mg, 2.52 mmol) was added. The mixture was stirred at room temperature for 24 h. To MTBE (90 mL) was added the reaction mixture slowly dropwise, and a large amount of solid was precipitated. The mixture was filtered, and the cake was washed twice with MTBE. The solid was dried *in vacuo* to give a white solid (823 mg).

**Synthesis of compound 88**

To a solution of **87-5** (760 mg, 0.32 mmol), **2-SM** (525 mg, 0.57 mmol), HOBt (89 mg, 0.66 mmol), and PyBOP (342 mg, 0.66 mmol) in DMF (8 mL) was added NMM (150 mg, 1.48 mmol). The mixture was stirred at room temperature for 16 h. To MTBE (80 mL) was added the reaction mixture slowly dropwise, and a large amount of solid was precipitated. The solid was collected and dissolved in methanol (40 mL) and water (40 mL), and the mixture was purified by ultrafiltration. The ultrafiltrate was lyophilized to give a white solid (795 mg, 97.9% purity by HPLC, 11.90% SN38 conjugation content).

Biological Evaluation Example 1: Nano-scale particle size study on compounds of the present disclosure

Sample preparation: A required volume of purified water or water for injection was added into a glass bottle and filtered through a 0.22 μm filter membrane. A proper amount of sample was dissolved in 0.5 mL of the above water for injection, and the solution was filtered through a 0.22 μm aqueous filter membrane, transferred into a 10 mL volumetric flask, and diluted with a diluent to the volume (final compound concentration 10 mg/mL). The flask was gently shaken to avoid bubble production, and the solution was preserved for later use.

Sample detection: The sample solution was slowly transferred to a cuvette by using a dropper for sample detection. The detection system was Zetasizer Pro Blue.

Results: The nano-scale particle size data for the compounds of the present disclosure are shown in FIGs. 1-8 and Table 1.

Table 1. Nano-scale particle size data for compounds of the present disclosure

| Example | Average particle size (nm) | Polydispersity index (PDI) |
|---|---|---|
| Example **5** | 12.36 | 0.07 |
| Example **6** | 13.93 | 0.18 |
| Example **7** | 17.63 | 0.16 |
| Example **14** | 13.58 | 0.16 |
| Example **15** | 23.10 | 0.13 |
| Example **17** | 18.10 | 0.27 |
| Example **20** | 18.59 | 0.2 |
| Example **21** | 21.31 | 0.18 |
| Example **26** | 17.32 | 0.23 |
| Example **27** | 18.73 | 0.1 |
| Example **40** | 14.71 | 0.13 |
| Example **41** | 11.6 | 0.07 |
| Example **47** | 15.4 | 0.13 |
| Example **48** | 15.27 | 0.22 |
| Example **50** | 11.87 | 0.16 |
| Example **53** | 24.10 | 0.16 |
| Example **75** | 12.99 | 0.18 |
| Example **71** | 11.6 | 0.03 |
| Example **78** (comparative) | 12.70 (multimodal) | 0.52 |

The results in Table 1 show that the compounds of the present disclosure all exhibited an average particle size in the range of 10-30 nm, a monomodal curve (see FIGs. 1-8), and a narrow PDI range, suggesting that the compounds were present in monomodal, small-nano-sized particles; in contrast, the compound of comparative Example **78** exhibited a multimodal curve, and as can be seen in FIG. **9,** the multimodal position was around 100 nm, indicating that Example **78** is prone to aggregation to generate large particles, resulting in a poor dispersity index.

Biological Evaluation Example 2: Nano-scale particle size stability study on compound of Example 71

Sample preparation: A required volume of purified water or water for injection was added into a glass bottle and filtered through a 0.22 μm filter membrane. A proper amount of sample was dissolved in 0.5 mL of the above water for injection, and the solution was filtered through a 0.22 μm aqueous filter membrane, transferred into a 10 mL volumetric flask, and diluted with a diluent to the volume (final compound concentration 10 mg/mL). The flask was gently shaken to avoid bubble production, and the solution was preserved for later use.

Sample detection: At the detection time points, samples were taken and slowly transferred to a cuvette by using a dropper for sample detection. The detection system was 0--Zetasizer Pro Blue.

Results: The particle size stability study data on the compound of Example **71** of the present disclosure are shown in Table 2 and FIGs. 10-13.

Table 2. Particle size stability study on compound of Example **71** of the present disclosure

| Temperature / Days | 2-8 °C | | 25 °C | |
|---|---|---|---|---|
| | Average particle size | PDI | Average particle size | PDI |
| Day 0 | 11.6 | 0.03 | 11.6 | 0.03 |
| Day 30 | 11.1 | 0.04 | 11.0 | 0.06 |
| Day 60 | 11.6 | 0.11 | 11.6 | 0.11 |
| Day 90 | 11.5 | 0.05 | 12.2 | 0.17 |

The results in Table 2 show that the compound of the present disclosure (compound of Example 71) exhibited a very stable particle size with no significant changes either in a refrigeration setting (2-8 °C) or at room temperature (25 °C), and remained small nano-sized particles in monomodal distribution (see FIGs. 10-13), indicating that the compound of Example **71** does not aggregate and is very stable physico-chemically during long-term preservation.

Biological Evaluation Example 3: Pharmacodynamic study on compounds of Examples 20 and 52 and Abraxane in mouse BxPC-3 model

Experimental animal and tumor grafting

BALB/c nude mice, female, aged 4-5 weeks, 18-20 g in weight, housed in the experimental environment for one week. BxPC-3 tumor mass was subcutaneously grafted on the right back of the nude mice. When the tumors reached an average volume of 150 $mm^3$, the mice were divided into groups of 6 mice each.

Test sample preparation

To a certain volume of normal saline was added a proper amount of the compound, and the mixture was ultrasonicated for dissolution and filtered through a 0.2 μm filter membrane.

Test compound administration

The dose and treatment regimen are shown in Table 3. The volume of subcutaneous tumors in the nude mice

(tumor volume was calculated by the formula: $V = 0.5a \times b^2$, and a and b denote the long and short diameters of the tumor, respectively) and the body weight of the mice were measured twice or thrice every week, and the data were recorded.

Table 3

| No. | Group | Dose (mg/kg) | Route | Number of animals | Dose cycle |
|---|---|---|---|---|---|
| 1 | Vehicle | - | iv | 6 | qwx2 |
| 2 | Example **20** | 30 | iv | 6 | qwx2 |
| 3 4 | Example **52** | 30 | iv | 6 | qwx2 |
| | Abraxane | 30 | iv | 6 | qwx2 |
| Note: The administration volume was 10 mg/mL. | | | | | |

Analysis and assessment

Experimental evaluation index: The efficacy was assessed by the tumor growth inhibition TGI (%) or the relative tumor growth rate T/C (%), wherein T denotes the treatment group and C denotes the control group.

Calculation of relative tumor growth rate T/C (%): When $T > T_0$, T/C (%) = $(T - T_0)/(C - C_0) \times 100\%$; When $T < T_0$, T/C (%) = $(T - T_0)/T_0 \times 100\%$, where T and C denote the tumor volumes at the end of study; $T_0$ and $C_0$ denote the tumor volumes at the start of study.

Calculation of tumor growth inhibition TGI (%): TGI (%) = $(1 - T/C) \times 100\%$.

Assessment criterion: T/C (%) > 40 (i.e., TGI (%) < 60%) denotes ineffective; T/C (%) ≤ 40 (i.e., TGI (%) ≥ 60%) and $P < 0.05$ denote effective.

Results of pharmacodynamic study

The inhibitory effects of compounds of Examples **20** and **52** and Abraxane in the BxPC-3 tumor model are shown in FIG. 14.

The results are shown in FIG. 14. After the compounds were administered intravenously once every week in a total of 1 dose, on day 28, the tumor inhibition rates were 90.3% and 88.56% for the compounds of Examples **20** and **52** at a dose of 30 mg/kg, respectively, which were significantly different from that of the blank control group and were significantly superior to the efficacy of the albumin-bound paclitaxel positive control group (tumor inhibition rate 44.95%), indicating that the compounds of the present disclosure may have high infiltration and excellent efficacy in refractory tumors due to highly dense matrix barrier.

Biological Evaluation Example 4: Pharmacodynamic study on compounds of Examples 71 and 78 in HepG2 model

Experimental animal and tumor grafting

BALB/c nude mice, female, aged 4-5 weeks, 20-25 g in weight, housed in the experimental environment for one week after arrival. HepG2 tumor mass was subcutaneously grafted on the right back of the nude mice. When the tumors reached ~170 $mm^3$, the mice were divided into groups of 6 mice each.

Test sample preparation

To a certain volume of normal saline was added a proper amount of the compound, and the mixture was shaken for dissolution and filtered through a 0.2 μm filter membrane.

Test compound administration

The dose and treatment regimen are shown in Table 4. The volume of subcutaneous tumors in the nude mice (tumor volume was calculated by the formula: $V = 0.5a \times b^2$, and a and b denote the long and short diameters of the tumor, respectively) and the body weight of the mice were measured twice or thrice every week, and the data were recorded.

Table 4

| No. | Group | Dose (mg/kg) | Route | Number of animals | Dose cycle |
|---|---|---|---|---|---|
| 1 | Vehicle | - | iv | 6 | qw x 1 |

(continued)

| No. | Group | Dose (mg/kg) | Route | Number of animals | Dose cycle |
|---|---|---|---|---|---|
| 2 | Example **71** | 10 | iv | 6 | qw x 1 |
| 3 | Example **78** | 10 | iv | 6 | qw x 1 |
| Note: The administration volume was 10 mg/mL. | | | | | |

Analysis and assessment

Experimental evaluation index: The efficacy was assessed by the tumor growth inhibition TGI (%) or the relative tumor growth rate T/C (%), wherein T denotes the treatment group and C denotes the control group.

Calculation of relative tumor growth rate T/C (%): When $T > T_0$, $T/C (\%) = (T - T_0)/(C - C_0) \times 100\%$; When $T < T_0$, $T/C (\%) = (T - T_0)/T_0 \times 100\%$, where T and C denote the tumor volumes at the end of study; $T_0$ and $C_0$ denote the tumor volumes at the start of study.

Calculation of tumor growth inhibition TGI (%): $TGI (\%) = (1 - T/C) \times 100\%$.

Assessment criterion: T/C (%) > 40 (i.e., TGI (%) < 60%) denotes ineffective; T/C (%) ≤ 40 (i.e., TGI (%) ≥ 60%) and $P < 0.05$ denote effective.

Results of pharmacodynamic study

The inhibitory effects of compounds of Examples **71** and **78** in the HepG2 tumor model are shown in FIG. 15.

The results are shown in FIG. 15. After the compounds were administered intravenously once every week in a total of 1 dose, on day 29, the tumor inhibition rates were 99.89% and 81.41% for the compounds of Examples **71** and **78** at a dose of 10 mg/kg, respectively, which were significantly different from that of the blank control group. The compound of Example **71** of the present disclosure exhibited very strong inhibitory effects against the tumor growth in the HepG2 cell nude mouse model where the tumors completely regressed, and the effect is significantly superior to that of comparative Example **78.** The results suggest that the stable small-nano-sized compound of the present disclosure possesses significantly superior infiltration capacity in tumor tissues and inhibitory effects against tumors to those of the unstable compound of comparative Example **78,** which is prone to aggregation into large-sized nanoparticles.

Biological Evaluation Example 5: Pharmacodynamic study on compound of Example 71 and irinotecan hydrochloride in human colon cancer HT-29 nude mouse tumor model

Experimental animal and tumor grafting

BALB/c nude mice, female, aged 4-5 weeks, 20-25 g in weight, housed in the experimental environment for one week after arrival. A 3 × 3 mm HT-29 tumor mass was subcutaneously grafted at the right scapula of the mice to establish the human colon cancer HT-29 subcutaneous xenograft model. When the tumors grew to an average volume of 900 $mm^3$, the mice were randomized into 2 groups of 6 mice each by the tumor volume.

Test sample preparation

A proper amount of the compound was diluted in a 5% glucose buffer, and the mixture was shaken for dissolution and filtered through a 0.2 μm filter membrane.

Test compound administration

The dose and treatment regimen are shown in Table 5. The volume of subcutaneous tumors in the nude mice (tumor volume was calculated by the formula: $V = 0.5a \times b^2$, and a and b denote the long and short diameters of the tumor, respectively) and the body weight of the mice were measured twice or thrice every week, and the data were recorded.

Table 5

| No. | Group | Dose (mg/kg) | Route | Number of animals | Dose cycle |
|---|---|---|---|---|---|
| 1 | Vehicle | - | iv | 6 | qwx3 |
| 2 | Example **71** | 10 | iv | 6 | qwx3 |

(continued)

| No. | Group | Dose (mg/kg) | Route | Number of animals | Dose cycle |
|---|---|---|---|---|---|
| 3 | Irinotecan hydrochloride | 10 | iv | 6 | qwx3 |
| Note: The administration volume was 10 mg/mL. | | | | | |

Analysis and assessment

Experimental evaluation index: The efficacy was assessed by the tumor growth inhibition TGI (%) or the relative tumor growth rate T/C (%), wherein T denotes the treatment group and C denotes the control group.

Calculation of relative tumor growth rate T/C (%): When $T > T_0$, T/C (%) = $(T - T_0)/(C - C_0) \times 100\%$; When $T < T_0$, T/C (%) = $(T - T_0)/T_0 \times 100\%$, where T and C denote the tumor volumes at the end of study; $T_0$ and $C_0$ denote the tumor volumes at the start of study.

Calculation of tumor growth inhibition TGI (%): TGI (%) = (1 - T/C) $\times$ 100%.

Assessment criterion: T/C (%) > 40 (i.e., TGI (%) < 60%) denotes ineffective; T/C (%) $\leq$ 40 (i.e., TGI (%) $\geq$ 60%) and $P < 0.05$ denote effective.

Results of pharmacodynamic study

The inhibitory effects of the compound of Example **71** and irinotecan hydrochloride in the human colon cancer HT-29 nude mouse tumor model are shown in FIG. 16.

The results, as shown in FIG. 16, show that after 3 doses of the compound were administered intravenously once every week, the average tumor volume on day 34 was 4000 $mm^3$ for the irinotecan hydrochloride group, whereas for the Example **71** group, the average tumor volume on day 34 was 600 $mm^3$, and on day 79, the volume was reduced to 100 $mm^3$, which was significantly superior to that of the irinotecan group. It is shown that the compound of Example **71** of the present disclosure exhibited significant inhibitory effects against the large tumors in the human colon cancer HT-29 nude mouse model, while the irinotecan hydrochloride control group exhibited no significant effects. The small nanoparticle of the compound has high infiltration capacity in large tumors and can continuously infiltrate into the deep interior of the tumor, thus possessing good therapeutic effects on late-stage tumors and large tumors.

Biological Evaluation Example 6: Pharmacodynamic study on compound of Example 71 and irinotecan liposome in human myeloma NCI-H929 nude mouse tumor model

Experimental animal and tumor grafting

BALB/c nude mice, female, aged 4-5 weeks, 19-22 g in weight, housed in the experimental environment for one week after arrival. A 3 $\times$ 3 mm NCI-H929 tumor mass was subcutaneously grafted at the right scapula of the mice to establish the human myeloma cell NCI-H929 subcutaneous xenograft model. When the tumors grew to an average volume of 1100 $mm^3$, the mice were randomized into 2 groups of 6 mice each by the tumor volume.

Test sample preparation

A proper amount of the compound was diluted in a 5% glucose buffer, and the mixture was shaken for dissolution. The samples were prepared right before use.

Test compound administration

The dose and treatment regimen are shown in Table 6. The volume of subcutaneous tumors in the nude mice (tumor volume was calculated by the formula: $V = 0.5a \times b^2$, and a and b denote the long and short diameters of the tumor, respectively) and the body weight of the mice were measured twice or thrice every week, and the data were recorded.

Table 6

| No. | Group | Dose (mg/kg) | Route | Number of animals | Dose cycle |
|---|---|---|---|---|---|
| 1 | Vehicle | - | iv | 6 | qw x 3 |
| 2 | Example **71** | 10 | iv | 6 | qw x 3 |

(continued)

| No. | Group | Dose (mg/kg) | Route | Number of animals | Dose cycle |
|---|---|---|---|---|---|
| 3 | Irinotecan liposome | 10 | iv | 6 | qw x 3 |
| Note: The administration volume was 10 mg/mL. | | | | | |

Analysis and assessment

Experimental evaluation index: The efficacy was assessed by the tumor growth inhibition TGI (%) or the relative tumor growth rate T/C (%), wherein T denotes the treatment group and C denotes the control group.

Calculation of relative tumor growth rate T/C (%): When $T > T_0$, $T/C (\%) = (T - T_0)/(C - C_0) \times 100\%$; When $T < T_0$, $T/C (\%) = (T - T_0)/T_0 \times 100\%$, where T and C denote the tumor volumes at the end of study; $T_0$ and $C_0$ denote the tumor volumes at the start of study.

Calculation of tumor growth inhibition TGI (%): $TGI (\%) = (1 - T/C) \times 100\%$.

Assessment criterion: T/C (%) > 40 (i.e., TGI (%) < 60%) denotes ineffective; T/C (%) ≤ 40 (i.e., TGI (%) ≥ 60%) and $P < 0.05$ denote effective.

Results of pharmacodynamic study

The inhibitory effects of the compound of Example **71** and irinotecan liposome in the human myeloma NCI-H929 nude mouse tumor model are shown in FIG. 17.

The results, as shown in FIG. 17, show that after 3 doses of the compound were administered intravenously once every week, the average tumor volume on day 17 was 1900 $mm^3$ for the irinotecan liposome group, whereas the average tumor volume was 140 $mm^3$ for the Example **71** group, which was significantly superior to that of the irinotecan liposome group. The results indicate that the compound of Example **71** of the present disclosure has high inhibitory effects on the large tumors in the human myeloma NCI-H929 nude mouse model. Although irinotecan liposome effectively inhibited tumor growth at the early stage, significant rebound occurred after discontinuation, while the compound of Example 71 of the present disclosure exhibited a lasting therapeutic effect. This suggests that small nano-scale particle of the compound has a lasting, sustained-release effect and high infiltration capacity in large tumors, and can continuously infiltrate into the deep interior of the tumor, thus possessing significant, lasting therapeutic effects and good universality.

Biological Evaluation Example 7: Pharmacodynamic study on compound of Example 71 and irinotecan liposome in human small cell lung cancer NCI-H69 nude mouse tumor model

Experimental animal and tumor grafting

BALB/c nude mice, female, aged 4-5 weeks, 18-25 g in weight, housed in the experimental environment for one week after arrival. $5 \times 10^8$ NCI-H69 tumor cells were subcutaneously grafted at the right scapula of the mice. When the tumors grew to an average volume of about 1085 $mm^3$, the mice were randomized into groups of 6 mice each by the tumor volume and body weight.

Test sample preparation

A proper amount of the compound was diluted in a 5% glucose buffer, and the mixture was shaken for dissolution. The samples were prepared right before use.

Test compound administration

The dose and treatment regimen are shown in Table 7. The volume of subcutaneous tumors in the nude mice (tumor volume was calculated by the formula: $V = 0.5a \times b^2$, and a and b denote the long and short diameters of the tumor, respectively) and the body weight of the mice were measured twice or thrice every week, and the data were recorded.

Table 7

| No. | Group | Dose (mg/kg) | Route | Number of animals | Dose cycle |
|---|---|---|---|---|---|
| 1 | Vehicle | - | iv | 6 | qw x 3 |
| 2 | Example **71** | 10 | iv | 6 | qw x 3 |

(continued)

| No. | Group | Dose (mg/kg) | Route | Number of animals | Dose cycle |
|---|---|---|---|---|---|
| 3 | Irinotecan liposome | 10 | iv | 6 | qw x 3 |
| Note: The administration volume was 10 mg/mL. | | | | | |

Analysis and assessment

Experimental evaluation index: The efficacy was assessed by the tumor growth inhibition TGI (%) or the relative tumor growth rate T/C (%), wherein T denotes the treatment group and C denotes the control group.

Calculation of relative tumor growth rate T/C (%): When $T > T_0$, $T/C (\%) = (T - T_0)/(C - C_0) \times 100\%$; When $T < T_0$, $T/C (\%) = (T - T_0)/T_0 \times 100\%$, where T and C denote the tumor volumes at the end of study; $T_0$ and $C_0$ denote the tumor volumes at the start of study.

$$\text{Calculation of tumor growth inhibition TGI (\%): TGI (\%)} = (1 - T/C) \times 100\%.$$

Assessment criterion: T/C (%) > 40 (i.e., TGI (%) < 60%) denotes ineffective; T/C (%) ≤ 40 (i.e., TGI (%) ≥ 60%) and $P < 0.05$ denote effective.

Results of pharmacodynamic study

The inhibitory effects of the compound of Example **71** and irinotecan liposome in the human small cell lung cancer NCI-H69 nude mouse tumor model are shown in FIG. 18.

The results, as shown in FIG. 18, show that after 3 doses of the compound were administered intravenously once every week, the average tumor volume on day 21 was 820 $mm^3$ for the irinotecan liposome group, whereas the average tumor volume was 250 $mm^3$ for the Example **71** group, which was significantly superior to that of the irinotecan liposome group. It is shown that the compound of Example **71** of the present disclosure exhibited a strong inhibitory effect against the large tumors in the human small cell lung cancer NCI-H69 nude mouse model, which was superior to that of the irinotecan liposome in the control group.

**Biological Evaluation Example 8: Pharmacodynamic study on compound of Example 79 in human fibrosarcoma cell HT-1080 nude mouse tumor model**

Experimental animal and tumor grafting

BALB/c nude mice, female, aged 4-5 weeks, 18-25 g in weight, housed in the experimental environment for one week after arrival. HT-1080 tumor cells were subcutaneously grafted at the right scapula of the mice. When the tumors grew to an average volume of about 1085 $mm^3$, the mice were randomized into groups of 6 mice each by the tumor volume and body weight.

Test sample preparation

A proper amount of the compound was diluted in a 5% glucose buffer, and the mixture was shaken for dissolution. The samples were prepared right before use.

Test compound administration

The dose and treatment regimen are shown in Table 8. The volume of subcutaneous tumors in the nude mice (tumor volume was calculated by the formula: $V = 0.5a \times b^2$, and a and b denote the long and short diameters of the tumor, respectively) and the body weight of the mice were measured twice or thrice every week, and the data were recorded.

Table 8

| No. | Group | Dose (mg/kg) | Route | Number of animals | Dose cycle |
|---|---|---|---|---|---|
| 1 | Vehicle | - | iv | 6 | qw x 3 |

(continued)

| No. | Group | Dose (mg/kg) | Route | Number of animals | Dose cycle |
|---|---|---|---|---|---|
| 2 | Example **79** | 10 | iv | 6 | qw x 3 |
| Note: The administration volume was 10 mg/mL. | | | | | |

Analysis and assessment:

Experimental evaluation index: The efficacy was assessed by the relative tumor growth rate T/C (%) or the tumor growth inhibition TGI (%), wherein T denotes the treatment group and C denotes the control group.

The calculation formula for relative tumor proliferation rate T/C (%) is as follows: T/C% = $T_{RTV}/C_{RTV} \times 100\%$ ($T_{RTV}$: average RTV of treatment group; $C_{RTV}$: average RTV of negative control group). Relative tumor volume (RTV) was calculated based on the tumor measurement results, and the calculation formula was $RTV = V_t/V_0$, where $V_0$ is the average tumor volume measured at the time of grouping for administration (i.e., $d_0$), and $V_t$ is the average tumor volume at a certain measurement. $T_{RTV}$ and $C_{RTV}$ were derived from data measured on the same day.

$$\text{Tumor growth inhibition TGI (\%)} = (1 - T/C) \times 100\%.$$

Assessment criterion: T/C (%) > 40 denotes ineffective; T/C (%) ≤ 40 and P < 0.05 denote effective.

Results of pharmacodynamic study

The inhibitory effect of the compound of Example **79** in the human fibrosarcoma cell HT-1080 nude mouse tumor model is shown in FIG. 19.

The results, as shown in FIG. 19, show that after 3 doses of the compound were administered intravenously once every week, on day 18, the tumor inhibition rate was 91.37% for the compound of Example **79** at a dose of 10 mg/kg, which was significantly different from that of the blank control group, suggesting that the compound of the present disclosure has an extremely strong tumor inhibitory effect on the model.

**Biological Evaluation Example 9: Pharmacodynamic study on compound of Example 80 in human small cell lung cancer NCI-H69 nude mouse tumor model**

Experimental animal and tumor grafting

BALB/c nude mice, female, aged 4-5 weeks, 18-25 g in weight, housed in the experimental environment for one week after arrival. $5 \times 10^8$ NCI-H69 tumor cells were subcutaneously grafted at the right scapula of the mice. When the tumors grew to an average volume of about 150 $mm^3$, the mice were randomized into groups of 6 mice each by the tumor volume and body weight.

Test sample preparation

A proper amount of the compound was diluted in a 5% glucose buffer, and the mixture was shaken for dissolution. The samples were prepared right before use.

Test compound administration

The dose and treatment regimen are shown in Table 9. The volume of subcutaneous tumors in the nude mice (tumor volume was calculated by the formula: $V = 0.5a \times b^2$, and a and b denote the long and short diameters of the tumor, respectively) and the body weight of the mice were measured twice or thrice every week, and the data were recorded.

Table 9

| No. | Group | Dose (mg/kg) | Route | Number of animals | Dose cycle |
|---|---|---|---|---|---|
| 1 | Vehicle | - | iv | 6 | qwx3 |

(continued)

| No. | Group | Dose (mg/kg) | Route | Number of animals | Dose cycle |
|---|---|---|---|---|---|
| 2 | Example 80 | 10 | iv | 6 | qwx3 |
| Note: The administration volume was 10 mg/mL. | | | | | |

Analysis and assessment

The analysis and assessment criterion is the same as that in Biological Evaluation Example 8.

Results of pharmacodynamic study

The inhibitory effect of the compound of Example **80** in the human small cell lung cancer NCI-H69 nude mouse tumor model is shown in FIG. 20.

The results, as shown in FIG. 20, show that after 3 doses of the compound were administered intravenously once every week, on day 30, the tumor inhibition rate was 95.04% for the compound of Example **80** at a dose of 10 mg/kg, which was significantly different from that of the blank control group, suggesting that the compound of the present disclosure has an extremely strong tumor inhibitory effect on the model.

**Biological Evaluation Example 10: Pharmacodynamic study on compounds of Examples 82, 83, 86, 92, 94, 97, and 98 in human small cell lung cancer NCI-H69 nude mouse tumor model**

Experimental animal and tumor grafting

BALB/c nude mice, female, aged 4-5 weeks, 18-25 g in weight, housed in the experimental environment for one week after arrival. $5 \times 10^8$ NCI-H69 tumor cells were subcutaneously grafted at the right scapula of the mice. When the tumors grew to an average volume of about 170 $mm^3$, the mice were randomized into groups of 6 mice each by the tumor volume and body weight.

Test sample preparation

A proper amount of the compound was diluted in normal saline, and the mixture was shaken for dissolution. The samples were prepared right before use.

Test compound administration

The dose and treatment regimen are shown in Table 10. The volume of subcutaneous tumors in the nude mice (tumor volume was calculated by the formula: $V = 0.5a \times b^2$, and a and b denote the long and short diameters of the tumor, respectively) and the body weight of the mice were measured twice or thrice every week, and the data were recorded.

Table 10

| No. | Group | Dose (mg/kg) | Route | Number of animals | Dose cycle |
|---|---|---|---|---|---|
| 1 | Vehicle | - | iv | 6 | qw x 3 |
| 2 | Example **82** | 10 | iv | 6 | qw x 3 |
| 3 4 | Example **83** | 10 | iv | 6 | qw x 3 |
| | Example **86** | 10 | iv | 6 | qw x 3 |
| 5 | Example **92** | 10 | iv | 6 | qw x 3 |
| 6 | Example **94** | 10 | iv | 6 | qw x 3 |
| 7 | Example **97** | 10 | iv | 6 | qw x 3 |
| 8 | Example **98** | 10 | iv | 6 | qw x 3 |
| Note: The administration volume was 10 mg/mL. | | | | | |

Analysis and assessment

The analysis and assessment criterion is the same as that in Biological Evaluation Example 8.

Results of pharmacodynamic study

The inhibitory effects of the compounds of Example **82** and other examples in the human small cell lung cancer NCI-H69 nude mouse tumor model are shown in FIG. 21.

The results, as shown in FIG. 21, show that after 3 doses of the compound were administered intravenously once every week, on day 40, the tumor inhibition rates were 98.55%, 98.48%, 98.47%, 96.69%, 93.45%, 99.08%, and 95.04% for the compounds of Examples **82, 83, 86, 92, 94, 97,** and **98,** respectively, at a dose of 10 mg/kg, which were significantly different from that of the blank control group, suggesting that the compounds of the present disclosure have extremely strong tumor inhibitory effects on the model.

**Biological Evaluation Example 11: Pharmacodynamic study on compounds of Examples 84, 85, 87, 114, and 115 in human breast cancer cell BCaP-37 nude mouse tumor model**

Experimental animal and tumor grafting

BALB/c nude mice, female, aged 4-5 weeks, 18-25 g in weight, housed in the experimental environment for one week after arrival. $5 \times 10^8$ BCaP-37 tumor cells were subcutaneously grafted at the right scapula of the mice. When the tumors grew to an average volume of about 170 $mm^3$, the mice were randomized into groups of 6 mice each by the tumor volume and body weight.

Test sample preparation

A proper amount of the compound was diluted in normal saline, and the mixture was shaken for dissolution. The samples were prepared right before use.

Test compound administration

The dose and treatment regimen are shown in Table 11. The volume of subcutaneous tumors in the nude mice (tumor volume was calculated by the formula: $V = 0.5a \times b^2$, and a and b denote the long and short diameters of the tumor, respectively) and the body weight of the mice were measured twice or thrice every week, and the data were recorded.

Table 11

| No. | Group | Dose (mg/kg) | Route | Number of animals | Dose cycle |
|---|---|---|---|---|---|
| 1 | Vehicle | - | iv | 6 | qw x 3 |
| 2 | Abraxane | 30 | iv | 6 | qw x 3 |
| 3 4 | Example **84** | 30 | iv | 6 | qw x 3 |
| | Example **85** | 30 | iv | 6 | qw x 3 |
| 5 | Example **87** | 30 | iv | 6 | qw x 3 |
| 6 | Example **114** | 30 | iv | 6 | qw x 3 |
| 7 | Example **115** | 30 | iv | 6 | qw x 3 |

Note: The administration volume was 10 mg/mL.

Analysis and assessment

The analysis and assessment criterion is the same as that in Biological Evaluation Example 8.

Results of pharmacodynamic study

The inhibitory effects of the compounds of Example **84** and other examples in the human breast cancer cell BCaP-37 nude mouse tumor model are shown in FIG. 22.

The results, as shown in FIG. 22, show that after 3 doses of the compound were administered intravenously once

every week, on day 31, the tumor inhibition rates were 98.41%, 97.47%, 97.0%, 97.65%, and 83.33% for the compounds of Examples **84, 85, 87, 114,** and 115 at a dose of 30 mg/kg, while the tumor inhibition rate in the albumin-bound paclitaxel control group was only 29.66%, with significant difference, suggesting that the compounds of the present disclosure have extremely strong tumor inhibitory effects on the model, superior to that of albumin-bound paclitaxel.

**Biological Evaluation Example 12: Pharmacodynamic study on compounds of Examples 80 and 93 in human breast cancer cell MCF-7 nude mouse tumor model**

Experimental animal and tumor grafting

BALB/c nude mice, female, aged 4-5 weeks, 20-25 g in weight, housed in the experimental environment for one week after arrival. Estrogen sustained-release microspheres were subcutaneously administered in the middle of the back, and MCF-7 tumor mass was grafted *in situ* in the second nipple fat pad on the right side two days later. When the tumors grew to an average volume of about 167 $mm^3$, the mice were randomized into groups of 6 mice each by the tumor volume and body weight.

Test sample preparation

A proper amount of the compound was diluted in normal saline, and the mixture was shaken for dissolution. The samples were prepared right before use.

Test compound administration

The dose and treatment regimen are shown in Table 12. The volume of subcutaneous tumors in the nude mice (tumor volume was calculated by the formula: $V = 0.5a \times b^2$, and a and b denote the long and short diameters of the tumor, respectively) and the body weight of the mice were measured twice or thrice every week, and the data were recorded.

Table 12

| No. | Group | Dose (mg/kg) | Route | Number of animals | Dose cycle |
|---|---|---|---|---|---|
| 1 | Vehicle | - | iv | 6 | qw x 2 |
| 2 | Example **80** | 10 | iv | 6 | qw x 2 |
| 3 | Example **93** | 10 | iv | 6 | qw x 2 |

Note: The administration volume was 10 mg/mL.

Analysis and assessment

The analysis and assessment criterion is the same as that in Biological Evaluation Example 8.

Results of pharmacodynamic study

The inhibitory effects of the compounds of Examples **80** and **93** in the human breast cancer cell MCF-7 nude mouse tumor model are shown in FIG. 23.

The results, as shown in FIG. 23, show that after 2 doses of the compound were administered intravenously once every week, on day 21, the tumor inhibition rates were 99.36% and 99.62% for the compounds of Examples 80 and 93, respectively, at a dose of 10 mg/kg, which were significantly different from that of the blank control group, suggesting that the compounds of the present disclosure has an extremely strong tumor inhibitory effect on the model.

**Biological Evaluation Example 13: Pharmacodynamic study on compounds of Examples 100, 102, 103, 106, and 108 in human liver cancer cell HepG2 nude mouse tumor model**

Experimental animal and tumor grafting

BALB/c nude mice, female, aged 4-5 weeks, 20-25 g in weight, housed in the experimental environment for one week after arrival. HepG2 tumor mass was subcutaneously grafted on the right back of the nude mice. When the tumors reached ~140 $mm^3$, the mice were divided into groups of 6 mice each.

Test sample preparation

A proper amount of the compound was diluted in 5% glucose, and the mixture was shaken for dissolution. The samples were prepared right before use.

Test compound administration

The dose and treatment regimen are shown in Table 13. The volume of subcutaneous tumors in the nude mice (tumor volume was calculated by the formula: $V = 0.5a \times b^2$, and a and b denote the long and short diameters of the tumor, respectively) and the body weight of the mice were measured twice or thrice every week, and the data were recorded.

Table 13

| No. | Group | Dose (mg/kg) | Route | Number of animals | Dose cycle |
|---|---|---|---|---|---|
| 1 | Vehicle | - | iv | 6 | qw x 3 |
| 2 | Example **100** | 10 | iv | 6 | qw x 3 |
| 3 | Example **102** | 10 | iv | 6 | qw x 3 |
| 4 | Example **103** | 10 | iv | 6 | qw x 3 |
| 5 | Example **106** | 10 | iv | 6 | qw x 3 |
| 6 | Example **108** | 10 | iv | 6 | qw x 3 |
| Note: The administration volume was 10 mg/mL. | | | | | |

Analysis and assessment

The analysis and assessment criterion is the same as that in Biological Evaluation Example 8.

Results of pharmacodynamic study

The inhibitory effects of the compounds of Example **100** and other examples in the human liver cancer HepG2 nude mouse tumor model are shown in FIG. 25.
The results, as shown in FIG. 24, show that after 3 doses of the compound were administered intravenously once every week, on day 40, the tumor inhibition rates were 98.12%, 94.23%, 90.12%, 99.12%, and 98.96% for the compounds of Examples **100, 102, 103, 106,** and **108,** respectively, at a dose of 10 mg/kg, which were significantly different from that of the blank control group, suggesting that the compound of the present disclosure has a strong tumor inhibitory effect on the model.

**Biological Evaluation Example 14: Nano-scale particle size study on compounds of the present disclosure**

Sample preparation: A required volume of purified water or water for injection was added into a glass bottle and filtered through a 0.22 μm filter membrane. A proper amount of sample was dissolved in 0.5 mL of the above water for injection, and the solution was filtered through a 0.22 μm aqueous filter membrane, transferred into a 10 mL volumetric flask, and diluted with a diluent to the volume (final compound concentration 10 mg/mL). The flask was gently shaken to avoid bubble production, and the solution was preserved for later use.
Sample detection: The sample solution was slowly transferred to a cuvette by using a dropper for sample detection. The detection system was Zetasizer Pro Blue.
Results: The nano-scale particle size data for the representative compounds of the present disclosure are shown in FIGs. 25-30 and Table 14.

Table 14. Nano-scale particle size data for representative compounds of the present disclosure

| Example | Average particle size (nm) | Polydispersity index (PDI) |
|---|---|---|
| Example **79** | 14.4 | 0.12 |
| Example **80** | 13.6 | 0.13 |
| Example **83** | 13.4 | 0.14 |
| Example **84** | 23.6 | 0.10 |

(continued)

| Example | Average particle size (nm) | Polydispersity index (PDI) |
|---|---|---|
| Example **85** | 18.9 | 0.13 |
| Example **87** | 20.2 | 0.23 |
| Example **88** | 18.5 | 0.11 |
| Example **92** | 15.3 | 0.12 |
| Example **93** | 12.3 | 0.17 |
| Example **94** | 13.7 | 0.13 |
| Example **96** | 13.7 | 0.13 |
| Example **97** | 14.6 | 0.08 |
| Example **112** | 13.6 | 0.17 |

The results in Table 14 show that the compounds of the present disclosure all have an average nanoparticle size in the range of 10-30 nm and a narrow particle size dispersity index.

**Biological Evaluation Example 15: Particle size stability study on compounds of Examples 79 and 80**

Sample preparation: A required volume of purified water or water for injection was added into a glass bottle and filtered through a 0.22 μm filter membrane. A proper amount of sample was dissolved in 0.5 mL of the above water for injection, and the solution was filtered through a 0.22 μm aqueous filter membrane, transferred into a 5 mL centrifuge tube, and incubated in an incubator at 25 °C.

Sample detection: At the detection time points, samples were taken and slowly transferred to a cuvette by using a pipette for sample detection. The detection system was Zetasizer Pro Blue.

Results: The particle size stability study data on compounds of Examples **79 and 80** of the present disclosure are shown in Table 15 and FIGs. 31-34.

Table 15. Particle size stability study on compounds of Examples **79** and **80** of the present disclosure

| Compound / Time | Example **79** | | Example **80** | | Example **78** (comparative) | |
|---|---|---|---|---|---|---|
| | Average particle size (nm) | PDI | Average particle size (nm) | PDI | Average particle size (nm) | PDI |
| Day 0 | 14.4 | 0.12 | 13.6 | 0.13 | 12.7 | 0.52 |
| Day 30 | 14.6 | 0.14 | 13.5 | 0.12 | 13.4 | 0.76 |
| Day 60 | 14.3 | 0.13 | 13.4 | 0.13 | 16.9 | 0.78 |

The results show that the compounds of Examples **79** and **80** of the present disclosure exhibited very stable particle sizes and no significant changes during the long-term preservation at room temperature (25 °C), suggesting stable physicochemical properties; the compound of Comparative Example **78** exhibited an increase in particle size and aggregation and other phenomena unfavorable to the stability during long-term storage.

**Biological Evaluation Example 16: Pharmacodynamic study on compounds of Examples 71, 78, 79, 80, and 112 in human breast cancer cell MCF-7 nude mouse tumor model**

Experimental animal and tumor grafting

BALB/c nude mice, female, aged 4-5 weeks, 20-25 g in weight, housed in the experimental environment for one week after arrival. Estrogen sustained-release microspheres were subcutaneously administered in the middle of the back, and MCF-7 tumor mass was grafted *in situ* in the second nipple fat pad on the right side two days later. When the tumors grew to an average volume of about 200 $mm^3$, the mice were randomized into groups of 6 mice each by the tumor volume and body weight.

Test sample preparation

A proper amount of the compound was diluted in normal saline, and the mixture was shaken for dissolution. The samples were prepared right before use.

Test compound administration

The dose and treatment regimen are shown in Table 16. The volume of subcutaneous tumors in the nude mice (tumor volume was calculated by the formula: $V = 0.5a \times b^2$, and a and b denote the long and short diameters of the tumor, respectively) and the body weight of the mice were measured twice or thrice every week, and the data were recorded.

Table 16

| No. | Group | Dose (mg/kg) | Route | Number of animals | Dose cycle |
|---|---|---|---|---|---|
| 1 | Vehicle | - | iv | 6 | qw x 2 |
| 2 | Example **71** | 5 | iv | 6 | qw x 2 |
| 3 4 | Example **78** | 5 | iv | 6 | qw x 2 |
| | Example **79** | 5 | iv | 6 | qw x 2 |
| 5 | Example **80** | 5 | iv | 6 | qw x 2 |
| 6 | Example **112** | 5 | iv | 6 | qw x 2 |
| Note: The administration volume was 10 mg/mL. | | | | | |

Analysis and assessment

The analysis and assessment criterion is the same as that in Biological Evaluation Example 8.

Results of pharmacodynamic study

The inhibitory effects of the compounds of Example **71** and other examples in the human breast cancer cell MCF-7 nude mouse tumor model are shown in FIG. 35.

The results, as shown in FIG. 35, show that after 2 doses of the compound were administered intravenously once every week, on day 25, the tumor inhibition rates were 96.54%, 97.41%, and 94.95% for the compounds of Examples **79, 80,** and **112,** respectively, at a dose of 5 mg/kg, which were significantly different from that of the blank control group, slightly superior to that of Example **71** (tumor inhibition rate 92.05%), and significantly superior to Comparative Example **78** (tumor inhibition rate 77.16%).

**Biological Evaluation Example 17: Pharmacodynamic study on compounds of Examples 79, 80, 97 and 113 and irinotecan liposome in human small cell lung cancer NCI-H69 nude mouse tumor model**

Experimental animal and tumor grafting

BALB/c nude mice, female, aged 4-5 weeks, 18-25 g in weight, housed in the experimental environment for one week after arrival. $5 \times 10^8$ NCI-H69 tumor cells were subcutaneously grafted at the right scapula of the mice. When the tumors grew to an average volume of about 200 $mm^3$, the mice were randomized into groups of 6 mice each by the tumor volume and body weight.

Test sample preparation

A proper amount of the compound was diluted in 5% glucose, and the mixture was shaken for dissolution. The samples were prepared right before use.

Test compound administration

The dose and treatment regimen are shown in Table 17. The volume of subcutaneous tumors in the nude mice (tumor volume was calculated by the formula: $V = 0.5a \times b^2$, and a and b denote the long and short diameters of the tumor, respectively) and the body weight of the mice were measured twice or thrice every week, and the data were recorded.

Table 17

| No. | Group | Dose (mg/kg) | Route | Number of animals | Dose cycle |
|---|---|---|---|---|---|
| 1 | Vehicle | - | iv | 6 | qw x 3 |
| 2 | Example **79** | 1.5 | iv | 6 | qw x 3 |
| 3 4 | Example **80** | 1.5 | iv | 6 | qw x 3 |
| | Example **97** | 1.5 | iv | 6 | qw x 3 |
| 5 | Example **113** | 1.5 | iv | 6 | qw x 3 |
| 6 | Irinotecan liposome | 1.5 | iv | 6 | qw x 3 |
| Note: The administration volume was 10 mg/mL. | | | | | |

Analysis and assessment

The analysis and assessment criterion is the same as that in Biological Evaluation Example 8.

Results of pharmacodynamic study

The inhibitory effects of the compounds of Example **79** and other examples in the human small cell lung cancer NCI-H69 nude mouse tumor model are shown in FIG. 36.

The results, as shown in FIG. 36, show that after 3 doses of the compound were administered intravenously once every week, on day 19, the tumor inhibition rates were 97.90%, 96.43%, 93.84%, and 89.73% for the compounds of Examples **79, 80, 97,** and **113,** respectively, at a dose of 1.5 mg/kg, which were significantly different from that of the blank control group and significantly superior to the efficacy of irinotecan liposome (tumor inhibition rate 52.57%).

**Biological Evaluation Example 18: Pharmacodynamic study on compound of Example 119 in HepG2 model**

Experimental animal and tumor grafting

BALB/c nude mice, female, aged 4-5 weeks, 20-25 g in weight, housed in the experimental environment for one week after arrival. HepG2 tumor mass was subcutaneously grafted on the right back of the nude mice. When the tumors reached ~450 $mm^3$, the mice were divided into groups of 6 mice each.

Test sample preparation

To a certain volume of normal saline was added a proper amount of the compound, and the mixture was shaken for dissolution and filtered through a 0.2 μm filter membrane.

Test compound administration

The dose and treatment regimen are shown in Table 18. The volume of subcutaneous tumors in the nude mice (tumor volume was calculated by the formula: $V = 0.5a \times b^2$, and a and b denote the long and short diameters of the tumor, respectively) and the body weight of the mice were measured twice or thrice every week, and the data were recorded.

Table 18

| No. | Group | Dose (mg/kg) | Route | Number of animals | Dose cycle |
|---|---|---|---|---|---|
| 1 | Vehicle | - | iv | 6 | qw x 3 |
| 2 | Example **119** | 10 | iv | 6 | qw x 3 |
| Note: The administration volume was 10 mg/mL. | | | | | |

Analysis and assessment

The analysis and assessment criterion is the same as that in Biological Evaluation Example 8.

Results of pharmacodynamic study

The inhibitory effect of the compound of Example **119** in the human small cell lung cancer NCI-H69 nude mouse tumor model is shown in FIG. 37.

The results, as shown in FIG. 37, show that after 3 doses of the compound were administered intravenously once every week, on day 28, the tumor inhibition rate was 99.48% for the compound of Example **119** of the present disclosure at a dose of 10 mg/kg, which was significantly different from that of the blank control group, suggesting that the compound of the present disclosure has an extremely strong tumor inhibitory effect on the tumor model.

Although specific embodiments of the present disclosure have been described above, it will be appreciated by those skilled in the art that these embodiments are merely illustrative and that many changes or modifications can be made to these embodiments without departing from the principles and spirit of the present disclosure. The scope of protection of the present disclosure is therefore defined by the appended claims.

## Claims

1. A stable polymer-drug conjugate of formula (I) with a controllable number of conjugated groups and a controllable nano-scale size,

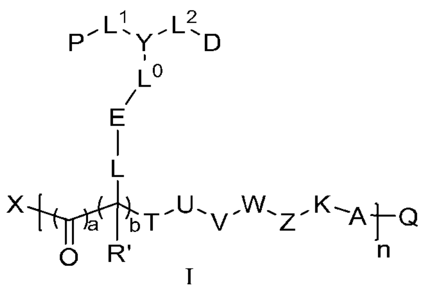

I

comprising:

(1) a polymer residue of formula (II), with a polymer backbone repeating unit number n,

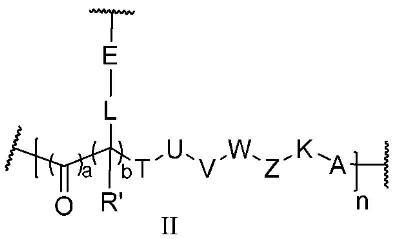

II

,

wherein n is selected from integers of 4-100;
(2) a branched center Y having at least trifunctionality;
(3) a pharmacokinetic regulator residue P;

(4) a pharmaceutically active agent residue D, wherein D may be one or more drug residues;
(5) a terminal group X;
(6) $L^0$, $L^1$, and $L^2$, each independently being a covalent bond or a $C_1$-$C_{40}$ linker either containing a heteroatom or not, wherein the heteroatom is O, S, Se, N, P, Si, or B, the number of the heteroatom(s) is one or more, and when more than one heteroatom is present, the heteroatoms are identical or different; the linker may either contain an unsaturated group or not; $L^0$ links E and Y, $L^1$ links P and Y, and $L^2$ links D and Y; and
(7) a residue Q of formula (III), which is H, $R^a$, a hydroxy protecting group, a sulfhydryl protecting group, an amino protecting group:

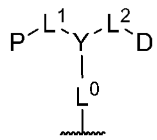
III ;

wherein, (8) any hydrogen in formula (I) may be substituted by deuterium;
(9) any chiral center in formula (I) may be *R* configuration, *S* configuration, or a mixture of *R* configuration and S configuration.

2. The polymer-drug conjugate according to claim 1, wherein the nano-scale size is an average nanoparticle size in the range of 1-100 nm.

3. The polymer-drug conjugate according to claim 1, wherein the nano-scale size is an average nanoparticle size in the range of 1-50 nm.

4. The polymer-drug conjugate according to claim 1, wherein the nano-scale size is an average nanoparticle size in the range of 5-30 nm.

5. The polymer-drug conjugate according to claim 1, wherein
the polymer residue of formula (II) meets one or more of the following conditions:

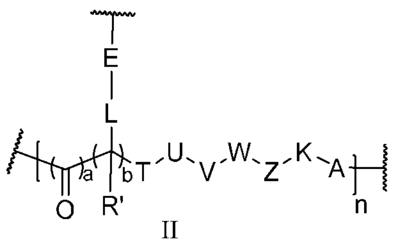
II

(1) L is independently a covalent bond,

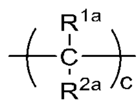
,

or a $C_1$-$C_{10}$ linker either containing a heteroatom or not, wherein the heteroatom is O, S, Se, N, P, Si, or B, the number of the heteroatom(s) is one or more, and when more than one heteroatom is present, the heteroatoms are identical or different; the linker may either contain an unsaturated group or not; L links the polymer backbone and E;
(2) E is independently a covalent bond, O, S, $NR^a$, C(=O), S(=O), $S(=O)_2$, $C(=O)NR^a$, or one of the following residues:

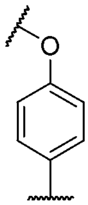 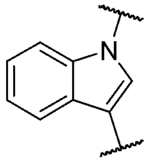 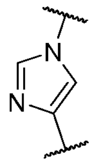 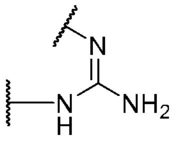 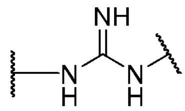 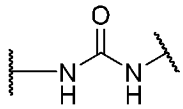 ;

(3) A is independently a covalent bond, O, S, or $NR^d$, wherein $R^d$ is selected from H, substituted or unsubstituted $C_1$-$C_{10}$ alkyl, or a residue of formula (III):

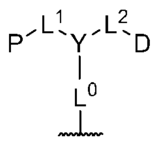

III ;

(4) T, U, V, W, Z, and K are independently a covalent bond, O, S, $NR^d$, C(=O), S(=O), $S(=O)_2$,

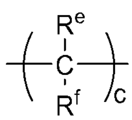, 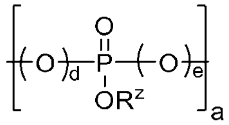, or 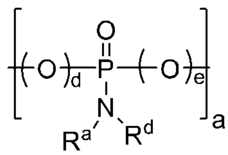,

with the proviso that: the -T-U-V-W-Z-K-A- chain does not contain the following linking forms: -O-O-, -O-S-, -S-O-,

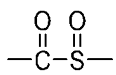,

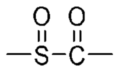, 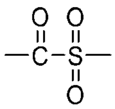, 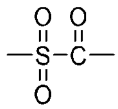, 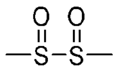, 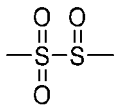, or 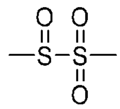;

(5) the configuration of the chiral carbon atom in the polymer residue of formula (II) may be *R* configuration, *S* configuration, or a mixture of *R* configuration and *S* configuration,
wherein,

n is selected from integers of 4-100;
a is selected from 0 and 1;
b is selected from 0 and 1;
c is selected from 0 and integers of 1-10;
d is selected from 0 and 1;
e is selected from 0 and 1;
R', $R^{1a}$, and $R^{2a}$ are selected from hydrogen, deuterium, $C_1$-$C_{10}$ alkyl, $C_1$-$C_{10}$ alkoxy, $C_3$-$C_{10}$ alkenyl, $C_3$-$C_{10}$ alkynyl, $C_3$-$C_8$ cycloalkyl, $C_2$-$C_8$ heterocycloalkyl, $C_6$-$C_{10}$ aryl, or $C_5$-$C_{10}$ heteroaryl;
$R^z$ is selected from hydrogen, $C_1$-$C_{10}$ alkyl, $C_1$-$C_{10}$ alkoxy, $C_3$-$C_{10}$ alkenyl, $C_3$-$C_{10}$ alkynyl, $C_3$-$C_8$ cycloalkyl, $C_2$-$C_8$ heterocycloalkyl, $C_6$-$C_{10}$ aryl, $C_5$-$C_{10}$ heteroaryl, a hydroxy protecting group, or a residue of formula (III):

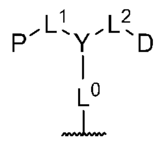

III ;

$R^e$ and $R^f$ are independently selected from hydrogen, deuterium, $C_1$-$C_{10}$ alkyl, $C_1$-$C_{10}$ alkoxy, $C_3$-$C_{10}$ alkenyl, $C_3$-$C_{10}$ alkynyl, $C_3$-$C_8$ cycloalkyl, $C_2$-$C_8$ heterocycloalkyl, $C_6$-$C_{10}$ aryl, $C_5$-$C_{10}$ heteroaryl, or a residue of formula (IV):

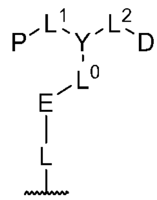

IV ;

$R^a$ is selected from H, $C_1$-$C_{10}$ alkyl, $C_1$-$C_{10}$ alkoxy, $C_3$-$C_{10}$ alkenyl, $C_3$-$C_{10}$ alkynyl, $C_3$-$C_8$ cycloalkyl, $C_2$-$C_8$ heterocycloalkyl, $C_6$-$C_{10}$ aryl, $C_5$-$C_{10}$ heteroaryl, or an amino protecting group;
$R^d$ is selected from H, $C_1$-$C_{10}$ alkyl, $C_1$-$C_{10}$ alkoxy, $C_3$-$C_{10}$ alkenyl, $C_3$-$C_{10}$ alkynyl, $C_3$-$C_8$ cycloalkyl, $C_2$-$C_8$ heterocycloalkyl, $C_6$-$C_{10}$ aryl, $C_5$-$C_{10}$ heteroaryl, an amino protecting group, or a residue of formula (III):

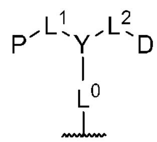

III ;

the heteroatom in the $C_2$-$C_8$ heterocycloalkyl is O, S, or N, the number of the heteroatom(s) is one or more, and when more than one heteroatom is present, the heteroatoms are identical or different; the heteroatom in the $C_3$-$C_{10}$ heteroaryl is O, S, or N, the number of the heteroatom(s) is one or more, and when more than one heteroatom is present, the heteroatoms are identical or different.

**6.** The polymer-drug conjugate according to claim 1, wherein the polymer-drug conjugate meets one or more of the following conditions:

(1) the polymer backbone repeating unit number n is an integer of 5-70, preferably an integer of 20-40;
(2) the branched center Y is a branched center containing the following structure, or a multifunctional branched center consisting of two or more branching structures:

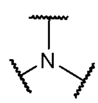 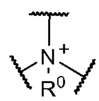 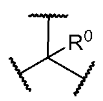 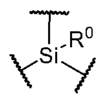 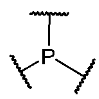 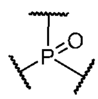 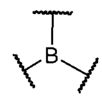

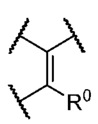 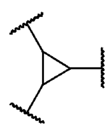 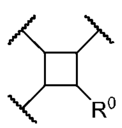 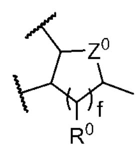 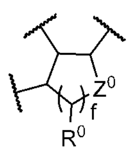 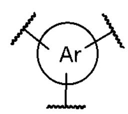

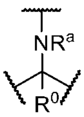 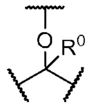 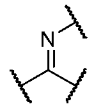 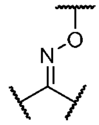 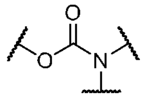 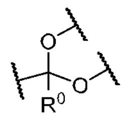 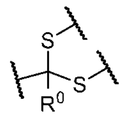

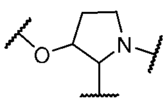 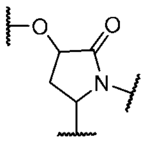 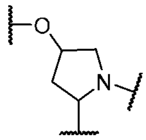

wherein, $Z^0$ is O, S, S(O), $S(O)_2$, $NR^a$, or $CHR^0$;

$R^0$ is selected from H, D, halogen, nitro, cyano, $C_1$-$C_{10}$ alkyl, $C_1$-$C_{10}$ alkoxy, $C_3$-$C_{10}$ alkenyl, $C_3$-$C_{10}$ alkynyl, $C_3$-$C_8$ cycloalkyl, $C_2$-$C_8$ heterocycloalkyl, $C_6$-$C_{10}$ aryl, $C_5$-$C_{10}$ heteroaryl, or a group containing a primary amine, secondary amine, tertiary amine, hydroxy, sulfhydryl, carboxyl, ester group, amide, boric acid, borate, phosphoric acid, sulfonic acid, sulfoxide, aldehyde group, or ketone functional group; the heteroatom in the $C_2$-$C_8$ heterocycloalkyl is O, S, or N, the number of the heteroatom(s) is one or more, and when more than one heteroatom is present, the heteroatoms are identical or different; the heteroatom in the $C_5$-$C_{10}$ heteroaryl is O, S, or N, the number of the heteroatom(s) is one or more, and when more than one heteroatom is present, the heteroatoms are identical or different;

Ar is $C_6$-$C_{20}$ aryl or $C_5$-$C_{20}$ heteroaryl; the heteroatom in the $C_5$-$C_{20}$ heteroaryl is O, S, or N, the number of the heteroatom(s) is one or more, and when more than one heteroatom is present, the heteroatoms are identical or different;

f is 0 or an integer of 1-3,

wherein $R^a$ is as defined in claim 1.

**7.** The polymer-drug conjugate according to any one of claims 1, 5, and 6, wherein the branched center Y is a substituted or unsubstituted amino acid or a derivative thereof having at least trifunctionality, the amino acid is a natural amino acid or an unnatural amino acid, the configuration of the amino acid is *D* or *L*, or a mixture of *D*/*L* configurations, and when the amino acid is a mixture of *D*/*L* configurations, the proportion of *L* configuration is greater than 0% but less than 100%; the branched center Y is preferably an amino acid having at least trifunctionality, and the amino acid is selected from one or more of aspartic acid, glutamic acid, lysine, ornithine, arginine, citrulline, histidine, serine, threonine, tryptophan, tyrosine, hydroxyproline, cystine, cysteine, and selenocysteine, the configuration of the amino acid is *D* or *L,* or a mixture of *D*/*L* configurations, and when the amino acid is a mixture of *D*/*L* configurations, the proportion of *L* configuration is greater than 0% but less than 100%.

**8.** The polymer-drug conjugate according to claim 1, wherein the linkers $L^0$ and $L^1$ are each independently selected from a covalent bond, an environmentally responsive linker, or a non-environmentally responsive linker; $L^2$ is an environment-responsive linker with a structure of $L^{2a}$-$L^{2b}$, wherein $L^{2a}$ or $L^{2b}$ may be present alone or together; $L^{2a}$ and $L^{2b}$ are each independently selected from a covalent bond, an environmentally responsive linker, or a non-environmentally responsive linker; the structure of the polymer-drug conjugate has a structure of formula (V):

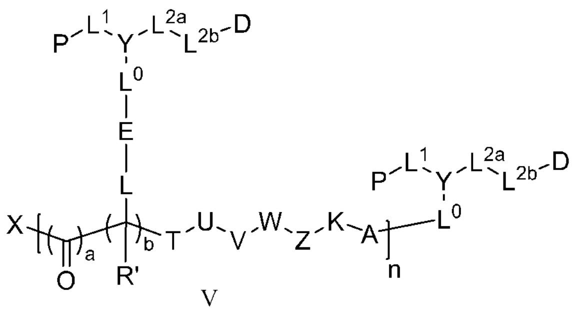

V

wherein, X, R', T, U, V, W, Z, K, A, P, $L^1$, Y, $L^{2a}$, $L^{2b}$, D, $L^0$, E, L, a, and b are each as defined in claim 1 or 5.

**9.** The polymer-drug conjugate according to claim 8, wherein the linkers $L^0$ and $L^1$ are covalent bonds, and $L^2$ is an environment-responsive linker with a structure of $L^{2a}$-$L^{2b}$, wherein $L^{2a}$ is linked to Y, $L^{2b}$ is linked to D, and $L^{2a}$ or $L^{2b}$

may be present alone or together; $L^{2a}$ and $L^{2b}$ are each independently selected from a covalent bond, an environmentally responsive linker, or a non-environmentally responsive linker; the structure of the polymer-drug conjugate has a structure of formula (VI):

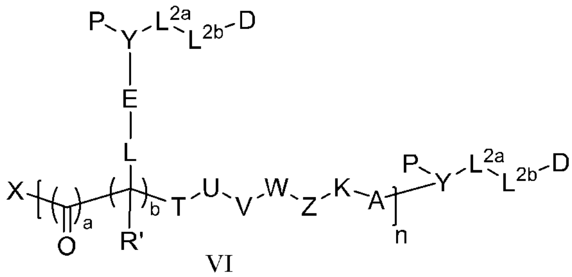

VI

wherein, X, R', T, U, V, W, Z, K, A, P, Y, $L^{2a}$, $L^{2b}$, D, E, L, a, and b are each as defined in claim 1 or 5.

**10.** The polymer-drug conjugate according to any one of claims 1, 5, 8, and 9, wherein the polymer residue is selected from the following structures:

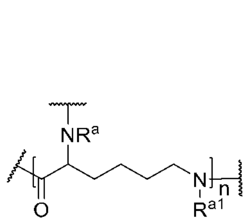
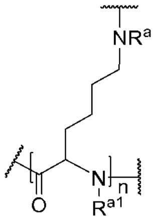
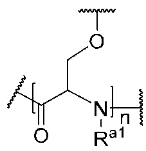
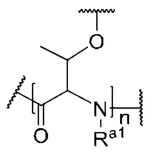
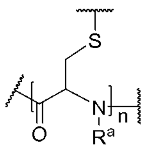
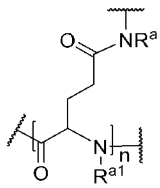
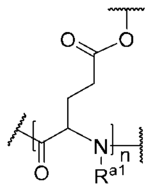
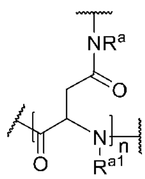
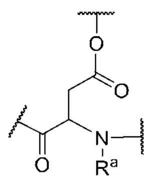
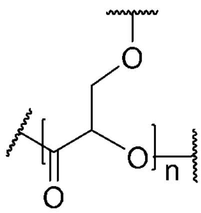
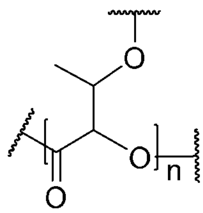
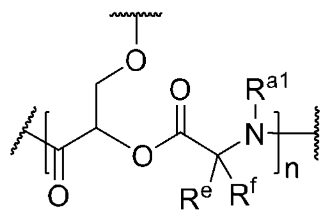
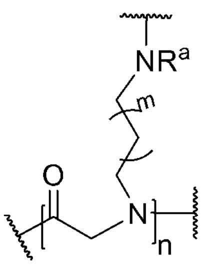
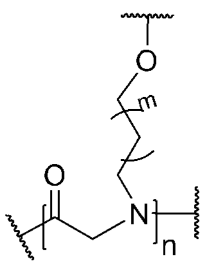
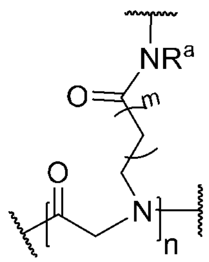
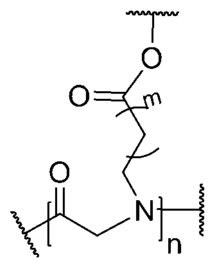

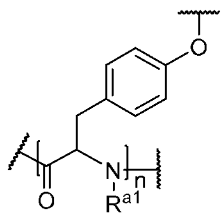
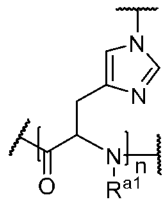
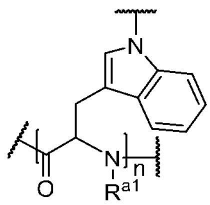
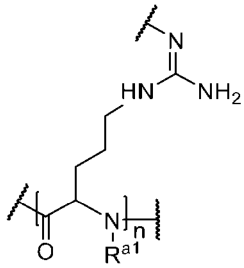

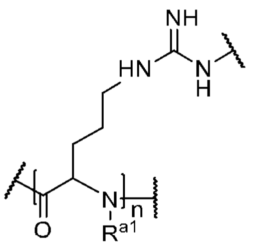
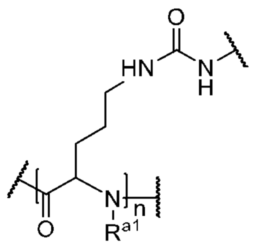
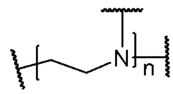

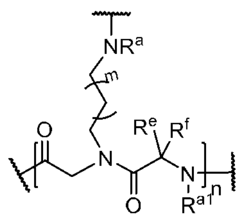
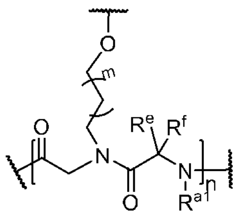
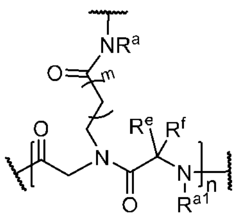
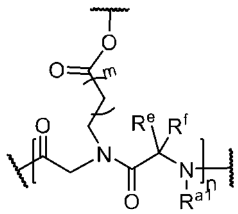

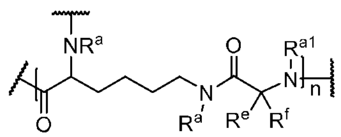
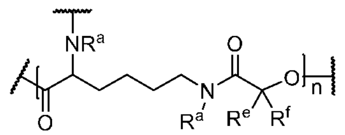

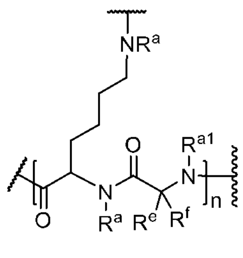
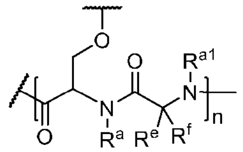
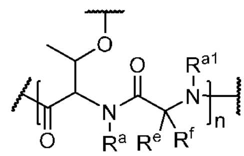
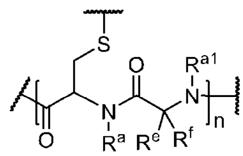

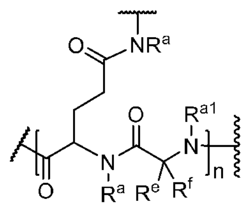
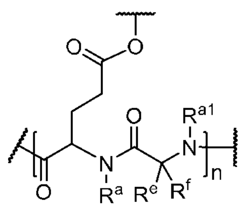
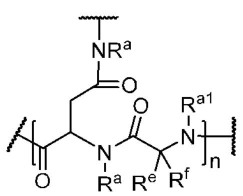
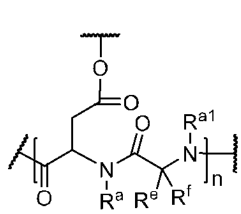

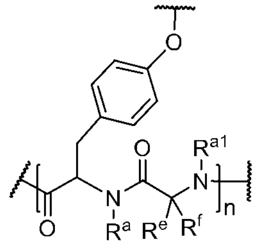
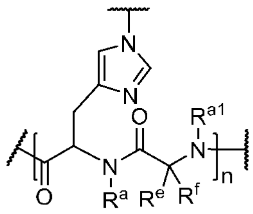
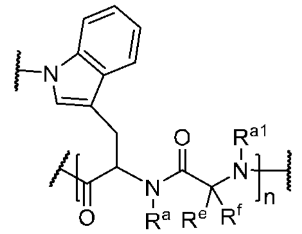

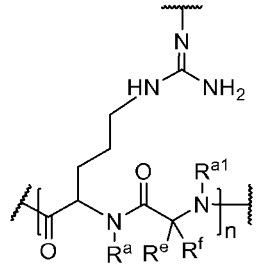
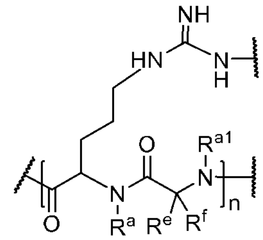
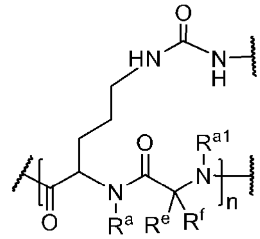

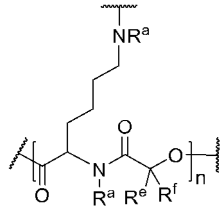
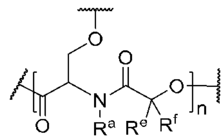
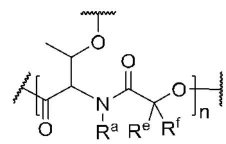
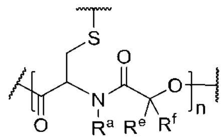

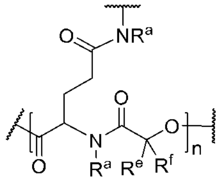
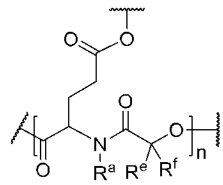
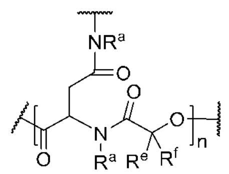
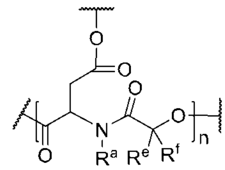

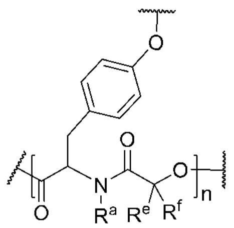
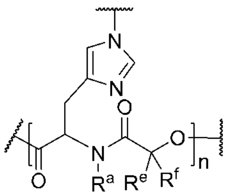
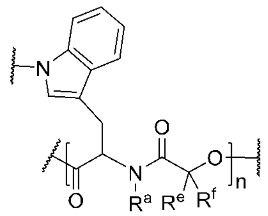

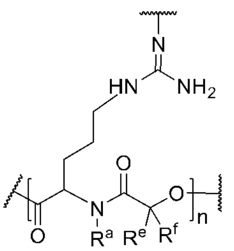
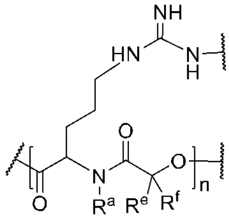
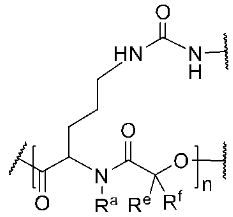

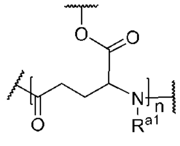 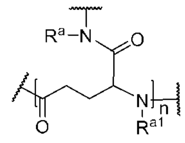 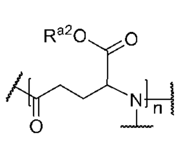 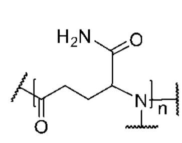 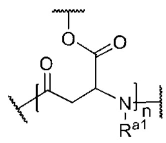

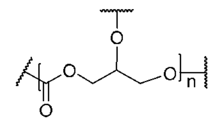 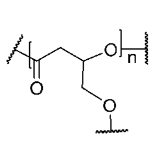 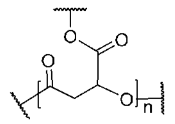 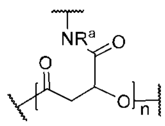 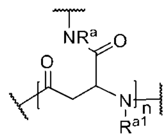

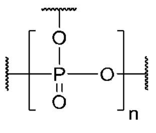 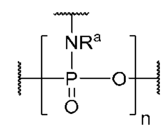 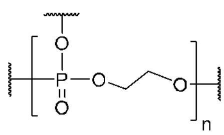 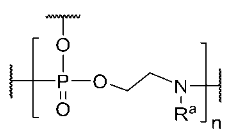

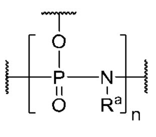 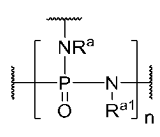 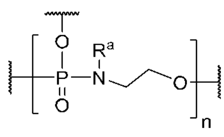 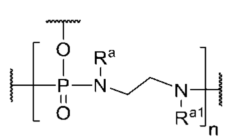

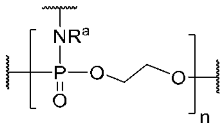 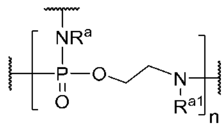 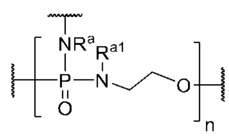 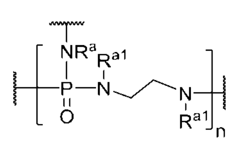

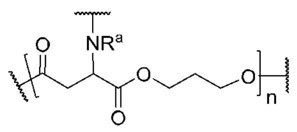 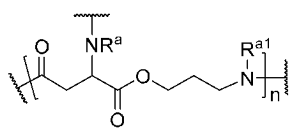 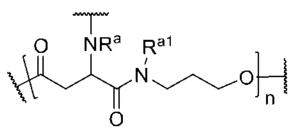

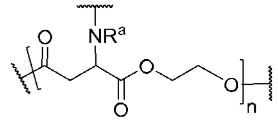 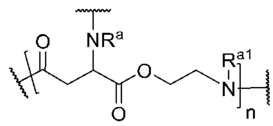 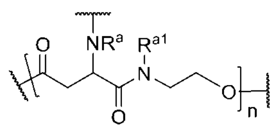

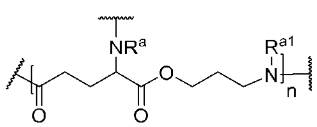 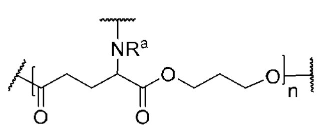 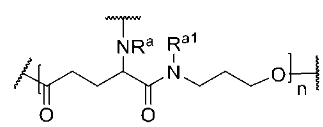

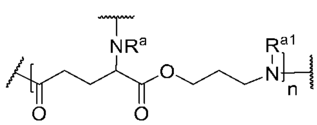 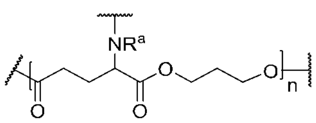 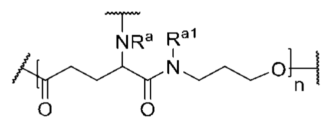

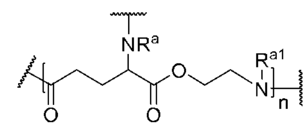 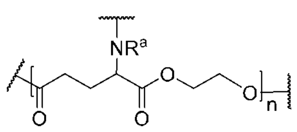 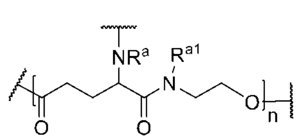

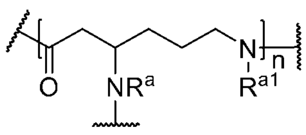
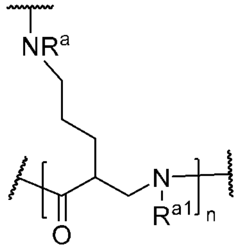
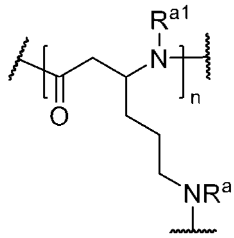

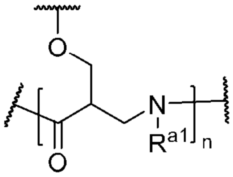
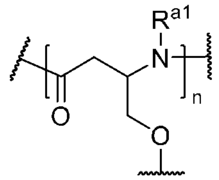
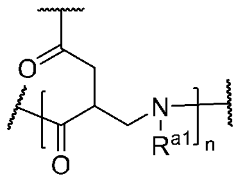
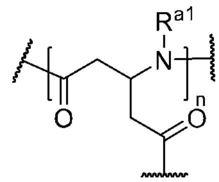

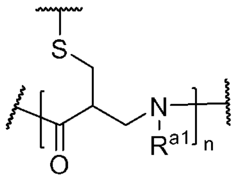
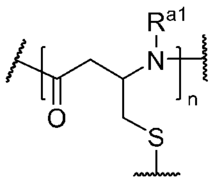
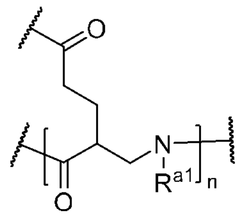
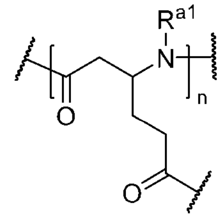

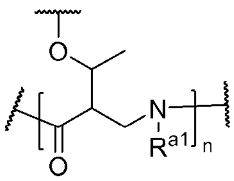
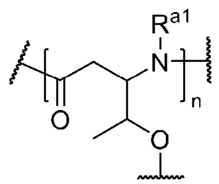

wherein, $R^{a1}$ is selected from H, $C_1$-$C_{10}$ alkyl, $C_1$-$C_{10}$ alkoxy, $C_3$-$C_{10}$ alkenyl, $C_3$-$C_{10}$ alkynyl, $C_3$-$C_8$ cycloalkyl, $C_2$-$C_8$ heterocycloalkyl, $C_6$-$C_{10}$ aryl, $C_5$-$C_{10}$ heteroaryl, an amino protecting group, or a residue of formula (III):

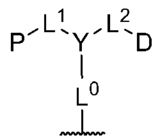

III ;

$R^{a2}$ is selected from H, $C_1$-$C_{10}$ alkyl, $C_1$-$C_{10}$ alkoxy, $C_3$-$C_{10}$ alkenyl, $C_3$-$C_{10}$ alkynyl, $C_3$-$C_8$ cycloalkyl, $C_2$-$C_8$ heterocycloalkyl, $C_6$-$C_{10}$ aryl, $C_5$-$C_{10}$ heteroaryl, or a hydroxy protecting group;
n is selected from integers of 4-100;
m is selected from integers of 0-5;
$R^a$, $R^e$, and $R^f$ are each as defined in claim 5.

**11.** The polymer-drug conjugate according to any one of claims 1, 8, and 9, wherein when an electrophilic group in Y is linked to $L^2$, $L^{2a}$ is absent, i.e., $L^2 = L^{2b}$, and the linkage of the trifunctional branched center Y to the linkers $L^0$, $L^1$, and $L^{2b}$ is selected from any of the following structures:

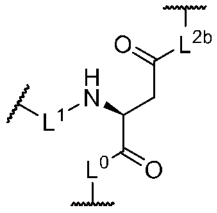
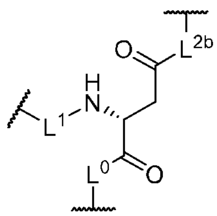
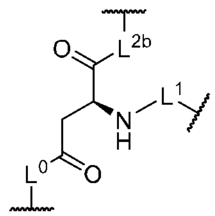
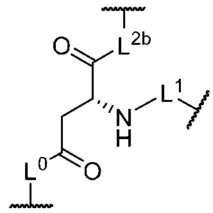

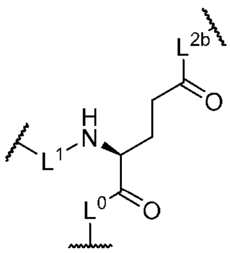
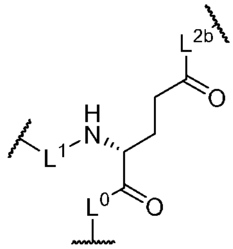
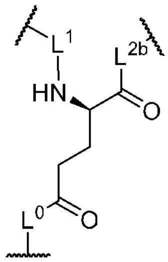
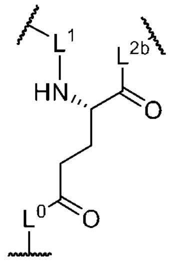
,

preferably and

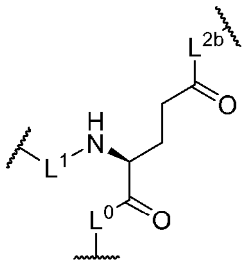
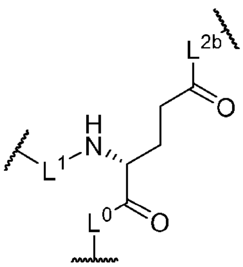
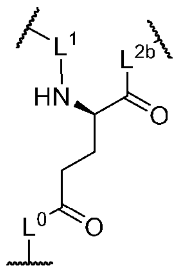
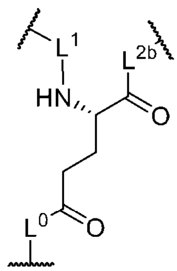
,

more preferably

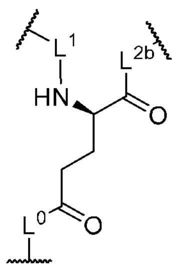
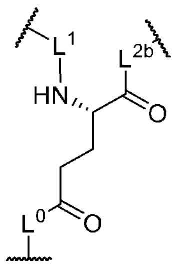
;

or, when a nucleophilic group in Y is linked to $L^2$, $L^{2a}$ is present alone or $L^{2a}$ and $L^{2b}$ are present together, i.e., $L^2 = L^{2a}$ or $L^2 = L^{2a}-L^{2b}$, and the linkage of the trifunctional branched center Y to the linkers $L^0$, $L^1$, $L^{2a}$, and $L^{2b}$ is selected from any of the following structures:

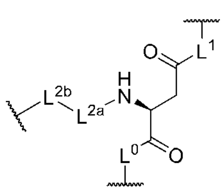
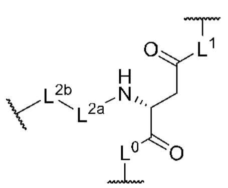
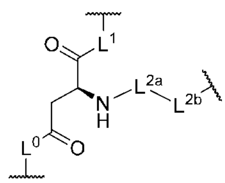
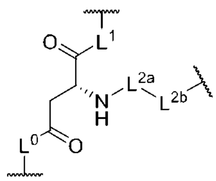

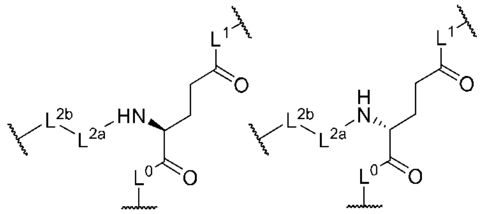
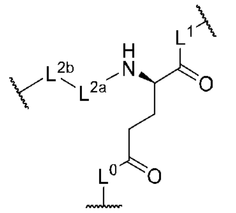
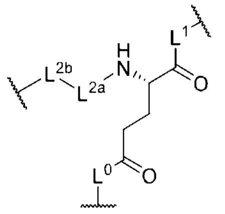

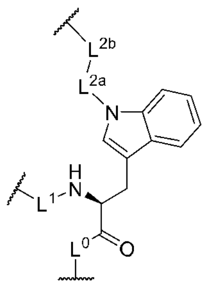
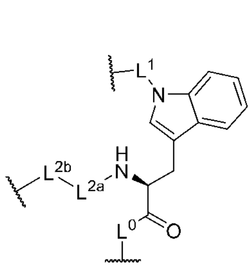
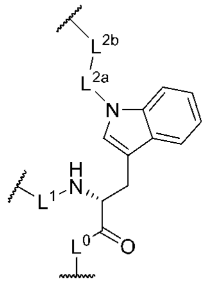
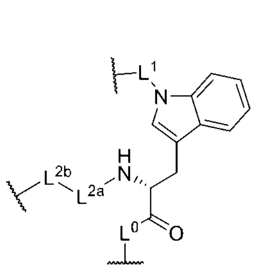

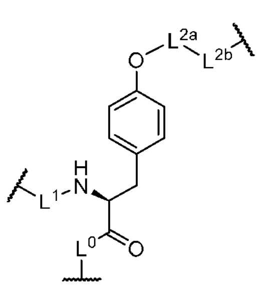
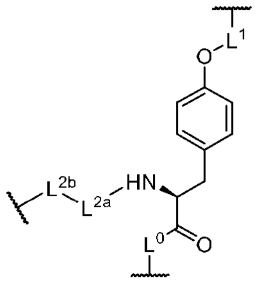
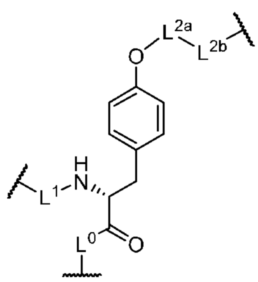
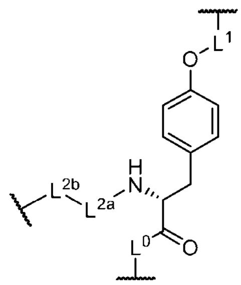

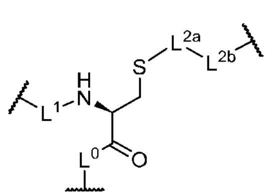
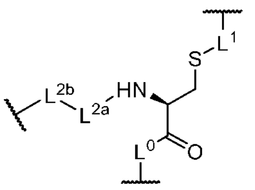
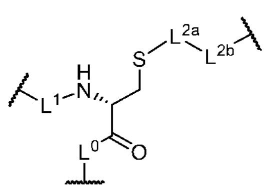
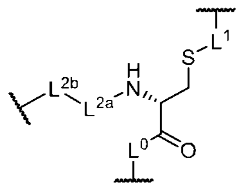

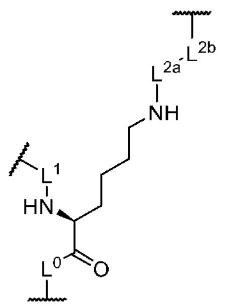
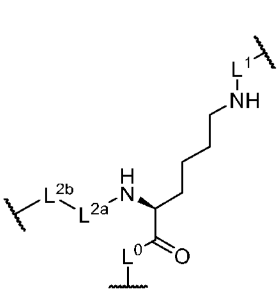
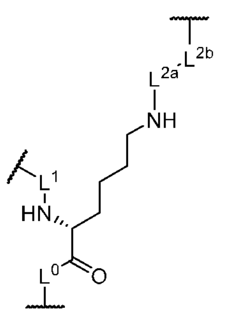
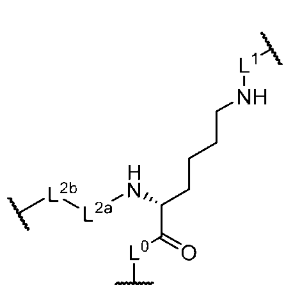

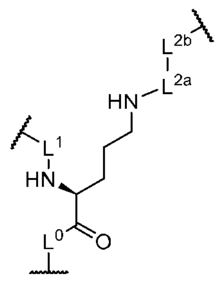
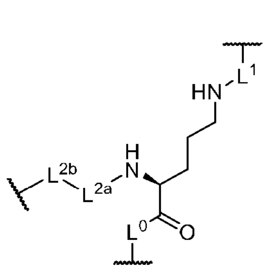
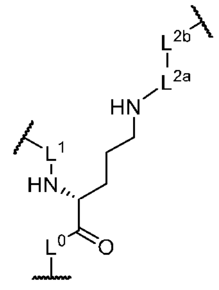
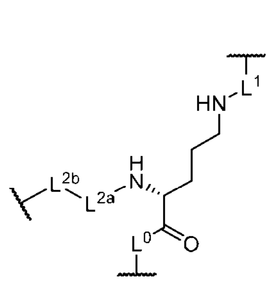

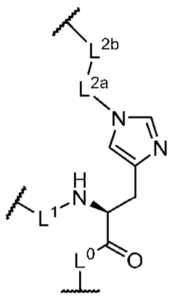 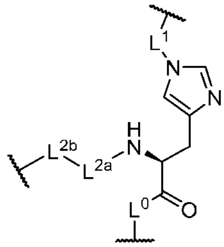 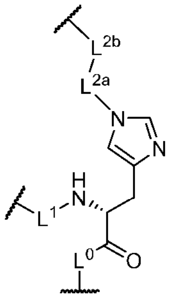 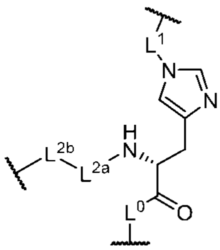

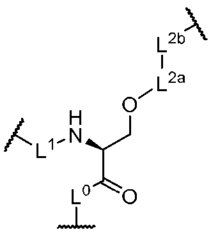 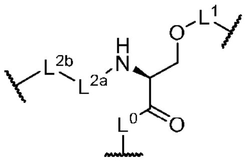 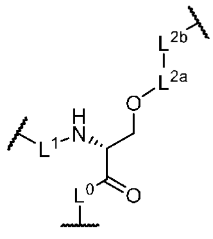 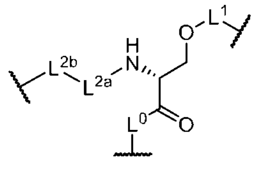

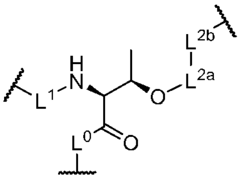 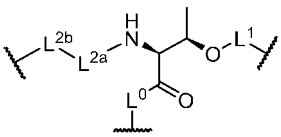 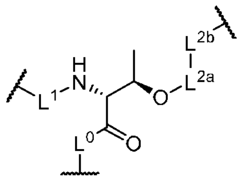 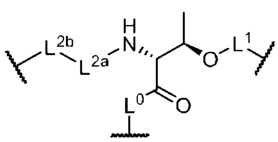

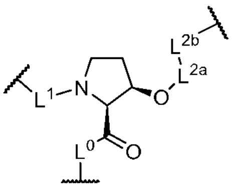 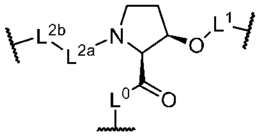 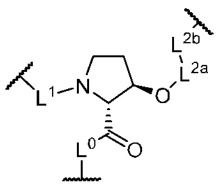 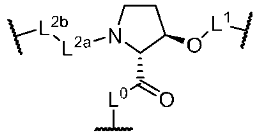

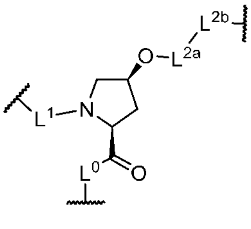 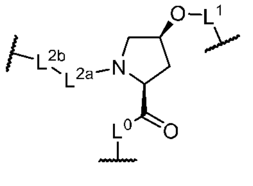 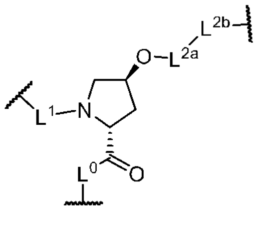 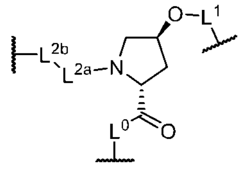

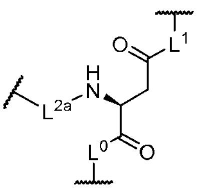 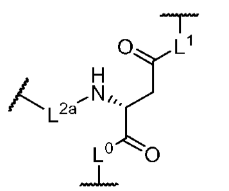 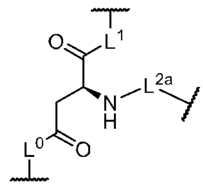 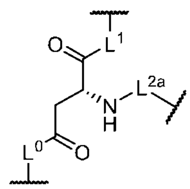

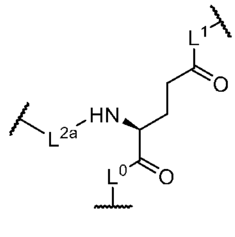 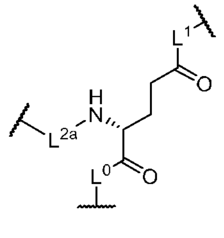 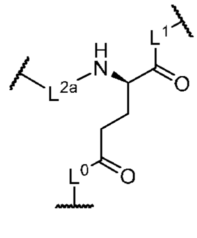 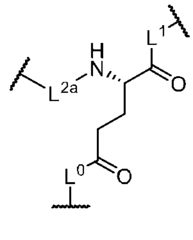

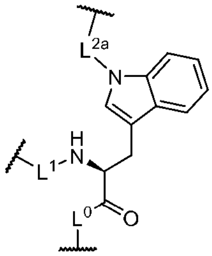 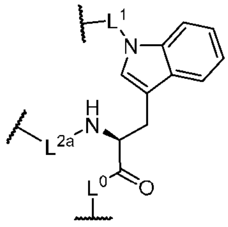 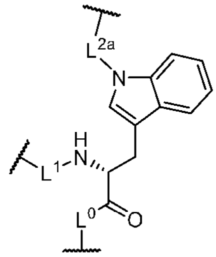 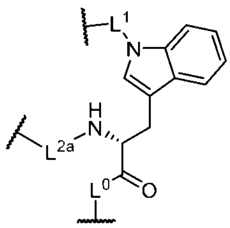

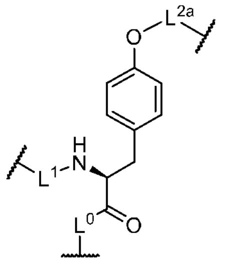 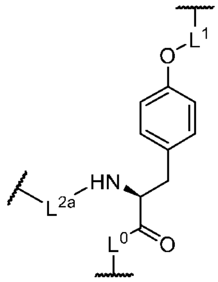 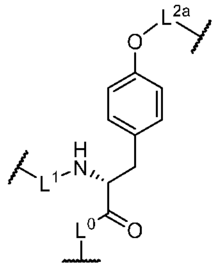 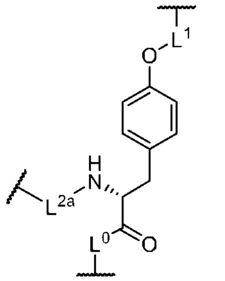

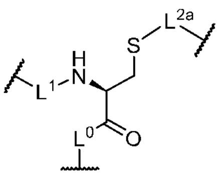 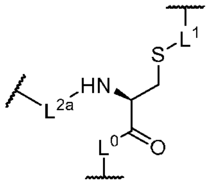 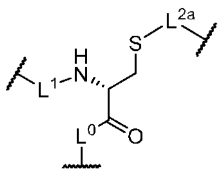 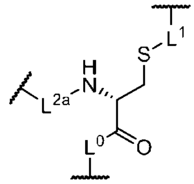

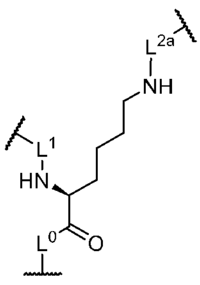 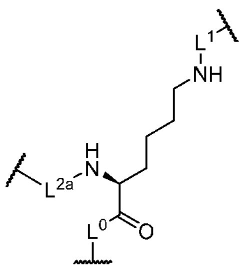 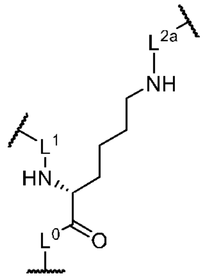 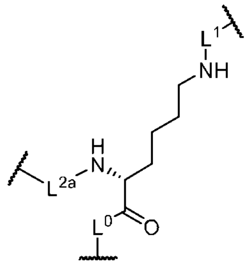

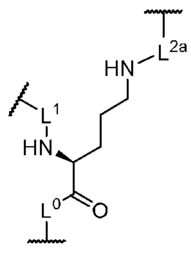 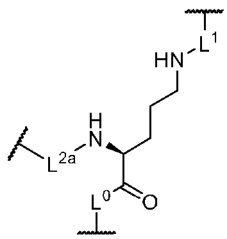 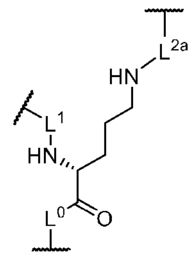 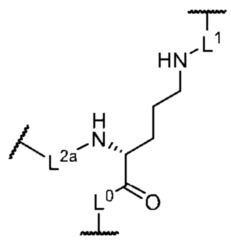

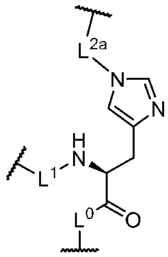 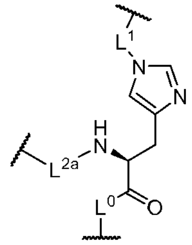 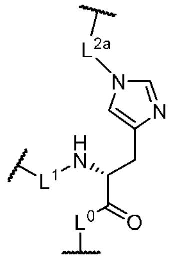 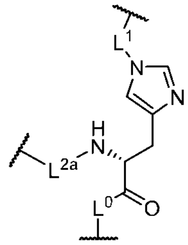

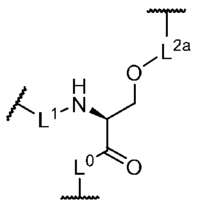 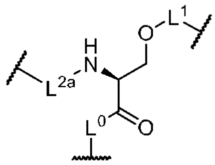 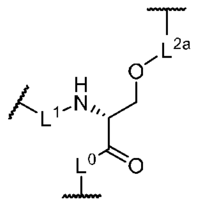 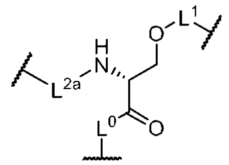

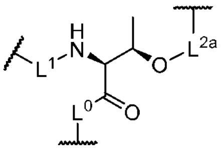 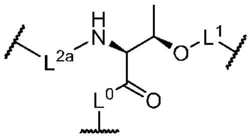 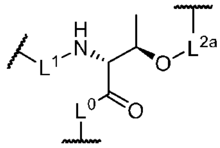 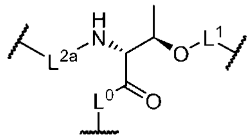

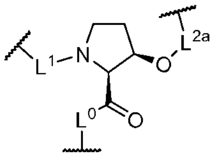 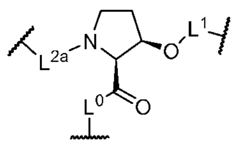 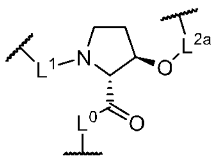 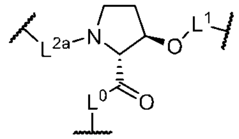

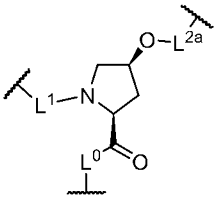 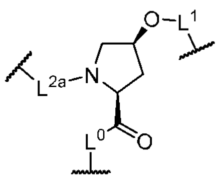 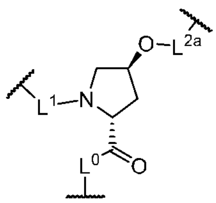 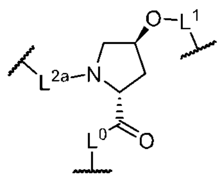 ;

preferably

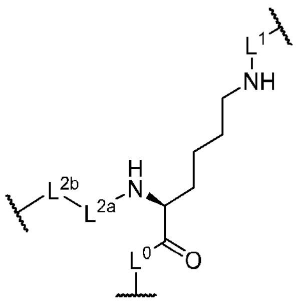 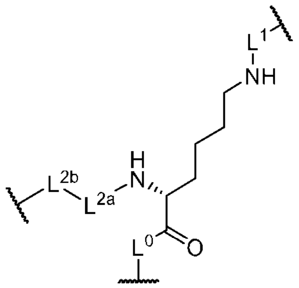 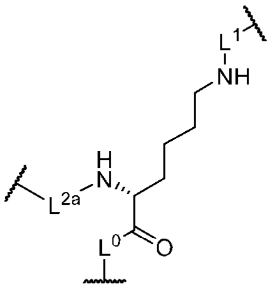

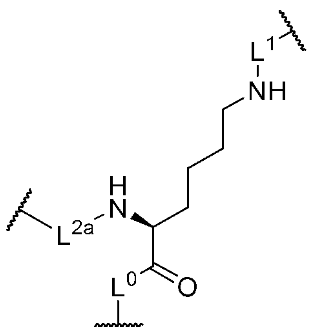 ;

wherein, $L^0$, $L^1$, $L^{2a}$, and $L^{2b}$ are each as defined in any one of claims 1, 8, and 9.

**12.** The polymer-drug conjugate according to any one of claims 1, 8, and 9, wherein the terminal group X is selected from OR, SR, $NR^1R^2$, a carboxyl protecting group, or $L^{2b}$-D, and the R, $R^1$, and $R^2$ are independently selected from H,

$C_1$-$C_{30}$ alkyl, $C_1$-$C_{30}$alkoxy, $C_3$-$C_{30}$ alkenyl, $C_3$-$C_{30}$ alkynyl, $C_3$-$C_8$ cycloalkyl, $C_2$-$C_8$ heterocycloalkyl, $C_6$-$C_{20}$ aryl, or $C_5$-$C_{20}$ heteroaryl; $R^1$ and $R^2$, together with the N atom to which they are linked, may form a $C_2$-$C_8$ heterocycloalkyl; the heteroatom in the $C_2$-$C_8$ heterocycloalkyl is O, S, or N, the number of the heteroatom(s) is one or more, and when more than one heteroatom is present, the heteroatoms are identical or different; the heteroatom in the $C_5$-$C_{20}$ heteroaryl is O, S, or N, the number of the heteroatom(s) is one or more, and when more than one heteroatom is present, the heteroatoms are identical or different; the terminal group X is preferably OR, SR, $NR^1R^2$, a carboxyl protecting group, or $L^{2b}$-D, and R, $R^1$, and $R^2$ are independently selected from H or $C_1$-$C_{10}$ alkyl.

**13.** The polymer-drug conjugate according to any one of claims 1, 8, 9, and 11, wherein the environmentally responsive linker is one or more of an enzyme-responsive linker, a pH-responsive linker, a photo-responsive linker, or a redox-responsive linker.

**14.** The polymer-drug conjugate according to any one of claims 1, 8, 9, 11, and 13, wherein the polymer-drug conjugate meets one or more of the following conditions:

(1) the enzyme-responsive linker is cleavable via one or more of the following enzymes: secretory phospholipase A2, acid phosphatase, serum alkaline phosphatase, cytochrome P450, sulfatase, prostate specific antigen, phospholipase A1, phospholipase A2, phospholipase B, phospholipase C, phospholipase D, neutrophil elastase, cysteine protease-3, cathepsin, matrix metalloproteinase, β-glucuronidase, β-galactosidase, DTP, nitroreductase, reduced coenzyme II, aminopeptidase N, carboxylesterase, diaphorase, histone deacetylase, asparaginyl endopeptidase, urokinase-type plasminogen activator, urokinase-type plasminogen activator receptor, and collagenase, preferably, cleavable via one or more of the following enzymes: cysteine protease-3, cathepsin, matrix metalloproteinase, elastase, or β-glucuronidase;
(2) the pH-responsive linker comprises the following structures: one or more of a hydrazone, imine, oxime, carboxylate, thioester, sulfate, sulfonate, orthoester, carbonate, carbamate, substituted carbamate, ketal, acetal, silyl ether, phosphate, borate, phosphoramide, or *cis*-aconitic acid group;
(3) the photo-responsive linker comprises the following structures: one or more of an *o*-nitrophenyl, coumarin, benzoin, BODIPY, or cyanine group;
(4) the redox-responsive linker comprises the following structures: one or more of thioketal, phenylboronate, phenylboronic acid, oxalate, vinyl ether, thioether group, aminoacrylate, disulfide group, diselenide group, 2,4-dinitrobenzene sulfonate, 2-azidomethylbenzoate, 4-azidobenzyl, unsaturated ester, or azobenzene group.

**15.** The polymer-drug conjugate according to claim 13 or 14, wherein the enzyme-responsive linker comprises the following amino acid sequences: one or more of Cit-Phe, Lys-Lys, Phe-Lys, Arg-Arg, Val-Cit, Val-Ala, Val-Lys, Ala-Lys, Phe-Cit, Leu-Cit, Ile-Cit, Trp-Cit, Phe-Arg, Asn-Pro-Val, Gly-Pro-Nle, Glu-Val-Cit, Glu-Val-Ala, Gly-Phe-Gly, Gly-Phe-Phe, Gly-Leu-Gly, Gly-Val-Ala, Gly-Phe-Leu-Gly, Gly-Phe-Phe-Leu, Gly-Leu-Leu-Gly, Gly-Phe-Tyr-Ala, Gly-Phe-Gly-Phe, Ala-Gly-Val-Phe, Gly-Phe-Phe-Gly, Gly-Gly-Phe-Gly, Asp-Glu-Val-Asp, Gly-Phe-Leu-Gly-Phe, Gly-Phe-Ala-Gly-Leu-Phe, Gly-Leu-Ala-Ala-Val-Ala, Gly-Gly-Phe-Leu-Gly-Phe, or Gln-Ser-Phe-Arg-Phe-Lys.

**16.** The polymer-drug conjugate according to any one of claims 13-15, wherein the polymer-drug conjugate meets one or more of the following conditions:

(1) the enzyme-responsive linker comprises the following structures:

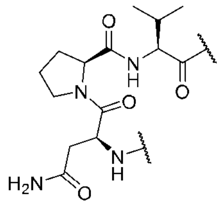
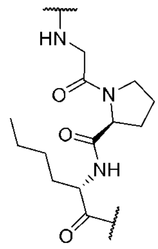
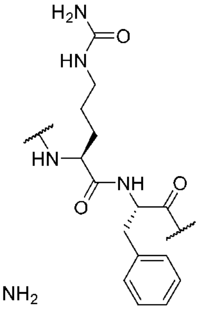
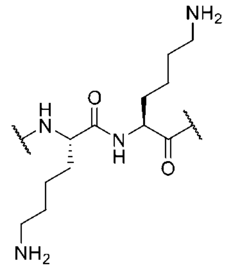

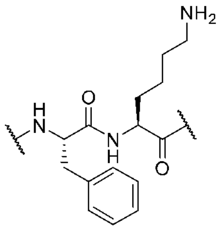
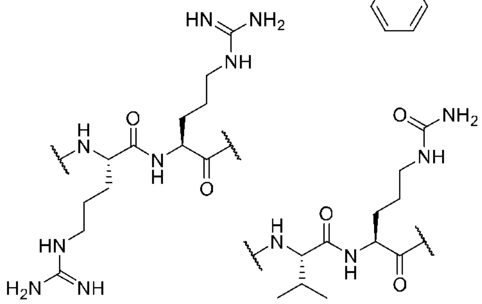
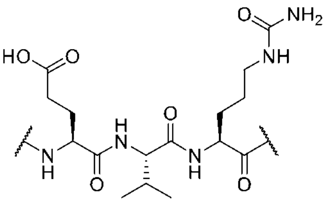

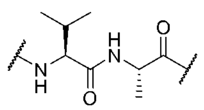
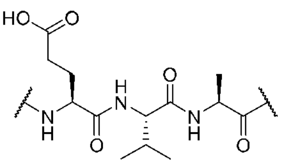
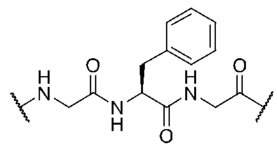
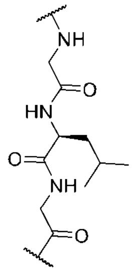

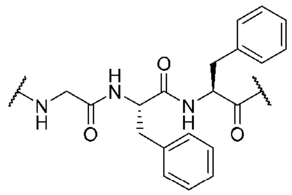
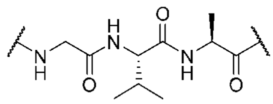
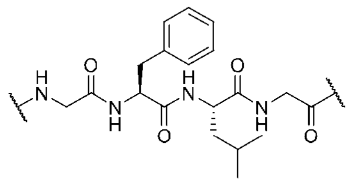

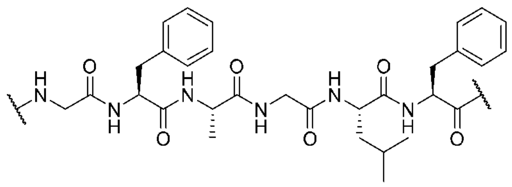
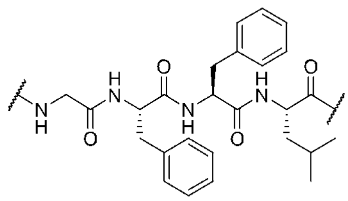

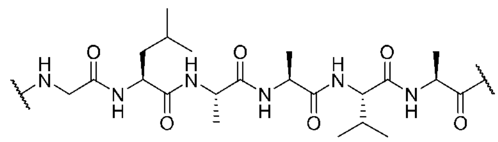
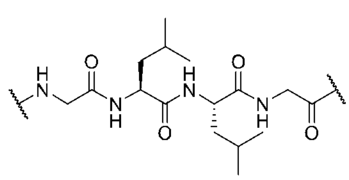

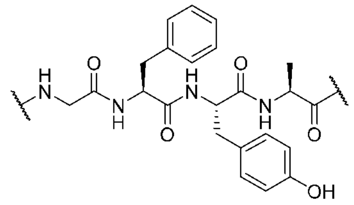

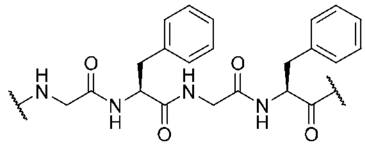

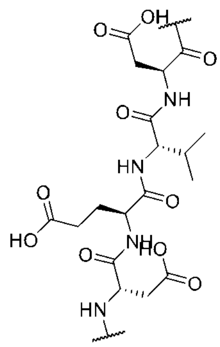

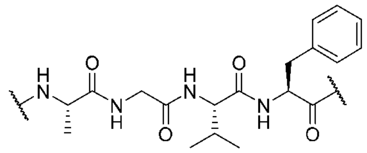

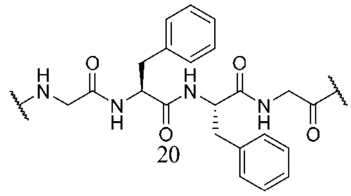

20

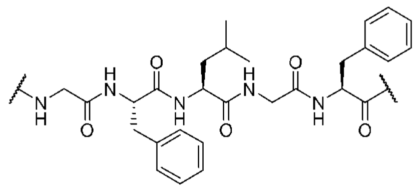

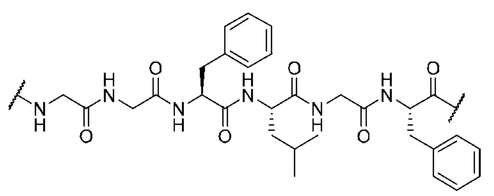

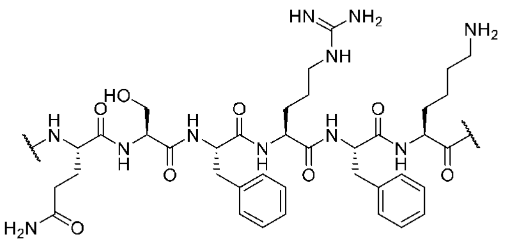

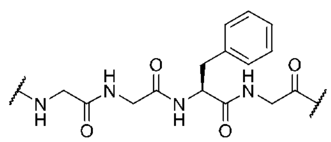

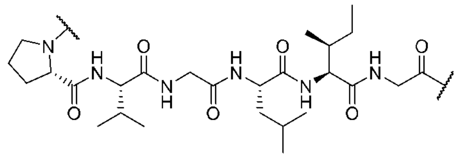

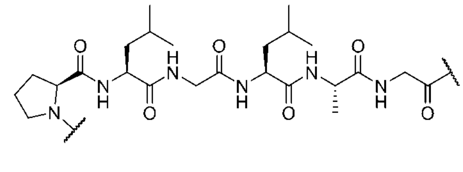

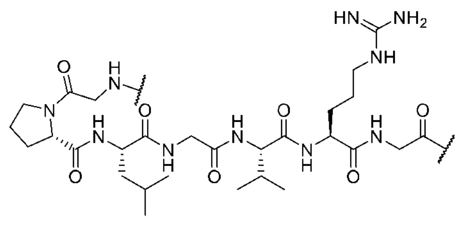

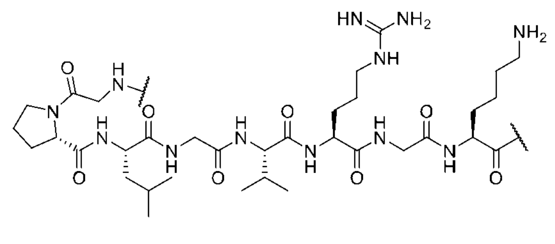

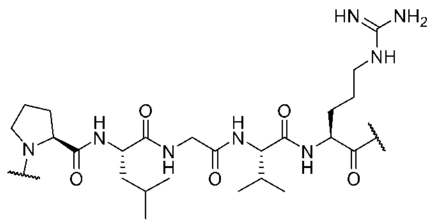

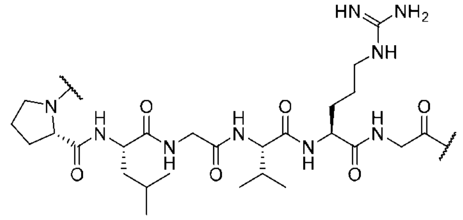

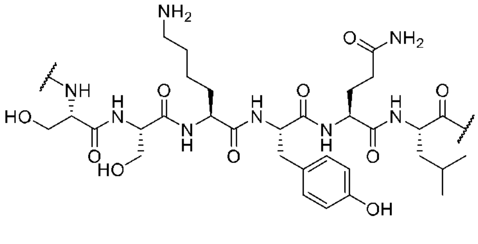

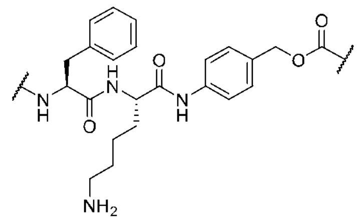

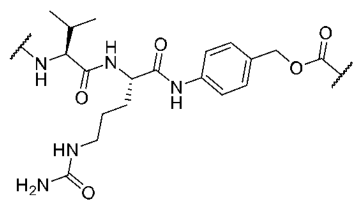

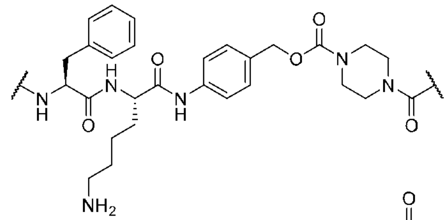

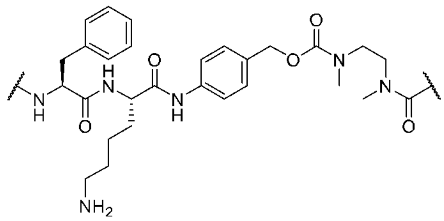

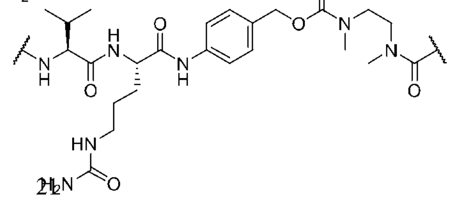

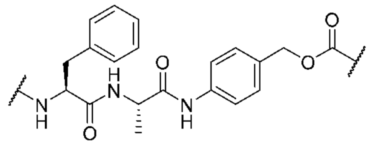

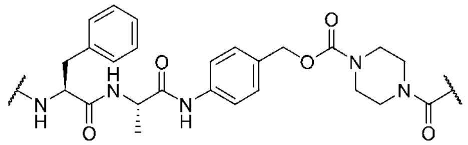

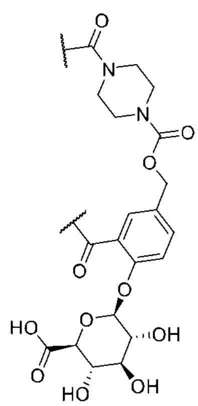

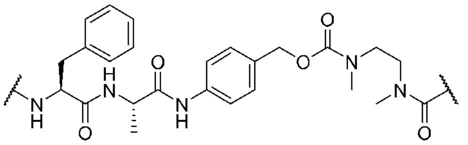

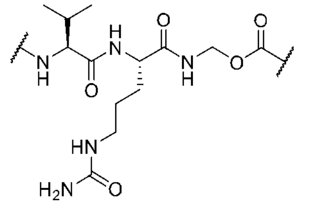

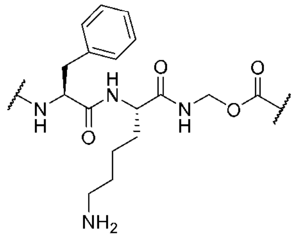

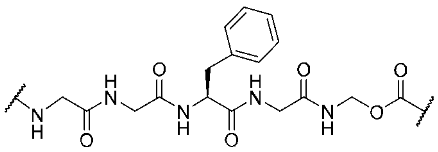

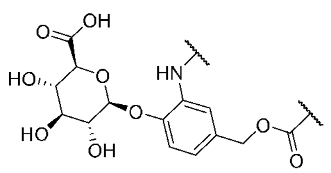

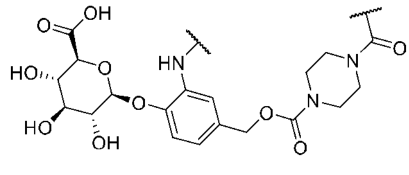

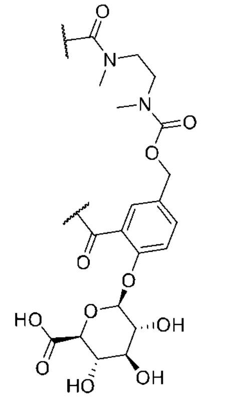

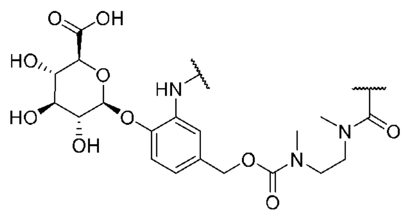

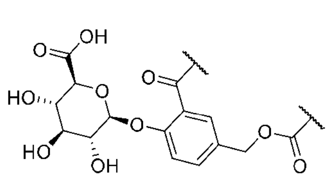

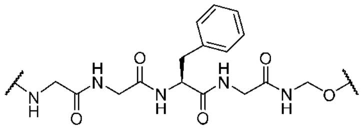 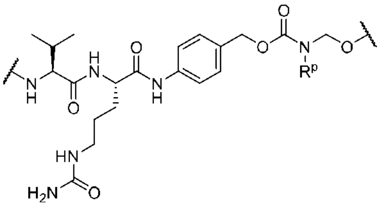 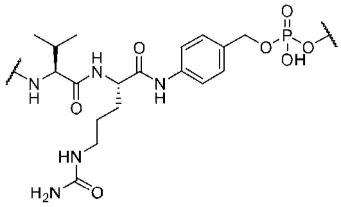

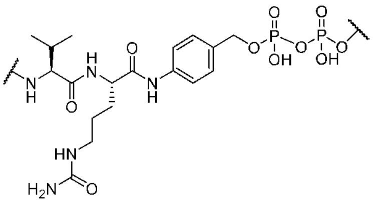 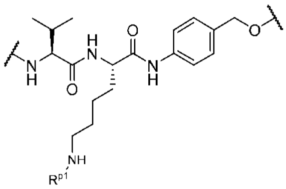 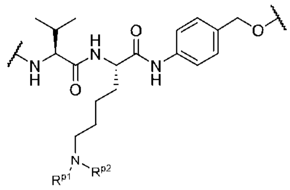

;

wherein, $R^p$ is selected from H and $C_1$-$C_{10}$ alkyl; $R^{p1}$ and $R^{p2}$ are each independently selected from $C_1$-$C_{10}$ alkyl; (2) the pH-responsive linker comprises the following structures:

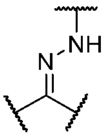 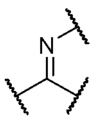 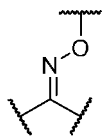 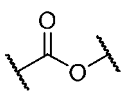 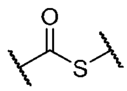 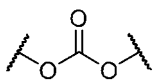 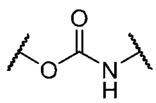

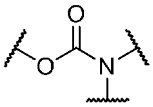 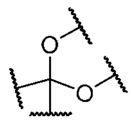 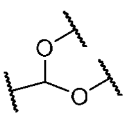 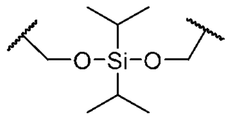 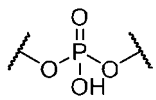 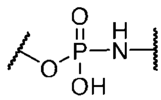

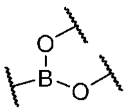 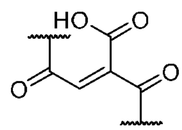 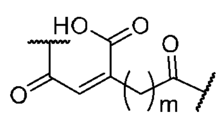 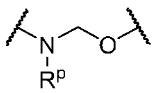 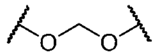

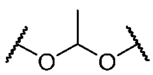 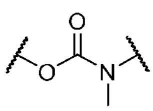 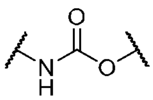 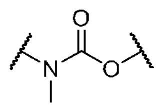

,

wherein m = 0-4; $R^p$ is selected from H and substituted $C_1$-$C_{10}$ alkyl;
(3) the photo-responsive linker comprises the following structures:

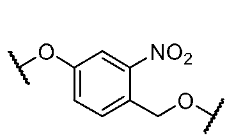 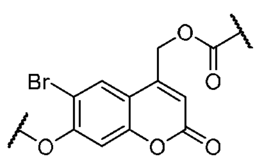 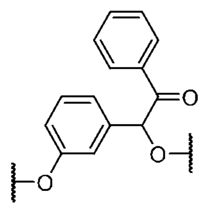

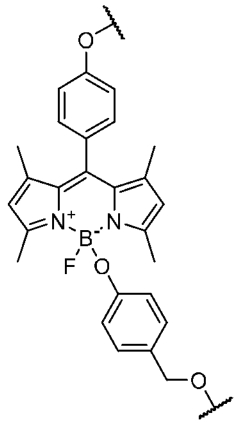
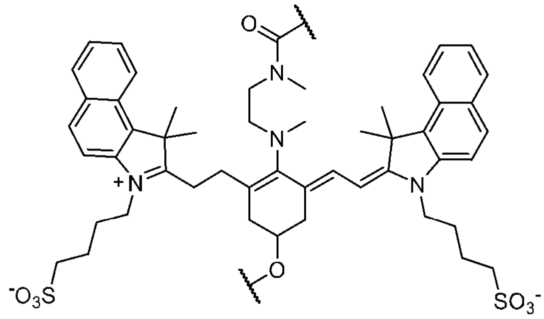

;

(4) the redox-responsive linker comprises the following structures:

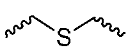
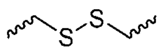
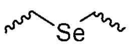
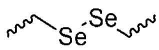
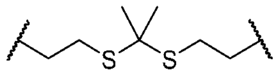

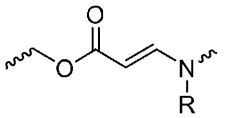
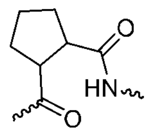
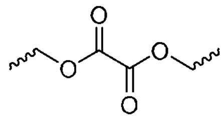
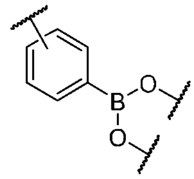

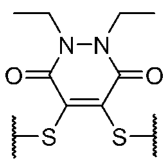
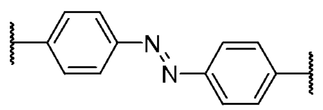

**17.** The polymer-drug conjugate according to any one of claims 8, 9, and 11, wherein the linker $L^{2a}$ is a covalent bond or a linker of formula (VII):

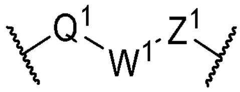

VII

$Q^1$ is selected from one of

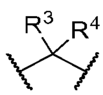
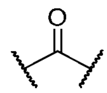
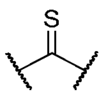
,

wherein $R^3$ and $R^4$ are independently selected from H, D, $C_1$-$C_6$ alkyl, $C_1$-$C_6$ alkoxy, $C_3$-$C_6$ alkenyl, $C_3$-$C_6$ alkynyl, $C_3$-$C_8$ cycloalkyl, $C_2$-$C_8$ heterocycloalkyl, $C_6$-$C_{10}$ aryl, or $C_3$-$C_{10}$ heteroaryl; or $R^3$ and $R^4$, together with the C atom to which they are linked, may form a $C_3$-$C_8$ alkyl or heterocycloalkyl; the heteroatom in the $C_2$-$C_8$ heterocycloalkyl is O, S, or N, the number of the heteroatom(s) is one or more, and when more than one heteroatom is present, the heteroatoms are identical or different; the heteroatom in the $C_3$-$C_{10}$ heteroaryl is O, S, or N, the number of the heteroatom(s) is one or more, and when more than one heteroatom is present, the heteroatoms are identical or different; $W^1$ is a covalent bond or a $C_0$-$C_{20}$ fragment either containing a heteroatom or not, wherein the heteroatom is O, S, Se, N, P, Si, or B, the

number of the heteroatom(s) is one or more, and when more than one heteroatom is present, the heteroatoms are identical or different; the W fragment may or may not contain an unsaturated bond;
$Z^1$ is selected from one of

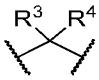 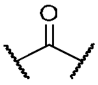 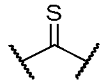 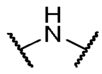 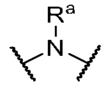 ,

wherein $R^a$ is as defined in claim 5.

**18.** The polymer-drug conjugate according to claim 17, wherein the linker $L^{2a}$ is selected from the following structures and a covalent bond:

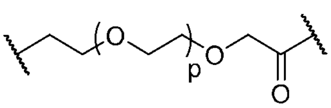 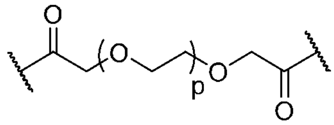 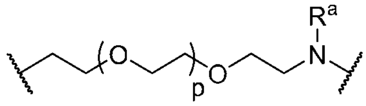

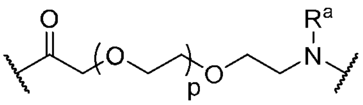 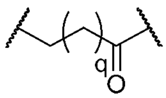 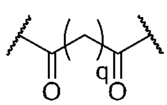

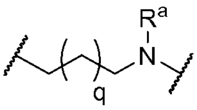 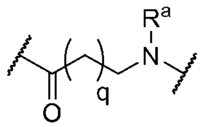 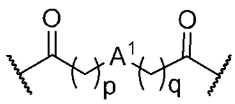

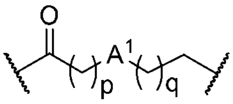 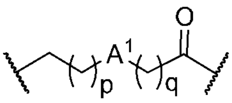 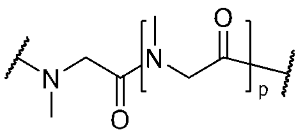

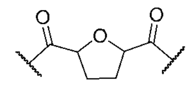 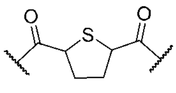 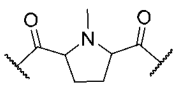 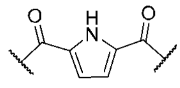 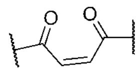

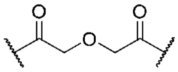 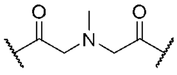 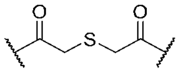 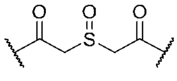 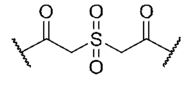

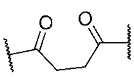 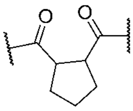 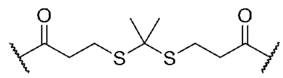 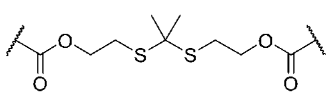 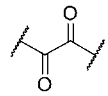

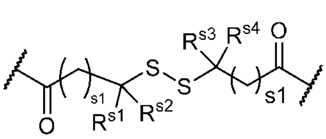 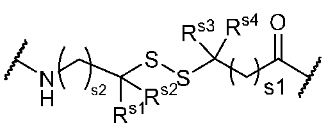 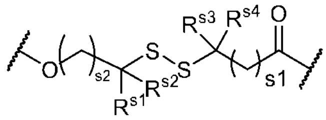

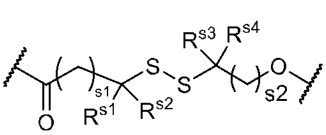 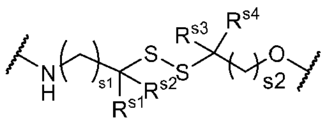 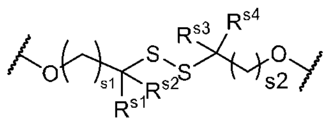

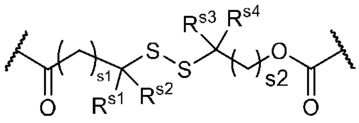 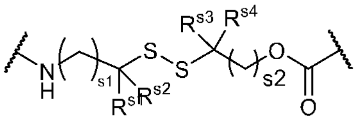 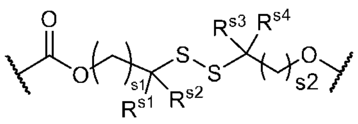

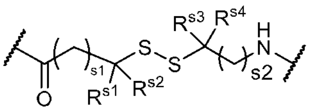 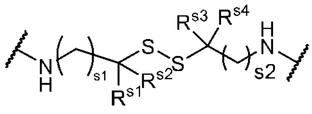 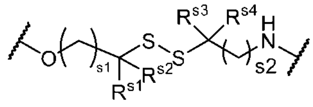

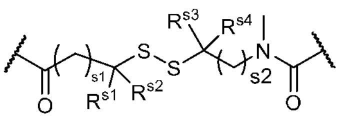 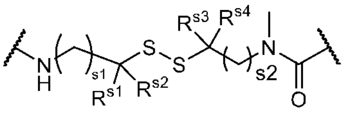 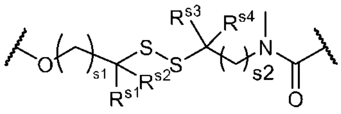

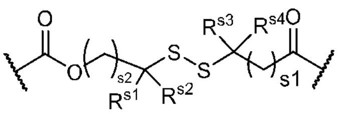 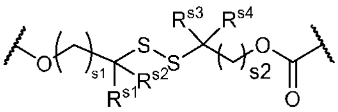 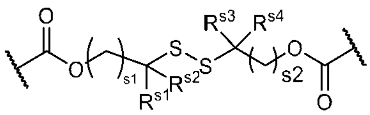

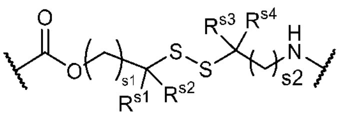 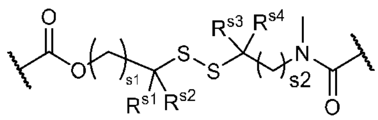

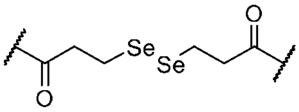 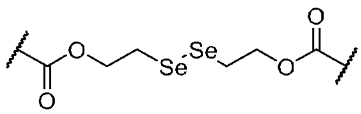 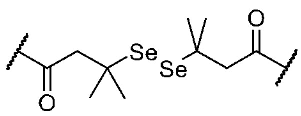

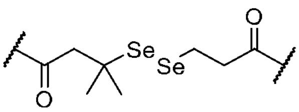 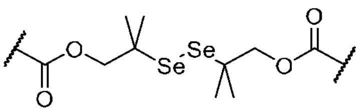 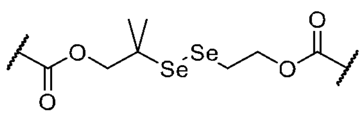

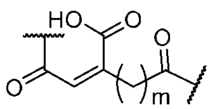

wherein,

$A^1$ is O, S, S(O), $S(O)_2$, $NR^a$, or $C(R^3R^4)$;
p is selected from integers of 0-16;
q is selected from integers of 0-16;
m is selected from integers of 0-4;
s1 is selected from integers of 0-16;
s2 is selected from integers of 1-15;
$R^{s1}$, $R^{s2}$, $R^{s3}$, and $R^{s4}$ are each independently selected from hydrogen and methyl;
$R^a$ is as defined in claim 5.

**19.** The polymer-drug conjugate according to any one of claims 1, 8, and 9, wherein the pharmacokinetic regulator residue P is selected from a polyethylene glycol derivative residue with a repeating unit number $a^1$ and a terminal group $R^b$, a hyaluronic acid derivative residue with a repeating unit number $b^1$, a polyphosphate residue with a repeating unit number $c^1$, a polysarcosine residue with a repeating unit number $d^1$ and a terminal group $R^{d1}$, and a polyoxazoline residue with a repeating unit number $f^1$;

wherein, $a^1$ is selected from integers of 5-250, $b^1$ is selected from integers of 5-250, $c^1$ is selected from integers of 5-250, $d^1$ is selected from integers of 5-250, and $f^1$ is selected from integers of 5-250; $R^b$ is H, $C_1$-$C_{10}$ alkyl, $C_1$-$C_{10}$ heteroalkyl, $C_3$-$C_{10}$ cycloalkyl, $C_3$-$C_{10}$ alkenyl, $C_3$-$C_{10}$ alkynyl, or a hydroxy protecting group; $R^{d1}$ is H, $C_1$-$C_{10}$ alkyl, $C_1$-$C_{10}$ alkoxy, $C_1$-$C_{10}$ heteroalkyl, $C_3$-$C_{10}$ cycloalkyl, $C_3$-$C_{10}$ alkenyl, $C_3$-$C_{10}$ alkynyl, a hydroxy protecting group, or an amino protecting group;
preferably, the pharmacokinetic regulator residue P meets one or more of the following conditions:

(1) when the pharmacokinetic regulator residue P is a polyethylene glycol derivative residue with a repeating unit number $a^1$ and a terminal group $R^b$, $a^1$ is selected from integers of 5-150, preferably integers of 10-60, and more preferably integers of 15-50, e.g., 21, 43, or 44;
(2) when the pharmacokinetic regulator residue P is a polyethylene glycol derivative residue with a repeating unit number $a^1$ and a terminal group $R^b$, $R^b$ is $C_1$-$C_{10}$ alkyl, preferably $C_1$-$C_6$ alkyl, and more preferably $C_1$-$C_3$ alkyl, e.g., methyl, ethyl, n-propyl, or isopropyl;
(3) when the pharmacokinetic regulator residue P is a polysarcosine residue with a repeating unit number $d^1$ and a terminal group $R^{d1}$, $R^{d1}$ is preferably hydrogen, $C_1$-$C_6$ alkyl, $C_1$-$C_6$ alkoxy, an amino substituted with $C_1$-$C_6$ alkyl, or $C_1$-$C_6$ acyl, e.g., methylamino, carboxylic acid, methyl carboxylate, or acetylamino.

**20.** The polymer-drug conjugate according to claim 19, wherein the pharmacokinetic regulator residue P is a polyethylene glycol derivative residue with a repeating unit number $a^1$ and a terminal group $R^b$, a hyaluronic acid derivative residue with a repeating unit number $b^1$, or a polysarcosine derivative residue with a repeating unit number $d^1$; the polyethylene glycol derivative residue is selected from the following structures:

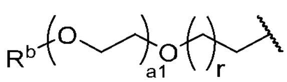
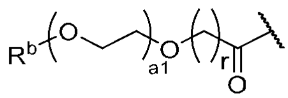

wherein $a^1$ is selected from integers of 5-150, $b^1$ is selected from integers of 5-150, and r is selected from integers of 0-8; the polyethylene glycol derivative residue is preferably

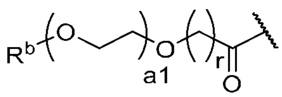,

$a^1$ is selected from integers of 20-45, $b^1$ is selected from integers of 5-10, and r is selected from integers of 0-3; $R^b$ is as defined in claim 19;
the polysarcosine derivative residue is selected from the following structures:

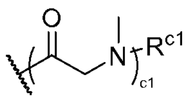 or 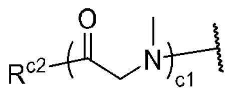

wherein $c^1$ is selected from integers of 5-150, and $R^{c1}$ is selected from H, $C_1$-$C_{10}$ alkyl, $C_1$-$C_{10}$ alkoxy, $C_3$-$C_{10}$ alkenyl, $C_3$-$C_{10}$ alkynyl, $C_3$-$C_8$ cycloalkyl, $C_2$-$C_8$ heterocycloalkyl, $C_6$-$C_{10}$ aryl, $C_5$-$C_{10}$ heteroaryl, and an amino protecting group; $R^{c2}$ is selected from $OR^{c3}$, $SR^{c3}$, and $NR^{c4}R^{c5}$, wherein $R^{c3}$ is selected from H, $C_1$-$C_{10}$ alkyl, $C_3$-$C_{10}$ alkenyl, $C_3$-$C_{10}$ alkynyl, $C_3$-$C_8$ cycloalkyl, $C_2$-$C_8$ heterocycloalkyl, $C_6$-$C_{10}$ aryl, and $C_5$-$C_{10}$ heteroaryl, and $R^{c4}$ and $R^{c5}$ are each independently selected from H, $C_1$-$C_{10}$ alkyl, $C_1$-$C_{10}$ alkoxy, $C_3$-$C_{10}$ alkenyl, $C_3$-$C_{10}$ alkynyl, $C_3$-$C_8$ cycloalkyl, $C_2$-$C_8$ heterocycloalkyl, $C_6$-$C_{10}$ aryl, $C_5$-$C_{10}$ heteroaryl, and an amino protecting group.

**21.** The polymer-drug conjugate according to claim 20, wherein the pharmacokinetic regulator residue P is a methyl-terminated polyethylene glycol derivative residue with a repeating unit number $a^1$; the methyl-terminated polyethylene glycol derivative residue is selected from the following structures:

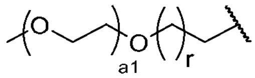
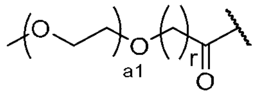

wherein $a^1$ is selected from integers of 5-150, and r is selected from integers of 0-8; the methyl-terminated polyethylene glycol derivative residue is preferably

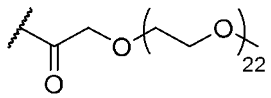

or

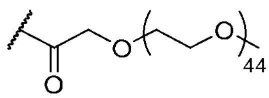

.

**22.** The polymer-drug conjugate according to any one of claims 1, 5, and 8-10, wherein the polymer residue is selected from the following structures:

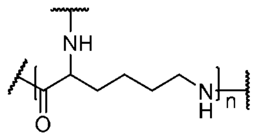
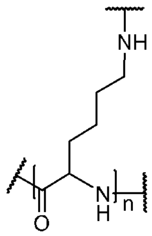
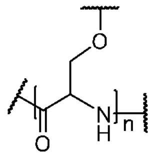
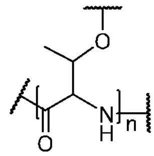

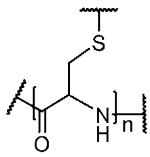
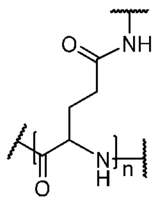
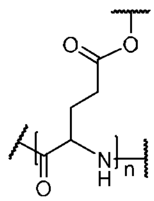
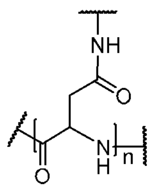
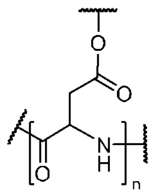

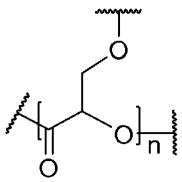
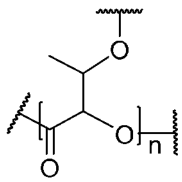
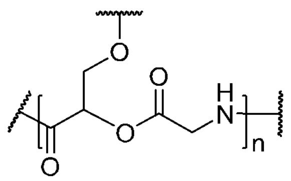

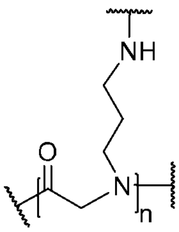
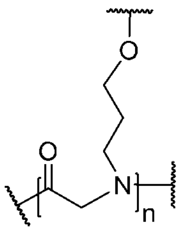
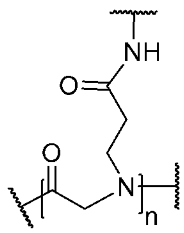
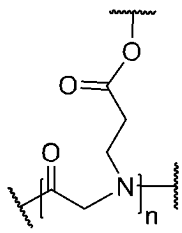

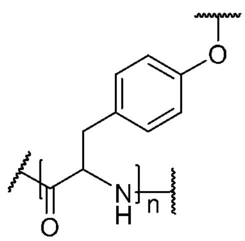
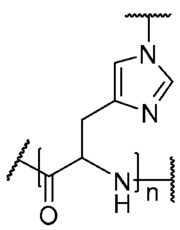
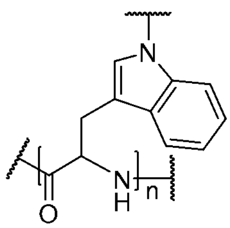

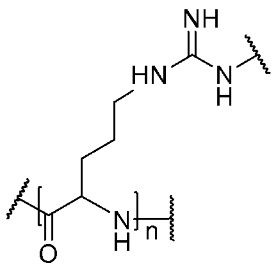
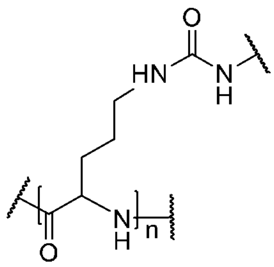
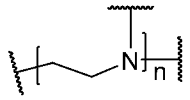

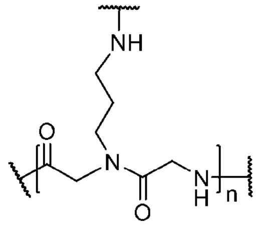
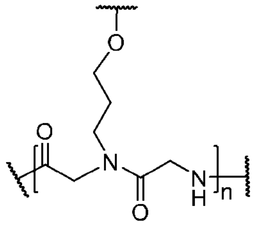
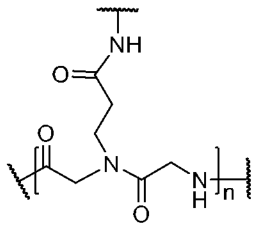
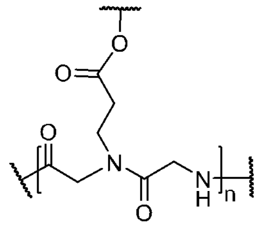

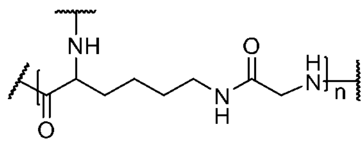
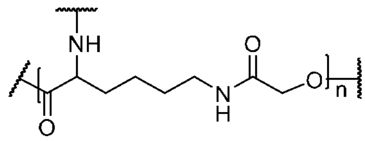

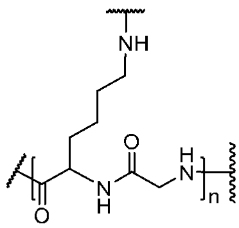
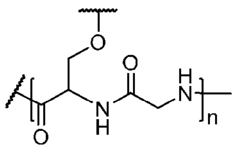
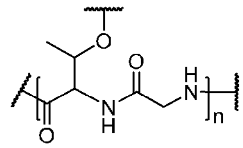
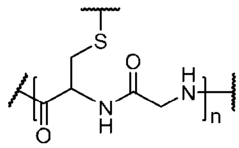

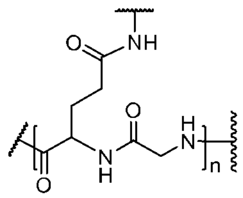
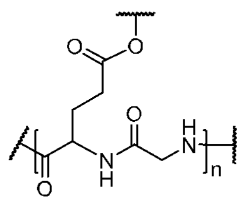
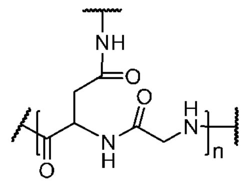
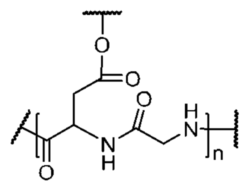

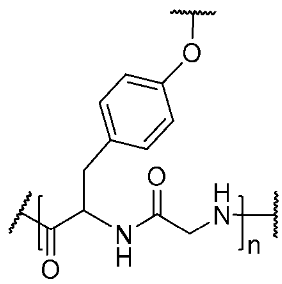
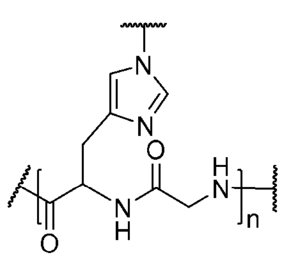
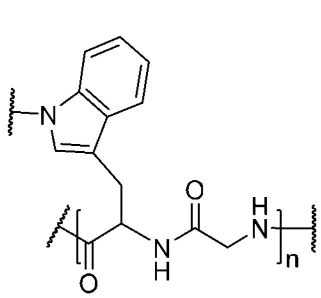

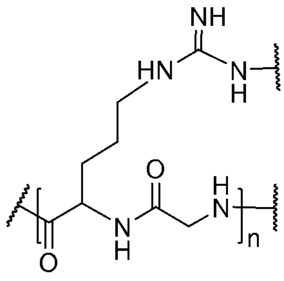
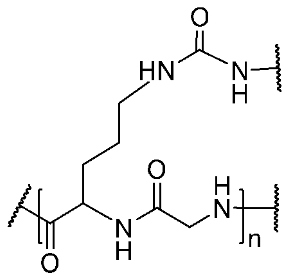

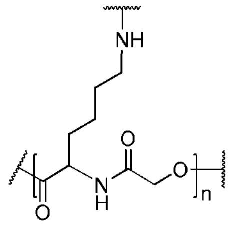
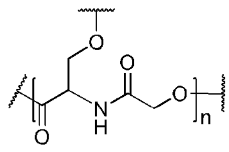
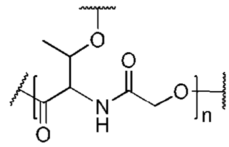
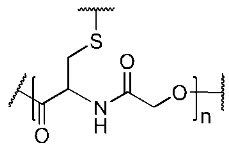

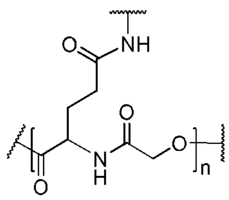
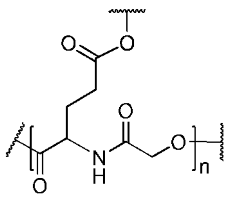
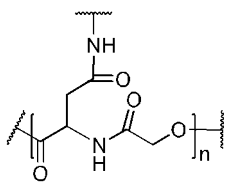
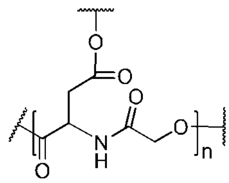

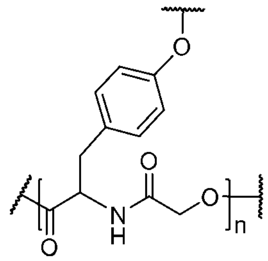
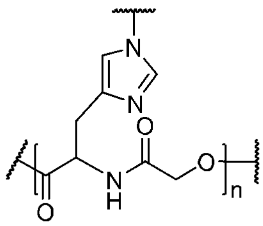
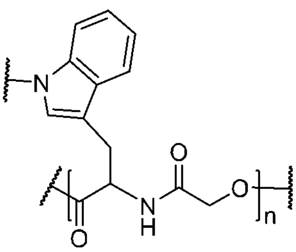

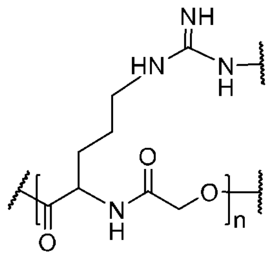
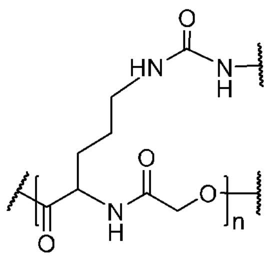

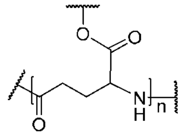
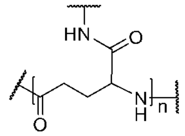
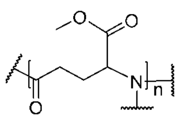
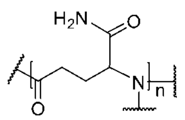
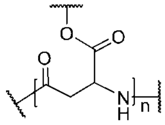

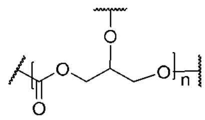 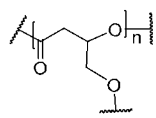 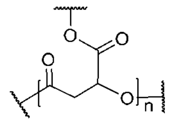 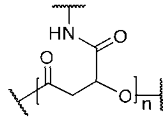 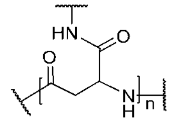

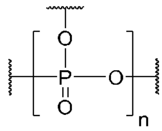 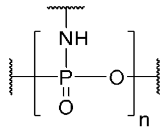 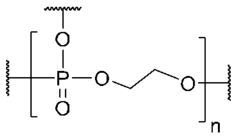 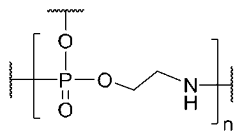

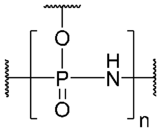 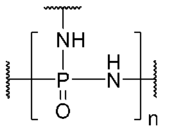 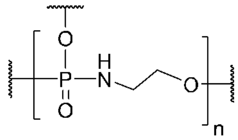 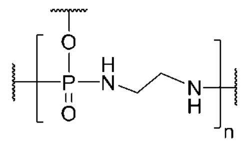

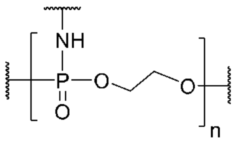 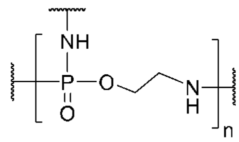 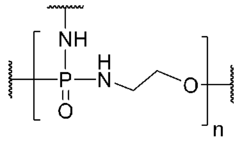 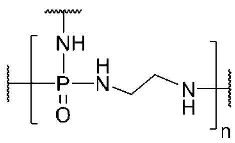

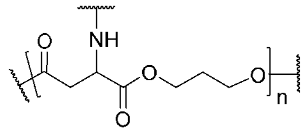 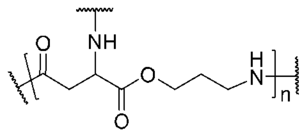 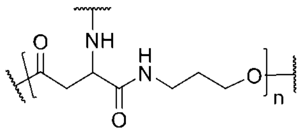

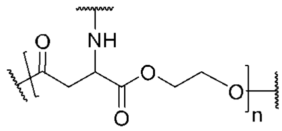 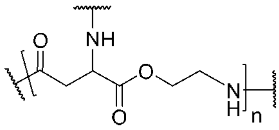 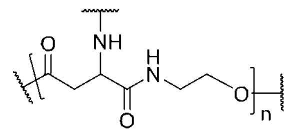

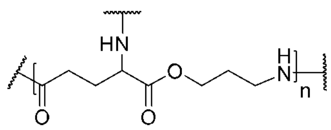 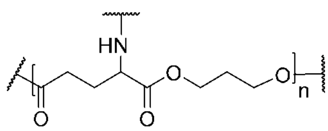 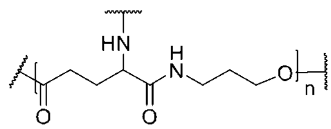

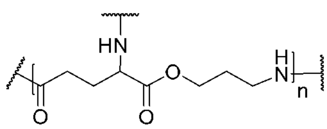 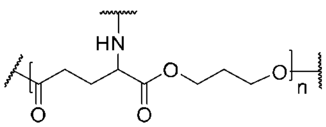 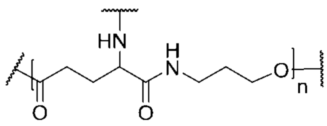

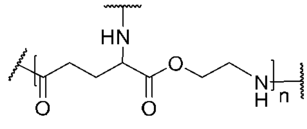 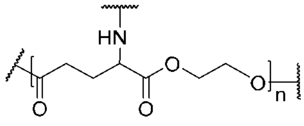 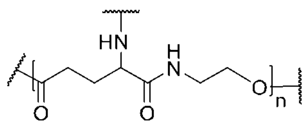

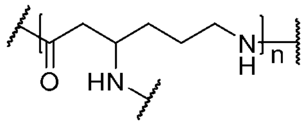
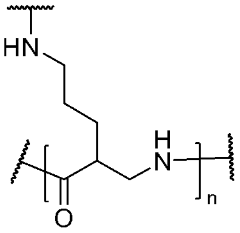
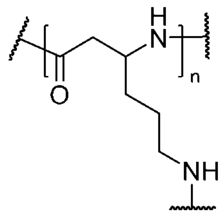

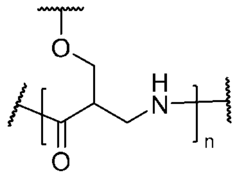
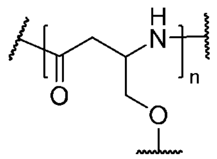
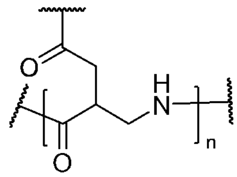
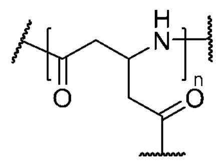

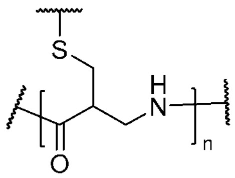
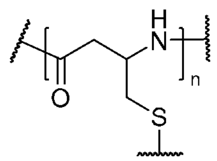
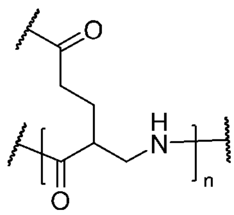
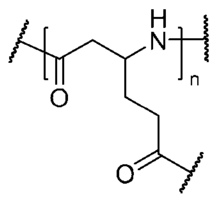

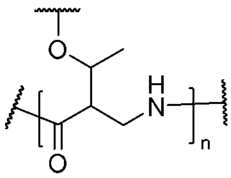
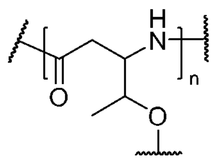

wherein n is selected from integers of 4-100.

**23.** The polymer-drug conjugate according to any one of claims 1, 8, and 9, wherein the pharmaceutically active agent D has an active functional group selected from one or more of primary amine, secondary amine, tertiary amine, hydroxy, sulfhydryl, carboxyl, ester group, amide, boric acid, borate, phosphoric acid, sulfonic acid, sulfoxide, aldehyde group, and ketone group.

**24.** The polymer-drug conjugate according to any one of claims 1, 8, 9, and 23, wherein the pharmaceutically active agent is selected from one or more of: an anesthetic, an antacid, an anti-infective, a cardiovascular agent, a diuretic, a hematinic, an immunosuppressant, a GLP-1 receptor agonist, a hormone and an analog, an ophthalmic drug, an analgesic, a respiratory drug, an antiarthritic, an anticonvulsant, an antihistamine, an anti-inflammatory agent, an antiulcer agent, a behavior modification drug, an antineoplastic, an anti-cancer antigen, a central nervous system agent, an antipsychotic, a contraceptive agent, a diabetes drug, a growth promoter, a hemostat, an immunostimulant, an immunomodulator, a muscle relaxant, an obesity drug, an osteoporosis drug, a sedative, a tranquilizer, a urinary acidifying agent, a vitamin, a polypeptide drug, an oligonucleotide drugs, an mRNA drug, an antibody drug, a biologic, a targeted protein degrader, a PROTAC (proteolysis targeting chimera) drug, a molecular glue degrader, an oligosaccharide drug, and a targeted drug.

**25.** The polymer-drug conjugate according to any one of claims 1, 8, 9, 23, and 24, wherein the pharmaceutically active agent residue D is an antineoplastic residue; the antineoplastic is selected from one or more of an anti-tumor targeted

drug, a targeted protein degrader, a PROTAC drug, a molecular glue degrader, an anti-tumor immunomodulator, and a chemotherapeutic.

**26.** The polymer-drug conjugate according to claim 25, wherein the antineoplastic is selected from one or more of abemaciclib, abiraterone, abrocitinib, acalabrutinib, afatinib, aldesleukin, alectinib, alflutinib, almonertinib, altretamine, amcenestrant, aminoglutethimide, amsacrine, anastrozole, anlotinib, apalutamide, apatinib, arzoxifene, asciminib, asparaginase, avapritinib, avitinib, axitinib, azacitidine, baricitinib, belinostat, bendamustine, bexarotene, bicalutamide, bicyclol, binimetinib, bleomycin, boanmycin, bortezomib, bosutinib, brigatinib, buserelin, busulfan, cabazitaxel, cabozantinib, calaspargase, calicheamycin, capecitabine, capmatinib, carboplatin, carfilzomib, carmustine, carmofur, cedazuidine, ceritinib, cetrorelix, chidamide, chlorambucil, cisplatin, cladribine, clofarabine, cobimetinib, colchicine, copanlisib, crizotinib, cyclophosphamide, cytarabine, dabrafenib, dacarbazine, dacomitinib, dactinomycin, dalpiciclib, darolutamide, dasatinib, daunorubicin, decitabine, degarelix, delgociclib, denileukin, deruxtecan, deucravacitinib, docetaxel, donafenib, doxorubicin, duvelisib, enasidenib, encorafenib, ensartinib, entrectinib, enzalutamide, enzastaurin, elacestrant, epirubicin, erdafitinib, eribulin, erlotinib, estradiol, estramustine, etoposide, everolimus, exemestane, fasudil, fedatinib, filgotinib, floxuridine, fludarabine, flumatinib, fluorouracil, flutamide, fluzoparib, formestane, fostamatinib, fruquintinib, fulvestrant, futibatinib, gefitinib, gemcitabine, gilteritinib, giredestrant, glasdegib, goserelin, histrelin, hydroxyurea, ibrutinib, ibudilast, icaritin, icotinib, idarubicin, idelalisib, ifosfamide, imatinib, imiquimod, infigratinib, ingenol mebutate, interferon alfa-2b, irinotecan, ivosidenib, ixabepilone, ixazomib, lanreotide, lapatinib, larotrectinib, lenalidomide, lenvatinib, letrozole, leucovorin, euprolide, lomustine, lonafarnib, lorlatinib, lurbinectedin, maytansine, mechlorethamine, medroxyprogesterone, megestrol, melphalan, melphlan flufenamide, mercaptopurine, methotrexate, methoxsalen, methylprednisolone, midostaurin, mitomycin, mitotane, mitoxantrone, mitozolomide, mobocertinib, monomethylauristatin E, monomethylauristatin F, nelarabine, nandrolone, neratinib, nearsudil, nilotinib, nilutamide, nintedanib, niraparib, octreotide, olaparib, olmutinib, olverembatinib, omacetaxine, orelabrutinib, osimertinib, oxaliplatin, paclitaxel, pacritinib, palbociclib, pamidronate, pamiparib, panobinostat, pazopanib, peficitinib, pegaptanib, pegaspargase, peginteferon alfa-2b, pemigatinib, pemetrexed, pentetreotide, pentostatin, pexidartinib, phenoxybenzamine, pidotimod, plinabulin, plitidepsin, pomalidomide, ponatinib, porfimer, pralatrexate, pralsetinib, prednisolone, procarbazine, pyrotinib, quizartinib, radotinib, raloxifene, raltitrexed, regorafenib, ribociclib, rintatolimod, ripretinib, romidepsin, rucaparib, ruxolitinib, savolitinib, selinexor, selpercatinib, selumetinib, sonidegib, sorafenib, sotorasib, streptozocin, sunitinib, surufatinib, talazoparib, tamoxifen, tazemetostat, tegafur, temozolomide, temsirolimus, teniposide, tepotinib, teprenone, thalidomide, thioguanine, thiotepa, thyrotropin alfa, tipiracil, tipifamib, tirabrutinib, tirbanibulin, tivozanib, trametinib, tofacitinib, topotecan, toremifene, trabectedin, tretinoin, trifluride, trilaciclib, triptorelin, tucatinib, upadacitinib, umbralisib, utidelone, uroacitide, valrubicin, vandetanib, vemurafenib, venetoclax, vinblastine, vincristine, vindesine, vinflunine, vinorelbine, vismodegib, vorinostat, zanubrutinib, zoledronic acid, amatoxins, anthacyclines, anthracenes, anthramycins, auristatins, bryostatins, camptothecins, carmaphycins, combretastatins, cyclosporines, cryptomycins, ecteinascidins, ellipticenes, esperamicins, mustines, neothramycins, ozogamicins, phenoxazines, podophyllotoxins, pyrrolobenzodiazepines, sibiromycins, thailanstatins, tomamycns, tubulysins, taxanes, vinca alkaloids, 7-epitaxol, 2'-acetyltaxol, 10-deacetyltaxol, 10-deacetyl-7-epitaxol, 7-xylosyltaxol, 10-deacetyl-7-glutaryl taxol, 7-*N*,*N*-dimethylglycyltaxol, 7-L-propyltaxol, larotaxel, camptothecin, 9-aminocamptothecin, 9-nitrocamptothecin, lurtotecan, gimatecan, belotecan, 10-hydroxycamptothecin, 10-hydroxy-7-ethylcamptothecin (SN-38), exatecan, pirarubicin, aclacinomycin, sirolimus, tacrolimus, progesterone, estrogen, rapamycin, plicamycin, cephalotaxin, and curcumin.

**27.** The polymer-drug conjugate according to claim 25 or 26, wherein the polymer-drug conjugate meets one or more of the following conditions:

(1) the antineoplastic is an anti-tumor targeted drug selected from one or more of aldesleukin, abemaciclib, abiraterone, abrocitinib, acalabrutinib, afatinib, alectinib, alflutinib, almonertinib, amcenestrant, anastrozole, anlotinib, apalutamide, apatinib, arzoxifene, asciminib, avapritinib, avitinib, axitinib, baricitinib, belinostat, bexarotene, bicalutamide, binimetinib, bleomycin, boanmycin, bortezomib, bosutinib, brigatinib, buserelin, cabozantinib, capmatinib, carfilzomib, carmustine, ceritinib, cetrorelix, chidamide, cobimetinib, copanlisib, crizotinib, dabrafenib, dacomitinib, dalpiciclib, darolutamide, dasatinib, degarelix, delgociclib, deucravacitinib, donafenib, duvelisib, enasidenib, encorafenib, ensartinib, entrectinib, enzalutamide, enzastaurin, elacestrant, erdafitinib, erlotinib, everolimus, fedatinib, filgotinib, flumatinib, fluzoparib, formestane, fostamatinib, fruquintinib, fulvestrant, futibatinib, gefitinib, gilteritinib, giredestrant, glasdegib, goserelin, histrelin, ibrutinib, ibudilast, icotinib, idarubicin, idelalisib, imatinib, imiquimod, infigratinib, ivosidenib, ixazomib, lanreotide, lapatinib, larotrectinib, lenalidomide, lenvatinib, letrozole, leucovorin, leuprolide, lonafarnib, lorlatinib, medroxyprogesterone, megestrol, methylprednisolone, midostaurin, mobocertinib, nandrolone, neratinib, nilotinib, nilutamide, nintedanib, niraparib, olaparib, olmutinib, olverembatinib, orelabrutinib, osimertinib, pacritinib, palbociclib, pamidronate, pamiparib, panobinostat, pazopanib, peficitinib, pegaptanib, pemigatinib, pexidartinib, pidotimod, pomalidomide, ponatinib, pralsetinib, pyrotinib, quizartinib, radotinib, raloxifene, regorafenib, ribociclib, rintatolimod, ripretinib, rucaparib, ruxolitinib, savolitinib, selinexor, selpercatinib, selumetinib, sonidegib, sorafenib, sotorasib, sunitinib, surufatinib, talazoparib, tamoxifen, tazemetostat, temsirolimus, tepotinib, thalidomide, tipifarnib, tirabrutinib, tivozanib, trametinib, tofacitinib, toremifene, tretinoin, trilaciclib, triptorelin, tucatinib, upadacitinib, umbralisib, vandetanib, vemurafenib, venetoclax, vismodegib, vorinostat, zanubrutinib, and zoledronic acid; and (2) the antineoplastic is a chemotherapeutic selected from one or more of altretamine, aminoglutethimide, amsacrine, asparaginase, azacitidine, bendamustine, bexarotene, bicyclol, bleomycin, boanmycin, buserelin, busulfan, cabazitaxel, calaspargase, calicheamycin, capecitabine, carboplatin, carmustine, carmofur, cedazuidine, chlorambucil, cisplatin, cladribine, clofarabine, colchicine, cyclophosphamide, cytarabine, dacarbazine, dactinomycin, daunorubicin, decitabine, denileukin, deruxtecan, docetaxel, doxorubicin, epirubicin, eribulin, estradiol, estramustine, etoposide, exemestane, fasudil, floxuridine, fludarabine, fluorouracil, flutamide, formestane, gemcitabine, hydroxyurea, icaritin, idarubicin, ifosfamide, ingenol mebutate, irinotecan, ixabepilone, leucovorin, lomustine, lurbinctedin, maytansine, mechlorethamine, medroxyprogesterone, megestrol, melphalan, melphlan flufenamide, mercaptopurine, methotrexate, methoxsalen, methylprednisolone, mitomycin, mitotane, mitoxantrone, mitozolomide, monomethylauristatin E, monomethylauristatin F, nelarabine, nandrolone, nearsudil, octreotide, omacetaxine, oxaliplatin, paclitaxel, pamidronate, pemetrexed, pentetreotide, pentostatin, phenoxybenzamine, plinabulin, plitidepsin, porfimer, pralatrexate, prednisolone, procarbazine, procarbazine, raltitrexed, romidepsin, streptozocin, tegafur, temozolomide, teniposide, teprenone, thioguanine, thiotepa, thyrotropin alfa, tipiracil, tirbanibulin, topotecan, trabectedin, trifluride, utidelone, uroacitide, valrubicin, vinblastine, vincristine, vindesine, vinflunine, vinorelbine, amatoxins, anthacyclines, anthracenes, anthramycins, auristatins, bryostatins, camptothecins, carmaphycins, combretastatins, cyclosporines, cryptomycins, ecteinascidins, ellipticenes, esperamicins, mustines, neothramycins, ozogamicins, phenoxazines, podophyllotoxins, pyrrolobenzodiazepines, sibiromycins, thailanstatins, tomamycns, tubulysins, taxanes, vinca alkaloids, 7-epitaxol, 2'-acetyltaxol, 10-deacetyltaxol, 10-deacetyl-7-epitaxol, 7-xylosyltaxol, 10-deacetyl-7-glutaryl taxol, 7-N,N-dimethylglycyltaxol, 7-L-propyltaxol, larotaxel, camptothecin, 9-aminocamptothecin, 9-nitrocamptothecin, lurtotecan, gimatecan, belotecan, 10-hydroxycamptothecin, 10-hydroxy-7-ethylcamptothecin (SN-38), exatecan, pirarubicin, aclacinomycin, sirolimus, tacrolimus, progesterone, estrogen, rapamycin, plicamycin, cephalotaxin, and curcumin.

**28.** The polymer-drug conjugate according to any one of claims 1, 5, 8, and 9, wherein the pharmaceutically active agent residue D is selected from the following structures:

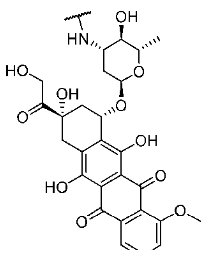
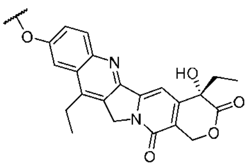
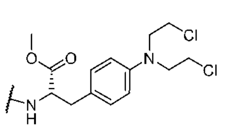
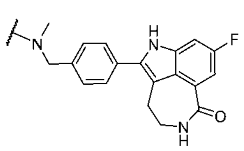

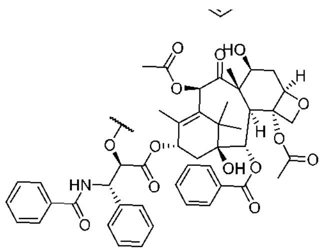
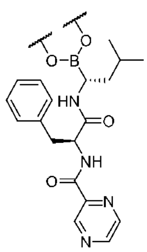
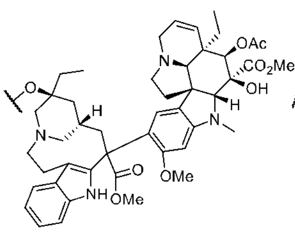
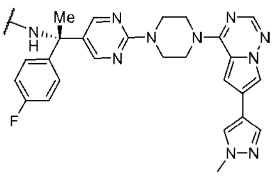

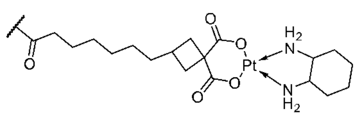

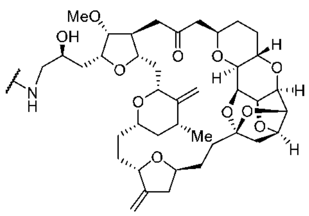

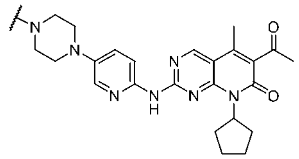

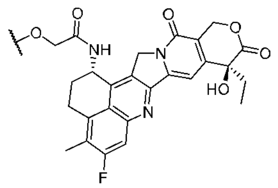

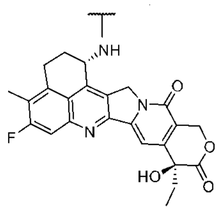

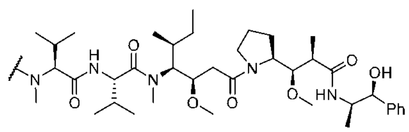

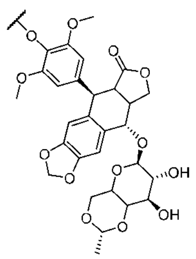

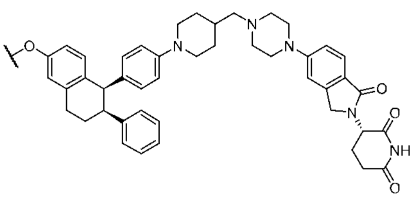

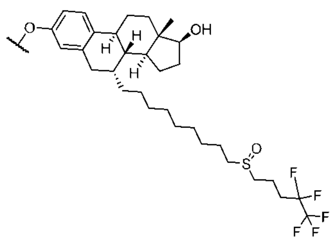

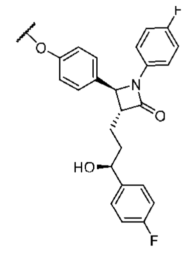

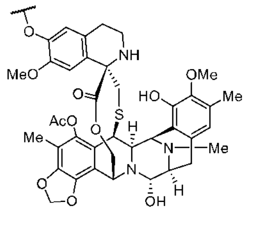

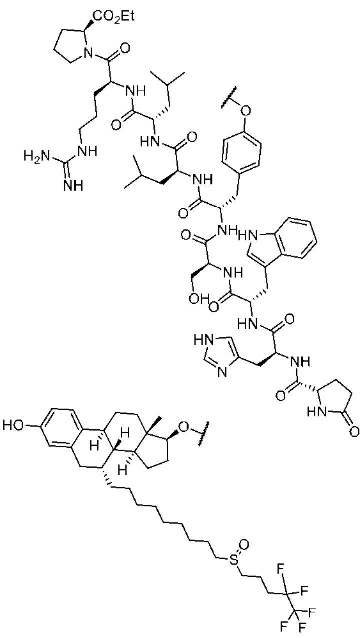

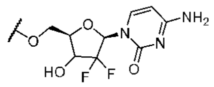

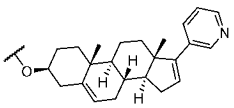

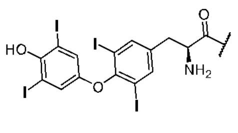

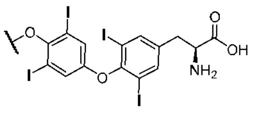

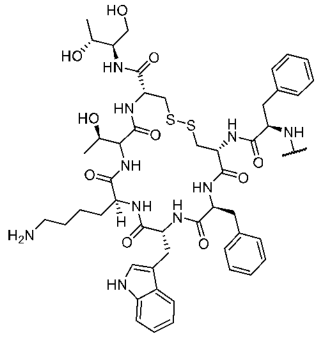

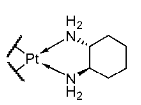

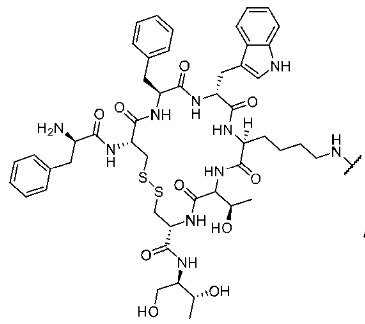

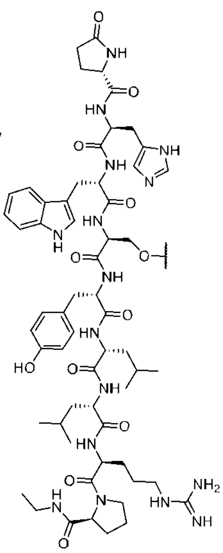

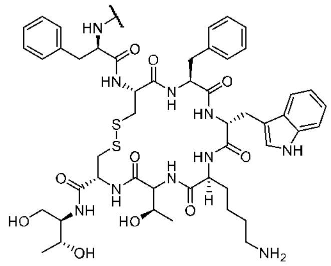

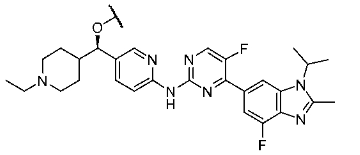

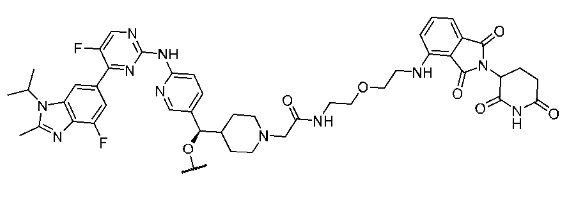

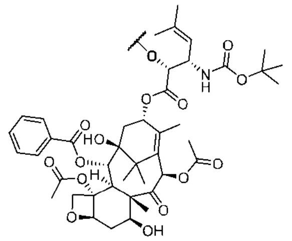

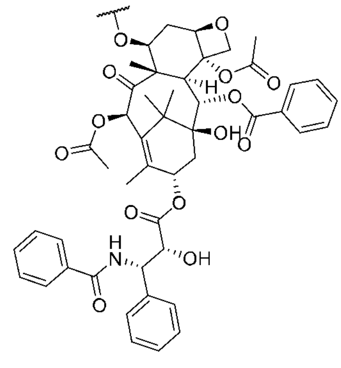
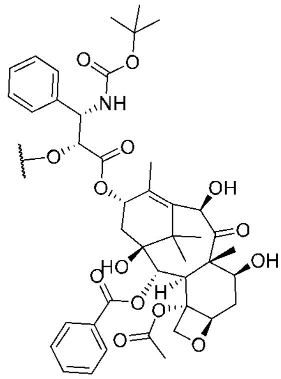
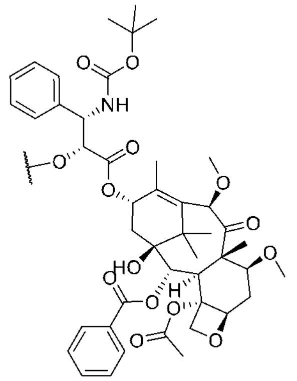

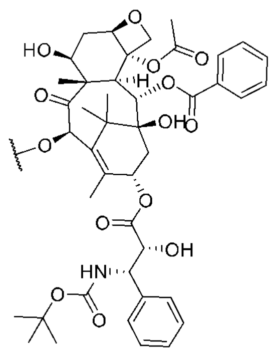
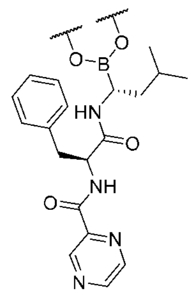
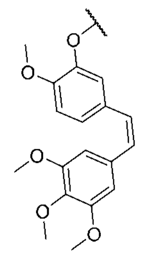
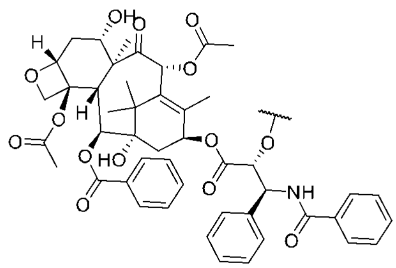

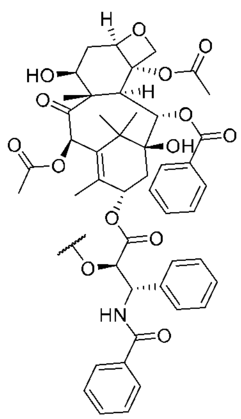
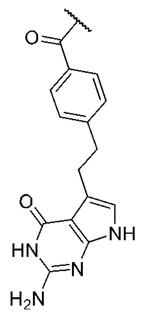
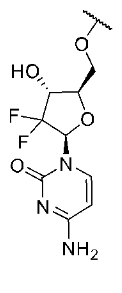
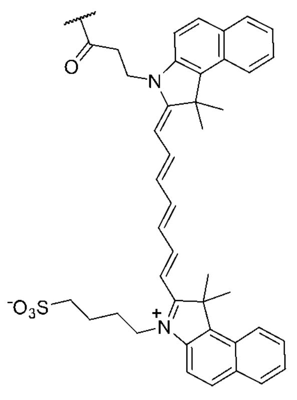

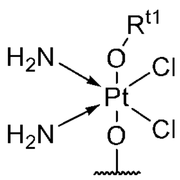
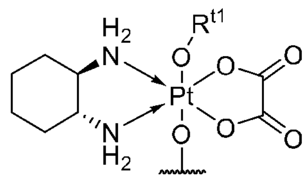
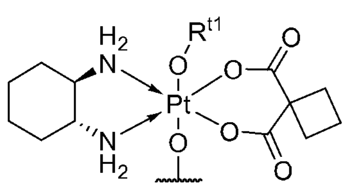

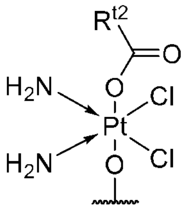
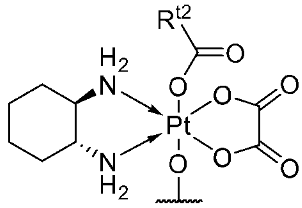
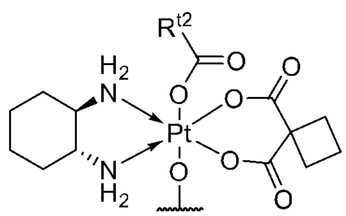
;

wherein $R^{t1}$ is selected from H and $C_1$-$C_{20}$ alkyl; $R^{t2}$ is selected from $NHR^{t1}$ and $C_1$-$C_{20}$ alkyl.

**29.** The polymer-drug conjugate according to any one of claims 8, 9, 11, 17, and 18, wherein the linker $L^{2a}$ is selected from the following structures:

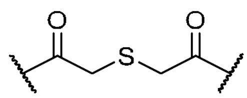,
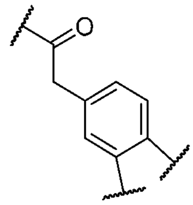,
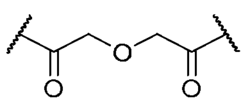,
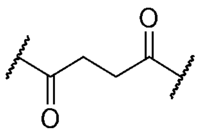,
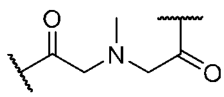,
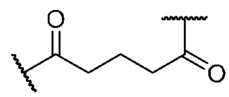,
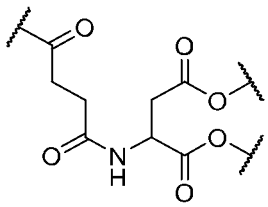,
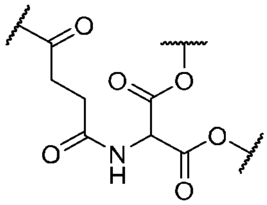,
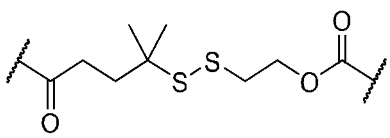,
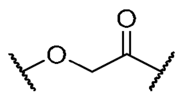,
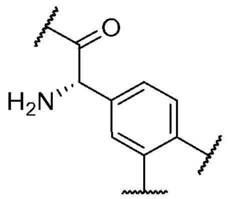,
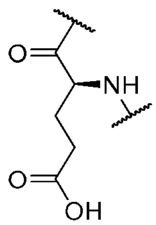,
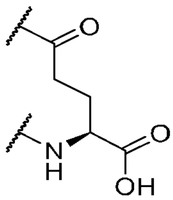,
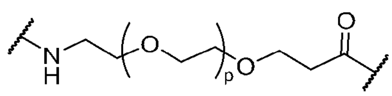,
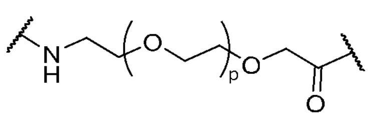,
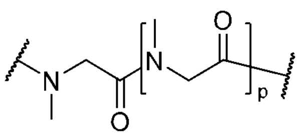,
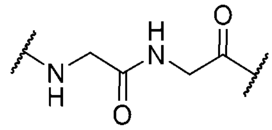,
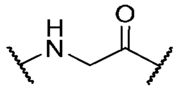,
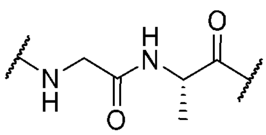,
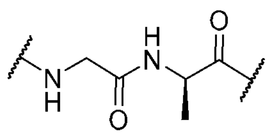,
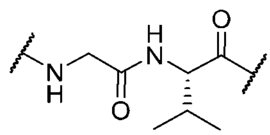,
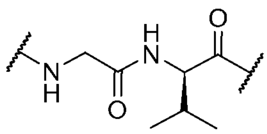,
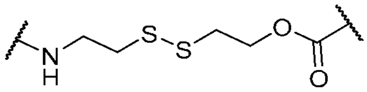,
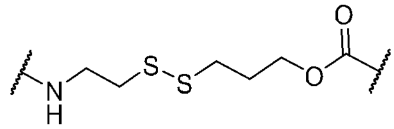,
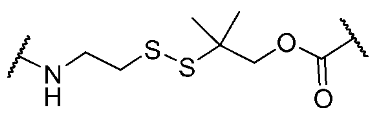,

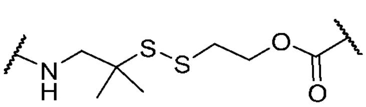 , 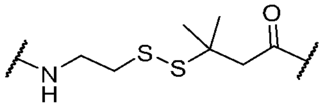 , 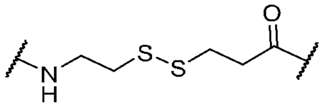 ,

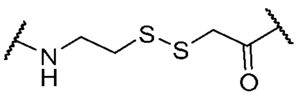 , 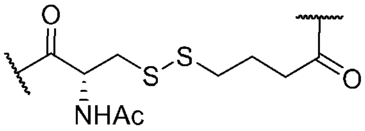 , 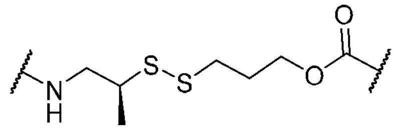 ,

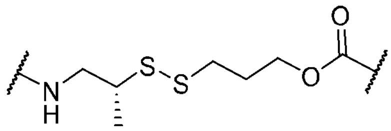 , and 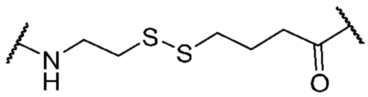 .

**30.** The polymer-drug conjugate according to any one of claims 8, 9, and 11, wherein the linker $L^{2b}$ is selected from the following structures:

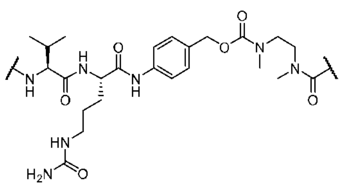
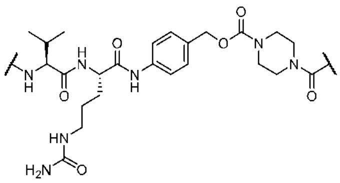
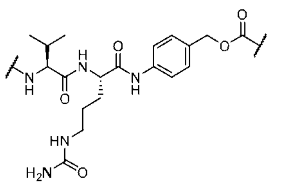

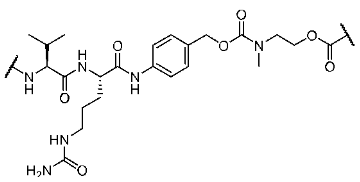
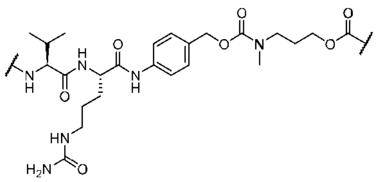
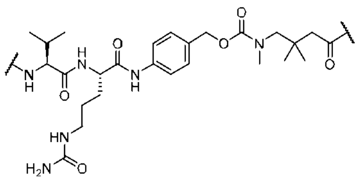

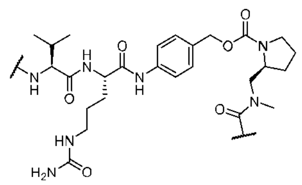
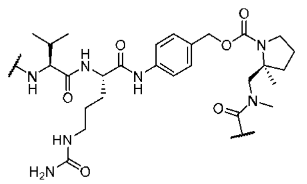
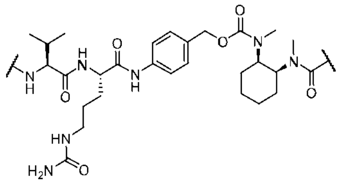

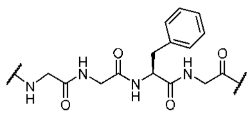
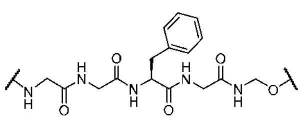
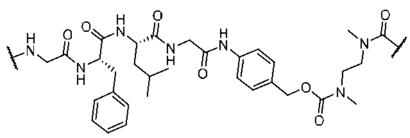

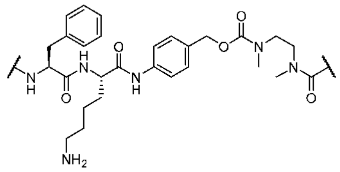
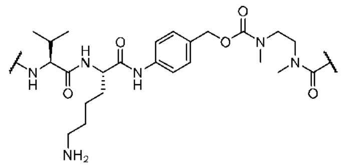
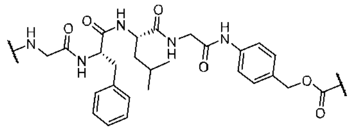

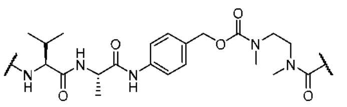
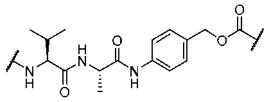
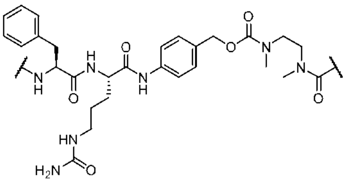

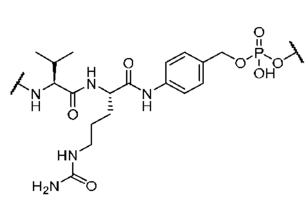
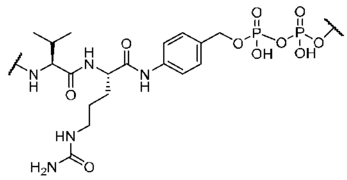
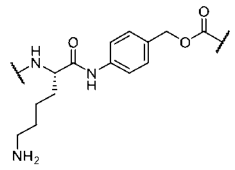

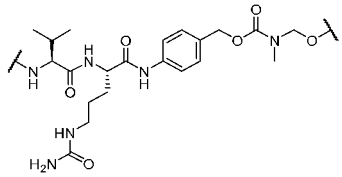
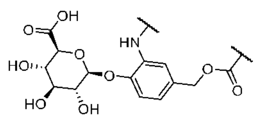
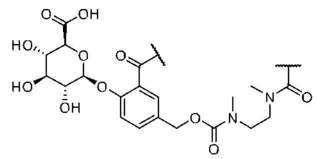

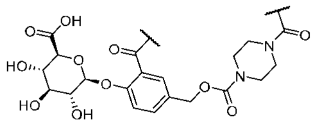
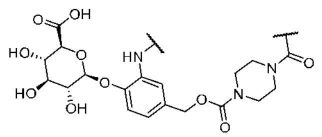
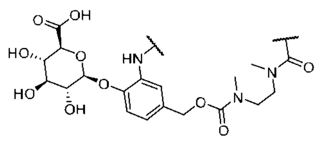

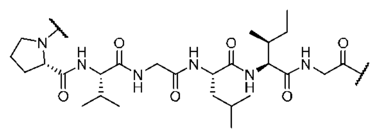

**31.** The polymer-drug conjugate according to any one of claims 1, 5, 8, 9, 11, and 12, wherein the polymer-drug conjugate is selected from the following structures:

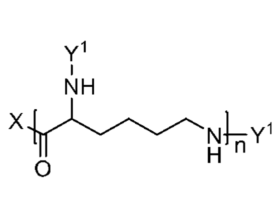
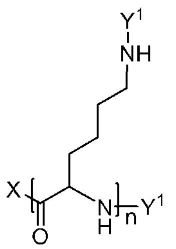
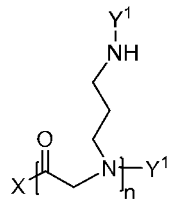
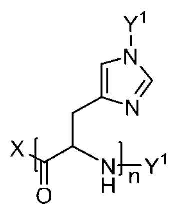

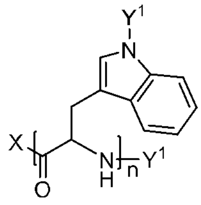
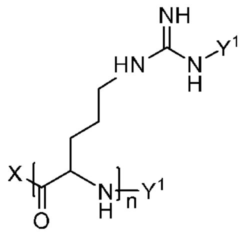
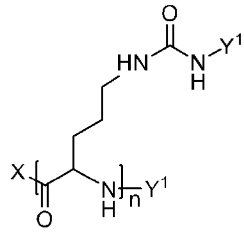
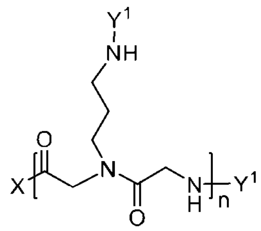

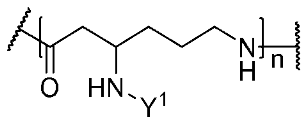
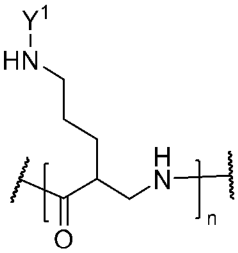
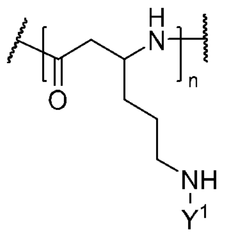

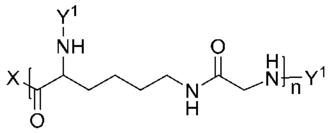
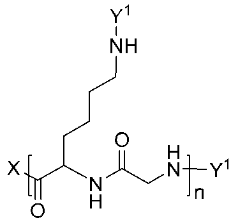
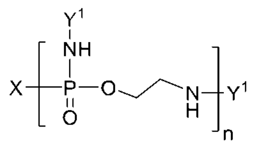

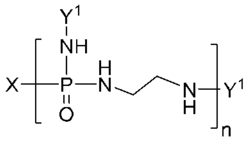
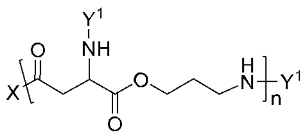
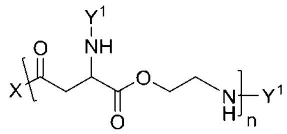

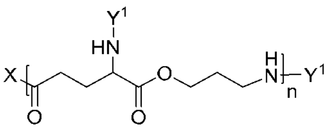
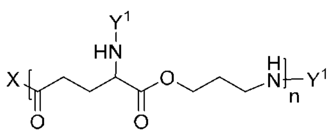
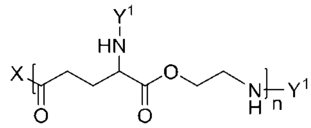

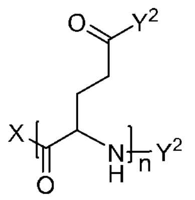
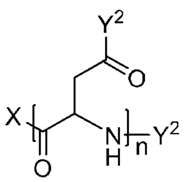
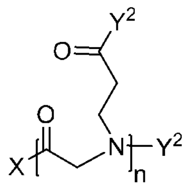

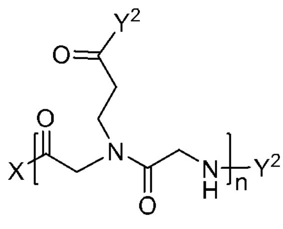
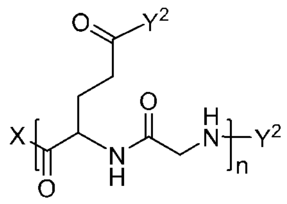
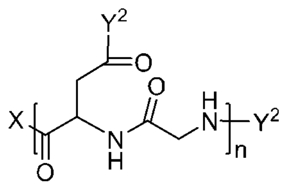

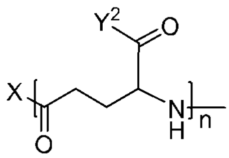
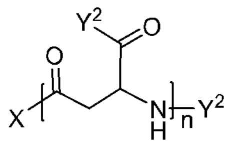

wherein, $Y^1$ =

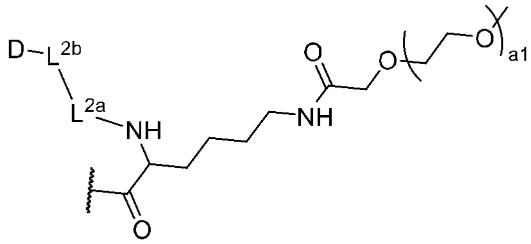
;

$Y^2$ =

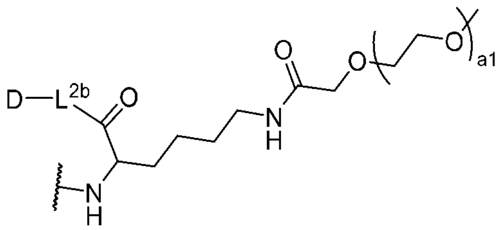
;

$L^{2a}$ and $L^{2b}$ are each as defined in any one of claims 1 and 4-15;
D is as defined in any one of claims 1 and 19-24;
X is as defined in claim 1 or 12;
n is an integer of 10-70;
$a^1$ is an integer of 5-150.

**32.** The polymer-drug conjugate according to any one of claims 19-21, wherein $R^b$ is methyl, and the PEG residue has a number-average molecular weight of 550, 1000, 2000, 3000, 4000, or 5000.

**33.** The polymer-drug conjugate according to any one of claims 1, 8, and 9, wherein the polymer-drug conjugate meets one or more of the following conditions:

(1) the branched center Y is linked to the polymer residue via the linker $L^0$, and the molar ratio of the branched center Y to the polymer structural unit is 0.5:1 to 1.5:1;
(2) the pharmacokinetic regulator residue P is linked to the branched center Y via the linker $L^1$, and the molar ratio of the pharmacokinetic regulator residue to the polymer structural unit is 0.5:1 to 1.5:1; and
(3) the pharmaceutically active agent residue D is linked to the trifunctional branched center Y via the linker $L^2$, and the molar ratio of the pharmaceutically active agent residue D to the polymer structural unit is 0.5:1 to 1.5:1.

**34.** The polymer-drug conjugate according to any one of claims 1-32, wherein $-L^2$-D meets either of the following conditions:

condition 1: $-L^2$-D is any one of the following structures:

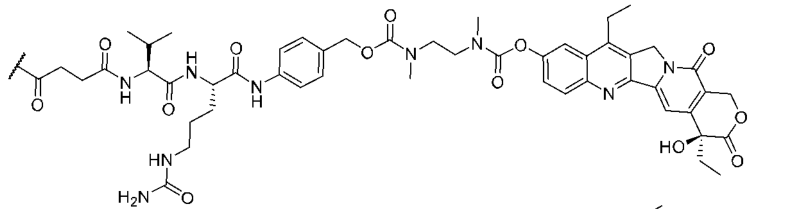

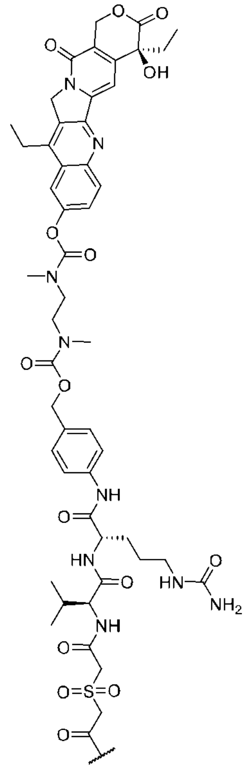

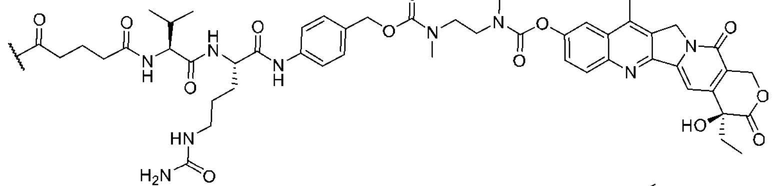

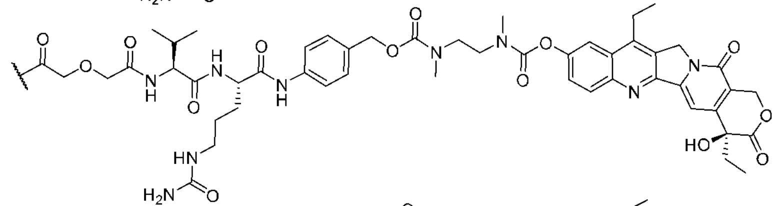

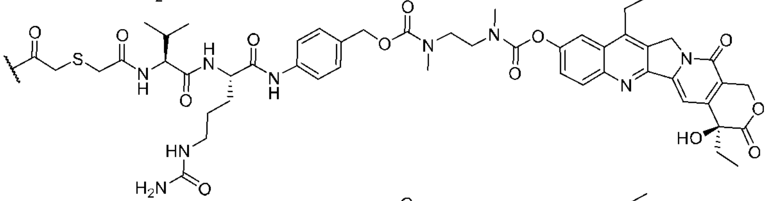

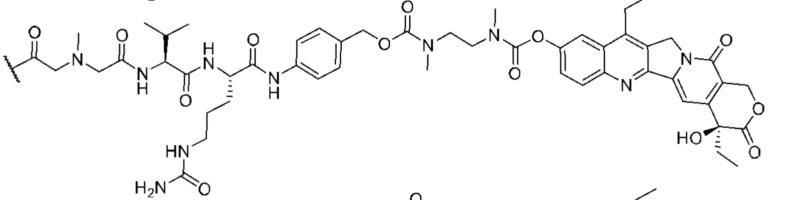

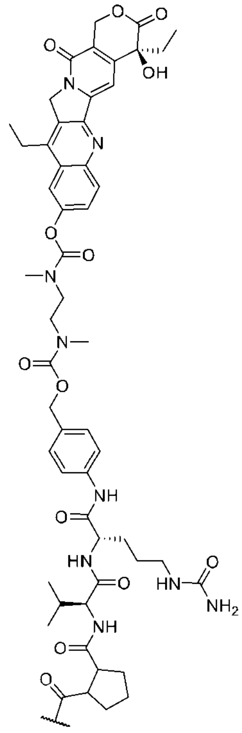

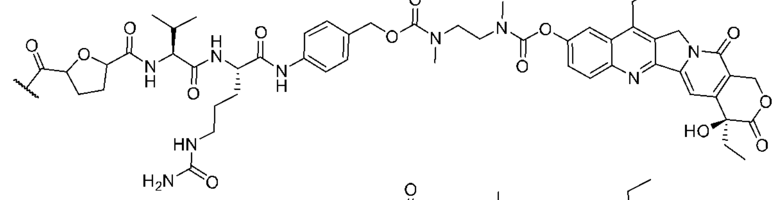

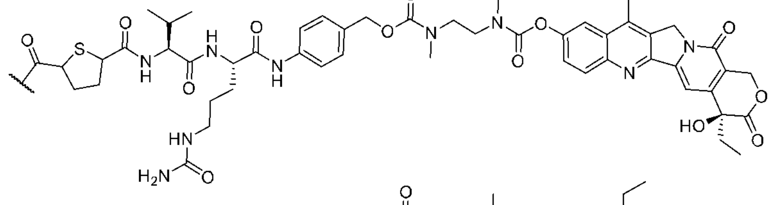

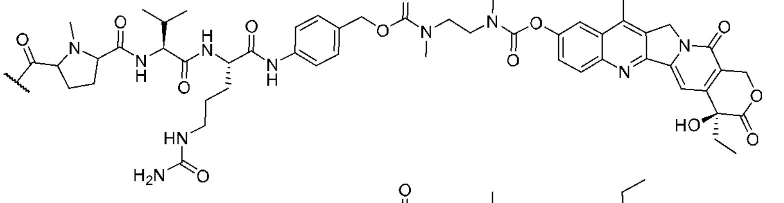

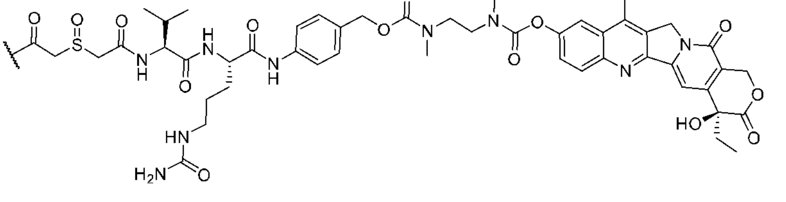

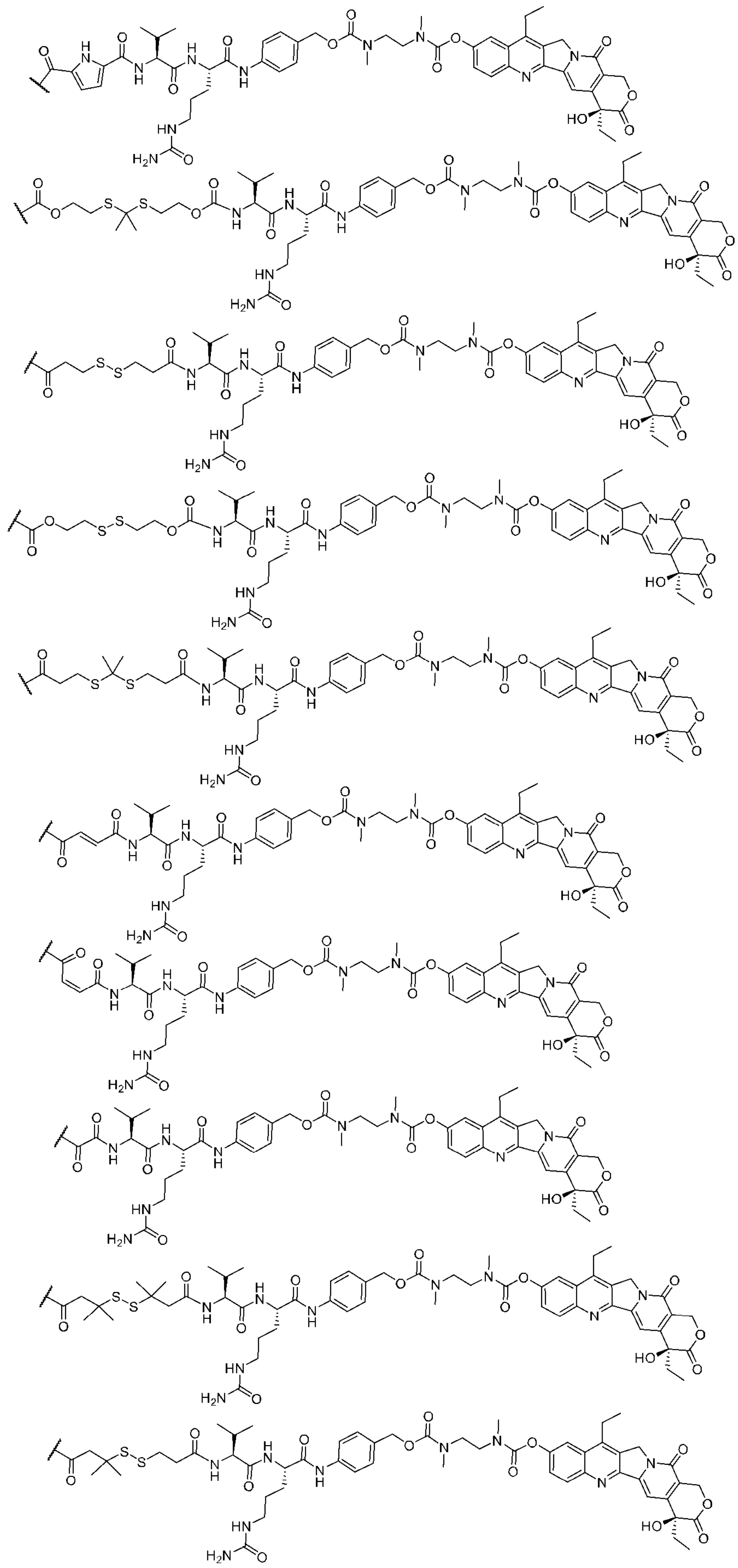

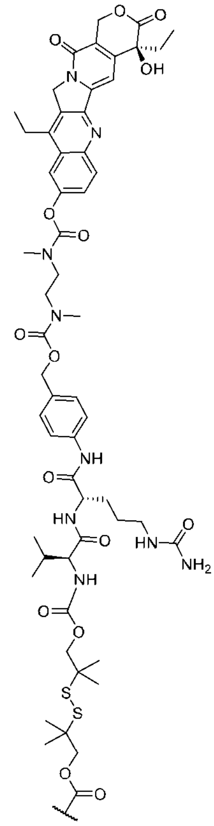

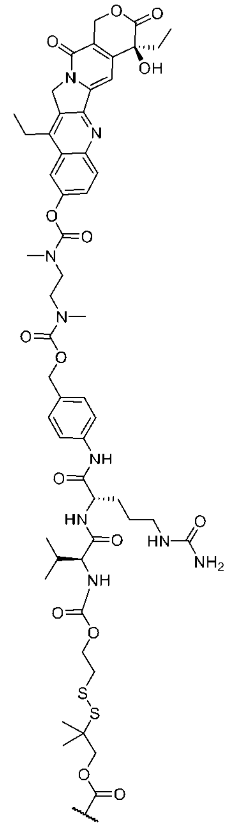

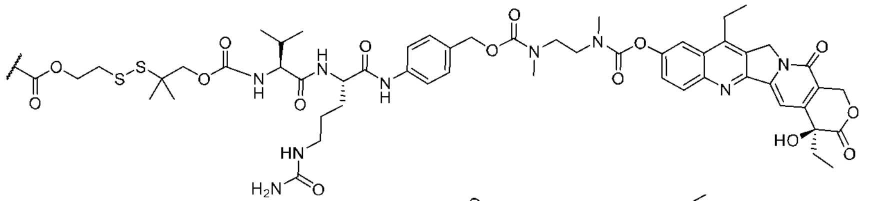

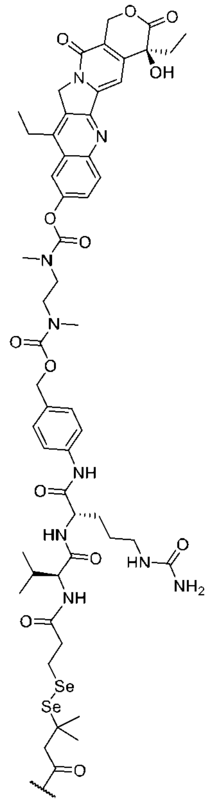

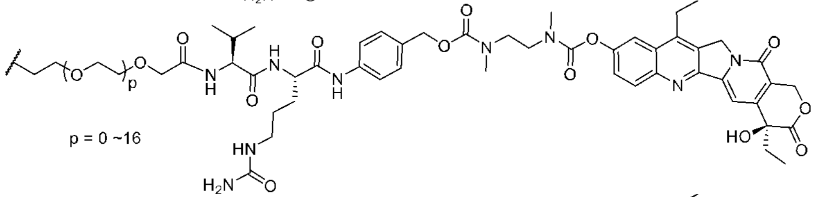

p = 0 ~16

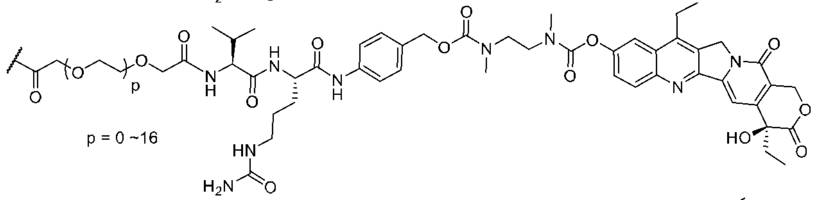

p = 0 ~16

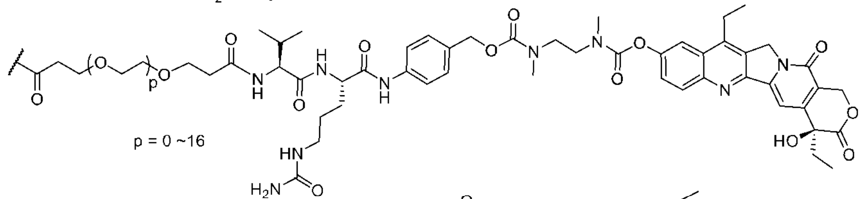

p = 0 ~16

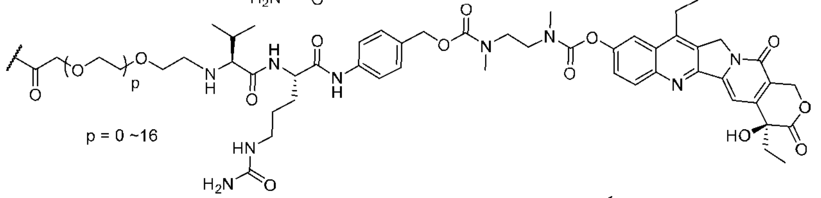

p = 0 ~16

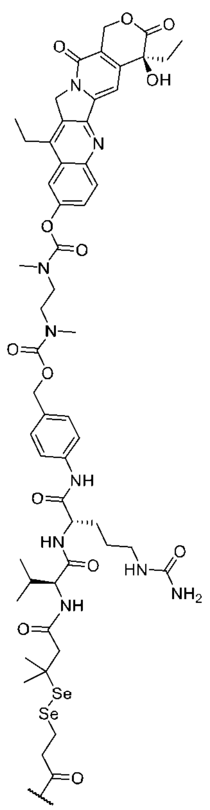

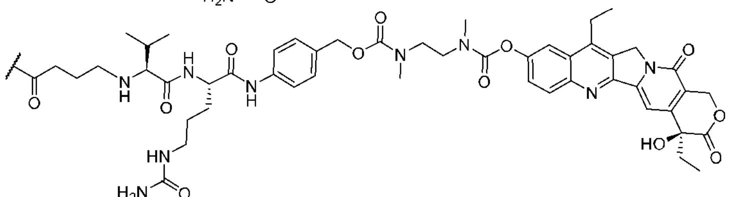

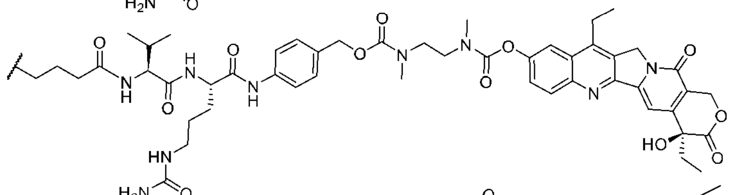

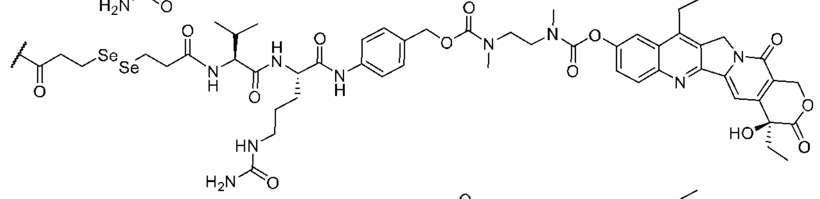

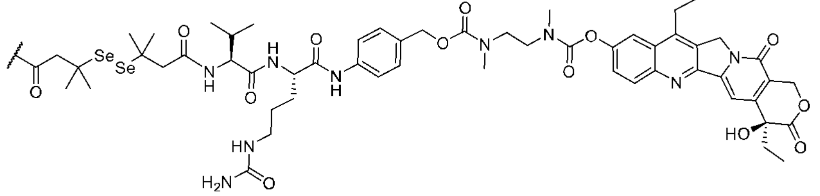

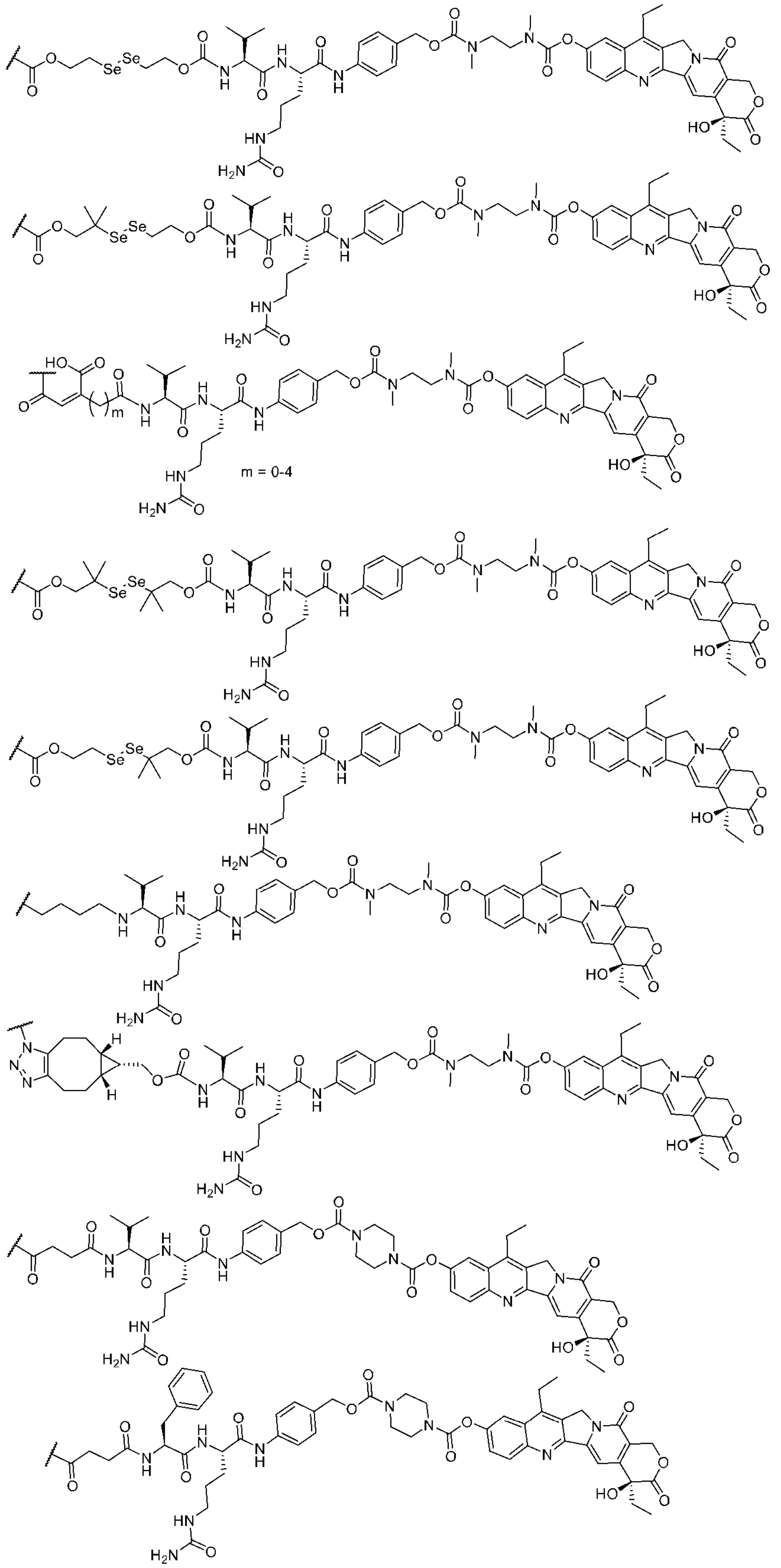

m = 0-4

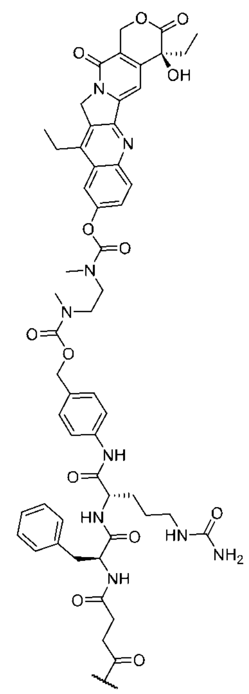

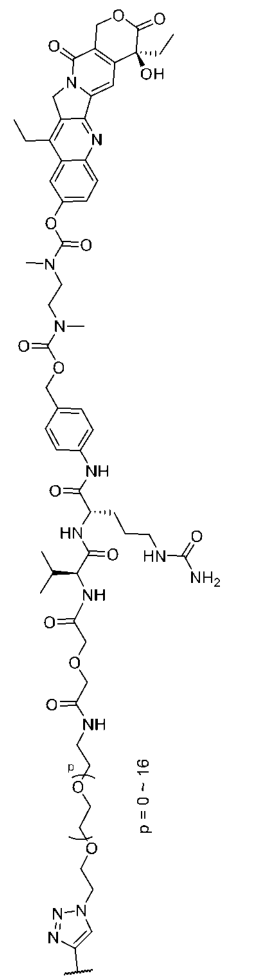

p = 0 ~ 16

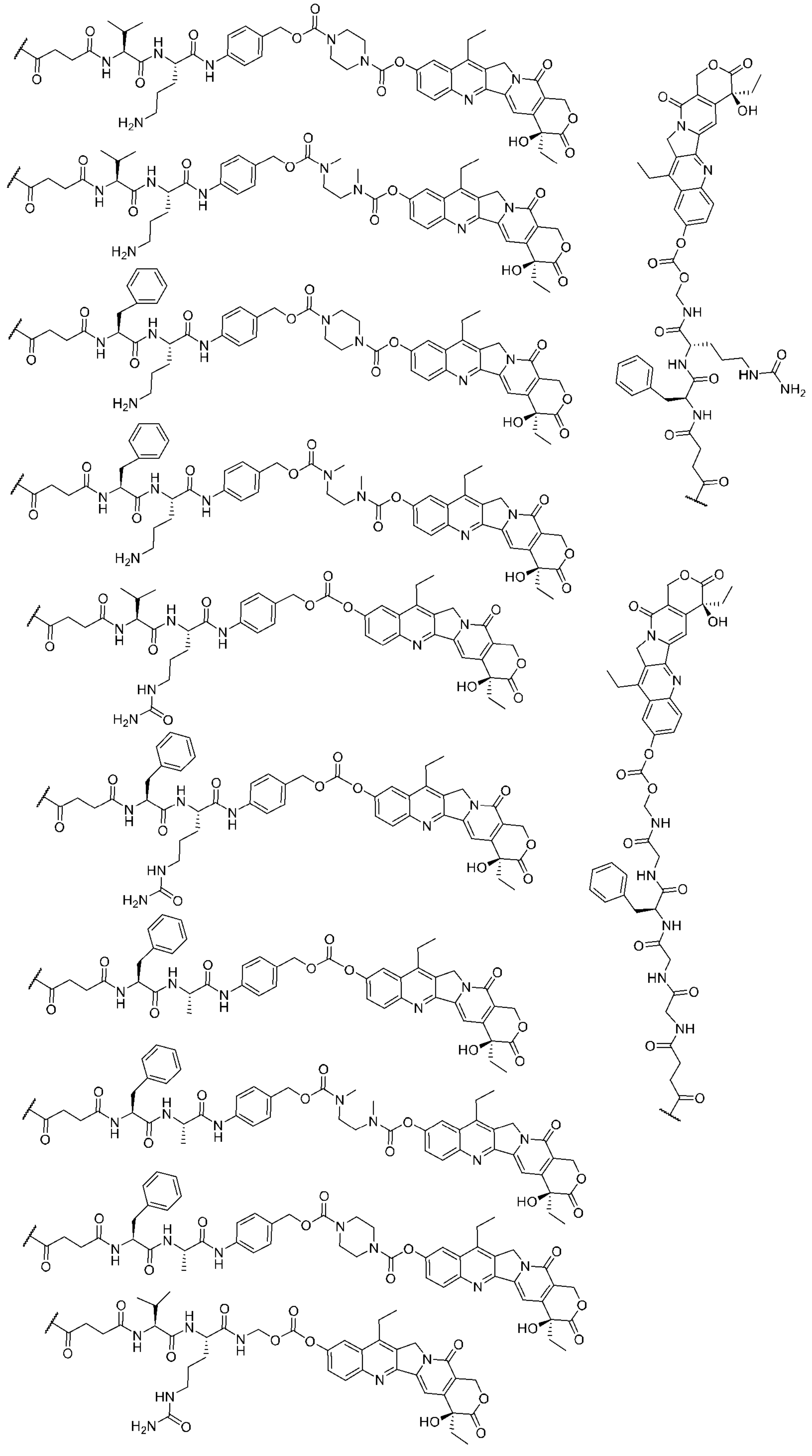

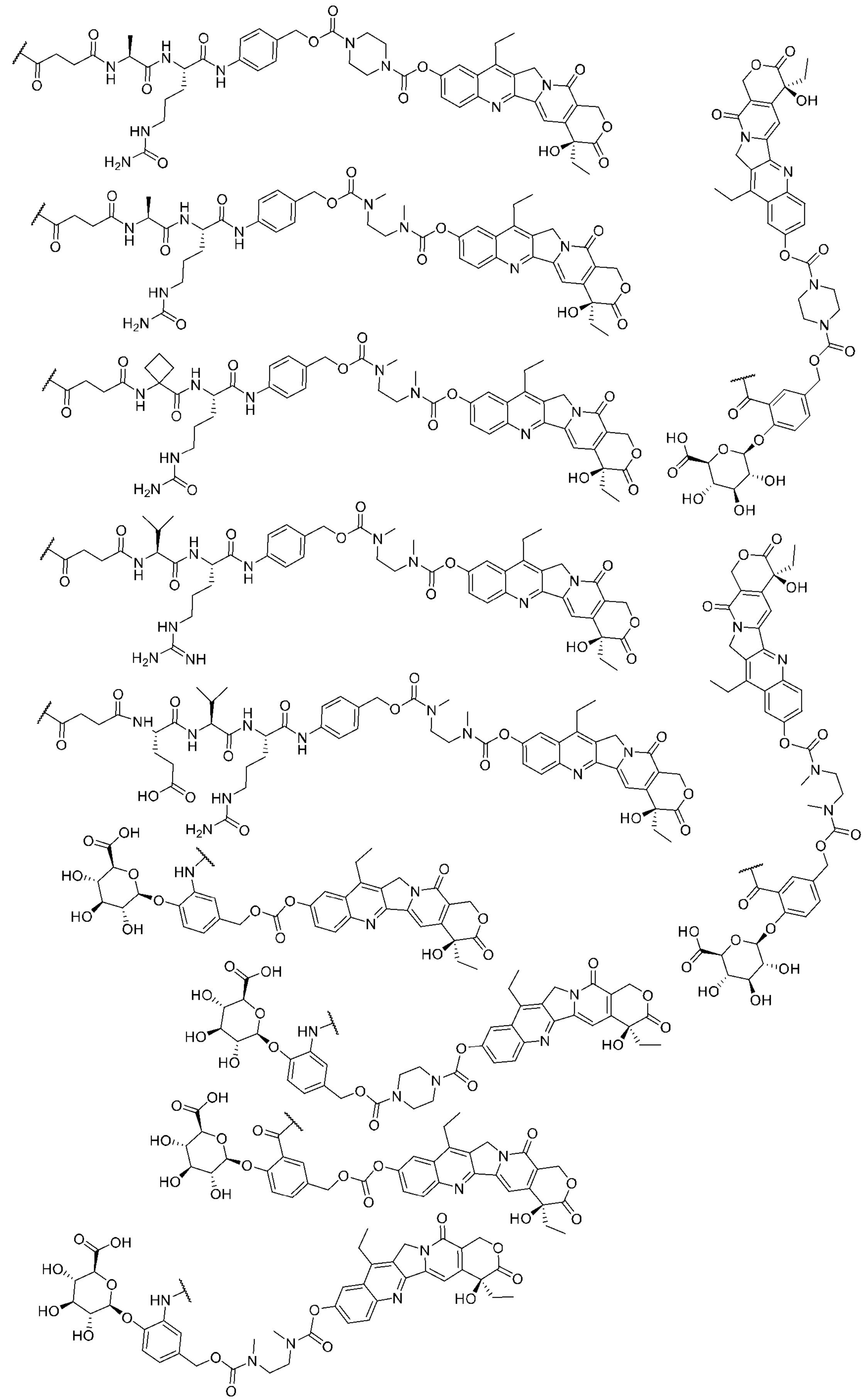

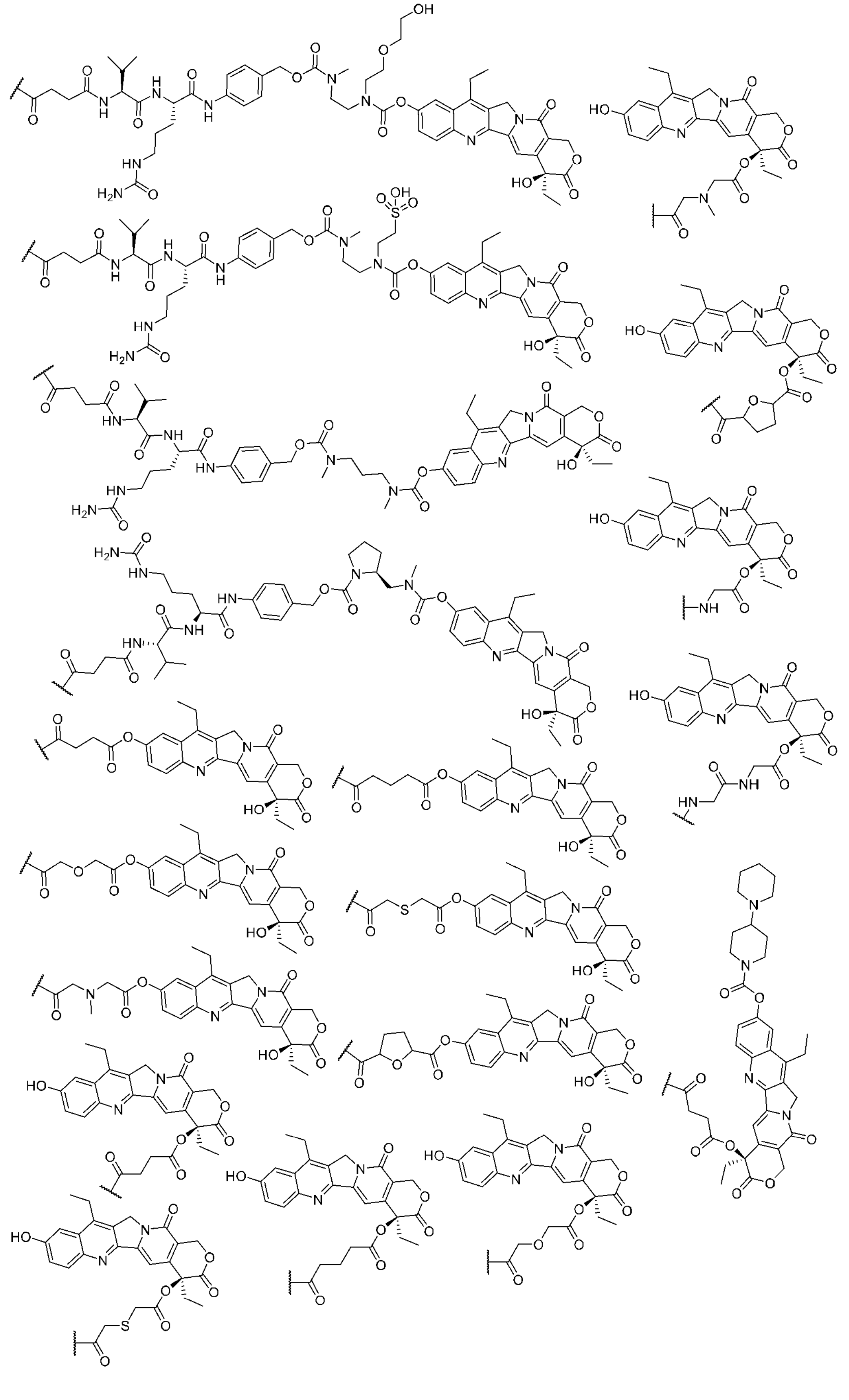

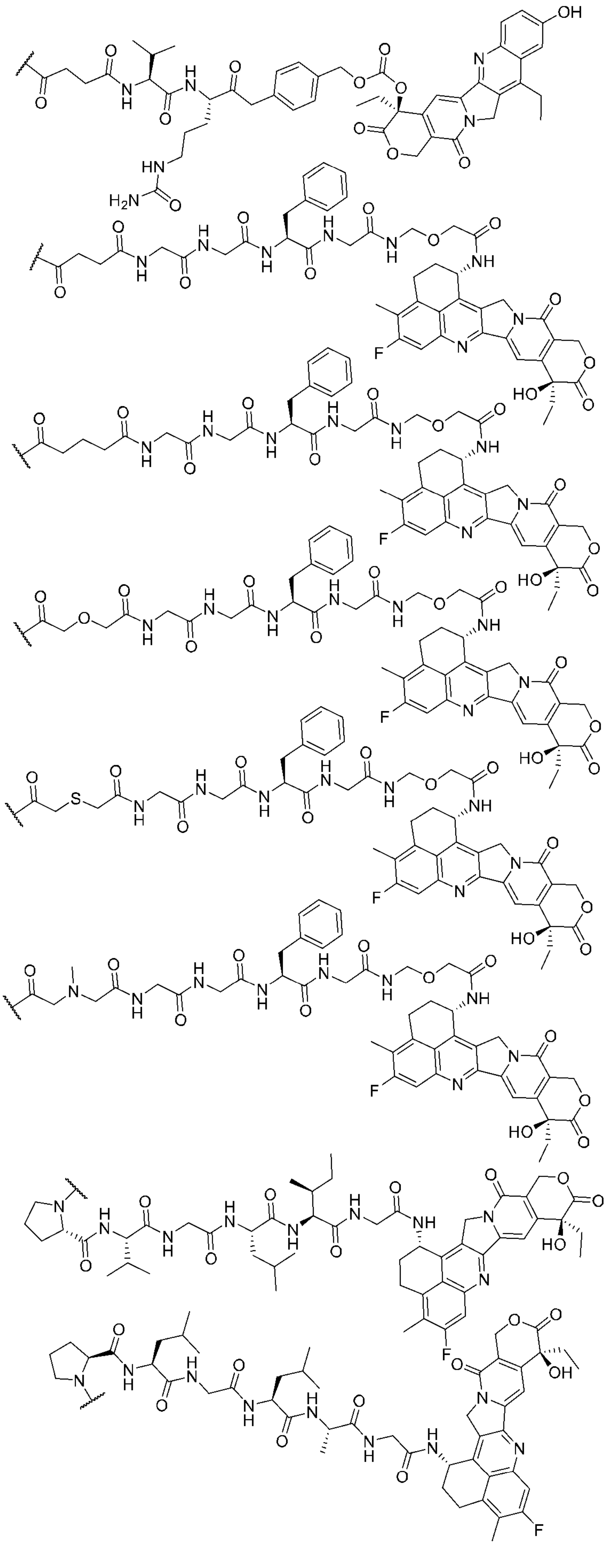

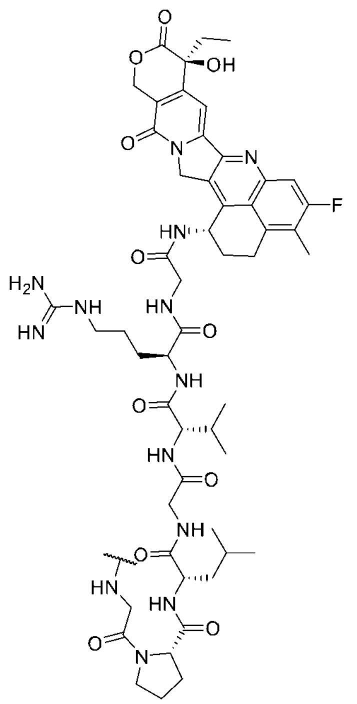

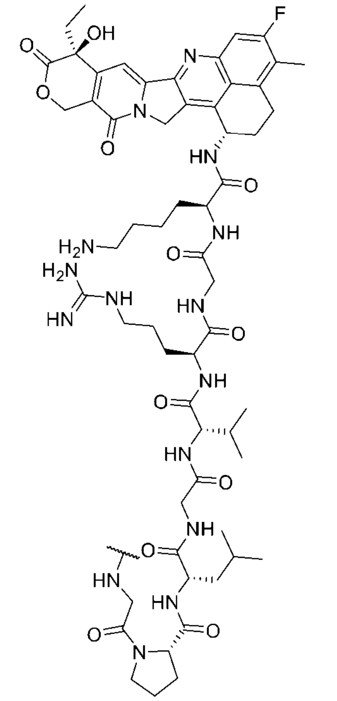

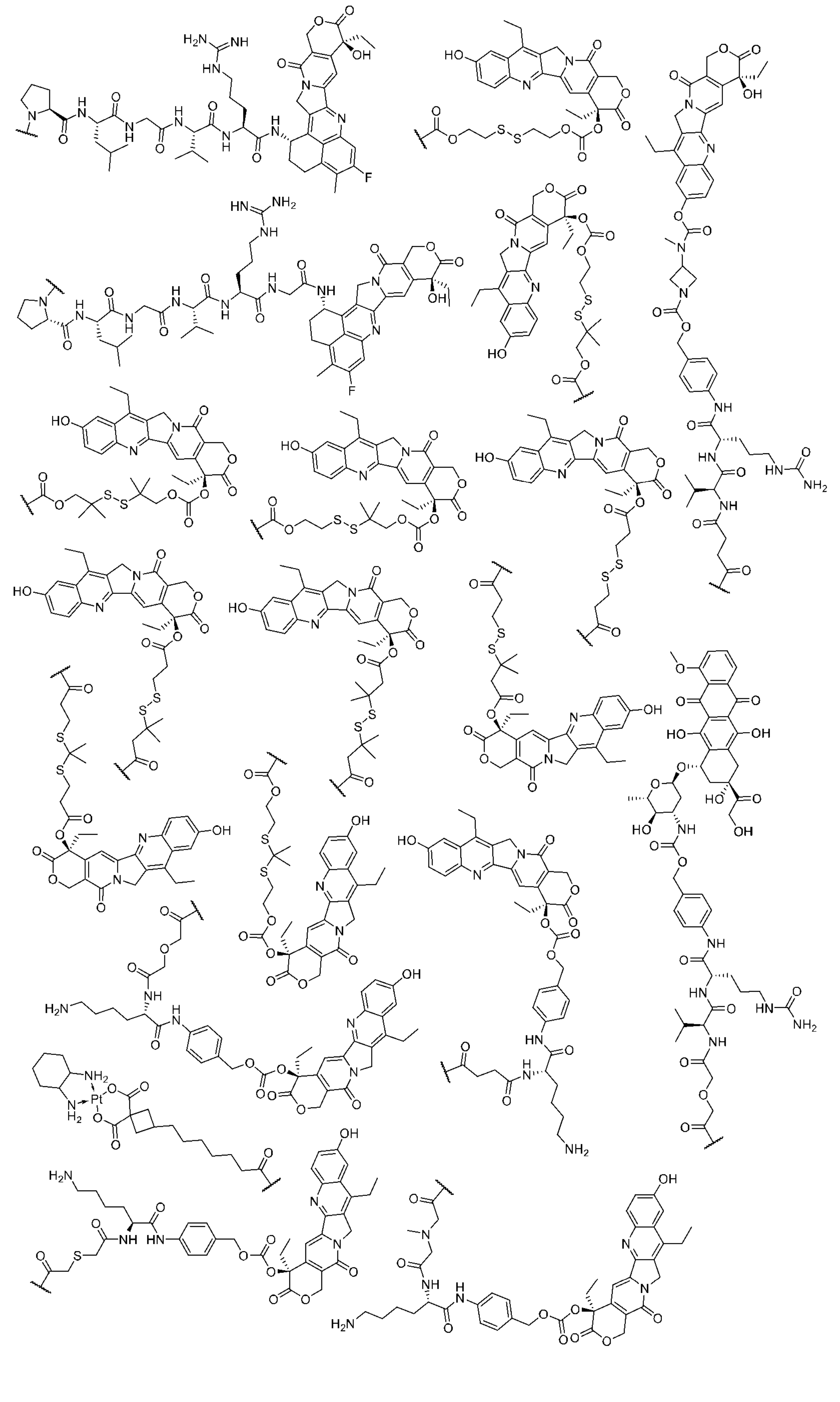

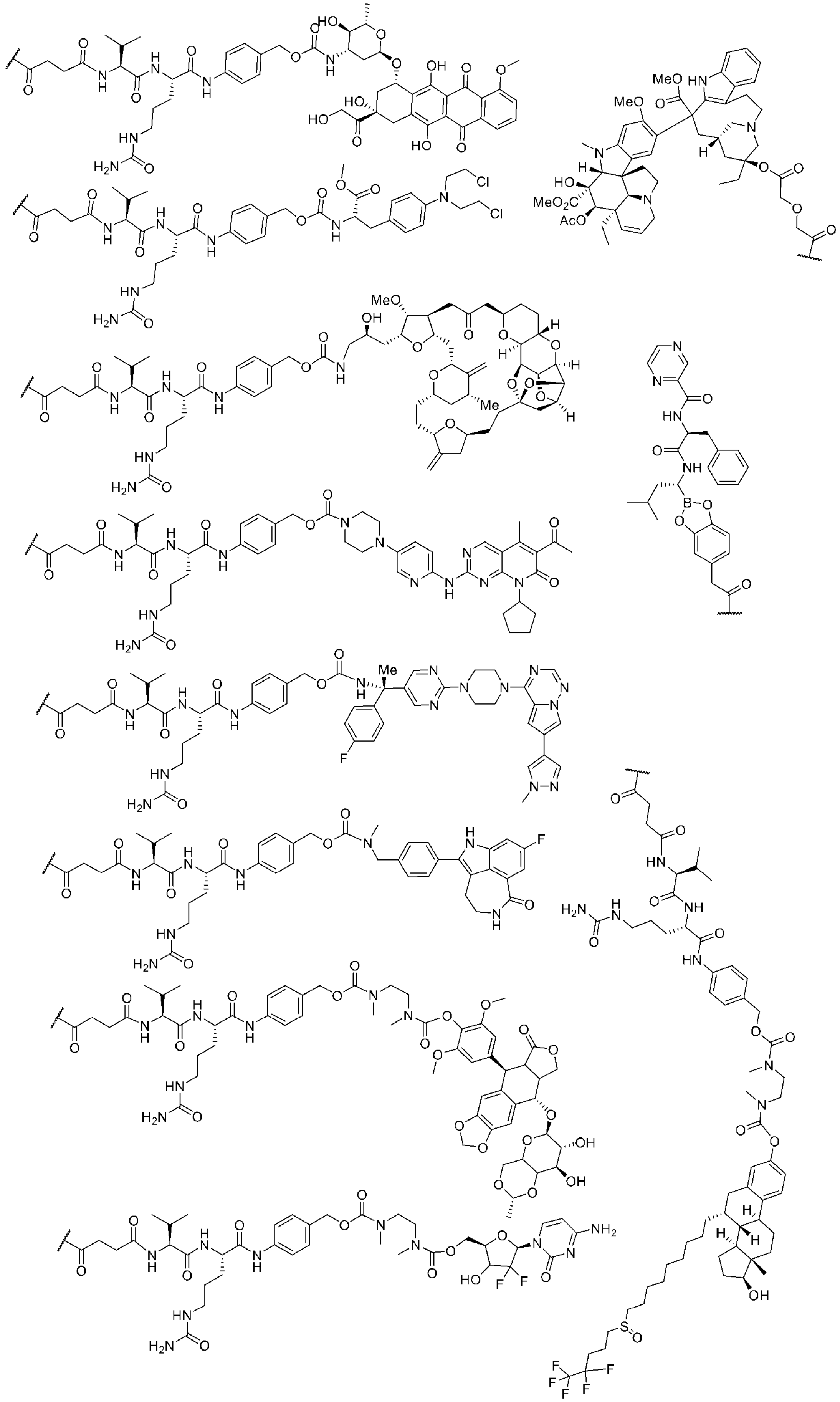

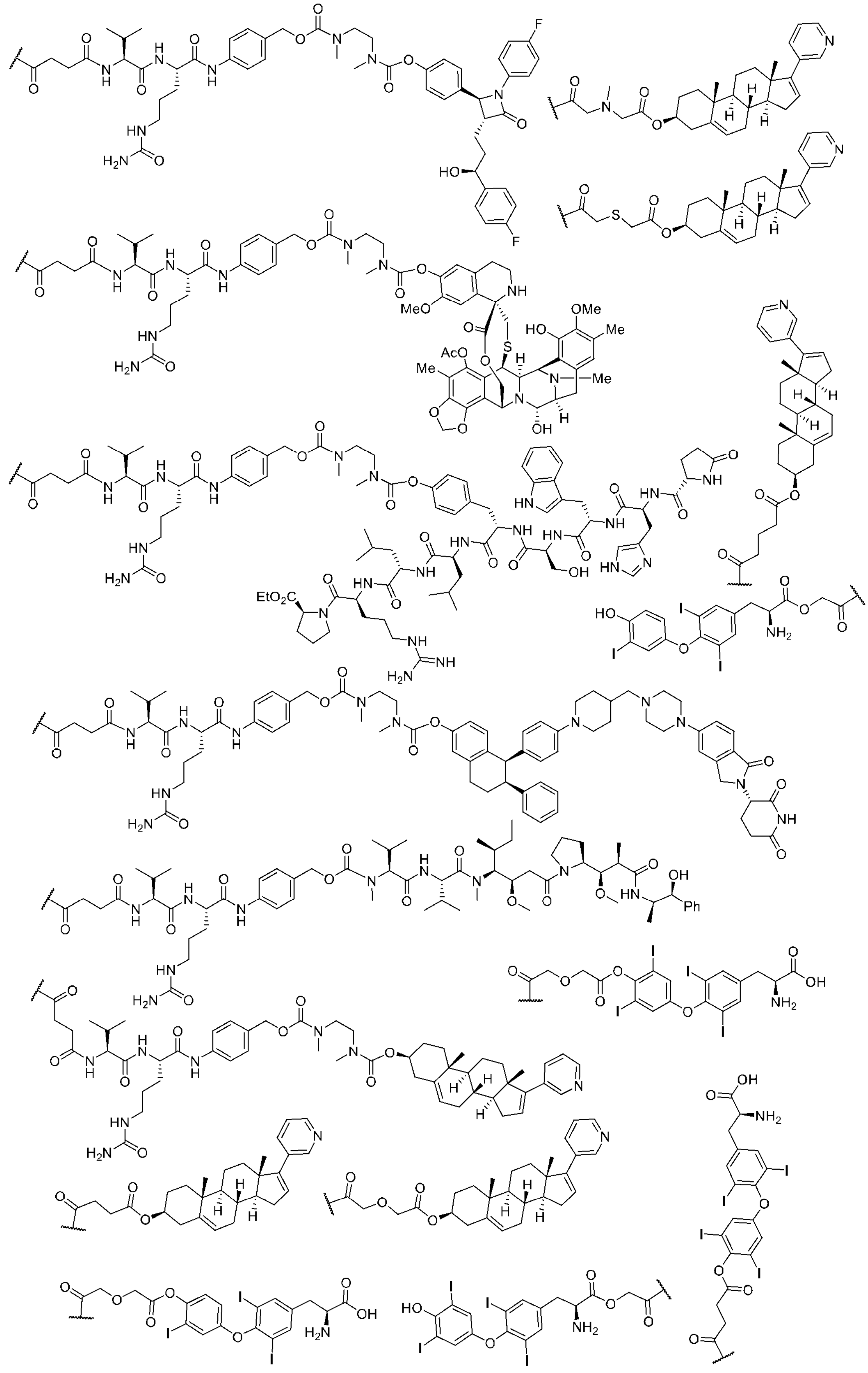

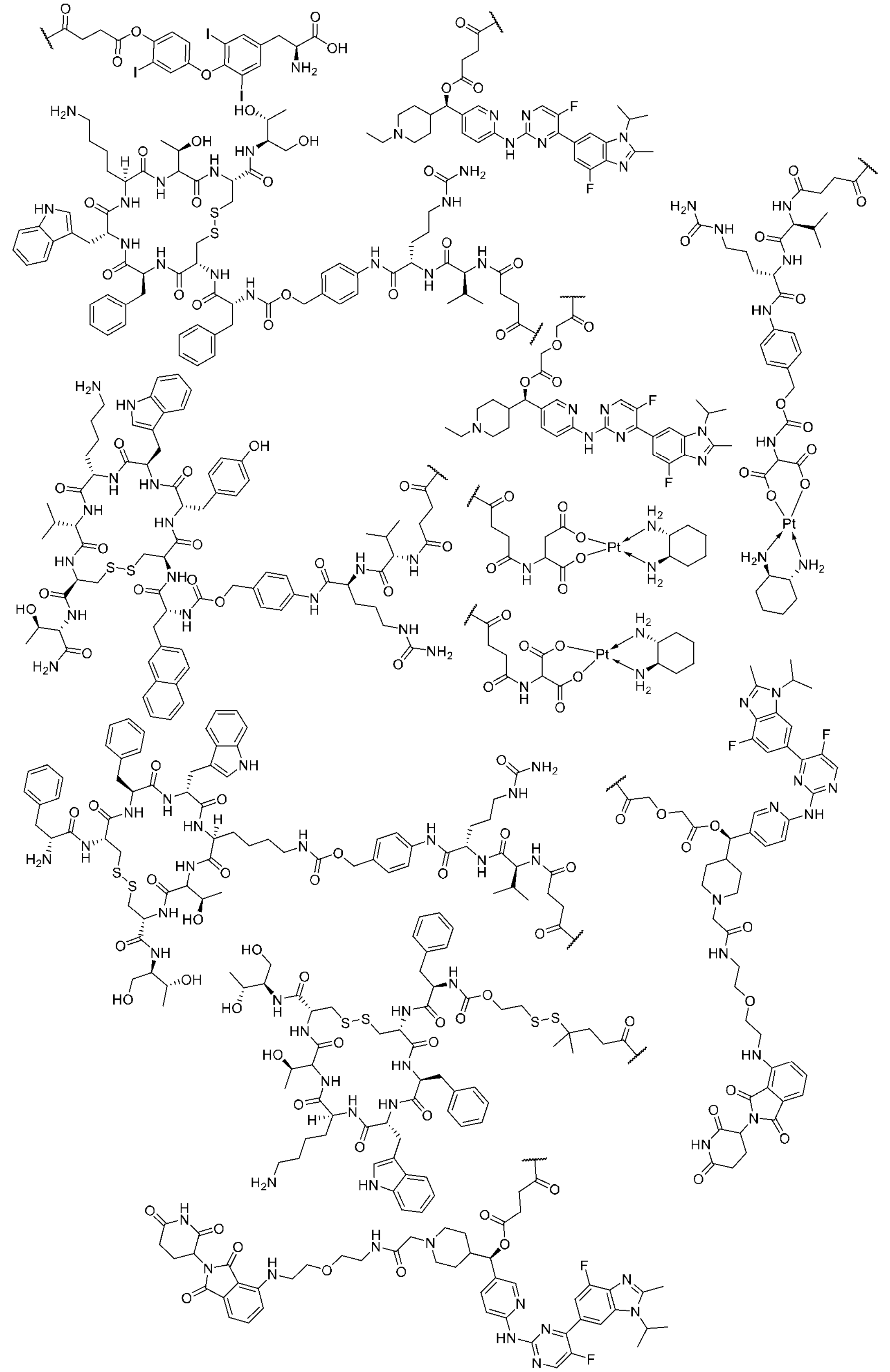

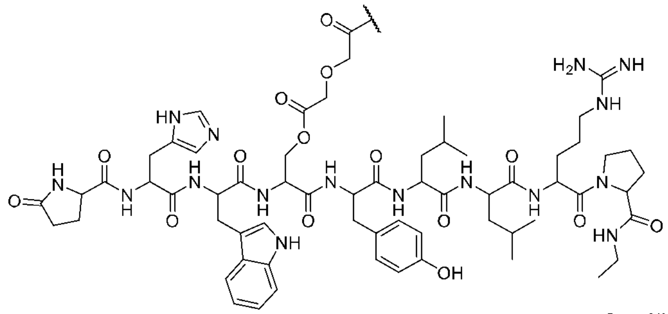

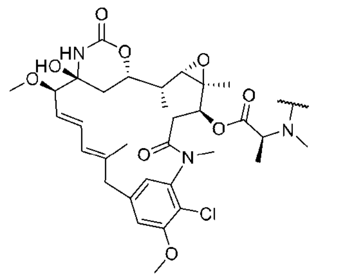

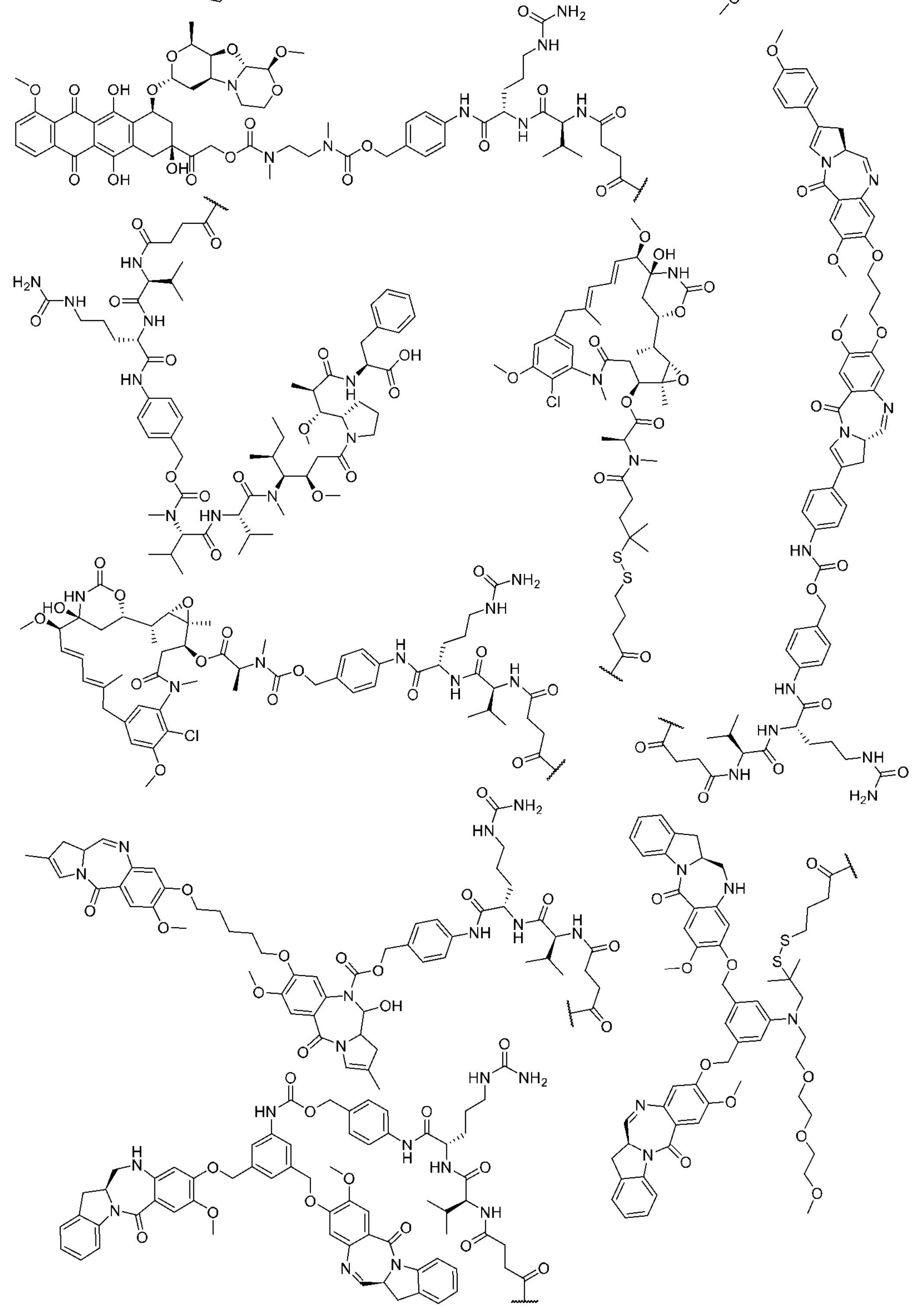

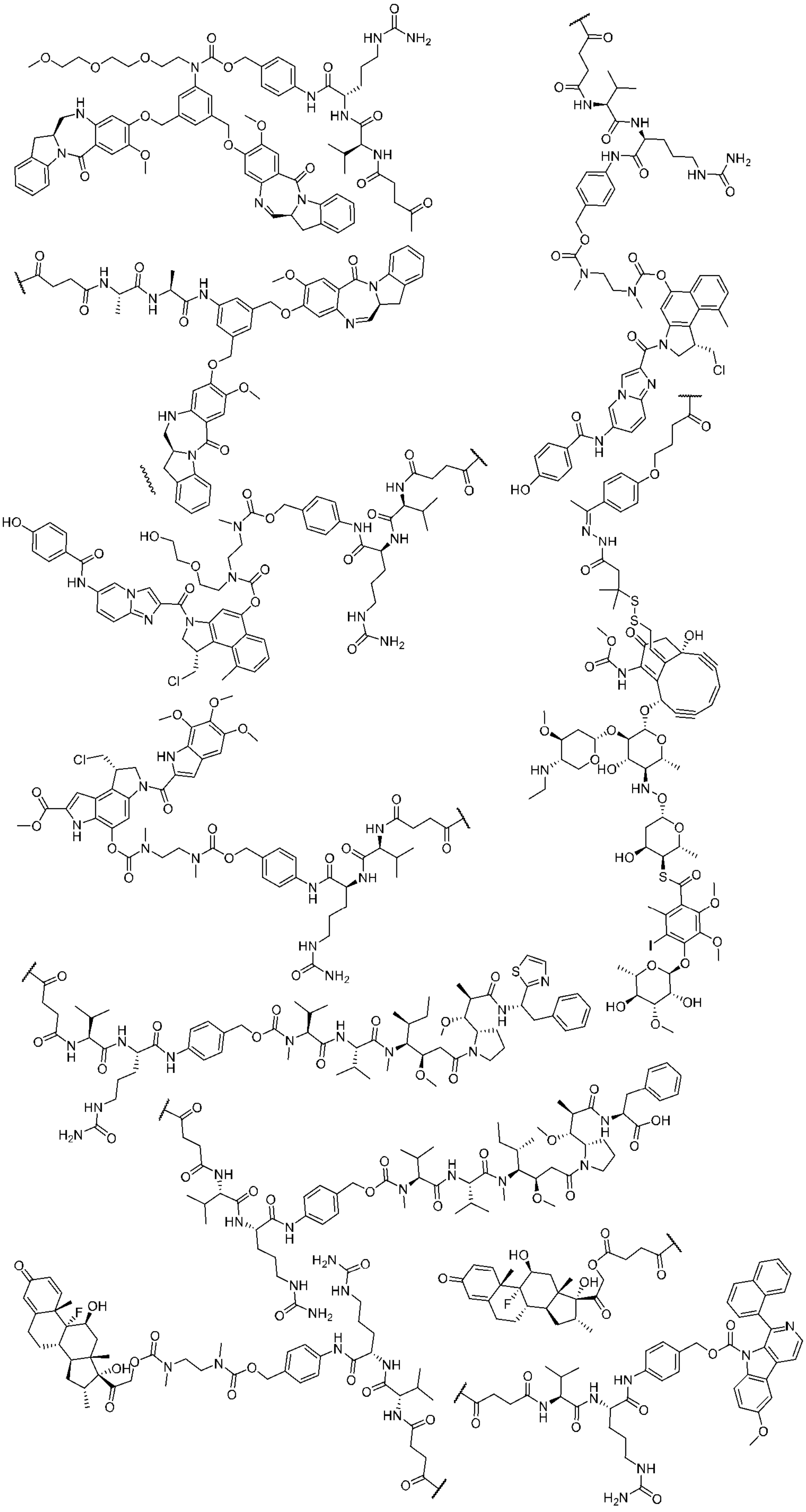

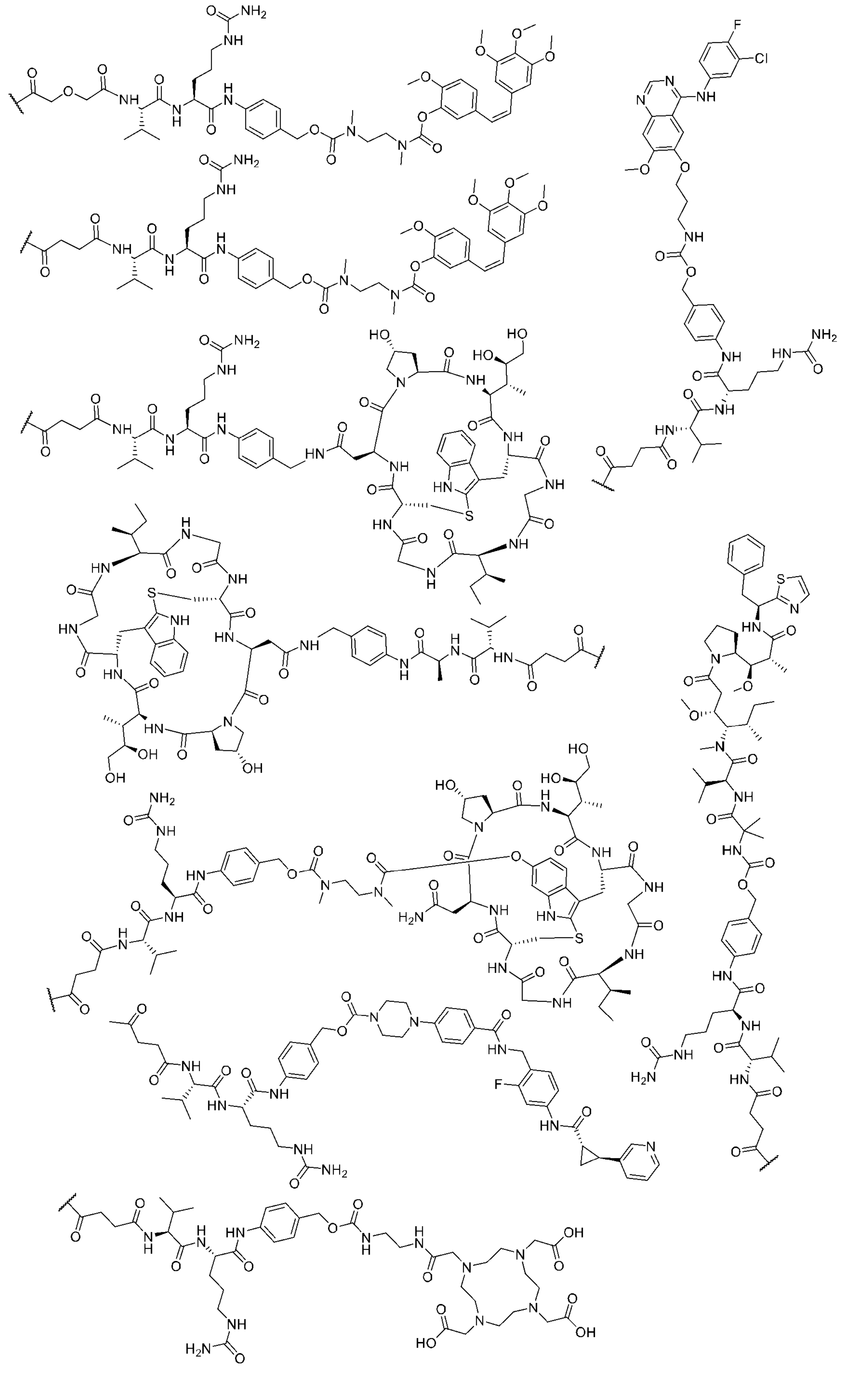

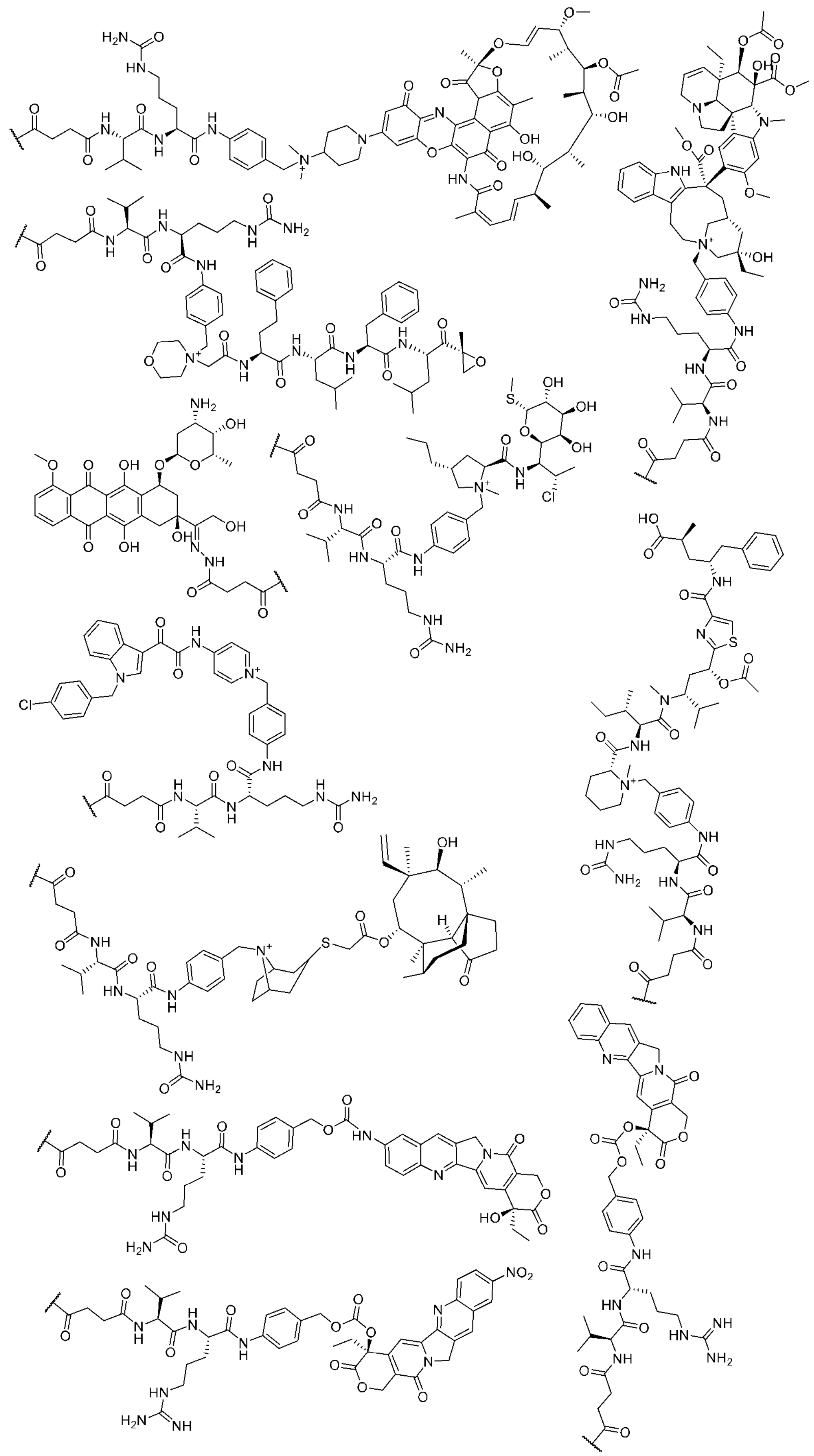

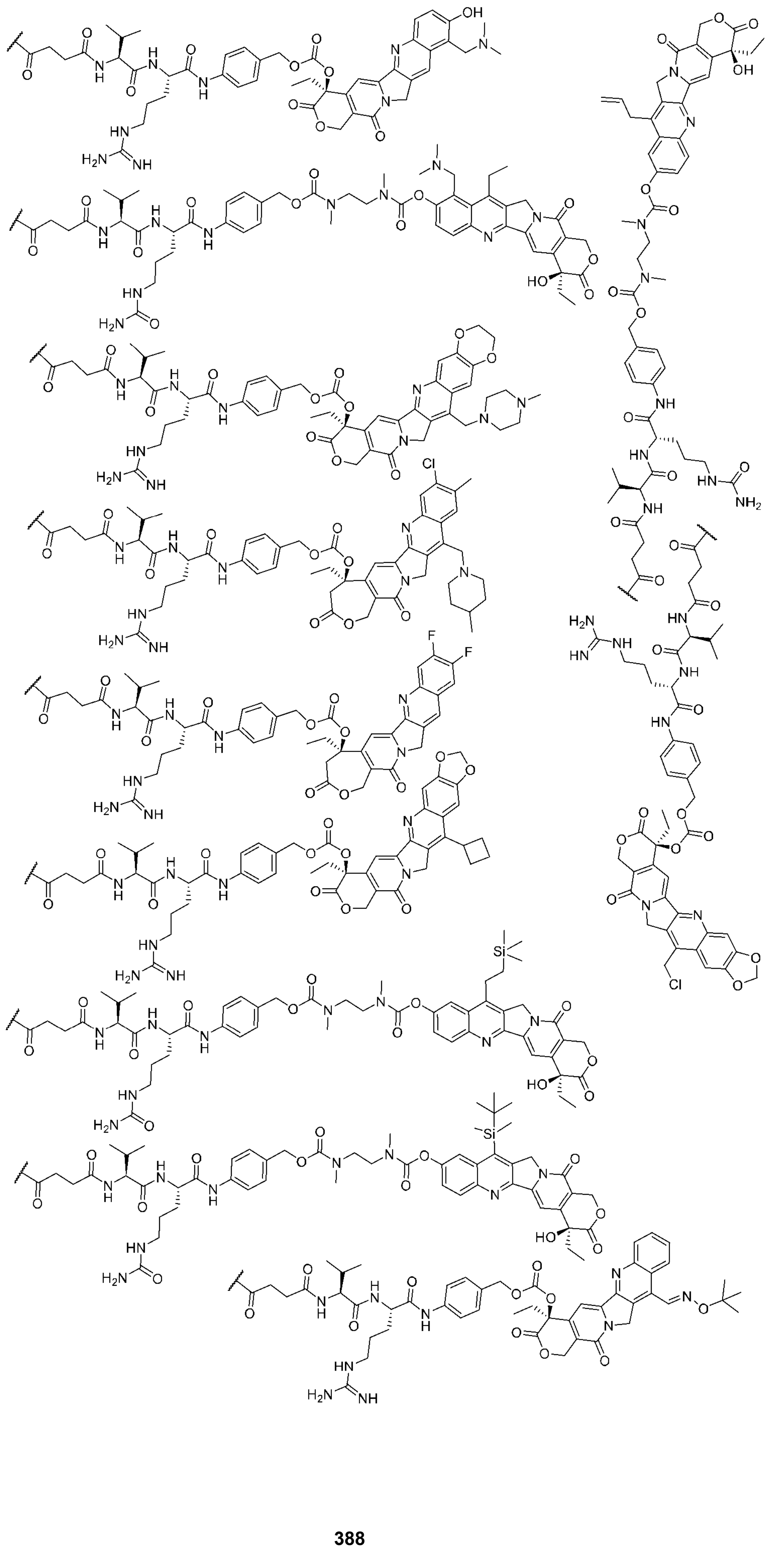

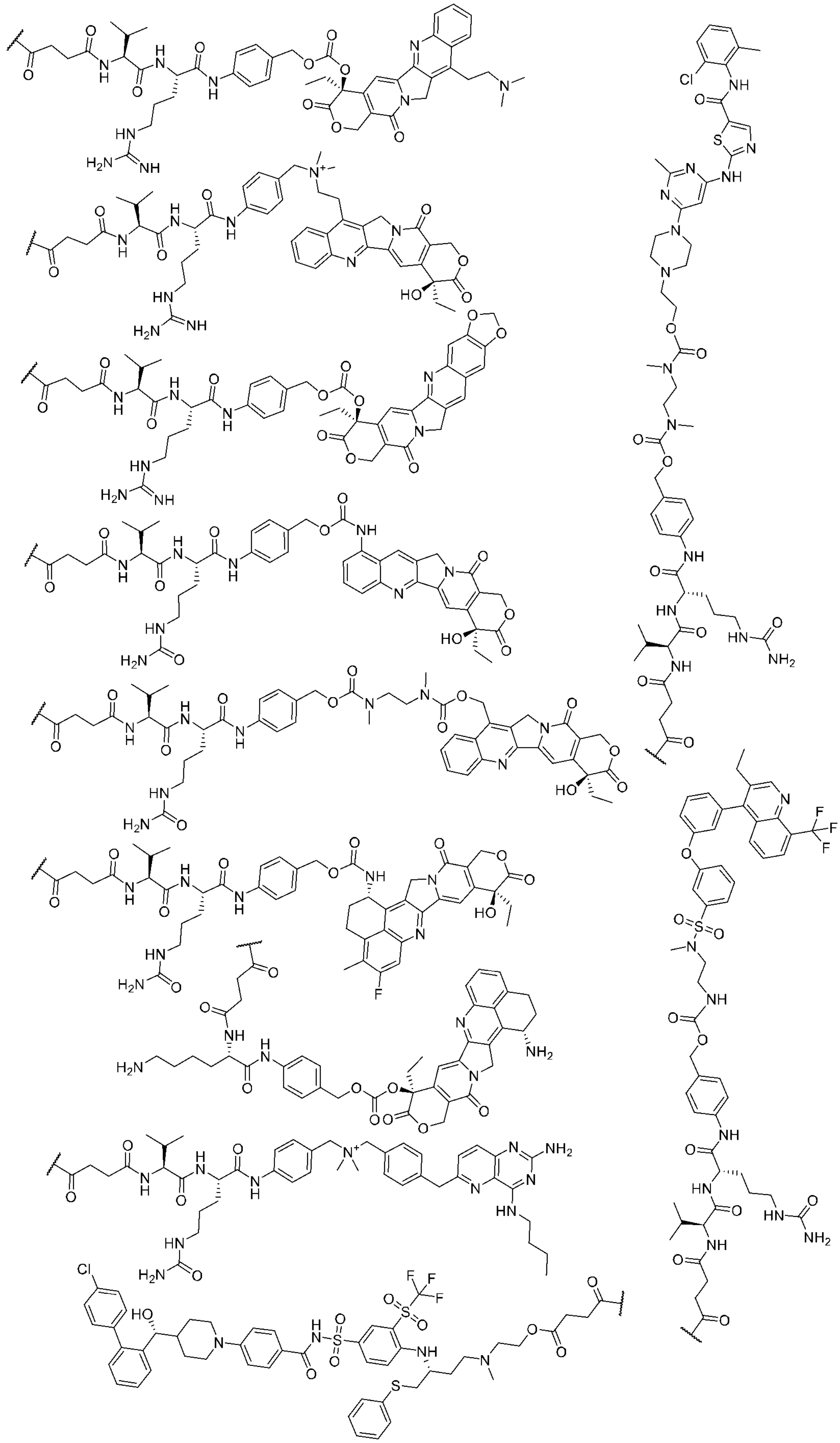

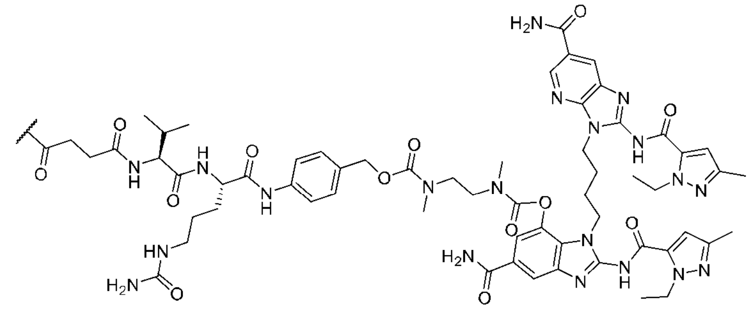

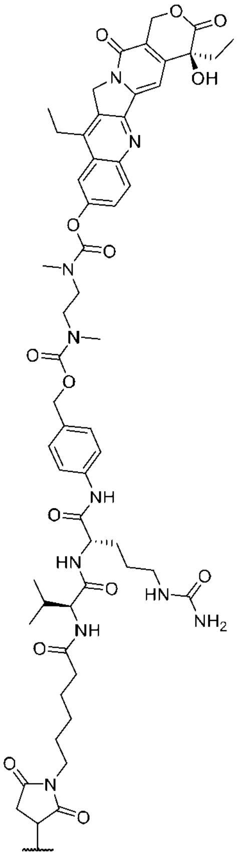

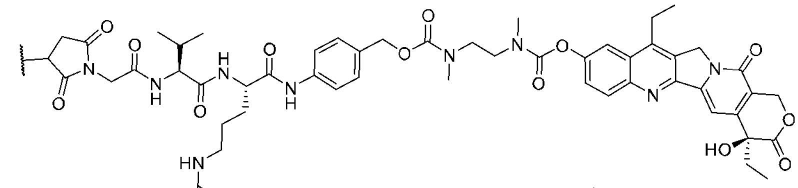

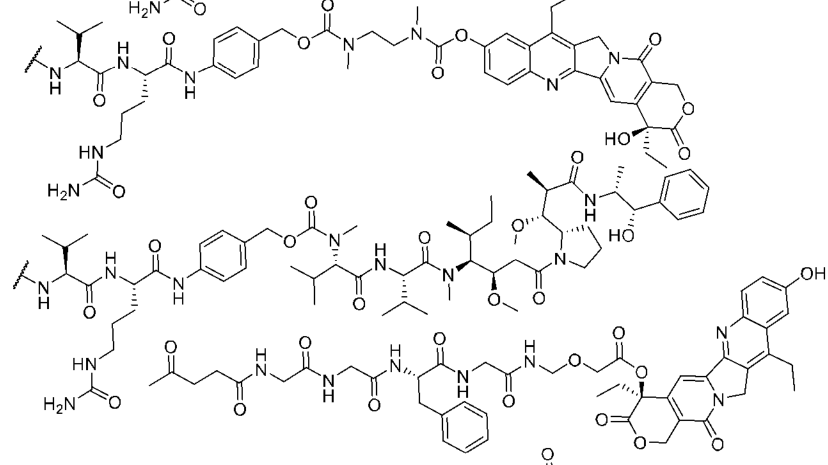

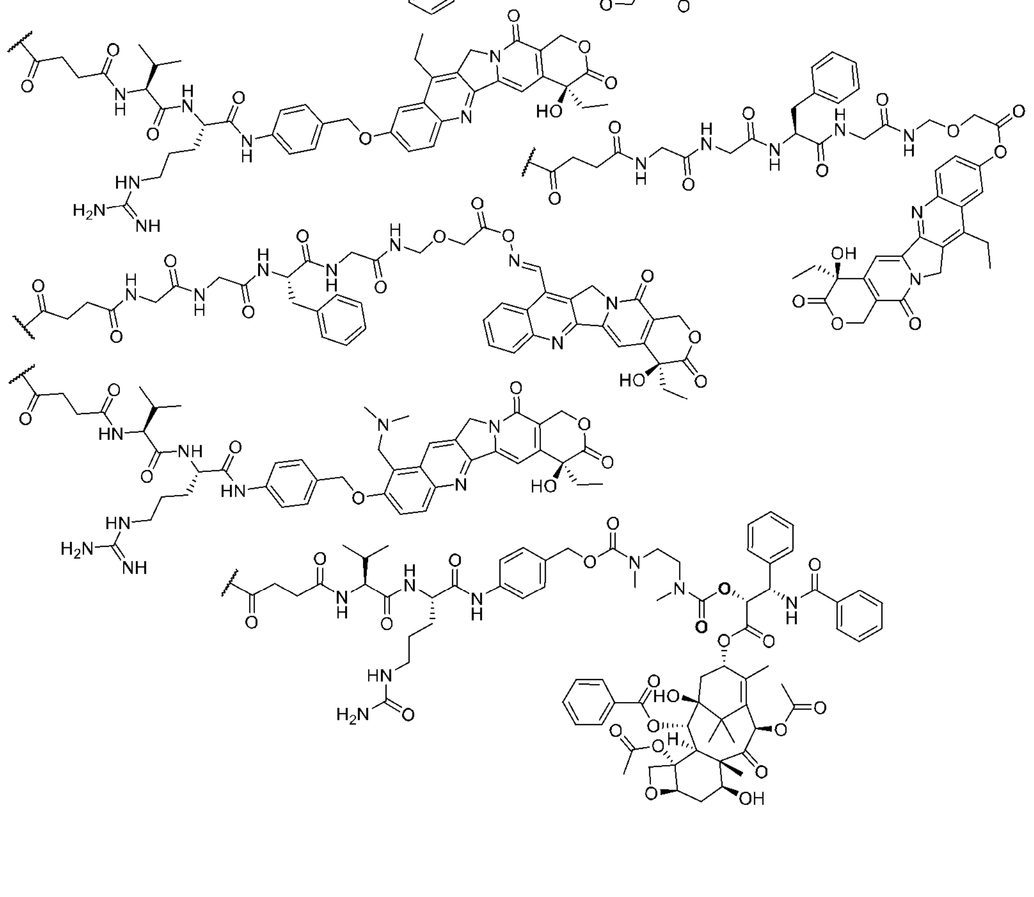

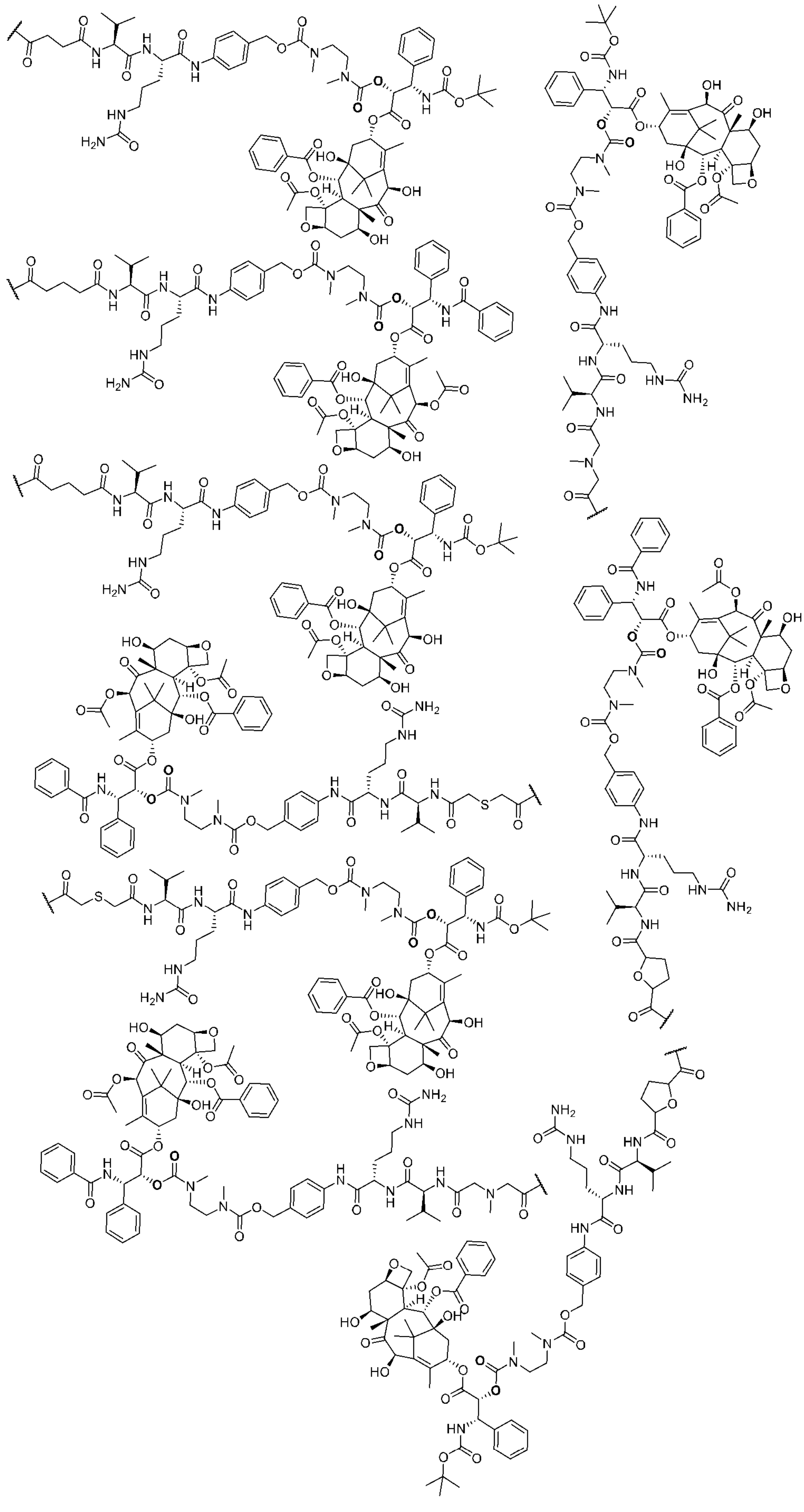

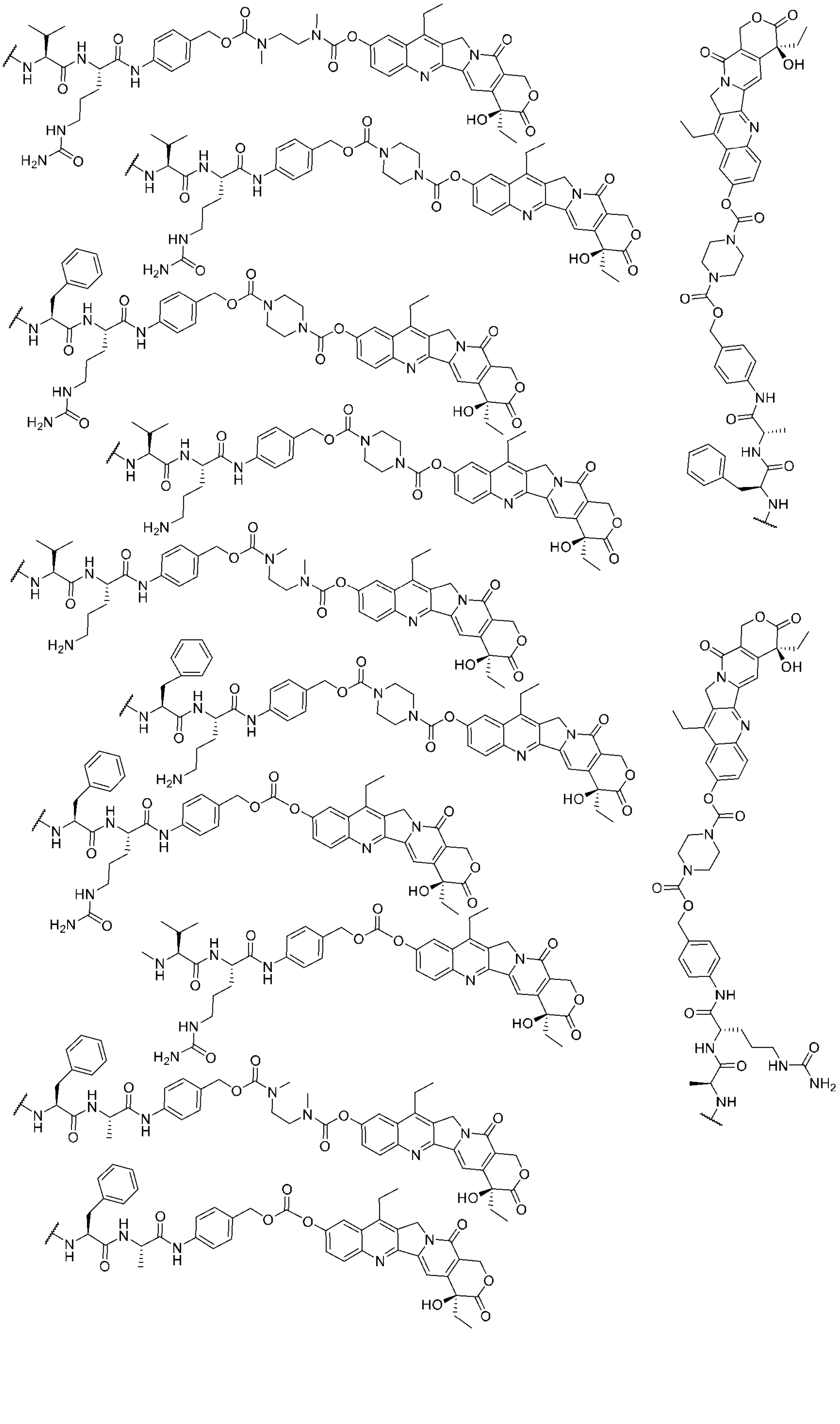

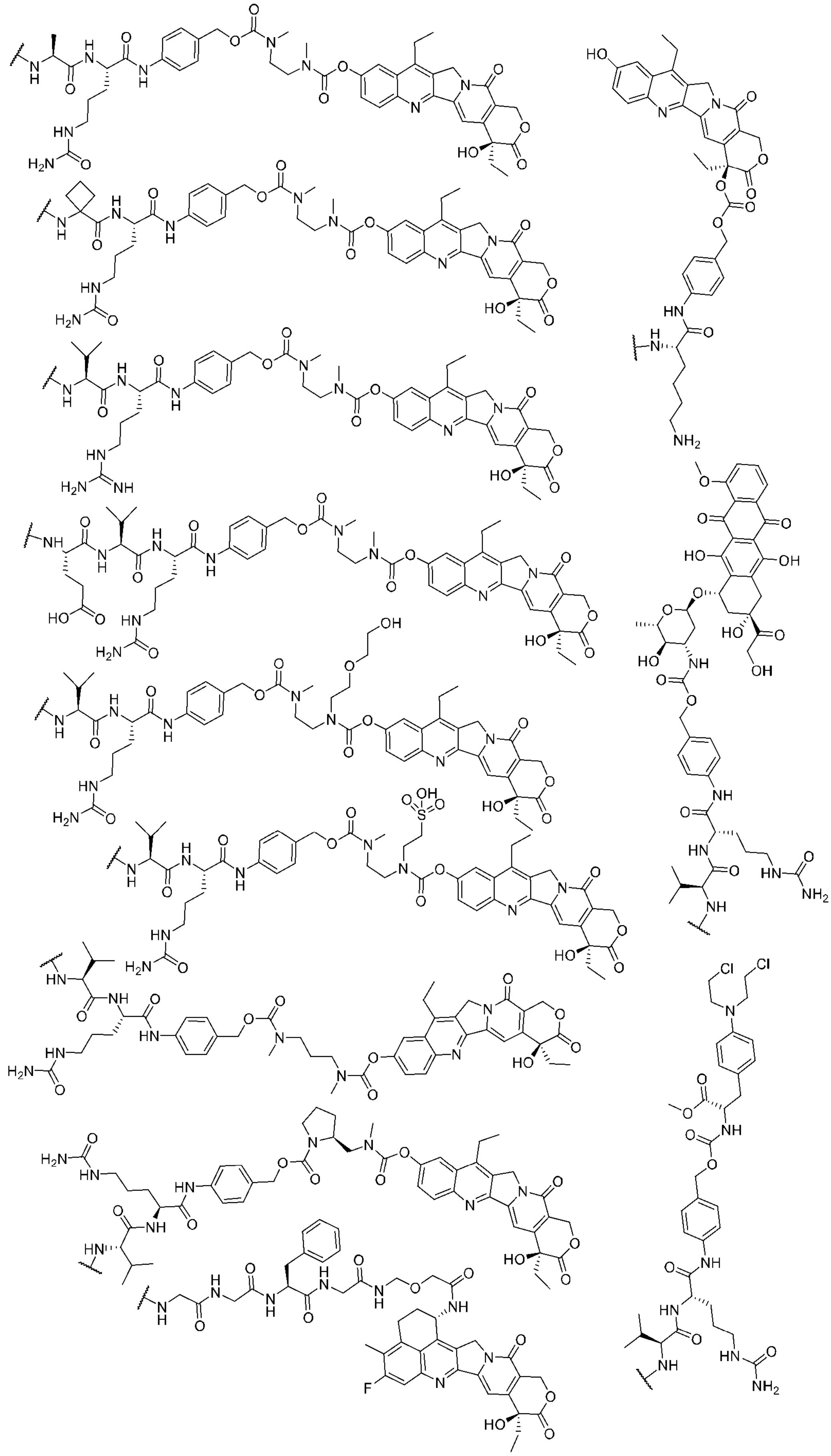

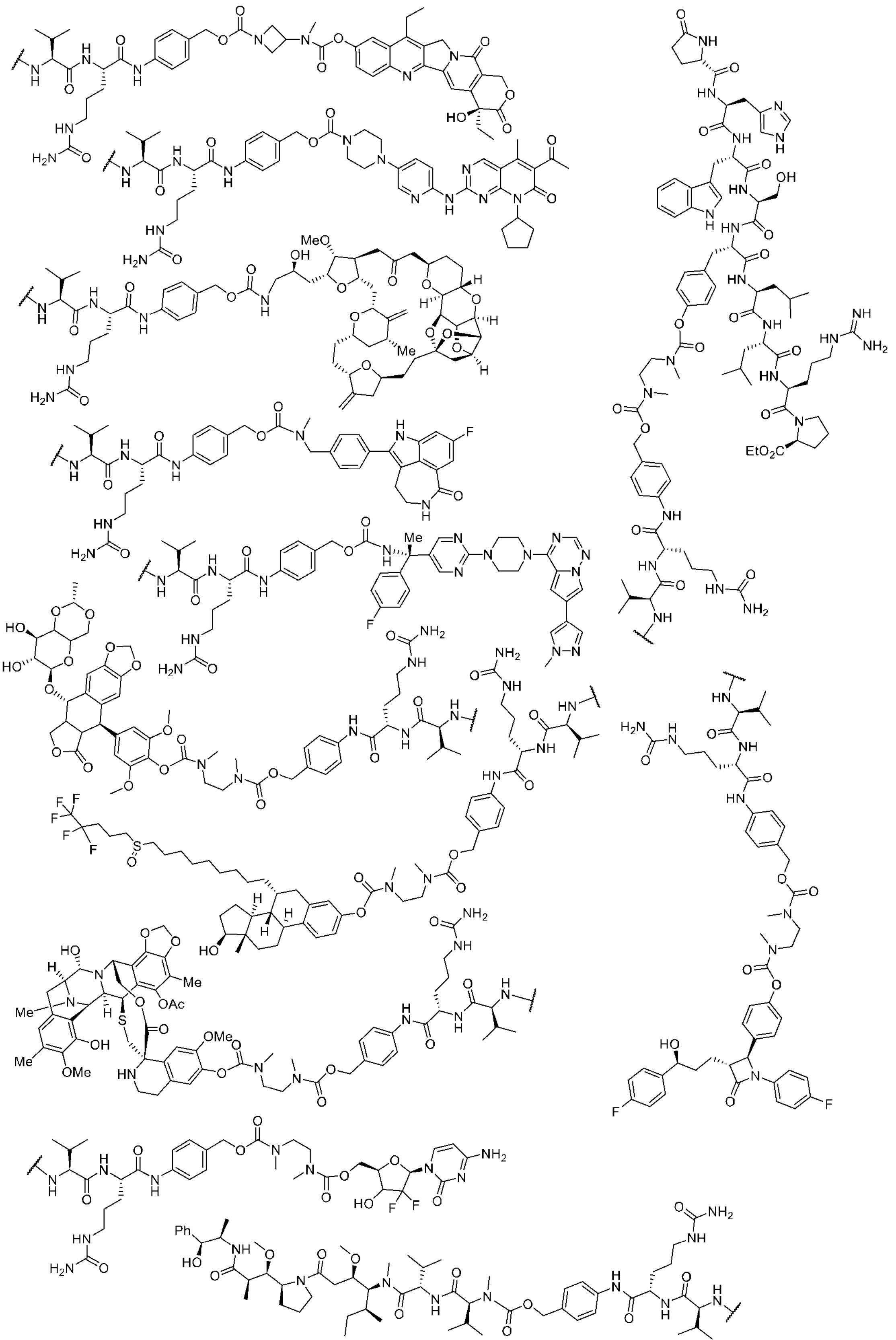

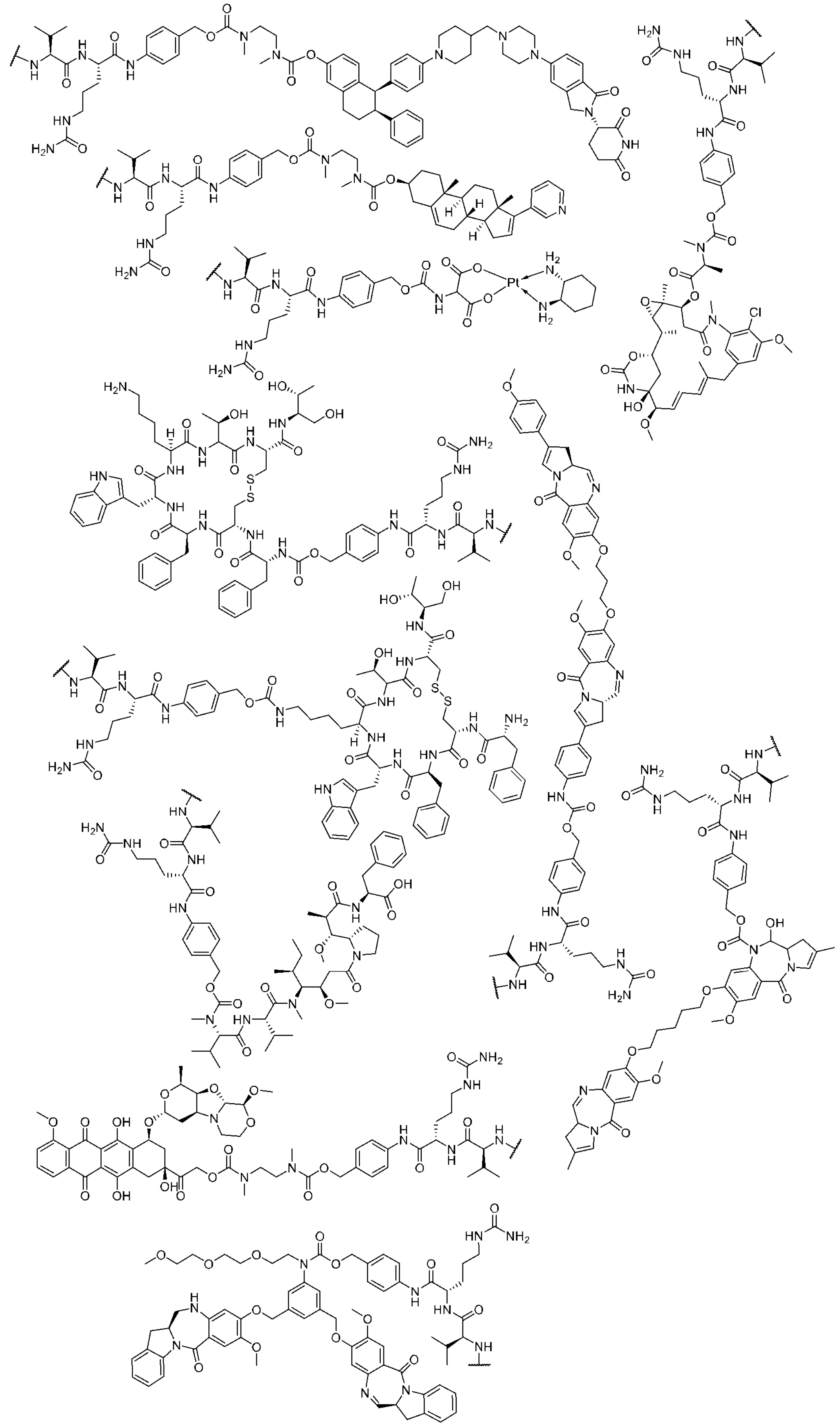

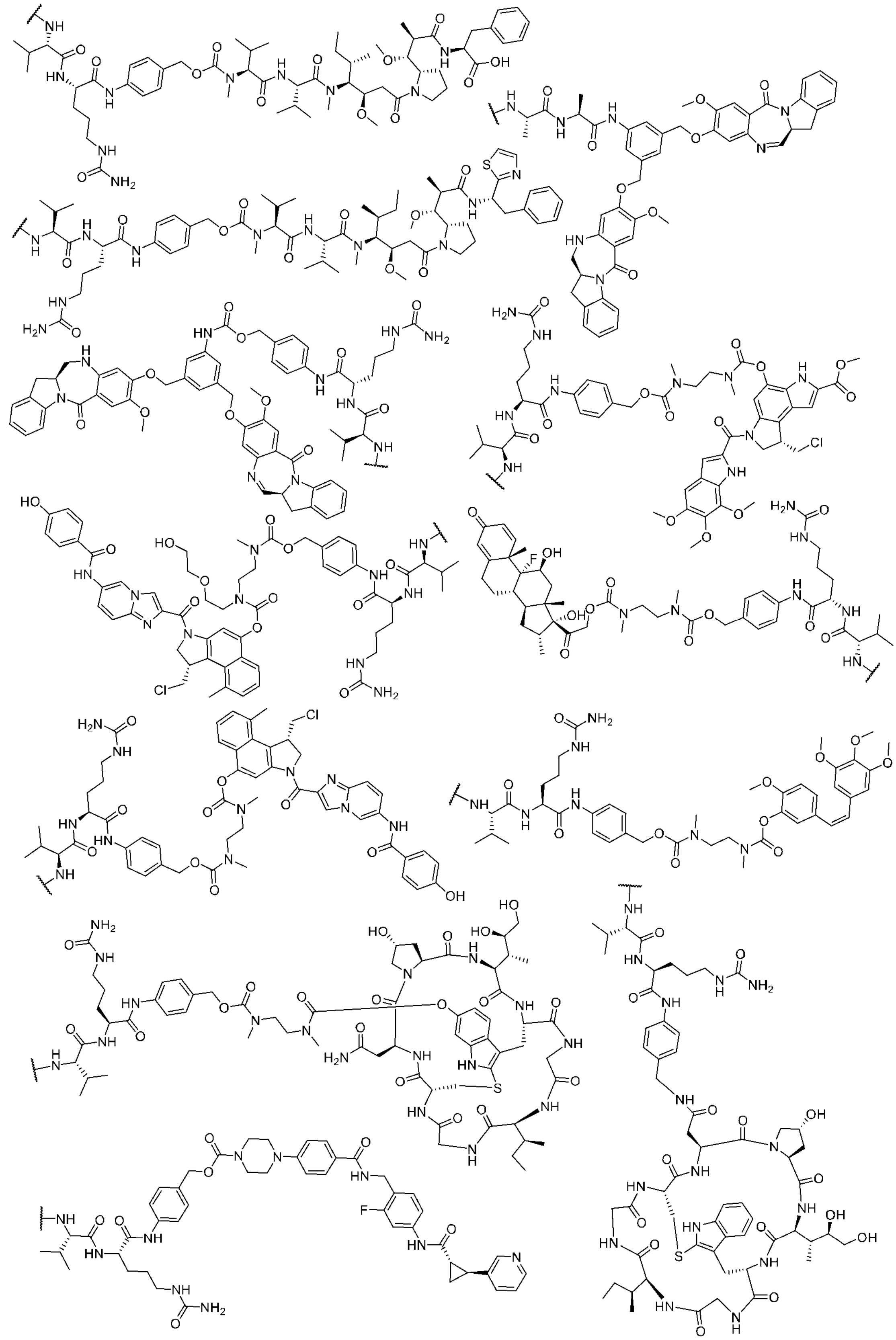

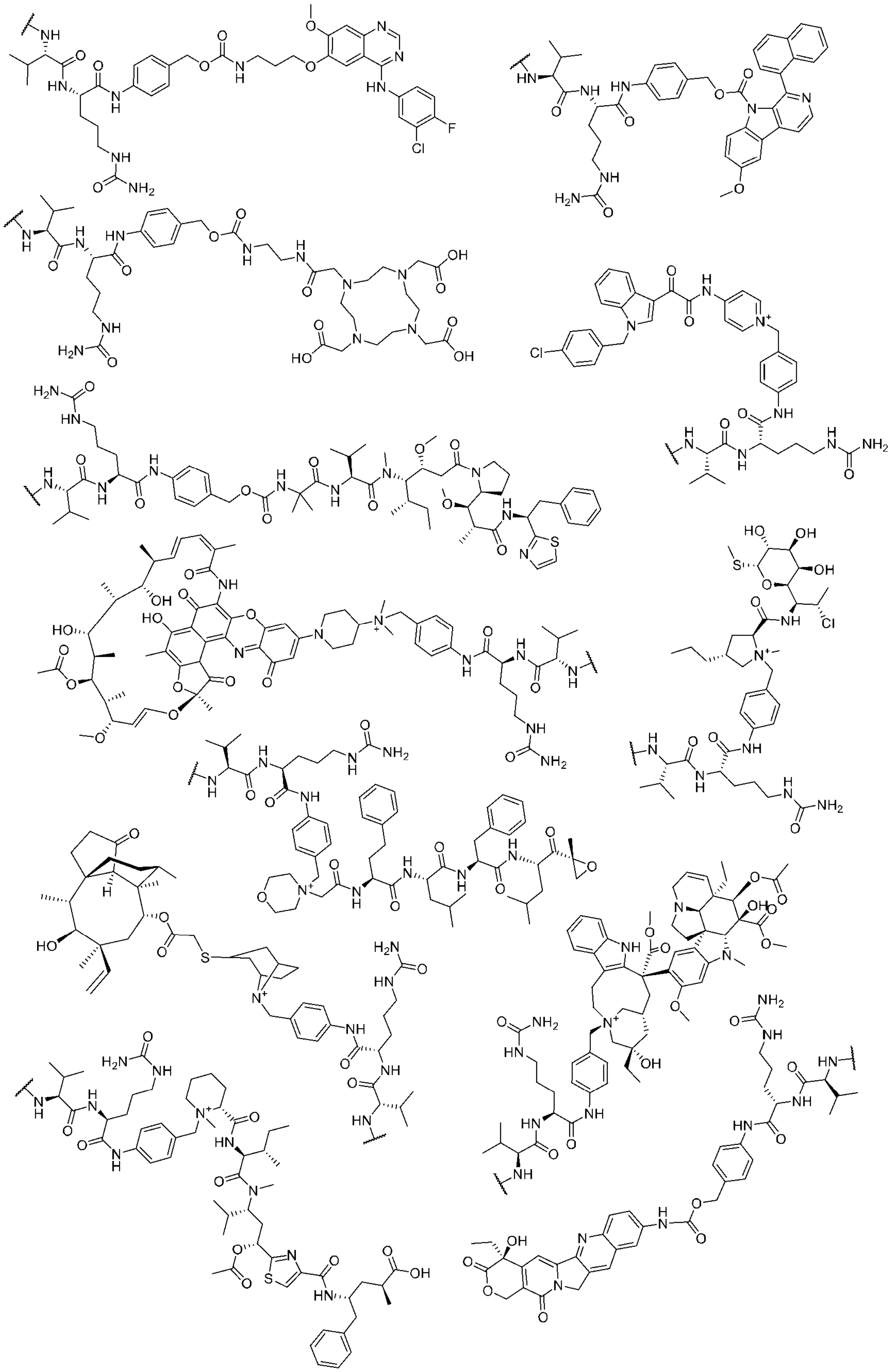

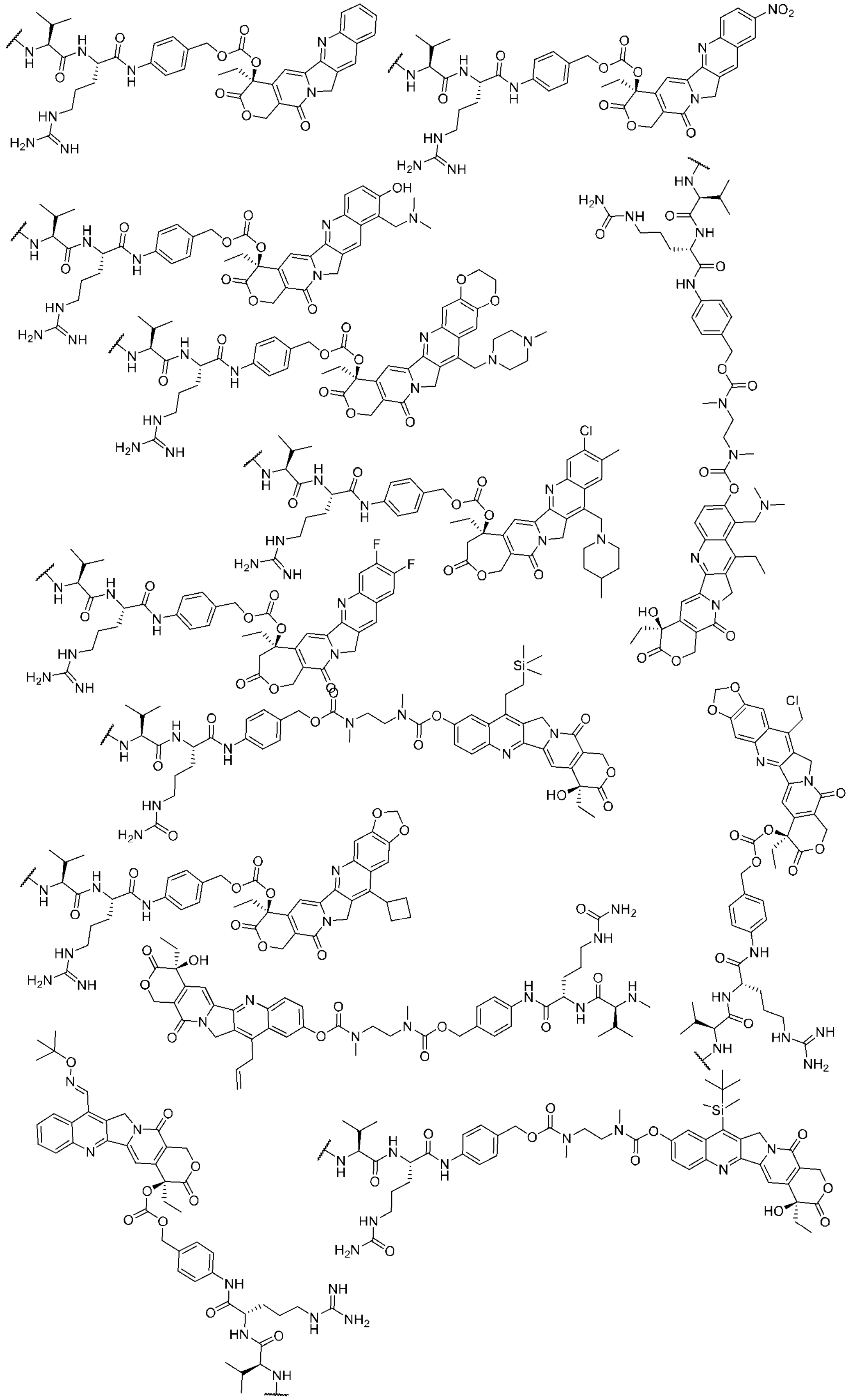

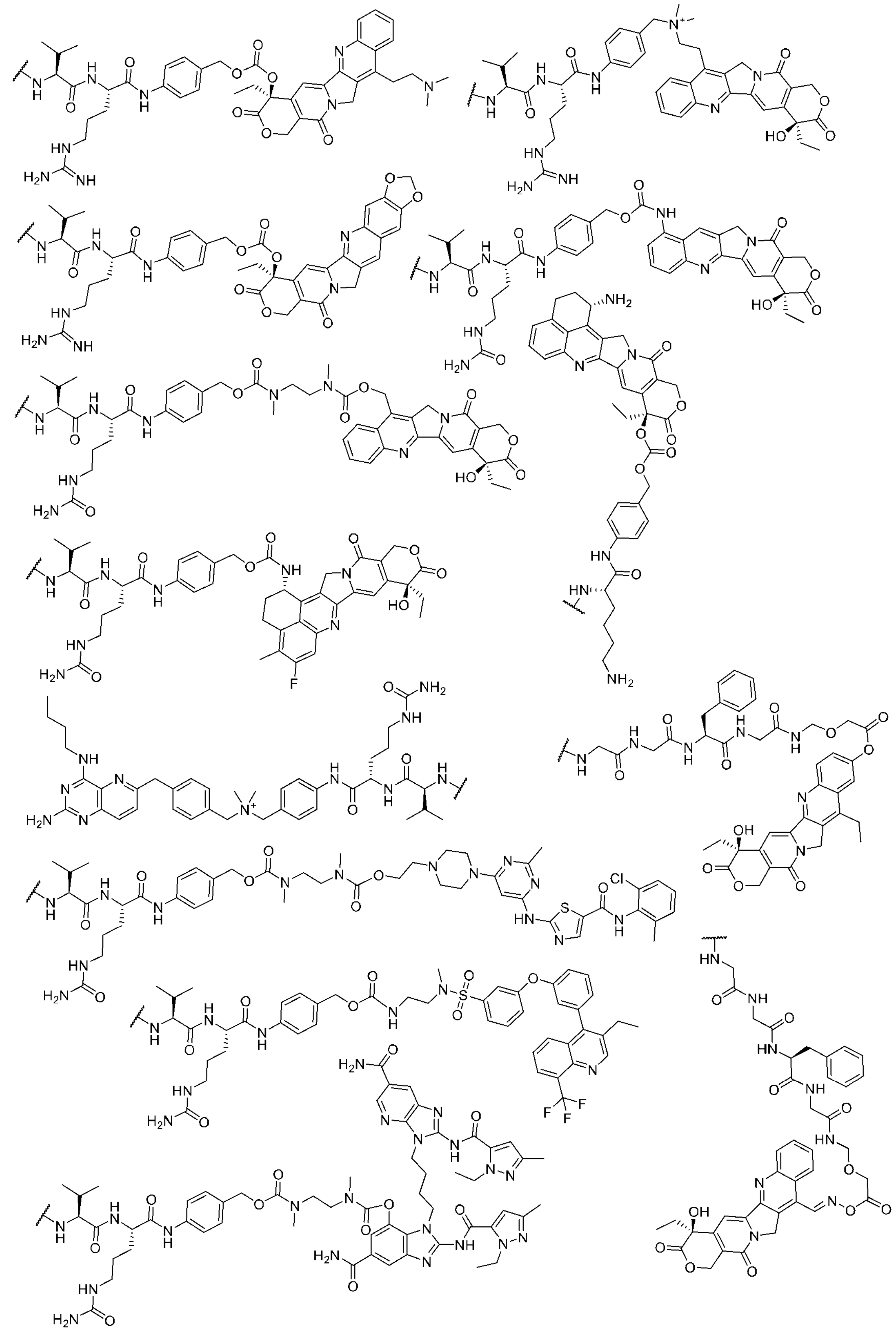

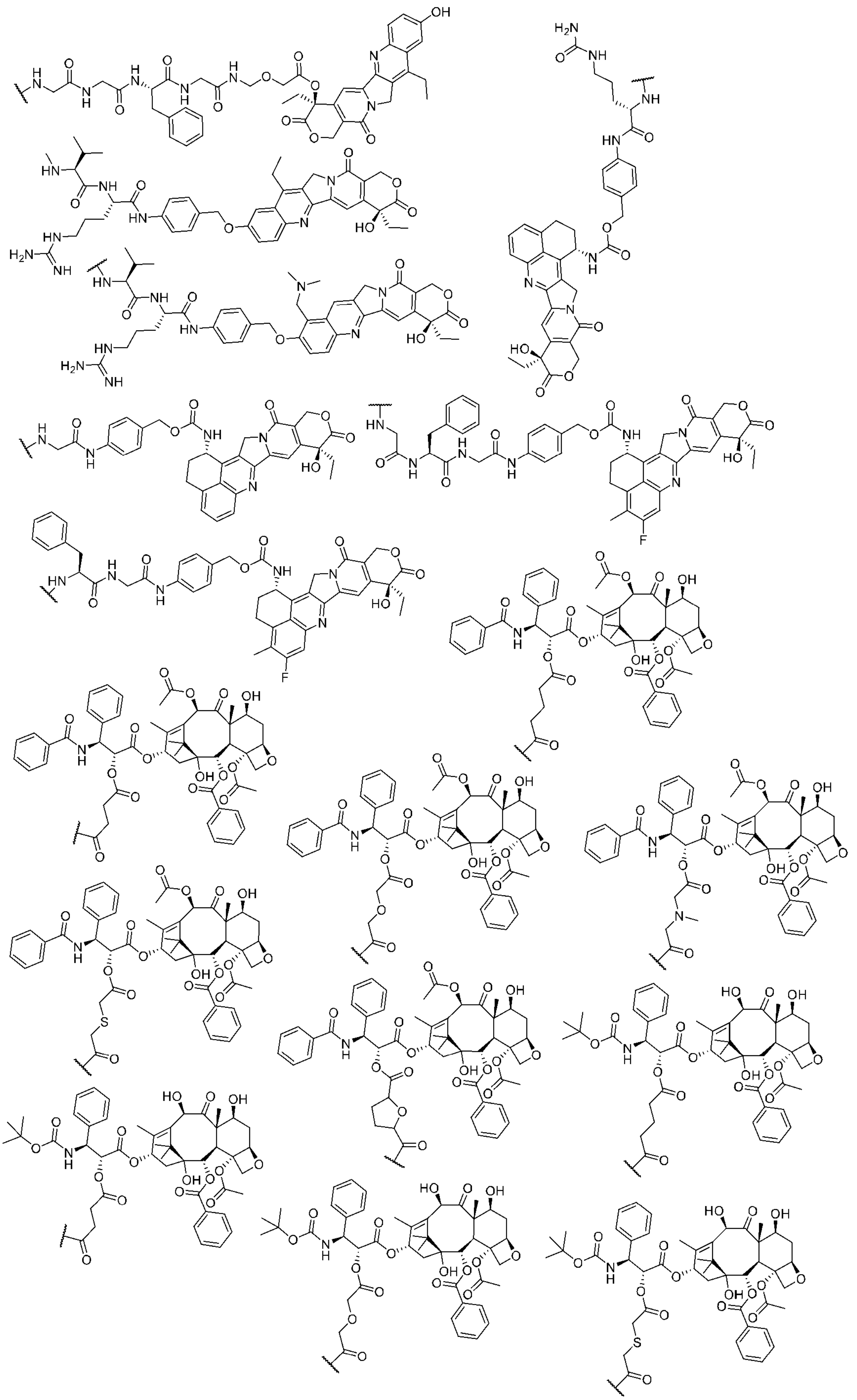

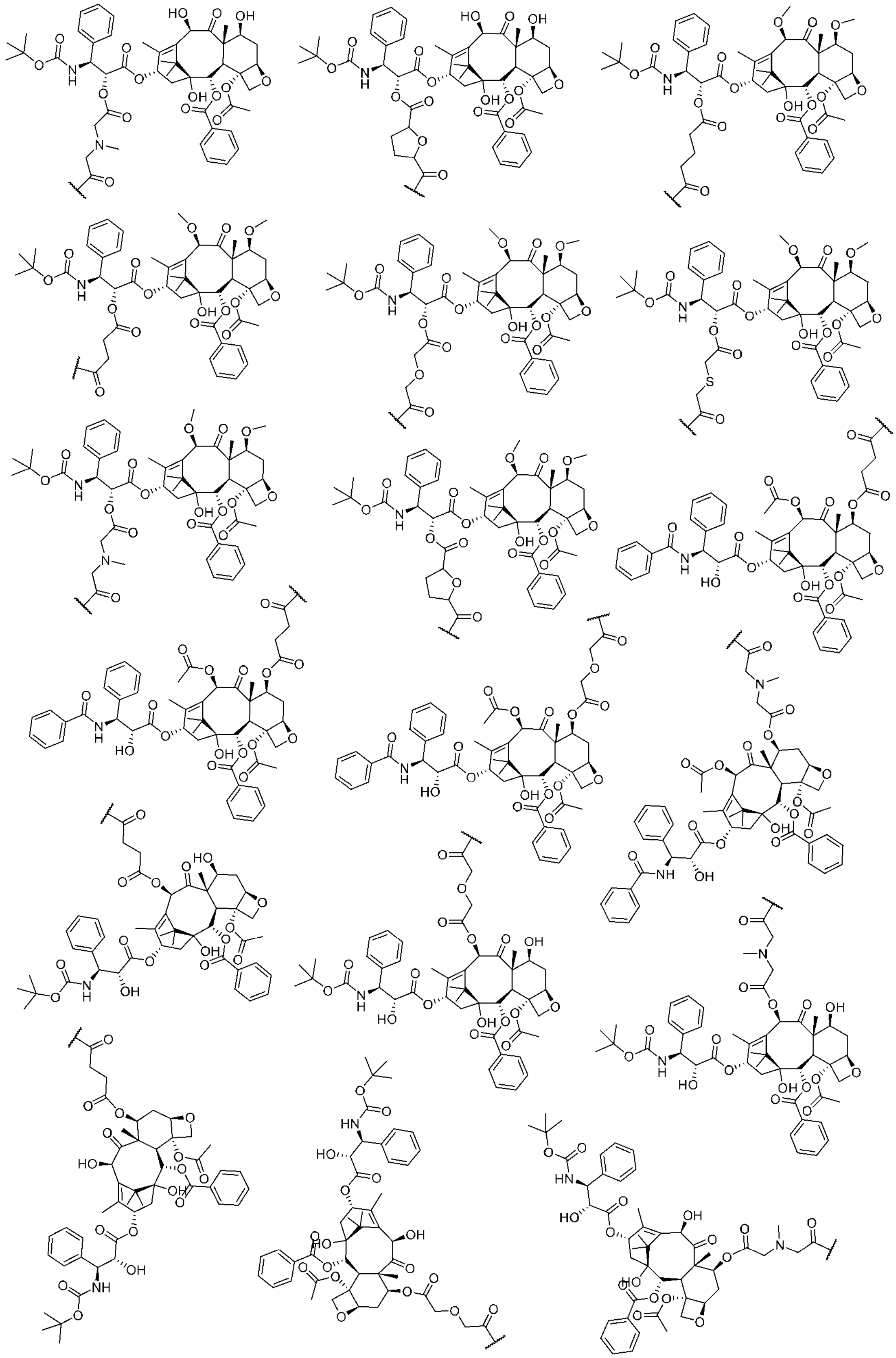

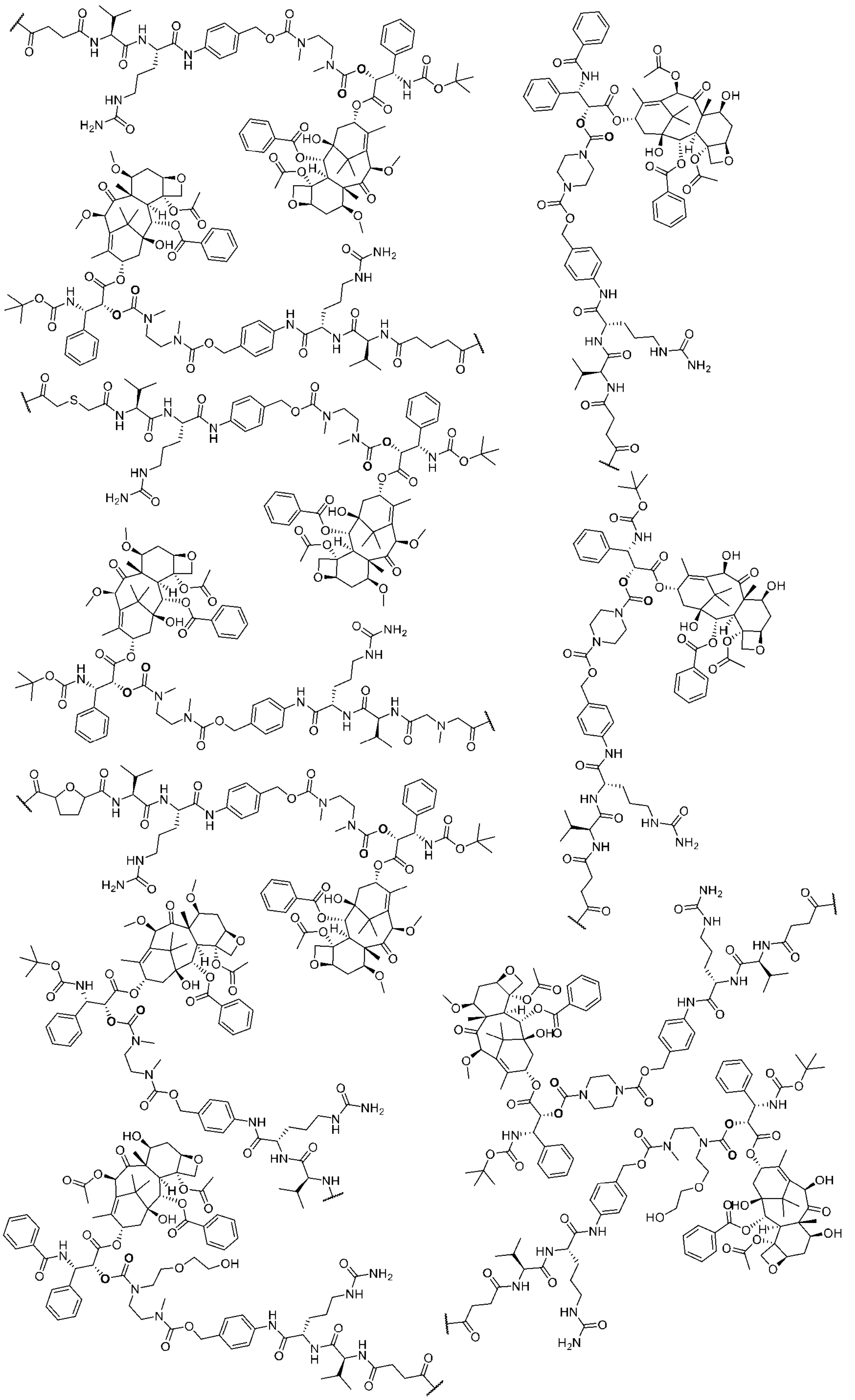

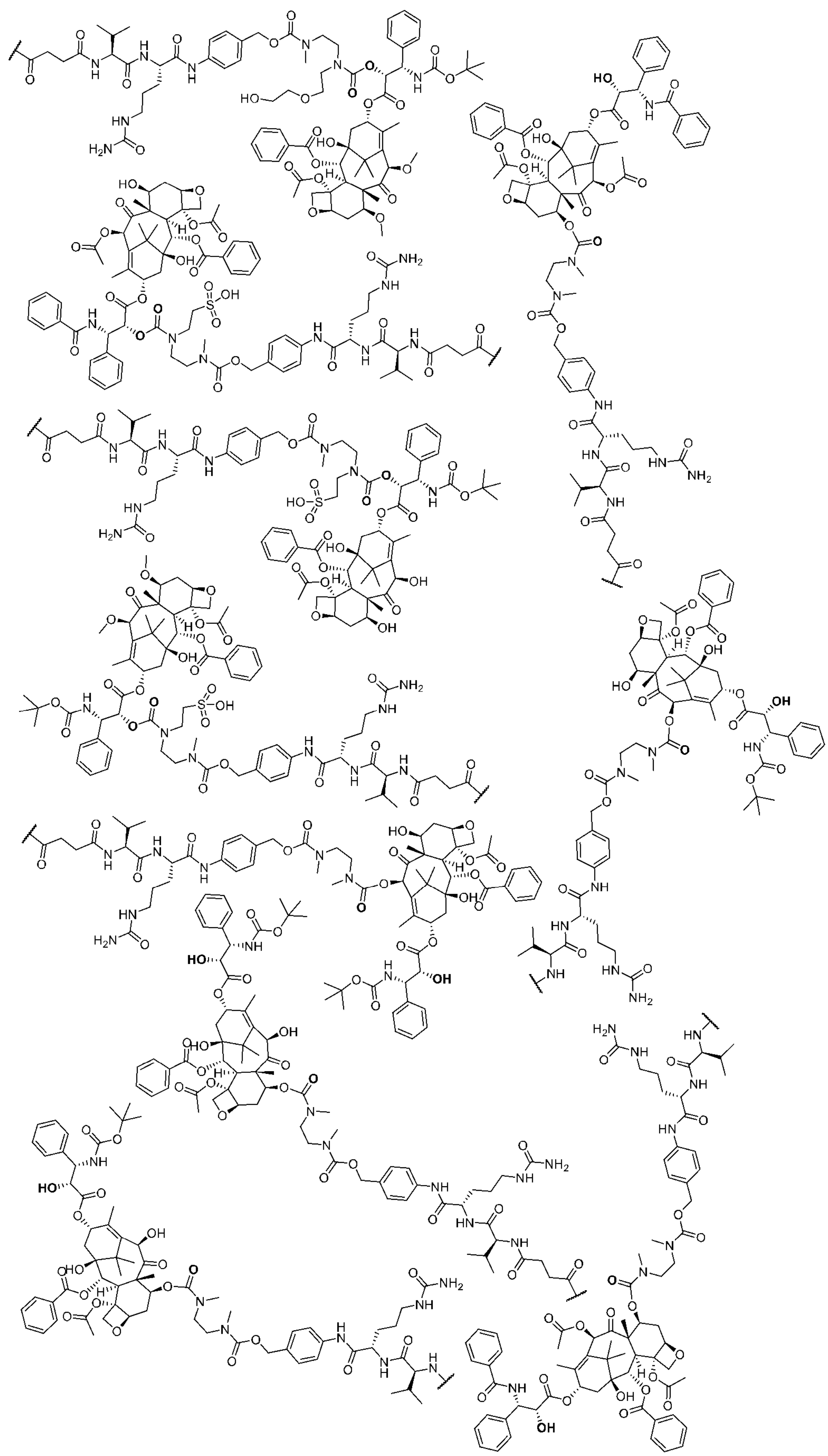

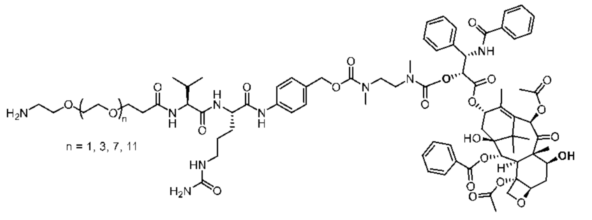

n = 1, 3, 7, 11

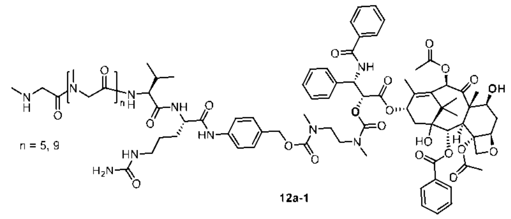

n = 5, 9

12a-1

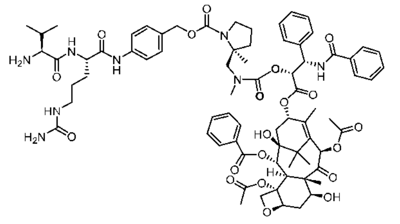

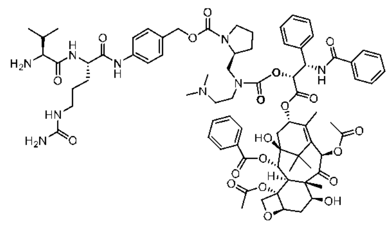

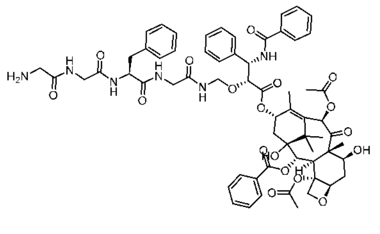

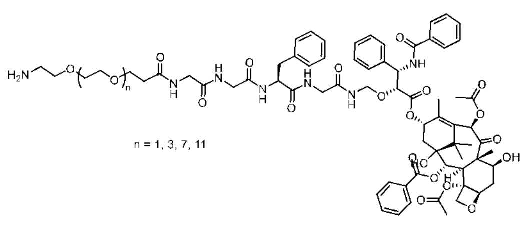

n = 1, 3, 7, 11

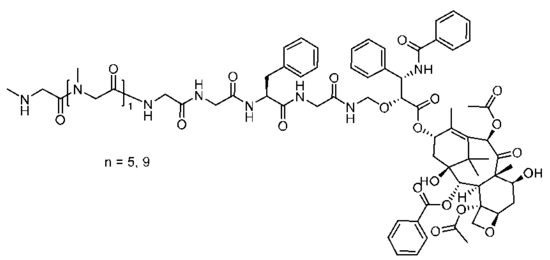

n = 5, 9

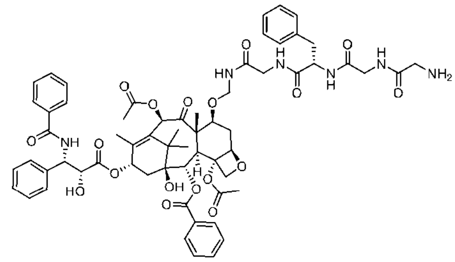

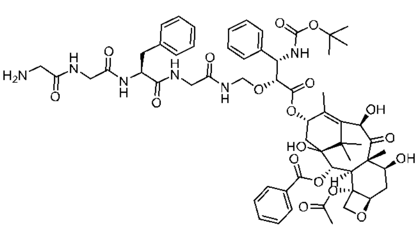

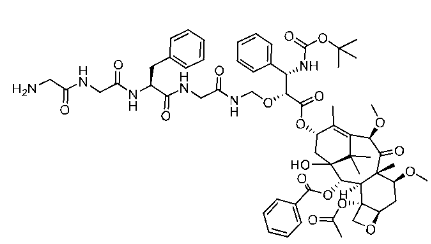

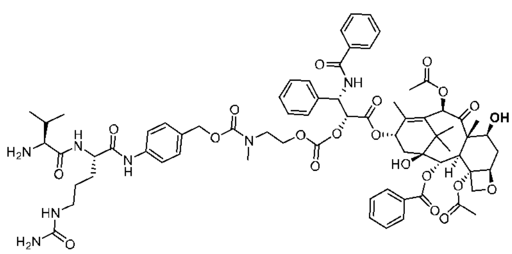

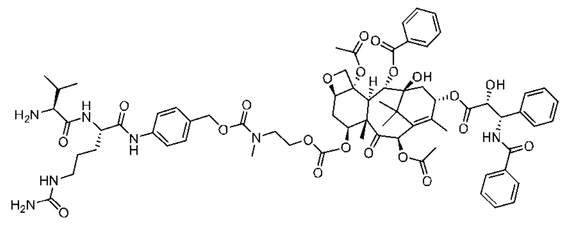

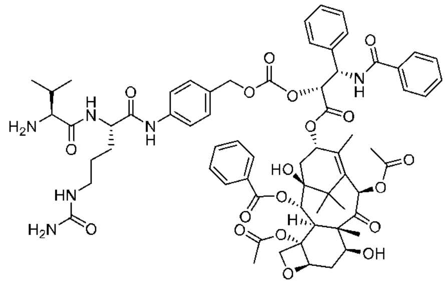

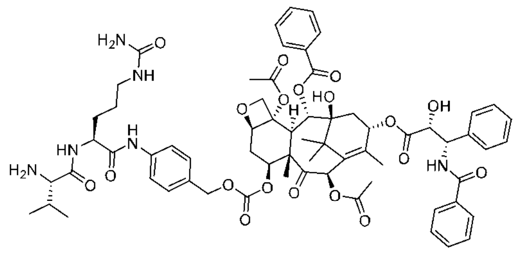

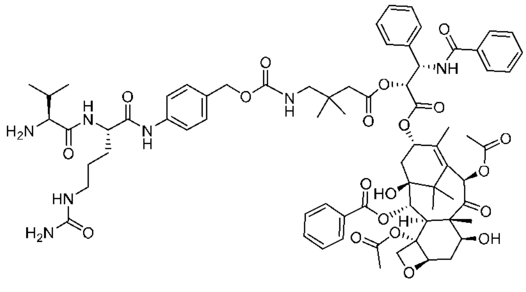

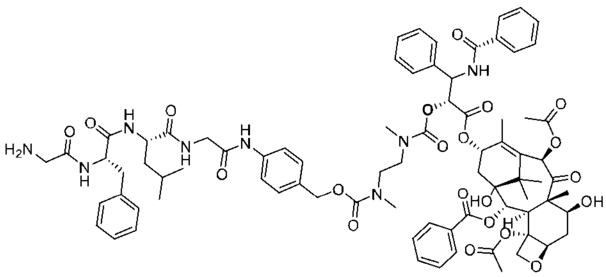

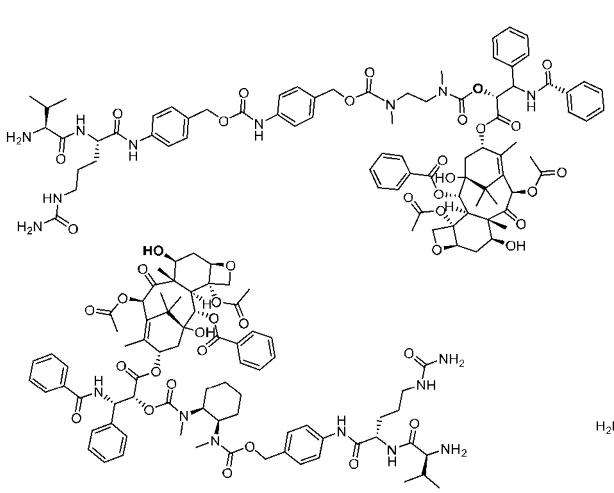

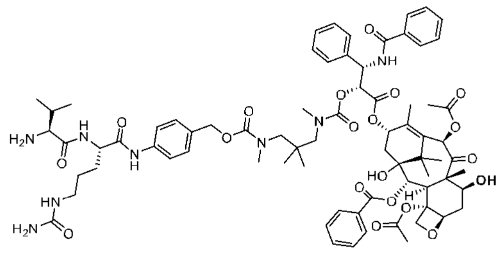

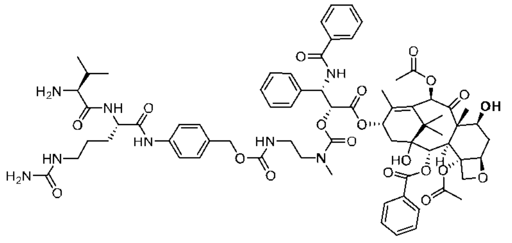

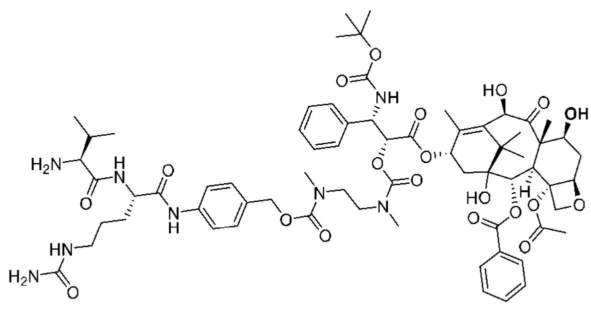

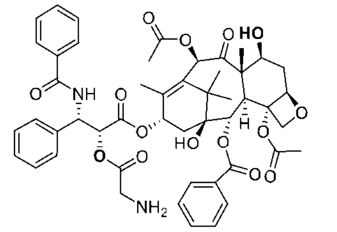

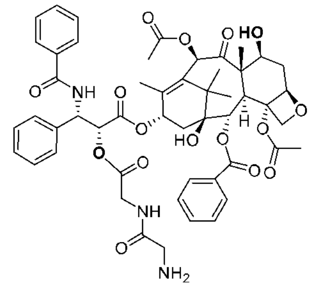

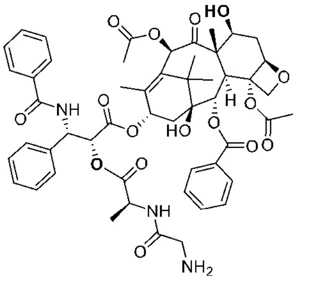

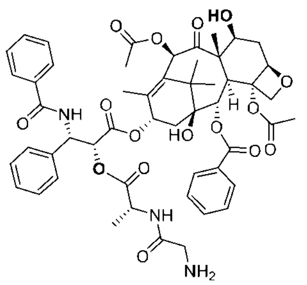

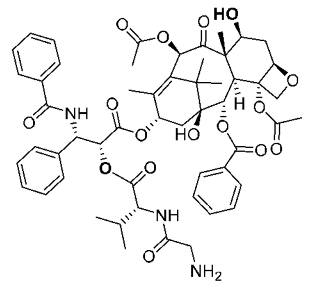

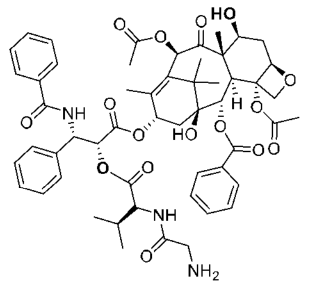

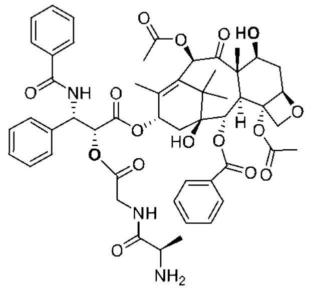

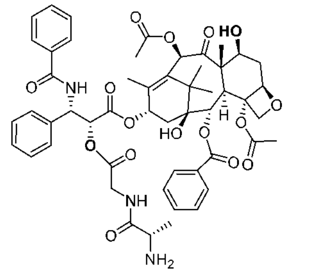

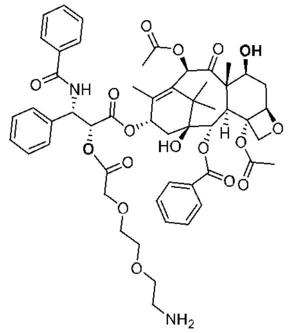

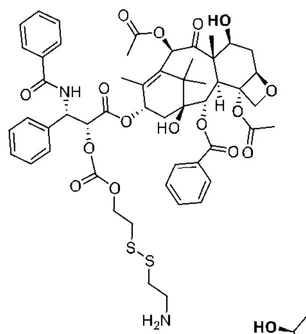

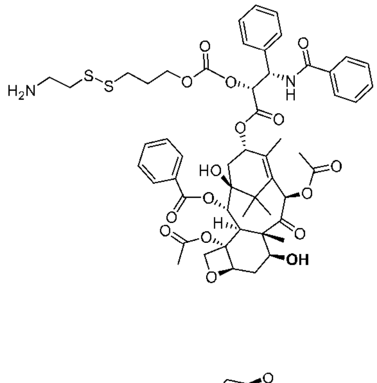

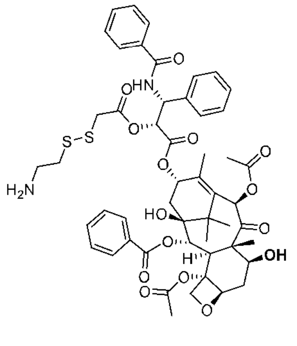

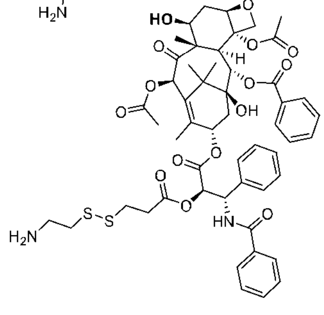

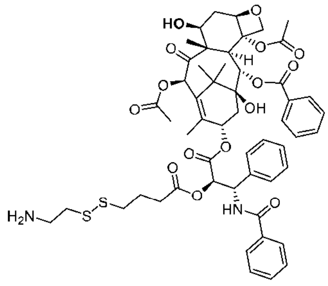

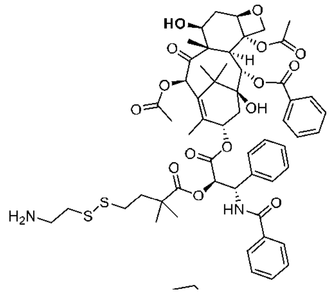

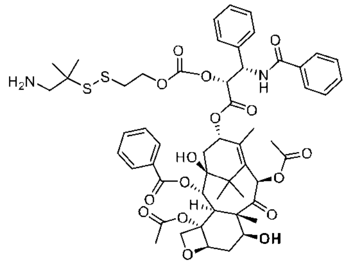

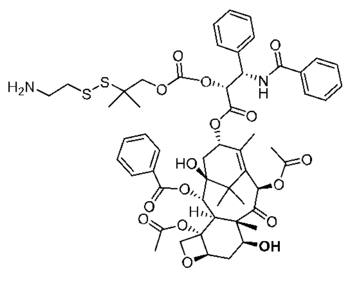
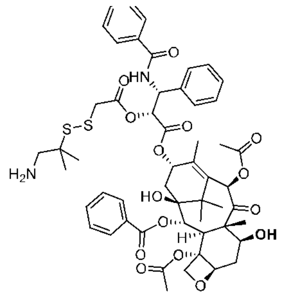
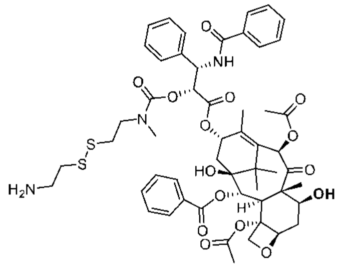

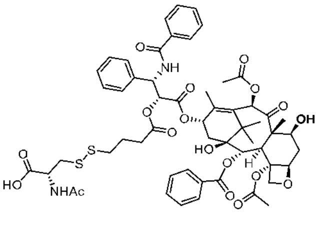
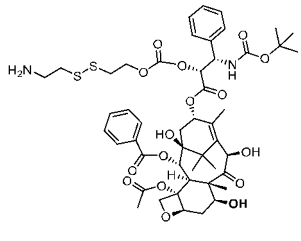
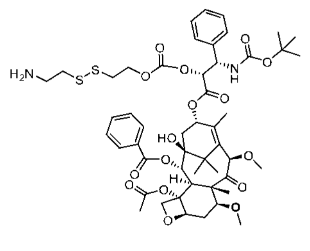

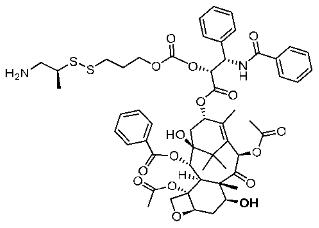
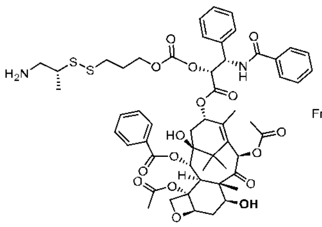
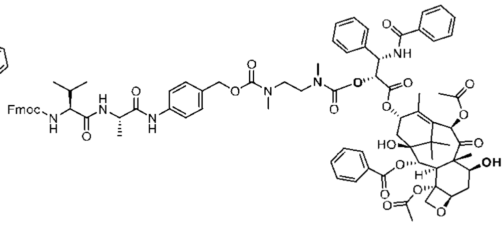

condition 2: $-L^2-D$ is any one of the following structures:

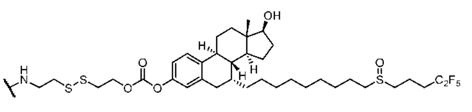
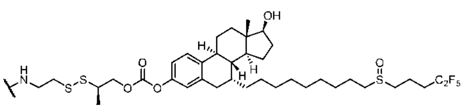

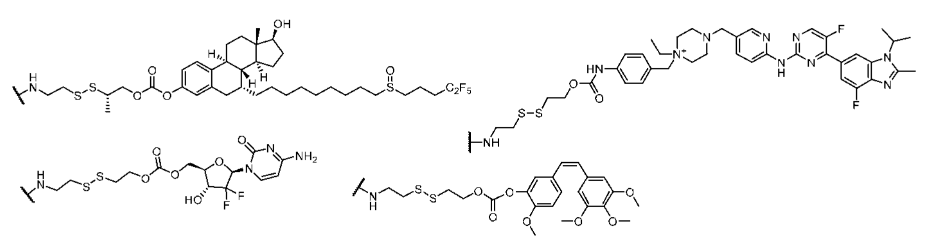

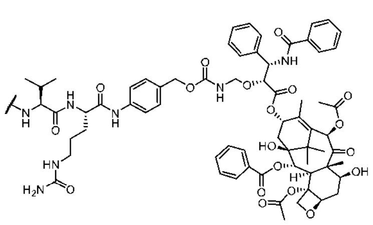
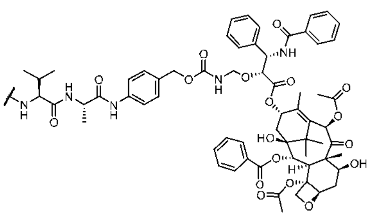
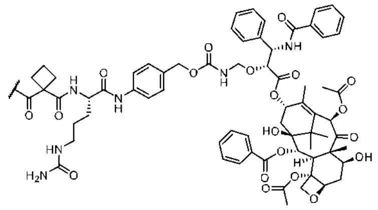

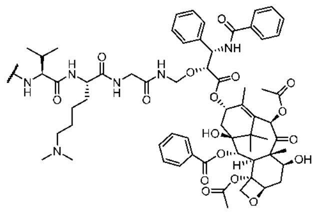
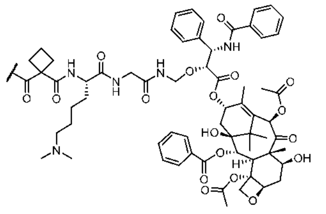
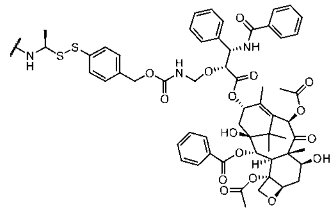

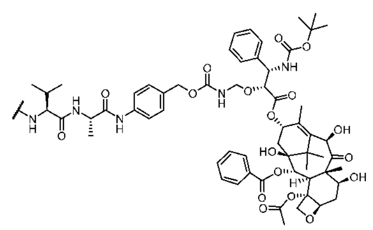
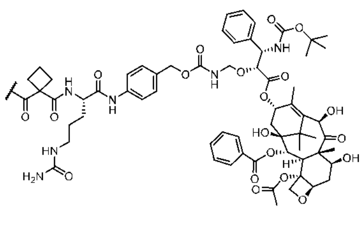
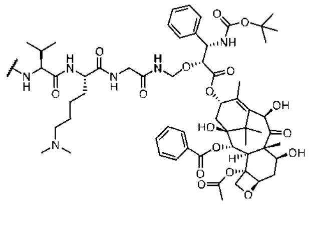

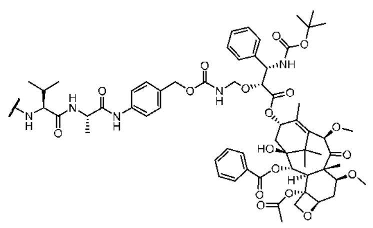
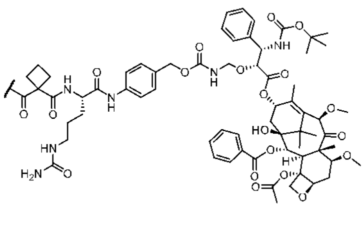
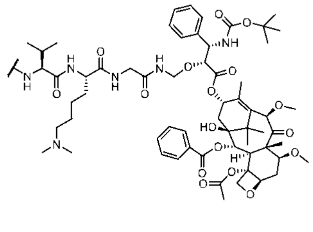

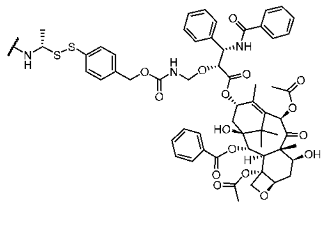
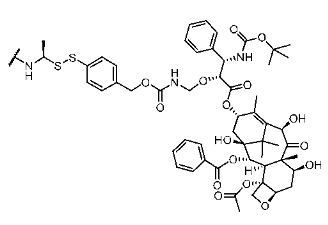
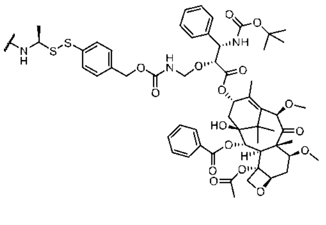

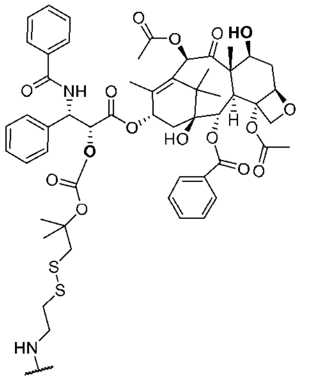
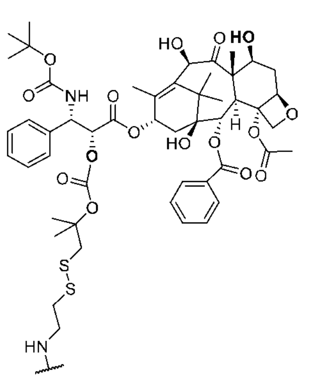
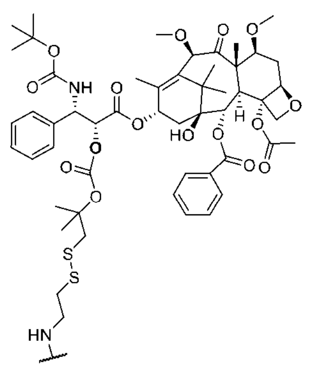

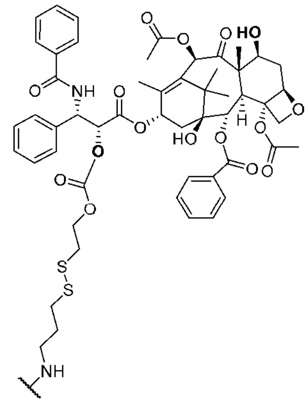
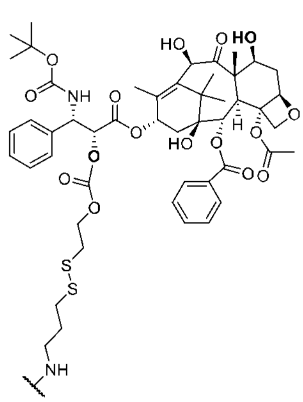
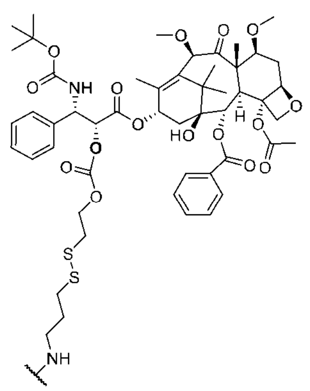

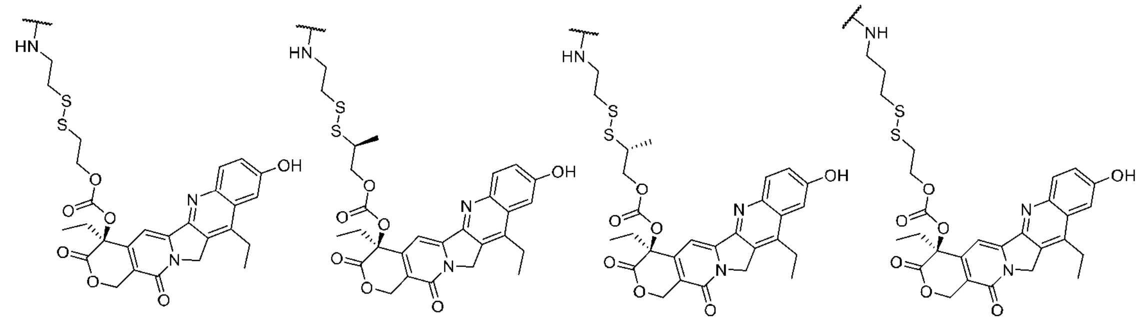
.

**35.** A compound of formula (I-1) or a pharmaceutically acceptable salt thereof:

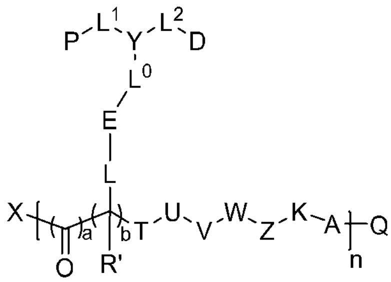

(I-1)

wherein,

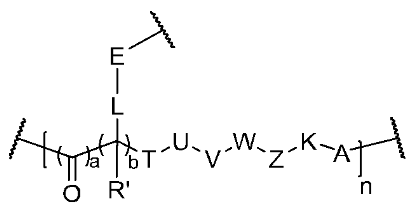

is:

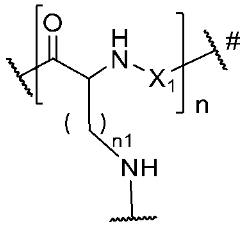 , 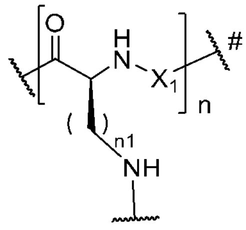 , 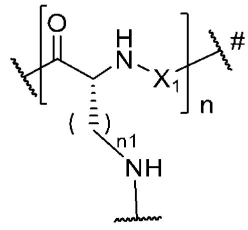 , 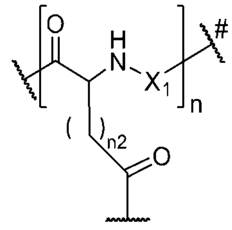 ,

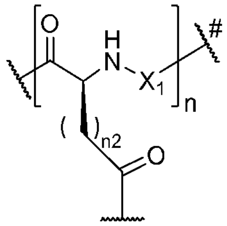 , 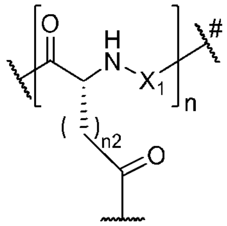 , 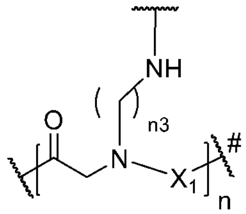 , 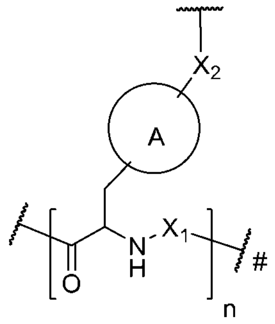 ,

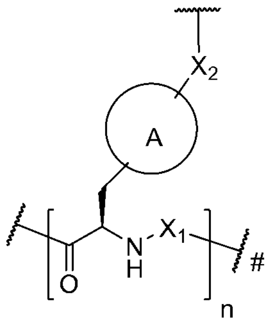 , 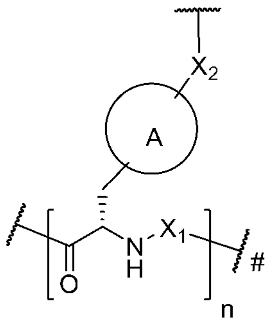 , 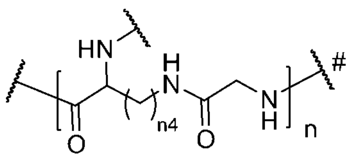 ,

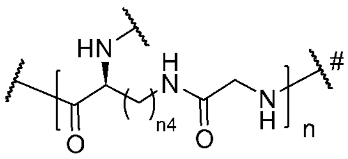 , 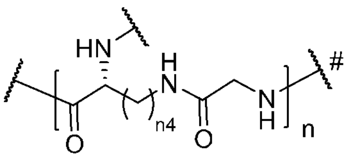 , or 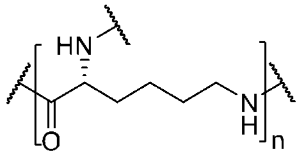 ,

with the "#" terminal linked to Q;

n is independently any integer of 4-100;
n1, n2, n3, and n4 are each independently 0, 1, 2, 3, 4, or 5;
$X_1$ is a covalent bond or

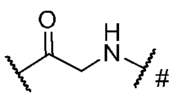 ;

ring A is $C_6$-$C_{10}$ aryl or 5- to 10-membered heteroaryl;
$X_2$ is a covalent bond, O, S, or NH;
$L^0$ is a covalent bond;
Y is

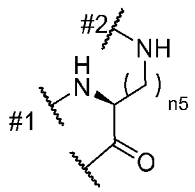

or

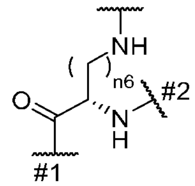

,

the "#1" terminal is linked to $L^2$, and the "#2" terminal is linked to $L^1$;
n5 and n6 are each independently 0, 1, 2, 3, 4, or 5;
$L^1$ is a covalent bond;
P is

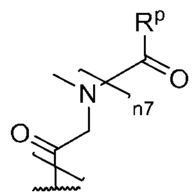

or

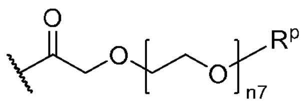

;

$R^p$ is independently $C_1$-$C_6$ alkyl or $C_1$-$C_6$ alkyl substituted with one or more $R^{p-1}$
$R^{p-1}$ is independently halogen, hydroxy, $C_1$-$C_6$ alkoxy, or $NR^{p1}R^{p2}$;
$R^{p1}$ and $R^{p2}$ are each independently -H or $C_1$-$C_6$ alkyl;
n7 is independently any integer of 4-100;
$L^2$ is $^{\#3}$-$L^{2a}$-$L^{2b}$-$^{\#4}$, the "#3" terminal is linked to Y, and the "#4" terminal is linked to D;
$L^{2a}$ is a covalent bond,

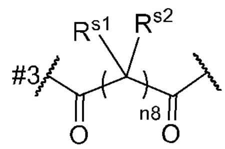

,

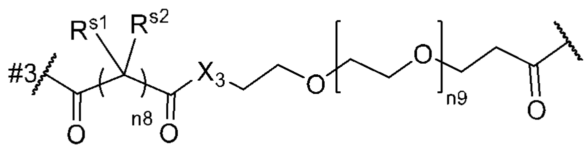

,

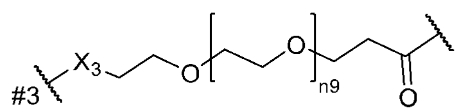

,

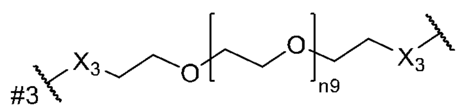

,

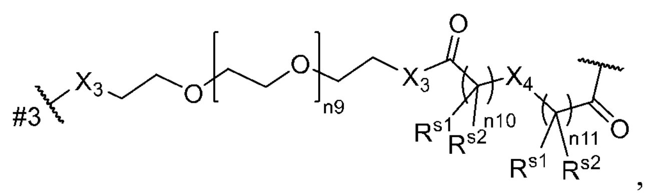

,

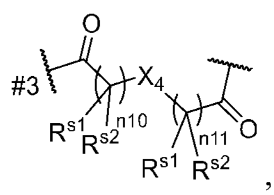

,

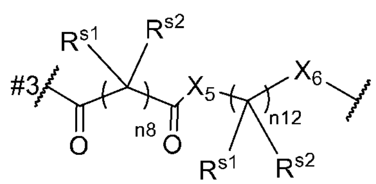

,

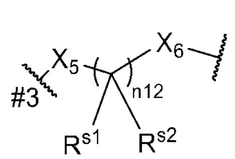

,

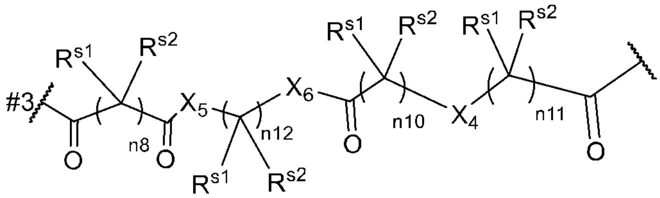

,

or

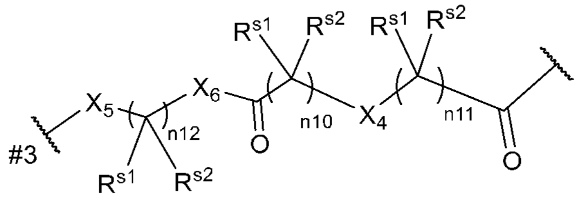

;

n8, n9, n10, n11, n12, n13, and n14 are each independently any integer of 1-20;
$R^{s1}$ and $R^{s2}$ are each independently -H or $C_1$-$C_6$ alkyl;
or, $R^{s1}$ and $R^{s2}$, together with the carbon atom to which they are linked, form a $C_3$-$C_6$ cycloalkyl or a 3- to 6-membered heterocycloalkyl, wherein in the 3- to 6-membered heterocycloalkyl, the heteroatom is selected from one or more of N, O, and S, and the number of heteroatom(s) is 1, 2, or 3;
$X_3$, $X_4$, $X_5$, $X_6$, $X_7$, and $X_8$ are each independently $NR^{s3}$ or O, and $R^{s3}$is H or $C_1$-$C_6$ alkyl;
$L^{2b}$ is a covalent bond,

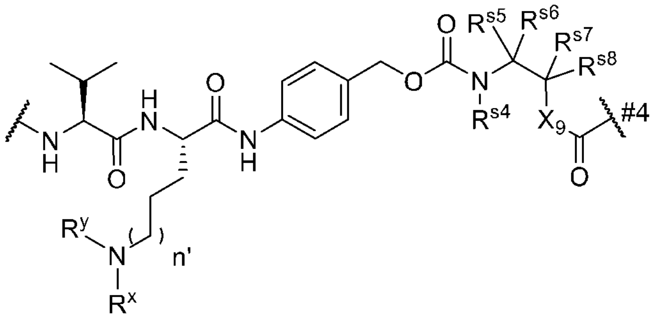

,

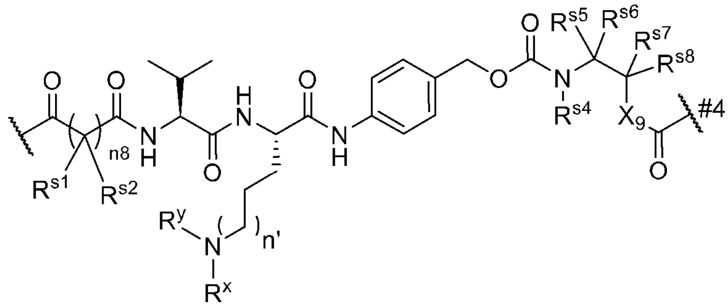

,

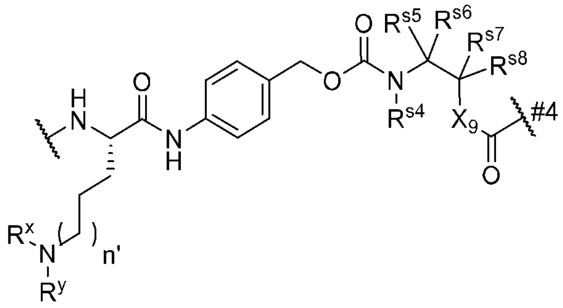

,

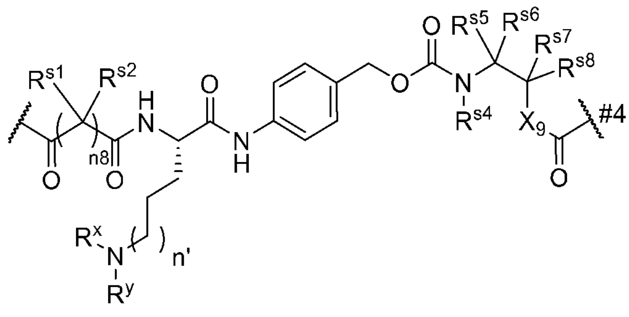

,

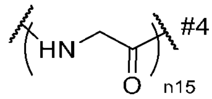

,

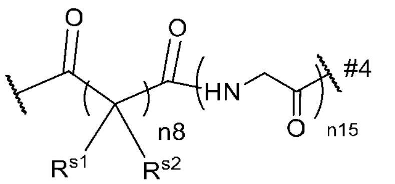

,

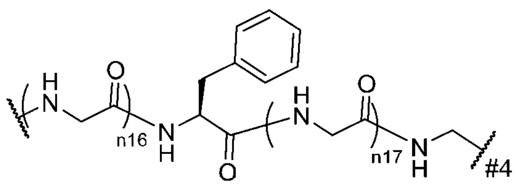

,

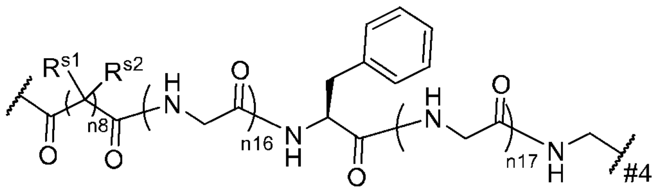

,

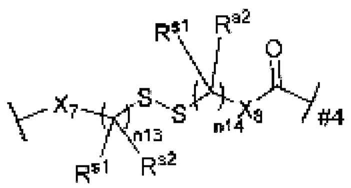

,

or

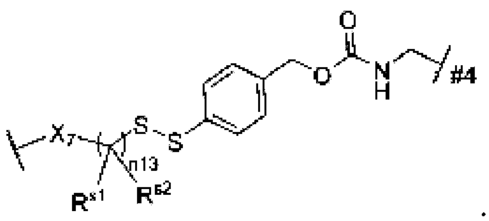

;

$R^x$ is independently -H or $C_1$-$C_3$ alkyl;
$R^y$ is independently H, $C_1$-$C_3$ alkyl, or

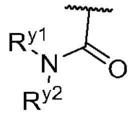

;

$R^{y1}$ and $R^{y2}$ are each independently -H or $C_1$-$C_3$ alkyl;

n' is independently 1 or 2;
$X_9$ is O or $NR^{s9}$;
$R^{s4}$, $R^{s5}$, $R^{s6}$, $R^{s7}$ , $R^{s8}$, and $R^{s9}$ are each independently -H or $C_1$-$C_6$ alkyl;
or, "$R^{s4}$ and $R^{s5}$", "$R^{s6}$ and $R^{s7}$", "$R^{s8}$ and $R^{s9}$", or "$R^{s4}$ and $R^{s9}$", together with the atom to which they are linked, form a 5- to 6-membered heterocycloalkyl, wherein, in the 5- to 6-membered heterocycloalkyl, the heteroatom is N, O, or N and O, and the number of heteroatom(s) is 1 or 2;
n15, n16, and n17 are each independently 1, 2, 3, or 4;
D is a pharmaceutically active agent residue;
X is OH, $NH_2$, -$L_2$-D, -D, or

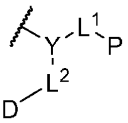;

Q is

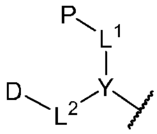

or -P.

**36.** The compound of formula (I-1) or the pharmaceutically acceptable salt thereof according to claim 35, wherein one or more of the following conditions are met:

(1)

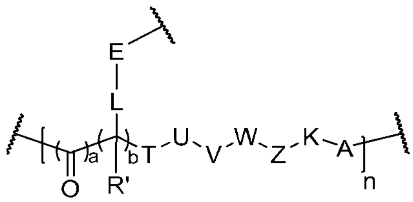

is

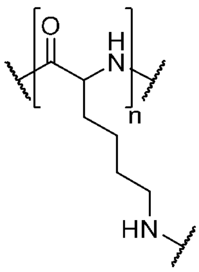, 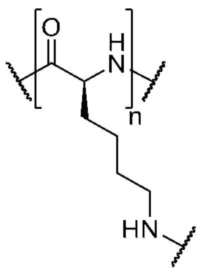, 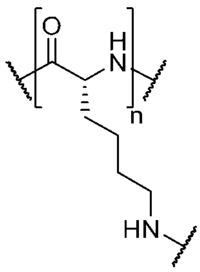,

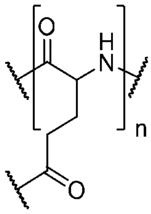, 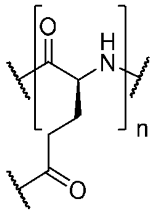, 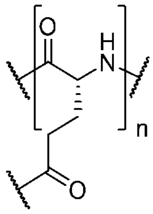, 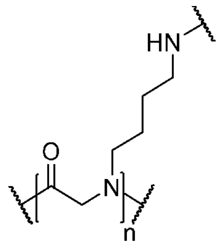, 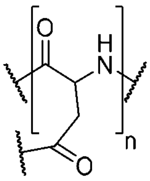, 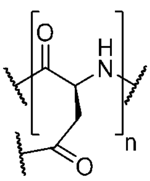,

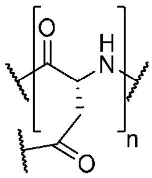, 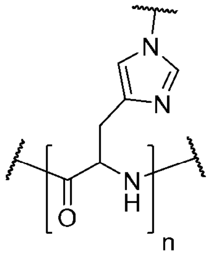, 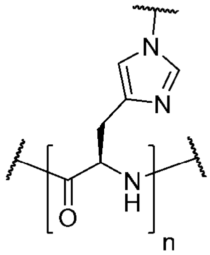, 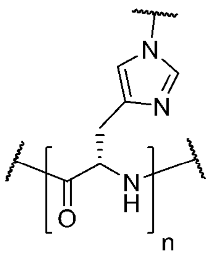, 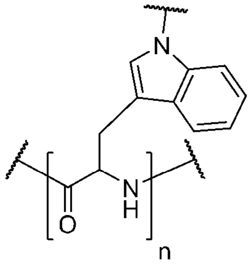,

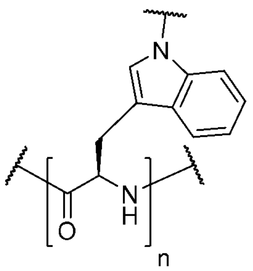, 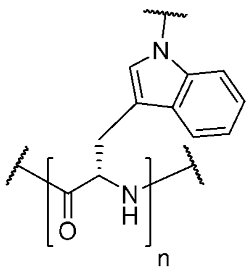, 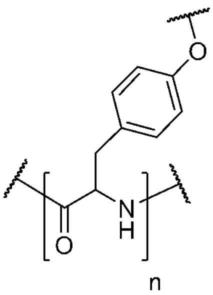, 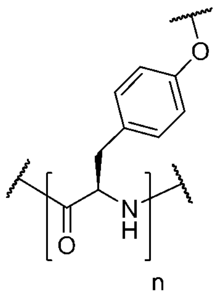,

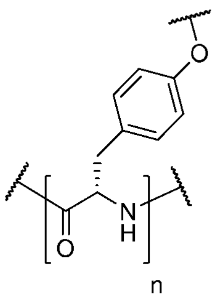, 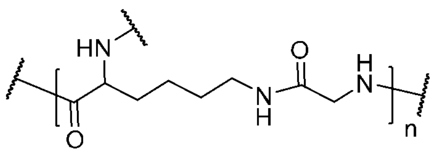, 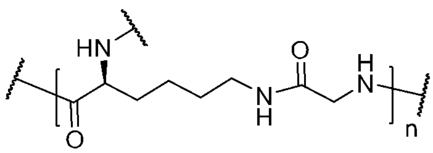,

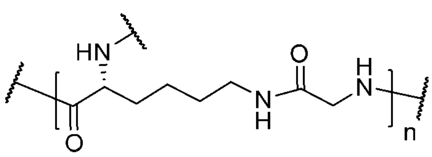, 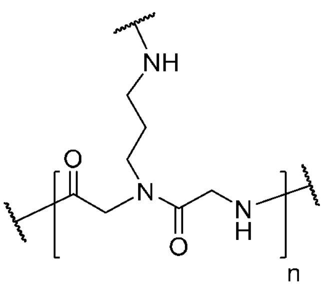, 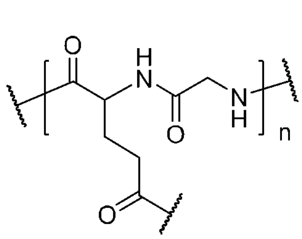,

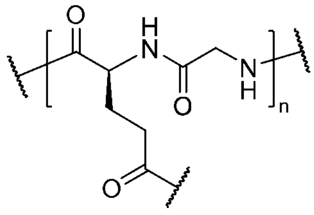 , 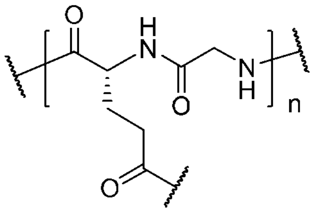 , or 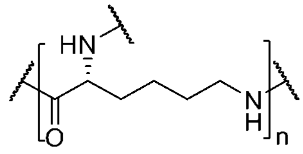 ;

(2) n is independently any integer of 20-60, for example, 20-30 or 50-60, such as 28, 29, 30, 31, 59, or 60;
(3) Y is

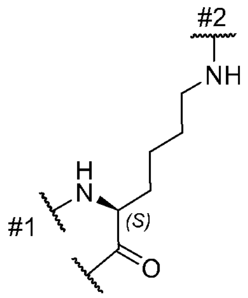 or 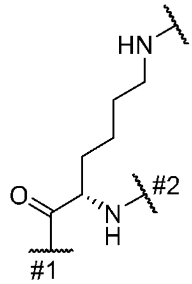 ;

(4) n7 is each independently any integer of 20-50, e.g., 22, 28, 44, or 49;
(5) $R^p$ is independently methyl, methoxy, $-CH_2NH_2$, or $-CH_2NHCH_3$;
(6) n8, n9, n10, n11, n12, n13, and n14 are each independently any integer of 1-15, e.g., 1, 2, 3, 7, or 11;
(7) $R^{s1}$ and $R^{s2}$ are each independently -H, or $R^{s1}$ and $R^{s2}$, together with the carbon atom to which they are linked, form a $C_3$-$C_6$ cycloalkyl, e.g., cyclobutyl.

**37.** The compound of formula (I-1) or the pharmaceutically acceptable salt thereof according to claim 35, wherein one or more of the following conditions are met:

(1)

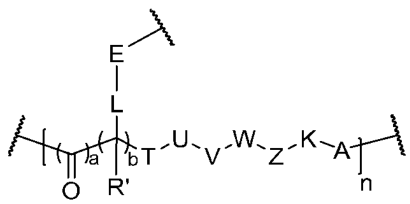

is

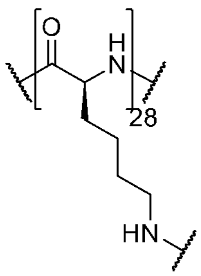 , 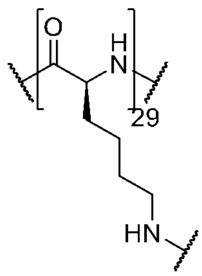 , 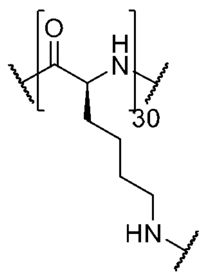 ,

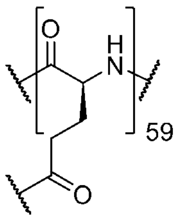, 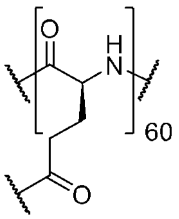, 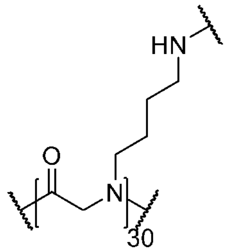, or 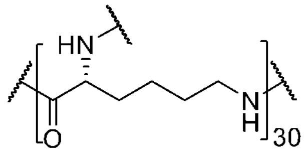;

(2) P is

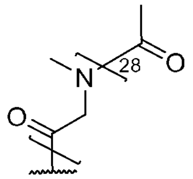, 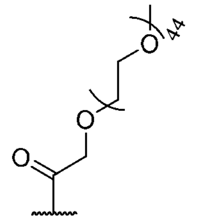, 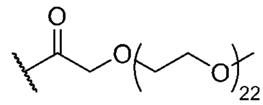, or 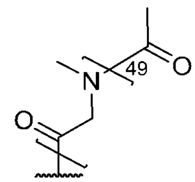;

(3) Y is

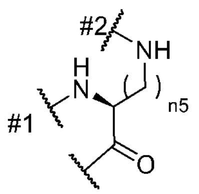,

$L^{2a}$ is a covalent bond,

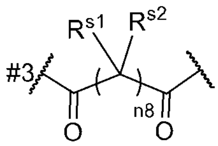,

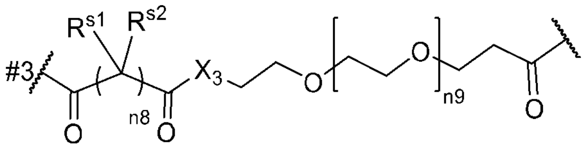, 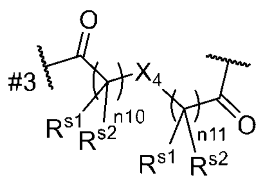,

or

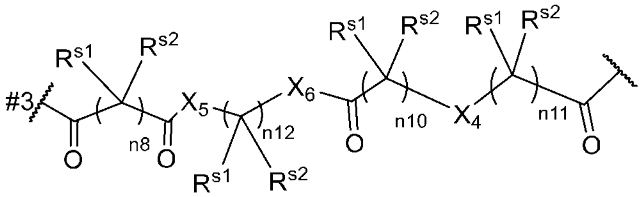,

and $L^{2b}$ is

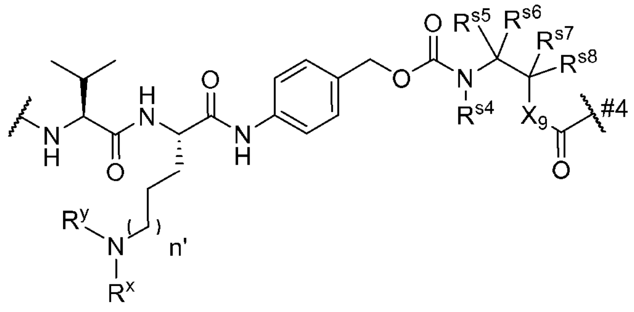

,

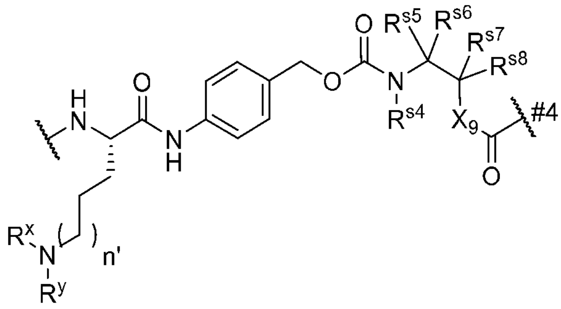

,

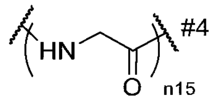

,

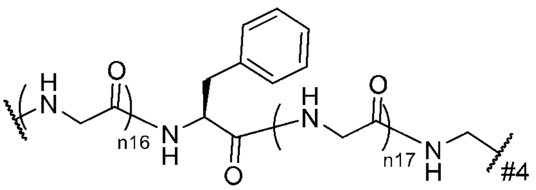

,

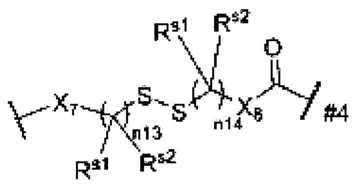

,

or

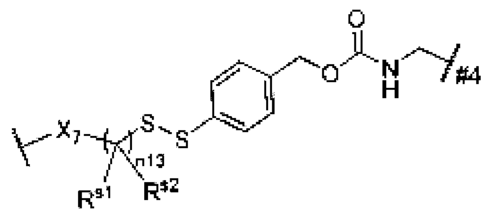

;

(4) Y is

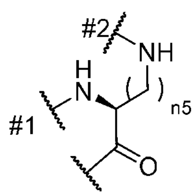

,

$L^{2a}$ is a covalent bond or

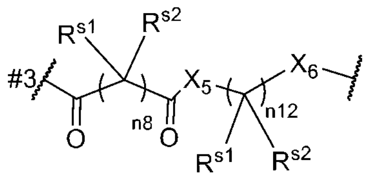

,

and $L^{2b}$ is a covalent bond,

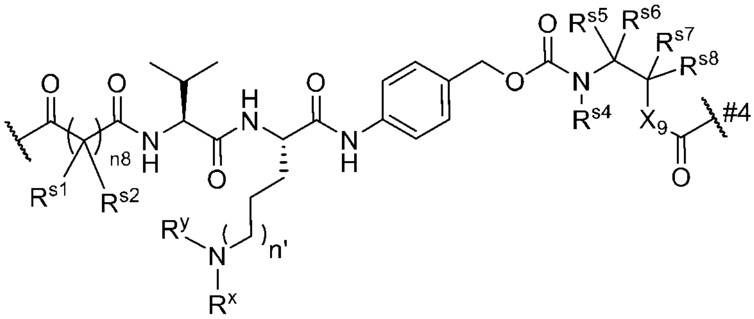

,

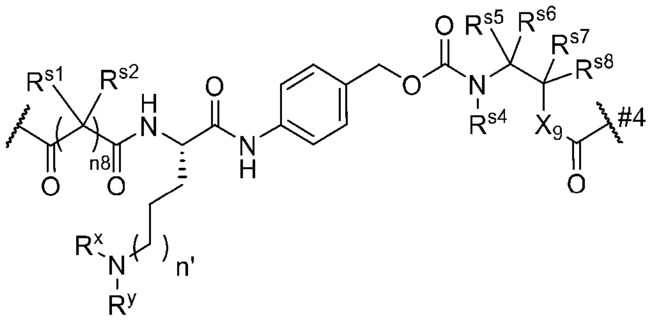

,

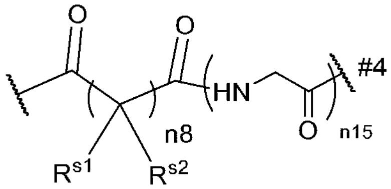

,

or

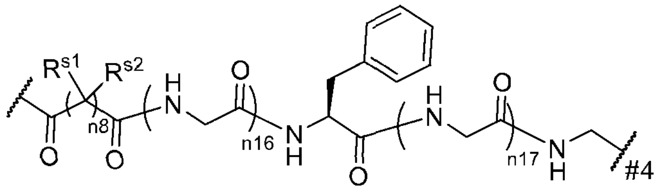

;

(5) Y is

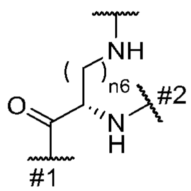

,

$L^{2a}$ is a covalent bond,

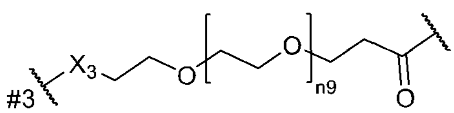

,

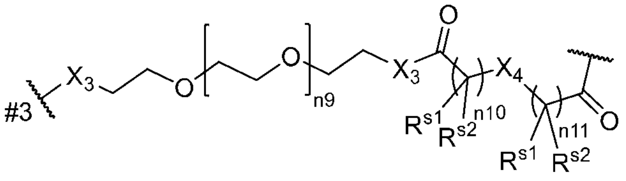

,

or

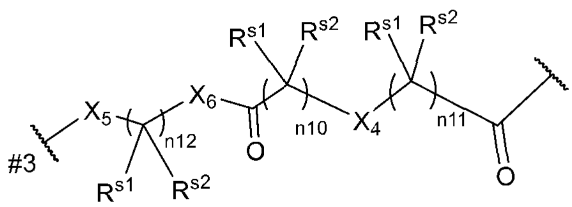

,

and $L^{2b}$ is a covalent bond,

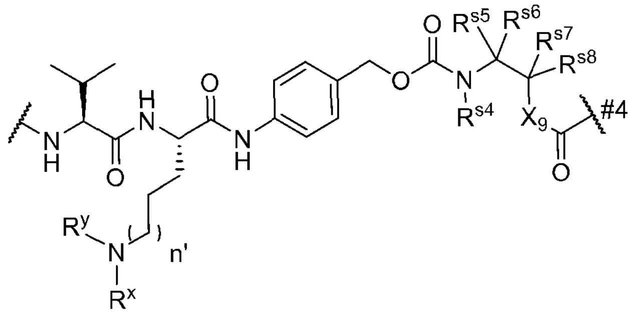

,

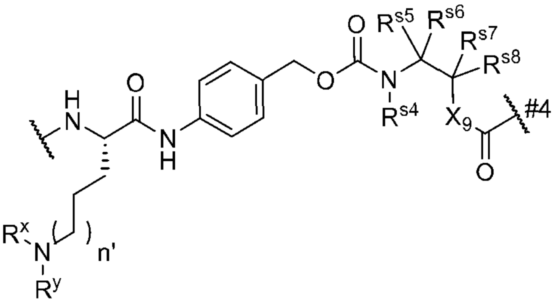

,

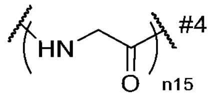

,

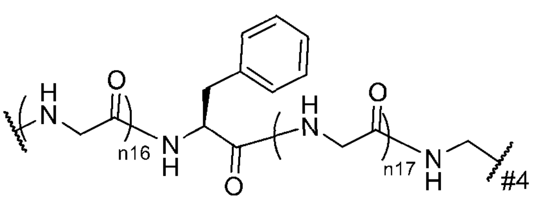

,

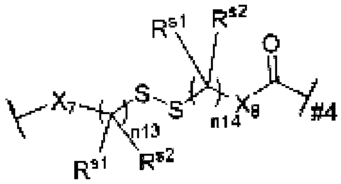

,

or

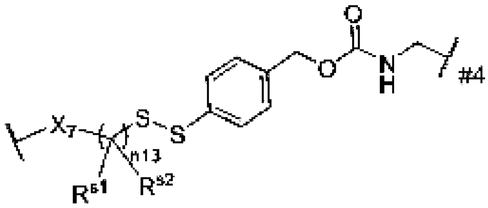

;

(7) Y is

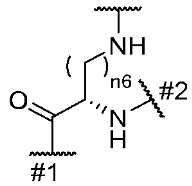

,

$L^{2a}$ is a covalent bond,

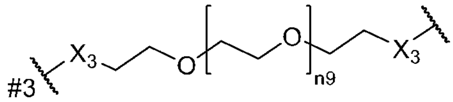

,

or

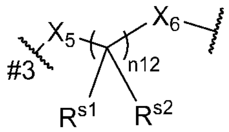

,

and $L^{2b}$ is a covalent bond,

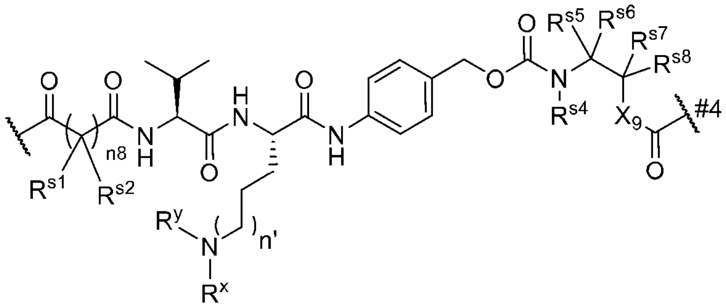

,

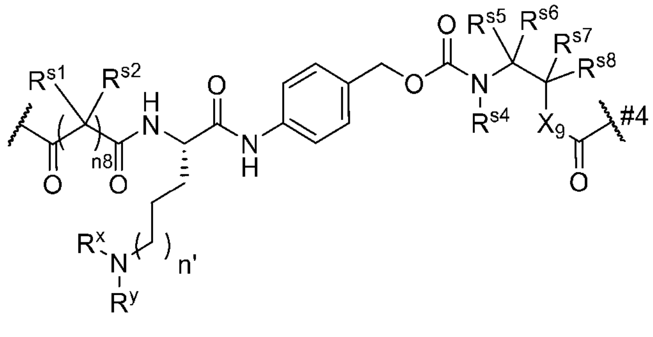

,

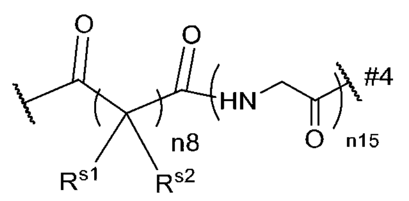

,

or

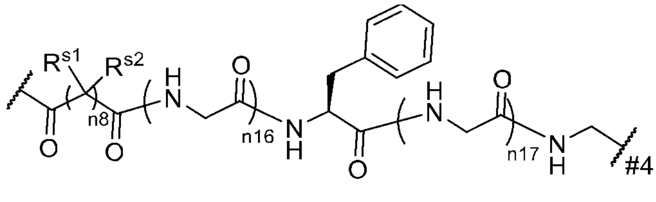;

(8) X is OH, $NH_2$, -D, $-L^{2b}-D$,

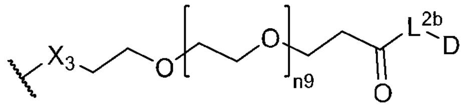,

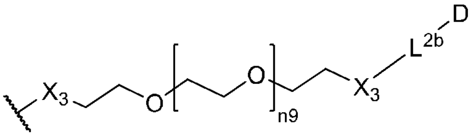, 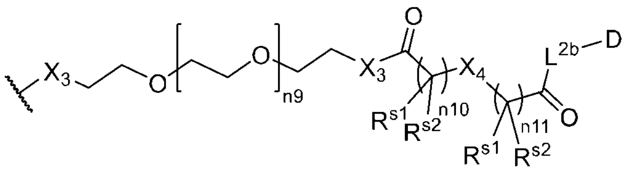,

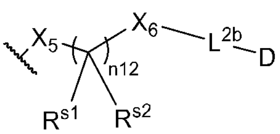, 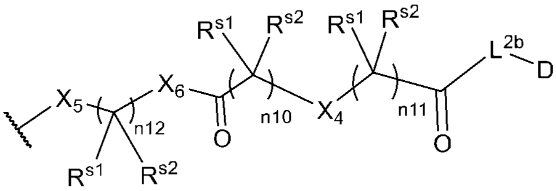, or 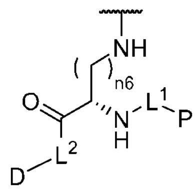,

wherein in

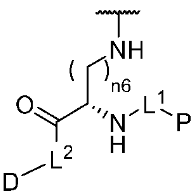,

$-L^2-D$ is $-L^{2b}-D$,

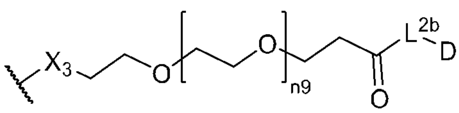,

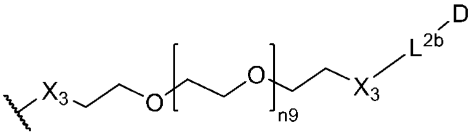, 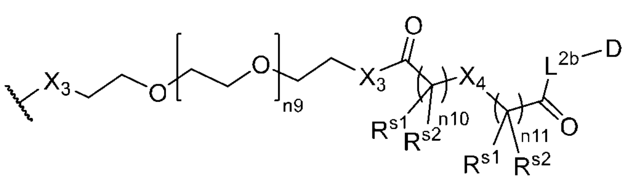,

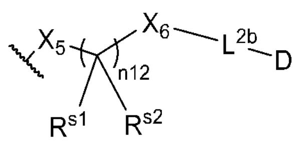

,

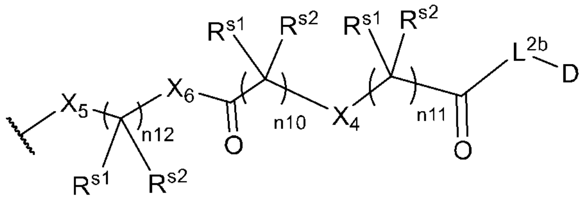

,

or

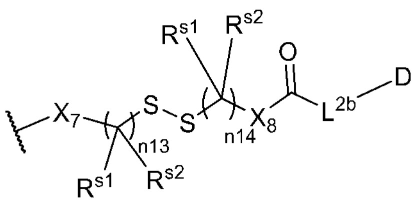

;

(9) Q is

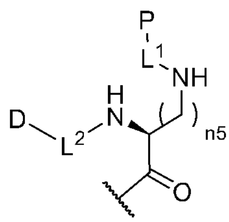

or -P, and in

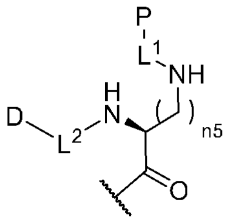

,

$-L^2$-D is

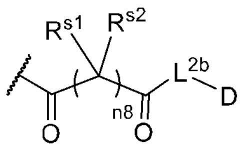

,

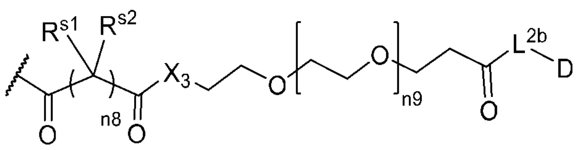

,

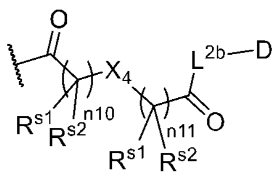

,

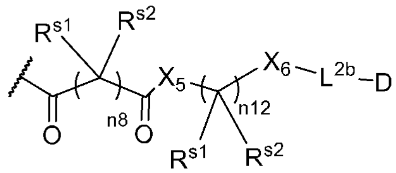 , 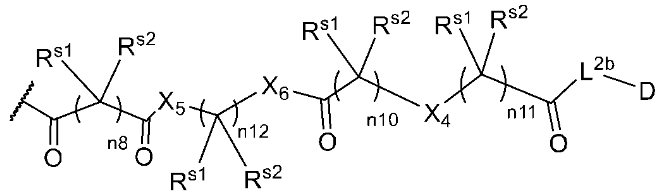 ,

or

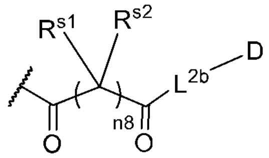 .

**38.** The compound of formula (I-1) or the pharmaceutically acceptable salt thereof according to claim 35, wherein one or more of the following conditions are met:

(1) Y is

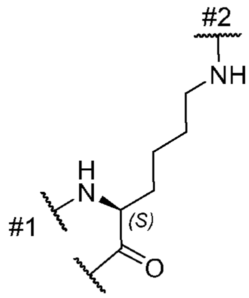 ,

$L^{2a}$ is a covalent bond,

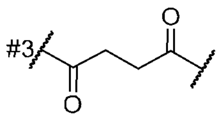 ,

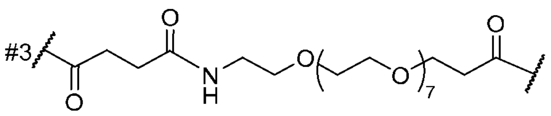 , 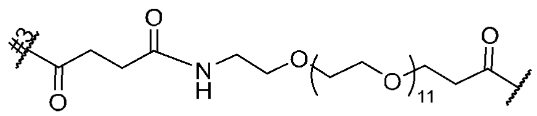 ,

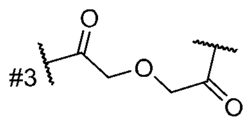 , or 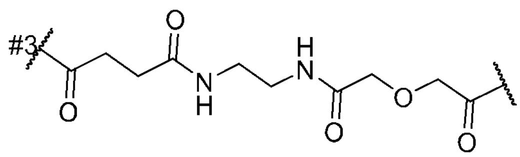 ,

and $L^{2b}$ is a covalent bond,

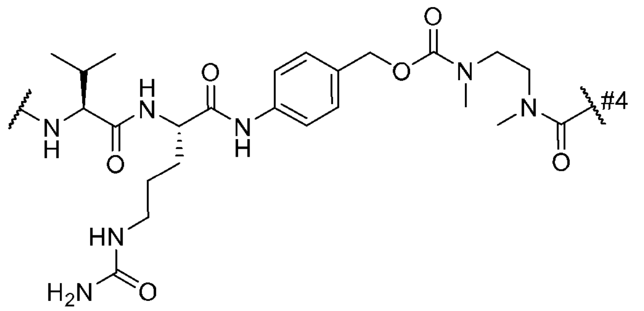

,

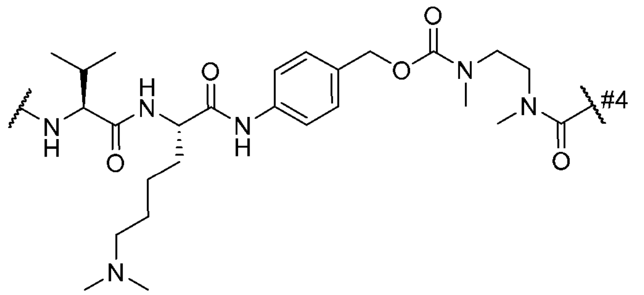

,

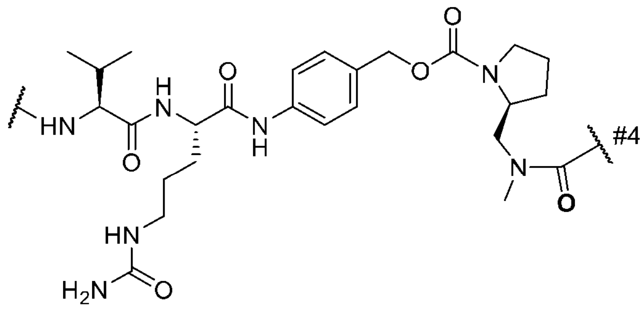

,

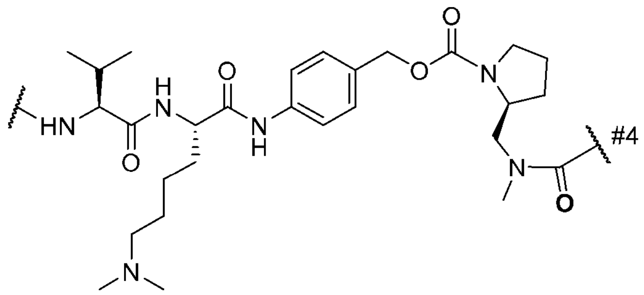

,

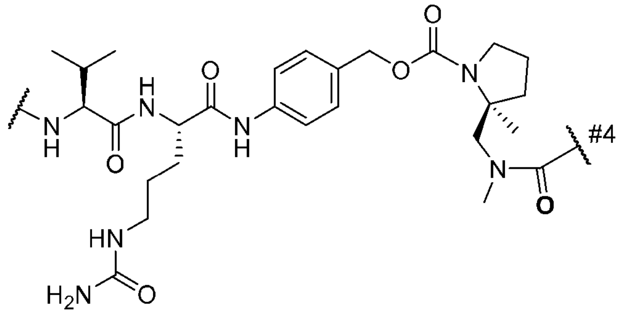

,

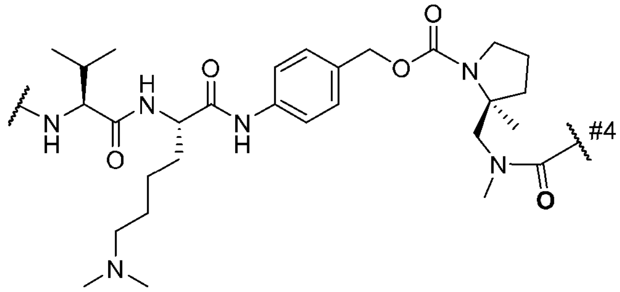

,

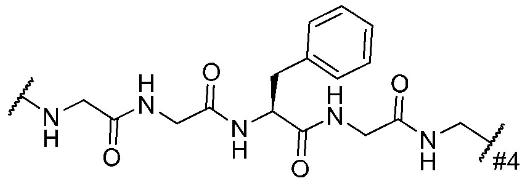

,

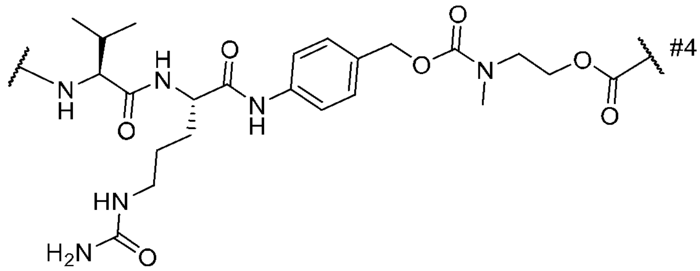

,

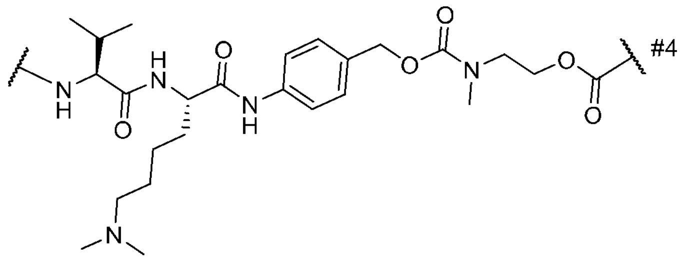

,

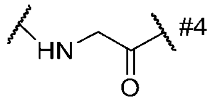

,

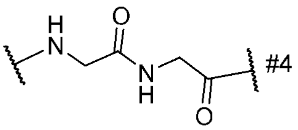

,

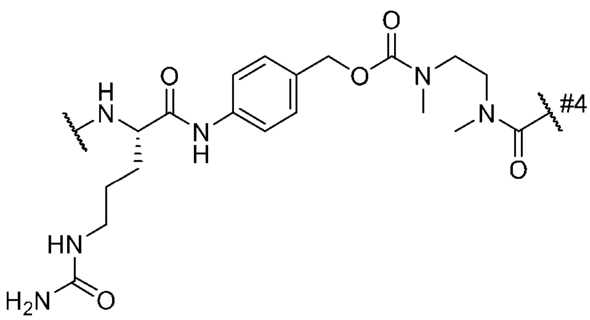

,

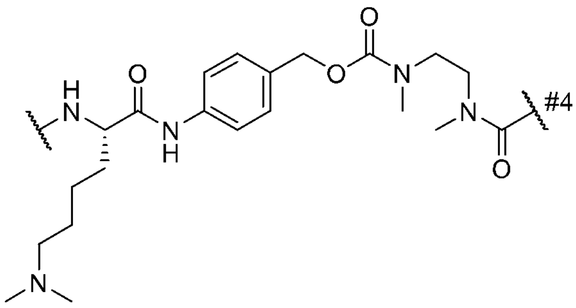

,

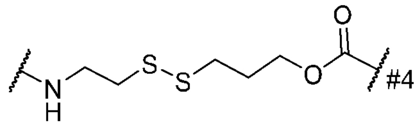

,

or

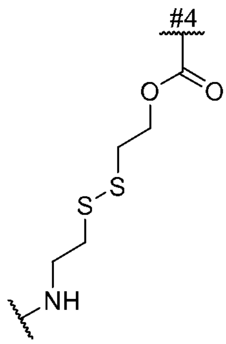

;

(2) Y is

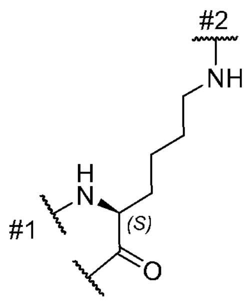

,

$L^{2a}$ is a covalent bond or

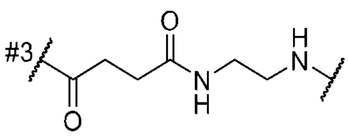

,

and $L^{2b}$ is a covalent bond,

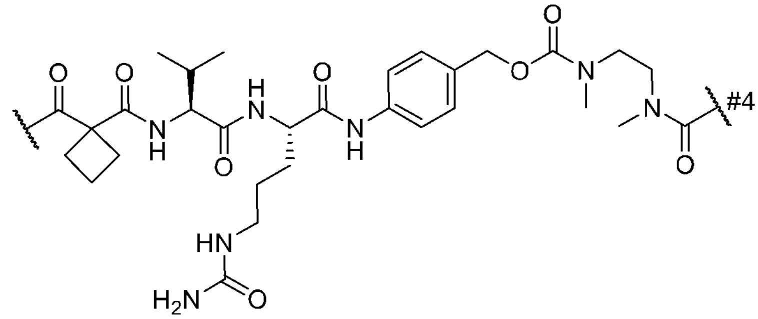

,

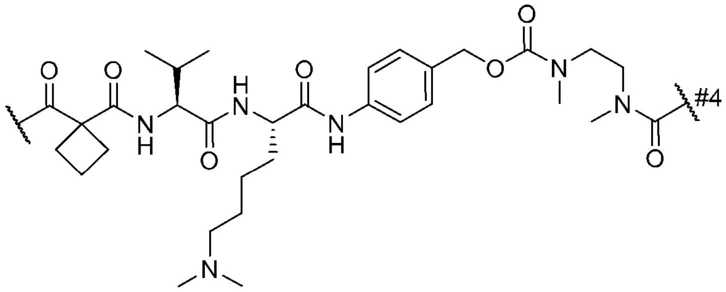

,

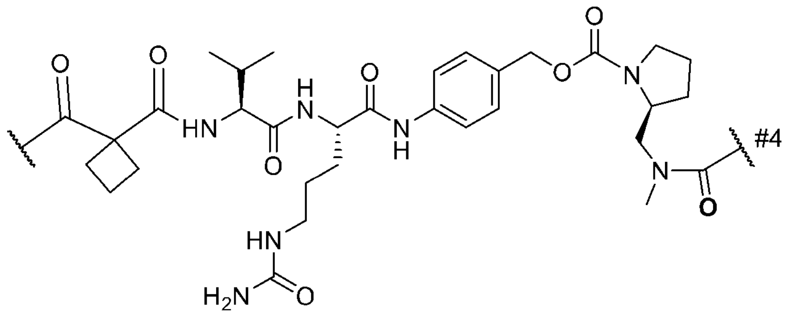

,

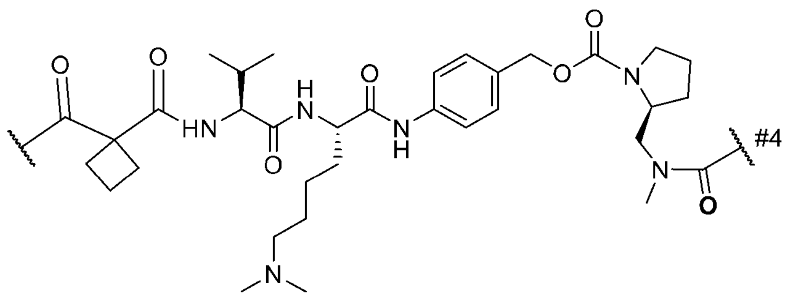

,

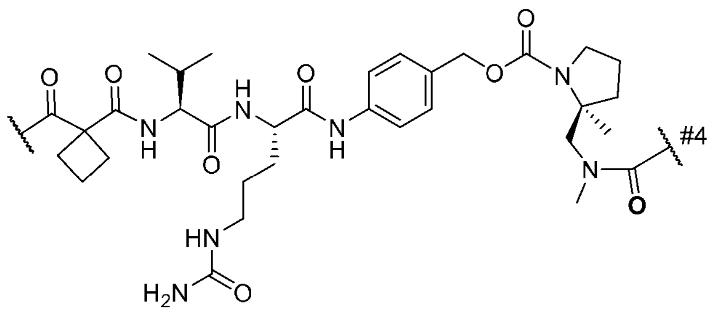

,

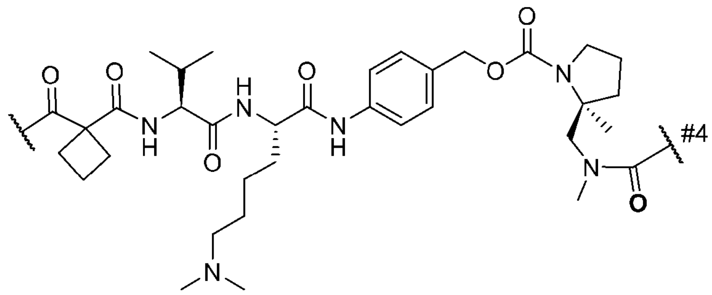

,

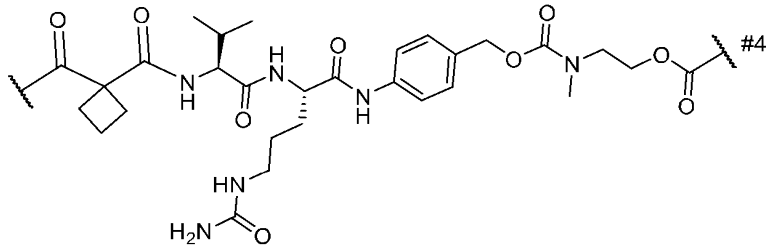

,

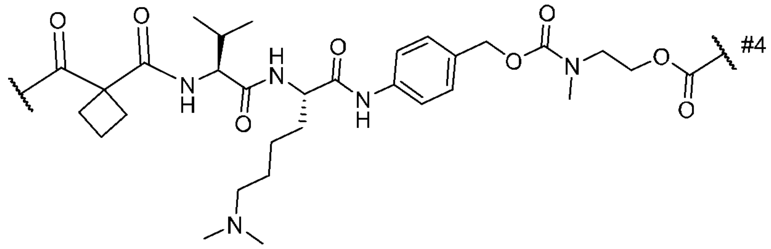

,

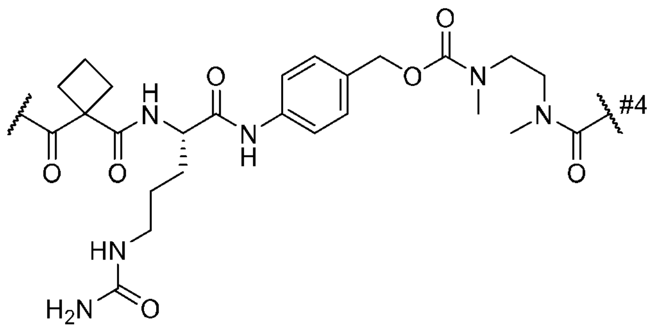

,

or

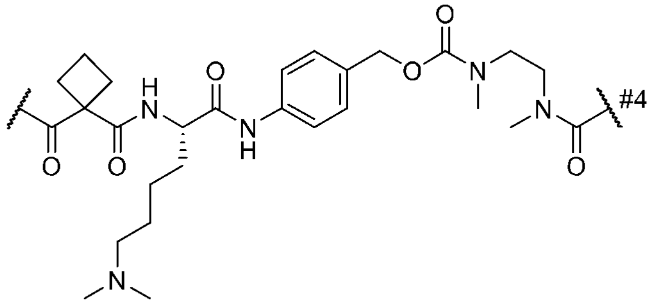

;

(3) Y is

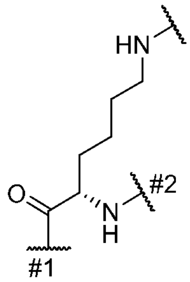

,

$L^{2a}$ is a covalent bond,

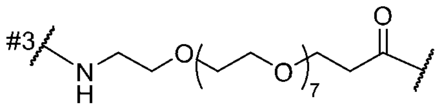

,

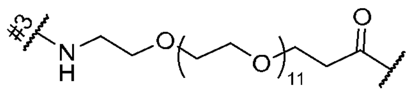

, or

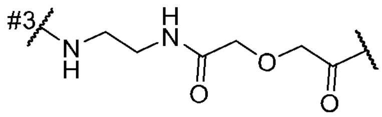

,

and $L^{2b}$ is a covalent bond,

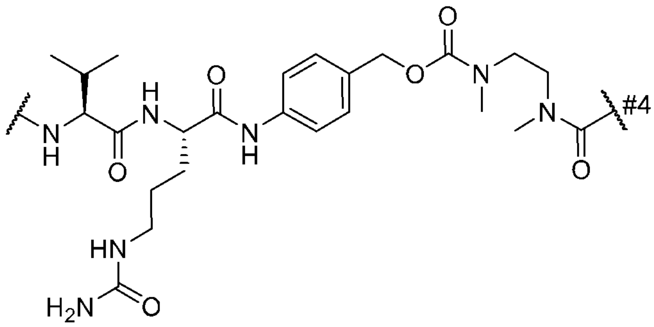

,

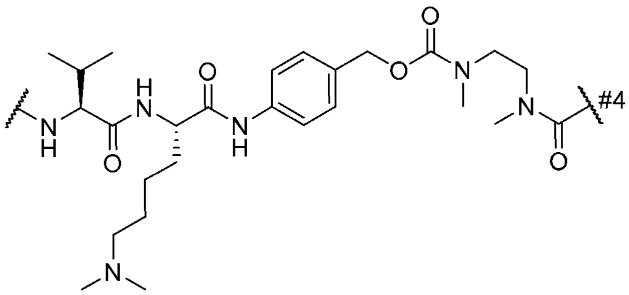

,

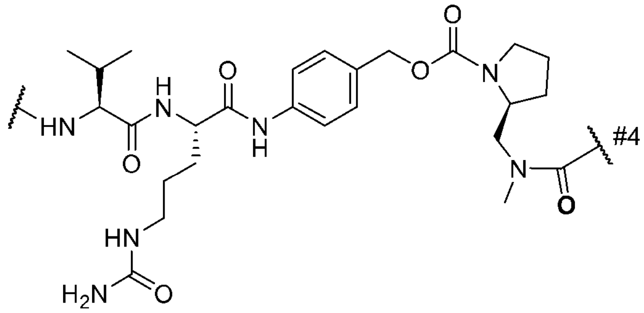

,

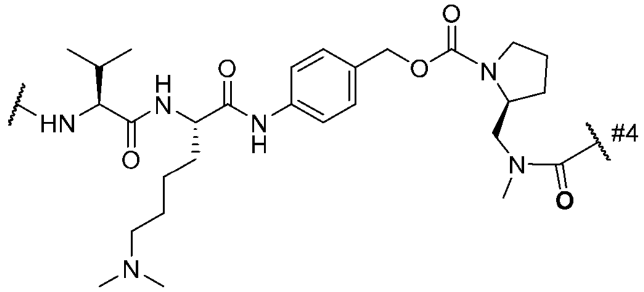

,

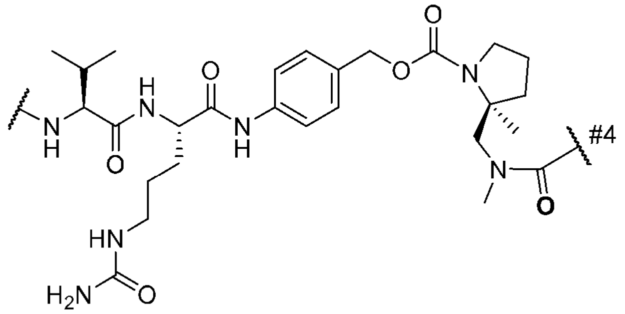

,

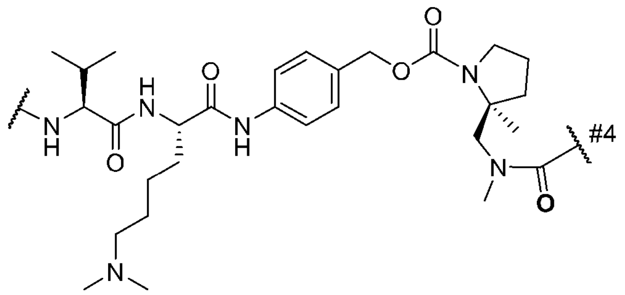

,

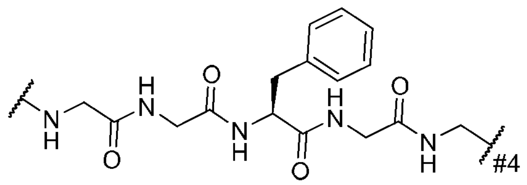

,

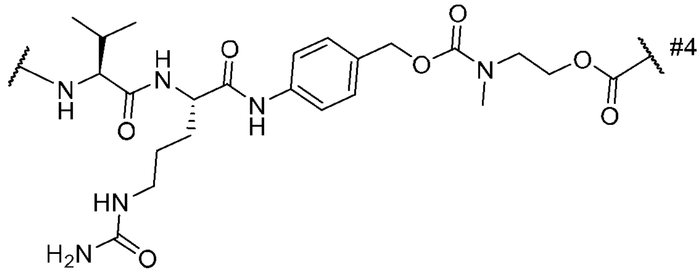

,

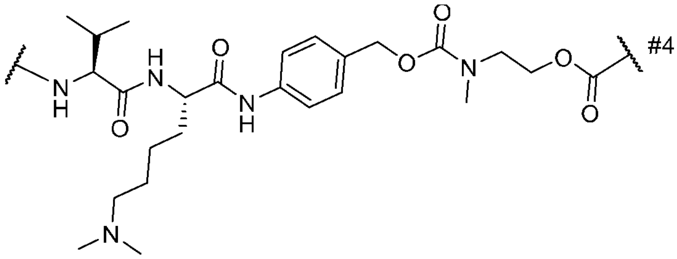

,

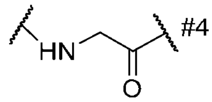

,

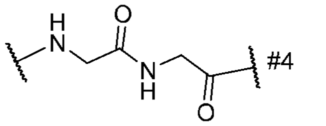

,

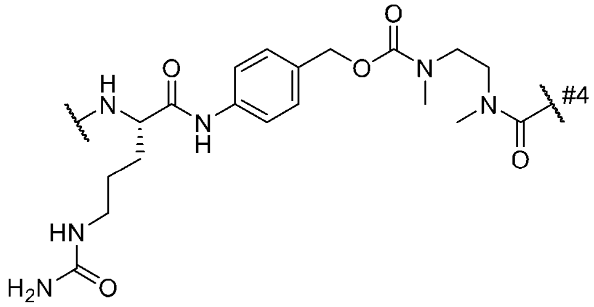

,

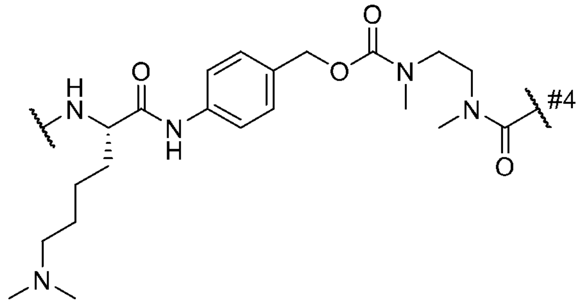

,

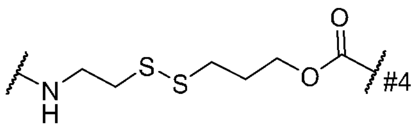

or

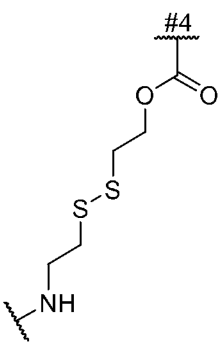

,

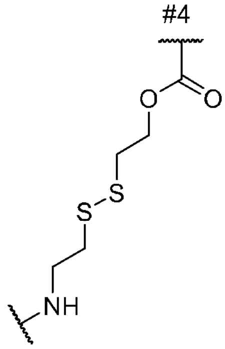

, or

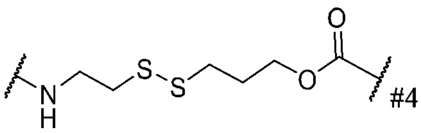

;

(4) Y is

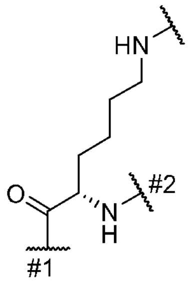

,

$L^{2a}$ is a covalent bond or

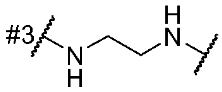

,

and $L^{2b}$ is a covalent bond,

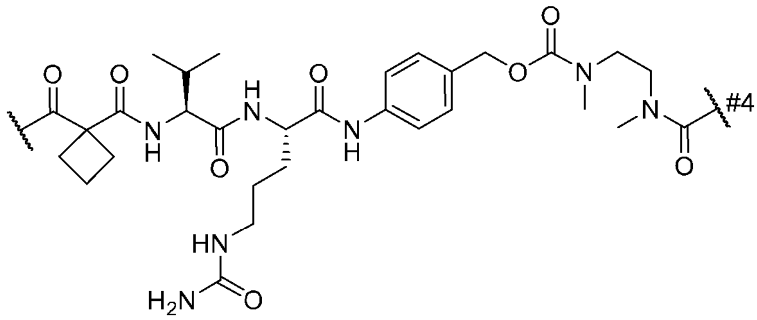

,

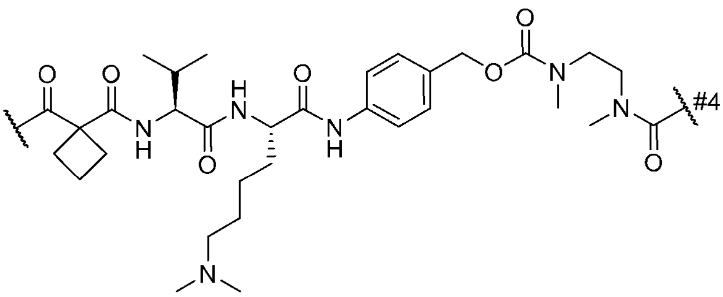

,

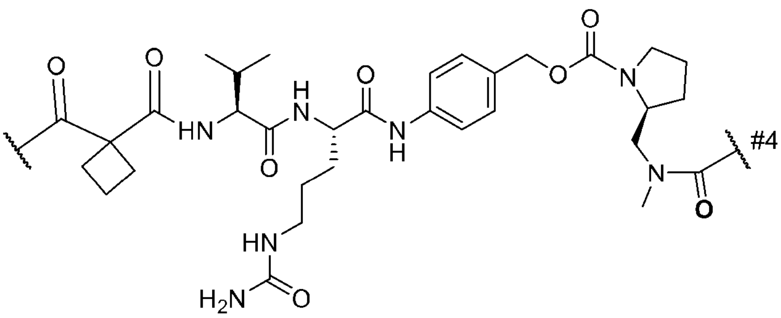

,

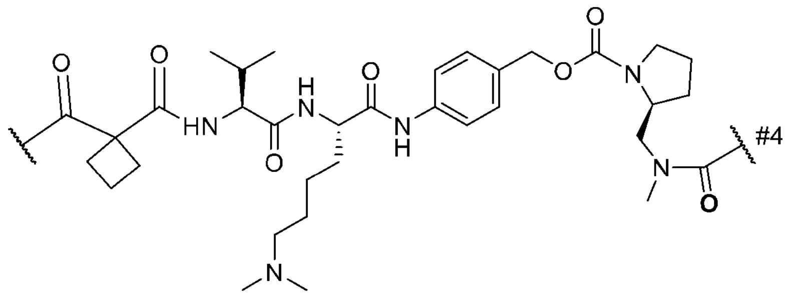

,

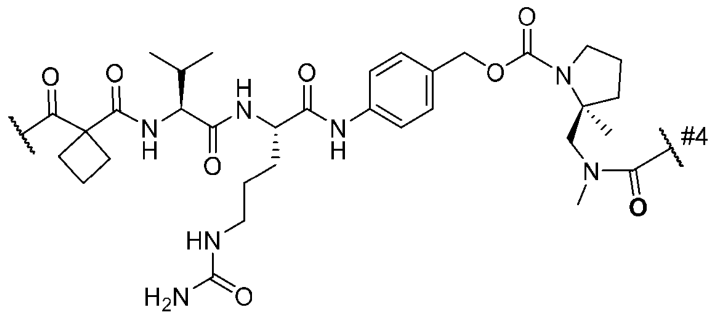

,

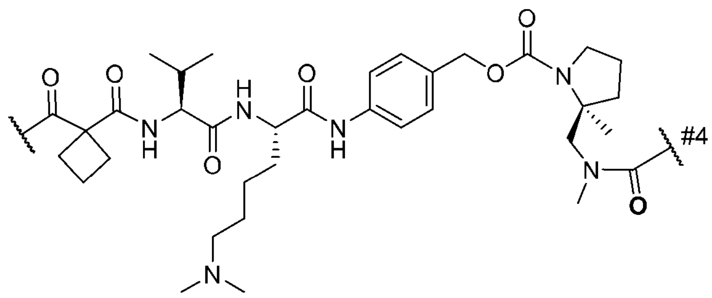

,

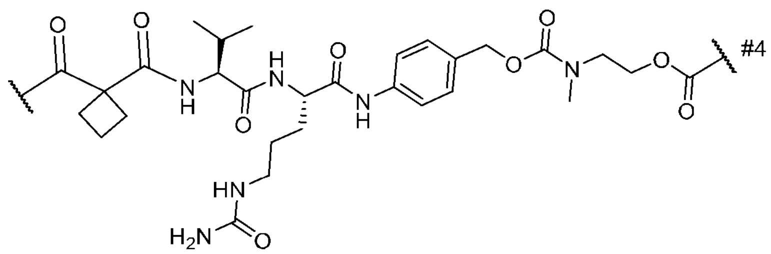

,

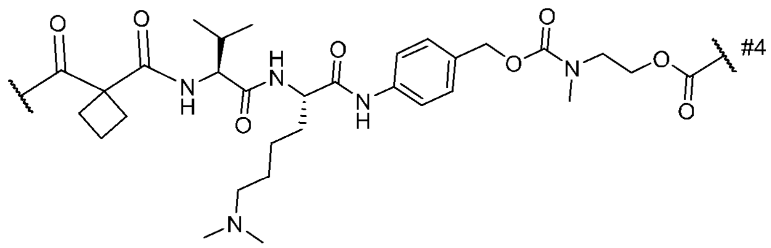

,

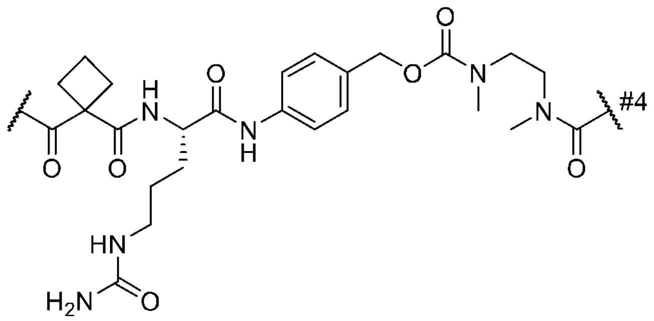

,

or

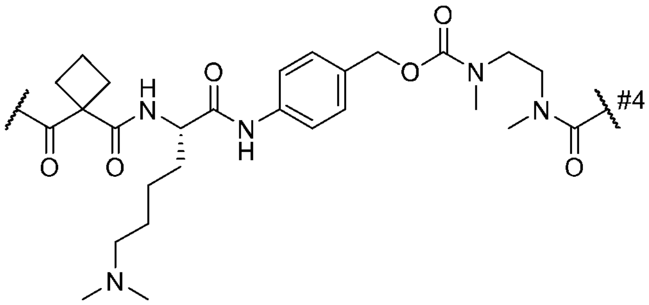

;

(5) X is OH, $NH_2$, -D,

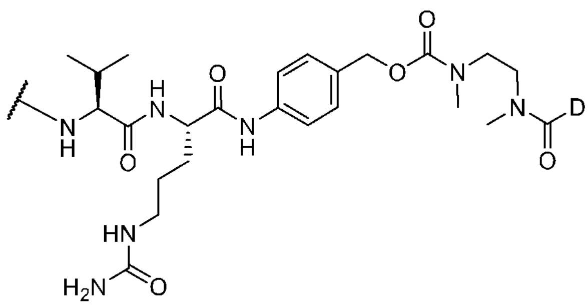

,

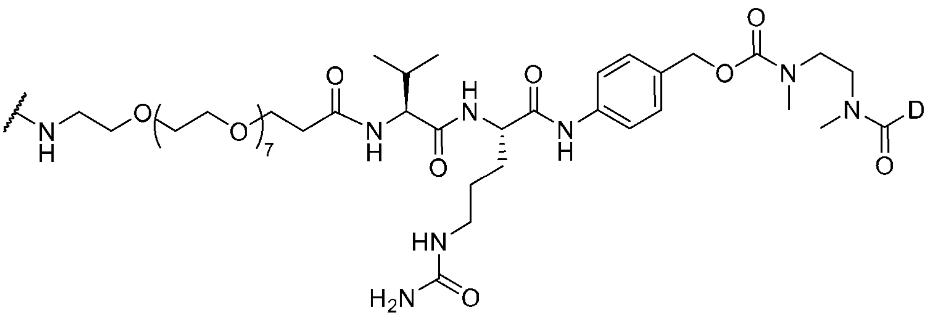

,

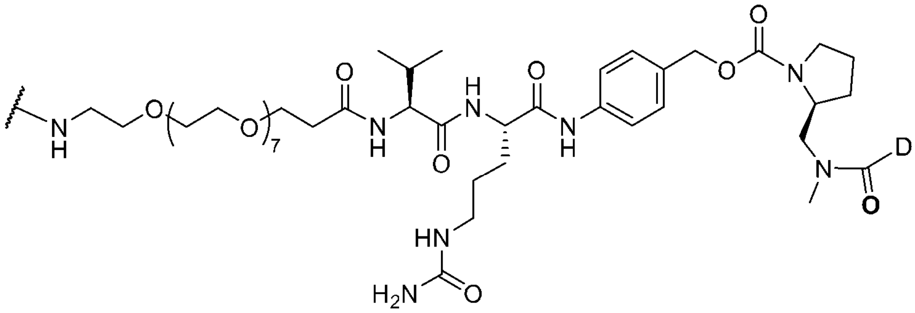

,

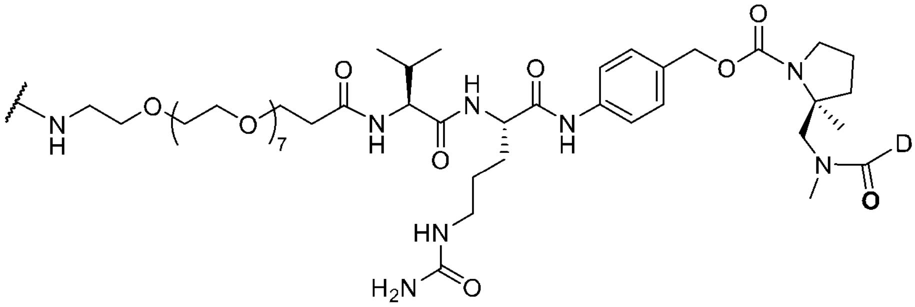

,

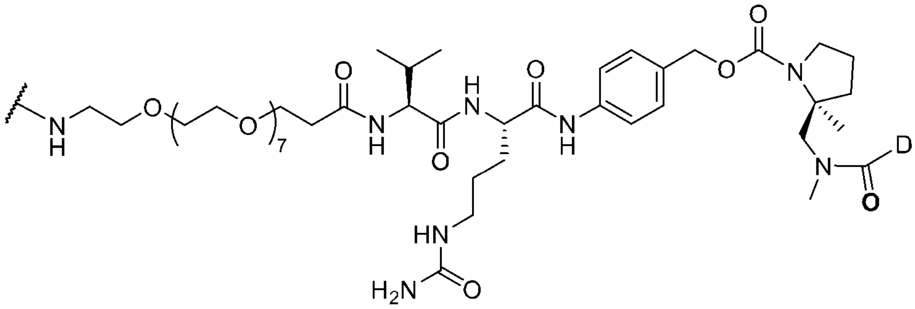

,

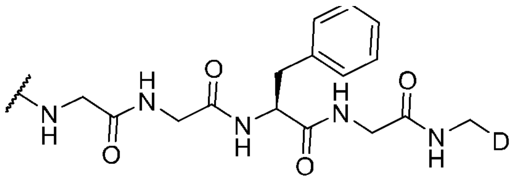

,

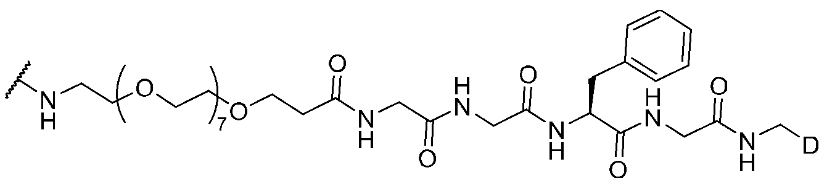

,

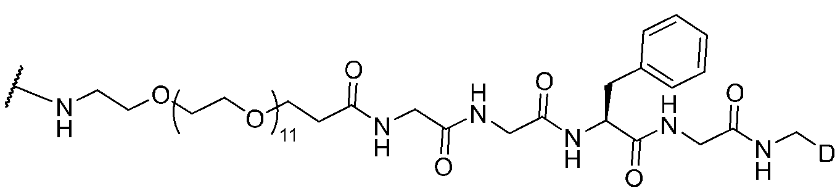

,

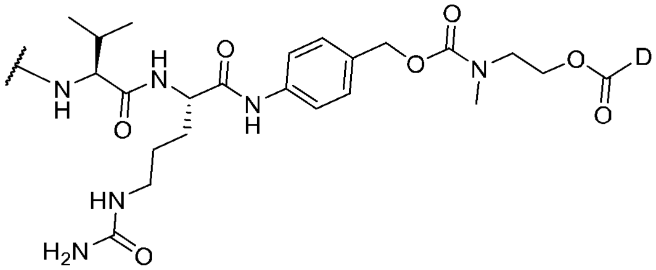

,

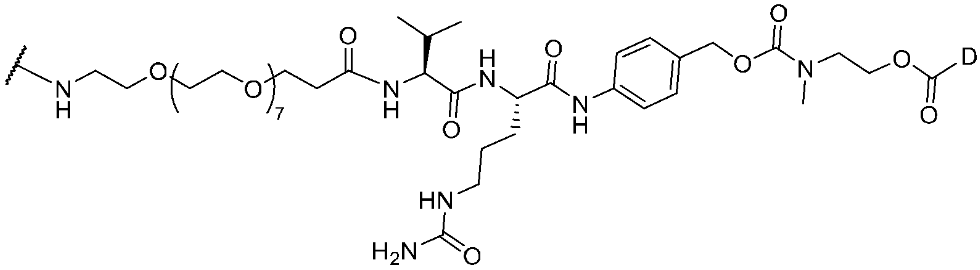

,

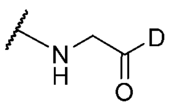

,

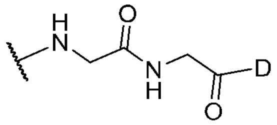

,

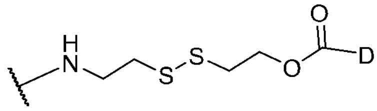

,

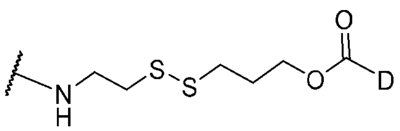

,

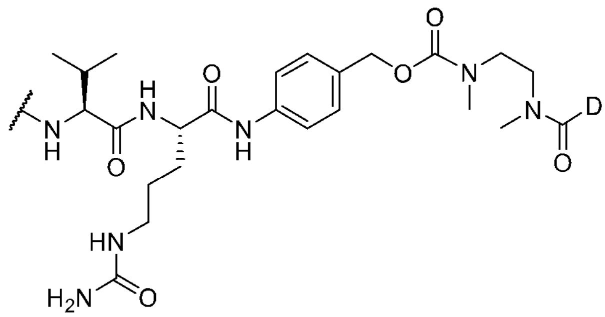

,

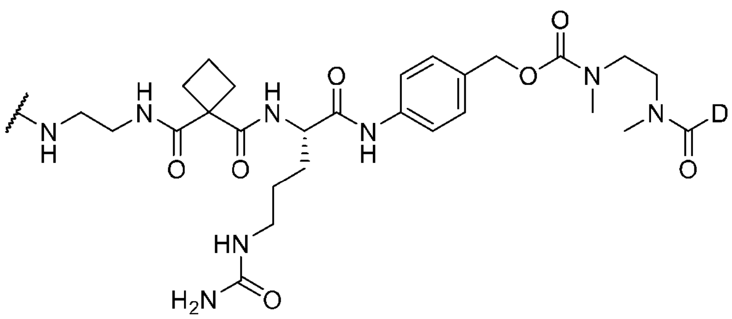

,

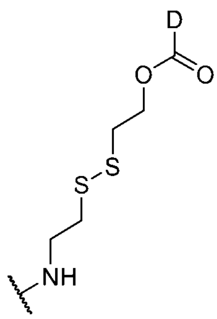

,

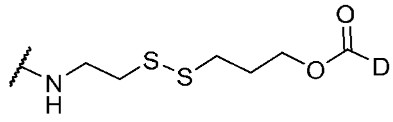 , or 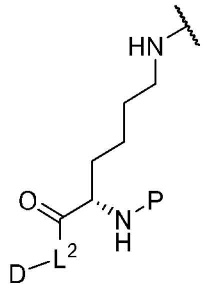 ,

wherein, in

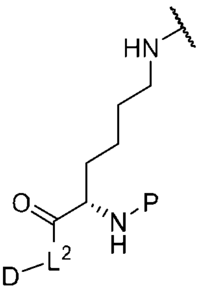 ,

-$L^2$-D is

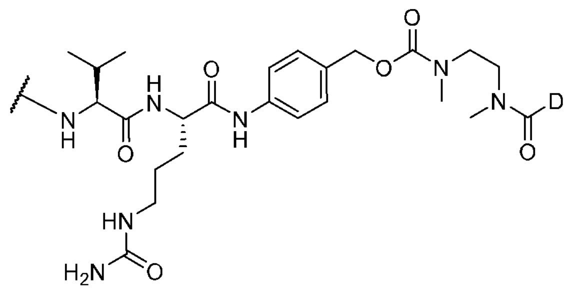 ,

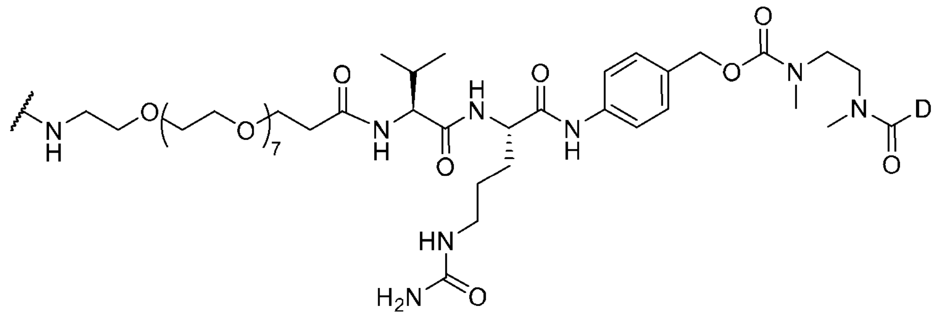 ,

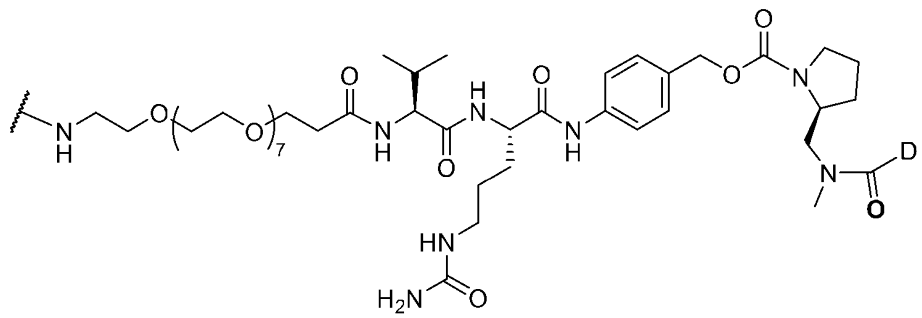 ,

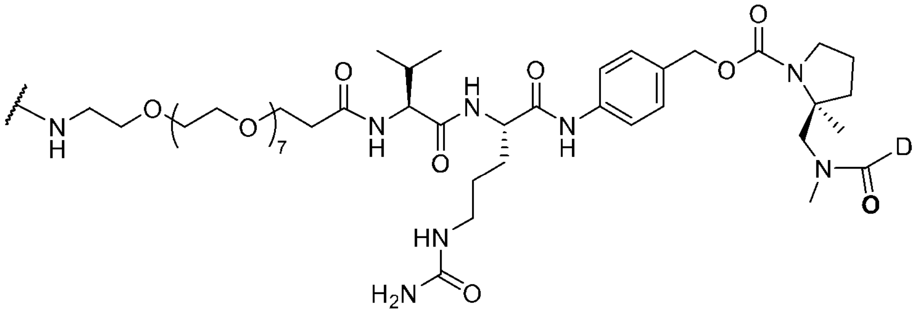

,

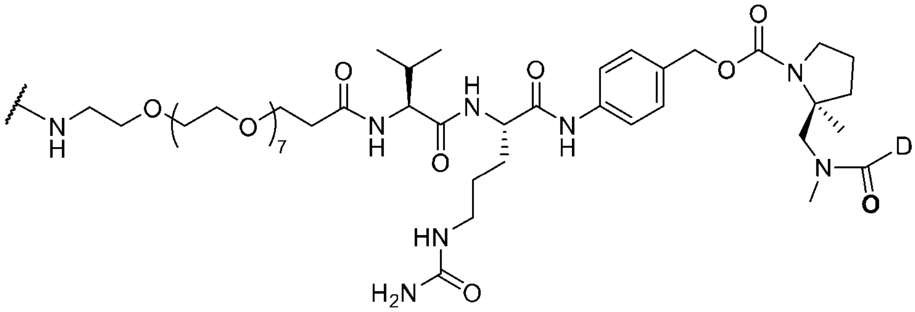

,

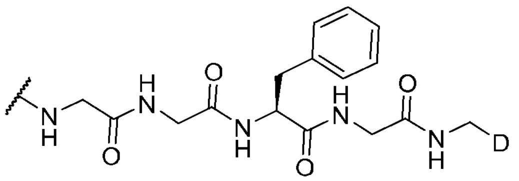

,

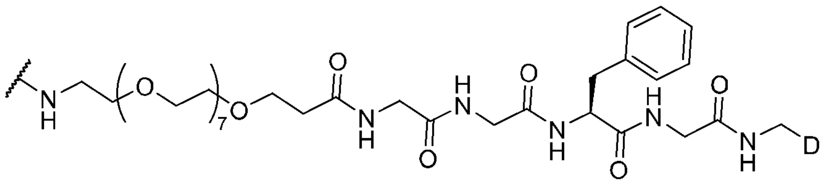

,

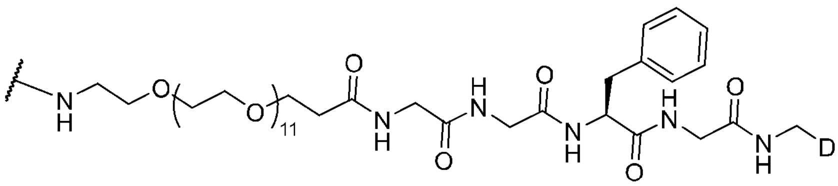

,

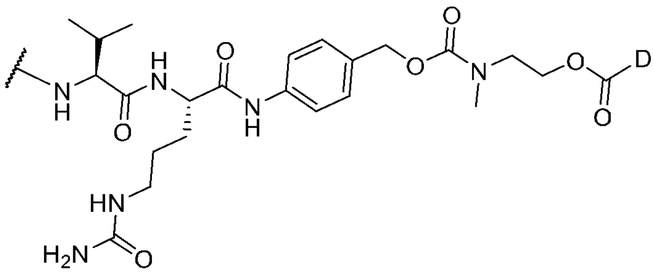

,

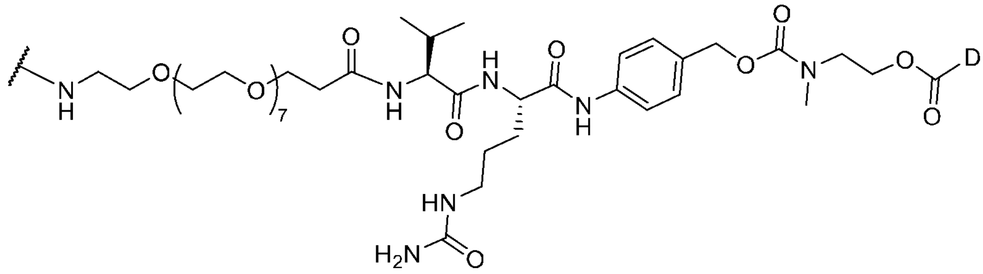

,

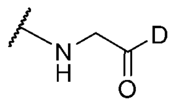

,

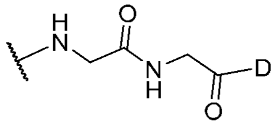

,

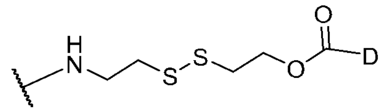

,

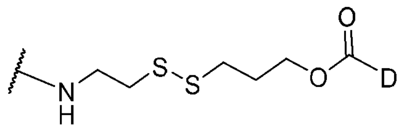

,

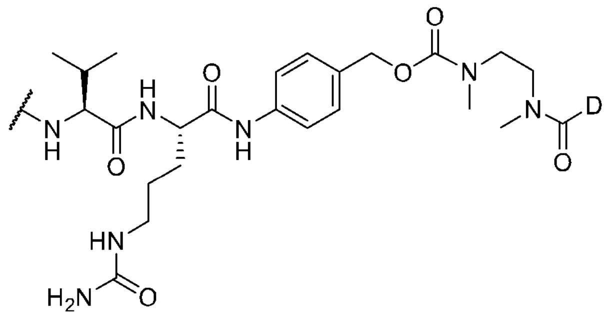

,

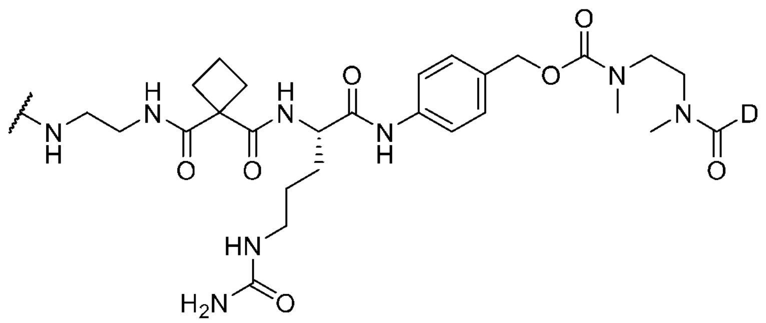

,

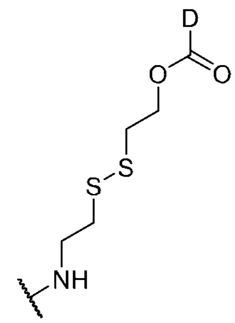

,

or

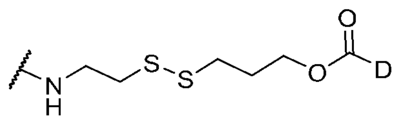

;

(6) D is the pharmaceutically active agent residue as defined in any one of claims 23-28.

**39.** The compound of formula (I-1) or the pharmaceutically acceptable salt thereof according to claim 35, wherein one or more of the following conditions are met:

(1) Y is

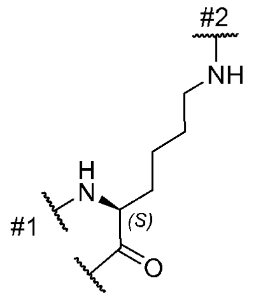

,

and $L^2$ is a covalent bond,

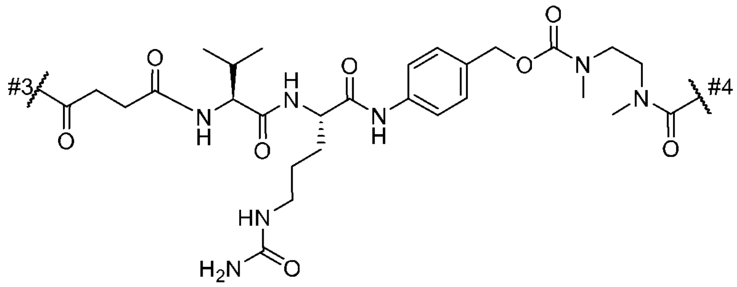

,

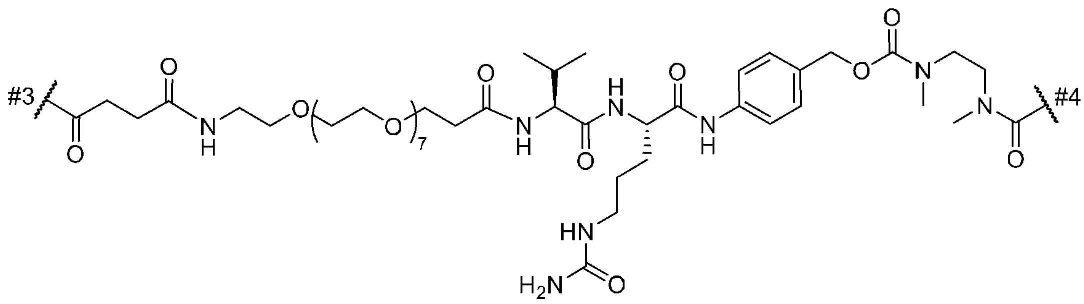

,

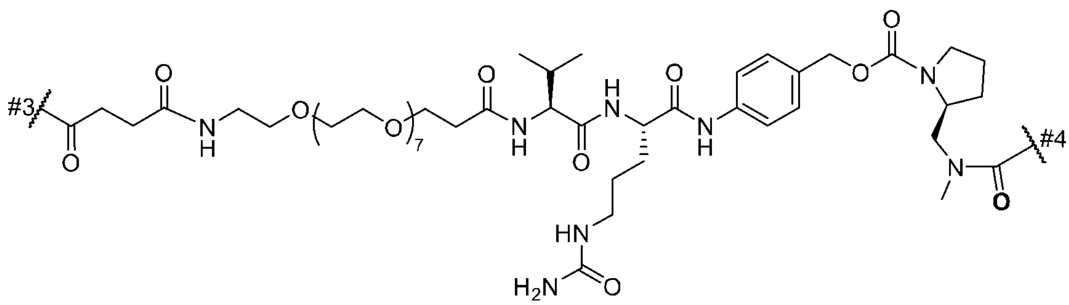

,

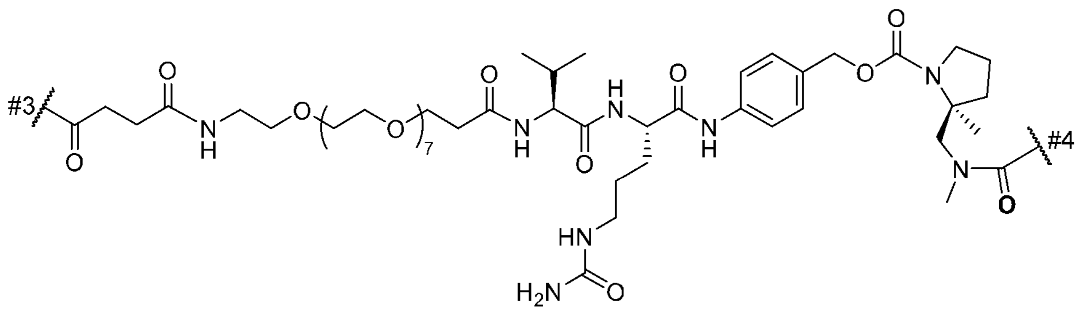

,

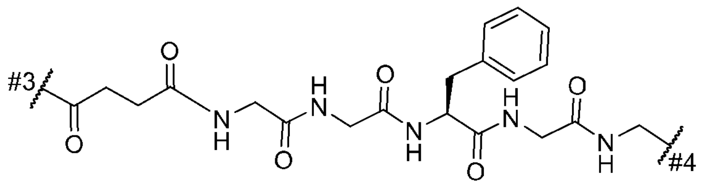

,

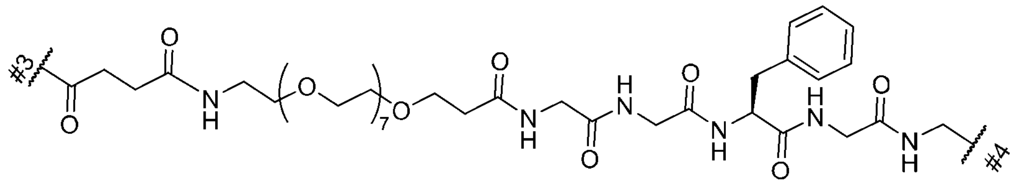

,

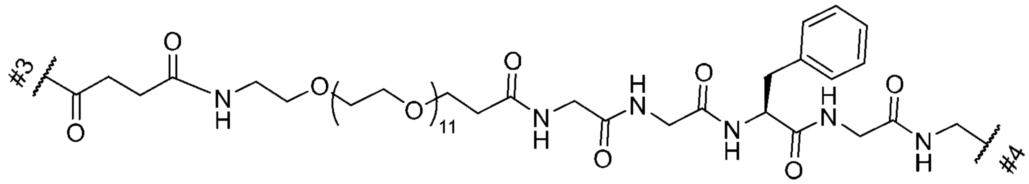

,

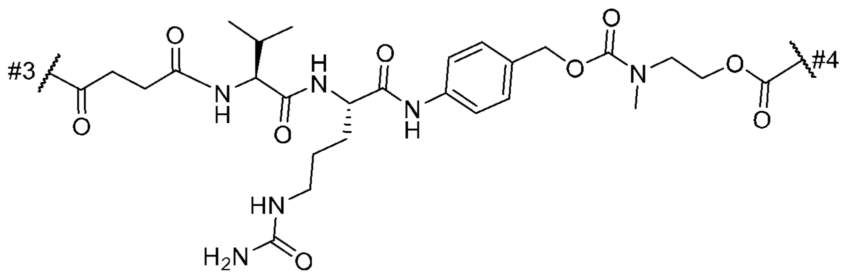

,

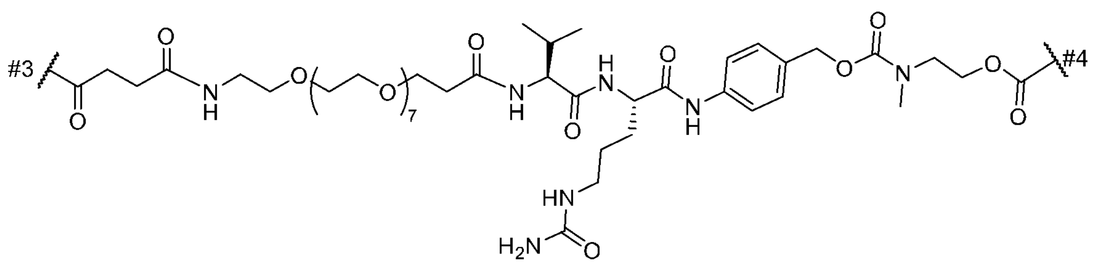

,

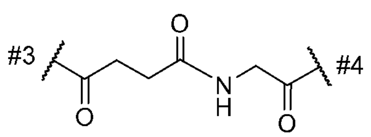

,

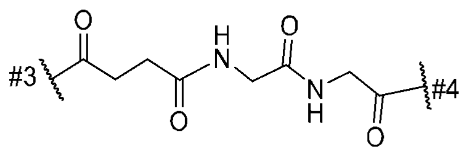

,

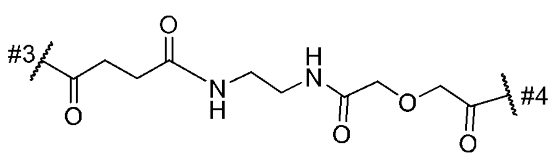

,

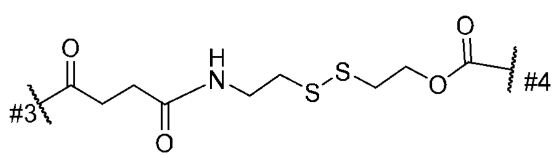

,

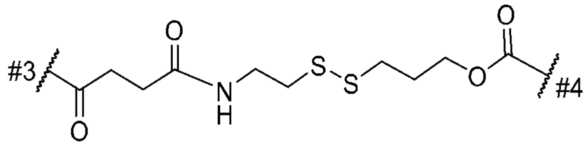, 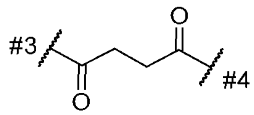, 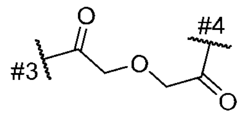,

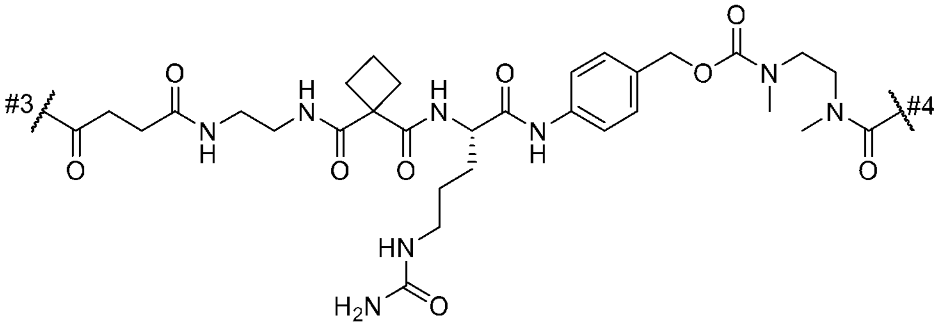,

or

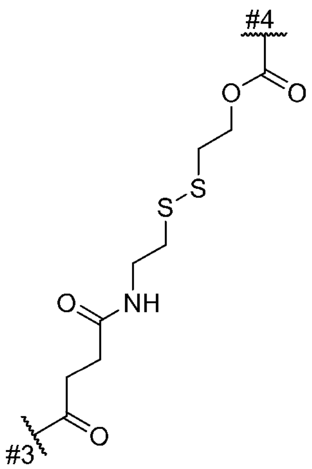;

(2) Y is

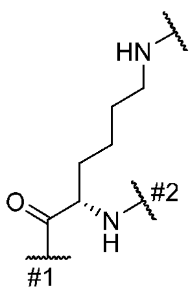,

and $L^2$ is a covalent bond,

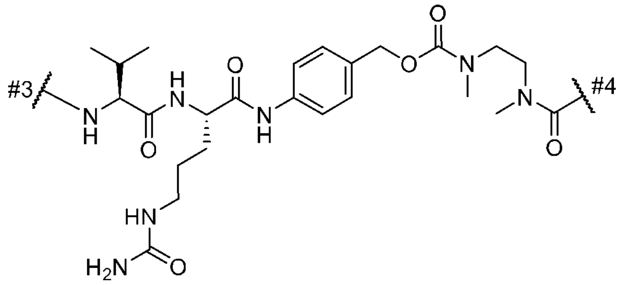

,

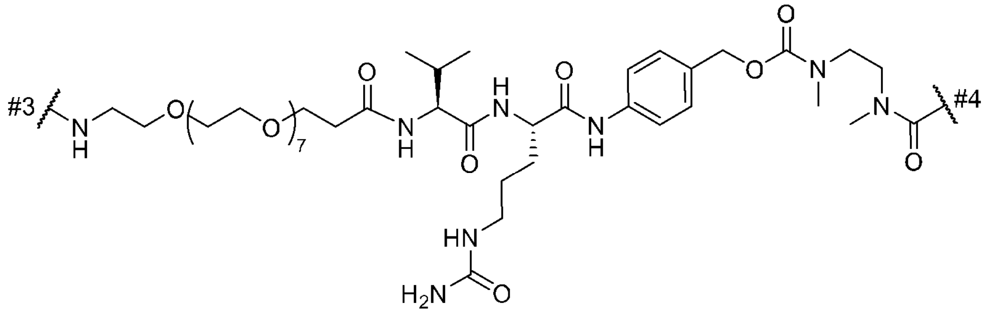

,

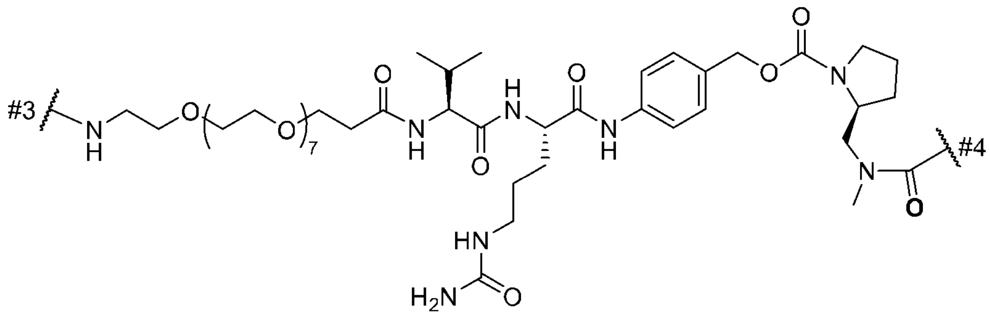

,

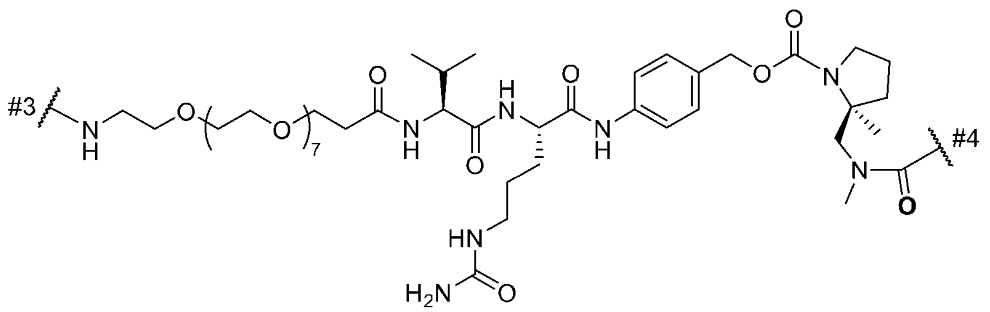

,

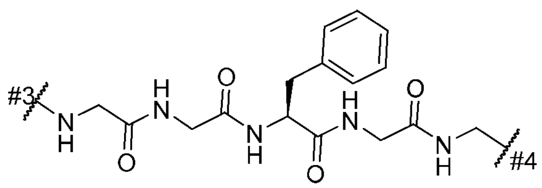

,

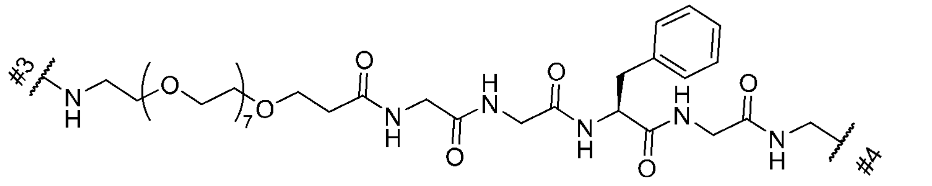

,

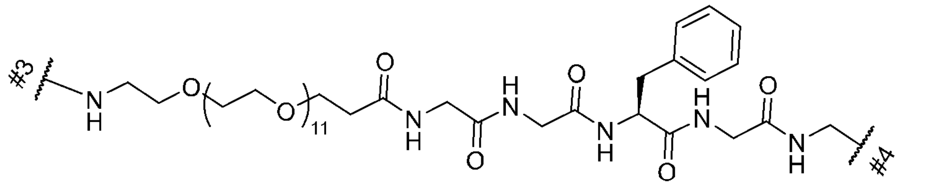

,

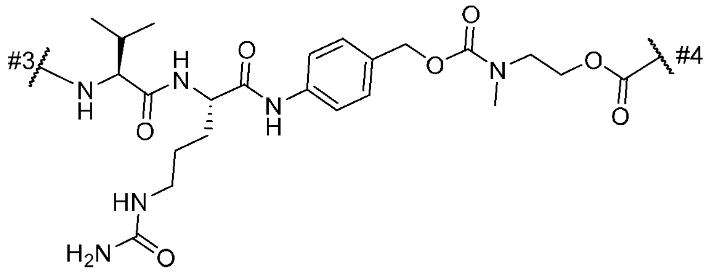

,

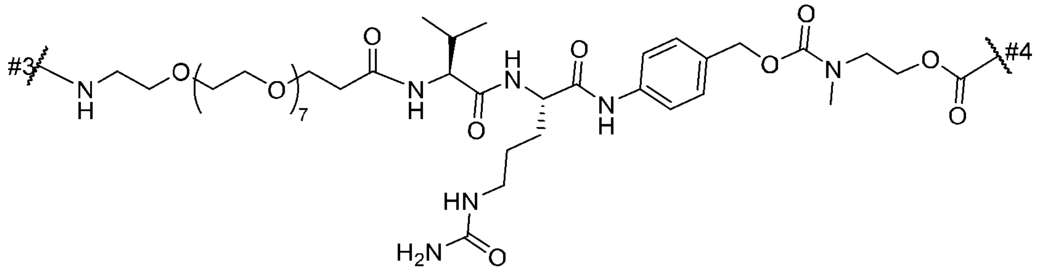

,

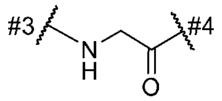 , 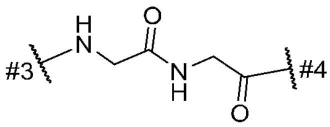 , 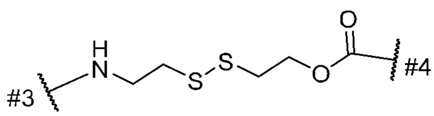 ,

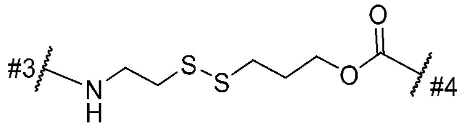 , 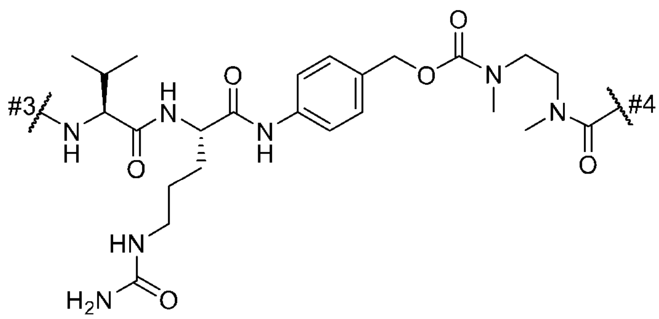 ,

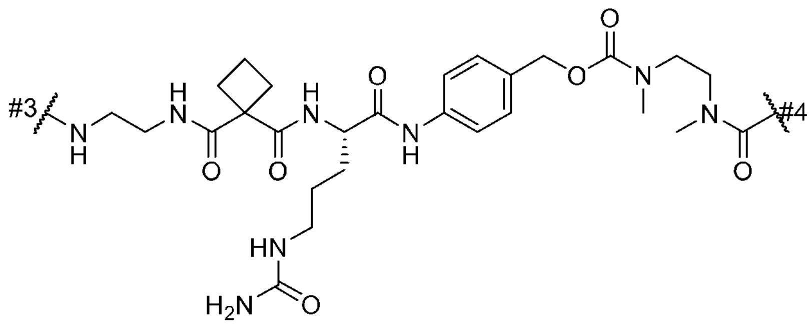

,

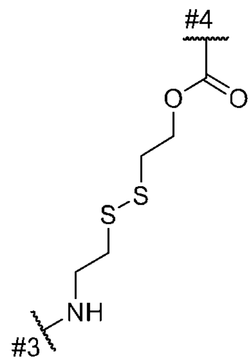

,

or

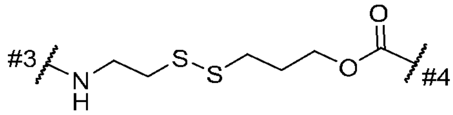

;

(3) D is a residue of a drug having an active functional group, and the active functional group is selected from one or more of primary amine group, secondary amine group, hydroxy, and sulfhydryl; preferably, the drug is an antineoplastic, e.g., one or more of camptothecin, a camptothecin derivative, paclitaxel, a paclitaxel derivative, cisplatin, and a cisplatin derivative; D is, e.g.,

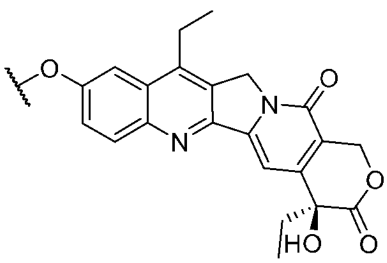

,

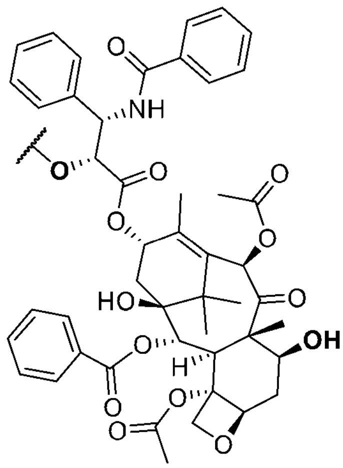

,

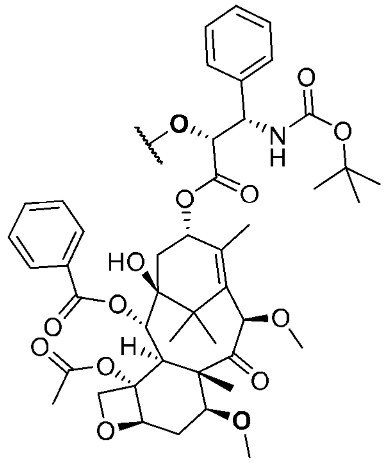

,

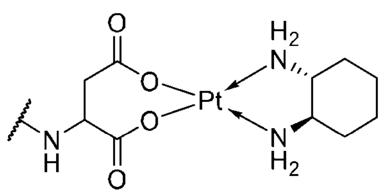

,

or

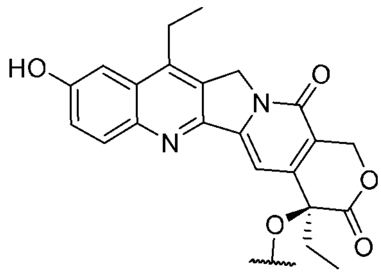

.

**40.** The compound of formula (I-1) or the pharmaceutically acceptable salt thereof according to any one of claims 35-39, wherein the compound of formula (I-1) is a compound of formula (A) or formula (B):

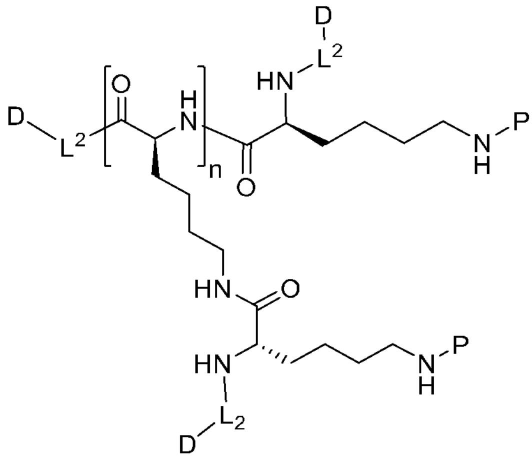

(A)

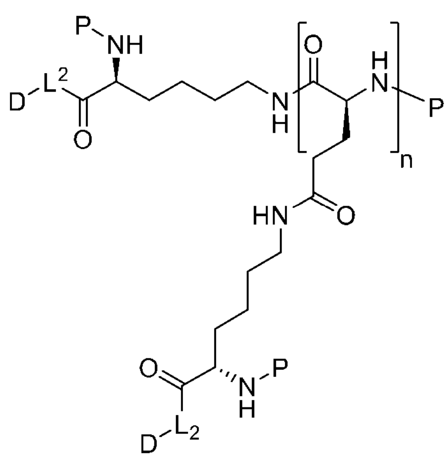

(B)

;

in the compound of formula (A) or formula (B), the variables are as defined in any one of claims 35-39.

**41.** A compound of any one of the following structures:

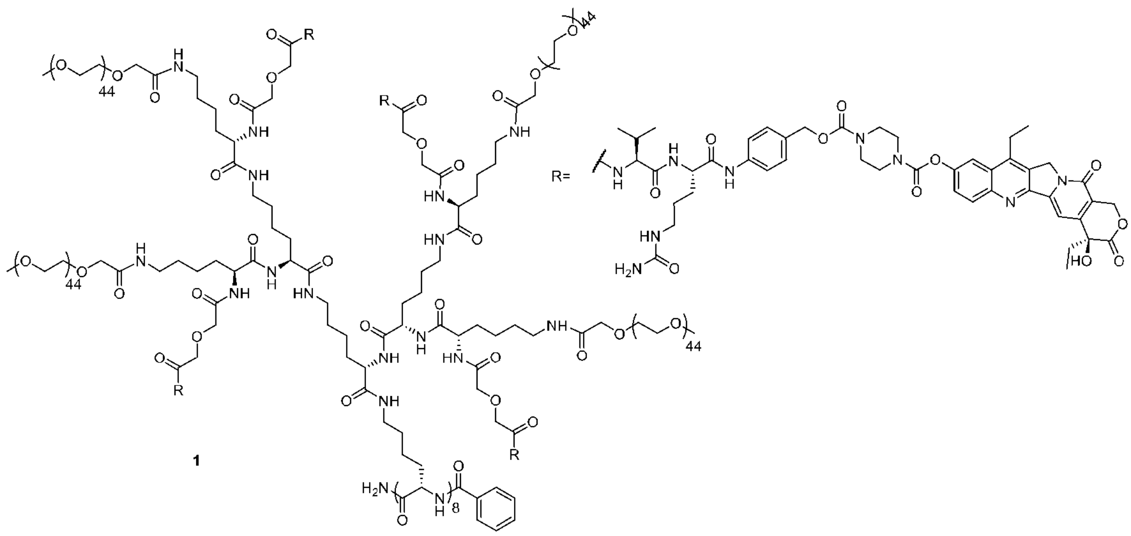

1

,

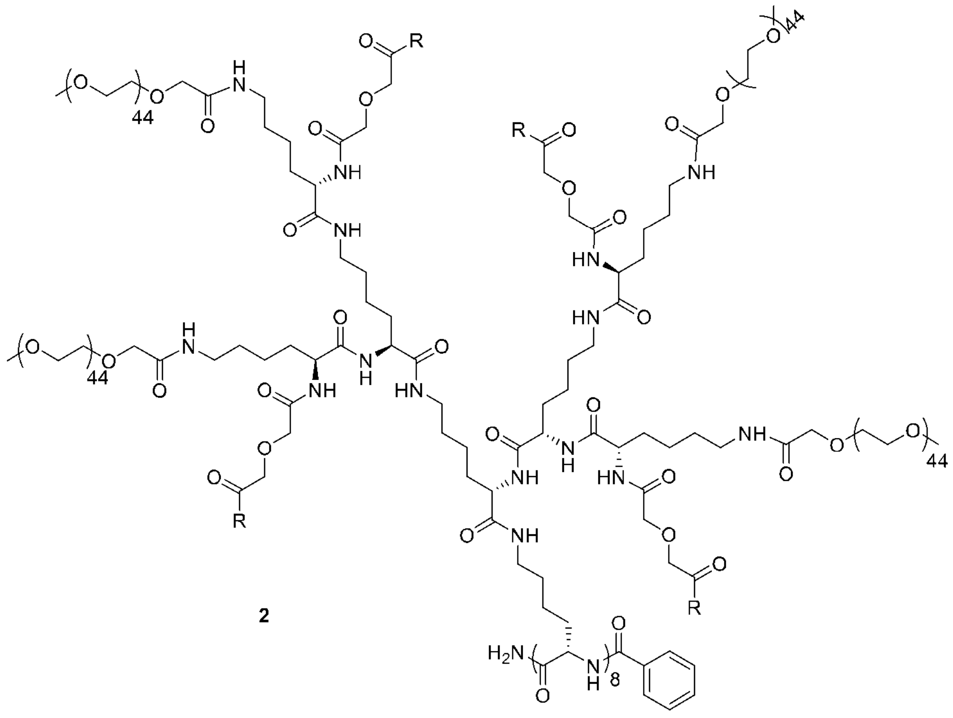

2

R= 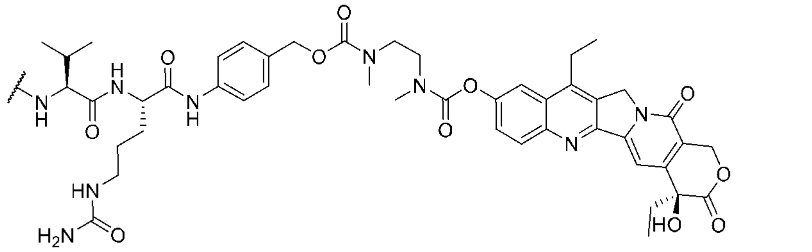

,

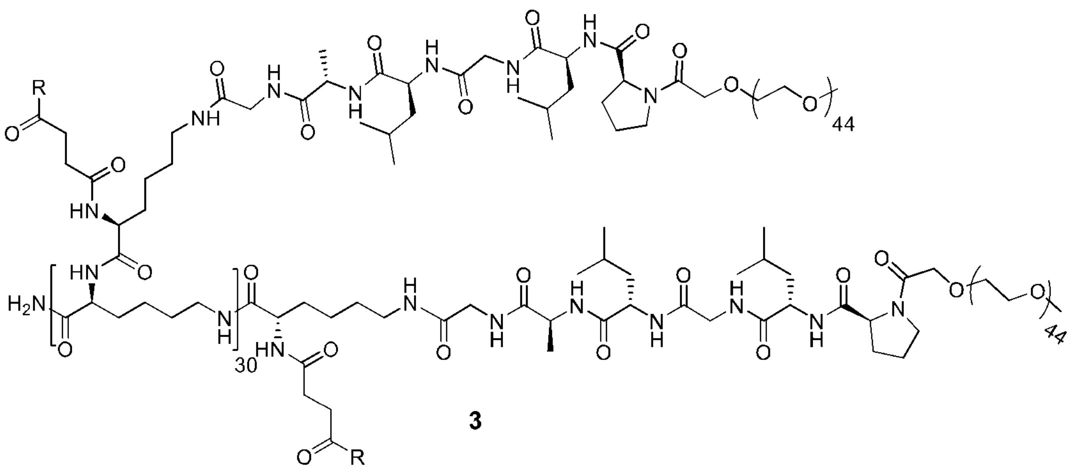

3

R =

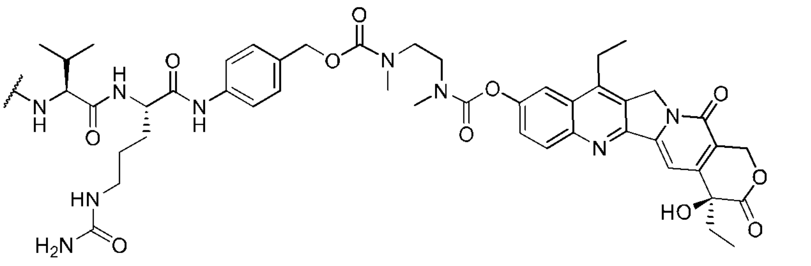

,

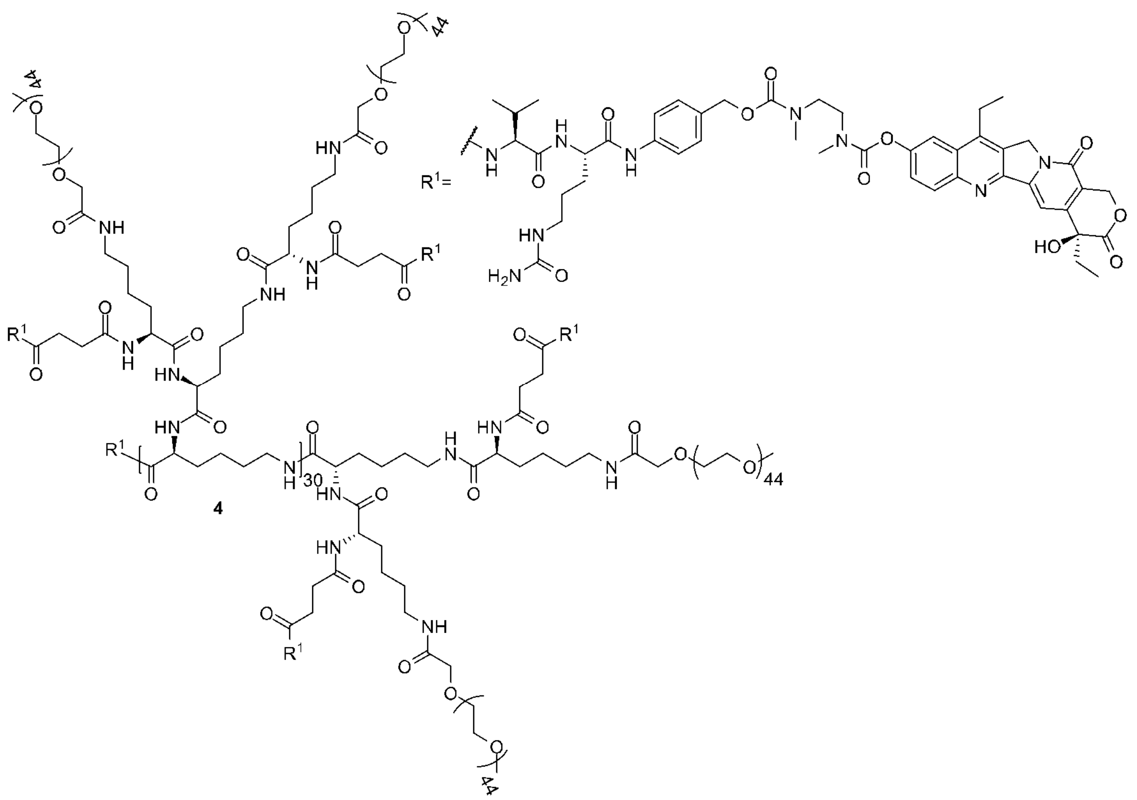

$R^1$ =

4

,

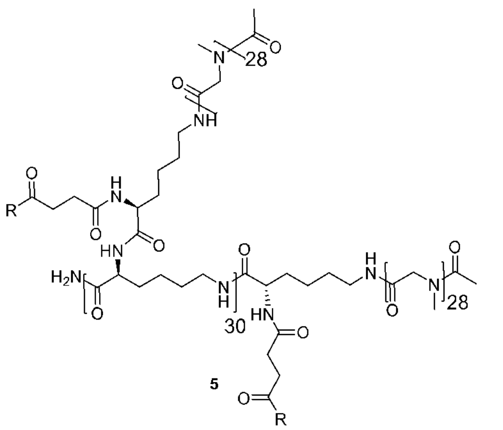

5

R =

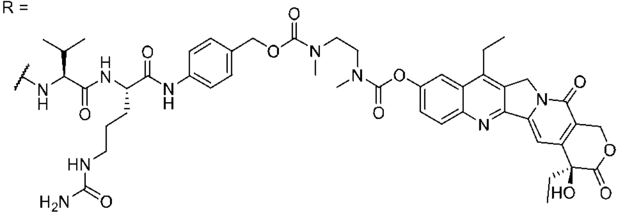

,

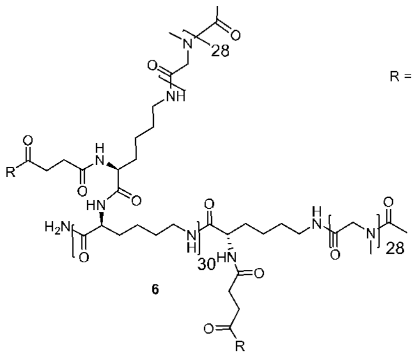

6

R =

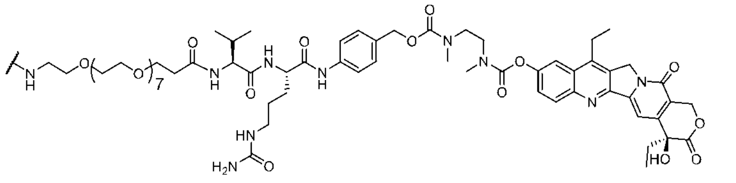

,

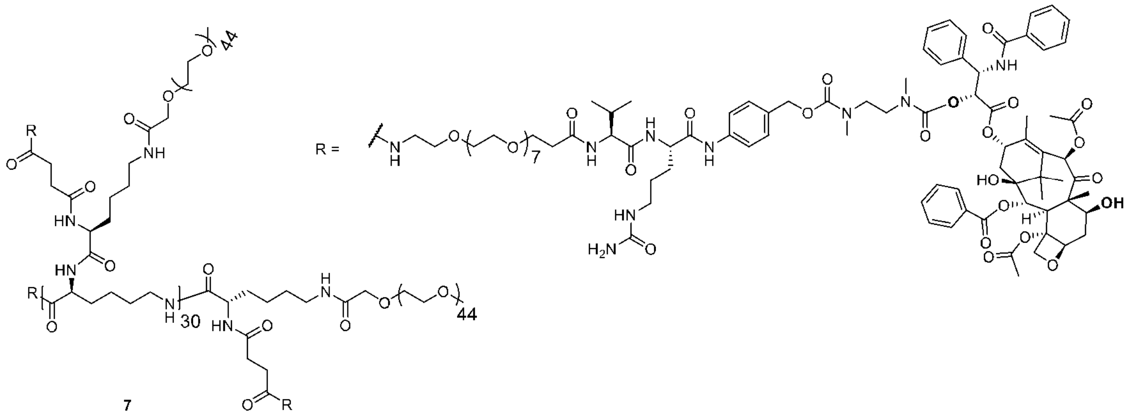

7

R =

,

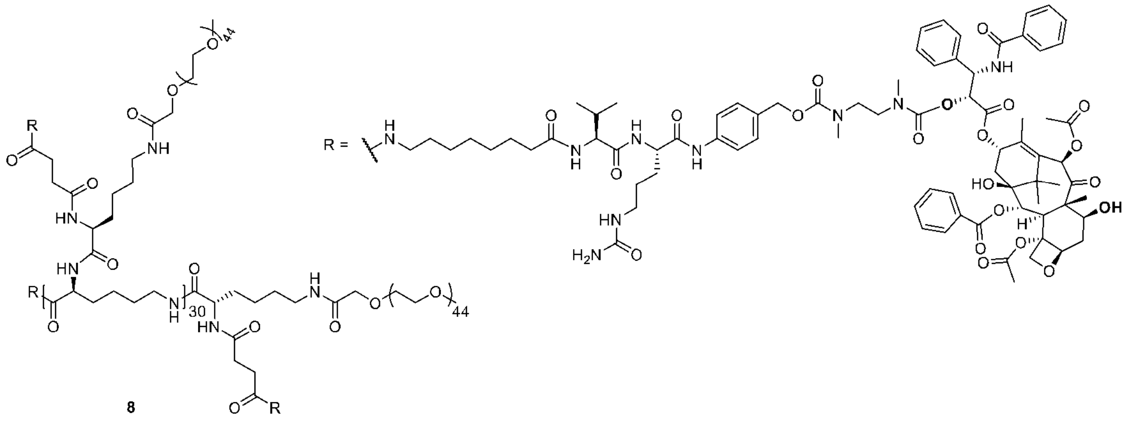

8

R =

,

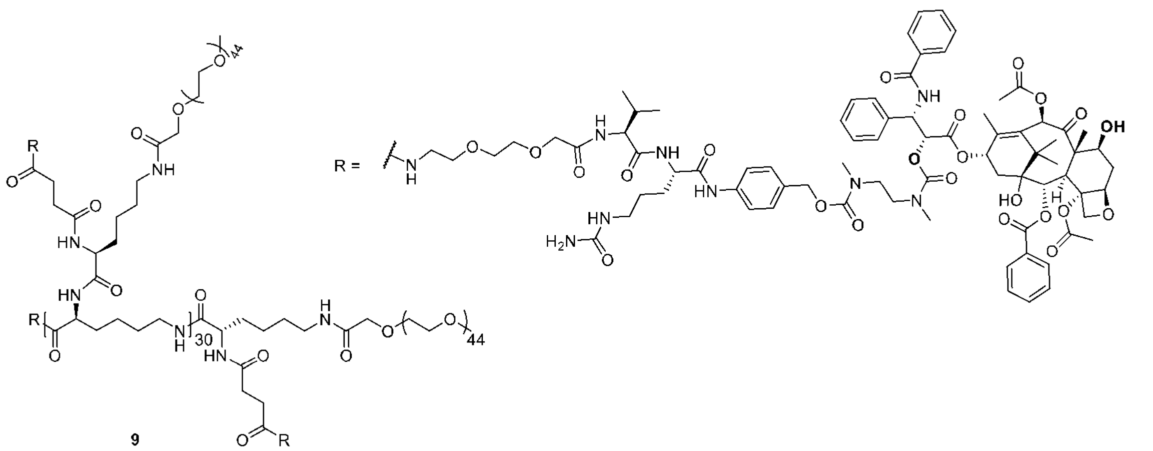

9

R =

,

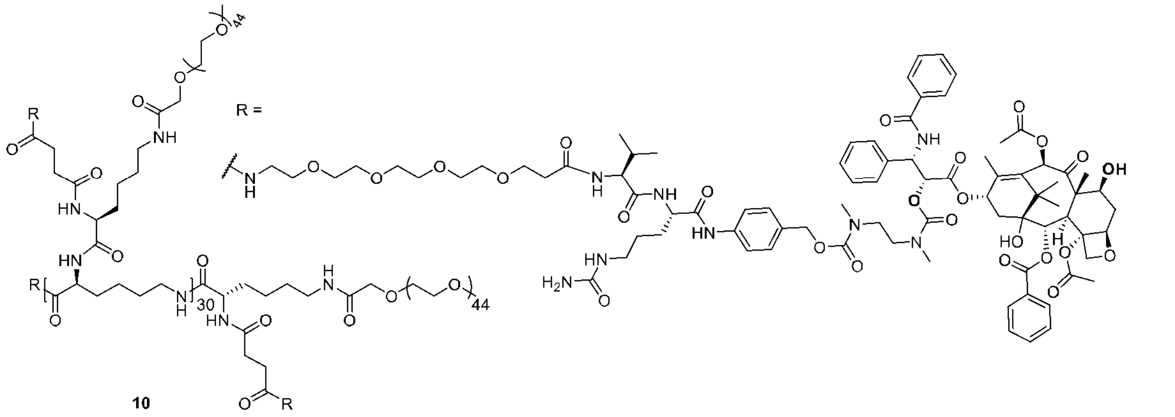

10

,

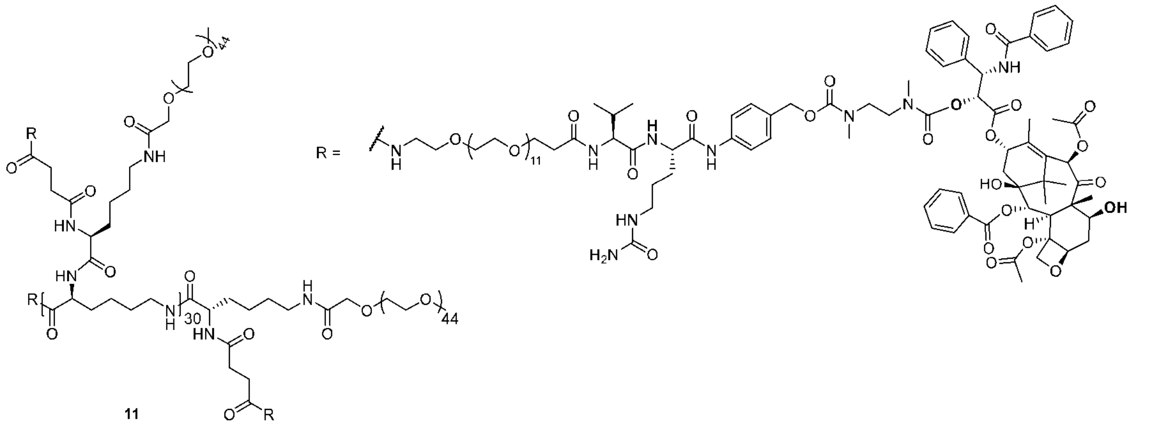

11

,

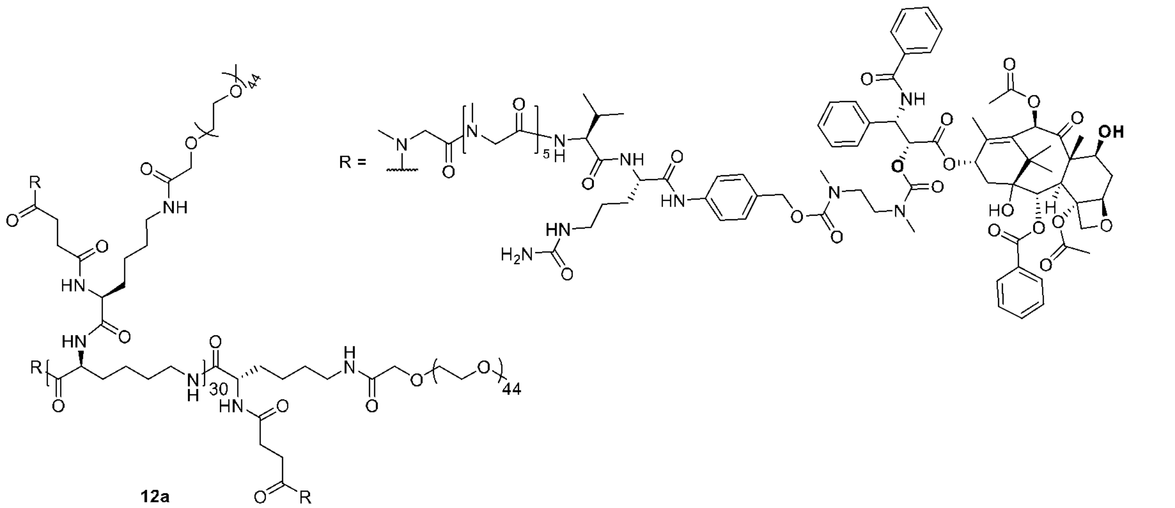

12a

,

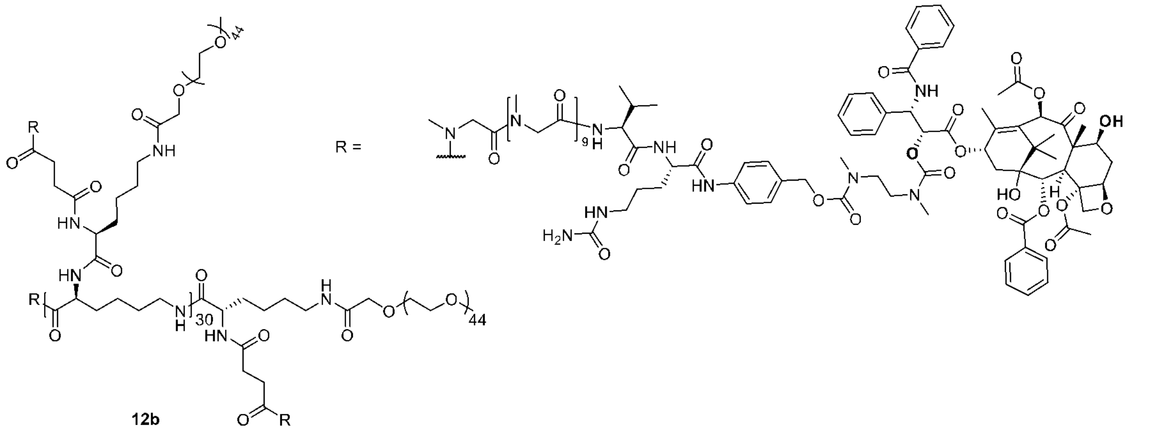

12b

,

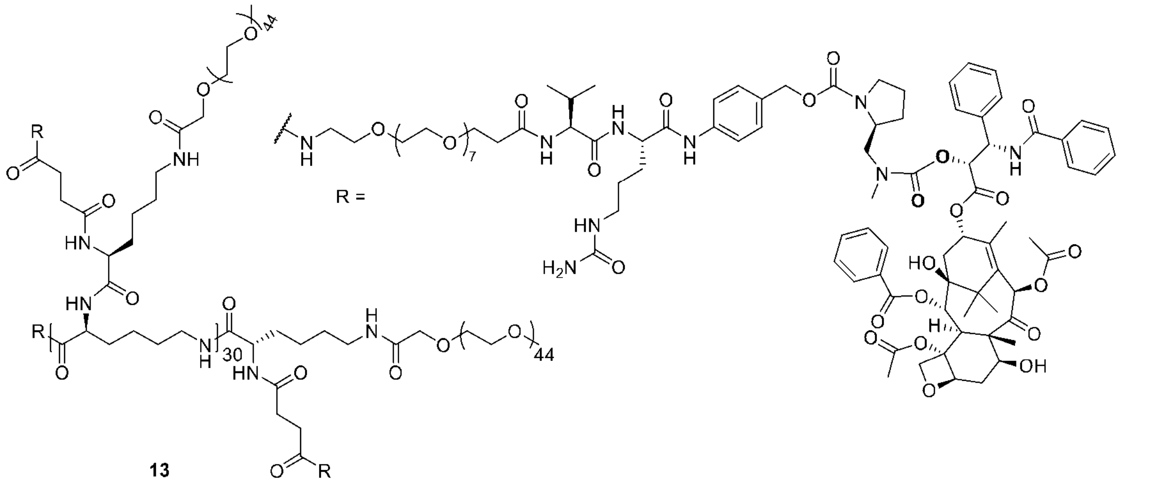

13

,

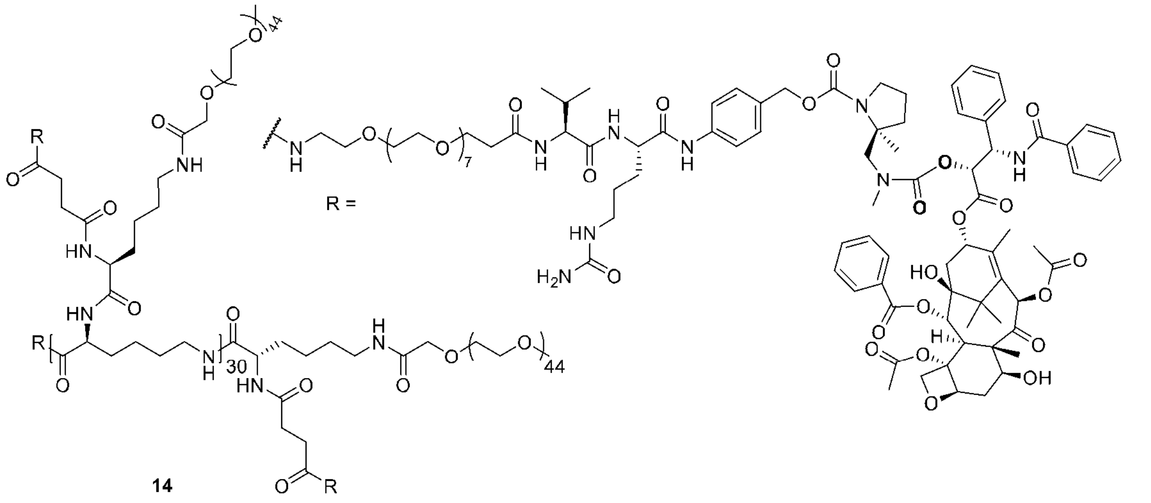

14

,

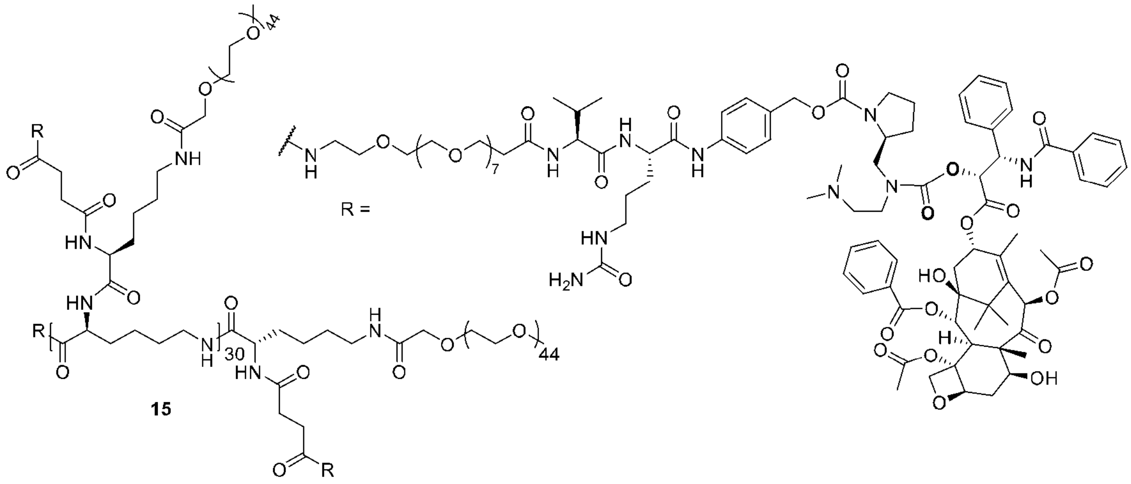

R =

15

,

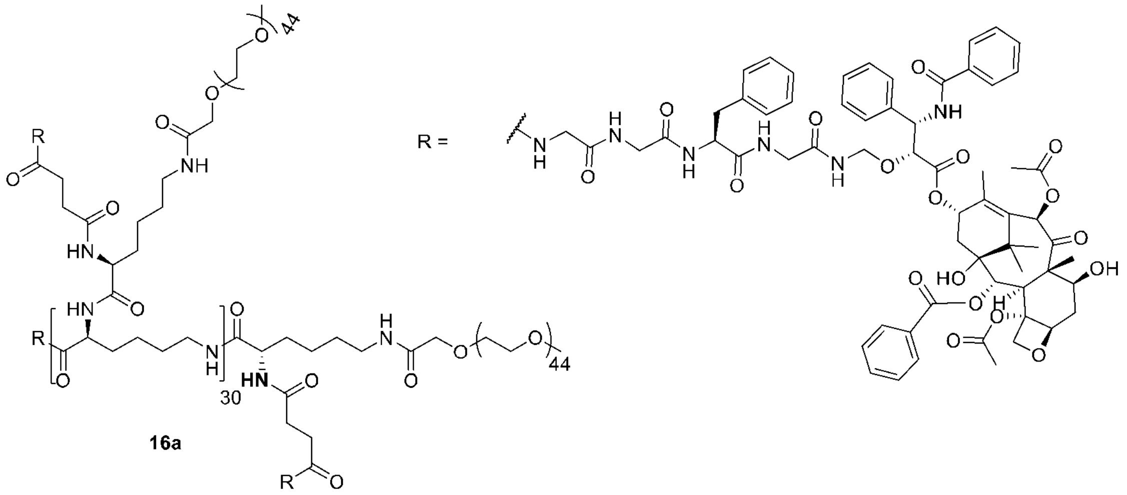

R =

16a

,

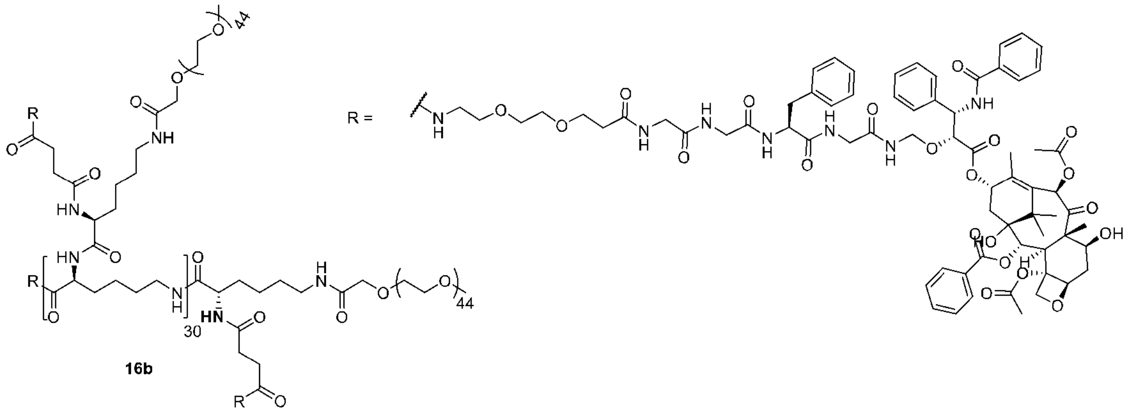

R =

16b

,

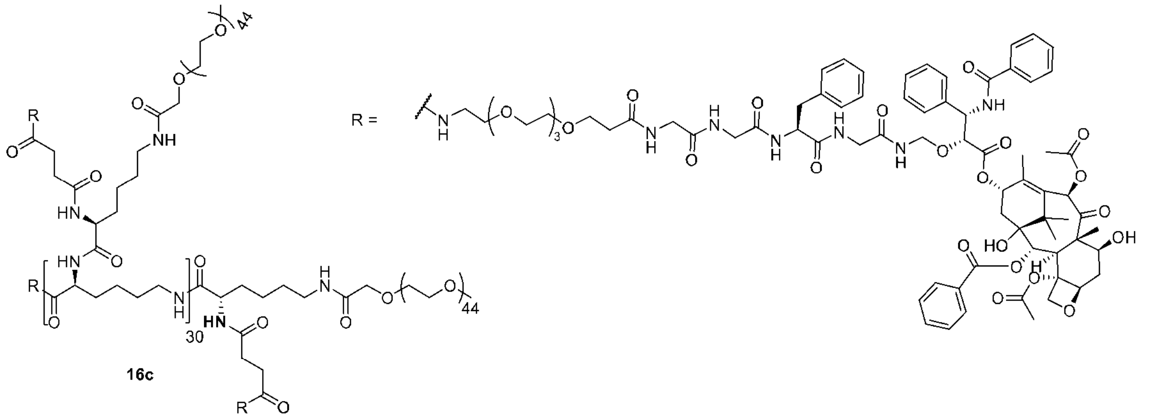

R =

16c

,

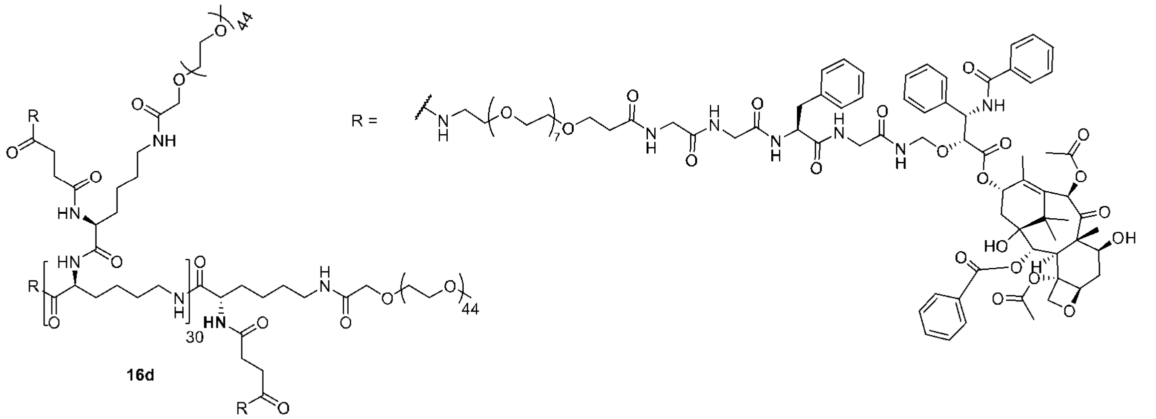

R =

16d

,

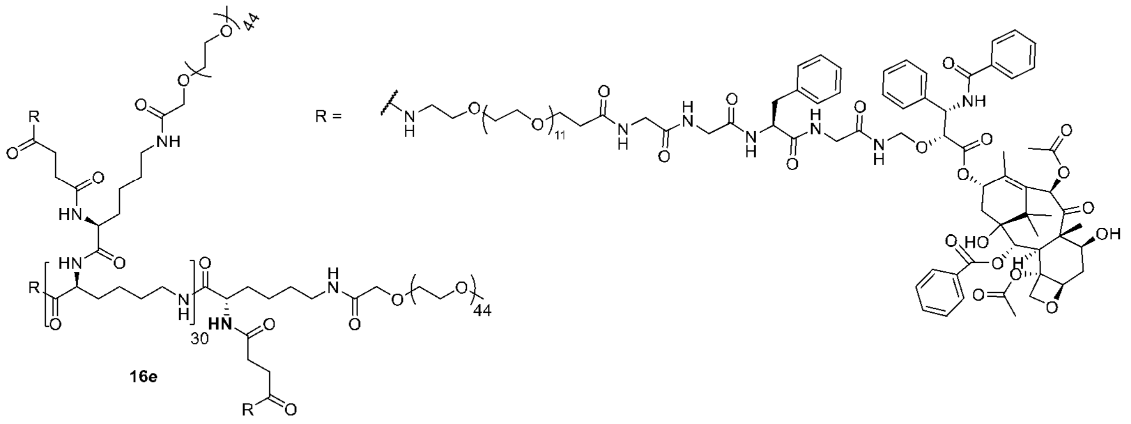

R =

16e

,

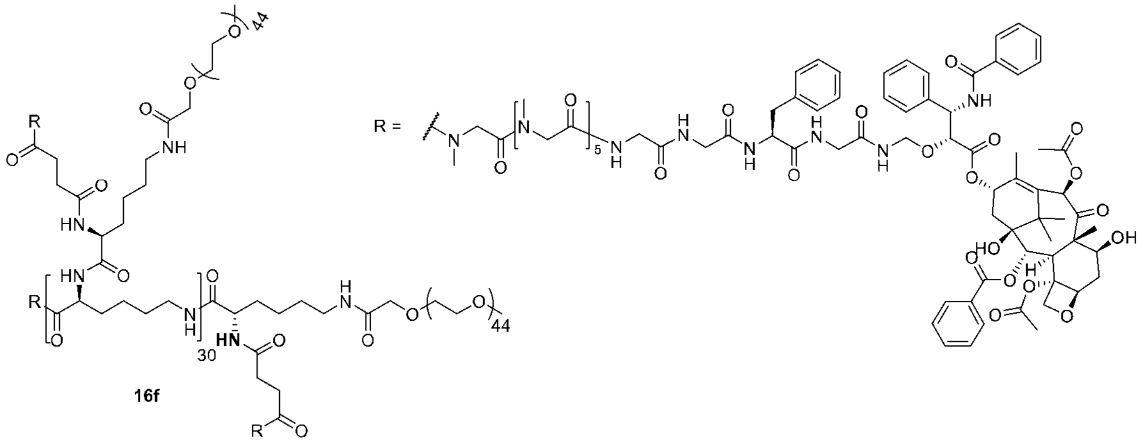

16f

,

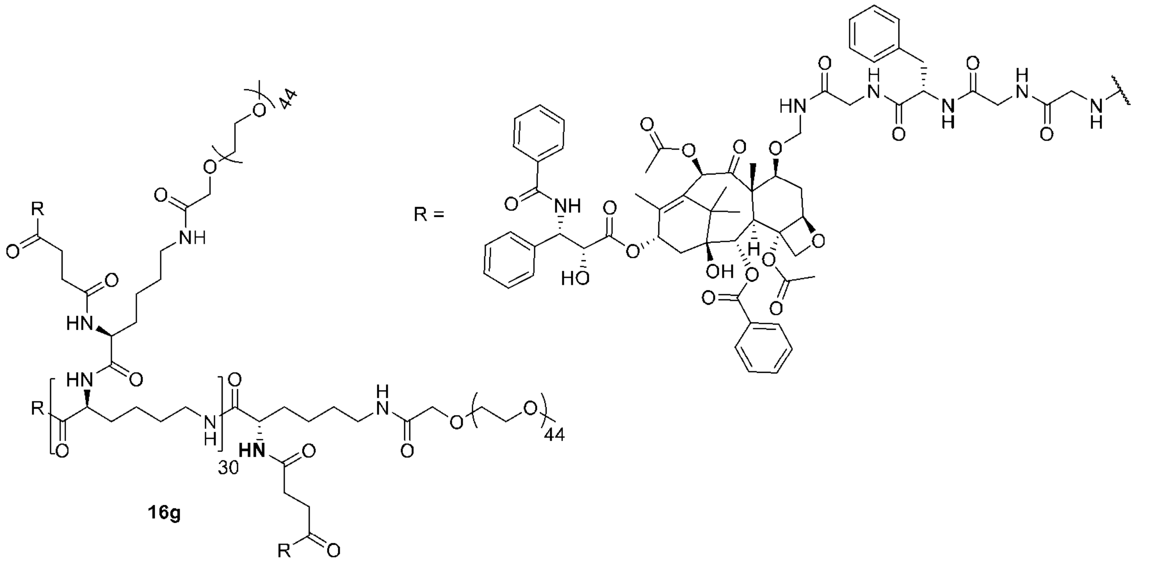

16g

,

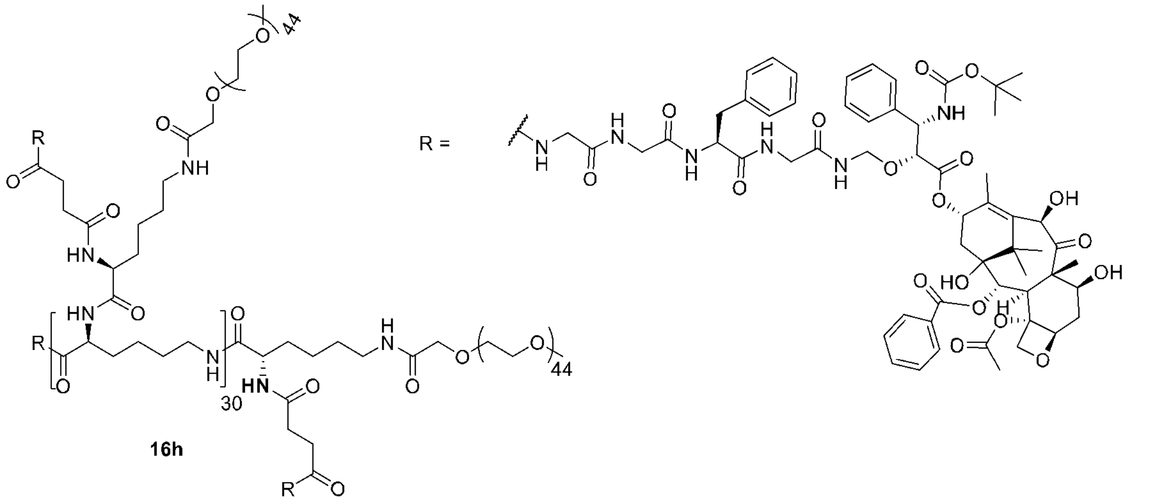

16h

,

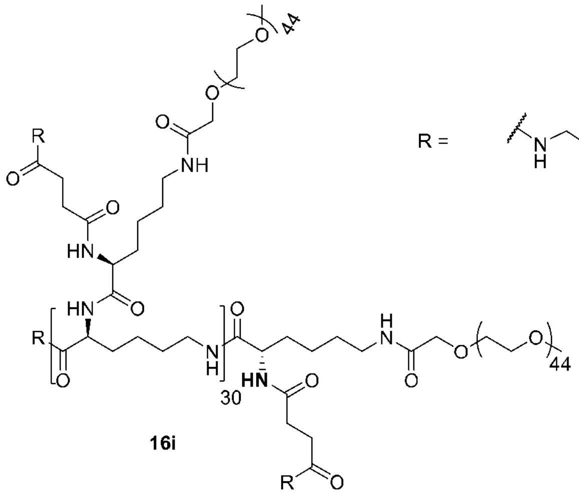

16i

R =

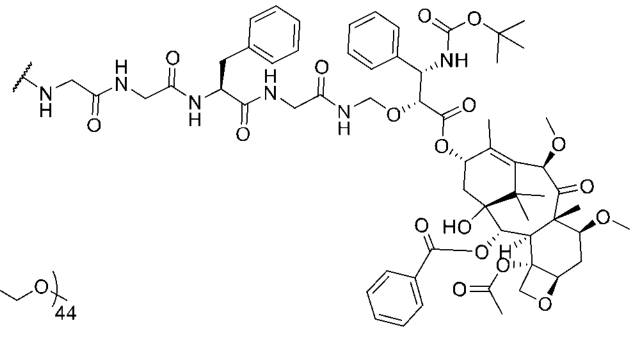

,

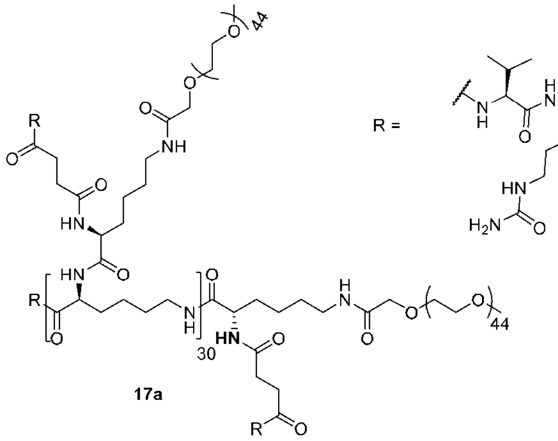

17a

R =

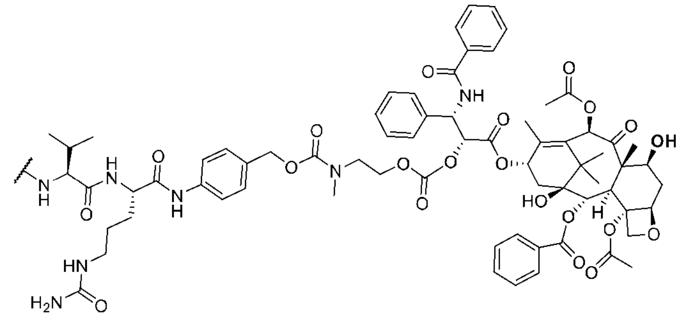

,

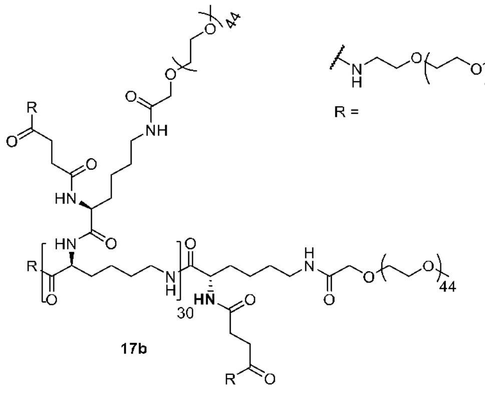

17b

R =

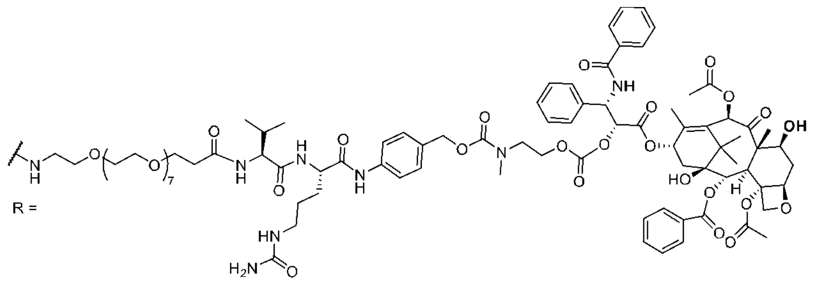

,

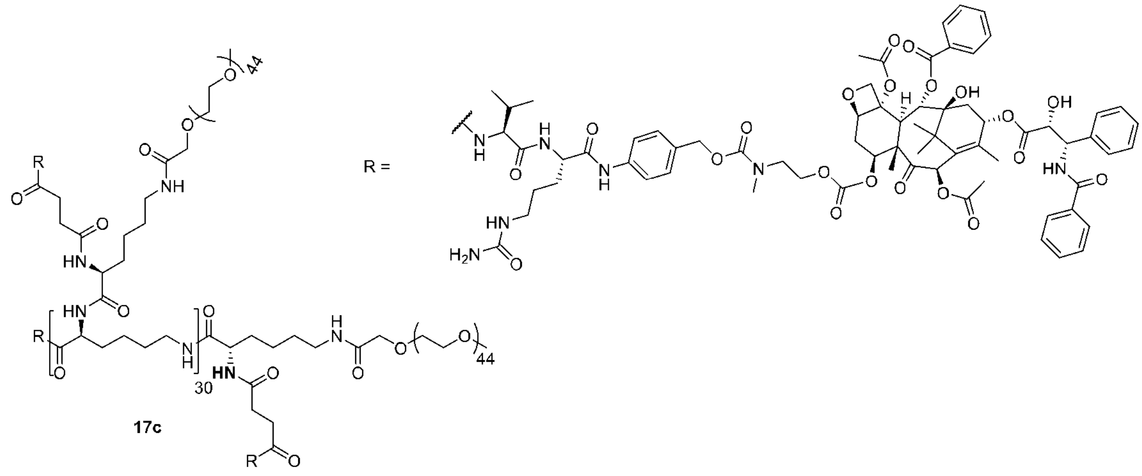

R =

17c

,

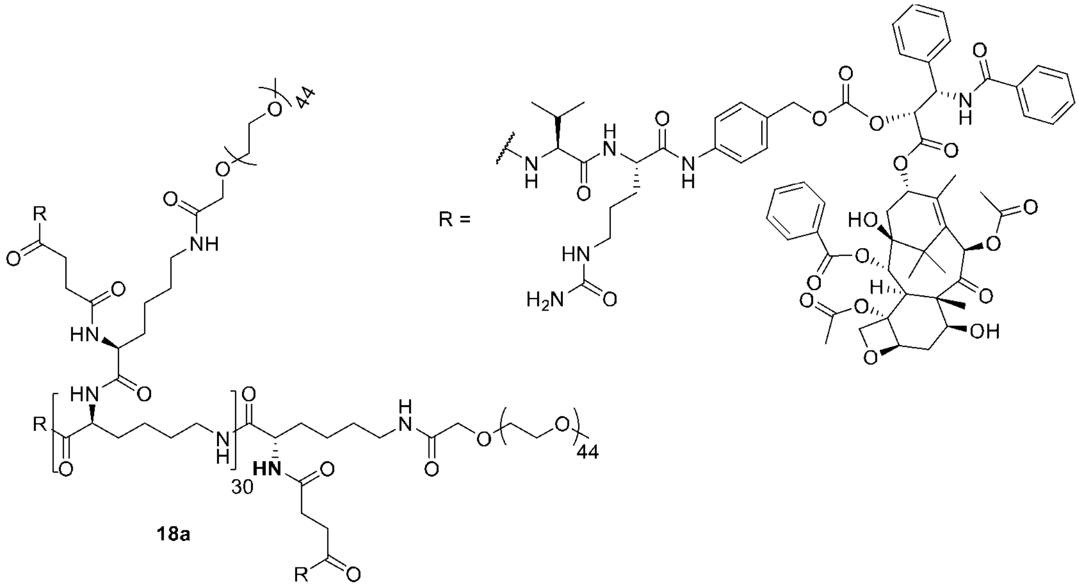

R =

18a

,

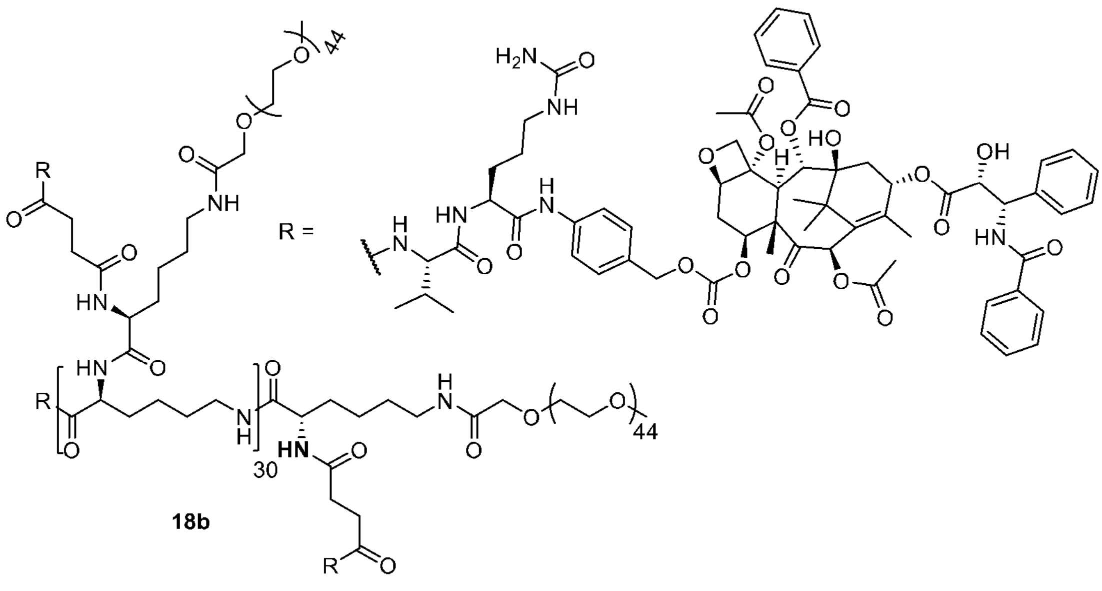

R =

18b

,

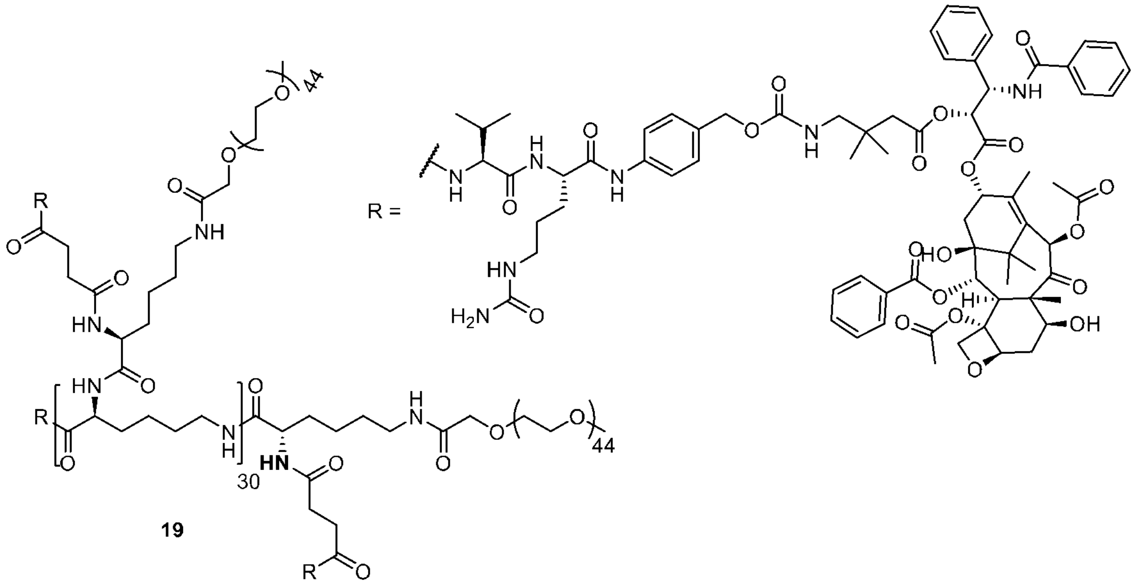

19

,

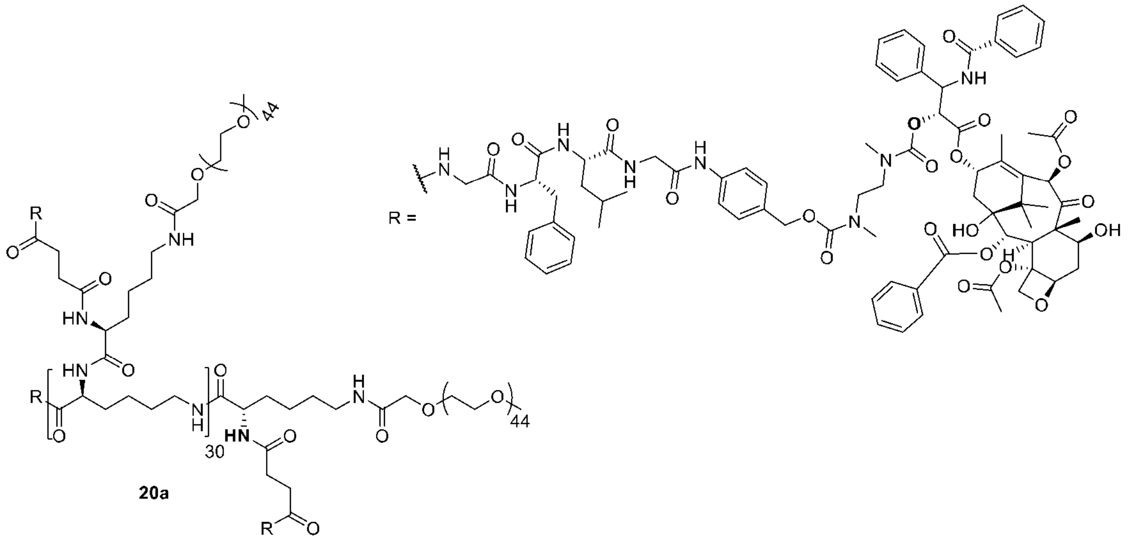

20a

,

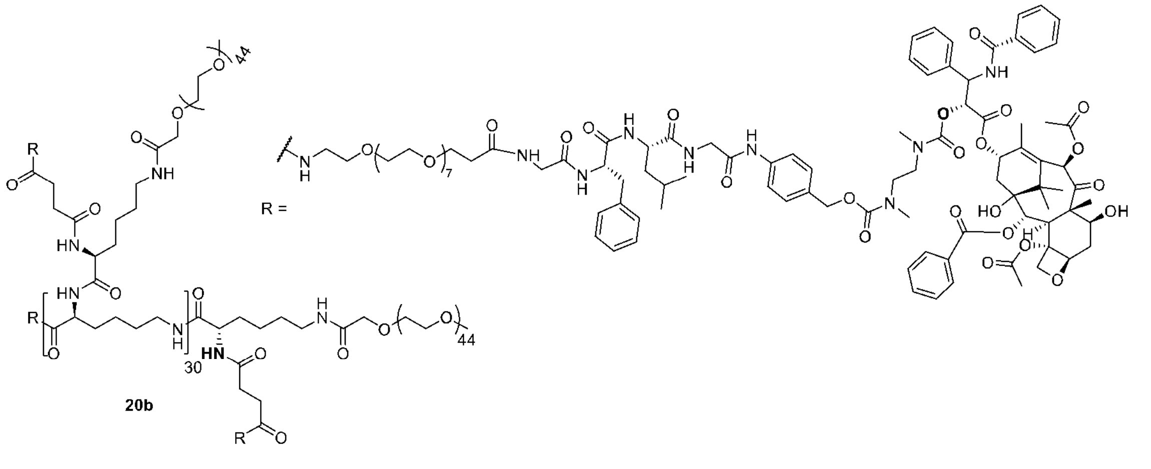

20b

,

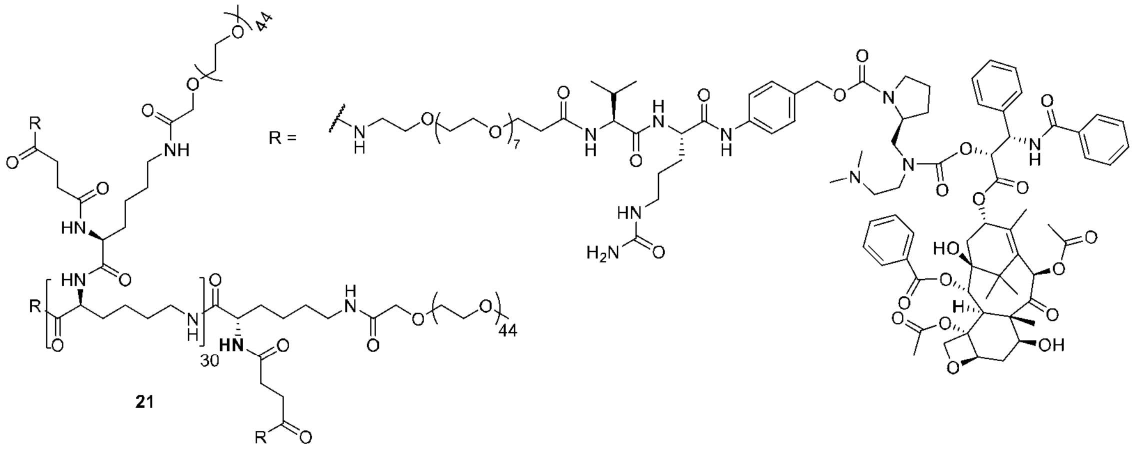

R =

21

,

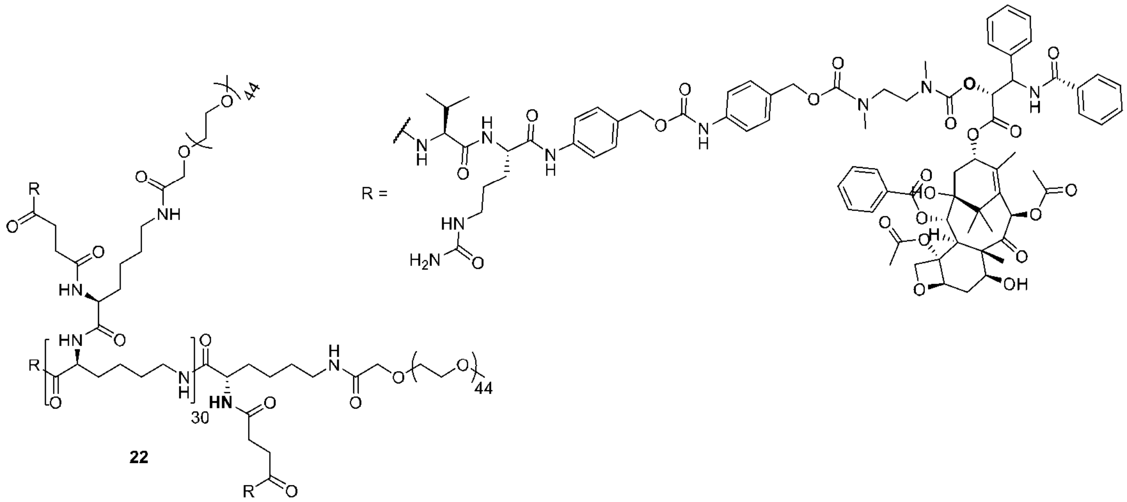

R =

22

,

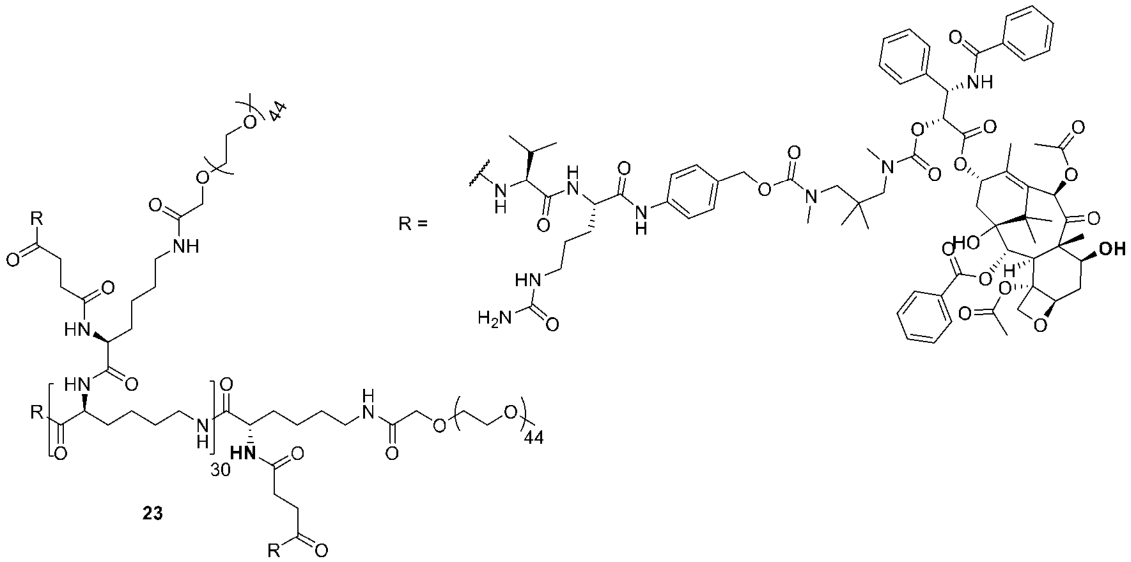

R =

23

,

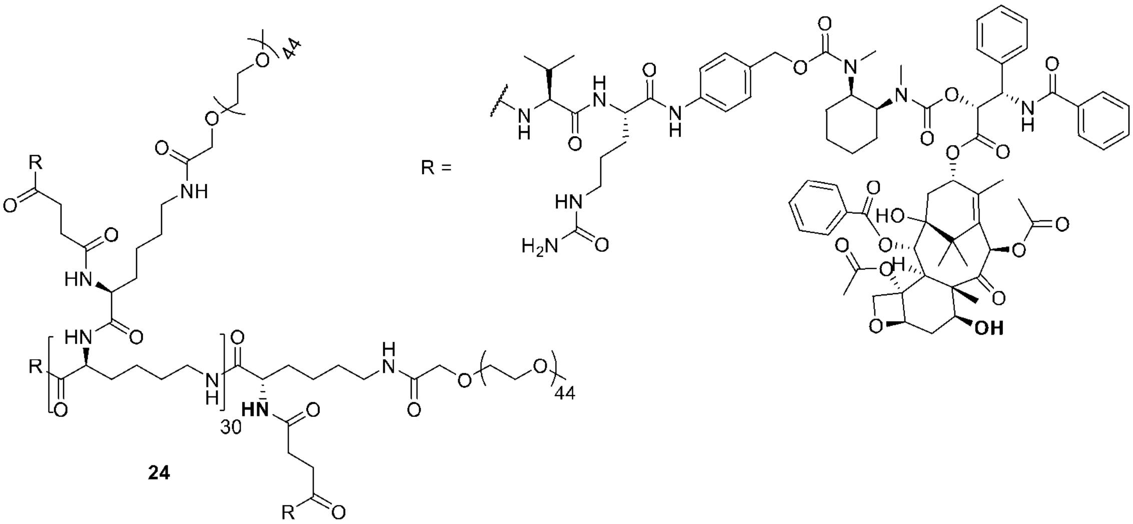

R =

24

,

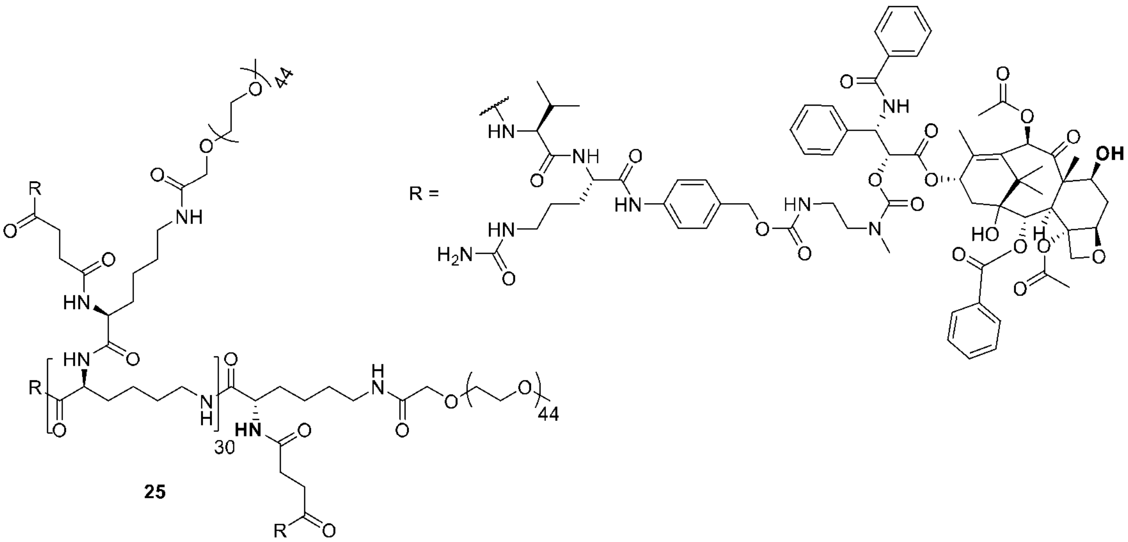

R =

25

,

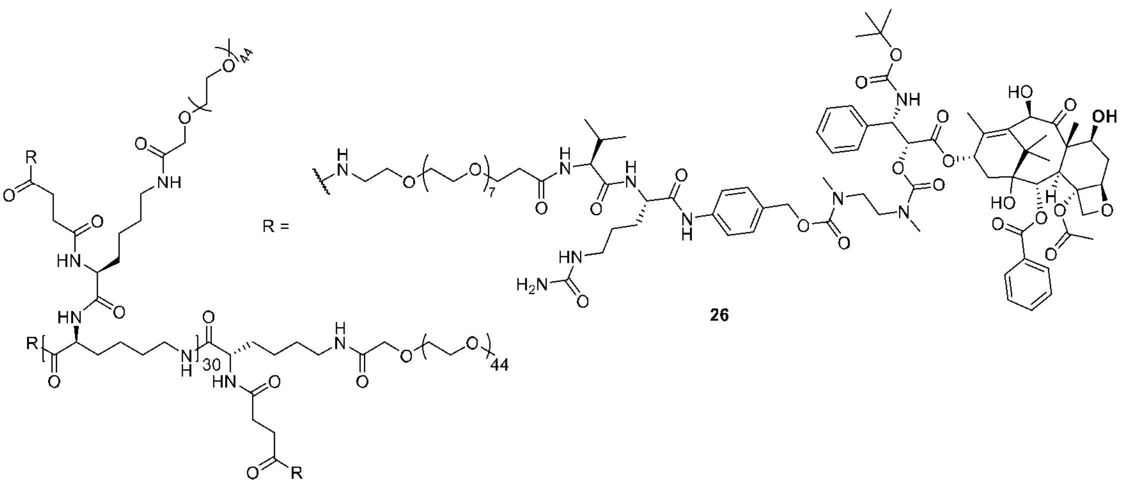

R =

26

,

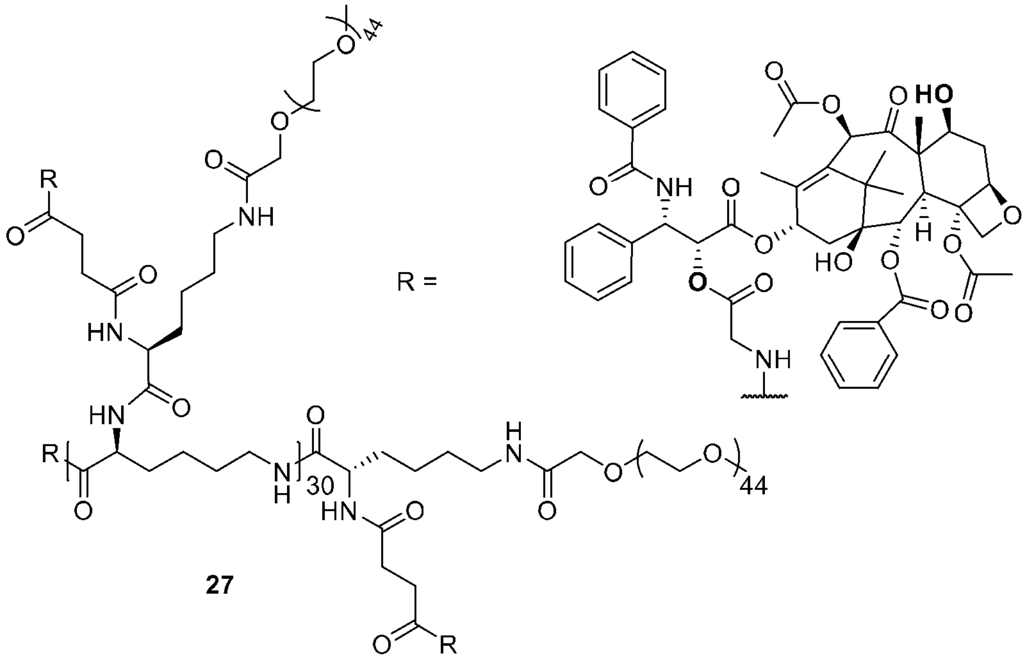

R =

27

,

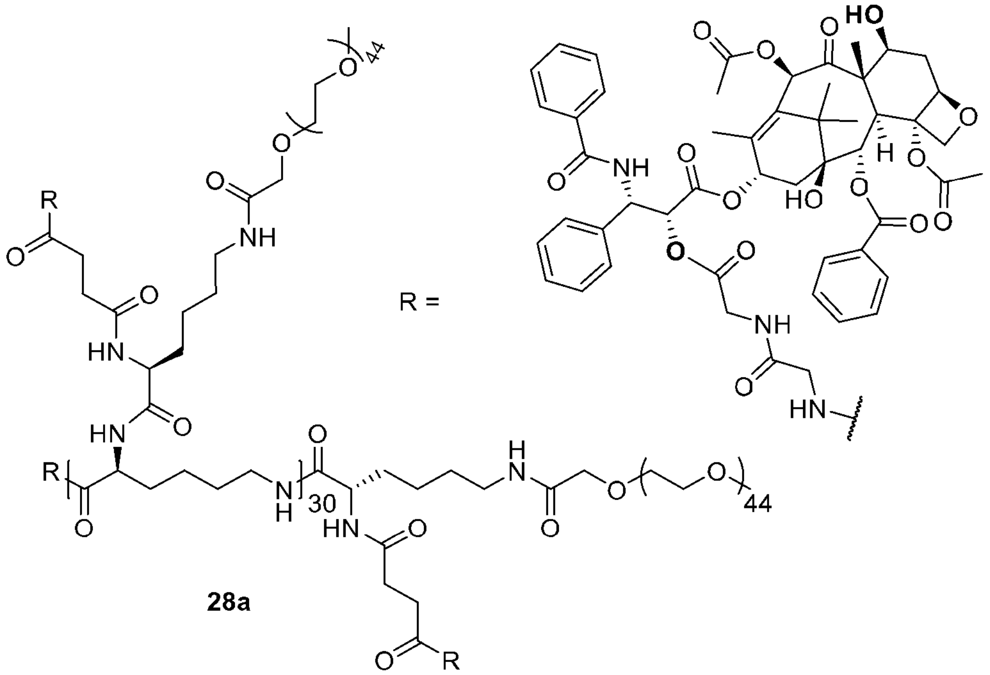

R =

28a

,

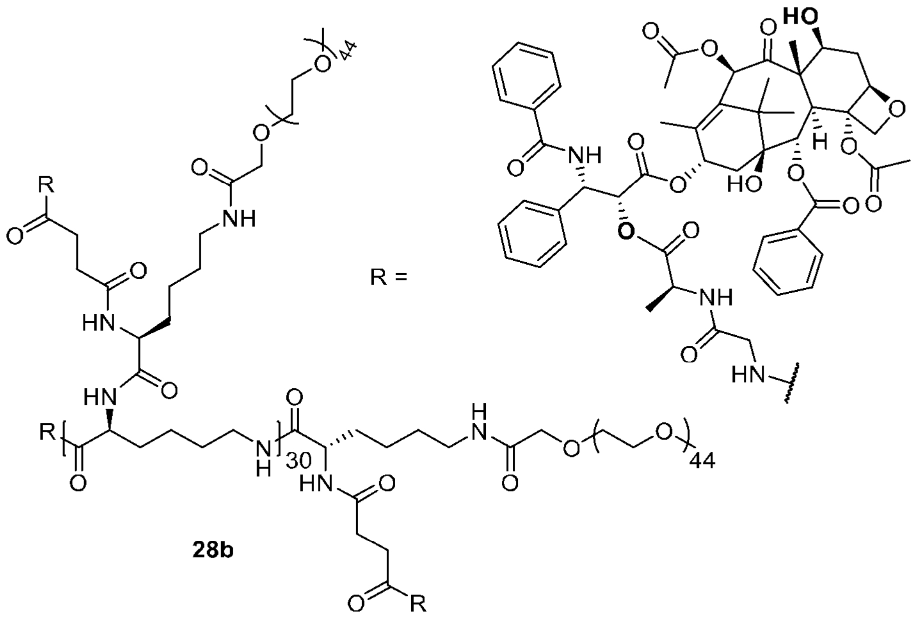

R =

**28b**

,

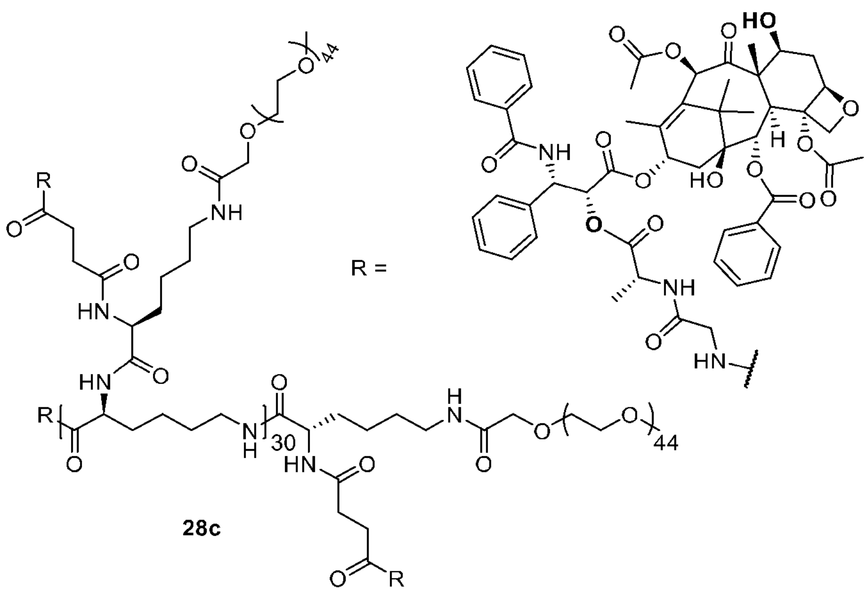

R =

**28c**

,

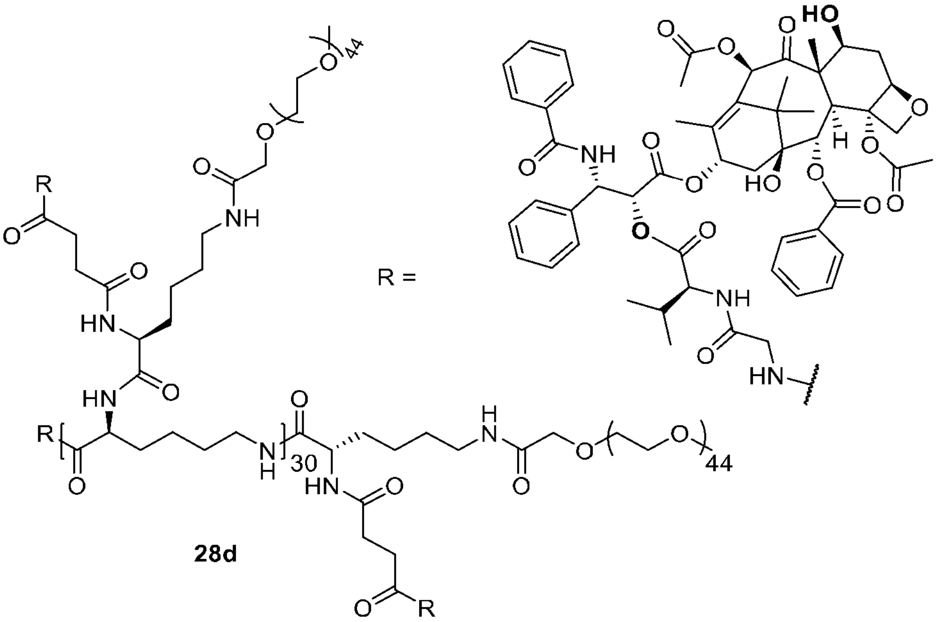

28d

,

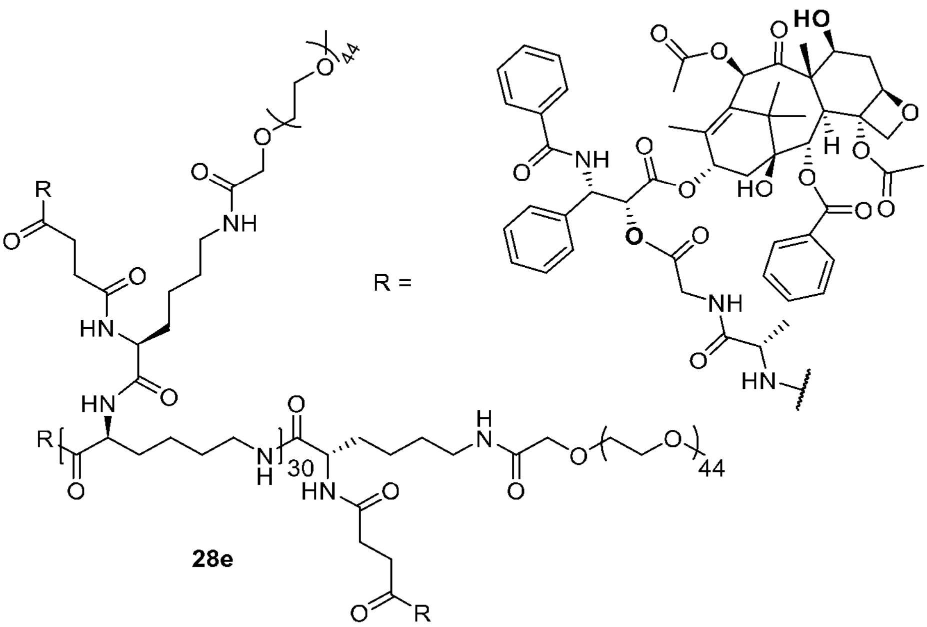

28e

,

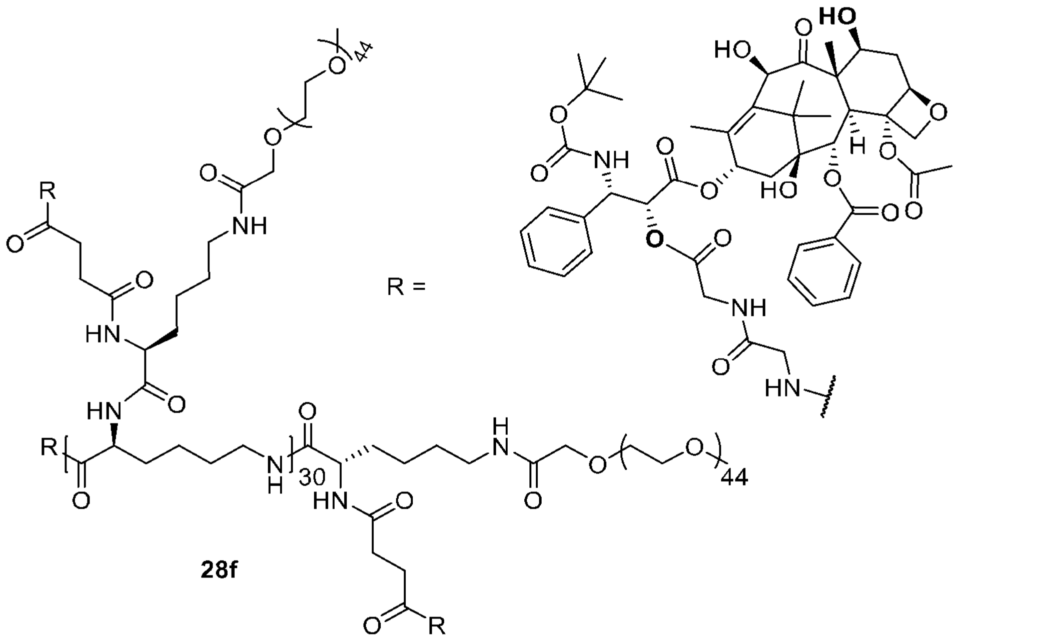

R =

**28f**

,

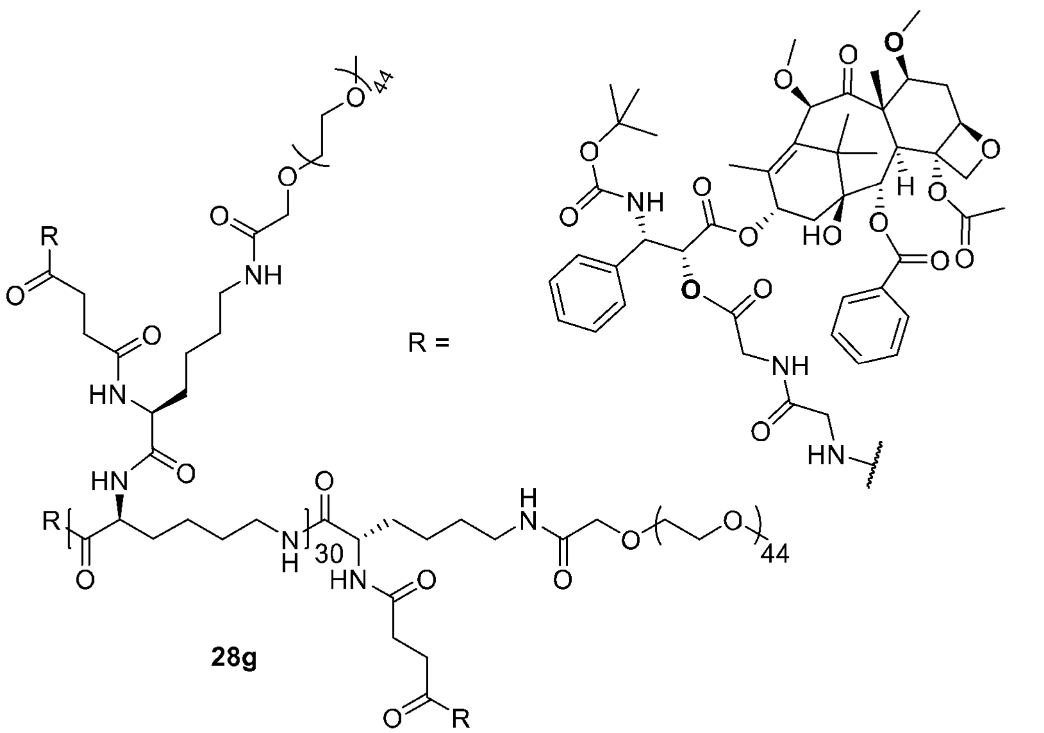

R =

**28g**

,

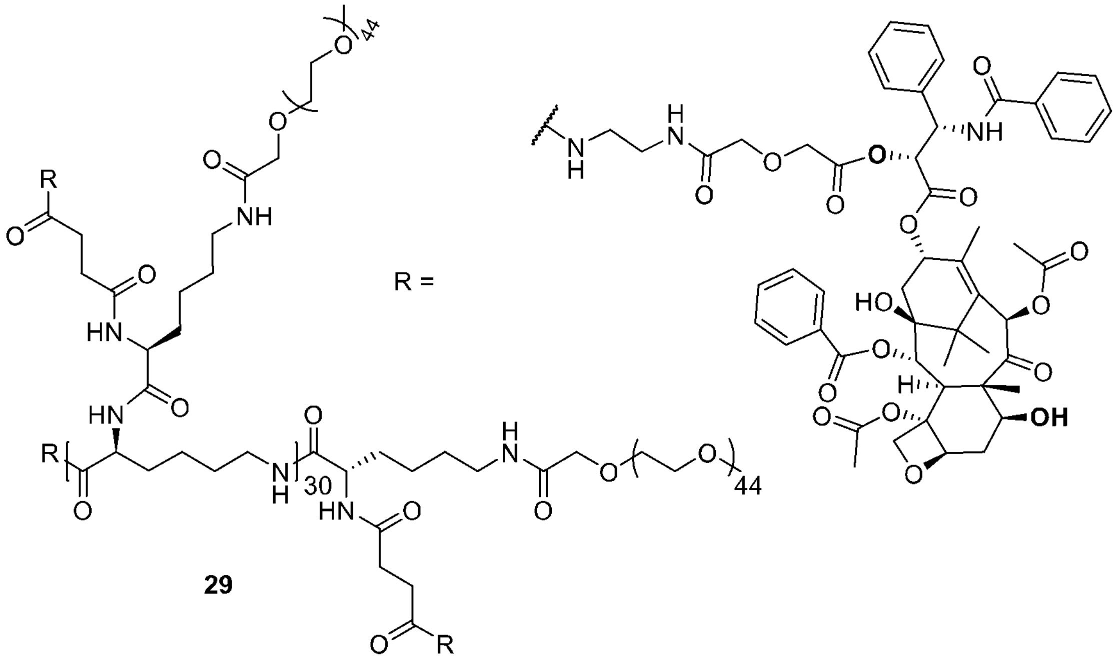

R =

29

,

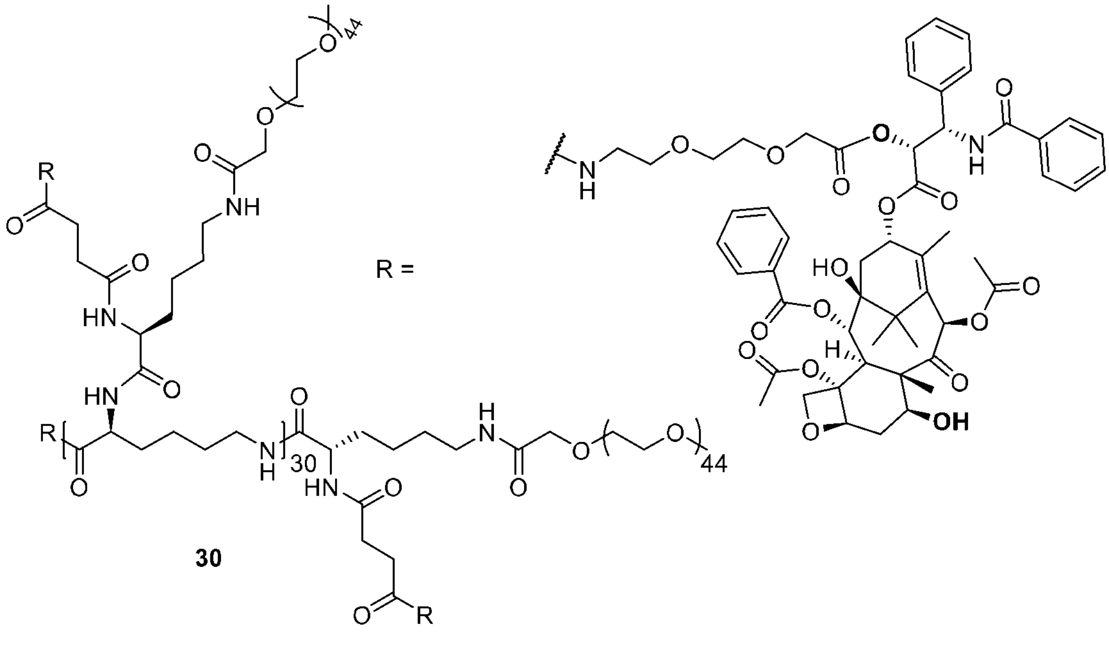

R =

30

,

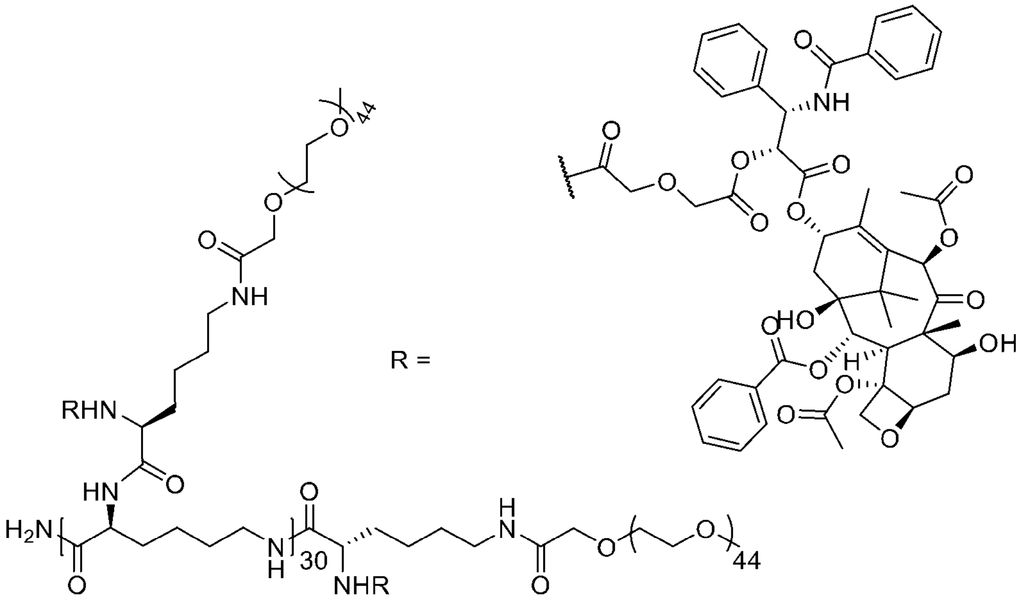

**31**

,

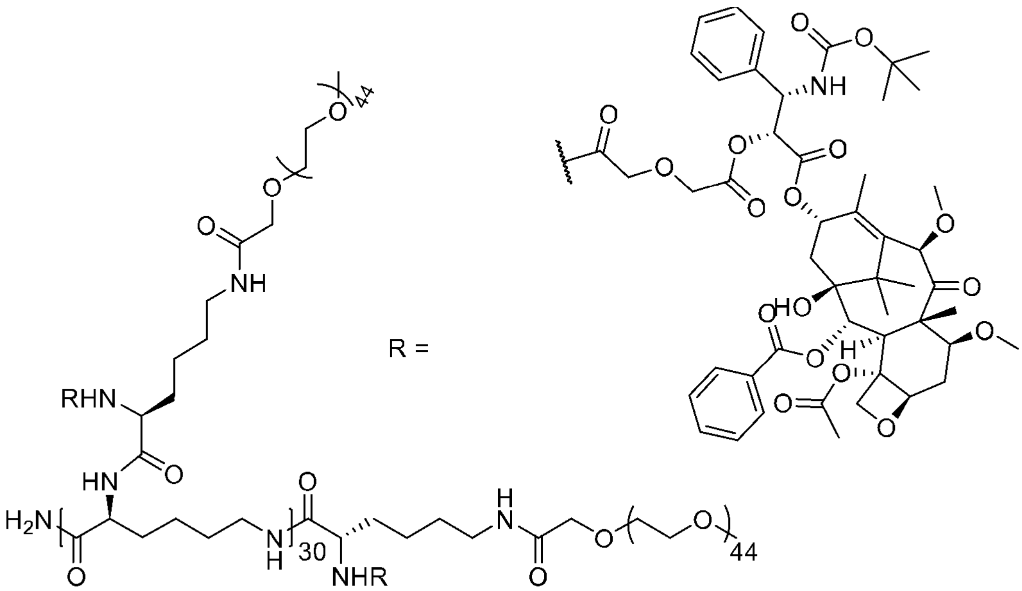

**32**

,

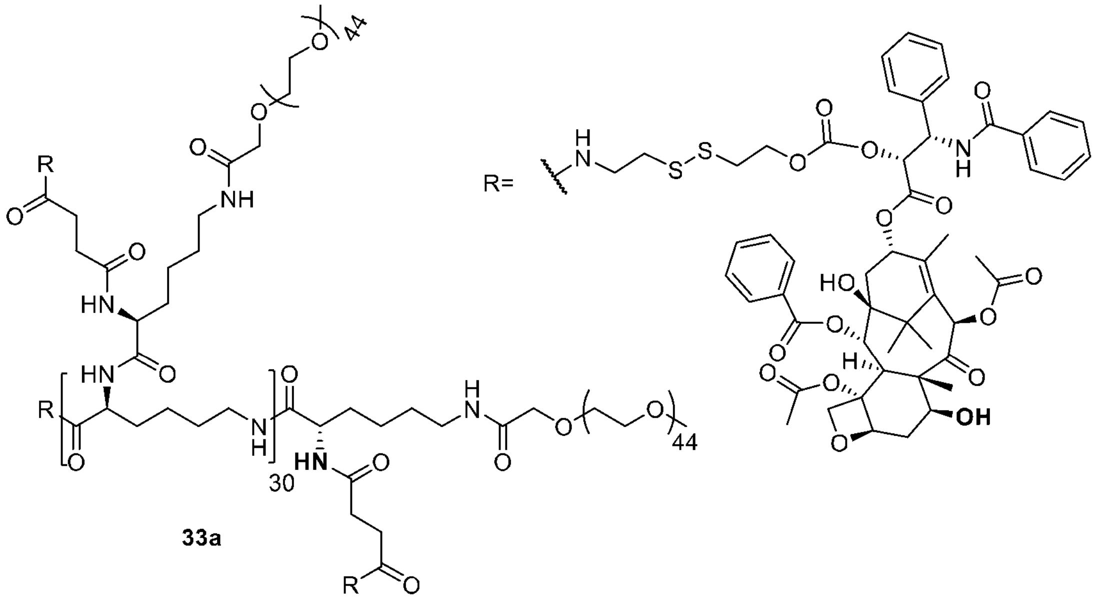

33a

,

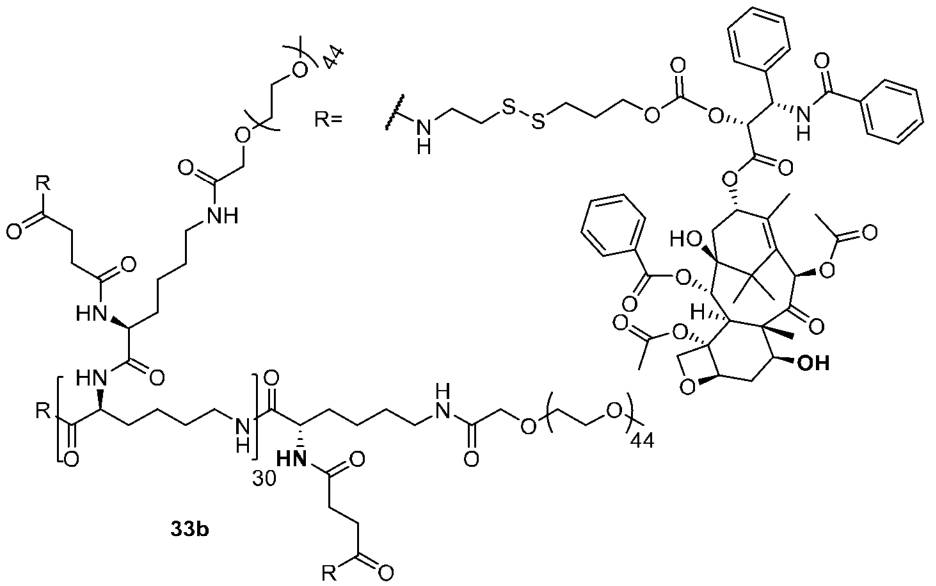

33b

,

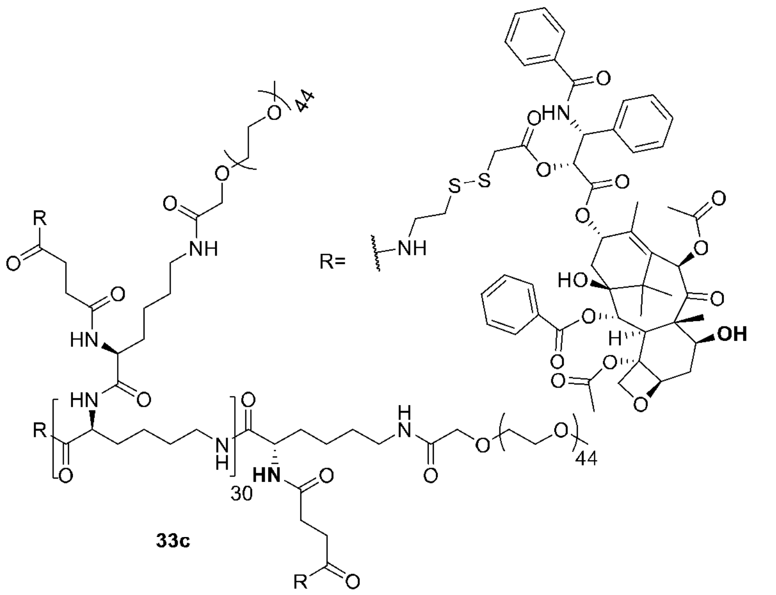

33c

,

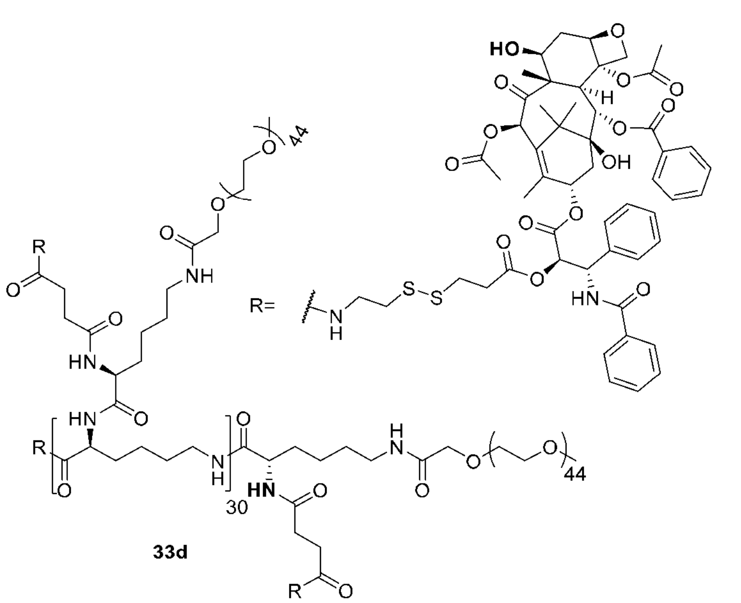

33d

,

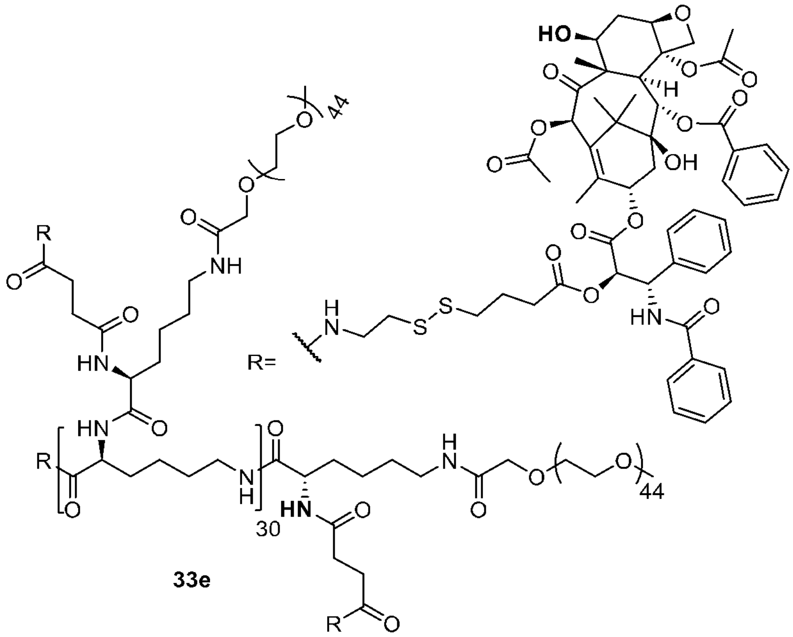

33e

,

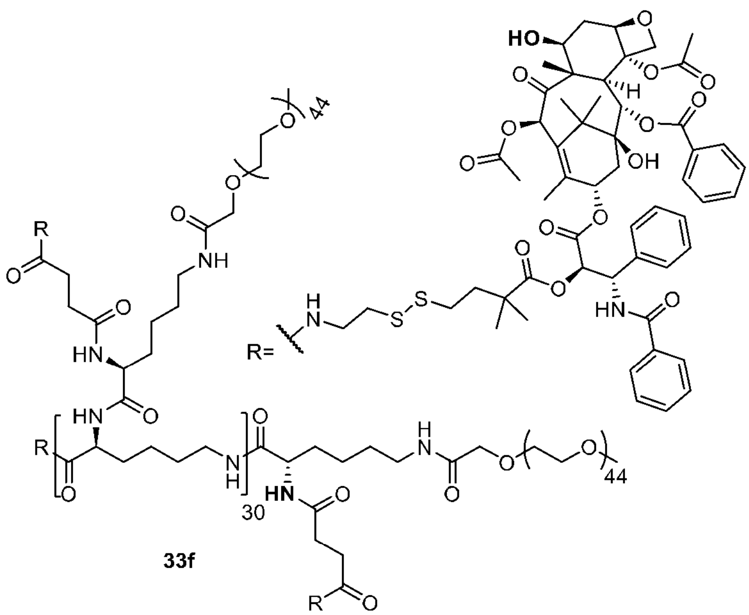

33f

,

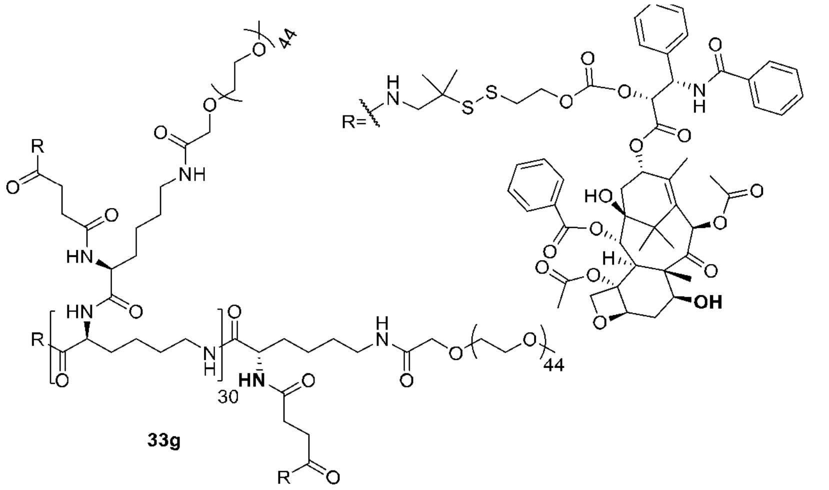

33g

,

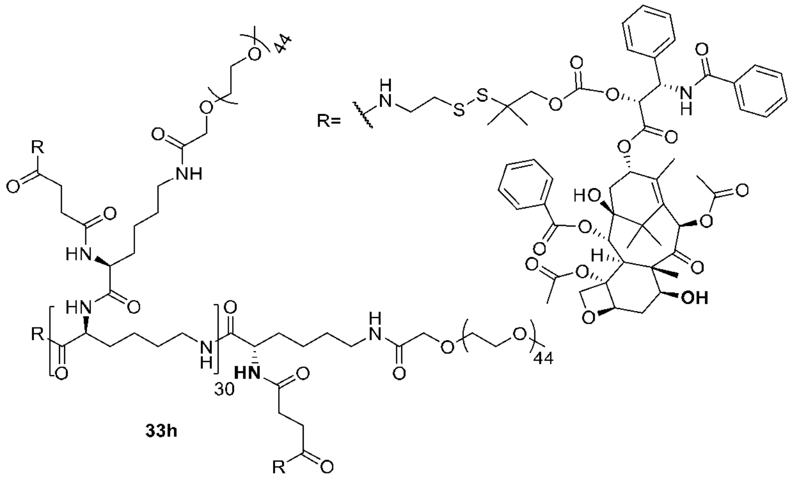

33h

,

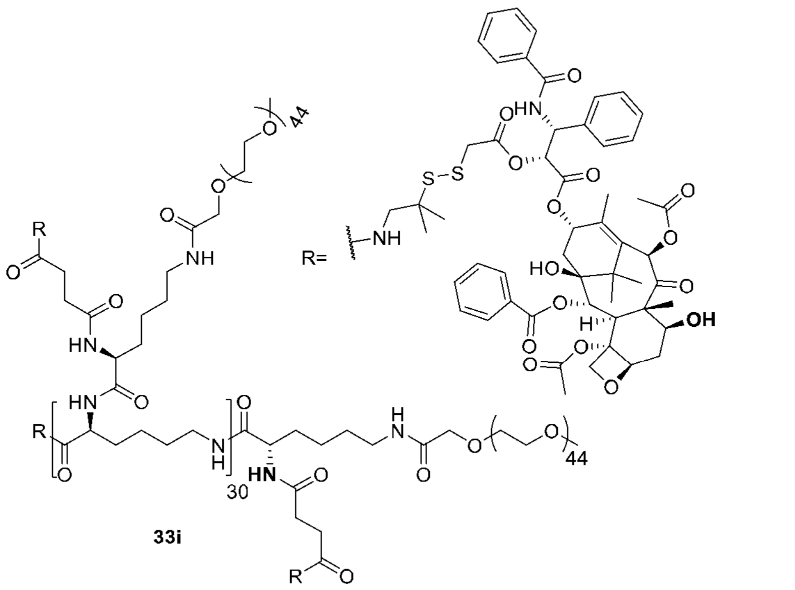

33i

,

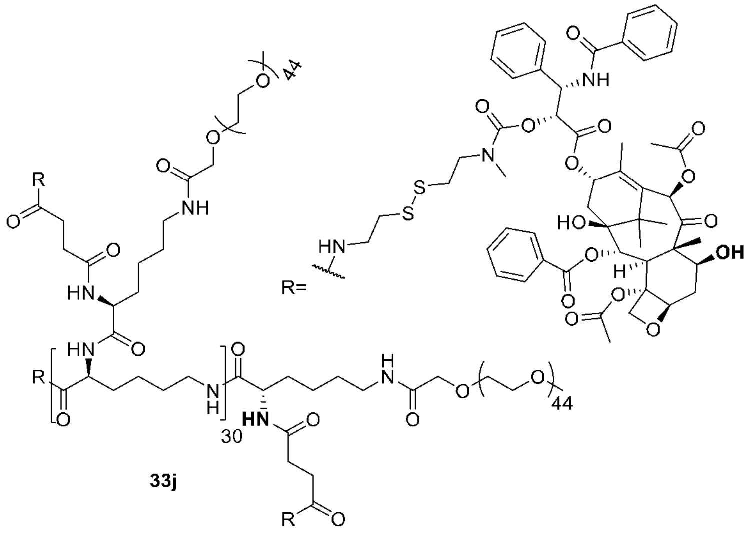

33j

,

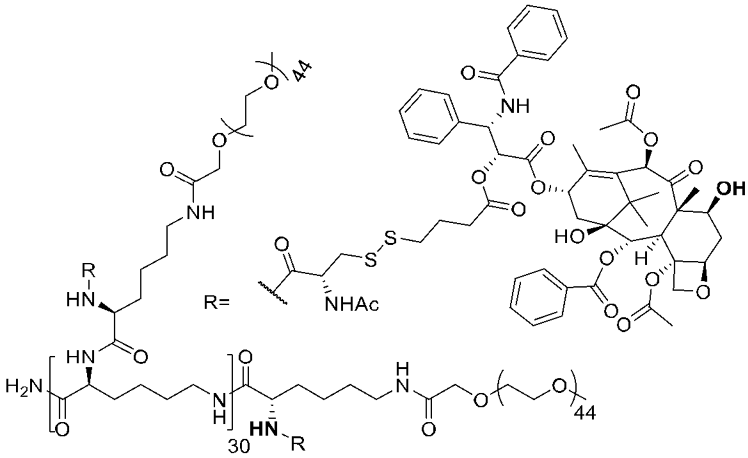

33k

,

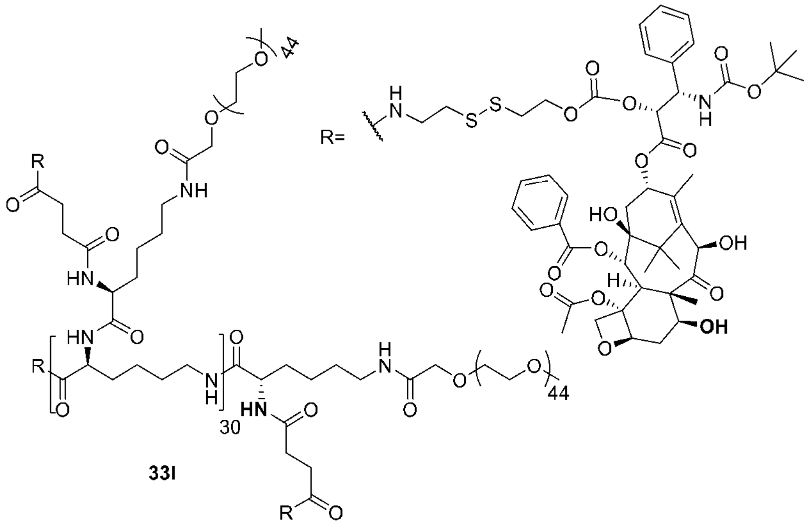

33l

,

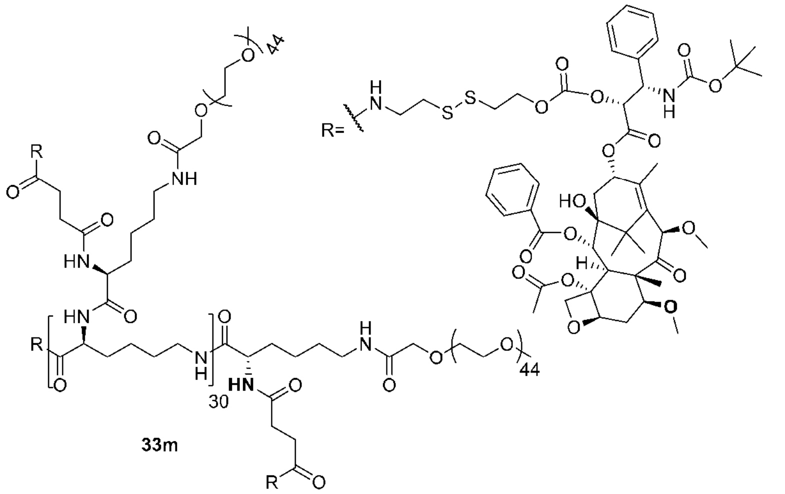

33m

,

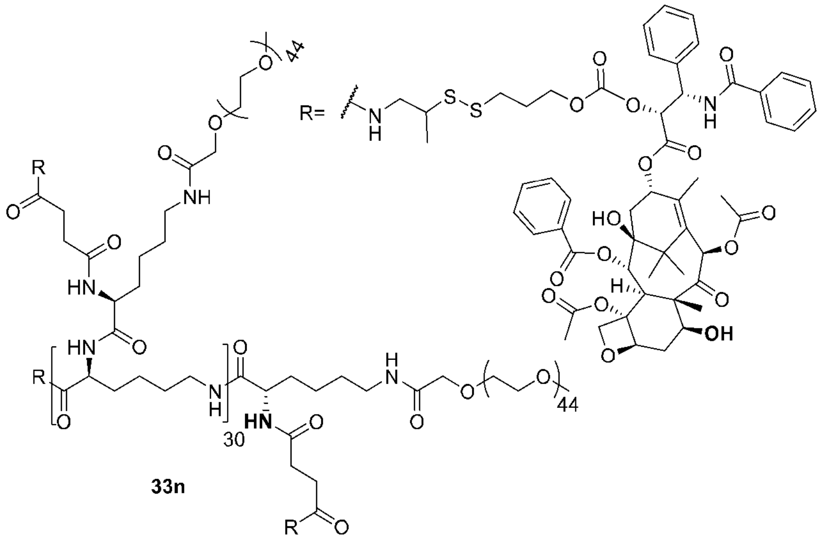

33n

,

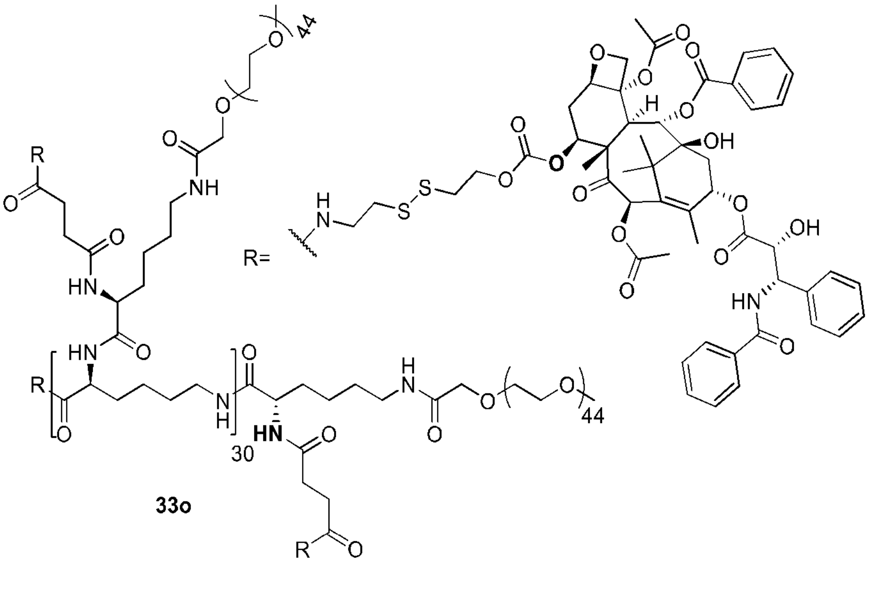

33o

,

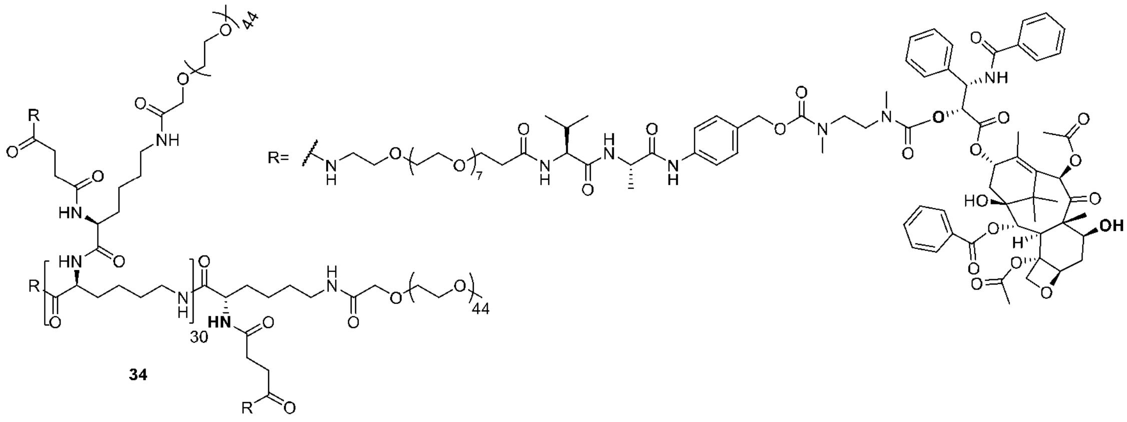

34

,

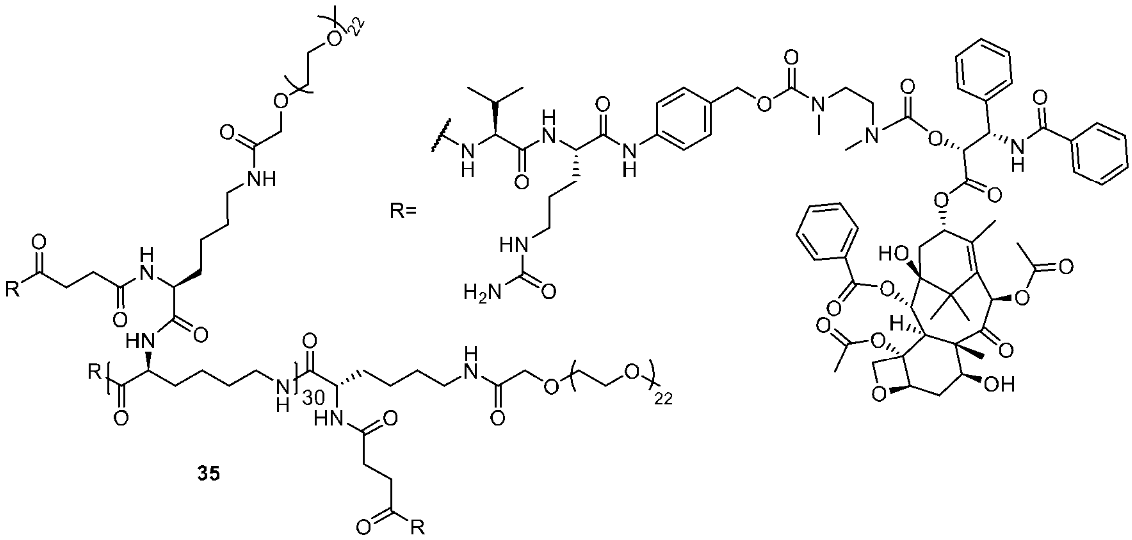

35

,

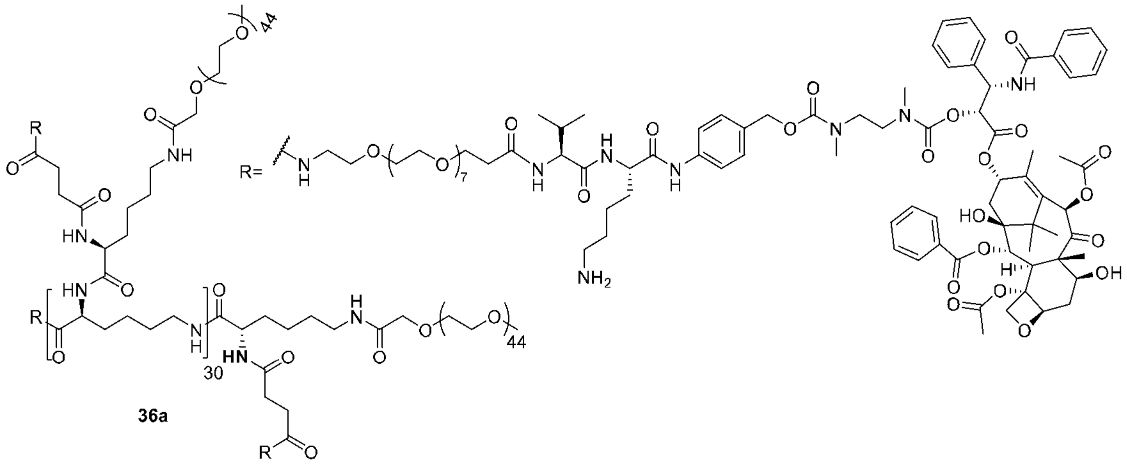

36a

,

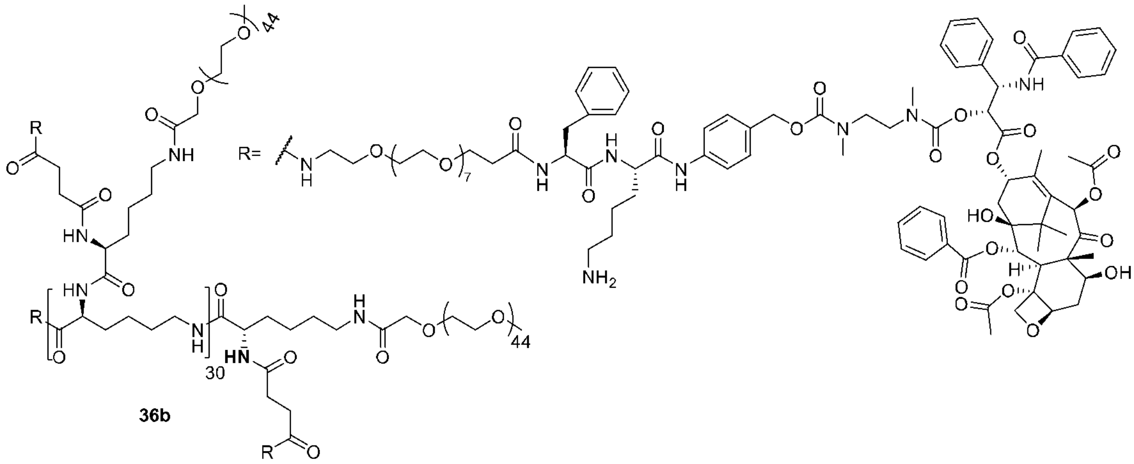

36b

,

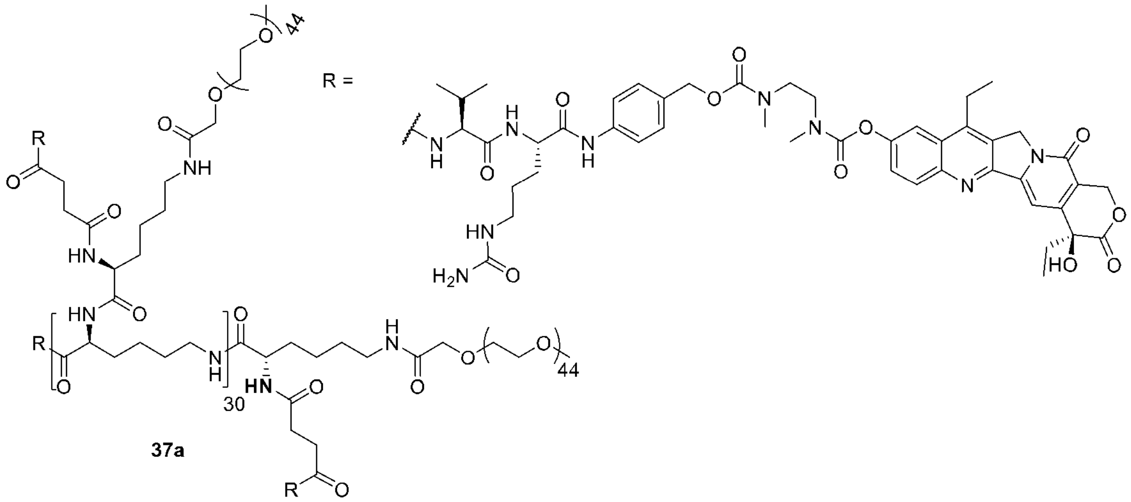

37a

,

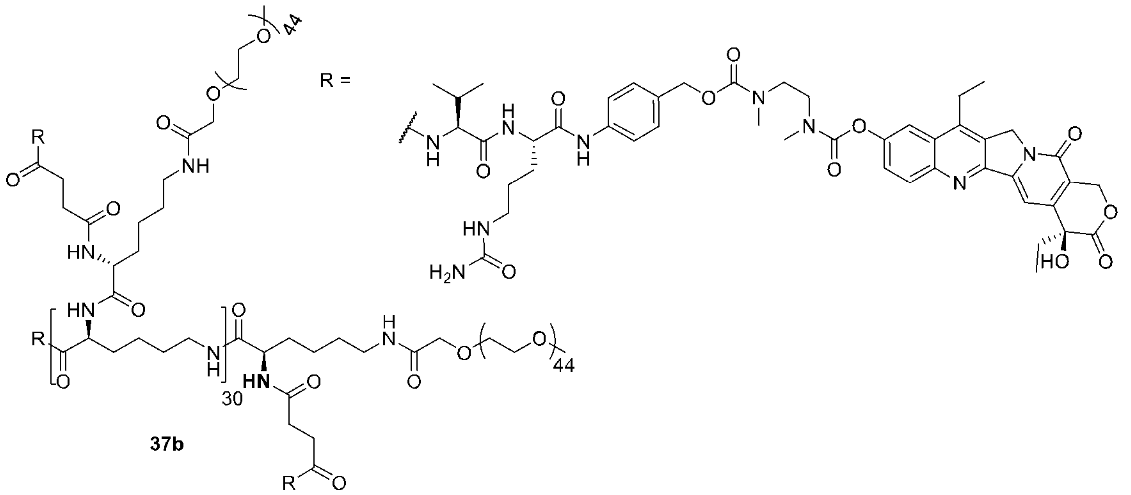

37b

,

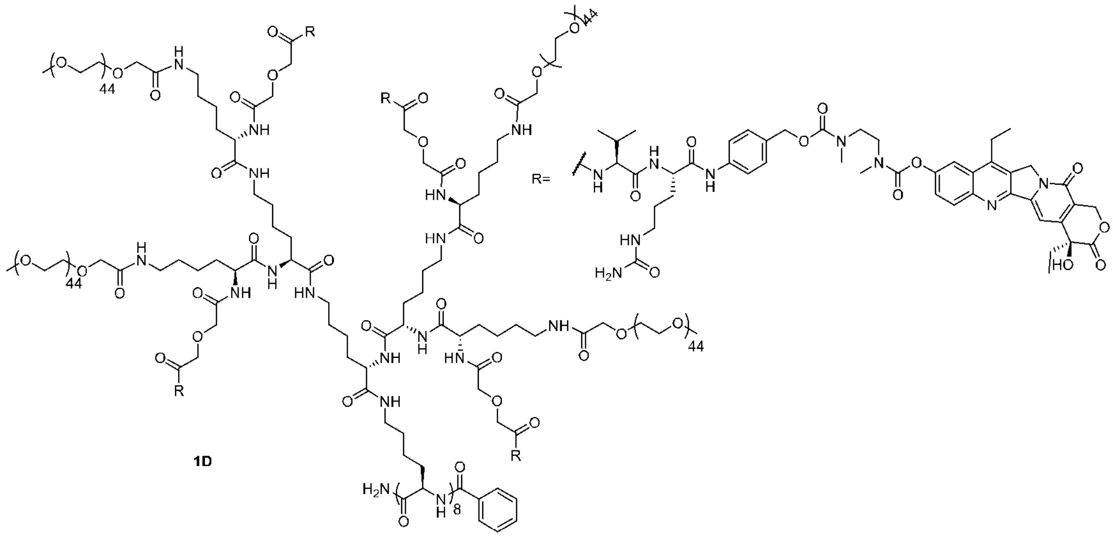

1D

,

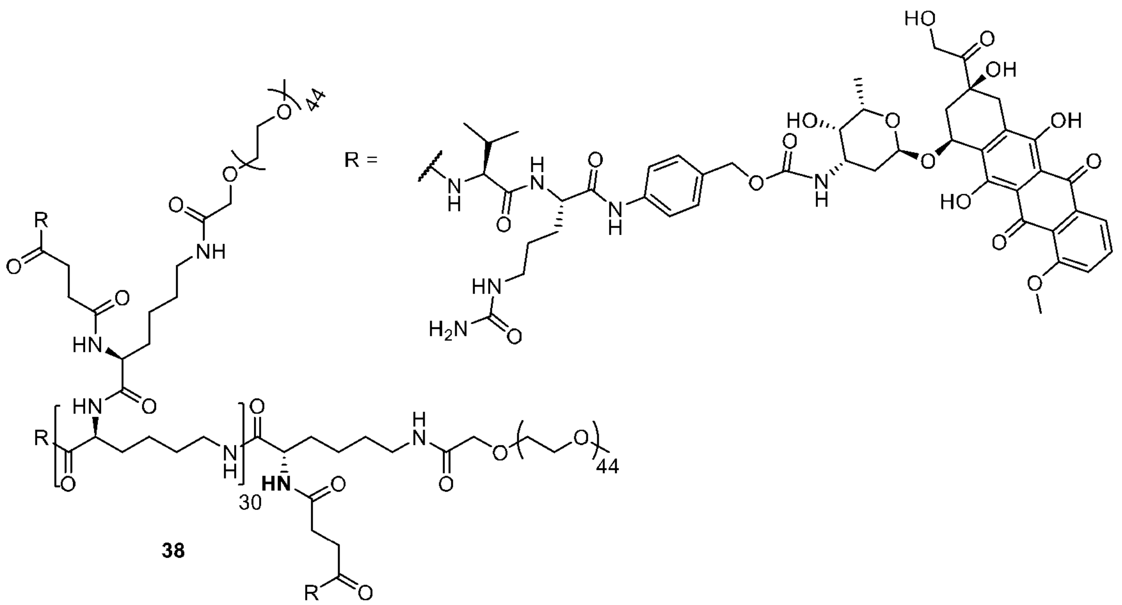

38

,

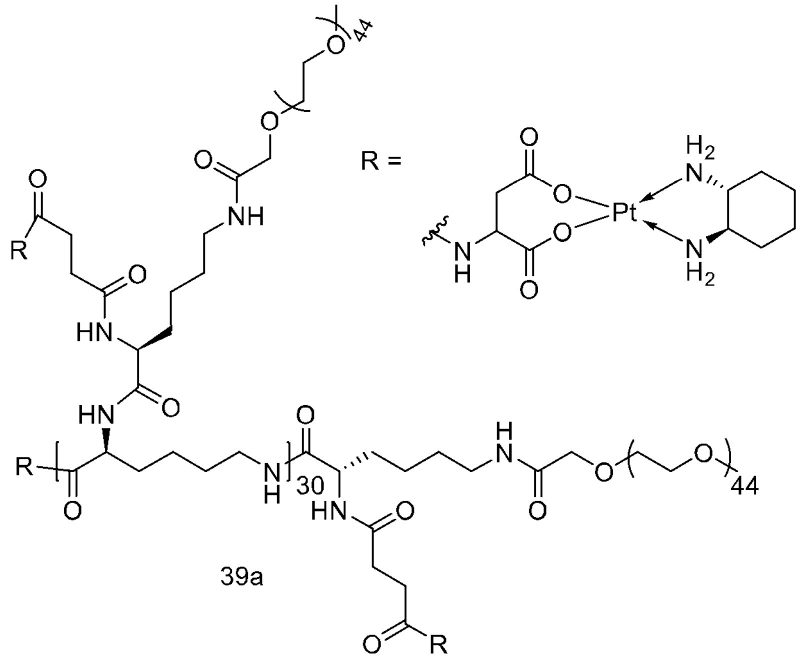

39a

,

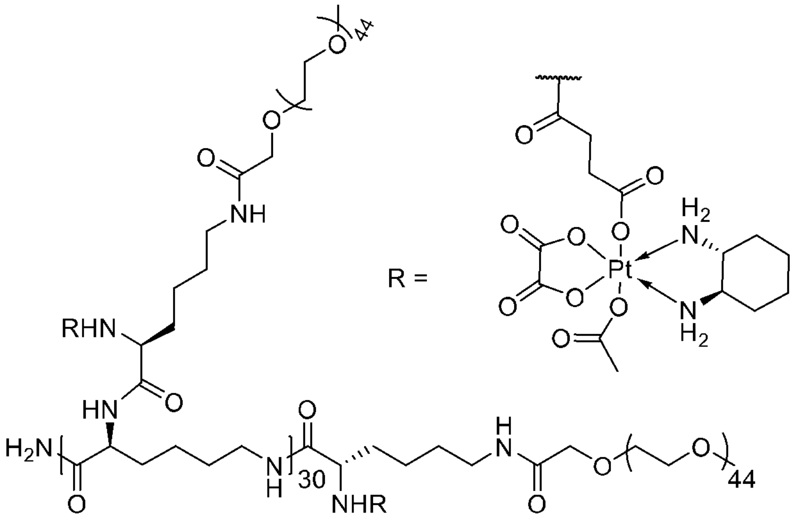

**39b**

,

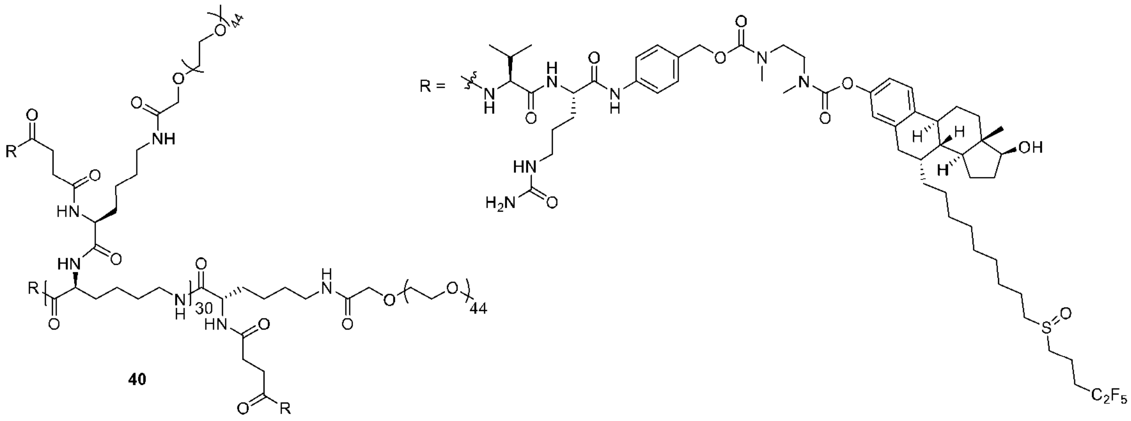

40

,

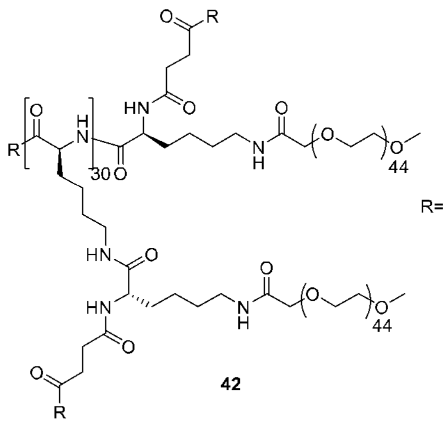

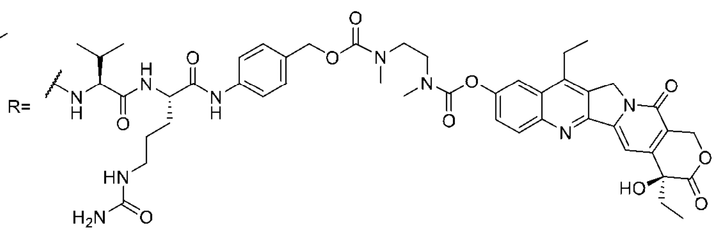

42

,

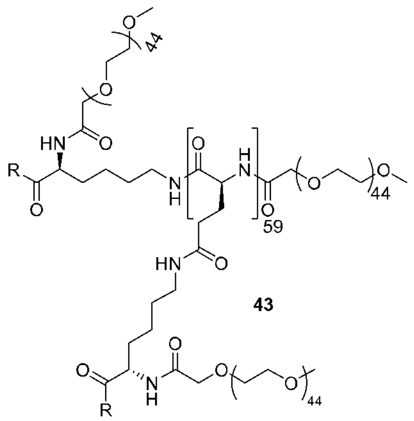

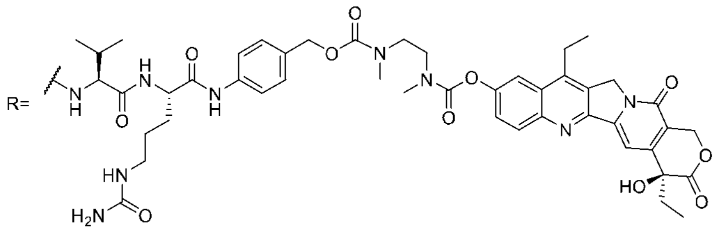

43

,

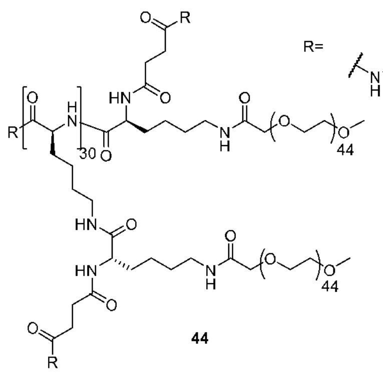

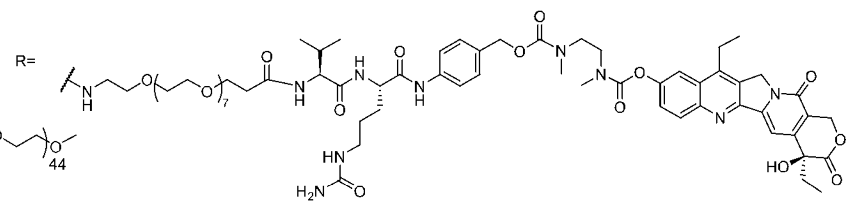

44

,

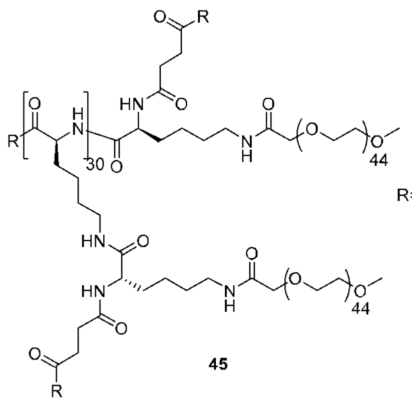

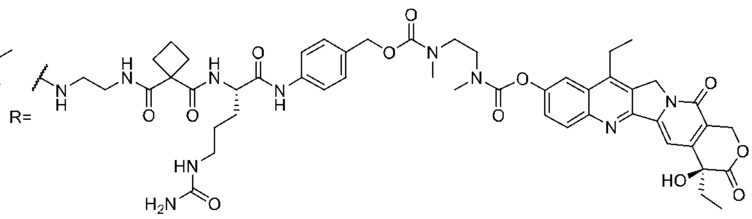

45

,

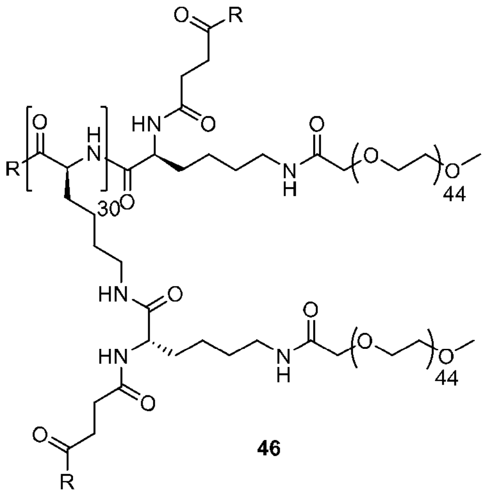

R=

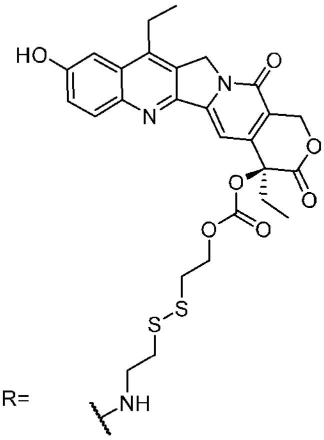

46

,

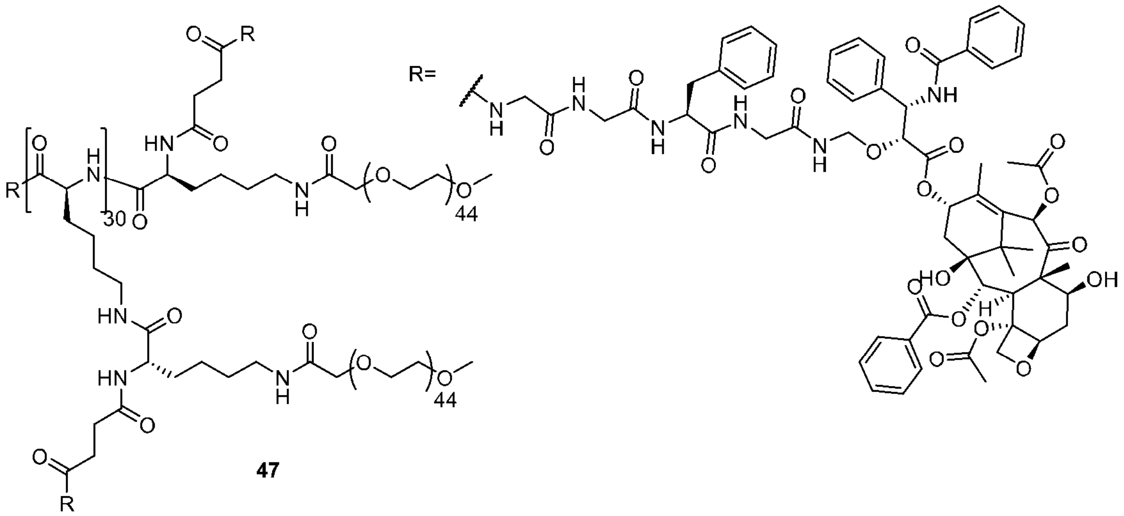

R=

47

,

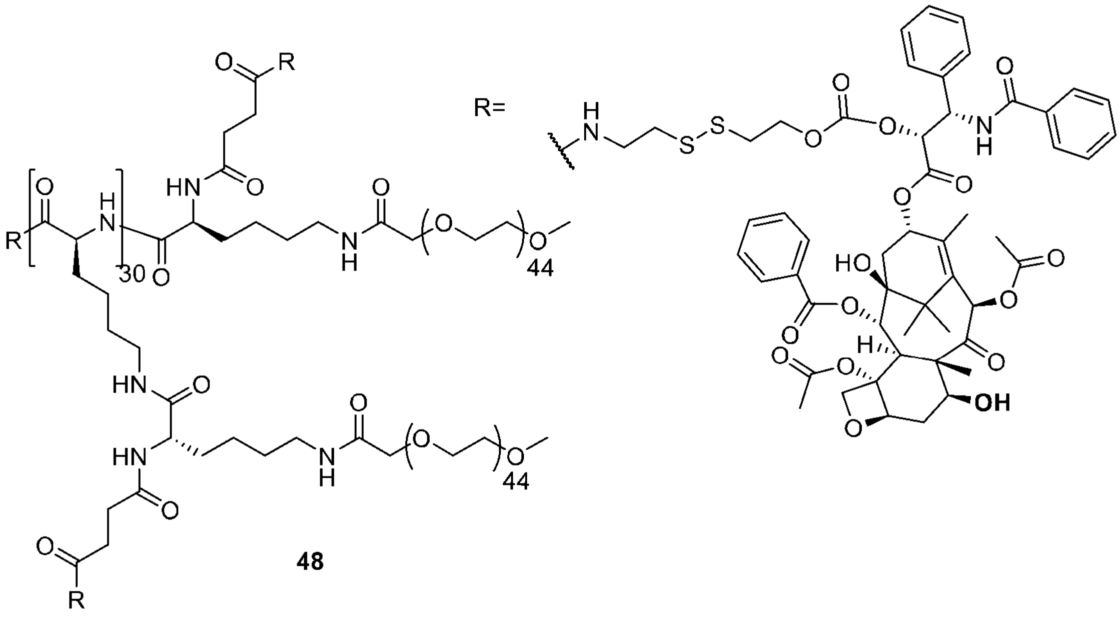

R=

48

,

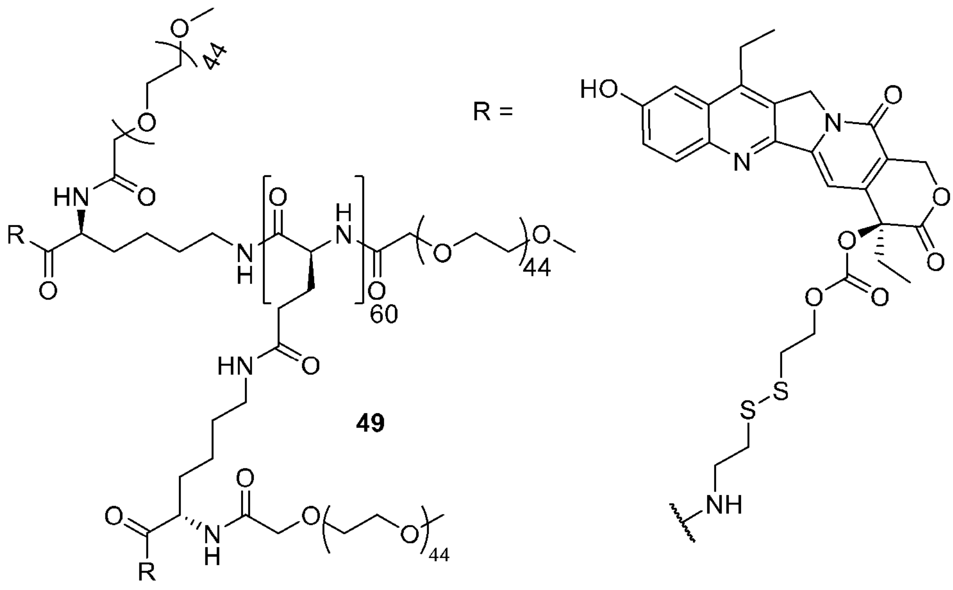

R =

**49**

,

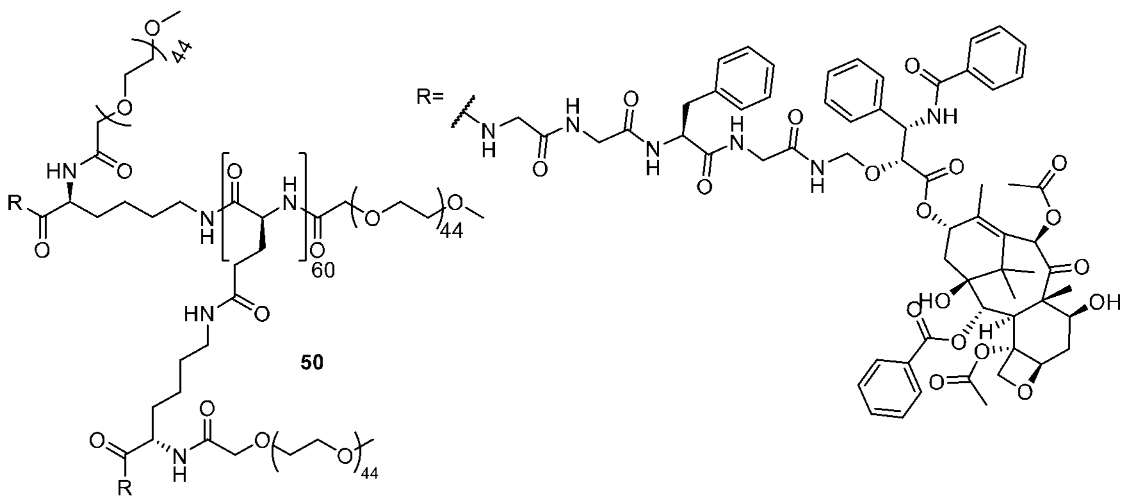

R=

**50**

,

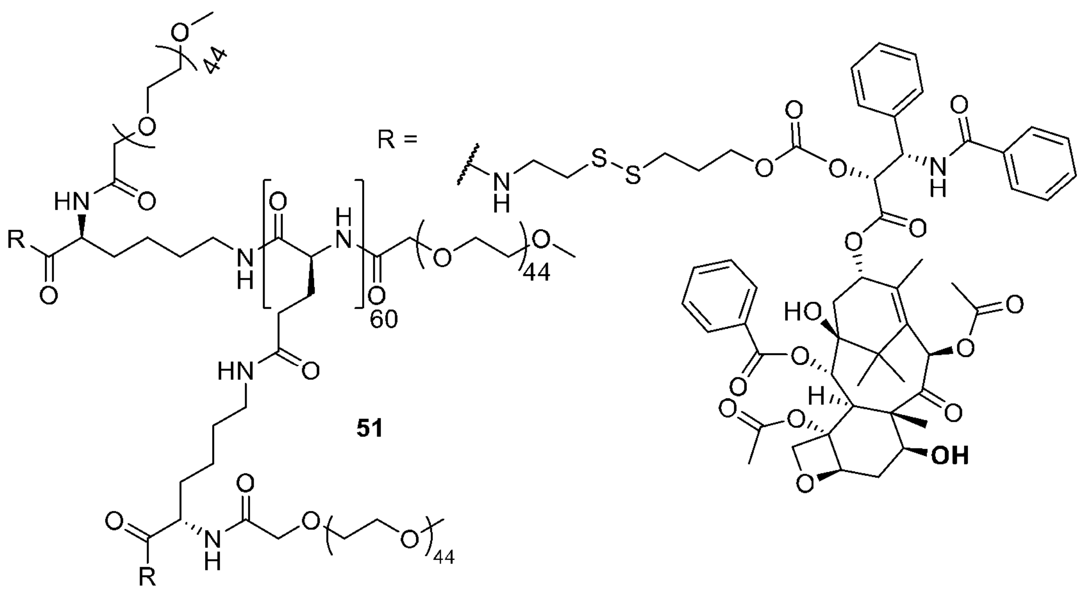

R =

**51**

,

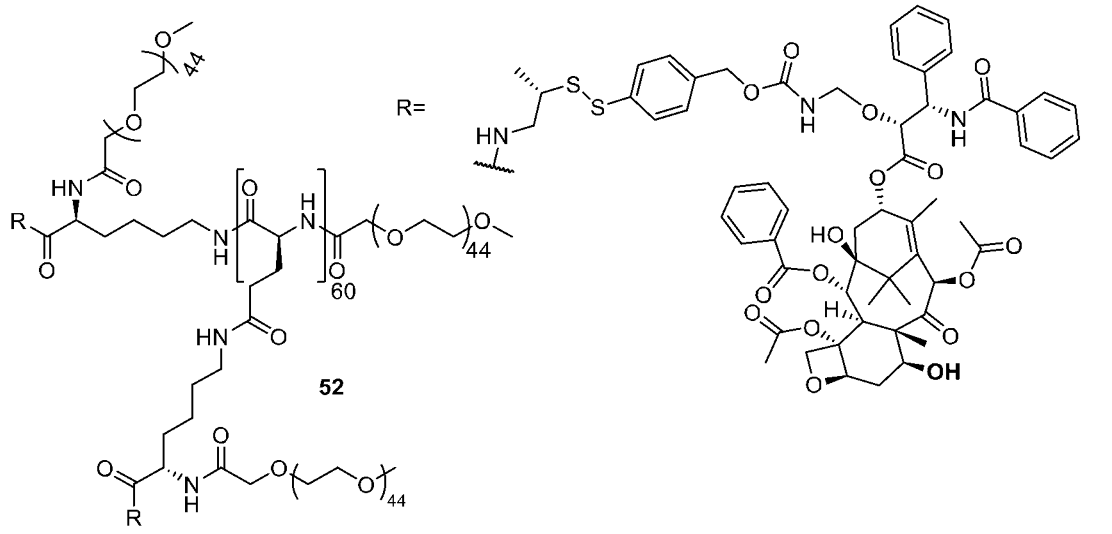

R=

52

,

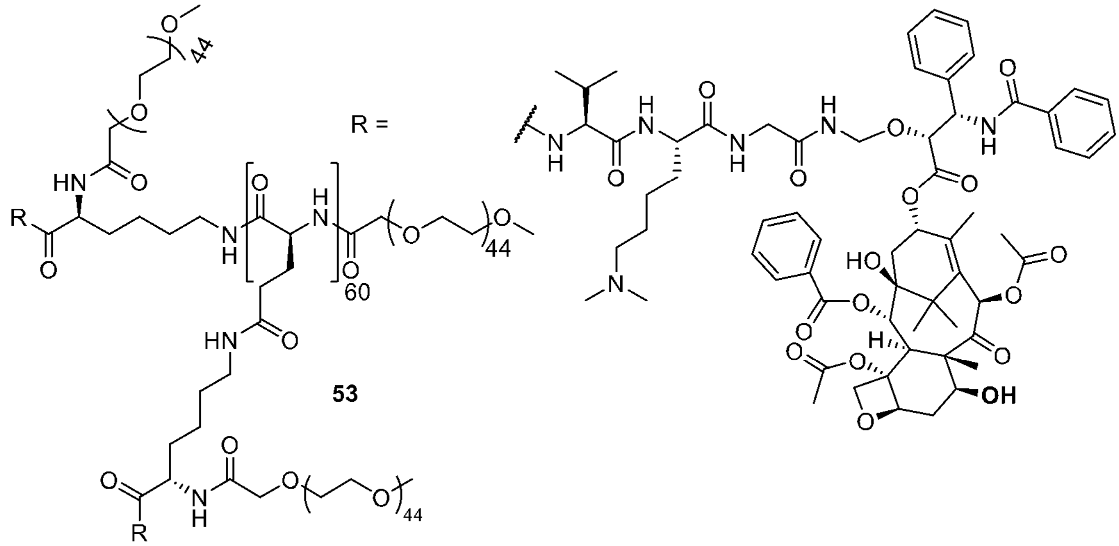

R =

53

,

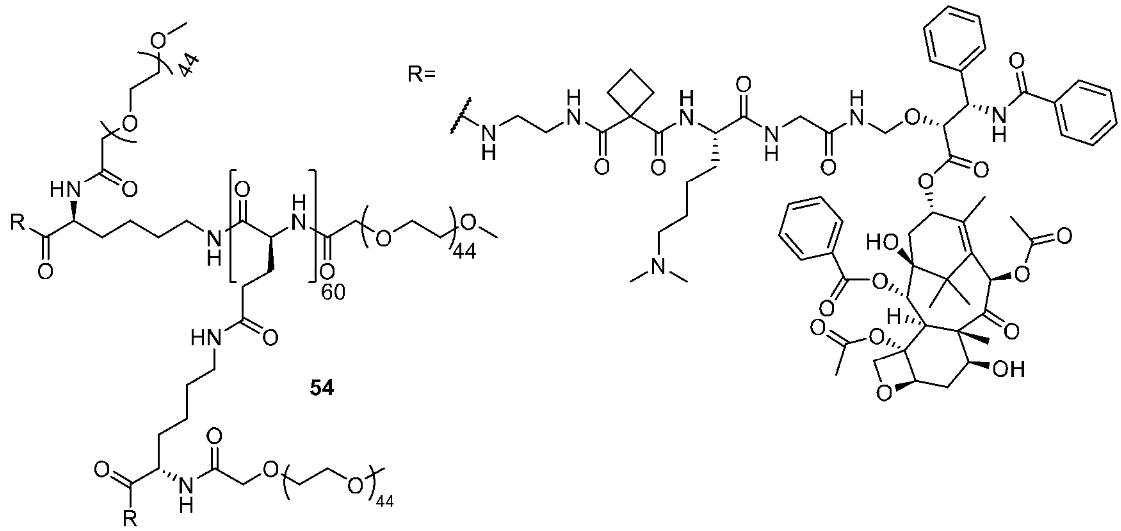

R=

54

,

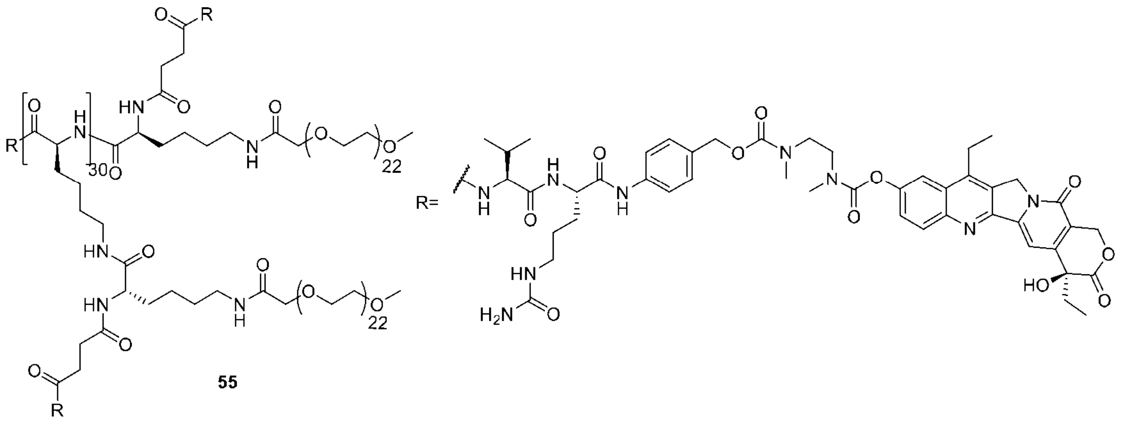

55

,

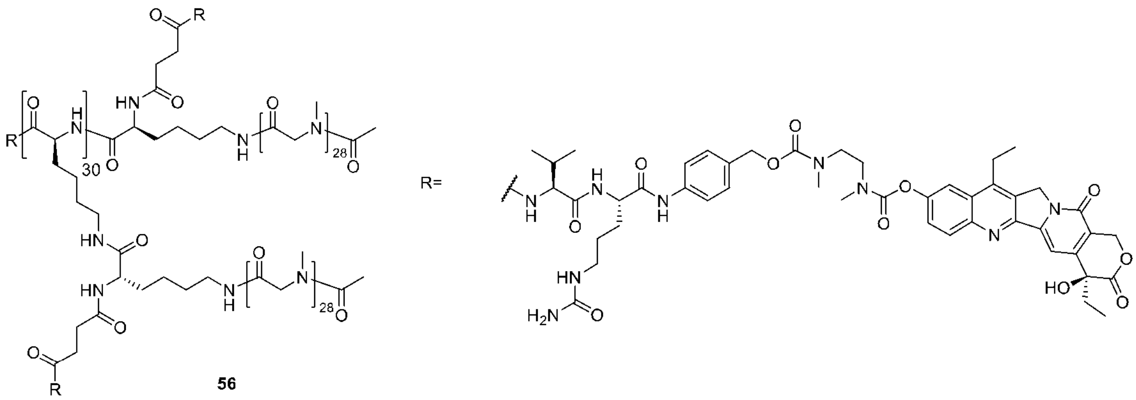

56

,

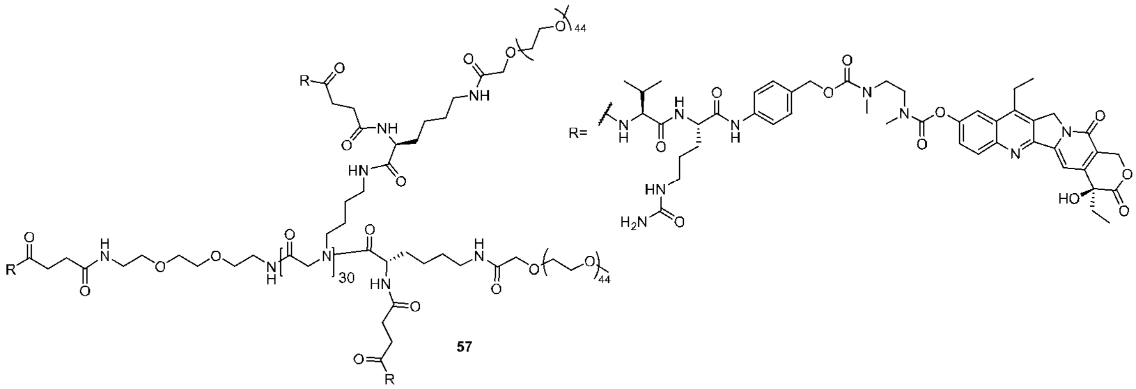

57

,

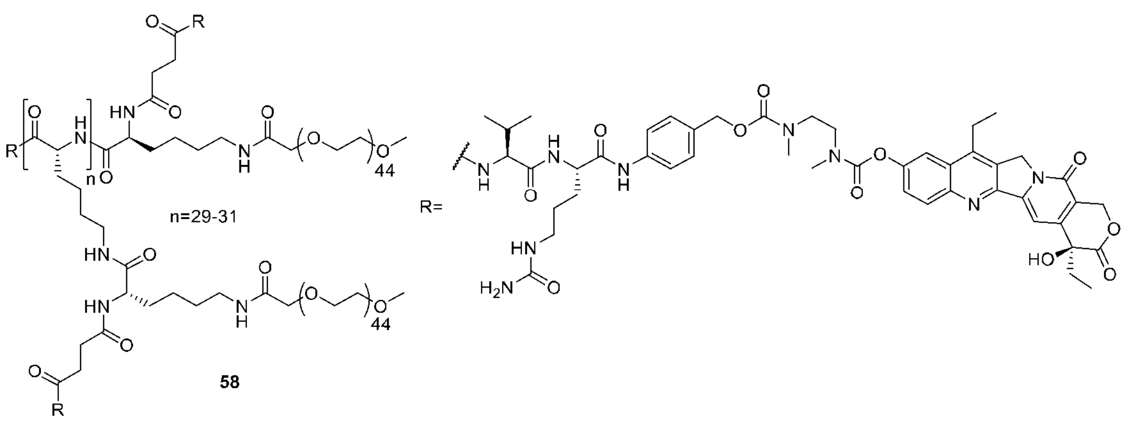

58

,

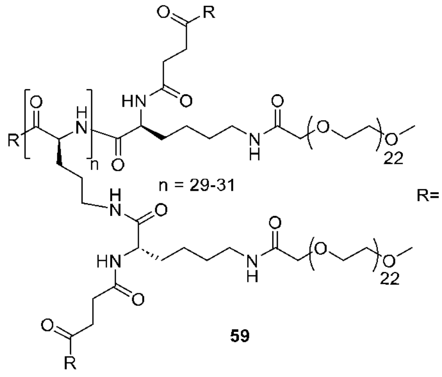

n = 29-31

R= 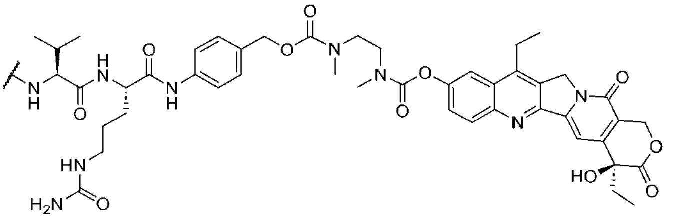

59

,

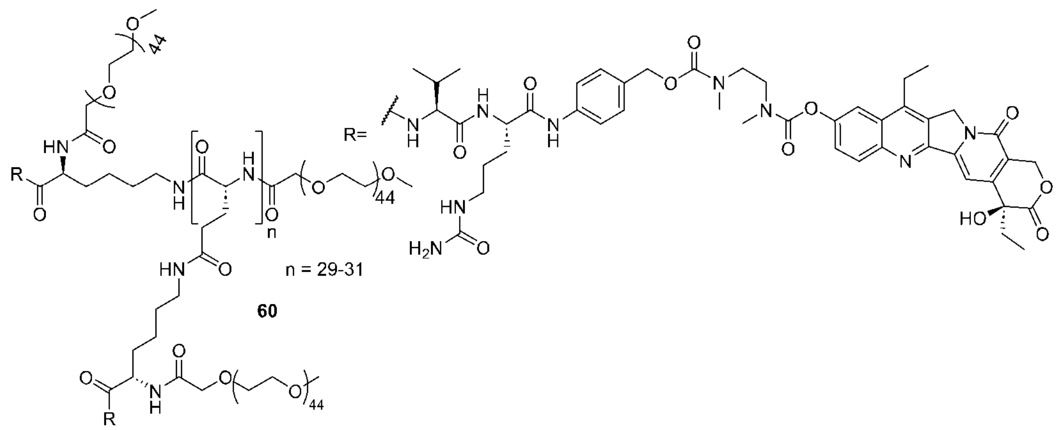

n = 29-31

60

,

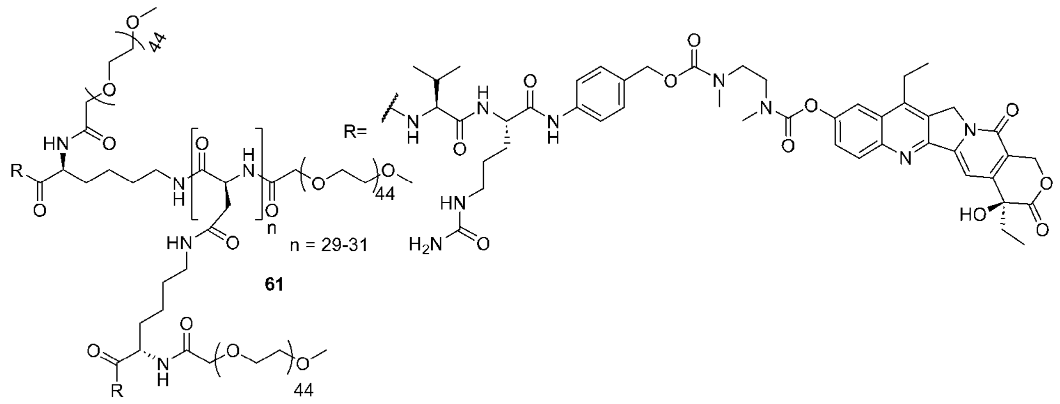

n = 29-31

61

,

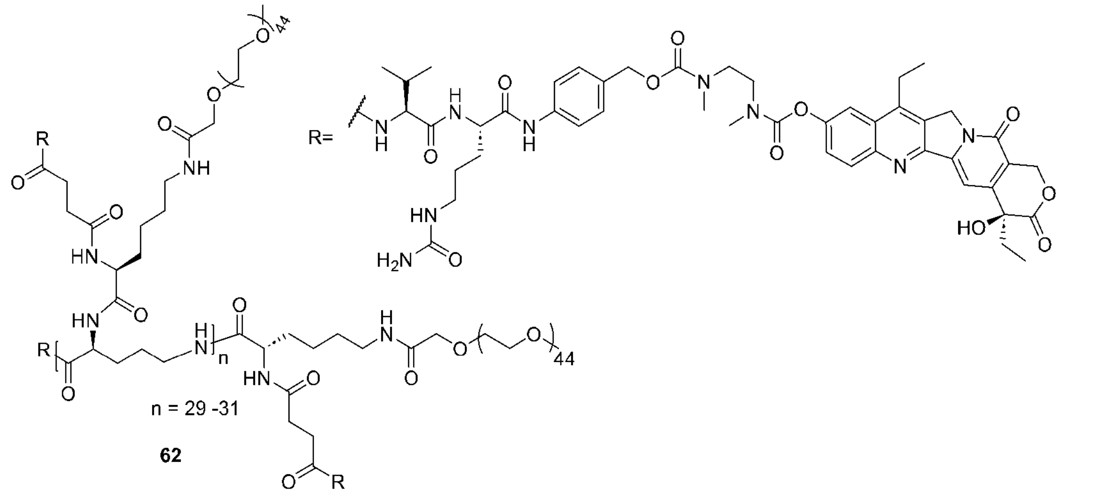

n = 29 -31

62

,

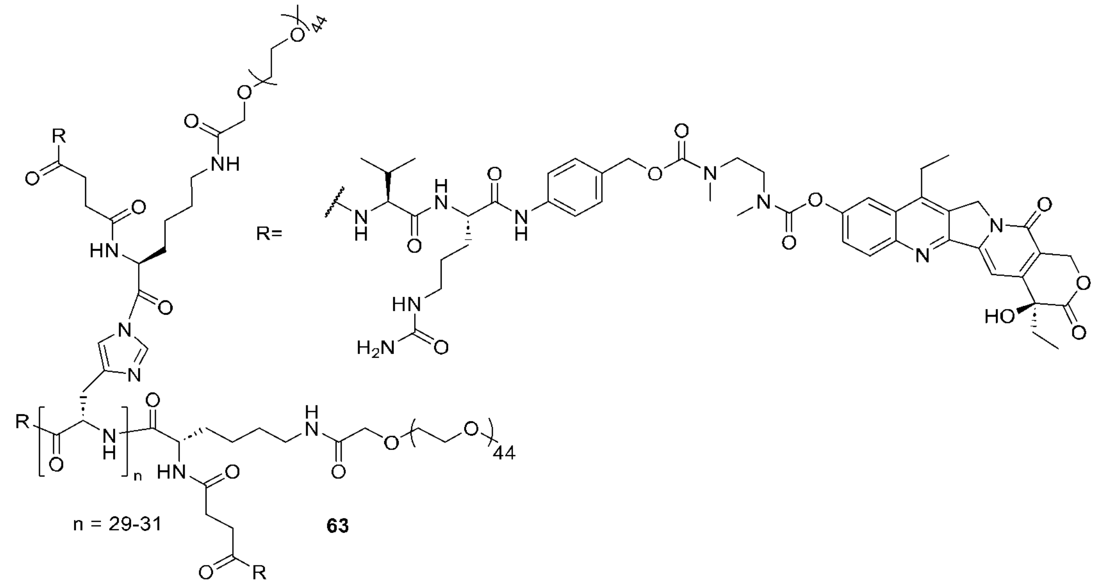

n = 29-31

63

,

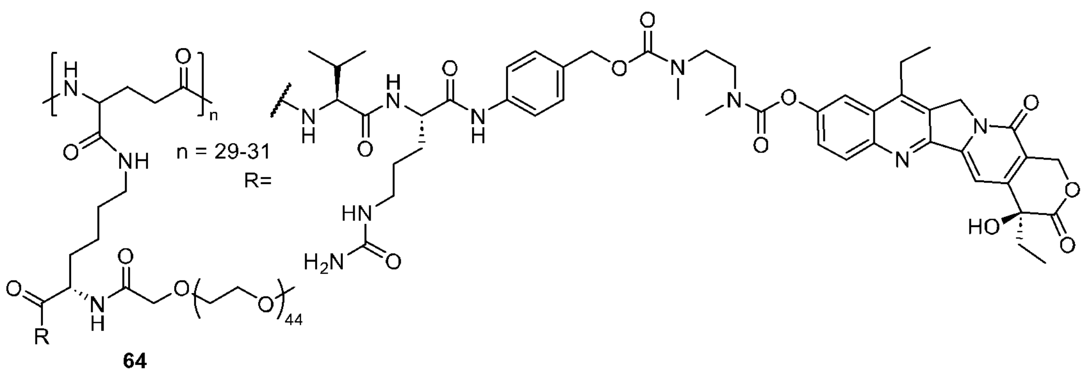

n = 29-31

64

,

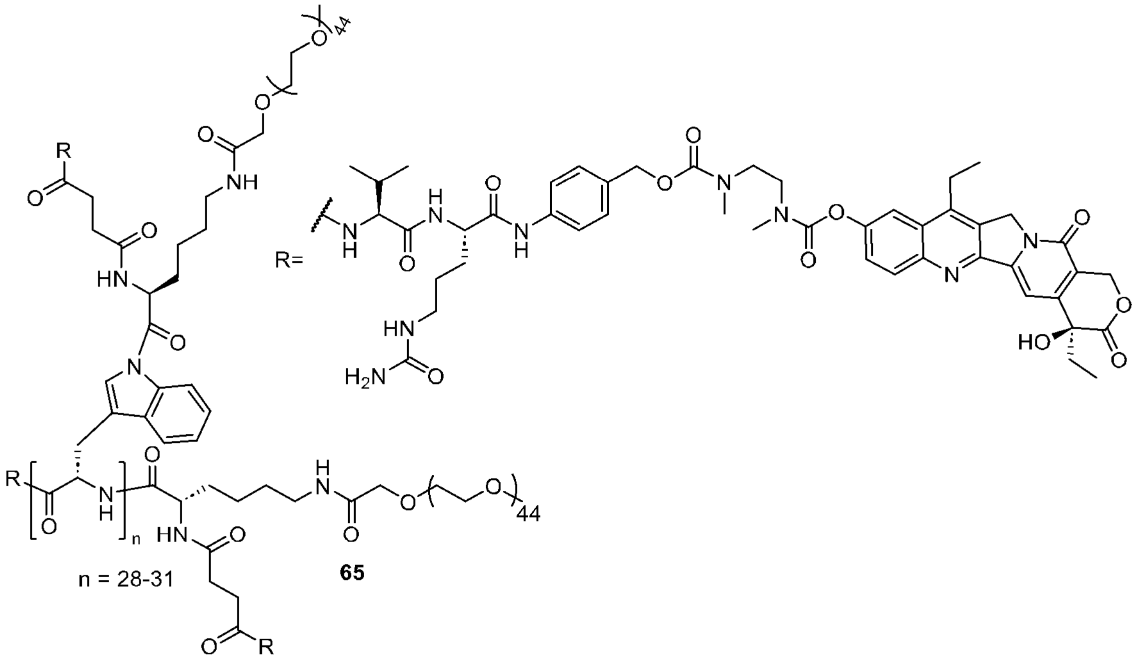

n = 28-31

65

,

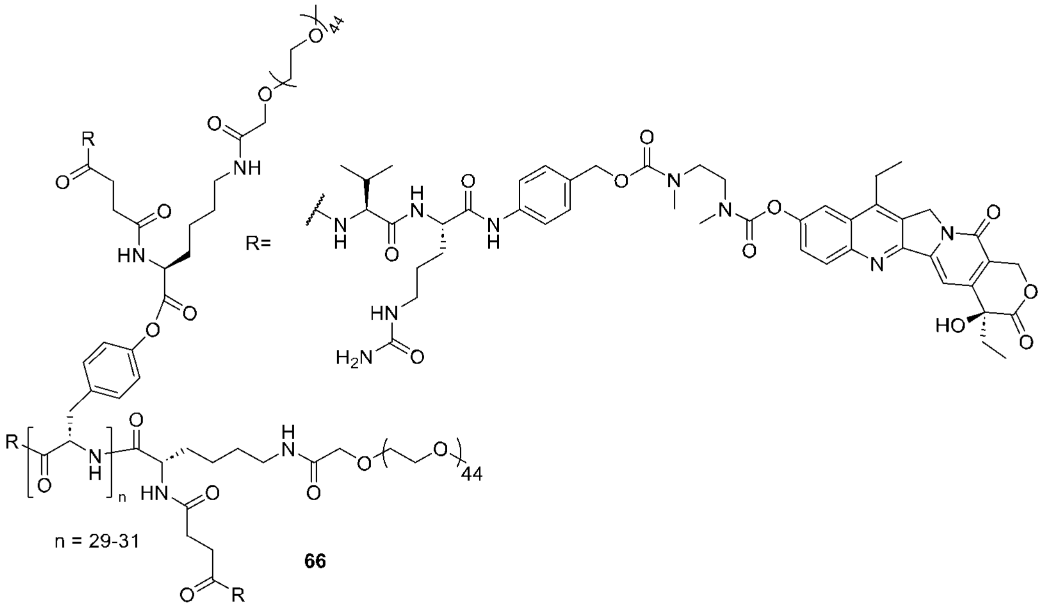

n = 29-31

66

,

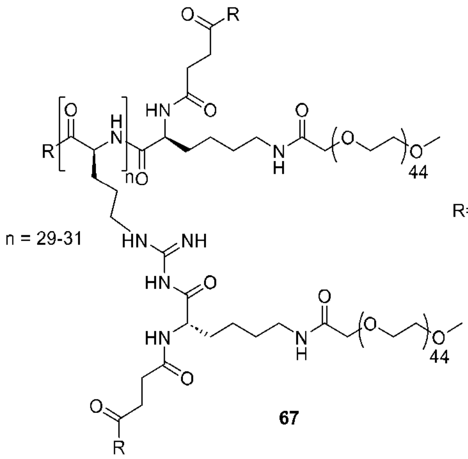

n = 29-31

67

R=

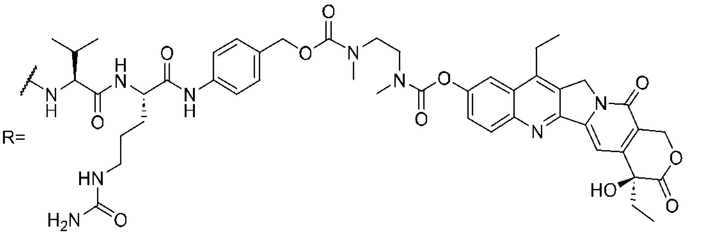

,

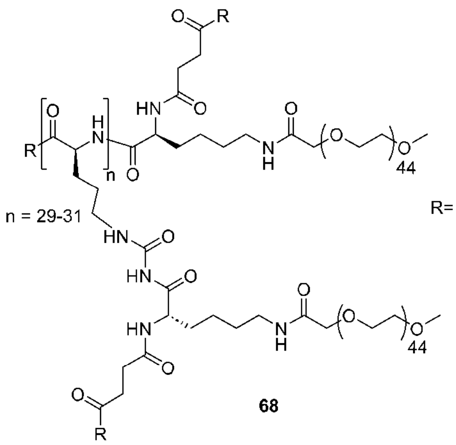

n = 29-31

68

R=

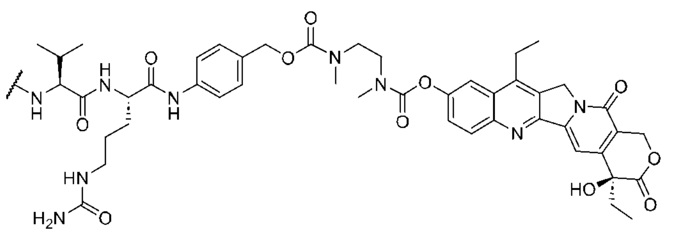

,

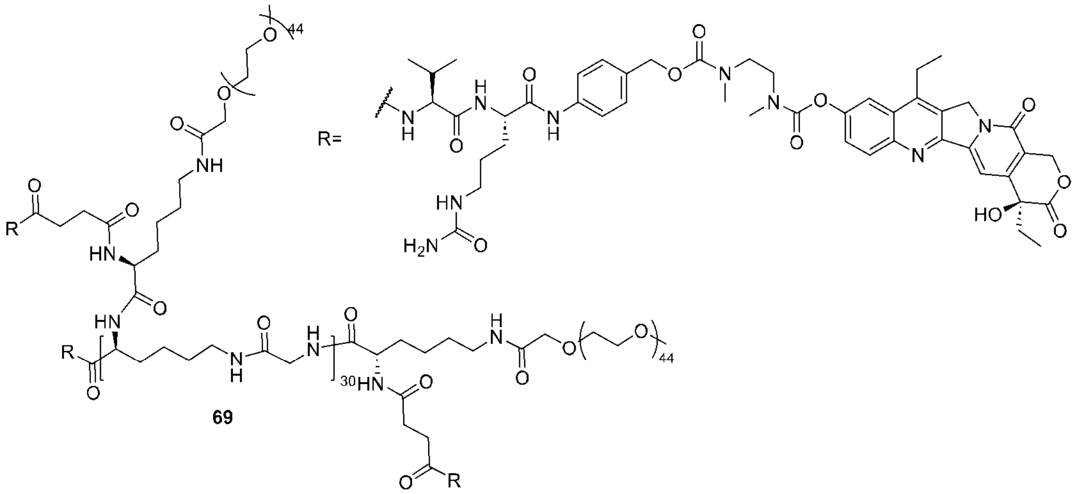

R=

69

,

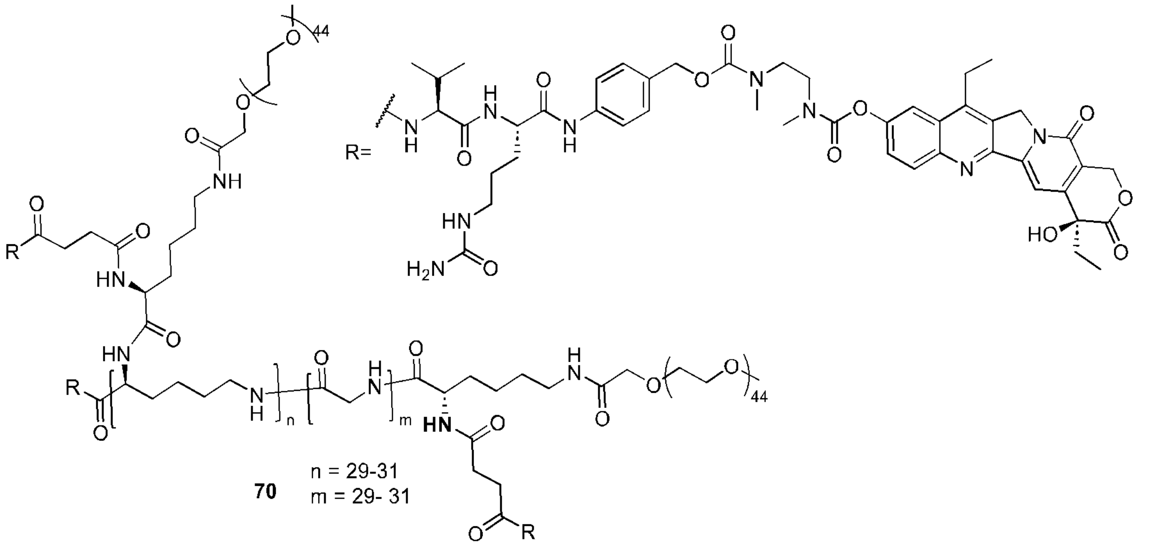

n = 29-31
m = 29- 31
**70**

,

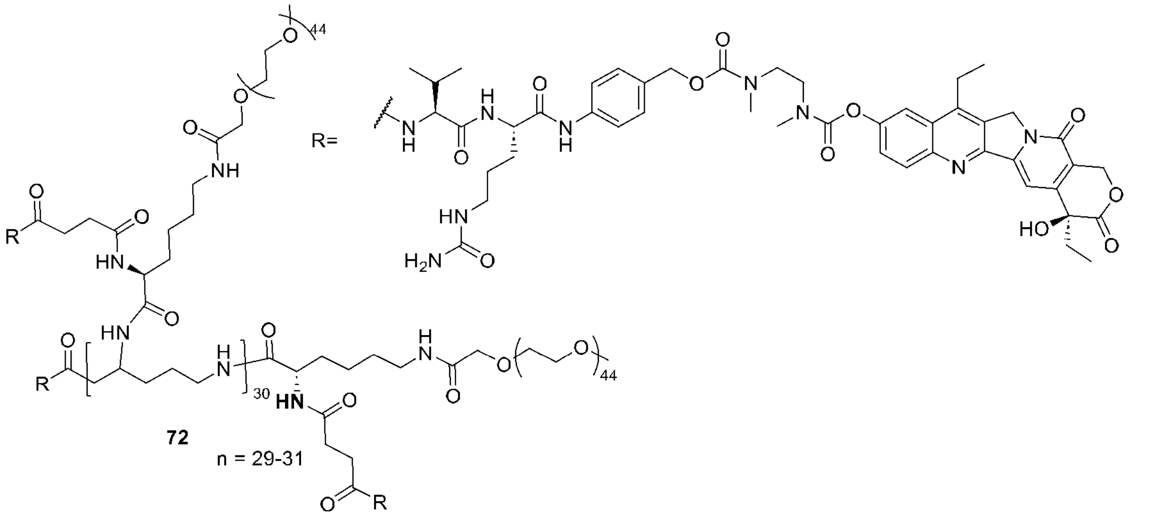

n = 29-31
**72**

,

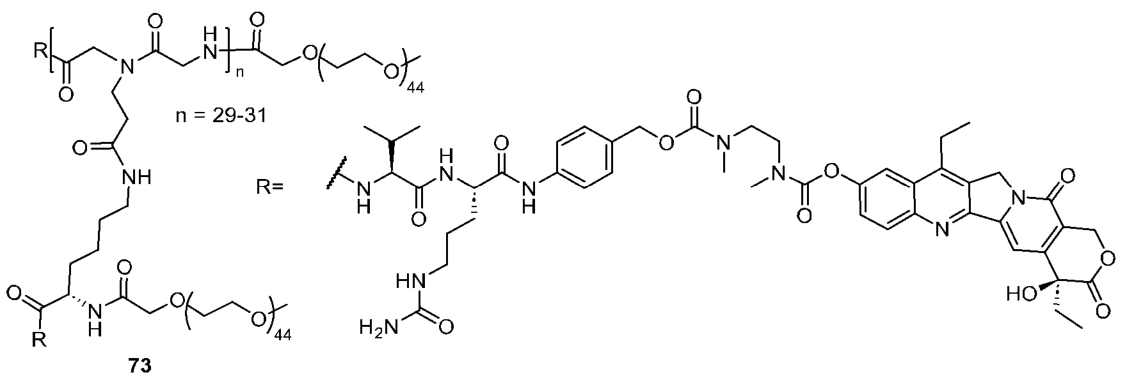

n = 29-31
**73**

,

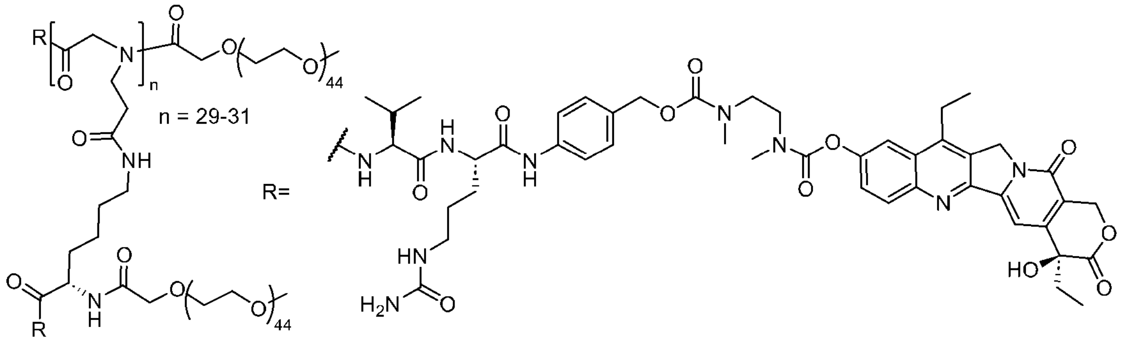

**74**

,

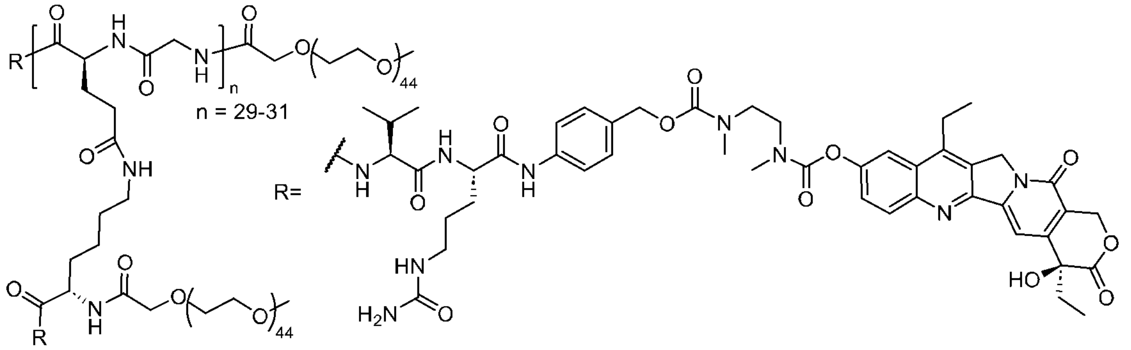

**75**

,

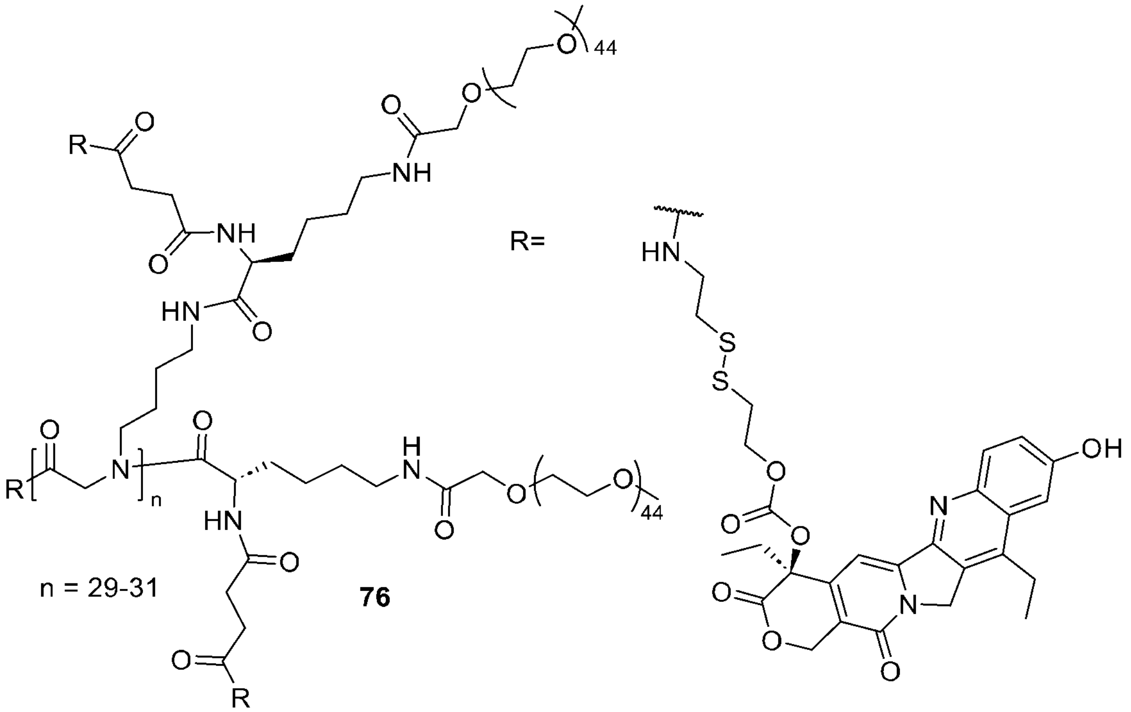

**76**

,

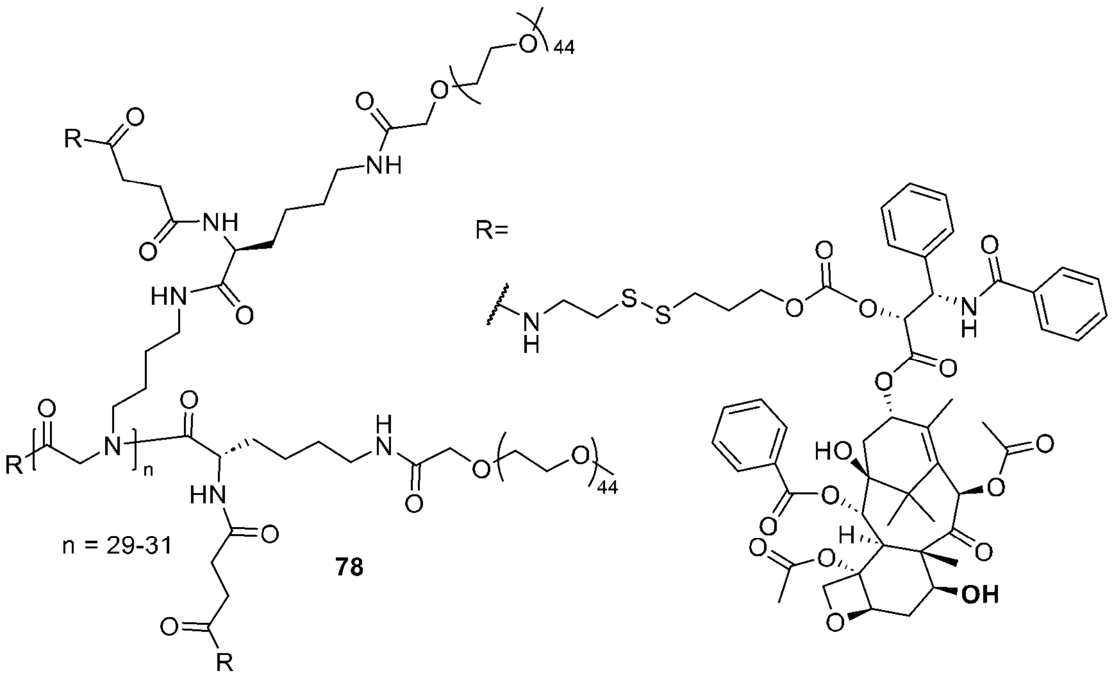

78

,

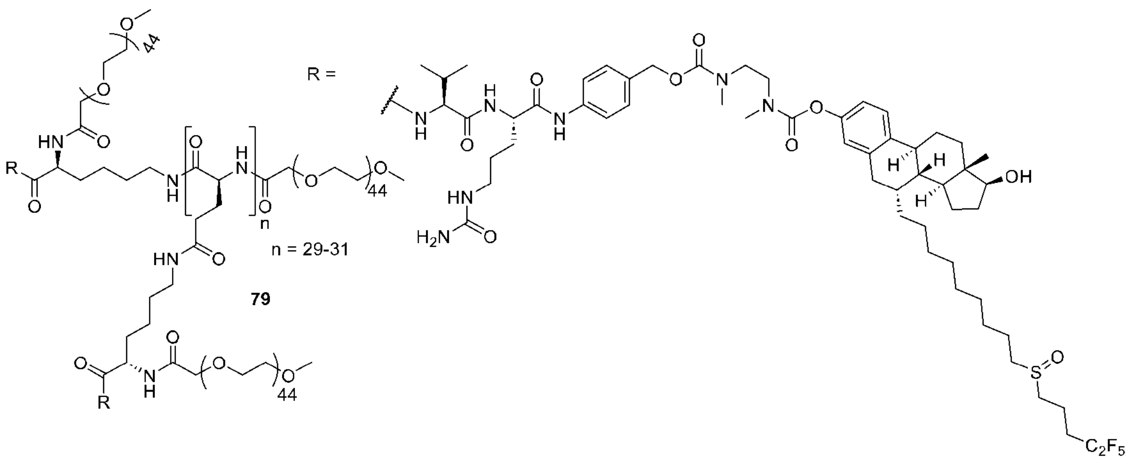

79

,

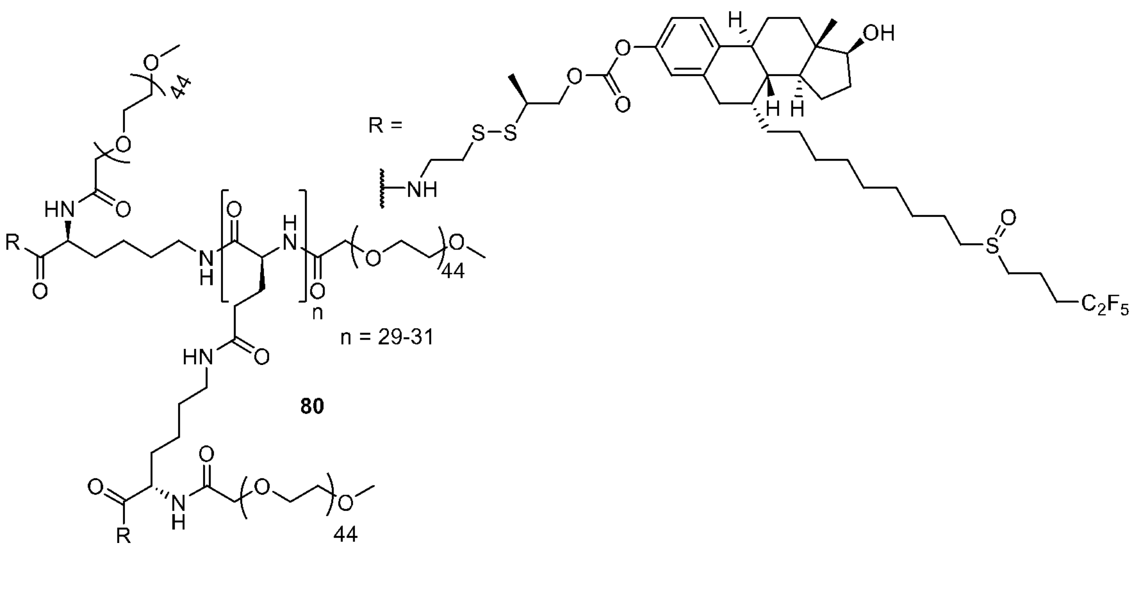

80

,

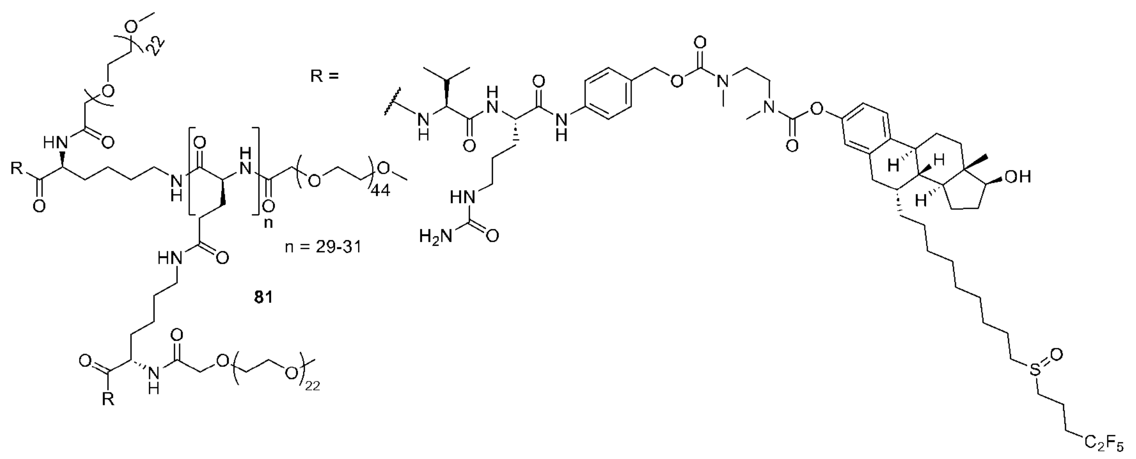

81

n = 29-31

R =

,

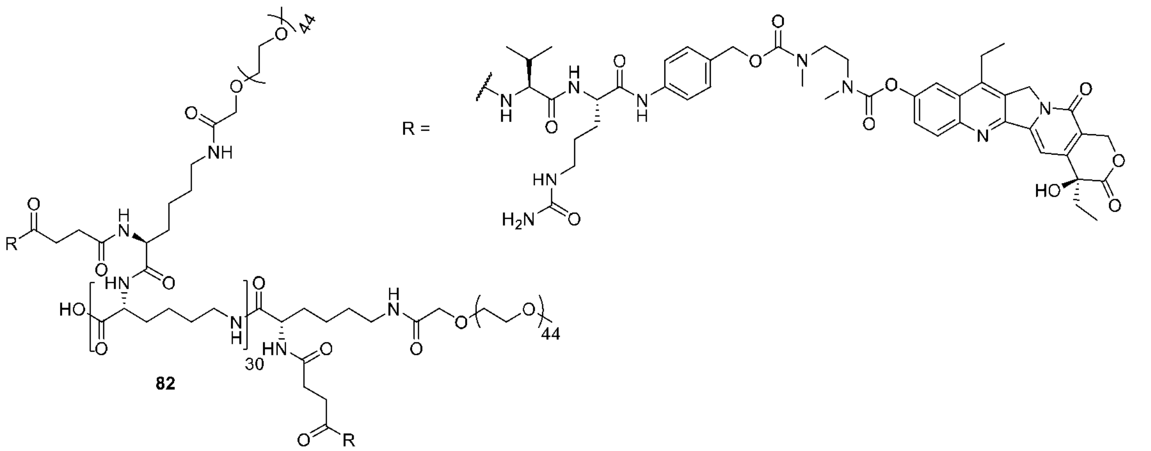

82

R =

,

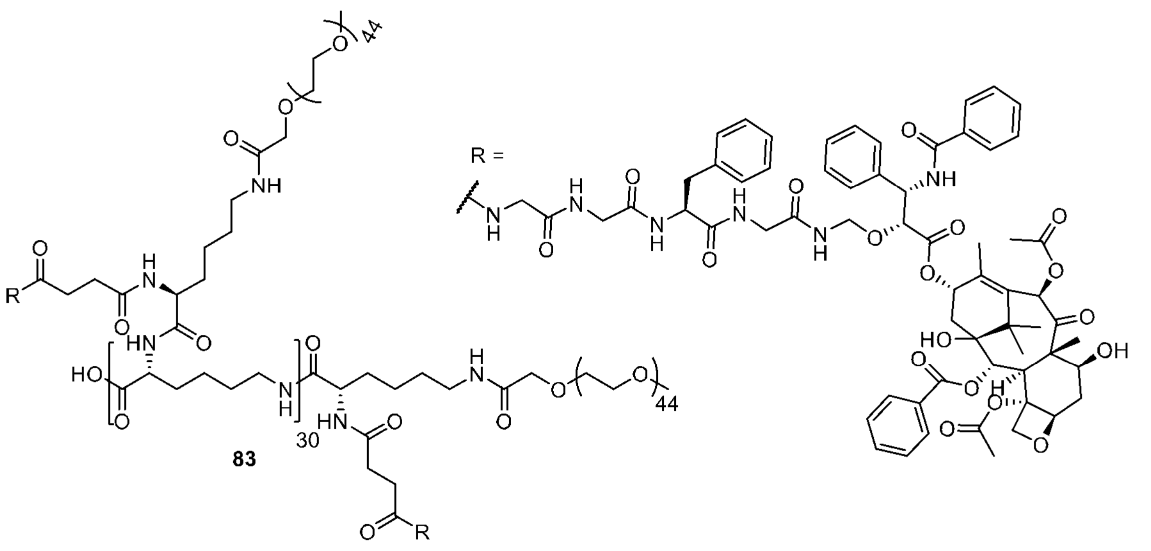

83

R =

,

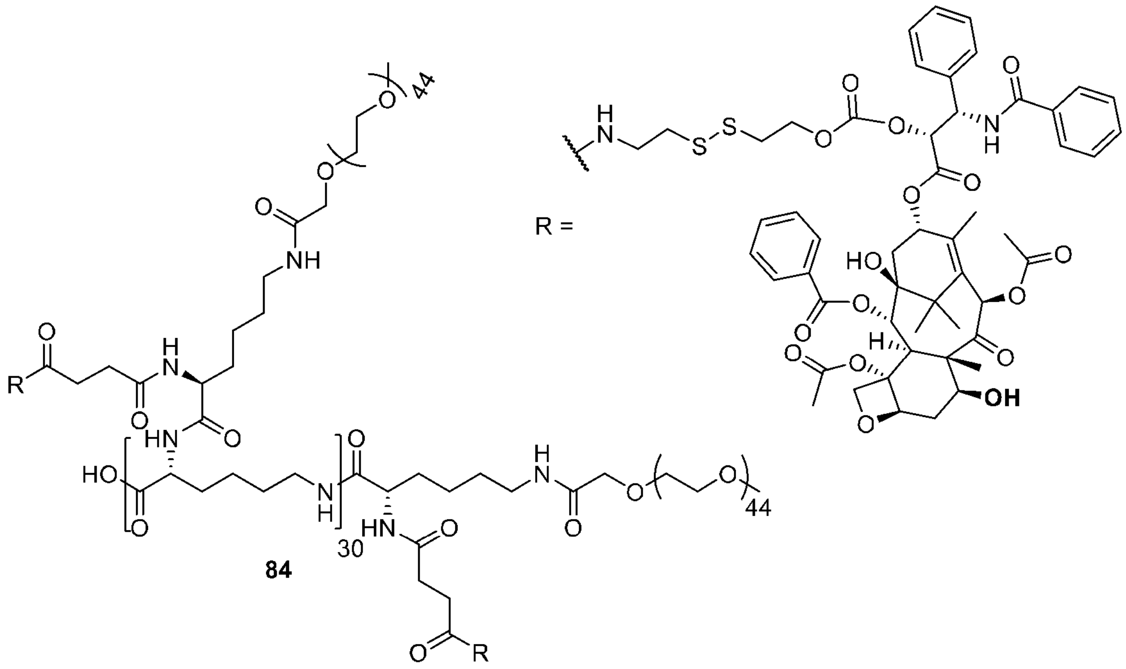

R =

84

,

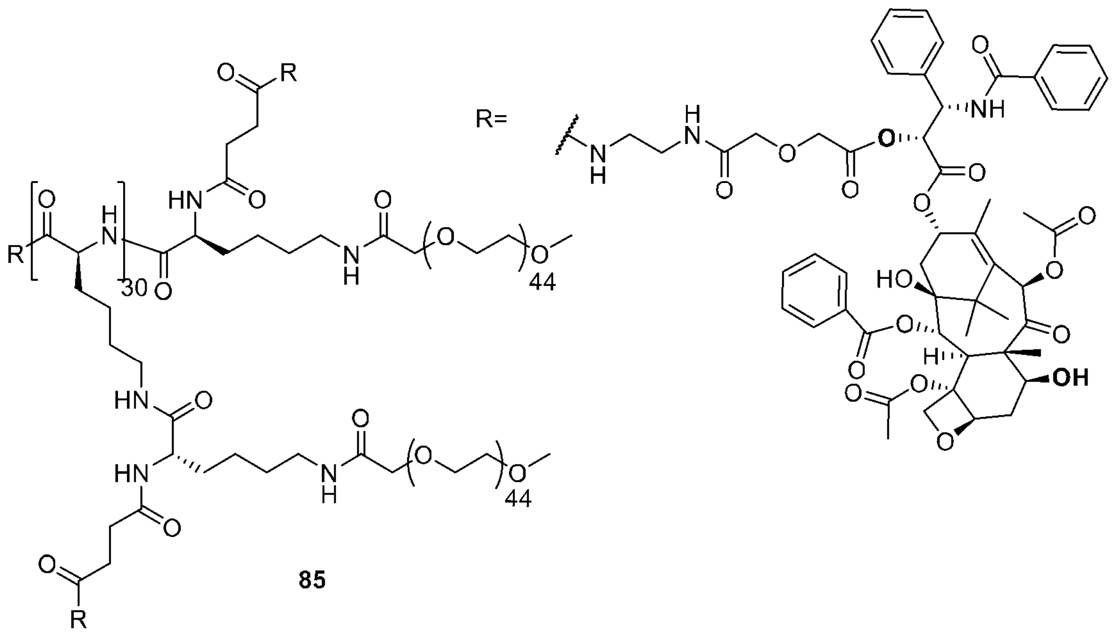

R=

85

,

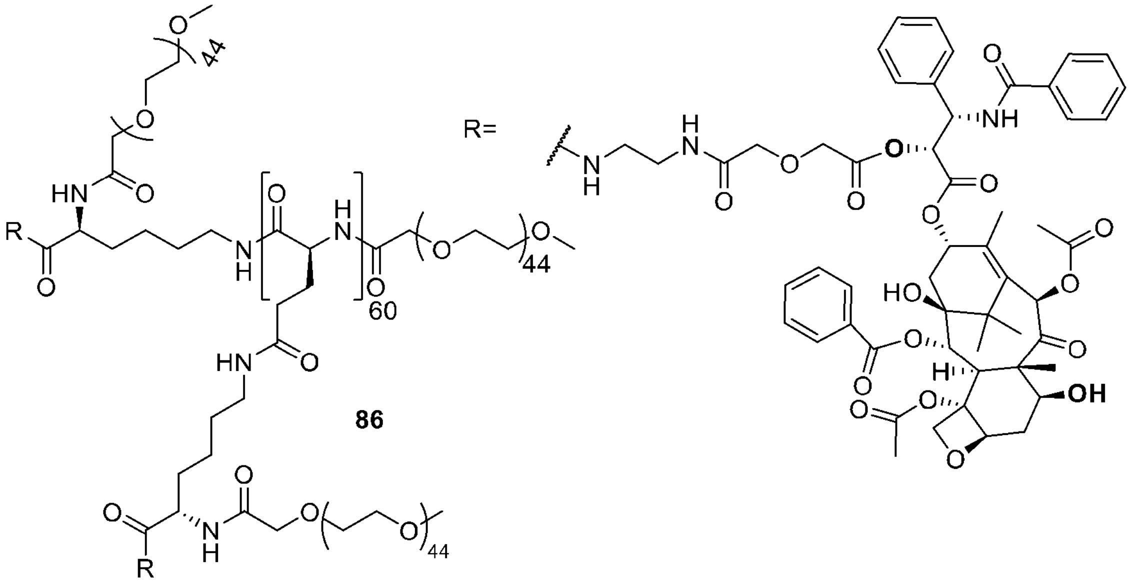

86

,

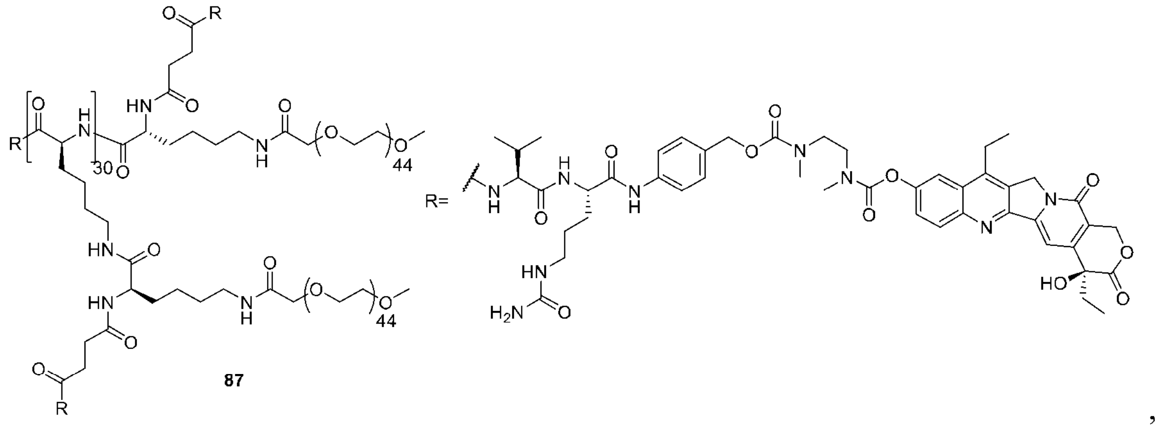

87

,

or

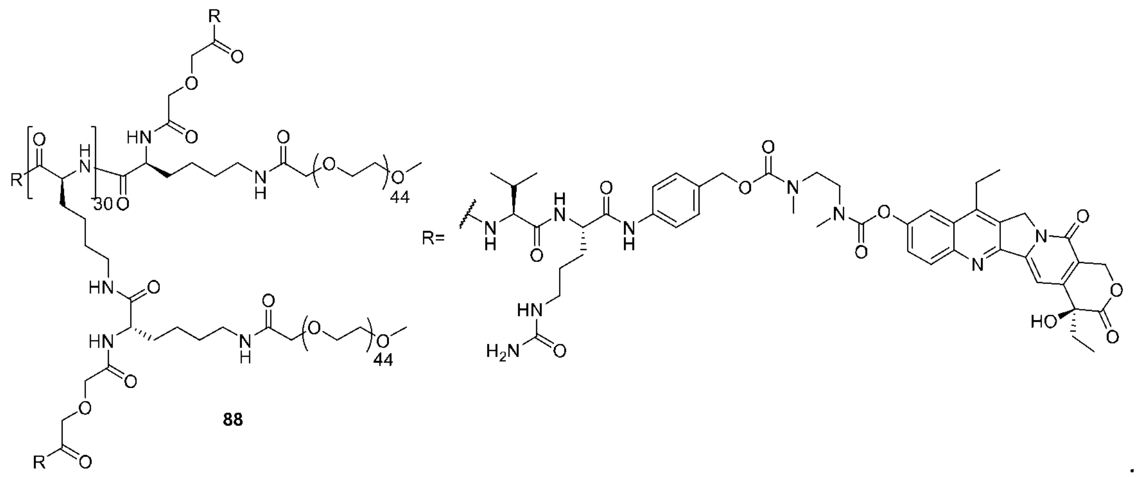

88

.

**42.** A pharmaceutical composition, comprising the compound or the pharmaceutically acceptable salt thereof according to any one of claims 1-41, and a pharmaceutically acceptable excipient.

**43.** Use of the compound or the pharmaceutically acceptable salt thereof according to any one of claims 1-41 or the

pharmaceutical composition according to claim 42 in preparing a medicament for preventing and/or treating a disease, wherein the disease is a cancer selected from, for example: one or more of breast cancer, ovarian cancer, prostate cancer, melanoma, brain cancer, nasopharyngeal cancer, esophageal cancer, gastric cancer, liver cancer, pancreatic cancer, colorectal cancer, lung cancer, renal cancer, skin cancer, glioblastoma, neuroblastoma, sarcoma, liposarcoma, osteochondroma, bone cancer, osteosarcoma, seminoma, testicular tumor, uterine tumor, head and neck tumor, multiple myeloma, malignant lymphoma, polycythemia vera, leukemia, thyroid tumor, ureteral tumor, bladder tumor, gallbladder cancer, bile duct cancer, chorioepithelial cancer, and pediatric tumor, preferably one or more of pancreatic cancer, liver cancer, colon cancer, myeloma, lung cancer (e.g., small cell lung cancer), fibrosarcoma, and breast cancer.

**44.** A method for preventing and/or treating a disease, comprising: administering to a subject in need a therapeutically effective amount of the compound or the pharmaceutically acceptable salt thereof according to any one of claims 1-41, wherein the disease is a cancer selected from, for example: one or more of breast cancer, ovarian cancer, prostate cancer, melanoma, brain cancer, nasopharyngeal cancer, esophageal cancer, gastric cancer, liver cancer, pancreatic cancer, colorectal cancer, lung cancer, renal cancer, skin cancer, glioblastoma, neuroblastoma, sarcoma, liposarcoma, osteochondroma, bone cancer, osteosarcoma, seminoma, testicular tumor, uterine tumor, head and neck tumor, multiple myeloma, malignant lymphoma, polycythemia vera, leukemia, thyroid tumor, ureteral tumor, bladder tumor, gallbladder cancer, bile duct cancer, chorioepithelial cancer, and pediatric tumor, preferably one or more of pancreatic cancer, liver cancer, colon cancer, myeloma, lung cancer (e.g., small cell lung cancer), fibrosarcoma, and breast cancer.

**45.** A compound of formula (I-1-A), (I-1-B), or (I-1-C):

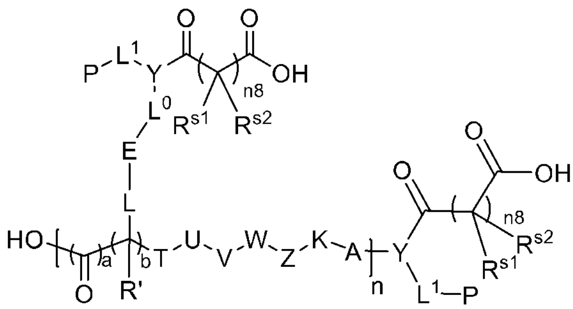

(I-1-A)

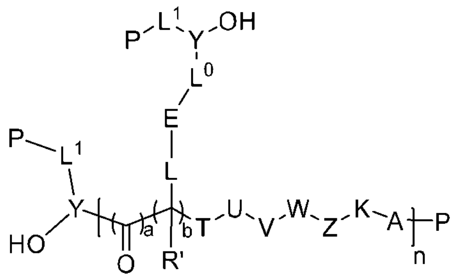

(I-1-B)

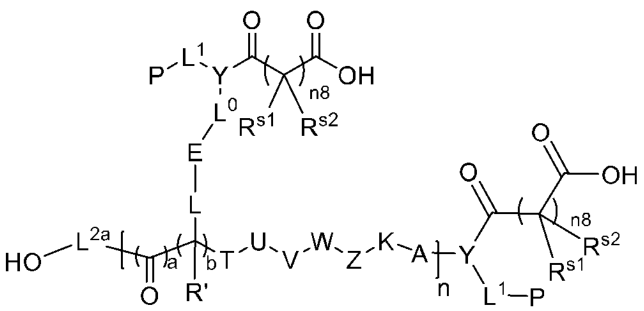

(I-1-C)

wherein, in the compound of formula (I-1-A), Y is

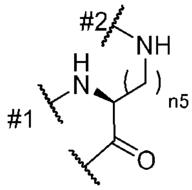

,

the "#1" terminal is linked to $L^2$, and the "#2" terminal is linked to $L^1$;

in the compound of formula (I-1-B), Y is

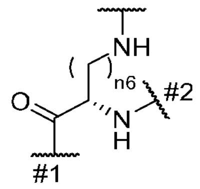

,

the "#1" terminal is linked to $L^2$, and the "#2" terminal is linked to $L^1$;
in the compound of formula (I-1-C), Y is

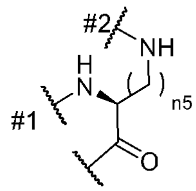

,

the "#1" terminal is linked to $L^2$, and the "#2" terminal is linked to $L^1$;
in the compound of formula (I-1-A), (I-1-B), or (I-1-C), the variables are as defined in any one of claims 35-41.

**46.** A compound of any one of the following structures:

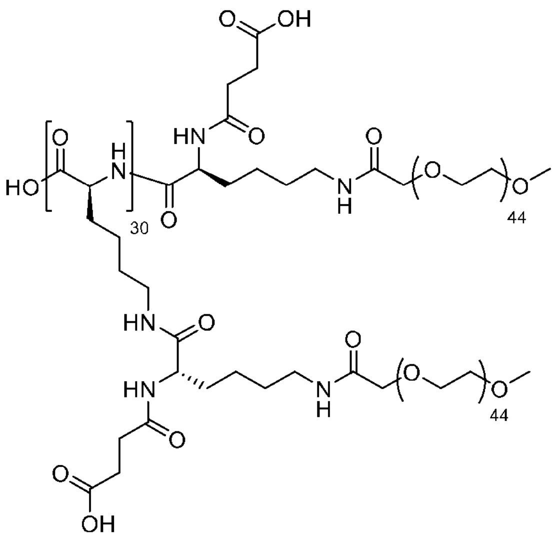

,

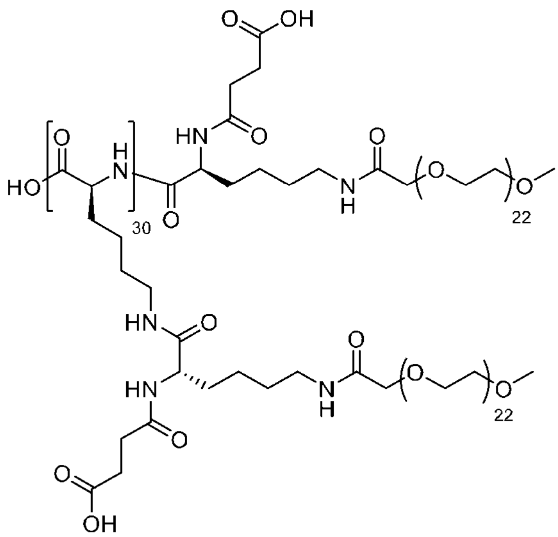

,

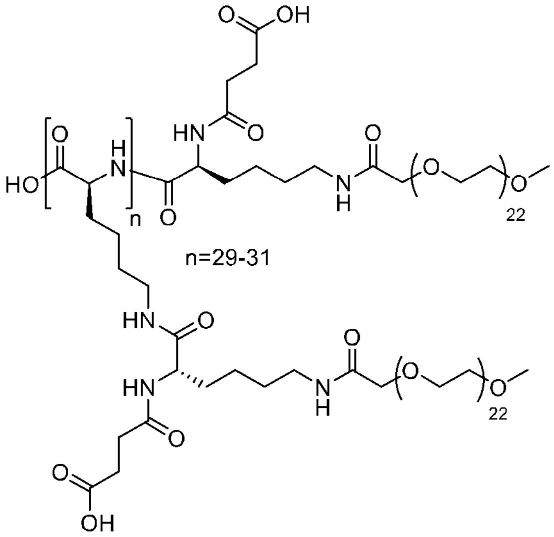

,

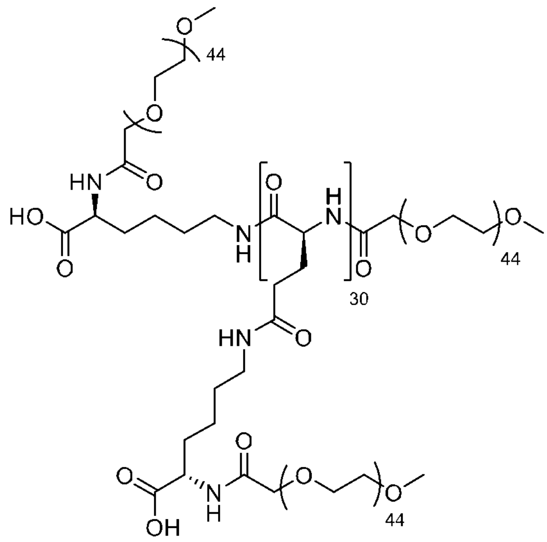

,

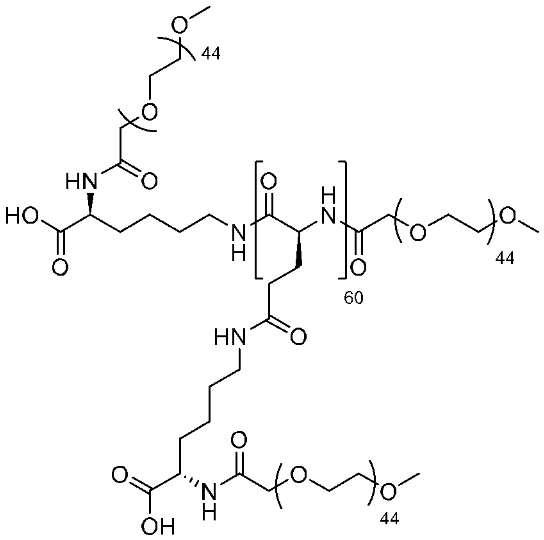

,

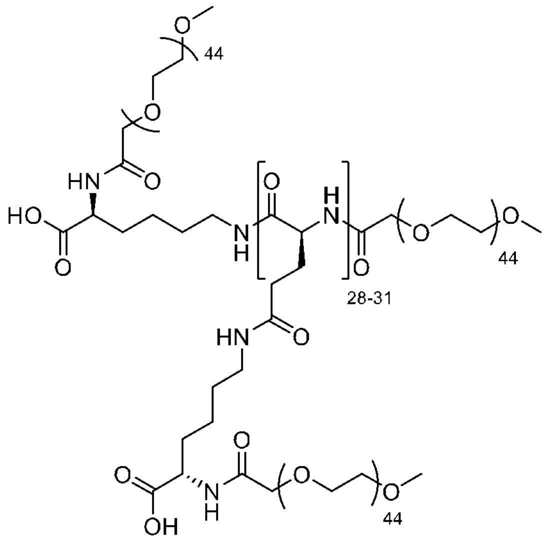

,

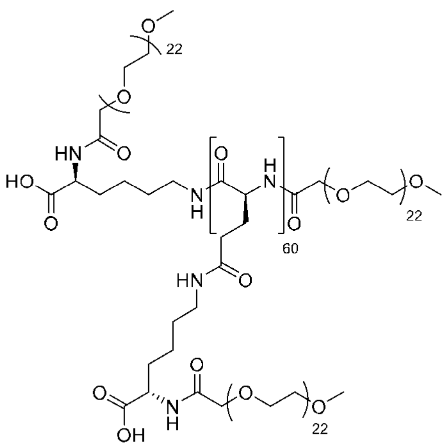

,

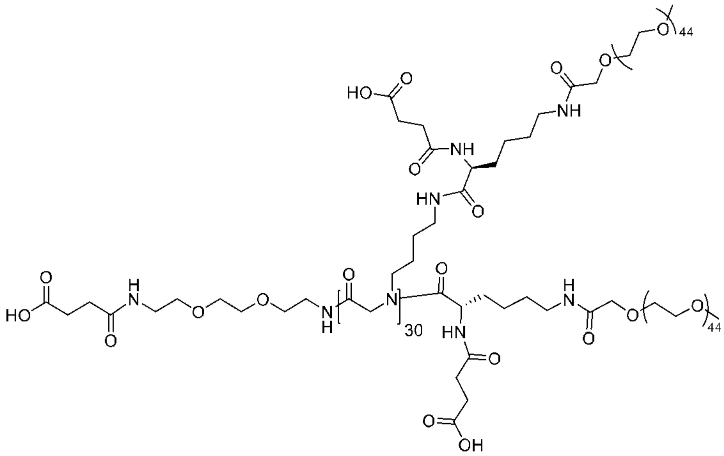

,

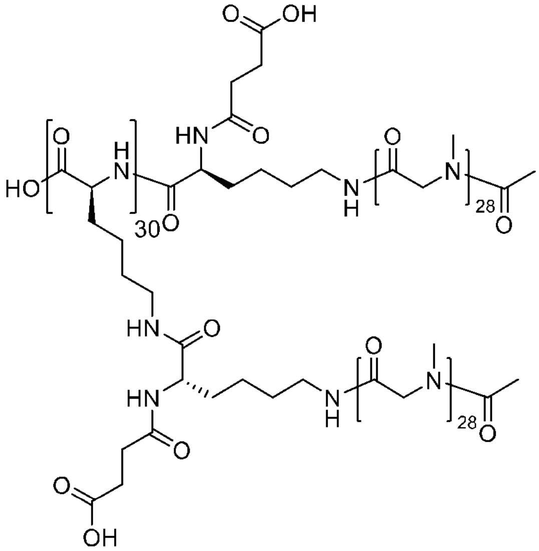

,

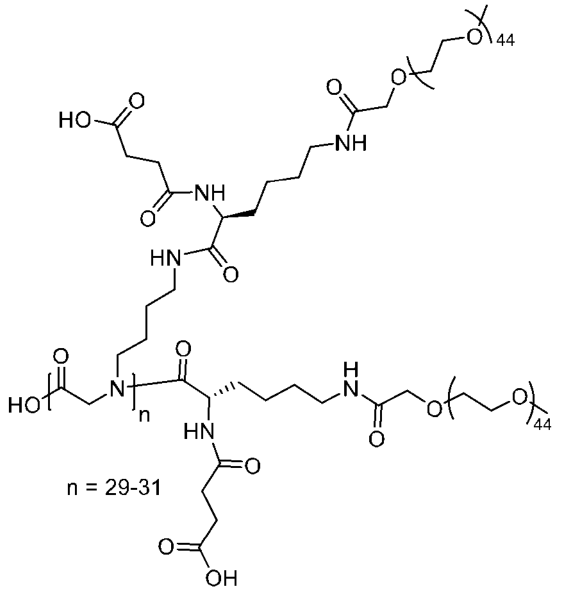

n = 29-31

,

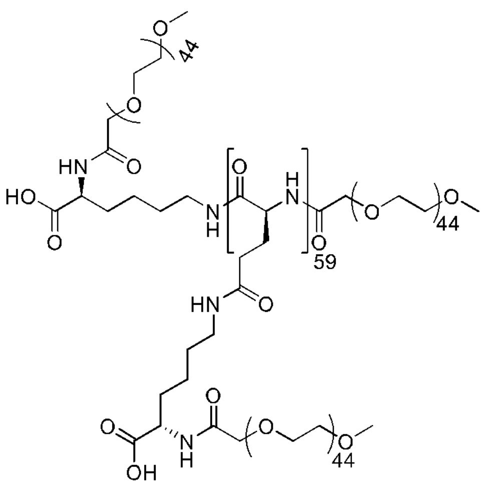

,

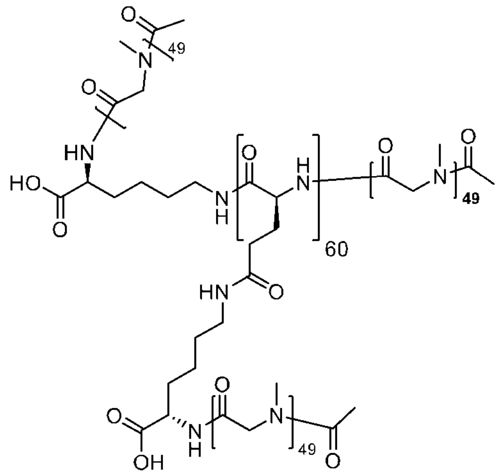

,

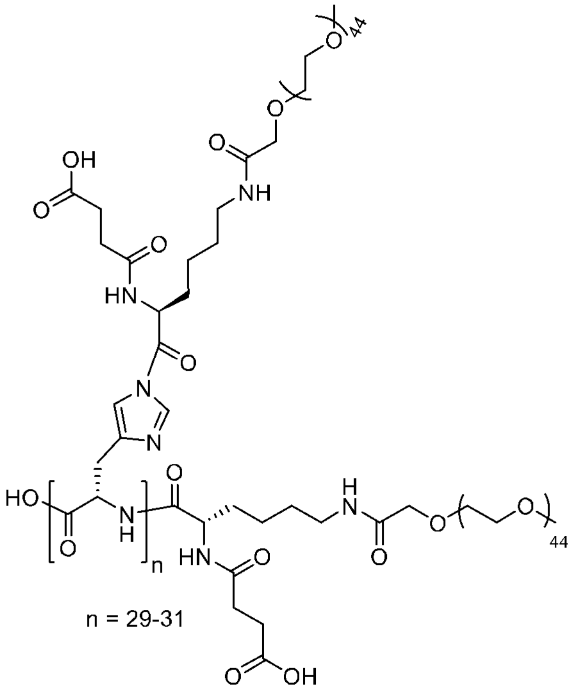

n = 29-31

,

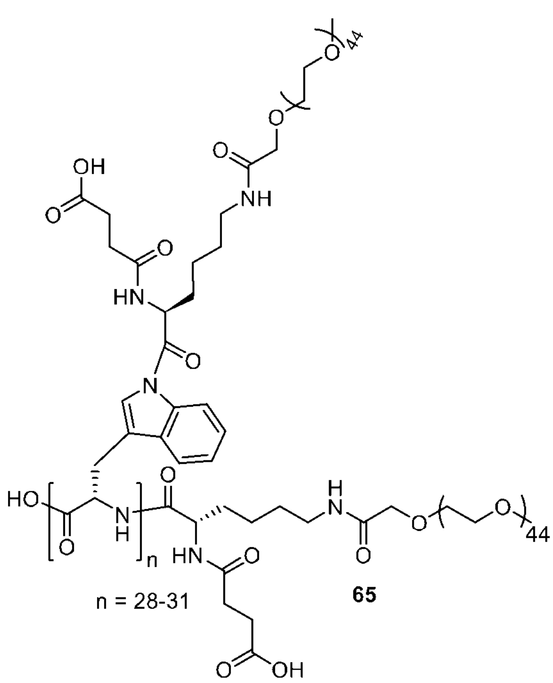

n = 28-31

**65**

,

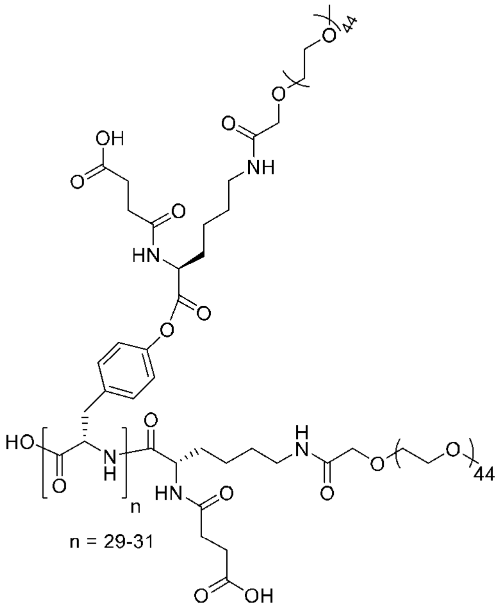

n = 29-31

,

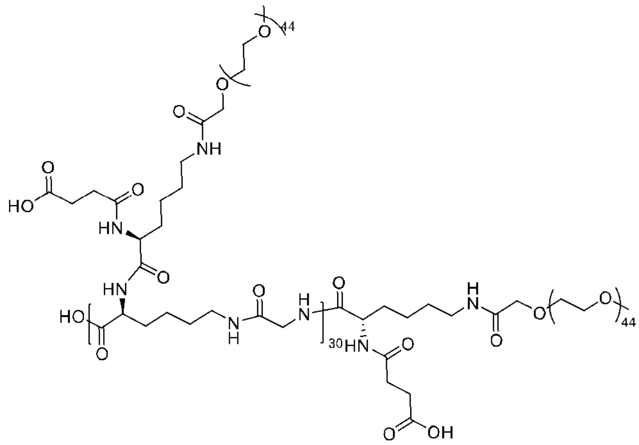

,

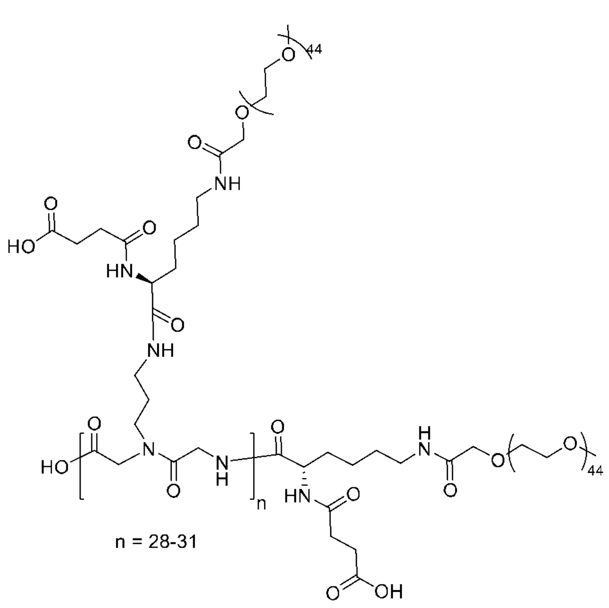

,

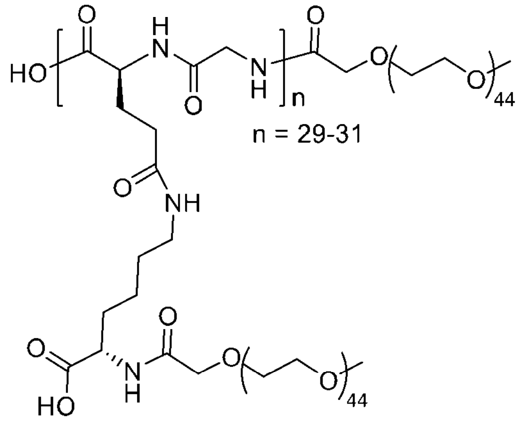

,

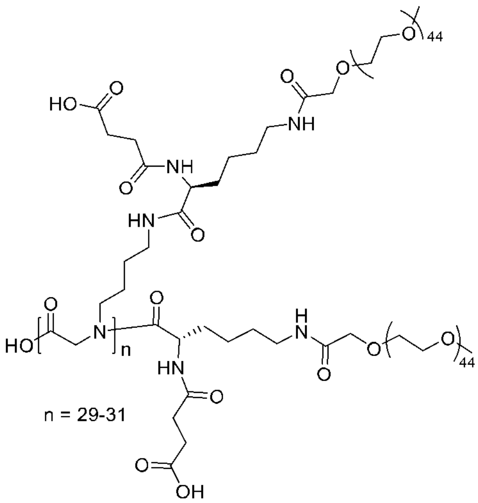

, or

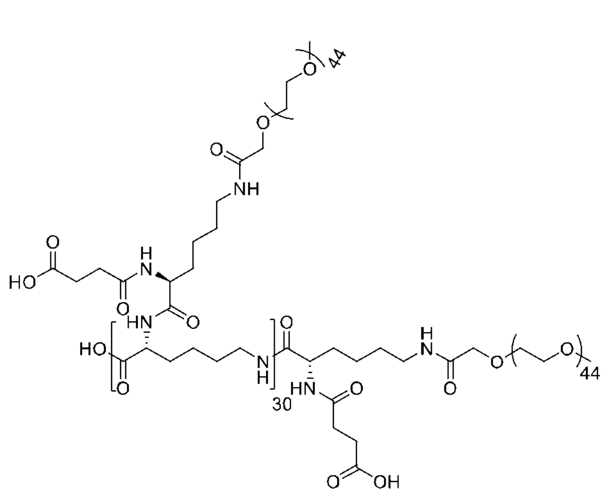

.

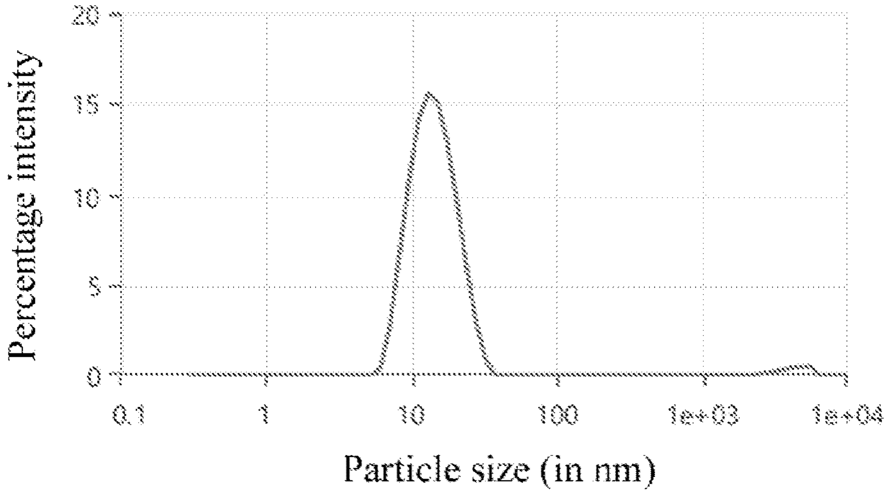


**Example 7**

FIG. 1

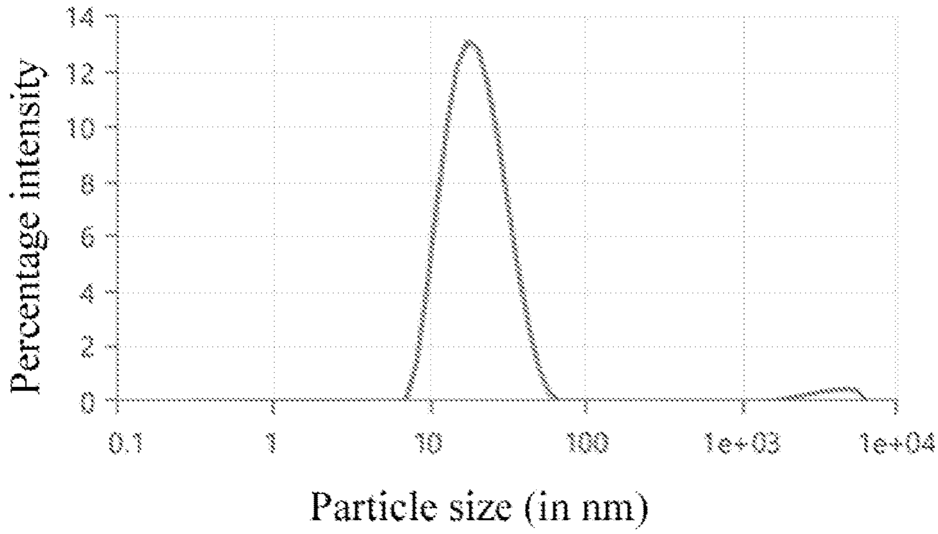


**Example 20**

FIG. 2

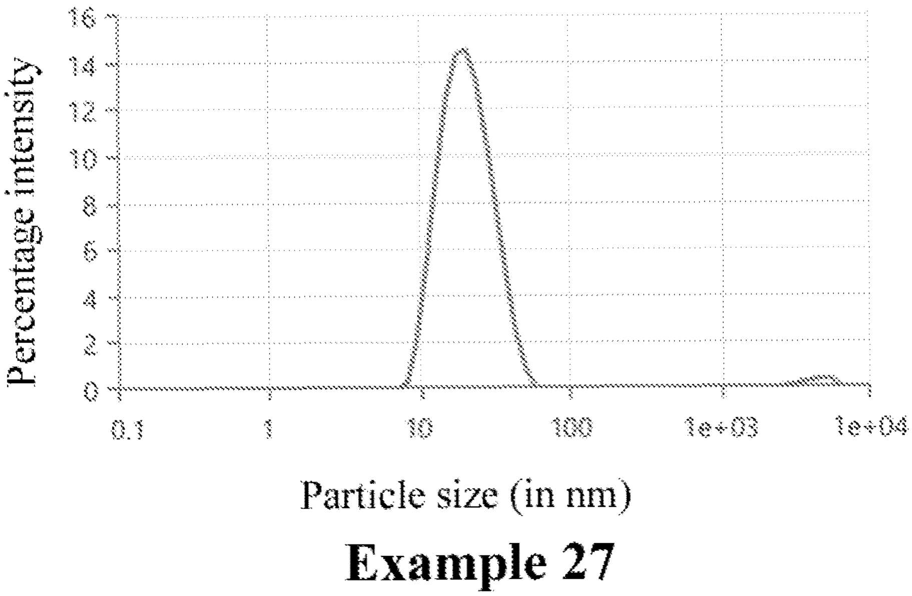
16
14
12
10
8
6
4
2
0
Percentage intensity
0.1
1
10
100
1e+03
1e+04
Particle size (in nm)
Example 27

FIG. 3

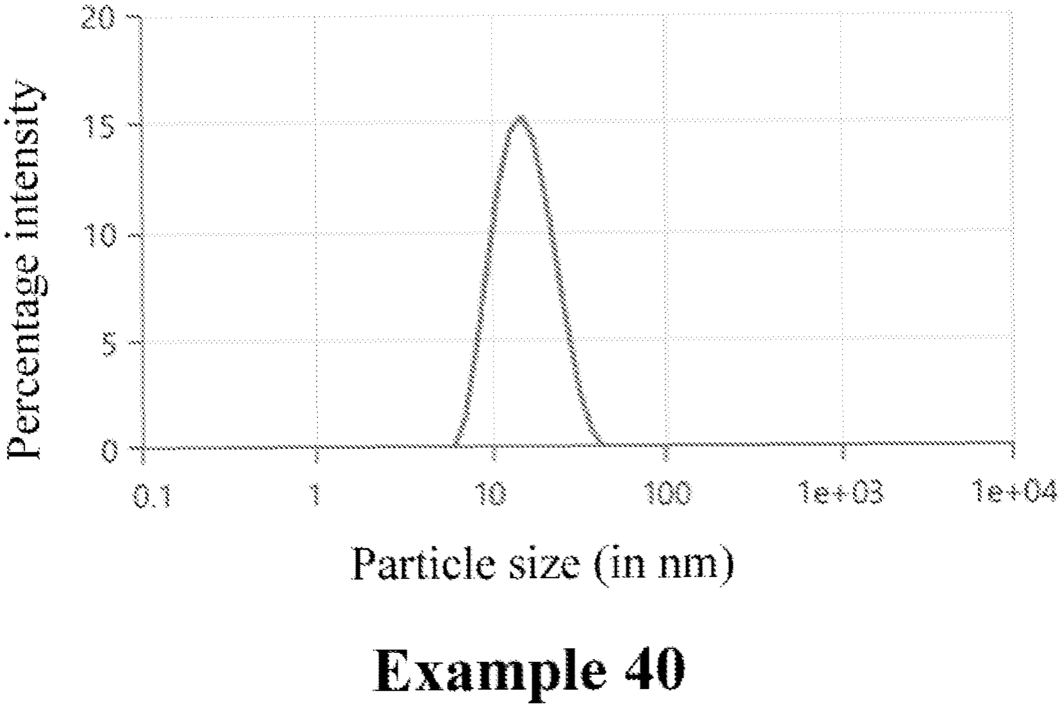
20
15
10
5
0
Percentage intensity
0.1
1
10
100
1e+03
1e+04
Particle size (in nm)
Example 40

FIG. 4

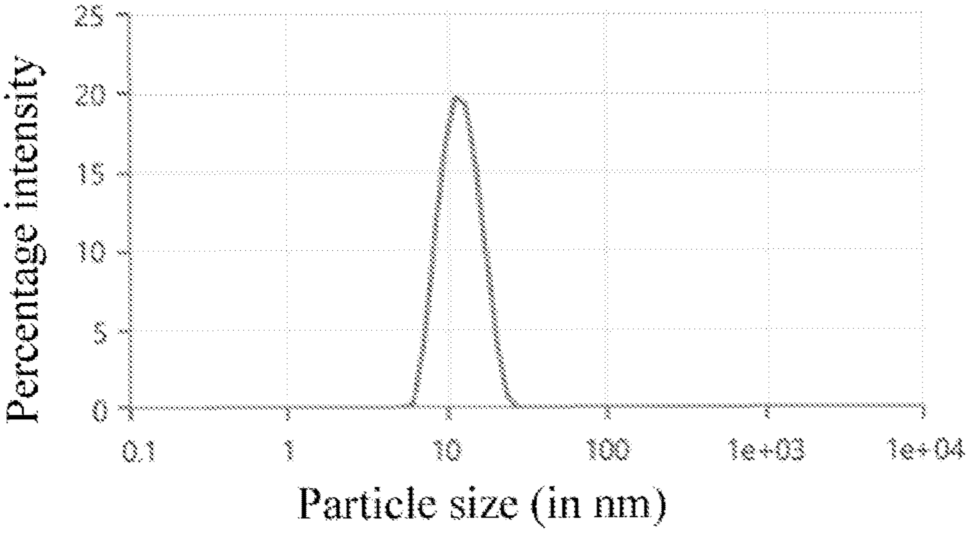


**Example 41**

FIG. 5

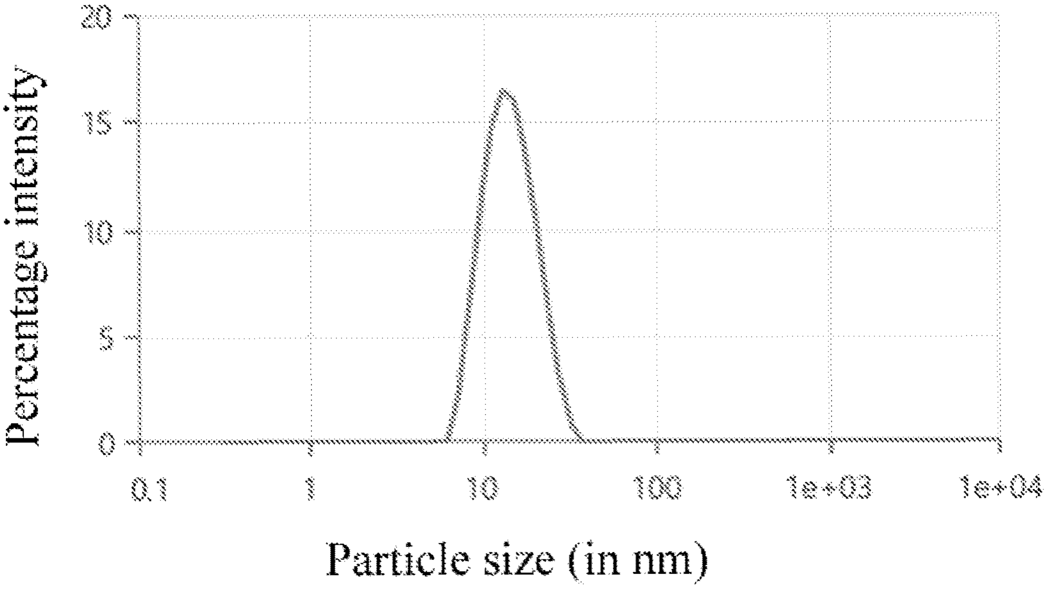


**Example 48**

FIG. 6

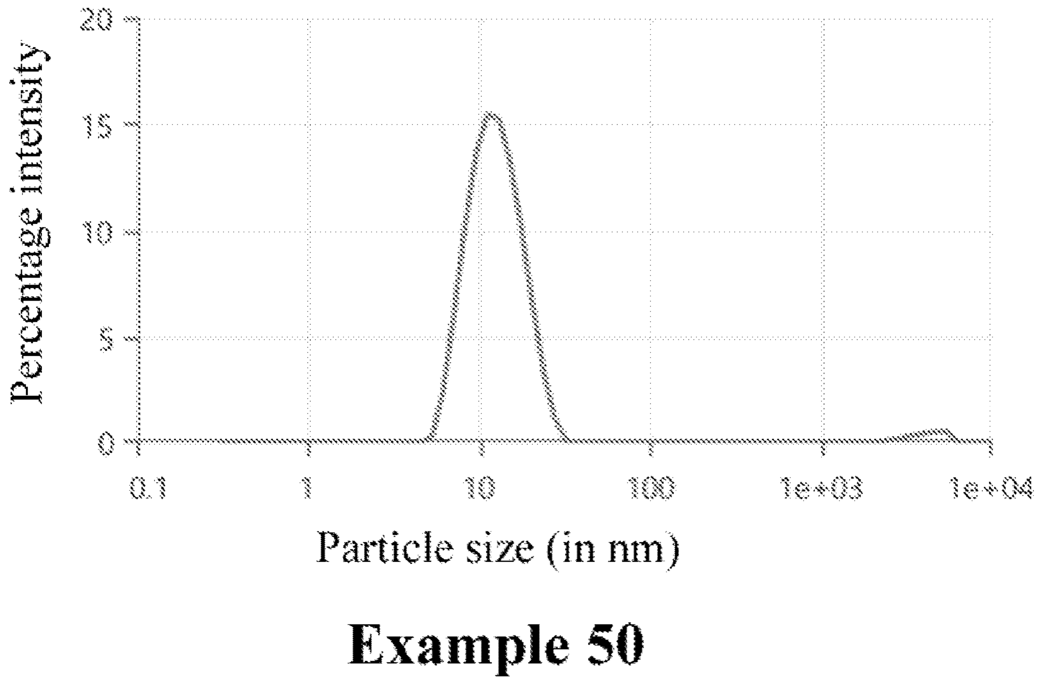
Percentage intensity
20
15
10
5
0
0.1
1
10
100
1e+03
1e+04
Particle size (in nm)
Example 50

FIG. 7

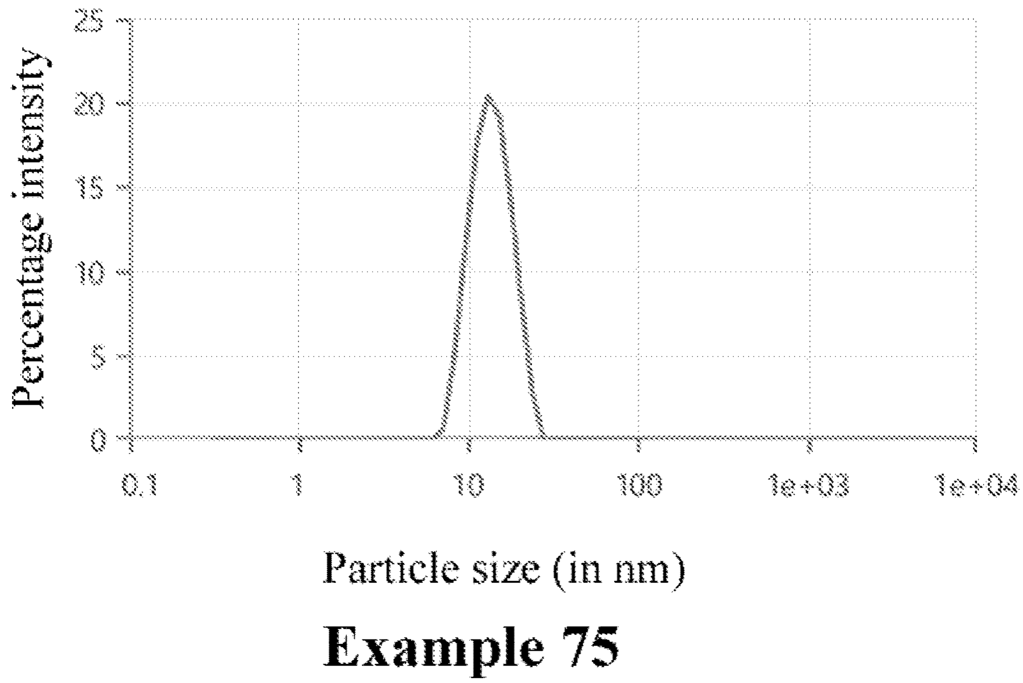
Percentage intensity
25
20
15
10
5
0
0.1
1
10
100
1e+03
1e+04
Particle size (in nm)
Example 75

FIG. 8

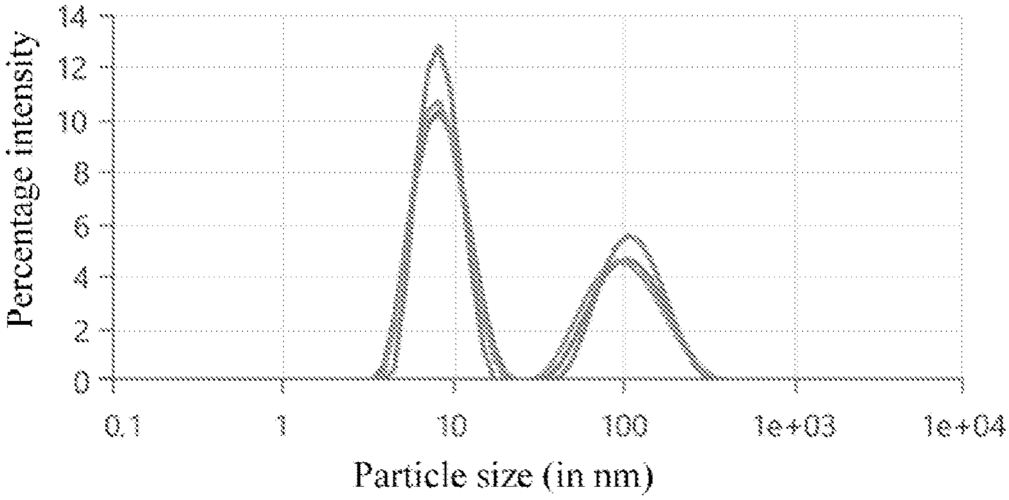


**Example 78**

FIG. 9

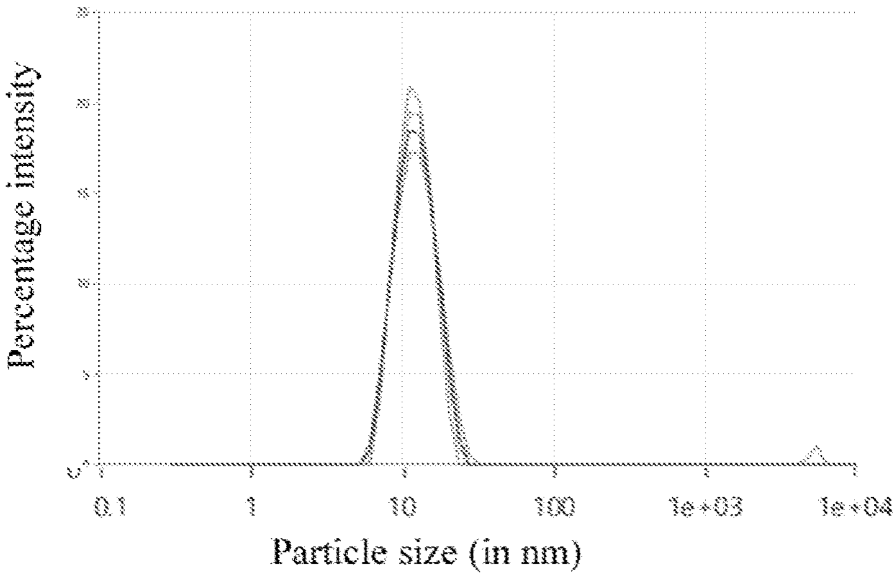


**Example 71, 25 °C preservation, day 0**

FIG. 10

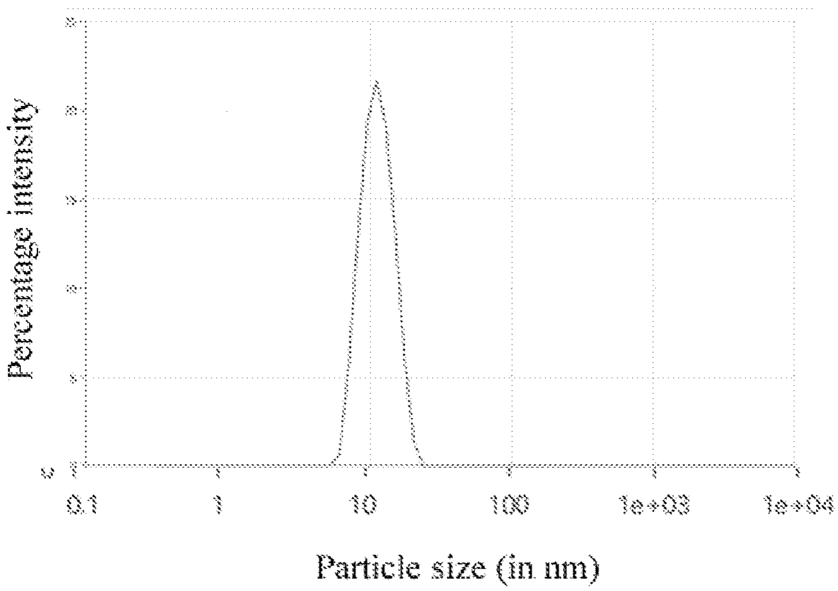


**Example 71, 25 °C preservation, day 30**

FIG. 11

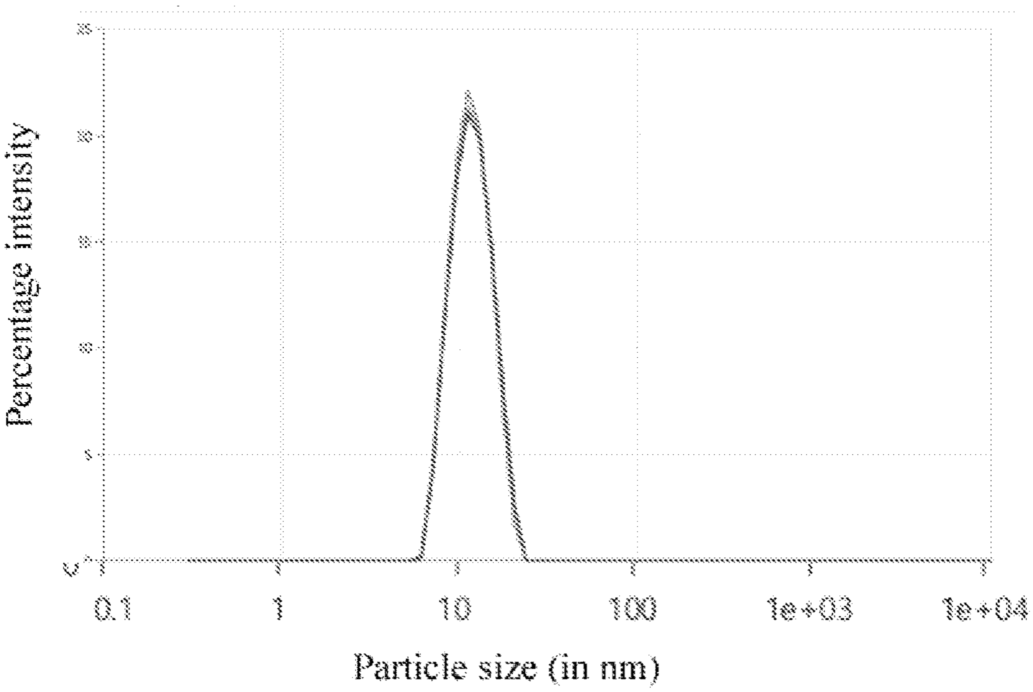


**Example 71, 25 °C preservation, day 60**

FIG. 12

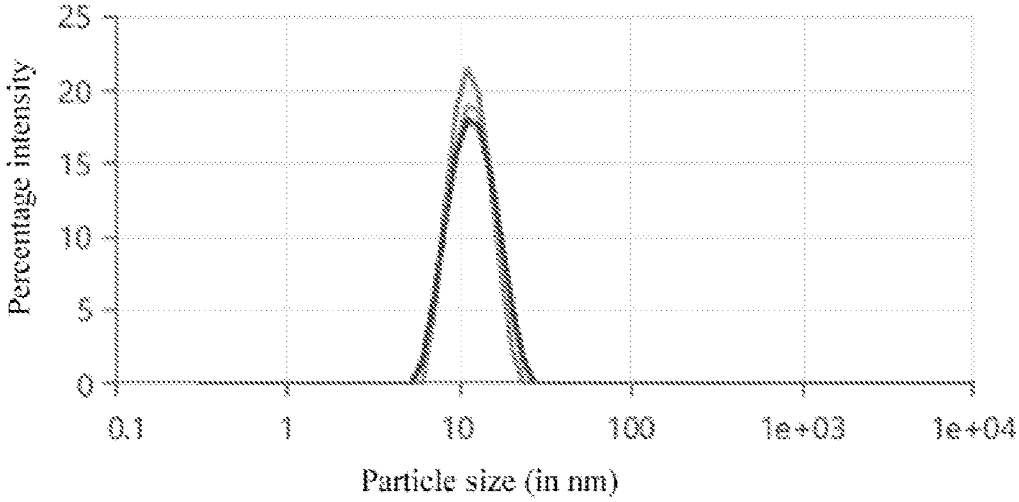


**Example 71, 25 °C preservation, day 90**

FIG. 13

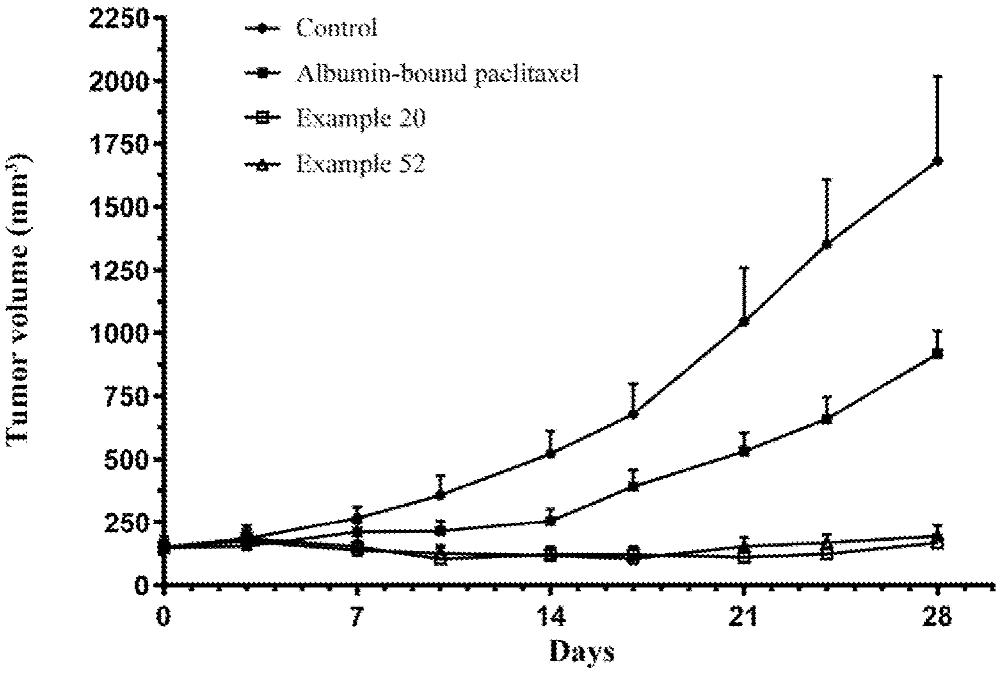


FIG. 14

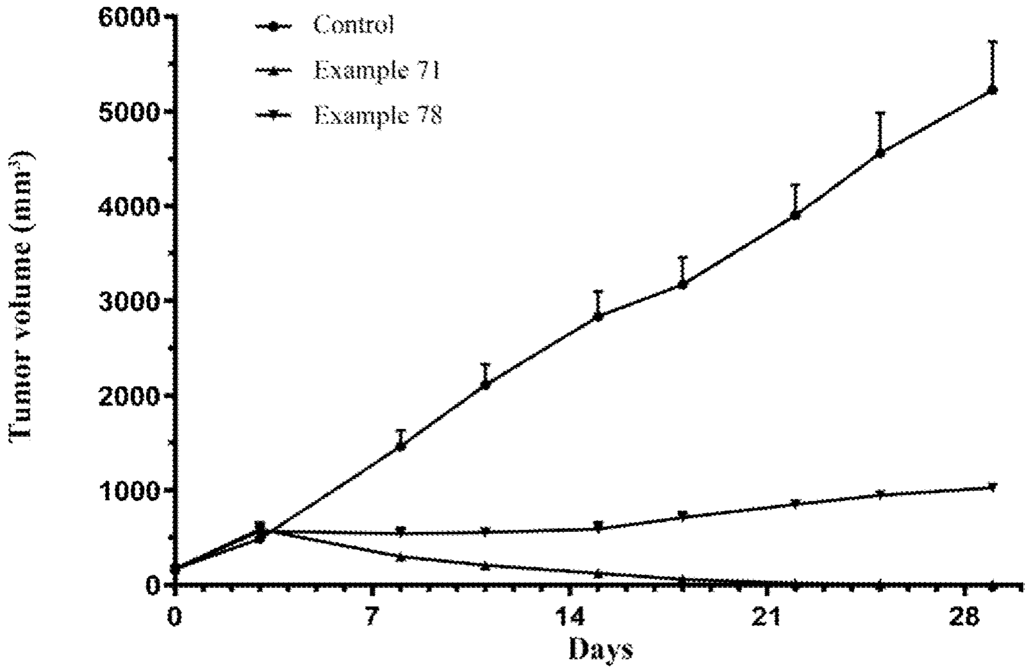

Control
Example 71
Example 78
Tumor volume (mm³)
6000
5000
4000
3000
2000
1000
0
0
7
14
21
28
Days


FIG. 15

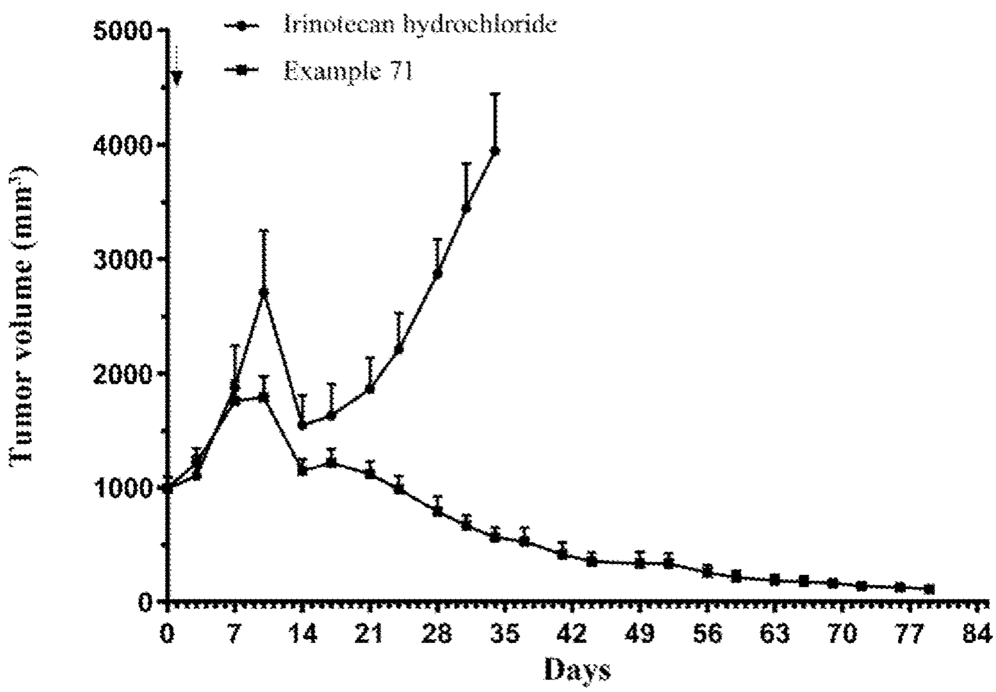

Irinotecan hydrochloride
Example 71
Tumor volume (mm³)
5000
4000
3000
2000
1000
0
0
7
14
21
28
35
42
49
56
63
70
77
84
Days


FIG. 16

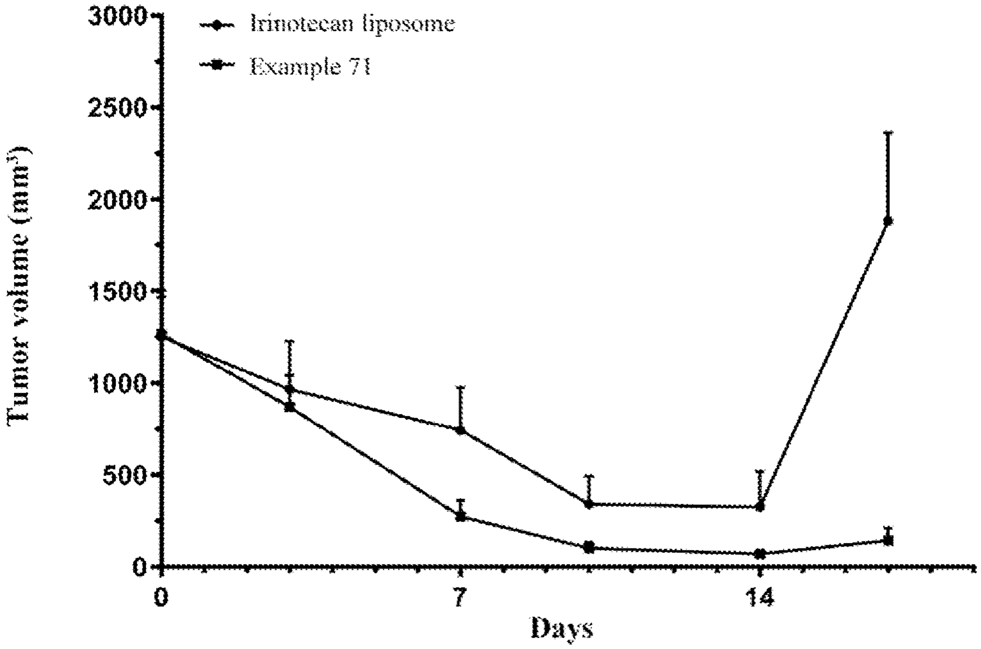
Irinotecan liposome
Example 71
Tumor volume (mm³)
3000
2500
2000
1500
1000
500
0
0
7
14
Days

FIG. 17

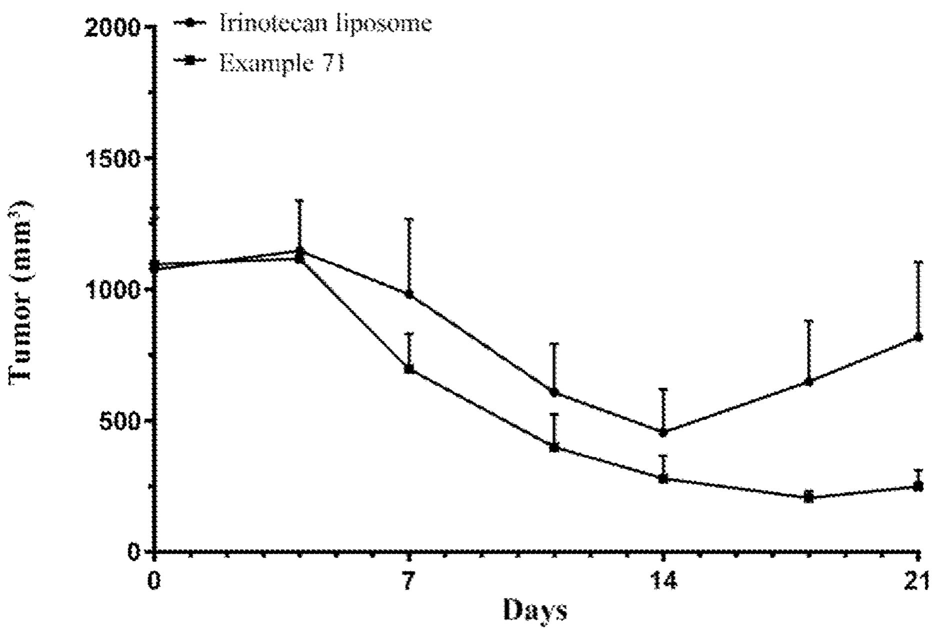
Irinotecan liposome
Example 71
Tumor (mm³)
2000
1500
1000
500
0
0
7
14
21
Days

FIG. 18

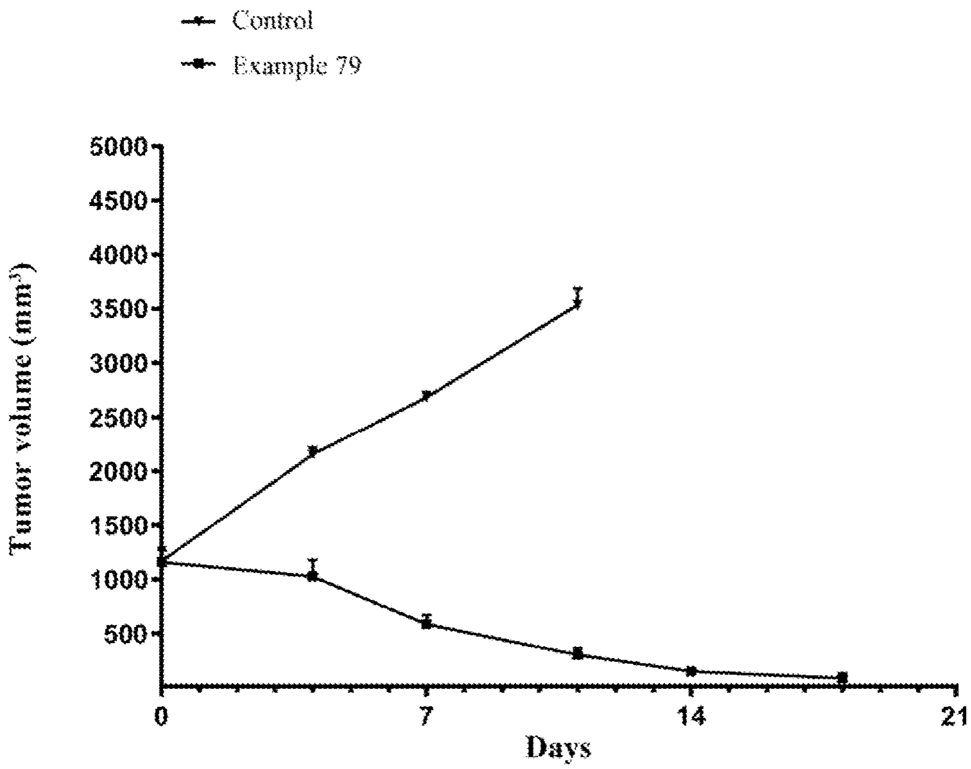
Control
Example 79
5000
4500
4000
3500
3000
2500
2000
1500
1000
500
Tumor volume (mm³)
0
7
14
21
Days

FIG. 19

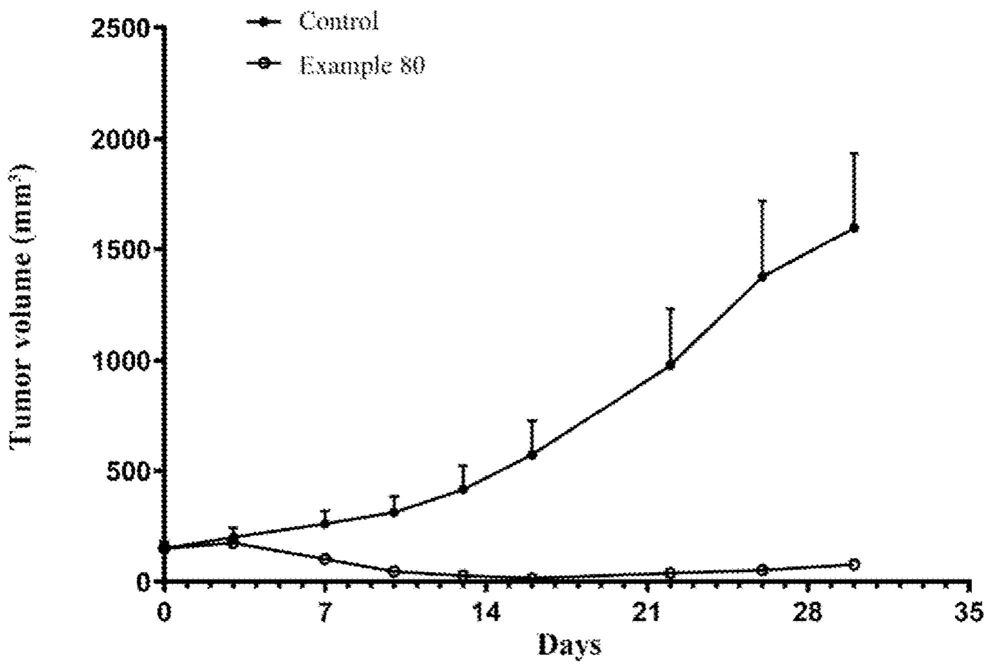
Control
Example 80
2500
2000
1500
1000
500
0
Tumor volume (mm³)
0
7
14
21
28
35
Days

FIG. 20

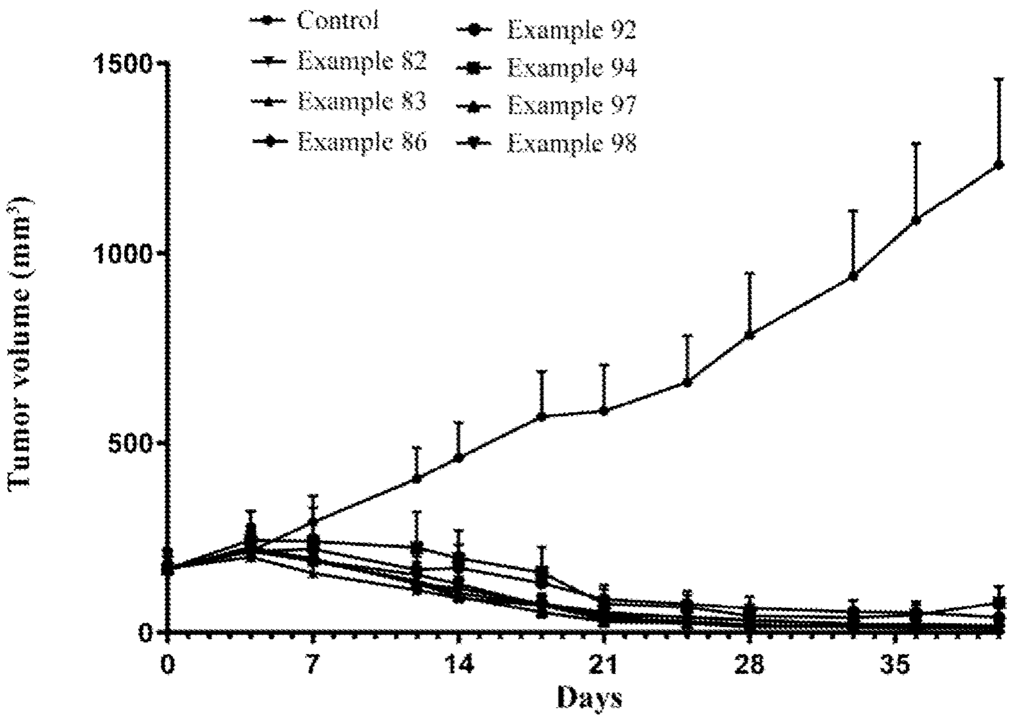
Control
Example 82
Example 83
Example 86
Example 92
Example 94
Example 97
Example 98
1500
1000
500
0
Tumor volume (mm³)
0
7
14
21
28
35
Days

FIG. 21

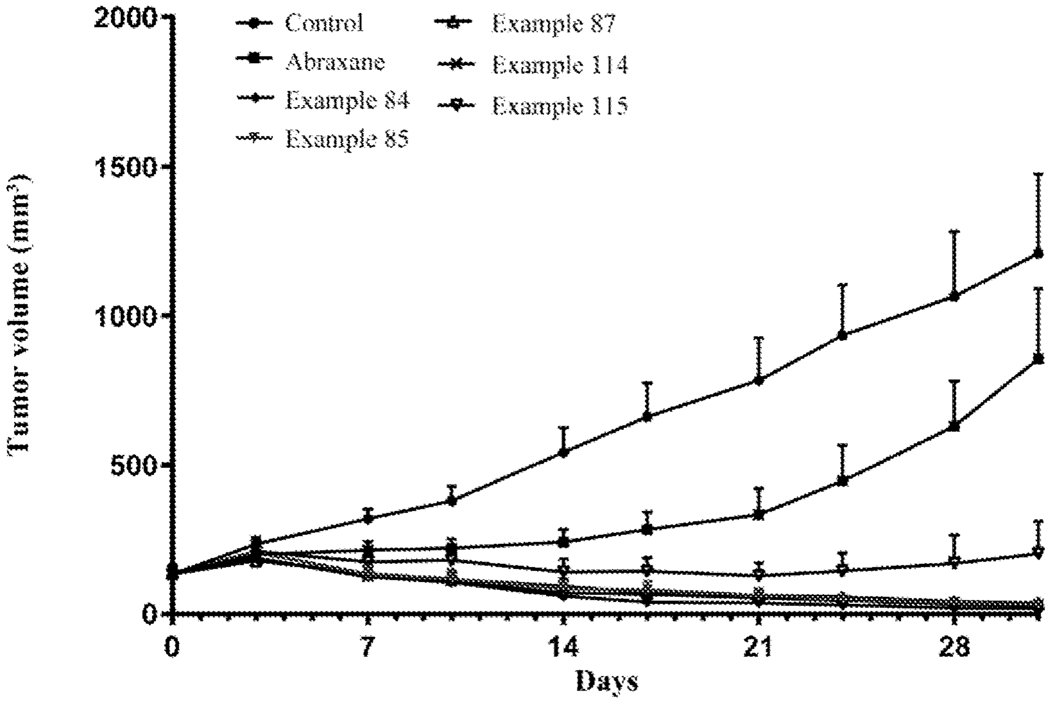
Control
Abraxane
Example 84
Example 85
Example 87
Example 114
Example 115
2000
1500
1000
500
0
Tumor volume (mm³)
0
7
14
21
28
Days

FIG. 22

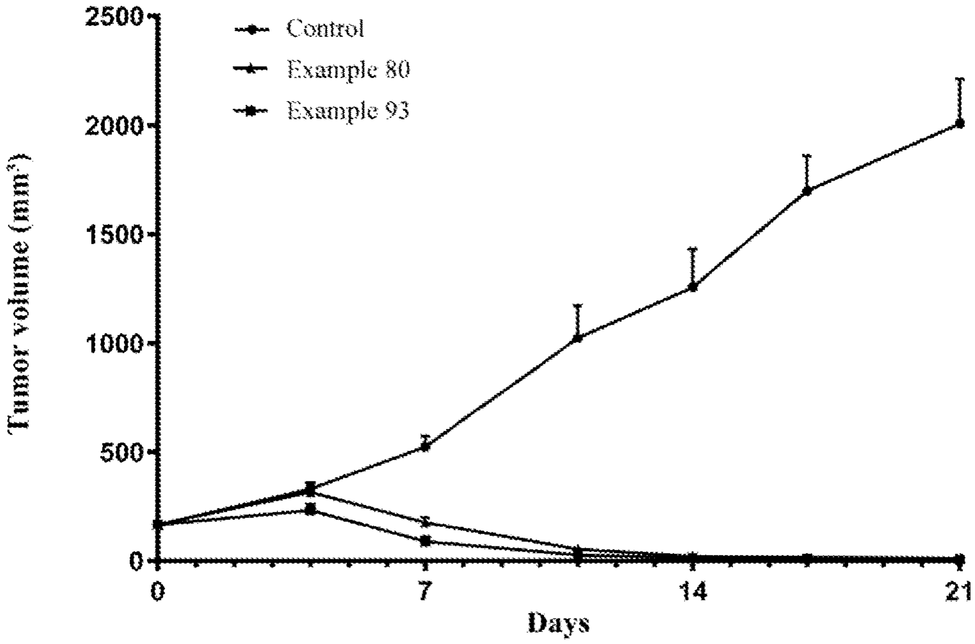

2500
2000
1500
1000
500
0
Tumor volume (mm³)
Control
Example 80
Example 93
0
7
14
21
Days


FIG. 23

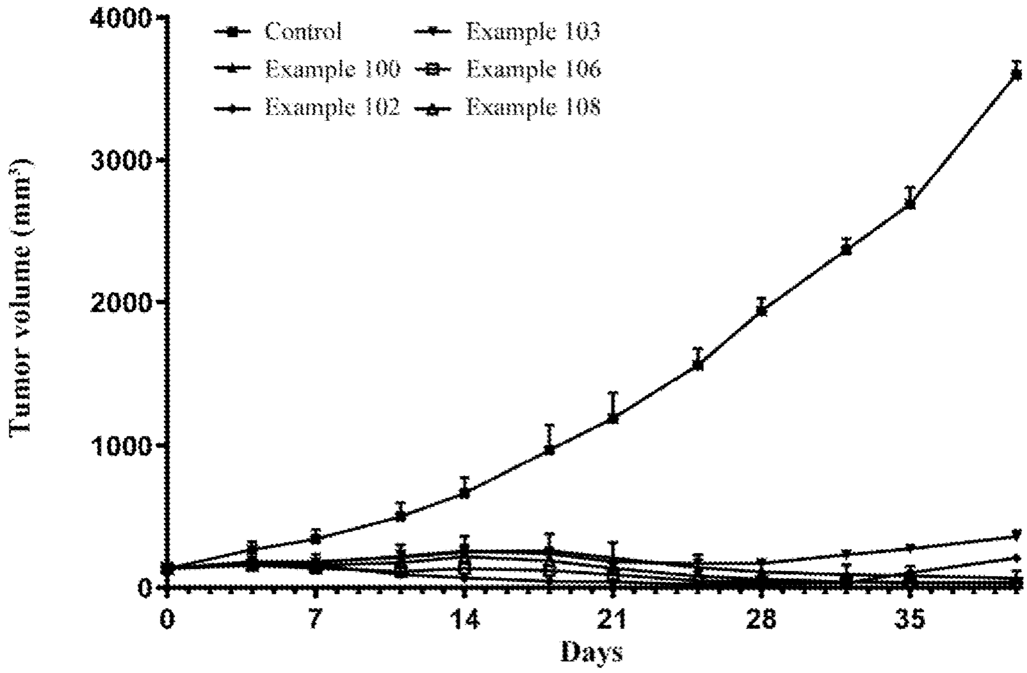

4000
3000
2000
1000
0
Tumor volume (mm³)
Control
Example 100
Example 102
Example 103
Example 106
Example 108
0
7
14
21
28
35
Days


FIG. 24

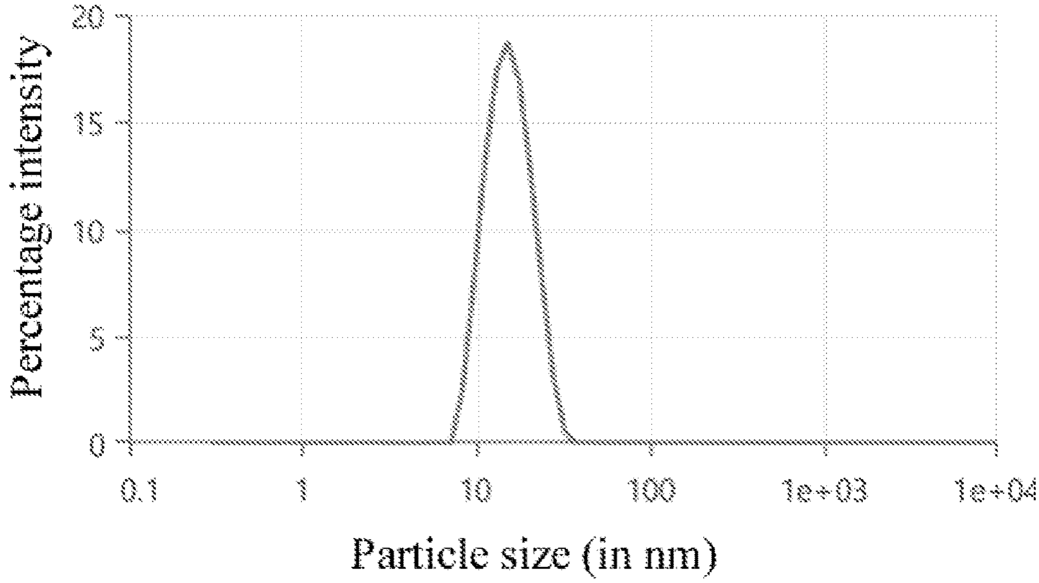


**Example 79**

FIG. 25

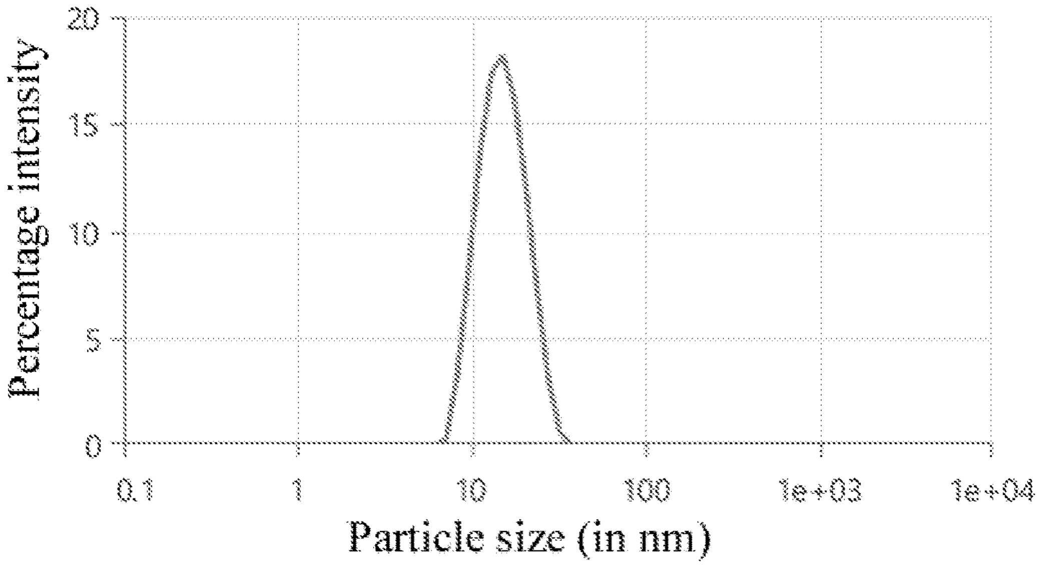


**Example 80**

FIG. 26

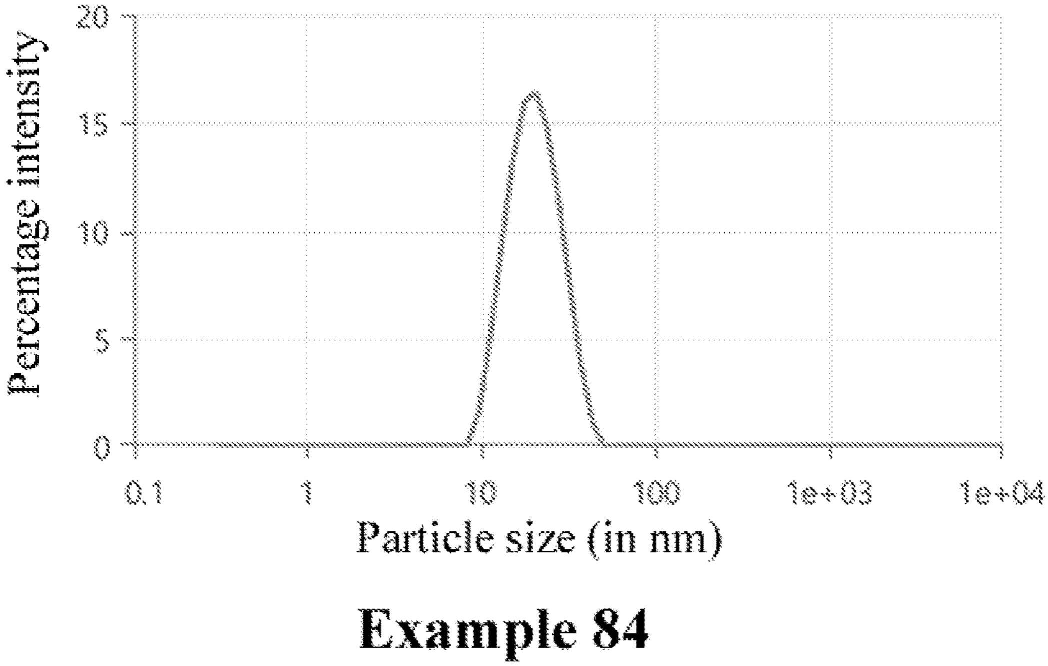
Percentage intensity
20
15
10
5
0
0.1
1
10
100
1e+03
1e+04
Particle size (in nm)
Example 84

FIG. 27

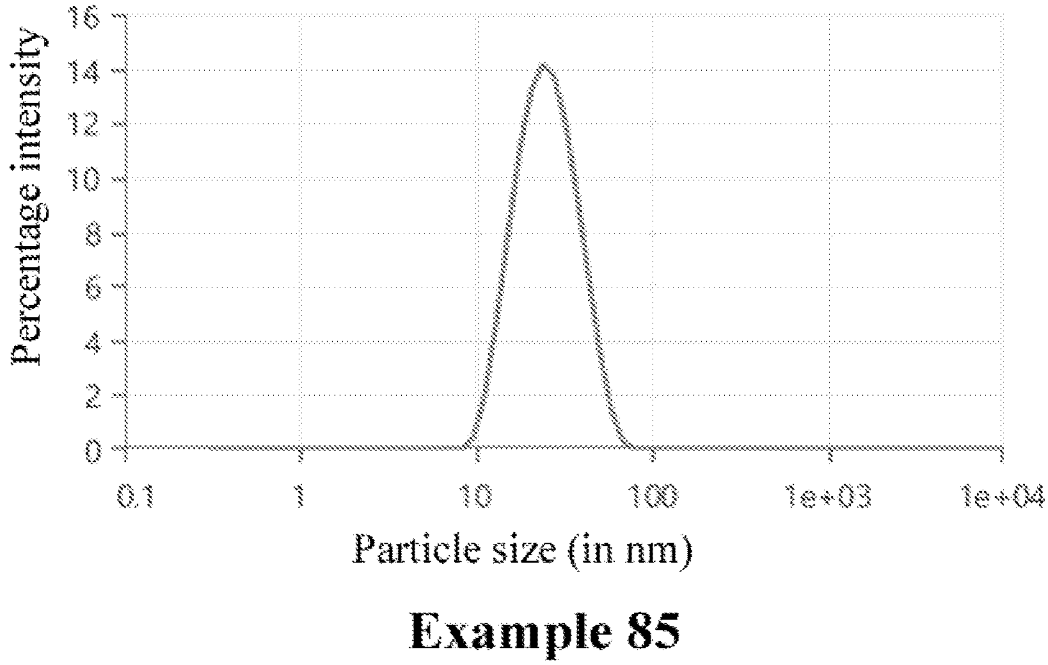
Percentage intensity
16
14
12
10
8
6
4
2
0
0.1
1
10
100
1e+03
1e+04
Particle size (in nm)
Example 85

FIG. 28

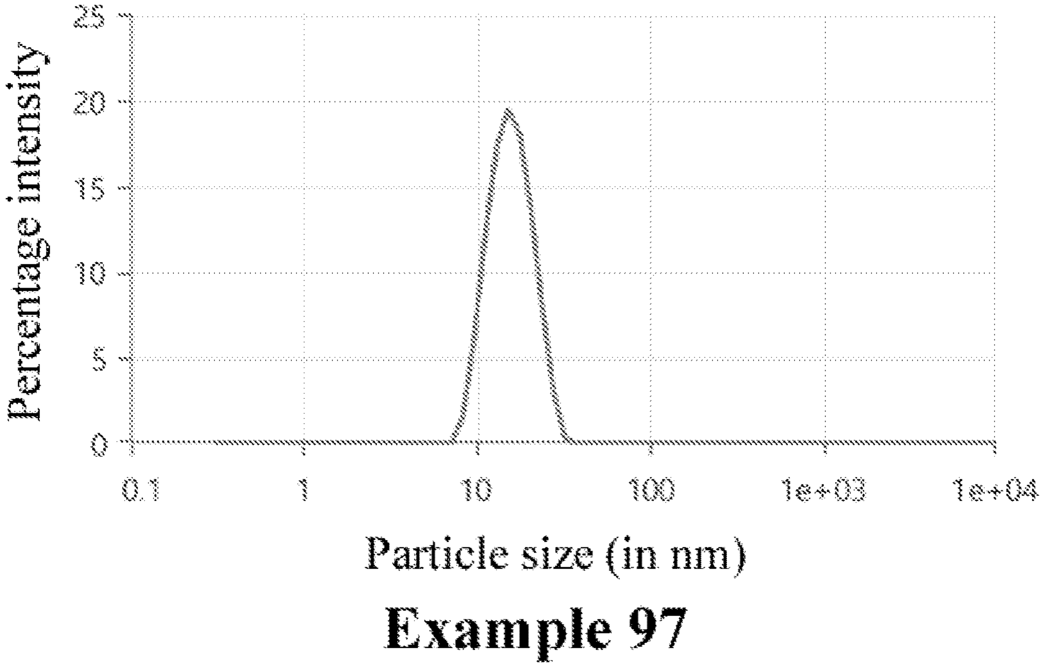
25
20
15
10
5
0
Percentage intensity
0.1
1
10
100
1e+03
1e+04
Particle size (in nm)
Example 97

FIG. 29

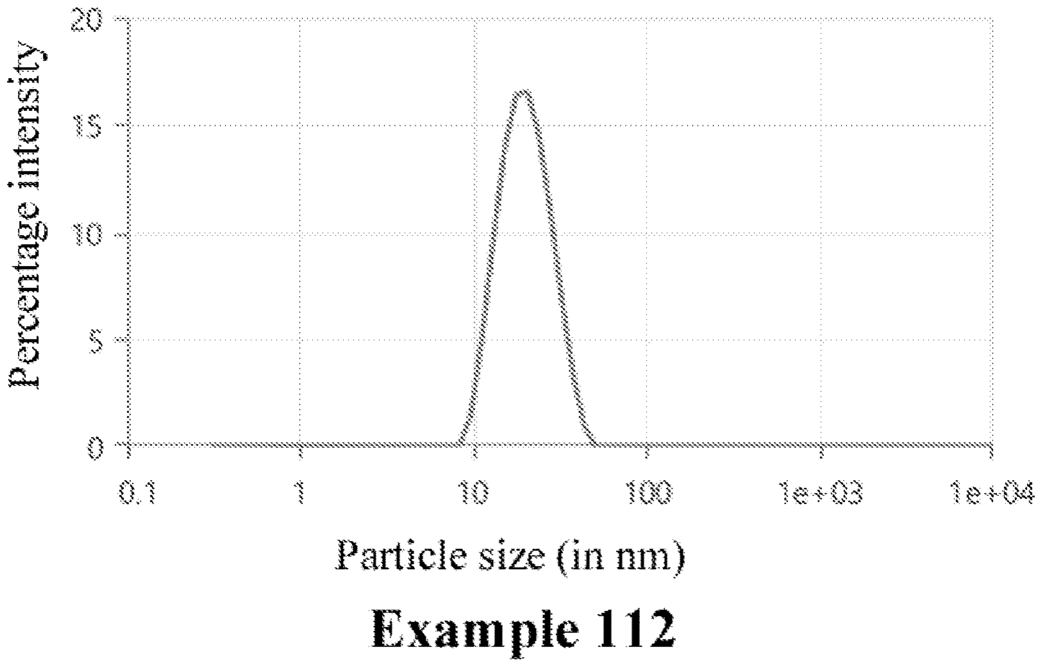
20
15
10
5
0
Percentage intensity
0.1
1
10
100
1e+03
1e+04
Particle size (in nm)
Example 112

FIG. 30

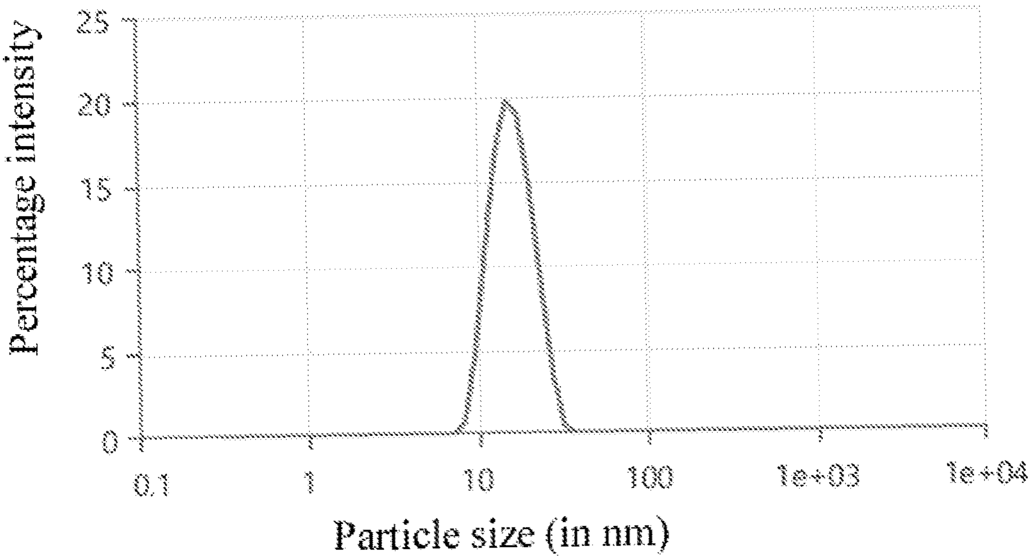


**Example 79, 25 °C, day 0**

FIG. 31

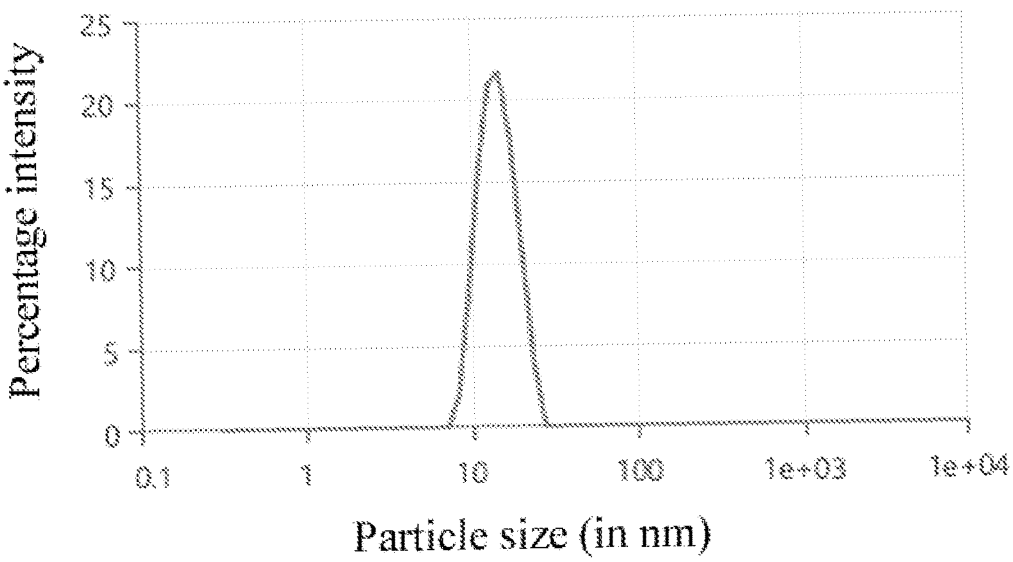


**Example 79, 25 °C, day 60**

FIG. 32

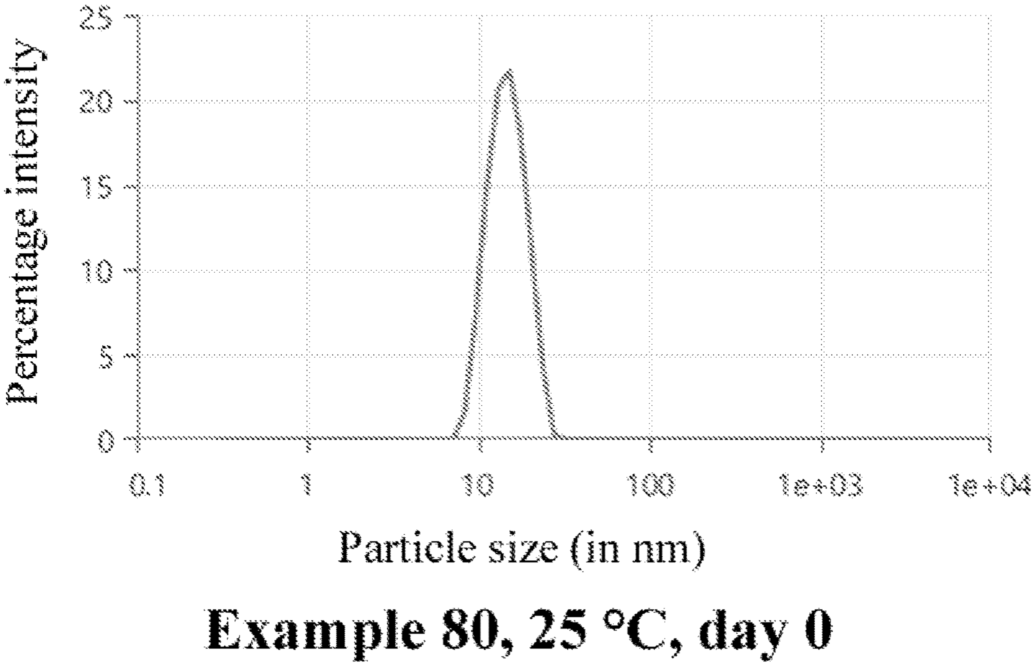
25
20
15
10
5
0
Percentage intensity
0.1
1
10
100
1e+03
1e+04
Particle size (in nm)
Example 80, 25 °C, day 0

FIG. 33

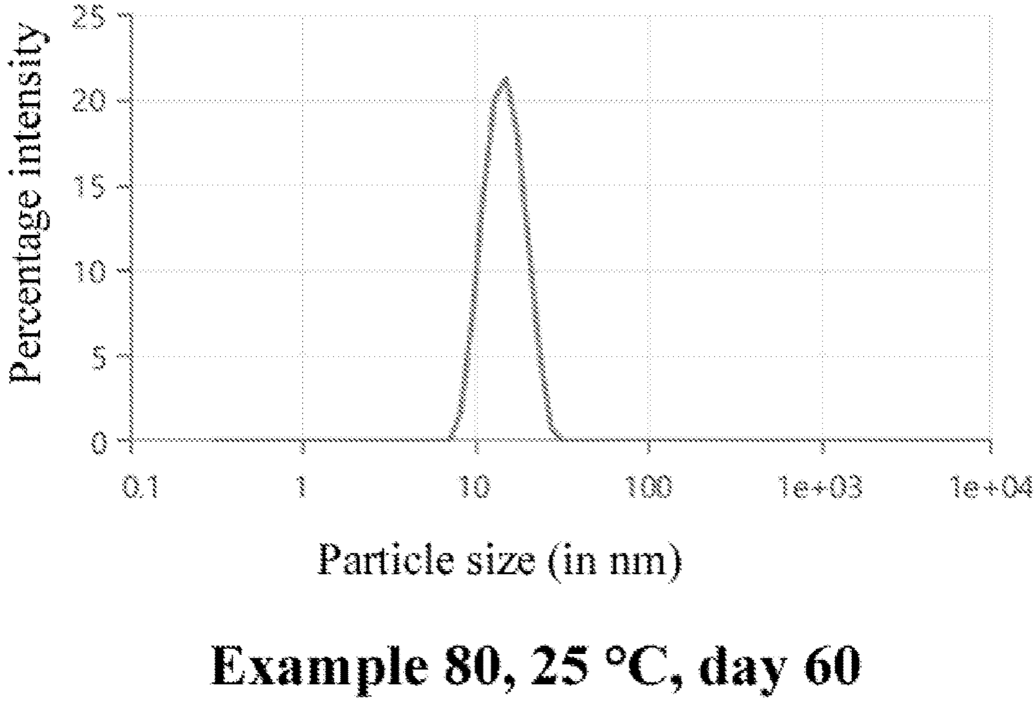
25
20
15
10
5
0
Percentage intensity
0.1
1
10
100
1e+03
1e+04
Particle size (in nm)
Example 80, 25 °C, day 60

FIG. 34

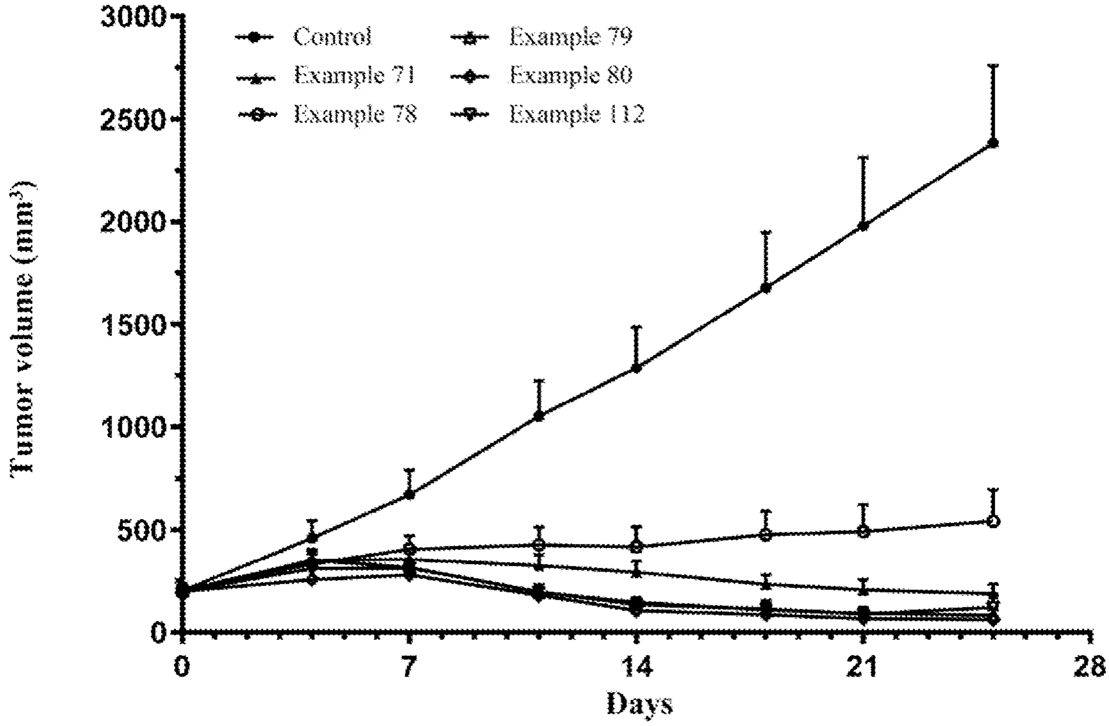
Control
Example 71
Example 78
Example 79
Example 80
Example 112
Tumor volume (mm³)
Days
0
7
14
21
28
0
500
1000
1500
2000
2500
3000

FIG. 35

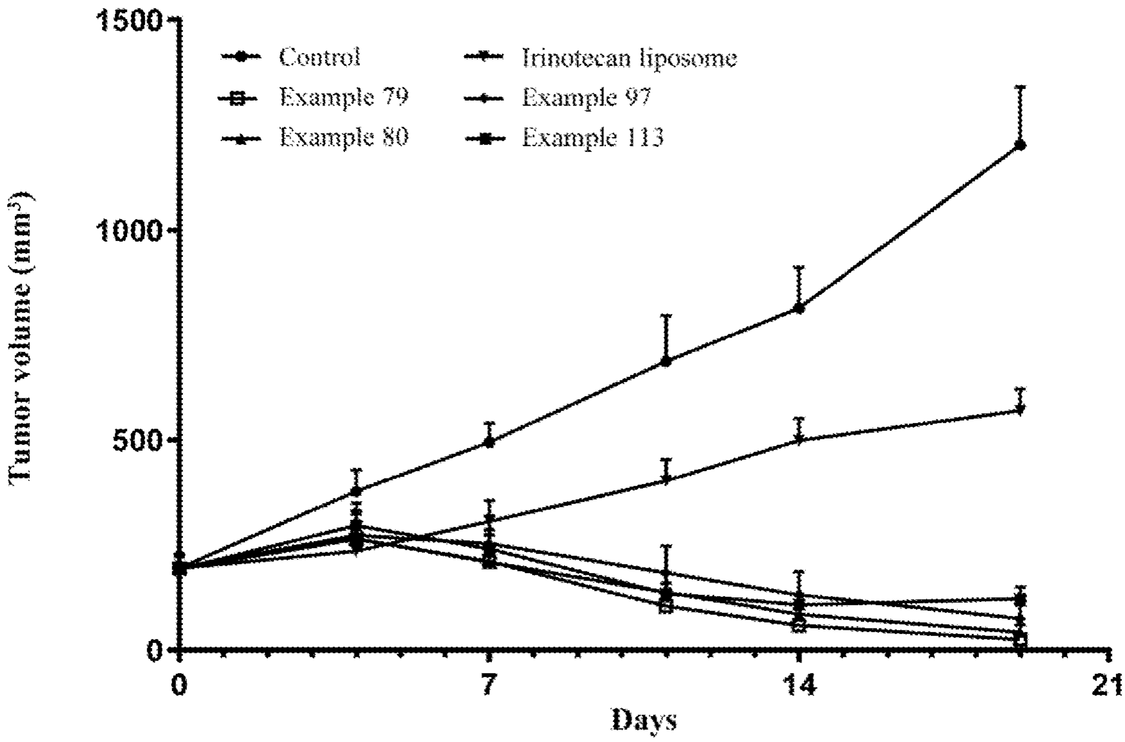
Control
Example 79
Example 80
Irinotecan liposome
Example 97
Example 113
Tumor volume (mm³)
Days
0
7
14
21
0
500
1000
1500

FIG. 36

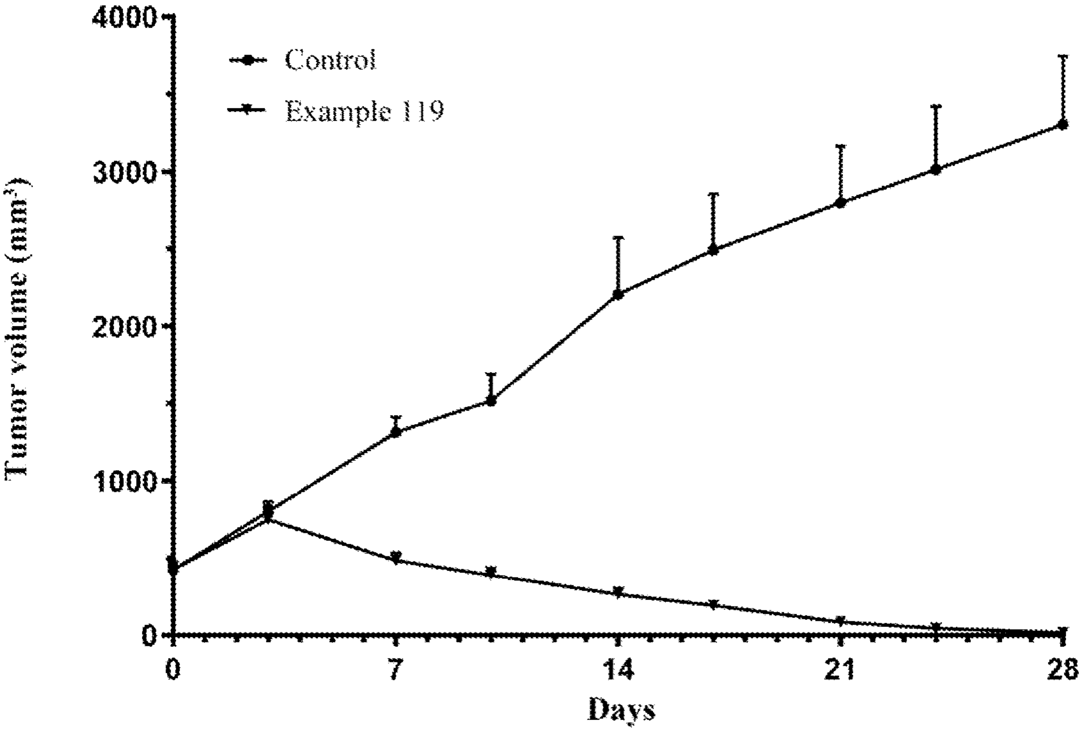
4000
3000
2000
1000
0
Tumor volume (mm³)
Control
Example 119
0
7
14
21
28
Days

FIG. 37

**INTERNATIONAL SEARCH REPORT**

International application No.
**PCT/CN2024/090418**

**A. CLASSIFICATION OF SUBJECT MATTER**

A61K47/65(2017.01)i; A61K47/54(2017.01)i; A61P35/00(2006.01)i

According to International Patent Classification (IPC) or to both national classification and IPC

**B. FIELDS SEARCHED**

Minimum documentation searched (classification system followed by classification symbols)

IPC: A61K,A61P

Documentation searched other than minimum documentation to the extent that such documents are included in the fields searched

Electronic data base consulted during the international search (name of data base and, where practicable, search terms used)

CNABS, DWPI, CNTXT, EPTXT, WOTXT, USTXT, CNKI, ISI web of science , baidu, Pubmed, STNext, NCBI: 上海弼领生物技术有限公司, 张富尧, 聚合物, 聚-赖氨酸, poly-Lys, 树枝状, dendrimers, 偶联物, conjugate, 喜树碱, CPT, PEG, PEGlated

**C. DOCUMENTS CONSIDERED TO BE RELEVANT**

| Category* | Citation of document, with indication, where appropriate, of the relevant passages | Relevant to claim No. |
|---|---|---|
| PX | WO 2023078464 A1 (SHANGHAI BEST-LINK BIOSCIENCE, LLC) 11 May 2023 (2023-05-11)<br>claims 1-43 | 1-46 |
| A | MA, B. et al. "pH-Sensitive Doxorubincin-Conjugated Prodrug Micelles with Charge-Conversion for Cancer Theray"<br>*Acta Biomaterialia,* 13 February 2018 (2018-02-13),<br>page 186, abstract, and page 187, scheme 1 | 1-46 |
| A | NOH, I. et al. "Co-Delivery of Paclitaxel and Gemcitabine via CD44-Targeting Nanocarriers as a Prodrug with Synergistic Antitumor Activity against Human Biliary Cancer"<br>*Biomaterials,* 30 March 2015 (2015-03-30),<br>pp. 763-774 | 1-46 |
| A | ZHOU, Z.X. et al. "Charge-Reversal Drug Conjugate for Targeted Cancer Cell Nuclear Drug Delivery"<br>*Adv. Funct. Mater.,* 24 September 2009 (2009-09-24),<br>pp. 3580-3589 | 1-46 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **22 July 2024** | **02 August 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

INTERNATIONAL SEARCH REPORT

International application No.
PCT/CN2024/090418

C. DOCUMENTS CONSIDERED TO BE RELEVANT

| Category* | Citation of document, with indication, where appropriate, of the relevant passages | Relevant to claim No. |
|---|---|---|
| A | MEHTA, D. et al. "Reducing Dendrimer Generation and PEG Chain Length Increases Drug Release and Promotes Anticancer Activity of PEGylated Polylysine Dendrimers Conjugated with Doxorubicin via a Cathepsin-Cleavable Peptide Linker" *Mol. Pharmaceutics,* 14 August 2018 (2018-08-14), pp. 4568-4576 | 1-46 |
| A | KAMINSKAS, L.M. et al. "The Impact of Molecular Weight and PEG Chain Length on the Systemic Pharmacokinetics of PEGylated Poly L-Lysine Dendrimers" *Molecular Pharmaceutics,* 04 May 2008 (2008-05-04), pp. 449-463 | 1-46 |

Form PCT/ISA/210 (second sheet) (July 2022)

**INTERNATIONAL SEARCH REPORT**

International application No.
**PCT/CN2024/090418**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **44**
because they relate to subject matter not required to be searched by this Authority, namely:

Claim 44 relates to a method for treating a disease as defined in PCT Rule 39.1(iv). However, the following search is still made on the basis of the pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/090418**

| Patent document cited in search report | Publication date (day/month/year) | Patent family member(s) | Publication date (day/month/year) |
|---|---|---|---|
| WO 2023078464 A1 | 11 May 2023 | AU 2022381111 A1 | 27 June 2024 |
| | | CA 3237677 A1 | 11 May 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023104743553
- WO 2014141094 A1
- WO 2016046574 A1
- US 2021093729 A1
- US 2016271270 A1
- WO 2019081455 A1
- US 2012296074 A1
- WO 2015095223 A2
- WO 2023078464 A1
- WO 2020102852 A1


### Non-patent literature cited in the description


- **ZHANG, CHENGYUAN et al.** *Acta biomaterialia*, 2017, vol. 55, 153-162
- **ZHANG, CHENGYUAN et al.** *Polymer Chemistry*, 2014, vol. 18 (5), 5227-5235
- **T. W. GREENE** ; **P. G. M. WUTS**. Protective Groups in Organic Synthesis
- *Biochem.*, 1972, vol. 11, 942-944
- *J. Med. Chem.*, 2000, vol. 43 (16), 3093-3102
- *J. Am. Chem. Soc.*, 2007, vol. 129 (37), 11653-11661
- *J. Org. Chem.*, 2001, vol. 66 (26), 8815-8830
- *Bioconjugate Chem.*, 2012, vol. 23 (8), 1610-1622
- *Eur. J. Org. Chem.*, 2021, 2383-2387
- *CHEMICAL ABSTRACTS*, 159857-60-0
- *Chem. Eur. J.*, 2015, vol. 21, 1-10
- *Bioorganic & medicinal chemistry*, 2005, vol. 13 (7), 2523-2536
- *Angewandte Chemie*, 2016, vol. 128 (7), 2596-2600
- *European Journal of Inorganic Chemistry*, 2014, vol. 2014 (3), 484-492
- *J. Am. Chem. Soc.*, 2021, vol. 143 (10), 3697-3702